(19) Europäisches Patentamt
European Patent Office
Office européen des brevets

(11) EP 1 721 283 B1

(12) **EUROPEAN PATENT SPECIFICATION**

(45) Date of publication and mention of the grant of the patent:
**30.11.2022 Bulletin 2022/48**

(21) Application number: **05718952.4**

(22) Date of filing: **07.02.2005**

(51) International Patent Classification (IPC):
***G01N 33/68*** *(2006.01)* ***G16B 40/00*** *(2019.01)*

(52) Cooperative Patent Classification (CPC):
**G01N 33/6803; G16B 20/00; G16B 20/20; G16B 20/30; G16B 40/00; G16B 40/20;**
G01N 2333/195; Y02A 90/10

(86) International application number:
**PCT/IN2005/000037**

(87) International publication number:
**WO 2005/076010 (18.08.2005 Gazette 2005/33)**

(54) **COMPUTATIONAL METHOD FOR IDENTIFYING ADHESIN AND ADHESIN-LIKE PROTEINS OF THERAPEUTIC POTENTIAL**

BERECHNUNGSVERFAHREN ZUR IDENTIFIZIERUNG VON ADHÄSIN UND ADHÄSIN-ÄHNLICHEN PROTEINEN MIT THERAPIEPOTENZIAL

PROCEDE DE CALCUL POUR IDENTIFIER L’ADHESINE ET DES PROTEINES DU TYPE DE L’ADHESINE POSSEDANT UN POTENTIEL THERAPEUTIQUE

(84) Designated Contracting States:
**DE DK FR GB SE**

(30) Priority: **06.02.2004 IN DE01732004**
**20.07.2004 US 589227 P**

(43) Date of publication of application:
**15.11.2006 Bulletin 2006/46**

(73) Proprietor: **Council of Scientific and Industrial Research**
**New Delhi 110 001,**
**Maharashtra (IN)**

(72) Inventors:
- **SACHDEVA, Gaurav**
**Mall Road,**
**Dehli 110 007 (IN)**
- **KUMAR, Kaushal**
**Mall Road,**
**Dehli 110 007 (IN)**
- **JAIN, Preti**
**Mall Road,**
**Dehli 110 007 (IN)**
- **BRAHMACHARI, Samir, Kumar**
**Mall Road,**
**New Delhi 110 007 (IN)**
- **RAMACHANDRAN, Srinivasan**
**Mall Road,**
**Dehli 110 007 (IN)**

(74) Representative: **Hirsch & Associés**
**154 Boulevard Haussmann**
**75008 Paris (FR)**

(56) References cited:
**WO-A-2005/057464**

- **ZUEGGE J ET AL: "Deciphering apicoplast targeting signals - feature extraction from nuclear-encoded precursors of Plasmodium falciparum apicoplast proteins" GENE: AN INTERNATIONAL JOURNAL ON GENES AND GENOMES, ELSEVIER, AMSTERDAM, NL, vol. 280, no. 1-2, 12 December 2001 (2001-12-12), pages 19-26, XP004313161 ISSN: 0378-1119**
- **BRADLEY PHIL ET AL: "BETAWRAP: Successful prediction of parallel beta-helices from primary sequence reveals an association with many microbial pathogens" PROCEEDINGS OF THE NATIONAL ACADEMY OF SCIENCES OF THE UNITED STATES OF AMERICA, vol. 98, no. 26, 18 December 2001 (2001-12-18), pages 14819-14824, XP002350912 ISSN: 0027-8424**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- FINLAY R BRETT ET AL: "Common themes in microbial pathogenicity revisited" MICROBIOLOGY AND MOLECULAR BIOLOGY REVIEWS, vol. 61, no. 2, 1997, pages 136-169, XP002350913 ISSN: 1092-2172
- SACHDEVA GAURAV ET AL: "SPAAN: a software program for prediction of adhesins and adhesin-like proteins using neural networks" BIOINFORMATICS (OXFORD), vol. 21, no. 4, 15 February 2005 (2005-02-15), pages 483-491, XP002350914 ISSN: 1367-4803

## Description

### Field of the present Invention

A computer implemented computational method for identifying adhesin and adhesin-like proteins; computer system for performing the method; and genes and proteins encoding adhesin and adhesin-like proteins

### Background and prior art of the present Invention

The progress in genome sequencing projects has generated a large number of inferred protein sequences from different organisms. It is expected that the availability of the information on the complete set of proteins from infectious human pathogens will enable us to develop novel molecular approaches to combat them. A necessary step in the successful colonization and subsequent manifestation of disease by microbial pathogens is the ability to adhere to host cells.

Microbial pathogens encode several proteins known as adhesins that mediate their adherence to host cell surface receptors, membranes, or extracellular matrix for successful colonization. Investigations in this primary event of host-pathogen interaction over the past decades have revealed a wide array of adhesins in a variety of pathogenic microbes. Presently, substantial information on the biogenesis of adhesins and the regulation of adhesin factors is available. One of the best understood mechanisms of bacterial adherence is attachment mediated by pili or fimbriae. Several afimbrial adhesins also have been reported. In addition, limited knowledge on the target host receptors also has been gained (Finlay, B.B. and Falkow, S 1997, "Common themes in microbial pathogenicity revisited", MICROBIOLOGY AND MOLECULAR BIOLOGY REVIEWS, vol. 61, no. 2, 1997, pages 136 - 169, XP002350913, ISSN: 1092-2172, reviews mechanisms by which pathogens adhere to host cells).

New approaches to vaccine development focus on targeting adhesins to abrogate the colonization process (Wizemann, *et al* 1999). However, the specific role of particular adhesins has been difficult to elucidate. Thus, prediction of adhesins or adhesin-like proteins and their functional characterization is likely to aid not only in deciphering the molecular mechanisms of host pathogen interaction but also in developing new vaccine formulations, which can be tested in suitable experimental model systems.

One of the best understood mechanisms of bacterial adherence is attachment mediated by pili or fimbriae. For example, FimH and PapG adhesins of *Escherichia coli* (Maurer, L., Orndorff, P.(1987), Bock, K., *et al.*(1985). Other examples of pili group adhesins include type IV pili in *Pseudomonas aeruginosa*, Neisseria species, Moraxella species, Enteropathogenic *Escherichia coli* and *Vibrio cholerae* (Sperandio V *et al* (1996).

Several afimbrial adhesins are HMW proteins of *Haemophilus influenzae* (van Schilfgaarde 2000), the filamentous hemagglutinin, pertactin, of *Bordetella pertussis* (Bassinet *et al* 2000), the BabA of *H. pylori* (Yu J *et al* 2002) and the YadA adhesin of *Yersinia enterocolitica* (Neubauer *et al* 2000). The intimin receptor protein (Tir) of Enteropathogenic *E. coli* (EPEC) is another type of adhesin (Ide T *et al* 2003). Other class of adhesins includes MrkD protein of *Kleibsella pneumoniae*, Hia of *H. influenzae* (St Geme *et al* 2000), Ag I/II of *Streptococcus mutans* and SspA, SspB of *Streptococcus gordonii* (Egland *et al* 2001), FnbA, FnbB of *Staphylococcus aureus* and Sfbl, protein F of *Streptococcus pyogenes* , the PsaA of *Streptococcus pneumoniae* (De *et al* 2003).

A known example of adhesins approved as vaccine is the acellular pertussis vaccine containing FHA and pertactin against *B. pertussis* the causative agent of whooping cough (Halperin, S *et al* 2003). Immunization with FimH is being evaluated for protective immunity against pathogenic *E. coli* (Langermann S *et al* 2000), in *Streptococcus pneumoniae*, PsaA is being investigated as a potential vaccine candidate against pneumococcal disease (Rapola, S *et al* 2003). Immunization results with BabA adhesin showed promise for developing a vaccine against *H. pylori* (Prinz, C *et al* 2003). A synthetic peptide sequence anti-adhesin vaccine is being evaluated for protection against *Pseudomonas aeruginosa* infections.

Screening for adhesin and adhesin like proteins by conventional experimental method is laborious, time consuming and expensive. As an alternative, homology search is used to facilitate the identification of adhesins. Although, this procedure is useful in the analysis of genome organization (Wolf *et al* 2001) and of metabolic pathways (Peregrin-Alvarez *et al* 2003, Rison *et al* 2002), it is somewhat limited in allowing functional predictions when the homologues are not functionally characterized or the sequence divergence is high. Assignment of functional roles to proteins based on this technique has been possible for only about 60% of the predicted protein sequences (Fraser *et al* 2000). Thus, we explored the possibility of developing a non-homology method based on sequence composition properties combined with the power of the Artificial Neural Networks to identify adhesins and adhesin-like proteins in species belonging to wide phylogenetic spectrum.

Twenty years ago, Nishikawa *et al* carried out some of the early attempts to classify proteins into different groups based on compositional analysis (Nishikawa *et al* 1983).

More recently, the software PropSearch was developed for analyzing protein sequences where conventional alignment tools fail to identify significantly similar sequences (Hobohm, U. and Sander, C 1995). PropSearch uses 144 compositional properties of protein sequences to detect possible structural or functional relationships between a new sequence and sequences in the database. Recently the compositional attributes of proteins have been used to develop softwares for predicting secretory proteins in bacteria and apicoplast targeted proteins in *Plasmodium falciparum* by training Artificial Neural Networks (Zuegge et al 2001: "Deciphering apicoplast targeting signals - feature extraction from nuclear-encoded precursors of Plasmodium falciparum apicoplast proteins", GENE: AN INTERNATIONAL JOURNAL ON GENES AND GENOMES, ELSEVIER, AMSTERDAM, NL, vol. 280, no. 1-2, 12 December 2001 (2001-12-12), pages 19 - 26, XP004313161, ISSN: 0378-1119).

Zuegge et al have used the 20 amino acid compositional properties. Their objective was to extract features of apicoplast targeted proteins in *Plasmodium falciparum.* This is distinct from our software SPAAN that focuses on adhesins and adhesin-like proteins involved in host-pathogen interaction.

Hobohm and Sander have used 144 compositional properties including isoelectric point and amino acid and dipeptide composition to generate hypotheses on putative functional role of proteins that are refractory to analysis using other sequence alignment based approaches like BLAST and FASTA. Hobohm and Sander do not specifically address the issue of adhesins and adhesin-like proteins, which is the focus of SPAAN. Nishikawa et al had originally attempted to classify proteins into various functional groups. This was a curiosity driven exercise but eventually lead to the development of a software to discriminate extra-cellular proteins from intracellular proteins. This work did not address the issue of adhesins and adhesin-like proteins, which is the focus of SPAAN.

BRADLEY PHIL ET AL: "BETAWRAP: Successful prediction of parallel beta-helices from primary sequence reveals an association with many microbial pathogens", PROCEEDINGS OF THE NATIONAL ACADEMY OF SCIENCES OF THE UNITED STATES OF AMERICA, vol. 98, no. 26, 18 December 2001 (2001-12-18), pages 14819 - 14824, XP002350912, ISSN: 0027-8424 discloses a computational method for the prediction of parallel beta helices which identifiens bacterial and fungal sequences that play a role in human infectous disease among whichadhesins can be found.Thus, none of the aforementioned research groups have been able to envisage the methodology of the instant application. The inventive method of this application provides novel proteins and corresponding gene sequences.

Adhesins and adhesin-like proteins mediate host-pathogen interactions. This is the first step in colonization of a host by microbial pathogens. Attempts Worldwide are focused on designing vaccine formulations comprising adhesin proteins derived from pathogens. When immunized, host will have its immune system primed against adhesins for that pathogen. When a pathogen is actually encountered, the surveillance mechanism will recognize these adhesins, bind them through antigen-antibody interactions and neutralize the pathogen through complement mediate cascade and other related clearance mechanisms. This strategy has been successfully employed in the case of Whooping cough and is being actively pursued in the case of Pneumonia, Gastric Ulcer and Urinary tract infections.

**Objects of the present Invention**

The main object of the present invention is to provide a computer implemented computational method for identifying adhesin and adhesin-like proteins of therapeutic potential.

Another object of invention is to provide a method for screening the proteins with unique compositional characteristics as putative adhesins in different pathogens. The invention is set out in the apended set of claims. Embodiments described herein, which do not fall within the scope of the claims are not part of the invention.

**Summary of the present Invention**

A computer implemented computational method for identifying adhesin and adhesin-like proteins, according to claim 1. Another object of the invention relates to a computer system for performing the method of claim 1.

**Detailed description of the present Invention**

Accordingly, the present invention relates to a computational method for identifying adhesin and adhesin-like proteins, said method comprising steps of computing the sequence-based attributes of protein sequences using five attribute modules of software SPAAN, (i) amino acid frequencies, (ii) multiplet frequency, (iii) dipeptide frequencies, (iv) charge composition, and (v) hydrophobic composition, training the artificial neural Network (ANN) for each of the computed five attributes, and identifying the adhesin and adhesin-like proteins having probability of being an adhesin (Pad) as ≥ 0.51; a computer system for performing the method; and genes and proteins encoding adhesin and adhesin-like proteins.

In an embodiment of the present invention, wherein the invention relates to a computational method for identifying adhesin and adhesin-like proteins, said method comprising steps of:

a. computing the sequence-based attributes of protein sequences using five attribute modules of a neural network software, wherein the attributes are software, (i) amino acid frequencies, (ii) multiplet frequency, (iii) dipeptide frequencies, (iv) charge composition, and (v) hydrophobic composition,

b. training the artificial neural Network (ANN) for each of the computed five attributes, and

c. identifying the adhesin and adhesin-like proteins having probability of being an adhesin (Pad) as ≥ 0.51 <insert page 5a>

In another embodiment of the present invention, wherein the invention relates to a method wherein the protein sequences is obtained from pathogens, eukaryotes, and multicellular organisms.

In an embodiment of the present invention, wherein the invention relates to a method, wherein the protein sequences are obtained from the pathogens selected from a group of organisms comprising *Escherichia coli*, *Haemophilus influenzae*, *Helicobacter pylori, Mycoplasma pneumoniae, Mycobacterium tuberculosis, Rickettsiae prowazekii, Porphyromonas gingivalis, Shigella flexneri, Streptococcus mutans, Streptococcus pneumoniae*, *Neisseria meningitides*, *Streptococcus pyogenes*, *Treponema pallidum* and Severe Acute Respiratory Syndrome associated human coronavirus (SARS ).

In yet another embodiment, the method is a non-homology method based on sequence composition properties combined with the power of the Artificial Neural Networks to identify adhesins and adhesin-like proteins in species belonging to wide phylogenetic spectrum.

In yet another embodiment of the present invention, wherein the method of the invention is a non-homology method.

In still another embodiment of the present invention, wherein the invention relates to the method using 105 compositional properties of the sequences.

In still another embodiment of the present invention, wherein the invention relates to a method showing sensitivity of at least 90%.

In still another embodiment of the present invention, wherein the invention relates to the method showing specificity of 100%.

In still another embodiment of the present invention, wherein the invention relates to a method identifying adhesins from distantly related organisms.

In still another embodiment of the present invention, wherein the invention relates to the neural network has multi-layer feed forward topology, consisting of an input layer, one hidden layer, and an output layer.

In still another embodiment of the present invention, wherein the invention relates to the number of neurons in the input layer are equal to the number of input data points for each attribute.
wherein the "$P_{ad}$" is a weighted linear sum of the probabilities from five computed attributes, wherein

- the amino acid frequency $f_i$ = (counts of ith amino acid in the sequence) / 1 ; i, = 1...20, 1 is the length of the protein,
- the multiplets are defined as homopolymeric stretches $(X)_n$ where X is any of the 20 amino acids and n is an integer > 2. After identifying all the multiplets, the frequencies of the amino acids in the multiplets were computed as

$$f_i(m) = (\text{counts of } i^{th} \text{ amino acid occurring as multiplet}) / l;$$

- the frequency of a dipeptide (i, j) $f_{ij}$ = (counts of $ij^{th}$ dipeptide) / (total dipeptide counts); i, j ranges from 1 to 20.
- the charge composition refers to the input frequency of charged amino acids (R, K, E and D considering the ionization properties of the side chains at pH 7.2) given by $f_c$ = (counts of charged amino acids) / 1, distribution of said charged amino acids in a given protein sequence is provided by computing the moments of the positions of the occurrences of the charged amino acids; and general expression to compute moments of a given order; say 'i' is

$$M_r = r^{th} \text{ order moment of the positions of charged amino acids}$$

$$= \sum \frac{(X_i - X_m)^r}{N}$$

where, $X_m$ = mean of all positions of charged amino acids

$X_i$ = position of $i^{th}$ charged amino acid
N = number of charged amino acids in the sequence

- the hydrophobic composition refers to the hydrophobic scores of five groups of amino acids: (-8 for K, E, D, R), (-4 for S, T, N, Q), (-2 for P, H), (+1 for A, G, Y, C, W), (+2 for L, V, I, F, M) with the following inputs given for each of the group

(a) $f_i$ = (counts of $i^{th}$ group) / (total counts in the protein); i ranges from 1 to 5
(b) $m_{ji}$ = $j^{th}$ order moment of positions of amino acids in $i^{th}$ group; j ranges from 2 to 5.

According to the invention, the "Pad" is a weighted linear sum of the probabilities from five computed attributes.
In still another embodiment of the present invention, wherein the invention relates to each trained network assigns a probability value of being an adhesin for the protein sequence.
Another object of the present invention, relates to a computer system for performing the method of claim 1, said system comprising

- a central processing unit, executing a software program, for prediction of adhesion and adhesion-like proteins using neural networks, giving probabilities based on different attributes using Artificial Neural Network and in built other programs of assessing attributes, all stored in a memory device accessed by CPU,
- a memory device accessed by the CPU;
- a display on which the central processing unit displays the screens of the above mentioned programs in response to user inputs; and,
- a user interface device.

The following embodiments are not according to the invention and are present for illustration purposes :

- a set of 274 annotated genes encoding adhesin and adhesin-like proteins, having SEQ ID Nos. 385 to 658.
- a set of 105 hypothetical genes encoding adhesin and adhesin-like proteins, having SEQ ID Nos. 659 to 763. a set of 279 annotated adhesin and adhesin-like proteins of SEQ ID Nos. 1 to 279.
- a set of 105 hypothetical adhesin and adhesin-like proteins of SEQ ID Nos. 280 to 384.

One more embodiment of the present invention, wherein the invention also relates to a fully connected multilayer feed forward Artificial Neural Network based on the computational method as claimed in claim 1, comprising of an input layer, a hidden layer and an output layer which are connected in the said sequence, wherein each neuron is a binary digit number and is connected to each neuron of the subsequent layer for identifying adhesin or adhesin like proteins, wherein the program steps comprise:-[a] feeding a protein sequence in FASTA format; [b] processing the sequence obtained in step [a] through the 5 modules named A, C, D, H and M, wherein attribute A represents an amino acid composition, attribute C represents a charge composition, attribute D represents a dipeptide composition of the 20 dipeptides [NG, RE, TN, NT, GT, TT, DE, ER, RR, RK, RI, AT, TS, IV, SG, GS, TG, GN, VI and HR], attribute H represents a hydrophobic composition and attribute M represents amino acid frequencies in multiplets to quantify 5 types of compositional attributes of the said protein sequence to obtain numerical input vectors respectively for each of the said attributes wherein the sum of numerical input vectors is 105;

[c] processing of the numerical input vectors obtained in step [b] by the input neuron layer to obtain signals, wherein the number of neurons is equal to the number of numerical input vectors for each attribute;
[d] processing of signals obtained from step [c] by the hidden layer to obtain synaptic weighted signals, wherein the optimal number of neurons in the hidden layer was determined through experimentation for minimizing the error at the best epoch for each network individually;
[e] delivering synaptic weighted signals obtained from step [d] to the output layer for assigning of a probability value for each protein sequence fed in step [a] as being an adhesin by each network module;
[f] using the individual probabilities obtained from step [e] for computing the final probability of a protein sequence being an adhesin denoted by the $P_{ad}$ value, which is a weighted average of the individual probabilities obtained from step [e] and the associated fraction of correlation which is a measure of the strength of the prediction.

In still another embodiment of the present invention, wherein the input neuron layer consists of a total of 105 neurons corresponding to 105 compositional properties.
In still another embodiment of the present invention, wherein the hidden layer comprises of neurons represented as 30 for amino acid frequencies, 28 for multiplet frequencies, 28 for dipeptide frequencies, 30 for charge composition and 30 for hydrophobic composition.
In still another embodiment of the present invention, wherein the output layer comprises of neurons to deliver the output values as probability value for each protein sequence.
Identification of novel adhesins and their characterization are important for studying host-pathogen interactions and testing new vaccine formulations. We have employed Artificial Neural Networks to develop an algorithm SPAAN (Software for Prediction of Adhesin and Adhesin-like proteins using Neural Networks) that can identify adhesin proteins using 105 compositional properties of a protein sequence. SPAAN could correctly predict well characterized adhesins from several bacterial species and strains. SPAAN showed 89% sensitivity and 100% specificity in a test data set that did not contain proteins in the training set. Putative adhesins identified by the software can serve as potential preventive therapeutics.

The present invention provides a novel computational method for identifying adhesin and adhesin-like proteins of therapeutic potential. More particularly, the present application describes candidate genes for these adhesins. The invention further provides new leads for development of candidate genes, and their encoded proteins in their functional relevance to preventive approaches. This computational method involves calculation of several sequence attributes and their subsequent analyses lead to the identification of adhesin proteins in different pathogens. Thus, the present invention is useful for identification of the adhesin proteins in pathogenic organisms. The adhesin proteins from different genomes constitute a set of candidates for functional characterization through targeted gene disruption, microarrays and proteomics. Further, these proteins constitute a set of candidates for further testing in development of preventive therapeutics. Also, are provided the genes encoding the candidate adhesin proteins.

The present method offers novelty in the principles used and the power of Neural Networks to identify new adhesins compared to laborious and time consuming conventional methods. The present method is based on compositional properties of proteins instead of sequence alignments. Therefore this method has the ability to identify adhesin and adhesin like proteins from bacteria belonging to a wide phylogenetic spectrum. The predictions made from this method are readily verifiable through independent analysis and experimentation. The invention has the potential to accelerate the development of new preventive therapeutics, which currently requires high investment in terms of requirement of skilled labor and valuable time.

The present invention relates to a computational method for the identification of candidate adhesin proteins of therapeutic potential. The invention particularly describes a novel method to identify adhesin proteins in different genomes of pathogens. These adhesin proteins can be used for developing preventive therapeutics.

Accordingly, a computational method for identifying adhesin and adhesin-like proteins of therapeutic potential which comprises calculation of 105 compositional properties under the five sequence attributes, namely, Amino Acid frequency, Multiplet frequency, dipeptide frequency, charge composition and hydrophobic composition; and then training Artificial Neural Network (ANN, Feed Forward Error Back Propagation) using these properties for differentiating between adhesin and non-adhesin class of proteins. This computational method involves quantifying 105 compositional attributes of query proteins and qualifying them as adhesins or non-adhesins by a $P_{ad}$ value (Probability of being an adhesin). The present invention is useful for identification of adhesin and adhesin-like proteins in pathogenic organisms. These newly identified adhesin and adhesin-like proteins constitute a set of candidates for development of new preventive therapeutics that can be tested in suitable experimental model systems readily. In addition, the genes encoding the candidate adhesin and adhesin-like proteins are provided.

The application provides a set of candidate adhesin and adhesin-like proteins and their coding genes for further evaluation as preventive therapeutics. The method of the invention is based on the analysis of protein sequence attributes instead of sequence patterns classified to functional domains. Present method is less dependent on sequence relationships and therefore offers the potential power of identifying adhesins from distantly related organisms. The invention provides a computational method, which involves prediction of adhesin and adhesin-like proteins using Artificial Neural Networks. The proteins termed adhesin were found to be predicted with a high probability (Pad $\geq$ 0.51) in various pathogens. Some adhesin sequences turned out to be identical or homologous to proteins that are antigenic or implicated in virulence. By this approach, proteins could be identified and short-listed for further testing in development of new vaccine formulations to eliminate diseases caused by various pathogenic organisms.

## DESCRIPTION OF TABLES

**Table 1:** Output file format given by SPAAN.
**Table 2:** Organism Name, Accession number, Number of base pairs, Date of release and Total number of proteins.
**Table 3.** Prediction of well characterized adhesins from various bacterial pathogens using SPAAN.
**Table 4**. Analysis of predictions made by SPAAN on genome scans of a few selected pathogenic organisms.
**Table 5:** GI numbers and Gene IDs of new putative adhesins predicted by SPAAN in the genomes listed in Table 2.
**Table 6:** GI numbers and Gene IDs of hypothetical proteins predicted as putative adhesins by SPAAN in the genomes listed in Table 2.
**Table 7:** The list of 198 adhesins found in bacteria

**Brief description of the accompanying drawings**

**Figure 1** shows the Neural Network architecture
**Figure 2** shows assessment of SPAAN using defined test dataset.
**Figure 3 (a)** shows Histogram plots of the number of proteins in the various $P_{ad}$ value ranges are shown. **(b)** Pairwise sequence relationships among the adhesins were determined using CLUSTAL W and plotted on X-axis. Higher scores indicate similar pairs. **(c)** plot for non-adhesins. Data are plotted in the 4 quadrant format for clear inspection.

Software program was written in C Language and operated on Red Hat Linux 8.0 operating system. The computer program accepts input protein sequences in FastA format and produces a tabulated output. The output Table contains one row for each protein listing the probability outputs of each of the five modules, a weighted average probability of these five modules ($P_{ad}$), and the function of the protein as described in the input sequence file. This software is called SPAAN (A Software for Prediction of Adhesins and Adhesin-like proteins using Neural Networks) and a software copyright has been filed. Although this software has multiple modules, the running of these modules have been integrated and automated. The user only needs to run one command.

**AAcompo.c:**

**Input:** File containing protein sequences in the fasta format.
**Output:** File containing frequencies of all 20 AAs for each protein in one row.

**charge.c:**

**Input:** File containing protein sequences in the fasta format.
**Output:** File containing frequency of charged amino acids (R, K, E and D) and moments (up to 18th order) of the positions of charged amino acids.

**hdr.c:**

**Input:** File containing protein sequences in the fasta format.
**Output:** File containing frequencies of 5 groups of amino acids formed on the basis their Hydrophobicity and moments of their positions up to 5th order.

**multiplets.c:**

**Input:** File containing protein sequences in the fasta format.
**Output:** File containing fractions of multiplets of each of the 20 amino acids.

**querydipep.c**:

**Input:**

File.1 containing protein sequences in the fasta format.
File.2 containing list of the significant dipeptides in dipeptide analysis.

**Output:** File containing frequencies of the dipeptides listed in the input File.2 for each protein in the input File.1.

**train.c:**

**Input:** File containing following specifications -

1. Number of input and output parameters.
2. Number of nodes in the hidden layers.
3. Names of the training, validate and test data files.
4. Learning rate, coefficient of moment.
5. Maximum number of cycles for training.

**Output:** Outputs are as follows.

1. Output of the trained NN for the test data set.
2. Values of the weight connections in the trained NN.
3. Some extra information about training.

**recognize.c**:

**Input:** File containing following specifications -

1. Number of input and output parameters.
2. Number of nodes in the hidden layers.
3. Names of the query input file.
4. Name of the file containing values of the weight connections for trained NN.
5. Name of the output file.

**Output:** Outputs for the query entries calculated by the trained NN.

**standard.c**:

**Input:** File containing protein sequences in fasta format.
**Output:** File containing protein sequences in fasta format with all the new line characters removed lying within a sequence.

**filter.c:**

**Input:** File containing protein sequences in fasta format.
**Output:** File containing protein sequences from the input except those which are short in length (<50 AAs) and which contain any amino acid other than the 20 known amino acids.

***The five attributes:***

Amino Acid frequencies

Amino acid frequency $f_i$ = (counts of ith amino acid in the sequence) / 1 ; i, = 1...20, 1 is the length of the protein.

Multiplet frequency

Multiplets are defined as homopolymeric stretches $(X)_n$ where X is any of the 20 amino acids and n is an integer > 2. After identifying all the multiplets, the frequencies of the amino acids in the multiplets were computed as

$$f_i(m) = (\text{counts of } i^{th} \text{ amino acid occurring as multiplet}) / l$$

Dipeptide frequencies

The frequency of a dipeptide (i, j) $f_{ij}$ = (counts of ij$^{th}$ dipeptide) / (total dipeptide counts); i, j ranges from 1 to 20.
It has been found that dipeptide repeats in proteins are important for functional expression of the clumping factor present on *Staphylococcus aureus* cell surface that binds to fibrinogen (Hartford *et al* 1999). Thus we included the dipeptide frequency module. The total number of dipeptides is 400. For optimal training of Neural Network, the ratio of total number of input vectors to the total number of weight connections must be around 2 to avoid over fitting (Andrea *et al*). Therefore, we identified the dipeptides whose frequencies in the adhesin data set (469 proteins, see database construction) were significantly different from that in the non-adhesin dataset (703 proteins) using *t-test.* The frequencies of top 20 dipeptides (when arranged in the descending order of the p-values of *t-test*), were fed to the Neural Network. These dipeptides were (using single letter IUPAC-IUB code) NG, RE, TN, NT, GT, TT, DE, ER, RR, RK, RI, AT, TS, IV, SG, GS, TG, GN, VI, AND HR. With frequency inputs for 20 dipeptides and 28 neurons in the 2nd layer, the total number of weight connections is 588, and is in keeping with the criterion of avoiding over fitting.

Charge composition

The input frequency of charged amino acids (R, K, E and D considering the ionization properties of the side chains at pH 7.2) given by $f_c$ = (counts of charged amino acids) / 1 Further, information on the characteristics of the distribution of the charged amino acids in a given protein sequence was provided by computing the moments of the positions of the occurrences of the charged amino acids. Since moments characterize the patterns of distribution such as skewness and kurtosis (sharpness of the peak) we have used them to represent the distribution patterns of the charged residues in the sequence.
The general expression to compute moments of a given order; say 'i' is

$$M_r = r^{th} \text{ order moment of the positions of charged amino acids}$$
$$= \sum \frac{(X_i - X_m)^r}{N}$$

Where,

$X_m$ = mean of all positions of charged amino acids
$X_i$ = position of i$^{th}$ charged amino acid
N = number of charged amino acids in the sequence

The moments 2$^{nd}$ to 19$^{th}$ order were used to train the ANN constituting a total 20 inputs in addition to frequency of charged amino acids and the length of the protein. The upper limit of 19$^{th}$ order was set based on assessments of sensitivity and specificity on a small dataset of adhesins and non-adhesins. Moments of order greater than 19 were not useful in improvement of performance.

Hydrophobic composition

A given protein sequence was digitally transformed using the hydrophobic scores of the amino acids according to Brendel *et al.* (43). The scores for five groups of amino acids: (-8 for K, E, D, R), (-4 for S, T, N, Q), (-2 for P, H), (+1 for A, G, Y, C, W), (+2 for L, V, I, F, M).
Following inputs were given for each of the group

(a) $f_i$ = (counts of i$^{th}$ group) / (total counts in the protein); i ranges from 1 to 5
(b) $m_{ji}$ = j$^{th}$ order moment of positions of amino acids in i$^{th}$ group; j ranges from 2 to 5.

A total of 25 inputs representing the hydrophobic composition of a protein were fed to the Neural Network. The rationale for using moments was same as described in the section on charge composition inputs.
Taken together a total of 105 compositional properties of a given protein sequence were used to predict their adhesin characteristics.

The software PropSearch uses 144 compositional properties of protein sequences to detect possible structural or functional relationships between a new sequence and sequences in the database (Hobohm and Sander 1995). The approach defines protein sequence dissimilarity (or distance) as a weighted sum of differences of compositional properties such as singlet and doublet amino acid composition, molecular weight, isoelectric point (protein property search or PropSearch). Compositional properties of proteins have also been used for predicting secretory proteins in bacteria and apicoplast targeted proteins in Plasmodium falciparum (Zuegge, et al. 2001). The properties used here are statistical methods, principal component analysis, self-organizing maps, and supervised neural networks. In SPAAN, we have used 105 compositional properties in the five modules viz. Amino Acid frequencies, Multiplet frequencies, Dipeptide frequencies, Charge composition, Hydrophobic composition. The total of 105 properties used in SPAAN are 20 for Amino acid frequencies, 20 for Multiplets frequencies, 20 for Dipeptide frequencies (Top 20 significant dipeptides are used, based on *t-test*), 20 for Charge composition (frequency of charged amino acids (R, K, E and D) and moments of 2nd to 19th order), and 25 for Hydrophobic composition (Amino acids were classified into five groups (-8 for K, E, D, R), (-4 for S, T, N, Q), (-2 for P, H), (+1 for A, G, Y, C, W), (+2 for L, V, I, F, M). A total of 25 inputs consisted of the following: Frequency of each group, Moments of positions of amino acids in each group from 2nd to 5th order.

*Neural Network*

A feed forward error back propagation Neural Network was used. The program is a kind gift from Charles W. Anderson, Department of Computer Science, Colorado State University, Fort Collins, CO 80523, anderson@cs.colostate.edu

*Neural Network architecture*

The Neural Network used here has a multi-layer feed-forward topology. It consists of an input layer, one hidden layer and an output layer. This is a 'fully-connected' Neural Network where each neuron *i* is connected to each unit *j* of the next layer (Figure 1).

The weight of each connection is denoted by $w_{ij}$. The state $I_i$ of each neuron in the input layer is assigned directly from the input data, whereas the states of hidden layer neurons are computed by the sigmoid function,

$$h_j = 1 / (1 + \exp -(w_{j0} + \quad w_{ij} I_i)),$$

where, $w_{j0}$ is the bias weight

The back propagation algorithm was used to minimize the differences between the computed output and the desired output. Ten thousand cycles (epochs) of iterations are performed. Subsequently, the best epoch with minimum error was identified. At this point the network produces approximate target values for a given input in the training set.

A network was trained optimally for each attribute. Thus five networks were prepared. The schematic diagram (Figure 1) shows the procedure adopted. The number of neurons in the input layer was equal to the number of input data points for each attribute (for example 20 neurons for 20 numerical input vectors of the amino acid composition attribute). The optimal number of neurons in the hidden layer was determined through experimentation for minimizing the error at the best epoch for each network individually. An upper limit for the total number of weight connections was set to half of the total number of input vectors to avoid over fitting as suggested previously (Andrea *et al*).

Computer programs to compute individual compositional attributes were written in C and executed on a PC under Red Hat Linux ver 7.3 or 8.0. The network was trained on the training set, checks error and optimizes using the validate set through back propagation. The validate set was different from the training set. Since, the number of well annotated adhesins were not many, we used the 'validate set' itself as test set for preliminary evaluation of the performance and to obtain the fraction of correlation to compute the weighted average probability ($P_{ad}$ value) described in the next section. The training set had 367 adhesins and 580 non-adhesins. The validate set had 102 adhesins and 123 non-adhesins. The adhesins were qualified with a digit '1' and the non-adhesins were qualified with a digit '0'.

During predictions, the network is fed with new data from the sequences that were not part of training set. Each network assigns a probability value of being an adhesin to a given sequence. The final probability is computed as described in the next section.

*Probability of being an adhesin, the $P_{ad}$ value*

Query proteins are processed modularly through network trained for each attribute. Thus, five probability outputs are obtained. Final prediction was computed using the following expression which is a weighted linear sum of the probabilities from five modules:

$$P_{ad} = \frac{(P_A * fc_A + P_C * fc_C + P_D * fc_D + P_H * fc_H + P_M * fc_M)}{(fc_A + fc_C + fc_D + fc_H + fc_M)}$$

$P_i$ = Probability from i module,
$fc_i$ = fraction of correlation of i module of the trained Neural Network,
Where i = A (Amino acid frequencies), C (Charge composition), D (Dipeptide frequencies), H (Hydrophobic composition), or M (Multiplet frequencies).

The fraction of correlation $fc_i$ represents the fraction of total entries that were correctly predicted ($P_{i,adhesin} > 0.5$ and $P_i$, non-adhesin < 0.5) by the trained network on the test set used in preliminary evaluation (Charles Anderson).

**Neural Network**

A feed forward error back propagation Neural Network was used. The program was downloaded from the web site with permission from the author, Charles W. Anderson, Department of Computer Science, Colorado State University, Fort Collins, CO 80523, anderson@cs.colostate.edu

**Statistical Analysis**

All statistical procedures were carried out using Microsoft Excel (Microsoft Corporation Inc. USA).

**Sequence analysis**

Homology analysis was carried out using CLUSTAL W (Thompson *et al* 1994), BLAST (Altschul *et al* 1990), CDD (conserved domain database) search (Marchler-Bauer *et al* 2002).

The whole genome sequences of microbial pathogens present new opportunities for the development of clinical applications such as diagnostics and vaccines. The present invention provides new leads for the development of candidate genes, and their encoded proteins in their functional relevance to preventive therapeutics.

The protein sequences of both the classes, i.e. adhesin and non-adhesin, were downloaded from the existing database (National Centre for Biotechnology Information (NCBI), USA). A total of 105 compositional properties under the five sequence attributes namely, amino acid composition, multiplet composition, dipeptide composition, charge composition and hydrophobic composition were computed by computer programs written in C language. The attributes were computed for all the proteins in both the databases. The sequence-based attributes were then used to train Artificial Neural Network for each of the protein attributes. Adhesins were qualified by the digit '1' and non-adhesins were qualified by the digit '0'. Finally each trained Artificial Neural Network was used to identify potential adhesins which can be envisaged to be useful for the development of preventive therapeutics against pathogenic infections. Accordingly, the invention provides a computational method for identifying adhesin and adhesin-like proteins of therapeutic potential, which comprises:

1. preparing two comprehensive data-sets of adhesin and non-adhesin proteins from publicly available information on protein sequences,
2. calculating computationally the sequence based attributes of the protein sequences in the publicly available protein datasets using specially developed Software for Prediction of Adhesins and Adhesin-like proteins using Neural Networks (SPAAN),
3. training the Artificial Neural Network (ANN) for the selected attributes,
4. assigning probability value suitable for an adhesin, "Pad" to the query protein and identifying adhesin like property in the query proteins with the help of trained Artificial Neural Network implemented in SPAAN,
5. validating computationally the protein sequences as therapeutic potentials by comparing with the known protein sequences that are biochemically characterized in the pathogen genome.

In an embodiment of the invention, the protein sequence data may be taken from an organism, specifically but not limited to organisms such as *Escherichia coli, Haemophilus influenzae, Helicobacter pylori, Mycoplasma pneumoniae, Mycobacterium tuberculosis, Rickettsiae prowazekii, Porphyromonas gingivalis*, *Shigella flexneri, Streptococcus mutans, Streptococcus pneumoniae, Neisseria meningitides, Streptococcus pyogenes*, *Treponema pallidum*, Severe Acute Respiratory Syndrome associated coronavirus.

In another embodiment of the present invention, different sequence-based attributes used for identification of proteins of therapeutic potential, comprise amino acid composition, charge composition, hydrophobicity composition, multiplets frequencies, and dipeptide frequencies.

In an embodiment, the non-homologous adhesin protein sequence may be compared with that of known sequences of therapeutic applications in the selected pathogens.

In the application, the sequences of adhesin or adhesin like proteins comprise sequences of sequences IDs listed in Tables 5 and 6 identified by the method of invention.

Another embodiment of the invention the computer system comprises a central processing unit, executing SPAAN program, giving probabilities based on different attributes using Artificial Neural Network and in built other programs of assessing attributes, all stored in a memory device accessed by CPU, a display on which the central processing unit displays the screens of the above mentioned programs in response to user inputs; and a user interface device.

In the present application, the particulars of the organisms such as their name, strain, accession number in NCBI database and other details are given in Table 2:

The invention is further explained with the help of the following examples, which are given by illustration and should be construed to limit the scope of the present invention in any manner.

**Example 1**

Operating SPAAN:

The purpose of the program is to computationally calculate various sequence-based attributes of the protein sequences.

The program works as follows:

The internet downloaded FASTA format files obtained from http://www.ncbi.nlm.nih.gov were saved by the name <organism_name>.faa are converted in the standard format by C program and passed as input to another set of C programs which computes the 5 different attributes of protein sequences (a total of 105 compositional properties in all 5 modules).

The computed properties were fed as input to the 5 different Neural Networks. Each trained network assigns a probability value of being an adhesin for a query protein. The final probability (Pad) was calculated as weighted average of these five individual probabilities. The weights were determined from a correlation value of correct prediction during test runs of each of the five modules.

**Input/Output format:**

**Downloaded Files and their format:**

<organism_name>.faa: file which stores the annotation and the protein sequence.

Input file Format: FASTA

">gi.vertline."<annotation>

For example,

>gi.vertline.2314605.vertline.gb.vertline.AAD08472.vertline.histidine and glutamine-rich protein

MAHHEQQQQQQANSQHHHHHHHAHHHHYYGGEHHHHNAQQHAEQQAEQQ
AQQQQQQQAHQQQQQKAQQQNQQY

>gi.vertline.3261822.vertline.gnl.vertline.PID.vertline.e328405 PE_PGRS

MIGDGANGGPGQPGGPGGLLYGNGGHGGAGAAGQDRGAGNSAGLIGNGGAG GAGGNGGIGGAGAPGGLGGDGGKGGFADEFTGGFAQGGRGGFGGNGNTGAS GGMGGAGGAGGAGGAGGLLIGDGGAGGAGGIGGAGGVGGGGGAGGTGGGG VASAFGGGNAFGGRGGDGGDGGDGGTGGAGGARGAGGAGGAGGWLSGHSG AHGAMGSGGEGGAGGGGGARGEAGAGGGTSTGTNPGKAGAPGTQGDSGDP GPPG

>gi.vertline....

Table 1: Output file format given by SPAAN

| <organism_name>.out | | | | | | | |
|---|---|---|---|---|---|---|---|
| SN | $P_A$ | $P_C$ | $P_D$ | $P_H$ | $P_M$ | $P_{ad}$-value | Protein Name |
| 1 | 0.05683 | 0.290803 | 0.441338 | 0.50304 | 0.029503 | 0.260485 | >gi.vertline.32454344.vert line.gb.vertline.AAP82966 . 1. vertline.orfla polyprotein [SARS coronavirus Hong Kong ZY-2003] |
| 2 | 0.639235 | 0.166721 | 0.054583 | 0.935385 | 0.453498 | 0.462452 | >gi.vertline.32454345.vert line.gb.vertline.AAP82967 . 1. vertline.orflab polyprotein [SARS coronavirus Hong Kong ZY-2003] |
| 3 | 0.65111 1 | 0.91150 4 | 0.43869 6 | 0.54394 4 | 0.92404 4 | 0.690247 | >gi.vertline.32454346.vert line.gb.vertline.AAP82968 . 1. vertline.spike glycoprotein [SARS coronavirus Hong Kong ZY-2003] |
| 4 | 0.464324 | 0.655003 | 0.179503 | 0.008700 | 0.241573 | 0.300970 | >gi.vertline.32454347.vert line.gb.vertline.AAP82969 . 1. vertline.Orf3a [SARS coronavirus Hong Kong ZY-2003] |

Where $P_A$, $P_C$, $P_D$, $P_H$, $P_M$ are the outputs of the five Neural Networks.

**Example 2** organisms and sequence numbers

**[0095]**

Table 2: Organism Name, Accession number, Number of base pairs, Date of release and Total number of proteins analyzed

| Organism Name | Accession Number | Number of base pairs | Date of release | Total no. of proteins |
|---|---|---|---|---|
| *E. coli* 0157 H7 | NC_0026 95 | 5498450 | 7-Mar-2001 | 5361 |

(continued)

| Organism Name | Accession Number | Number of base pairs | Date of release | Total no. of proteins |
|---|---|---|---|---|
| *H. influenzae* Rd | NC_0009 07 | 1830138 | 30-Sep-1996 | 1709 |
| *H. pylori* J99 | NC_0009 21 | 1643831 | 10-Sep-2001 | 1491 |
| *M. pneumoniae* | NC_0009 12 | 816394 | 2-Apr-2001 | 689 |
| *M. tuberculosis* H37Rv | NC_0009 62 | 4411529 | 7-Sep-2001 | 3927 |
| *R. prowazekii* strain Madrid E | NC_0009 63 | 1111523 | 10-Sep-2001 | 835 |
| *P. gingivalis* W83 | NC_0029 50 | 2343476 | 9-Sep-2003 | 1909 |
| *S. flexneri* 2a str. 2457T | NC_0047 41 | 4599354 | 23- Apr-2003 | 4072 |
| *S. mutans* UA159 | NC_0043 50 | 2030921 | 25-Oct-2002 | 1960 |
| *S. pneumoniae* R6 | NC_0030 98 | 2038615 | 6-Sep-2001 | 2043 |
| *N. meningitidis* serogroup A strain Z2491 | NC_0031 16 | 2184406 | 27-Sep-2001 | 2065 |
| *S. pyogenes MGAS8232* | NC_0034 85 | 1895017 | Jan 31, 2002 | 1845 |
| *T. pallidum* subsp. pallidum str. Nichols | NC_0009 19 | 1138011 | 7-Sep-2001 | 1036 |
| *Severe Acute Respiratory Syndrome (SARS) associated coronavirus* Frankfurt 1 | AY29131 5 | 29727 | 11-JUN-2003 | 14 |
| *SARS coronavirus* HSR 1 | AY32397 7 | 29751 | 15-OCT-2003 | 14 |
| *SARS coronavirus* ZJ01 | AY29702 8 | 29715 | 19-MAY-2003 | 3 |
| *SARS coronavirus* TW1 | AY29145 1 | 29729 | 14-MAY-2003 | 11 |
| *SARS coronavirus* CUHK-Su10 | AY28275 2 | 29736 | 07-MAY-2003 | 4 |
| *SARS coronavirus* Urbani | AY27874 1 | 29727 | 12-AUG-2003 | 12 |
| *SARS coronavirus* | NC_0047 18 | 29751 | 9-Sep-2003 | 29 |
| *SARS coronavirus* Tor2 | AY27411 9 | 29751 | 16-MAY-2003 | 15 |
| *SARS coronavirus* GD01 | AY27848 9 | 29757 | 18-AUG-2003 | 12 |
| *SARS coronavirus* CUHK-W1 | AY27855 4 | 29736 | 31-JUL-2003 | 11 |
| *SARS coronavirus* BJ01 | AY27848 8 | 29725 | 01-MAY-2003 | 11 |

**Example 3**

The multi-layered feed forward Neural Network architecture implemented in SPAAN (figure 1). A given protein sequence in FASTA format is first processed through the 5 modules A, C, D, H, and M to quantify the five types of compositional attributes. A: Amino acid composition, C: Charge composition, D: dipeptide composition of the 20 dipeptides (NG, RE, TN, NT, GT, TT, DE, ER, RR, RK, RI, AT, TS, IV, SG, GS, TG, GN, VI, HR), H: Hydrophobic composition, M: Amino acid frequencies as Multiplets. The sequence shown is part of the FimH precursor (gi 5524634) of *E. coli.* Subsequently, these numerical data are input to the input neuron layer. The directions of arrows show data flow. The number of neurons chosen in the input layer was equal to the number of the numerical input vectors of each module. The network was optimally trained through minimization of error of detection based on validate set through back propagation. The details are described in the methods. Each network module assigns a probability value of the protein being an adhesin based on the corresponding attribute. The final probability of a protein sequence being an adhesin is the $P_{ad}$ value a weighted average of the individual probabilities and the associated fraction of correlation which is a measure of the strength of the prediction.

**Example 4**

Performance of SPAAN assessed using a test set of 37 adhesins and 37 non-adhesins that were not part of the training set. Matthew's correlation coefficient (Mcc, plotted on Y-axis) for all the proteins with $P_{ad}$ values above a given threshold (plotted on X-axis) (figure 2). The Matthew's correlation is defined as:

$$Mcc = \frac{(TP*TN)-(FP*FN)}{\sqrt{(TN+FN)(TN+FP)(TP+FN)(TP+FP)}}$$

Where TP = True Positives, TN = True Negatives, FP = False Positives, FN = False Negatives.

Here TPs are adhesins, TNs are non-adhesins. In general, adhesins have high $P_{ad}$ value, whereas non-adhesins have low $P_{ad}$ value. Thus known adhesins with $P_{ad}$ value above a given threshold are true positives whereas known non-adhesins with $P_{ad}$ value below the given threshold are true negatives. The sensitivity, Sn is given by $\left(\frac{TP}{TP+FN}\right)$ and specificity, Sp is given by $\left(\frac{TP}{TP+FP}\right)$. False negatives are those cases, wherein a known adhesin had $P_{ad}$ value lower than the chosen threshold. Similarly, a known non-adhesin with a $P_{ad}$ value higher than the chosen threshold was taken as false positive. A theoretical polynomial curve of second order (dashed line) was fitted to the observed curve (smooth line) with a Karl-Pearson correlation coefficient $R^2$ = 0.9799. The maximum point of the theoretical curve (where first derivative vanishes and second derivative is negative) was chosen as reference (vertical dotted line) to identify the maximum Mcc = 0.94 on the observed curve (shown by arrow). The corresponding $P_{ad}$ value threshold was 0.51. At this $P_{ad}$ value threshold, Sn and Sp were 0.89 and 1.0 respectively. Note that the Mcc does not drop down to the x-axis because the highest $P_{ad}$ value attained by adhesins was 0.939 in comparison to the theoretical attainable limit of 1.O.

**Example 5**

Assessment of SPAAN on well known adhesins from various bacterial pathogens.

Table 3. Prediction of well characterized adhesins from various bacterial pathogens using SPAAN.

| Species | Disease caused | Adhesin[a] | Host ligand | $P_{ad}$ value[b] (Range) |
|---|---|---|---|---|
| *E. coli* | Diarrhoea | PapG (27)<br>SfaS (5) | $\alpha$-D-gal(1-4) $\beta$-D-Gal- containing receptors<br>alpha-sialyl-beta-2,3-b-galactose | 0.84-0.76<br>0.94-0.94 |
| | | FimH (63) | D-mannosides | 0.96-0.23[c] |
| | | Intimin (12) | tyrosine-phosphorylated form of host cell receptor Hp90 | 0.95-0.78 |
| | | PrsG (5) | Gal(alpha1-4)Gal | 0.86-0.85 |
| Nontypeable *H. influenzae* | Influenza | HMW1, HMW2 | Human epithelial cells | 0.97 |
| | | Hia (8) | human conjuctival cells | 0.93-0.90 |
| *H. influenzae* | bacterial meningitis[d] | HifE (18) | Sialylganglioside-GM1 | 0.85-0.73 |
| *K. pneumoniae* | Pneumonia | MrkD | type V collagen | 0.82 |

(continued)

| Species | Disease caused | Adhesin[a] | Host ligand | $P_{ad}$ value[b] (Range) |
|---|---|---|---|---|
| *B. pertussis* | Whooping cough | FHA | Sulphated sugars on cell-surface glycoconjugates | 0.85 |
| | | Pertactin | Integrins | 0.43 |
| *Y. enterocolitica* | Enterocolitis | YadA (5) | $\beta_1$ integrins | 0.88-0.79 |
| *S. mutans* | Dental Caries | SpaP (2)<br>PAc | Salivary glycoprotein Salivary glycoprotein | 0.88, 0.87<br>0.88 |
| *Streptococcus gordonii* | Oral cavity | SspA (2) | Salivary glycoprotein | 0.85, 0.84 |
| | | CshA | Fibronectin | 0.78 |
| | | CshB | Fibronectin | 0.63 |
| | | ScaA | Co-aggregation | 0.71 |
| | | SspB (2) | Salivary glycoprotein | 0.85,0.84 |
| *Streptococcus sobrinus* | Tooth decay | SpaA<br>PAg (2) | Salivary glycoprotein<br>Salivary glycoprotein | 0.89<br>0.89, 0.73 |
| *Streptococcus pyogenes* | Scarlet Fever | Protein F | Fibronectin | 0.49 |
| *Streptococcus pneumoniae* | Bacterial Pneumonia | PsaA (5) | Human nasopharyngeal cells | 0.82-0.78 |
| | | CbpA[e] / SpsA / PbcA/ PspC | phosphorylcholine of the teichoic acid. | 0.81-0.49 |
| *Streptococcus parasanguis* | Valve endocarditis | FimA | Salivary glycoprotein fibrin | 0.76 |
| *Streptococcus sanguis* | Tooth Decay | SsaB | Salivary glycoprotein | 0.71 |
| *Enterococcus faecalis* | Empyma in patients with liver disease | EfaA | Unknown | 0.83 |
| *Staphylococcus aureus* | Food Poisoning | FnbA<br>FnbB (3) | Fibronectin<br>Fibronectin | 0.8<br>0.78, 0.77, 0.69 |
| *Helicobacter pylori* | Peptic Ulcers | BabA (17) | difucosylated Lewis[b] blood group antigen | 0.87-0.68 |

[a]: The number of sequences from different strains and homologs from related species analyzed are shown in parantheses.
[b]: Rounded off to the second decimal.
[c]: Out of 63 FimH proteins, 54 were from *E. coli,* 6 from *Shigella flexineri*, 2 from *Salmonella enterica* and 1 was from *Salmonella typhimurium.* Except 2 FimH proteins, the rest had $P_{ad}$ 0.51. The 2 exceptions (gi numbers: 5524636, 1778448) were from E. *coli.* The gi:5524636 protein is annotated as a FimH precursor but is much shorter (129 amino acids) than other members of the family. The gi:1778448 protein is a *S. typhimurium* homolog in *E. coli.*
[d]: Other ailments include pneumonia, epiglottitis, osteomyelitis, septic arthritis and sepsis in infants and older children.
[e]: The adhesin CbpA is also known by alternative names SpsA, PbcA and PspC. A total of seven sequences were analyzed. Except 1 PspC sequence, the rest all had $P_{ad}$ 0.51.

**Example 6**

Ability of SPAAN to discriminate adhesins from non-adhesins at Pad 0.51 (figure 3-a).

**Example 7**

The non-homology character of SPAAN assesses in both adhesins and non-adhesins (figure 3b and 3c).

Figure 3 (a - c). SPAAN is non-homology based software. A total of 130 adhesins and 130 non-adhesins were analyzed to assess whether the predictive power of SPAAN could be influenced by the sequence relationships, (a) Histogram plots of the number of proteins in the various $P_{ad}$ value ranges are shown. Shaded bars represent adhesins whereas open bars represent non-adhesins. Note the SPAAN's ability to segregate adhesins and non-adhesins into two distinct cohesive groups. (b) Pairwise sequence relationships among the adhesins were determined using CLUSTAL W and plotted on X-axis. Higher scores indicate similar pairs. The corresponding differences in $P_{ad}$ values in the same protein pair was plotted on the Y-axis. Each point in the diagram represents a pair. Arrow points to protein pairs of the FimH family with high $\Delta P_{ad}$ values in spite of high similarity: Since one of the FimH proteins (gi: 5524636) had very low $P_{ad}$ value all pairs with this false negative protein show high $\Delta P_{ad}$ values. The protein (gi: 5524636) is of much shorter length compared with other members of the same family. (c) plot for non-adhesins. Data are plotted in the 4 quadrant format for clear inspection. Note that among protein pairs with CLUSTAL W score < 20 the majority (82% in adhesins and 86% in non-adhesins) have $\Delta P_{ad}$ < 0.2. These data support the non-homology character of SPAAN.

**Example 8**

Genomescan of pathogens by SPAAN identifies well known adhesins and new adhesins and adhesin-like proteins

Table 4. Analysis of predictions made by SPAAN on genome scans of a few selected pathogenic organisms[a]

| Species / Protein Class | *Escherichia coli* O157:H7 | *Mycobacterium tuberculosis* H37Rv | SARS associated corona virus (11 strains) |
|---|---|---|---|
| Total number of proteins with $P_{ad}$ 0.51 | 575 | 435 | 5 |
| Known adhesins | 17[b] | - | - |
| Putative proteins with adhesin like characteristics | 92[c] | 105[j] | - |
| Hypothetical proteins with adhesin-like characteristics | 22[d] | - | - |
| Proteins likely to be extracytoplasmic or located at surface | 190[e] | 191[k] | 5[m] |
| Phage proteins | 30[f] | - | - |
| Others | 13[g] | 6[l] | - |
| Hypothetical proteins | 157[h] | 86[h] | - |
| Wrong predictions | 54[i] | 47[i] | - |

[a]: SPAAN has general applicability. The three pathogens chosen here are those in which intense investigations are being conducted presently. *M. tuberculosis* is of special importance to developing countries.

[b]: Fimbrial adhesins, AidA-I, gamma intimin, curlin, translocated intimin receptor, putative adhesin and transport, Iha, prepilin peptidase dependent protein C.

[c]: These proteins have been annotated as proteins with a putative function. These sequences were analyzed using CDD (Conserved domain database, NCBI) and BLAST searches. Adhesin like domains were found in these proteins.

[d]: These proteins have been annotated as 'hypothetical'. These sequences were analyzed using CDD and BLAST searches. Adhesin like domains were found in these proteins.

[e]: These proteins are outer membrane, extracellular, transport, surface, exported, flagellar, periplasmic lipoprotein, and proteins annotated as 'hypothetical' but found to have similar functions listed here using BLAST and CDD searches.

[f]: The phage proteins were of the following functional roles – tail fiber, head decoration, DNA injection, tail, major capsid, host specificity, endolysin.

[g]: Proteins predicted by SPAAN but not readily classifiable into the classes listed here have been collectively grouped as 'Others'. However, some of these proteins are known to participate in host-pathogen interactions. The annotated functional roles are typeIII secretion, antibiotic resistance, heat shock, acid shock, structural, tellurium resistance, terminase, Hcp-like, Sec-independent translocase, uncharacterized nucleoprotein, HicB-like.

[h]: These proteins have been annotated as hypothetical. Re-analyses of these proteins using BLAST and CDD failed identify any function for these proteins.

[i]: These proteins have been annotated with functional roles that are very likely to occur within the cell. Hence these proteins may have remote possibility of functioning as adhesins or adhesin-like proteins. Therefore this set of proteins have been incorrectly predicted as adhesins or adhesin-like by SPAAN.

[j]: These proteins are PE_PGRS, PE proteins. Several reports (for example Brennan *et al.*) indicate that PE_PGRS proteins may be localized to cell surface and aid in host-pathogen interaction.

[k]: Lipoproteins (lpp, lpq, lpr), PPE, outer membrane, surface, transport, secreted, periplasmic, extracellular, ESAT-6, peptidoglycan binding, exported, mpt (with extracellular domains), and proteins annotated as 'hypothetical' but found to have similar functions listed here using BLAST and CDD searches.

[l]: These proteins were of the following functions - glutaredoxin-like thioltransferase, putative involvement in molybdate uptake, ATP synthase chain, sulphotransferases, S.erythraea rhodanese-like protein M29612|SERCYSA_5, unknown function.

[m]: These proteins were the spike glycoprotein with antigenic properties, and nsp2, nsp5, nsp6 and nsp7.

**Table 5: New putative adhesins predicted by SPAAN in the genomes listed in table 2- (Total number = 279)**

| Protein GI Number | Gene ID | Protein name |
|---|---|---|
| *Escherichia coli* O157:H7 | | |
| 13360742 | 912619 | hemagglutinin/hemolysin-related protein |
| 13362986 | 914770 | putative ATP-binding component of a transport system |
| 13361114 | 913228 | putative tail fiber protein |

(continued)

| (**Total number = 279**) | | |
|---|---|---|
| Protein GI Number | Gene ID | Protein name |
| *Escherichia coli* O157:H7 | | |
| 13364757 | 913676 | minor fimbrial subunit/D-mannose specific adhesin |
| 13362687 | 915687 | putative fimbrial-like protein |
| 13360856 | 912599 | AidA-I adhesin-like protein |
| 13364140 | 915374 | putative fimbrial protein |
| 13359793 | 914435 | putative invasin |
| 13364768 | 913650 | putative invasin |
| 13364034 | 915471 | Gamma intimin |
| 13362703 | 915668 | putative DNA transfer protein precursor |
| 13364141 | 915376 | putative fimbrial protein |
| 13359819 | 914463 | AidA-I adhesin-like protein |
| 13360480 | 917768 | putative fimbrial-like protein |
| 13362692 | 915681 | putative fimbrial-like protein |
| 13362585 | 916824 | putative ATP-binding component of a transport system |
| 13359881 | 914526 | putative flagellin structural protein |
| 13361579 | 917311 | putative type 1 fimbrial protein precursor |
| 13360880 | 913991 | curlin major subunit CsgA |
| 13364036 | 915465 | translocated intimin receptor Tir |
| 13360740 | 912615 | putative major pilin protein |
| 13361582 | 917317 | putative ATP-binding component of a transport system and adhesin protein |
| 13364754 | 913683 | export and assembly outer membrane protein of type 1 fimbriae |
| 13360484 | 917767 | homolog of Salmonella FimH protein |
| 13364751 | 913688 | major type 1 subunit fimbrin |
| 13359597 | 913742 | putative fimbrial protein |
| 13362550 | 916787 | putative ATP-binding component of a transport system |
| 13359595 | 913739 | putative fimbrial protein |
| 13359599 | 913748 | probable outer membrane porin protein involved in fimbrial assembly |
| 13363900 | 915704 | putative fimbrial protein precursor |
| 13361575 | 917307 | putative fimbrial-like protein |
| 13364756 | 913678 | fimbrial morphology |
| 13363496 | 916142 | truncated putative fimbrial protein |
| 13359601 | 913761 | putative fimbrial-like protein |
| 13364145 | 915368 | putative type 1 fimbrial protein |
| 13363902 | 915708 | putative outer membrane usher protein precursor |
| 13361576 | 917309 | putative outer membrane protein |
| 13361013 | 913353 | putative major tail subunit |
| 13364755 | 913682 | fimbrial morphology |
| 13360738 | 912793 | putative outer membrane usher protein |
| 13363928 | 915608 | alpha-amylase |
| 13363495 | 916144 | putative outer membrane protein |
| 13362383 | 916617 | putative type-1 fimbrial protein |
| 13364373 | 914972 | outer membrane vitamin B12 receptor protein BtuB |
| 13360879 | 912479 | minor curlin subunit precursor CsgB |
| 13360739 | 912756 | putative chaperone protein |
| 13361574 | 917314 | putative fimbrial-like protein |
| 13361127 | 913212 | outer membrane protease precursor |
| 13363210 | 916442 | putative lipoprotein |
| 13361104 | 913238 | major tail protein |
| 13361709 | 917446 | putative major tail subunit |

(continued)

| (**Total number = 279**) | | |
|---|---|---|
| Protein GI Number | Gene ID | Protein name |
| *Escherichia coli* O157:H7 | | |
| 13359725 | 914366 | outer membrane pore protein PhoE |
| 13360875 | 913765 | curli production assembly/transport component CsgF |
| 13362170 | 913927 | putative outer membrane protein |
| 13361473 | 917203 | putative BigB-like protein |
| 13364025 | 915286 | EspF protein |
| 13360081 | 916982 | outer membrane receptor for ferric enterobactin (enterochelin) and colicins B and D |
| 13362977 | 914779 | hypothetical lipoprotein |
| 13360351 | 917632 | outer membrane protein X |
| 13360696 | 914208 | putative outer membrane precursor |
| 13361456 | 917106 | putative outer membrane protein |
| 13361626 | 917374 | putative outer host membrane protein precursor |
| 13361698 | 917449 | putative outer membrane protein |
| 13362186 | 913421 | putative outer membrane protein precursor |
| 13362697 | 915676 | long-chain fatty acid transport protein FadL |
| 13360918 | 914188 | flagellar hook protein FlgE |
| 13360737 | 912506 | putative outer membrane protein |
| 13360342 | 917629 | putative outer membrane receptor for iron transport |
| 13363396 | 916248 | outer membrane channel TolC |
| 13361958 | 912705 | putative scaffolding protein in the formation of a murein-synthesizing holoenzyme |
| 13359921 | 914566 | nucleoside-specific channel-forming protein TSX |
| 13360944 | 913890 | outer membrane receptor for ferric iron uptake |
| 13359998 | 914644 | putative outer membrane transport protein |
| 13363390 | 916251 | putative ferrichrome iron receptor precursor |
| 13364227 | 915153 | outer membrane phospholipase A |
| 13361982 | 912846 | putative outer membrane protein |
| 13360129 | 917032 | a minor lipoprotein |
| 13361817 | 912692 | putative outer membrane protein |
| 13360233 | 917507 | membrane spanning protein TolA |
| 13362837 | 915218 | putative outer membrane lipoprotein |
| 13362328 | 912985 | putative colanic acid biosynthesis glycosyl transferase |
| *Haemophilus influenzae* Rd | | |
| 16272254 | 949521 | prepilin peptidase-dependent protein D |
| 16272928 | 950762 | immunoglobin A1 protease |
| 16272129 | 951072 | lipoprotein |
| 16273251 | 950616 | hemoglobin-binding protein |
| 30995429 | 950130 | opacity protein |
| 16272854 | 949634 | protective surface antigen D15 |
| 16272283 | 950648 | opacity associated protein |
| 16272604 | 949701 | hemoglobin-binding protein |
| *Helicobacter pylori* J99 | | |
| 4155101 | 889167 | putative vacuolating cytotoxin (VacA) paralog |
| 4154798 | 890022 | putative vacuolating cytotoxin (VacA) paralog |
| 4155426 | 890036 | putative vacuolating cytotoxin (VacA) paralog |
| 4155390 | 890075 | vacuolating cytotoxin |
| 4155400 | 890058 | outer membrane protein - adhesin |
| 4155681 | 889718 | putative Outer membrane protein |

(continued)

| | | |
|---|---|---|
| *Helicobacter pylori* J99 | | |
| 4155420 | 890042 | Outer membrane protein/porin |
| 4155775 | 889799 | outer membrane protein - adhesin |
| 4155419 | 890044 | Outer membrane protein/porin |
| 4154526 | 889066 | putative Outer membrane protein |
| 4154724 | 889419 | putative Outer membrane protein |
| 4155862 | 890404 | putative Outer membrane protein |
| 4156048 | 889958 | putative IRON(III) DICITRATE TRANSPORT PROTEIN |
| 4154510 | 889297 | putative Outer membrane protein |
| 4155432 | 889515 | putative outer membrane protein |
| 4155623 | 889671 | putative Outer membrane protein |
| 4155700 | 889739 | putative Outer membrane function |
| 4154740 | 889426 | Outer membrane protein/porin |
| 4155692 | 889743 | putative Outer membrane protein |
| 4155594 | 889648 | putative outer membrane protein |
| 4155680 | 889719 | putative Outer membrane protein |
| 4155217 | 890243 | putative Outer membrane protein |
| 4155958 | 889905 | putative Outer membrane protein |
| 4155201 | 890259 | putative Outer membrane protein |
| 4155013 | 889232 | cag island protein |
| 4154974 | 889032 | putative Outer membrane protein |
| 4155214 | 890244 | putative Outer membrane protein |
| 4154973 | 889042 | Outer membrane protein |
| 4155344 | 890115 | putative Outer membrane protein |
| 4155099 | 889160 | FLAGELLIN A |
| 4155023 | 888978 | cag island protein |
| 4155035 | 889201 | cag island protein, CYTOTOXICITY ASSOCIATED IMMUNODOMINANT ANTIGEN |
| 4155289 | 890164 | NEURAMINYLLACTOSE-BINDING HEMAGGLUTININ PRECURSOR |
| *Mycoplasma pneumoniae* | | |
| 13507881 | 877207 | involved in cytadherence |
| 13507880 | 877268 | ADP1_MYCPN adhesin P1 |
| 13508228 | 877211 | species specific lipoprotein |
| 13508181 | 877124 | species specific lipoprotein |
| 13508179 | 877071 | Mollicute specific lipoprotein, MG307 homolog, from M. genitalium |
| 13508178 | 877118 | Mollicute specific lipoprotein, MG307 homolog, from M. genitalium, |
| 13508176 | 876797 | Mollicute specific lipoprotein, MG307 homolog, from M. genitalium |
| 13508175 | 876848 | Mollicute specific lipoprotein, MG307 homolog, from M. genitalium |
| 13508106 | 876953 | involved in cytadherence |
| 13508350 | 877112 | similar to phosphate binding protein Psts |
| *Mycobacterium tuberculosis* H37 Rv | | |
| 15607496 | 886491 | PPE |
| 15607445 | 886592 | PPE |
| 15610644 | 888270 | PE_PGRS |
| 15608588 | 886605 | PE_PGRS |
| 15609627 | 887941 | PE_PGRS |
| 15610643 | 888256 | PE_PGRS |
| 15607718 | 887725 | PE_PGRS |
| 15609054 | 885362 | PPE |
| 15610486 | 888113 | PPE |
| 15610483 | 888120 | PPE |

(continued)

| | | |
|---|---|---|
| *Mycobacterium tuberculosis* H37 Rv | | |
| 15610479 | 888033 | PPE |
| 15609771 | 888573 | PE_PGRS |
| 15610648 | 888306 | PE_PGRS |
| 15610481 | 888114 | PE_PGRS |
| 15608117 | 885264 | PE_PGRS |
| 15607973 | 885391 | PE_PGRS |
| 15608231 | 885258 | PE_PGRS |
| 15608906 | 885429 | PE_PGRS |
| 15608891 | 885544 | PPE |
| 15609990 | 888171 | PE_PGRS |
| 15609055 | 885506 | PPE |
| 15608227 | 887094 | PE_PGRS |
| 15610524 | 888151 | PE_PGRS |
| 15609490 | 886003 | PPE |
| 15607886 | 888664 | PE_PGRS |
| 15609624 | 887909 | PE_PGRS |
| 15607420 | 886621 | PE_PGRS |
| 15608897 | 885325 | PE_PGRS(wag22) |
| 15608590 | 886595 | PE_PGRS |
| 15609728 | 887992 | PE_PGRS |
| 15608012 | 885742 | PE_PGRS |
| 15608534 | 886745 | PE_PGRS |
| 15608940 | 885730 | PE_PGRS |
| 15607887 | 888662 | PE_PGRS |
| 15609235 | 888312 | PE_PGRS |
| 15610694 | 887822 | PPE |
| 15609533 | 885517 | PE_PGRS |
| 15610480 | | PE_PGRS |
| *Rickettsia prowazekii* strain Madrid E | | |
| 15604316 | 883411 | CELL SURFACE ANTIGEN (sca3) |
| 15604546 | 883694 | CELL SURFACE ANTIGEN (sca5) |
| *Porphyromonas gingivalis* W83 | | |
| 34541453 | 2551934 | hemagglutinin protein HagA |
| 34540040 | 2551409 | lipoprotein, putative |
| 34540364 | 2552375 | extracellular protease, putative |
| 34541613 | 2552074 | hemagglutinin protein HagE |
| 34540183 | 2551891 | internalin-related protein |
| *Shigella flexneri* 2a str. 2457T | | |
| 30065424 | 1080663 | minor fimbrial subunit, D-mannose specific adhesin |
| 30062726 | 1077662 | putative adhesion and penetration protein |
| 30063758 | 1078834 | putative fimbrial-like protein |
| 30065431 | 1080671 | major type 1 subunit fimbrin (pilin) |
| 30063366 | 1078379 | flagellar protein FliD |
| 30064308 | 1079668 | outer membrane fluffing protein |
| 30062613 | 1077555 | flagellar hook protein FlgE |
| 30061954 | 1076843 | conserved hypothetical lipoprotein |
| 30065173 | 1080393 | putative lipase |
| 30065425 | 1080664 | minor fimbrial subunit, precursor polypeptide |
| 30064485 | 1079637 | putative fimbrial protein |
| 30062615 | 1077558 | flagellar basal body L-ring protein FlgH |

(continued)

| | | |
|---|---|---|
| *Shigella flexneri* 2a str. 2457T | | |
| 30064307 | 1079452 | outer membrane fluffing protein |
| 30065601 | 1080859 | putative glycoprotein/receptor |
| 30062118 | 1077025 | putative fimbrial-like protein |
| 30064099 | 1079223 | lipoprotein |
| 30062616 | 1077559 | flagellar basal body P-ring protein FlgI |
| 30063546 | 1078596 | putative fimbrial-like protein |
| 30062940 | 1077910 | putative outer membrane protein |
| 30065426 | 1080665 | minor fimbrial subunit, precursor polypeptide |
| 30062779 | 1077721 | putative outer membrane protein |
| 30064194 | 1079329 | putative lipoprotein |
| 30063365 | 1078378 | flagellin |
| 30062298 | 1077222 | outer membrane protein X |
| 30064968 | 1080175 | putative major fimbrial subunit |
| 30061858 | 1076740 | outer membrane pore protein E (E,Ic,NmpAB) |
| 30062178 | 1080410 | minor lipoprotein |
| 30062479 | 1077412 | putative fimbrial-like protein |
| 30062565 | 1077506 | minor curlin subunit precursor |
| 30063880 | 1078972 | putative outer membrane lipoprotein |
| 30064531 | 1079686 | cytoplasmic membrane protein |
| 30065033 | 1080243 | putative receptor protein |
| *Streptococcus mutans* UA159 | | |
| 24378550 | 1029610 | putative secreted antigen GbpB/SagA; putative peptidoglycan hydrolase |
| 24379087 | 1028055 | cell surface antigen SpaP |
| 24380463 | 1029310 | putative membrane protein |
| 24379075 | 1028046 | penicillin-binding protein 2b |
| 24378955 | 1027967 | penicillin-binding protein 1a; membrane carboxypeptidase |
| 24379801 | 1028662 | glucan-binding protein C, GbpC |
| 24379528 | 1029536 | hypothetical protein; possible cell wall protein, WapE |
| 24379231 | 1028158 | putative glucan-binding protein D; BglB-like protein |
| 24380488 | 1029325 | conserved hypothetical protein; possible transmembrane protein |
| 24380291 | 1029139 | putative amino acid binding protein |
| 24379342 | 1028247 | putative penicillin-binding protein, class C; fmt-like protein |
| 24380047 | 1028904 | putative ABC transporter, branched chain amino acid-binding protein |
| 24378698 | 1029755 | putative ABC transporter, metal binding lipoprotein; surface adhesin precursor; saliva-binding protein; lipoprotein receptor LraI (LraI family) |
| 24378708 | 1029768 | putative transfer protein |
| 24379427 | 1028331 | cell wall-associated protein precursor WapA |
| 24379272 | 1028196 | putative amino acid transporter, amino acid-binding protein |
| 24379641 | 1028511 | putative ABC transporter, amino acid binding protein |
| *Streptococcus pneumoniae* R6 | | |
| 15902395 | 934801 | Choline-binding protein |
| 15902381 | 934810 | Choline-binding protein F |
| 15902165 | 932894 | Surface protein pspA precursor |
| 15904047 | 934859 | Choline binding protein D |
| 15904036 | 933487 | Choline binding protein A |
| 15903986 | 933069 | Choline-binding protein |
| 15903796 | 933669 | Autolysin (N-acetylmuramoyl-L-alanine amidase) |
| *Neisseria meningitidis* Z2491 | | |
| 15794121 | 907145 | putative membrane protein |
| 15794144 | 907168 | putative surface fibril protein |

(continued)

| | | |
|---|---|---|
| *Neisseria meningitidis* Z2491 | | |
| 15793284 | 906275 | truncated pilin |
| 15793460 | 906456 | IgA-specific serine endopeptidase |
| 15793282 | 906273 | fimbrial protein precursor (pilin) |
| 15793337 | 906332 | adhesin |
| 15793253 | 906243 | putative lipoprotein |
| 15794356 | 907848 | putative lipoprotein |
| 15793684 | 906699 | putative membrane protein |
| 15793290 | 906281 | truncated pilin |
| 15793283 | 906274 | truncated pilin |
| 15793475 | 906471 | haemoglobin-haptoglobin-utilization protein |
| 15793406 | 906401 | porin, major outer membrane protein P.I |
| 15794985 | 907333 | adhesin MafA2 |
| 15794344 | 907836 | putative lipoprotein |
| 15794622 | 908118 | hypothetical outer membrane protein |
| 15793599 | 906604 | pilus-associated protein |
| 15793763 | 906779 | putative periplasmic binding protein |
| *Streptococcus pyogenes* MGAS8232 | | |
| 19745214 | 995235 | putative secreted protein |
| 19746570 | 994224 | putative penicillin-binding protein 1a |
| 19745593 | 994771 | putative 42 kDa protein |
| 19745813 | 993958 | putative adhesion protein |
| 19745225 | 994839 | putative choline binding protein |
| 19745828 | 995250 | streptolysin S associated protein |
| 19746229 | 995021 | putative minor tail protein |
| 19746909 | 994105 | putative laminin adhesion |
| 19745560 | 995061 | putative cell envelope proteinase |
| *Treponema pallidum* subsp. pallidum str. Nichols | | |
| 15639714 | 2611034 | flagellar hook protein (flgE) |
| 15639609 | 2611657 | tpr protein J (tprJ) |
| 15639111 | 2610909 | tpr protein C (tprC) |
| 15639125 | 2610968 | tpr protein D (tprD) |
| SARS coronavirus | | |
| 31581505 | | spike protein S [SARS coronavirus Frankfurt 1] |
| 32187357 | | spike protein S [SARS coronavirus HSR 1] |
| 32187342 | | spike glycoprotein [SARS coronavirus ZJ01] |
| 30698329 | | putative spike glycoprotein S [SARS coronavirus TW1] |
| 30421454 | | putative spike glycoprotein [SARS coronavirus CUHK-Su10] |
| 30027620 | | S protein [SARS coronavirus Urbani] |
| 29836496 | 1489668 | E2 glycoprotein precursor; putative spike glycoprotein [SARS coronavirus] |
| 30795145 | | spike glycoprotein [SARS coronavirus Tor2] |
| 31416295 | | spike glycoprotein S [SARS coronavirus GD01] |
| 30023954 | | putative E2 glycoprotein precursor [SARS coronavirus CUHK-W1] |
| 30275669 | | spike glycoprotein S [SARS coronavirus BJ01] |
| 29837498 | | 3C-like proteinase nsp5-ppla/pplab (3CL-PRO) [SARS coronavirus] |
| 29837501 | | putative nsp8-ppla/pplab [SARS coronavirus] |
| 29837503 | | putative nsp10-pp1a/pp1ab; formerly known as growth-factor-like protein [SARS coronavirus] |
| 29837502 | | putative nsp9-pp1a/pp1ab [SARS coronavirus] |

Table 6: Hypothetical proteins predicted as putative adhesins by SPAAN in the genomes listed in table 2 - (Total number of proteins = 105)

| Protein GI number | Gene ID |
|---|---|
| *Escherichia coli* O157:H7 | |
| 13363955 | 915578 |
| 13360000 | 914929 |
| 13362244 | 912369 |
| 13359999 | 914888 |
| 13361583 | 917316 |
| 13361172 | 913156 |
| 13361131 | 913207 |
| 13359780 | 914422 |
| 13360571 | 912499 |
| 13362197 | 912893 |
| 13362260 | 912399 |
| 13360947 | 913505 |
| 13361464 | 917196 |
| 13361635 | 917367 |
| 13362421 | 916655 |
| 13361463 | 917195 |
| *Haemophilus influenzae* Rd | |
| 16272115 | 951058 |
| 30995442 | 950581 |
| *Helicobacter pylori* J99 | |
| 4155526 | 889586 |
| 4155712 | 889748 |
| 4155632 | 889684 |
| 4156035 | 889468 |
| 4155499 | |
| *Mycoplasma pneumoniae* | |
| 13507870 | 877230 |
| 13508239 | 877245 |
| 13508109 | 876868 |
| 13508025 | 877084 |
| 13507838 | 876784 |
| 13507883 | 877183 |
| 13507871 | 877239 |
| 13507944 | 877056 |
| 13508241 | 876750 |
| 13507942 | 877055 |
| 13507840 | 877387 |
| 13507867 | 877242 |
| 13508201 | 877044 |
| 13507941 | 876985 |
| 13508114 | 877397 |
| *Mycobacterium tuberculosis* H37Rv | |
| 15611014 | 886198 |
| 15610173 | 887320 |
| 15609513 | 885515 |
| 15608094 | 885411 |
| 15610958 | 886155 |

(continued)

| | |
|---|---|
| *Mycobacterium tuberculosis* H37Rv | |
| 15607528 | 886436 |
| 15607678 | 887473 |
| 15609587 | 885760 |
| 15610708 | 887227 |
| 15609526 | 885246 |
| 15611033 | 886225 |
| 15609028 | 885094 |
| 15607730 | 887771 |
| 15609121 | 885813 |
| 15608255 | 885951 |
| 15608409 | 887039 |
| 15609124 | 885815 |
| 15607734 | 887797 |
| *Rickettsia prowazekii* strain Madrid E | |
| 15604649 | 883964 |
| 15604322 | 883472 |
| 15604659 | 883996 |
| 15604417 | 883217 |
| *Porphyromonas* | *gingivalis* W83 |
| 34540233 | 2551594 |
| *Shigella flexneri* 2a str. 2457T | |
| 30062687 | 1077638 |
| 30062956 | 1080449 |
| 30063681 | 1078754 |
| 30065435 | 1080675 |
| 30063891 | 1078983 |
| 30063211 | 1078195 |
| 30065233 | 1080463 |
| 30064387 | 1079531 |
| 30062638 | 1077590 |
| 30065236 | 1080466 |
| 30061839 | 1076721 |
| *Streptococcus mutans* UA159 | |
| 24378864 | 1029452 |
| 24380475 | 1029319 |
| 24380237 | 1029088 |
| 24379203 | 1028139 |
| 24380480 | 1029320 |
| 24379275 | 1029489 |
| 24379291 | 1028216 |
| 24379295 | 1028215 |
| 24379804 | 1028663 |
| 24379162 | 1029417 |
| 24378987 | 1029363 |
| 24379179 | 1028118 |
| 24379166 | 1028107 |
| 24378827 | 1029444 |
| 24380216 | 1029067 |
| *Streptococcus pneumoniae* R6 | |
| 15902140 | 932867 |

(continued)

| | |
|---|---|
| *Streptococcus pneumoniae* R6 | |
| 15903446 | 934616 |
| 15903916 | 934001 |
| 15903848 | 933609 |
| 15902832 | 934332 |
| 15902372 | 934804 |
| 15902152 | 932889 |
| *Neisseria meningitidis* Z2491 | |
| 15793668 | 906680 |
| 15794714 | 907603 |
| *Streptococcus pyogenes* MGAS8232 | |
| 19747011 | 993608 |
| 19747024 | 994165 |
| 19747012 | 994373 |
| 19746396 | 995057 |
| 19746651 | 993824 |
| 19745883 | 995045 |
| 19745912 | 994077 |
| *Treponema pallidum* subsp. pallidum str. Nichols | |
| 15639844 | 2611061 |
| 15639720 | 2611059 |

**Table 7:** The list of 198 adhesins found in bacteria PapG (E. coli)

| | |
|---|---|
| | 7407207 |
| | 7407205 |
| | 147096 |
| | 4240529 |
| | 7407203 |
| | 42308 |
| | 7443327 |
| | 78746 |
| | 18265934 |
| | 26111419 |
| | 26250987 |
| | 26109826 |
| | 26249418 |
| | 13506767 |
| | *42301* |
| | 78745 |
| | 129622 |
| | 147092 |
| | 13506906 |
| | 7407209 |
| | 147080 |
| | 281926 |
| | 7407199 |
| | 147100 |
| | 78744 |
| SfaS (E.coli) | |
| | *477910* |

(continued)

| | |
|---|---|
| | 264035 |
| | 42959 |
| | 134449 |
| | 96425 |
| FimH (E.coli) | |
| | 26251208 |
| | 26111640 |
| | 5524634 |
| | 29422425 |
| | 5524630 |
| | 29422435 |
| | 29422415 |
| | 10946257 |
| | 29422419 |
| | 11120564 |
| | 29422457 |
| | 11120562 |
| | 29422459 |
| | 5524632 |
| | 29422455 |
| | 29422453 |
| | 29422451 |
| | 29422449 |
| | 29422447 |
| | 29422445 |
| | 29422443 |
| | 29422437 |
| | 29422433 |
| | 29422431 |
| | 29422429 |
| | 29422427 |
| | 29422423 |
| | 29422421 |
| | 29422417 |
| | 729494 |
| | 1361011 |
| | 1790775 |
| | 3599571 |
| | 29422441 |
| | 12620398 |
| | 29422439 |
| | 5524628 |
| | *1787779* |
| | 1742472 |
| | 1742463 |
| | 15801636 |
| | 25321294 |
| | 12515169 |
| | 11120566 |
| | 24051859 |
| | 24112911 |

(continued)

| | |
|---|---|
| | 13360484 |
| | 15800801 |
| | 15830279 |
| | 25392018 |
| | 25500156 |
| | 12514120 |
| | 1787173 |
| | 16128908 |
| | 16501811 |
| | 16759519 |
| | 24051219 |
| | 24112354 |
| | 30040724 |
| | 30062478 |
| | 6650093 |
| | 5524636 |
| | 1778448 |
| Intimin (E.coli) | |
| | *17384659* |
| | 4388530 |
| | 1389879 |
| | 15723931 |
| | 4323336 |
| | 4323338 |
| | 4323340 |
| | 4323342 |
| | 4323344 |
| | 4323346 |
| | 4323348 |
| | 4689314 |
| PrsG (E.coli) | |
| | *42523* |
| | 42529 |
| | 7443328 |
| | 7443329 |
| | 1172645 |
| HMW1 (Nontypeable *H. influenzae*) | |
| | 282097 |
| HMW2 (Nontypeable H. influenzae) | |
| | *5929966* |
| Hia (Nontypeable H. influenzae) | |
| | *25359682* |
| | 25359489 |
| | 25359709 |
| | 25359628 |
| | 25359414 |
| | 25359389 |
| | 21536216 |
| | 25359445 |
| HifE (H. influenzae) | |
| | *13506868* |

(continued)

| | |
|---|---|
| | 13506870 |
| | 13506872 |
| | 13506874 |
| | 13506876 |
| | 3688787 |
| | 3688790 |
| | 3688793 |
| | 2126301 |
| | 1170264 |
| | 1170265 |
| | 533127 |
| | 535169 |
| | 3025668 |
| | 3025670 |
| | 3025672 |
| | 3025674 |
| | 642038 |
| MrkD (K. pneumoniae) | |
| | *127307* |
| FHA (B. pertussis) | |
| | *17154501* |
| Pertactin (B. pertussis) | |
| | *33571840* |
| YadA (Y. enterocolitica) | *10955604* |
| | 4324391 |
| | 28372996 |
| | 23630568 |
| | 32470319 |
| SpaP (S. mutans) | |
| | *26007028* |
| | 47267 |
| PAc (S. mutans) | |
| | *129552* |
| SspA (Streptococcus gordonii) | |
| | *25990270* |
| | 1100971 |
| CshA (Streptococcus gordonii) | |
| | *457707* |
| CshB (Streptococcus gordonii) | |
| | *18389220* |
| ScaA (Streptococcus gordonii) | |
| | *310633* |
| SspB (Streptococcus gordonii) | |
| | *25055226* |
| | 3220006 |
| SpaA (Streptococcus sobrinus) | |
| | 546643 |
| PAg (Streptococcus sobrinus) | |
| | *217036* |
| | 47561 |
| Protein F (Streptococcus pyogenes) | *19224134* |

(continued)

| | |
|---|---|
| PsaA (Streptococcus pneumoniae) | |
| | *18252614* |
| | 7920456 |
| | 7920458 |
| | 7920460 |
| | 7920462 |
| CbpA[e] / SpsA / PbcA/ PspC (Streptococcus pneumoniae) | |
| | 14718654 |
| | 2425109 |
| | 2576331 |
| | 2576333 |
| | 3153898 |
| | 9845483 |
| | 19548141 |
| FimA (Streptococcus parasanguis) | |
| | *97883* |
| SsaB (Streptococcus sanguis) | |
| | *97882* |
| EfaA (Enterococcus faecalis) | |
| | *493017* |
| FnbA (Staphylococcus aureus) | |
| | *120457* |
| FnbB (Staphylococcus aureus) | |
| | *581562* |
| | 21205592 |
| | 13702452 |
| BabA (Helicobacter pylori) | |
| | *13309962* |
| | 13309964 |
| | 13309966 |
| | 13309968 |
| | 13309970 |
| | 13309972 |
| | 13309974 |
| | 13309976 |
| | 13309978 |
| | 13309980 |
| | 13309982 |
| | 13309984 |
| | 13309986 |
| | 13309988 |
| | 13309990 |
| | 13309992 |
| | 13309994 |

**Advantages:**

1. The method helps in discovering putative adhesins, which are of great importance in drug discoveries and preventive therapeutics.

2. The method is useful in predicting the adhesive nature of even unique proteins, because it is independent of the homology of the query proteins with other proteins.
3. This method is easy to use. For calculating the output, only the amino acid sequence is required as input. No other information is required to get the information about its adhesive nature.

**REFERENCES MAY BE MADE TO**

1. Andrea, T.A., Kalayeh, H.(1991) Applications of neural networks in quantitative structure-activity relationships of dihydrofolate reductase inhibitors. J. Med. Chem. 34, 2824-2836.
2. Altschul SF, Gish W, Miller W, Myers EW, Lipman DJ.(1990) Basic local alignment search tool. J Mol Biol. 215(3), 403-410.
3. Bassinet L, Gueirard P, Maitre B, Housset B, Gounon P, Guiso N.(2000) Role of adhesins and toxins in invasion of human tracheal epithelial cells by Bordetella pertussis. Infect Immun. 68(4), 1934-1941.
4. Bock, K., et al.(1985). Specificity of binding of a strain of uropathogenic Escherichia coli to Gal alpha 1----4Gal-containing glycosphingolipids. J. Biol. Chem. 260, 8545-8551.
5. Brendel, V., Bucher, P., Nourbakhsh, I.R., Edwin Blaisdell, B., and Karlin, S.(1992) Methods and algorithms for statistical analysis of protein sequences. Proc. Natl. Acad. Sci. USA 89, 2002-2006.
6. Brennan, M.J., Delogu, G., Chen, Y., Bardarov, S., Kriakov, J., Alavi, M., Jacobs, W.R., (2001).
7. Evidence that Mycobacterial PE_PGRS proteins are cell surface constituents that influence interactions with other cells. Infect. Immun, 69, 7326-7333.
8. De BK, Woolfitt AR, Barr JR, Daneshvar MI, Sampson JS, Ades EW, Carlone GM. (2003) Analysis of recombinant acylated pneumococcal surface adhesin A of Streptococcus pneumoniae by mass spectrometry. Arch Biochem Biophys. 15,419(2), 147-157.
9. Egland PG, Du LD, Kolenbrander PE (2001) Identification of independent Streptococcus gordonii SspA and SspB functions in coaggregation with Actinomyces naeslundii. Infect Immun. 69(12), 7512-7516
10. Finlay, B.B. and Falkow, S.(1997) Common themes in microbial pathogenicity revisited. Microbiol. Mol. Biol. Rev. 61, 136-169,
11. Fraser, C.M., Eisen, J., Fleischmann, R.D., Ketchum, K.A., Peterson, S.(2000) Comparative genomics and understanding of microbial biology. Emerg. Infect. Dis. 6,505-6512
12. Halperin, S. A., Scheifele, D., Mills, E., Guasparini, R., Humphreys, G., Barreto, L., Smith, B.(2003) Nature, evolution, and appraisal of adverse events and antibody response associated with the fifth consecutive dose of a five-component acellular pertussis-based combination vaccine. Vaccine 21, 2298-2306.
13. Hartford O, McDevitt D, Foster TJ.(1999) Matrix-binding proteins of Staphylococcus aureus: functional analysis of mutant and hybrid molecules. Microbiology. 145 (Pt 9), 2497-2505.
14. Hobohm, U. and Sander, C.(1995) A sequence property approach to searching protein databases. J. Mol. Biol. 251, 390-399.
15. Ide T, Michgehl S, Knappstein S, Heusipp G, Schmidt MA.(2003) Differential modulation by Ca2+ of type III secretion of diffusely adhering enteropathogenic Escherichia coli. Infect Immun. 71(4), 1725-1732.
16. Langermann S et al.(2000) Vaccination with FimH adhesin protects cynomolgus monkeys from colonization and infection by uropathogenic Escherichia coli. J. Infect. Dis. 181, 774-778.
17. Lowe A.M., Lambert, P.A., Smith, A.W.(1995) Cloning of an Enterococcus faecalis endocarditis antigen: homology with adhesins from some oral streptococci. Infect Immun. 63, 703-706.
18. Maurer, L., Orndorff, P.(1987). Identification and characterization of genes determining receptor binding and pilus length of Escherichia coli type 1 pili. J. Bacteriol. 169, 640-645
19. Marchler-Bauer A, Panchenko AR, Shoemaker BA, Thiessen PA, Geer LY, Bryant SH. (2002) CDD: a database of conserved domain alignments with links to domain three-dimensional structure. Nucleic Acids Res. 1, 30(1), 281-283.
20. Neubauer H, Hensel A, Aleksic S, Meyer H.(2000) Evaluation of a Yersinia adhesion gene (yadA) specific PCR for the identification of enteropathogenic Yersinia enterocolitica. Int J Food Microbiol. 15, 57(3), 225-227.
21. Nishikawa, K., Kubota, Y. and Ooi, T.(1983) Classification of proteins into groups based on amino acid composition and other characters. II. grouping into four types. J. Biochem. 94, 997-1007.
22. Peregrin-Alvarez, J.M., Tsoka, S., Ouzounis, C.A.(2003) The phylogenetic extent of metabolic enzymes and pathways. Genome Res. 13, 422-427.
23. Prinz, C., Hafsi, N. Voland, P.(2003) Helicobacter pylori virulence factors and the host immune response: implications for therapeutic vaccination. Trends in Microbiol. 11, 134-138.
24. Rapola, S., Jäntti, V., Eerola, M., Helena Mäkelä, P., Käyhty, H., Kilpi, T.(2003) Anti-PsaA and the risk of pneumococcal AOM and carriage. Vaccine 21, 3608-3613.
25. Rison. S.C., Teichmann, S.A., Thornton, J.M.(2002) Homology, pathway distance and chromosomal localization of the small molecule metabolism enzymes in Escherichia coli. J. Mol. Biol. 318, 911-932
26. Sperandio V, Bailey C, Giron JA, DiRita VJ, Silveira WD, Vettore AL, Kaper JB.(1996) Cloning and characterization of the gene encoding the OmpU outer membrane protein of Vibrio cholerae. Infect Immun. 64(12), 5406-5409.
27. St Geme JW 3rd, Cutter D.(2000) The Haemophilus influenzae Hia adhesin is an autotransporter protein that remains uncleaved at the C terminus and fully cell associated. J Bacteriol. 182(21), 6005-6013.
28. Thompson, J.D., Higgins, D.G., Gibson, T.J.(1994) CLUSTAL W: improving the sensitivity of progressive multiple sequence alignment through sequence weighting, position-specific gap penalties and weight matrix choice. Nucleic Acids Res. 22, 4673-4680
29. Van Schilfgaarde M, van Ulsen P, Eijk P, Brand M, Stam M, Kouame J, van Alphen L, Dankert J.(2000) Characterization of adherence of nontypeable Haemophilus influenzae to human epithelial cells. Infect Immun. 68(8), 4658-4665.
30. Wizemann, T.M., Adamou, J.E., Langermann, S.(1999). Adhesins as targets for vaccine development. Emerg. Infect. Dis. 5, 395-403,
31. Wolf, Y.I., Rogozin, I.B., Kondrashov, A.S., and Koonin, E.V.(2001) Genome alignment, evolution of prokaryotic genome organization and prediction of gene function using genomic context. Genome Res. 11, 356-372
32. Yu J, Leung WK, Go MY, Chan MC, To KF, Ng EK, Chan FK, Ling TK, Chung SC, Sung JJ.(2002) Relationship between Helicobacter pylori babA2 status with gastric epithelial cell turnover and premalignant gastric lesions. Gut. 51(4), 480-484.
33. Zuegge, J., Ralph, S., Schmuker, M., McFadden, G.I., Schneider, G.(2001) Deciphering apicoplast targeting signals--feature extraction from nuclear-encoded precursors of Plasmodium falciparum apicoplast proteins. Gene 280, 19-26.

Sequence --------
<213> OrganismName : Escherichia coli O157:H7
<400> PreSequenceString :

```
MARGWASSEA SGAMTDWLNN FGTARISLGV DEDFSLKNSQ FDFLHPWYDT PDYLLFSQHT     60
LHRTDDRTQI NTGLGWRHFT SSWMSGINLF FDHDLSRYHS RAGLGAEYWR DYLKLSSNAY    120
IGLTGWRSAP ELDNDFEARP ANGWDLRAEG WLPAWPQLGG KLVYEQYYGD EVALFDKNDR    180
QSNPHAITAG LNYTPFPLLT LSAEQRQGKQ GENDTRFAVD LTWQPSSSMQ KQLNPDEVAG    240
RRSLAGSRYD LIDRNNNIVL EYRKKELIRL SLLDPVKGKS GEIKPLVSSL QTKYALKGYN    300
IEAAALEAAG GKVSTSGKDI TVTLPGYRFT NTPETDNTWS IDVTAEDVKG NLSRHEQSMV    360
VIQAPTLSQK DSLLSVNPLT VAADKKSTTT LTVTAHDSDG TPVPGLALQT RSEGVQDITL    420
SDWTDNGDGS YTQILTAGTT SGSVTLTPQI NGESAVKESI VVNIVPVVSS RDHSSITIDN    480
VSYYAGDDIK VRVELKDDSN QPVAYQKEEL VKAVTVENSK PGATIVWHEE QPGVYAANYP    540
AYKQGTALRA QLSLHNWNAP LQSHIYNIEA NQNKARVATL SATNNDVYAD KKTFNTLTIN    600
VTDESDNPLT NHQVTFKNEK GSAEFVEPPQ QNTDAYGVAT INMVSQVAEE NTISATLPNG    660
FSQRIIAKFV SDSSTPKFKQ LVADPDTIIA GNSQGSTLTA IITDFHNNPL KDMKVNFVAP    720
GGSQLDNTTA TTDQSGIVRV HLTSSKAGSY SVDASLEVDK NIHQSVTITV VPNREQSVMT    780
LNAGSGSAIA NNTNIVTLTA SVKDVYGHPL PDEDVKFTLP ASMTGNFTLS SETARTDANG    840
DAVVTLRGTK AGEFTVTATL TRNNTVAYQQ VTFIGDTNSA QLQPLTASLN SIVAGNSTGS    900
TLTATILDAY QNPLKDQLVT FQSNDVTLSE TEVTTNTLGQ ATVTMTSNIA GQHNVVVSRK    960
AQASDNKTFS LSVLPDESSA KVISITGAEK TITVGENITL RILVQDAFNN VIAGQRVRLS   1020
AQPTTNITIG DTAYTDNNGY AYVNLLSTQP GVYQVTATLD NNSSSKVDVN VANGKLELTS   1080
SKPETTVHNS EGITLTATAR NARGELMPGQ IITFSVTPEG ATLSNTGEVL TDQSGQAKVT   1140
LTSDKVNVYT VTAIMGKDVP VQSQVTVAVK ADAKTAHVVS VVASPDTITA DGIDSSTITS   1200
RVEDDYGFPV EGVDISHGLD TKGSPVVNIP TTRTDQSGQV TATITSTLAE TLTVNVQVPG   1260
TANQSATITL VAGTADESKS ILKSDVDTLK ADYQQSAKLT LTLQDKYGNP IVTSDHLEFV   1320
QSGPFVNFLK LSDIDYSQRN YGEYTVTVTG GKEGTATLIP MLNGVHQANL SISLNLIQSI   1380
KEMSGHVTAN NHTFSTAKFP SEGFAGAYYT LNNDNFEAGK TVDDYMFSSS QGWVSVDASG   1440
KVSFANIGDQ TSVTISAVPR QGGTTYQTLI KLKGWWVNNG NHTNIWLAAN ALCHAKNDGY   1500
NLPGITHLTS GENKRTQGSL YGEWGNVGAF SSNSQFTPGA YWTSESDDYS RHYYVQMLTG   1560
MTGSDADSSP QLTACRKSL                                                1579
```

<212> Type : PRT
<211>
Length : 1579
SequenceName : SEQ ID 9
SequenceDescription :
Sequence --------
<213> OrganismName : Escherichia coli O157:H7

<400> PreSequenceString :

```
MITHGCYTRT RHKHKLKKTL IMLSAGLGLF FYVNQNSFAN GENYFKLGSD SKLLTHDSYQ   60
NRLFYTLKTG ETVADLSKSQ DINLSTIWSL NKHLYSSESE MMKAAPGQQI ILPLKKLPFE  120
YSALPLLGSA PLVAAGGVAG HTNKLTKMSP DVTKSNMTDD KALNYAAQQA ASLGSQLQSR  180
SLNGDYAKDT ALGIAGNQAS SQLQAWLQHY GTAEVNLQSG NNFDGSSLDF LLPFYDSEKM  240
LAFGQVGARY IDSRFTANLG AGQRFFLPAN MLGYNVFIDQ DFSGDNTRLG IGGEYWRDYF  300
KSSVNGYFRM SGWHESYNKK DYDERPANGF DIRFNGYLPS YPALGAKLIY EQYYGDNVAL  360
FNSDKLQSNP GAATVGVNYT PIPLVTMGID YRHGTGNEND LLYSMQFRYQ FDKSWSQQIE  420
PQYVNELRTL SGSRYDLVQR NNNIILEYKK QDILSLNIPH DINGTEHSTQ KIQLIVKSKY  480
GLDRIVWDDS ALRSQGGQIQ HSGSQSAQDY QAILPAYVQG GSNIYKVTAR AYDRNGNSSN  540
NVQLTITVLS NGQVVDQVGV TDFTADKTSA KADNADTITY TATVKKNGVA QANVPVSFNI  600
VSGTATLGAN SAKTDANGKA TVTLKSSTPG QVVVSAKTAE MTSALNASAV IFFDQTKASI  660
TEIKADKTTA VANGKDAIKY TVKVMKNGQP VNNQSVTFST NFGMFNGKSQ TQATTGNDGR  720
ATITLTSSSA GKATVSATVS DGAEVKATEV TFFDELKIDN KVDIIGNNVR GELPNIWLQY  780
GQFKLKASGG DGTYSWYSEN TSIATVDASG KVTLNGKGSV VIKATSGDKQ TVSYTIKAPS  840
YMIKVDKQAY YADAMSICKN LLPSTQTVLS DIYDSWGAAN KYSHYSSMNS ITAWIKQTSS  900
EQRSGVSSTY NLITQNPLPG VNVNTPNVYA VCVE                              934
```

<212> Type : PRT

<211>

Length : 934

SequenceName : SEQ ID 10

SequenceDescription :

Sequence

--------

<213> OrganismName : Escherichia coli O157:H7

<400> PreSequenceString :

```
MLVLSESFKN KLLPMNGYMK GGSDSGSKAQ ARATEKGIEL QREMWQTNMQ NLAPFTPLAQ   60

QYVSQLQNLS SLQGQGQALN QYYNSQQYKD LAGQARYQSL AAAEATGGLG STATGNQLAA  120
IAPTLGQNWL SGQMNNYNNL ANIGLGALTG QANAGQNYAN NVSQLYQQQA AASAANANKP  180
SGLQSFATGA IGGAASGAMI GSAVPVIGTG IGALAGGVIG GLGSLF                 226
```

<212> Type : PRT

<211>

Length : 226

SequenceName : SEQ ID 11

SequenceDescription :

Sequence

--------

<213> OrganismName : Escherichia coli O157:H7

<400> PreSequenceString :

```
MKKILSGLIL LLCCPYGFAA NGDGATHMSN LSFGPLTVAA ANNHSGYNIF EALSNTTGTY   60
PVRCHCDDTH GGPGQQTAFF PIFYTGDAAP GLVLERTLNG LNYYALNDYL SVGVTIFIIN  120
NQYAAIPFEH LSNQSTSPQH TCGAGNNGST VNLDSGRSAK LSFYVRHSIT GTVTIPTTEV  180
AWLYAGMSDH FPKTTPVSKV TIRGQLTAPQ NCELTPNQSI DVDFQKINSA EFSSTAGSII  240
AERKIKTEVT VSCTGMEDVR STEVVSASMI AANRSADATM IVTSNPDVGI KIFDKNDRPV  300
NVDGGNLPAD MGAISRLGKT DGSVTFYSAP ASLTGAKPAP DNGFTATATL VIEFTN      356
```

<212> Type : PRT

<211>

Length : 356

SequenceName : SEQ ID 12

SequenceDescription :

Sequence

--------

<213> OrganismName : Escherichia coli O157:H7

<400> PreSequenceString :

```
MNKIYRLKWN RSRNCWSVCS ELGSRVKGKK SRAVLISAIS LYSSLVFADD VIVNQDKTID     60
FGKENQSIDY RITVTDNANL VINATDTSRP RLTLASGGGL DITGGKVTIN GPLNFLLKGT    120
GFLNVSNAGS ELYADDLYES NSGMRHDRGY FNVSNGGKIH VKGTSRLTYL QGNVSGEGSQ    180
VNSETFFMGV YGSYGGNQYL SVNNGGEVNA RKQISLGYYD QVSDTTLAVS EGGKISAPTI    240
SLSTNSELAL GAQEGSAAKA AGIIDAEKIE FVWAKTSEKK ITLNHTDKDA TISADIVSGS    300
EGLGYINALN GTTYLTGDNS AFSGKVKIEQ NGALGITQNI GTAEINNRGK LHLKADDSMT    360
FANKISGNGT ISIDSGTVEL TGNNYAFSGY IDVASGAVAV ISEDKNIGRA ELDVDGKLQI    420
NANKDWVFDN DLEGRGIVEI NMGNHEFSFD EFAYTDWFQG SLAFQNTTFN LEKNAEFLQK    480
GGITAGQGSL VTVGKGAHSI STLGFSGGTV DFGALTAGAQ MTEGTVNVSK TLDLRGEGVI    540
QVSDSDVVRS VSRDIDSALS LTEVDDGNST IKLVDAQGAE VLGDAGNLQL QDKNGQILSS    600
SAQRDIQQNG QKAAVGTYDY RLTSGVNNDG LYIGYGLTQL DLHATDSDAL VLSSNGKSEN    660
AADLSAKITG SGDLAFSSQK GQTVSLSNKD NDYTGVTDLR SGTLLLNNDN VLGNTHELRL    720
AAETELDMNG HSQTVGTLNG SADSLLSLNG GSLTVTNGGT STGSLTGSGE LNIQGGTLDI    780
AGDNSNLTAN VNIANSANVL VSHAQGLGSA NVENNGTLAL NNSAEKRAAA SVNYALGGNL    840
TNNGTLMTGM SGQQAGNVLV VKGNYHGNNG QLVMNTVLNG DDSVTDKLVV EGDTSGTTAV    900
TVNNAGGTGA KTLNGIELIH VDGKSEGEFV QAGRIVAGAY DYTLARGQGA NSGNWYLTSG    960
SDSPELQPEP DPMPNPEPNP NPEPNPNPTP TPGPDLNVDN DLRPEAGSYI ANLAAANTMF   1020
TTRLHERLGN TYYTDMVTGE QKQTTMWMRH EGGHNKWRDG SGQLKTQSNR YVLQLGGDVA   1080
QWSQNGSDRW HVGVMAGYGN SDSKTISSRT GYRAKASVNG YSTGLYATWY ADDESRNGAY   1140
LDSWAQYSWF DNTVKGDDLQ SESYKSKGFT ASLEAGYKHK LAEFNGSQGT RNEWYVQPQA   1200
QVTWMGVKAD KHRESNGTLV HSNGDGNVQT RLGVKTWLKS HHKMDDGKSR EFQPFVEVNW   1260
LHNSKDFSTS MDGVSVTQDG ARNIAEIKTG VEGQLNANLN VWGNVGVQVA DRGYNDTSAM   1320
VGIKWQF                                                             1327
```

<212> Type : PRT
<211>
Length : 1327
SequenceName : SEQ ID 13
SequenceDescription :
Sequence --------
<213> OrganismName : Escherichia coli O157:H7
<400> PreSequenceString :

```
MITMKKSVLT AFITVVCATS SVMAADDNAI TDGSVTFNGK VIAPACTLVA ATKDSVVTLP     60
DVSATKLQTN GQVSGVQTDV PIELKDCDTT VTKNATFTFN GTADTTQITA FANQASSDAA    120
TNVALQMYMN DGTTAIKPDT ETGNILLQDG DQTLTFKVDY IATGKATSGN VNAVTNFHIN    180
YY                                                                  182
```

<212> Type : PRT
<211>
Length : 182
SequenceName : SEQ ID 14
SequenceDescription :
Sequence
--------
<213> OrganismName : Escherichia coli O157:H7
<400> PreSequenceString :

```
MSKFVKTAIA ATMVMGAFAS TSTIAAGNNG TARFYGTIED SPCSIVPDDH KLEVDMGDIG     60
SGILKNNGTS TPKAFQIHLQ DCVFDTQTTM TTTFTGNASS TNSGNYYTIY NTDTGAAFNN    120
VSLAIGDAQG TSYKSGAGIE QKIVNDTATN KGKAKQTLDF KAWLVGAADA PDLGNFEANT    180
TFQITYL                                                             187
```

<212> Type : PRT
<211>
Length : 187
SequenceName : SEQ ID 15
SequenceDescription :
Sequence
--------

<213> OrganismName : Escherichia coli O157:H7
<400> PreSequenceString :

```
MRVIFLRKEY LSLLPSMIAS LFSANGVAAA IDLCQGYDIK ASCHASRQSL SGITQVWSIA    60
DGQWLVFSDM TNNASGGAVF LQQGAEFTLS PENETGMTLF ANNTVSGEYN NGGAIFAKEN   120
STLNLTDVIF SGNVAGGYGG AIYSSGTNDT GAIDLRVTNA VFRNNIANDG KGGAIYTINN   180
DIYLSDDVFN NNQAYTSTSY SDGDGGAIDV TDNNSDSKHP SGYTIINNTA FTNNTAEGYG   240
GAIYTNSATA PYLIDISVDD SYSQNGGVLV DENNSAAGYG DGPSSAAGGF MYLGLSEVTF   300
DIADGKTLVI GNTENDGAVD SIAGTGLITK TGSGDLVLNA DNNDFTGEMQ IENGEVTLGR   360
SNSLMNVGDT HCQDDPQDCY GLTIGSIDKY QNQAELNVGS TQQTFAHSLT GFQNGTLNID   420
AGGNVTVNQG SFAGTIEGAG QLTIAQNGSY VLAGAQSMAL TGDIVVDAGA VLSLEGDAAD   480
LAALQDDPQS IVLNGGMLDL SDFSTWQSGT SYKDGLEVSG SSGTVIGSQD VVDLAGGNDM   540
HIGGDGKDGV YVVIDAGDGQ VSLANDNQYL GTTQIASGTL MVSDNSQLGY THYNRQVIFT   600
DKPQESVMEI TANVDTRSTT TEHGRDIEMR ADGEVAVDAG VDTQWGALMA DSSGQHQDEG   660
STLTKTGAGT LELTASGTTQ SAVRVEEGTL QGDVADIFPY ASSLWVGDGA TFVTGADQDI   720
QSIDATSSGT IDISDGTVLR LTGQDTSVAL NASLFNCDGT LVNATDGVTL TGELNTNLET   780
DSLTYLSNVT VNGNLTNTSG AVSLQNGVAG DTLTVNGDYT GGGTLLLDSE LNGDDSVSDQ   840
LVMNGNTAGN TTVVVNSITG IGEPTSTGIK VVDFAADPTQ FQNNAQFSLA GSGYVNMGAY   900
DYTLVEDNND WYLRSQEVTP PSPPDPDPTP DPDPTQDPDP TPDPEPTPAY QPVLNAKVGG   960
YLNNLRAANQ AFMMERRDHA GGDGQTLNLR VIGGDYHYTA AGQLAQHEDT STVQLSGDLF  1020
SGRWGTDGEW MLGIVGGYSD NQGDSRSSMT GTRADNQNHG YAVGLTSSWF QHGKQKQGAW  1080
LDNWLQYAWF SNDVSEHEDG VDHYHSSGII ASLEAGYQWL PGRGVVIEPQ AQVIYQGVQQ  1140
DDFTAANRAR VSQSQGDDIQ TRLGLHSEWR TAVHVIPTLD LNYYHDPHST EIEEDASTIS  1200
DDAVKQRGEI KVGVTGNISQ RVSLRGSVAW QKGSDDFAQT AGFLSMTVKW             1250
```

<212> Type : PRT
<211> Length : 1250
SequenceName : SEQ ID 16
SequenceDescription :
Sequence
--------
<213> OrganismName : Escherichia coli O157:H7
<400> PreSequenceString :

```
MHSWKKKLIV SQLALACTLA ITSQANAATN DISGQTYNTF HHYNDATYAD DVYYDGYVGW    60
NNYAADSYYN GDIYPVINNA TVNGVISTYY LDDGISTNTN ANSLTIKNST IHGMITSECM   120
TTDCADDRAT GYVYDRLTLS VDNSTIDDNY EHYTYNGTYN NAADTHVVDV YDMGTAITLD   180
QEVDLSITNN SHVAGITLTQ GYEWEDIDDN TVSTGVNSSE VFNNTITVKD STVTSGSWTD   240
EGTTGWFGHT GNASNYSNTL TADDVAIAAI ANPYADNAMQ TTVTLDNSTL MGDVVFSSNF   300
DENFFPQGAN SYRDADGDVD TNGWDGTDRM DVTLNNGSKW VGAAMSVHMV DEDGDGSYDG   360
YAVGTEATAT LLDIAANSLW PSSTVGVDNI NTQYDENGHI VGNEVYQSGL FNVTLNGGSE   420
WDTTKSSLID TLSINSGSQV NVADSRLISD TVSLTGGSNL NIGEDGHVAT NTLTIDNSTV   480
KMSDDVSAGW GLEDAALYAN TITVTNDGLL DINVDQFDAN PFQADTLNLT STTDTNGNIH   540
AGVFDIHSSD YVMDTDLVND RTNDTTKSNY GYGLIAMNSD GHLTINGNGD NDNTASIEAG   600
QNEVDNNGDH VAAATGNYKV RIDNATGAGS IADYNGNELI YVNDKNSNAT FSAANKADLG   660
AYTYQAEQRG NTVVLQQMEL TDYANMALSI PSANTNIWNL EQDTVGTRLT NSRHGLADNG   720
GAWVSYFGGN FNGDNGTINY DQDVNGIMVG VDTKIDGNNA KWIVGAAAGF AKGDMNDRSG   780
QVDQDSQTAY IYSSAHFANN VFVDGSLSYS HFNNDLSATM SNGTYVDGST NSDAWGFGLK   840
AGYDFKLGDA GYVTPYGSIS GLFQSGDDYQ LSNDMKVDGQ SYDSMRYELG VDAGYTFTYS   900
EDQALTPYFK LAYVYDDSNN DNDVNGDSID NGTEGSAVRV GLGTQFSFTK NFSAYTDANY   960
LGGGDVDQDW SANVGVKYTW                                               980
```

<212> Type : PRT
<211>

Length : 980
SequenceName : SEQ ID 17
SequenceDescription :

Sequence
<213> OrganismName : Escherichia coli 0157:H7
<400> PreSequenceString :

```
MKLKHVGMIV VSVLAMSSAA VSAAEGDESV TTTVNGGVIH FKGEVVNAAC AIDSESMNQT    60
VELGQVRSSR LAKAGDLSSA VGFNIKLNDC DTNVSSNAAV AFLGTTVTSN DDTLALQSSA   120
AGSAQNVGIQ ILDRTGEVLI LDGATFSAKT DLIDGTNILP FQARYIALGQ SVAGTANADA   180
TFKVQYL                                                             187
```

<212> Type : PRT
<211>
Length ; 187
SequenceName : SEQ ID 18
SequenceDescription :
Sequence
<213> OrganismName : Escherichia coli 0157:H7
<400> PreSequenceString :

```
MKLLKVAAIA AIVFSGSALA GVVPQYGGGG GNHGGGGNNS GPNSELNIYQ YGGGNSALAL    60
QADARNSDLT ITQHGGGNGA DVGQGSDDSS IDLTQRGFGN SATLDQWNGK DSHMTVKQFG   120
GGNGAAVDQT ASNSTVNVTQ VGFGNNATAH QY                      .          152
```

<212> Type : PRT
<211>
Length : 152
SequenceName : SEQ ID 19
SequenceDescription :
Sequence
<213> OrganismName : Escherichia coli 0157:H7
<400> PreSequenceString :

```
MPIGNLGHNP NVNNSIPPAP PLPSQTDGAG GRGQLINSTG PLGSRALFTP VRNSMADSGD    60
NRASDVPGLP VNPMRLAASE ITLNDGFEVL HDHGPLDTLN RQIGSSVFRV ETQEDGKHIA   120
VGQRNGVETS VVLSDQEYAR LQSIDPEGKD KFVFTGGRGG AGHAMVTVAS DITEARQRIL   180
ELLEPKGTGE SKGAGESKGV GELRESNSGA ENTTETQTST STSSLRSDPK LWLALGTVAT   240
GLIGLAATGI VQALALTPEP DSPTTTDPDA AASATETATR DQLTKEAFQN PDNQKVNIDE   300
LGNAIPSGVL KDDVVANIEE QAKAAGEEAK QQAIENNAQA QKKYDEQQAK RQEELKVSSG   360
AGYGLSGALI LGGGIGVAVT AALHRKNQPV EQTTTTTTTT TTTSARTVEN KPANNTPAQG   420
NVDTPGSEDT MESRRSSMAS TSSTFFDTSS IGTVQNPYAD VKTSLHDSQV PTSNSNTSVQ   480
NMGNTDSVVY STIQHPPRDT TDNGARLLGN PSAGIQSTYA RLALSGGLRH DMGGLTGGSN   540
SAVNTSNNPP APGSHRFV                                                 558
```

<212> Type : PRT
<211> Length : 558
SequenceName : SEQ ID 20
SequenceDescription :
Sequence
--------
<213> OrganismName : Escherichia coli O157:H7
<400> PreSequenceString :

```
MFSTFKKAAL LAAIALPFST MAAPTVTFQG EVTDQTCSVN INGQTNSVVL MPTVAMADFG    60
ATLADGQSAG QTPFTVSVSN CQAPTGADQA INTTFLGYDV DASTGVMGNR DTSSDAAKGF   120
GIQLMDSSTS GNPVTLAGAT NVPGLTLKVG DTEASYDFGA RYFVIDSAAA TAGKITAVAE   180
YTLSYL                                                              186
```

<212> Type : PRT
<211> Length : 186
SequenceName : SEQ ID 21
SequenceDescription :
Sequence
<213> OrganismName : Escherichia coli 0157:H7
<400> PreSequenceString :

```
MNSEGGKPGN VLTVNGNYTG NNGLMTFNAT LGGDNSPTDK MNVKGDTQGN TRVRVDNIGG    60
VGAQTVNGIE LIEVGGNSAG NFALTTGTVE AGAYVYTLAK GKGNDEKNWY LTSKWDGVTP   120

ADTPDPINNP PVVDPEGPSV YRPEAGSYIS NIAAANSLFS HRLHDRLGEP QYTDSLHSQD   180
SASSMWMRHV GGHERSSAGD GQLNTQANRY VLQLGGDLAQ WSSNAQDRWH LGVMAGYANQ   240
HSNTQSNRVG YKSDGRISGY SAGLYATWYQ NDANKTGAYV DSWALYNWFD NSVSSDNRSA   300
DDYDSRGVTA SVEGGYTFEA GTCSGSEGTL NTWYVQPQAQ ITWMGVKDSD HARKDGTRIE   360
TEGDGNVQTR LGVKTYLNSH HQRDDGKQRE FQPYIEANWI NNSKVYAVKM NGQTVSRDGA   420
RNLGEVRTGV EAKVNNNLSL WGNVGVQLGD KGYSDTQGML GVKYSW                  466
```

<212> Type : PRT
<211> Length : 466
SequenceName : SEQ ID 22
SequenceDescription :
Sequence
<213> OrganismName : Escherichia coli 0157:H7
<400> PreSequenceString :

```
MSYLNLRLYQ RNTQCLHIRK HRLAGFFVRL FVACAFAVQA PLSSAELYFN PRFLADDPQA    60
VADLSRFENG QELPPGTYRV DIYLNNGYMA TRDVTFNTGD SEQGIVPCLT RAQLASMGLN   120
TASVAGMNLL ADDACVPLTT MVQDATAHLD VGQQRLNLTI PQAFMSNRAR GYIPPELWDP   180
GINAGLLNYN FSGNSVQNRI GGNSHYAYLN LQSGLNIGAW RLRDNTTWSY NSSDRSSGSK   240
NKWQHINTWL ERDIIPLRSR LTLGDGYTQG DIFDGINFRG AQLASDDNML PDSQRGFAPV   300
IHGIARGTAQ VTIKQNGYDI YNSTVPPGPF TINDIYAAGN SGDLQVTIKE ADGSTQIFTV   360
PYSSVPLLQR EGHTRYSITA GEYRSGNAQQ EKPRFFQSTL LHGLPAGWTI YGGTQLADRY   420
RAFNFGIGKN MGALGALSVD MTQANSTLPD DSQHDGQSVR FLYNKSLNES GTNIQLVGYR   480
YSTSGYFNFA DTTYSRMNGY NIETQDGVIQ VKPKFTDYYN LAYNKRGKLQ LTVTQQLGRS   540
STLYLSGSHQ TYWGTSNVDE QFQAGLNTAF EDINWTLSYS LTKNAWQKGR DQMLARNVNI   600
PFSHWLRSDS KSQWRHASAS YSMSHDLNGR MTNLAGVYGT LLEDNNLSYS VQTGYAGGGD   660
GNSGSTGYAT LNYRGGYGNA NIGYSHSDDI KQLYYGVSGG VLAHANGVTL GQPLNDTVVL   720
VKAPGAKDAK VENQTGVRTD WRGYAVLPYA TEYRENRVAL DTNTLADNVD LDNAVANVVP   780
TRGAIVRAEF KARVGIKLLM TLTHNNKPLP FGAMVTSESS QSSGIVADNG QVYLSGMPLA   840
GKVQVKWGEE ENAHCVANYQ LPPESQQQLL TQLSAECR                          878
```

<212> Type : PRT
<211> Length : 878
SequenceName : SEQ ID 23
SequenceDescription :
Sequence
<213> OrganismName : Escherichia coli 0157:H7
<400> PreSequenceString :

```
MQIIFGEKCV SLLRLFFAAV LMLWCAQTAA YSGQCHTTQG NPYIGVNFGV KTLEEEENTT    60
GVVKDKFYQW NESNDYYVSC DCDKDNVRSG RWAFAADSPL VYLGDNWYKI NDYLAAKVLL   120
QVKGSSPTAV PFENVGTGAD TRWHICDPGG QRLGGQGASG NSGSFSLKIL QPFVGSVVIP   180
PMALARLFEC YNIPAGDSCT TTGTPVLVYY LSGTINSLGS CSVNAGETIE VDLGDVFAAN   240
FRVVGHKPLG ARTAELAIPV RCNTGNAGLV NVNLSLTATT DPSYPQAIKT SRPGVGVVVT   300
DSQNNIISPA GGTLPLSIPD DADSIA                                       326
```

<212> Type : PRT
<211> Length : 326
SequenceName : SEQ ID 24
SequenceDescription :
Sequence
<213> OrganismName : Escherichia coli 0157:H7
<400> PreSequenceString :

```
MKIKTLAIVV LSALSLSSTA ALAAATTVNG GTVHFKGEVV NAACAVDAGS VDQTVQLGQV    60
RTASLAQDGA TSSAVGFNIQ LNDCDTNVAS KAAVAFLGTV IDAGHTNVLA LQSSAAGSAT   120
NVGVQILDRT GAALTLDGAT FSEQTTLNNG TNTIPFQARY YAIGEATPGA ANADATFKVQ   180
YQ                                                                  182
```

<212> Type : PRT
<211> Length : 182
SequenceName : SEQ ID 25
SequenceDescription :
Sequence
<213> OrganismName : Escherichia coli 0157:H7
<400> PreSequenceString :

```
MKLKVIATLI ATVAVGVSFN SNFASASTTS ASLTVNSNLT MGTCSAQIMD NSNKVINEVV    60
FGNVYISELG AKSKVQQFKI RFSNCSGLPQ NSAQIVLAPN GISCAGSQSS SAGFSNKFTD   120
ASAATRTAVE VWTTDTPESN GSTQFHCAQK IPVPVTLPAD TTTQPYDYPL SARMTVAEGR   180
LVTDVRPGNF RSPTTFTITY Q                                             201
```

<212> Type : PRT
<211> Length : 201
SequenceName : SEQ ID 26
SequenceDescription :
Sequence
--------
<213> OrganismName : Escherichia coli O157:H7
<400> PreSequenceString :

```
MASTVEYGET VDGVVLEKDI QLVYGTANNT KINPGGEQHI KEFGISSNTE INGGYQYIEM    60
NGTAEYSVLN DGYQIVQMGG AANQTTLNNG VLQVYGAAND PTIKGGRLIV EKDGITVLAA   120
IEKGGLLEVK EGGLAIAVDQ KAGGAIKAST RVMEVFGTNR LGQFEIKNGI ANNMLLENGG   180
SLRVEENDFA YNTTVDSGGL LEVMDGGTAT GVDKKAGGKL IVSTNALEVS GTNSKGQFSI   240
KDGVSKNYEL DDGSGLIVME DTQAIDTILD EHATMQSLGK DTGTRVQANA VYDLGRSDQN   300
GSITYSSKAI SENMVINNGR ANVWAGTMVN VSVRGNDGIL EVMKPQINYA PAMLVGKVVV   360
SEGASLRTHG AVDTSKADVS LENSAWTIIA DITTTNQNTR LNLANLAMSG ANVIMMDESV   420
TRSSVTASAE NFTTLTTNTL SGNGNFYMRT DMANHQSDQL NVTGQATGDF KIFVTDTGAS   480
PAAGDSLTLV TTGGGDAAFT LGNAGGVVDI GTYEYTLLDN GNHSWSLAEN RAQITPSTTD   540
VLNMAAAQPL VFDAELDTVR ERLGSVKGVS YDTAMWSSAI NTRNNVTTDA GAGFEQTLTG   600
LTLGIDSRFS REESSTIRGL FFGYSHSDIG FDRGGKGNVD SYTLGAYAGW EHQNGAYVDG   660
VVKVDRFANT IHGKMSNGAT AFGDYNSNGA GAHVESGFRW VDGLWSVRPY LAFTGFTTDG   720
QDYTLSNGMR ADVGNTRILR AEAGTAVSYH MDLQNGTTLE PWLKAAVRQE YADSNQVKVN   780
DDGKFNNDVA GTRGVYQAGI RSSFTPTLSG HLSVSYGNGA GVESPWNTQA GVVWTF       836
```

<212> Type : PRT
<211> Length : 836
SequenceName : SEQ ID 27
SequenceDescription :
Sequence
--------
<213> OrganismName : Escherichia coli O157:H7
<400> PreSequenceString :

```
MQRKGNKLLI QLCSVILLFF TTSWYALANE CYIERNAEGD YHMKISSTQL SLASQMVEVP    60
TEIAEATWDV NIQLRGDAIG CKSLGDSKAV HFLNTADPSL ISTYTTTNGA ALLKTTVPGI   120
VYSVELLCLS CGAADELDLW LPAQSGADNF IPSTQTKWAY EYSDQSWYLR FRLFITPEFK   180
PKNGVSSGTT IAGKIASWYI GTNDQPWINF YIDNDSLKFF VDEPTCATVA LAQDQGNVSG   240
NQVTLGNSYV SEVKNGLTRE IPFSIRAEYC YASKITVKLK AANKPSDATL VGKTTGSASG   300
VAVKVNSTYD NSKVLLKADG SNTVDYNFAA WSNNLLFLPF TAQLVPDGSG NAVGVGTFSG   360
NATFSFTYE                                                           369
```

<212> Type : PRT
<211> Length : 369

SequenceName : SEQ ID 28
SequenceDescription :
Sequence
--------
<213> OrganismName : Escherichia coli O157:H7
<400> PreSequenceString :

```
MYQFTHQKSR IPKKTLLAAC CALFYSSNGA AADTVEYDSS FLMGTGASTI DVKRYAQGNP    60
TPPGLYNVRV FVNGQATSSL EIPFVDIGEN SAAACLTHKN LAQLHIKQPE QPVTLLAREG   120
EEEDCLDLAK SYEKADVCFD GSDQFLDLTI PQAYVLKSYG GYVDPSLWES GINAATLAYT   180
LNAYHTSSDN DNSDSVYGAF NSGINLGAWH FRARGNYNWT TDNGSDFDFQ DRYLQRDIPA   240
IRSQIIMGDA YTTGETFDSV NVRGVRLYSD SRMLPSALAS YAPTIRGVAN SNAKVTVTQS   300
GYKIYETTVP PGEFVIDDIS PSGFGSELVV TIEEADGSKR TFTQPFSSVV QMQRPGVGRW   360
DFSAGKVIDD SLRSEPNMGQ ASYYYGLNNL FTGYTGIQFT DNNYLAGLLG VGINTSIGAF   420
AVDVTHSRAE IPDDKTYQGQ SYRVTWNKLF QDTGTSFNLA AYRYSTQDYL GLHDALVLID   480
DAKHLSADED KNTMQTYSRM KNQFTVSINQ PLNIAYEDYG SLFISGSWTY YWAANNSRTE   540
YNVGYSKSVS WGSFSVNLQR SWNEDGEKDD AMYVSVSVPI ENILGGKRKS SGFRNLNTQL   600
NTDFDGSHQL NVNSSGNTEN NLVNYSVNAG YSLDKNAGDL ASVGGYLNYE SGLGGISASA   660
SATSDNSQQY SISTDGGFVL HSGGLTFTNN SFSSNDTLVL INALGAKGAR INNSNNEIDR   720
WGYAVTSSVS PYRENRVGLN IETLENDVEL KSTSATTVPR SGSVVLTRFE TDEGRSAVLN   780
ITAANGKSIP FAAEVYQGEV MIGSMGQGGQ AFVRGINDSG ELIVRWYENN QTIDCKLHYQ   840

FPAQPQTQGS TNTLLLNNLT CQVANH                                          866
```

FPAQPQTQGS TNTLLLNMT CQVANH 866
<212> Type : PRT
<211> Length : 866
SequenceName : SEQ ID 29
SequenceDescription :
Sequence
--------
<213> OrganismName : Escherichia coli O157:H7
<400> PreSequenceString :

```
MKFKRLLHSG IASLSLVACG VNAATDLGPA GDIHFSITIT TKACEMEKSD LEVDMGTMTL    60
QKPAAVGTVL SKKDFTIELK ECDGISKATV EMDSQSDSDD DSMFALEAGG ATGVALKIED   120
DKGTQQVPKG SSGTPIEWAI DGETTSLHYQ ASYVVVNTQA TGGTANALVN FSITYE       176
```

<212> Type : PRT
<211> Length : 176
SequenceName : SEQ ID 30
SequenceDescription :
Sequence
--------
<213> OrganismName : Escherichia coli O157:H7
<400> PreSequenceString :

```
MKYNNIIFLG LCLGLTTYSA LSADSVIKIS GRVLDYGCTV SSDSLNFTVD LQKNSARQFP    60
TTGSTSPAVP FQITLSECSK GTTGVRVAFN GIEDAENNTL LKLDEGSNTA SGLGIEILDG   120
NMRPVKLNDL HAGMQWIPLV PEQNNILPYS ARLKSTQKSV NPGLVRASAT FTLEFQ       176
```

<212> Type : PRT
<211> Length : 176
SequenceName : SEQ ID 31
SequenceDescription :

Sequence
--------
<213> OrganismName : Escherichia coli O157:H7
<400> PreSequenceString :

```
MKWRKRGYLL AAILALASAT IQAADVTITV NGKVVAKPCT VSTTNATVDL GDLYSFSLMS      60
AGAASAWHDV ALELTNCPVG TSRVTASFSG AADSTGYYKN QGTAQNIQLE LQDDSGNTLN     120
TGATKTVQVD DSSQSAHFPL QVRALTVNGG ATQGTIQAVI SITYTYS                   167
```

<212> Type : PRT
<211> Length : 167
SequenceName : SEQ ID 32
SequenceDescription :
Sequence
--------
<213> OrganismName : Escherichia coli O157:H7
<400> PreSequenceString :

```
MKRAPLITGL LLISTSCAYA SSEGCGADST SGATNYSSVV DDVTVNQTDN VTGREFTSAT      60
LSSTNWQYAC SCSAGKAVKL VYMVSPVLTT TGHQTGYYKL NDSLDIKTMN RPGNPGD        117
```

<212> Type : PRT
<211> Length : 117
SequenceName : SEQ ID 33
SequenceDescription :
Sequence
--------
<213> OrganismName : Escherichia coli O157:H7
<400> PreSequenceString :

```
MKKALLAAAL VMASGSALAV DGGHIDFNGM VQSGTCKVGV VDTGMHSVTT DGVVTLDTAN      60
VTDTFAEVSA TAVGLLPKEF MISVECDPGA PKNAELTMGS ASYANTSGTL NNNMNITVNG     120
IAPAQNVNIA VHNMKNKAGA AEIKQVHMNN SSEVQELTLD AEGKGQYVFN ASYVKAPNSP     180
AVTAGHVTTN ALYTVAYK                                                   198
```

<212> Type : PRT
<211> Length : 198
SequenceName : SEQ ID 34
SequenceDescription :
Sequence
--------
<213> OrganismName : Escherichia coli O157:H7
<400> PreSequenceString :

```
MKPNMIVGAL ALTSVFMAGH LQAADGTVHF RGEIIDSTCE VTPETKDQVV DLGKVNRTAF      60
SGVDDVAAPT AFSIDLTQCP ETFKSAAIRF DGNEDAHGNG NLAIGTPLDN SNDAAAGISP     120
SDNSGDYTGA GAVSAAKGVA IRLYNRADNT QVKLYENSAS TPISNGNASM KFMARYIATE     180
TTIDPGTANA DSQFTVEYIK                                                 200
```

<212> Type : PRT
<211> Length : 200
SequenceName : SEQ ID 35
SequenceDescription :
Sequence
--------
<213> OrganismName : Escherichia coli O157:H7
<400> PreSequenceString :

```
MPIFQREGHL KYSFAAGEYQ AGNYDSASPR FGQLDLIYGL PWGMTAYGGV LISNNYNAFT     60
LGIGKNFGYI GAISIDVTQA KSELNNDRDS QGQSYRFLYS KSFESGTDFR LAGYRYSTSG    120
FYTFQEATDV RSDADSDYNR YHKRSEIQGN LTQQLGAYGS VYLNLTQQDY WNDAGKQNTV    180
SAGYNGRIGK VSYSIAYSWN KSPEWDESDR LWSFNISVPL GRAWSNYRVT TDQDGRTNQQ    240
VGVSGTLLED RNLSYSVQEG YASNGVGNSG NANVGYQGGS GNVNVGYSYG KDYRQLNYSV    300
RGGVIVHSEG VTLSQPLGET MTLISVPGAR NARVVNNGGV QVDWMGNAIV PYAMPYRENE    360
ISLRSDSLGD DVDVENAFQK VVPTRGAIVR ARFDTRVGYR VLMTLLRSAG SPVPFGATAT    420
LITDKQNEVS SIVGEEGQLY ISGMPEEGRV LIKWGNDASQ QCVAPYKLSL ELKQGGIIPV    480
SANCQ                                                                485
```

<212> Type : PRT
<211> Length : 485
SequenceName : SEQ ID 36
SequenceDescription :
Sequence
--------
<213> OrganismName : Escherichia coli O157:H7
<400> PreSequenceString :

```
MSGYTVKPPT GDSNEQTQFI DYFNLFYSKR DQEQISISQQ LGNYGATFFS ASRQSYWNTS     60
RSDQQISFGL NVPFGDITTS LNYSYSNNIW QNDRDHLLAF TLNVPFSHWM RTDSQSAFRN    120
SNASYSMSND LKGGMTNLSG VYGTLLPDNN LNYSVQVGNT HGGNTSSGTS GYSTLNYRGA    180
YGNTNVGYSR SGDSSQIYYG MSGGIIAHAD GITFGQPLGD TMVLVKAPGA DNVKIENQTG    240
IHTDWRGYAI LPFATEYREN RVALNANSLA DNVELDETVV TVIPTHGAIA RATFNAQIGG    300
KVLMTLKYGN KSVPFGAIVT HGENKNGSIV AENGQVYLTG LPQSGKLQVS WGNDKNSNCI    360
VDYKLPEVSP GTLLNQQTAI CR                                             382
```

<212> Type : PRT
<211> Length : 382
SequenceName : SEQ ID 37
SequenceDescription :
Sequence
--------
<213> OrganismName : Escherichia coli O157:H7
<400> PreSequenceString :

```
MSALYERSQL TQVMISSAPA TAETMEKAEY LRLDCTIKEV QFTAGQKQDI DVTTLCSTEQ     60
ENINGLGASS EISMSGNFYL NQAQNALRDA YDNDTVYAFK VQFPSGKGFK FLAEVRQHTW    120
SSGTNGVVAA TFSLRLKGKP VSYVVPLAFV KNLDKTLTVN TGALLTMSVS VNGGTPPYKH    180
AWKKDGQPVE GQTTDTFSKP GAQSGDKGAY TCEVTDSAEQ PQSITSDACT VTVNGAGG      238
```

<212> Type : PRT
<211> Length : 238
SequenceName : SEQ ID 38
SequenceDescription :
Sequence
--------
<213> OrganismName : Escherichia coli O157:H7
<400> PreSequenceString :

```
MRNKPFYLLC AFLWLAVSHA LAADSTITIR GYVRDNGCSV AAESTNFTVD LMENAAKQFN     60
NIGATTPVVP FRILLSPCGN AVSAVKVGFT GVADSHNANL LALENTVSAA SGLGIQLLNE    120
QQNQIPLNAP SSAISWTTLT PGKPNTLNFY ARLMATQVPV TAGHINATAT FTLEYQ        176
```

<212> Type : PRT
<211> Length : 176
SequenceName : SEQ ID 39
SequenceDescription :
Sequence
--------

<213> OrganismName : Escherichia coli O157:H7
<400> PreSequenceString :

```
MNKSVVSISA AMLVLLCQPV MGSEISPATP SDEDNYTFDP QLFRGSRFSQ SSLAKLTTRE    60
SVAPGNYKMD IYTNNKLSGS WNVTFKEAAD GRVLPCLTPE VADAIGLKTG EDKGEKDPVC   120
TFAKELAPGI TSQTQLSQLR LDLSVPQSQL ISRPRGYVPP SELDTGASLA FMNYIANYYN   180
VAYSGQNAHS QRSLWASFNG GINLGAWQYR QLSNMTWDND KGNQWNNIRS YLQRPLPAIN   240
SQLMMGQLIT SGRFFSGLSY HGVSLATDER MLPDSMRGYA PTIRGVAATN ARVSVMQNGH   300
EIYQTTVAPG PFEINDLYPT SYSGDLDVTV TEANGAVSRF SVPFSAVPES MRPGTSRYNV   360
EVGKTQDSGD DSMFGDLTWQ HGMTNTLTFN SGSRIADGYQ ALMLGGVYGS SLGAFGANLT   420
WSHARVPESE AQSGWMSQLT WSKTFQPTST TVSLAGYRYS TSGYRDLADV LGERHAASNK   480
QSWDSSQWRQ QSRFDLTLSQ SLANYGNLFV SGSTQNYRGG KSRDTQLQLG YSNSFSHGIS   540
MNLSVGRQRM GGYKDNSDDM QTVTSLSFSF PLGGNGPRVP SLSNSWTHST DGSSQLQSSL   600
TGMLDEAQTT NYSLNVMRDQ QYKQTTLSGN MQKRFSQTTV GLNASKGQDY WQASGNVQGA   660
MAVHGGGITF GPYLGETFAL VEAKGAEGAK VYNSSQLEIN DSGYALVPAV TPYRYNRISL   720
DPQGMDGDAE LVDSERQVAP VAGAAVKVIF RTRPGKALLI KSRMADGSEL PMGADVLDEN   780
NTVVGIAGQG GQIYLRTEQT KGHLSVRWGE GANDSCQLPF DISGKDSNSP IIRLNETCQS   840
```

<212> Type : PRT
<211> Length : 840
SequenceName : SEQ ID 40
SequenceDescription :
Sequence
--------
<213> OrganismName : Escherichia coli O157:H7
<400> PreSequenceString :

```
MKLAACFLTL LPGFAVAASW TSPGFPAFSE QGTGTFVSHA QLPKGTRPLT LNFDQQCWQP    60
ADAIKLNQML SLQPCSNTPP QWRLFRDGKY TLQIDTRSGT PTLMISIQNA AEPVANLVRE   120
CPKWDGLPLT LDVSATFPEG AAVRDYYSQQ IAIVKNGQIT LQPAATSNGL LLLERAETDA   180
SAPFDWHNAT VYFVLTDRFE NGDPSNDQSY GRHKDGMAEI GTFHGGDLRG LTNKLDYLQQ   240
LGVNALWISA PFEQIHGWVG GGTKGDFPHY AYHGYYTQDW TNLDANMGNE ADLRTLVDSA   300
HQRGIRILFD VVMNHTGYAT LADMQEYQFG ALYLSGDEVK KTLGERWSDW KPAAGQTWHS   360
FNDYINFSDK TGWDKWWGKN WIRTDIGDYD NPGFDDLTMS LAFLPDIKTE STTASGLPVF   420
YKNKTDTHAK AIDGFTPRDY LTHWLSQWVR DYGIDGFRVD TAKHVELPAW QQLKTEASAA   480
LREWKKANPD KALDDKPFWM TGEAWGHGVM QSDYYRHGFD AMINFDYQEQ AAKAVDCIAQ   540
MDTTWQQMAE KLQGFNVLSY LSSHDTRLFR EGGDKAAELL LLAPGAVQIF YGDESSRPFG   600
PTGSDPLQGT RSDMNWQDVS GKSAANVAHW QKISQFRARH PAIGAGKQTT LSLKQGYGFV   660
REHGDDKVLV IWAGQQ                                                 676
```

<212> Type : PRT
<211> Length : 676
SequenceName : SEQ ID 41
SequenceDescription :
Sequence
--------
<213> OrganismName : Escherichia coli O157:H7
<400> PreSequenceString :

```
MPQRHHQGHK RTPKQLALII KRCLPMVLTG SGMLCTTANA EEYYFDPIML ETTKSGMQTT    60
DLSRFSKKYA QLPGTYQVDI WLNKKKVSQK KITFTANAEQ LLQPQFTVEQ LRELGIKVDE   120
IPALAEKDDD SVINSLEQII PGTAAEFDFN HQRLNLSIPQ IALYRDARGY VSPSRWDDGI   180
PTLFTNYSFT GSDNRYRQGN RSQRQYLNMQ NGANFGPWRL RNYSTWTRND QASSWNTISS   240
YLQRDIKALK SQLLLGESAT SGSIFSSYNF TGVQLASDDN MLPNSQRGFA PTVRGIANSS   300
AIVTIRQNGY VIYQSNVPAG AFEINDLYPS SNSGDLEVTI EESDGTQRRF IQPYSSLPMM   360
QRPGHLKYSA TAGRYRADAN SDSKEPEFAE ATAIYGLNNT FTLYGGLLGS EDYYALGIGI   420
GGTLGALGAL SMDINRADTQ FDNQHSFHGY QWRTQYIKDI PETNTNIAVS YYRYTNDGYF   480
SFDEANTRNW DYNSRQKSEI QFNISQTIFD GVSLYASGSQ QDYWGNNEKN RNISVGVSGQ   540
```

QWGIGYSLNY QYSRYTDQNN DRALSLNLSI PLERWLPRSR VSYQMTSQKD RPTQHEMRLD 600
GSLLDDGRLS YSLEQSLDDD NNHNSSVNAS YRSPYGTFSA GYSYGNDSSQ YNYGVTGGVV 660
IHPHGVTLSQ YLGNAFALID ANGASGVRIQ NYPGIATDPF GYAVVPYLTT YQENRLSVDT 720
TQLPDNVDLE QTTQFVVPNR GAMVAARFNA NIGYRVLVTV SDRNGKPLPF GALASNDDTG 780
QQSIVDEGGI LYLSGISSKS QSWTVRWGNQ ADQQCQFAFS TPDSEPTTSV LQGTAQCH 838

<212> Type : PRT
<211> Length : 838
SequenceName : SEQ ID 42
SequenceDescription :
Sequence --------
<213> OrganismName : Escherichia coli O157:H7
<400> PreSequenceString :

MMFRNRILLI FILWANFTWA GCRTTASLNI TDGINVGEIL ANETSFSKSV VFTGISCDTS 60
TDKIVYKNIQ SDWVEVGPFG NGEKLKVKIE SLGKTSDTIG KSSNAQAVLP YVVKIARGTP 120
DFTGERKSTW FISDTVIANI GGESSSSIDF WLGICKALKF NWCVNYLTSK LAGDTFTLGL 180
NISYYPKNTT CKPENTVIKV DDIALFQLRN QGKIAANSKE GTITLKCDNL FGDKKQASRN 240
MVVYLSSSDL VKGSNTILRG KTDNGVGFVL DLTEPPKGTE AAIKISANGD QGAATSLWKT 300
DKPGVSLNSN IINIPVMASY YVYDEKKVKS GALEATALIN VKYD 344

<212> Type : PRT
<211> Length : 344
SequenceName : SEQ ID 43
SequenceDescription :
Sequence
--------
<213> OrganismName : Escherichia coli O157:H7
<400> PreSequenceString :

MIKKASLLTA CSVTAFSAWA QDTSPDTLVV TANRFEQPRS TVLAPTTVVT RQDIDRWQST 60
SVNDVLRRLP GVDITQNGGS GQLSSIFIRG TNASHVLVLI DGVRLNLAGG SGSADLSQFP 120
IALVQRVEYI RGPRSAVYGS DAIGGVVNII TTRDEPGTEI SAGWGSNSYQ NYDVSTQQQL 180
GDKTRVTLLG DYAHTHGYDV VAYGNTGTQA QPDNDGFLSK TLYGALEHNF TDAWSGFVRG 240
YGYDNRTNYD AYYSPGSPLV DTRKLYSQSW DAGLRYNGEL IKSQLITSYS HSKDYNYDPH 300
YGRYDSSATL DEMKQYTVQW ANNIIIGHGN VGAGVDWQKQ STAPGTAYVK DGYDQRNTGI 360
YLTGLQQVGD FTFEGAARSD DNSQFGRHGT WQTSAGWEFI EGYRFIASYG TSYKAPNLGQ 420
LYGFYGNPNL DPEKSKQWEG AFEGLTAGVN WRISGYRNDV SDLIDYDDHT LKYYNEGKAR 480
IKGVEATANF DTGPLTHTVS YDYVDARNAI TDTPLLRRAK QQVKYQLDWQ LYDFDWGITY 540
QYLGTRYDKD YSSYPYQTVK MGGVSLWDLA VAYPVTSHLT VRGKIANLFD KDYETVYGYQ 600
TAGREYTLSG SYTF 614

<212> Type : PRT
<211> Length : 614
SequenceName : SEQ ID 44
SequenceDescription :
Sequence --------
<213> OrganismName : Escherichia coli O157:H7
<400> PreSequenceString :

MKNKLLFMML TILGAPGIAA AAGYDLANSE YNFAVNELSK SSFNQAAIIG QAGTNNSAQL 60
RQGGSKLLAV VAQEGSSNRA KIDQTGDYNL AYIDQAGSAN DASISQGAYG NTAMIIQKGS 120
GNKANITQYG TQKTAIVVQR QSQMAIRVTQ R 151

<212> Type : PRT
<211> Length : 151
SequenceName : SEQ ID 45
SequenceDescription :
Sequence
--------

<213> OrganismName : Escherichia coli O157:H7
<400> PreSequenceString :

```
MNIFAYLLVL VFSMSMSSSA FASVVMTGTR IIFPGDAKEK TIQLRNTSDQ PYIINIHVED    60
ERGSDKNVPF MPTPQTFRME AAAGQALRLL YTGNNLPQDR ESVFWFSFSQ LPYLNKNDKS   120
QNQLILALTN RVKIFYRPSS IVGKSSDAPK NLTYQVKQNR IEVTNPTGYY VTIRAAELLN   180
NGKKVPLANS VMIAPQSTTE WTLPSGISVA PGAQIHLVTV NDYGVNVTSE HAL          233
```

<212> Type : PRT
<211> Length : 233
SequenceName : SEQ ID 46
SequenceDescription :
Sequence
--------
<213> OrganismName : Escherichia coli O157:H7
<400> PreSequenceString :

```
MKRLHKRFLL ATFCALLTAT LQAADVTITV NGRVVAKPCT IQTKEANVNL GDLYTRNLQQ    60
PGSASGWHNI TLSLTDCPAE TSAVTAIVTG STDNTGYYKN EGTAENIQIE LRDDQDATLK   120
NGDSKTVIVD EITRNAQFPL KARAITVNGN ASQGTIEALI NVIYTWQ                 167
```

<212> Type : PRT
<211> Length : 167
SequenceName : SEQ ID 47
SequenceDescription :
Sequence --------
<213> OrganismName : Escherichia coli O157:H7
<400> PreSequenceString :

```
MRAKLLGIVL TTPIAISSFA STETLSFTPD NINADISLGT LSGKTKERVY LAEEGGRKVS    60
QLDWKFNNAA IIKGAINWDL MPQISIGAAG WTTLGSRGGN MVDQDWMDSS NPGTWTDESR   120
HPDTQLNYAN EFDLNIKGWL LNEPNYRLGL MAGYQESRYS FTARGGSYIY SSEEGFRDDI   180
GSFPNGERAI GYKQRFKMPY IGLTGSYRYE DFELGGTFKY SGWVEASDND EHYDPGKRIT   240
YRSKVKDQNY YSVSVNAGYY VTPNAKVYVE GTWNRVTNKK GNTSLYDHND NTSDYSKNGA   300
GIENYNFITT AGLKYTF                                                  317
```

<212> Type : PRT
<211> Length : 317
SequenceName : SEQ ID 48
SequenceDescription :
Sequence
--------
<213> OrganismName : Escherichia coli O157:H7
<400> PreSequenceString :

```
MFFKRGKILS AGRLNKKSLG IVMLLSVGLL LAGCSGSKSS DTGTYSGSVY TVKRGDTLYR    60
ISRTTGTSVK ELARLNGISP PYTIEVGQKL KLGGAKSSSS TRKSTAKSTT KTASVTPSSA   120
VPKSSWPPVG QRCWLWPTTG KVIMPYSTAD GGNKGIDISA PRGTPIYAAG AGKVVYVGNQ   180
LRGYGNLIMI KHSEDYITAY AHNDTMLVNN GQSVKAGQKI ATMGSTDAAS VRLHFQIRYR   240
ATAIDPLRYL PPQGSKPKC                                                259
```

<212> Type : PRT
<211> Length : 259
SequenceName : SEQ ID 49
SequenceDescription :
Sequence --------
<213> OrganismName : Escherichia coli O157:H7
<400> PreSequenceString :

MPTPNPLAPV KGAGTTLWVY NGNGDPYANP LSDNDWSRLA KVKDLTPGEL TAESYDDSYL 60
DDEDADWAAT GQGQKSAGDT SFTLAWMPGE QGQQALLAWF NEGDTRAYKI RFPNGTVDVF 120
RGWVSSIGKA VTAKEVITRT VKVTNVGRPS MAEDRSTVTA ATGMTVTPAS TSVVKGQSTT 180
LTVAFQPEGA TDKSFRAVSA DKTKATVSVS GMTITVKGVA AGKVNIPVVS GNGEFAAVAE 240
INVTAS 246

<212> Type : PRT
<211> Length : 246
SequenceName : SEQ ID 50
SequenceDescription :
Sequence --------
<213> OrganismName : Escherichia coli O157:H7
<400> PreSequenceString :

MSALYERSQL TQVMISSAPA TAETMDKAEY LRLDCTIKEV QFTAGQKQDI DVTTLCSTEQ 60
ENINGLGASS EISMSGNFYL NQAQNALRDA YDNDALYAFK VLFPSGKGFK FLAEVRQHTW 120
SSGTNGVVAA TFSLRLKGKP VSFVVPLAFV KNLDKTLTVN TGALLTMSVS ANGGTPPYKY 180

AWKKDGQPVD GQTTDTFSKP GAQSADAGKY TCVVTDSAEK AQSVTSVECT VTVSAAAG 238

<212> Type : PRT
<211> Length : 238
SequenceName : SEQ ID 51
SequenceDescription :
Sequence
--------
<213> OrganismName : Escherichia coli O157:H7
<400> PreSequenceString :

MKKSTLALVV MGIVASASVQ AAEIYNKDGN KLDVYGKVKA MHYMSDNDSK DGDQSYIRFG 60
FKGETQINDQ LTGYGRWEAE FAGNKAESDT AQQKTRLAFA GLKYKDLGSF DYGRNLGALY 120
DVEAWTDMFP EFGGDSSAQT DNFMTKRASG LATYRNTDFF GVIDCLNLTL QYQGKNENRD 180
VKKQNGDGFG TSLTYDFGGS DFAISGAYTN SDRTNEQNLQ SRGTGKRAEA WATGLKYDAN 240
NIYLATFYSE TRKMTPITGG FANKTQNFEA VAQYQFDFGL RPSLGYVLSK GKDIEGIGDE 300
DLVNYIDVGA TYYFNKNMSA FVDYKINQLD SDNKLNINND DIVAVGMTYQ F 351

<212> Type : PRT
<211> Length : 351
SequenceName : SEQ ID 52
SequenceDescription :
Sequence --------
<213> OrganismName : Escherichia coli O157:H7
<400> PreSequenceString :

MRVKHAVVLL MLISPLSWAG TMTFQFRNPN FGGNPNNGAF LLNSAQAQNS YKDPSYNDDF 60
GIETPSALDN FTQAIQSQIL GGLLSNINTG KPGRMVTNDY IVDIANRDGQ LQLNVTDRKT 120
GQTSTIQVSG LQNNSTDF 138

<212> Type : PRT
<211> Length : 138
SequenceName : SEQ ID 53
SequenceDescription :
Sequence --------
<213> OrganismName : Escherichia coli O157:H7
<400> PreSequenceString :

```
MKRKVLAMLV PALLVAGAAN AAEIYNKDGN KLDLYGKVAG LHYFSDDASS DGDMSYARIG      60
FKGETQIADQ FTGYGQWEFN IGANGPESDK GNTATRLAFA GFGFGQNGTF DYGRNYGVVY     120
DVEAWTDMLP EFGGDTYAGA DNFMNGRANS VATYRNNGFF GQVDGLNFAL QYQGNNEKSG     180
LFDQEGSGNG NGRKLAKENG DGSVCPLPMT LTLV                                214
```

<212> Type : PRT
<211> Length : 214
SequenceName : SEQ ID 54
SequenceDescription :
Sequence --------
<213> OrganismName : Escherichia coli O157:H7
<400> PreSequenceString :

```
MNTVTLEGGT FNNNGTLNDV VKIEKNSNAV INNTGSLSTL QLHDGTVNNS GIASARVNAQ      60
GDAVFNNLAG GEARKGAILY NSAVVNNAGT WKMGYQDENN NAGTLDIDDK STFNNSGKLI     120
LDNSKNAIRF QGSNANATLY NTGEMTLDAA LGAGAILYDD GASEFINKGV VDAKVTVAVS     180
TAGATESDAF LWNQDGGVIN FDKDNASAVK FTHNNYVALN DGVMNISGNN AVAMEGDKNA     240
QLVNNGVINL GTEGTTDTGL TGMQLDANAT ADAVIENNGT INIFANDSFA FSVLGTEGHI     300
VNNGTVVIAD GVTGSGLIKQ GDSVNVEGVN GNSGNNTEVH YTDYTLPDMP NTYTTSPFSE     360
TTDSGSSDGS SNNLNGYIVG TNVDGSAGKL KVNNASMNGV GINTGFAAGT ADTTVSFDNV     420
VEGINLTDAD AITSTSVVWT AKGSTDASGN VDVIMSKNAY TDVATDASVN DVAKALDAGY     480
TNNELYTSLN VGTTAELNSA LKQVSGSQAT TVFREARVLS NRFSMLADAA PKVGNGLAFN     540
VVAKGDPRAE LGNNTEYDML ALRKTVDLSE SQSMSLEYGI ARLDGDGAQK AGDNGVTGGY     600
SQFFGLKHQM SFDNGMRWNN ALRYDVHNLD SSRSVAYGDV SKTADTDVKQ QYLELRSEGA     660
KTFEPREGLK ITPYAGVKLR HSLEGGYQER NAGDFNLSMN SGSETAVDSI VGLKLDYAGK     720
GGWSANATLE GGPNLSYSKS QRTASLAGAG SQHFNVDDGQ KGGGINSLAS VGVKYSSKES     780
SLNLDAYHWK EDGISDKGVM LNFKKTF                                        807
```

<212> Type : PRT
<211> Length : 807
SequenceName : SEQ ID 55
SequenceDescription :
Sequence
--------
<213> OrganismName : Escherichia coli O157:H7
<400> PreSequenceString :

```
MLNGISNAAS TLGRQLVGIA SRVSSAGGTG FSVAPQAVRL TPVKVHSPFS PGSSNVNART      60
IFNVSSQVTS FTPSRPAPPP PTSGQASGAS RPLPPIAQAL KEHLAAYEKS KGPEALGFKP     120
ARQAPPPPTS GQASGASRPL PPIAQALKEH LAAYEKSKGP EALGFKPARQ APPPPTSGQA     180
SGASRPLPPI AQALKEHLAA YEKSKGPEAL GFKPARQAPP PPTGPSGLPP LAQALKDHLA     240
AYEQSKKG                                                             248
```

<212> Type : PRT
<211> Length : 248
SequenceName : SEQ ID 56
SequenceDescription :
Sequence --------
<213> OrganismName : Escherichia coli O157:H7
<400> PreSequenceString :

```
MNKKIHSLAL LVNLGIYGVA QAQEPTDTPV SHDDTIVVTA AEQNLQAPGV STITADEIRK    60
NPVARDVSEI IRTMPGVNLT GNSTSGQRGN NRQIDIRGMG PENTLILIDG KPVSSRNSVR   120
QGWRGERDTR GDTSWVPPEM IERIEVLRGP AAARYGNGAA GGVVNIITKK GSGEWHGSWD   180
AYFNAPEHKE EGATKRTNFS LTGPLGDEFS FRLYGNLDKT QADAWDINQG HQSARAGTYA   240
TTLPAGREGV INKDINGVVR WDFAPLQSLE LEAGYSRQGN LYAGDTQNTN SDAYTRSKYG   300
DETNRLYRQN YSLTWNGGWD NGVTTSNWVQ YEHTRNSRIP EGLAGGTEGK FNEKATQDFV   360
DNDLDDVMLH SEVNLPIDFL VNQTLTLGTE WNQQRMKDLS SNTQALTGTN TGGAIDGVSA   420
TDRSPYSKAE IFSLFAENNM ELTDSTIVTP GLRFDHHSIV GNNWSPALNI SQGLGDDFTL   480
KMGIARAYKA PSLYQTNPNY ILYSKGQGCY ASAGGCYLQG NDDLKAETSI NKEIGLEFKR   540
DGWLAGITWF RNDYRNKIEA GYVAVGQNAV GTDLYQWDNV PKAVVEGLEG SLNVPVSETV   600
MWTNNITYML KSENKTTGDR LSIIPEYTLN STLSWQARED LSMQTTFTWY GKQQPKKYNY   660
KGQPAVGPET KEISPYSIVG LSATWDVTKN VSLTGGVDNL FDKRLWRAGN AQTTGDLAGA   720
NYIAGAGAYT YNEPGRTWYM SVNTHF                                        746
```

<212> Type : PRT
<211> Length : 746
SequenceName : SEQ ID 57
SequenceDescription :
Sequence --------
<213> OrganismName : Escherichia coli O157:H7
<400> PreSequenceString :

```
MGGRFSLRYK KLSYRFVFLT LAGCSSVGNQ SLKNETQESV KTKIVKGKTT KQDVLASFGE    60
PDSRSLIDGE EQWSYTMYNS QSKATSFIPV VGLLAGGADS QTKSLTVSFK GEKVSTYIFN   120
AGTSNVKTGI F                                                        131
```

<212> Type : PRT
<211> Length : 131
SequenceName : SEQ ID 58
SequenceDescription :
Sequence
--------
<213> OrganismName : Escherichia coli O157:H7
<400> PreSequenceString :

```
MKKIACLSAL AAVLAFTAGT SVAATSTVTG GYAQSDAQGQ MNKMGGFNLK YRYEEDNSPL    60
GVIGSFTYTE KSRTASSGDY NKNQYYGITA GPAYRINDWA SIYGVVGVGY GKFQTTEYPT   120
YKHDTSDYGF SYGAGLQFNP MENVALDFSY EQSRIRSVDV GTWIAGVGYR F            171
```

<212> Type : PRT
<211> Length : 171
SequenceName : SEQ ID 59
SequenceDescription :
Sequence
--------
<213> OrganismName : Escherichia coli 0157:H7
<400> PreSequenceString :

```
MKSIATLVVC AISGIACVNL SAHAAEGEHT ISLGYAHFQF PGLKDFVKDA TAHNRETFSH    60
FVNRNYFSSL GEYTDGRVSG YEGKDKNPQG INIRYRYEIT DDFGVITSFT WTRSLTNSQT   120
FIDVQSADHT RKIKNPAASA RTDIRANYWS LLAGPSWRVN QYMSLYAMAG MGVAKVSADL   180
KIKDNINSSG GFSESNSTKK TSLAWAAGAQ FNLNESVTLD VAYEGSGSGD WRTSGVTAGI   240
GLKF                                                                244
```

<212> Type : PRT
<211> Length : 244
SequenceName : SEQ ID 60
SequenceDescription :
Sequence
--------

<213> OrganismName : Escherichia coli O157:H7
<400> PreSequenceString :

```
MRKLYAAILS AAICLTVSGA PAWASEQQAT LSAGYLHVST NAPGSDNLNG INVKYRYEFT     60
DTLGLVTSFS YAGDRNRQIT RYSDTRWHED SVRNRWFSVM AGPSVRVNEW FSAYAMAGVA    120
YSRVSTFSGD YLRVTDNKGK THDVLTGSDD GRHSNTSLAW GAGVQFNPTE SVAIDIAYEG    180
SGSGDWRTDG FIVGVGYKF                                                199
```

<212> Type : PRT
<211> Length : 199
SequenceName : SEQ ID 61
SequenceDescription :
Sequence
--------
<213> OrganismName : Escherichia coli O157:H7
<400> PreSequenceString :

```
MRKLYAAILS AAICLAVSGA PAWASEQQAT LSAGYLHART SAPGSDNLNG INVKYRYEFT     60
DTLGLVTSFS YAGDKNRQLT RYSDTRWHED SVRNRWFSVM AGPSVRVNEW FSAYAMAGVA    120
YSRVSTFSGD YLRVTDNKGK THDVLTGSDD GRHSNTSLAW GAGVQFNPTE SVAIDIAYEG    180
SGSGDWRTDG FIVGVGYKF                                                199
```

<212> Type : PRT
<211> Length : 199
SequenceName : SEQ ID 62
SequenceDescription :
Sequence --------
<213> OrganismName : Escherichia coli O157:H7
<400> PreSequenceString :

```
MRKLYAAILS AAICLAVSGA PAWASEQQAT LSAGYLHART SAPGSDNLNG INVKYRYEFT     60
DTLGLVTSFS YAGDKNRQLT RYSDTRWHED SVRNRWFSVM AGPSVRVNEW FSAYAMAGVA    120
YSRVSTFSGD YLRVTDNKGK THDVLTGSDD GRHSNTSLAW GAGVQFNPTE SVAIDIAYEG    180
SGSGDWRTDG FIVGVGYKF                                                199
```

<212> Type : PRT
<211> Length : 199
SequenceName : SEQ ID 63
SequenceDescription :
Sequence --------
<213> OrganismName : Escherichia coli O157:H7
<400> PreSequenceString :

```
MRKLYAAILS AAICLAVSGA PAWASEQQAT LSAGYLHART SAPGSDNLNG INVKYRYEFT     60
DTLGLVTSFS YAGDKNRQLT RYSDTRWHED SVRNRWFSVM AGPSVRVNEW FSAYAMAGVA    120
YSRVSTFSGD YLRVTDNKGK THDVLTGSDD GRHSNTSLAW GAGVQFNPTE SVAIDIAYEG    180
SGSGDWRTDG FIVGVGYKF                                                199
```

<212> Type : PRT
<211> Length : 199
SequenceName : SEQ ID 64
SequenceDescription :
Sequence
--------
<213> OrganismName : Escherichia coli O157:H7
<400> PreSequenceString :

```
MVMSQKTLFT KSALAVAVAL ISTQAWSAGF QLNEFSSSGL GRAYSGEGAI ADDAGNVSRN     60
PALITMFDRP TFSAGAVYID PDVNISGTSP SGRSLKADNI APTAWVPNMH FVAPINDQFG    120
WGASITSNYG LATEFNDTYA GGSVGGTTDL ETMNLNLSGA YRLNNAWSFG LGFNAVYARA    180
KIERFAGDLG QLVAGQIMQS PAGKTPQGQA LAATANGIDS NTKIAHLNGN QWGFGWNAGI    240
LYELDKNNRY ALTYRSEVKI DFKGNYSSDL NRVFNNYGLP IPTATGGATQ SGYLTLNLPE    300
MWEVSGYNRV DPQWAIHYSL AYTSWSQFQQ LKATSTSGDT LFQKHEGFKD AYRIALGTTY    360
YYDDNWTFRT GIAFDDSPVP AQNRSISIPD QDRFWLSAGT TYAFNKDASV DVGVSYMHGQ    420
SVKINEGPYQ FESEGKAWLF GTNFNYAF                                       448
```

<212> Type : PRT
<211> Length : 448
SequenceName : SEQ ID 65
SequenceDescription :
Sequence
--------
<213> OrganismName : Escherichia coli O157:H7
<400> PreSequenceString :

```
MAFSQAVSGL NAAATNLDVI GNNIANSATY GFKSGTASFA DMFAGSKVGL GVKVAGITQD     60
FTDGTTTNTG RGLDVAISQN GFFRLVDSNG SVFYSRNGQF KLDENRNLVN MQGLQLTGYP    120
ATGTPPTIQQ GANPTNISIP NTLMAAKTTT TASMQINLNS SDPLPSVNAF DASNADSYNK    180
KGSVTVFDSQ GNAHDMSVYF VKTGDNNWQV YTQDSSDPTG TAEPAMKLVF NANGVLTSNP    240
TENITTGAIN GAEPATFSLS FLNSMQQNTG ANNIVATTQN GYKPGDLVSY QINDDGTVVG    300
NYSNEQTQLL GQIVLANFAN NEGLASEGDN VWSATQSSGV ALLGTAGTGN FGTLTNGALE    360
ASNVDLSKEL VNMIVAQRNY QSNAQTIKTQ DQILNTLVNL R                        401
```

<212> Type : PRT
<211> Length : 401
SequenceName : SEQ ID 66
SequenceDescription :
Sequence
--------
<213> OrganismName : Escherichia coli O157:H7
<400> PreSequenceString :

```
MSKSTFLHIL ISSIILVALI QSSAWANCTN TQIGQTEDGR TALIEFGKIN MTDTYFAPAG     60
SLLATTVVPP TNYTSGGATG SSVLWECDAT DLPNIYFLVA TNGDDRVGGF YDAGGPDGLS    120
DVYATWFAFV GLKQTMAGVT LGRYWKKVPI TSYATQGTKI QIRLQDIPPL HAELYRISTL    180
PDTSATTSWC GNNNTDSSGV GFAKPSGTIY NCVQPNAYIQ LSGTSGILFG HDEPGEDSSV    240
HWDFWGADNG FGYGMRSANR LYNNATCVAR SATPLVLLPT IAEAQLNAGM ESTGNFNVRV    300
ECSNSVQSGI SDTQTALGIQ VSEGAYTAAQ KLGIINSNGG VSALVSDNYD AAEMAKGVGI    360
YISNSAHPDT AMTLVGQPGI AKLTPGGNAA GWYPVFEGAT LEGATHPGYS SYSYSFIARL    420
KKLPNQTVSA GKVRATAYIL VKMQ                                           444
```

<212> Type : PRT
<211> Length : 444
SequenceName : SEQ ID 67
SequenceDescription :
Sequence
--------
<213> OrganismName : Escherichia coli O157:H7
<400> PreSequenceString :

MENNRNFPAR QFHSLTFFAG LCIGITPVAQ ALAAEGQTNA DDTLVVEAST PSLYAPQQSA 60
DPKFSRPVAD TTRTMTVISE QVIKDQGATN LTDALKNVPG VGAFFAGENG NSTTGDAIYM 120
RGADTSNSIY IDGIRDIGSV SRDTFNTEQV EVIKGPSGTD YGRSAPTGSI NMISKQPRND 180
SGIDASASIG SAWFRRGTLD VNQVIGDTTA VRLNVMGEKT HDAGRDKVKN ERYGVAPSIA 240
FGLGTANRLY LNYLHVTQHN TPDGGIPTIG LPGYSAPSAG TATLNHSGKV DTHNFYGTDS 300
DYDDSTTDTA TMRFEHDIND NTTIRNTTRW SRVKQDYLMT AIMGGASNIT QPTSDVNSWT 360
WSRTANTKDV SNKILTNQTN LTSTFYTASI GHDVSTGVEF TRETQTNYGV NPVTLPAVNI 420
YHPDSSIHPG GLTRNGANAN GQTDTFAIYA FDTLQITRDF ELNGGIRLDN YHTEYDSATA 480
CGGSGRGAIT CPAGVAKGSP VTTVDTAKSG NLVNWKAGAL YHLTENGNVY INYAVSQQPP 540
GGNNFALAQS GSGNSANRTD FKPQKANTSE IGTKWQVLDK RLLLTAALFR TDIENEVEQN 600
DDGTYSQYGK KRVEGYEISV AGNITPAWQV IGGYTQQKAT IKNGKDVAQD GSSSLPYTPE 660
HAFTLWSQYQ ATDDISVGAG ARYIGSMHKG SDGAVGTPAF TEGYWVADAK LGYRVNRNLD 720
FQLNVYNLFD TDYVASINKS GYRYHPGEPR TFLLTANMHF 760

<212> Type : PRT
<211> Length : 760
SequenceName : SEQ ID 68
SequenceDescription :
Sequence
--------
<213> OrganismName : Escherichia coli O157:H7
<400> PreSequenceString :

MQMKKLLPIL IGLSLSGFSS LSQAENLMQV YQQARLSNPE LRKSAADRDA AFEKINEARS 60
PLLPQLGLGA DYTYSNGYRD ANGINSNATS ASLQLTQSIF DMSKWRALTL QEKAAGIQDV 120
TYQTDQQTLI LNTATAYFNV LNAIDVLSYT QAQKEAIYRQ LDQTTQRFNV GLVAITDVQN 180
ARAQYDTVLA NEVTARNNLD NAVEQLRQIT GNYYPELAAL NVENFKTDKP QPVNALLKEA 240
EKRNLSLLQA RLSQDLAREQ IRQAQDGHLP TLDLTASSGI SDTSYSGSKT RGAAGTQYDD 300
SNMGQNKVGL SFSLPIYQGG MVNSQVKQAQ YNFVGASEQL ESAHRSVVQT VRSSFNNINA 360
SISSINAYKQ AVVSAQSSLD AMEAGYSVGT RTIVDVLDAT TTLYNAKQEL ANARYNYLIN 420
QLNIKSALGT LNEQDLLALN NALSKPVSTN PENVAPQTPE QNAIADGYAP DSPAPVVQQT 480
SARTTTSNGH NPFRN 495

<212> Type : PRT
<211> Length : 495
SequenceName : SEQ ID 69
SequenceDescription :
Sequence
--------
<213> OrganismName : Escherichia coli O157:H7
<400> PreSequenceString :

MTKLKLLALG VLIATSAGVA HAEGKFSLGA GVGVVEHPYK DYDTDVYPVP VINYEGDNFW 60
FRGLGGGYYL WNDATDKLSI TAYWSPLYFK AKDSGDHQMR HLDDRKSTMM AGLSYAHFTQ 120
YGYLRTTLAG DTLDNSNGIV WDMAWLYRYT NGGLTVTPGI GVQWNSENQN EYYYGVSRKE 180
SARSGLRGYN SNDSWSPYLE LSASYNFLGD WSVYGTARYT RLSDEVTDSP IVDKSWTGLI 240
STGITYKF 248

<212> Type : PRT
<211> Length : 248
SequenceName : SEQ ID 70
SequenceDescription :
Sequence
--------
<213> OrganismName : Escherichia coli O157:H7
<400> PreSequenceString :

```
MKKTLLAAGA VLALSSSFTV NAAENDKPQY LSDWWHQSVN VVGSYHTRFG PQIRNDTYLE    60
YEAFAKKDWF DFYGYADAPV FFGGNSDAKG IWNHGSPLFM EIEPRFSIDK LTNTDLSFGP   120
FKEWYFANNY IYDMGRNKDG RQSTWYMGLG TDIDTGLPMS LSMNVYAKYQ WQNYGAANEN   180
EWDGYRFKIK YFVPITDLWG GQLSYIGFTN FDWGSDLGDD SGNAINGIKT RTNNSIASSH   240
ILALNYDHWH YSVVARYWHD GGQWNDDAEL NFGNGNFNVR STGWGGYLVV GYNF         294
```

<212> Type : PRT
<211> Length : 294
SequenceName : SEQ ID 71
SequenceDescription :
Sequence --------
<213> OrganismName : Escherichia coli O157:H7
<400> PreSequenceString :

```
MLSTQFNRDN QYQAITKPSL LAGCIALALL PSAAFAAPAT EETVIVEGSA TAPDDGENDY    60
SVTSTSAGTK MQMTQRDIPQ SVTIVSQQRM EDQQLQTLGE VMENTLGISK SQADSDRALY   120
YSRGFQIDNY MVDGIPTYFE SRWNLGDALS DMALFERVEV VRGATGLMTG TGNPSAAINM   180
VRKHATSREF KGDVSAEYGS WNKERYVADL QSPLTEDGKI RARIVGGYQN NDSWLDRYNS   240
EKTFFSGIVD ADLGDLTTLS AGYEYQRIDV NSPTWGGLPR WNTDGSSNSY DRARSTAPDW   300
AYNDKEINKV FMTLKQRFAD TWQATLNATH SEVEFDSKMM YVDAYVNKAD GMLVGPYSNY   360
GPGFDYVGGT GWNSGKRKVD ALDLFADGSY ELFGRQHNLM FGGSYSKQNN RYFSSWANIF   420
PDEIGSFYNF NGNFPQTDWS PQSLAQDDTT HMKSLYAATR VTLADPLHLI LGARYTNWRV   480
DTLTYSMEKN HTTPYAGLVF DINDNWSTYA SYTSIFQPQN DRDSSGKYLA PITGNNYELG   540
LKSDWMNSRL TTTLAIFRIE QDNVAQSTGT PIPGSNGETA YKAVDGTVSK GVEFELNGAI   600
TDNWQLTFGA TRYIAEDNEG NAVNPNLPRT TVKMFTSYRL PVMPELTVGG GVNWQNRVYT   660
DTVTPYGTFR AEQGSYALVD LFTRYQVTKN FSLQGNVNNL FDKTYDTNVE GSIVYGAPRN   720
FSITGTYQF                                                           729
```

<212> Type : PRT
<211> Length : 729
SequenceName : SEQ ID 72
SequenceDescription :
Sequence
--------
<213> OrganismName : Escherichia coli O157:H7
<400> PreSequenceString :

```
MARFQFKNRK NNGLIFFISF MVMGEAAIAA PLPQWANAPA VTPVAQLSLQ ESILRAFARN    60
PGVTQQAAQI GIGEAQIDEA KSAWYPHVGL TGNAGPSRQT DSSGRLDNNV SYGITLTQLV   120
YDFGKTNNDI NLQTAARDSY RFKLMATLTD VAEKTATAYM EVSRYQALCD AAQRNIHSLE   180
NVYNMAALRA NAGLNSSSDE LQAQTRIAGM RSTLEQYQAQ MASAKAQLAV LTGVQPEAIA   240
APPAELAEQP VSLKNIDYQS IPLVLAAENL RQSAQYGVEK TKAQYWPTLS IQGGKTRYQT   300
SDRSYWDDQL QLNVNAPLYQ GGAVSAQVQQ AEGQQKISAS QVEQAKLDVL QRASVAYANW   360
TGARGREEAG LAQSESAHKT RDVYQNEYKL GKRSLNDLLT VEQDVFQAQS AEINANYDGW   420
VAAVNYAAAV NNLIPLAGIK QGLYNDLPDL K                                  451
```

<212> Type : PRT
<211> Length : 451
SequenceName : SEQ ID 73
SequenceDescription :
Sequence
--------
<213> OrganismName : Escherichia coli O157:H7
<400> PreSequenceString :

MAKFTPSFSG IKGRALFSLL FAAPMIHATD TATTKDGETI TVTADANTAT EATDGYQPLS 60
TSTATLTDMP MLDIPQVVNT VSDQVLENQN ATTLDEALYN VSNVVQTNTL GGTQDAFVRR 120
GFGANRDGSI MTNGLRTVLP RSFNAATERV EVLKGPASTL YGILDPGGLI NVVTKRPEKT 180
FHGSVSATSS SFGGGTGQLD ITGPIEGTQL AYRLTGEVQD EDYWRNFGKE RSTFIAPSLT 240
WFGDNATVTM LYSHRDYKTP FDRGTIFDLT TKQPVNVDRK IRFDEPFNIT DGQSDLAQLN 300
AEYHLNSQWT ARFDYSYSQD KYSDNQARVT AYDATTGTLT RRVDATQGST QRMHSTRADL 360
QGNVDIAGFY NEILGGVSYE YYDLLRTDMI RCKNAKDFNI YNPVYGNTSK CTTVSASDSD 420
QTIKQESYSA YAQDALYLTD NWIAVAGIRY QYYTQYAGKG RPFNVNTDSR DEQWTPKLGL 480
VYKLTPSVSL FANYSQTFMP QSSIASYIGD LPPESSNAYE VGAKFELFDG ITADIALFDI 540
HKRNVLYTES IGDETIAKTA GRVRSRGVEV DLAGALTENI NIIASYGYTD AKVLEDPDYA 600
GKPLPNVPRH TGSLFLTYDI HNMPGNNTLT FGGGGHCVSR RSATNGADYY LPGYFVADAF 660
AAYKMKLQYP VTLQLNVKNL FDKTYYTSSI ATNNLGNQIG DPREVQFTVK MEF 713

<212> Type : PRT
<211> Length : 713
SequenceName : SEQ ID 74
SequenceDescription :
Sequence
--------
<213> OrganismName : Escherichia coli O157:H7
<400> PreSequenceString :

MRTLQGWLLP VFMLPMAVYA QEATVKEVHD APAVRGSIIA NMLQEHDNPF TLYPYDTNYL 60
IYTQTSDLNK EAIASYDWAE NARKDEVKFQ LSLAFPLWRG ILGPNSVLGA SYTQKSWWQL 120
SNSEESSPFR ETNYEPQLFL GFATDYRFAG WTLRDVEMGY NHDSNGRSDP TSRSWNRLYT 180
RLMAENGNWL VEVKPWYVVG NTDDNPDITK YMGYYQLKIG YHLGDAVLSA KGQYNWNTGY 240
GGAELGLSYP ITKHVRLYTQ VYSGYGESLI DYNFNQTRVG VGVMLNDLF 289

<212> Type : PRT
<211> Length : 289
SequenceName : SEQ ID 75
SequenceDescription :
Sequence
--------
<213> OrganismName : Escherichia coli O157:H7
<400> PreSequenceString :

MAVQKNVIKG ILAGTFALML SGCVTVPDAI KGSSPTPQQD LVRVMSAPQL YVGQEARFGG 60
KVVAVQNQQG KTRLEIATVP LDSGARPTLG EPSRGRIYAD VNGFLDPVDF RGQLVTVVGP 120
ITGAVDGKIG NTPYKFMVMQ ATGYKRWHLT QQVIMPPQPI DPWFYGGRGW PYGHGGWGWY 180
NPGPARVQTV VTE 193

<212> Type : PRT
<211> Length : 193
SequenceName : SEQ ID 76
SequenceDescription :
Sequence --------
<213> OrganismName : Escherichia coli O157:H7
<400> PreSequenceString :

MRKQWLGICI AAGMLAACTS DDGQQQTVSV PQPAVCNGPI VEISGADPRF EPLNATANQD 60
YQRDGKSYKI VQDPSRFIQA GLAAIYDAEP GSNLTASGEA FDPTQLTAAH PTLPIPSYAR 120
ITNLANGRMI VVRINDRGPY GNDRVISLSR AAADRLNTSN NTKVRIDPII VAQDGSLSGP 180
GMACTTVAKQ TYALPAPPDL SGGAGTSSVS GPQGDILPVS NSTLKSEDPT GAPVTSSGFL 240
GAPTTLAPGV LEGSEPTPAP QPVVTAPSTT PATSPAMVTP QAASQSASGN FMVQVGAVSD 300
QARAQQYQQQ LGQKFGVPGR VTQNGAVWRI QLGPFANKAE ASTLQQRLQT EAQLQSFITT 360
AQ 362

<212> Type : PRT
<211> Length : 362
SequenceName : SEQ ID 77

SequenceDescription :
Sequence
--------
<213> OrganismName : Escherichia coli O157:H7
<400> PreSequenceString :

```
MIKRVLVVSM VGLSLVGCVN NDTLSGDVYT ASEAKQVQNV SYGTIVNVRP VQIQGGDDSN     60
VIGAIGGAVL GGFLGNTVGG GTGRSLATAA GAVAGGVAGQ GVQSAMNKTQ GVELEIRKDD    120
GNTIMVVQKQ GNTRFSPGQR VVLASNGSQV TVSPR                               155
```

<212> Type : PRT
<211> Length : 155
SequenceName : SEQ ID 78
SequenceDescription :
Sequence
--------
<213> OrganismName : Escherichia coli O157:H7
<400> PreSequenceString :

```
MSKATEQNDK LKRAIIISAV LHVILFAALI WSSFDENIEA SAGGGGGSSI DAVMVDSGAV     60
VEQYKRMQSQ ESSAKRSDEQ RKMKEQQAAE ELREKQAAEQ ERLKQLEKER LAAQEQKKQA    120
EEAAKQAELK QKQAEEAAAK AAADAKAKAE ADDKAAEEAA KKAAADAKKK AEAEAAKAAA    180
EAQKKAEAAA AALKKKAEAA EAAAAEARKK AAAEKAAADK KAAEKAAAEK AAADKKAAAE    240
KAAADKKAAA AKAAAEKAAA AKAAAEADDI FGELSSGKNA PKTGGGAKGN NASPAGSGNT    300
KNNGASGADI NNYAGQIKSA IESKFYDASS YAGKTCTLRI KLAPDGMLLD IKPEGGDPAL    360
CQAALAAAKL AKIPKPPSQA VYEVFKNAPL DFKP                                394
```

<212> Type : PRT
<211> Length : 394
SequenceName : SEQ ID 79
SequenceDescription :
Sequence
--------
<213> OrganismName : Escherichia coli O157:H7
<400> PreSequenceString :

```
MMKFKKCLLP VAMLASFTLA GCQSNADDHA ADVYQTDQLN TKQETKTVNI ISILPAKVAV     60
DNSQNKRNAQ AFGALIGAVA GGVIGHNVGS GSNSGTTAGA VGGGAVGAAA GSMVNDKTLV    120
EGVSLTYKEG TKVYTSTQVG KECQFTTGLA VVITTTYNET RIQPNTKCPE KS            172
```

<212> Type : PRT
<211> Length : 172
SequenceName : SEQ ID 80
SequenceDescription :
Sequence
--------
<213> OrganismName : Escherichia coli O157:H7
<400> PreSequenceString :

```
MLLSIITVAF RNLEGIVKTH ASLAHLAQAE DISFEWIVVD GGSNDGTREY LENLNGIYNL     60
RFVSEPDNGI YDAMNKGIAM AQGKFALFLN SGDIFHQDAA YFVRKLKMQK DNVMITGDAL    120
LDFGDGHKIK RSAKPGWYIY HSLPASHQAI FFPVSGLKKW RYDLEYKVSS DYALAAKMYK    180

AGYAFKKLNG LVSEFSMGGV STTNNMELCA DAKKVQRQIL HVPGFWAELS WHLRQRTTSK    240
TKALYNKS                                                             248
```

<212> Type : PRT
<211> Length : 248
SequenceName : SEQ ID 81

SequenceDescription :
Sequence --------
<213> OrganismName : Haemophilus influenzae Rd
<400> PreSequenceString :

```
MKLTTLQTLK KGFTLIELMI VIAIIAILAT IAIPSYQNYT KKAAVSELLQ ASAPYKADVE     60
LCVYSTNETT SCTGGKNGIA ADIKTAKGYV ASVITQSGGI TVKGNGTLAN MEYILQAKGN    120
AAAGVTWTTT CKGTDASLFP ANFCGSVTK                                      149
```

<212> Type : PRT
<211> Length : 149
SequenceName : SEQ ID 82
SequenceDescription :
Sequence
--------
<213> OrganismName : Haemophilus influenzae Rd
<400> PreSequenceString :

```
MLNKKFKLNF IALTVAYALT PYTEAALVRD DVDYQIFRDF AENKGRFSVG ATNVEVRDKN     60
NHSLGNVLPN GIPMIDFSVV DVDKRIATLI NPQYVVGVKH VSNGVSELHF GNLNGNMNNG    120
NAKSHRDVSS EENRYFSVEK NEYPTKLNGK AVTTEDQTQK RREDYYMPRL DKFVTEVAPI    180
EASTASSDAG TYNDQNKYPA FVRLGSGSQF IYKKGDNYSL ILNNHEVGGN NLKLVGDAYT    240
YGIAGTPYKV NHENNGLIGF GNSKEEHSDP KGILSQDPLT NYAVLGDSGS PLFVYDREKG    300
KWLFLGSYDF WAGYNKKSWQ EWNIYKPEFA KTVLDKDTAG SLTGSNTQYN WNPTGKTSVI    360
SNGSESLNVD LFDSSQDTDS KKNNHGKSVT LRGSGTLTLN NNIDQGAGGL FFEGDYEVKG    420
TSDSTTWKGA GVSVADGKTV TWKVHNPKSD RLAKIGKGTL IVEGKGENKG SLKVGDGTVI    480
LKQQADANNK VKAFSQVGIV SGRSTVVLND DKQVDPNSIY FGFRGGRLDA NGNNLTFEHI    540
RNIDDGARLV NHNTSKTSTV TITGESLITD PNTITPYNID APDEDNPYAF RRIKDGGQLY    600
LNLENYTYYA LRKGASTRSE LPKNSGESNE NWLYMGKTSD EAKRNVMNHI NNERMNGFNG    660
YFGEEEGKNN GNLNVTFKGK SEQNRFLLTG GTNLNGDLKV EKGTLFLSGR PTPHARDIAG    720
ISSTKKDQHF AENNEVVVED DWINRNFKAT NINVTNNATL YSGRNVANIT SNITASDNAK    780
VHIGYKAGDT VCVRSDYTGY VTCTTDKLSD KALNSFNATN VSGNVNLSGN ANFVLGKANL    840
FGTISGTGNS QVRLTENSHW HLTGDSNVNQ LNLDKGHIHL NAQNDANKVT TYNTLTVNSL    900
SGNGSFYYLT DLSNKQGDKV VVTKSATGNF TLQVADKTGE PTKNELTLFD ASNATRNNLN    960
VSLVGNTVDL GAWKYKLRNV NGRYDLYNPE VEKRNQTVDT TNITTPNNIQ ADVPSVPSNN   1020
EEIARVETPV PPPAPATPSE TTETVAENSK QESKTVEKNE QDATETTAQN GEVAEEAKPS   1080
VKANTQTNEV AQSGSETEET QTTEIKETAK VEKEEKAKVE KDEIQEAPQM ASETSPKQAK   1140
PAPKEVSTDT KVEETQVQAQ PQTQSTTVAA AEATSPNSKP AEETQPSEKT NAEPVTPVVS   1200
KNQTENTTDQ PTEREKTAKV ETEKTQEPPQ VASQASPKQE QSETVQPQAV LESENVPTVN   1260
NAEEVQAQLQ TQTSATVSTK QPAPENSINT GSATAITETA EKSDKPQTET AASTEDASQH   1320
KANTVADNSV ANNSESSDPK SRRRRSISQP QETSAEETTA ASTDETTIAD NSKRSKPNRR   1380
SRRSVRSEPT VTNGSDRSTV ALRDLTSTNT NAVISDAMAK AQFVALNVGK AVSQHISQLE   1440
MNNEGQYNVW VSNTSMNENY SSSQYRRFSS KSTQTQLGWD QTISNNVQLG GVFTYVRNSN   1500
NFDKASSKNT LAQVNFYSKY YADNHWYLGI DLGYGKFQSN LKTNHNAKFA RHTAQFGLTA   1560
GKAFNLGNFG ITPIVGVRYS YLSNANFALA KDRIKVNPIS VKTAFAQVDL SYTYHLGEFS   1620
VTPILSARYD TNQGSGKINV NQYDFAYNVE NQQQYNAGLK LKYHNVKLSL IGGLTKAKQA   1680
EKQKTAELKL SFSF                                                     1694
```

<212> Type : PRT
<211> Length : 1694
SequenceName : SEQ ID 83
SequenceDescription :
Sequence
--------
<213> OrganismName : Haemophilus influenzae Rd
<400> PreSequenceString :

```
MALVNKIKTL SSVGILAATL FLAGCQAQSN ILAFTPPAPS ASMNVNRTAV VSVTTKDSRA     60
IQEIASYTKH GELIKLNASP SVTQLFQQVM QQNLISKGFR VGQLNGSNAW VTVDVREFGT    120
QVEQGNLRYK LNTKIQATVY VQGAKGSYNK SFNVTHSQEG VFNAGNDEIH KVLSQTFNDI    180
VNNIYQDQEV AAAINQYSN                                                199
```

<212> Type : PRT

<211> Length : 199
SequenceName : SEQ ID 84
SequenceDescription :
Sequence --------
<213> OrganismName : Haemophilus influenzae Rd
<400> PreSequenceString :

```
MLCWIGYKNG ILPQQNSTLY PWLNPSKCGV IFDGFQLVGD DFNSDQTAEN TSPAWQVLYT    60
THLQSCSPIH SGENFAPIPL YKQLKNQPHL SQDLIKWQEN WQACDQLQMN GAVLEQQSLA   120
EISDHQSTLS KHGRYLAQEI EKETGIPTYY YLYRVGGQSL ESEKSRCCPS CGANWALKDA   180
IFDTFHFKCD TCRLVSNLSW NFL                                          203
```

<212> Type : PRT
<211> Length : 203
SequenceName : SEQ ID 85
SequenceDescription :
Sequence
--------
<213> OrganismName : Haemophilus influenzae Rd
<400> PreSequenceString :

```
MGAFAFASVT NANIYAEGDI GLSQTKANGS NNTRVGPRVS VGYKVGNTRV AGDYTHHGKV    60
DGTKIQGLGA SVLYDFDTNS KVQPYVGARV ATNQFKYTNR AEQKFKSSSD IKLGYGVVAG   120
AKYKLDGNWY ANGGVEYNRL GNFDSTKVNN YGAKVGVGYG F                       161
```

<212> Type : PRT
<211> Length : 161
SequenceName : SEQ ID 86
SequenceDescription :
Sequence
--------
<213> OrganismName : Haemophilus influenzae Rd
<400> PreSequenceString :

```
MKKLLIASLL FGTTTTVFAA PFVAKDIRVD GVQGDLEQQI RASLPVRAGQ RVTDNDVANI    60
VRSLFVSGRF DDVKAHQEGD VLVVSVVAKS IISDVKIKGN SIIPTEALKQ NLDANGFKVG   120
DVLIREKLNE FAKSVKEHYA SVGRYNATVE PIVNTLPNNR AEILIQINED DKAKLASLTF   180
KGNESVSSST LQEQMELQPD SWWKLWGNKF EGAQFEKDLQ SIRDYYLNNG YAKAQITKTD   240
VQLNDEKTKV NVTIDVNEGL QYDLRSARII GNLGGMSAEL EPLLSALHLN DTFRRSDIAD   300
VENAIKAKLG ERGYGSATVN SVPDFDDANK TLAITLVVDA GRRLTVRQLR FEGNTVSADS   360
TLRQEMRQQE GTWYNSQLVE LGKIRLDRTG FFETVENRID PINGSNDEVD VVYKVKERNT   420
GSINFGIGYG TESGISYQAS VKQDNFLGTG AAVSIAGTKN DYGTSVNLGY TEPYFTKDGV   480
SLGGNVFFEN YDNSKSDTSS NYKRTTYGSN VTLGFPVNEN NSYYVGLGHT YNKISNFALE   540
YNRNLYIQSM KFKGNGIKTN DFDFSFGWNY NSLNRGYFPT KGVKASLGGR VTIPGSDNKY   600
YKLSADVQGF YPLDRDHLWV VSAKASAGYA NGFGNKRLPF YQTYTAGGIG SLRGFAYGSI   660
GPNAIYAEHG NGNGTFKKIS SDVIGGNAIT TASAELIVPT PFVSDKSQNT VRTSLFVDAA   720
SVWNTKWKSD KSGLDNNVLK SLPDYGKSSR IRASTGVGFQ WQSPIGPLVF SYAKPIKKYE   780
NDDVEQFQFS IGGSF                                                   795
```

<212> Type : PRT
<211> Length : 795
SequenceName : SEQ ID 87
SequenceDescription :
Sequence
--------
<213> OrganismName : Haemophilus influenzae Rd
<400> PreSequenceString :

MLKKTSLIFT ALLMTGCVQN ANVTTPQAQK MQVEKVDKAL QKGEADRYLC QDDRVVRVVH 60
ATHKKYKKNL HYVTVTFQGV SEKLTLMISE RGKNYANIRW MWQERDDFST LKTNLGEILA 120
TQCVSQTSER LSGQ 134

<212> Type : PRT
<211> Length : 134
SequenceName : SEQ ID 88
SequenceDescription :
Sequence
--------
<213> OrganismName : Haemophilus influenzae Rd
<400> PreSequenceString :

MRIIIIFFMG LNMTNFRLER ACLFRYAWAN GRCCLCSSTN QPTNQPTNQP TNQPTNQPTN 60
QPTNQNSNVS EQLEQINVSG STENSDTKTP PKIAETVKTA KTLEREQANN IKDIVKYETG 120
VTVVEAGRFG QSGFAIRGVD ENRVAINIDG LRQAETLSSQ GFKELFEGYG NFNNTRNGAE 180
IETLKEVNIT KGADSIKNGS GSLGGSVIYK TKDARDYLIN KDYYVSYKKG YATENNQSFD 240
TLTLAGRYKK FDVLVVTTSR NGHELENYGY KNYNDKIQGK KREKADPYKI EQDSTLLKLS 300
FNPTENHRFT FAADLYEHRS RGQDLSYTLK YQRSGNETPE VDSRHTNDKT KRRNISFSYE 360
NFSQTPFWDT LKLTYSDQRI KTRARTDEYC DAGVRHCEGT DNPTGLKVTN GKITRRDGSD 420
LQFEEKNNTA KSSDKTYDFK KFIDTDKRVI DDKLVLNNPS DTWYDCSIFN CENNAKIKVF 480
KGNNYYGYDG KWKEVDLEIK ELNGKKFAKI KDNDRKIKSI LPSSPGYLER LWQERDLDTN 540
TQQLNLDLTK DFKIWHIEHN LQYGGSYNTA MKRMVNRAGN DASDVQWWAT PTLGEDSWTG 600
KPHTCATTYE WNANLCPRVD PEFSYLLPIK TTGKSVYLFD NFVITDYLSF DLGYRYDNIH 660
YQPKYKHGIT PKLPDDIVKG LFIPLPNNSN SDPNKVKENV QQNIDYIAKQ NKKYKAHSYS 720
FVSTIDPTSF LRLQLKYSKG FRTPTSDEMY FTFKHPDFTI LPNTDLKPEI AKTKEIAFTL 780
HNDDWGFIST SLFKTNYKNF IDLIFKKQET FKVGGSGRGE TLPFSLYQNI NRDNASLKGI 840
EINSKVFLGK MAKFMDGFNL SYKYTYQKGR MNGNIPMNAI QPRTMVYGLG YDHPNHKFGF 900
DFYTTHVASK NPEDTYNMFY KEENKKDSTI KWRSKSYTIL DLIGYVQPIK NLTIRAGVYN 960
LTNRKYITWD SARSIRSFGT SNVIDQSTGL GINRFYAPGR NYKMSVQFEF 1010

<212> Type : PRT
<211> Length : 1010
SequenceName : SEQ ID 89
SequenceDescription :
Sequence --------
<213> OrganismName : Helicobacter pylori J99
<400> PreSequenceString :

MTYRNGKIDL KERFSKNRSF KGIKKKIAKK YTIKNSLSII YSLKTHSNSS LSINKKIFLG 60
LGFVSALSAQ SEDYNSSVYW LNSVNENNNN KSYYISPLRT WAGGNRSFTQ NYNNSQLYIG 120
TKNASATPNH SSVWFGEKGY IGFITGVFKA RDIFITGAVG SGNELKTGGG AILVFESSNE 180
LTTNGAYFQN NRAGTQTSWI NLISNNSVNL TNTDFGNQTP NGGFNVMGRK ITYNGGSVNG 240
GNFGFDNVDS NGATTISGVT FNNNGALTYK GGNGIGGSIT FTNSNINHYK LNLNANSVTF 300
NNSTLGSMPN GNANTIGNAY ILNANNITFN NLTFNGGWFV FNRSDAHVNF QGTTTINNPT 360
SPFVNMTGKV TINPNAIFNI QNYTPTIGNA YTLFSMKNGN IAYDDVNNLW NIIRLKNTQA 420
TKDNSKNATS NNNTHTYYVT YNLGGTLYHF RQIFSPDSIV LQSVYYGANN LYYTNSVNIH 480
DNVFNLKNIN DDRADTIFYL NGLNTWNYTQ ARFAQTYGGK NSALVFNATT PWANGAIPKS 540
NSTVRFGGYE GVNWGKTGYI TGTFTADRVY ITGNMMSGNG AQTGGGATLN FVGATEINIA 600
GATFKNLKTT SQNSYMTFMA LGNGSGSGKI NVSQSDFYDW TDGGYDFTGN GVFDSVNFNK 660
AYYKFQGAEN SYNFKNTNFL AGNFKFQGKT TIEKSVLNDA SYAFDGVNNA FNEDKFNGGS 720
FNFNHAEQTN AFNNNSFSGG SFSFNAKQVD FNGNSFNGGV FNFNNTPKAS FTNDTFNVNN 780
QFKINGAQTD FTFSKGVVFN MQGLLSSLSV GTTYQLLNAK SVGYKDNNNA LYQMLRWTSG 840
ENPSGKLVDE NKTAPNSAKI YNVQFTDNGL TYYIKENFNN GITLTRLCTL GYTHCVNIDN 900
DAFNLKNVNN NASNTVFYLN GMTTWKTAGT GVFTQDYSGT NSVLVFNQTT PFLAGANPTS 960
NSVVGFGKTS GAEWGLVGYI QGVFKANQID ITGTIRSGNG AKTGGGATLV FNAQERLNIA 1020
NANLNNDKAG LQNSWMNFIV NNGNLNVTNA NFSNQTPHGG FNLKANNITW DKGSVSGGGN 1080
FGVDNANANG NAVIKNVNFS DNGTLIYKGG ENSAGNSLTL ENNTFNSYNI NAKAQNLIFN 1140
NNSFNSGSYS FNDTKNVTFK GTNTLINSDP FSRLKGSVSI DNNSIFNIER DLTDKTTYTL 1200
LSGDNIKYNN QALADNVFSK NLWDLIHYDG EQGTLLRTDN NTYFVQFTQS NGQKFVFEET 1260
FNPGSITYKY FTIHSSPFHT EADSKDIWNQ VRKQFDFIPG KTPVCVGVCY IAPYKNQDLI 1320
GSSAFAWSLN FGATVVGTLL LGSAQEKANN NGGSIWFGKN NLLYLHGNFN ATNIFLTNNF 1380
NVGNPNAGGG ATINFNADET LSADGLNYTN FQTVAMGLQT SASQHSWANF NSKLSMEIKN 1440
SNFRDFTWGG FRFNSGRITF ENTTFSGWTN INGATESGSS YVNMVANTDL IFTDSILGGG 1500
IRYDLKANNI IFNNTQMVVD VSKNVNQSSL NGNVTFNHSR LSVKPNAAIN IGGDQTQTTL 1560
ENASSLSFYN DSVANFNGTT AFNGVSYLNL NPNAQVSFNQ ANFNNANVTF YGIPLFGKTP 1620
NFGNSVRLIN FKGDAKFNQA TLNLRAKNIH LNFQGASTFE NNSTMNLAES SQASFNALSV 1680
EGETNFNLNG SSLLSFNGNS VFNAPVNFYA NNSQISFTHS ATFNADASFD LGNNSTLNFQ 1740
SVLLNSALNL LGNGGNNLAI NAKGNFSFGS QGILNLSYMN LFGGDKKASV YDVLQAQNID 1800
GLRGNNGYEK IRFYGIQIEK ADYSFNNGVH SWSFTNPLNT TETITETLHN NRLKVQISQN 1860
GASNNAMFNL APSLYDYQQN PYDESENSYN HTSDKAGTYY LSSSIKGFGK NNEIPGTYNA 1920
QNQPLQALHI YNQAISKQDL NMIASLGKEF LPKVAKLIAS GALDNLNLNS PDSFETIFSI 1980
LKEYGITLNQ ANWKSLLKII NNFSNTANYH FSQGSLVVGA IKEGQTNTNS VVWFGGDGYK 2040
NPCAVGDNTC QMFRQTNLGQ LLNSSVPYLG YINANFKAKN IYITGTIGSG NAWGSGGSAN 2100
VSFESATNLV LNQANIDAQG TDKIFSYLGK EGIDKLFGEK GLGNVLSNIV YEESLNDNAI 2160
PKDLANMIPK DLGSKTLSSL LSPTEVNNLL GVSAFKNAIM EILNSKTVGD VFGENGLLNA 2220
LDPVKRKEID QMLLEQIQAH SSGFEKFIVK TLGIENVENF INNWYGKQSL SSFANNFVPG 2280
GLNQALDKIG SSSDAKDLQS FLDKTTFGDI LNQMINQAPL INKLISWLGP QDLSVLVNIA 2340
LNSITNPSKE LLGAISGMGQ KVLNDLLGEG VVNKIMSNQV LGQMINKIIA DKGFGGVYHQ 2400
GLGSILPKSL QDELKKLGMG SLLKPKGLHN LWQKGNFNFV AKNHVFVNNS LFSNATGGEL 2460

NFVAGKSIIF NGKNTINFTQ YQGRLSFVSK DFSNISLDTL NATNGLTLNA SKNDISVQKG 2520
QICVNVLDCM TAKGKTTQTN SSSSATAPTN ETLEVSANNF AFLGTIKANG LVDFSKVLQN 2580
TTIGTLDLGP NATFKANNLI VNNAFNNNSN YRANISGNFN VAKGATFSTN ENGLNVGGNF 2640
NSEGPLIFNL NNPTHQTIIN VTGTSTIMSY NNQALINFNT QLKQGAYTLI NANRMVYGYD 2700
NQTILGGSLS DYLKLYTLID FNGKRMQLNG DSLSYDNQPV SIKDGGLVVS FKDNQGQMVY 2760
SSILYDKIQV TVSDKPMSIQ APSLEYYVKR IQGSAGLNAI KSAGNNSIMW LSELFAAKGG 2820
NPLFAPYYLQ DNPTEHIVTL MKDITSALGM LSNSNLKNNS TDVLQLNTYT QQMSRLAKLS 2880
NFASFDSTDF SERLSSLKNQ RFADAVPNAM DVILKYSQRD KLKNNLWATG VGGVSFVENG 2940
TGTLYGVNVG YDRFVRGVIV GGYAAYGYSG FYERITSSKS DNVDVGLYAR AFIKKSELTF 3000
SVNETWGANK TQISSNDALL SMINQSYKYS TWTTNAKVNY GYDFMFKNKS IILKPQIGLR 3060
YYYIGMSGLE GVMNNVLYNQ FKANADPSKK SVLTIDFALE NRHYFNTNSY FYAIGGVGRD 3120
LLVNSMGDKL VRFIGNNTLS YRKGDLYNTF ANITTGGEVR LFKSFYANAG VGARFGLDYK 3180
MIDIIGNIGM RLAF 3194

<212> Type : PRT
<211> Length : 3194
SequenceName : SEQ ID 90
SequenceDescription :
Sequence --------
<213> OrganismName : Helicobacter pylori J99
<400> PreSequenceString :

MKQFKKKPKK IKRSHQNQKT ILKRPLWLMP LLIGGFASGV YADGTDILGL SWGEKSQKVC 60
VHRPWYAIWS CDKWEEKTQQ FTGNQLITKT WAGGNAANYY HSQNNQDITA NLKNDNGTYF 120
LSGLYNYTGG EYNGGNLDIE LGSNATFNLG ASSGNSFTSW YPNGHTDVTF SAGTINVNNS 180
VEVGNRVGSG AGTHTGTATL NLNANKVTIN SNISAYKTSQ VNVGNANSVI TINSVSLNGD 240
TCSSLARVGV GANCSTSGPS YSFKGTTNAT NTTFSNSSGS FTFEENATFS GAKLNGGAFT 300
FNKKFNATNN TAFNSGSFTF KGTSSFNGAN FSNASYTFNN QATFQNSSFN GGTFTFNDQT 360
NQSTQHPQIQ NSSFSGSATT LKGFATFEQA FNNSNHQLTI QNASFNNATF NNTGKITIEK 420
DASFNNTSFN TPVDTNNMTI SGGVTLSGKN DLKNGATLDF GSSKITLTQG TTFNLTSLGS 480
EKSVTILNSR GGITYNHLLN HAINSLTNAL KTNESSSKPQ SFAQGLWDMI TYNGVTGQLL 540
NENAATSKPT DSSPSKSSTN STQVYQVGYK IGDTIYKLQE TFSHNSIIIQ ALESGTYTPP 600
PVINGSKFDL SASNYINADM PWYNHKYYIP KSQNFTESGT YYLPSVQIWG SYTNSFKQTF 660
SASNSNLVIG YNATWTDHNV SSSDTVAFGD TSGSALNGHC GPWPYYQCTG TTNGTYSAYH 720
VYITANLRSG NRIGTGGAAN LIFNGVDSIN IANATITQHN AGAYSSSMTF STQNMDNSQN 780
LNGLNSNGKL LVYGTTFTNQ AKDGKFIFNA GQATFENTNF NGGSYQFSGD SLNFSNNNQF 840
NSGSFEIGAK NTIFNNANFN NSTSFNFNNS SATTSFVGDF TNANSNLQIA GNAVFGNSTN 900
GSQNTANFNN TGSVNIAGNA TFDNVVFNSP TNTSVKGKVT LNNITLKNLN APLSFGDGTI 960
VFSAHSVINI GEAITNGNPI TLVSSSKAIE YNDAFSKNLW QLINYQGHGA SSEKLVSSAG 1020
NGVYDVVYSF NNQTYNFQEV FSPNSISIRR LGVGMVFDYV DMEKSDRLYY QNALGFMTYM 1080
PNSYNNNLGN LNNTIYYYDN SIDFYASGKT LFTKAEFSQT FTGQNSAIVF GAKNIWTSVS 1140
DAPQSNVIIR FGDNKGAGSN DASGHCWNLQ CIGFITGHYE AQKIYITGSI ESGNRISSGG 1200
GASLNFNGLQ GILLTNATLY NRAAGTQSSS MNFVSNSANI QAQNSYFIDD TAQNKGNPNF 1260
SFNALNLDFS NSSFRGYVGQ TQSVFKFNAV NAISFTNSSN LSSGLYQMQA KSVLFDNSNL 1320
SVSVGTSSIK ANAINLSQNA SINASNHSTL ELQGDLNLND TSSLNLNQSA INVSNNATIN 1380
DYASLIASNG SHLNFNGAVN FNSANITTSL SSSSIVFKGA VSLRGQFNLS NNSSLDFQGS 1440
SAITSNTAFN FYDNAFSQSP ITFHQALDIK VPLSLGGNLL NPNNSSVLNL KNSQLVFSDQ 1500
GSLNIANIDL LSDLNGNKNR VYNIIQADMN GNWYERINFF GMRINDGIYD AKNQTYSFTN 1560
PLNNALKITE SFKNNQLSVT LSQIPGIKNT LYNIGSEIFN YQKVYNNANG VYSYSDDAQG 1620
VFYLTSSVKG YYNPNQSYQA SGSNNTTKNN NLTSESSVIS QTYNAQGNPI SALHVYNKGY 1680
NFSNIKALGQ MALKLYPEIK KILGNDFSLS SLSNLKGDAL NQLTKLITPS DWKNINELID 1740
NANNSVVQNF NNGTLIIGAT KIGQTDTNSA VVFGGLGYQK PCDYTDIVCQ KFRGTYLGQL 1800
LESISADLGY IDTTFNAKEI YLTGTLGSGN AWGTGGSASV TFNSQTSLIL NQANIVSSQT 1860
DGIFSMLGQE GINKVFNQAG LANILGEVAM QSINKAGGLG NLIVNTLGSD SVIGGYLTPE 1920
QKNQTLSQLL GQNNFDNLMN DSGLNTAIKD LIRQKLGFWT GLVGGLAGLG GIDLQNPEKL 1980
IGSMSINDLL SKKGLFNQIT GFISANDIGQ VISVMLQDIV KPSDALKNDV AALGKQMIGE 2040
FLGQDTLNSL ESLLQNQQIK SVLDKVLAAK GLGSIYEQGL GDLIPNLGKK GIFAPYGLSQ 2100
VWQKGDFSFN AQGNVFVQNS TFSNANGGTL SFNAGNSLIF AGNNHIAFTN HSGTLNLLSN 2160
QVSNINVTML NASNGLKINA TNNNVSVSQG NLFINASCVQ QSDPTTASAT NPCTTAQNNA 2220
SSSNASNNAP IALNNNDESL VVTANGFNFS GNIYANGVVD FSKIKGSANV KNLYLYNNAQ 2280
FQANNLTISN QAVLEKNASF VTNNLNIQGA FNNNATQKIE VLQNLVIASN ASLSTGIYGL 2340
EVGGALNNLG AIHFNLENSQ TPVNPLIQVG GIINLNTTQT PFMNVSVANG GTYTLLKSSR 2400
YIDYNINPNS LQSYLKLYTL ININGNHIEE KNGVLTYLGQ RVLLQDKGLL LSVALPNSNN 2460
ASQNNILSLS VLHNQIKMSY GNKVMDFTPP TLQDYIVGIQ GQSALNQIEA VGGNNAIKWL 2520
STLMMETKEN PLFAPIYLEN HSLNEILGVT KDLQNTASLI SNPNFRNNAT SLLEMASYTQ 2580
QTSRLTKLSD FRAREGESNF SERLLELKNK RFSDPNPSEV FVKYSQLSKH PNNLWIQGVG 2640
GASFISGGNG TLYGLNVGYD RLVKSVILGG YVAYGYSGFN GNIMHSLANN VDVGMYARAF 2700

LKRNEFTLSA NETYGGNASH INSSNSLLSV LNQRYNYNTW TTSVNGNYGY DFMFKQKSVV 2760
LKPQVGLSYH FIGLSGMKGK MQNPAYQQFV MHSNPSNESV LTLNMGLESR KYFGKNSYYF 2820
VTARLGRDLL IKAKGDNVVR FVGENTLLYR KGEIFNTFAS VITGGEMHLW RLMYVNAGVG 2880
LKMGLQYQDL NITGNVGMRV AF 2902

<212> Type : PRT
<211> Length : 2902
SequenceName : SEQ ID 91
SequenceDescription :
Sequence
--------
<213> OrganismName : Helicobacter pylori J99
<400> PreSequenceString :

```
MAFKKARLIS RFISKGSFKL NKISKKFFTL NQILKREKPL KRHKKTKSIE KPFNKNKSFL     60
KASVLLIGAL GGLSHLRANE CRYWSWSSWS YQDNIESGPN SPTHNSYCLF SSAQGSGTYY    120
LNTLTTYSAG GASFTQKFNG GTLDIGGNIR FGGTGINGGD VGYITGTYNA QTMNFNSSHI    180
TTGNSYADGG GTTLNFNATN NITINQASFD NSDAGTQKSY MNFKGSNIKI SGSSFTDDTN    240
GGFNFSGNNN NSTISFNQTS FNQGTYNFSN SATLSFNNSN FNQGTYHFNS AQSTFENSNF    300
NQGTYNFNDN TSFNNDTFNQ GTYNFNSSKV SFSGANTLNS SSPFASLKGS VSFNSGAIFN    360
LNQTLNNNQT YDILTTNGAI QYGVYQSYLW DLINYKGDKA ISHVEVSNNT YDVTFDINGQ    420
DETLQETFSN QSIITQFLGD DLQQQAQQTY QEDVANSQNA LNKVASDNTI ANNDTSYTQS    480
SNPTILKDAQ GLENTNQQIQ QDEKALEKDL AQIKQLANST TGFNEQAFTQ AQKQEQQDEQ    540
ALQNDENAFN TEQEGLEQAI ANAKHANPTP NPTPSPTPTP IKHTAPNTPP SQVPPTPPSQ    600
NLPKTNVWNG VYWLQNKTYS NKGIYYIDPN LSGQSGQSGN TLSTYTANLL GRSFGVNANN    660
GTLIIGNNTE SVNDNGLIWI GHGGFGYITG TFSAANIYLT NNFKTGEGVS NSDGGANIT     720
FKASDNITMD GLNYNNAETV TKMIQTGASQ HSYTTFDATN NISVTDSDFS DMTWGKFSFS    780
AKNISFSNAS FSGFTNPGGS STISTNASNS LSFTDSRLNG GAIYNLQANS LIFNNTQAVF    840
NVLYSRGTSN FNATTQLLGN TSFTLSSQSL LNFNGDTTLQ NNANITLGNK SQAAFKNSLT    900
LDNNSNLSLD NQSVLNANGT SAFNNQASLN IYNGSQAAFS SLFFNGGTLS LNANSKLNAS    960
SASFSNNTTI NLDDSVLNAN NTSSLNANIN FQGASQADFG GNTTIDTASF NFDSASSLNF   1020
NNLTANGALN FNGYAPSLTK ALMNVSGQFV LGNNGDINLS DINIFDNITK SVTYNILNAQ   1080
KGITGISGAN GYEKILFYGM KIQNATYSDN NNIQTWSFIN PLNSSQIIQE SIKNGDLTIE   1140
VLNNPNSASN TIFNIAPELY NYQDSKQNPT GYSYDYSDNQ AGTYYLTSNI KGLFTPKGSQ   1200
TPQTPGTYSP FNQPLNSLNI YNKGFSSENL KTLLGILSQN SATLKEMIES NQLDNITNIN   1260
EVLQLLDKIK ITQAQKQALL ETINHLTDNI NQTFNNGNLV IGATQDNVTN STSSIWFGGN   1320
GYSSPCALDS ATCSSFRNTY LGQLLGSTSP YLGYINADFK AKSIYITGTI GSSNAFESGG   1380
SADVTFQSAN NLVLNKANIE AQATDNIFNL LGQEGIDKIF NQGNLANVLS QMAMEKIKQA   1440
GGLGNFIENA LSPLSKELPA SLQDETLGQL IGQNNLDDLL NNSGVMNEIQ NIISQKLSIF   1500
GNFVTPSIIE NYLAKQSLKS MLDDKGLLNF IGGYIDASEL SSILGVILKD ITNPPTSLQK   1560
DIGVVANDLL NEFLGQDVVK KLESQGLVSN IINNVISQGG LSGVYNQGLG SVLPPSLQNA   1620
LKENDLGTLL SPRGLHDFWQ KGYFNFLSNG YVFVNNSSFS NATGGSLNFV ANKSIIFNGD   1680
NTIDFSKYQG ALIFASNGVS NINITTLNAT NGLSLNAGLN NVSVQKGEIC INLANCPTTK   1740
NSSPANSSVT PTNESLSVHA NNFTFLGTII SNGAIDLSQV TNNSVIGTLN LNENATLQAN   1800
NLTITNAFNN ASNSTANIDG NFTLNQQATL STNASGLNVM GNFNSYGDLV FNLSHSVSHA   1860
IINTQGTATI MANNNPLIQF NASSKEVGTY TLIDSAKAIY YGYNNQITGG SSLDNYLKLY   1920
ALIDINGKHM VMTDNGLTYN GQAVSVKDGG LVVGFKDSQN QYIYTSILYN KVKIAVSNDP   1980
INNPQAPTLK QYIAQIQGVQ SVDSIDQAGG NQAINWLNKI FETKGSPLFA PYYLESHSTK   2040
DLTTIAGDIA NTLEVIANPN FKNDATNILQ INTYTQQMSR LAKLSDTSTF ARSDFLERLE   2100
ALKNKRFADA IPNAMDVILK YSQRNRVKNN VWATGVGGAS FISGGTGTLY GINVGYDRFI   2160
KGVIVGGYAA YGYSGFHANI TQSGSSNVNV GVYSRAFIKR SELTMSLNET WGYNKTFINS   2220
YDPLLSIINQ SYRYDTWTTD AKINYGYDFM FKDKSVIFKP QVGLSYYYIG LSGLRGIMDD   2280
PIYNQFRANA DPNKKSVLTI NFALESRHYF NKNSYYFVIA DVGRDLFINS MGDKMVRFIG   2340
NNTLSYRDGG RYNTFASIIT GGEIRLFKTF YVNAGIGARF GLDYKDINIT GNIGMRYAF    2399
```

<212> Type : PRT

<211> Length : 2399

SequenceName : SEQ ID 92

SequenceDescription :

Sequence --------

<213> OrganismName : Helicobacter pylori J99

<400> PreSequenceString :

```
MEIQQTHRKI NRPLVSLVLA GALISAIPQE SHAAFFTTVI IPAIVGGIAT GTAVGTVSGL     60
LSWGLKQAEE ANKTPDKPDK VWRIQAGKGF NEFPNKEYDL YKSLLSSKID GGWDWGNAAR    120
HYWVKGGQWN KLEVDMKDAV GTYKLSGLRN FTGGDLDVNM QKATLRLGQF NGNSFTSYKD    180
SADRTTRVNF NAKNISIDNF VEINNRVGSG AGRKASSTVL TLQASEGITS SKNAEISLYD    240
```

GATLNLASNS VKLNGNVWMG RLQYVGAYLA PSYSTINTSK VQGEVDFNHL TVGDQNAAQA 300
GIIASNKTHI GTLDLWQSAG LNIIAPPEGG YKDKPNSTTS QSGTKNDKKE ISQNNNSNTE 360
VINPPNNTQK TETEPTQVID GPFAGGKDTV VNIFHLNTKA DGTIKVGGFK ASLTTNAAHL 420
NIGKGGVNLS NQASGRTLLV ENLTGNITVD GPLRVNNQVG GYALAGSSAN FEFKAGVDTK 480
NGTATFNNDI SLGRFVNLKV DAHTANFKGI DTGNGGFNTL DFSGVTDKVN INKLITASTN 540
VAVKNFNINE LIVKTNGISV GEYTHFSEDI GSQSRINTVR LETGTRSIFS GGVKFKSGEK 600
LVINDFYYSP WNYFDARNVK NVEITRKFAS STPENPWGTS KLMFNNLTLG QNAVMDYSQF 660
SNLTIQGDFI NNQGTINYLV RGGKVATLNV GNAAAMMFNN DIDSATGFYK PLIKINSAQD 720
LIKNTEHVLL KAKIIGYGNV STGTNGISNV NLEEQFKERL ALYNNNNRMD TCVVRNTDDI 780
KACGMAIGNQ SMVNNPDNYK YLIGKAWRNI GISKTANGSK ISVYYLGNST PTENGGNTTN 840
LPTNTTNNAH SANYALVKNA PFAHSATPNL VAINQHDFGT IESVFELANR SKDIDTLYTH 900
SGAQGRDLLQ TLLIDSHDAG YARQMIDNTS TGEITKQLNA ATDALNNVAS LEHKQSGLQT 960
LSLSNAMILN SRLVNLSRKH TNHINSFAQR LQALKGQEFA SLESAAEVLY QFAPKYEKPT 1020
NVWANAIGGA SLNSGSNASL YGTSAGVDAF LNGNVEAIVG GFGSYGYSSF SNQANSLNSG 1080
ANNANFGVYS RFFANQHEFD FEAQGALGSD QSSLNFKSTL LQDLNQSYNY LAYSATARAS 1140
YGYDFAFFRN ALVLKPSVGV SYNHLGSTNF KSNSQSQVAL KNGASSQHLF NANANVEARY 1200
YYGDTSYFYL HAGVLQEFAH FGSNDVASLN TFKINAARSP LSTYARAMMG GELQLAKEVF 1260
LNLGVVYLHN LISNASHFAS NLGMRYSF 1288

<212> Type : PRT
<211> Length : 1288
SequenceName : SEQ ID 93
SequenceDescription :
Sequence
-------- <213> OrganismName : Helicobacter pylori J99
<400> PreSequenceString :

MKKHILSLTL GSLLVSTLSA EDDGFYTSVG YQIGEAAQMV TNTKGIQDLS DRYESLNNLL 60
NRYSTLNTLI KLSADPSAIN AVRENLGASA KNLIGDKANS PAYQAVLLAI NAAVGFWNVV 120
GYVTQCGGNA NGQKSISSKT IFNNEPGYRS TSITCSLNGH SPGYYGPMSI ENFKKLNEAY 180
QILQTALKRG LPALKENNGK VNVTYTYTCS GDGNNNCSSQ VTGVNNQKDG TKTKIQTIDG 240
KSVTTTISSK VVDSRADGNT TGVSYTEITN KLEGVPDSAQ ALLAQASTLI NTINNACPYF 300
HASNSSEANA PKFSTTTGKI CGAFSEEISA IQKMITDAQE LVNQTSVINE HEQTTPVGNN 360
NGKPFNPFTD ASFAQGMLAN ASAQAKMLNL AEQVGQAINP ERLSGTFQNF VKGFLATCNN 420
PSTAGTGGTQ GSAPGTVTTQ TFASGCAYVG QTITNLKNSI AHFGTQEQQI QQAENIADTL 480
VNFKSRYSEL GNTYNSITTA LSNIPNAQSL QNAVSKKNNP YSPQGIDTNY YLNQNSYNQI 540
QTINQELGRN PFRKVGIVSS QTNNGAMNGI GIQVGYKQFF GQKRKWGARY YGFFDYNHAF 600
IKSSFFNSAS DVWTYGFGAD ALYNFINDKA TNFLGKNNKL SVGLFGGIAL AGTSWLNSEY 660
VNLATMNNVY NAKMNVANFQ FLFNMGVRMN LARPKKKDSD HAAQHGIELG LKIPTINTNY 720
YSFMGAELKY RRLYSVYLNY VFAY 744

<212> Type : PRT
<211> Length : 744
SequenceName : SEQ ID 94
SequenceDescription :
Sequence
--------
<213> OrganismName : Helicobacter pylori J99
<400> PreSequenceString :

```
MIKKAKKFIP FFLIGSLLAE DNGWYMSVGY QIGGTQQFIN NKQLLENQNI INSITQSAIN     60
IAGPTTGLIT LSSQTVIDAL GYGVSNTVGN QLEGISNILN QIGKRKDFYS SRQISSISQQ    120
IIGLKGSSDP LKAHSSQITA KLLSNTQSAF DQGIALSSNI ISAVNSLNPS NNSQEVKAQL    180
QNTAQSMAEL LQQIEHSITK TTSTTYAQSL LSNLTDAVNA SSNNTTYVSA LVNALNTLGV    240
GVFPTTTSTH VVLNPPGQVV FYPTNSLLGS TSSNSNNQQQ YNNTLLMNTL QGELSTNNQN    300
NPNGCANQIQ CLEQFIQNLT PLAATPTSTN QANQQVQAIA QKLQSVAINA LDNNAINNTT    360
YNLNNLHNAL NFQAYQSTIE QYNNALKQIS WISFSEPKNL LKNTSNNYQI GTVTNDQGQN    420
ISAYDCTSAT GSLSSDASSG ISCSATSSTN NTNSFDNSLV ATSKVQTING KEQIGVNSFN    480
LVSQVWSVYN SLKTSEENLQ KNAKILCNNG SQSGTSPCNS SSGGLSISGN AQLQNILSPT    540
NGTTTNTQAK SNASKLKAMV MVNNEEEAKT TNFNQSSGPT TQSSNSTVMG ALNTVLQNVS    600
NFQQSIQSAF QNQENNIQAW ANALYNTSNP NGNQSQNLTT NNNQDLRIQL RANFYQLINT    660
INQQVPTDMN ALINQSQQTQ QTSGSASTTN NACASGMGSS GNWCYQQWSD SKAYYSGLQS    720
ALGYQTQATT QNGSSGGSNI TYNVQQITLT SGGLLNQIIT NLKSVNGGSN GGSSGNGTSQ    780
INTAYQMLTD ASDGKLGTYN SSNSSNSSNS GNNNGYTPCN STNGSNGTSG SNCYEPNKQQ    840
NATTATTTTD SNLQKVYNDA QKIANIIASS GNNKGVENGL KQFFEALKSN SSSLSNLCGN    900
GSSGSSSTCS GGLINLLGAI PTNGVSDTNN LINLLTEFIK TAGFIQNKDS NVSTSLTSAF    960
QAITSAISQG FQALQNDISP NAILTLLQEI TSNTTTIQSF SQTLRQLLGD KTFFMVQQKL   1020
IDAMINARNQ VQNAQNQANN YGSQPVLSQY AAAKSTQHGM SNGLGVGIGY KYFFGKARKL   1080

GLRHYFFFDY GFSEIGLANQ SVKANIFAYG VGTDFLWNLF RRTYNTKALN FGLFAGVQLG   1140
GATWLSSLRQ QIIDNWGNAN DIHSTNFQVA LNFGVRTNFA EFKRFAKKFH NQGVISQKSV   1200
EFGIKVPLIN QAYLNSAGAD VSYRRLYTFY INYIMGF                            1237
```

<212> Type : PRT
<211> Length : 1237
SequenceName : SEQ ID 95
SequenceDescription :
Sequence --------
<213> OrganismName : Helicobacter pylori J99
<400> PreSequenceString :

```
MKQNLKPFKM IKENLMTQSQ KVRFLAPLSL ALSLSFNPVG AEEDGGFMTF GYELGQVVQQ     60
VKNPGKIKAE ELAGLLNSTT TNNTNINIAG TGGNVAGTLG NLFMNQLGNL IDLYPTLKTN    120
NLHQCGSTNS GNGATAAAAT NNSPCFQGNL ALYNEMVDSI KTLSQNISKN IFQGDNNTTS    180
ANLSNQLSEL NTASVYLTYM NSFLNANNQA GGIFQNNTNQ AYENGVTAQQ IAYVLKQASI    240
TMGPSGDSGA AGAFLDAALA QHVFNSANAG NDLSAKEFTS LVQNIVNNSQ NALTLANNAN    300
ISNSTGYQVS YGGNIDQARS TQLLNNTTNT LAKVTALNNE LKANPWLGNF AAGNSSQVNA    360
FNGFITKIGY KQFFGENKNV GLRYYGFFSY NGAGVGNGPT YNQVNLLTYG VGTDVLYNVF    420
SRSFGSRSLN AGFFGGIQLA GDTYISTLRN SPQLASRPTA TKFQFLFDVG LRMNFGILKK    480
DLKSHNQHSI EIGVQIPTIY NTYYKAGGAE VKYFRPYSVY WVYGYAF                  527
```

<212> Type : PRT
<211> Length : 527
SequenceName : SEQ ID 96
SequenceDescription :
Sequence
--------
<213> OrganismName : Helicobacter pylori J99
<400> PreSequenceString :

```
MKKTLLLSLS LSFGLHAEDD GFYASAGIRI GEAAQMVKNT KGIQQLSENY EKLNNLLNNY     60
NTLNTLVKLS SDPSAVNDAR DNLGSSTRNL LDVKANSPAY QAVLLALNAA VGLWQVTSYA    120
FTACGPGSNE NANGGIQTFN NVPGQNTTTI TCNSYYEPGH GGPISTKNYA IINKAYQIIQ    180
KALTANGEGI PVLSNTTTKL DFTINGDKRT GGEPNKKLVY PWSHGKAIST SWNATITAPT    240
TENINTTNSA QELLKQASII ITTLNSACPN FQNGGSGYWA GISGNGTMCG MFKNEISAIQ    300
GMIANAQEAV AQAKIVSENT QNQNSLDAGK PFNPYTDASF AESMLKNAQA QAEILNQAEQ    360
VVKNFEKIPT AFVNDSLGVC YEVQGGERRG TNPGQTTSNT WGAGCAYVGQ TITNLKNSIA    420
HFGTQEQQIQ QAENIADTLV NFKSRYSELG NTYNSITTAL SNIPNAQSLQ NAVSKKNNPY    480
SPQGIDTNYY LNQNSYNQIQ TINQELGRNP FRKVGIVSSQ TNNGAMNGIG IQVGYKQFFG    540
QKRKWGARYY GFFDYNHAFI KSSFFNSASD VWTYGFGADA LYNFINDKAT NFLGKNNKLS    600
VGLFGGIALA GTSWLNSEYV NLATMNNVYN AKMNVANFQF LFNMGVRMNL ARPKKKDSDH    660
AAQHGIELGL KIPTINTNYY SFMGAELKYR RLYSVYLNYV FAY                      703
```

<212> Type : PRT

<211> Length : 703
SequenceName : SEQ ID 97
SequenceDescription :
Sequence --------
<213> OrganismName : Helicobacter pylori J99
<400> PreSequenceString :

```
MIKKNRTLFL SLALCASISY AEDDGGFFTV GYQLGQVMQD VQNPGGAKSD ELARELNADV    60
TNNILNNNTG GNVAGALSNA FSQYLYSLLG AYPTKLNGND VSANALLSGA VGSGTCAAAG   120
TAGGTTLNTQ SACTAAGYYW LPSLTDRILS TIGSQTNYGT NTNFPNMQQQ LTYLNAGNVF   180
FNAMNKALEK NGTATANSTS STSGATGSDG QTYSQQAIQY LQGQQNILNN AANLLKQDEL   240
LLEAFNSAVA ANIGNKEFNS AAFTGLVQGI IDQSQLVYNE LTKNTISGSA VNNAGINSNQ   300
ANAVQGRASQ LPNALYNVQV TLDKINALNN QVRSMPYLPQ FRAGNSRATN ILNGFYTKVG   360
YKQFFGKKRN IGLRYYGFFS YNGASVGFRS TQNNVGLYTY GVGTDVLYNI FSRSYQNRSV   420
DMGFFSGIQL AGETFQSTLR DDPNVKLHGK INNTHFQFLF DFGMRMNFGK LDGKSNRHNQ   480
HTVEFGVVVP TIYNTYYKSA GTTVKYFRPY SVYWSYGYSF                         520
```

<212> Type : PRT
<211> Length : 520
SequenceName : SEQ ID 98
SequenceDescription :
Sequence --------
<213> OrganismName : Helicobacter pylori J99
<400> PreSequenceString :

```
MKKKFLSLTL GSLLVSALSA EDNGFFVSAG YQIGESAQMV KNTKGIQDLS DSYERLNNLL    60
TNYSVLNALI RQSADPNAIN NARGNLNASA KNLINDKKNS PAYQAVLLAL NAAAGLWQVM   120
SYAISPCGPG KDTSKNGGVQ TFHNTPSNQW GGTTITCGTT GYEPGPYSIL STENYAKINK   180
AYQIIQKAFG SSGKDIPALS DTNTELKFTI NKNNGNTNTN NNGEEIVTKN NAQVLLEQAS   240
TIITTLNSAC PWINNGGAGG ASSGSLWEGI YLKGDGSACG IFKNEISAIQ DMIKNAAIAV   300
EQSKIVAANA QNQRNLDTGK TFNPYKDANF AQSMFANAKA QAEILNRAQA VVKDFERIPA   360
EFVKDSLGVC HEVQNGHLRG TPSGTVTDNT WGAGCAYVGE TVTNLKDSIA HFGDQAERIH   420
NARNLAYTLA NFSSQYQKLG EHYDSITAAI SSLPDAQSLQ NVVSKKTNPN SPQGIQDNYY   480
IDSNIHSQVQ SRSQELGSNP FRRAGLIAAS TTNNGAMNGI GFQVGYKQFF GKNKRWGARY   540
YGFVDYNHTY NKSQFFNASS DVWTYGVGSD LLVNFINDKA TKHNKISFGA FGGIALAGTS   600
WLNSQYVNLA NVNNYYKAKI NTANFQFLFN LGLRMNLARK KHRATDNAAQ HGIELGTKIP   660
TINTNYYSLL-GTTLQYRRLY SVYLNYVFAY                                   690
```

<212> Type : PRT
<211> Length : 690
SequenceName : SEQ ID 99
SequenceDescription :
Sequence --------
<213> OrganismName : Helicobacter pylori J99
<400> PreSequenceString :

```
MKIKKSLFAL SFSLMASLSR AEDDGFYMSV GYQIGEAVQK VKNTGALQNL ADRYDNLSNL    60
LNQYNYLNSL VNLASTPSAI TGAIDNLSSS AINLTSATTT SPAYQAVALA LNAAVGMWQV   120
IAFGISCGPG PNLGPEHLEN GGVRSFDNTP NYSYNTGSGT TTTTCNGASN VGPNGILSSS   180
EYQVLNTAYQ TIQTALNQNQ GGGMPALNSS KNMVVNINQT FTKNPTTEYT YPDGNGNYYS   240
GGSSIPIQLK ISSVNDAENL LQQAATIINV LTTQNPHVNG GGGAWGFGGK TGNVMDIFGD   300
SFNAINEMIK NAQAVLEKTQ QLNANENTQI TQPDNFNPYT SKDTQFAQEM LNRANAQAEI   360
LSLAQQVADN FHSIQGPIQQ DLEECTAGSA GVINDNTYGS GCAFVKETLN SLEQHTAYYG   420
NQVNQDRALS QTILNFKEAL STLGNDSKAI NSGISNLPNA KSLQNMTHAT QNPNSPEGLL   480
TYSLDTSKYN QLQTVAQELG KNPFRRIGVI NYQNNNGAMN GIGVQAGYKQ FFGKKRNWGL   540
RYYGFFDYNH AYIKSNFFNS ASDVWTYGVG MDALYNFIND KNTNFLGKNN KLSVGLFGGF   600
ALAGTSWLNS QQVNLTMMNG IYNANVSASN FQFLFDLGLR MNLARPKKKD SDHAAQHGME   660
LGVKIPTINT DYYSFMGAEL KYRRLYSVYL NYVFAY                             696
```

<212> Type : PRT
<211> Length : 696
SequenceName : SEQ ID 100
SequenceDescription :

Sequence ------
<213> OrganismName : Helicobacter pylori J99
<400> PreSequenceString :

```
MKIKKSLFAL SFSLMASLSR AEDDGFYMSV GYQIGEAVQK VKNTGALQNL ADRYDNLSNL    60
LNQYNYLNSL VNLASTPSAI TGAIDNLSSS AINLTSATTT SPAYQAVALA LNAAVGMWQV   120
IAFGISCGPG PNLGPEHLEN GGVRSFDNTP NYSYNTGSGT TTTTCNGASN VGPNGILSSS   180
EYQVLNTAYQ TIQTALNQNQ GGGMPALNSS KNMVVNINQT FTKNPTTEYT YPDGNGNYYS   240
GGSSIPIQLK ISSVNDAENL LQQAATIINV LTTQNPHVNG GGGAWGFGGK TGNVMDIFGD   300
SFNAINEMIK NAQAVLEKTQ QLNANENTQI TQPDNFNPYT SKDTQFAQEM LNRANAQAEI   360
LSLAQQVADN FHSIQGPIQQ DLEECTAGSA GVINDNTYGS GCAFVKETLN SLEQHTAYYG   420
NQVNQDRALS QTILNFKEAL STLGNDSKAI NSGISNLPNA KSLQNMTHAT QNPNSPEGLL   480
TYSLDTSKYN QLQTVAQELG KNPFRRIGVI NYQNNNGAMN GIGVQAGYKQ FFGKKRNWGL   540
RYYGFFDYNH AYIKSNFFNS ASDVWTYGVG MDALYNFIND KNTNFLGKNN KLSVGLFGGF   600
ALAGTSWLNS QQVNLTMMNG IYNANVSASN FQFLFDLGLR MNLARPKKKD SDHAAQHGME   660
LGVKIPTINT DYYSFMGAEL KYRRLYSVYL NYVFAY                             696
```

<212> Type : PRT
<211> Length : 696
SequenceName : SEQ ID 101
SequenceDescription :
Sequence --------
<213> OrganismName : Helicobacter pylori J99
<400> PreSequenceString :

```
MHKKVLLALT ASLICQESLF AKDKDYTLGK VSTAGKKDRS DYSGQVNLGY SGITAPKSWQ    60
DEEVKKYTGS RTVISNKALT QQANQSIEEA LQNVPGLQIR NATGVGAMPT IQIRGFGAGG   120

SGHSDATLML VNGIPVYMAP YAHIELDIFP VTFQAIDRID VIKGGGSVQY GPNTYGGIVN   180
IITKPIPNQW ENQAAERITY WAKARNAGFA APPDKTGDPS FIKSLGNNLL YNTYVRSGGM   240
INKHVGIQAQ ANWVRGQGFR DNSPSSISNY WLDGVYDINE SNGIKAYYQY YDFAIAQPGS   300
LSEQDYKINR FANLRPLNQK GGRSQRFGAV YENRFGDLDR VGGTFSFTYY GQLMTRDFQV   360
SSSYNSANMV TCFSEAACRA AGLPAGYNLA VPYYATNYNG WAEVENPVRS INNAFEPKVN   420
LIVNTGKVRQ TFIMGLRFMT TTFLQRQYLN TNECATKTSG EGAGFLCEGP NVMSGWKPHI   480
KHGVYRNWNN WRNNYTAVYL SDRIEAWDGR FFIVPGLRYA FVQYNNENAS NWMQIPEKDL   540
RKIKHMNNWM PSTNIGFIPV QGDHNVLTYF NYQRSFVPPQ LDVLSYGGAE YFTQHFDTVE   600
AGARYTYKDK FSFNADYFRI WARDFATGQY SVYTSGPMKG NVRPINGYSQ GVELELYYRP   660
IRGLQFHAAF NYIDTRVTSH GPLTDLNGDV LKGTSYNKHF PFVSPFQFIF DARYNWRKTT   720
IGISSYFYSR AYSGISNSAA GGYYGMQYYS GGNNYESVLN SGYQCEAWCM TQHEGLLPWY   780
WVWNIQVSQI FWENGRHRVT GSLQINNIFN MKYYFTGIGS SPAGLQPAPG RSVTAYLNYT   840
F                                                                  841
```

<212> Type : PRT
<211> Length : 841
SequenceName : SEQ ID 102
SequenceDescription :
Sequence
--------
<213> OrganismName : Helicobacter pylori J99
<400> PreSequenceString :

MKKTLLLSLS ASSLLNAEDN GFFISAGYQI GEAAQMVKNT GELKKLSDTY ENLSNLLTNF 60
NNLNQAVTNA SSPSEINAAI DNLKANTQGL IGEKTNSPAY QAVYLALNAA VGLWNVIAYN 120
VQCGPGNSGQ QSVTFEGQPG HNSSSINCNL TGYNNGVSGP LSIENFKKLN QAYQTIQQAL 180
KQDSGFPVLD SAGKQVTITI TTQTNGANKS ETTTTTTTTN DAQTLLQEAS KMISVLTTNC 240
PWVNHNQGQN GGAPWGLDTA GNVCQVFATE FSAVTSMIKN AQEIVTQAQS LNQQNNQNAP 300
QDFNPYTSAD RAFAQNMLNH AQAQAKILEL ADQMKKDLNT IPSQFITNYL AACHNGGGTL 360
PDAGVTNNTW GAGCAYVEET ITALNNSLAH FGTQAEQIKQ SELLARTILD FRGSLSNLNN 420
TYNSITTTAS NTPNSPFLKN LISQSTNPNN PGGLQAVYQV NQSAYSQLLS ATQELGHNPF 480
RRVGLISSQT NNGAMNGIGV QVGYKQFFGE KRRWGLRYYG FFDYNHAYIK SSFFNSASDV 540
FTYGVGTDVL YNFINDKTTK NSKISFGVFG GIALAGTSWL NSQYVNLATF NNFYSAKMNV 600
ANFQFLFNLG LRMNLAKNKK KASDHAAQHG VELGVKIPTI NTNYYSLLGT QLQYRRLYSV 660
YLNYVFAY 668

<212> Type : PRT
<211> Length : 668
SequenceName : SEQ ID 103
SequenceDescription :
Sequence
--------
<213> OrganismName : Helicobacter pylori J99
<400> PreSequenceString :

MRKLFIPLLL FSALEANEKN GFFIEAGFET GLLEGTQTQE KRHTTTKNTY ATYNYLPTDT 60
ILKRAANLFT NAEAISKLKF SSLSPVRVLY MYNGQLTIEN FLPYNLNNVK LSFTDAQGNT 120
IDLGVIETIP KHSKIVLPGE AFDSLKEAFD KIDPYTLFLP KFEATSTSIS DTNTQRVFET 180
LNNIKTNLIM KYSNENPNNF NTCPYNNNGN TKNDCWQNFT PQTAEEFTNL MLNMIAVLDS 240
QSWGDAILNA PFEFTNSSTD CDSDPSKCVN PGVNGRVDTK VDQQYILNKQ GIINNFRKKI 300
EIDAVVLKNS GVVGLANGYG NDGEYGTLGV EAYALDPKKL FGNDLKTINL EDLRTILHEF 360
SHTKGYGHNG NMTYQRVPVT KDGQVEKDSN GKPKDSDGLP YNVCSLYGGS NQPAFPSNYP 420
NSIYHNCADV PAGFLGVTAA VWQQLINQNA LPINYANLGS QTNYNLNASL NTQDLANSML 480
STIQKTFVTS SVTNHHFSNA SQSFRSPILG VNAKIGYQNY FNDFIGLAYY GIIKYNYAKA 540
VNQKVQQLSY GGGIDLLLDF ITTYSNKNSP TGIQTKRNFS SSFGIFGGLR GLYNSYYVLN 600
KVKGSGNLDV ATGLNYRYKH SKYSVGISIP LIQRKASVVS SGGDYTNSFV FNEGASHFKV 660
FFNYGWVF 668

<212> Type : PRT
<211> Length : 668
SequenceName : SEQ ID 104
SequenceDescription :
Sequence --------
<213> OrganismName : Helicobacter pylori J99
<400> PreSequenceString :

MNKTTIKILM GMALLSSLQA AEAELDEKSK KPKFADRNTF YLGVGYQLSA INTSFSTSSI 60
DKSYFMTGNG FGVVLGGKFV AKTQAVEHVG FRYGLFYDQT FSSHKSYIST YGLEFSGLWD 120
AFNSPKMFLG LEFGLGIAGA TYMPGGAMHG IIAQYLGKEN SLFQLLVKVG FRFGFFHNEI 180

TFGLKFPVIP NKKTEIVDGL SATTLWQRLP VAYFNYIYNF 220

<212> Type : PRT
<211> Length : 220
SequenceName : SEQ ID 105
SequenceDescription :
Sequence --------
<213> OrganismName : Helicobacter pylori J99
<400> PreSequenceString :

MKKTKKTILL SLTLAASLLH AEDNGVFLSV GYQIGEAVQK VKNADKVQKL SDVYEQLSKL 60
LANDNGTSSK TSAQAINQAV NNLNESAKTL AGGTTNSPAY QATLLALRSA LGLWNSMGYA 120
VVCGGYIKKP GENNQKNFHY TDENGNGTTI NCGGSTNSNG THSPNGTNTL KADKNVSLSI 180
EQYEKIHEAY QILSKALKQA GLAPLNSKGE KLEAHVTTSK DQQGTSSDQT TTTTSVIDTT 240
NDAQNLLTQA QTIVNTLKDY CPMLIAKSSS NGGTNGANTP SWQTAGGGKN SCATFGAEFS 300
AISDMISNAQ KIVQETQQLN ANQPKNITQP NNFNLNSPGS LTALAQSMLK NAQSQTEILK 360
LANQVASDFD KLSSGYLKDY IGKCDVSGVS SSNMTPQNMN TTWGKGCAGV EETLTSLKAS 420
TTDFNNQTTP QLDQAQTLAN TLTQELGNNP FKRVGIIGSQ TNNGAMNGLG VQAGYKQFFG 480
QKRRWGLRYY GFFDYNHTYI KSSFFNSSSD VLTYGVGSDL LFNFINDKNT NFLGKNNKIS 540
VGLFGGIALA GTSWLNSQFV NLKTISNVYS AKVNTANFQF LFNLGLRTNL ARPKKKDSDH 600
SAQHGMELGV KIPTINTNYY SYLGTKLEYR RLYSVYLNYV FAY 643

<212> Type : PRT
<211> Length : 643
SequenceName : SEQ ID 106
SequenceDescription :
Sequence
--------
<213> OrganismName : Helicobacter pylori J99
<400> PreSequenceString :

MKKTILLSLM VSSLFAENDG VYMSVGYQIG EAAQMVKNTG EIQKVSNAYE NLNNLLTRYN 60
ELKQTASNTD SSTAQAIDNL EKSASRLKTT PNTANQAVSS ALSSAVGMWQ VIASNLANNS 120
LSSSEYEKLK ATSQLLQNTL ENKNNNLKIE NDYDQLLTQA STIINTLQSQ CPGVDGGNGK 180
PWGINTSGNA CAIFGSTFNA INSMIDSAKK AAADARRTAP ESPNQQNAFT NADFNKNLNQ 240
VSSVINDTIS YLKGDNLETI YNTIQKTPNS KGFQSLVSRS SYSYSLNETQ YSQFQTTTKE 300
FGHNPFRSVG LINSQSNNGA MNGVGVQLGY KQFFGKNKFF GIRYYGFFDY NYAYIKSNFF 360
NSASNVFTYG AGSDLLLNFI NGGSDRNRKV SFGIFGGIAL AGTTWLNNQS ANLKITNSAY 420
SAKINNTNFQ FLFNTGLRLQ GIHHGIELGV KIPTINTNYY SFMGAKLAYR RLYSLYLNYV 480
LAY 483

<212> Type : PRT
<211> Length : 483
SequenceName : SEQ ID 107
SequenceDescription :
Sequence --------
<213> OrganismName : Helicobacter pylori J99
<400> PreSequenceString :

MPKASQVLFF GAFLSTSLQG FEAKLNGFVD QSSTIGFNQH KINKERGIYP MQQFATIAGY 60
LGLGFSLLPK KVSDHVLKGK IGGMVGSIFY DGTKKFEDGS VAYNLFGYYD GFMGVYTNIL 120
QTDSLETQNM KHNKNVRNYV FSDAYLEYAY KNYFEIKAGR YLSTMPYKSG QTQGFQVSGQ 180
YKHARLTWFS SWGRAFAYGS FLMDWFAART TYSGGFTKNN NGGYDSHGRK VLYGTHAVQL 240
TYKPHRFLIE GFYYLSPQIF NAPGVKIGWD SNPNFSGTGF RSDTAIIGFF PIYYPWMIVK 300
SNGSPVYRYD TPATQNGQNL IIRQRFDINN YNVSIAFYKV FQNANGWIGN MGNPSGVIMG 360
SNSVYAGFTG TALKRDAATI FLSCGGTHFA KKFTWKFATQ YSNSVVSWEA RAMISLGYKF 420
TEYLSGSVDL AYYGVHTNKG FKPGENGPVP KNFPALYSDR SALYTALVAS F 471

<212> Type : PRT
<211> Length : 471
SequenceName : SEQ ID 108
SequenceDescription :
Sequence
--------
<213> OrganismName : Helicobacter pylori J99
<400> PreSequenceString :

MLRLVSKTIC LSLISLFNPL EAFQKHQKDV FFVEAGFETG LLEGAQTKEQ AIAQNTQNTQ 60

KIYENPLTHP QTKEQPKEQN KSDTATPQSV YGRYYILQNT ILEKATELFT AANINGNGLT 120
FYSQNPVYVM AYNKDNAEFE GYGNNSVVVI QNFLPYNLNN IELSYTDAQG KAVNLGVIET 180
IPKDSQIILP ASLFNNFSND SPFNSDGLQQ LQTTTTPFSD ANTQSLFEKL SQITTNLQMT 240
YENTDPFSSG NNDPNGPLAS PKPHYECPGY KKSCQVASVS FTPQTAEELT NLMLDMIAVF 300
DSKSWEEAVL NAPFQFSNSP SECGIDYPKC VNPFNNGLVD PKDEKYALTP EEVINSYRVA 360
NELTVNLLNA AKGFLGLGSQ LGSANAPDDD GFNQGVLGIA PFALDPEKLF GKNLNKVAIL 420
ALRDIIHEYG HTLGYTHNGN MTYQRVRLCQ EGNGPEARCE GGHEVEKNGK EELEFSNGHE 480
VRDHDGYTYD VCSRFGGKNQ PAFPSNYPNS IYTNCAQVPA GLIGVTTAVW QQLINQNALP 540
INFANLNSQT SHLNAGLNAQ NFATSMVSAI AQNFSTTSTT TYRSSSKNFR SPILGVNVKI 600
GYQHYFNDYI GLAYYGIIQY NYAQANDEKI QQLSYGGGMD VLFDFITYT NKKQDHPTKK 660
VFASSFGVFG GLRGLYNSYY VFNQVKGSGN LDIVTGFNYR YKHSKYSIGV SVPLIQSGIK 720
IASNNGIYAD SVVLNEGGSH FKVFFNYGWV F 751

<212> Type : PRT
<211> Length : 751
SequenceName : SEQ ID 109
SequenceDescription :
Sequence --------
<213> OrganismName : Helicobacter pylori J99
<400> PreSequenceString :

MQNFVFNKKW LIYSSLLPLF FLNPLMAEDD GFFMGVSYQT SLAVQRVDNS GLNASQDAST 60
YIRQNAIALE SAAVPLAYYL EAMGQQTRVL MQMLCPDPSK RCLLYAGGYQ NGQNNNGDTG 120
NNPPRGNVNA TFDMQSLVNN LNKLTQLIGE TLIRNPENLP NSKVFNVKFG NQSTVIALPE 180
GLANTMDALN NDITNALTTL WYNQTLTNKS FSTPSNTSVN FSPQVLQHLL QDGLATANNN 240
QTICSTQNQC TATNEAKSIA QNAQNIFQAL MQAGILGGLA NEKQFGFTYN KAPNGSDSQQ 300
GYQSFSGPGY YTKNDNTTQA PLKALPAGAT IGSGNGQYTY HPSSAVYYLA DSIIANGITA 360
SMIFSGMQNF ANKAAKLIGT SSYNQMQDAI NYGESLLSNT VAYGDFITNW VAPYLDLNNK 420
GLNFLPNYGG QLNGANNQTP QLTPQQAQQE QKVIMNQLEQ ATNAPTPAQI NRILANPYSP 480
TAKTLMAYGL YRSKAVIGGV IDEMQTKVNQ VYQMGFARNF LEHNSNSNNM NGFGVKMGYK 540
QFFGKKRMFG LRYYGFYDFG YAQFGTESSL VKATLSSYGA GTDFLYNVFT RKRGTEAIDI 600
GFFAGIQLAG QTWKTNFLDQ VDGNHLKPKD TSFQFLFDLG IRTNFSKIAH QKRSRFSQGI 660
EFGLKIPVLY HTYYQSEGVT AKYRRDFSFY VGYNIGF 697

<212> Type : PRT
<211> Length : 697
SequenceName : SEQ ID 110
SequenceDescription :
Sequence --------
<213> OrganismName : Helicobacter pylori J99
<400> PreSequenceString :

MKKTILLSLS LSLASSLLHA EDNGFFVSAG YQIGEAVQMV KNTGELKNLN EKYEQLSQYL 60
NQVASLKQSI QNANNIELVN SSLNYLKSFT NNNYNSTTQS PIFNAVQAVI TSVLGFWSLY 120
AGNYLTFFVV NKDTQKPASV QGNPPFSTIV QNCSGIENCA MNQTTYDKMK KLAEDLQAAQ 180
QNATTKANNL CALSGCATTQ GQNPSSTVSN ALNLAQQLMD LIANTKTAMM WKNIVIAGVS 240
NVSGAIDSTG YPTQYAVFNN IKAMIPILQQ AVTLSQSNHT LSASLQAQAT GSQTNPKFAK 300
DIYAFAQNQK QVISYAQDIF NLFSSIPKDQ YRYLEKAYLK IPNAGKTPTN PYRQEVNLNQ 360
EIQTIQNNVS YYGNRVDAAL SVAKDVYNLK SNQTEIVTTY NNAKNLSQEI SKLPYNQVNT 420
KDIITLPYDQ NAPAAGQYNY QINPEQQSNL SQALAAMSNN PFKKVGMISS QNNNGALNGL 480
GVQVGYKQFF GESKRWGLRY YGFFDYNHGY IKSSFFNSSS DIWTYGGGSD LLVNFINDSI 540
TRKNNKLSVG LFGGIQLAGT TWLNSQYMNL TAFNNPYSAK VNASNFQFLF NLGLRTNLAT 600
AKKKDSERSA QHGVELGIKI PTINTNYYSF LGTKLEYRRL YSVYLNYVFA Y 651

<212> Type : PRT
<211>
Length : 651
SequenceName : SEQ ID 111
SequenceDescription :
Sequence --------
<213> OrganismName : Helicobacter pylori J99
<400> PreSequenceString :

```
MLKLASKTIC LSLISSFTAV EAFQKHQKDG FFIEAGFETG LLQGTQTQEQ TIATTQEKPK     60
PKPKPKPITP QSTYGKYYIS QSTILKNATE LFAEDNITNL TFYSQNPVYV TAYNQESAEE    120
AGYGNNSLIM IQNFLPYNLN NIELSYTDDQ GNVVSLGVIE TIPKQSQIIL PASLFNDPQL    180
NADGFQQLQT NTTRFSDAST QNLFNKLSKV TTNLQMTYIN YNQFSSGNGS GSKPPCPPYE    240

NQANCVAKVP PFTSQDAKNL TNLMLNMMAV FDSKSWEDAV LNAPFQFSDN NLSAPCYSDY    300
LTCVNPYNDG LVDPKLIAKN KGDEYNIENG QTGSVILTPQ DVIYSYRVAN NIYVNLLPTR    360
GGDLGLGSQY GGPNGPGDDG TNFGALGILS PFLDPEILFG KELNKVAIMQ LRDIIHEYGH    420
TLGYTHNGNM TYQRVRMCEE NNGPEERCQG GRIEQVDGKE VQVFDNGHEV RDTDGSTYDV    480
CSRFKDKPYT AGSYPNSIYT DCSQVPAGLI GVTSAVWQQL IDQNALPVDF TNLSSQTNYL    540
NASLNTQDFA TTMLSAISQS LSSSKSSATT YRTSKTSRPF GAPLLGVNLK MGYQKYFNDY    600
LGLSSYGIIK YNYAQANNEK IQQLSYGVGM DVLFDFITNY TNEKNPKSNL TKKVFTSSLG    660
VFGGLRGLYN SYYLLNQYKG SGNLNVTGGL NYRYKHSKYS IGISVPLVQL KSRIVSSDGA    720
YTNSITLNEG GSHFKVFFNY GWIF                                           744
```

<212> Type : PRT
<211> Length : 744
SequenceName : SEQ ID 112
SequenceDescription :
Sequence
--------
<213> OrganismName : Helicobacter pylori J99
<400> PreSequenceString :

```
MRKLFIPLLL FSALEANEKN GFFIEAGFET GLLEGTQTQE KRHTTTKNTY ATYNYLPTDT     60
ILKRAANLFT NAEAISKLKF SSLSPVRVLY MYNGQLTIEN FLPYNLNNVK LSFTDAQGNV    120
IDLGVIETIP KHSKIVLPGE AFDSLKIDPY TLFLPKIEAT STSISDANTQ RVFETLNKIK    180
TNLVVNYRNE NKFKDHENHW EAFTPQTAEE FTNLMLNMIA VLDSQSWGDA ILNAPFEFTN    240
SPTDCDNDPS KCVNPGTNGL VNSKVDQKYV LNKQDIVNKF KNKADLDVIV LKDSGVVGLG    300
SDITPSNNDD GKHYGQLGVV ASALDPKKLF GDNLKTINLE DLRTILHEFS HTKGYGHNGN    360
MTYQRVPVTK DGQVEKDSNG KPKDSDGLPY NVCSLYGGSN QPAFPSNYPN SIYHNCADVP    420
AGFLGVTAAV WQQLINQNAL PINYANLGSQ TNYNLNASLN TQDLANSMLS TIQKTFVTSS    480
VTNHHFSNAS QSFRSPILGV NAKIGYQNYF NDFIGLAYYG IIKYNYAKAV NQKVQQLSYG    540
GGIDLLLDFI TTYSNKNSPT GIQTKRNFSS SFGIFGGLRG LYNSYYVLNK VKGSGNLDVA    600
TGLNYRYKHS KYSVGISIPL IQRKASVVSS GGDYTNSFVF NEGASHFKVF FNYGWVF       657
```

<212> Type : PRT
<211> Length : 657
SequenceName : SEQ ID 113
SequenceDescription :
Sequence
--------
<213> OrganismName : Helicobacter pylori J99
<400> PreSequenceString :

```
MSLATSYNVS NNFSKFNIKR VRGYLICLVC NTPKMIQRGL NGVSFYGCSD YVNKGDCKGV     60
LREINGSMKM VCLHCENTPI MEKVESGRGG AYACKNCNRK FYFIDLAKQN ERKKDLEKEK    120
KELLNKIEKQ KIKHLERFIL AGVKANIKEN SFFLGCKNYP KCEWTASMDS QDLKCPKCNR    180
LMKRKKNFKN NEFFTATSLT LNAIEFCLYI NLKKKETNV                           219
```

<212> Type : PRT
<211> Length : 219
SequenceName : SEQ ID 114
SequenceDescription :
Sequence --------
<213> OrganismName : Helicobacter pylori J99
<400> PreSequenceString :

MEIKKYFLYA LFFLLFSGLF LSKLQAYKFN MSIVGKVSSY TKFGFNNQRY QPSKDIYPTG 60
SYTSLLGELN LSMGLYKGLR AEVGAMMAAL PYDSTAYQGN NIPNGQPGSR TDPFGAGIFW 120
QYIGWYAGHS GLNVQKPRLA MVHNAFLSYN YKKDKFSFGV KGGRYDAEEY DWFTSYTQGV 180
EGFVKYKDTR LRVMYSDARA SASSDWFWYF GRYYTSGKAL MIADLKYEKD NLKINPYFYA 240
IFQRMYAPGI NITYDTNPNF NNKGFRFVGT FVGFFPIFAT PANQNDIILF QQVPLGKSGQ 300
TYFFRTRFYY NKWQFGGSVY KNIGNANGDI GIYGDPLGYN IWTNSIYDAE INNIVGADVI 360
NGFLYVGSQY RGFSWKILGR WTDSPRADER SLALFLSYFS NKYNIRMDLK LEYYGNITKK 420
GYCIGYCGMY VPVDPNGPGT QPLTHNVYSD RSHIMFNITY GFRIY 465

<212> Type : PRT
<211> Length : 465
SequenceName : SEQ ID 115
SequenceDescription :
Sequence--------
<213> OrganismName : Helicobacter pylori J99
<400> PreSequenceString :

MKKTILLSLS LSLASSLLHA EDNGFFVSAG YQIGEAVQMV KNTGELKNLN DKYEQLSQSL 60
AQLASLKKSI QTANNIQAVN NALSDLKSFA SNNHTNKETS PIYNTAQAVI TSVLAFWSLY 120
AGNALSFHVT GLNDGSNSPL GRIHRDGNCT GLQQCFMSKE TYDKMKTLAE NLQKAQGNLC 180
ALSECSSNQS NGGKTSMTTA LQTAQQLMDL IEQTKVSMVW KNIVIAGVTN KPNGAGAITS 240
TGHVTDYAVF NNIKAMLPIL QQALTLSQSN HTLSTQLQAR AMGSQTNREF AKDIYALAQN 300
QKQILSNASS IFNLFNSIPK DQLKYLENAY LKVPHLGKTP TNPYRQNVNL NKEINAVQDN 360
VANYGNRLDS ALSVAKDVYN LKSNQTEIVT TYNDAKNLSE EISKLPYNQV NVTNIVMSPK 420
DSTAGQYQIN PEQQSNLNQA LAAMSNNPFK KVGMISSQNN NGALNGLGVQ VGYKQFFGES 480
KRWGLRYYGF FDYNHGYIKS SFFNSSSDIW TYGGGSDLLV NFINDSITRK NNKLSVGLFG 540
GIQLAGTTWL NSQYMNLTAF NNPYSAKVNA SNFQFLFNLG LRTNLATAKK KDSERSAQHG 600
VELGIKIPTI NTNYYSFLGT KLEYRRLYSV YLNYVFAY 638

<212> Type : PRT
<211> Length : 638
SequenceName : SEQ ID 116
SequenceDescription :
Sequence --------
<213> OrganismName : Helicobacter pylori J99
<400> PreSequenceString :

MKLKKRKVAA TLLKRLTLPL LFTTGSLGAV TYEVHGDFIN FSKVGFNRSP INPVKGIYPT 60
ETFVNLTGKL EGSVHLGRGW TVNVGGVLGG QVYDNTRYDR WAKDFTPPSY WDKTSCGTDS 120
LSLCMNATKM WQQQGPGGII DPRGIGYMYM GEWNGLFPNY YPANAYLPGH SRRYEVYKAN 180
LTYDSDRVHM VMGRFDVTEQ EQMDWIYQLF QGFYGTFKLT KNMKFLLFSS WGRGIADGQW 240
LFPIYREKPW GIHKAGIIYR PTKNLMIHPY VYLIPMVGTL PGAKIEYDTN PEFSGRGIRN 300
KTTFYVLYDY RWNNAEYGRY APARYNTWDP FLDNGKWRGL QGPGGATLYL HHHIDINNYF 360
VVGGAYLNIG NPNMNLGTWG NPVALDGIEQ WVGGIYSLGF AGIDNITDAD AFTEYVKGGG 420
KHGKFSWSVY QRFTTAPRAL EYGIGMYLDY QFSKHVKAGL KLVWLEFQIR AGYNPGTGFL 480
GPNGQPLNLN NGLFESSAFA QGPQNMGGIA KSITQDRSHL MTHISYSF 528

<212> Type : PRT
<211> Length : 528
SequenceName : SEQ ID 117
SequenceDescription :
Sequence --------
<213> OrganismName : Helicobacter pylori J99
<400> PreSequenceString :

```
MKNFSPLYCL KKLKKRHLIA LSLPLLSYAN GFKIQEQSLN GTALGSAYVA GARGADASFY    60
NPANMGFTND WGENRSEFEM TTTVINIPAF SFKVPTTNQG LYSVTSLEID KSQQNILGII   120
NTIGLGNILK ALGNTAATNG LSQAINRVQG LMNLTNQKVV TLASKPDTQI VNGWTGTTNF   180
VLPKFFYKTR THNGFTFGGS FTAPSGLGMK WNGKGGEFLH DVFIMMVELA PSMSYTINKR   240
FSVGVGLRGL YATGSFNNTV YVPLEGASVL SAEQILNLPN NVFADQVPSN MMTLLGNIGY   300
QPALNCQKAG GDMSDQSCQE FYNGLKKIMG YSGLIKASAN LYGTTQVVQK SNGQGVSGGY   360
RVGSSLRVFD HGMFSVVYNS SVTFNMKGGL VAITELGPSL GSVLTKGSLN INVSLPQTLS   420
LAYAHQFFKD RLRVEGVFER TFWSQGNKFL VTPDFANATY KGLSGTVASL DSETLKKMVG   480
LANFKSVMNM GAGWRDTNTF RLGVTYMGKS LRLMGAIDYD QAPSPQDAIG IPDSNGYTVA   540
FGTKYNFRGF DLGVAGSFTF KSNRSSLYQS PTIGQLRIFS ASLGYRW                 587
```

<212> Type : PRT
<211> Length : 587
SequenceName : SEQ ID 118
SequenceDescription :
Sequence
--------
<213> OrganismName : Helicobacter pylori J99
<400> PreSequenceString :

```
MAFQVNTNIN AMNAHVQSAL TQNALKTSLE RLSSGLRINK AADDASGMTV ADSLRSQASS    60
LGQAIANTND GMGIIQVADK AMDEQLKILD TVKVKATQAA QDGQTTESRK AIQSDIVRLI   120
QGLDNIGNTT TYNGQALLSG QFTNKEFQVG AYSNQSIKAS IGSTTSDKIG QVRIATGALI   180
TASGDISLTF KQVDGVNDVT LESVKVSSSA GTGIGVLAEV INKNSNRTGV KAYASVITTS   240
DVAVQSGSLS NLTLNGIHLG NIADIKKNDS DGRLVAAINA VTSETGVEAY TDQKGRLNLR   300
SIDGRGIEIK TDSVSNGPSA LTMVNGGQDL TKGSTNYGRL SLTRLDAKSI NVVSASDSQH   360
LGFTAIGFGE SQVAETTVNL RDVTGNFNAN VKSASGANYN AVIASGNQSL GSGVTTLRGA   420
MVVIDIAESA MKMLDKVRSD LGSVQNQMIS TVNNISITQV NVKAAESQIR DVDFAEESAN   480

FNKNNILAQS GSYAMSQANT VQQNILRLLT                                    510
```

<212> Type : PRT
<211> Length : 510
SequenceName : SEQ ID 119
SequenceDescription :
Sequence --------
<213> OrganismName : Helicobacter pylori J99
<400> PreSequenceString :

```
MAGTQAIYES SSAGFLSQVS SIISSTSGVA GPFAGIVAGA MTAAIIPIVV GFTNPQMTAI    60
MTQYNQSIAE AVSVPMKAAN QQYNQLYQGF NDQSMAVGNN ILNISKLTGE FNAQGNTQSA   120
QISAVNSQIA SILASNTTPK NPSAIEAYAT NQIAVPSVPT TVEMMSGILG NITSAAPKYA   180
LALQEQLRSQ ASNSSMNDTA DSLDSCTALG ALVGSSKVFF SCMQISMTPM SVSMPTVYAK   240
YQAVATKALT SGVNPMTTPA CPIGDKVLAV YCYAEKVAEI LREYYIEFVK NNTNLLQNAS   300
QMILNQSGLA TSTYDTQAIS NISSLYNYNI VANKSFLKSH LTYLDYIKDK LKGQKDSYLT   360
ERVQTKIIVK                                                          370
```

<212> Type : PRT
<211> Length : 370
SequenceName : SEQ ID 120
SequenceDescription :
Sequence
--------
<213> OrganismName : Helicobacter pylori J99
<400> PreSequenceString :

MTNEAINQQP QTEAAFNPQQ FINNLQVAFI KVDNVVASFD PNQKPIVDKN DRDNRQAFEK 60
ISQLREEFAN KAIKNPTKKN QYFSSFISKS NDLIDKDNLI DTGSSIKSFQ KFGTQRYQIF 120
MNWVSHQNDP SKINTQKIRG FMENIIQPPI SDDKEKAEFL RSAKQAFAGI IIGNQIRSDQ 180
KFMGVFDESL KERQEAEKNG EPNGDPTGGD WLDIFLSFVF NKKQSSDLKE TLNQEPVPHV 240
QPDVATTTTD IQSLPPEARD LLDERGNFSK FTLGDMNMLD VEGVADIDPN YKFNQLLIHN 300
NALSSVLMGS HNGIEPEKVS LLYGNNGGPE ARHDWNATVG YKNQRGDNVA TLINVHMKNG 360
SGLVIAGGEK GINNPSFYLY KEDQLTGSQR ALSQEEIQNK VDFMEFLAQN NAKLDNLSKK 420
EKEKFQNEIE DFQKDSKAYL DALGNDHIAF VSKKDKKHLA LVAEFGNGEL SYTLKDYGKK 480
ADKALDREAK TTLQGSLKHD GVMFVDYSNF KYTNASKSPD KGVGATNGVS HLEAGFSKVA 540
VFNLPNLNNL AITSVVRQDL EDKLIAKGLS PQEANKLVKD FLSSNKELVG KALNFNKAVA 600
EAKNTGNYDE VKQAQKDLEK SLKKRERLEK DVAKNLESKS GNKNKMEAKS QANSQKDEIF 660
ALINKEANRD ARAIAYAQNL KGIKRELSDK LENINKDLKD FSKSFDEFKN GKNKDFSKAE 720
ETLKALKGSV KDLGINPEWI SKVENLNAAL NEFKNGKNKD FSKVTQAKSD LENSIKDVII 780
NQKITDKVDN LNQAVSVAKA TGDFSGVEQA LADLKNFSKE QLAQQAQKNE DFNTGKNSAL 840
YQSVKNGVNG TLVGNGLSKA EATTLSKNFS DIKKELNAKL GNFNNNNNNG LENSTEPIYT 900
QVAKKVKAKI DRLDQIASGL GDVGQAASFL LKRHDKVDDL SKVGLSANHE PIYATIDDLG 960
GPFPLKRHDK VDDLSKVGLS REQKLTQKID NLNQAVSEAK ASHFDNLDQM IDKLKDSTKK 1020
NVVNLYVESA KKVPTSLSAK LDNYATNSHT RINSNVKNGT INEKATGMLT QKNSEWLKLV 1080
NDKIVAHNVG SAPLSAYDKI GFNQKNMKDY SDSFKFSTRL SNAVKDIKSG FVQFLTNIFS 1140
MGSYSLMKAS VEHGVKNTNT KGGFQKS 1167

<212> Type : PRT
<211> Length : 1167
SequenceName : SEQ ID 121
SequenceDescription :
Sequence --------
<213> OrganismName : Helicobacter pylori J99
<400> PreSequenceString :

MKTNGHFKDF AWKKCFLGAS VVALLVGCSP HIIETNEVAL KLNYHPASEK VQALDEKILL 60
LRPAFQYSDN IAKEYENKFK NQTTLKVEEI LQNQGYKVIN VDSSDKDDFS FAQKKEGYLA 120
VAMNGEIVLR PDPKRTIQKK SEPGLLFSTG LDKMEGVLIP AGFVKVTILE PMSGESLDSF 180
TMDLSELDIQ EKFLKTTHSS HSGGLVSTMV KGTDNSNDAI KSALNKIFAS IMQEMDKKLT 240
QRNLESYQKD AKELKNKRNR 260

<212> Type : PRT
<211> Length : 260
SequenceName : SEQ ID 122
SequenceDescription :
Sequence --------
<213> OrganismName : Mycoplasma pneumoniae
<400> PreSequenceString :

MKSKLKLKRY LLFLPLLPLG TLSLANTYLL QDHNTLTPYT PFTTPLNGGL DVVRAAHLHP 60
SYELVDWKRV GDTKLVALVR SALVRVKFQD TTSSDQSNTN QNALSFDTQE SQKALNGSQS 120
GSSDTSGSNS QDFASYVLIF KAAPRATWVF ERKIKLALPY VKQESQGSGD QGSNGKGSLY 180
KTLQDLLVEQ PVTPYTPNAG LARVNGVAQD TVHFGSGQES SWNSQRSQKG LKNNPGPKAV 240
TGFKLDKGRA YRKLNESWPV YEPLDSTKEG KGKDESSWKN SEKTTAENDA PLVGMVGSGA 300
AGSASSLQGN GSNSSGLKSL LRSAPVSVPP SSTSNQTLSL SNPAPVGPQA VVSQPAGGAT 360
AAVSVNRTAS DTATFSKYLN TAQALHQMGV IVPGLEKWGG NNGTGVVASR QDATSTNLPH 420
AAGASQTGLG TGSPREPALT ATSQRAVTVV AGPLRAGNSS ETDALPNVIT QLYHTSTAQL 480
AYLNGQIVVM GSDRVPSLWY WVVGEDQESG KATWWAKTEL NWGTDKQKQF VENQLGFKDD 540
SNSDSKNSNL KAQGLTQPAY LIAGLDVVAD HLVFAAFKAG AVGYDMTTDS SASTYNQALA 600
WSTTAGLDSD GGYKALVENT AGLNGPINGL FTLLDTFAYV TPVSGMKGGS QNNEEVQTTY 660
PVKSDQKATA KIASLINASP LNSYGDDGVT VFDALGLNFN FKLNEERLPS RTDQLLVYGL 720
VNESELKSAR ENAQSTSDDN SNTKVKWTNT ASHYLPVPYY YSANFPEAGN RRRAEQRNGV 780
KISTLESQAT DGFANSLLNF GTGLKAGVDP APVARGHKPN YSAVLLVRGG VVRLNFNPDT 840
DKLLDSTDKN SEPISFSYTP FGSAESAVDL TTLKDVTYIA ESGLWFYTFD NGEKPTYDGK 900
QQQVKNRKGY AVITVSRTGI EFNEDANTTT LSQAPAALAV QNGIASSQDD LTGILPLSDE 960
FSAVITKDQT WTGKVDIYKN TNGLFEKDDQ LSENVKRRDN GLVPIYNEGI VDIWGRVDFA 1020
ANSVLQARNL TDKTVDEVIN NPDILQSFFK FTPAFDNQRA MLVGEKTSDT TLTVKPKIEY 1080
LDGNFYGEDS KIAGIPLNID FPSRIFAGFA ALPSWVIPVS VGSSVGILLI LLILGLGIGI 1140
PMYKVRKLQD SSFVDVFKKV DTLTTAVGSV YKKIITQTSV IKKAPSALKA ANNAAPKAPV 1200
KPAAPTAPRP PVQPPKKA 1218

<212> Type : PRT
<211> Length : 1218
SequenceName : SEQ ID 123
SequenceDescription :
Sequence --------
<213> OrganismName : Mycoplasma pneumoniae
<400> PreSequenceString :

```
MHQTKKTALS KSTWILILTA TASLATGLTV VGHFTSTTTT LKRQQFSYTR PDEVALRHTN      60
AINPRLTPWT YRNTSFSSLP LTGENPGAWA LVRDNSAKGI TAGSGSQQTT YDPTRTEAAL     120
TASTTFALRR YDLAGRALYD LDFSKLNPQT PTRDQTGQIT FNPFGGFGLS GAAPQQWNEV     180
KNKVPVEVAQ DPSNPYRFAV LLVPRSVVYY EQLQRGLGLP QQRTESGQNT STTGAMFGLK     240
VKNAEADTAK SNEKLQGAEA TGSSTTSGSG QSTQRGGSSG DTKVKALKIE VKKKSDSEDN     300
GQLQLEKNDL ANAPIKRSEE SGQSVQLKAD DFGTALSSSG SGGNSNPGSP TPWRPWLATE     360
QIHKDLPKWS ASILILYDAP YARNRTAIDR VDHLDPKAMT ANYPPSWRTP KWNHHGLWDW     420
KARDVLLQTT GFFNPRRHPE WFDGGQTVAD NEKTGFDVDN SENTKQGFQK EADSDKSAPI     480
ALPFEAYFAN IGNLTWFGQA LLVFGGNGHV TKSAHTAPLS IGVFRVRYNA TGTSATVTGW     540
PYALLFSGMV NKQTDGLKDL PFNNNRWFEY VPRMAVAGAK FVGRELVLAG TITMGDTATV     600
PRLLYDELES NLNLVAQGQG LLREDLQLFT PYGWANRPDL PIGAWSSSSS SSHNAPYYFH     660
NNPDWQDRPI QNVVDAFIKP WEDKNGKDDA KYIYPYRYSG MWAWQVYNWS NKLTDQPLSA     720
DFVNENAYQP NSLFAAILNP ELLAALPDKV KYGKENEFAA NEYERFNQKL TVAPTQGTNW     780
SHFSPTLSRF STGFNLVGSV LDQVLDYVPW IGNGYRYGNN HRGVDDITAP QTSAGSSSGI     840
STNTSGSRSF LPTFSNIGVG LKANVQATLG GSQTMITGGS PRRTLDQANL QLWTGAGWRN     900
DKASSGQSDE NHTKFTSATG MDQQGQSGTS AGNPDSLKQD NISKSGDSLT TQDGNAIDQQ     960
EATNYTNLPP NLTPTADWPN ALSFTNKNNA QRAQLFLRGL LGSIPVLVNR SGSDSNKFQA    1020
TDQKWSYTDL HSDQTKLNLP AYGEVNGLLN PALVETYFGN TRAGGSGSNT TSSPGIGFKI    1080
PEQNNDSKAT LITPGLAWTP QDVGNLVVSG TTVSFQLGGW LVTFTDFVKP RAGYLGLQLT    1140
GLDASDATQR ALIWAPRPWA AFRGSWVNRL GRVESVWDLK GVWADQAQSD SQGSTTTATR    1200
NALPEHPNAL AFQVSVVEAS AYKPNTSSGQ TQSTNSSPYL HLVKPKKVTQ SDKLDDDLKN    1260
LLDPNQVRTK LRQSFGTDHS TQPQPQSLKT TTPVFGTSSG NLSSVLSGGG AGGGSSGSGQ    1320
SGVDLSPVEK VSGWLVGQLP STSDGNTSST NNLAPNTNTG NDVVGVGRLS ESNAAKMNDD    1380
VDGIVRTPLA ELLDGEGQTA DTGPQSVKFK SPDQIDFNRL FTHPVTDLFD PVTMLVYDQY    1440
IPLFIDIPAS VNPKMVRLKV LSFDTNEQSL GLRLEFFKPD QDTQPNNNVQ VNPNNGDFLP    1500
LLTASSQGPQ TLFSPFNQWP DYVLPLAITV PIVVIVLSVT LGLAIGIPMH KNKQALKAGF    1560
ALSNQKVDVL TKAVGSVFKE IINRTGISQA PKRLKQTSAA KPGAPRPPVP PKPGAPKPPV    1620
QPPKKPA                                                             1627
```

<212> Type : PRT
<211> Length : 1627
SequenceName : SEQ ID 124
SequenceDescription :
Sequence --------
<213> OrganismName : Mycoplasma pneumoniae
<400> PreSequenceString :

```
MGYKLKRWPL VAFTFTGIGL GVVLAACSAL NTSNLFPRQN RSKQLIGFTE NNIIKPEAVL      60
KAALAEDNGT ETILRVNFGE ALKSWYQNNK DRNIATRLTI FSENVEDEHD NLLDQKQQAE     120
PINWPIELQK EYDQWGGSES SWKALKLYDR LIADFQSLIF SNIVANVQLT DGSDQFKPTT     180
KDNLDSTSNK IKFVNSKPND PNGEFFANLQ AYLFAQWVVE ENPLPLTQAF FAYQAPKDGL     240
DSLYDQAAIG SALQLGYAFP AFREPNNGQS QGKTTFDPTP NSAQNFGDFI KAVFPEQKNG     300
QTQQSNTSSR TGLFDWQTKW NTNGAANKLL VTKSNLRGAF KGVGLATAII DQYEYLVGGS     360
KTSSLPEVKV DSNKSNQNPL DSFFMEGKDA VAIRSIVSRA KIAMTDQTPG FKVNPAFVKV     420
KQSQQNDTFY QNQRKLSGGQ SGDNNSQGKH HYLQDAVRLT SSQAMAAAST GADSSSGTNV     480
GGSSGGNSVL IPLPRSAALT HTQQQVQQTT STLQTPVYAR GDDGTYALAI DGGDYFLANN     540
KRDFTKQADI LLYRYLQAKS NNFKENGVEF SLNLLESGSL FQTWAQTGLT AKLYGALVAM     600
MGSGQGTQVK GSVQGSSRAA SVSVQTTQQN RQQSTDTQES EVVKLAKSLL KSSADLAKPF     660
TDNPTFKKAL TDIQSEYKDY LAAAGKLSEF KKDLGEVSGL QQAIIDRADK YIQLEKQAQK     720
SAIGLGQPLP YQRASDGSYP ALEKFFIPED SAADGKVKAS ESGSAALVTL KTTDSQKSTN     780
TVKQPDIKPT RENNDKKLKQ LTSDVETKAS SLITKWGATP QIGSQFSEIV SLKSKDNKPQ     840
TNMILALLSD VGIKWTKILN SFKEWFFTNT NDFKNNYDSE KKELKGNEYK DFNDLVKQTL     900
YLRSWQRLTS KEKFGYYKEL GSVKAQAAQS GMVSLSSSAA VANAVASSGM QKSGDQTLLE     960
LGKKAFESEL EASSSDGQYK YLRFLSTLMW LVKDGAKNYK RLLQQAITVG TRAFVSWTVS    1020
YDDTATASAA AAKAQVAVLK TAQATNTQSD NPFNKFVQNP DYVQGSETNW FNDKSTPIKP    1080
DSLLESESTY NFTAEPFDDK TKSQKRSTGG TTNEKHFFGF NGLTINSPQS VSTASAGLTE    1140
QIFNNFGQLV TSSDKSGALS QYKDKATLKR LIQNTNSDAE LNAFGEVLHR AVNVDTSNLG    1200
RFNSSGEPLI SFDNKKKFLV DVVDKLDDVY FNKFEGYVGQ TKVKMSDSSS SSQGTKTIRK    1260
PKPHHSPRTR VSRLWAMSFR LPTRTLTKFL LVEKLIRTVL                         1300
```

<212> Type : PRT
<211> Length : 1300
SequenceName : SEQ ID 125
SequenceDescription :
Sequence --------
<213> OrganismName : Mycoplasma pneumoniae
<400> PreSequenceString :

```
MKKLLIKPQF WFLTLGGFIS SSVILVACAT PSNSALQTVF KARSNQFFNG EQGSLQNALA   60
TALKDPEANK QFVAAPLLKA LTAWYENNQD KQVTQFFKDT KKSVDEQYNQ AVDKVVSASR  120
NKNLFVQQDL LDSAGGVRNL KSPEVVWTAH                                   150
```

<212> Type : PRT
<211> Length : 150
SequenceName : SEQ ID 126
SequenceDescription :
Sequence --------
<213> OrganismName : Mycoplasma pneumoniae
<400> PreSequenceString :

```
MQQQGETKDQ YNTFGLRLVR NSVGVSVLGL DGFVKFIKGG SGGSNGGSSS AKKIDKEEQK   60
KFLKFRAFQA KIGTFYNTNF AFSFPLNETL KGWFDKHRGL ILANALVKVT LDTKEKASKA  120
LVDAFSSYKN WLSEYTPVGL ATTMISFYFD QMKALNNKLL ERVRSLNQNV NQANPTPWLN  180
GLSAKLPYVN TNGNYEKLNN YFTFLITKVL WPKVGTEDTN VSEEKSKLKT KTEDVNKIRE  240
KILNNIDSKL KTFVQKLKPT LAPRPAYSNV ILLNINNDKV WSAGANWSLA VLLDPKKVNP  300
LSFMLLKQMF DQNSLFKKAK TLFENIQNKA KTSGSGKSGT TTNDDADALS KVIGNYYYNT  360
WAKLTDKSIY GNLKDDKFDD LFKLAFDSSI NEKSFNVDYK AVIEHYRFIY TLEWLVDKNL  420
KNFKDLLKAN LKFGEIAFIA YKNTETQNFS NPQGIFGSYF NYENETNAAK SATQIIDPNS  480
FFYKTTTKPE AKTTQSANTA VMVQNTQMNN QQTNSYGFTG LSTSSGSMLG AATQQAILDQ  540
ITKTSLQQYG SQADLKKIIG ETKNQLLLDR IANQLIALKP NTSGNSGTQK TIAAYFQTDA  600
VGNPTLDFKA KQKLLLDVLD QYKDFFGNNA QAVQRDSGKS GTGNYLTYTD GSDKITYLQF  660
SYKDIDGLSL SSSNGTSSKF ASDVVAALLL FQAAYKGTQQ LALSSINKPQ LPIGDKRIKT  720
GIDLLK                                                             726
```

<212> Type : PRT
<211> Length : 726
SequenceName : SEQ ID 127
SequenceDescription :
Sequence --------
<213> OrganismName : Mycoplasma pneumoniae
<400> PreSequenceString :

```
MKSFLRKPKF WLLLLGGLST SSIILSACAT PSNSALQAVF KPTSNQFFNG EHGTIQSALN   60
TALRDPETNK KFVAAPLLKA LEAWYENNQD KNITQFLKDT KTNVDNQYKT VVDKVVSAPR  120
NKSLFVQQDL LDSSGGSEAT WKARKLFEQL ISDFASRVFQ KNYLSYKENG KVSAGPFLYD  180
TISKNSNWQN IVFDAVNFPE TNDDFFAKIQ SEVFDQWAEY TDPTIISSVT LKYSAPN      237
```

<212> Type : PRT
<211> Length : 237
SequenceName : SEQ ID 128 SequenceDescription :
Sequence --------
<213> OrganismName : Mycoplasma pneumoniae
<400> PreSequenceString :

MINFLFNQMN ALNNKFLERA KALNQNVNQA NPTPWLNGLS AKLPYVRTNG NYEKLNNYFT 60
FLIVKYMWKK VGNEDASLSK DSSINKLKTK TEDVNKIRDK ILEDIQKKVQ EFVKNKLKPT 120
LAPRQTYSNV ILLNVNNDKV WSMGANWALA NLLDTSKINP LSFMLLKQTF DQNDLFKKAK 180
KLFEDIQSKT NGGSSGGMQG SNTSSSEGAD ALSKVIGNYY YNSWAKLTDK SIYGNPKDNK 240
FDDLFKLAFE DSINEKSFNV DYKAVIEHYR FIYTLEWLVN GNLKNFKDLL KANLKFGEIA 300
FIAYKNTETK EFSNPQGVFG SAFNYENETN EVKIAAQNLD PNNFFYKTTT KPEEVKTAQN 360
GASMMVMQQK MQSTMQDSNH YGFTGLNTST SSMLGAATQQ AILDQITKNS LQQYGSQQEL 420
KTLIEKTNNQ LLLDRIASQL SGLNPSTTGN SNNGKGKNIA TYFQLDAIGN PTLSFQQKRK 480
LLLDVLDQYK DFFGTNTQAA QRDSGKGGHG SYSTYQDGSD KITYLQFSYK DIDNLSLSDK 540
GNSKLASDVV AALLLFQAAD KGTQQLALSA IN 572

<212> Type : PRT
<211> Length : 572
SequenceName : SEQ ID 129
SequenceDescription :
Sequence --------
<213> OrganismName : Mycoplasma pneumoniae
<400> PreSequenceString :

MKKFLRKPQF WLLTLGGFLS TSVILAACAT PSNSALQTVF KARSSQFFNG EQGSLQSALT 60
TALKNPVANK QFIAAPLLKA LEAWYENNED KKITQFLKDT KSNVDSQYTT AVDKVVSASR 120
NKSLFVQQDL LDNAGGSEAT WKAQKLLEQL ISDFASRVFQ KNYLNYKKDG QVSTGPFTYD 180
ELHKEESWKN FEFSAPRFSE TNDDFFAKIQ SQVFDQWVEY TDPTLISQVN YKYSAPSQGL 240
GQIYNREKLK DKLTPSYAFP FFAEEKDIAP NQNVGNKRWK QLVKGEGAIT DNNIGQSGTN 300
SQKTGLLKYR NESNKGDFLD FPLNLSDTNE TKQLVDASNI VDQLEAANLG AALNLKLQVF 360
EQDNDELPQI KELKEDLNNT IVVDKSKDVE KASKTNALFY NDQEGKQQQS DSDPIAGALD 420
DIFAQNTSEG TNLSKLAEQV KKAAATKMEA KTAVLRTNNS KGQQNNYVVL DAAIPTFNST 480
TSKSKNNSAS NEVLVALKSG SINLRQVQQT DQNSYSPIKF RIVRNSTGVT VFGLDGGSYY 540
LKQDSTNKKS VSKQSLTLLT KSSSGNSNKV LRDLDKQKQF LKFRAFQAKT NTFYSTNFAF 600
SFPLNETLKS WFDKHRELIL ANALVNASLD QKDKASKALT EAFNPYKELI KEFAPVALAT 660
TMISFYFDQM KALNNKLLER ARNLNQNVNQ ANPTPWLNGL SAKLPYVNTN GNYEKLNNYF 720
TFLITKTLWP KVGQEETSIS EESNKLKTKT ADVDKIRDKI LENIQTKVND FVKNKLKPAL 780
APRPAYSNVI LLNVNNDKVL SSGANWSLAS LLQSDKVNPL SFMLLKQAFD NNDLFKKAQK 840
LFKDIQEKSS NNGGMQSSST TNSDADALSK VIGNYYYTTW AKLTDKSIYG NPKDNKFDEL 900
FKLAFEASID EKSFNVDYKA VIDHYRFIYT LQWLVDQKLK NFKSLLKTNL KFGEVAFIAY 960
KNTETTNFSN PQGVFGSYFN YENSASEVKE STQTLDPNNF FYKTTTKPTV QAIQQVASLA 1020
LVQKQQMQQN STDHYGFTGL STSTSSMFDA SSRDAILQQI TKTSLQQYGS KDQLKKIIQG 1080
TNNQLLLDRI AVQLSGLNPS TTNGGSGKTI ATYFQVDAVG NPTLDFQAKR KLLLDLLDQY 1140
QNYFGNGAQK SQRDSTPSGT GNYLTYQNGS DKYTYTQFTY QDIDSLSLTT TSGTNNKIAS 1200
DVVAALLLFQ AADKGTQQLA LSAINKPQLN IGDKRIESGL KLLK 1244

<212> Type : PRT
<211> Length : 1244
SequenceName : SEQ ID 130
SequenceDescription :
Sequence --------
<213> OrganismName : Mycoplasma pneumoniae
<400> PreSequenceString :

MVGSGAAGSA SSLQGNGSNS SGLKSLLRSA PVSVPPSSTS NQTLSLSNPA PVGPQAVVSQ 60
PAGGATAAVS VNRTASDTAT FSKYLNTAQA LHQMGVIVPG LEKWGGNNGT GVVASRRDAT 120
STNLPHAAGA SQTGLGTGSP REPALTATSQ RAVTVVAGPL RAGNSSETDA LPNVITQLYH 180
TSTAQLAYLN GQIVVMSSAR VPSLWYWVVG EDQESGKATW WAKTELNWGT DKQKQFVENQ 240

LGFKDDSNSD SKNSNLKTQG LTQPAYLIAG LDVVADHLVF AAFKAGAVGY DMTTDSNAST 300
YNQALVWSTT AGLDSDGGTR LW 322

<212> Type : PRT
<211> Length : 322
SequenceName : SEQ ID 131
SequenceDescription :
Sequence --------

<213> OrganismName : Mycoplasma pneumoniae
<400> PreSequenceString :

```
MPVFLKLTHT IRKVLRVARL SRLALLSLTA VIFSGCANIN LISAVGSSSV QPLLSKLSSH    60
YVLNHNDKDN LVEISVQAGG SSAGVKAITK GLADIGNVSK NTKSYAEENK QLWMDKKLKT   120
ITLGKDAIAV IYKAPSEFKG KLVLTKDNLN DLYDLFAGSK SVDINKFVEN GQTTKNSNHN   180
LIGFPRTGGA FASGTAEAFL KFSGLTQTKT LDKDSKEILE GQRNYGPNAR PTSETNIEAF   240
NTFVTTLRQP NLYGMVYLSL GFVNNNMNLI KSEGFEVLKV KYDNNAVTPS SQAVSSNTYK   300
WVRPLNSVVS LLPKQKTLPS IQRFFNWLLF SNNSEIKKIY DDFGVLELTA DEKKKMFKTG   360
NAEMSNIANF WVDDYSLNNQ TFGAL                                         385
```

<212> Type : PRT
<211> Length : 385
SequenceName : SEQ ID 132
SequenceDescription :
Sequence --------
<213> OrganismName : Mycobacterium tuberculosis H37Rv
<400> PreSequenceString :

```
MSFAVLPPEI NSARLYVGAG LAPMLDAAAA WDGLADELGS AAASFSAVTA GLAGSSWLGA     60
ASTAMTGAAA PYLGWLSAAA AQAQQAATQT RLAAAAFEAA LAATVHPAII SANRALFVSL    120
VVSNLLGQNA PAIAATEAAY EQMWAQDVAA MFGYHAGASA AVSALTPFGQ ALPTVAGGGA    180
LVSAAAAQVT TRVFRNLGLA NVGEGNVGNG NVGNFNLGSA NIGNGNIGSG NIGSSNIGFG    240
NVGPGLTAAL NNIGFGNTGS NNIGFGNTGS NNIGFGNTGD GNRGIGLTGS GLLGFGGLNS    300
GTGNIGLFNS GTGNVGIGNS GTGNWGIGNS GNSYNTGFGN SGDANTGFFN SGIANTGVGN    360
AGNYNTGSYN PGNSNTGGFN MGQYNTGYLN SGNYNTGLAN SGNVNTGAFI TGNFNNGFLW    420
RGDHQGLIFG SPGFFNSTSA PSSGFFNSGA GSASGFLNSG ANNSGFFNSS SGAIGNSGLA    480
NAGVLVSGVI NSGNTVSGLF NMSLVAITTP ALISGFFNTG SNMSGFFGGP PVFNLGLANR    540
GVVNILGNAN IGNYNILGSG NVGDFNILGS GNLGSQNILG SGNVGSFNIG SGNIGVFNVG    600
SGSLGNYNIG SGNLGIYNIG FGNVGDYNVG FGNAGDFNQG FANTGNNNIG FANTGNNNIG    660
IGLSGDNQQG FNIASGWNSG TGNSGLFNSG TNNVGIFNAG TGNVGIANSG TGNWGIGNPG    720
TDNTGILNAG SYNTGILNAG DFNTGFYNTG SYNTGGFNVG NTNTGNFNVG DTNTGSYNPG    780
DTNTGFFNPG NVNTGAFDTG DFNNGFLVAG DNQGQIAIDL SVTTPFIPIN EQMVIDVHNV    840
MTFGGNMITV TEASTVFPQT FYLSGLFFFG PVNLSASTLT VPTITLTIGG PTVTVPISIV    900
GALESRTITF LKIDPAPGIG NSTTNPSSGF FNSGTGGTSG FQNVGGGSSG VWNSGLSSAI    960
GNSGFQNLGS LQSGWANLGN SVSGFFNTST VNLSTPANVS GLNNIGTNLS GVFRGPTGTI   1020
FNAGLANLGQ LNIGSANLGD FNLGSGNVGS FNVFSGNQGS YNIGPANLGN YNIGFANLGN   1080
YNIGFGNAGD FNQGFANTGN NNIGFANTGN NNIGIGLSGD NQQGFNFAGG WNSGTANIGL   1140
FNSGTNNVGI GNSGTGNWGI GNSGSGNTGI GNTGSTNTGF FNTGIVNTGV ANAGSYNTGW   1200
YNTGDTNTGI ANLGDFNTGF YNTGNFSTGF ANQGDIATGA FITGDMGNGA FWRGDQQGLF   1260
SAGYRVHVPE IPAHVTVEVP VNIPITASFT NTVYSGITLE QINFGFTIDI AGIPLLAGAI   1320
SKAVLPPITG TGPAITVNIG DPGGSTAIRI PATASVGPFD VTFVNIAATT GFFNATTDPS   1380
SGFFNGGPGT VSGIANIGAN ISGFQNVANS ATSGFNNYGS LQSGLANLGD TVSGVFNTGI   1440
GAPANVSGMF NIGSNLAGFF HDQATGMSMF NLGLGNIGQF NVGFSNVGDS NAGLANIGSF   1500
NLGSGNLGSF NVFGGNQGSY NIGPANLGNY NIGLGNLGSY NFGFGNAGDF NLGFANTGNN   1560
NIGFANTGNN NIGIGLSGDN QQGFNFAGGW NSGSGNSGLF NSGTNNIGLF NSGTGNIGIG   1620
NSGTGNWGIA NTGDTNTGIF NTGDVNTGLL NAGNVNTGIF NTGHYNTGSF NAGSFNTAGF   1680
NPGSYNTGYL NTGSYNTGLA NSGDVNTGGF ITGNYSNGFW WRGDYQGLAG ISQTITVPDT   1740
AVPVKLHVPI FLDIPVTGTL GTFTVHGFRF PEITGDIFLI GIPFNAATLD AFSFPNISIV   1800
LPNIGINLGS GPDPLIDIAG TGGLLPIKIP LIDIPAAPGF GNSTTTPSSG FFNAGTGTVS   1860
GVGNVGSNSS GFFNLTSGSS GISGVQNFGE LISGGFNFGN TVSGLVNAST LGLSMPANLS   1920
GGGNVGATVA GFVNNTQILN LGFGNVGSGN VGHGNIGDSN VGLGNLGNAN VGHGNIGSFN   1980
VFSGNRGSYN IGPANLGNYN IGLGNLGSYN FGFGNAGDFN LGFANSGSNN IGFANTGNNN   2040
IGIGLSGHNQ QGFGSWNSGT ANTGLFNSGT NNIGLFNSGT GNIGIGNSGI GNTGIGNPGV   2100
GNTGLGNSGT GNWGLWNPGT GNMGVANVGT YNTGGYNVGS TNTGIANVGI ANTGSYNTGS   2160
TNTGSFNDGD FNTGFYNTGD YNTGFYNTGD VNTGAFIGGN FSNGAFWQSD HQGQWGAHYA   2220
ITVPQIPLLN FSLNIPVNIP IHLDFGTLAV NGFQIPAITL RALGVTHFSV GPIIVPRIAG   2280
TLPVIDINIG DPGGSSSIPI TITSGAGPVV IPLLDIPPAP GFGNSTTGPS SGFFNSGTGS   2340
SSGFGNVGAN NSGFWNTAFA GIGNSGLQNF GSLQSGWANL GNTVSGFYNT SAADFATPAN   2400
```

```
LSGLSNVGAD LTGVLRGPNG STFNAGLANL GQFNVGSANL GSANLGSANL GSANLGNSNV    2460
GFGNIGNANI GGANIGDFNV GIANTGPGLT AAVNNIGIGN TGNYNIGVGN TGNYNIGFGN    2520
TGNNNIGIGL SGDNQIGFGP LNAGIANMGL FNLGDNNFGM ANAGNFNQGI ANTGNNNIGL    2580
FNTGNNNVGI WLTGDGLSGF SSLNSGAGNT GFFNSGTANT GLFNSGTGNT GLFNSGTGNV    2640
GIGNMGTGGF GVGLSGDSQV GIGGTNSGSF NIGLFNSGTG NVGIGNSGTG NVGIGNTGTG    2700
NTGIGNSGNY NTGLLNAGLV NTGIANPGNH NTGLFNIGTF NTGIANPGHY NTGSYNTGSY    2760
NTGMANAGDY GTGAFITGSM NNGLLWRADR QGLLAANYTI TIERPAAFLN VDIPVNIPIT    2820
GDITNVSIPA ITFPRIDASG SVDIGILSGT VLAPVGPITL HGGDASAPLD TPIEIDFGPS    2880
PAINLNIGKP DGSTVINIVG GAGAGPISIP IIDLRPAPGF FNATTGPSSG FLNWGAGSAS    2940
GLLNFGNNSG LYNFATSSMG NSGFQNYGSL QSGWANLGNS ISGIYNTGLG APANVSGLLN    3000
IGTNLAGWLQ NGPTETTFSV GLANLGFWNL GSANIGNYNL GSANIGVYNL GSANIGDFNL    3060
GSANIGDFNL GSANIGSSNI GFGNVGPGLT AAIGNIGFGN TGNGNIGIGN TGTGNIGFGN    3120
TGNGNIGIGL TGDTMTGFGG WNSGTGNIGL FNSGTGNIGF GNSGTGNWGI GNSGDYNTGI    3180
GNTGSTNSGF FNTGLVNTGI GNSGDYNTGL FNAGNTNTGS FNPGDYNTGG FNPGNYNTGY    3240
FNPGNSNTGI ANSGDVNTGA FNSGNYSNGF FWRGDYQGLG GFAYQSAVSE IPWSYDRFQH    3300
```

<212> Type : PRT
<211> Length : 3300
SequenceName : SEQ ID 133
SequenceDescription :
Sequence
--------
<213> OrganismName : Mycobacterium tuberculosis H37Rv
<400> PreSequenceString :

```
MNLVSTTSGM SGFLNVGALG SGVANVGNTI SGIYNVGTSD LSTPAVNSGL ANIGTNIAGL      60
LRDGAGTAAI NLGLANHGNL NVGFASLGGF NFGGATIGHN NVGIGNTGIF DVGLANLGSY     120
NIGFGNLGDD NLGFGNFGSY NIGFGNVGND NLGFANAGGG NIGFANTGSN NVGFGNTGSN     180
NVGIGLTGNG QIGFGSFNSG SGNIGLFNSG SNNIGFFNSG SGNFGIANSG SFNTGIGNTG     240
NTNTGLFNSG DVNTGAFNPG SFNTGSFNTG SFNTGGFNPG NTNTGYLNIG NYNTGIANTG     300
DVDTGAFITG NYSNGLFLSG DYQGLVGLNL VIDMPLPISL GVNIPIDIPI TASAGNITLM     360
GVTIPPTGDI VLSSIAGQRA HFGPITIPNI TVVGPTTTVA IGGPNTAITI TGGGAIRIPL     420
ISIPAAPGFG NSTTNPSSGF FNTGAGGASG FGNFGGANSG FWNLASATSG ASGLLNVGAL     480
GSGLANVGTT VSGFYNTSTS DLATPAFNSG LANISTSIAG LLRDSTGTMV LNLGLANHGT     540
LNVGIANLGD YNIGFANLGS ANFGSANIGG NNIGGANTGI FDIGLANLGS YNIGFGNFGD     600
DNLGFGNLGS YNVGFGNLGN DNLGFANTGS NNIGFANTGS NNIGIGLTGD GQIGFGSLNS     660
GSGNIGLFNS GSGNIGFFNS GNGNVGIGNT GTANFGLGNT GSTNTGFFNS GDVNTGIGNT     720
GSFNTGSFNP GDSNTGDFNP GSYNTGLGNT GDVDTGAFIS GSYSNGFLWS GNYQGLIGLH     780
AALAIPEIAL TFGVDIPIHI PINIDAGVVT LQGFSIVAAE NNIDFTPIII PTINITLPTA     840
AITVGGPTTS IGITASAGIG SITIPIIDIP ATSGFGNSTT SPSSGFFNSG AGSASGFLNV     900
VAGASGISGY LNVGALGSGV TNVGHTVSGF YNASALDLVT PAFASGLMRD GMGTMTLNLG     960
LANLGSNNAG FGNTGIFDVG VANLGNYNIG FGNFGDDNLG FANLGSYNIG VANTGSNNIG    1020
FANTGSNNIG IGLTGTGQIG IGALNSGSGN IGLFNSGDGN IGFFNSGTGN FGIGNTGTGN    1080
FGIGNSGSTS TGLFNSGDGN TGGFNPGNFN TGNFNTGSFN TGGFNAGNTN TGHFNTGNYN    1140
TGIANTGDVS TGAFISGNYS NGILWRGDYQ GLIGYSYALT IPEIPAHLDV NIPIDIPITG    1200
SFTDLVVDNF TIPIIGFESF AFSFHIHTEP DIGPIIVPSF VLSVPTFAIA VGGPTTAINI    1260
SATAGLGPIT IPIIDIPAAP GIGNSTTSPS SGFFNTGAGT ASGFGNVGGN TSGLWNLASA    1320
ASGVSGLLNV GALGSGVANV GNTISGIYNT SPLDLGTPAF GSGLANIAGL LQGGAGTTIL    1380
DLAGLGNLNV GLANLGGSNF GIGNTGIFNV GFANVGNHNI GLANLGNYSV GFANSGNYHI    1440
GIANTGSANI GFANTGSGNI GIGLTGTGQI GFGSFNSGSH NIGLFNSGDG NVGFFNSGTG    1500
NVGIGNTGTA NFGIANSGSF NTGLGNTGST NTGLFNPGNV NTGVGNTGSI NTGSINTGSF    1560
NTGSTNTGSF NLGDHNTGSF NSGDYNTGYF NAGDYNTGVA NTGNVNTGAF ISGNYSNGFF    1620
WRGDYQGLIG LSTTITIPEI PYRYDLSVPI DIPITGTVVA TTPNSFTIPG FQIRVLLGPA    1680
AVLVNEMIGP ITIDVNQVIA IDSPIQQTIS MVGTGGFGPI PIGISIGGTP GFGNSTTGPS    1740
SGFFHTGAGH VSGFGNFGAG NMSGSGNFGA GNSGFFNAGG LGNSGLLNFG ALQSGLANLG    1800
NTISGVYNTS TLDLATPAFG SGIANIGANL AGLFLDNTGN LTLNFGVANQ GGLNAGIGNL    1860
GSVNIGFVNT GDSNLGIGNL GDLNFGGVNI GGNNIGIANT GIFDIGLANL GSYNIGLANL    1920
GDDNLGFGNA GSYNIGFANF GSDNLGFANT GSYNIGFANT GNNNIGVGLT GNGQIGIGSL    1980
NSGSNNIGLF NSGSGNIGFF NSGTGNVGIF NTGTGNFGLA NSGGFNTGIG NAGSTNTGVF    2040
NPGDLNTGSF NPGSFNTGGF NPGSGNTGYL NTGDYNTGVA NTGDVDTGAF ITGSYSNGFL    2100
VSGDYQGLIG LPLLGIPVTP GYFNLTGGPS SGFFNSGAGS VSGFVNSGAG LSGYLNTGAL    2160
GSGVANVGNT ISGWLNASAL DLATPGFLSG IGNFGTNLAG FFRG                     2204
```

<212> Type : PRT
<211> Length : 2204
SequenceName : SEQ ID 134
SequenceDescription :

Sequence --------
<213> OrganismName : Mycobacterium tuberculosis H37Rv
<400> PreSequenceString :

```
MSFVLIAPEF VTAAAGDLTN LGSSISAANA SAASATTQVL AAGADEVSAR IAALFGGFGL   60
EYQAISAQVA AYHQRFVQAL STGAGAYASA EAAAAEQIVL GVINAPTQAL LGRPLIGDGA  120
NATTPGGAGG AGGLLFGNGG AGAAGAPGQA GGPGGPAGLW GNGGPGGAGG SGGGTGGAGG  180
AGGWLFGVGG AGGVGGAGGG TGGAGGPGGL IWGGGGAGGV GGAGGGTGGA GGRAELLFGA  240
GGAGGAGTDG GPGATGGTGG HGGVGGDGGW LAPGGAGGAG GQGGAGGAGS DGGALGGTGG  300
TGGTGGAGGA GGRGALLLGA GGQGGLGGAG GQGGTGGAGG DGVLGGVGGT GGKGGVGGVA  360
GLGGAGGAAG QLFSAGGAAG AVGVGGTGGQ GGAGGAGAAG ADAPASTGLT GGTGFAGGAG  420
GVGGQGGNAI AGGINGSGGA GGTGGQGGAG GMGGSGADNA SGIGADGGAG GTGGNAGAGG  480
AGGAAGTGGT GGVVGAAGKA GIGGTGGQGG AGGAGSAGTD ATATGATGGT GFSGGAGGAG  540
GAGGNTGVGG TNGSGGQGGT GGAGGAGGAG GVGADNPTGI GGTGGTGGKG GAGGAGGQGG  600
SSGAGGTNGS GGAGGTGGQG GAGGAGGAGA DNPTGIGGAG GTGGTGGAAG AGGAGGAIGT  660
GGTGGAVGSV GNAGIGGTGG TGGVGGAGGA GAAAAAGSSA TGGAGFAGGA GEGGAGGNS   720
GVGGTNGSGG AGGAGGKGGT GGAGGSGADN PTGAGFAGGA GGTGGAAGAG GAGGATGTGG  780
TGGVVGATGS AGIGGAGGRG GDGGDGASGL GLGLSGFDGG QGGQGGAGGS AGAGGINGAG  840
GAGGNGGDGG DGATGAAGLG DNGGVGGDGG AGGAAGNGGN AGVGLTAKAG DGGAAGNGGN  900
GGAGGAGGAG DNNFNGGQGG AGGQGGQGGL GGASTTSINA NGGAGGNGGT GGKGGAGGAG  960
TLGVGGSGGT GGDGGDAGSG GGGGFGGAAG KAGGGGNGGR GGDGGDGASG LGLGLSGFDG 1020
GQGGQGGAGG SAGAGGINGA GGAGGNGGDG GDGATGAAGL GDNGGVGGDG GAGGAAGNGG 1080
NAGVGLTAKA GDGGAAGNGG NGGAGGAGGA GDNNFNGGQG GAGGQGGQGG LGGASTTSIN 1140
ANGGAGGNGG TGGKGGAGGA GTLGVGGSGG TGGDGGDAGS GGGGGFGGAA GKAGGGGNGG 1200
VGGDGGEGAS GLGLGLSGFD GGQGGQGGAG GSAGAGGING AGGAGGTGGA GGDGAPATLI 1260
GGPDGGDGGQ GGIGGDGGNA GFGAGVPGDG GDGGNAGFGA GVPGDGGIGG TGGAGGAGGA 1320
GADGDPSIDG GQGGAGGHGG QGGKGGLNST GLASAASGDG GNGGAGGAGG NGGDGDGFIG 1380
GSGGTGGTGG DAGVGGLANT GGTAGNAGIG GAGGRGGDGG AGDSGALSQD GNGFAGGQGG 1440
QGGVGGNAGA GGINGAGGTG GTGGAGGDGQ NGTTGVASEG GAGGQGGDGG QGGIGGAGGN 1500
AGFGAGVPGD GGIGGTGGAG GAGGAGADGD PSIDGGQGGA GGHGGQGGKG GLNSTGLASA 1560
ASGDGGNGGA GGAGGNGGDG DGFIGGSGGT GGTGGDAGVG GLANTGGTAG NAGIGGAGGR 1620
GGDGGAGDSG ALSQDGNGFA GGQGGQGGVG GNAGAGGING AGGTGGTGGA GGDGQNGTTG 1680
VASEGGAGGQ GGDGGQGGIG GAGGNAGFGA GVPGDGGIGG TGGAGGAGGA GADGDPSIDG 1740
GQGGAGGHGG QGGKGGLNST GLASAASGDG GNGGAGGAGG NGGAGGLGGG GGTGGTNGNG 1800
GLGGGGGNGG AGGAGGTPTG SGTEGTGGDG GDAGAGGNGG SATGVGNGGN GGDGGNGGDG 1860
GNGAPGGFGG GAGAGGLGGS GAGGGTDGDD GNGGSPGTDG S                     1901
```

<212> Type : PRT
<211> Length : 1901
SequenceName : SEQ ID 135 .
SequenceDescription :
Sequence --------
<213> OrganismName : Mycobacterium tuberculosis H37Rv
<400> PreSequenceString :

```
MSLVIVAPET VAAAALDVAR IGSSIGAANA AAAGSTTSVL AAGADEVSAA IATLFGSHAR   60
EYQAISTQVA AFHDRFAQTL SAAVGSYVSA EATNAAPLAT LEHNVLNALN APTQALLGRP  120
LIGDGAAGAP GTGGQAGGAGG ILWGNGGAGG SGAPGQVGGA GGAAGLFGTG GAGGAGGAGA 180
AGGAGGSGGW LLGNGGVGGA GGQSLLGGAT GGAGGNAGLF GVGGTGGPGG PGGPGGVGGT  240
GGAGGLGGTL YGAGGHGGAG GPGPIGGVGG HGGVGGAAGL LGVGGHGGAG GHGAEGVAGA  300
AGEDLSPHGT SGGVGGDAGD GGTGGRGGWL AGAGGAGGAG GVGGTGGAGG AGFSRALIVA  360
GDNGGDPGAG GAGGTGGAGS TIGAHGAAGA SPTSGGNGGA GGNGAHFSSG GKAGGNGGAG  420
GAGGLVGNGG AGGAGGNGAP GAPPSGGDPN GGGGGAGGAG GKGGDGGAQA GDGGAGGAGG  480
KGGNGGNGAT GATGLNGLGA GADGTDGGKG GNGGAGGGGG AGGQGGKALA ATHQDGSMGA  540
GGAGGNGGAG GMGGDGGNGA KGTFDNGGDG VGGNGGNGGS RGIGGAGGIG GAGSTAGADG  600
ARGATPTSGG NGGTGGNGAN ATVAGGAGGA GGKGGNGGLV GNGGAGGKGG DGMAGVAGSS  660
PTTAGESGTS GQNGGAGGAG GAGGRGGDFG GDGGTGGAGG NGANGANATT PGAKGGDGGH  720
GGPGAQGGNG GQGGPGGLAG NLFGQNGIQG VGGSGGKGGA GGLAGDGGNG ANGNFAFGDG  780
NGGHGGNGGN PGAGGQGGSG GAGSTPGAKG AHGFTPTSGG DGGDGGNGGN SQVVGGNGGD  840
GGNGGNGGSA GTGGNGGRGG DGAFGGMSAN ATNPGENGPN GNPGGNGGAG GAGGAGLNGG  900
NGGAGGNGGL GGFGGNGAAG ANGVAVGAPG QPGGAGGHGG AGGNGGAGGN GGQGVVSDGA  960
GGAGGAGGDG GAPGDGANGG NGQGAGAFAG GGGGRGGDGG NAGNAGAGGP GGTGSTAGKA 1020
GPAGSILHDG GNGGHGGHGA ASGGNGGPGG HGGNGGNGGT GANGGNGGIG GTGGAGSTGA 1080
KGVLGTNEGD GGDGGRGGNG GRGGNGGQGL TGAGGNGGTG GTPGNGGNGG NGASGDLVTS 1140
PGDGGGGGRG GDAGRGGDAG LGGSSGPGGT PGDWGTGGTG GTGGTGGQGA NGGLTGGRGG 1200
TGGNGGNGNT GGTGGAGGTG GTGHNGSQPG MGGNGGAGGF GGNGFAGVGG RGGMGGSGGT 1260
GGTGDAGPFG TGTGGTGGHG GQGGGGGFSI LLGLGGLGGL GSPGSIATGT AGGAGGGGGF 1320
```

GGLGGGEFV 1329

<212> Type : PRT
<211> Length : 1329
SequenceName : SEQ ID 136
SequenceDescription :
Sequence --------
<213> OrganismName : Mycobacterium tuberculosis H37Rv
<400> PreSequenceString :

```
MSYVIATPEM MATAAFDLAR IGSQVSAASA VAAMPTTEVV AAGADEVSAG IAALFSAHAQ      60
EYQALSAQAA AFHDQFVHTL TAAARWYTAT EIANAAAMRV VLGAVNAPTQ TLLGRPLIGD     120
GAHGTAPGQP GGAGGLLFGN GGNGAAGAVG QVGGAGGAAG LFGIGGAGGA GGAGAPGGTG     180
GTGGWLAGGG GVGGMGGAGG GAGGAGGNAG LFGNGGAGGA GGAGGGAGGA GGNAGWFGHG     240
GAGGVGGVGA AGANGATPGQ DGAAGVAGSD DGAGGDGLAG SDGGDGGAGG VGGNGGRGGW     300
LLGNGGAGGV GGVGGAGGAG AAGGAGGAGA TGINGPAGIS AAGGDGGAGG NGGAGGNGGV     360
GGAGGAGGSA GLLGYVGRAG DGGAGGGGGL GGAPGDGGAG GNGGSWLAAG DGGAGGHGGD     420
PGLGGAGGAG GASGGAGARA GANGLAAGND GPVSGGNGGK GGNGAHAPVA GGHGGNGGAG     480
GNGGLVGDGG AGGHGGDGAA GAGYADMTAI FLGSSGTPGE DGGNGGAGGA GGAGGAHAGD     540
GGAGGAGGNG GAGGAGGNGA HGFNAVLVSD GGNGGDGGAG GRGGDGGAGG AGGDAPAGRA     600
GSQGVGGDGG AGGAGGAPGN GGSGGRGDMA FKDGDGGAGG DGGDPGAGGK GGAGGAGATE     660
GVTGATGATV HSGGNGGKGG NGADATVAGA NGGKGGAGGN GGLVGDGGAG GDGGSGAAGA     720
NGANVGEDGA DGTLSGQPGE GSEANGGQGG VGGGGAGGAG GDGGAGSSAL GSGGNGGRGD     780
AGQAGGAGGA GGAGGAGGSV SGDGGPGGKG GAGGAGGAGA SGGGGGKGAS GADSAEAVGG     840
AGGKGGDGGV GGVGGDGGPG GDGGAGGAAP AGQVGSHGVG GVGGDGGLGG AGGNGGDGGH     900
GSDGGDGGDG GDPGAGGLGG LGGDSGNGTR AASGVDASDH GPGSGGNGGN GGNGAQASVA     960
GGAGGNGGDG GNAGRVGDGG AGGNGGDGAA GANGANSGAP GSDALALGQP GGNGGQGDAG    1020
QAGGAGGAGG AGGAGGSVSG DGGAGGNGGA GGNGGVGASG GAGARGANGI DSIGGTGGAG    1080
GGGGDGGAGG VGGHGGDGGV GGAAPSGTVG SHGTGGVGGD GGLGGAGGVG GAGGNGGIGI    1140
TVGGAGGAGG NGGDPGAGGR GGLGGDSGNG TSAANGVDAS KHGPLTGGDG GVGGNGAKAA    1200
AAGGDGGQGG DGGNAGLFGD GGAGGDGADG TAAEALGGDG GAGGAGGKGG DAGDIGDGGD    1260
GGKGGDGAHG ALGGLTVAGG NGGAGGAGGA GGAGGAFLGD GGNGGAGGQG GAGRGGSPGG    1320
GGGVGGHGGA GGDAGMNGGG GTGGQGGNGA AGGAGWSPDS DLKGFDGFDG GSGGAGGDGG    1380
AGGAGGTQTG DGGDGGAGGL GGAGGVGGNG VDGFDINETT GRDGGDGGDG GYGGWGGAGG    1440
NGGAGGSAPA GEVGNRGVGG DGGDGGSGGD AGNGGLGGDG FTYLADFDGE PGGDGGDGGD    1500
GGWGRPGGQG GFGSTSGAHG KAGFGAPGGD GGDGGNGGHG GDGNGSFADA GDGGPGGNGG    1560
NGGLGGAGRD GGAPGGDGGD GGTGGSGGFG APPPRSIGGG DGGDGGRGGD GGRGAGGLTS    1620
GGVGSSGESG GSGNGRGDPG SGGSGGEGGE GGPSISVNVT                          1660
```

<212> Type : PRT
<211> Length : 1660
SequenceName : SEQ ID 137
SequenceDescription :
Sequence --------
<213> OrganismName : Mycobacterium tuberculosis H37Rv
<400> PreSequenceString :

```
MSFVLVSPET VAAVATDLKR IGASLAHENA SAAASTTAVV SAAADEVSTA VAALFSQHAQ      60
GYQAAAAQVA AFHSRFVQAL TAGAGAYAFA EAANASPLQS AMGAVSASAQ TLLSRPLIGN     120
GANATTPGGN GGDGGWLFGS GGNGAPGAAG QSGGNGGSAG LWGNGGAGGA GGSGGAAGGN     180
GGNGGWLFGA GGTGGIGGTG APGAMGGTGG NGGNGALLIG GGGLGGAGGM GGTGGGTGGT     240
GGNGGNGALL IGAGGVGGAG GIGGQGTGAG GAAGAGGTGG NGGAGGLFMN GGDGGAGGQG     300
GDGAAGDAAA SAGGTGGKGG QGGDGGTGGA GGAGPVLFGH GGAGGMGGQG GTGGMGGAGG     360
DGTTVIAAGT GGEGGTGGAA GAGGAAGARG ALTSGGLAGG VGAGGTGGTG GTGGNGADAA     420
AVVGFGANGD PGFAGGKGGN GGIGGAAVTG GVAGDGGTGG KGGTGGAGGA GNDAGSTGNP     480
GGKGGDGGIG GAGGAGGAAG TGNGGHAGNT GDGGDGGTGG NGGNGTGGVN GADNTLNPDT     540
PGGAGEPGGA GGAGGAGGAA GGPGGTGGTG GNGGNGGNGG NGGNGGNGGN GGNAGNNSTN     600
APVGGEGGAG GDGGAGGAGG AANGGTAGSQ GTGGVGGDGG AGGNGGGGKA GTGNSGNFGV     660
DGEAGFSGGA GGNGGVGGAA GANGGTGGSG GNGGDGGAGG IGGAGGNGIP GTGTEPAGGT     720
GAKGGDGGDG GAGGAGGNAG GAGGQGGNAG QGGAGGAGGN AVIPGDGVGK APHGDAGGSG     780
GDGGKGGQGG SGGTGGSGAP IGGGAGGTGG SGGHAGKGGA GGIGAQGTTI TVPGNGGNAG     840
DGGNGGNAGA GGNGGSGDFG GNTTSGASGS GGNGGNAGTA GSGGAGGTGG TGLSGGNGGN     900
GGNGGNGGDG GNGAHGTVGA QFVPATSLPT PNGGAGGNGG TGSNGGAPGP AGAPGPTTGG     960
NAGSQGIGGD GGNGGDGGKG GDGADAVNVV FMPTEPQAAT GTAGSAGDPT GGNGGPGTPG    1020
SPMVAPPPPT PITQVQQGGD GGAGGTGSTN ANDGTATGGK GGEGGVGSIL GGPGGNGGTG    1080
GNASATGTNG VANAGNGGKG GDGGQFGAGG NGGAGGSVTD GSAGSTAGNG GNGGNATNGT    1140
IAGQPAGGNG SAGGKGGDGG NIAAGATGTA GNGGNGGNGN DGAVNAGTGG SGGNGGNAGG    1200


GGANGGDGGA GGAGGAGGRG GKGIDGGFGG DGGNGGSNNG TGAGGNGGNG GTGGVGSVGA    1260
AGGDGGNGGT GGFAGFGGTA GNGGSGGTGG AGGDGGTGGD GGNGVIAGGG GTGGNGGASG    1320
AGGAGGTGGF AGNGNAGGNG GTGGASEDGD NGNAGSGATG GTGGNGGTGG DGGAAGLGGV    1380
A                                                                   1381
```

<212> Type : PRT
<211> Length : 1381
SequenceName : SEQ ID 138
SequenceDescription :
Sequence --------
<213> OrganismName : Mycobacterium tuberculosis H37Rv
<400> PreSequenceString :

```
MSFVIATPEM LTTAATDLAK IGSTITAANT AAAAVAKVLP ASADEVSVAV AALFGTHAQE      60
YQTVSAQVAT FHDRFVQTLS AAASSYVAAE AVNVEQSLLA AVNAPTQALF GRPLIGNGAD     120
GSPGTGQAGG PGGILYGNGG NGGSGAPGQR GGAGGAAGLI GNGGNGGAGG VGTTGGAGGH     180
GGAGGWLYGN GGAGGFGGAG AVGGNGGAGG TAGLFGVGGA GGAGGNGIAG VTGTSASTPG     240
GSGTAGGAGG IGGNGGAGGA GGVLMGNGGN GGAGGEGGPG GAGGAGASGA HATNLGADGQ     300
AGGNGGNGGA GGTGGVGGPG GGHGLLGLGG SHGAGGAGGS GGDGGAPGDG GNGATGTWGH     360
NLGAGGTGGN GGNPGAGGAG GAGGASVGGS AHGANGAPGT TSTSGGNGGD GGKGADAISS     420
GQTGANGGRG GDGGQVGNGG AGGAGGRGGA GGLGFGSEAP GRPGGAGGTG GAGGNGGTQA     480
GDGGTGGAGG AGGDGGSGGA GSIGFNASAP GAAGSPGGNG GNGGPGGAGG EGGAGGLALA     540
ASGQNGSQGA GGDGGAGGNG GTPGNGGHGA AGALGVNGGV GGAGGHGGDP GVGGAGGQGG     600
SGSTPGANGA PGNTPTSGGN GGNGGRGADA TGFGQTGASG GRGGDGGLVG NGGAGGAGGN     660
GSKGLPGLGR LGNPGLDGGT GGNGGAGGSG GAWAGNGGTG GAGGTGGVGG TGGSGSDGVN     720
GSSAGADGHP GGTGGVGGTG GKGGDGGDGG AAPNGVAGSQ GPGGAGGDGG TGGVGGNGGR     780
GIDGADGATA GARGQDGGAG GAGGKGGRGG TGGPGGAGPA GTTGSQGAGG NGGSGGTGGD     840
PGDGGNGANG SVFTNNGIGG NGGNGGNAGP SGAGGSGGAG STFGATGSSS SIHVNGGNGG     900
NGGNGDHALS GNGAAGGNGG NGGNGSLRGS GGAGGHGGNG GNASRGMGGD GGTGGAGGNA     960
GQIGNGGAGG NGGDGGTGSD GNPGAITGSG GRGGDGGVGG QGGSVAGDGA DGGRGGAGGT    1020
GGTGLRGTTG ATGATGTFDA GADGHGGNGG TGGVGGTGGA GGGGGNGGAG GKALSPTGNN    1080
GSQGAGGDGG AGGAGGTGGT GGDGGRGAHG TLFSSLAGTG GTGGNGGTGG TGGTGGAGGA    1140
GGTGSTLGAT GATGAAGRAG NGGVGGSGGL GSAFGPGGTG GMGGAGGTST VSAGGDGGRG    1200
GFGGDGLDAS SGGNGGDGGH GGDGFRTAGA GGRGGDGGKG ADPGGLFPIP GAGGKGGTGG    1260
TGGTAHLGPL AIIGQSGQPG QFGSPGADGR GGAGGAGGGG GAGGSF                  1306
```

<212> Type : PRT
<211> Length : 1306
SequenceName : SEQ ID 139
SequenceDescription :
Sequence --------
<213> OrganismName : Mycobacterium tuberculosis H37Rv
<400> PreSequenceString :

```
MSAAAVAWDQ LAMELASAAA SFNSVTSGLV GESWLGPSSA AMAAAVAPYL GWLAAAAAQA     60
QRSATQAAAL VAEFEAVRAA MVQPALVAAN RSDLVSLVFS NFFGQNAPAI AAIEAAYEQM    120
WAIDVSVMSA YHAGASAVAS ALTPFTAPPQ NLTDLPAQLA AAPAAVVTAA ITSSKGVLAN    180
LSLGLANSGF GQMGAANLGI LNLGSLNPGG NNFGLGNVGS NNVGLGNTGN GNIGFGNTGN    240
GNIGFGLTGD NQQGFGGWNS GTGNIGLFNS GTGNIGIGNT GTGNFGIGNS GTSYNTGIGN    300
TGQANTGFFN AGIANTGIGN TGNYNTGSFN LGSFNTGDFN TGSSNTGFFN PGNLNTGVGN    360
TGNVNTGGFN SGNYSNGFFW RGDYQGLIGF SGTLTIPAAG LDLNGLGSVG PITIPSITIP    420
EIGLGINSSG ALVGPINVPP ITVPAIGLGI NSTGALVGPI NIPPITLNSI GLELSAFQVI    480
NVGSISIPAS PLAIGLFGVN PTVGSIGPGS ISIQLGTPEI PAIPPFFPGF PPDYVTVSGQ    540
IGPITFLSGG YSLPAIPLGI DVGGGLGPFT VFPDGYSLPA IPLGIDVGGG LGPFTVFPDG    600
YSLPAIPLGI DVGGGLGPFT VFPDGYSLPA IPLGIDVGGA IGPLTTPPIT IPSIPLGIDV    660
SGSLGPINIP IEIAGTPGFG NSTTTPSSGF FNSGTGGTSG FGNVGSGGSG FWNIAGNLGN    720
SGFLNVGPLT SGILNFGNTV SGLYNTSTLG LATSAFHSGV GNTDSQLAGF MRNAAGGTLF    780
NFGFANDGTL NLGNANLGDY NVGSGNVGSY NFGSGNIGNG SFGFGNIGSN NFGFGNVGSN    840
NLGFANTGPG LTEALHNIGF GNIGGNNYGF ANIGNGNIGF GNTGTGNIGI GLTGDNQVGF    900
GALNSGSGNI GFFNSGNGNI GFFNSGNGNV GIGNSGNYNT GLGNVGNANT GLFNTGNVNT    960
GIGNAGSYNT GSYNAGDTNT GDLNPGNANT GYLNLGDLNT GWGNIGDLNT GALISGSYSN   1020
GILWRGDYQG LIGYSDTLSI PAIPLSVEVN GGIGPIVVPD ITIPGIPLSL NALGGVGPIV   1080
VPDITIPGIP LSLNALGGVG PIVVPDITIP GIPLSLNALG GVGPIVVPDI TIPGIPLSLN   1140
ALGGVGPIVV PDITIPGIPL SLNALGGVGP ITVPGVPISR IPLTINIRIP VNITLNELPF   1200
NVAGIFTGYI GPIPLSTFVL GVTLAGGTLE SGIQGFSVNP FGLNIPLSGA TNAVTIPGFA   1260
INPFGLNVPL SGGTSPVTIP GFAINPFGLN VPLSGGTSPV TIPGFTIPGS PLNLTANGGL   1320
GPINIPINIT SAPGFGNSTT TPSSGFFNSG DGSASGFGNV GPGISGLWNQ VPNALQGGVS   1380

GIYNVGQLAS GVANLGNTVS GFNNTSTVGH LTAAFNSGVN NIGQMLLGFF SPGAGP        1436
```

<212> Type : PRT
<211> Length : 1436
SequenceName : SEQ ID 140
SequenceDescription :
Sequence --------
<213> OrganismName : Mycobacterium tuberculosis H37Rv
<400> PreSequenceString :

MEFPVLPPEI NSVLMYSGAG SSPLLAAAAA WDGLAEELGS AAVSFGQVTS GLTAGVWQGA 60
AAAAMAAAAA PYAGWLGSVA AAAEAVAGQA RVVVGVFEAA LAATVDPALV AANRARLVAL 120
AVSNLLGQNT PAIAAAEAEY ELMWAADVAA MAGYHSGASA AAAALPAFSP PAQALGGGVG 180
AFLTALFASP AKALSLNAGL GNVGNYNVGL GNVGVFNLGA GNVGGQNLGF GNAGGTNVGF 240
GNLGNGNVGF GNSGLGAGLA GLGNIGLGNA GSSNYGFANL GVGNIGFGNT GTNNVGVGLT 300
GNHLTGIGGL NSGTGNIGLF NSGTGNVGFF NSGTGNFGVF NSGNYNTGVG NAGTASTGLF 360
NAGNFNTGVV NVGSYNTGSF NAGDTNTGGF NPGGVNTGWL NTGNTNTGIA NSGNVNTGAF 420
ISGNFNNGVL WVGDYQGLFG VSAGSSIPAI PIGLVLNGDI GPITIQPIPI LPTIPLSIHQ 480
TVNLGPLVVP DIVIPAFGGG IGIPINIGPL TITPITLFAQ QTFVNQLPFP TFSLGKITIP 540
QIQTFDSNGQ LVSFIGPIVI DTTIPGPTNP QIDLTIRWDT PPITLFPNGI SAPDNPLGLL 600
VSVSISNPGF TIPGFSVPAQ PLPLSIDIEG QIDGFSTPPI TIDRIPLTVG GGVTIGPITI 660
QGLHIPAAPG VGNTTTAPSS GFFNSGAGGV SGFGNVGAGS SGWWNQAPSA LLGAGSGVGN 720
VGTLGSGVLN LGSGISGFYN TSVLPFGTPA AVSGIGNLGQ QLSGVSAAGT TLRSMLAGNL 780
GLANVGNFNT GFGNVGDVNL GAANIGGHNL GLGNVGDGNL GLGNIGHGNL GFANLGLTAG 840
AAGVGNVGFG NAGINNYGLA NMGVGNIGFA NTGTGNIGIG LVGDHRTGIG GLNSGIGNIG 900
LFNSGTGNVG FFNSGTGNFG IGNSGRFNTG IGNSGTASTG LFNAGSFSTG IANTGDYNTG 960
SFNAGDTNTG GFNPGGINTG WFNTGHANTG LANAGTFGTG AFMTGDYSNG LLWRGGYEGL 1020
VGVRVGPTIS QFPVTVHAIG GVGPLHVAPV PVPAVHVEIT DATVGLGPFT VPPISIPSLP 1080
IASITGSVDL AANTISPIRA LDPLAGSIGL FLEPFRLSDP FITIDAFQVV AGVLFLENII 1140
VPGLTVSGQI LVTPTPIPLT LNLDTTPWTL FPNGFTIPAQ TPVTVGMEVA NDGFTFFPGG 1200
LTFPRASAGV TGLSVGLDAF TLLPDGFTLD TVPATFDGTI LIGDIPIPII DVPAVPGFGN 1260
TTTAPSSGFF NTGGGGGSGF ANVGAGTSGW WNQGHDVLAG AGSGVANAGT LSSGVLNVGS 1320
GISGWYNTST LGAGTPAVVS GIGNLGQQLS GFLANGTVLN RSPIVNIGWA DVGAFNTGLG 1380
NVGDLNWGAA NIGAQNLGLG NLGSGNVGFG NIGAGNVGFA NSGPAVGLAG LGNVGLSNAG 1440
SNNWGLANLG VGNIGLANTG TGNIGIGLVG DYQTGIGGLN SGSGNIGLFN SGTGNVGFFN 1500
TGTGNFGLFN SGSFNTGIGN SGTGSTGLFN AGNFNTGIAN PGSYNTGSFN VGDTNTGGFN 1560
PGDINTGWFN TGIMNTGTRN TGALMSGTDS NGMLWRGDHE GLFGLSYGIT IPQFPIRITT 1620
TGGIGPIVIP DTTILPPLHL QITGDADYSF TVPDIPIPAI HIGINGVVTV GFTAPEATLL 1680
SALKNNGSFI SFGPITLSNI DIPPMDFTLG LPVLGPITGQ LGPIHLEPIV VAGIGVPLEI 1740
EPIPLDAISL SESIPIRIPV DIPASVIDGI SMSEVVPIDA SVDIPAVTIT GTTISAIPLG 1800
FDIRTSAGPL NIPIIDIPAA PGFGNSTQMP SSGFFNTGAG GGSGIGNLGA GVSGLLNQAG 1860
AGSLVGTLSG LGNAGTLASG VLNSGTAISG LFNVSTLDAT TPAVISGFSN LGDHMSGVSI 1920
DGLIAILTFP PAESVFDQII DAAIAELQHL DIGNALALGN VGGVNLGLAN VGEFNLGAGN 1980
VGNINVGAGN LGGSNLGLGN VGTGNLGFGN IGAGNFGFGN AGLTAGAGGL GNVGLGNAGS 2040
GSWGLANVGV GNIGLANTGT GNIGIGLTGD YRTGIGGLNS GTGNLGLFNS GTGNIGFFNT 2100
GTGNFGLFNS GSYSTGVGNA GTASTGLFNA GNFNTGLANA GSYNTGSLNV GSFNTGGVNP 2160
GTVNTGWFNT GHTNTGLFNT GNVNTGAFNS GSFNNGALWT GDYHGLVGFS FSIDIAGSTL 2220
LDLNETLNLG PIHIEQIDIP GMSLFDVHEI VEIGPFTIPQ VDVPAIPLEI HESIHMDPIV 2280
LVPATTIPAQ TRTIPLDIPA SPGSTMTLPL ISMRFEGEDW ILGSTAAIPN FGDPFPAPTQ 2340
GITIHTGPGP GTTGELKISI PGFEIPQIAT TRFLLDVNIS GGLPAFTLFA GGLTIPTNAI 2400
PLTIDASGAL DPITIFPGGY TIDPLPLHLA LNLTVPDSSI PIIDVPPTPG FGNTTATPSS 2460
GFFNSGAGGV SGFGNVGSNL SGWWNQAASA LAGSGSGVLN VGTLGSGVLN VGSGVSGIYN 2520
TSVLPLGTPA VLSGLGNVGH QLSGVSAAGT ALNQIPILNI GLADVGNFNV GFGNVGDVNL 2580
GAANLGAQNL GLGNVGTGNL GFANVGHGNI GFGNSGLTAG AAGLGNTGFG NAGSANYGFA 2640
NQGVRNIGLA NTGTGNIGIG LVGDNLTGIG GLNSGAGNIG LFNSGTGNIG FFNSGTGNFG 2700
IGNSGSFNTG IGNSGTGSTG LFNAGSFNTG VANAGSYNTG SFNAGDTNTG GFNPGTINTG 2760
WFNTGHTNTG IANSGNVGTG AFMSGNFSNG LLWRGDHEGL FSLFYSLDVP RITIVDAHLD 2820
GGFGPVVLPP IPVPAVNAHL TGNVAMGAFT IPQIDIPALT PNITGSAAFR IVVGSVRIPP 2880
VSVIVEQIIN ASVGAEMRID PFEMWTQGTN GLGITFYSFG SADGSPYATG PLVFGAGTSD 2940
GSHLTISASS GAFTTPQLET GPITLGFQVP GSVNAITLFP GGLTFPATSL LNLDVTAGAG 3000
GVDIPAITWP EIAASADGSV YVLASSIPLI NIPPTPGIGN STITPSSGFF NAGAGGGSGF 3060
GNFGAGTSGW WNQAHTALAG AGSGFANVGT LHSGVLNLGS GVSGIYNTST LGVGTPALVS 3120
GLGNVGHQLS GLLSGGSAVN PVTVLNIGLA NVGSHNAGFG NVGEVNLGAA NLGAHNLGFG 3180
NIGAGNLGFG NIGHGNVGVG NSGLTAGVPG LGNVGLGNAG GNNWGLANVG VGNIGLANTG 3240
TGNIGIGLTG DYQTGIGGLN SGAGNLGLFN SGAGNVGFFN TGTGNFGLFN SGSFNTGVGN 3300
SGTGSTGLFN AGSFNTGVAN AGSYNTGSFN VGDTNTGGFN PGSINTGWLN AGNANTGVAN 3360

AGNVNTGAFV TGNFSNGILW RGDYQGLAGF AVGYTLPLFP AVGADVSGGI GPITVLPPIH 3420
IPPIPVGFAA VGGIGPIAIP DISVPSIHLG LDPAVHVGSI TVNPITVRTP PVLVSYSQGA 3480
VTSTSGPTSE IWVKPSFFPG IRIAPSSGGG ATSTQGAYFV GPISIPSGTV TFPGFTIPLD 3540
PIDIGLPVSL TIPGFTIPGG TLIPTLPLGL ALSNGIPPVD IPAIVLDRIL LDLHADTTIG 3600
PINVPIAGFG GAPGFGNSTT LPSSGFFNTG AGGGSGFSNT GAGMSGLLNA MSDPLLGSAS 3660
GFANFGTQLS GILNRGAGIS GVYNTGALGV VTAAVVSGFG NVGQQLSGLL FTGVGP 3716

<212> Type : PRT
<211> Length : 3716
SequenceName : SEQ ID 141
SequenceDescription :

Sequence
<213> OrganismName : Mycobacterium tuberculosis H37Rv
<400> PreSequenceString :

```
MNFPVLPPEI NSVLMYSGAG SSPLLAAAAA WDGLAEELGS AAVSFGQVTS GLTAGVWQGA    60
AAAAMAAAAA PYAGWLGSVA AQAVAVAGQA RAAVAAFEAA LAATVDPAAV AVNRMAMRAL   120
AMSNLLGQNA AAIAAVEAEY ELMWAADVAA MAGYHSGASA AAAALPAFSP PAQALGGGVG   180
AFLNALFAGP AKMLRLNAGL GNVGNYNVGL GNVGIFNLGA ANVGAQNLGA ANAGSGNFGF   240
GNIGNANFGF GNSGLGLPPG MGNIGLGNAG SSNYGLANLG VGNIGFANTG SNNIGIGLTG   300
DNLTGIGGLN SGTGNLGLFN SGTGNIGFFN SGTGNFGVFN SGSYNTGVGN AGTASTGLFN   360
VGGFNTGVAN VGSYNTGSFN AGNTNTGGFN PGNVNTGWLN TGNTNTGIAN SGNVNTGAFI   420
SGNFSNGVLW RGDYEGLWGL SGGSTIPAIP IGLELNGGVG PITVLPIQIL PTIPLNIHQT   480
FSLGPLVVPD IVIPAFGGGT AIPISVGPIT ISPITLFPAQ NFNTTFPVGP FFGLGVVNIS   540
GIEIKDLAGN VTLQLGNLNI DTRINQSFPV TVNWSTPAVT IFPNGISIPN NPLALLASAS   600
IGTLGFTIPG FTIPAAPLPL TIDIDGQIDG FSTPPITIDR IPLNLGASVT VGPILINGVN   660
IPATPGFGNT TTAPSSGFFN SGDGGVSGFG NFGAGSSGWW NQAQTEVAGA GSGFANFGSL   720
GSGVLNFGSG VSGLYNTGGL PPGTPAVVSG IGNVGEQLSG LSSAGTALNQ SLIINLGLAD   780
VGSVNVGFGN VGDFNLGAAN IGDLNVGLGN VGGGNVGFGN IGDANFGLGN AGLAAGLAGV   840
GNIGLGNAGS GNVGFGNMGV GNIGFGNTGT NNLGIGLTGD NQTGIGGLNS GAGNIGLFNS   900
GTGNVGLFNS GTGNFGLFNS GSFNTGIGNG GTGSTGLFNA GNFNTGVANP GSYNTGSFNV   960
GDTNTGGFNP GSINTGWFNT GNANTGVANS GNVDTGALMS GNFSNGILWR GNFEGLFGLN  1020
VGITIPEFPI HWTSTGGIGP IIIPDTTILP PIHLGLTGQA NYGFAVPDIP IPAIHIDFDG  1080
AADAGFTAPA TTLLSALGIT GQFRFGPITV SNVQLNPFNV NLKLQFLHDA FPNEFPDPTI  1140
SVQIQVAIPL TSATLGGLAL PLQQTIDAIE LPAISFSQSI PIDIPPIDIP ASTINGISMS  1200
EVVPIDVSVD IPAVTITGTR IDPIPLNFDV LSSAGPINIS IIDIPALPGF GNSTELPSSG  1260
FFNTGGGGGS GIANFGAGVS GLLNQASSPM VGTLSGLGNA GSLASGVLNS GVDISGMFNV  1320
STLGSAPAVI SGFGNLGNHV SGVSIDGLLA MLTSGGSGGS GQPSIIDAAI AELRHLNPLN  1380
IVNLGNVGSY NLGFANVGDV NLGAGNLGNL NLGGGNLGGQ NLGLGNLGDG NVGFGNLGHG  1440
NVGFGNSGLG ALPGIGNIGL GNAGSNNVGF GNMGLGNIGF GNTGTNNLGI GLTGDNQTGF  1500
GGLNSGAGNL GLFNSGTGNI GFFNTGTGNW GLFNSGSYNT GIGNSGTGST GLFNAGSFNT  1560
GLANAGSYNT GSLNAGNTNT GGFNPGNVNT GWFNAGHTNT GGFNTGNVNT GAFNSGSFNN  1620
GALWTGDHHG LVGFSYSIEI TGSTLVDINE TLNLGPVHID QIDIPGMSLF DIHELVNIGP  1680
FRIEPIDVPA VVLDIHETMV IPPIVFLPSM TIGGQTYTIP LDTPPAPAPP PFRLPLLFVN  1740
ALGDNWIVGA SNSTGMSGGF VTAPTQGILI HTGPSSATTG SLALTLPTVT IPTITTSPIP  1800
LKIDVSGGLP AFTLFPGGLN IPQNAIPLTI DASGVLDPIT IFPGGFTIDP LPLSLALNIS  1860
VPDSSVPIII VPPTPGFGNA TATPSSGFFN SGAGGVSGFG NFGAGSSGWW NQAHAALAGA  1920
GSGVLNVGTL NSGVLNVGSG ISGLYNTAIV GLGTPALVSG AGNVGQQLSG VLAAGTALTQ  1980
SPIINLGLAD VGNYNLGLGN VGDFNLGAAN LGDLNLGLGN IGNANVGFGN IGHGNVGFGN  2040
SGLGAALGIG NIGLGNAGST NVGLANMGVG NIGFANTGTN NLGIGLTGDN QTGIGGLNSG  2100
AGNIGLFNSG TGNIGFFNSG TGNWGLFNSG SFNTGIGNSG TGSTGLFNAG GFTTGLANAG  2160
SYNTGSFNVG DTNTGGFNPG SINTGWFNTG NANTGIANSG NVDTGALMSG NFSNGILWRG  2220
NYEGLFSYSY SLDVPRITIL DAHFTGAFGP VVVPPIPVLA INAHLTGNAA MGAFTIPQID  2280
IPALNPNVTG SVGFGPIAVP SVTIPALTAA RAVLDMAASV GATSEIEPFI VWTSSGAIGP  2340
TWYSVGRIYN AGDLFVGGNI ISGIPTLSTT GPVHAVFNAA SQAFNTPALN IHQIPLGFQV  2400
PGSIDAITLF PGGLTFPANS LLNLDVFVGT PGATIPAITF PEIPANADGE LYVIAGDIPL  2460
INIPPTPGIG NTTTVPSSGF FNTGAGGGSG FGNFGANMSG WWNQAHTALA GAGSGIANVG  2520
TLHSGVLNLG SGLSGIYNTS TLPLGTPALV SGLGNVGDHL SGLLASNVGQ NPITIVNIGL  2580
ANVGNGNVGL GNIGNLNLGA ANIGDVNLGF GNIGDVNLGF GNIGGGNVGF GNIGDANFGF  2640
GNSGLAAGLA GMGNIGLGNA GSGNVGWANM GLGNIGFGNT GTNNLGIGLT GDNQSGIGGL  2700
NSGTGNIGLF NSGTGNIGFF NSGTANFGLF NSGSYNTGIG NSGVASTGLV NAGGFNTGVA  2760
NAGSYNTGSF NAGDTNTGGF NPGSTNTGWF NTGNANTGVA NAGNVNTGAL ITGNFSNGIL  2820
WRGNYEGLAG FSFGYPIPLF PAVGADVTGD IGPATIIPPI HIPSIPLGFA AIGHIGPISI  2880
PNIAIPSIHL GIDPTFDVGP ITVDPITLTI PGLSLDAAVS EIRMTSGSSS GFKVRPSFSF  2940
FAVGPDGMPG GEVSILQPFT VAPINLNPTT LHFPGFTIPT GPIHIGLPLS LTIPGFTIPG  3000
GTLIPQLPLG LGLSGGTPPF DLPTVVIDRI PVELHASTTI GPVSLPIFGF GGAPGFGNDT  3060

TAPSSGFFNT GGGGGSGFSN SGSGMSGVLN AISDPLLGSA SGFANFGTQL SGILNRGAGI  3120
SGVYNTGTLG LVTSAFVSGF MNVGQQLSGL LFAGTGP                           3157
```

<212> Type : PRT
<211> Length : 3157
SequenceName : SEQ ID 142
SequenceDescription :
Sequence --------
<213> OrganismName : Mycobacterium tuberculosis H37Rv
<400> PreSequenceString :

```
MSFVVMPPEI NSLLIYTGAG PGPLLAAAAA WDELAAELGS AAAAFGSVTS GLVGGIWQGP    60
SSVAMAAAAA PYAGWLSAAA ASAESAAGQA RAVVGVFEAA LAETVDPFVI AANRSRLVSL   120
ALSNLFGQNT PAIAAAEFDY ELMWAQDVAA MLGYHTGASA AAEALAPFGS PLASLAAAAE   180
PAKSLAVNLG LANVGLFNAG SGNVGSYNVG AGNVGSYNVG GGNIGGNNVG LGNVGWGNFG   240
LGNSGLTPGL MGLGNIGFGN AGSYNFGLAN MGVGNIGFAN TGSGNFGIGL TGDNLTGFGG   300
FNTGSGNVGL FNSGTGNVGF FNSGTGNWGV FNSGSYNTGI GNSGIASTGL FNAGGFNTGV   360
VNAGSYNTGS FNAGEANTGG FNPGSVNTGW LNTGDINTGV ANSGDVNTGA FISGNYSNGV   420
LWRGDYQGLL GFSSGANVLP VIPLSLDING GVGAITIEPI HILPDIPINI NETLYLGPLV   480
VPPINVPAIS LGVGIPNISI GPIKINPITL WPAQNFNQTI TLAWPVSSIT IPQIQQVALS   540
PSPIPTTLIG PIHINTGFSI PVTFSYSTPA LTLFPVGLSI PTGGPLTLTL GVTAGTEAFT   600
IPGFSIPEQP LPLAINVIGH INALSTPAIT IDNIPLNLHA IGGVGPVDIV GGNVPASPGF   660
GNSTTAPSSG FFNTGAGGVS GFGNVGAHTS GWFNQSTQAM QVLPGTVSGY FNSGTLMSGI   720
GNVGTQLSGM LSGGALGGNN FGLGNIGFDN VGFGNAGSSN FGLANMGIGN IGLANTGNGN   780
IGIGLSGDNL TGFGGFNSGS ENVGLFNSGT GNVGFFNSGT GNLGVFNSGS HNTGFFLTGN   840
NINVLAPFTP GTLFTISEIP IDLQVIGGIG PIHVQPIDIP AFDIQITGGF IGIREFTLPE   900
ITIPAIPIHV TGTVGLEGFH VNPAFVLFGQ TAMAEITADP VVLPDPFITI DHYGPPLGPP   960
GAKFPSGSFY LSISDLQING PIIGSYGGPG TIPGPFGATF NLSTSSLALF PAGLTVPDQT  1020
PVTVNLTGGL DSITLFPGGL AFPENPVVSL TNFSVGTGGF TVFPQGFTVD RIPVDLHTTL  1080
SIGPFPFRWD YIPPTPANGP IPAVPGGFGL TSGLFPFHFT LNGGIGPISI PTTTVVDALN  1140
PLLTVTGNLE VGPFTVPDIP IPAINFGLDG NVNVSFNAPA TTLLSGLGIT GSIDISGIQI  1200
TNIQTQPAQL FMSVGQTLFL FDFRDGIELN PIVIPGSSIP ITMAGLSIPL PTVSESIPLN  1260
FSFGSPASTV KSMILHEILP IDVSINLEDA VFIPATVLPA IPLNVDVTIP VGPINIPIIT  1320
EPGSGNSTTT TSDPFSGLAV PGLGVGLLGL FDGSIANNLI SGFNSAVGIV GPNVGLSNLG  1380
GGNVGLGNVG DFNLGAGNVG GFNVGGGNIG GNNVGLGNVG FGNVGLANSG LTPGLMGLGN  1440
IGFGNAGSYN FGLANMGVGN IGFANTGSGN FGIGLTGDNL TGFGGFNTGS GNVGLFNSGT  1500
GNVGFFNSGT GNWGVFNSGS YNTGIGNSGI ASTGLFNAGG FNTGVVNAGS YNTGSFNAGQ  1560
ANTGGFNPGS VNTGWLNTGD INTGVANSGD VNTGAFISGN YSNGAFWRGD YQGLLGFSYR  1620
PAVLPQTPFL DLTLTGGLGS VVIPAIDIPA IRPEFSANVA IDSFTVPSIP IPQIDLAATT  1680
VSVGLGPITV PHLDIPRVPV TLNYLFGSQP GGPLKIGPIT GLFNTPIGLT PLALSQIVIG  1740
ASSSQGTITA FLANLPFSTP VVTIDEIPLL ASITGHSEPV DIFPGGLTIP AMNPLSINLS  1800
GGTGAVTIPA ITIGEIPFDL VAHSTLGPVH ILIDLPAVPG FGNTTGAPSS GFFNSGAGGV  1860
SGFGNVGAMV SGGWNQAPSA LLGGGSGVFN AGTLHSGVLN FGSGMSGLFN TSVLGLGAPA  1920
LVSGLGSVGQ QLSGLLASGT ALHQGLVLNF GLADVGLGNV GLGNVGDFNL GAGNVGGFNV  1980
GGGNIGGNNV GLGNVGWGNF GLGNSGLTPG LMGLGNIGFG NAGSYNFGLA NMGVGNIGFA  2040
NTGSGNFGIG LTGDNLTGFG GFNTGSGNVG LFNSGTGNVG FFNSGTGNWG VFNSGSYNTG  2100
IGNSGIASTG LFNAGGFNTG VVNAGSYNTG SFNAGQANTG GFNPGSVNTG WLNTGDINTG  2160
VANSGDVNTG AFISGNYSNG AFWRGDYQGL LGFSYTSTII PEFTVANIHA SGGAGPIIVP  2220
SIQFPAIPLD LSATGHIGGF TIPPVSISPI TVRIDPVFDL GPITVQDITI PALGLDPATG  2280
VTVGPIFSSG SIIDPFSLTL LGFINVNVPA IQTAPSEILP FTVLLSSLGV THLTPEITIP  2340
GFHIPVDPIH VELPLSVTIG PFVSPEITIP QLPLGLALSG ATPAFAFPLE ITIDRIPVVL  2400
DVNALLGPIN AGLVIPPVPG FGNTTAVPSS GFFNIGGGGG LSGFHNLGAG MSGVLNAISD  2460
PLLGSASGFA NFGTQLSGIL NRGADISGVY NTGALGLITS ALVSGFGNVG QQLAGLIYTG  2520
TGP                                                                2523
```

<212> Type : PRT
<211> Length : 2523
SequenceName : SEQ ID 143
SequenceDescription :
Sequence --------
<213> OrganismName : Mycobacterium tuberculosis H37Rv
<400> PreSequenceString :

```
MSFVIAVPEA LTMAASDLAN IGSTINAANA AAALPTTGVV AAAADEVSAA VAALFGSYAQ    60
SYQAFGAQLS AFHAQFVQSL TNGARSYVVA EATSAAPLQD LLGVVNAPAQ ALLGRPLIGN   120
GANGADGTGA PGGPGGLLLG NGGNGGSGAP GQPGGAGGDA GLIGNGGTGG KGGDGLVGSG   180
AAGGVGGRGG WLLGNGGTGG AGGAAGATLV GGTGGVGGAT GLIGSGGFGG AGGAAAGVGT   240

TGGVGGSGGV GGVFGNGGFG GAGGLGAAGG VGGAASYFGT GGGGGVGGDG APGGDGGAGP   300
LLIGNGGVGG LGGAGAAGGN GGAGGMLLGD GGAGGQGGPA VAGVLGGMPG AGGNGGNANW   360
FGSGGAGGQG GTGLAGTNGV NPGSIANPNT GANGTDNSGN GNQTGGNGGP GPAGGVGEAG   420
GVGGQGGLGE SLDGNDGTGG KGGAGGTAGT DGGAGGAGGA GGIGETDGSA GGVATGGEGG   480
DGATGGVDGG VGGAGGKGGQ GHNTGVGDAF GGDGGIGGDG NGALGAAGGN GGTGGAGGNG   540
GRGGMLIGNG GAGGAGGTGG TGGGGAAGFA GGVGGAGGEG LTDGAGTAEG GTGGLGGLGG   600
VGGTGGMGGS GGVGGNGGAA GSLIGLGGGG GAGGVGGTGG IGGIGGAGGN GGAGGAGTTT   660
GGGATIGGGG GTGGVGGAGG TGGTGGAGGT TGGSGGAGGL IGWAGAAGGT GAGGTGGQGG   720
LGGQGGNGGN GGTGATGGQG GDFALGGNGG AGGAGGSPGG SSGIQGNMGP PGTQGADG     778
```

<212> Type : PRT
<211> Length : 778
SequenceName : SEQ ID 144
SequenceDescription :
Sequence
--------
<213> OrganismName : Mycobacterium tuberculosis H37Rv
<400> PreSequenceString :

```
PQGADGNAGN GGDGGVGGNG GNGADNTTTA AAGTTGGAGG AGGAGGTGGT GGAAGTGTGG    60
QQGNGGNGGN GGTGGKGGTG GDGALAGSSG GAGGKGGNGG DAGKAGTGSA PGTAGTGGDG   120
GKGGNGGIGA AGTTGPVGTG ASGGTGGSGG AGGTGGDGGA ANGGTAGAGG AGGNGGKGGD   180
GGAGVTSSTA GNSGGAGGSG GKGGDAGAGG AGATPGANGI AGNGGDGGDG AAGAVGISGA   240
TGAGDGGHGG TGAAGGNGGT GGAGGSGIDG VGGGTGGTGG NGGNGAIGGA GGDAGGSGNS   300
GGNGGIGGKG GNAGAGGAAG SNGGTVGANG TGGDGGNGGA AGAATAGSNG GAGTGSAGGN   360
GGTGGRGGSG GAGGDGIGGV GGGKGGNGAD GEVGGAGGAG GSGPNTSPGG NGGQGGQGGS   420
GGAGGAAGAG GAGGGANGTA GNGGQGGAGG TGGAGAASSA TNGGSGGAGG TGGDGGSGGA   480
GGTGGAGGTG GAAGDGGQGG QGGAGGGAGG QGGAGGAGGT GGNGGNITGG TAGTAGAAGN,  540
GGAAGKGGAG GQGGTGGGTG GQGGAGGDGG AGGTGGDRTV GGGTVPAGSG GQGGNAGGGG   600
AGGQGGADGG SGGDGGDAGT GGNGGNGGNR NSGNGTGGAG GNGGGGANGG AGGAGGSGGG   660
TGGNGGAGGD AGDAGNGGNG NGTGNGGNGG NGGIAGMGGN GGAGTGSGNG GNGGSGGNGG   720
NAGMGGNSGT GSGDGGAGGN GGAAGTGGTG GDGGLTGTGG TGGSGGTGGD GGNGGNGADN   780
TANMTAQAGG DGGNGGDGGF GGGAGAGGGG LTAGANGTGG QGGAGGDGGN GAIGGHGPLT   840
DDPGGNGGTG GNGGTGGTGG AGIGSLGGGT GGDGGNGGNG GTGGEGGEVG GAGGTGGAAG   900
NGGDGGTGGT GGGDGGAGGT GGTGGTGGLG DPRVGGSGGD GGTGGSGGAA GNGGNGGNAG   960
AGGNGNGGTG GAGGIGGTGG NGGDAEPGVP PGAGGAGGAG TTGGKGGTGG NGSGTGSGGT  1020
GGDGGTGGGG GNGGTGWNGG KGDTGSGGGA GDGGKAPAGG TGGAGGDGGA GGKGGSGGV   1079
```

<212> Type : PRT
<211> Length : 1079
SequenceName : SEQ ID 145
SequenceDescription :
Sequence --------
<213> OrganismName : Mycobacterium tuberculosis H37Rv
<400> PreSequenceString :

```
MVMSLMVAPE LVAAAAADLT GIGQAISAAN AAAAGPTTQV LAAAGDEVSA AIAALFGTHA    60
QEYQALSARV ATFHEQFVRS LTAAGSAYAT AEAANASPLQ ALEQQVLGAI NAPTQLWLGR   120
PLIGDGVHGA PGTGQPGGAG GLLWGNGGNG GSGAAGQVGG PGGAAGLFGN GGSGGSGGAG   180
AAGGVGGSGG WLNGNGGAGG AGGTGANGGA GGNAWLFGAG GSGGAGTNGG VGGSGGFVYG   240
NGGAGGIGGI GGIGGNGGDA GLFGNGGAGG AGAAGLPGAA GLNGGDGSDG GNGGTGGNGG   300
RGGLLVGNGG AGGAGGVGGD GGKGGAGDPS FAVNNGAGGN GGHGGNPGVG GAGGAGGLLA   360
GAHGAAGATP TSGGNGGDGG IGATANSPLQ AGGAGGNGGH GGLVGNGGTG GAGGAGHAGS   420
TGATGTALQP TGGNGTNGGA GGHGGNGGNG GAQHGDGGVG GKGGAGGSGG AGGNGFDAAT   480
LGSPGADGGM GGNGGKGGDG GKAGDGGAGA AGDVTLAVNQ GAGGDGGNGG EVGVGGKGGA   540
GGVSANPALN GSAGANGTAP TSGGNGGNGG AGATPTVAGE NGGAGGNGGH GGSVGNGGAG   600
GAGGNGVAGT GLALNGGNGG NGGIGGNGGS AAGTGGDGGK GGNGGAGANG QDFSASANGA   660
NGGQGGNGGN GGIGGKGGDA FATFAKAGNG GAGGNGGNVG VAGQGGAGGK GAIPAMKGAT   720
GADGTAPTSG GDGGNGGNGA SPTVAGGNGG DGGKGGSGGN VGNGGNGGAG GNGAAGQAGT   780
PGPTSGDSGT SGTDGGAGGN GGAGGAGGTL AGHGGNGGKG GNGGQGGIGG AGERGADGAG   840
PNANGANGEN GGSGGNGGDG GAGGNGGAGG KAQAAGYTDG ATGTGGDGGN GGDGGKAGDG   900
GAGENGLNSG AMLPGGGTVG NPGTGGNGGN GGNAGVGGTG GKAGTGSLTG LDGTDGITPN   960
GGNGGNGGNG GKGGTAGNGS GAAGGNGGNG GSGLNGGDAG NGGNGGGALN QAGFFGTGGK  1020
GGNGGNGGAG MINGGLGGFG GAGGGGAVDV AATTGGAGGN GGAGGFASTG LGGPGGAGGP  1080
GGAGDFASGV GGVGGAGGDG GAGGVGGFGG QGGIGGEGRT GGNGGSGGDG GGGISLGGNG  1140
GLGGNGGVSE TGFGGAGGNG GYGGPGGPEG NGGLGGNGGA GGNGGVSTTG GDGGAGGKGG  1200

NGGDGGNVGL GGDAGSGGAG GNGGIGTDAG GAGGAGGAGG NGGSSKSTTT GNAGSGGAGG  1260
NGGTGLNGAG GAGGAGGNAG VAGVSFGNAV GGDGGNGGNG GHGGDGTTGG AGGKGGNGSS  1320
GAASGSGVVN VTAGHGGNGG NGGNGGNGSA GAGGQGGAGG SAGNGGHGGG ATGGDGGNGG  1380
NGGNSGNSTG VAGLAGGAAG AGGNGGGTSS AAGHGGSGGS GGSGTTGGAG AAGGNGGAGA  1440
GGGSLSTGQS GGPRRQRWCR WQRRRWLGRQ RRRRWCRWQR RCRRQRWRWR CRQRRLRRQW  1500
RQGRRRCRPW LHRRRGRQGR RWRQRRFQQR QRSRWQRR                          1538
```

<212> Type : PRT
<211> Length : 1538
SequenceName : SEQ ID 146
SequenceDescription :
Sequence --------
<213> OrganismName : Mycobacterium tuberculosis H37Rv
<400> PreSequenceString :

```
MSFVVTAPPV LASAASDLGG IASMISEANA MAAVRTTALA PAAADEVSAA IAALFSSYAR   60
DYQTLSVQVT AFHVQFAQTL TNAGQLYAVV DVGNGVLLKT EQQVLGVINA PTQTLVGRPL  120
IGDGTHGAPG TGQNGGAGGI LWGNGGNGGS GAPGQPGGRG GDAGLFGHGG HGGVGGPGIA  180
GAAGTAGLPG GNGANGGSGG IGGAGGAGGN GGLLFGNGGA GGQGGSGGLG GSGGTGGAGM  240
AAGPAGGTGG IGGIGGIGGA GGVGGHGSAL FGHGGINGDG GTGGMGGQGG AGGNGWAAEG  300
ITVGIGEQGG QGGDGGAGGA GGIGGSAGGI GGSQGAGGHG GDGGQGGAGG SGGVGGGGAG  360
AGGDGGAGGI GGTGGNGSIG GAAGNGGNGG RGGAGGMATA GSDGGNGGGG GNGGVGVGSA  420
GGAGGTGGDG GAAGAGGAPG HGYFQQPAPQ GLPIGTGGTG GEGGAGGAGG DGGQGDIGFD  480
GGRGGDGGPG GGGGAGGDGS GTFNAQANNG GDGGAGGVGG AGGTGGTGGV GADGGRGGDS  540
GRGGDGGNAG HGGAAQFSGR GAYGGEGGSG GAGGNAGGAG TGGTAGSGGA GGFGGNGADG  600
GNGGNGGNGG FGGINGTFGT NGAGGTGGLG TLLGGHNGNI GLNGATGGIG STTLTNATVP  660
LQLVNTTEPV VFISLNGGQM VPVLLDTGST GLVMDSQFLT QNFGPVIGTG TAGYAGGLTY  720
NYNTYSTTVD FGNGLLTLPT SVNVVTSSSP GTLGNFLSRS GAVGVLGIGP NNGFPGTSSI  780
VTAMPGLLNN GVLIDESAGI LQFGPNTLTG GITISGAPIS TVAVQIDNGP LQQAPVMFDS  840
GGINGTIPSA LASLPSGGFV PAGTTISVYT SDGQTLLYSY TTTATNTPFV TSGGVMNTGH  900
VPFAQQPIYV SYSPTAIGTT TFN                                          923
```

<212> Type : PRT
<211> Length : 923
SequenceName : SEQ ID 147
SequenceDescription :
Sequence
<213> OrganismName : Mycobacterium tuberculosis H37Rv
<400> PreSequenceString :

```
MIGNGGAGGS GAPGAIGGAG GPAGLIGVGG AGGAGGDSAV AGVIGGAGGA GGAALLFGAG   60
GAGGAGGSGG SGAAGGAGGA GGAGGLFASG GSGGFGGFAS TGTGGAGGTG GAGGLFASGG  120
VGGTGGGAGS GGTGGVGGTG GAGGLFASGG AGGAGGSGGT GGAGGTGGAG GLFGAGGAGG  180
LGGQGNHTGG HGGAGGSAGL LALGDGGAGG AGGAATTGTG GAGGAGGKAG LLFGSGGAGG  240
SGGAAGTFGD TGNSGGAGGA GGKAGLLFGS GGAGGSGGAG GFANGSTGGA GGAGGGAGLI  300
GNGGNGGSGG TSVATGGAGN GGAGGAGGGA GLIGNGGNGG SGGMGDAPGG TGVGGIGGLL  360
LGLDGANAPA STNPLHTAQQ QALAAVNAPI QAVTGRPLIG NGANGAPGSG APGGHGGWLF  420
GGGGTGGSGV SGGAGGDGGA GGILFGAGGA GGAGGAVTGT GATGGSGGAG GGALLFGAGG  480
AGGAGGSSGI GGFAAGGAGG PGGAGGLFNG GGAGGAGGSG VSGGAGGEGG AGGAGGLFAG  540
GGAGGAGGSG NNVGGAGGAG GVGGLFGAGG AGGSGGGGSV AGDSGAGGNA GLLAPGLAGG  600
AGGGGGQGFD TGGAGGPGGD AGLLVGSGGV GGAGGFGLTT GGPGAAGGDA GLLFGSGGAG  660
GAGGSGRTDL GGAGGAGGKA GLIGNGGNGG AGGAGGNGGG DGGPGGAAFG LGNGGNGGNG  720
GTGTSAGSPG AGGAGGSLIG AEGLPGLLP                                    749
```

<212> Type : PRT
<211> Length : 749
SequenceName : SEQ ID 148
SequenceDescription :
Sequence
<213> OrganismName : Mycobacterium tuberculosis H37Rv
<400> PreSequenceString :

```
MSFVIAAPEA LVAVASDLAG IGSALAEANA AALAPTTALL AAGADEVSAA IAALFGAHGQ   60
AYQTVSAQAS AFHAQFVQAL TGGGGAYAAA EAANVSAAQS TDQRLLDLIN GPTQALLGRP  120
LIGDGANGGP GQDGGPGGLL YGNGGNGGTS TTAGVAGGNG GAAGLIGNGG AGGGGGAGAA  180
GGNGGAGGWL YGNGGAGGAG GTSVIPGVAG GNGGAGGSAG LWGTGGAGGD GGNGRSGPVN  240
VAGSAGGNGG AGGAAGLFGD AGAGGNGGKG GAGGAAFSIN FTAGDGGAGG AGGSGGHALL  300
```

```
WGAGGAGGNG GSGGTGGAGG STAGAGGNGG AGGGGGTGGL LFGNGGAGGH GAAAGNGLAA   360
GNGVSSSGGG GAGGTGGAGG DGGAGGAGGN ARLWGVGGAG GAGGDGGAGG AGGKGGSGLS   420
GNANGGAGGD SGRGGTGGAG GEGGAAGLLV GTGGHGGDGG AGGAAVKGGD GGAAAGTGIA   480
GAGGRGGAGG SGGSGGDGGG GAAGPAGWLF GDGGAGGNGG AAAAGGAGGQ AGGGGGNGGN   540
GGNGGNGGNG GNGATGGWLY GNGGAGGQGA TAGAGGAGAN GVSSTNGGGT GGNGGIGGTG   600
GSGGAGGNAG LLGVGGAGGH GASGGAGDRG GAGGTGFISS DGGAGGDGGD GGNGGAGGTG   660
GLLFGAGGNG GPGGSGGAAD IGGNGGAGNG GGTDGNGGNG GSGGGAGSGG DGGGAGGNGA   720
WLFGNGGAGG GGGKGGNGAG GGLGGGSFGL PGLNGSGGDG GDGGNGAPGG VLYGNGGAGG   780
QGSSGGIGGP GATGGAGGKG GDGGDAQLIG DGGNGGNGGA GGTGGTPGPG GPGGSGGLGG   840
LLFGQTGTAG VSP                                                      853
```

<212> Type : PRT
<211> Length : 853
SequenceName : SEQ ID 149
SequenceDescription :
Sequence --------
<213> OrganismName : Mycobacterium tuberculosis H37Rv
<400> PreSequenceString :

```
MSYLVVVPEL VAAAATDLAN IGSSISAANA AAAAPTTALV AAGGDEVSAA IAALFGAHAR    60
AYQALSAQAA MFHEQFVRAL AAGGNSYAVA EAATAQSVQQ DLLNLINAPT QALLGRPLIG   120
NGANGLPGTG QNGGDGGILY GNGGNGGSGG VNQAGGNGGN AGLWGNGGSG GAGGNATTAG   180
RNGFNGGAGG SGGLLWGNGG AGGAGGNGGP APLVGGVGTT GGAGGNGGGA GLFYGFGGAG   240
GNGGMGGVAP STGPSMGILP AGGVGGPGGS GGASALAFGS GGVGGAGGLG GPTDGTVQGV   300
GGFGGQGGNG GQSGLLFGNA GAGGAGAAGG AGTGDTESFG GHGGAGGDGG AVGLIGNGGA   360
GGTGSPGAVV GGNGGVGGLG GAGSPGGLLY GTGGAGGNGG PGGDGGTGAT VGFAGSGGFG   420
GAGGIAQLFG TGGMGGSGGG IGAGTTTVVP PDVAPVGGTG GNGGRAGLLL GVGGMGGNGG   480
ATSVGGTLYA AGGNGGDGGL VWGNGGTGGS GGAGGAGSVG NGGAGGNAAL LFGNGGAGGA   540
GGAGGIGAGG AGGFGAVLFG NGGAGGSGAP GGIGAGGNGG NALLVGNGGN GGAGTGGAAG   600
GAGGSGGLLF GQNGMPGP                                                 618
```

<212> Type : PRT
<211> Length : 618
SequenceName : SEQ ID 150
SequenceDescription :
Sequence
--------
<213> OrganismName : Mycobacterium tuberculosis H37Rv
<400> PreSequenceString :

```
MNFSVLPPEI NSALIFAGAG PEPMAAAATA WDGLAMELAS AAASFGSVTS GLVGGAWQGA    60
SSSAMAAAAA PYAAWLAAAA VQAEQTAAQA AAMIAEFEAV KTAVVQPMLV AANRADLVSL   120
VMSNLFGQNA PAIAAIEATY EQMWAADVSA MSAYHAGASA IASALSPFSK PLQNLAGLPA   180
WLASGAPAAA MTAAAGIPAL AGGPTAINLG IANVGGGNVG NANNGLANIG NANLGNYNFG   240
SGNFGNSNIG SASLGNNNIG FGNLGSNNVG VGNLGNLNTG FANTGLGNFG FGNTGNNNIG   300
IGLTGNNQIG IGGLNSGTGN FGLFNSGSGN VGFFNSGNGN FGIGNSGNFN TGGWNSGHGN   360
TGFFNAGSFN TGMLDVGNAN TGSLNTGSYN MGDFNPGSSN TGTFNTGNAN TGFLNAGNIN   420
TGVFNIGHMN NGLFNTGDMN NGVFYRGVGQ GSLQFSITTP DLTLPPLQIP GISVPAFSLP   480
AITLPSLNIP AATTPANITV GAFSLPGLTL PSLNIPAATT PANITVGAFS LPGLTLPSLN   540
IPAATTPANI TVGAFSLPGL TLPSLNIPAA TTPANITVGA FSLPGLTLPS LNIPAATTPA   600
NITVGAFSLP GLTLPSLNIP AATTPANITV SGFQLPPLSI PSVAIPPVTV PPITVGAFNL   660
PPLQIPEVTI PQLTIPAGIT IGGFSLPAIH TQPITVGQIG VGQFGLPSIG WDVFLSTPRI   720
TVPAFGIPFT LQFQTNVPAL QPPGGGLSTF TNGALIFGEF DLPQLVVHPY TLTGPIVIGS   780
FFLPAFNIPG IDVPAINVDG FTLPQITTPA ITTPEFAIPP IGVGGFTLPQ ITTQEIITPE   840
LTINSIGVGG FTLPQITTPP ITTPPLTIDP INLTGFTLPQ ITTPPITTPP LTIDPINLTG   900
FTLPQITTPP ITTPPLTIEP IGVGGFTTPP LTVPGIHLPS TTIGAFAIPG GPGYFNSSTA   960
PSSGFFNSGA GGNSGFGNNG SGLSGWFNTN PAGLLGGSGY QNFGGLSSGF SNLGSGVSGF  1020
ANRGILPFSV ASVVSGFANI GTNLAGFFQG TTS                               1053
```

<212> Type : PRT
<211> Length : 1053
SequenceName : SEQ ID 151
SequenceDescription :

Sequence
--------
<213> OrganismName : Mycobacterium tuberculosis H37Rv
<400> PreSequenceString :

```
MLYVVASPDL MTAAATNLAE IGSAISTANG AAALPTVEVV AAAADEVSTQ IAALFGAHAR   60

SYQTLSTQAA AFHSRFVQAL TTAAASYASV EAANASPLQV ALDVINAPAQ TLLGRPLIGN  120
GADGSTPGQA GGPGGLLYGN GGNGAAGGPN QAGGAGGNAG LIGNGGAGGA GGVGAVGGKR  180
GTGGLLFGNG GAGGQGGLGL AGINGGSGGQ GGHGGNAILF GQGGAGGPGG TGAMGVAGTN  240
PTPIGTAAPG SDGVNQIGNG GNTDLTGGAG GDGNAGSTTV NGGNGGTGGA ARNSSGGTGN  300
SFGGAGGAGG DGANGGDGGA GGEALTEGGA TAVSGAGGKG GNAEASGGAG GNGGKGGFAQ  360
ATTSVTGGNG GNGGNGHDSN APGGAGGSGG VGGDGGRGGL LAGNGGTGGA GGNGGTGGAG  420
APGGAGGAGG KADIANSLGD NATVTGGNGG TGGDGGSALG TGGAGGAGGL GGHGGAGGLL  480
IGNGGAGGAG GLGGAGGAGG AGGEGGAGGA GGEAIPGGAS TNSAGGDGGA GGTGGNGGDG  540
GAGGAPGLGG AGGAGGWLIG QSGSTGGGGA GGAGGAGGAG GAGGSGGAGG HGDTTSGKNG  600
SSGTAGFDGN PGQPG                                                615
```

<212> Type : PRT
<211> Length : 615
SequenceName : SEQ ID 152
SequenceDescription :
Sequence
--------
<213> OrganismName : Mycobacterium tuberculosis H37Rv
<400> PreSequenceString :

```
MHYSVLPPEI NSALIFAGAG SGPMLAAASA WDGLATELAS AAVSFGSVTA GLVGGSWQGR   60
SSVAMAAAAA PYAGWLAAAA TQAEQAATQA QVMVAEFEAV RLAMVQPALV AANRSGLISL  120
VISNLFGQNA PAIAAAEAAY EEMWALDVSA MAAYHSGASA VAVALPAFAL PLRLPAGLAA  180
GPAAVVTALT TAVGMPTFAG RAIAASLGLA NVGGGNLGNA NNGLGNIGNA NLGNNNLGSG  240
NFGSFNIGSA NLGGNNIGIG NAGANNFGLA NLGNLNTGFA NAGIGNFGIA NTGNNNIGNG  300
LTGNNQIGIG GLNSGNGNVG LFNAGSANIG FFNSGNGNFG IGNSGNFSTG LFNPGHGNTG  360
FLNAGSFNTG MFDVGNANTG SFNVGHYNFG AFNPGPSNTG TFNTGGANTG WFNTGSINTG  420
AFNIGDMNNG LFNTGDMNNG VFYRGVGQGS LQFAITSPDL TLPSLEIPGI SVPAFSLPAI  480
TLPSLTIPAV TTPANVTVGA FDLPGLTVPS LTIPAAMTPA NITVGAFDLP GLTVPSLTIP  540
ATTTPANITV GAFNLPQLSI PSVTVPPITI PAGTALGAFN LPTLSIPSVT VPPITIPAGT  600
TVGGFTLPTI HTPLISTPQI SIGGFSTPGI ATQANSGVIN LPTFSLNGIT ITNLVVFIPN  660
NITALQTNMP GVFPQIGGFA NTPPAFINTG TITVGGGQIN GVGFSIGAIN VTPFTLPNVV  720
IQPWSLGGIS VDGFTLPEIS TQEFTTPALT ISPIGVGALS LPDITTQQFT TPELTIDPIT  780
LGGFTLPQLS IPAITTPAFT IDPIALGGFT LPQIMTPEIT TPPFAIDPIG LSGFTLPQVN  840
IPEITTPEFT IQPVGLAAFT TPALTIASIH LPSTTMGGFA IPAGPGYFNS SATPSLGFFN  900
AGIGGNSGFG NSGSGLSGWF NTSPVGLLAG SGYQNYGGLI SGFSNLGSGI SGFANTGTLP  960
FAVTSLVSGL ANIGNNLSGL FFQSTTP                                    987
```

<212> Type : PRT
<211> Length : 987
SequenceName : SEQ ID 153
SequenceDescription :
Sequence --------
<213> OrganismName : Mycobacterium tuberculosis H37Rv
<400> PreSequenceString :

MSFVVVAPEV LAAAASDLAG IGSTLAQANA AALAPTTAVL AAGADEVSAA IASLFGAHGQ 60
AYQAVSAQMS AFHAQFMQAL TGAGGAYAAA EAVNVSAAQS VEQDLLAAIN ARFERIFGRP 120
LIGDGANGGP GQDGGPGGLL YGNGGNGGTS TTVGMAGGNG GAAGLIGNGG FGGGGGPGAA 180
GGNGGAGGWL FGNGGAGGAG GLGVAPGVPG GAGGAGGAGG VGGPAGLWGH GGAGGAGGAG 240
VAGAGGFEGT IGAGGAGGVG GAGGVGGAGG AGGWLYGDAG AGGDGGVGGA GGTGGLGNRG 300
GAGGAGGAGG VGGAGGAAGL WGGGGAGGVG GTGGGAGLGA QSVTFSSSLS GLSGGDGGAG 360
GAGGAGGAGG TGGWLYGGGG AAGSGGDGGT GGQGGAGGAG VFSLFGSGGG PGGNGGVGGV 420
GGVGGAGGRA GLFGVGGLGG AGGDAGDSGE GGFGGPGLAG GLFGNPGNGG VGGIGGDAAA 480
GGAGGAGGNG GAGGNGGWLF GNGGAGGSGG DGGAAGRGGA GNLGSAGGIN APAGNPGSGS 540
VGIGGAGGAG GTAGLFGDGG AGGAGGAGAA GGFGGISAAT PSAGSEGAMG GAGGVGGNAR 600
LLGTGGAGGV GGGGGAGGDG GRGGVATPGG QGGDAGDGGA GGAGGNGGGA SGAGGWLLGT 660
GGAGGAGGNG GNGGKAGFSP GPTNFGLNGA GGGGGVGGNG ATGPWLFGDG GPTPGSTGAG 720
AAGGHGGDAQ LIGNGGHGGA GGTGVPNGSG GAGGLSGLLF GEPGANG 767

<212> Type : PRT
<211> Length : 767
SequenceName : SEQ ID 154
SequenceDescription :
Sequence --------
<213> OrganismName : Mycobacterium tuberculosis H37Rv
<400> PreSequenceString :

MSFVIANPEM LAAAATDLAG IRSAISAATA AAAAPTIQVA AAGADEVSLA ISALFGQHAQ 60
AYQALSAQAT IFHDQFVQAL TSGGNLYAAA ESHTVEQMVL NAINAPTQTL FGRPLIGDGA 120
NGTAENPDGQ NGGLLFGNGG NGFTQTTAGV AGGNGGSAGL IGNGGAGGGG GAGAAGGLGG 180
NGGWLYGNGG AGGIGGAGTG TGGHGGAGGA GGRAWLWGTG GAGGAGGDGG WLFGDGGAGG 240
TGGNGGSGFN SLTSSVGGAG GAGGHAGLFG AGGTGGTGGI GGQNTETGPA ASNGGAGGAG 300
GGGGYLVGDG GAGGTGGAGG KNSSGGATLT GGTGGTGGAG GAAGWLYGSG GAGGAGGAGG 360
LNNAGGATGG TGGTGGAGGS GAWLYGNGGA AGAGGNGGNN TSAGTGGVGA SGGTGGNAGL 420
IGAGGHGGAG GAGGNQTGGV GNGGAGGNGG AGGAGGQLYG NGGDGGNGGA GGANIAGGNG 480
SDGGAAGHGG AGGSARLIGA GGHGGDGGAG GNTAGRRADA IAGTGGDGGN GGNGGLLSGN 540
AGAGGHGGAG GSSTATTTTG TPPTGATGGN GGNGGAGGTA GFTGSGGIGG NGGAGGTGGN 600
AGVALSVGST GGLGGNGGSG GLGGGGGSLF GNGGAGGVGA TGGNGGSGIG PASVGGNGGK 660
GGVGAAGGLA GQIGNGGSGG SGGAGGNGGT GDTAGNGGNG GAGAVGGNAQ LIGNGGNGGG 720
GGNGGTGADG T 731

<212> Type : PRT
<211> Length : 731
SequenceName : SEQ ID 155
SequenceDescription :
Sequence
--------
<213> OrganismName : Mycobacterium tuberculosis H37Rv
<400> PreSequenceString :

MPGRFRNFGS QNLGSGNIGS TNVGSGNIGS TNVGSGNIGD TNFGNGNNGN FNFGSGNTGS 60
NNIGFGNTGS GNFGFGNTGN NNIGIGLTGD GQIGIGGLNS GSGNIGFGNS GTGNVGLFNS 120
GTGNVGFGNS GTANTGFGNA GNVNTGFWNG GSTNTGLANA GAGNTGFFDA GNYNFGSLNA 180
GNINSSFGNS GDGNSGFLNA GDVNSGVGNA GDVNTGLGNS GNINTGGFNP GTLNTGFFSA 240
MTQAGPNSGF FNAGTGNSGF GHNDPAGSGN SGIQNSGFGN SGYVNTSTTS MFGGNSGVLN 300
TGYGNSGFYN AAVNNTGIFV TGVMSSGFFN FGTGNSGLLV SGNGLSGFFK NLFG 354

<212> Type : PRT
<211> Length : 354
SequenceName : SEQ ID 156
SequenceDescription :
Sequence --------
<213> OrganismName : Mycobacterium tuberculosis H37Rv
<400> PreSequenceString :

```
MSFVLAMPEV LGSAATDLAA LGSVLGAADA AAAATTTGIV AAAQDEVSAA IAALFSAHGR    60
AYQVASAQAA AVHAQFVEAL SAGAGAYASA EAAGAAVLAN PAQSVQQDLL AAVNAQSVAL   120
TGRPLIGNGA NGAPGTGANG APGGWLLGNG GAGGSAAAGS GLPGGAGGAA GLFGTGGAGG   180
AGGSSTVGDG EAGGAGGSGG WLLGTGGVGG VGGLGAGAGG AGGVGGAGGL LGAGGHGGAG   240
GLGAVTGGVG GTGGAGGLLA GLLAGPGGAG GTGGRGFLNN GGVGGAGGNA GLLFGAGGTG   300
GSGGAGLGGD GGAGGAGGNT GVLFGNAGSG GTGGFGDTDG GAGGAGGDAG WLGSGGVGGA   360
GGFGETGDGG VGGAGGKAGL LIGNGGAGGA GGQGAVTGGT GGAGGDGVLI GNGGNAGIGG   420
TGPTAGDTGA GGISGLLLGA DGFNTPASAS PLHTLKQQAL AAINAPTQTL TGRPLIGNGT   480
PGAVGSGATG APGGWLLGDG GAGGSGAAGS GAPGGAGGAA GLWGTGGAGG AGGSSAGGGG   540
AGGAGGAGGW LLGDGGAGGI GGASTVLGGT GGGGGVGGLW GAGGAGGAGG TGLVGGDGGA   600
GGAGGTGGLL AGLIGAGGGH GGTGGLSTNG DGGVGGAGGN AGMLAGPGGA GGAGGDGENL   660
DTGGDGGAGG SAGLLFGSGG AGGAGGFGFL GGDGGAGGNA GLLLSSGGAG GFGGFGTAGG   720
VGGAGGNAGW LGFGGAGGVG GSAGLIGTGG NGGNGGTGAN AGSPGTGGAG GLLLGQNGLN   780
GLP                                                                783
```

<212> Type : PRT
<211> Length : 783
SequenceName : SEQ ID 157
SequenceDescription :
Sequence
--------
<213> OrganismName : Mycobacterium tuberculosis H37Rv
<400> PreSequenceString :

```
MSLVIATPQL LATAALDLAS IGSQVSAANA AAAMPTTEVV AAAADEVSAA IAGLFGAHAR    60
QYQALSVQVA AFHEQFVQAL TAAAGRYAST EAAVERSLLG AVNAPTEALL GRPLIGNGAD   120
GTAPGQPGAA GGLLFGNGGN GAAGGFGQTG GSGGAAGLIG NGGNGGAGGT GAAGGAGGNG   180
GWLWGNGGNG GVGGTSVAAG IGGAGGNGGN AGLFGHGGAG GTGGAGLAGA NGVNPTPGPA   240
ASTGDSPADV SGIGDQTGGD GGTGGHGTAG TPTGGTGGDG ATATAGSGKA TGGAGGDGGT   300
AAAGGGGGNG GDGGVAQGDI ASAFGGDGGN GSDGVAAGSG GGSGGAGGGA FVHIATATST   360

GGSGGFGGNG AASAASGADG GAGGAGGNGG AGGLLFGDGG NGGAGGAGGI GGDGATGGPG   420
GSGGNAGIAR FDSPDPEAEP DVVGGKGGDG GKGGSGLGVG GAGGTGGAGG NGGAGGLLFG   480
NGGNGGNAGA GGDGGAGVAG GVGGNGGGGG TATFHEDPVA GVWAVGGVGG DGGSGGSSLG   540
VGGVGGAGGV GGKGGASGML IGNGGNGGSG GVGGAGGVGG AGGDGGNGGS GGNASTFGDE   600
NSIGGAGGTG GNGGNGANGG NGGAGGIAGG AGGSGGFLSG AAGVSGADGI GGAGGAGGAG   660
GAGGSGGEAG AGGLTNGPGS PGVSGTEGMA GAPG                               694
```

<212> Type : PRT
<211> Length : 694
SequenceName : SEQ ID 158
SequenceDescription :
Sequence
<213> OrganismName : Mycobacterium tuberculosis H37Rv
<400> PreSequenceString :

```
MSFVIAAPEV IAAAATDLAS LESSIAAANA AAAANTTALL AAGADEVSTA VAALFGAHGQ    60
AYQALSAQAQ AFHAQFVQAL TSGGGAYAAA EAAATSPLLA PINEFFLANT GRPLIGNGTN   120
GAPGTGANGG DGGWLIGNGG AGGSGAAGVN GGAGGNGGAG GLIGNGGAGG AGGRASTGTG   180
GAGGAGGAAG MLFGAAGVGG PGGFAAAFGA TGGAGGAGGN GGLFADGGVG GAGGATDAGT   240
GGAGGSGGNG GLFGAGGTGG PGGFGIFGGG AGGDGGSGGL FGAGGTGGSG GTSIINVGGN   300
GGAGGDAGML SLGAAGGAGG SGGSNPDGGG GAGGIGGDGG TLFGSGGAGG VCGLGFDAGG   360
AGGAGGKAGL LIGAGGAGGA GGGSFAGAGG TGGAGGAPGL VGNAGNGGNG GASANGAGAA   420
GGAGGSGVLI GNGGNGGSGG TGAPAGTAGA GGLGGQLLGR DGFNAPASTP LHTLQQQILN   480
AINEPTQALT GRPLIGNGAN GTPGTGADGG AGGWLFGNGG NGGHGATGAD GGDGGSGGAG   540
GILSGIGGTG GSGGIGTTGQ GGTGGTGGAA LLIGSGGTGG SGGFGLDTGG AGGRGGDAGL   600
FLGAAGTGGQ AALSQNFIGA GGTAGAGGTG GLFANGGAGG AGGFGANGGT GGNGLLFGAG   660
GTGGAGTLGA DGGAGGHGGL FGAGGTGGAG GSSGGTFGGN GGSGGNAGLL ALGASGGAGG   720
SGGSALNVGG TGGVGGNGGS GGSLFGFGGA GGTGGSSGIG SSGGTGGDGG TAGVFGNGGD   780
GGAGGFGADT GGNSSSVPNA VLIGNGGNGG NGGKAGGTPG AGGTSGLIIG ENGLNGL     837
```

<212> Type : PRT

<211> Length : 837
SequenceName : SEQ ID 159
SequenceDescription :
Sequence --------
<213> OrganismName : Mycobacterium tuberculosis H37Rv
<400> PreSequenceString :

```
MSFVIAVPET IAAAATDLAD LGSTIAGANA AAAANTTSLL AAGADEISAA IAALFGAHGR     60
AYQAASAEAA AFHGRFVQAL TTGGGAYAAA EAAAVTPLLN SINAPVLAAT GRPLIGNGAN    120
GAPGTGANGG DAGWLIGNGG AGGSGAKGAN GGAGGPGGAA GLFGNGGAGG AGGTATANNG    180
IGGAGGAGGS AMLFGAGGAG GAGGAATSLV GGIGGTGGTG GNAGMLAGAA GAGGAGGFSF    240
STAGGAGGAG GAGGLFTTGG VGGAGGQGHT GGAGGAGGAG GLFGAGGMGG AGGFGDHGTL    300
GTGGAGGDGG GGGLFGAGGD GGAGGSGLTT GGAAGNGGNA GTLSLGAAGG AGGTGGAGGT    360
VFGGGKGGAG GAGGNAGMLF GSGGGGGTGG FGFAAGGQGG VGGSAGMLSG SGGSGGAGGS    420
GGPAGTAAGG AGGAGGAPGL IGNGGNGGNG GESGGTGGVG GAGGNAVLIG NGGEGGIGAL    480
AGKSGFGGFG GLLLGADGYN APESTSPWHN LQQDILSFIN EPTEALTGRP LIGNGDSGTP    540
GTGDDGGAGG WLFGNGGNGG AGAAGTNGSA GGAGGAGGIL FGTGGAGGAG GVGTAGAGGA    600
GGAGGSAFLI GSGGTGGVGG AATTTGGVGG AGGNAGLLIG AAGLGGCGGG AFTAGVTTGG    660
AGGTGGAAGL FANGGAGGAG GTGSTAGGAG GAGGAGGLYA HGGTGGPGGN GGSTGAGGTG    720
GAGGPGGLYG AGGSGGAGGH GGMAGGGGGV GGNAGSLTLN ASGGAGGSGG SSLSGKAGAG    780
GAGGSAGLFY GSGGAGGNGG YSLNGTGGDG GTGGAGQITG LRSGFGGAGG AGGASDTGAG    840
GNGGAGGKAG LYGNGGDGGA GGDGATSGKG GAGGNAVVIG NGGNGGNAGK AGGTAGAGGA    900
GGLVLGRDGQ HGLT                                                      914
```

<212> Type : PRT
<211> Length : 914
SequenceName : SEQ ID 160
SequenceDescription :
Sequence --------
<213> OrganismName : Mycobacterium tuberculosis H37Rv
<400> PreSequenceString :

```
MSLVIVTPET VAAAASDVAR IGSSIGVANS AAAGSTTSVL AAGADEVSAA IATLFGSHAR     60
EYQAISTQVA AFHDRFAQTL SAAVGSYVSA EATNAAPLAT LEHNVLNALN APTQALLGRP    120
LIGDGAAGAP GTGQAGGAGG ILWGNGGAGG SGAPGQVGGA GGAAGLFGTG GAGGAGGAGA    180

AGGAGGSGGW LLGNGGVGGA GGQSLLGGAT GGAGGNAGLF GVGGTGGPGG PGGPGGVGGT    240
GGAGGLGGTL YGAGGHGGAG GPGPIGGVGG HGGVGGAAGL LGVGGHGGAG GHGAEGVAGA    300
AGEDLSPHGT SGGVGGDAGD GGTGGRGGWL AGAGGAGGAG GVGGTGGAGG AGFSRALIVA    360
GDNGGDGGNG GMGGAGGAGG PGGAGGLISL LGGQGAGGAG GTGGAGGVGG DRGAGGPGNQ    420
AFNAGAGGAG GHGGDPGAGG AGGTGGAGSI TGAQGAIGAT PTSGGNGGAG GNGANATTAG    480
TNGANGGPGG HGGLVGNGGA GGNGANGAAG TNASDSGAVG GKGNSGGNGG QGGAGGDGGT    540
LAGNGGAGGT GGRGADGGLG GSGAEGANAT TAGERGQDGG KGGNGGVGGT GGNAVAPGAN    600
GGHGGNGGNP GFSGAGGLGG LSGDGVTRAA QGATPDFADT GGKGGNGGNG ANAVAPGGTG    660
ASGGAGGNAG AGGKGGENII GDGGGGNGGA GGKGGAGTLL GLTVFGDNGG AGVLGDSTDP    720
DGSGGAGGAG GAGGAGGDPT I                                              741
```

<212> Type : PRT
<211> Length : 741
SequenceName : SEQ ID 161
SequenceDescription :
Sequence
--------
<213> OrganismName : Mycobacterium tuberculosis H37Rv
<400> PreSequenceString :

```
MSFVTAAPEM LATAAQNVAN IGTSLSAANA TAAASTTSVL AAGADEVSQA IARLFSDYAT    60
HYQSLNAQAA AFHHSFVQTL NAAGGAYSSA EAANASAQAL EQNLLAVINA PAQALFGRPL   120
IGNGANGTAA SPNGGDGGIL YGNGGNGFSQ TTAGVAGGAG GSAGLIGNGG NGGAGGAGAA   180
GGAGGAGGWL LGNGGAGGPG GPTDVPAGTG GAGGAGGDAP LIGWGGNGGP GGFAAFGNGG   240
AGGNGGASGS LFGVGGAGGV GGSSEDVGGT GGAGGAGRGL FLGLGGDGGA GGTSNNNGGD   300
GGAGGTAGGR LFSLGGDGGN GGAGTAIGSN AGDGGAGGDS SALIGYAQGG SGGLGGFGES   360
TGGDGGLGGA GAVLIGTGVG GFGGLGGGSN GTGGAGGAGG TGATLIGLGA GGGGGIGGFA   420
VNVGNGVGGL GGQGGQGAAL IGLGAGGAGG AGGATVVGLG GNGGDGGDGG GLFSIGVGGD   480
GGNAGNGAMP ANGGNGGNAG VIANGSFAPS FVGFGGNGGN GVNGGTGGSG GILFGANGAN   540
GPS                                                                 543
```

<212> Type : PRT
<211> Length : 543
SequenceName : SEQ ID 162
SequenceDescription :
Sequence
--------
<213> OrganismName : Mycobacterium tuberculosis H37Rv
<400> PreSequenceString :

```
MSYVLATPEM VAAAANNLAQ IGSTLSAANA AALAPTTGVL AAGADEVSAA VASLFSGHAQ    60
AYQTLGTQAA AFHERFIQAL STAAGAYGSA EAANASPLQQ ALNVINAPTQ TLLGRPLIGN   120
GTNGAPGTGQ AGGPGGLLYG NGGNGGSGGV GQAGGAGGSA GLIGIGGTGG AGGAGAVGGV   180
GGNGGWLYGN GGAGGLGGTG VAGVNGGMGA AGGAGGNAYL FGSGGAGGQG GMGAAGADGV   240
NPTPTGTADA GSTGTDQTLG GNAIGGNGGP GDAGDAMTSG GAGGSGGNAV STVNGDAVGG   300
EGGKGGEGAY GGAGGAGGSA ASIGNAAIGG NGGAGGNAQA PGGVGGAGGE GGDAQVGTNS   360
PSNAEAGNGG SGGNGFDSFA SGGTGGAGGT GGAGGRGGLL IGDGGAGGAG GVGGTGGSGA   420
PGGGGGAGGD GGAANTDSAG SSRKAFGGDG GVGGDGASAL GTGGEGGIGG QGGNGGAGGL   480
LIGNGGAGGV GGTAGAGGTG GSGGAGGAGG AGGGGTNSGP GAAFGGNGNT GGNGGNGGAP   540
GALGGKGGSG GLIGRAGSDG GVGAGGAGGA GGAGGTGGEG GTGGDGKTTD GNPGMGGSPG   600
SAGQPG                                                              606
```

<212> Type : PRT
<211> Length : 606
SequenceName : SEQ ID 163
SequenceDescription :
Sequence --------
<213> OrganismName : Mycobacterium tuberculosis H37Rv
<400> PreSequenceString :

```
MSFLFAQPEM LGAAATDLAS IGSAISTANA AAAAATTRVL AAGADEVSAA VAALFSGHAQ    60
TYQALRTQAA AFHQQIVQTL TSTAGAYASA EAANVEQQLL GAINAPTMAL LGRPLIGHGA   120
DGAPGTGQAG GAGGILYGNG GNGGSGATGQ AGGAGGAAGL IGHGGAGGLG GTGASGGAGG   180
AGGWLWGNGG AGGNGGVGVA GDPGGVGGAG GAGGAAGLWG SGGSGGTGGQ GGVGGGKSGD   240
GGTGGIGGAG GGGGWLHGDG GAGGHGGQGG TGVSSGGNGG AGGTGGDGRG LSGSGGAGGR   300
GGQTGVGGKV GENNFGGAGG AGGTGGLIGN GGAGGNGGQG AISGAGGAGG NAWLIGDGGA   360
GGNGGDIRGQ GGGAGGAGGA GGQLIGNGGT GGAGGTVTSP NGLGGAGGAG GSAGLIGHGG   420
TGGAGGHSAQ GPDGNGGIGG AGGAGGNGGQ LYGTGGTGGT GGKGGDGFGV FGKGGAGGTG   480
GRGGAAGLIG DAGTGGTGGK GGTAGEDGTG GNGGTGGNGG AAVLIGNGGG GGAGGNGGAG   540

NDGTPGNGGG GGVGGTGGTL FGQPGQPGPP GQPGPA                             576
```

<212> Type : PRT
<211> Length : 576
SequenceName : SEQ ID 164
SequenceDescription :
Sequence --------
<213> OrganismName : Mycobacterium tuberculosis H37Rv
<400> PreSequenceString :

MWTSQMIVAP AFVDAAAKDL ATIGSAISRA NAEALVPITA LLPAGADDVS AAIAALFATH 60
GQAYQELSAH AVAFHEQFVQ LMSAGAAQYA SAEAANSSPL QIVGQTALDA INSPVQTLTG 120
RPLIGNGANG VAGTGQNGGD GGWLYGNGGN GGSGGTGQNG GNGGSAGLWG SGGNGGQGGA 180
GANGAAGQPG KAGGSGGNGG AGGWIYGHGG HGGAGGNGGN ATAPGGASAG FDGGAGGNGG 240
SGGRGGLLFG NGGNGSVGGM GGQGTNDTAG DSAGSGGLGG NGGNGAQGGW LIGNGGQGGD 300
SGAGGGTDST QTGVMNGASG GSAGIAGNGG DAGLVGNGGA GGNGGNGAAG SALGTTIFGG 360
SGGVGGSGGD GGNGGWLFGS GASGGNGGQG GDAGTNGFAG FGGSAGGGGW VGAVNFGPIS 420
VQGFGLFGHG GDGGNGGDVG AGSLSIQFGA SGGDGGQGGV LYGNGGNGGN AGSGGGTGFE 480
GSAGQGGAAI LIGNGGAGGN GATGGTGVGN IIQEAGGDGS DGGAGGSGGL LFGSGGAGGI 540
GGAGGVGGSG NDGGNGGDGG QGGASGLGIG NGGPGGSGGT GGAGGTGGSA GTGGAGGDGG 600
NAALLIGTGG DGGDGVPPAP GGQGGKGGLI GLPGQNGQP 639

<212> Type : PRT
<211> Length : 639
SequenceName : SEQ ID 165
SequenceDescription :
Sequence --------
<213> OrganismName : Mycobacterium tuberculosis H37Rv
<400> PreSequenceString :

MSWVMVSPEL VVAAAADLAG IGSAISSANA AAAVNTTGLL TAGADEVSTA IAALFGAQGQ 60
AYQAASAQAA AFYAQFVQAL SAGGGAYAAA EAAAVSPLLA PINAQFVAAT GRPLIGNGAN 120
GAPGTGANGG PGGWLIGNGG AGGSGAPGAG AGGNGGAGGL FGSGGAGGAS TDVAGGAGGA 180
GGAGGNAGML FGAAGVGGVG GFSNGGATGG AGGAGGAGGL FGAGRERGSG GSGNLTGGAG 240
GAGGNAGTLA TGDGGAGGTG GASRSGGFGG AGGAGGDAGM FFGSGGSGGA GGISKSVGDS 300
AAGGAGGAPG LIGNGGNGGN GGASTGGGDG GPGGAGGTGV LIGNGGNGGS GGTGATLGKA 360
GIGGTGGVLL GLDGFTAPAS TSPLHTLQQD VINMVNDPFQ TLTGRPLIGN GANGTPGTGA 420
DGGAGGWLFG NGGNGGQGTI GGVNGGAGGA GGAGGILFGT GGTGGSGGPG ATGLGGIGGA 480
GGAALLFGSG GAGGSGGAGA VGGNGGAGGN AGALLGAAGA GGAGGAGAVG GNGGAGGNGG 540
LFANGGAGGP GGFGSPAGAG GIGGAGGNGG LFGAGGTGGA GGGSTLAGGA GGAGGNGGLF 600
GAGGTGGAGS HSTAAGVSGG AGGAGGDAGL LSLGASGGAG GSGGSSLTAA GVVGGIGGAG 660
GLLFGSGGAG GSGGFSNSGN GGAGGAGGDA GLLVGSGGAG GAGASATGAA TGGDGGAGGK 720
SGAFGLGGDG GAGGATGLSG AFHIGGKGGV GGSAVLIGNG GNGGNGGNSG NAGKSGGAPG 780
PSGAGGAGGL LLGENGLNGL M 801

<212> Type : PRT
<211> Length : 801
SequenceName : SEQ ID 166
SequenceDescription :
Sequence --------
<213> OrganismName : Mycobacterium tuberculosis H37Rv
<400> PreSequenceString :

GQSYQAVSAQ AAAFHDRFVQ LLNAGGGSYA SAEIANAQQN LLNAVNAPTQ TLLGRPLVGD 60
GADGASGPVG QPGGDGGILW GNGGNGGDST SPGVAGGAGG SAGLIGNGGR GGNGAPGGAG 120
GNGGLGGLLL GNGGAGGVGG TGDNGVGDLG AGGGGGDGGL GGRAGLIGHG GAGGNGGDGG 180
HGGSGKAGGS GGSGGFGQFG GAGGLLYGNG GAAGSGGNGG DAGTGVSSDG FAGLGGSGGR 240
GGDAGLIGVG GGGGGNGGDP GLGARLFQVG SRGGDGGVGG WLYGDGGGGG DGGNGGLPFI 300
GSTNAGNGGS ARLIGNGGAG GSGGSGAPGS VSSGGVGGAG NPGGSGGNGG VWYGNGGAGG 360
AAGQGGPGMN TTSPGGPGGV GGHGGTAILF GDGGAGGAGA AGGPGTPDGA AGPGGSGGTG 420
GLLFGVPGPS GPDG 434

<212> Type : PRT
<211> Length : 434
SequenceName : SEQ ID 167
SequenceDescription :
Sequence
--------
<213> OrganismName : Mycobacterium tuberculosis H37Rv
<400> PreSequenceString :

```
MAHFSVLPPE INSLRMYLGA GSAPMLQAAA AWDGLAAELG TAASSFSSVT TGLTGQAWQG   60
PASAAMAAAA APYAGFLTTA SAQAQLAAGQ AKAVASVFEA AKAAIVPPAA VAANREAFLA  120
LIRSNWLGLN APWIAAVESL YEEYWAADVA AMTGYHAGAS QAAAQLPLPA GLQQFLNTLP  180
NLGIGNQGNA NLGGGNTGSG NIGNGNKGSS NLGGGNIGNN NIGSGNRGSD NFGAGNVGTG  240
NIGFGNQGPI DVNLLATPGQ NNVGLGNIGN NNMGFGNTGD ANTGGGNTGN GNIGGGNTGN  300
NNFGFGNTGN NNIGIGLTGN NQMGINLAGL LNSGSGNIGI GNSGTNNIGL FNSGSGNIGV  360
FNTGANTLVP GDLNNLGVGN SGNANIGFGN AGVLNTGFGN ASILNTGLGN AGELNTGFGN  420
AGFVNTGFDN SGNVNTGNGN SGNINTGSWN AGNVNTGFGI ITDSGLTNSG FGNTGTDVSG  480
FFNTPTGPLA VDVSGFFNTA SGGTVINGQT SGIGNIGVPG TLFGSVRSGL NTGLFNMGTA  540
ISGLFNLRQL LG                                                      552
```

<212> Type : PRT
<211> Length : 552
SequenceName : SEQ ID 168
SequenceDescription :
Sequence --------
<213> OrganismName : Mycobacterium tuberculosis H37Rv
<400> PreSequenceString :

```
MSFLIASPEA LAATATYLTG IGSAISAANA VAAAPTTEIL AAGTDEVSTA ISALFGAHAQ   60
AYQALSAHVA AFHDQFVHTL TAGAGSYMAA EAAAASPLQA LQLELLNAIN APTLALLGRP  120
LIGDGTDAAP GSGGAGGAGG ILIGNGGTGG ASDLAGTGRG GVGGAGGAGG LFGIGGAGGG  180
CGSAVAIGGD GGAGGAGGVF SGGGAGGAGD AIGGSGGAGG TGGLLGGGGG AGGAGGAGGN  240
GGGASNSASI GGDGGSGGAG GMLYGAGGVG GNGGAAVAIG GDGGAGGRAG AIGNGGDGGN  300
GGTSNTPGGS GGDGGNGGNA GLIGNGGNGG NAEIVISGGS VAGTGGNGGL LLGFNGTNGL  360
P                                                                  361
```

<212> Type : PRT
<211> Length : 361
SequenceName : SEQ ID 169
SequenceDescription :
Sequence --------
<213> OrganismName : Mycobacterium tuberculosis H37Rv
<400> PreSequenceString :

```
AQASPAAHGG SGGAGGNGGA GSAGNGGAGG AGGNGGAGGN GGGGDAGNAG SGGNGGKGGD   60
GVGPGSTGGA GGKGGAGANG GSSNGNARGG NAGNGGHGGA GGSGDTGGAG GAGGQGGFGG  120
TGGSGSGIGG GAGGNGGNGG AGGTGVVLGG KGGDGGNGDH GGPATNPGSG SRGGAGGSGG  180
NGGAGGNATG SGGKGGAGGN GGDGSFGATS GPASIGVTGA PGGNGGKGGA GGSNPNGSGG  240
DGGKGGNGGA GGNGGSIGAN SGIVGGSGGA GGAGGAGGNG SLSSGEGGKG GDGGHGGDGV  300
GGNSSVTQGG SGGGGGAGGA GGSGFFGGKG GFGGDGGQGG PNGGGTVGTV AGGGGNGGVG  360
GRGGDGVFAG AGGQGGLGGQ GGNGGGSTGG NGGLGGAGGG GGNAPDGGFG GNGGKGGQGG  420
IGGGTQSATG LGGDGGDGGD GGNGGNSGAK AGGAGGKGQA GQPNSGTEPG FGGDGGLGGA  480
GATP                                                               484
```

<212> Type : PRT
<211> Length : 484
SequenceName : SEQ ID 170
SequenceDescription :
Sequence --------
<213> OrganismName : Rickettsia prowazekii
<400> PreSequenceString :

```
MKKSKILRKF LATASLCGTL FTNSNATGTI IPNNGSVSLN TDAGLVGGVF NNGDIIQIVN     60
GGREIKISAD KANAIIGGIN TLKELPDFGG VEVSQNVSIG PLNAGEDLNT NFGPLKFISN    120
NVTSIITGVG TKTFSNIDFA GKNATLQINK DLNITTKIDN TVAGNNGSIT FEGSGIISNH    180
IGYTNSLLGI NVGNGEAKIY APEANNITIN AKNINLTHNN SILTLCDGNI TTLKGNINNT    240
TEIDGQGILN LAYDLGSSSI ITGDIGNIGS LDTINVLLGS ATFNSTILKA TNINLKHNTS    300
TLNLDDNIIV IGNIKGNNNK DILNFKVHGT NLDNEMIIPA PQKTHGTLNF KGNATLNGNI    360
NNLNILKFSG GHGKTLNLQG NTKVDNLVFA DSVLDSGTIS VNGLLDTDCV TFNNSNVNGG    420
TLIINAKNTI SAKLLNATKA KIQINANLTM NHPSAGDISD IRIADNTIYT IDAKNGNVNL    480
LNNNAKIIFE GADSMLALIN TGVTADRTFT IYNNLNQSGN DEYGIVKIEA IKKVITIANQ    540
SGPYTIGQDN THRLKELIVE GAGDIIIDDT IFTKLLSINS TGQITFNRTL DLGAGGNIAF    600
GKHGTLVVNG VTGSITTSEN NQGILTINSG NITGVIGTNE LGLKLVNIGA DPVTCSANVF    660

ASVALTNPSS VLILADGVTL TGEVTTHNNT KGVLSLGTGS NITGQIGTNS AALEKINIGA    720
GASNIDSNIY AGSTVLTDQT SELTLNNDVV VNSNIITTAG NNSGKLIFTG NGGITGNIGA    780
NGAALQEVVF NGTTNIGGTA NSQNFTVAHS AANVVITGLT TGALKYKDTG TIIAHGGLVG    840
DIDFNNKAGK FILGDGAMID GSVLCNGGVA GTLDFIGDGN VTQNIGADNA NSISTINIQG    900
DNTKNVTIAN DIFVDNIHFT NGGILQLGGN LTTHNIDFGA NGGTLEFNGN NTYNLNAIIV    960
NGQNGILNAF TNLKASDDTI GTVKIINIGQ IGTPQNFTIQ VNNKNLTLVS SVNSSINFGD   1020
ANSQLILSAP VDQTIKFINN LNETGGGIIT LDSNGNNLTI SGNNGIKLGS KGNELSSLNI   1080
KGKVTVTNDL DIQNIHQLNI NNGALFDDQS LTSAKIKNIN IGTVAGGATY TLDAINDNFD   1140
LNTSGMVFKH QDSILELKNS SNTNDHTITL TSALDPGNNQ FGIIKLITDT NKLTIDNNGN   1200
VAYTLGTANH MLKQLTFASI DNGAIALKVG INVENVTLNI KDIELNEVNA NVLFNKNTTY   1260
TATGNINGHV DFQGNAGVIN LNDDIEIDGS VTSTGNVNGT LNFNGSGKVT GLINNIVMLQ   1320
AGAGDVSLSA SGNYSITEIQ GNGNNNLTFA ANSHLTTDIN KTGGQDLNLV FINGGSVSGS   1380
IGANAAVGDI IINAGSVNFS NTLKSGNIVI SDGATMQVNN NVTATDISGK NANNGTLKLN   1440
NHTPINITST LGNNNAIGTI EVANNDVTIT GTLQAQNIHF SNATQAATLT LGAASQVTNI   1500
TTAGNNIHTL EVTDFDTGND GIIGDANNRL KSIELTGNGT VTINSPHVYS SITTANNAQG   1560
NVKLNIEGGI TYDLGSKIKS LANVQISEDT TIRGDVYSKY LNIDAGKTIN FDRGDNNMNP   1620
KNLDIPDALI DLDVLPRSLS LFNYFTDIKA DNLNFADDTA TANFKDAVVI DAHIDNGGIL   1680
KFNDNAWLTQ EIKNANIIEI ASDKFMLLQK NIKAATLIAD NANLVLLDNV EVNTNLNVRD   1740
IVLDLANYEL KYTGNVTHNG LLTIITYFDT ALQKGGHILV SQGSNVDMSD LDNLIIKIKA   1800
HSDITNITSD TKHQIVKLET GAIYTPVPQT KVIIDASEEQ NKFVKWVADA NGLVLLTDTG   1860
GRDDTGGRDD TRGRGNTDNG CRDNCDVGNI SNNSSNEAGG SSSDKNYGIT DVVPIFDPSP   1920
ILDYTKNNYV ASGIANQLIN HVKDFGNTTD AGKLLNDLGF MSPNRVTETL DRLSNRINVN   1980
GLNEGVVGLN GIEVENFLTD IAINMDNFTA KEIGNRLEEL SDANTVNGLN KTNTLLNNKI   2040
NLKRLNTNNQ AIIAAGDEDN IVTGIWGMSF YGKIKQNSKN SASGYQSNTG GGIIGFDYNI   2100
DNSIVIGAAY TMADSKVKHK NDKNGDRTKA KSNIYSIYGL YNWLTNNFFV EAIGVYGRNK   2160
IKNYEKRITT ITDQIAIGKF INTFYSYELL GGYNYLISHR TTITPMFGMR YATFKNNGYK   2220
ENNTTFQNLS IKKNYYDKFE TILGLNSVTH YLSQDIIIKP ELHWFINYQC KNKLPNIDAR   2280
LDGIDEPLTT IRFKPAKITY NLGGGISTKN NMIEFGIRYN LSLAKKYTAH QGSLKIKVNL   2340
```

<212> Type : PRT
<211> Length : 2340
SequenceName : SEQ ID 171
SequenceDescription :
Sequence --------
<213> OrganismName : Rickettsia prowazekii
<400> PreSequenceString :

```
MAQKPNFLKK IISAGLVTAS TATIVAGFSG VAMGAAMQYN RTTNAAATTF DGIGFDQAAG     60
ANIPVAPNSV ITANANNPIT FNTPNGHLNS LFLDTANDLA VTINEDTTLG FITNIAQQAK    120
FFNFTVAAGK ILNITGQGIT VQEASNTINA QNALTKVHGG AAINANDLSG LGSITFAAAP    180
SVLEFNLINP TTQEAPLTLG ANSKIVNGGN GTLNITNGFI QVSDNTFAGI KTINIDDCQG    240
LMFNSTPDAA NTLNLQVGGN TINFNGIDGT GKLVLVSKNG AATEFNVTGT LGGNLKGIIE    300
LNTAAVAGKL ISQGGAANAV IGTDNGAGRA AGFIVSVDNG NAATISGQVY AKNMVIQSAN    360
AGGQVTFEHI VDVGLGGTTN FKTADSKVII TENSNFGSTN FGNLDTQIVV PDTKILKGNF    420
IGDVKNNGNT AGVITFNANG ALVSASTDPN IAVTNINAIE AEGAGVVELS GIHIAELRLG    480
NGGSIFKLAD GTVINGPVNQ NALMNNNALA AGSIQLDGSA IITGDIGNGG VNAALQHITL    540
ANDASKILAL DGANIIGANV GGAIHFQANG GTIKLTNTQN NIVVNFDLDI TTDKTGVVDA    600
SSLTNNQTLT INGSIGTVVA NTKTLAQLNI GSSKTILNAG DVAINELVIE NNGSVQLNHN    660
TYLITKTINA ANQGQIIVAA DPLNTNTTLA DGTNLGSAEN PLSTIHFATK AANADSILNV    720
GKGVNLYANN ITTNDANVGS LHFRSGGTSI VSGTVGGQQG HKLNNLILDN GTTVKFLGDT    780
TFNGGTKIEG KSILQISNNY TTDHVESADN TGTLEFVNTD PITVTLNKQG AYFGVLKQVI    840
ISGPGNIVFN EIGNVGIVHG IAANSISFEN ASLGTSLFLP SGTPLDVLTI KSTVGNGTVD    900
NFNAPIVVVS GIDSMINNGQ IIGDKKNIIA LSLGSDNSIT VNANTLYSGI RTTKNNQGTV    960
TLSGGMPNNP GTIYGLGLEN GSPKLKQVTF TTDYNNLGSI IANNVTINDY VTLTTGGIAG   1020
TDFDAKITLG SVNGNANVRF VDSTFSDPRS MIVATQANKG TVTYLGNALV SNIGSLDTPV   1080
ASVRFTGNDS GAGLQGNIYS QNIDFGTYNL TILNSNVILG GGTTAINGEI DLLTNNLIFA   1140
NGTSTWGDNT SISTTLNVSS GNIGQVVIAE DAQVNATTTG TTTIKIQDNA NANFSGTQAY   1200
TLIQGGARFN GTLGAPNFAV TGSNIFVKYE LIRDSNQDYV LTRTNDVLNV VTTAVGNSAI   1260
ANAPGVSQNI SRCLESTNTA AYNNMLLAKD PSDVATFVGA IATDTSAAVT TVNLNDTQKT   1320
QDLLSNRLGT LRYLSNAETS DVAGSATGAV SSGDEAEVSY GVWAKPFYNI AEQDKKGGIA   1380
GYKAKTTGVV VGLDTLASDN LMIGAAIGIT KTDIKHQDYK KGDKTDINGL SFSLYGSQQL   1440
VKNFFAQGNA IFTLNKVKSK SQRYFFESNG KMSKQIAAGN YDNMTFGGNL IFGYDYNAMP   1500
NVLVTPMAGL SYLKSSNENY KETGTTVANK RINSKFSDRV DLIVGAKVAG STVNITDIVI   1560
YPEIHSFVVH KVNGKLSNSQ SMLDGQTAPF ISQPDRTAKT SYNIGLSANI KSDAKMEYGI   1620
GYDFNSASKY TAHQGTLKVR VNF                                           1643
```

<212> Type : PRT
<211> Length : 1643
SequenceName : SEQ ID 172
SequenceDescription :
Sequence --------
<213> OrganismName : Porphyromonas gingivalis W83
<400> PreSequenceString :

```
MARIILEAHD VWEDGTGYQM LWDADHNQYG ASIPEESFWF ANGTIPAGLY DPFEYKVPVN   60
ADASFSPTNF VLDGTASADI PAGTYDYVII NPNPGIIYIV GEGVSKGNDY VVEAGKTYHF  120
TVQRQGPGDA ASVVVTGEGG NEFAPVQNLQ WSVSGQTVTL TWQAPASDKR TYVLNESFDT  180
QTLPNGWTMI DADGDGHNWL STINVYNTAT HTGDGAMFSK SWTASSGAKI DLSPDNYLVT  240
PKFTVPENGK LSYWVSSQEP WTNEHYGVFL STTGNEAANF TIKLLEETLG SGKPAPMNLV  300
KSEGVKAPAP YQERTIDLSA YAGQQVYLAF RHFGCTGIFR LYLDDVAVSG EGSSNDYTYT  360
VYRDNVVIAQ NLTATTFNQE NVAPGQYNYC VEVKYTAGVS PKVCKDVTVE GSNEFAPVQN  420
LTGSAVGQKV TLKWDAPNGT PNPNPGTTTL SESFENGIPA SWKTIDADGD GNNWTTTPPP  480
GGSSFAGHNS AICVSSASYI NFEGPQNPDN YLVTPELSLP NGGTLTFWVC AQDANYASEH  540
YAVYASSTGN DASNFANALL EEVLTAKTVV TAPEAIRGTR VQGTWYQKTV QLPAGTKYVA  600
FRHFGCTDFF WINLDDVEIK ANGKRADFTE TFESSTHGEA PAEWTTIDAD GDGQGWLCLS  660
SGQLGWLTAH GGTNVVASFS WNGMALNPDN YLISKDVTGA TKVKYYYAVN DGFPGDHYAV  720
MISKTGTNAG DFTVVFEETP NGINKGGARF GLSTEANGAK PQSVWIERTV DLPAGTKYVA  780
FRHYNCSDLN YILLDDIQFT MGGSPTPTDY TYTVYRDGTK IKEGLTETTF EEDGVATGNH  840
EYCVEVKYTA GVSPKECVNV TVDPVQFNPV QNLTGSAVGQ KVTLKWDAPN GTPNPNPGTT  900
TLSESFENGI PASWKTIDAD GDGNNWTTTP PPGGTSFAGH NSAICVSSAS YINFEGPQNP  960
DNYLVTPELS LPNGGTLTFW VCAQDANYAS EHYAVYASST GNDASNFANA LLEEVLTAKT 1020
VVTAPEAIRG TRVQGTWYQK TVQLPAGTKY VAFRHFGCTD FFWINLDDVE IKANGKRADF 1080
TETFESSTHG EAPAEWTTID ADGDGQGWLC LSSGQLDWLT AHGGTNVVAS FSWNGMALNP 1140
DNYLISKDVT GATKVKYYYA VNDGFPGDHY AVMISKTGTN AGDFTVVFEE TPNGINKGGA 1200
RFGLSTEANG AKPQSVWIER TVDLPAGTKY VAFRHYNCSD LNYILLDDIQ FTMGGSPTPT 1260
DYTYTVYRDG TKIKEGLTET TFEEDGVATG NHEYCVEVKY TAGVSPKECV NVTVDPVQFN 1320
PVQNLTGSAV GQKVTLKWDA PNGTPNPNPG TTTLSESFEN GIPASWKTID ADGDGNNWTT 1380
TPPPGGTSFA GHNSAICVSS ASYINFEGPQ NPDNYLVTPE LSLPNGGTLT FWVCAQDANY 1440
ASEHYAVYAS STGNDASNFA NALLEEVLTA KTVVTAPEAI RGTRVQGTWY QKTVQLPAGT 1500
KYVAFRHFGC TDFFWINLDD VEIKANGKRA DFTETFESST HGEAPAEWTT IDADGDGQGW 1560
LCLSSGQLGW LTAHGGTNVV ASFSWNGMAL NPDNYLISKD VTGATKVKYY YAVNDGFPGD 1620
HYAVMISKTG TNAGDFTVVF EETPNGINKG GARFGLSTEA NGAKPQSVWI ERTVDLPAGT 1680
KYVAFRHYNC SDLNYILLDD IQFTMGGSPT PTDYTYTVYR DGTKIKEGLT ETTFEEDGVA 1740
TGNHEYCVEV KYTAGVSPKE CVNVTINPTQ FNPVQNLTAE QAPNSMDAIL KWNAPASKRA 1800
EVLNEDFENG IPASWKTIDA DGDGNNWTTT PPPGGSSFAG HNSAICVSSA SYINFEGPQN 1860
PDNYLVTPEL SLPGGGTLTF WVCAQDANYA SEHYAVYASS TGNDASNFAN ALLEEVLTAK 1920
TVVTAPEAIR GTRVQGTWYQ KTVQLPAGTK YVAFRHFGCT DFFWINLDDV VITSGNAPSY 1980
TYTIYRNNTQ IASGVTETTY RDPDLATGFY TYGVKVVYPN GESAIETATL NITSLADVTA 2040
QKPYTLTVVG KTITVTCQGE AMIYDMNGRR LAAGRNTVVY TAQGGHYAVM VVVDGKSYVE 2100
KLAVK                                                             2105
```

<212> Type : PRT
<211> Length : 2105
SequenceName : SEQ ID 173
SequenceDescription :
Sequence --------
<213> OrganismName : Porphyromonas gingivalis W83
<400> PreSequenceString :

```
MKTSERILSY FFLLCAVFSL GSCEGLYAQV TFPNYSPTAA SSIAVCSGEE TLIIDFTVVQ   60
EDSNGIKVNV KLADGVEYVV GTAVVSVTQG NAVTVAETNV SNPNEPVFTV KSADGNNVVE  120
LGTIVKLTIK RRAVCTAWSN AINAAETGFV FKDKVTVTIG DHSDSKESNS YSVNYPNLTI  180
KQPAPQVNKQ IGETIVREFS ITNGSQNPTQ TVYLSIEYPD EAYLTGVGAM TLQAKLGASG  240
TYADLTPTVT NGKVRIYTLS GSSLGPDHLL TNGEIIYLKE TFKLKTCAPV TVYRVGWGCS  300
IDSQCEIKTT AATITMAAGA ANITGYSVTG PDYRSPTFSL CQPFELTIKF SNSGAGGSMG  360
AAFNINTIGR NDYYRPRGFV LHEFIDVKVN GKPVTNFKTD GSELDLRFDG QFTEDPDGPG  420
VGLDDVDGDG FYDDLPVGAT ITITVTVRLK CDQFTACNNA PNDLSDRGLI LKTLYQTSCD  480
RTSWIDPNTW FNLSSTHLYL SRESVQDASH MPTVIEKDTP FDLKIMTSYY SILSSYNNIW  540
YANPNTRYVV EIVFPQGMTM PPKSDIEWTN IKNHPIDGSL VFTPPINLPD ANITTSGNTM  600
TIVSPSQERG FVTLHGVKYD CTNNHEMVVE YKIREVFNYL HFPDCLCPVG PIMCNTAKRY  660
VLGCDPPCGR GAETSVPKIE RADNSLGWTD YTMRTRQSRS NISAYDLAKA LYMDEVNITA  720
TSIQHGTASS LGARFVLATG VDRVETLTPL SADIKIFRDG VQIVSVDGYT TFRSIRRNNN  780
AEQVIDWDFT SILPAGGLLD RDKVDVVTRY RVTSQNAHRV DTQVGREWFF YNSTANVSPI  840
```

WDEANPLTCL ILVPEIYIMG TFVVNGTDPH VISQCTPTDL GRVANHYARR FGSGAFEYAN 900
EYRPGVKIRN IYLKVPKSYT LNRVEYSNHR NHSSLGTTMP FEEINHTDVT SQGEYNIYKY 960
QLADNEKAHF NITVKNAYGA ALKVNVSPTC ASSAVATNYD KISYYVDYID YYYYAATQPT 1020
VPNSLDIVAD QSAGSNGIYS VSALNVYNRP ILYTNKPSIA LVNQSGEVEL VGKTGEWKLR 1080
ISNPSSATAP YVWLALPTTS GLTIEKVTDA AGTEMAFTTY SGGKMYRLSE AGVPVGSALD 1140
YTIHFTYSGC SPIALKAMGG WNCSAYPLSL DEYVCSSQVI DLKLKPLPAA MELTEIAVPD 1200
PTAAATLCST LEYIYSIQST DNANVYSPTF SIFPEEGLVV TPNQVQVEYP AGSGNWAALN 1260
VVNNSVNLLQ HPALTTIGYL KGLKEGESND NQRKILVKFY IKTECSFVSG KNFRVRADGR 1320
NACNQNAKGS GLAISTPPIR INGAIEPYTT SASTQLVTTT TSQSDCKAPK RVKVVQTVVG 1380
GETTPKAYLE ITLPLGFKYV TGSYAPDNTH PGGVNASPAG TEEVTLTANG EDKIKINVKA 1440
GLTSGQSFAY TLEMKEDDDN VPACGNHTIE IVNVEEIEGL WCEGVQCAET LVVTGANKFE 1500
FELDKPYLDI TVISAVSTFS GGKENLTIEY KVSNTSTTQP LKPGAVVTLF SDKDNNQVFS 1560
GGDVAVATQE LVAEITNTTP LTQIMKVKGV SSSHTGNLVL TILPKDGCYC EIKSPMVTLN 1620
HLPSNYWIGG TVGKPNEWKE PNNWTNDQVP DAAEDVEFAT EVNNPTDPNN PKSGPAKENL 1580
HLDDIHQNGT AGRVIGNLIN DSDKDLVITT GNQLTINGVV EDNNPNVGTI VVKSSKDNPT 1740
GTLLFANPGN NQNVGGTVEF YNQGYDCADC GMYRRSWQYF GIPVNESDFP YDHVDGNATV 1800
NQWVEPFNGD KWRPAPYAPD TKLQKFKGYQ ITNDVQAQPT GVYSFKGTLC VCDAFLNLTR 1860
TSGVNYSGAN LIGNSYTGAI DIKQGIVFPP EVEQTVYLFN TGTRDQWRKL NGSTVSGYRA 1920
GQYLSVPKNT AGQDNLPDRI PSMHSFLVKM QNGASCTLQI LYDKLLKNTT VNNGNGTQIT 1980
WRSGNSGSAN MPSLVMDVLG NESADRLWIF TDGGLSFGFD NGWDGRKLTE KGLSQLYAMS 2040
DIGNDKFQVA GVPELNNLLI GFDADKDGQY TLEFALSDHF AKGGVFLEDL SRGVTRRVVD 2100
GGSYSFDAKR GDSGARFRLS YDEEWVESAE VSVLVGTAGK RIVITNNSEH ACQANVYTTD 2160
GKLLIRLDVK PGSKSMTEPL VDGVYVVSLQ SPATSSNVRK VVVN 2204

<212> Type : PRT
<211> Length : 2204
SequenceName : SEQ ID 174
SequenceDescription :
Sequence --------
<213> OrganismName : Porphyromonas gingivalis W83
<400> PreSequenceString :

MNKFYKSLLQ SGLAAFVSMA TALTASAQIS FGGEPLSFSS RSAGTHSFDD AMTIRLTPDF 60
NPEDLIAQSR WQSQRDGRPV RIGQVIPVDV DFASKASHIS SIGDVDVYRL QFKLEGAKAI 120
TLYYDAFNIP EGGRLYIYTP DHEIVLGAYT NATHRRNGAF ATEPVPGSEL IMDYEVSRGG 180
TLPDIKISGA GYIFDKVGGR PVTDNHYGIG EDDSDSDCEI NINCPEGADW QAEKNGVVQM 240
IMVKGQYISM CSGNLLNNTK GDFTPLIISA GHCASITTNF GVTQSELDKW IFTFHYEKRG 300
CSNGTLAIFR GNSIIGASMK AFLPIKGKSD GLLLQLNDEV PLRYRVYYNG WDSTPDIPSS 360
GAGIHHPAGD AMKISILKKT PALNTWISSS GSGGTDDHFY FKYDQGGTEG GSSGSSLFNQ 420
NKHVVGTLTG GAGNCGGTEF YGRLNSHWNE YASDGNTSRM DIYLDPQNNG QTTILNGTYR 480
DGYKPLPSVP RLLLQSTGDQ VELNWTAVPA DQYPSSYQVE YHIFRNGKEI ATTKELSYSD 540
AIDESIIGSG IIRYEVSARF IYPSPLDGVE SYKDTDKTSA DLAIGDIQTK LKPDVTPLPG 600
GGVSLSWKVP FLSQLVSRFG ESPNPVFKTF EVPYVSAAAA QTPNPPVGVV IADKFMAGTY 660
PEKAAIAAVY VMPSAPDSTF HLFLKSNTNR RLQKVTTPSD WQAGTWLRIN LDKPFPVNND 720
HMLFAGIRMP NKYKLNRAIR YVRNPDNLFS ITGKKISYNN GVSFEGYGIP SLLGYMAIKY 780
LVVNTDAPKI DMSLVQEPYA KGTNVAPFPE LVGIYVYKNG TFIGTQDPSV TTYSVSDGTE 840
SDEYEIKLVY KGSGISNGVA QIENNNAVVA YPSVVTDRFS IKNAHMVHAA ALYSLDGKQV 900
RSWNNLRNGV TFSVQGLTAG TYMLVMQTAN GPVSQKIVKQ 940

<212> Type : PRT
<211> Length : 940
SequenceName : SEQ ID 175
SequenceDescription :
Sequence --------
<213> OrganismName : Porphyromonas gingivalis W83
<400> PreSequenceString :

MKNLNKFVSI ALCSSLLGGM AFAQQTELGR NPNVRLLEST QQSVTKVQFR MDNLKFTEVQ 60
TPKGMAQVPT YTEGVNLSEK GMPTLPILSR SLAVSDTREM KVEVVSSKFI EKKNVLIAPS 120
KGMIMRNEDP KKIPYVYGKS YSQNKFFPGE IATLDDPFIL RDVRGQVVNF APLQYNPVTK 180
TLRIYTEITV AVSETSEQGK NILNKKGTFA GFEDTYKRMF MNYEPGRYTP VEEKQNGRMI 240
VIVAKKYEGD IKDFVDWKNQ RGLRTEVKVA EDIASPVTAN AIQQFVKQEY EKEGNDLTYV 300
LLIGDHKDIP AKITPGIKSD QVYGQIVGND HYNEVFIGRF SCESKEDLKT QIDRTIHYER 360
NITTEDKWLG QALCIASAEG GPSADNGESD IQHENVIANL LTQYGYTKII KCYDPGVTPK 420
NIIDAFNGGI SLANYTGHGS ETAWGTSHFG TTHVKQLTNS NQLPFIFDVA CVNGDFLFSM 480
PCFAEALMRA QKDGKPTGTV AIIASTINQS WASPMRGQDE MNEILCEKHP NNIKRTFGGV 540
TMNGMFAMVE KYKKDGEKML DTWTVFGDPS LLVRTLVPTK MQVTAPAQIN LTDASVNVSC 600

DYNGAIATIS ANGKMFGSAV VENGTATINL TGLTNESTLT LTVVGYNKET VIKTINTNGE 660
PNPYQPVSNL TATTQGQKVT LKWDAPSTKT NATTNTARSV DGIRELVLLS VSDAPELLRS 720
GQAEIVLEAH DVWNDGSGYQ ILLDADHDQY GQVIPSDTHT LWPNCSVPAN LFAPFEYTVP 780
ENADPSCSPT NMIMDGTASV NIPAGTYDFA IAAPQANAKI WIAGQGPTKE DDYVFEAGKK 840
YHFLMKKMGS GDGTELTISE GGGSDYTYTV YRDGTKIKEG LTATTFEEDG VAAGNHEYCV 900
EVKYTAGVSP KVCKDVTVEG SNEFAPVQNL TGSAVGQKVT LKWDAPNGTP NPNPNPNPNP 960
NPGTTTLSES FENGIPASWK TIDADGDGHG WKPGNAPGIA GYNSNGCVYS ESFGLGGIGV 1020
LTPDNYLITP ALDLPNGGKL TFWVCAQDAN YASEHYAVYA SSTGNDASNF TNALLEETIT 1080
AKGVRSPEAI RGRIQGTWRQ KTVDLPAGTK YVAFRHFQST DMFYIDLDEV EIKANGKRAD 1140
FTETFESSTH GEAPAEWTTI DADGDGQGWL CLSSGQLDWL TAHGGTNVVS SFSWNGMALN 1200
PDNYLISKDV TGATKVKYYY AVNDGFPGDH YAVMISKTGT NAGDFTVVFE ETPNGINKGG 1260
ARFGLSTEAD GAKPQSVWIE RTVDLPAGTK YVAFRHYNCS DLNYILLDDI QFTMGGSPTP 1320
TDYTYTVYRD GTKIKEGLTE TTFEEDGVAT GNHEYCVEVK YTAGVSPKKC VNVTVNSTQF 1380
NPVKNLKAQP DGGDVVLKWE APSAKKTEGS REVKRIGDGL FVTIEPANDV RANEAKVVLA 1440
ADNVWGDNTG YQFLLDADHN TFGSVIPATG PLFTGTASSD LYSANFEYLI PANADPVVTT 1500
QNIIVTGQGE VVIPGGVYDY CITNPEPASG KMWIAGDGGN QPARYDDFTF EAGKKYTFTM 1560
RRAGMGDGTD MEVEDDSPAS YTYTVYRDGT KIKEGLTETT YRDAGMSAQS HEYCVEVKYT 1620
AGVSPKVCVD YIPDGVADVT AQKPYTLTVV GKTITVTCQG EAMIYDMNGR RLAAGRNTVV 1680
YTAQGGYYAV MVVVDGKSYV EKLAIK 1706

<212> Type : PRT
<211> Length : 1706
SequenceName : SEQ ID 176
SequenceDescription :
Sequence --------
<213> OrganismName : Porphyromonas gingivalis W83
<400> PreSequenceString :

MKRKPLFSAL VILSGFFGSV HPASAQKVPA PVDGERIIME LSEADVECTI KIEAEDGYAN 60
DIWADLNGNG KYDSGERLDS GEFRDVEFRQ TKAIVYGKMA KFLFRGSSAG DYGATFIDIS 120
NCTGLTAFDC FANLLTELDL SKANGLTFVN CGKNQLTKLD LPANADIETL NCSKNKITSL 180
NLSTYTKLKE LYVGDNGLTA LDLSANTLLE ELVYSNNEVT TINLSANTNL KSLYCINNKM 240
TGLDVAANKE LKILHCNNNQ LTALNLSANT KLTTLSFFNN ELTNIDLSDN TALEWLFCNG 300
NKLTKLDVSA NANLIALQCS NNQLTALDLS KTPKLTTLNC YSNRIKDTAM RALIESLPTI 360
TEGEGRFVPY NDDEGGEEEN VCTTEHVEMA KAKNWKVLTS WGEPFPGITA LISIEGESEY 420
SVYAQDGILY LSGMEQGLPV QVYTVGGSMM YSSVASGSAM EIQLPRGAAY VVRIGSHAIK 480
TAMP 484

<212> Type : PRT
<211> Length : 484
SequenceName : SEQ ID 177
SequenceDescription :
Sequence --------
<213> OrganismName : Shigella flexneri 2a str. 2457T
<400> PreSequenceString :

MKRAITLFAV LLMGWSVNAW SFACKTANGT AIPIGGGSAN VYVNLAPVVN VGQNLVVDLS 60
TQIFCHNDYP ETITDYVTLQ RGSAYGGVLS NFSGTVKYSG SSYPFPTTSE TPRVVYNSRT 120
DKPWPVALYL TPVSSAGGVA IKAGSLIAVL ILRQTNNYNS DDFQFVWNIY ANNDVVVPTG 180
GCDVSARDVT VTLPDYPGSV PIPLTVYCAK SQNLGYYLSG TTADAGNSIF TNTASFSPAQ 240
GVGVQLTRNG TIIPANNTVS LGAVGTSAVS LGLTANYART GGQVTAGNVQ SIIGVTFVYQ 300

<212> Type : PRT
<211> Length : 300
SequenceName : SEQ ID 178
SequenceDescription :
Sequence --------
<213> OrganismName : Shigella flexneri 2a str. 2457T
<400> PreSequenceString :

MGIKQHNGNT KADRLAELKI RSPSIQLIKF GAIGLNAIIF SPLLIAADTG SQYGTNITIN 60
DGDRITGDTA DPSGNLYGVM TPAGNTPGNI NLGNDVTVNV NDASGYAKGI IIQGKNSSLT 120
ANRLTVDVVG QTSAIGINLI GDYTHADLGT GSTIKSNDDG IIIGHSSTLT ATQFTIENSN 180
GIGLTINDYG TSVDLGSGSK IKTDGSTGVY IGGLNGNNAN GAARFTATDL TIDVQGYSAM 240
GINVQKNSVV DLGTNSTIKT NGDNAHGLWS FGQVSANALT VDVTGAAANG VEVRGGTTTI 300
GADSHISSAQ GGGLVTSSSD ATINFSGTAA QRNSIFSGGS YGASAQTATA VINMQNTDIT 360

VDRNGSLALG LWALSGGRIT GDSLAITGAA GARGIYAMTN SQIDLTSDLV IDMSTPDQMA 420
IATQHDDGYA ASRINASGRM LINGSVLSKG GLINLDMHPG SVWTGSSLSD NVNGGKLDVA 480
MNNSVWNVTS NSNLDTLALS HSTVDFASHG STAGTFTTLN VENLSGNSTF IMRADVVGEG 540
NGVNNRGDLL NISGSSAGNH VLAIRNQGSE ATTGNEVLTV VKTTDGAASF SASSQVELGG 600
YLYDVRKNGT NWELYASGTV PEPTPNPEPT PAPAQPPIVN PDPTPEPAPT PKPTTTADAG 660
GNYLNVGYLL NYVENRTLMQ RMGDLRNQSK DGNIWLRSYG GSLDSFASGK LSGFDMGYSG 720
IQFGGDKRLS DVMPLYVGLY IDSTHASPDY SGGDGTARSD YMGMYASYMA QNGFYSDLVI 780
KASRQKNSFH VLDSQNNGVN ANGTANGMSI SLEAGQRFNL SPTGYGFYIE PQTQLTYSHQ 840
NEMAMKASNG LNIHLNHYES LLGRASMILG YDITAGNSQL NVYVKTGAIR EFSGDTEYLL 900
NDSREKYSFK GNGWNNGVGV SAQYNKQHTF YLEADYTQGN LFDQKQVNGG YRFSF 955

<212> Type : PRT
<211> Length : 955
SequenceName : SEQ ID 179
SequenceDescription :
Sequence
--------
<213> OrganismName : Shigella flexneri 2a str. 2457T
<400> PreSequenceString :

MSKFVKTAIA AAMVMGVFTS TATIAAGNNG TARFYGTIED SVCSIVPDDH KLEVDMGDIG 60
AEKLKNNGTT TPKSFQIRLQ DCVFDTQETM TTTFTGTVSS ANSGNYYTIF NTDTGAAFNN 120
VSLAIGDSLG TSYKSGMGID QKIVKDTSTN KGKAKQTLNF NAWLVGAADA PDLGNFEANT 180
TFQITYL 187

<212> Type : PRT
<211> Length : 187
SequenceName : SEQ ID 180
SequenceDescription :
Sequence --------
<213> OrganismName : Shigella flexneri 2a str. 2457T
<400> PreSequenceString :

MKIKTLAIVV LSALSLSSAA ALADTTTVNG GTIHFKGEVV NAACAVDAGS VDQTVQLGQV 60
RTASLKQAGA TSSAVGFNIQ LNDCDTTVAT KAAVAFLGTA IDATRTDVLA LQSSAAGSAT 120
NVGVQILDRT GNALTLDGAT FSAQTTLNNG TNTIPFQARY YAIGEATPGA ANADATFKVQ 180
YQ 182

<212> Type : PRT
<211> Length : 182
SequenceName : SEQ ID 181
SequenceDescription :
Sequence --------
<213> OrganismName : Shigella flexneri 2a str. 2457T
<400> PreSequenceString :

```
MASISSLGVG SGLDLSSILD SLTAAQKATL TPISNQQSSF TAKLSAYGTL KSALTTFQTA    60
NTALSKADLF SATSTTSSTT AFSATTAGNA IAGKYTISVT HLAQAQTLTT RTTRDDTKTA   120
IATSDSKLTI QQGDDKDPIT IDISAANSSL SGIRDAINNA KAGVSASIIN VGNGEYRLSV   180
TSNDTGLDNA MTLSVSGDDA LQSFMGYDAS ASSNGMEVSV AAQNAQLTVN NVAIENSSNT   240
ISDALENITL NLNDVTTGNQ TLTITQDTSK VQTAIKDWVN AYNSLIDTFS SLTKYTAVDA   300
GADSQSSSNG ALLGDSTLRT IQTQLKSMLS NTVSSSSYKT LAQIGITTDP SDGKLELDAD   360
KLTAALKKDA SGVGALIVGD GKKTGITTTI GSNLTSWLST TGIIKAATDG VSKTLNKLTK   420
DYNAASDRID AQVARYKEQF TQLDVLMTSL NSTSSYLTQQ FENNSNSK               468
```

<212> Type : PRT
<211> Length : 468
SequenceName : SEQ ID 182
SequenceDescription :
Sequence --------
<213> OrganismName : Shigella flexneri 2a str. 2457T
<400> PreSequenceString :

```
MEGKADNVVL ENGGRLDVLT GHTATNTRVD DGGTLDVRNG GTATTVSMGN GGVLLADSGA    60
AVSGTRSDGK AFSIGGGQAD ALMLEKGSSF TLNAGDTATD TTVNGGLFTA RGGTLAGTTT   120
LNNGAILTLS GKTVNNDTLT IREGDALLQG GALTGNGSVE KSGSGTLTVS NTTLTQKAVN   180
LNEGTLTLND STVTTDVIAQ RGTALKLTGS TVLNGAIDPT NVTLASGATW NIPDNATVQS   240
```

```
VVDDLSHAGQ IHFTSTRTGK FVPATLKVKN LNGQNGTISL RVRPDMAQNN ADRLVIDGGR   300
ATGKTILNLV NAGNSASGLA TSGKGIQVVE AINGATTEEG AFIQGNKLQA GAFNYSLNRD   360
SDESWYLRSE NAYRAEVPLY ASMLTQAMDY DRILAGSRSH QTGVSGENNS VRLSIQGGHL   420
GHDNNGGIAR GATPESSGSY GFVRLEGDLL RTEVAGMSVT AGVYGAAGHS SVDVKDDDGS   480
RAGTVRDDAG SLGGYLNLIH NASGLWADIV AQGTRHSMKA SSDNNDFRVR GWGWLGSLET   540
GLPFSITDNL MLEPQLQYTW QGLSLDDGQD NASYVKFGHG SAQHVRAGFR LGSHHDMNFG   600
KGTSSRDTLR GSAKHSVREL PVNWWVQPSV IRTFSSRGDM SMGTAAAGSN MTFSPSQNGT   660
SLDLQAGLEA RVRENITLGV QASYAHSING SSAEGYNSQA TLNVTF                   706
```

<212> Type : PRT
<211> Length : 706
SequenceName : SEQ ID 183
SequenceDescription :
Sequence --------
<213> OrganismName : Shigella flexneri 2a str. 2457T
<400> PreSequenceString :

```
MAFSQAVSGL NAAATNLDVI GNNIANSATY GFKSGTASFA DMFAGSKVGL GVKVAGITQD    60
FTDGTTTNTG RGLDVAISQN GFFRLVDSNG SVFYSRNGQF KLDENRNLLN TQGLQLTGYP   120
VTGTPPTIQQ GANPTNISIP NTLMAAKTTT TASMQINLNS SDPLPTVTPF SASNADSYNK   180
KGSVTVFDSQ GNAHDMSVYF VKTGDNNWQV YTQDSSDPNS IAKTATTLEF NANGTLVDGA   240
MANNIATGAI NGAEPATFSL SFLNSMQQNT GANNIVATTQ NGYKPGDLVS YQINDDGTVV   300
GNNSNEQTQL LGQIVLANFA NNEGLASEGD NVWSATQSSG VALLGTAGTG NFGTLTNGAL   360
EASNVDLSKE LVNMIVAQRN YKSNAQTIKT QDQILNTRVN LR                       402
```

<212> Type : PRT
<211> Length : 402
SequenceName : SEQ ID 184
SequenceDescription :

Sequence --------
<213> OrganismName : Shigella flexneri 2a str. 2457T
<400> PreSequenceString :

```
MKLVHMASGL AVAIALAACA DKSADIQTPA PAANTSISAT QQPAIQQPNV SGTVWIRQKV   60
ALPPDAVLTV TLSDASLADA PSKVLAQKAV RTEGKQSPFS FVLPFNPADV QPNARILLSA  120
AITVNDKLVF ITDTVQPVIN QGGTKADLTL VPVQQTAVPV QASGGATTTV PSTSPTQVNP  180
SSAVPAPTQY                                                         190
```

<212> Type : PRT
<211> Length : 190
SequenceName : SEQ ID 185
SequenceDescription :
Sequence --------
<213> OrganismName : Shigella flexneri 2a str. 2457T
<400> PreSequenceString :

```
MIIKKSGGRW QLSLLASVVI SAFFLNTAYA WQQEYIVDTQ PGHSTERYTW DSDHQPDYND   60
ILSQRIQSSQ RALGLEVNLA EETPVDVTSS MSMGWNFPLY EQVTTGPVAA LHYDGTTTSM  120
YNEFGDSTTT LTDPLWHASV SSLGWRVDSR LGDLRPWAQI SYNQQFGENI WKAQSGLSRM  180
TATNQNGNWL DVTVGADMLL NQNIAAYAAL TQAENTTNNS DYLYTMGVSA RF           232
```

<212> Type : PRT
<211> Length : 232
SequenceName : SEQ ID 186
SequenceDescription :
Sequence --------
<213> OrganismName : Shigella flexneri 2a str. 2457T
<400> PreSequenceString :

```
MKWCKRGYVL AAMLALASAT IQAADVTITV NGKVVAKPCT VSTTNATVDL GDLYSFSLMS   60
AGAASAWHDV ALELTNCPVG TSRVTASFSG AADSTGYYKN QGTAQNIQLE LQDDSGNTLN  120
TGATKTVQVD DSSQSAHFPL QVRALTVNGG ATQGTIQAVI SITYTYS                167
```

<212> Type : PRT
<211> Length : 167
SequenceName : SEQ ID 187
SequenceDescription :
Sequence --------
<213> OrganismName : Shigella flexneri 2a str. 2457T
<400> PreSequenceString :

```
MKRAPLITGL LLISTSCAYA SSGGCGADST SGATNYSSVV DDVTVNQTDN VTGREFTSAT   60
LSSTNWQYAC SCSAGKAVKL VYMVSPVLTT TGHQTGYYKL NDSLDIKTTL QANDIPGLTT  120
DQVVSVNTRF TQIKSSTVYS AATQTGVCQG DTSRYGPVNI GANTTFTLYV TKPFLGSMTI  180
PKTDIAVIKG AWVDGMGSPS TGDFHDLVKL SIQGNLTAPQ SCKINQGDVI KVNFGFINGQ  240
KFTTRNAMPD GFTPVDFDIT YDCGDTSKIK NSLQMRIDGT TGVVDQYNLV ARRRSSDNVP  300
DVGIRIENLG GGVANIPFQN GILPVDPSGH GTVNMRAWPV NLVGGELETG KFQGTATITV  360
MVR                                                                363
```

<212> Type : PRT
<211> Length : 363
SequenceName : SEQ ID 188
SequenceDescription :
Sequence --------
<213> OrganismName : Shigella flexneri 2a str. 2457T
<400> PreSequenceString :

```
MQKNAAHTYA ISSLLVLSLT GCAWIPSTPL VQGATSAQPV PGPTPVANGS IFQSAQPINY    60
GYQPLFEDRR PRNIGDTLTI VLQENVSASK SSSANASRDG KTNFGFDTVP RYLQGLFGNA   120
RADVEASGGN TFNGKGGANA SNTFSGTLTV TVDQVLVNGN LHVVGEKQIA INQGTEFIRF   180
SGVVNPRTIS GSNTVPSTQV ADARIEYVGN GYINEAQNMG WLQRFFLNLS PM           232
```

<212> Type : PRT
<211> Length : 232
SequenceName : SEQ ID 189
SequenceDescription :
Sequence --------
<213> OrganismName : Shigella flexneri 2a str. 2457T
<400> PreSequenceString :

```
MKRHLNTCYR LVWNHITGAF VVASELARAQ GKRGGVAVAL SLAAVTSLPV LAADIVVHPG    60
ETVNGGTLVN HDNQFVSGTA DGVTVSTGLE LGPDSDENTG GQWIKAGGTG RNTTVTANGR   120
QIVQAGGTAS DTVIRDGGGQ SLNGLAVNTT LDNRGEQWVH GGGKAAGTII NQDGYQTIKH   180
GGLATGTIVN TGAEGGPESE NVSSGQMVGG TAESTTINKN GRQVIWSSGM ARDTLIYAGG   240
DQTVHGEAHN TRLEGGNQYV HNGGTATETL INRDGWQVIK EGGTAAHTTI NQKESCR      297
```

<212> Type : PRT
<211> Length : 297
SequenceName : SEQ ID 190
SequenceDescription :
Sequence --------
<213> OrganismName : Shigella flexneri 2a str. 2457T
<400> PreSequenceString :

```
MMMKTIKHLL CCAIAASALI STGVHAASWK DALSSAASEL GNQNSTTQEG GWSLASLTNL    60
LSSGNQALSA DNMNNAAGIL QYCAKQKLAS VTDAENIKNQ VLEKLGLNSE EQKEDTNYLD   120
GIQGLLKTKD GQQLNLDNIG TTPLAEKVKT KACDLVLKQG LNFIS                  165
```

<212> Type : PRT
<211> Length : 165
SequenceName : SEQ ID 191
SequenceDescription :
Sequence --------
<213> OrganismName : Shigella flexneri 2a str. 2457T
<400> PreSequenceString :

```
MFKGQKTLAA LAVSLLFTAP VYAADEGSGE IHFKGEVIEA PCEIHQDDID KEVELGQVTT    60
SHINQSHHSD AVAVDLLLVN CDLENSSNGS GGKISKVAVT FDSSAKTTGA DPILNNTSTG   120
EATGVGVRLM NKDQSNIVLG TATPDIDLAP TSSEQTLNFF AWMEQIDQAT PVTPGAVTAN   180
ATYVLDYK                                                           188
```

<212> Type : PRT
<211> Length : 188
SequenceName : SEQ ID 192
SequenceDescription :
Sequence --------
<213> OrganismName : Shigella flexneri 2a str. 2457T
<400> PreSequenceString :

```
MSAGSPKFTV RRIAALSLVS LWLAGCSDTS NPPAPVSSVN GNAPANTNSG MLITPPPKMG    60
TTSTAQQPQI QPVQQPQIQA TQQPQIQPVQ PVAQQPVQME NGRIVYNRQY GNIPKGSYSG   120
STYTVKKGDT LFYIAWITGN DFRDLAQRNN IQAPYALNVG QTLQVGNASG TPITGGNAIT   180
QADAAEQGVV IKPAQNSTVA VASQPTITYS ESSGEQSANK MLPNNKPTAT TVTAPVTVPT   240
ASTTEPTVSS TSTSTPISTW RWPTEGKVIE TFGASEGGNK GIDIAGSKGQ AIIATADGRV   300
VYAGNALRGY GNLIIIKHND DYLSAYAHND TMLVREQQEV KAGQKIATMG STGTSSTRLH   360
FEIRYKGKSV NPLRYLPQR                                               379
```

<212> Type : PRT
<211> Length : 379
SequenceName : SEQ ID 193
SequenceDescription :
Sequence --------
<213> OrganismName : Shigella flexneri 2a str. 2457T
<400> PreSequenceString :

```
MIKFLSALIL LLVTTAAQAE RIRDLTSVQG VRQNSLIGYG LVVGLDGTGD QTTQTPFTTQ     60
TLNNMLSQLG ITVPTGTNMQ LKNVAAVMVT ASLPPFGRQG QTIDVVVSSM GNAKSLRGGT    120
LLMTPLKGVD SQVYALAQGN ILVGGAGASA GGSSVQVNQL NGGRITNGAV IERELPSQFG    180
VGNTLNLQLN DEDFSMAQQI ADTINRVRGY GSATALDART IQVRVPSGNS SQVRFLADIQ    240
NMQVNVTPQD AKVVINSRTG SVVMNREVTL DSCAVAQGNL SVTVNRQANV SQPDTPFGGG    300
QTVVTPQTQI DLRQSGGSLQ SVRSSASLNN VVRALNALGA TPMDLMSILQ SMQSAGCLRA    360
KLEII                                                               365
```

<212> Type : PRT
<211> Length : 365
SequenceName : SEQ ID 194
SequenceDescription :
Sequence --------
<213> OrganismName : Shigella flexneri 2a str. 2457T
<400> PreSequenceString :

```
MKRSIIAAAV FSSFFMSAGV FAADVDTGTL TIKGNIAESP CKFEAGGDSV SINMPTVPTT     60
VFEGKAKYST YDDAVGVTSS MLKISCPKEV AGVKLSLITN DKITGNDKAI ASSNDTVGDN    120
SDVLDVSAPF NIESYKTAEG QYAIPFKAKY LKLTDNSVQS GDVLSSLVMR VAQD          174
```

<212> Type : PRT
<211> Length : 174
SequenceName : SEQ ID 195
SequenceDescription :
Sequence --------
<213> OrganismName : Shigella flexneri 2a str. 2457T
<400> PreSequenceString :

```
MAVQKNVIKG ILAGTFALML SGCVTVPDAI KGSSTTPQQD LVRVMSAPQL YVGQEARFGG     60
KVVAVQNQQG KTRLEIATVP LDSGARPTLG EPSRGRIYAD VNGFLDPVDF RGQLVTVVGP    120
ITGAVDGKIG NTPYKFMVMQ VTGYKRWHLT QQVIMPPQPI DPWFYGGRGW PYGYGGWGWY    180
NPGPARVQTV VTE                                                      193
```

<212> Type : PRT
<211> Length : 193
SequenceName : SEQ ID 196
SequenceDescription :
Sequence --------
<213> OrganismName : Shigella flexneri 2a str. 2457T
<400> PreSequenceString :

```
MRNKPFYLLC AFLWLAVSRV LAADSTITIR GYVRDNGCSV AAESTNFTVD LMENAAKQFN     60

NIGATTPVVP FRILLSPCGN AVSAVKVGFT GVADSHNANL LALENTVSAA AGLGIQLLNE    120
QQNQIPLNAP SSAISWTTLT PGKPNTLNFY ARLMATQVPV TAGHINATAT FTLEYQ        176
```

<212> Type : PRT
<211> Length : 176
SequenceName : SEQ ID 197
SequenceDescription :

Sequence --------
<213> OrganismName : Shigella flexneri 2a str. 2457T
<400> PreSequenceString :

```
MKKLTVAALA VTTLLSGSAF AHEAGEFFMR AGSATVRPTE GAGGTLGSLG GFSVTNNTQL     60
GLTFTYMATD NIGVELLAAT PFRHKIGTRA TGDIATVHHL PPTLMAQWYF GDASSKFRPY    120
VGAGINYTTF FDNGFNDHGK EAGLSDLSLK DSWGAAGQVG VDYLINRDWL VNMSVWYMDI    180
DTTANYKLGG AQQHDSVRLD PWVFMFSAGY RF                                 212
```

<212> Type : PRT
<211> Length : 212
SequenceName : SEQ ID 198
SequenceDescription :
Sequence --------
<213> OrganismName : Shigella flexneri 2a str. 2457T
<400> PreSequenceString :

```
MFFKRGKILS AGRLNKKSLG IVMFLSVGLL LAGCSGSKSS DTGTYSGSVY TVKRGDTLYR     60
ISRTTGTSVK ELARLNGISP PYTIEVGQKL KLGGAKSSSS TRKSTAKSTT KTASVTPSSA    120
VPKSSWPPVG QRCWLWPTTG KVIMPYSTAD GGNKGIDISA PRGTPIYAAG AGKVVYVGNQ    180
LRGYGNLIMI KHSEDYITAY AHNDTMLVNN GQSVKAGQKI ATMGSTDAAS VRLHFQIRYR    240
ATAIDPLRYL PPQGSKPKC                                                259
```

<212> Type : PRT
<211> Length : 259
SequenceName : SEQ ID 199
SequenceDescription :
Sequence --------
<213> OrganismName : Shigella flexneri 2a str. 2457T
<400> PreSequenceString :

```
MAQVINTNSL SLITQNNINK NQSALSSSIE RLSSGLRINS AKDDAAGQAI ANRFTSNIKG     60
LTQAARNAND GISVAQTTEG ALSEINNNLQ RIRELTVQAS TGTNSDSDLD SIQDEIKSRL    120
DEIDRVSGQT QFNGVNVLAK DGSMKIQVGA NDGQTITIDL KKIDSDTLGL NGFNVNGGGA    180
VANTAASKAD LVAANATVVG NKYTVSAGYD AAKASDLLAG VSDGDTVQAT INNGFGTAAS    240
ATNYKYDSAS KSYSFDTTTA SAADVQKYLT PGVGDTAKGT ITIDGSAQDV QISSDGKITA    300
SNGDKLYIDT TGRLTKNGSG ASLTEASLST LAANNTKATT IDIGGTSISF TGNSTTPDTI    360
TYSVTGAKVD QAAFDKAVST SGNNVDFTTA GYSVNGTTGA VTKGVDSVYV DNNEALTTSD    420
TVDFYLQDDG SVTNGSGKAV YKDADGKLTT DAETKAATTA DPLKALDEAI SSIDKFRSSL    480
GAVQNRLDSA VTNLNNTTTN LSEAQSRIQD ADYATEVSNM SKAQIIQQAG NSVLAKANQV    540
PQQVLSLLQG                                                          550
```

<212> Type : PRT
<211> Length : 550
SequenceName : SEQ ID 200
SequenceDescription :
Sequence --------
<213> OrganismName : Shigella flexneri 2a str. 2457T
<400> PreSequenceString :

```
MKKIACLSAL AAVLAFTAGT SVAATSTVTG GYAQSDAQGQ MNKMGGFNLK YRYEEDNSPL     60
GVIGSFTYTE KSRTASSGDY NKNQYYGITA GPAYRINDWA SIYGVVGVGY GKFQTTEYPT    120
YKHDTSDYGF SYGAGLQFNP MENVALDFSY EQSRIRSVDV GTWIAGVGYR F            171
```

<212> Type : PRT
<211> Length : 171
SequenceName : SEQ ID 201
SequenceDescription :
Sequence --------

<213> OrganismName : Shigella flexneri 2a str. 2457T
<400> PreSequenceString :

```
MKRNIIGGAF TLASLMLAGH ALAEDGVVNF VGEIVDTTCE VTSDTADQIV PLGKVSKNAF    60
SGVGSLASPQ KFSIKLENCP ATYTQAAVRF DGTEAPGGDG DLKVGTPLTA GNPGDFTGTG   120
QAIAATGVGI RIFNQSDNSQ VKLYNDSAYT AIDAEGKAEM KFIARYVATN ATVTAGTANA   180
DSQFTVEYKK                                                          190
```

<212> Type : PRT
<211> Length : 190
SequenceName : SEQ ID 202
SequenceDescription :
Sequence --------
<213> OrganismName : Shigella flexneri 2a str. 2457T
<400> PreSequenceString :

```
MKKSTLALVV MGIVASASVQ AAEIYNKDGN KLDVYGKVKA MHYMSDNASK DGDQSYIRFG    60
FKGETQINDQ LTGYGRWEAE FAGNKAESDT AQQKTRLAFA GLKYKDLGSF DYGRNLGALY   120
DVEAWTDMFP EFGGDSSAQT DNFMTKRASG LATYRNTDFF GVIDGLNLTL QYQGKNENRD   180
VKKQNGDGFG TSLTYDFGGS DFAISGAYTN SDRTNEQNLQ SRGTGKRAEA WATGLKYDAN   240
NIYLATFYSE TRKMTPITGG FANKTQNFEA VAQYQFDFGL RPSLGYVLSK GKDIEGIGDE   300
DLVNYIDVGA TYYFNKNMSA FVDYKINQLD SDNKLNINND DTVAVGMTYQ F            351
```

<212> Type : PRT
<211> Length : 351
SequenceName : SEQ ID 203
SequenceDescription :
Sequence --------
<213> OrganismName : Shigella flexneri 2a str. 2457T
<400> PreSequenceString :

```
MRKQWLGICI AAGMLAACTS DDGQQQTVSV PQPAVCNGPI VEISGADPRF EPLNATANQD    60
YQRDGKSYKI VQDPSRFSQA GLAAIYDAEP GSNLTASGEA FDPTKLTAAH PTLPIPSYAR   120
ITNLANGRMI VVRINDRGPY GNDRVISLSR AAADRLNTSN NTKVRIDPII VAQDGSLSGP   180
GMACTTVAKQ TYALPAPPDL SGGAGTSSVS GPQGDILPVS NSTLKSEDPT GAPVTSSGFL   240
GAPTTLAPGV LEGSEPTPAP QPVVTASSTT PATSPAMVTP QAASQSASGN FMVQVGAVSD   300
QARAQQYQQQ LGQKFGVPGR VTQNGAVWRI QLGPFASKAE ASTLQQRLQT EAQLQSFITT   360
AQ                                                                  362
```

<212> Type : PRT
<211> Length : 362
SequenceName : SEQ ID 204
SequenceDescription :
Sequence --------
<213> OrganismName : Shigella flexneri 2a str. 2457T
<400> PreSequenceString :

```
MKKKTIYQCV ILFFSLLNIH VGMAGPEQVS MHIYGNVVDQ GCDVATKSAL QNIHIGDFNI    60
SDFQAANTVS TAADLNIDIT GCAAGITGAD VLFSGEADTL APTLLKLTDT GGSGGMATGI   120
AVQILDAQSQ QEIPLNQVQP LTPLKAGDNT LKYQLRYKST KAGATGGNAT AVLYFDLVYQ   180
```

<212> Type : PRT
<211> Length : 180
SequenceName : SEQ ID 205
SequenceDescription :
Sequence --------
<213> OrganismName : Shigella flexneri 2a str. 2457T
<400> PreSequenceString :

MKNKLLFMML TILGAPGIAA AAGYDLANSE YNFAVNELSK SSFNQAAIIG QAGTNNSAQL 60
RQGGSKLLAV VAQEGSSNRA KIDQTGDYNL AYIDQAGSAN DASISQGAYG NTAMIIQKGS 120
GNKANITQYG TQKTAVVVQR QSQMAIRVTQ R 151

<212> Type : PRT
<211> Length : 151
SequenceName : SEQ ID 206
SequenceDescription :
Sequence --------
<213> OrganismName : Shigella flexneri 2a str. 2457T
<400> PreSequenceString :

MMKFKKCLLP VAMLASFTLA GCQSNADDHA ADVYQTDQLN TKQETKTVNI ISILPAKVAV 60
DNSQNKRNAQ AFGALIGAVA GGVIGHNVGS GSNSGTTAGA VGGGAVGAAA GSMVNDKTLV 120
EGVSLTYKEG TKVYTSTQEG KECQFTTGLA VVITTTYNET RIQPNTKCPE KS 172

<212> Type : PRT
<211> Length : 172
SequenceName : SEQ ID 207
SequenceDescription :
Sequence --------
<213> OrganismName : Shigella flexneri 2a str. 2457T
<400> PreSequenceString :

MQTKKNEIWV GIFLLAALLA ALFVCLKAAN VTSIRTESTY TLYATFDNIG GLKARSPVSI 60
GGVVVGRVAD ITLDPKTYLP RVTLEIEQRY NHIPDTSSLS IRTSGLLGEQ YLALNVGFED 120
PELGTAILKD GDTIQDTKSA MVLEDLIGQF LYGSKGDDNK NSGDAPAAAP GNNETTEPVG 180
TTK 183

<212> Type : PRT
<211> Length : 183
SequenceName : SEQ ID 208
SequenceDescription :
Sequence
--------
<213> OrganismName : Shigella flexneri 2a str. 2457T
<400> PreSequenceString :

MAPLAFSAQS LAESLTVEQR LELLEKALRE TQSELKKYKD EEKKKYTPAT VNRSVSTNDQ 60
GYAANPFPTS SAAKPDAVLV KNEEKNASET GSIYSSMTLK DFSKFVKDEI GFSYNGYYRS 120
GWGTASHGSP KSWAIGSLGR FGNEYSGWFD LQLKQRVYNE NGKRVDAVVM IDGNVGQQYS 180
TGWFGDNAGG ENFMQFSDMY VTTKGFLPFA PEADFWVGKH GAPKIEIQML DWKTQRTDAA 240
AGVGLENWKV GPGKIDIALV REDVDDYDRS LQNKQQINTH TIDLRYKDIP LWDKATLMVS 300
GRYVTANESA SEKDNQDNNG YYDWKDTWMF GTSLTQKFDK GGFNEFSFLV ANNSIARNFG 360
RYAGASPFTT FNGRYYGDHT GGTAVRLTSQ GEAYIGDHFI VANAIVYSFG NNIYSYETGA 420
HSDFESIRAV VRPAYIWDQY NQTGVELGYF TQQNKDANSN KFNESGYKTT LFHTFKVNTS 480
MLTSRLEIRF YATYIKALEN ELDGFTFEDN KDAQFAVGAQ AEIWW 525

<212> Type : PRT
<211> Length : 525
SequenceName : SEQ ID 209
SequenceDescription :
Sequence --------
<213> OrganismName : Streptococcus mutans UA159
<400> PreSequenceString :

```
MKKRILSAVL VSGVTLSSAT TLSAVKADDF DAQIASQDSK INNLTAQQQA AQAQVNTIQG      60
QVSALQTQQA ELQAENQRLE AQSATLGQQI QTLSSKIVAR NESLKQQARS AQKSNAATSY     120
INAIINSKSV SDAINRVSAI REVVSANEKM LQQQEQDKAA VEQKQQENQA AINTVAANQE     180
TIAQNTNALN TQQAQLEAAQ LNLQAELTTA QDQKATLVAQ KAAAEEAARQ AAAAQAAAEA     240
KAAAEAKALQ EQAAQAQAAA NNNTQATDVS DQQAAAADNT QAAQTGDSTE QSAAQAVNNS     300
DQESTTATEA QPSASSASTA AVAANTSSAN TYPAGQCTWG VKSLAPWVGN YWGNGGQWAA     360
SAAAAGYRVG STPSAGAVAV WNDGGYGHVA YVTGVQGGQI QVQEANYAGN QSIGNYRGWF     420
NPGSVSYIYP N                                                         431
```

<212> Type : PRT
<211> Length : 431
SequenceName : SEQ ID 210
SequenceDescription :
Sequence --------
<213> OrganismName : Streptococcus mutans UA159
<400> PreSequenceString :

```
MKVKKTYGFR KSKISKTLCG AVLGTVAAVS VAGQKVFADE TTTTSDVDTK VVGTQTGNPA      60
TNLPEAQGSA SKEAEQSQNQ AGETNGSIPV EVPKTDLDQA AKDAKSAGVN VVQDADVNKG     120
TVKTAEEAVQ KETEIKEDYT KQAEDIKKTT DQYKSDVAAH EAEVAKIKAK NQATKEQYEK     180
DMAAHKAEVE RINAANAASK TAYEAKLAQY QADLAAVQKT NAANQAAYQK ALAAYQAELK     240
RVQEANAAAK AAYDTAVAAN NAKNTEIAAA NEEIRKRNAT AKAEYETKLA QYQAELKRVQ     300
EANAANEADY QAKLTAYQTE LARVQKANAD AKAAYEAAVA ANNAKNAALT AENTAIKQRN     360
ENAKATYEAA LKQYEADLAA VKKANAANEA DYQAKLTAYQ TELARVQKAN ADAKAAYEAA     420
VAANNAANAA LTAENTAIKK RNADAKADYE AKLAKYQADL AKYQKDLADY PVKLKAYEDE     480
QASIKAALAE LEKHKNEDGN LTEPSAQNLV YDLEPNANLS LTTDGKFLKA SAVDDAFSKS     540
TSKAKYDQKI LQLDDLDITN LEQSNDVASS MELYGNFGDK AGWSTTVSNN SQVKWGSVLL     600
ERGQSATATY TNLQNSYYNG KKISKIVYKY TVDPKSKFQG QKVWLGIFTD PTLGVFASAY     660
TGQVEKNTSI FIKNEFTFYD EDGKPINFDN ALLSVASLNR ENNSIEMAKD YTGKFVKISG     720
SSIGEKNGMI YATDTLNFRQ GQGGARWTMY TRASEPGSGW DSSDAPNSWY GAGAIRMSGP     780
NNSVTLGAIS STLVVPADPT MAIETGKKPN IWYSLNGKIR AVNVPKVTKE KPTPPVKPTA     840
PTKPTYETEK PLKPAPVAPN YEKEPTPPTR TPDQAEPNKP TPPTYETEKP LEPAPVEPSY     900
EAEPTPPTRT PDQAEPNKPT PPTYETEKPL EPAPVEPSYE AEPTPPTPTP DQPEPNKPVE     960
PTYEVIPTPP TDPVYQDLPT PPSVPTVHFH YFKLAVQPQV NKEIRNNNDI NIDRTLVAKQ    1020
SVVKFQLKTA DLPAGRDETT SFVLVDPLPS GYQFNPEATK AASPGFDVTY DNATNTVTFK    1080
ATAATLATFN ADLTKSVATI YPTVVGQVLN DGATYKNNFT LTVNDAYGIK SNVVRVTTPG    1140
KPNDPDNPNN NYIKPTKVNK NENGVVIDGK TVLAGSTNYY ELTWDLDQYK NDRSSADTIQ    1200
KGFYYVDDYP EEALELRQDL VKITDANGNE VTGVSVDNYT NLEAAPQEIR DVLSKAGIRP    1260
KGAFQIFRAD NPREFYDTYV KTGIDLKIVS PMVVKKQMGQ TGGSYENQAY QIDFGNGYAS    1320
NIIINNVPKI NPKKDVTLTL DPADTNNVDG QTIPLNTVFN YRLIGGIIPA DHSEELFEYN    1380
FYDDYDQTGD HYTGQYKVFA KVDITFKDGS IIKSGAELTQ YTTAEVDTAK GAITIKFKEA    1440
FLRSVSIDSA FQAESYIQMK RIAVGTFENT YINTVNGVTY SSNTVKTTTP EDPTDPTDPQ    1500
DPSSPRTSTV INYKPQSTAY QPSSVQETLP NTGVTNNAYM PLLGIIGLVT SFSLLGLKAK    1560
KD                                                                  1562
```

<212> Type : PRT
<211> Length : 1562
SequenceName : SEQ ID 211
SequenceDescription :
Sequence --------
<213> OrganismName : Streptococcus mutans UA159
<400> PreSequenceString :

MLTELKAVLK KPMLWITMVG VALVPALYNI IFLSSMWDPY GKVSDLPVAV VNKDKTATYE 60
GKKMTIGKDM TDNMVRNKSL DYHFVDSEKA QKGLEKGDYY MIITLPEDLS QNAASVLTDE 120
PKKLTIPYQT SKGHSFVASK MSETAAKTLK ESVSKNITSS YTKSLFKNMS TLKTGLGSAA 180
NASQKIATGS KQLANGSQVM TDNLNLLSNS SQSFAQGTNT LYSGLTAYTG GVGQLSAGLN 240
NLNNGLTAYT NGVGQLANGS SQLSNQSQKL LGGVAQLANG SASIQQLVNA SSQLNQGLIK 300
LSTATGLSEE QVQQFSSLIN QLGTLNQSIQ NYSDNGTATT ANSPDLSTYL SAITTAAQAI 360
VNSGNTSQQT TTNQSNALAA VQATGAYQRL SAEDQSEIAA ALANTGSSTT TTGADANAVS 420
QAQAILNNVQ SIQSALSTLQ TTTANTPTSP SASLTQIKNT ANSVLPSAAT SLTTLSSGLT 480
QAKTALDSQV VPVSTALANG TAQLGSTFST GANSLMTGVG QYTNAVDILN AGANTLAAKN 540
NQLTDGTSQL VNGANQLNSN SGQLTKGTAQ LANGANQIET GAGKLAAGGE SLTAGLTTLS 600
SGSGELSKAL STAKNKLSLV AVDNDNAKTL SSPVTIKHTD KDNVKTNGVG MAPYMMSAAL 660
MVMAISTNTI FRVALSGKQA KTLREWIDQK LAVNGLIAVT GAIILYFGVH IIGLSANFEL 720
KTLGLIILTS ITFMVLVTTL VTWHDKFGSF AALILLLLQL GSSAGTYPLA VTDKFFQVVN 780
PYLPMSYSVS GLRETISMAG TIGNQLLALS LFFLTFAALG LLIARRRIRS VKVA 834

<212> Type : PRT
<211> Length : 834
SequenceName : SEQ ID 212
SequenceDescription :
Sequence --------
<213> OrganismName : Streptococcus mutans UA159
<400> PreSequenceString :

MVSQKNKSKK GQSKTFTLIS NRINLLFFLI VALFTVLLLR LAQMQLYDAK FYKSKLTEST 60
TYTIKTSSPR GQIYDAKGVA LVENEVKEVV AFTRSNTMTA KDIKANAKKL ADMVTLTESK 120
VTKRQKKDYY LADPKNYQKI VKKLPNNKKY DNFGNNLTES KIYANAVKAV PNSAIDYSED 180
EKKIIHIFSQ MNATSVFNTA SLTTGDLTAE QIAVLATSKS DLKGISVKTD WERKTDKNSI 240
TSIIGKVSSQ KTGLPAEEAN NYVKKGYSLN DRVGTSYLEK QYENDLQGSR TVQAIKVNKE 300
GKIISDKTTA KGTKGKNLKL TLDLEFQKGV EQILNQYFNS ELASGNTKYS EGVYAVVLNP 360

NTGAVLSMAG LEHDLKTGEV SSNALGAVTE VFTPGSVVKG ATLTAGWENG VLSGNQVLND 420
QPIQFAGSSP INSWFTNGST PLTASQSLEY SSNTYMVQLA LKLMGQDYHS GMTLSTDGYK 480
EAMEKLRATY AQYGLGVSTG IDLPGESKGY TPEHYDPSNV LTESFGQFDN YTAMQLAQYA 540
AAVANGGKRI APHLVEGIYD NNKTGGLGNL VQSIDTKVLN NVSISSDDMG IIKEGFYNVV 600
NGGSYATGKT LAKGASVPIS AKTGTAEAYV TGDDGKSVYT SNLNVVAYAP SSNPQIAVAV 660
VLPHETDLHG TTSHAITRDI INLYQKMYPM NQ 692

<212> Type : PRT
<211> Length : 692
SequenceName : SEQ ID 213
SequenceDescription :
Sequence --------
<213> OrganismName : Streptococcus mutans UA159
<400> PreSequenceString :

MTVLKYGLGI LLSAIILAII IGGLLFTYYV SSTPKLSEAK LKATNSSLVY DSNNNLIADL 60
GAEKRESISS DSIPMKLVNA VTSIEDHRFF KHRGVDIYRI IGAAWSNLLH KSTQGGSTLD 120
QQLIKLAYFS TKESDQTLKR KAQEVWLSLQ MEKKYTKEEI LTFYVNKVYM GNGNYGMRTA 180
AKSYYGKDLK DLSIAQLATL AGIPQAPTQY DPYAQPKAAT SRRNTVLSQM YKHKKITKRE 240
YDAAVATPIS DGLQELKRSS SYPKYMDNYL KQVISEVKKR TGQDIFSAGM KVYTNVNADA 300
QQYLWNIYNT DEYIAYPDDN FQVASTVMDV TNGKVIAQLG GRHQDTNVSF GTNQAVLTDR 360
DWGSTMKPIS AYGPALESEA FTTTAQMLND SVYYYPGTTT QVYDWDHRYN GWMTIQTAIQ 420
QSRNVPAVRA IDAAGLDTAK GFLSGLGIDY PEMRYSNAIS SNTSSSEQKY GASSEKMAAA 480
YAAFSNGGTY YEPQYVNKIE FKDGTSETYD AKGNRAMKET TAYMMTDMLK TVLTYGTGTE 540
AAIPGLYQAG KTGTSNYDDN ELVEMSEKLG INPYGLGTIA PDENFVGYTP QYSMAVWTGY 600
KNRLMPVYGD SMKIAAQVYR TMMAYLSSSG NSDWTMPDGL YRSGGYLYLN GSSGSNSRYG 660
AAPATSSSSS SSSSSDSNNN DQNNNQTTEA SSDSSSSSSD ATTSSNP 707

<212> Type : PRT
<211> Length : 707
SequenceName : SEQ ID 214

SequenceDescription :
Sequence --------
<213> OrganismName : Streptococcus mutans UA159
<400> PreSequenceString :

```
MKSKTAKITL LSSLALAAFG ATNVFADEAS TQLNSDTVAA PTADTQASEP AATEKEQSPV    60
VAVVESHTQG NTTTTTSQVT SKELEDAKAN ANQEGLEVTE TEAQKQPSVE AADADNKAQA   120
QTINTAVADY QKAKAEFPQK QEQYNKDFEK YQSDVKEYEA QKAAYEQYKK EVAQGLASGR   180
VEKAQGLVFI NEPEAKLSIE GVNQYLTKEA RQKHATEDIL QQYNTDNYTA SDFTQANPYD   240
PKEDTWFKMK VGDQISVTYD NIVNSKYNDK KISKVKINYT LNSSTNNEGS ALVNLFHDPT   300
KTIFIGAQTS NAGRNDKISV TMQIIFYDEN GNEIDLSGNN AIMSLSSLNH WTTKYGDHVE   360
KVNLGDNEFV KIPGSSVDLH GNEIYSAKDN QYKANGATFN GDGADGWDAV NADGTPRAAT   420
AYYGAGAMTY KGEPFTFTVG GNDQNLPTTI WFATNSAVAV PKDPGAKPTP PEKPELKKPT   480
VTWHKNLVVE TKTEEVPPVT PPTTPDEPTP EKPKTPEDPQ SPVVAKSVSF RTARKGEMRV   540
RERDYQPTLP HAGAAKQNGL ATLGAISTAF AAATLIAARK KEN                     583
```

<212> Type : PRT
<211> Length : 583
SequenceName : SEQ ID 215
SequenceDescription :
Sequence --------
<213> OrganismName : Streptococcus mutans UA159
<400> PreSequenceString :

```
MEQKIFSKRK SKIAGLCGAI LTTTVVALAS GTVIEADETI EQPVAAETVS QADGDNPEQT    60
TSVQQETAPQ QTKTSQSSDA TVDSEESATS PSDEQTVSQN DSNSSSQIDQ TIADTNRSDS   120
DHISKTSAAT TEDQEEKVNS AKAQTAAATN NQDTRYSAKD AYGNSNFNKT LTEFGKNANV   180
ADVTYNGVRD EYIVVNDPSA PYVPNANEIA KYLKEYLTEL RNINNIAIPV PSVDQVMQKY   240
AQDRANEEAN EKNGLDHDTN LPIPNNLTWV AEDGHLDMDS SIQSKSQEGY TLASDKATAY   300
YLALNWFSDY FNIYDDPNDG LKSFGHAVSI LSDGGTGMGL GLASGQDNEK GMWYAQLEFG   360
GNDNEDNTND FSSLKNGKGE WVLYYKGSPV KFLPNTTFWY VKKGTSPDAA STPHNSDKPS   420
FQSSKDLDPN FKADNRFQEG KEASVHQAIP ATFKSHRDEV GNKDQNSLSA QLPDTGVQKN   480
NQLALIALGT GLILLSGLLL SKRKSLK                                       507
```

<212> Type : PRT
<211> Length : 507
SequenceName : SEQ ID 216
SequenceDescription :
Sequence --------
<213> OrganismName : Streptococcus mutans UA159
<400> PreSequenceString :

```
MTFEKQKHFS LRKLKFGLVS VAIIAFLFAV TKTAEADETV ITEQRQTSKI NASSQKVENQ    60
TSNQVEAKTD SANKDPQEKT GSVATDAPSM NSANNMSQSD KQNTVNEISS DSQQTKTDEQ   120
TDLPQNSFKQ QSAHVKMTTE AEKTPSHSIN TFVNDGNGNW YYLGADGRNV TGSHTIGGKT   180
MYFAQDGKQV KGAFAQDSDG NKHYYDRDSG EMWTNRFVND QGNWYYLNND GVPVTGSITV   240
NGQSLYFNSD GSQVKGNFVE EDGSLRYYDK NSGDLLRKTS RTINGVNYQF DNDGNARAID   300
KIEVVKTSLV VDSYEFGPSV SKIILEFNHK VTPAVVHAGA MVTTAGVQRK ILNSYVSNAS   360
GHVVYFDSSH YVTLELDIPY DPNDSSRNAS PFIFDSAAFR NNWVNSYTVK VDNLQVQADG   420
SNSSQIISSE QDAINNRFLP TTDRFSERGS YGNFNYAAYQ PEAAIGGEKN PLIVWLHGIG   480
EVGTDINIPL LASNVARLTE DPIQSHFTST GSGGQKGAYV LVPQSSIPWS QNQTASLMAL   540
IKAYVASHPD IDSRRIYLAG VSNGGGMTLD MGVAYPNYFA ALVPIAASYS NQLTDNQITA   600
AALKALKGQP MWLIHTRTDK TISADSSVLP FYKELLQAGA QNKWLSYYET NVGKHHSGVT   660
YNGHWSWIYF LNDQVTGTQN TDNAKNWSGL SGMVATNPTY GGDAKATVNG RTYSNVFDWL   720
NGQRRR                                                              726
```

<212> Type : PRT
<211> Length : 726
SequenceName : SEQ ID 217
SequenceDescription :
Sequence --------
<213> OrganismName : Streptococcus mutans UA159

<400> PreSequenceString :

```
MKIFIKKHQQ SILYYSLSFL LPSFIMFLVL FSKNIYWGSS TTILASDGFH QYVIFDALFR     60
NILHGTDSLF YSFKAGLGFN IFALTSYYLG SFLTPFTYFF NVKNMADAFY LFTLIKFGLI    120
GLSAFYSLGQ IYTKISKSLV LMLSTSYALM SFTSSQLELN NWLDVFILLP LIMLGLQRLV    180
EKRGIFLYFL TLTCLFIQNY YFGFMTAIFL TLWFFTQVSW DIRNRMKRLS DFVLVSIFAT    240
LTSAFMLLPT FLDLKSHGEV LTEQISLFSS DIWYFDFFAK SLLGSYDTTK YGSIPTIYIG    300
LLPLIFAITF FFVKSIKWQV KVAYFLLLAI IIASFIFQPL DLFWQGMHSP NMFLHRYSWA    360
FSLVIVIMAA ETLTRIKDIK LKNFYPAFTF LGVGLLATFL FKDYYNYLTQ VNFILTTIFL    420
VSYFIILFTF FNQLVSYKVI ISFTLIFTSF EIALNTFYQI EGIQTDWNFP SREVYEDNVK    480
EIDNYVKKTK KDNLEFFRTE KQIPQTYNDG MKFNYNSISQ FSSVKNNLSA QLLNSLGYYS    540
QGNHSTISYP NNTILMDSLF SIKYNINNQN PHKFGFHLKQ KNNKLQLYKN FYSLPLALMS    600
NHIYKDVKFD SYPLDNQQKF VNELTDLNLT LFKEIPIISS VGMQVLDNRV TINGSKGNKA    660
QVYYTVKCPA NSQLYISLPN LTVNNKDENV FITTNKHTSS YIIDESYYLF NLGNYKKTQT    720
LIFKLSFPKN KTVSYDLPHI YALDLTAYQK SIKQLKSQTV KTTTKKNKIF TTYVAKKRTS    780
LIYTLPYDKG WFAKQNGKAI KISKAQNGLM KIDVSKGSGK IIMTFVPQGL YQGILLTCLG    840
IFLFVFYQLY YKKFNLK                                                   857
```

<212> Type : PRT
<211> Length : 857
SequenceName : SEQ ID 218
SequenceDescription :
Sequence --------
<213> OrganismName : Streptococcus mutans UA159
<400> PreSequenceString :

```
MKLKHILRIG AVAFASILLL TACGSKTSKK TVTLATVGTT NPFSYEKKGK LTGYDIEVAK     60
EVFKASDKYD VKYQKTEWTS IFSGLDSDKY QIGANNISYT KERANKYLYS NPTASNPLVL    120
VVPKDSDIKS YNDIAGHSTQ VVQGNTTVSM LQKFNKNHEN NQVKLNFTSE DLAHQIRNVS    180
DGKYDFKIFE KISAETIIKE QGLDNLKVID LPSDQKPYVY FIFAQDQKDL QKFVNKRLKK    240
LYENGTLEKL SKKYLGGSYL PDKKDMK                                        267
```

<212> Type : PRT
<211> Length : 267
SequenceName : SEQ ID 219
SequenceDescription :
Sequence --------
<213> OrganismName : Streptococcus mutans UA159
<400> PreSequenceString :

```
MRFLVFLIAF FAAFYKFIET ERIDSNTVAV NPDSLILKRF LKTNQLNGIM IVTGPDGKAQ     60

VFSNQSKVDG SPVSIKDYFP LASLQKLITG VAIQQLIDKG KLSLNTPLSK YYPQIENSEN    120
ITIQNLLTHT SGLADRKEVP QQVLTTQEQQ LDFSLTNYRV TYRKKWKYAN INYALLAGII    180
SQISGQNYAT YVRQHFLTAG KGWHFKKYIQ IKDKSKLAAL SVMDQSTTWD KLSKEVTSTF    240
GAGDYASRPV DYWKFMMAFI NDQFVPVSEY QRSMKMTSKS YYGGLYISQK MLHANGGGFD    300
TYSCFAYSNP KTKQVMVLFI TNGKYKRVKS LAAKAFKLYA DSYALRKNET SK            352
```

<212> Type : PRT
<211> Length : 352
SequenceName : SEQ ID 220
SequenceDescription :
Sequence --------
<213> OrganismName : Streptococcus mutans UA159
<400> PreSequenceString :

MKKKIALAAL SFVSAAVLAA CSSAPGGSSD AAGNKIGDTV KIGYNLELSG DVAAYGQAEK 60
NGANLAVEEI NKAGGIDGKK IKVISKDNKS DNGEASTIST NLATQSKVNA ILGPATSGAT 120
AAAAPNANDA AVPLVTPSGT QDNLTYSKGK VQDYIFRTTF QDSFQGKIIA KYATDNLKAK 180
KVALYYDKSS DYAQGIADAF KKAYKGKITV EDTFQAKDQD FQAALTKFKN KDFDAIVIPG 240
YYTETGLITK QARDMGLTQP ILGPDGFNDE KYVEGAGAAN TNNVHYVSGY STKVALTNKA 300
EKFLKDYKAK YGEEPNMFAA LAYDSVYMIA DAAKDAKTSK DIATNLAKLK NFKGVTGKMT 360
IDKKHNPVKS AVMVGLKDGK EDTATAVEAK 390

<212> Type : PRT
<211> Length : 390
SequenceName : SEQ ID 221
SequenceDescription :
Sequence --------
<213> OrganismName : Streptococcus mutans UA159
<400> PreSequenceString :

MKKLSLLLLV CLSLLGLFAC TSKKTADKKL TVVATNSIIA DITKNIAGNK VVLHSIVPVG 60
RDPHEYEPLP EDVKKTSQAD VIFYNGINLE NGGNAWFTKL VKNAHKKTDK DYFAVSDSVK 120
TIYLENAKEK GKEDPHAWLD LKNGIIYAKN IMKRLSEKDP KNKSYYQKNF QAYSAKLEKL 180
HKVAKEKISR IPTEKKMIVT SEGCFKYFSK AYDIPSAYIW EINTEEEGTP NQIKALVKKL 240
RKSRVSALFV ESSVDDRPMK TVSKDTGIPI AAKIFTDSVA KKGQAGDSYY AMMKWNIDKI 300
ANGLSQ 306

<212> Type : PRT
<211>
Length : 306
SequenceName : SEQ ID 222
SequenceDescription :
Sequence --------
<213> OrganismName : Streptococcus mutans UA159
<400> PreSequenceString :

MFVHTKTKKK RKWQRKVFLL LLLFLLPIVS VLAFIVLFIG GGTAESHDVE ATTGGVKLSA 60
KQFADKTKLG ISEEEAKNAL AFADRLMSRH HFTAQATAGV LAVGFRESGF DVKAVNNSGG 120
VAGFFQWSGW GSSVNGDRWK VASKRELTLE VEVDLMSTEL DGRYADVVKK VGSATDEKQA 180
AKDWSQYYEG VAVSDGQTKA DKIESWATTI CEALKSGGTN YAKVNNTGTS STAIPQGWEN 240
ISAFDGHAYE GSENYPQGQC TWYVYNRAKQ LGVSFSPYMG NGGQWYQVQG YHSSHTPKAH 300
TALSFVNGQA GSDPTYGHVA FVEAVKDDGS ILISEMNVYG QPAMTVAYRT FDAETAKQFW 360
YVEGK 365

<212> Type : PRT
<211> Length : 365
SequenceName : SEQ ID 223
SequenceDescription :
Sequence --------
<213> OrganismName : Streptococcus mutans UA159
<400> PreSequenceString :

MKMKRKLLSL VSVLTILLGA FWVTKIVKAD QVTNYTNTAS ITKSDGTALS NDPSKAVNYW 60
EPLSFSNSIT FPDEVSIKAG DTLTIKLPEQ LQFTTALTFD VMHTNGQLAG KATTDPNTGE 120
VTVTFTDIFE KLPNDKAMTL NFNAQLNHNN ISIPGVVNFN YNNVAYSSYV KDKDITPISP 180
DVNKVGYQDK SNPGLIHWKV LINNKQGAID NLTLTDVVGE DQEIVKDSLV AARLQYIAGD 240
DVDSLDEAAS RPYAEDFSKN VTYQTNDLGL TTGFTYTIPG SSNNAIFISY TTRLTSSQSA 300

GKDVSNTIAI SGNNINYSNQ TGYARIESAY GRASSRVKRQ AETTTVTETT TSSSSETTTS 360
EATTETSSTT NNNSTTTETA TSTTGASTTQ TKTTASQTNV PTTTNITTTS KQVTKQKAKF 420
VLPSTGEQAG LLLTTVGLVI VAVAGVYFYR TRR 453

<212> Type : PRT
<211> Length : 453

SequenceName : SEQ ID 224
SequenceDescription :
Sequence --------
<213> OrganismName : Streptococcus mutans UA159
<400> PreSequenceString :

```
MTFKKLVLGL LSFVAVFTLV ACSSSNSKNL QDDIKEKKKL VVAVSPDYAP FEFKALVNGK   60
DTVVGADIDL AKAIAKELGV KLELSSMSFD NVLSSLKTGK ADIAISGLSY TKERAQAYDF  120
SEAYYKTENA ILIKKSDLNK YTMISSFNNK TKVAVQKGTI EEGLAKNQLK QSNITSLTSM  180
GEAVNELKSG QVDAIDLEKP VAEGYVSQNS DLVLAKVALK TGEGDAKAVA LPKDSGQLVK  240
TVNKVIKKLK KEDKYKQFIS DAVKLTGQQV D                                271
```

<212> Type : PRT
<211> Length : 271
SequenceName : SEQ ID 225
SequenceDescription :
Sequence --------
<213> OrganismName : Streptococcus mutans UA159
<400> PreSequenceString :

```
MKKHFFMTFS LLLAAVFLVA CSNLSDSGQR NWDKINKRGM LKIATAGTLY PQSYHDDHNK   60
LTGYDVEILK EIGKRLGLKV QFTEMGVDGM LTAIKSGQID VANYSLEDGN KNISKFLRTS  120
PYKYSFTSMV VRSKDDSGIH SWSDLKGKKA AGAASTNYMK IAKKLGAKLV VYDNVTNDVY  180
MKDLVNGRTD VIINDYYLQK IAVAAVKDKY AIKINQGLYA NPYSTSFTLS LKNKVLQKKI  240
NKAVKDMRKD GTLTKLSKKF FQGEDVTKKH YNSYKKIDIS DVD                    283
```

<212> Type : PRT
<211> Length : 283
SequenceName : SEQ ID 226
SequenceDescription :
Sequence --------
<213> OrganismName : Streptococcus pneumoniae R6
<400> PreSequenceString :

```
MKLLKKMMQV ALATFFFGLL GTSTVFADDS EGWQFVQENG RTYYKKGALK ETYWRVIDGK   60
YYYFDPLSGE MVVGWQYIPA PHKGVTIGPS PRIEIALRPD WFYFGQDGVL QEFVGKQVLE  120
AKTATNTNKH HGEEYDSQAE KRVYYFEDQR SYHTLKTGWI YEEGYWYYLQ KDGGFDSRIN  180
RLTVGELARG WVKDYPLTYD EEKLKAAPWY YLDPATGWQN LGNKWYYLRS SGAMATGWYQ  240
EGSTWYYLNA SNGDMKTGWF QVNGNWYYAY DSGALAVNTT VGGYYLNYNG EWVK         294
```

<212> Type : PRT
<211> Length : 294
SequenceName : SEQ ID 227
SequenceDescription :
Sequence --------
<213> OrganismName : Streptococcus pneumoniae R6
<400> PreSequenceString :

```
MKLLKKMMQV LLAVFFFGLL ATNTVFANTT GGRFVDKDNR KYYVKDDHKA IYWHKIDGKT   60
YYFGDIGEMV VGWQYLEIPG TGYRDNLFDN QPVNEIGLQE KWYYFGQDGA LLEQTDKQVL  120
EAKTSENTGK VYGEQYPLSA EKRTYYFDNN YAVKTGWIYE DGNWYYLNKL GNFGDDSYNP  180
LPIGEVAKGW TQDFHVTIDI DRSKPAPWYY LDASGKMLTD WQKVNGKWYY FGSSGSMATG  240
WKYVRGKWYY LDNKNGDMKT GWQYLGNKWY YLRSSGAMVT GWYQDGLTWY YLNAGNGDMK  300
TGWFQVNGKW YYAYSSGALA VNTTVDGYSV NYNGEWVQ                          338
```

<212> Type : PRT
<211> Length : 338
SequenceName : SEQ ID 228

SequenceDescription :
Sequence
--------
<213> OrganismName : Streptococcus pneumoniae R6
<400> PreSequenceString :

```
MNKKKMILTS LASVAILGAG FVASQPTVVR AEESPVASQS KAEKDYDAAK KDAKNAKKAV     60
EDAQKALDDA KAAQKKYDED QKKTEEKAAL EKAASEEMDK AVAAVQQAYL AYQQATDKAA    120
KDAADKMIDE AKKREEEAKT KFNTVRAMVV PEPEQLAETK KKSEEAKQKA PELTKKLEEA    180
KAKLEEAEKK ATEAKQKVDA EEVAPQAKIA ELENQVHRLE QELKEIDESE SEDYAKEGFR    240
APLQSKLDAK KAKLSKLEEL SDKIDELDAE IAKLEDQLKA AEENNNVEDY FKEGLEKTIA    300
AKKAELEKTE ADLKKAVNEP EKPAPAPETP APEAPAEQPK PAPAPQPAPA PKPEKPAEQP    360
KPEKTDDQQA EEDYARRSEE EYNRLTQQQP PKAEKPAPAP KTGWKQENGM WYFYNTDGSM    420
ATGWLQNNGS WYYLNSNGAM ATGWLQYNGS WYYLNANGAM ATGWAKVNGS WYYLNANGAM    480
ATGWLQYNGS WYYLNANGAM ATGWAKVNGS WYYLNANGAM ATGWLQYNGS WYYLNANGAM    540
ATGWAKVNGS WYYLNANGAM ATGWVKDGDT WYYLEASGAM KASQWFKVSD KWYYVNGLGA    600
LAVNTTVDGY KVNANGEWV                                                 619
```

<212> Type : PRT
<211> Length : 619
SequenceName : SEQ ID 229
SequenceDescription :
Sequence --------
<213> OrganismName : Streptococcus pneumoniae R6
<400> PreSequenceString :

```
MKILPFIARG TSYYLKMSVK KLVPFLVVGL MLAAGDSVYA YSRGNGSIAR GDDYPAYYKN     60
GSQEIDQWRM YSRQCTSFVA FRLSNVNGFE IPAAYGNANE WGHRARREGY RVDNTPTIGS    120
ITWSTAGTYG HVAWVSNVMG DQIEIEEYNY GYTESYNKRV IKANTMTGFI HFKDLDSGSV    180
GNSQSSASTG GTHYFKTKSA IKTEPLVSAT VIDYYYPGEK VHYDQILEKD GYKWLSYTAY    240
NGSYRYVQLE AVNKNPLGNS VLSSTGGTHY FKIKSAIKTE PLVSATVIDY YYPGEKVHYD    300
QILEKDGYKW LSYTAYNGSR RYIQLEGVTS SQNYQNQSGN ISSYGSNNSS TVGWKKINGS    360
WYHFKSNGSK STGWLKDGSS WYYLKLSGEM QTGWLKENGS WYYLGSSGAM KTGWYQVSGE    420
WYYSYSSGAL AINTTVDGYR VNSDGERV                                       448
```

<212> Type : PRT
<211> Length : 448
SequenceName : SEQ ID 230
SequenceDescription :
Sequence --------
<213> OrganismName : Streptococcus pneumoniae R6
<400> PreSequenceString :

```
MFASKSERKV HYSIRKFSIG VASVAVASLV MGSVVHATEN EGSTQAATSS NMAKTEHRKA     60
AKQVVDEYIE KMLREIQLDR RKHTQNVALN IKLSAIKTKY LRELNVLEEK SKDELPSEIK    120
AKLDAAFEKF KKDTLKPGEK VAEAKKKVEE AKKKAEDQKE EDRRNYPTNT YKTLELEIAE    180
FDVKVKEAEL ELVKEEAKES RNEGTIKQAK EKVESKKAEA TRLENIKTDR KKAEEEAKRK    240
ADAKLKEANV ATSDQGKPKG RAKRGVPGEL ATPDKKENDA KSSDSSVGEE TLPSSSLKSG    300
KKVAEAEKKV EEAEKKAKDQ KEEDRRNYPT NTYKTLDLEI AESDVKVKEA ELELVKEEAK    360
EPRDEEKIKQ AKAKVESKKA EATRLENIKT DRKKAEEEAK RKAAEEDKVK EKPAEQPQPA    420
PATQPEKPAP KPEKPAEQPK AEKTDDQQAE EDYARRSEEE YNRLTQQQPP KTEKPAQPST    480
PKTGWKQENG MWYFYNTDGS MATGWLQNNG SWYYLNANGA MATGWLQNNG SWYYLNANGS    540
MATGWLQNNG SWYYLNANGA MATGWLQYNG SWYYLNSNGA MATGWLQYNG SWYYLNANGD    600
MATGWLQNNG SWYYLNANGD MATGWLQYNG SWYYLNANGD MATGWVKDGD TWYYLEASGA    660
MKASQWFKVS DKWYYVNGSG ALAVNTTVDG YGVNANGEWV N                        701
```

<212> Type : PRT
<211> Length : 701
SequenceName : SEQ ID 231
SequenceDescription :
Sequence --------
<213> OrganismName : Streptococcus pneumoniae R6
<400> PreSequenceString :

```
MKKTTILSLT TAAVILAAYV PNEPILAAYV PNEPILADTP SSEVIKETKV GSIIQQNNIK    60
YKVLTVEGNI GTVQVGNGVT PVEFEAGQDG KPFTIPTKIT VGDKVFTVTE VASQAFSYYP   120
DETGRIVYYP SSITIPSSIK KIQKKGFHGS KAKTIIFDKG SQLEKIEDRA FDFSELEEIE   180
LPASLEYIGT SAFSFSQKLK KLTFSSSSKL ELISHEAFAN LSNLEKLTLP KSVKTLGSNL   240
FRLTTSLKHV DVEEGNESFA SVDGVLFSKD KTQLIYYPSQ KNDESYKTPK ETKELASYSF   300
NKNSYLKKLE LNEGLEKIGT FAFADAIKLE EISLPNSLET IERLAFYGNL ELKELILPDN   360

VKNFGKHVMN GLPKFLTLSG NNINSLPSFF LSGVLDSLKE IHIKNKSTEF SVKKDTFAIP   420
ETVKFYVTSE HIKDVLKSNL STSNDIIVEK VDNIKQETDV AKPKKNSNQG VVGWVKDKGL   480
WYYLNESGSM ATGWVKDKGL WYYLNESGSM ATGWVKDKGL WYYLNESGSM ATGWVKDKGL   540
WYYLNESGSM ATGWVKDKGL WYYLNESGSM ATGWVKDKGL WYYLNESGSM ATGWVKDKGL   600
WYYLNESGSM ATGWVKDKGL WYYLNESGSM ATGWVKDKGL WYYLNESGSM ATGWVKVSGK   660
WYYTYNSGDL LVNTTTPDGY RVNANGEWVG                                    690
```

<212> Type : PRT
<211> Length : 690
SequenceName : SEQ ID 232
SequenceDescription :
Sequence --------
<213> OrganismName : Streptococcus pneumoniae R6
<400> PreSequenceString :

```
MEINVSKLRT DLPQVGVQPY RQVHAHSTGN PHSTVQNEAD YHWRKDPELG FFSHIVGNGC    60
IMQVGPVDNG AWDVGGGWNA ETYAAVELIE SHSTKEEFMT DYRLYIELLR NLADEAGLPK   120
TLDTGSLAGI KTHEYCTNNQ PNNHSDHVDP YPYLAKWGIS REQFKHDIEN GLTIETGWQK   180
NDTGYWYVHS DGSYPKDKFE KINGTWYYFD SSGYMLADRW RKHTDGNWYW FDNSGEMATG   240
WKKIADKWYY FNEEGAMKTG WVKYKDTWYY LDAKEGAMVS NAFIQSADGT GWYYLKPDGT   300
LADRPEFTVE PDGLITVK                                                 318
```

<212> Type : PRT
<211> Length : 318
SequenceName : SEQ ID 233
SequenceDescription :
Sequence --------
<213> OrganismName : Neisseria meningitidis Z2491
<400> PreSequenceString :

```
MTFAYWCILI AYLLPLFCAA YAKKAGGFRF KDNHNPRDFL ARTQGTAARA HAAQQNGFEA    60
FAPFAAAVLT AHATGNAGQA TVNTLAGLFI LFRLAFIWCY IADKAALRSL MWVGGFVCTV   120
GLFVVAA                                                             127
```

<212> Type : PRT
<211> Length : 127
SequenceName : SEQ ID 234 SequenceDescription :
Sequence --------
<213> OrganismName : Neisseria meningitidis Z2491
<400> PreSequenceString :

```
MNKIYRIIWN SALNAWVAVS ELTRNHTKRA SATVKTAVLA TLLFATVQAN ATDEDEEEEL    60
ESVQRSVVGS IQASMEGSGE LETISLSMTN DSKEFVDPYI VVTLKAGDNL KIKQNTNENT   120
NASSFTYSLK KDLTGLINVE TEKLSFGANG KKVNIISDTK GLNFAKETAG TNGDTTVHLN   180
GIGSTLTDTL AGSSASHVDA GNQSTHYTRA ASIKDVLNAG WNIKGVKTGS TTGQSENVDF   240
VRTYDTVEFL SADTKTTTVN VESKDNGKRT EVKIGAKTSV IKEKDGKLVT GKGKGENGSS   300
TDEGEGLVTA KEVIDAVNKA GWRMKTTTAN GQTGQADKFE TVTSGTNVTF ASGKGTTATV   360
SKDDQGNITV MYDVNVGDAL NVNQLQNSGW NLDSKAVAGS SGKVISGNVS PSKGKMDETV   420
NINAGNNIEI SRNGKNIDIA TSMAPQFSSV SLGAGADAPT LSVDDEGALN VGSKDANKPV   480
RITNVAPGVK EGDVTNVAQL KGVAQNLNNR IDNVDGNARA GIAQAIATAG LVQAYLPGKS   540
MMAIGGGTYR GEAGYAIGYS SISDGGNWII KGTASGNSRG HFGASASVGY QW           592
```

<212> Type : PRT
<211> Length : 592
SequenceName : SEQ ID 235

SequenceDescription :
Sequence --------
<213> OrganismName : Neisseria meningitidis Z2491
<400> PreSequenceString :

```
MLLAEGQKSA VTEYYLNHGT WPSNNSDAGV ASTATDIKGK YVKEVKVEKG VITATMLSSG     60
VNNEIKGKKL SLWAKRQAGS VKWFCGQPVE RAANNAANDA VTAATANGNG KIDTKHLPST    120
CRDAASAVCI ETPPTAFYKN T                                              141
```

<212> Type : PRT
<211> Length : 141
SequenceName : SEQ ID 236
SequenceDescription :
Sequence --------
<213> OrganismName : Neisseria meningitidis Z2491
<400> PreSequenceString :

```
MKTTDKRTTE THRKAPKTGR IRFSPAYLAI CLSFGILPQA WAGHTYFGIN YQYYRDFAEN     60
KGKFAVGAKD IEVYNKKGEL VGKSMTKAPM IDFSVVSRNG VAALVGDQYI VSVAHNGGYN    120
NVDFGAEGRN PDQHRFSYQI VKRNNYKPDN SHPYNGDYHM PRLHKFVTDA EPVEMTSDMR    180
GNTYSDKEKY PERVRIGSGH HYWRYDDDKH GDLSYSGAWL IGGNTHMQGW GNNGVVSLSG    240
DVRHANDYGP MPIAGAAGDS GSPMFIYDKT NNKWLLNGVL QTGYPYSGRE NGFQLIRKDW    300
FYDDIYRGDT HTVFFEPRSN GHFSFTSNNN GTGTVTETNE KVSNPKLKVQ TVRLFDESLN    360
ETDKEPVYAA GGVNQYRPRL NNGENLSFID YGNGKLILSN NINQGAGGLY FEGDFTVSPE    420
NNETWQGAGV HISEDSTVTW KVNGVANDRL SKIGKGTLHV QAKGENQGSI SVGDGTVILD    480
QQADDKGKKQ AFSEIGLVSG RGTVQLNADN QFNPDKLYFG FRGGRLDLNG HSLSFHRIQN    540
TDEGAMIVNH NATTTSTVTI TGNESITQPS GKNINRLNYS KEIAYNGWFG EKDTTKTNGR    600
LNLVYQPAAE DRTLLLSGGT NLNGNITQTN GKLFFSGRPT PHAYNHLGSG WSKMEGIPQG    660
EIVWDNDWIN RTFKAENFHI QGGQAVISRN VAKVEGDWHL SNHAQAVFGV APHQSHTICT    720
RSDWTGLTNC VEKTITDDKV IASLTKTDIS GNVSLADHAH LNLTGLATLN GNLSANGDTR    780
YTVSHNATQN GNLSLVGNAQ ATFNQATLNG NTSASGNASF NLSNNAAQNG SLTLSDNAKA    840
NVSHSALNGN VSLADKAVFH FENSRFTGQL SGSKDTALHL KDSEWTLPSG TELGNLNLDN    900
ATITLNSAYR HDAAGAQTGS VSDTPRRRSR RSLLSVTPPT SVESRFNTLT VNGKLNGQGT    960
FRFMSELFGY RSDKLKLAES SEGTYTLAVN NTGNEPVSLD QLTVVEGKDN KPLSENLNFT   1020
LQNEHVDAGA WRYQLIRKDG EFRLHNPVKE QELSDKLGKA EAKKQAEKDN AQSLDALIAA   1080
GRDAAEKTES VAEPARQAGG ENVGIMQAEE EKKRVQADKD SALAKQREAE TRPATTAFPR   1140
ARRARRDLPQ PQPQPQPQPQ PQRDLISRYA NSGLSEFSAT LNSVFAVQDE LDRVFAEDRR   1200
NAVWTSGIRD TKHYRSQDFR AYRQQTDLRQ IGMQKNLGSG RVGILFSHNR TENTFDDGIG   1260
NSARLAHGAV FGQYGIGRFD IGISTGAGFS SGSLSDGIGG KIRRRVLHYG IQARYRAGFG   1320
GFGIEPYIGA TRYFVQKADY RYENVNIATP GLAFNRYRAG IKADYSFKPA QHISITPYLS   1380
LSYTDAASGK VRTRVNTAVL AQDFGKTRSA EWGVNAEIKG FTLSLHAAAA KGPQLEAQHS   1440
AGIKLGYRW                                                           1449
```

<212> Type : PRT
<211> Length : 1449
SequenceName : SEQ ID 237
SequenceDescription :
Sequence
--------
<213> OrganismName : Neisseria meningitidis Z2491
<400> PreSequenceString :

```
MNTLQKGFTL IELMIVIAIV GILAAVALPA YQDYTARAQV SEAILLAEGQ KSAVTEYYLN     60
HGEWPSNNTS AGVASSTDIK GKYVQSVEVK NGVVTATMAS SNVNNEIKGK KLSLWAKRQD    120
GSVKWFCGQP VKRNDTATTN DDVKADTAAN GKQIDTKHLP STCRDAASAG               170
```

<212> Type : PRT
<211> Length : 170
SequenceName : SEQ ID 238
SequenceDescription :
Sequence --------
<213> OrganismName : Neisseria meningitidis Z2491

<400> PreSequenceString :

```
MQARLLIPIL FSVFILSACG TLTGIPSHGG GKRFAVEQEL VAASARAAVK DMDLQALHGR    60
KVALYIATMG DQGSGSLTGG RYSIDALIRG EYINSPAVRT DYTYPRYETT AETTSGGLTG   120
LTTSLSTLNA PALSRTQSDG SGSKSSLGLN IGGMGDYRNE TLTTNPRDTA FLSHLVQTVF   180
FLRGIDVVSP ANADTDVFIN IDVFGTIRNR TEMHLYNAET LKAQTKLEYF AVDRTNKKLL   240
IKPKTNAFEA AYKENYALWM GPYKVSKGIK PTEGLMVDFS DIQPYGNHMG NSAPSVEADN   300
SHEGYGYSDE AVRRHRQGQP                                               320
```

<212> Type : PRT
<211> Length : 320
SequenceName : SEQ ID 239
SequenceDescription :
Sequence --------
<213> OrganismName : Neisseria meningitidis Z2491
<400> PreSequenceString :

```
MRPIFLSFVL FPILITACST PDKSARWENI GTISNGNIHT YINKDSVRKN GNLMIFQDKK    60
VVTNLKQERF ANTPAYKTAI AEWEIHCNNK TYRLSSLQLF DTKNTEISTQ NYTASSLRPM   120
SILSGTLTEK QYETVCGKKL                                               140
```

<212> Type : PRT
<211> Length : 140
SequenceName : SEQ ID 240
SequenceDescription :
Sequence--------
<213> OrganismName : Neisseria meningitidis Z2491
<400> PreSequenceString :

```
MNKLFITALS ALALSACAGT WEGAKQDTAE NLDKTQAAAE RAAEQTGNAV EKGWDKTKEA    60
VKKGGNAVGR GISHLGGKIE NATE                                           84
```

<212> Type : PRT
<211> Length : 84
SequenceName : SEQ ID 241
SequenceDescription :
Sequence --------
<213> OrganismName : Neisseria meningitidis Z2491
<400> PreSequenceString :

```
MKLLFIPLVL FVAVEHFYIA WLEMTQIPSE KAAETFKLPY EFMEQNRVQT LFGNQGLYNG    60
FLGIGLVWSR FAAPDNAVYG ATVLFLGFVL IAAAWGAFSS GNKGILVKQG LPAFLAAAAV   120
LAV                                                                 123
```

<212> Type : PRT
<211> Length : 123
SequenceName : SEQ ID 242
SequenceDescription :
Sequence --------
<213> OrganismName : Neisseria meningitidis Z2491
<400> PreSequenceString :

```
MASSNVNNEI KDKKLSLWAK RQDGSVKWFC GQPVKRDAAT DADVTADSGN EIDTKHLPST    60
CRDAASAVCT KTPEYYPNHG EWPKNFVIPA QAGIQVCRHG NLSGKKVSPV LSSRFPLSWE   120
```

<212> Type : PRT
<211> Length : 120
SequenceName : SEQ ID 243

SequenceDescription :
Sequence --------
<213> OrganismName : Neisseria meningitidis Z2491
<400> PreSequenceString :

```
MLLAEGQKSA VTEYYLNHGE WPSNNTSAGV ATSTDIKGKY VQSVEVKNGV VTATMASSNV     60
NNEIKGKKLS LWAKRQDGSV KWFCGQPVKR NDTATTNDDV KADTAANGKQ IDTKHLPSTA    120
STRKSTPN                                                             128
```

<212> Type : PRT
<211> Length : 128
SequenceName : SEQ ID 244
SequenceDescription :
Sequence --------
<213> OrganismName : Neisseria meningitidis Z2491
<400> PreSequenceString :

```
MPIPFKPVLA AAAIAQAFPA FAADPAPQSA QTLNEITVTG THKTQKLGEE KIRRKTLDKL     60
LVNDEHDLVR YDPGISVVEG GRAGSNGFTI RGVDKDRVAI NVDGLAQAES RSSEAFQELF    120
GAYGNFNANR NTSEPENFSE VTITKGADSL KSGSGALGGA VNYQTKSASD YVSEDKPYHL    180
GIKGGSVGKN SQKFSSITAA GRLFGLDALL VYTRRFGKET KNRSTEGDIE IKNDGYVYNP    240
TDTGGPSKYL TYVATGVARS QPDPQEWVNK STLFKLGYNF NDQNRIGWIF EDSRTDRFTN    300
ELSNLWTGTT TSAATGDYRH RQDVSYRRRS GVEYKNELEH GPWDSLKLRY DKQRIDMNTW    360
TWDIPKNYDK RGINGEVYHS FRHIRQNTAQ WTADFEKQLD FSKAVWAAQY GLGGGKGDNA    420
NSDYSYFAKL YDPKILASNQ AKITMLIENR SKYKFAYWNN AFHLGGNDRF RLNAGIRYDK    480
NSSSAKDDPK YTTAIRGQIP HLGSERAHAG FSYGTGFDWR FTKHLHLLAK YSTGFRAPTS    540
DETWLLFPHP DFYLKANPNL KAEKAKNWEL GLAGSGKAGN FKLSGFKTKY RDFIELTYMG    600
VSSDDKNNPR YAPLSDGTAL VSSPVWQNQN RSAAWVKGIE FNGTWNLDSI GLPKGLHTGL    660
NVSYIKGKAT QNNGKETPIN ALSPWTAVYS LGYDAPSKRW GINAYATRTA AKKPSDTVHS    720
NDDLNNPWPY AKHSKAYTLF DLSAYLNIGK QVTLRAAAYN ITNKQYYTWE SLRSIREFGT    780
VNRVDNKTHA GIQRFTSPGR SYNFTIEAKF                                     810
```

<212> Type : PRT
<211> Length : 810
SequenceName : SEQ ID 245
SequenceDescription :
Sequence --------
<213> OrganismName : Neisseria meningitidis Z2491
<400> PreSequenceString :

```
MKKSLIALTL AALPVAAMAD VTLYGTIKTG VETSRSVEHN GGQVVSVETG TGIVDLGSKI     60
GFKGQEDLGN GLKAIWQVEQ KASIAGTDSG WGNRQSFIGL KGGFGKLRVG RLNSVLKDTG    120
DINPWDSKSD YLGVNKIAEP EARLISVRYD SPEFAGLSGS VQYALNDNVG RHNSESYHAG    180
FNYKNGGFFV QYGGAYKRHQ DVDDVKIEKY QIHRLVSGYD NDALYASVAV QQQDAKLVED    240
NSHNSQTEVA ATLAYRFGNV TPRVSYAHGF KGSVDDAKRD NTYDQVVVGA EYDFSKRTSA    300
LVSAGWLQEG KGENKFVATA GGVGLRHKF                                      329
```

<212> Type : PRT
<211> Length : 329
SequenceName : SEQ ID 246
SequenceDescription :
Sequence --------
<213> OrganismName : Neisseria meningitidis Z2491
<400> PreSequenceString :

MKTLLLLIPL VLTACGTLTG IPAHGGGKRF AVEQELVAAS SRAAVKEMDL SALKGRKAAL 60
YVSVMGDQGS GNISGGRYSI DALIRGGYHN NPESATQYSY PAYDTTATTK SDALSSVTTS 120
TSLLNAPAAA LTKNSGRKGE RSAGLSVNGT GDYRNETLLA NPRDVSFLTN LIQTVFYLRG 180
IEVVPPEYAD TDVFVTVDVF GTVRSRTELH LYNAETLKAQ TKLEYFAVDR DSRKLLIAPK 240
TAAYESQYQE QYALWMGPYS VGKTVKASDR LMVDFSDITP YGDTTAQNRP DFKQNNGKKP 300
DVGNEVIRRR KGG 313

<212> Type : PRT
<211> Length : 313
SequenceName : SEQ ID 247
SequenceDescription :
Sequence --------
<213> OrganismName : Neisseria meningitidis Z2491
<400> PreSequenceString :

MNKTLSILPV AILLGGCAAG GGNTFGSLDG GTGMGGSIVK MAVESQCRAE LNKRSEWRLT 60
ALAMSAEKQA EWENKICACV AQEAPNQLTG NDVMQMLDPS TRNQALAALT AKTVSACFKH 120
LYR 123

<212> Type : PRT
<211> Length : 123
SequenceName : SEQ ID 248
SequenceDescription :
Sequence --------
<213> OrganismName : Neisseria meningitidis Z2491
<400> PreSequenceString :

MNPLIHQAKE SSMQTRILSA VLLAFSTAAF AGGAFTLQFD NPSEDGGFTQ NQILSAPYGF 60
GCSGGNASPA LSWKNPPAGT KSFVLTVYDK DAPTGLGWMH WVVADIPADV RRRNATSLQL 120
SRCASIADDQ SAAISAVISL QICRIRLTPS YTAKPMPSCC NHANTPQSAA SAALCGTSSS 180
VSTAAA 186

<212> Type : PRT
<211> Length : 186
SequenceName : SEQ ID 249
SequenceDescription :
Sequence
--------
<213> OrganismName : Neisseria meningitidis Z2491
<400> PreSequenceString :

MNKTLKRRVF RHTALYAAIL MFSHTGGGGG AMAQTRQYAI IMNERNQPEV QWNGSYSIKD 60
KDRKREYTHH NHQQGGSSVS FNNSDELVSR QSGTAVFGTA TYLPPYGKVS GFDAAALKER 120
NNAVDWIHTT HPGLIGYSYD GVVCRSATDC PKLVYKTRFS FDNPDLAKTG GGLDKHTEPS 180
RDNSPIYKLK DHPWLGVSFN LGAEGIAKNG KTINKLVSSF NEKNSNNNLV YTTEGRDISL 240
GNWQRETTAM AYYLNAKLHL LDKKQIQNIT DKTVQLGVLK PSIDVRTRNT GTAGILSYWA 300
KWDIKDTGQI PVKLSLTQVK AGRCVNKDNP NKNTKTSSPA LTAPALWFGA GQDGKAEMYS 360
ASVSTYPDSS SSRIFLQNLK RKTDTSRPGR YSLATLNKSD IESREPSFTS RQTVIRLDGG 420
VQQIKLDRNN TEVTGFNGND GKNDTFGIVS EGSFMPDASE WKKVLLPWTV RAFNYDGRFN 480
TVNKEENNGK PKYSQKYRSR NNGKHERNLG DIVNSPIVAV GEYLATSAND GMVHIFKQSG 540
GDKRSYNLKL SYIPGTMPRK DIESKDSTLA KELRAFAEKG YVGDRYGVDG GFVLRRITDD 600
QDKQKHFFMF GAMGLGGRGA YALDLTKADD NDPTKASLFD VKDNGNNGNN GNNRVELGYT 660
VGTPQIGKTH NGKYAAFLAS GYATKQIDSG ENKTALYVYD LESNNGTLIR KIEVTDGKGG 720
LSSPTLVDKD LDGTVDIAYA GDRGGKMYRF DLSGNNPNSW TVRTIFQGTK PITSAPAISQ 780
LKDKRVVIFG TGSDLSEDDV LSTDEQHIYG IFDNDTNTGT AQEGLGKGLL EQKLSEENKT 840
LFLTDYKRSD GSGDKGWVVK LKDGQRVTVK PTVVLRTAFV TIHKYTGNDK CGAETAILGI 900
NTADGGKLTK KSARPIVPAA NSKVAQYSGD KKTSSGKSIP IGCMEKDGGT VCPNGYVYDK 960
PVNVRYLDEK KTDGFSTTAD GDAGGSGTFK EGKKPARNNR CFSGKGVRTL LMNDLDSLDI 1020
TGPMCGMKRI SWREVFY 1037

<212> Type : PRT
<211> Length : 1037

SequenceName : SEQ ID 250
SequenceDescription :
Sequence --------
<213> OrganismName : Neisseria meningitidis Z2491
<400> PreSequenceString :

```
MKHPKLTLIA ALLTTAATAA PLPVVTSFSI LGDVAKQIGG ERVSIQSLVG ANQDTHAYHM    60
TSGDIKKIRS AKLVLINGLG LEAADIQRAV KQSKVSYAEA TKGIQPLKAE EEGGHHHDHD   120
HDHDHDHEGH HHDHGEYDPH VWNDPVLMSA YAQNVAEALI KADPEGKVYY QQRLGNYQMQ   180
LKKLHSDAQA AFNAVPAAKR KVLTGHDAFS YMGKRYHIEF IAPQGVSSEA EPSAKQVAAI   240
IRQIKREGIK AVFTENIKDT RMVDRIAKET GVNVSGKLYS DALGNAPADT YIGMYRHNIK   300
ALTNAMKQ                                                           308
```

<212> Type : PRT
<211> Length : 308
SequenceName : SEQ ID 251
SequenceDescription :
Sequence --------
<213> OrganismName : Streptococcus pyogenes MGAS8232
<400> PreSequenceString :

```
MKKRILSAVL VSGVTLGAAT TVGAEDLSTK IAKQDSIISN LTTEQKAAQN QVSALQAQVS    60
SLQSEQDKLT ARNTELEALS KRFEQEIKAL TSQIVARNEK LKNQARSAYK NNETSGYINA   120
LLNSKSISDV VNRLVAINRA VSANAKLLEQ QKADKVSLEE KQAANQTAIN TIAANMAMAE   180
ENQNTLRTQQ ANLEAATANL ALQLASATED KANLVAQKEA AEKAAAEALA QEQAAKVKAQ   240
EQAAQQAASV EAAKSAITPA PQATPAAQSS NAIEPAALTA PAAPSARPQT SYDSSNTYPV   300
GQCTWGAKSL APWAGNNWGN GGQWAYSAQA AGYRTGSTPM VGAIAVWNDG GYGHVAVVVE   360
VQSASSIRVM ESNYSGRQYI ADHRGWFNPT GVTFIYPH                          398
```

<212> Type : PRT
<211> Length : 398
SequenceName : SEQ ID 252
SequenceDescription :
Sequence --------
<213> OrganismName : Streptococcus pyogenes MGAS8232
<400> PreSequenceString :

```
MITIKNPKIL KWLKYVLSAI LSLIILVIII GGLLFTFYIS SAPKLSEAQL KSTNSSLVYD    60
GNNNLIADLG SEKRENVTAD SIPINLVNAI TSIEDKRFFN HRGVDLYRIF GAAFHNLTSQ   120
TTQGGSTLDQ QLIKLAYFST NESDQTLKRK AQEVWLALQM ERKYTKQEIL TFYINKVYMG   180
NGNYGMLTAA KSYYGKDLKD LSYAQLALLA GIPQAPSQYD PYLHPEAAQN RRNVVLQQMY   240
MEKHLTKAEY ETAIATPVAE GLQSLQQRST YPKYMDNYLK QVIEEVKKET NKDIFTAGLK   300

VYTNIIPDAQ QTLYNIYHSG DYVYYPDQDF QVASTIVDVT NGHVIAQLGG RNQDENVSFG   360
TNQAVLTDRD WGSTMKPITA YAPAIESGVY TSTAQSTNDS VYYWPGTTTQ LFNWDLRYNG   420
WMTIQAAIML SRNVPAVRAL EAAGLDYARS FLSSLGINYP EMHYSNAISS NNSSSDKKYG   480
ASSEKMAAAY AAFANGGIYH KPRYVNKVEF SDGTSKTFDE KGKRAMKETT AYMMTDMLKT   540
VLTYGTGTAA AIPGVAQAGK TGTSNYTDEE LAKIGEKYGL YPDYVGTLAP DENFVGFTKR   600
YAMAVWTGYK NRLTPVYGSS LEIASDVYRS MMTYLTNGYS EDWTMPNGLY RSGGFLYLSG   660
TYASNTDYTN SVYNNLYSNN TTTASSQTTS DDTSSSNDTS NSTNTDNNGS HPSTDDKKTT   720
H                                                                  721
```

<212> Type : PRT
<211> Length : 721
SequenceName : SEQ ID 253
SequenceDescription :
Sequence --------
<213> OrganismName : Streptococcus pyogenes MGAS8232
<400> PreSequenceString :

MIITKKSLFV TSVALSLAPL VTAQAQEWTP RSVTEIKSEL VLVDNVFTYT VKYGDTLSTI 60
AEAMGIDVHV LGDINHIANI DLIFPDTILT ANYNQHGQAT TLTVQAPASS PASVSHVPSS 120
EPLPQASATS QSTVPMAPSA TPSDVPTTPL ASAKPDSFVT ASSELTSSTN DVSTELSSES 180
QKQPEVSQEA VPTPKAAETT EVEPKTDISE DPTSANRPVP NESASEEASS AAPAQAPAEK 240
EETSQMLTAP AAQKAVADTT SVATSNGLSY APNHAYNPMN AGLQPQTAAF KEEVASAFGI 300
TSFSGYRPGD PGDHGKGLAI DFMVPVSSTL GDQVAQYAID HMAERGISYV IWKQRFYAPF 360
ASIYGPAYTW NPMPDRGSIT ENHYDHVHVS FNA 393

<212> Type : PRT
<211> Length : 393
SequenceName : SEQ ID 254
SequenceDescription :
Sequence --------
<213> OrganismName : Streptococcus pyogenes MGAS8232
<400> PreSequenceString :

MKKKILLMMS LISVFFAWQL TQAKQVLAEG KVKVVTTFYP VYEFTKGVIG NDGDVSMLMK 60
AGTEPHDFEP STKDIKKIQD ADAFVYMDDN METWVSDVKK SLTSKKVTIV KGTGNMLLVA 120
GAGHDHHHED ADKKHEHNKH SEEGHNHAFD PHVWLSPYRS ITVVENIRDS LSKAYPEKAE 180
NFKANAATYI EKLKELDKDY TAALSDAKQK SFVTQHAAFG YMALDYGLNQ ISINGVTPDA 240
EPSAKRIATL SKYVKKYGIK YIYFEENASS KVAKTLAKEA GVKAAVLSPL EGLTKKEMKA 300
GQDYFTVMRK NLETLRLTTD VAGKEILPEK DTTKTVYNGY FKDKEVKDRQ LSDWSGSWQS 360
VYPYLQDGTL DQVWDYKAKK SKGKMTAAEY KDYYTTGYKT DVEQIKINGK KKTMTFVRNG 420
EKKTFTYTYA GKEILTYPKG NRGVRFMFEA KEPNAGEFKY VQFSDHAIAP EKAEHFHLYW 480
GGDSQEKLHK ELEHWPTYYG SDLSGREIAQ EINAH 515

<212> Type : PRT
<211> Length : 515
SequenceName : SEQ ID 255
SequenceDescription :
Sequence --------
<213> OrganismName : Streptococcus pyogenes MGAS8232
<400> PreSequenceString :

MKKFHRFLVS GVILLGFNGL VPTMPSTLIS QQENLVHAAV LGDNYPSKWK KGNGIDSWNM 60
YIRQCTSFAA FRLSSANGFQ LPKGYGNACT WGHIAKNQGY PVNKTPSIGA IAWFDKNAYQ 120
SNAAYDHVAW VADIRGDTVT IEEYNYNAGQ GPERYHKRQI PKSQVSGYIH FKDLSSQTSH 180
SYPRQLKHIS QASFDPSGTY HFTTRLPVKG QTSIDSPDLA YYEAGQSVYY DKVVTAGGYT 240
WLSYLSFSGN RRYIPIKEPA QSVVQNDNTK PSIKVGDTVT FPGVFRVDQL VNNLIVNKEL 300
AGGDPTPLNW IDPTPLDETD NQGKVLGNQI LRVGEYFTVT GSYKVLKIDQ PSNGIYVQIG 360
SRGTWVNADK ANKL 374

<212> Type : PRT
<211> Length : 374
SequenceName : SEQ ID 256
SequenceDescription :
Sequence --------
<213> OrganismName : Streptococcus pyogenes MGAS8232
<400> PreSequenceString :

MLKFTSNILA TSVAETTQVA PGGCCCCCTT CCFSIATGSG NSQGGSGSYT PGK

<212> Type : PRT
<211> Length : 53
SequenceName : SEQ ID 257
SequenceDescription :
Sequence --------
<213> OrganismName : Streptococcus pyogenes MGAS8232
<400> PreSequenceString :

```
MGESYSVEAV LTAVDKTFGK TLQSAIRSIE GLEKRSTGFS SVSQKASSMF KSMLGANLAG    60
QAISAMTRTV SSGLGSMLGE MNSSAKAWKT FDANLADIGF GKKQILAVKT AMQDYATKTI   120
YSASDMASTY AQLAAVGVKD TGKLVKAFGG LAASAENPKQ AMKSISQQMT QAVGRPTVAW   180
QDFRIMLEQT PAGMAKVAKS MGKNLDELVA DIQAGRVKTS DFLEAVKKAG NDKSFQKMAT   240
EFKTVDQAID GMREGLSNKL QPAFEKVNQF GIRAIEAIGK QLDKVDFSKF ASNLGKFLEG   300
INIDKIVSNI SSAVSSVTSK VKEFWDGFKQ TGAISAFSGA LQSVWGALKN VASAMSGGNW   360
KTFGATVGGI VKHVSNFAKA VSDVLGKMDP GRLRSWIATF AAVAGGFKLF EKLTGQSVIG   420
SFLDKIGSKF GLFGNKAKEG TDKASNGARR SGGIISQIFS GLGNIVKSAG TAISTAAKGI   480
GVGIKTALSG IPPYH                                                   495
```

<212> Type : PRT
<211> Length : 495
SequenceName : SEQ ID 258
SequenceDescription :
Sequence --------
<213> OrganismName : Streptococcus pyogenes MGAS8232
<400> PreSequenceString :

```
MKKGFFLMVM VVSLVMIAGC DKSANPKQPT QGMSVVTSFY PMYAMTKEVS GDLNDVRMIQ    60
SGAGIHSFEP SVNDVAAIYD ADLFVYHSHT LEAWARDLDP NLKKSKVDVF EASKPLTLDR   120
VKGLEDMEVT QGIDPATLYD PHTWTDPVLA GEEAVNIAKE LGRLDPKHKD SYTKNAKAFK   180
KEAEQLTEEY TQKFKKVRSK TFVTQHTAFS YLAKRFGLKQ LGISGISPEQ EPSPRQLKEI   240
QDFVKEYNVK TIFAEDNVNP KIAHAIAKST GAKVKTLSPL EAAPSGNKTY LENLRANLEV   300
LYQQLK                                                             306
```

<212> Type : PRT
<211> Length : 306
SequenceName : SEQ ID 259
SequenceDescription :
Sequence --------
<213> OrganismName : Streptococcus pyogenes MGAS8232
<400> PreSequenceString :

```
MEKKQRFSLR KYKSGTFSVL IGSVFLMMTT TVAADELSTM SEPTITNHTQ QQAQHLTNTE    60
LSSAESKSQD TSQITPKTNR EKEQPQGLVS EPTTTELADT DAAPMANTGP DATQKSASLP   120
PVNTDVHDWV KTKGAWDKGY KGQGKVVAVI DTGIDPAHQS MRISDVSTAK VKSKEDMLAR   180
QKAAGINYGS WINDKVVFAH NYVENSDNIK ENQFEDFDED WENFEFDAEA EPKAIKKHKI   240
YRPQSTQAPK ETVIKTEETD GSHDIDWTQT DDDTKYESHG MHVTGIVAGN SKEAAATGER   300
FLGIAPEAQV MFMRVFANDV MGSAESLFIK AIEDAVALGA DVINLSLGTA NGAQLSGSKP   360
LMEAIEKAKK AGVSVVVAAG NERVYGSDHD DPLAINPDYG LVGSPSTGRT PTSVAAINSK   420
WVIQRLMTVK ELENRADLNH GKAIYSESVD FKNIKDSLGY DKSHQFAYVK ESTDAGYKAQ   480
DVKDKIALIE RDPNKTYDEM IALAKKHGAL GVLIFNNKPG QSNRSMRLTA NGMGIPSAFI   540
SHEFGKAMSQ LNGNGTGSLE FDSVVSKAPS QKGNEMNHFS NWGLTSDGYL KPDITAPGGD   600
IYSTYNDNHY GSQTGTSMAS PQIAGASLLV KQYLEKTQPN LPKEKIADIV KNLLMSNAQI   660
HVNPETKTTT SPRQQGAGLL NIDGAVTSGL YVTGKDNYGS ISLGNITDTM TFDVTVHNLS   720
NKDKTLRYDT ELLTDHVDPQ KGRFTLTSRS LKTYQGGEVT VPANGKVTVR VTMDVSQFTK   780
ELTKQMSNGY YLEGFVRFRD SQDDQLNRVN IPFVGFKGQF ENLAVAEESI YRLKSQGKTG   840
FYFDESGPKD DIYVGKHFTG LVTLGSETNV STKTISDNGL HTLGTFKNAD GKFILEKNAQ   900
GNPVLAISPN GDNNQDFAAF KGVFLRKYQG LKASVYHASD KEHKNPLWVS PESFKGDKNF   960
NSDIRFAKST TLLGTAFSGK SLTGAELPDG YYHYVVSYYP DVVGAKRQEM TFDMILDRQK  1020
PVLSQATFDP ETNRFKPEPL KDRGLAGVRK DSVFYLERKD NKPYTVTIND SYKYVSVEDN  1080
KTFVERQADG SFILPLDKAK LGDFYYMVED FAGNVAIAKL GDHLPQTLGK TPIKLKLTDG  1140
NYQTKETLKD NLEMTQSDTG LVTNQAQLAV VHRNQPQSQL TKMNQDFFIS PNEDGNKDFV  1200
AFKGLKNNVY NDLTVNVYAK DDHQKQTPIW SSQAGASASA IESTAWYGIT ARGSKVMPGD  1260
YQYVVTYRDE HGKEHQKQYT ISVNDKKPMI TQGRFDTING VDHFTPDKTK ALGSSGIVRE  1320
EVFYLAKKNG RKFDVTEGKD GITVSDNKMY IPKNPDGSYT ISKRDGVTLS DYYYLVEDRA  1380

GNVSFATLRD LKAVGKDKAV VNFGLDLPVP EDKQIVNFTY LVRDADGKPI ENLEYYNNSG  1440
NSLILPYGKY TVELLTYDTN AAKLESDKIV SFTLSADNNF QQVTFKMTML ATSQITAHFD  1500
HLLPEGSRVS LKTAQGQLIP LEQSLYVPKA YGKTVQEGTY EVVVSLPKGY RIEGNTKVNT  1560
LPNEVHELSL RLVKVGDASD STGDHKVMSK NNSQALTAFA TPTKTTTSAT AKALPSAGEK  1620
MGLKLRIVGL VLLGLTCVFS RKKSTKD                                     1647
```

<212> Type : PRT
<211> Length : 1647
SequenceName : SEQ ID 260
SequenceDescription :
Sequence --------
<213> OrganismName : Treponema pallidum subsp. pallidum str. Nichols
<400>. PreSequenceString :

```
MMRSLFSGVS GMQNHQTRMD VIGNNVANVN TTGFKRGRVN FQDLISQQLS AAARPNEEVG   60
GVNPKEVGLG VLIASIDTVH TQGALQTTGI NTDVSIQGSG FFVLKSGEKT FFTRAGAFGV  120
DNAGTLVNPA NGMRVQGWMA QDVAGERLIN SSAQTQDLVI PIGQKIDAQQ TSTVHYACNL  180
DKRLPELAAD ANEADVRKST WTTDFQVYDS FGQQHTLQIN FSRVPGTNNQ WQATVAVDPG  240
TEVDTQTRVG VGTSDGAANT FIVNFDNFGH LASVTDTAGN VTGPTGQVLL EASYDVVGAN  300
PDDAGQVTRH AFTLNLGEIG TARNTITQFA ERSTTKAYRQ DGYAMGYLEN FKIDQSGVIT  360
GVYSNGVSQD IGQLALAGFA NQGGLEKAGE NTYVQSNNSG IANISTSGVM GKGKLIAGTL  420
EMSNVDLTDQ FTDMIITQKG FQAGAKTIQT SDTMLDTVLS LKR                    463
```

<212> Type : PRT
<211> Length : 463
SequenceName : SEQ ID 261
SequenceDescription :
Sequence --------
<213> OrganismName : Treponema pallidum subsp. pallidium str. Nichols
<400> PreSequenceString :

```
MGCMRWGSVL CVVVGVGASG GVLGQEFSPK LTGSATLEWG ISYGKGVGSH GQAPGAVMGT   60
GPYNLKHGFR TTNTVGVSFP LVMRTTHTRR GQHPALYAEL KVADLQADLS QGKAGFAVKR  120
KGKVEATLHC YGAYLTIGKN PTFLTNFARL WKPWVTAQYQ EDAVQYAPGF GGLGGKVGYR  180
AQDIGGSGVS LDVGFLSFAS NGAWDSTDPT HSKYGFGADL KLMYARAGHP LCTVELASNV  240
TLEDGYLIGA QKDANNQNKD KLLWNVGGRL TLEPGAGFRF SFALDAGNQH QSAQDFQNRT  300
QRAQSELTAL SNNLFQGESQ KQEAWVTQVV QQATQTVTAG VRSALESRGT TYINALEAVQ  360
PNPAKPTGKV VQNLHTPQGS PPNLPPLPAL PAFSLMGQVL LQYDAEQVVK GFEQVQTQIV  420
TEINQKVQAA VAKNNANMQA VGGSLGDTAR MVGEALIKQQ LSRKQNSILT MVSVQDEVKQ  480
DLADLVPMMR TEITAFFASV QQHITEEVKK KTDALNAGQQ IRQAIQNLRA SAWRAFLMGV  540
SAVCLYLDTY NVAFDALFTA QWKWLSSGIY FATAPANVFG TRVLDNTIAS CGDFAGFLKL  600
ETKSGDPYTH LLTGLDAGVE TRVYIPLTHD LYKNNNGNPL PSGGSSGHIG LPVVGKAWCS  660
YRIPVQDYGW VKPSVTVHAS TNRAHLNAPA AGGAVGATYL TKEYCAQLRA GISASLIEKT  720
VFSLDWEQGM LSDVPYLLVS ECLTQGIGRI VCGVTLSW                          758
```

<212> Type : PRT
<211> Length : 758
SequenceName : SEQ ID 262
SequenceDescription :
Sequence --------
<213> OrganismName : Treponema pallidum subsp. pallidum str. Nichols
<400> PreSequenceString :

```
MGRQVMQAGV LAGMVCAASG YAGVLTPQVS GTAQLQWGIA FQKNPRTGPG KHTHGFRTTN   60
SLTISLPLVS KHTHTRRGEA RSGVWAQLQL KDLAVELASS KSSTALSFTK PTASFQATLH  120
CYGAYLTVGT SPSCVVNFAQ LWKPFVTRAY SEKDTRYAPG FSGSGAKLGY QAHNVGNSGV  180
DVDIGFLSFL SNGAWDSTDT THSKYGFGAD ATLSYGVDRQ RLLTLELAGN ATLDQNYVKG  240
TEDSKNENKT ALLWGVGGRL TLEPGAGFRF SFALDAGNQH QSNAHAQTQE RAILKAREVF  300
RRVEGKLVQN LPNIMMPPGI TEQTTLIEMV GLAALIAEGT LGSAIQTVLA AGALAALVSQ  360
LVPNIEQGVR DVFRSSDPRV VTAKLLAFLE RAPMNALNID ALLRMQWKWL SSGIYFATAG  420
TNIFGKRVFA TTRAHYFDFA GFLKLETKSG DPYTHLLTGL NAGVEARVYI PLTYIRYRNN  480
GGYELNGAVP PGTINMPILG KAWCSYRIPL GSHAWLAPHT SVLGTTNRFN IINPAGNLLN  540
ERALQYQVGL TFSPFEKVEL SAQWEQGVLA DAPYMGIAES IWSERHFGTL VCGMKVTW     598
```

<212> Type : PRT
<211> Length : 598
SequenceName : SEQ ID 263 SequenceDescription :
Sequence --------

<213> OrganismName : Treponema pallidum subsp. pallidum str. Nichols
<400> PreSequenceString :

```
MGRQVMQAGV LAGMVCAASG YAGVLTPQVS GTAQLQWGIA FQKNPRTGPG KHTHGFRTTN   60
SLTISLPLVS KHTHTRRGEA RSGVWAQLQL KDLAVELASS KSSTALSFTK PTASFQATLH  120
CYGAYLTVGT SPSCVVNFAQ LWKPFVTRAY SEKDTRYAPG FSGSGAKLGY QAHNVGNSGV  180
DVDIGFLSFL SNGAWDSTDT THSKYGFGAD ATLSYGVDRQ RLLTLELAGN ATLDQNYVKG  240
TEDSKNENKT ALLWGVGGRL TLEPGAGFRF SFALDAGNQH QSNAHAQTQE RAILKAREVF  300
RRVEGKLVQN LPNIMMPPGI TEQTTLIEMV GLAALIAEGT LGSAIQTVLA AGALAALVSQ  360
LVPNIEQGVR DVFRSSDPRV VTAKLLAFLE RAPMNALNID ALLRMQWKWL SSGIYFATAG  420
TNIFGKRVFA TTRAHYFDFA GFLKLETKSG DPYTHLLTGL NAGVEARVYI PLTYIRYRNN  480
GGYELNGAVP PGTINMPILG KAWCSYRIPL GSHAWLAPHT SVLGTTNRFN IINPAGNLLN  540
ERALQYQVGL TFSPFEKVEL SAQWEQGVLA DAPYMGIAES IWSERHFGTL VCGMKVTW     598
```

<212> Type : PRT
<211> Length : 598
SequenceName : SEQ ID 264
SequenceDescription :
Sequence --------
<213> OrganismName : SARS coronavirus Frankfurt 1
<400> PreSequenceString :

```
MFIFLLFLTL TSGSDLDRCT TFDDVQAPNY TQHTSSMRGV YYPDEIFRSD TLYLTQDLFL   60
PFYSNVTGFH TINHTFGNPV IPFKDGIYFA ATEKSNVVRG WVFGSTMNNK SQSVIIINNS  120
TNVVIRACNF ELCDNPFFAV SKPMGTQTHT MIFDNAFNCT FEYISDAFSL DVSEKSGNFK  180
HLREFVFKNK DGFLYVYKGY QPIDVVRDLP SGFNTLKPIF KLPLGINITN FRAILTAFSP  240
AQDIWGTSAA AYFVGYLKPT TFMLKYDENG TITDAVDCSQ NPLAELKCSV KSFEIDKGIY  300
QTSNFRVVPS GDVVRFPNIT NLCPFGEVFN ATKFPSVYAW ERKKISNCVA DYSVLYNSTF  360
FSTFKCYGVS ATKLNDLCFS NVYADSFVVK GDDVRQIAPG QTGVIADYNY KLPDDFMGCV  420
LAWNTRNIDA TSTGNYNYKY RYLRHGKLRP FERDISNVPF SPDGKPCTPP ALNCYWPLND  480
YGFYTTTGIG YQPYRVVVLS FELLNAPATV CGPKLSTDLI KNQCVNFNFN GLTGTGVLTP  540
SSKRFQPFQQ FGRDVSDFTD SVRDPKTSEI LDISPCSFGG VSVITPGTNA SSEVAVLYQD  600
VNCTDVSTAI HADQLTPAWR IYSTGNNVFQ TQAGCLIGAE HVDTSYECDI PIGAGICASY  660
HTVSLLRSTS QKSIVAYTMS LGADSSIAYS NNTIAIPTNF SISITTEVMP VSMAKTSVDC  720
NMYICGDSTE CANLLLQYGS FCTQLNRALS GIAAEQDRNT REVFAQVKQM YKTPTLKYFG  780
GFNFSQILPD PLKPTKRSFI EDLLFNKVTL ADAGFMKQYG ECLGDINARD LICAQKFNGL  840
TVLPPLLTDD MIAAYTAALV SGTATAGWTF GAGAALQIPF AMQMAYRFNG IGVTQNVLYE  900
NQKQIANQFN KAISQIQESL TTTSTALGKL QDVVNQNAQA LNTLVKQLSS NFGAISSVLN  960
DILSRLDKVE AEVQIDRLIT GRLQSLQTYV TQQLIRAAEI RASANLAATK MSECVLGQSK  1020
RVDFCGKGYH LMSFPQAAPH GVVFLHVTYV PSQERNFTTA PAICHEGKAY FPREGVFVFN  1080
GTSWFITQRN FFSPQIITTD NTFVSGNCDV VIGIINNTVY DPLQPELDSF KEELDKYFKN  1140
HTSPDVDFGD ISGINASVVN IQKEIDRLNE VAKNLNESLI DLQELGKYEQ YIKWPWYVWL  1200
GFIAGLIAIV MVTILLCCMT SCCSCLKGAC SCGSCCKFDE DDSEPVLKGV KLHYT       1255
```

<212> Type : PRT
<211> Length : 1255
SequenceName : SEQ ID 265
SequenceDescription :
Sequence --------
<213> OrganismName : SARS coronavirus HSR 1
<400> PreSequenceString :

```
MFIFLLFLTL TSGSDLDRCT TFDDVQAPNY TQHTSSMRGV YYPDEIFRSD TLYLTQDLFL   60
PFYSNVTGFH TINHTFGNPV IPFKDGIYFA ATEKSNVVRG WVFGSTMNNK SQSVIIINNS  120
TNVVIRACNF ELCDNPFFAV SKPMGTQTHT MIFDNAFNCT FEYISDAFSL DVSEKSGNFK  180
HLREFVFKNK DGFLYVYKGY QPIDVVRDLP SGFNTLKPIF KLPLGINITN FRAILTAFSP  240
AQDIWGTSAA AYFVGYLKPT TFMLKYDENG TITDAVDCSQ NPLAELKCSV KSFEIDKGIY  300
QTSNFRVVPS GDVVRFPNIT NLCPFGEVFN ATKFPSVYAW ERKKISNCVA DYSVLYNSTF  360
FSTFKCYGVS ATKLNDLCFS NVYADSFVVK GDDVRQIAPG QTGVIADYNY KLPDDFMGCV  420
LAWNTRNIDA TSTGNYNYKY RYLRHGKLRP FERDISNVPF SPDGKPCTPP ALNCYWPLND  480
YGFYTTTGIG YQPYRVVVLS FELLNAPATV CGPKLSTDLI KNQCVNFNFN GLTGTGVLTP  540
SSKRFQPFQQ FGRDVSDFTD SVRDPKTSEI LDISPCSFGG VSVITPGTNA SSEVAVLYQD  600
```

```
VNCTDVSTAI HADQLTPAWR IYSTGNNVFQ TQAGCLIGAE HVDTSYECDI PIGAGICASY    660
HTVSLLRSTS QKSIVAYTMS LGADSSIAYS NNTIAIPTNF SISITTEVMP VSMAKTSVDC    720
NMYICGDSTE CANLLLQYGS FCTQLNRALS GIAAEQDRNT REVFAQVKQM YKTPTLKYFG    780
GFNFSQILPD PLKPTKRSFI EDLLFNKVTL ADAGFMKQYG ECLGDINARD LICAQKFNGL    840
TVLPPLLTDD MIAAYTAALV SGTATAGWTF GAGAALQIPF AMQMAYRFNG IGVTQNVLYE    900
NQKQIANQFN KAISQIQESL TTTSTALGKL QDVVNQNAQA LNTLVKQLSS NFGAISSVLN    960
DILSRLDKVE AEVQIDRLIT GRLQSLQTYV TQQLIRAAEI RASANLAATK MSECVLGQSK   1020
RVDFCGKGYH LMSFPQAAPH GVVFLHVTYV PSQERNFTTA PAICHEGKAY FPREGVFVFN   1080
GTSWFITQRN FFSPQIITTD NTFVSGNCDV VIGIINNTVY DPLQPELDSF KEELDKYFKN   1140
HTSPDVDLGD ISGINASVVN IQKEIDRLNE VAKNLNESLI DLQELGKYEQ YIKWPWYVWL   1200
GFIAGLIAIV MVTILLCCMT SCCSCLKGAC SCGSCCKFDE DDSEPVLKGV KLHYT        1255
```

<212> Type : PRT
<211> Length : 1255
SequenceName : SEQ ID 266
SequenceDescription :
Sequence --------
<213> OrganismName : SARS coronavirus ZJ01
<400> PreSequenceString :

```
MFIFLLFLTL TSGSDLDRCT TFDDVQAPNY TQHTSSMRGV YYPDEIFRSD TLYLTQDLFL     60
PFYSNVTGFH TINHTFGNPV IPFKDGIYFA ATEKSNVVRG WVFGSTMNNK SQSVIIINNS    120
TNVVIRACNF ELCDNPFFAV SKPMGTQTHT MIFDNAFNCT FEYISDAFSL DVSEKSGNFK    180
HLREFVFKNK DGFLYVYKGY QPIDVVRDLP SGFNTLKPIF KLPLGINITN FRAILTAFSP    240
AQDIWGTSAA AYFVGYLKPT TFMLKYDENG TITDAVDCSQ NPLAELKCSV KSFEIDKGIY    300
QTSNFRVVPS GDVVRFPNIT NLCPFGEVFN ATKFPSVYAW ERKKISNCVA DYSVLYNSTF    360
FSTFKCYGVS ATKLNDLCFS NVYADSFVVK GDDVRQIAPG QTGVIADYNY KLPDDFMGCV    420
LAWNTRNIDA TSTGNYNYKY RYLRHGKLRP FERDISNVPF SPDGKPCTPP ALNCYWPLND    480
YGFYTTTGIG YQPYRVVVLS FELLNAPATV CGPKLSTDLI KNQCVNFNFN GLTGTGVLTP    540
SSKRFQPFQQ FGRDVSDFTD SVRDPKTSEI LDISPCSFGG VSVITPGTNA SSEVAVLYQD    600
VNCTDVSTAI HADQLTPAWR IYSTGNNVFQ TQAGCLIGAE HVDTSYECDI PIGAGICASY    660
HTVSLLRSTS QKSIVAYTMS LGADSSIAYS NNTIAIPTNF SISITTEVMP VSMAKTSVDC    720
NMYICGDSTE CANLLLQYGS FCTQLNRALS GIAAEQDRNT REVFAQVKQM YKTPTLKYFG    780
GFNFSQILPD PLKPTKRSFI EDLLFNKVTL ADAGFMKQYG ECLGDINARD LICAQKFNGL    840
TVLPPLLTDD MIAAYTAALV SGTATAGWTF GAGAALQIPF AMQMAYRFNG IGVTQNVLYE    900
NQKQIANQFN KAISQIQESL TTTSTALGKL QDVVNQNAQA LNTLVKQLSS NFGAISSVLN    960
DILSRLDKVE AEVQIDRLIT GRLQSLQTYV TQQLIRAAEI RASANLAATK MSECVLGQSK   1020
RVDFCGKGYH LMSFPQAAPH GVVFLHVTYV PSQERNFTTA PAICHEGKAY FPREGVFVFN   1080
GTSWFITQRN FFSPQIITTD NTFVSGNCDV VIGIINNTVY DPLQPELDSF KEELDKYFKN   1140
HTSPDVDLGD ISGINASVVN IQKEIDRLNE VAKNLNESLI DLQELGKYEQ YIKWPWYVWL   1200
GFIAGLIAIV MVTILLCCMT SCCSCLKGAC SCGSCCKFDE DDSEPVLKGV KLHYT        1255
```

<212> Type : PRT
<211> Length : 1255
SequenceName : SEQ ID 267
SequenceDescription :
Sequence --------
<213> OrganismName : SARS coronavirus TW1
<400> PreSequenceString :

```
MFIFLLFLTL TSGSDLDRCT TFDDVQAPNY TQHTSSMRGV YYPDEIFRSD TLYLTQDLFL    60
PFYSNVTGFH TINHTFGNPV IPFKDGIYFA ATEKSNVVRG WVFGSTMNNK SQSVIIINNS   120
TNVVIRACNF ELCDNPFFAV SKPMGTQTHT MIFDNAFNCT FEYISDAFSL DVSEKSGNFK   180
HLREFVFKNK DGFLYVYKGY QPIDVVRDLP SGFNTLKPIF KLPLGINITN FRAILTAFSP   240
AQDIWGTSAA AYFVGYLKPT TFMLKYDENG TITDAVDCSQ NPLAELKCSV KSFEIDKGIY   300
QTSNFRVVPS GDVVRFPNIT NLCPFGEVFN ATKFPSVYAW ERKKISNCVA DYSVLYNSTF   360
FSTFKCYGVS ATKLNDLCFS NVYADSFVVK GDDVRQIAPG QTGVIADYNY KLPDDFMGCV   420
LAWNTRNIDA TSTGNYNYKY RYLRHGKLRP FERDISNVPF SPDGKPCTPP ALNCYWPLND   480
YGFYTTTGIG YQPYRVVVLS FELLNAPATV CGPKLSTDLI KNQCVNFNFN GLTGTGVLTP   540
SSKRFQPFQQ FGRDVSDFTD SVRDPKTSEI LDISPCSFGG VSVITPGTNA SSEVAVLYQD   600
VNCTDVSTAI HADQLTPAWR IYSTGNNVFQ TQAGCLIGAE HVDTSYECDI PIGAGICASY   660
HTVSLLRSTS QKSIVAYTMS LGADSSIAYS NNTIAIPTNF SISITTEVMP VSMAKTSVDC   720
NMYICGDSTE CANLLLQYGS FCTQLNRALS GIAAEQDRNT REVFAQVKQM YKTPTLKYFG   780
GFNFSQILPD PLKPTKRSFI EDLLFNKVTL ADAGFMKQYG ECLGDINARD LICAQKFNGL   840
TVLPPLLTDD MIAAYTAALV SGTATAGWTF GAGAALQIPF AMQMAYRFNG IGVTQNVLYE   900

NQKQIANQFN KAISQIQESL TTTSTALGKL QDVVNQNAQA LNTLVKQLSS NFGAISSVLN   960
DILSRLDKVE AEVQIDRLIT GRLQSLQTYV TQQLIRAAEI RASANLAATK MSECVLGQSK  1020
RVDFCGKGYH LMSFPQAAPH GVVFLHVTYV PSQERNFTTA PAICHEGKAY FPREGVFVFN  1080
GTSWFITQRN FFSPQIITTD NTFVSGNCDV VIGIINNTVY DPLQPELDSF KEELDKYFKN  1140
HTSPDVDLGD ISGINASVVN IQKEIDRLNE VAKNLNESLI DLQELGKYEQ YIKWPWYVWL  1200
GFIAGLIAIV MVTILLCCMT SCCSCLKGAC SCGSCCKFDE DDSEPVLKGV KLHYT        1255
```

<212> Type : PRT
<211> Length : 1255
SequenceName : SEQ ID 268
SequenceDescription :
Sequence --------
<213> OrganismName : SARS coronavirus CUHK-Su10
<400> PreSequenceString :

```
MFIFLLFLTL TSGSDLDRCT TFDDVQAPNY TQHTSSMRGV YYPDEIFRSD TLYLTQDLFL    60
PFYSNVTGFH TINHTFGNPV IPFKDGIYFA ATEKSNVVRG WVFGSTMNNK SQSVIIINNS   120
TNVVIRACNF ELCDNPFFAV SKPMGTQTHT MIFDNAFNCT FEYISDAFSL DVSEKSGNFK   180
HLREFVFKNK DGFLYVYKGY QPIDVVRDLP SGFNTLKPIF KLPLGINITN FRAILTAFSP   240
AQDIWGTSAA AYFVGYLKPT TFMLKYDENG TITDAVDCSQ NPLAELKCSV KSFEIDKGIY   300
QTSNFRVVPS GDVVRFPNIT NLCPFGEVFN ATKFPSVYAW ERKKISNCVA DYSVLYNSTF   360
FSTFKCYGVS ATKLNDLCFS NVYADSFVVK GDDVRQIAPG QTGVIADYNY KLPDDFMGCV   420
LAWNTRNIDA TSTGNYNYKY RYLRHGKLRP FERDISNVPF SPDGKPCTPP ALNCYWPLND   480
YGFYTTTGIG YQPYRVVVLS FELLNAPATV CGPKLSTDLI KNQCVNFNFN GLTGTGVLTP   540
SSKRFQPFQQ FGRDVSDFTD SVRDPKTSEI LDISPCSFGG VSVITPGTNA SSEVAVLYQD   600
VNCTDVSTAI HADQLTPAWR IYSTGNNVFQ TQAGCLIGAE HVDTSYECDI PIGAGICASY   660
HTVSLLRSTS QKSIVAYTMS LGADSSIAYS NNTIAIPTNF SISITTEVMP VSMAKTSVDC   720
NMYICGDSTE CANLLLQYGS FCTQLNRALS GIAAEQDRNT REVFAQVKQM YKTPTLKYFG   780
GFNFSQILPD PLKPTKRSFI EDLLFNKVTL ADAGFMKQYG ECLGDINARD LICAQKFNGL   840
TVLPPLLTDD MIAAYTAALV SGTATAGWTF GAGAALQIPF AMQMAYRFNG IGVTQNVLYE   900
NQKQIANQFN KAISQIQESL TTTSTALGKL QDVVNQNAQA LNTLVKQLSS NFGAISSVLN   960
DILSRLDKVE AEVQIDRLIT GRLQSLQTYV TQQLIRAAEI RASANLAATK MSECVLGQSK  1020
RVDFCGKGYH LMSFPQAAPH GVVFLHVTYV PSQERNFTTA PAICHEGKAY FPREGVFVFN  1080
GTSWFITQRN FFSPQIITTD NTFVSGNCDV VIGIINNTVY DPLQPELDSF KEELDKYFKN  1140
HTSPDVDLGD ISGINASVVN IQKEIDRLNE VAKNLNESLI DLQELGKYEQ YIKWPWYVWL  1200
GFIAGLIAIV MVTILLCCMT SCCSCLKGAC SCGSCCKFDE DDSEPVLKGV KLHYT        1255
```

<212> Type : PRT
<211> Length : 1255
SequenceName : SEQ ID 269
SequenceDescription :
Sequence --------
<213> OrganismName : SARS coronavirus Urbani
<400> PreSequenceString :

MFIFLLFLTL TSGSDLDRCT TFDDVQAPNY TQHTSSMRGV YYPDEIFRSD TLYLTQDLFL 60
PFYSNVTGFH TINHTFGNPV IPFKDGIYFA ATEKSNVVRG WVFGSTMNNK SQSVIIINNS 120
TNVVIRACNF ELCDNPFFAV SKPMGTQTHT MIFDNAFNCT FEYISDAFSL DVSEKSGNFK 180
HLREFVFKNK DGFLYVYKGY QPIDVVRDLP SGFNTLKPIF KLPLGINITN FRAILTAFSP 240
AQDIWGTSAA AYFVGYLKPT TFMLKYDENG TITDAVDCSQ NPLAELKCSV KSFEIDKGIY 300
QTSNFRVVPS GDVVRFPNIT NLCPFGEVFN ATKFPSVYAW ERKKISNCVA DYSVLYNSTF 360
FSTFKCYGVS ATKLNDLCFS NVYADSFVVK GDDVRQIAPG QTGVIADYNY KLPDDFMGCV 420
LAWNTRNIDA TSTGNYNYKY RYLRHGKLRP FERDISNVPF SPDGKPCTPP ALNCYWPLND 480
YGFYTTTGIG YQPYRVVVLS FELLNAPATV CGPKLSTDLI KNQCVNFNFN GLTGTGVLTP 540
SSKRFQPFQQ FGRDVSDFTD SVRDPKTSEI LDISPCSFGG VSVITPGTNA SSEVAVLYQD 600
VNCTDVSTAI HADQLTPAWR IYSTGNNVFQ TQAGCLIGAE HVDTSYECDI PIGAGICASY 660
HTVSLLRSTS QKSIVAYTMS LGADSSIAYS NNTIAIPTNF SISITTEVMP VSMAKTSVDC 720
NMYICGDSTE CANLLLQYGS FCTQLNRALS GIAAEQDRNT REVFAQVKQM YKTPTLKYFG 780
GFNFSQILPD PLKPTKRSFI EDLLFNKVTL ADAGFMKQYG ECLGDINARD LICAQKFNGL 840
TVLPPLLTDD MIAAYTAALV SGTATAGWTF GAGAALQIPF AMQMAYRFNG IGVTQNVLYE 900
NQKQIANQFN KAISQIQESL TTTSTALGKL QDVVNQNAQA LNTLVKQLSS NFGAISSVLN 960
DILSRLDKVE AEVQIDRLIT GRLQSLQTYV TQQLIRAAEI RASANLAATK MSECVLGQSK 1020
RVDFCGKGYH LMSFPQAAPH GVVFLHVTYV PSQERNFTTA PAICHEGKAY FPREGVFVFN 1080
GTSWFITQRN FFSPQIITTD NTFVSGNCDV VIGIINNTVY DPLQPELDSF KEELDKYFKN 1140
HTSPDVDLGD ISGINASVVN IQKEIDRLNE VAKNLNESLI DLQELGKYEQ YIKWPWYVWL 1200

GFIAGLIAIV MVTILLCCMT SCCSCLKGAC SCGSCCKFDE DDSEPVLKGV KLHYT 1255

<212> Type : PRT
<211> Length : 1255
SequenceName : SEQ ID 270
SequenceDescription :
Sequence --------
<213> OrganismName : SARS coronavirus
<400> PreSequenceString :

MFIFLLFLTL TSGSDLDRCT TFDDVQAPNY TQHTSSMRGV YYPDEIFRSD TLYLTQDLFL 60
PFYSNVTGFH TINHTFGNPV IPFKDGIYFA ATEKSNVVRG WVFGSTMNNK SQSVIIINNS 120
TNVVIRACNF ELCDNPFFAV SKPMGTQTHT MIFDNAFNCT FEYISDAFSL DVSEKSGNFK 180
HLREFVFKNK DGFLYVYKGY QPIDVVRDLP SGFNTLKPIF KLPLGINITN FRAILTAFSP 240
AQDIWGTSAA AYFVGYLKPT TFMLKYDENG TITDAVDCSQ NPLAELKCSV KSFEIDKGIY 300
QTSNFRVVPS GDVVRFPNIT NLCPFGEVFN ATKFPSVYAW ERKKISNCVA DYSVLYNSTF 360
FSTFKCYGVS ATKLNDLCFS NVYADSFVVK GDDVRQIAPG QTGVIADYNY KLPDDFMGCV 420
LAWNTRNIDA TSTGNYNYKY RYLRHGKLRP FERDISNVPF SPDGKPCTPP ALNCYWPLND 480
YGFYTTTGIG YQPYRVVVLS FELLNAPATV CGPKLSTDLI KNQCVNFNFN GLTGTGVLTP 540
SSKRFQPFQQ FGRDVSDFTD SVRDPKTSEI LDISPCAFGG VSVITPGTNA SSEVAVLYQD 600
VNCTDVSTAI HADQLTPAWR IYSTGNNVFQ TQAGCLIGAE HVDTSYECDI PIGAGICASY 660
HTVSLLRSTS QKSIVAYTMS LGADSSIAYS NNTIAIPTNF SISITTEVMP VSMAKTSVDC 720
NMYICGDSTE CANLLLQYGS FCTQLNRALS GIAAEQDRNT REVFAQVKQM YKTPTLKYFG 780
GFNFSQILPD PLKPTKRSFI EDLLFNKVTL ADAGFMKQYG ECLGDINARD LICAQKFNGL 840
TVLPPLLTDD MIAAYTAALV SGTATAGWTF GAGAALQIPF AMQMAYRFNG IGVTQNVLYE 900
NQKQIANQFN KAISQIQESL TTTSTALGKL QDVVNQNAQA LNTLVKQLSS NFGAISSVLN 960
DILSRLDKVE AEVQIDRLIT GRLQSLQTYV TQQLIRAAEI RASANLAATK MSECVLGQSK 1020
RVDFCGKGYH LMSFPQAAPH GVVFLHVTYV PSQERNFTTA PAICHEGKAY FPREGVFVFN 1080
GTSWFITQRN FFSPQIITTD NTFVSGNCDV VIGIINNTVY DPLQPELDSF KEELDKYFKN 1140
HTSPDVDLGD ISGINASVVN IQKEIDRLNE VAKNLNESLI DLQELGKYEQ YIKWPWYVWL 1200
GFIAGLIAIV MVTILLCCMT SCCSCLKGAC SCGSCCKFDE DDSEPVLKGV KLHYT 1255

<212> Type : PRT
<211> Length : 1255
SequenceName : SEQ ID 271
SequenceDescription :
Sequence --------
<213> OrganismName : SARS coronavirus Tor2
<400> PreSequenceString :

```
MFIFLLFLTL TSGSDLDRCT TFDDVQAPNY TQHTSSMRGV YYPDEIFRSD TLYLTQDLFL      60
PFYSNVTGFH TINHTFGNPV IPFKDGIYFA ATEKSNVVRG WVFGSTMNNK SQSVIIINNS     120
TNVVIRACNF ELCDNPFFAV SKPMGTQTHT MIFDNAFNCT FEYISDAFSL DVSEKSGNFK     180
HLREFVFKNK DGFLYVYKGY QPIDVVRDLP SGFNTLKPIF KLPLGINITN FRAILTAFSP     240
AQDIWGTSAA AYFVGYLKPT TFMLKYDENG TITDAVDCSQ NPLAELKCSV KSFEIDKGIY     300
QTSNFRVVPS GDVVRFPNIT NLCPFGEVFN ATKFPSVYAW ERKKISNCVA DYSVLYNSTF     360
FSTFKCYGVS ATKLNDLCFS NVYADSFVVK GDDVRQIAPG QTGVIADYNY KLPDDFMGCV     420
LAWNTRNIDA TSTGNYNYKY RYLRHGKLRP FERDISNVPF SPDGKPCTPP ALNCYWPLND     480
YGFYTTTGIG YQPYRVVVLS FELLNAPATV CGPKLSTDLI KNQCVNFNFN GLTGTGVLTP     540
SSKRFQPFQQ FGRDVSDFTD SVRDPKTSEI LDISPCAFGG VSVITPGTNA SSEVAVLYQD     600
VNCTDVSTAI HADQLTPAWR IYSTGNNVFQ TQAGCLIGAE HVDTSYECDI PIGAGICASY     660
HTVSLLRSTS QKSIVAYTMS LGADSSIAYS NNTIAIPTNF SISITTEVMP VSMAKTSVDC     720
NMYICGDSTE CANLLLQYGS FCTQLNRALS GIAAEQDRNT REVFAQVKQM YKTPTLKYFG     780
GFNFSQILPD PLKPTKRSFI EDLLFNKVTL ADAGFMKQYG ECLGDINARD LICAQKFNGL     840
TVLPPLLTDD MIAAYTAALV SGTATAGWTF GAGAALQIPF AMQMAYRFNG IGVTQNVLYE     900
NQKQIANQFN KAISQIQESL TTTSTALGKL QDVVNQNAQA LNTLVKQLSS NFGAISSVLN     960
DILSRLDKVE AEVQIDRLIT GRLQSLQTYV TQQLIRAAEI RASANLAATK MSECVLGQSK    1020
RVDFCGKGYH LMSFPQAAPH GVVFLHVTYV PSQERNFTTA PAICHEGKAY FPREGVFVFN    1080
GTSWFITQRN FFSPQIITTD NTFVSGNCDV VIGIINNTVY DPLQPELDSF KEELDKYFKN    1140
HTSPDVDLGD ISGINASVVN IQKEIDRLNE VAKNLNESLI DLQELGKYEQ YIKWPWYVWL    1200
GFIAGLIAIV MVTILLCCMT SCCSCLKGAC SCGSCCKFDE DDSEPVLKGV KLHYT         1255
```

<212> Type : PRT
<211> Length : 1255
SequenceName : SEQ ID 272
SequenceDescription :
Sequence
--------
<213> OrganismName : SARS coronavirus GD01
<400> PreSequenceString :

```
MFIFLLFLTL TSGSDLDRCT TFDDVQAPNY TQHTSSMRGV YYPDEIFRSD TLYLTQDLFL      60
PFYSNVTGFH TINHTFDNPV IPFKDGIYFA ATEKSNVVRG WVFGSTMNNK SQSVIIINNS     120
TNVVIRACNF ELCDNPFFAV SKPMGTQTHT MIFDNAFNCT FEYISDAFSL DVSEKSGNFK     180
HLREFVFKNK DGFLYVYKGY QPIDVVRDLP SGFNTLKPIF KLPLGINITN FRAILTAFLP     240
AQDTWGTSAA AYFVGYLKPT TFMLKYDENG TITDAVDCSQ NPLAELKCSV KSFEIDKGIY     300
QTSNFRVVPS RDVVRFPNIT NLCPFGEVFN ATKFPSVYAW ERKRISNCVA DYSVLYNSTF     360
FSTFKCYGVS ATKLNDLCFS NVYADSFVVK GDDVRQIAPG QTGVIADYNY KLPDDFMGCV     420
LAWNTRNIDA TSTGNYNYKY RYLRHGKLRP FERDISNVPF SPDGKPCTPP ALNCYWPLND     480
YGFYTTTGIG YQPYRVVVLS YELLNAPATV CGPKLSTDLI KNQCVNFNFN GLTGTGVLTP     540
SSKRFQPFQQ FGRDVSDFTD SVRDPKTSEI LDISPCSFGG VSVITPGTNA SSEVAVLYQD     600
VNCTDVSTAI HADQLTPAWR IYSTGNNVFQ TQAGCLIGAE HVDTSYECDI PIGAGICASY     660
HTVSLLRSTS QKSIVAYTMS LGADSSIAYS NNTIAIPTNF SISITTEVMP VSMAKTSVDC     720
NMYICGDSTE CANLLLQYGS FCTQLNRALS GIAAEQDRNT REVFAQVKQM YKTPTLKDFG     780
GFNFSQILPD PLKSTKRSFI EDLLFNKVTL ADAGFMKQYG ECLGDINARD LICAQKFNGL     840
TVLPPLLTDD MIAAYTAALV SGTATAGWTF GAGAALQIPF AMQMAYRFNG IGVTQNVLYE     900
NQKQIANQFN KAISQIQESL TTTSTALGKL QDVVNQNAQA LNTLVKQLSS NFGAISSVLN     960
DILSRLDKVE AEVQIDRLIT GRLQSLQTYV TQQLIRAAEI RASANLAATK MSECVLGQSK    1020
RVDFCGKGYH LMSFPQAAPH GVVFLHVTYV PSQERNFTTA PAICHEGKAY FPREGVFVFN    1080
GTSWFITQRN FFSPQIITTD NTFVSGNCDV VIGIINNTVY DPLQPELDSF KEELDKYFKN    1140
HTSPDVDLGD ISGINASVVN IQKEIDRLNE VAKNLNESLI DLQELGKYEQ YIKWPWYVWL    1200
GFIAGLIAIV MVTILLCCMT SCCSCLKGAC SCGSCCKFDE DDSEPVLKGV KLHYT         1255
```

<212> Type : PRT
<211> Length : 1255
SequenceName : SEQ ID 273
SequenceDescription :
Sequence --------
<213> OrganismName : SARS coronavirus CUHK-W1
<400> PreSequenceString :

```
MFIFLLFLTL TSGSDLDRCT TFDDVQAPNY TQHTSSMRGV YYPDEIFRSD TLYLTQDLFL   60
PFYSNVTGFH TINHTFDNPV IPFKDGIYFA ATEKSNVVRG WVFGSTMNNK SQSVIIINNS  120
TNVVIRACNF ELCDNPFFAV SKPMGTQTHT MIFDNAFNCT FEYISDAFSL DVSEKSGNFK  180
HLREFVFKNK DGFLYVYKGY QPIDVVRDLP SGFNTLKPIF KLPLGINITN FRAILTAFSP  240
AQDTWGTSAA AYFVGYLKPT TFMLKYDENG TITDAVDCSQ NPLAELKCSV KSFEIDKGIY  300
QTSNFRVVPS GDVVRFPNIT NLCPFGEVFN ATKFPSVYAW ERKKISNCVA DYSVLYNSTF  360
FSTFKCYGVS ATKLNDLCFS NVYADSFVVK GDDVRQIAPG QTGVIADYNY KLPDDFMGCV  420
LAWNTRNIDA TSTGNYNYKY RYLRHGKLRP FERDISNVPF SPDGKPCTPP ALNCYWPLND  480
YGFYTTTGIG YQPYRVVVLS FELLNAPATV CGPKLSTDLI KNQCVNFNFN GLTGTGVLTP  540
SSKRFQPFQQ FGRDVSDFTD SVRDPKTSEI LDISPCSFGG VSVITPGTNA SSEVAVLYQD  600
VNCTDVSTAI HADQLTPAWR IYSTGNNVFQ TQAGCLIGAE HVDTSYECDI PIGAGICASY  660
HTVSLLRSTS QKSIVAYTMS LGADSSIAYS NNTIAIPTNF SISITTEVMP VSMAKTSVDC  720
NMYICGDSTE CANLLLQYGS FCTQLNRALS GIAAEQDRNT REVFAQVKQM YKTPTLKYFG  780
GFNFSQILPD PLKPTKRSFI EDLLFNKVTL ADAGFMKQYG ECLGDINARD LICAQKFNGL  840
TVLPPLLTDD MIAAYTAALV SGTATAGWTF GAGAALQIPF AMQMAYRFNG IGVTQNVLYE  900
NQKQIANQFN KAISQIQESL TTTSTALGKL QDVVNQNAQA LNTLVKQLSS NFGAISSVLN  960
DILSRLDKVE AEVQIDRLIT GRLQSLQTYV TQQLIRAAEI RASANLAATK MSECVLGQSK 1020
RVDFCGKGYH LMSFPQAAPH GVVFLHVTYV PSQERNFTTA PAICHEGKAY FPREGVFVFN 1080
GTSWFITQRN FFSPQIITTD NTFVSGNCDV VIGIINNTVY DPLQPELDSF KEELDKYFKN 1140
HTSPDVDLGD ISGINASVVN IQKEIDRLNE VAKNLNESLI DLQELGKYEQ YIKWPWYVWL 1200
GFIAGLIAIV MVTILLCCMT SCCSCLKGAC SCGSCCKFDE DDSEPVLKGV KLHYT       1255
```

<212> Type : PRT
<211> Length : 1255
SequenceName : SEQ ID 274
SequenceDescription :
Sequence --------
<213> OrganismName : SARS coronavirus BJ01
<400> PreSequenceString :

```
MFIFLLFLTL TSGSDLDRCT TFDDVQAPNY TQHTSSMRGV YYPDEIFRSD TLYLTQDLFL   60
PFYSNVTGFH TINHTFDNPV IPFKDGIYFA ATEKSNVVRG WVFGSTMNNK SQSVIIINNS  120
TNVVIRACNF ELCDNPFFAV SKPMGTQTHT MIFDNAFNCT FEYISDAFSL DVSEKSGNFK  180
HLREFVFKNK DGFLYVYKGY QPIDVVRDLP SGFNTLKPIF KLPLGINITN FRAILTAFSP  240
AQDTWGTSAA AYFVGYLKPT TFMLKYDENG TITDAVDCSQ NPLAELKCSV KSFEIDKGIY  300
QTSNFRVVPS GDVVRFPNIT NLCPFGEVFN ATKFPSVYAW ERKKISNCVA DYSVLYNSTF  360
FSTFKCYGVS ATKLNDLCFS NVYADSFVVK GDDVRQIAPG QTGVIADYNY KLPDDFMGCV  420
LAWNTRNIDA TSTGNYNYKY RYLRHGKLRP FERDISNVPF SPDGKPCTPP ALNCYWPLND  480
YGFYTTTGIG YQPYRVVVLS FELLNAPATV CGPKLSTDLI KNQCVNFNFN GLTGTGVLTP  540
SSKRFQPFQQ FGRDVSDFTD SVRDPKTSEI LDISPCSFGG VSVITPGTNA SSEVAVLYQD  600
VNCTDVSTAI HADQLTPAWR IYSTGNNVFQ TQAGCLIGAE HVDTSYECDI PIGAGICASY  660
HTVSLLRSTS QKSIVAYTMS LGADSSIAYS NNTIAIPTNF SISITTEVMP VSMAKTSVDC  720
NMYICGDSTE CANLLLQYGS FCTQLNRALS GIAAEQDRNT REVFAQVKQM YKTPTLKYFG  780
GFNFSQILPD PLKPTKRSFI EDLLFNKVTL ADAGFMKQYG ECLGDINARD LICAQKFNGL  840
TVLPPLLTDD MIAAYTAALV SGTATAGWTF GAGAALQIPF AMQMAYRFNG IGVTQNVLYE  900
NQKQIANQFN KAISQIQESL TTTSTALGKL QDVVNQNAQA LNTLVKQLSS NFGAISSVLN  960
DILSRLDKVE AEVQIDRLIT GRLQSLQTYV TQQLIRAAEI RASANLAATK MSECVLGQSK 1020
RVDFCGKGYH LMSFPQAAPH GVVFLHVTYV PSQERNFTTA PAICHEGKAY FPREGVFVFN 1080
GTSWFITQRN FFSPQIITTD NTFVSGNCDV VIGIINNTVY DPLQPELDSF KEELDKYFKN 1140
HTSPDVDLGD ISGINASVVN IQKEIDRLNE VAKNLNESLI DLQELGKYEQ YIKWPWYVWL 1200
GFIAGLIAIV MVTILLCCMT SCCSCLKGAC SCGSCCKFDE DDSEPVLKGV KLHYT       1255
```

<212> Type : PRT
<211> Length : 1255
SequenceName : SEQ ID 275
SequenceDescription :
Sequence --------
<213> OrganismName : SARS coronavirus
<400> PreSequenceString :

```
SGFRKMAFPS GKVEGCMVQV TCGTTTLNGL WLDDTVYCPR HVICTAEDML NPNYEDLLIR    60
KSNHSFLVQA GNVQLRVIGH SMQNCLLRLK VDTSNPKTPK YKFVRIQPGQ TFSVLACYNG   120
SPSGVYQCAM RPNHTIKGSF LNGSCGSVGF NIDYDCVSFC YMHHMELPTG VHAGTDLEGK   180
FYGPFVDRQT AQAAGTDTTI TLNVLAWLYA AVINGDRWFL NRFTTTLNDF NLVAMKYNYE   240
PLTQDHVDIL GPLSAQTGIA VLDMCAALKE LLQNGMNGRT ILGSTILEDE FTPFDVVRQC   300
SGVTFQ                                                              306
```

<212> Type : PRT
<211> Length : 306
SequenceName : SEQ ID 276
SequenceDescription :
Sequence --------
<213> OrganismName : SARS coronavirus
<400> PreSequenceString :

```
AIASEFSSLP SYAAYATAQE AYEQAVANGD SEVVLKKLKK SLNVAKSEFD RDAAMQRKLE    60
KMADQAMTQM YKQARSEDKR AKVTSAMQTM LFTMLRKLDN DALNNIINNA RDGCVPLNII   120
PLTTAAKLMV VVPDYGTYKN TCDGNTFTYA SALWEIQQVV DADSKIVQLS EINMDNSPNL   180
AWPLIVTALR ANSAVKLQ                                                 198
```

<212> Type : PRT
<211> Length : 198
SequenceName : SEQ ID 277
SequenceDescription :
Sequence --------
<213> OrganismName : SARS coronavirus
<400> PreSequenceString :

```
AGNATEVPAN STVLSFCAFA VDPAKAYKDY LASGGQPITN CVKMLCTHTG TGQAITVTPE    60
ANMDQESFGG ASCCLYCRCH IDHPNPKGFC DLKGKYVQIP TTCANDPVGF TLRNTVCTVC   120
GMWKGYGCSC DQLREPLMQ                                                139
```

<212> Type : PRT
<211> Length : 139
SequenceName : SEQ ID 278
SequenceDescription :
Sequence
--------
<213> OrganismName : SARS coronavirus
<400> PreSequenceString :

```
NNELSPVALR QMSCAAGTTQ TACTDDNALA YYNNSKGGRF VLALLSDHQD LKWARFPKSD    60
GTGTIYTELE PPCRFVTDTP KGPKVKYLYF IKGLNNLNRG MVLGSLAATV RLQ          113
```

<212> Type : PRT
<211> Length : 113
SequenceName : SEQ ID 279
SequenceDescription :
Sequence --------
<213> OrganismName : Escherichia coli 0157:H7
<400> PreSequenceString :

```
MNKIFKVIWN PATGNYTVTS ETAKSRGKKS GRSKLLISAL VAGGMLSSFG ALANAGNDNG     60
QGVDYGSGSA GDGWVAIGKG AKANTFMNTS GSSTAVGYDA IAEGQYSSAI GSKTHAIGGA    120
SMAFGVSAIS EGDRSIALGA SSYSLGQYSM ALGRYSKALG KLSIAMGDSS KAEGANAIAL    180
GNATKATEIM SIALGDTANA SKAYSMALGA SSVASEENAI AIGAETEAAE NATAIGNNAK    240
AKGTNSMAMG FGSLADKVNT IALGNGSQAL ADNAIAIGQG NKADGVDAIA LGNGSQSRGL    300
NTIALGTASN ATGDKSLALG SNSSANGINS VALGADSIAD LDNTVSVGNS SLKRKIVNVK    360
NGAIKSDSYD AINGSQLYAI SDSVAKRLGG GAAVDVDDGT VTAPTYNLKN GSKNNVGAAL    420
AVLDENTLQW DQTKGKYSAA HGTSSPTASV ITDVADGTIS ASSKDAVNGS QLKATNDDVE    480
ANTANIATNT SNIATNTANI ATNTTNITNL TDSVGDLQAD ALLWNETKKA FSAAHGQDTT    540
SKITNVKDAD LTADSTDAVN GSQLKTTNDA VATNTTNIAN NTSNIATNTT NISNLTETVT    600
NLGEDALKWD KDNGVFTAAH GTETTSKITN VKDGDLTTGS TDAVNGSQLK TTNDAVATNT    660
TNIATNTTNI SNLTETVTNL GEDALKWDKD NGVFTAAHGN NTASKITNIL DGTVTATSSD    720
AINGSQLYDL SSNIATYFGG NASVNTDGVF TGPTYKIGET NYYNVGDALA AINSSFSTSL    780
GDALLWDATA GKFSAKHGTN GDASVITDVA DGEISDSSSD AVNGSQLHGV SSYVVDALGG    840
GAEVNADGTI TAPTYTIANA DYDNVGDALN AIDTTLDDAL LWDADAGENG AFSAAHGKDK    900
TASVITNVAN GAISAASSDA INGSQLYTTN KYIADALGGD AEVNADGTIT APTYTIANAE    960
YNNVGDALDA LDDNALLWDE TANGGAGAYN ASHDGKASII TNVANGSISE DSTDAVNGSQ   1020
LNATNMMIEQ NTQIINQLAG NTDATYIQEN GAGINYVRTN DDGLAFNDAS AQGVGATAIG   1080
YNSVAKGDSS VAIGQGSYSD VDTGIALGSS SVSSRVIAKG SRDTSITENG VVIGYDTTDG   1140
ELLGALSIGD DGKYRQIINV ADGSEAHDAV TVRQLQNAIG AVATTPTKYF HANSTEEDSL   1200
AVGTDSLAMG AKTIVNGDKG IGIGYGAYVD ANALNGIAIG SNAQVIHVNS IAIGNGSTTT   1260
RGAQTNYTAY NMDAPQNSVG EFSVGSADGQ RQITNVAAGS ADTDAVNVGQ LKVTDAQVSQ   1320
NTQSITNLDN RVTNLDSRVT NIENGIGDIV TTGSTKYFKT NTDGVDASAQ GKDSVAIGSG   1380
SIAAADNSVA LGTGSVATEE NTISVGSSTN QRRITNVAAG KNATDAVNVA QLKSSEAGGV   1440
RYDTKADGSI DYSNITLGGG NGGTTRISNV SAGVNNNDVV NYAQLKQSVQ ETKQYTDQRM   1500
VEMDNKLSKT ESKLSGGIAS AMAMTGLPQA YTPGASMASI GGGTYNGESA VALGVSMVSA   1560
NGRWVYKLQG STNSQGEYSA ALGAGIQW                                      1588
```

<212> Type : PRT
<211> Length : 1588
SequenceName : SEQ ID 280
SequenceDescription :
Sequence --------
<213> OrganismName : Escherichia coli O157:H7
<400> PreSequenceString :

```
MPASAVGALG EASYTVTANV TDSAGNSNSA SHNVQVNTAL PGVTINPVAT DDIINAAESG     60
NAQTISGQVT GAAAGDTVTV TLGGKTYTAT VQGNLSWSVD VPAADIQAIG NGNLTVNASV    120
TNGVGNTGSG SRDITIDANL PGLRVDTVAG DDVVNSIEHA QALVITGSSS GLAAGAALTV    180
VINTVTYAAT VLADGTWSVG VPAADVSNWP AGTVNITVSG TNTAGTTSTI THPVTVDLAA    240
VAISINTVSG DDVINAAEKG ADLTLSGSTS GVEVGQTVTV TFGGKTYTAT VAGDGSWTTT    300
VPAADLSVLR DGDATVQASV STINGNTASA THAYSVDATA PTLAINTIAT DDILNAAEAG    360
NPLTISGSST AEAGQTVTVT LNGVTYSGSV QADGSWSVSL PTADLSNLTA SQYTVSASVS    420
DKAGNPASAN HGLAVDLTVP VLTINTVSGD DIINAAEHGQ ALVISGSSTG GEAGDVITVT    480
LNSKTYTTML DASGNWSVGV PAADVTALGS GPQTITAAIT DAAGNSDDAS RTVTVNLAAP    540
TIGINTIATD DVIKATEKGA DLQITGTSNQ PAGTTITVTL NGQNYTATTD SNGNWSATVP    600
ASAVSALGEA NYTVTANVTD TAGNSNSASH NVLVNSALPA VTINAVATDD IINAAESGNA    660
QTISGQVTGA AQGDTVTVTL GGNTYTATVQ SNLSWSVDVP AADIQALGNG DLTVNASVTN    720
GVGNTGSGSR DITIDANLPG LRVDTVAGDD VINSIEHNQA LVITGSSSGL TAGTALTVEI    780
```

NNVTYGATVL ADGTWSLGVP AVDVSNWPAG TVNITVSGTN SAGTTSTITH PVTVDLAGVA 840
ITINTLSGDD VINAVEKGET LVVSGSTSGV EAGQTVTVTF GGKNYTTTVE ANGSWTVNVP 900
PADLAALPDG AGNVQASVSN INGNSAQADR AYSVDATAPL VTINTIASDD ILNVSEAGAG 960
ITISGTTTAQ AGQTLTVTLN NNTYQTTVLA DGTWSVNVPA ADLSGLTASS YTVTATVSDK 1020
AGNPASADHA LVVDITAPDL TINTVAGDDI INAIEHGQAL VVSGTSTGAA AGDVVTVTLN 1080
GKNYTTTLDA SGNWSVGIPA ADVTALATGS QTITASLSDR AGNSDSTTHD VTVDLSGPTL 1140
TINTVSGDDI INAAEIVVAQ TISGQVTGTA VAGNTVIVTI GGNQYNATVQ SDLSWSVSVP 1200
ANVLQALGNG ELTISASLTN SANNTGTATH DIVIDANLPG LRVDTVAGDD VINSIEHTQA 1260
LVITGSSSGL AAGAALTVVI NSVTYGATVL ADGSWSVGVP VADVTNWPAG TVNIAVSGTN 1320
TAGTTTSISH PVTVDLAAVA ITINTLSTDD VINAAEKGSD LQLSGTTSGV EAGQTITVIF 1380
GGKSYTTTVA ADNTWGLTIP AVDVATLPDG AANVQASVSN VAGNSTQATH AYSVDATAPS 1440
VTINTIATDD ILNAAEAGSA LTISGTSTAE AGQTVTVTLN GVNYSGNVQA DGSWSVSVPT 1500
GDLASLTASS YTVNASVSDK ARNSASATHN LTVDLAAPVV TINTVAGDDI INATEHGQAQ 1560
IISGSATGAT TGNTVSVTIG TTTYTTVLDA NGNWSIGVPA SVISALAQGD VTITATVTDS 1620
AGNSGTASHT VTVALGAPVL AINTIAVDDI INAAEKGADL AITGTSNQPA GTQITVTLNG 1680
QNYTTTADAS GNWSVTVPAS RVSALGEATY TVTAAATDAD GNSGSASHNV QVNTALPGVT 1740
INVVATDDII NAAEAGVEQT ISGQVTGAAA GDTVTVTLGG ATYTATVQAN LSWSVDVPAS 1800
ALQELGNGEL TISASVTNSV GNTGNGTREI TIDANLPGLR VDTVAGDDVV NIIEHGQALV 1860
ITGSSSGLAA GSNVTLTING QTYVAAVLAD GTWSVGVPAV DVSAWPAGSV TIAASGSTSA 1920
GNPVSVTHPV TVDLSAVAVS INAITADDVI NAAEKGAALT LSGSTSGVEA GQTVTVTFGG 1980
KTYSATVAAN GSWSTSVPAA DMAALRDGDA SAQASVSNVN GNSATTTHAY SVDASAPTVT 2040
INTIAGDDIL NAAEAGAALT ITGSSTAEAG QTVTVTLNGT NYTGTVQTDG SWSVSVPSAD 2100
LSTLTASNYT VNAAVSDKAG NPASVNHNLT VDTSVPVVTI NTVAGDDVIN ATEHAQAQII 2160
SGSATGAATG STVTVTIGTN TFTTVLDASG NWSVGVPASV VSALANGTVT INASVTDAGG 2220
NSGSATHQVT VNTGLPTITF NAISGDNILN ADEKGQPLTI SGGSTGLATG AQVTVTLNGH 2280
NYSATTDASG NWTLTVPVSD LAALGQANYT VSASATSAAG NTASSQANLL VDSGLPDVTI 2340
NTVAGDDIIN AAEAGADQTI SGVVTRAAAG DTVTVTLGGN TYTATVQSNL SWSVSVPTAD 2400
LQALGNGDLT ITASVTNANG NTGSGTRDIT IDANLPGLRV DTVAGDDIVN SIEHGQALVI 2460
TGGSSGLNAG AVLTVTINSV AYSATVQADG SWSVGIPAAN VSAWPAGPLT VEVDGQSSAN 2520
NPVSVSHPFT VDLTAVAISI NTVASDDVIN AAEKGTNLTL SGSTSGIESG QTVTVTFGGK 2580
TYTASVAANG SWSVNVPAAD LATLPEGAAN VQASVSSASG NSASATHAYS VDASAPTLTI 2640
NTIASDDILN AAEAGSPLTI SGTSTAETGQ TVTVTLNGAT YTGTVQADGS WSVSVPTSAL 2700
GALNASNYTV SATVNDKAGN PGSASHNLAV DTTAPVLTIN TVAGDDIIND AEHAQALVIS 2760
GTSSGGEAGD VVSVVLNGKT YTTTLDASGN WSVGVPAADV TALGSGAQTI TASVSDRAGN 2820
SDDASRTVTV SLSAPVISIN TIAGDDVINA TEKGSDLALS GTSDQPAGTA ITVTLNGQNY 2880
SATTDASGNW SVTVPASAVS ALGEATYSVT ASVTNAQGNS STASHNVQVN TALPGITINP 2940
VATDDIINAS EAGSAQTISG QVTGAAAGST VTVELGGKTY TATVQADLSW NVSVPAADWQ 3000
ALGNGELTVN ASVTNAVGNT GSGTRDITID ASLPGLRVDT VAGDDVVNII EHAQAQVITG 3060
SSSGFAAGTA LTVVINNQTY AATVLANGSW SVGVPATDVS NWPAGTLNIT VSGANSAGTQ 3120
TSITHPLTVD LTAVAISMNS ITSDDAINAA EKGAALTLSG STSGVEAGQT VTVTFGGKTY 3180
TTTVAANGSW STTVPAADLA ALRDGDASAQ VRVTNVNGNS ATATHEYSVD SAAPTVTINT 3240
IASDNIINAS EAAAGVTVSG TSTAQTGQTL TVTLNGTNYQ TTVQTDGSWS LTLPASDLTA 3300
LANNGYTLTA TVSDLAGNLG SASKGVTVDT TAPVISFNTV AGDDVINNVE HIQAQIISGT 3360
ATGAVAGDRL VVTIAGQQYV TSTDASGNWS VGVPASVISG LADGTVTISA TITDSAGNSS 3420
TQTHNVQVNT AAVSLSVSTI SGDNLINAAE AGSALTLSGT GTNFATGTVV TVLLNGKGYS 3480
ATIQSNGSWS VNVPAADVAA LSDGTSYTVS ASAQDSAGNG NSSTQTHNVQ VNTAAVSLSV 3540
STISGDNLIN AAEAGSALTL SGTGTNFATG TVVTVLLNGK GYSATIQSNG SWSVNVPAAD 3600
VAALSDGTSY TVSASAQDSA GNSATASRSV AVDLTAPVIS INTVSTDDRL NAAEQQQPLT 3660
LNGSTSAEVG QTVTVTFGGK TYTATVAANG TWALNVPAVD LAALGQGAQT ITASVNDRAG 3720
NPGQATHALT VDTVAPTVTI ATVAGDDIIN NAEQLAGQTI SGTTTAEVGQ TVTVTFNGQT 3780
WSATVGSGGS WSVFIPAQQF AGLSDGSYTI SATVSDQAGN PGSASRGVTL NGDVPTVTIN 3840
TFAGDDVVNA AEHGSSLVIS GTTTAPVGQT LTLTLNGKTY TTTVQTGGSW SYTLGSADVT 3900
ALADGNAYVI NASVSNAIGN TGSSNHTITV DLSAPAMGIN IDSLQADTGL SASDFITSVS 3960
PVVVNGSLTA ALASNETAQI SIDGGTTWTT LTVTGTTWRY NDSRTLTDGN YLYQVRVIDA 4020
AGNVGATDSQ NVVIDTTAPD PAVKTIAISA ITTDMGLITN DFVTSDTTLA VSGTLGATLS 4080
AGEFAQISLD GGVTWTTLTV VGTSWSYADG HTLTDGTWNY TVRVVDLAGN VGQTATQNVV 4140
VDTTSPEAAK SITITGISDD TGTSSSDFIT SDTTLTVRGV LGAALGANEF AQISTDNGAT 4200
WVNVTVAADS LNWSYVDGRT LTNGTTTWQV RVVDLAGNVG ATSSQSALID TVNPAQVLTI 4260
ASISTDTGSS ATDFITSDTM LTLTGSLGAG LASGEVAQIS LDSGATWTTL TTNGTQWTYT 4320
DSRTLTDGSY VYQVRVLDLA GNTGPVVSKT VVVDTINPTA TPTIVSYTDD VGQRQGTLSS 4380
SQATDDTTPL LNGVLSAPLA SGEVVYLYRN GLLLGAVTMV GALNWTYSDS GLVSGAYTYS 4440
ARVVDLAGNI TSSSDFVLTV DTSIPTTLAQ ITSQTTRDTT PIISGVITAA LASGQYVEVV 4500
INGKTYTSEP GGAVVVDPAH NTWYVQLPDT DALTVSATAY TVTAQVKSSA GNGNNANISN 4560
GTVTVNAAID YTPTWTTASK TTAWGLTYGL DSHGMWTVLA NQQVMQSTDP LTWSKTALTL 4620
YQSGNNYATS SIADYDRNGT GDLFITRDDY GTGYINGFTN NGDGTFSSAI QVTVGTLTWY 4680
GSIVAFDKEG DGYLDFWIGD AGGPDSNTFL WNNAGTLVGN STTSNSGGSA TVGGAVTGYL 4740
SLNEGSGVDL NNDGRIDLVQ HTYNLNNYYT LSSLINQGNG TFVWGQNTTN TFLSGAGSGA 4800

```
MSSSVSMTWA DFDGDGDMDL FLPASQGRAN YGSLLFNTNG VLGCPVAVGA TATTYASQFS    4860
LAVDWNHDGL MDIARIAQTG QSYLYTNVSN ASNWTQSALG GSQSGTTSGV AAMDYDWDGA    4920
VDVLVSKQSG SVFLSRNTNT VSYGTSLHLR ITDPNGINVY YGNTVKLYNS AGVLVATQII    4980
NPQSGMGVND TSALVNFYGL NAGETYNAVL IKSTGTTASN IDQTVNTSWG GLQATDATHA    5040
YDLSAEAGTA SNNGKFVGTG YNDTFFATAG TDTYDGSGGW VYSSGTGTWL ANGGMDVVDF    5100
RLSTVGVTAN LSSTAAQATG FNTSTFTNIE GISGSNFNDI LTGSSGDNQL EGRGGNDTLN    5160
IGNGGHDTLL YKLLNASDAT GGNGSDVVNG FTVGTWEGTA DTDRIDIREL LQGSGYTGNG    5220
KASYVNGVAT LDAQAGNIGD FVKVTQSGSD TIVQIDRDGT GGTFATTNVV TLTGVHTDLA    5280
TLLANHQLMV V                                                         5291
```

<212> Type : PRT
<211> Length : 5291
SequenceName : SEQ ID 281
SequenceDescription :
Sequence --------
<213> OrganismName : Escherichia coli O157:H7
<400> PreSequenceString :

```
MGVHTAEATL PNGNNDTKIV NIAPDASNAQ VTLNIPAQQV VTNNSDSVQL TATVKDPSNH      60
PVAGITVNFT MPQDVAANFT LENNGIAITQ ANGEAHVTLK GKKAGTHTVT ATLSNNNTSD     120
SQPVTFVADK TSALVVLQIS KNEITGNGVD SATLTATVKD QFDNEVNNLP VTFSTASSGL     180
TLTPGESNTN ESGIAQATLA GVAFGEQTVT ASLANNGASD NKTVHFIGDT AAAKIIELTP     240
VPDSIIAGTP QNSSGSVITA TVVDNNGFPV KGVTVNFTSN AATAEMTNGG QAVTNEQGKA     300
TVTYTNTRSS IESGARPDTV EASLENGSST LSTSINVNAD ASTAHLTLLQ ALFDTVSAGD     360
TTNLYIEVKD NYGNGVPQQE VTLSVSPSEG VTPSNNAIYT TNHDGNFYAS FTATKAGVYQ     420
VTATLENGDS MQQTVTYVPN VANAEISLAA SKDPVIANNN DLTTLTATVA DTEGNAIANS     480
EVTFTLPEDV RANFTLGDGG KVVTDTEGKA KVTLKGTKAG AHTVTASMAG GKSEQLVVNF     540
IADTLTAQVN LNVTEDNFIA NNVGMTRLQA TVTDGNGNPL ANEAVTFTLP ADVSASFTLG     600
QGGSAITDIN GKAEVTLSGT KSGTYPVTVS VNNYGVSDTK QVTLIADAGT AKLASLTSVY     660
SFVVSTTEGA TMTASVTDAN GNPVEGIKVN FRGTSVTLSS TSVETDDRGF AEILVTSTEV     720
GLKTVSASLA DKPTEVISRL LNAKADINSA TITSLEIPEG QVMVAQDVAV KAHVNDQFGN     780
PILNESVTFS AEPPEHMTIS QNIVSTDTHG IAEVTMTPER NGSYMVKASL ANGSSYEKDL     840
VVIDQKLTLS ASSPLIGVNS PTGATLTATL TSANGTPVEG QVINFSVTPE GATLSGGKVR     900
TNSSGQAPVV LTSNKVGTYT VTASFHNGVT IQTQTIVKVT GNSSTAHVAS FIADPSTIAA     960
TNSDLSTLKA TVEDGSGNLI EGLTVVYFALK SGSATLTSLT AVTDQNGIAT TSVRGAITGS   1020
VTVSAVTTAG GMQTVDITLV AGPADASQSV LKNNRSSLKG DFTDSAELHL VLHDISGNPI    1080
KVSEGLEFVQ SGTNAPYVQV SAIDYSKNFS GEYKATVTGG GEGIATLIPV LNGVHQAGLS    1140
TTIQFTRAED KIMSGTVLVN GANLPTTTFP SQGFTGAYYQ LNNDNFAPGK TAADYEFSSS    1200
ASWVDVDATG KVTFKNVGSK WERITATPKT GGPSYIYEIR VKSWWVNAGD AFMIYSLAEN    1260
FCSSNGYTLP LGDHLNHSRS RGIGSLYSEW GDMGHYTTEA GFHSNMYWSS SPANSNEQYV    1320
VSLATGDQSV FEKLGFAYAT CYKNL                                          1345
```

<212> Type : PRT
<211> Length : 1345
SequenceName : SEQ ID 282
SequenceDescription :
Sequence --------
<213> OrganismName : Escherichia coli O157:H7
<400> PreSequenceString :

MSLIIDVISR KTSVKQTLIN PGDVTVVIYE PSVVQVHAQA SAVARYVREG NDLLIYMQDG 60
TVIRCNGYFL QAANTAEQSE LVFADGQQLT HITFADTAAG GLAPVELTAQ TTAIESIAPF 120
LDTVAQTSAF PWGWLAGAAV GGGALGALLA SGGDGDSKTE VINNPTPPAE PGNATPSFLV 180
TDNQGDQRGI LATNDITDDT TPTFSGSGQA GATIQIKDSN GNTIASTQVD NNGHWSVSLP 240
TQSAGEHTWS VVQIVGSTIT DAGSITLTID NSQASVQVAT TAGDNIINAS EQAAGFTLSG 300
TSSHLAQGTE LTVTLNGKTY TTSVGANGAW SVQVPTADAQ ALGEGNQAVL VSGKDATGNT 360
VTGAQLLTVD TQPPTLAINT IAQDNIISAA EHNVALVLSG TSNAEAGQTV TLTVNGKSHT 420
ATVGSDGTWQ VTLPATEVQA LAEGNYAVNA SVSDRAGNTT SHSANFTVDT SAPVVSVNTV 480
AGDDILNNAE QAVAQIISGQ VSGASPGDTV TVKLGTHVLT GIVLADGSWN VALDPAVTRT 540
LDRGANTIFV TVTDAAGNTG AASRAITLVG VSPLITINTV SGDDIISGAE KGAPLTLTGS 600
TQQAETGQTV TVTLAGQSFT TTVQADGSWS LTVPAAAMGN LPDGAVAITA SVTDLSGNTG 660
NTSRTITVDS QAPALSIDPL TADNIINAAE SGQDLPITGT TDAQPGQTVT VTLNGQTYQG 720
VVQPDGTWSV TVPAANVGAL ADGNATVTAS VNDVAGNPSS VSRVALVDAT PPVVTINPVA 780
TDNVINTPEH AQAQIISGTV TGAQAGDIVT VTLNNVDYTT VVDGSGNWSL GVPASVVSGL 840
ADGSYPVSVS VTDKAGNTGS QSLTVTVNTA APLIGINSIA GDDVINASEK GADLQITGTS 900
DQPVNTAITV TLNGQNYTTT TDASGNWSVT VPASAVTALG QANYTVTAAV TSDIGNSATA 960
SHNVLVDSAL PGVTINPVAT DDIINAAEAG VAQTISGQVT GAEDGDTVTI TLGGNTYTAT 1020

VGSNLTWSVD VPAADIQALG NGDLTVNASV TNQNGNTGSG TRDITIDANL PGLRVDTVAG 1080
DDVVNIIEHG QALVVTGSSS GLAESTPLTV TINNVEYTTA VQADGSWSVG VTAAQVSAWP 1140
AGTVNIAVSG ESSAGNSVSI THPVTVDLTP AAITINTIAT DDVINAAEKG ADLTLSGTTT 1200
NVEPGQTVTV TFGGKNYTAS VASDGSWTAT VPAADLASLP EGSASALASV SNINGNSASA 1260
VHNYSVDSSA PTIIINTVAS DNIVNASEAD AGVTVSGSTT AEAGQIVTIT LNSPTVQTYQ 1320
ATVQADGSWS INIPAADLEA LTDGSHTLTA TVNDKAGNPA STTHNLAVDL TVPVLTINTI 1380
AGDDIINATE HGQALVISGS STGGEAGDVV TVTLNSKTYT TTLDASGNWS VGVPAADVTA 1440
LGSGPQTVTA TVTDAAGNSD N 1461

<212> Type : PRT
<211> Length : 1461
SequenceName : SEQ ID 283
SequenceDescription :
Sequence --------
<213> OrganismName : Escherichia coli O157:H7
<400> PreSequenceString :

MNRIYRVIWN CTLQVFQACS ELTRRVGKTS TVNLRKSSGL TTKFSRLTLG VLLALSGSVS 60
GASLEVDNGQ ITNIDTDVAY DAYLVGWYGT GVLNILAGGN ASLTTITTSV IGGNEDSEGT 120
VNVLGGTWRL YDSGNNARPL NVGQSGTGTL NIKQKGHVDG GYLRLGTQAA GVGTVNVEGE 180
DSVLTTELFE IGSYGTGSLN ITDKGYVTSS IVAILGYQAN SNGKVVVEKG GEWLIKNNDS 240
SIEFQIGNQG TGEATIREGG LITAENTIIG GNATGVGTLN VQDQDSVITV RRLYNGYFGN 300
GAVNISNNGL INNKEYSLVG VQDGSHGVVN VTDKGHWNFL GTGEAFRYIY IGDAGDGELN 360
VSREGKVDSG IITAGMKETG TGNLTVKDKN SVITNLGTNL GYDGHGEMNI SNEGLVVSNG 420
GSSLGYGETG VGKVSITTGG IWEVNKNVYT TIGVAGVGNL NISDGGKFVS QNITFLGDKA 480
SGIGTLNLMD ATSSFDTVGI NVGNFGSGIV NVSNGATLNS TGYGFIGGNA SGKGIVNIST 540
DSLWNLKTSS TNAQLLQVGV LGTGELNITT GGIVKARDTQ IALNDKSKGD VRVDGQNSLL 600
ETFNMYVGTS GTGTLTLTNS GTLNVEGGEV YLGVFEPAVG TLNIGAAHGE AAADAGFITN 660
ATKVEFGSGE GVFVFNHTNN SDAGYQVDML ITGDDKDGKV IHDAGHTVFN AGNTYSGKTL 720
VNDGLLTIAS HTADGVTGMG SSEVTIASPG TLDILASTNS AGDYTLTNAL KGDGLMRVQL 780
SSSDKMFGFT HATGTEFAGV AQLKDSTFTL ERDNTAALTH AMLQSDIENT TSVNVGEQSI 840
GGLAMNGGTL IFDTDIPAAT LAEGYISVDT LVVGASDYTW KGRNYQVNGT GDVLIGVPKP 900
WNDPMANNPL TTLNLLEHDD NHVGVQLVKA QTVIGSGGSL TLRDLQGDEV EADKTLHIAQ 960
NGTVVAEGDY GFRLTTAPGD GLYVNYGLKA LNIHGGQKLT LAEHGGAYGA TADMSAKIGG 1020
EGDLAINTVR QVSLSNGQND YQGATYVQMG TLRTDADGAL GNTRELNISN AAIVDLNGST 1080
QTVETFTGQM GSTVLFKEGS LTVNKGGISQ GELTGGGNLN VTGGTLAVEG LNARYNALTS 1140
VSPNAEVSLD NTQGLGRGNI ANDGLLTLKN VTGELRNSIS GKGIVSATAR TDVELDGDNS 1200
RFVGQFNIDT GSALSVNEQK NLGDASVINN GLLTISTERS WAMTHSISGS GDLTKLGTGI 1260
LTLNNDSSAY QGTTDIVGGE IAFGSDSAIN TASQHINIHN SGVMSGNVTT AGDVNVMSGG 1320
TLRVAKTTIG ESAATWRMAA RFK 1343

<212> Type : PRT
<211> Length : 1343
SequenceName : SEQ ID 284
SequenceDescription :
Sequence --------
<213> OrganismName : Escherichia coli O157:H7

<400> PreSequenceString :

MGIKQHNGNT KADRLAELKI RSPSIQLIKF GAIGLNAILF SPLLIAADTG SQYGTNITIN 60
DGDRITGDTA DPSGNLYGVM TPAGNTPGNI NLGNDVTVNV NDASGYAKGI IIQGKNSSLT 120
ANRLTVDVVG QTSAIGINLI GDYTHADLGT GSTIKSNDDG IIIGHSSTLT ATQFTIENSN 180
GIGLTINDYG TSVDLGSGSK IKTDGSTGVY IGGLNGNNAN GAARFTATDL TIDVQGYSAM 240
GINVQKNSVV DLGTNSSIKT SGDNAHGLWS FGQVSANALT VDVTGAAANG VEVRGGTTTI 300
GADSHISSAQ GGGLVTSGSD ATINFSGTAA QRNSIFSGGS YGASAQTATA VINMQNTDIT 360
VDRNGSLALG LWALSGGRIT GDSLAITGAA GARGIYAMTN SQIDLTSDLV IDMSTPDQMA 420
IATQHDDGYA ASRINASGRM LINGSVLSKG GLINLDMHPG SVWTGSSLSD NVNGGKLDVA 480
MNNSVWNVTS NSNLDTLALS HSTVDFASHG STAGTFTTLN VENLSGNSTF IMRADVVGEG 540
NGVKPWA 547

<212> Type : PRT
<211> Length : 547
SequenceName : SEQ ID 285
SequenceDescription :
Sequence --------
<213> OrganismName : Escherichia coli O157:H7

<400> PreSequenceString :
MGIDSRNDIP EGIATLGAFM GYSHSHIGFD RGGHGSVDSY SLGGYASWEH ESGFYLDGVV 60
KLNRFESNVA GKMSSGGAAN GSYHSNGLGG HIETGMRFTD GNWNLTPYAS LTGFTADNPE 120
YHLSNGMESK SVDTRSIYRE LGATLSYNMR LGNGMEVEPW LKAAVRKEFV DDNRVKVNSD 180
GNFVNDLSGR RGIYQAGIKA SFSSTLSGHL GVGYSNGAGM ESPWNAVAGV NWSF 234

<212> Type : PRT
<211> Length : 234
SequenceName : SEQ ID 286
SequenceDescription :
Sequence --------
<213> OrganismName : Escherichia coli O157:H7
<400> PreSequenceString :

MKKKVLAIAL VTVFTGMGVA QAADVTAQAV ATWSATAKKD TTSKLVVTPL GSLAFQYAEG 60
IKGFNSQKGL FDVAIEGDST ATAFKLTSRL ITNTLTQLDT SGSTLNVGVD YNGAAVEKTG 120
DTVMIDTANG VLGGNLSPLA NGYNASNRTT AQDGFTFSII SGTTNGTTAV TDYSTLPEGI 180
WSGDVSVQFD ATWTS 195

<212> Type : PRT
<211> Length : 195
SequenceName : SEQ ID 287
SequenceDescription :
Sequence --------
<213> OrganismName : Escherichia coli O157:H7
<400> PreSequenceString :

MTAESYDDNY LDDEDADWTA TGQGQKSAGD TSFTLAWKPG EEGQKGLIGW FESGDVRAYK 60
IRFPNGTVDV FRGWVSSIGK AVTAKEVITR TVKVTNVGKP SVAEERSKIT PVSAIKVTPT 120
SGTVAKGKTT TLTVSFEPES ATDKTFRAVS ADPSKATISV KDMTITVNGV ATGKVQIPVV 180
SGNGQFAAVA EVTVTEAGAA G 201

<212> Type : PRT
<211> Length : 201
SequenceName : SEQ ID 288
SequenceDescription :
Sequence --------
<213> OrganismName : Escherichia coli O157:H7
<400> PreSequenceString :

MTAESYDDNY LDDEDADWTA TGQGQKSAGD TSFTLAWKPG EEGQKGLIGW FESGDVRAYK 60
IRFPNGTVDV FRGWVSSIGK AVTAKEVITR TVKVTNVGKP SVAEERSKIT PVSAIKVTPT 120
SGTVAKGKTT TLTVSFEPES ATDKTFRAVS ADPSKATISV KDMTITVNGV ATGKVQIPVV 180
SGNGQFAAVA EVTVTEAGAA G 201

<212> Type : PRT
<211> Length : 201
SequenceName : SEQ ID 289
SequenceDescription :
Sequence --------
<213> OrganismName : Escherichia coli O157:H7
<400> PreSequenceString :

MLYNIPCRIY ILSTLSLCIS GIVSTATATS SETKISNEET LVVTTNRSAS NLWESPATIQ 60
VIDQQTLQNS TNASIADNLQ DIPGVEITDN SLAGRKQIRI RGEASSRVLI LIDGQEVTYQ 120
RAGDNYGVGL LIDESALERV EVVKGPYSVL YGSQAIGGIV NFITKKGGDK LASGVVKAVY 180
NSATAGWEES IAVQGSIGGF DYRINGSYSD QGNRDTPDGR LPNTNYRNNS QGVWLGYNSG 240
NHRFGLSLDR YRLATQTYYE DPDGSYEAFS VKIPKLEREK VGVFYDTDVD GDYLKKIHFD 300
AYEQTIQRQF ANEVKTTQPV PSPMIQALTV HNKTDTHDKQ YTQAVTLQSH FSLPANNELV 360
TGAQYKQDRV SQRSGGMTSS KSLTGFINKE TRTRSYYESE QSTVSLFAQN DWQFADHWTW 420
TMGVRQYWLS SKLTRGDGVS YTAGIISDTS LARESASDHE MVTSTSLRYS GFDNLELRAA 480
FAQGYVFPTL SQLFMQTSAG GSVTYGNPDL KAEHSNNFEL GARYNGNQWL IDSAVYYSEA 540
KDYIASLICD GSIVCNGNTN SSRSSYYYYD NIDRAKTWGL EISAEYNGWV FSPYISGNLI 600
RRQYETSTLK TTNTGEPAIN GRIGLKHTLV MGQANIISDV FIRAASSAKD DSNGTETNVP 660
GWATLNFAVN TEFGNEDQYR INLALNNLTD KRYRTAHETI PAAGFNAAIG FVWNF 715

<212> Type : PRT
<211> Length : 715
SequenceName : SEQ ID 290
SequenceDescription :
Sequence --------
<213> OrganismName : Escherichia coli O157:H7
<400> PreSequenceString :

MTKMSRYALI TALAMFLAGC VGQREPAPVE EVKPAPEQPA EPQQPVPTVP SVPTIPQQPG 60
PIEHEDQTAP PAPHIRHYDW NGAMQPMVSK MLGADGVTAG SVLLVDSVNN RTNGSLNAAE 120
ATETLRNALA NNGKFTLVSA QQLSMAKQQL GLSPQDSLGT RSKAIGIARN VGAHYVLYSS 180
ASGNVNAPTL QMQLMLVQTG EIIWSGKGAV SQQ 213

<212> Type : PRT
<211> Length : 213
SequenceName : SEQ ID 291
SequenceDescription :
Sequence --------
<213> OrganismName : Escherichia coli O157:H7
<400> PreSequenceString :

MKSKVLALLI PALLGAGAAH AAEVYNKDGN KLDLYGKVDG LHYFSDNSAK DGDQSYARLG 60
FKGETQINDQ LTGYGQWEYN IQANNTESSK NQSWTRLAFA GLKFSDYGSF DYGRNYGLDR 120
YAA 123

<212> Type : PRT
<211> Length : 123
SequenceName : SEQ ID 292
SequenceDescription :
Sequence --------
<213> OrganismName : Escherichia coli O157:H7
<400> PreSequenceString :

```
MATPNPLEPV KGAGTTLWVY NGKGDAYANP LSDDDWQRLA KVKDLTPGEM TAEPYDDNYL   60
DDEDADWTAT GQGQKSAGDT SFTLAWKPGE EGQKGLIGWF ESGDVRAYKI RFPNGTVDVF  120
RGWVSSIGKA VTAKEVITRT VKVTNVGKPS VAEERSEITP ATAIKVTPTS GTVAKGKTTT  180
LTVSFEPESA TDKTFRAVSA DPSKATISVK DMTITVNGVA TGKVQIPVVS GNGQFAAVAE  240
VTVTEAGAAG                                                         250
```

<212> Type : PRT
<211> Length : 250
SequenceName : SEQ ID 293
SequenceDescription :
Sequence --------
<213> OrganismName : Escherichia coli O157:H7
<400> PreSequenceString :

```
MATPNPLEPV KGAGTTLWVY NGKGDAYANP LSDDDWQRLA KVKDLTPGEM TAEPYDDNYL   60
DDEDADWTAT GQGQKSAGDT SFTLAWKPGE EGQKGLIGWF ESGDVRAYKI RFPNGTVDVF  120
RGWVSSIGKA VTAKEVITRT VKVTNVGKPS VAEERSEITP ATAIKVTPTS GTVAKGKTTT  180
LTVSFEPESA TDKTFRAVSA DPSKATISVK DMTITVNGVA TGKVQIPVVS GNGQFAAVAE  240
VTVTEAGAAG                                                         250
```

<212> Type : PRT
<211> Length : 250
SequenceName : SEQ ID 294
SequenceDescription :
Sequence --------
<213> OrganismName : Escherichia coli O157:H7
<400> PreSequenceString :

```
MGWTDMLPEF GGDSYTNADN FMTGRANGVA TYRNTDFFGL VNGLNFAVQY QGNNEGASNG   60
QEGTNNGRDV RHENGDGWGL STTYDLGMGF SAGAAYTSSD RTNDQVNHTA AGGDKADAWT  120
AGLKYDANNI YLATMYSETR NMTPFGDSDY AVANKTQNFE VTAQYQFDFG LRPAVSFLMS  180
KGRDLHAAGG ADNPAGVDDK DLVKYADVGA TYYFNKNMST YVDYKINLLD EDDSFYAANG  240
ISTDDIVALG LVYQF                                                   255
```

<212> Type : PRT
<211> Length : 255
SequenceName : SEQ ID 295
SequenceDescription :
Sequence --------
<213> OrganismName : Haemophilus influenzae Rd
<400> PreSequenceString :

```
MGFIMKLTKT ALCTALFATF TFSANAQTYP DLPVGIKGGT GALIGDTVYV GLGSGGDKFY   60
TLDLKDPSAQ WKEIATFPGG ERNQPVAAAV DGKLYVFGGL QKNEKGELQL VNDAYRYNPS  120
DNTWMKLPTR SPRGLVGSSG ASHGDKVYIL GGSNLSIFNG FFQDTVAAGE DKAKKDEIAA  180
AYFDQRPEDY FFTTELLSYE PSTNKWRNEG RIPFSGRAGA AFTIQGNELV VVNGEIKPGL  240
RTAETHQGKF TAKGVQWKNL PDLPAPKGKS QDGLAGALSG YSNGHYLVTG GANFPGSIKQ  300
FKEGKLHAHK GLSKAWHNEV YTLNNGKWRI VGELPMNIGY GFSVSYNNKV LLIGGETDGG  360
KALTSVKAIS YDGKKLTIE                                               379
```

<212> Type : PRT
<211> Length : 379
SequenceName : SEQ ID 296
SequenceDescription :
Sequence --------
<213> OrganismName : Haemophilus influenzae Rd
<400> PreSequenceString :

MGEQYMLTTI LSFLIVTTVV AYVSWLKTKG DDLKSSKGYF LAGRGLSGLV IGCSMVLTSL 60
STEQLIGVNA VSYKGNFSVI AWTVPTVIPL CFLALYIIGW L 101

<212> Type : PRT
<211> Length : 101
SequenceName : SEQ ID 297
SequenceDescription :
Sequence --------
<213> OrganismName : Helicobacter pylori J99
<400> PreSequenceString :

MKNQHKNPLT KALMKTYPYN HFLFFCFILG AFLLGLLSPA YALSIITTKE IDANLLNGAI 60
ESRVVLGKRV FKVEAHGFYF RNNATNSIDI EITSLLRDNQ SFPLTSSAKT SLKIPPNAKI 120
KKSTILVLKG ENAEEVAKIL GVSKEEYQKL ENIAQTKAAN DPMYANTPFS NGSDSSFYDN 180
NPNSPSNNAI NGKDGANGSN GYGANGNDGV NGISGSNGAN GSHSNNNAIG SGIDTDGVLG 240
VDGVNGSSSS SGGSVGGYEN NFTNHGSTNN NTGGYDNFNN GSSSGGSLGN GGLFPIPFGN 300
GDTNNSNNST NTTSPTNGSS SNNATNPSSQ ENNYSSQYCK VPELSPNNTM KLDVIAKDGS 360
CISMNALRDD TKCAYRYDFE AGKAIKQTQY YYVDRENKTQ NIGGCVDLQG AQYAMQLYKD 420
DSKCALQTTS DKGYGMGKTQ TFQTEIVFRG MDNLIHVAVP CSDYARVQDR IVRYEKNDKT 480
QTLTPIVDQY YNDPNNPNKQ EILNRGIATQ LSSQYQEFAC GQWEYNDAKL EAKRPTMLKS 540
YNKLNGEWVE VTPCNFEAGI KSGAVVSPYV MGVPSSKVLS DITTSHYFRI ERKNYGEREQ 600
CQKLYGVNRC QPQYSILILV SPIGAPLTKP LPPKPLNLIY AQPKIMKNTP QPIILSPLKP 660
PSTGLKAF 668

<212> Type : PRT
<211> Length : 668
SequenceName : SEQ ID 298
SequenceDescription :
Sequence --------
<213> OrganismName : Helicobacter pylori J99
<400> PreSequenceString :

MPVIRVLVML ATMMMKLVKT AKEKKVFKNV GISIMGIAFW EAIKDSIKKQ IKKSDWICGN 60
VKTADDYLKT HPNSWFNSAI GVTAITAMLM NVCFADDQSK KEVAQAQKEA ENARDRANKS 120
GIELEQEEQK TEQEKQKTEQ EKQKTEQEKQ KTEQEKQKTE QEKQKTSNIE TNNQIKVEQE 180
QQKTEQEKQK TNNTQKDLVN KAEQNCQENH NQFFIKKLGI KAGIAIEIEA ECKTPKPTKT 240
NQTPIQPKHL PNSKQPHSQR GSKAQELIAY LQKELESLPY SQKAIAKQVD FYRPSSIAYL 300
ELDPRDFNAT EEWQKENLKI RSKAQAKMLE MRSLKPDPQA HLSTSQSLLL VQKIFADVSK 360
EIKVVANTEK KVEKAGYGYS KRM 383

<212> Type : PRT
<211> Length : 383
SequenceName : SEQ ID 299
SequenceDescription :
Sequence --------
<213> OrganismName : Helicobacter pylori J99
<400> PreSequenceString :

MNYPNLPNSA LEISEQPEVK EITNELLKQL QNALRSNAHF SEQVELSLKC IVRILEVLLS 60
LDFFKNANEI DSSLRNSIEW LTNAGESLKL KMKEYERFFS EFNTSMHANE QEVTNTLNAN 120
AENIKSEIKK LENQLIETTT RLLTSYQIFL NQARDNANNQ ITKNKTQSLE AITQAKNNAN 180
NEISNNQTQA ITNITEAKTN ANNEISNNQT QAITNINEAK ESATTQINAN KQEAINNITQ 240
EKTQATSEIT EAKKTDHYQN IDFFEFE 267

<212> Type : PRT
<211> Length : 267
SequenceName : SEQ ID 300
SequenceDescription :
Sequence --------
<213> OrganismName : Helicobacter pylori J99
<400> PreSequenceString :

MKFFSKDLFK KVTPLFLSVY FLSPTLTQAK SRFYVASQYQ VGKMIMKKYN DLKRTIEGAS 60
FSLGWEINPT NYWFYSRYYF FMDYGNVILN KRTGAQANMF TYGFGGDLIM EYNKNPLYVF 120
SLFYGMQVAE NTWTISKHSA NFIIDDWRSI QGFSLKTSNF RMLGLVGFKF QTVLFHHDAS 180
IEVGIKWPFA FEYDSPFVRL FSVFISHTFY L 211

<212> Type : PRT
<211> Length : 211
SequenceName : SEQ ID 301
SequenceDescription :
Sequence --------
<213> OrganismName : Helicobacter pylori J99
<400> PreSequenceString :

MKKFTLSLFL CCTLLNAEED IFRNNTNETD LTNSFEHGKE NNNLIPAKSD SLESFKEQEN 60
KEKAKQLMDL KALQSVYFSK NRKLQDNNFN VLYVAGNTNK IRLRYAMTTT FIFDNDPIIY 120
VSLGDPSDFE LTYPTNDHYD LSNMLVIKPL LIGVDTNLTV VGASGTIYTL LFV 173

<212> Type : PRT
<211> Length : 173
SequenceName : SEQ ID 302
SequenceDescription :
Sequence --------
<213> OrganismName : Mycoplasma pneumoniae
<400> PreSequenceString :

MLDYVPWIGN GYRYGNNHRG SNSSTSGVTT QGQSQNASSN EPAPTFSNVG VGLKANVNGT 60
LSGSRTTPNQ QGTPWLTLDQ ANLQLWTGAG WRNDKNGQSD ENYTNFASAK GSTNQQGSTT 120
GGSAGNPDSL KQDKADKSGD SVTVAEATSG DNLTNYTNLP PTSPPHPTDR TRCHSPTRTT 180
PSGCSCSCAA CWAASRCWSI RVGKMITVSL IPPTKNGLTP N 221

<212> Type : PRT
<211> Length : 221
SequenceName : SEQ ID 303
SequenceDescription :
Sequence --------
<213> OrganismName : Mycoplasma pneumoniae
<400> PreSequenceString :

MDDITAPQTS AGSSSGTSTN TSGSRSFLPT FSNVGVGLKA NVQGTLGGRQ TTTTGNNIPK 60
WATLDQANLQ LWTGAGWRND KTTSGSTGNA NDTKFTSATG SGSGQGSSSG TNTSAGNPDG 120
LQADKVDQNG QVKTSVQEAT SGDNLTNYTN LPPANLTPTA DWPNALSFTN KNNAQRAQLF 180
LRGLLGSIPV LVNKSGQDDN SKFKAEDQKW SYTDLQSDQT KLNLPAYGEV NGLLNPALVE 240
TYFGNTRASG SGSNTTSSPG IGFKIPEQSG TNTTSKAVLI TPGLAWTPQD VGNIVVSGTS 300
FSFQLGGWLV TFTDFIKPRA GYLGLQLTGL DVSEATQREL IWAKRPWAAF RGSWVNRLGR 360
VESVWDFKGV WADQAQLAAQ AATSSTTTTA TGATLPEHPN ALAYQISYTD KDSYKASTQG 420
SGQTNSQNNS PYLHFIKPKK VESTTQLDQG LKNLLDPNQV RTKLRQSFGT DHSTQPQPQS 480

LKTTTPVFGR SSGNLSSVFS GGGAGGGSSG SGQSGVDLSP VERVSGH 527

<212> Type : PRT
<211> Length : 527
SequenceName : SEQ ID 304
SequenceDescription :
Sequence --------
<213> OrganismName : Mycoplasma pneumoniae
<400> PreSequenceString :

```
MLKLAVGIFI SPTLTRFSTG FNLAGSVLDQ VLDYVPWIGN GHRYGNNHRG VDDITAPKTG   60
AGSSSGTSTN TSGSRSFLPT FSNVGVGLKA NVQGTLGGSQ TTTTGKDIPK WPTLDPANLQ  120
LWTGAGWRND KASNKQSDEN HTTFKSATGS GQQGGSTTGG SAGNPDSLKQ DKISKSGQNL  180
TTQDGAPQSN STTESASNYD HLPPNLTPTS DWPNALSFTN KNNAQRAQLF LRGLLGSIPV  240
LVNRSGSDDS NKFQATDQKW SYTDLKSDQT KLNLPAYGEV NGLLNPALVE TYFGTTRAGG  300
SGSNTTSSPG IGFKIPEQNN DSKAVLITPG LAWTPQDVGN LVVSGTSLSF QLGGWLVTFT  360
DFVKPRAGYL GLQLTGLDAS DATQRALIWA KRPWAAFRGS WVNRLGRVES VWDLKGVWQD  420
QAQAAAQAAT TAAATGDALP EHPNALAYQI SSTDKDSYKA STQSSGQTNS QNTSPYLHLI  480
KPKKVENTTQ LDQGLKTCWT PTRFAPSCAK ALVQTIPPKP NPNPSKQPHR CLGRIVVTLA  540
VCLVVGVLEE QTAPIRWTSP PLNGWVGGLW GNYPVGVGGI VVRILKVCKT LLFISIFISI  600
FFLNCSLTLF IWTTASLATG LTVVGHFTST TTTLKRQQFS YTRPDEVALR HTNAINPRLT  660
PWTYRNTSFS SLPLTGENPG AWALVRDNTA KGITAGSGSQ QTTYDPTRTE AALTTATTFV  720
LRRYDLAGRC TTSTFRS                                                 737
```

<212> Type : PRT
<211> Length : 737
SequenceName : SEQ ID 305
SequenceDescription :
Sequence --------
<213> OrganismName : Mycoplasma pneumoniae
<400> PreSequenceString :

```
MLDYIPWIGN GHRYGNDHRG SNSSTSGVTT QGQQSQNASG TEPASTFSNV GVGLKANVQG   60
TLGGSQTTTT GKDIPKWPTL DQANLQLWTG AGWRNDKASS GQSDENHTKF TSATGSGQQG  120
SSSGTTNSAG NPDSLKQDKV DKSGDSVTVA ETTSGDNLTN YTNLPPNLTP TADWPNALSF  180
TNKNNAQRAQ LFLRALLGSI PVLVNKSGQD DSNKFQATDQ KWSYTELKSD QTKLNLPAYG  240
EVNGLLNPAL VEVYGLSSTQ GSSTGAGGAG GNTGGDTNTQ TYARPGIGFK LPSTDSESSK  300
ATLITPGLAW TAQDVGNLVV SGTSLSFQLG GWLVTFTDFI KPRSGYLGLQ LTGLDANDSD  360
QRELIWAPPA LNRLSWQLGQ PLGPRGECVG FQGGVGGSSS VRLASSYKYH HRNEGYLIGA  420
HQCFGLSGEL YRPGFVQGFH SKLRPKPKHL PLPALGAGEK SRFLW                  465
```

<212> Type : PRT
<211> Length : 465
SequenceName : SEQ ID 306
SequenceDescription :
Sequence --------
<213> OrganismName : Mycoplasma pneumoniae
<400> PreSequenceString :

```
MLGSIPVLVN RSGSDSNKFQ ATDQKWSYTD LQSDQTKLNL SAYGEVNGLL NPALVETYFG   60
TTRTSSTANQ NSTTVPGIGF KIPEQNNDSK ATLITPGLAW TPQDVGNLVV SGTTVSFQLG  120
GWLVTFTDFV KPRAGYLGLQ LSGLNASDSD QRELIWAPRP WAAFRGSWVN RLGRVESVWD  180
LKGVWADQAQ LAAQAATSST TTTATGATLP EHPNALAYQI SYTDKDSYKA STQGSGQTNS  240
QNNSLYLHLI KPKKVESTTQ LDQGLKNLLD PNQVRTKLRQ SFGTDHSTQP QPQSLKTTTP  300
VFGAMSGNLG SVLSGGGAGG AGSTNSVDLS PVERVSGSLT INRNFSY                347
```

<212> Type : PRT
<211> Length : 347
SequenceName : SEQ ID 307
SequenceDescription :
Sequence --------
<213> OrganismName : Mycoplasma pneumoniae
<400> PreSequenceString :

```
MGQQGQSGTS AGNPDSLKQD KISKSGDSLT TQDGNATGQQ EATNYTNLPP NLTPTADWPN   60
ALSFTNKNNA HRAQLFLRGL LGSIPVLVNR SGSDSNKFQA TDQKWSYTDL QSDQTKLNLP  120
AYGEVNGLLN PALVETYFGN TRAGGSGSNT TSSPGIGFKI PEQNNDSKAT LITPGLAWTP  180
```

QDVGNLVVSG TSLSFQLGGW LVSFTDFIKP RAGYLGLQLS GLDASDSDQR ELIWAKRPWA 240
AFRGSWVNRL GRVESVWDLK GVWADQAQLA AQAATSEASG SALAPHPNAL AFQVSVVEAS 300
AYSSSTSSSG SGSSSNTSPY LHLIKPKKVE STTQLDQGLK NLLDPNQVRT KLRQSFGTDH 360
STQPQSLKTT TPVFGTSSGN IGSVLSGGGA GGGSSGSGQS GVDLSPVERV SGH 413

<212> Type : PRT
<211> Length : 413
SequenceName : SEQ ID 308
SequenceDescription :
Sequence --------
<213> OrganismName : Mycoplasma pneumoniae
<400> PreSequenceString :

MGLQLSGLDA SDSDQRELIW AKRPWAAFRG SWVNRLGRVE SVWDLKGVWA DQAHSAVSES 60
QAATSSTTTT ATGDTLPEHP NALAYQISST DKDSYKASTQ GSGQTNSQNT SPYLHLIKPK 120
KVTASDKLDD DLKNLLDPNE VRVKLRQSFG TDHSTQPQPQ PLKTTTPVFG TNSGNLGSVL 180
SGGGTTQDSS TTNQLSPVQR VSGWLVGQLP STSDGNTSST NNLAPNTNTG NEVVGVGDLS 240
KRASIESSRL WIALKP 256

<212> Type : PRT
<211> Length : 256
SequenceName : SEQ ID 309
SequenceDescription :
Sequence. --------
<213> OrganismName : Mycoplasma pneumoniae
<400> PreSequenceString :

MRDNTAKGIT AGSGSQQTTY DPARTEATLT TTTFALRRYD LAGRALYDLD FSKLNPQTPT 60
RDANCQITFN PFGGFGLSGS APQQWNEVKN KVPVEVAQDP TDPYRFAVLL VPRSVVYYEQ 120
LQRGLALPNQ GSSSGSGQQN TTIGAYGLKV KNAEADTAKS NEKLQGDESK SSNGSSSTST 180
TTQRGSTNSD TKVKALKIEV KKKSDSEDNG QLQLEKNDLA NAPIKRGEES GQSVQLKADD 240
FGTAPSSSGS GGNSNPGSPT PWRPWLATEQ IHKDLPKWSA SILILYDAPY ARNRTAIDRV 300
DHLDPKVMTA NYPPSWRMPK WNHHGLWDWK ARDVLFQTTG FDESNTSNTK QGFQKEADSD 360
KSAPIALPFE AYFANIGNLT WFGQALLVFG GNGHVTKSAH TAPLSIWLYI YLVKAVTFRL 420
LLANSLLSKS NIYKKTAN 438

<212> Type : PRT
<211> Length : 438
SequenceName : SEQ ID 310
SequenceDescription :
Sequence --------
<213> OrganismName : Mycoplasma pneumoniae
<400> PreSequenceString :

MRDNIAKGIT AGSNTQQTTY DPTRTEATLT TATTFALRRY DLAGRALYDL DFSKLNPQTP 60
TRDQTGQITF NPFGGFGLSG AAPQQWNEVK DKVPVEVAQD PSNPYRFAVL LVPRSVVYYE 120
QLQRGLALPN QGSSSGSGQQ NTTIGAYGLK VKNAEADTAK SNEKLQGYES KSSNGSSSTS 180
TTQRGGSSNE NKVKALQVAV KKKSGSQGNS GDQGTEQVEL ESNDLANAPI KRGSNNNQQV 240
QLKADDFGTA PSSSGSGTQD GTPTPWTPWL TTEQIHNDPA KFAASILILY DAPYARNRTA 300
IDRVDHLDPK VMTANYPPSW RTPKWNHHGL WDWKARDVLL QTTGFFNPRR HPEWFDGGQT 360
VADNEKTGFD VDNSENTKQG FQKEADSDKS APIALPFEAY FANIGNLTWF EQALLVFGIC 420
LS 422

<212> Type : PRT
<211> Length : 422
SequenceName : SEQ ID 311
SequenceDescription :
Sequence --------
<213> OrganismName : Mycoplasma pneumoniae
<400> PreSequenceString :

```
MLWPFRWVWW KRVLTSQTRA PAKPNPLTVP PTCTWWSLRK LPNPTKLDDD LKNLLDPNEV    60
RARMLKSFGT ENFTQPQPQP QALKTTTPVF GTSSGNLGSV LSGGGYHAGL KHHQSTVTRS   120
TGEWVDR                                                             127
```

<212> Type : PRT
<211> Length : 127
SequenceName : SEQ ID 312
SequenceDescription :
Sequence --------
<213> OrganismName : Mycoplasma pneumoniae
<400> PreSequenceString :

```
MRDNSAKGIT AGSESQQTTY DPTRTEAALT ASTTFALRRY DLAGRALYDL DFSRLNPQTP    60
TRDQTGQITF NPFGGFGLSG AAPQQWNEVK NKVPVEVAQD PSNPYRFAVL LVPRSVVYYE   120
QLQRGLALPN QGSSSGSGQQ NTTIGAYGLK VKNAEADTAK SNEKLQGDES KSSNGSSSTS   180
TTTQRGGSSG DTKVKALQVA VKKKSGSQGN SGEQGTEQVE LESNDLANAP IKRGEESGQS   240
VQLKAADFGT TPSSSGSGGN SNPGSPTPWR PWLATEQIHK DLPKWSASIL ILYDAPYARN   300
RTAIDRVDHL DPKVMTANYP PSWRTPKWNH HGLWDWKARD VLLQTTGFFN SRRHPEWFDQ   360
GQAVADNTQT GFDTDDTDNK KTRLSKGSWL RQAGPDRPPV WSVLRQHWQP HLVRASAFGV   420
WDLFVLIN                                                            428
```

<212> Type : PRT
<211> Length : 428
SequenceName : SEQ ID 313
SequenceDescription :
Sequence
<213> OrganismName : Mycoplasma pneumoniae
<400> PreSequenceString :

```
MFGLKVKNAE ADTAKSNEKL QGAEATGSST TSGSGQSTQR GGSSGDTKVK ALQVAVKKKS    60
GSQGNSGDQG TEQVELESND LANAPIKRGS NPASPTQGSR LRHHPIQFGI WSIRHPHPLK   120
AVACDRANSQ GPPQMIRLDP HSVRCALCL                                     149
```

<212> Type : PRT
<211> Length : 149
SequenceName : SEQ ID 314
SequenceDescription :
Sequence
<213> OrganismName : Mycoplasma pneumoniae
<400> PreSequenceString :

```
MFGLKVKDAT VDSSKQSTES LKGEESSSSS TTSSTSTTQR GGSSGDTKVK ALQVAVKKKS    60
DSEDNGQIEL ETNNLANAPI KRGSNNNQQV QLKADDFGTS PSSSESGQSG TPTPWTPWLA   120
TEQIHKDLPK WSASILILYD APYARNRTAI DRVDHLDPKV MTANYPPSWR TPKWNHHGLW   180
DWKARDVLVQ TTGFFNPRRH PDWFDQGQAV AENTQTGFDT DDTDNKKQGF RKQGEQSPAP   240
IALPFEAYFA NIGNLTWFGQ ALLVFGICLS                                    270
```

<212> Type : PRT
<211> Length : 270
SequenceName : SEQ ID 315
SequenceDescription :
Sequence --------
<213> OrganismName : Mycoplasma pneumoniae
<400> PreSequenceString :

```
MGSQNQGSTT TTSAGNPDSL VTDKVDQKGQ VQTSGQNLSD TNYTNLSPNF TPTSDWPNAL    60
SFTNKNNAQR AQLFLHGLLG SIPVLVNKSG ENNEKFQATD QKWSYTELKS DQTKLNLPAY   120
GEVNGLLNPA LVETYFGTTR TSSTANQNST TVPGIGFKIP EQNNDSKAVL ITPGLAWTPQ   180
DVGNLVVSGT SFSFQLGGWL VSFTDFVKPR AGYLGLQLTG LDASDATQRA LIWAPPALSG   240
LSWQLGQPVG PRGECVGFEG GVGGSSSVRL ARIYHHRNRG YLTGAPECFG LSGECGGSEC   300
LQAKHELRPN PIH                                                      313
```

<212> Type : PRT
<211> Length : 313
SequenceName : SEQ ID 316
SequenceDescription :
Sequence
<213> OrganismName : Mycoplasma pneumoniae
<400> PreSequenceString :

```
MSFGLVGTVN NNGWKSPFRH ETKYRAGYDK FKYYKTHYRG AKKAGTNDDR WRWTAWFDLD    60
FAHQKIVLIE RGELHRQADL KKSDPATNET SKTVWGSIKE KLLQNVNNLH SEKGVFLWFR   120
```

QSGFTTTRN

QSGFTTTRN
<212> Type : PRT
<211> Length : 129
SequenceName : SEQ ID 317
SequenceDescription :
Sequence --------
<213> OrganismName : Mycobacterium tuberculosis H37Rv
<400> PreSequenceString :

```
MAEPLAVDPT GLSAAAAKLA GLVFPQPPAP IAVSGTDSVV AAINETMPSI ESLVSDGLPG    60
VKAALTRTAS NMNAAADVYA KTDQSLGTSL SQYAFGSSGE GLAGVASVGG QPSQATQLLS   120
TPVSQVTTQL GETAAELAPR VVATVPQLVQ LAPHAVQMSQ NASPIAQTIS QTAQQAAQSA   180
QGGSGPMPAQ LASAEKPATE QAEPVHEVTN DDQGDQGDVQ PAEVVAAARD EGAG[illegible]GQQ   240
PGGGVPAQAM DTGAGARPAA SPLAAPVDPS TPAPSTTTTL                         280
```

<212> Type : PRT
<211> Length : 280
SequenceName : SEQ ID 318
SequenceDescription :
Sequence --------
<213> OrganismName : Mycobacterium tuberculosis H37Rv
<400> PreSequenceString :

```
MRYLIATAVL VAVVLVGWPA AGAPPSCAGL GGTVQAGQIC HVHASGPKYM LDMTFPVDYP    60
DQQALTDYIT QNRDGFVNVA QGSPLRDQPY QMDATSEQHS SGQPPQATRS VVLKFFQDLG   120
GAHPSTWYKA FNYNLATSQP ITFDTLFVPG TTPLDSIYPI VQRELARQTG FGAAILPSTG   180
LDPAHYQNFA ITDDSLIFYF AQGELLPSFV GACQAQVPRS AIPPLAI                 227
```

<212> Type : PRT
<211> Length : 227
SequenceName : SEQ ID 319
SequenceDescription :
Sequence --------
<213> OrganismName : Mycobacterium tuberculosis H37Rv
<400> PreSequenceString :

MKMVKSIAAG LTAAAAIGAA AAGVTSIMAG GPVVYQMQPV VFGAPLPLDP ASAPDVPTAA 60
QLTSLLNSLA DPNVSFANKG SLVEGGIGGT EARIADHKLK KAAEHGDLPL SFSVTNIQPA 120
AAGSATADVS VSGPKLSSPV TQNVTFVNQG GWMLSRASAM ELLQAAGN 168

<212> Type : PRT
<211> Length : 168
SequenceName : SEQ ID 320
SequenceDescription :
Sequence --------
<213> OrganismName : Mycobacterium tuberculosis H37Rv
<400> PreSequenceString :

MTYSPGNPGY PQAQPAGSYG GVTPSFAHAD EGASKLPMYL NIAVAVLGLA AYFASFGPMF 60
TLSTELGGGD GAVSGDTGLP VGVALLAALL AGVALVPKAK SHVTVVAVLG VLGVFLMVSA 120
TFNKPSAYST GWALWVVLAF IVFQAVAAVL ALLVETGAIT APAPRPKFDP YGQYGRYGQY 180
GQYGVQPGGY YGQQGAQQAA GLQSPGPQQS PQPPGYGSQY GGYSSSPSQS GSGYTAQPPA 240
QPPAQSGSQQ SHQGPSTPPT GFPSFSPPPP VSAGTGSQAG SAPVNYSNPS GGEQSSSPGG 300
APV 303

<212> Type : PRT
<211> Length : 303
SequenceName : SEQ ID 321
SequenceDescription :
Sequence --------
<213> OrganismName : Mycobacterium tuberculosis H37Rv
<400> PreSequenceString :

MKCPGVSDCV ATVRHDNVFA IAAGLRWSAA VPPLHKGDAV TKLLVGAIAG GMLACAAILG 60
DGIASADTAL IVPGTAPSPY GPLRSLYHFN PAMQPQIGAN YYNPTATRHV VSYPGSFWPV 120
TGLNSPTVGS SVSAGTNNLD AAIRSTDGPI FVAGLSQGTL VLDREQARLA NDPTAPPPGQ 180

LTFIKAGDPN NLLWRAFRPG THVPIIDYTV PAPAESQYDT INIVGQYDIF SDPPNRPGNL 240
LADLNAIAAG GYYGHSATAF SDPARVAPRD [illegible]TNSLGA TTTTYFIRTD QLPLVRALVD 300
MAGLPPQAAG TVDAALRPII DRAYQPGPAP AVNPRDLVQG IRGIPAIAPA IAIPIGSTTG 360
ASAATSTAAA TAAATNALRG ANVGPGANKA LSMVRGLLPK GKKH 404

<212> Type : PRT
<211> Length : 404
SequenceName : SEQ ID 322
SequenceDescription :
Sequence --------
<213> OrganismName : Mycobacterium tuberculosis H37Rv
<400> PreSequenceString :

MS[illegible] LPPPFDAIPN PIEDLDVLVA [illegible] GV[illegible]AAQLGEI [illegible]Q 60
KAPHCPAESD QTPAGAAGDG DLPEVGGRVT SPPQPPVAAL TGYSANIGGL SVPHSWNLPP 120
AVRQVAAMFP GATPMYMTGS SDGSYAGLAA AGLAGTGLAG LAARGGSAPT PAAAAPAGAG 180
GAGPAATRPA AQQTPAVPAA AAGSAIPGLP PGLPPGVVAN LAATLAAIPG ATIIVVPPSP 240
NANQ 244

<212> Type : PRT
<211> Length : 244
SequenceName : SEQ ID 323
SequenceDescription :
Sequence --------
<213> OrganismName : Mycobacterium tuberculosis H37Rv

<400> PreSequenceString :

```
MDVALGVAVT DRVARLALVD SAAPGTVIDQ FVLDVAEHPV EVLTETVVGT DRSLAGENHR   60
LVATRLCWPD QAKADELQHA LQDSGVHDVA VISEAQAATA LVGAAHAGSA VLLVGDETAT  120
LSVVGDPDAP PTMVAVAPVA GADATSTVDT LMARLGDQAL APGDVFLVGR SAEHTTVLAD  180
QLRAASTMRV QTPDDPTFAL ARGAAMAAGA ATMAHPALVA DATTSLPRAE AGQSGSEGEQ  240
LAYSQASDYE LLPVDEYEEH DEYGAAADRS APLSRRSLLI GNAVVAFAVI GFASLAVAVA  300
VTIRPTAASK PVEGHQNAQP GKFMPLLPTQ QQAPVPPPPP DDPTAGFQGG TIPAVQNVVP  360
RPGTSPGVGG TPASPAPEAP AVPGVVPAPV PIPVPITIPP FPGWQPGMPT IPTAPPITPV  420
TTSATTPPTT PPTTPVTTPP TTPPTTPVTT PPTTPPTTPV TTPPTTVAPT TVAPTTVAPT  480
TVAPTTVAPA TATPTTVAPQ PTQQPTQQPT QQMPTQQQTV APQTVAPAPQ PPSGGRNGSG  540
GGDLFGGF                                                           548
```

<212> Type : PRT
<211> Length : 548
SequenceName : SEQ ID 324
SequenceDescription :
Sequence --------
<213> OrganismName : Mycobacterium tuberculosis H37Rv
<400> PreSequenceString :

```
MKNARTTLIA AAIAGTLVTT SPAGIANADD AGLDPNAAAG PDAVGFDPNL PPAPDAAPVD   60
TPPAPEDAGF DPNLPPPLAP DFLSPPAEEA PPVPVAYSVN WDAIAQCESG GNWSINTGNG  120
YYGGLRFTAG TWRANGGSGS AANASREEQI RVAENVLRSQ GIRAWPVCGR RG          172
```

<212> Type : PRT
<211> Length : 172
SequenceName : SEQ ID 325
SequenceDescription :
Sequence --------
<213> OrganismName : Mycobacterium tuberculosis H37Rv
<400> PreSequenceString :

```
MTRLIPGCTL VGLMLTLLPA PTSAAGSNTA TTLFPVDEVT QLETHTFLDC HPNGSCDFVA   60
GANLRTPDGP TGFPPGLWAR QTTEIRSTNR LAYLDAHATS QFERVMKAGG SDVITTVYFG  120
EGPPDKYQTT GVIDSTNWST GQPMTDVNVI VCTHMQVVYP GVNLTSPSTC AQANFS      176
```

<212> Type : PRT
<211> Length : 176
SequenceName : SEQ ID 326
SequenceDescription :
Sequence --------
<213> OrganismName : Mycobacterium tuberculosis H37Rv
<400> PreSequenceString :

```
MTPGLLTTAG AGRPRDRCAR IVCTVFIETA VVATMFVALL GLSTISSKAD DIDWDAIAQC   60
ESGGNWAANT GNGLYGGLQI SQATWDSNGG VGSPAAASPQ QQIEVADNIM KTQGPGAWPK  120
CSSCSQGDAP LGSLTHILTF LAAETGGCSG SRDD                              154
```

<212> Type : PRT
<211> Length : 154
SequenceName : SEQ ID 327
SequenceDescription :
Sequence --------
<213> OrganismName : Mycobacterium tuberculosis H37Rv
<400> PreSequenceString :

MMQQAVSGIT GALGGAVGGV MGPLTQLPQQ AMQAGQGAMQ PLMSALQQTY GAEGLDVADG 60
ARLVDSIEGE PGLGGEPGAG DVGAGGGGGG TTPTGYLGPP PVPTSSPPTT PAGAPAKSVT 120
PDPVSGTPRA SGPAGMTGMP MVPPGALGAG AEGANKDKPV EKRVTGCAEW STGQGPLNST 180
AECSGEICRR QAGGHQVDAT DPCCAERRQG 210

<212> Type : PRT
<211> Length : 210
SequenceName : SEQ ID 328
SequenceDescription :
Sequence --------
<213> OrganismName : Mycobacterium tuberculosis H37Rv
<400> PreSequenceString :

MIRELVTTAA ITGAAIGGAP VAGADPQRYD GDVPGMNYDA SLGAPCSSWE RFIFGRGPSG 60
QAEACHFPPP NQFPPAETGY WVISYPLYGV QQVGAPCPKP QAAAQSPDGL PMLCLGARGW 120
QPGWFTGAGF FPPEP 135

<212> Type : PRT
<211> Length : 135
SequenceName : SEQ ID 329
SequenceDescription :
Sequence --------
<213> OrganismName : Mycobacterium tuberculosis H37Rv
<400> PreSequenceString :

MKTTGTTIKL GIVWLVLSVF TVMIIVVFGQ VRFHHTTGYS AVFTHVSGLR AGQFVRAAGV 60
EVGKVAKVTL IDGDKQVLVD FTVDRSLSLD QATTASIRYL NLIGDRYLEL GRGHSGQRLA 120
PGATIPLEHT HPALDLDALL GGFRPLFQTL DPDKVNSIAS SIITVFQGQG ATINDILDQT 180
ASLTATLADR DHAIGEVVNN LNTVLATTVK HQTEFDRTVD KLEVLITGLK NRADPLAAAA 240
AHISSAAGTL ADLLGRIVHC CTAASGTSRA SSSRS 275

<212> Type : PRT
<211> Length : 275
SequenceName : SEQ ID 330
SequenceDescription :
Sequence --------
<213> OrganismName : Mycobacterium tuberculosis H37Rv
<400> PreSequenceString :

MTPRSLVRIV GVVVATTLAL VSAPAGGRAA HADPCSDIAV VFARGTHQAS GLGDVGEAFV 60
DSLTSQVGGR SIGVYAVNYP ASDDYRASAS NGSDDASAHI QRTVASCPNT RIVLGGYSQG 120
ATVIDLSTSA MPPAVADHVA AVALFGEPSS GFSSMLWGGG SLPTIGPLYS SKTINLCAPD 180
DPICTGGGNI MAHVSYVQSG MTSQAATFAA NRLDHAG 217

<212> Type : PRT
<211> Length : 217
SequenceName : SEQ ID 331
SequenceDescription :
Sequence --------
<213> OrganismName : Mycobacterium tuberculosis H37Rv
<400> PreSequenceString :

MISTTRIDFL WILSVAFASM IALATLLTLI NQVVGTPYIP GGDSPAGTDC SELASWVSNA 60
ATARPVFGDR FNTGNEEAAL AARGFQQGTA PNALVIGWNG HHTAVTLPDG TPVSSGEGGG 120
VRVGGGGAYQ PKFTHHMYLP MDVDAGEDQP PAPDEPVTAV DDVEPEMPAP CPTQRPPVTP 180
RHNLCNKLRT MPGALSAALA AAAPVWPAPI SGCRGFSTSL LAKRNHPVIV GK 232

<212> Type : PRT

<211> Length : 232
SequenceName : SEQ ID 332
SequenceDescription :
Sequence --------
<213> OrganismName : Mycobacterium tuberculosis H37Rv
<400> PreSequenceString :

```
MTTMITLRRR FAVAVAGVAT AAATTVTLAP APANAADVYG AIAYSGNGSW GRSWDYPTRA    60
AAEATAVKSC GYSDCKVLTS FTACGAVAAN DRAYQGGVGP TLAAAMKDAL TKLGGGYIDT   120
WACN                                                                124
```

<212> Type : PRT
<211> Length : 124
SequenceName : SEQ ID 333
SequenceDescription :
Sequence --------
<213> OrganismName : Mycobacterium tuberculosis H37Rv
<400> PreSequenceString :

```
MAGLNIYVRR WRTALHATVS ALIVAILGLA ITPVASAATA RATLSVTSTW QTGFIARFTI    60
TNSSTAPLTD WKLEFDLPAG ESVLHTWNST VARSGTHYVL SPANWNRIIA PGGSATGGLR   120
GGLTGSYSPP SSCLLNGQYP CT                                            142
```

<212> Type : PRT
<211> Length : 142
SequenceName : SEQ ID 334
SequenceDescription :
Sequence --------
<213> OrganismName : Mycobacterium tuberculosis H37Rv
<400> PreSequenceString :

```
MLTRAIKTQL VLLTVLAVIA VVVLGWYFLR IPSLVGIGRY TLYAELPRSG GLYRTANVTY    60
RGITIGKVTG VEPTERGARA TMSIDNGYQI PTDASANVHS VSAVGEQFVD LVSTRTSGPY   120
LRHGQTITTT TVPSQIGPAL DAANRGLAVL PKDRVASVLH EASEAVGGLG SSLNRLIEAT   180
QAIAHDVRGS LEDIDDIIER SAPIIDSQVN SGNEIARWAA NLNTLAAQTA QTDPAVRSIL   240
ANAAPTADQV NATFSDVRES LPQTLANLEV VIDMLKRYHN GVEQALVFLP QSGAIAQSVT   300
TEFPGQAGLG VGGLALNQPP PCLTGFLPAS EWRSPADTST APLPKGTYCR IPMDASNVVR   360
GARNNPCVDV PGKRAATPRE CRSNEAYVPG GTNPWYGDPN QMLSCPAPAA RCDQPVKPGQ   420
VIPAPSVNNG INPLPADQLP GTPPPVNDPL QRPGSGTVQC NGQQPNPCVY TPSTFPTTIY   480
DVQSGKVVAP DGVVYSVEAS THAGADGWKV MLAPTG                             516
```

<212> Type : PRT
<211> Length : 516
SequenceName : SEQ ID 335
SequenceDescription :
Sequence --------
<213> OrganismName : Rickettsia prowazekii
<400> PreSequenceString :

```
MLNNTQFLNL MKSYMKPEFY MSSIKNTTNL DLSSITNTIQ KAMNIFFTTN KISTESMQSL    60
FKKNSEIIQN NINTILNSTK EVINSKDFKQ ATEYHQKCVK SIYETSMDNA KELANIAYEA   120
SNKIFEAANK HITKNIHNAS NNIHNTAEQV QKNFNNKSA                          159
```

<212> Type : PRT
<211> Length : 159
SequenceName : SEQ ID 336
SequenceDescription :
Sequence
--------

<213> OrganismName : Rickettsia prowazekii
<400> PreSequenceString :

```
MNIKLVTYFL ILVSSLKVNA DLNHIQDSFK YQEAEQLTIE LPWNDCTAIH KFLEEKLFFS    60
EQQIKKENKI HEKYKQFYLQ HNNKLSDFSM QFLEKKSEIN SVETLISGFL KFCEDNFQTS   120
KSKSHSLNFF QKQQDQWLHN IRNENYKTYY KKKYEDNTFR NIN                     163
```

<212> Type : PRT
<211> Length : 163
SequenceName : SEQ ID 337
SequenceDescription :
Sequence --------
<213> OrganismName : Rickettsia prowazekii
<400> PreSequenceString :

```
MKKLLLIATA SATILSSSVS FAECIDNEWY LRADAGVAMF NKEQDKATGV KLKSNKAIPI    60
DLGIGYYISE NVRADLTLGT TIGGKLKKYG AATNTHFTGT NVSVSHKPTV TRLLINGYVD   120
LTSFDMFDVF VGGGVGPALV KEKISGVSGL ASNTKNKTNV SYKLIFGTSA QIADGVKVEL   180
AYSWINDGKT KTHNVMYKGA SVQTGGMRYQ SHNLTVGVRF GI                      222
```

<212> Type : PRT
<211> Length : 222
SequenceName : SEQ ID 338
SequenceDescription :
Sequence --------
<213> OrganismName : Rickettsia prowazekii
<400> PreSequenceString :

```
MKKNMRKQML KIISIIIISL LLSSCSESTR DENGLLTDSQ STIIRDYIIS QNSKNLKVNL    60
KEKFGSNLKG VKLIGIKLTN EDLSGIDFTS CEILRTDFMG SNLEKAILTN SVIQESNFAD   120
SVIKNISGYN ADFQGSIFNN ITLQNTNFVQ SNFSDTAFNK STIINVNFEN SKFSNVLWCH   180
SNIDSSNFQK THLKNNSFKN TNVMNSIFYG ADLGKSVINN TNFTNNYFES SDLSNTKFTS   240
VIIKDSNFTQ SIFNSVNFNN IQSNNSFFSY TSFEDSTLHN IHLTKCDLQN STINSSVFNN   300
FKIDNAILTN MSLNDNTFNN LSIKNSNTNF VRINKSKGFN ITLLNTNYSN AIFSNNDLKE   360
FKVINTDLNN SEIINSNFTN GQFNNVNFSQ SLIQNVNFTD VKITLGNLNQ VALINSNLIN   420
TNIINSVLSN SQINNINYQA YYSFINTNVS NNIVINDNSN QIPPNNIVIN SEKDLQNISN   480
LANMNLTNFN LSNLVFNGVD FSKSIFKKAN LTNTVIKNSI LKDANFSAAI LTKTDFSKSI   540
LTGSIFKFAQ IDQTCFSNSD LTNTDFTEAT IKNTAFDNAN THGIKGLE                588
```

<212> Type : PRT
<211> Length : 588
SequenceName : SEQ ID 339
SequenceDescription :
Sequence --------
<213> OrganismName : Porphyromonas gingivalis W83 <400> PreSequenceString :

MIQKFTNVKL NDMRKILSFL MMCSLHLGLQ SQTWHGDPDS VAALPSIGIQ ESSCTRITFE 60
VVFPGFYSVE KREGNQVFQR ISMPGCGSFG NLGEAELPVL KKMIAVPEFS TANVAVKIKE 120
TETFDNYNIY PNPTYVVEEL PEGGTYLVEA FAINNDYYSQ NVSLPSTHYV YSQDGYFRSQ 180
RFIEVTLYPF RYNPVRQEIL FAKKIEVTIT FDNPQPPLQK NTGIFNKVAS SAFINYEADG 240
KSAIENDMVF SRGTTTYISG NVASNLPQNC DYLVIYDDMF NVNQQPHDEI KRLCEHRAFY 300
NGFDVAAVSI KDVLNSFPSN ATSYINETKL KNFIRSVYNQ SNAKRTLDGK LGYVLLIGKP 360
LSKYLADTDN TKVPTSFIHN VSLIPSHPTF GSICASDYFF SCVSPLDTVG DLFIGRFSVT 420
NAHELHNLIE KTINKEISYN PIAHKNILYA EGKGCDAPIL RLFLKEIASG YTVNSILKSN 480
QVSAIDSIFD CLNNGSHHFY FNTHGMPTVW GIGQGLDVNT LTARLNNTSS QGLCTSLSCS 540
SAVADSTIRS LGEVLTTYAP NKGFSAFLGG SRATQYAVYL EGPCPPSEFY EYLPYSLYHN 600
LSTVVGEMLL SSIINTNSVD TYSKFNFNLL GDPALNIMAH GMEVSNCITL PNNTIISSPI 660
TIKNGGCLKI PEKGVLHFTN NGSIQVMSGG TLEIGNQAKI SGETGANPTF ITVYGDGLAI 720
NKQVEIDNID RLNLFSTHSV MPKFHFDSVK FNSAPLYTTN CIVEISNCEF TNRSDIISKN 780
CDLSVENSMF SSSGITVFKP MATSSITGLS TKAKITDNTF FATGNFAYHI TNTPGLTATS 840
NAAIKLDNIP EYYISGNKIV NCDEALVLNN SGNRTNRLHN ITRNVIKNCR IGSTLYNSYG 900
IYNRNKISNN HIGVRLLNNS CFYFDNAPVI NEEDKQTFIS NRTWQLYSSN GTFPLNFHYN 960
SLQGGDTDTW IYNDTYTNRY IDVSNNHWGN NDLFDPNQVF NTPDLFIWIP FWDGLPNGRS 1020
GNSSAEAVEF QTALDCIGNS DYLSAKVALK MMVETYPESD FAIAALKELF RIEKMSGNDY 1080
EGLKDYFRSN PTIISSQNLF PTADFLSARC DIVCENYQSA IDWYENRLNS EISYQDSVFA 1140
VIDLGDIYWN MQLDSLRGTG IDLNILSCEQ RKSLESHQNV KNYLLSTLPE STGTLLPPLE 1200

CNKSSLDKSK IISISPNPAK AVVTIIYYTD NPSCSVIKIY GINGASADIT GLPKHLSEGY 1260
YSIQFNTSNF DPGFYLVTLN VDQKIIDTEK LRIK 1294

<212> Type : PRT
<211> Length : 1294
SequenceName : SEQ ID 340
SequenceDescription :
Sequence
--------
<213> OrganismName : Shigella flexneri 2a str. 2457T <400> PreSequenceString :

MQGKNTIVTT GDYSIGLLSQ TSGNLNTDTI IRVNSDGSVT PSFSDGDDTF IVTAGNHAVG 60
VLACASPGSA CACVSSLDEE STADTGSNEN NAIAKLDMAK GEITTHGTES YAAYANGTVV 120
KAGDTLDYTN ASVTLTDVDI TTHGDNAHAI AARQGTVSFN QGEIYTTGPD AAIAKIYNGG 180
TVTLKNTSAV AHQGSGIVLE SSINGQEATV DILSGSSLRS ANEILYHKDE TSNVTITDSE 240
VSSAADVFIN NIKGHLTVDA TNSKITGSAN ISTDDNTHTY LSLSDNSTWD IKADSTVSNL 300
TVDNSTVYIS RADGRDVEPT RLTITENYVG NNGVLHLRTE LDDDNSATDK VVINGNTSGT 360
TRVKVTNAGG SGAYTLNGIE IISVEGESNG EFIKDSRIFA GAYEYSLTRG NTEATNKNWY 420
LTNFQATSGG ETNSGGSSAP TVAPTPVLRP EAGSYVANLA AANTLFVMRL NDRAGETRYI 480
DPVTEQERSS RLWLRQIGGH NAWRDSNGQL RTTSHRYVSQ LGGDLLTGGF TDSDSWRLGV 540
MAGYARDYNL THSSVSDYRS KGSVRGYSAG LYATWFADDI SKKGAYIDSW AQYSWFKNSV 600
KGDELAYESY SAKGATVSLE AGYGFALNKS FGLEAAKYTW IFQPQAQAIW MGVDHNAHTE 660
ANGSRIENDA NNNIQTRLGF RTFIRTQEKN SGPHGDDFEP FVEMNWIHNS KDFAVSMNGV 720
KVEQDGVSNL GEIKLGVNGN LNPAASVWGN VGVQLGDNGY NDTAVMVGLK YKF 773

<212> Type : PRT
<211> Length : 773
SequenceName : SEQ ID 341
SequenceDescription :
Sequence
--------
<213> OrganismName : Shigella flexneri 2a str. 2457T
<400> PreSequenceString :

MTKLKLLALG VLIATSAGVA HAEGKFSLGA GVGVVEHPYK DYDTDVYPVP VINYEGDNFW 60
FRGLGGGYYL WNDATDKLSI TAYWSPLYFK AKDSGDHQMR HLDDRKSTMM AGLSYAHFTQ 120
YGYLRTTLAG DTLDNSNGIV WDMAWLYRYT NGGLTVTPGI GVQWNSENQN EYYYGVSRKE 180
SARSGLRGYN PNDSWSPYLE LSASYNFLGD WSVYGTARYT RLSDEVTDSP MVDKSWTGLI 240
STGITYKF 248

<212> Type : PRT
<211> Length : 248

SequenceName : SEQ ID 342
SequenceDescription :
Sequence
--------
<213> OrganismName : Shigella flexneri 2a str. 2457T
<400> PreSequenceString :

```
MKKIALAGLA GMLLVSASVN AMSISGQAGK EYTNIGVGFG TESTGLALSG NWTHNDDDGD      60
VAGVGLGLNL PLGPLMATVG GKGVYTNPNY GDEGYAAAVG GGLQWKIGNS FRLFGEYYYS     120
PDSLSSGIQS YEEANAGARY TIMRPVSIEA GYRYLNLSGK DGNRDNAVAD GLYVGVNASF     180
```

<212> Type : PRT
<211> Length : 180
SequenceName : SEQ ID 343
SequenceDescription :
Sequence
--------
<213> OrganismName : Shigella flexneri 2a str. 2457T
<400> PreSequenceString :

```
MTTLTARVFT TAEIIYRKTV IALVCHLNCS RQETVTMNKT IMALAIMMAS FAANASVLPE      60
TPVPFKSGTG AIDNDTVYIG LGSAGTAWYK LDTQAKDKKW TALAAFPGGP REQATSAFID     120
GNLYVFGGIG KNSEGLTQVF NDVHKYNPKT NSWVKLMSHA PMGMAGHVTF VHNGKAYVTG     180
GVNQNIFNGY FEDLNEAGKD STAIDKINAH YFDKKAEDYF FNKFLLSFDP STQQWSYAGE     240
SPWYGTAGAA VVNKGDKTWL INGEAKPGLR TDAVFELDFT GNNLKWNKLD PVSSPDGVAG     300
GFAGISNDSL IFAGGAGFKG SRENYQNGKN YAHEGLKKSY STDIHLWHNG KWDKSGELSQ     360

GRAYGVSLPW NNSLLIIGGE TAGGKAVTDS VLISVKDNKV TVQN                     404
```

<212> Type : PRT
<211> Length : 404
SequenceName : SEQ ID 344
SequenceDescription :
Sequence
--------
<213> OrganismName : Shigella flexneri 2a str. 2457T
<400> PreSequenceString :

```
MATGGAALAG KAVMGAAAGA AGGASALQAA FQKASASMET GGDMSSMGSV VSSGGNGGGE      60
AGTAGSSPFA QAAGFGDSGS SSSGGGFAKA AKLATGTASE LAKGVGSQVK QGFQERVSET     120
TGGKLAASIR ESMEPKEASQ SGQFEGNSLG ADSGPDSNEV RS                       162
```

<212> Type : PRT
<211> Length : 162
SequenceName : SEQ ID 345
SequenceDescription :
Sequence
--------
<213> OrganismName : Shigella flexneri 2a str. 2457T
<400> PreSequenceString :

MKRVLIPGVI LCGADVAQAV DDKNMYMYFF EEMTVYAPVP VPVNGNTHYT SESIERLPTG 60
NGNISDLLRT NPAVRMDSTQ STSLNQGDIR PEKISIHGAS PYQNAYLIDG ISATNNLNPA 120
NESDASSATN ISGMSQGYYL DVSLLDNVTL YDSFVPVEFG RFNGGVIDAK IKRFNADDSK 180
VKLGYRTTRL DWLTSHIDEN NKSAFNQGSS GSTYFSPDFK KNFYTLSFNQ ELADNFGVTA 240
GLSRRQSDIT RADYVSNDGI VAGRAQYKNV IDTALSKFTW FASDRFTHDL TLKYTGSSRD 300
YNTSTFPQSD REMGNKSYGL AWDMDTQLAW AKLRTTVGWD HISDYTRHDH DIWYTELSCT 360
YGDITGRCTR GGLGHISQAV DNYTFKTRLD WQKFAVGDVS HQPYFGAEYI YSDAWTERHN 420
QSESYVINAA GKKTNHTIYH KGKGSLGIDN YTLYMADHIS WRNVSLMPGV RYDYDNYLSN 480
HNISPRFMTE WDIFADQTSM ITAGYNRYYG GNILDMGLRD IRNSWTESVS GNKTLTRYQN 540
LKTPYNDELA MGLQQKIDKN VIARASEAHD QISKSSRTDS ATKTTITEYN NDGKTKTHSF 600
NLSFELAEPL HIRQVDINPQ IVFSYIKSKG NLSLNNGYEE SNTGDNQVVY NGNLVSYDSV 660
PVADFNNPLK ISLNMDFTHQ PSGLVWANTL AWQEARKARI ILGKTNAQYI SEYSDYKQYV 720
DEKLDSSLTW DTRLSWTPQF LKQQNLTISA DILNVLDSKT AVDTTNTGVA TYASGRTFWL 780
DVSMKF 786

<212> Type : PRT
<211> Length : 786
SequenceName : SEQ ID 346
SequenceDescription :
Sequence
--------
<213> OrganismName : Shigella flexneri 2a str. 2457T
<400> PreSequenceString :

MKKTLLAIML AGTAFASQAG TLVSQGTEAS ANLTLTKPIV VNNTIQPVKG VYSGTLTAWT 60
PLATGIVGAS DGQSHDYAVT FPDDIYAESS TSADAVISGD NNPDHKLKVS LTTLEQDPPS 120
AASEEIGGKR YMMLKNTGTG GAYRVVSHMK EQVVEPDSYT IRTQAYIYAE 170

<212> Type : PRT
<211> Length : 170
SequenceName : SEQ ID 347
SequenceDescription :
Sequence
--------
<213> OrganismName : Shigella flexneri 2a str. 2457T
<400> PreSequenceString :

MGIYHWSRKT KMKRTKSIRH ASFRKNWSAR HLTPVALAVA TVFMLAGCEK SDETVSLYQN 60
ADDCSAANPG KSAECTTAYN NALKEAERTA PKYATREDCV AEFGEGQCQQ APAQAGMAPE 120
NQAQAQQSSG SFWMPLMAGY MMGRLMGGGA GFAQQPLFSS KNPASPAYGK YTDATGKNYG 180
AAQPGRTMTV PKTAMAPKPA TTTTVTRGGF GESVAKQSTM QRSATGTSSR SMGG 234

<212> Type : PRT
<211> Length : 234
SequenceName : SEQ ID 348
SequenceDescription :
Sequence
--------
<213> OrganismName : Shigella flexneri 2a str. 2457T
<400> PreSequenceString :

MTKMSRYALI TALAMFLAGC VGQREPAPVE EVKPAPEQPA EPQQPVPTVP SVPTIPQQPG 60
PIEHEDRTAP PAPHIRHYDW NGAMQPMVSK MLGADGVTAG SVLLVDSVNN RTNGSLNAAE 120
ATETLRNALA NNGKFTLVSA QQLSMAKQQL GLSPQDSLGT RSKAIGIARN VGAHYVLYSC 180
ASGNVNAPTL QMQLMLVQTG EIIWSGKGAV SQQ 213

<212> Type : PRT
<211> Length : 213
SequenceName : SEQ ID 349
SequenceDescription :

Sequence
--------
<213> OrganismName : Shigella flexneri 2a str. 2457T
<400> PreSequenceString :

```
MTKLMQFVQR CYYMTNKKMY FILILVFTLL QVCFFALWKA RDGSTTSLEC TSTLTRNAKT    60
DHSLYYSANL SVILKKDGSG SFTIVGLTDE DTPRKFSHSY FFTYKIDSNG RISGNAKAKV   120
SGLENQIKDE NFRLNFLDAS LTGKGNARLS KFNNVYIFSI PGLIINTCAP I            171
```

<212> Type : PRT
<211> Length : 171
SequenceName : SEQ ID 350
SequenceDescription :
Sequence
--------
<213> OrganismName : Shigella flexneri 2a str. 2457T
<400> PreSequenceString :

```
MGRISSGGMM FKAITTVAAL VIATSAMAQD DLTISSLAKG ETTKAAFNQM VQGHKLPAWV    60
MKGGTYTPAQ TVTLGDETYQ VMSACKPHDC GSQRIAVMWS EKSNQMTGLF SAIDEKTSQE   120
KLTWLNVNDA LSIDGKTVLF AALTGSLENH PDGFNFK                            157
```

<212> Type : PRT
<211> Length : 157
SequenceName : SEQ ID 351
SequenceDescription :
Sequence
--------
<213> OrganismName : Streptococcus mutans UA159
<400> PreSequenceString :

```
MKKQFLEKAV FTVAATAATV VLGNKMADAD TYTLQEGDSF FSVAQRYHMD AYELASMNGK    60
DITSLILPGQ TLTVNGSAAP DNQAAAPTDT TQATTETNDA NANTYPVGQC TWGVKAVATW   120
AGDWWGNGGD WASSASAQGY TVGNTPAVGS IMCWTDGGYG HVAYVTAVGE DGKVQVLESN   180
YKDQQWVDNY RGWFDPNNSG TPGSVSYIYP N                                  211
```

<212> Type : PRT
<211> Length : 211
SequenceName : SEQ ID 352
SequenceDescription :
Sequence
--------
<213> OrganismName : Streptococcus mutans UA159
<400> PreSequenceString :

```
MSIKNILENK TTTIKVSFAG IATAASLILP MAVQAETTYT VKSGDTLSEI ASTHGTTVDK    60
LAKLNKINNI HLIHAGQILE LDAATEDTDA TPVQESQINE AETSASAKTS QTSEVTTTAP   120
VQESQTSEVI TSAPAETSQT SEVPTEANQT NEVSSAVSVE TSQTSEATTS APVETSQTSE   180
ATTAEPTETK TSQTNEVAAS AEENQTTSNT SGLSTSDAAA KEFIAQKESG GNYNAKNGQY   240
YGRYQLSDSY LNGDLSEENQ ERVADAYVSS RYGSWTAAQA FWNANGWY                288
```

<212> Type : PRT
<211> Length : 288
SequenceName : SEQ ID 353
SequenceDescription :
Sequence
--------
<213> OrganismName : Streptococcus mutans UA159

<400> PreSequenceString :

```
MKCQAFEDFK ATSLNKLSYT TGGATDGEII ANRMLQGKAT KGEITMYTWN IIQNGWVNSL      60
VSWGIGGYNS SIGYSAQGNR GFSNYPYDVS MDSDNSSSSS NTTGGYVNYN QSFNSGW        117
```

<212> Type : PRT
<211> Length : 117
SequenceName : SEQ ID 354
SequenceDescription :
Sequence
--------
<213> OrganismName : Streptococcus mutans UA159
<400> PreSequenceString :

```
MRYSQICRKS LALLATGMIL TTSTLPSISI LAEDSTGAPA RPDGQAPAGG GANTTTYDYS      60
GINSGVLVAN GSKVTSSSKT KSTTSAQNTA LVQNGGSLTL HKANLIKSGD DNNGDNDNFY     120
GINSILLAVN ERSKAYVSNS KLKASSSGSN GIFATDKATI YANKTSIATT ADNSRGLDAT     180
YNGNIIANKM AISTKGAHSA AIATDRGGGN ISTTNSSLNT SGSGSPLLYS TGNIQVNHVT     240
GTSSNSQIAG MEGLNTILIH NSNLISTMTN KTASDPIANG VIIYQSQSGD AEATTGQSAH     300
FELSKSKLTS SITSGSMFYL TNTSANIILN QSTLNFDANK AKLLTVAGNS ANNWGTPGSN     360
GATVNFTGHK QTLKGDVDVD SISTLNMYLL DKTNYTGKTA VSTNSTNISP STSPITMNIS     420
KNSKWVLTGH STVTNLNAEK GAKIVDKDGK TVSVISSSGQ KLVKGKSKYS LTVTGTYSQK     480
VTTSSSNKPS SSYINRSDFD NYFKTTTAFV NNTKNTSN                             518
```

<212> Type : PRT
<211> Length : 518
SequenceName : SEQ ID 355
SequenceDescription :
Sequence
--------
<213> OrganismName : Streptococcus mutans UA159
<400> PreSequenceString :

```
MNKIGDTLRD ARIEKKLSFD DVVDKTGIAP HYILAMELDQ LKLLPEGKTN EYLEKYAHAV      60
GLDPVSIIHG YRNQEMSDEL ILPSSAELAA SSDSNIEKKN EGKSIEEPQE LAIDSLDVTQ     120
NITEETPQIE DFKVESEEAS KKIEKIPSRL SKYDYDEEPK KKFPWALILL ILLALTIISY     180
VGYVVYNQLQ TDSNKTELST STKKSKDTKN DANSTTQSQT SITTDFADGG NNITLSNTNG     240
KVEVTFTLTG DEESWVSATN TTDGESGTTL TATDKTYTVT LAEGSTTSML TVGSPSGVEI     300
TINGQKVDTT NLVNAGLTNI NLTVQ                                           325
```

<212> Type : PRT
<211> Length : 325
SequenceName : SEQ ID 356
SequenceDescription :
Sequence
--------
<213> OrganismName : Streptococcus mutans UA159
<400> PreSequenceString :

```
MKSRKRQRKG LVRKNEIIIL TLFVASAVSL LAFTNSFGVL AKSLHLEKIN KSITISLPFG      60
KKKMEQTARY YSGEQVQISS SAKKDSLGKG LSHYQNWIGT VKKIKSQKDS RQKHHYSYEV     120
TFDNGKALKY VQEKDLVKTK RSKYSKGQIV KLKSSATADL DGSSLTDYRA SAGKIDHISY     180
NHSNTTGGYK YDITFDEGGK VTNIQEKDLD KVYEVQLKSE NTAAQNNEIL KQAFAYAKQH     240
SGTILSLPNG EFKIGSQTPD KDYITLTSDT EIRGDNTTLL VEGSAYWFAF ATGTSASDGV     300
KNFTMRNINI KASDLEKGNQ FMIMADHGDN WKICNNSFTM VHKKGSHIFD LGSLQNSAFE     360
GNQFTGYAPE LTNVSKIDDN ADLHDFYSEV IQLDAAESSG VWDGGLIKAI DPNYENYNKE     420
KQLCNNITIA NNSFVPYIDS HGKIIAYSGT IGQHSSDVGL VKIYDNVFSN SLVSRFNQNG     480
KSEAWIFKAI HLKSNYNNAV YANSIS                                          506
```

<212> Type : PRT

<211> Length : 506
SequenceName : SEQ ID 357
SequenceDescription :
Sequence
--------
<213> OrganismName : Streptococcus mutans UA159
<400> PreSequenceString :

```
MRKLKVALFA SSILGMLAVS SYTAADTEDN QVTISHYNEQ AGTFDVNAVQ AANGKTIQSI    60
DVAIWSEENG QDDLKWYHAS NDGSNQLTVH FNAENHGSKV GSYIAHAYIT YTDGNRVGVN   120

LGKRKLSLSA PQLSLKQGGL QLFSKLKPSA ADQLFSAVWS DENGQDDLHW YTADADGNTL   180
AGYANHKGYG TYHVHTYLKQ NGKMIPISAQ DIDIPKPKVK IQIDKINDTS YDVVVNNVPP   240
YISSVAIPVW SEQNGQDDLK WYQATKVADG IFKTTVYLKT HRFELGNYQA HIYGDSQLSK   300
KLDGLGETHF NVPSIINYED PQVTIDHYNI NKGTFDVTVA ETDNSKAIQS ISAAVWSDAN   360
QANLYWYEAK QLANGKAAIT VDVQKHGNQT GSYNVHVYVH YNDGTTSGHV LANQQLNQIV   420
HYQPSAVRIT AYMNEKNTYP VGQCTWGVKE LAPWIPNWLG NGGQWASTVA VKGFKIGTVP   480
KVGAIACWSD GGYGHVAYVT HVESNNRIQV KEANYKNQQY ISNFRGWFDP TTSYLGRLTY   540
IYPD                                                               544
```

<212> Type : PRT
<211> Length : 544
SequenceName : SEQ ID 358
SequenceDescription :
Sequence
--------
<213> OrganismName : Streptococcus mutans UA159
<400> PreSequenceString :

```
MANNYSRRQQ PTKKTKGTSR KRPTEHIKTG FSALQKSVAI IAGILGIITA LITINNYRNS    60
SHNDKKDSTS KTTIIKEKEV DDSNSNNNAA NSQAENDSNN NNNSAESNQN QTATTANDSN   120
SNSANQNQAN SQSQANNQQN QNNANAGQ                                     148
```

<212> Type : PRT
<211> Length : 148
SequenceName : SEQ ID 359
SequenceDescription :
Sequence
--------
<213> OrganismName : Streptococcus mutans UA159
<400> PreSequenceString :

```
MKIFSFGTIR NNTALKPNYD DTTAFSGFGT IRNNTALKQS TNCASWFNRF GTIRNNTALK    60
LTILINGVSF CFGTIRNNTA LKPRGPIFVS TFRNRAIHLS QISASK                 106
```

<212> Type : PRT
<211> Length : 106
SequenceName : SEQ ID 360
SequenceDescription :
Sequence
--------
<213> OrganismName : Streptococcus mutans UA159
<400> PreSequenceString :

```
MKRKRNLYFL IGLFLTVFLL IGCSMQKKTK SESSSTSQKT TLQTKQSSEK STDAKQTTEA    60
HSESSQSSSH SNNEETLAPI DTGAVLKADY SSMAGTWKNE EGQTLTFDQR GLTTPGMTVS   120
LLNIDQDGNL LLNVETGTKK NLTLYIVPAN KTLSNQYFSN GQSDESDKTK DRIVSSESLN   180
SGKFTNRVYY HVSTH                                                   195
```

<212> Type : PRT
<211> Length : 195
SequenceName : SEQ ID 361
SequenceDescription :
Sequence
--------
<213> OrganismName : Streptococcus mutans UA159
<400> PreSequenceString :

MTPKKIKIAL TALISLMLAL FLFLFNHHSV RENSQQEKLK ISKASSKKSQ TSTSSVMTSS 60
RKATEQTSQA QTQSQSQAEQ SNPNVILPIP QELVGTYKGS SPQASEITFT ISSNGQLRAQ 120
ANFDPASDIN DVTATVSGVR KVGADTYIWE FVSGSSAALL PGVTGIGGLG KMQPGFILKG 180
GQLTPIMFTG SVDGEIDYSH PNPYPVSLNK Q 211

<212> Type : PRT
<211> Length : 211
SequenceName : SEQ ID 362
SequenceDescription :
Sequence
--------
<213> OrganismName : Streptococcus mutans UA159
<400> PreSequenceString :

MKKIINVIVL SLSVFFLIAC SNSSTGEKTS QSSEETKVRL IVKTDSNKTD EKVAFKKGAT 60

VMDVLKDNYK VKESGGFITT IDGVTQDKKA GRYWMFDVND KLASKAADKI KVKNGDKIEF 120
YLKVYKGKN 129

<212> Type : PRT
<211> Length : 129
SequenceName : SEQ ID 363
SequenceDescription :
Sequence
--------
<213> OrganismName : Streptococcus mutans UA159
<400> PreSequenceString :

MSNKPWEEKV TDATTDNEEM TRNSKDASII STPILTILLS LFFLIIIGIL FFVLYTSNGG 60
SNEKAATSGF YSSSKTVKKA KNEANSQTDE QTTEAETSSS ETTSSSSDSD GETITVQGGE 120
GAAAIAARAG ISVDKLYELN PEHMTHGYWY ANPGDNIKIK 160

<212> Type : PRT
<211> Length : 160
SequenceName : SEQ ID 364
SequenceDescription :
Sequence
--------
<213> OrganismName : Streptococcus mutans UA159
<400> PreSequenceString :

MPDNRMNYSI DSNMQFPLVE ITLETGEFAY IQRGSMVYHT PSVTLNTKVN GRGSGLGKLV 60
GAIGRSVTSG ESFFITQAVS NASDGKLALA PSMPGQVIAL ELGEKQYRLN DGAFLALDGS 120
AQYQMKAQSV GRALFGGQGG LFVMTTEGQG TLLANSFGSI KKIELQNQEI TIDNAHVVAW 180
SRDLNYDIHL ENGFMQSIGT GEGVVNTFRG TGEIYVQSLN LQQFAGVLQG FITNTNR 237

<212> Type : PRT
<211> Length : 237

SequenceName : SEQ ID 365
SequenceDescription :
Sequence
--------
<213> OrganismName : Streptococcus mutans UA159
<400> PreSequenceString :

```
MKKNYFWYGL LGLLALYLIT IAFIPGFHIF FSNMLMLALF FMLIALSNRS IFFFFLALGF      60
LSIYLKDIFH FDYSTGPLFT GIIIIGVILN SFLKPHYSYS YKGNHYFNMK QHANYIDNET     120
DVFLKTLFSE NTSYVTSQEL NKIIIDTKFG EQSVDLSQAQ FMTDSPEIHI DVSFGETNLR     180
IPNNWKIINK THSPFASISF SGFPSTNGDF INVTLTGTVA MGSLNIQY                  228
```

<212> Type : PRT
<211> Length : 228
SequenceName : SEQ ID 366
SequenceDescription :
Sequence
--------
<213> OrganismName : Streptococcus pneumoniae R6 <400> PreSequenceString :

```
MKSITKKIKA TLAGVAALFA VFAPSFVSAQ ESSTYTVKEG DTLSEIAETH NTTVEKLAEN      60
NHIDNIHLIY VDQELVIDGP VAPVATPAPA TYAAPAAQDE TVSAPVAETP VVSETVVSTV     120
SGSEAEAKEW IAQKESGGSY TATNGRYIGR YGSWTAAKNF WLNNGWY                   167
```

<212> Type : PRT
<211> Length : 167
SequenceName : SEQ ID 367
SequenceDescription :
Sequence
--------
<213> OrganismName : Streptococcus pneumoniae R6
<400> PreSequenceString :

```
MKHSHKKSFD WYSMQQRYSI RKYYFGAASV LLGTALVLGA AASVQTVQAE ENKQETTNSI      60
SVGRGEAATK PAEVSASNKE KTYAAPTVAN PVETTPVKTE EVTKPAEKVE EAKDKKEEVT     120
HQDAVDKSKL LTALSRAKKL ESKLYTEASA ANLQTSIQAG QSLLGKADAT EAELSAAESS     180
IQSFIIGLEL RSNSNKETVS ETPVAKKADA VESKEGAKPA ATTERSAVDS AILPTSTADK     240
VETTSAPASI NEILKLGLSL SDARQNPAIR KEDVNRGYSG FRAASNPANP IVSGSGNTVA     300
```

FADISQGGRS YSFRGYGNSR GGNSIHYDVT TVRSGNSVNF TISYSAPGDS REFVNNNFIL 360
DKGDGFGNPS NATITSSNPR VREQSKSISQ GANYVSHSGY SMTSAISTNT EQTIRFSLPI 420
INLNGDLSVR LKPVTFNVDQ GGGGAATSND PYSNSNYYYR ANPLYLDANP YGGTNNKTVS 480
EDIDFQTVYL PTSKLPEGQT RLVREGEKGQ RQITYKVHRF GNETLLGLPI SNSVTKEAKP 540
RIMQIGVAKD LIDTVKPRVD QNKVGDTNNL TFYLDNDGNG VYTEGVDELV QKIAIKDGAK 600
GEKGDQGERG LTGAKGEKGD RGERGLTGAQ GAKGEKGDRG ERGLTGAQGA KGEKGDRGER 660
GLTGAQGAKG EKGDRGERGL TGAQGAKGEK GAQGERGLTG AQGAKGEKGD QGERGLTGAQ 720
GAKGEKGDQG ERGLTGAQGA KGEKGAQGER GLTGTQGAKG EKGDRGERGL TGAQGAKGEK 780
GDRGERGLTG AQGAKGEKGA QGERGLTGAQ GAKGEKGDQG ERGLTGAQGE KGDRGERGLT 840
GAKGEKGDQG ERGITGAKGE KGAQGERGLT GAQGAKGEKG DQGERGLTGA QGEKGAQGQA 900
GRDGVTPTVT VKDNKNDGTH TITINDGRGN VTSTVVRDGF DGASPLVATQ RNDADKTTTV 960
IFYYDKNGNN ELDASDKKLK EVVIADGAKG EKGDKGEQGL QGRDGEQGPK GEDGKTPTVK 1020
VTDGQDGTHT ITINDGKGGI TTTVVRDGFD GASPLVSTHR NEADKTTTVI FYYDLNDNNQ 1080
FDEGDTKLKE VVIADGKQGP KGDKGDNGKD GFTPEVTVTD NNNGTHTITI TQPDNRPSLT 1140
TIVKNGEDGK TPKVKAERDD AKKQTTLTFY IDKDGDGSYT AGKDELVQTT VVKDGQDGAA 1200
GASGRDGKEV LNGKVDPTTE GKDGDTFVNT QTGDVFVKKG NTWEPAGNIK GPKGDKGADG 1260
AKGEKGAQGE RGLTGAQGVK GEKGDQGERG LTGSKGEKGD QGERGLTGAQ GAKGDKGEQG 1320
LQGRDGAQGP KGADGQRGPA GPQGPKGEQG NPGTPGKDGK SLIAVKNGVL VTITPVEGRP 1380
QTTFVEDGQK GADGKTPTVT ITEGQNGTHT LTVHNPGSPD VTTTIRDGAT GQAGRDGKDV 1440
LNGKVNPQPN QGKNGDKYIN IETGDVYVKN NGNWDKEGNI KGPKGDKGAD GAKGEKGDQG 1500
ERGLTGAQGA KGADGAVGRD GRDGKDVLNG KANPEAHQGK DGDKYVNTET GDVFVKNNGN 1560
WDKEGNIKGP KGDKGADGAK GEKGDRGERG LTGAQGAKGA DGAAGRDGRD GRDGKDVLNG 1620
KVNPEANQGK DGDKYVNTET GDVFVKNNGN WDKEGNIKGS KGDKGERGED GKTPEVTVTP 1680
GKDGHSTDIT FTVPGKDPVT VNVKDGENGL NGKTPKVDLL RVQGKNGNPS HTIVTFYTDE 1740
NNDGKYTPGT DELLGSEMIK DGAKGADGRD GKSLLTVKDG KETKVYQEDP ANPGQPLNPE 1800
KPLAVIRDGV DGKSPTVTAV RKDEAGHKGV EITVDNHDGS QPTTVFVQDG AKGKTGATGQ 1860
DGQTPTITTQ RGQDGQSTVV TITTSGKDPV TFTVKDGKNG KDGRAPKIKV EDITSPSRIR 1920
RDTDAAATPT RNGIRVTVYD DVNDNGVYDE GVDKVLNSKD IYNGIDGRDG SAPTITTKDN 1980
GDGTHTITVQ NPDGSESTTV VKDGKDGKTA NITTTENPDG SHTITVTNPD GSTKETVVKN 2040
GKDGKTPKVE VTDNNDGTHT VKVTDGDGNV TNAIIKDGKD GKAATATTTE NPDGSHTVTI 2100
TNPDGTKNEF VVKNGRDGVD GRTPTASVRD NGDGSHTIVI TNPEGVTTET TVRDGKSPKV 2160
TITDEQNGTH KISVLNGDGT TTETIIKDGK SPVATVRDNQ DGTYTIRVEN GNGTVSETTV 2220
RDGKSPTAKV VDNGDGTHTI TVVNSDGITT TTTVRDGREP KLEVIDNNDG SHTIKVTGAD 2280
GKGTTTTIFD GKSPKANIVD NGDGTHTLTI VDSDGREYKS IIKDGKDGKD SVSPTVTVKN 2340
NNDGTHVVTI TNPDGSKTEM VIKDGKDGKS PKVSVEDNGD GSHTITIINS DGTVTKTVIK 2400
DGKDGRDGRD GRDGKDGKDG KCGCQDKPVT PSNDKPVPPT PNVPTPEVPV KPVPAQPTPN 2460
VPTPEVPVQP TPAVSTPEVP VKPVPAVPEQ PVVPTPAQPA TPVNANPVAP TTGKENRGDK 2520
LPETGSQSDY ISVLLGSGIL LSLYVGRRKE D 2551

<212> Type : PRT
<211> Length : 2551
SequenceName : SEQ ID 368
SequenceDescription :
Sequence
--------
<213> OrganismName : Streptococcus pneumoniae R6
<400> PreSequenceString :

MKKRMLLAST VALSFAPVLA TQAEEVLWTA RSVEQIQNDL TKTDNKTSYT VQYGDTLSTI 60
AEALGVDVTV LANLNKITNM DLIFPETVLT TTVNEAEEVT EVEIQTPQAD SSEEVTTATA 120
DLTTNQVTVD DQTVQVADLS QPIAEAPKEV ASSSEVTKTV IASEEVAPST GTSVPEEQTA 180
ETSSAVAEEA PQETTPAEKQ ETQTSPQAAS AVEATTTSSE AKEVASSNGA TAAVSTYQPE 240
ETKIISTTYE APAAPDYAGL AVAKSENAGL QPQTAAFKEE IANLFGITSF SGYRPGDSGD 300
HGKGLAIDFM VPERSELGDK IAEYAIQNMA SRGISYIIWK QRFYAPFDSK YGPANTWNPM 360
PDRGSVTENH YDHVHVSMNG 380

<212> Type : PRT
<211> Length : 380
SequenceName : SEQ ID 369
SequenceDescription :
Sequence
--------
<213> OrganismName : Streptococcus pneumoniae R6
<400> PreSequenceString :

MTILGKDTVQ QSAKGESVTQ EATPEYKLEN TPGGDKGGNT GSSDANANEG GGSQAGGSAH 60
TGSQNSAQSQ ASKQLATEKE SAKNAIEKAA KNKQDEIKGA PLSDKEKAEL LARVEAEKQA 120
ALKEIENAKT MEDVKEAETI GVQAIAMVTV PKRPVAPNAA PKTTSAPQAT AGTMQDVTYQ 180
SPAGKQLPNT GSASSAALAS LGLVVATSGF ALLGRKTRRR K 221

<212> Type : PRT
<211> Length : 221
SequenceName : SEQ ID 370
SequenceDescription :
Sequence
--------
<213> OrganismName : Streptococcus pneumoniae R6
<400> PreSequenceString :

MMTTGCSMGA YHALNFFLQH PDVFTKVIAL SGVYDARFFV GDYYNDDAIY QNSPVDYIWN 60
QNDGWFIDRY RQAEIVLCTG LGAWEQDGLP SFYKLKEAFD KKQIPAWFAE WGHDVAHDWE 120
WWRKQMPYFL GNLYL 135

<212> Type : PRT
<211> Length : 135
SequenceName : SEQ ID 371
SequenceDescription :
Sequence
--------
<213> OrganismName : Streptococcus pneumoniae R6
<400> PreSequenceString :

MNKGLFEKRC KYSIRKFSLG VASVMIGATF FGTSPVLADS VQSGSTANLP ADLATALATA 60
KENDGHDFEA PKVGEDQGSP EVTDGPKTEE ELLALEKEKP AEEKPKEDKP AAAKPETPKT 120
VTPEWQTVEK KEQQGTVTIR EEKGVRYNQL SSTAQNDNAG KPALFEKKGL TVDANGNATV 180
DLTFKDDSEK GKSRFGVFLK FKDTKNNVFV GYDKDGWFWE YKSPTTSTWY RGSRVAAPET 240
GSTNRLSITL KSDGQLNASN NDVNLFDTVT LPAAVNDHLK NEKKILLKAG SYDDERTVVS 300
VKTDNQEGVK TEDTPAEKET GPEVDDSKVT YDTIQSKVLK AVIDQAFPRV KEYSLNGHTL 360
PGQVQQFNQV FINNHRITPE VTYKKINETT AEYLMKLRDD AHLINAEMTV RLQVVDNQLH 420
FDVTKIVNHN QVTPGQKIDD ERKLLSSISF LGNALVSVSS DQTGAKFDGA TMSNNTHVSG 480
DDHIDVTNPM KDLAKGYMYG FVSTDKLAAG VWSNSQNSYG GGSNDWTRLT AYKETVGNAN 540
YVGIHSSEWQ WEKAYKGIVF PEYTKELPSA KVVITEDANA DKKVDWQDGA IAYRSIMNNP 600
QGWKKVKDIT AYRIAMNFGS QAQNPFLMTL DGIKKINLHT DGLGQGVLLK GYGSEGHDSG 660
HLNYADIGKR IGGVEDFKTL IEKAKKYGAH LGIHVNASET YPESKYFNEK ILRKNPDGSY 720
SYGWNWLDQG INIDAAYDLA HGRLARWEDL KKKLGDGLDF IYVDVWGNGQ SGDNGAWATH 780
VLAKEINKQG WRFAIEWGHG GEYDSTFHHW AADLTYGGYT NKGINSAITR FIRNHQKDAW 840
VGDYRSYGGA ANYPLLGGYS MKDFEGWQGR SDYNGYVTNL FAHDVMTKYF QHFTVSKWEN 900
GTPVTMTDNG STYKWTPEMR VELVDADNNK VVVTRKSNDV NSPQYRERTV TLNGRVIQDG 960
SAYLTPWNWD ANGKKLSTDK EKMYYFNTQA GATTWTLPSD WAKSKVYLYK LTDQGKTEEQ 1020
ELTVKDGKIT LDLLANQPYV LYRSKQTNPE MSWSEGMHIY DQGFNSGTLK HWTISGDASK 1080
AEIVKSQGAN DMLRIQGNKE KVSLTQKLTG LKPNTKYAVY VGVDNRSNAK ASITVNTGEK 1140
EVTTYTNKSL ALNYVKAYAH NTRRNNATVD DTSYFQNMYA FFTTGSDVSN VTLTLSREAG 1200
DEATYFDEIR TFENNSSMYG DKHDTGKGTF KQDFENVAQG IFPFVVGGVE GVEDNRTHLS 1260
EKHDPYTQRG WNGKKVDDVI EGNWSLKTNG LVSRRNLVYQ TIPQNFRFEA GKTYRVTFEY 1320
EAGSDNTYAF VVGKGEFQSG RRGTQASNLE MHELPNTWTD SKKAKKATFL VTGAETGDTW 1380
VGIYSTGNAS NTRGDSGGNA NFRGYNDFMM DNLQIEEITL TGKMLTENAL KNYLPTVAMT 1440
NYTKESMDAL KEAVFNLSQA DDDISVEEAR AEIAKIEALK NALVQKKTAL VADDFASLTA 1500
PAQAQEGLAN AFDGNLSSLW HTSWGGGDVG KPATMVLKEA TEITGLRYVP RGSGSNGNLR 1560
DVKLVVTDES GKEHTFTATD WPDNNKPKDI DFGKTIKAKK IVLTGTKTYG DGGDKYQSAA 1620
ELIFTRPQVA ETPLDLSGYE AALAKAQKLT DKDNQEEVAS VQASMKYATD NHLLTERMVE 1680
YFADYLNQLK DSATKPDAPT VEKPEFKLSS VASDQGKTPD YKQEIARPET PEQILPATGE 1740
SQFDTALFLA SVSLALSALF VVKTKKD 1767

<212> Type : PRT
<211> Length : 1767
SequenceName : SEQ ID 372
SequenceDescription :
Sequence

--------

<213> OrganismName : Streptococcus pneumoniae R6

<400> PreSequenceString :

```
MKLYNKSELR YSRIFFDKRP PAFAFILIIS TAIILSGALV GAAYIPKNYI VKANGNSVIT      60
GTEFLSAISS GKVVTLHKSE GDMVNAGDVI ISLSSGQEGL QASSLNKQLV KLRAKEAIFQ     120
KFEQSLNEKY NRMSNSGEEQ EYYGKVEYYL SQLNSENYNN GTQYSKIQDE YTKLNKITAE     180
RNQLDADLQT LQNELIQLQQ QGDSPSLSDT TSADDKAKLE TKILEITTKI EALKTNITSK     240
NSEIDSQQSN IKDMNRTYND PTSQAYNIYA QLVSELGTAR SNNNKSITEL EANLGVATGQ     300
DKAHSILAPN EGTLHYLVPL KQGMSIQQGQ TIAEVSGKEK GYYVEAFVLA SDISRVSKGA     360
KVDVAITGVN SQKYGTLKGQ VRQIDSGTIS QETKEGNISL YKVMIELETL TLKHGSETVV     420

LQKDMPVEVR IVYDKETYLD WILEMLSFKQ                                     450
```

<212> Type : PRT

<211> Length : 450

SequenceName : SEQ ID 373

SequenceDescription :

Sequence

--------

<213> OrganismName : Neisseria meningitidis Z2491

<400> PreSequenceString :

```
MNKGLHRIIF SKKHSTMVAV AETANSQGKG KQAGSSVSVS LKTSGDLCGK LKTTLKTLVC      60
SLVSLSMVLP AHAQITTDKS APKNQQVVIL KTNTGAPLVN IQTPNGRGLS HNRYTQFDVD     120
NKGAVLNNDR NNNPFLVKGS AQLILNEVRG TASKLNGIVT VGGQKADVII ANPNGITVNG     180
GGFKNVGRGI LTIGAPQIGK DGALTGFDVR QGTLTVGAAG WNDKGGADYT GVLARAVALQ     240
GKLQGKNLAV STGPQKVDYA SGEISAGTAA GTKPTIALDT AALGGMYADS ITLIANEKGV     300
GVKNAGTLEA AKQLIVTSSG RIENSGRIAT TADGTEASPT YLSIETTEKG AAGTFISNGG     360
RIESKGLLVI ETGEDISLRN GAVVQNNGSR PATTVLNAGH NLVIESKTNV NNAKGSANLS     420
AGGRTTINDA TIQAGSSVYS STKGDTELGE NTRIIAENVT VLSNGSIGSA AVIEAKDTAH     480
IESGKPLSLE TSTVASNIRL NNGNIKGGKQ LALLADDNIT AKTTNLNTPG NLYVHTGKDL     540
NLNVDKDLSA ASIHLKSDNA AHITGTSKTL TASKDMGVEA GLLNVTNTNL RTNSGNLHIQ     600
AAKGNIQLRN TKLNAAKALE TTALQGNIVS DGLHAVSADG HVSLLANGNA DFTGHNTLTA     660
KADVNAGSVG KGRLKADNTN ITSSSGDITL VAGNGIQLGD GKQRNSINGK HISIKNNGGN     720
ADLKNLNVHA KSGALNIHSD RALSIENTKL ESTHNTHLNA QHERVTLNQV DAYAHRHLSI     780
TGSQIWQNDK LPSANKLVAN GVLALNARYS QIADNTTLRA GAINLTAGTA LVKRGNINWS     840
TVSTKTLEDN AELKPLAGRL NIEAGSGTLT IEPANRISAH TDLSIKTGGK LLLSAKGGNA     900
GAPSAQVSSL EAKGNIRLVT GETDLRGSKI TAGKNLVVAT TKGKLNIEAV NNSFSNYFPT     960
QKAAELNQKS KELEQQIAQL KKSSPKSKLI PTLQEERDRL AFYIQAINKE VKGKKPKGKE    1020
YLQAKLSAQN IDLISAQGIE ISGSDITASK KLNLHAAGVL PKAADSEAAA ILIDGITDQY    1080
EIGKPTYKSH YDKAALNKPS RLTGRTGVSI HAAAALDDAR IIIGASEIKA PSGSIDIKAH    1140
SDIVLEAGQN DAYTFLKTKG KSGKIIRKTK FTSTRDHLIM PAPVELTANG ITLQAGGNIE    1200
ANTTRFNAPA GKVTLVAGEE LQLLAEEGIH KHELDVQKSR RFIGIKVGKS NYSKNELNET    1260
KLPVRVVAQT AATRSGWDTV LEGTEFKTTL AGADIQAGVG EKARVDAKII LKGIVNRIQS    1320
EEKLETNSTV WQKQAGRGST IETLKLPSFE SPTPPKLSAP GGYIVDIPKG NLKTEIEKLS    1380
KQPEYAYLKQ LQVAKNINWN QVQLAYDRWD YKQEGLTEAG AAIIALAVTV VTSGAGTGAV    1440
LGLNGAAAAA TDAAFASLAS QASVSFINNK GDVGKTLKEL GRSSTVKNLV VAAATAGVAD    1500
KIGASALNNV SDKQWINNLT VNLANAGSAA LINTAINGGS LKDNLGDAAL GAIVSTVHGE    1560
VASKIKFNLS EDYITHKIAH AIAGCAAAAA NKGKCQDGAI GAAVGEIVGE ALTNGKNPAT    1620
LTAKEREQIL AYSKLVAGTV SGVVGGDVNT AANAAKVAIE NNLLSQEEYA LREKLIKKAK    1680
GKGLLSLDWG SLTEQEARQF IYLIEKDRYS NQLLDRYQKN PSSLNNQEKN ILAYFINQTS    1740
GGNTAWAASI LKTPQSMGNL TIPSKDINNT LSKAYQTLSR YDSFDYKSAV AAQPALYLLN    1800
GPLGFSVKAA TVAAGGYNIG QGAKAISNGE YLHGTVQVVN GTLMVAGSVS AQAAISAKPA    1860
PVTRYLSNDS APALRQALTA ESQRIRMKLP EEYRQIGNLA IAKIDVKGLP QRMEAFSSFQ    1920
KGEHGFISLP ETKIFKPISV DKYHNIASPP RGTLRNIDGE YKLLETIAQQ LGNNRNVSGR    1980
IDLFTELKAC QSCSNVILEF RNRYPNIQLN IFTGK                              2015
```

<212> Type : PRT

<211> Length : 2015

SequenceName : SEQ ID 374

SequenceDescription :

Sequence

--------

<213> OrganismName : Neisseria meningitidis Z2491
<400> PreSequenceString :

```
MDLIQTPNKQ FVDGDRRTPG TPVPAWWLNQ LQGELYSILN AVGIEPNKAD HAQVLSAIKT     60
LAADASQVAS IDALRKYSGT GYVNVNAYHA NTTVGGGVFV ADKADKSTAD NGCTVIVSTD    120
GTRWKRVFSG MLNLHDFGYV ASKNNALSTL NAAESAALDV VVDCLGLSID TGNIYPQKNK    180
YTNGKFVING KTVDVQYQPI RSGIGRFISG TGAAANLKSN EWTGAGLIVI GEGAMEQMEK    240
CVSSIAIGDR AQGFSKVSRD NIAIGADSLI NVQAATEWYD QSRMEGTRNI GIGGNAGRGI    300
TSGYSNVSIG RNAGQGLGEG SSNIALGAGA MAGTAPVGFS GDIEVFWPSS TSRTIAIGEA    360
VLQTYQGRAA QTAIGANAAR NTKKAEKVTA IGSAAMENLE RNRAPNGGDV VWTGTEAGTY    420
AQSGKNITLT FPNIRGAQAT YWVGIRLTSG TAQTLQNDVV PAQVVSVNGN TLIIQSSKEL    480
TATGAAELKY VYSVNSTATK NEELTIIGAN AMNKALTAGY STIIGVDAAL LGDNYQKTTA    540
IGASSLRTGS HISTTAIGYW VIPLASSEKC VAIGDSAGYR NVQGDFLTGK ITNSIAIGYG    600
ARINGDNEIQ IGTTGQTLYA PTAVNIRSDG RDKADVKPLT NGLDFVMKLK PMTGYYDRRD    660
SYVDELFKDL PADERADKVR EWWANPIKDG SHKEDRLRHW FIAQDIAALE DEYGRLPMVN    720
KTNDTYTVEY ETFIPVLTKA IQEMAARIET LETEMKESKK                          760
```

<212> Type : PRT
<211> Length : 760
SequenceName : SEQ ID 375
SequenceDescription :
Sequence
--------
<213> OrganismName : Streptococcus pyogenes MGAS8232
<400> PreSequenceString :

```
MKNISRKCFM TSVVCIILGG ILLGAGYATG GLQDIKHQTA PKKVIKTFDQ ITALDIDSSA     60
STITVETGPV QRPTVTYYTH PKFIDPIVTT LTGKTLSLSQ KPKDIVITGG IEILGFTLNN    120
SRQEKNYRSI TITVPEKTSL NEVKGSNVPH TTLSNLTVQD MQFDGNLTLL HTKVKKATIT    180
GMLEATKSQL TNLELKADYS FSNLTDSSVE NGTISLGNGQ LTTKDTTLKA INIQSLHPGG    240
IEAERTTLEN VTFTVSKSKE EEEENDYYDN DAIFTAHALT LKGTNTISGG DIDVDITLTK    300
AKAIAYRART ENGKVSLGSQ LTPAKIGKES TSDVISYVAE NKAATGNLTV NLNKGDITIK.   360
```

<212> Type : PRT
<211> Length : 360
SequenceName : SEQ ID 376
SequenceDescription :
Sequence
--------
<213> OrganismName : Streptococcus pyogenes MGAS8232
<400> PreSequenceString :

```
MFKKENLKQR YFNFGLVALA LTILAIIFAF SSKNADTKSY AKKSESKMVT IDKAPKNNHA     60
ITKEESKEKA KSIASEPIPT VENSVAPTVT EEAPVVQQEV TQTVQQVSSV AYNPNNVVLS    120
NGNTAGIVGS QAAAQMAAAT GVPQSTWEHI IARESNGNPN AANASGASGL FQTMPGWGST    180
ATVEDQVNAA LKAYSAQGLS AWGY                                           204
```

<212> Type : PRT
<211> Length : 204
SequenceName : SEQ ID 377
SequenceDescription :
Sequence
--------
<213> OrganismName : Streptococcus pyogenes MGAS8232
<400> PreSequenceString ;

MLEELKTLIK NPKLMITMIG VALVPALYNL SFLGSMWDPY GRVNDLPIAV VNHDKPAKRA 60
DKSLTIGNDM VDKMSKSKDL DYHFVSSKSA QKGLKKGDYY MVITLPEDLS QRATTLLNPE 120
PQKLTIRYQT SKGHGMVAAK MGETAMAKLK ESVSQNITKT YTSAVFSSMT DLQSGLKEAS 180
TGSQALDSGA KTAQMGSQML SDNLAGLSSA SWQFQQGTNR LTSGLTAYTA GVSQVKDGLG 240
QLSTDMPVYL NGVSRLSQGA SQLNQGLSQL TQSTTLSDDK AKRIQSLEVG LPVLNQGIQQ 300
LNENLSTMQV PKLNTDELGN NLAAIAQAAQ QLLVKEAAAH KEQLAVLQAT SAYQSLTAEQ 360
QGELTAALTQ TDKGEAVAPA QTILRSVQTL STSLQSLSQE DQSKQLEQLK EAVAQIANQS 420
NQALPGASSA LTELSTGLAK VNGSLNQQVL PGSNQLTTGL AQLNRYNTAI GSGVIKLSEG 480
ANALSSKSGE LLDGSHQLSE GATKLADGSS QLSQGGHQLT SGLTELSTGL SILNGSLAKA 540
SQQLSLVSVT DKNAKAVAKP LVLNEKDKDG VKTNGIGMAP YMIAVSLMVV ALSTNVIFAN 600
SLSGRPVKDK WDWAKQKFVI NGFISTMGSI VLYLAIQLLG FEARYGMETL GFIMLSGWTF 660
MALVTALVGW DDRYGSFASL VMLLLQVGSS GGSYPIELSG AFFQKLHPFL PMTYVVSGLR 720
QTISLSGHIG VEVKVLTGFL LAFMVLSLLI YRPKKTV 757

<212> Type : PRT
<211> Length : 757
SequenceName : SEQ ID 378
SequenceDescription :
Sequence
--------
<213> OrganismName : Streptococcus pyogenes MGAS8232
<400> PreSequenceString :

MSRDPTYTIN EHDLSFADGR FYVTFKADKS SETVRLNSSC LGNTIIKKLQ VEDDNTMHDF 60
VKPKVTTQQA FGLAQQVKEL DLQLKDPKSD LWGKIKFNNK AMLVEYANKE MSSAIAQSAE 120
QILLQVKSID DERYSKFEQT LNGIKQTVKS ESVESARTQL ASMFDSRISG LDGKYSRLSQ 180
TIDSLSSRLD DGVGNYSTLS QKVSGIDLRV SNAANDVSRL SQTAQGLQSQ ITNANQNYSS 240
LSQTVQGLQT TVRDNQSNAT SRINQLSDLI STKVSKGDVE TTIAQSYDKI AFAIRDKLPA 300
SKMSGSEIIS AINLDRSGVK ITGKNITLDG NSYISNAVIK DAHIANMDAG KINTGYLNAN 360
RIATEAITGE KIKMDYAFFN KLTANEGYFR TLFAKDIFAT SVQSVTLSAS KITGGVLAAT 420
NGASQWDLNN ANMTFNRDAT INFNSKNNAL VRKDGTHTAF VHFSNATPKG YRGSALYASI 480

GITSSGDGID SASSGRFAGL RSFRYATGYN HTAAVDQTEL YGDNVLIADD FSINRGFKFR 540
PDKMEKVLDM NDLYAAVVAL GRCWGHLANV GWNTAHSNFT SAVSRELNNY ITKI 594

<212> Type : PRT
<211> Length : 594
SequenceName : SEQ ID 379
SequenceDescription :
Sequence
--------
<213> OrganismName : Streptococcus pyogenes MGAS8232
<400> PreSequenceString :

MAADGKVTIL VDVDGKQVKV LNSELDKVAK HGDKGSSSLK KFAVGAGVFK LASAAVDLVS 60
QSLGKAITRE DTLEKYPRVM KAMGHSAEDV ARSTDKIANG IDGLPTTLDE VVGTAQRLTS 120
ITKDINKSTN LTLALNNAFL ASGASSEAAS RGLEQYAQML SAGKVDMQAW KTLQETMPYA 180
LQQTAEAFGF AGASAQKDFY EALKNGQITF DQFSNKLIEL NDGVGGFAEL AKENSKGIET 240
SFNNIKNAIA KGVANSIKAL DDLSKAATGK GIADHFDSLK VVINASFSAI NASIKASTPL 300
FKLLFSVIGA GISVVKALSP ALVGVASGLA AMRAVNETIT MIKALNRAWV MASASMSIGA 360
TTIKTVTAVQ AVSTTMTKAD MVARLSQLGV LKASTVIYGV MTGAISLSTA ATIASTAAVT 420
ALKAALVALT GPVGWVVGAI GALVAVGVSL WSWLTKESDE TKKLKKEQEG LVESNKQLRD 480
SVREGVQERK KGLESVKEST AAHQKLADEI IKLAAKENKT AGEKQNLKNK IDQLNGSIDG 540
LNLAYDKNSN SLSHNADQIK SRISAMEAES TWQTAQQNLL NIEQKRSEVS KKLAENADLR 600
KKWNEEANVS DSVRKEKIAE LTEEEAKLKN MQTQLQEEYN KTSATQQAAA DAMAAAEESG 660
SARQVIAYEN MSEAQRTAID NMRTKYSELL ETTTSIFDAI EQKTALSVDQ MNTNLEKNRA 720
ATEQWATNLE ILAQRGVDQG ILEQLRRMGP EGATQTQVFV DATDAELAPL QENFRAATET 780
AKNAMGSVLD SAGVEMPEKV KGMVTNVSTG LQAELQAANF AQLGQEIPNG VSQGISQGAG 840
KASDASVKMG QEVKRSFQGE LGIHSPSRVF TEYGGHITDG LSNGVTNGTS KVMQTMQSLA 900
QQMSQKGQQI VNDMRSKSNQ ITDAFSTMSG PMHSHGVNAM QGLANGIYAG SGAALAAAQS 960
IAARITATIQ SALDIHSPSR VMRDEVGRFI PQGIAVGIDA DRKVIDSSMQ KLKESMTINA 1020
TPEIASGFGG GVAGIANQTT NNSNNSFTLN VKVDESDGNS HEKYQRLFRE FSWYIQQQQG 1080
RLGDVK 1086

<212> Type : PRT
<211> Length : 1086
SequenceName : SEQ ID 380
SequenceDescription :
Sequence
--------
<213> OrganismName : Streptococcus pyogenes MGAS8232 <400> PreSequenceString :

```
MAKEPWEEKI VDDTIGTRTR KSRNAFISTP WLTALLSVFF VIIVAILFIF FYTSNSGSNR    60
QAETNGFYGA STHKKTRKAS NAKKTSSSST TTDTTPSSEE TLASSEGTGE TLTVLAGEGA   120
ASIAARAGIS VEQLQALNPE HMTQGYWYAN PGDQVTIK                           158
```

<212> Type : PRT
<211> Length : 158
SequenceName : SEQ ID 381
SequenceDescription :
Sequence
--------
<213> OrganismName : Streptococcus pyogenes MGAS8232
<400> PreSequenceString :

```
MSKRGKIKIT TKTKLITASV ITLVLIITGV VLWKQQQNTL TADIAKEPYS TVSVTEGSIA    60
SSTLLSGTVK ALSEEYIYFD ANKGNDATVT VKIGDQVTQG QQLVQYNTTT AQSAYDTAVR   120
SLNKIGRQIN HLKTYGVPAV STETNKDEAT GEETTTTVQP SAQQNANYKQ QLQDLNDAYA   180
DAQAEVNKAQ IALNDTVVIS SVSGTVVEVN NDIDPSSKNS QTLVHVATEG QLQVKGTLTE   240
YDLANVKVGQ SVKIKSKVYS NQEWTGKISY VSNYPTESNA GSTTPAGSTG AGSSTGAAYD   300
YKIDIISPLN QLKQGFTVSV EVVNEAKQAL VPLTAVIKKD KKHYVWTYDD ATGKAKKVEV   360
TLGNADAQQQ EIHKGVAVGD IVIANPDKNI KPDKKLEGVI SIGTNTKPEK DSQSKNKKSG   420
VDK                                                                 423
```

<212> Type : PRT
<211> Length : 423
SequenceName : SEQ ID 382
SequenceDescription :
Sequence
--------
<213> OrganismName : Treponema pallidum
<400> PreSequenceString :

```
MLRLPTARAC ITMGTMIRHT FTHRCGALLC ALALGSSTMA ATAAAKPKKG QMQKLRQRPV    60
WAPTGGRYAS LDGAFTALAN DASFFEANPA GSANMTHGEL AFFHTTGFGS FHAETLSYVG   120
QSGNWGYGAS MRMFFPESGF DFSTTTEPVC TPASNPIKQR GAIGIINFAR RIGGLSLGAN   180
LKAGFRDAQG LQHTSVSSDI GLQWVGNVAK SFTSEEPNLY IGLAATNLGL TVKVSDKIEN   240
CTSTCEKCGC CKERCCCNGK KACCKDCDCN CPCQDCNDKG TVHATDTMLR AGFAYRPFSW   300
FLFSLGATTS MNVQTLASSD AKSLYQNLAY SIGAMFDPFS FLSLSSSFRI NHKANMRVGV   360
GAEARIARIK LNAGYRCDVS DISSGSGCTG AKASHYLSLG GAILLGRN                408
```

<212> Type : PRT
<211> Length : 408
SequenceName : SEQ ID 383
SequenceDescription :
Sequence
--------
<213> OrganismName : Treponema pallidum
<400> PreSequenceString :

```
MSRTFRAWQC VGALCALSPL LPAYSSEGVR EVPPSQSPQV VVAYEPIRPG DQLLKIGIVA    60
GCQLYIAGGN GTNGSSSSGT NGNGNGKLLG GGGFHLGYEY FFTKNFSLGG QVSFECYRTT   120
GSNYYFSVPI TVNPTYTFAV GRWRIPLSLG VGLNIQSYLS KKAPGLIAEA SAGLYYQYTP   180
DWSIGGIVAY TQLGDIASSP DKCRAVGLAT IDFGVRYHF                          219
```

<212> Type : PRT
<211> Length : 219
SequenceName : SEQ ID 384
SequenceDescription :
Sequence
--------
<213> OrganismName : Escherichia coli 0157:H7
<400> PreSequenceString :

```
atgataaatt taagtaagga agcaacggtg gggaaagcat taacccctat tgctatactt     60
atgatgttgt cttttcctgt agcttctcaa gcggcgggat tagtcataaa aaatggaacg    120
gtatataacg ccaatggtgt gccagtcgtt gacatcaaca aacctaacgg tagcggttta    180
tctcataata tctgggataa cctaaacgtt gataaaaatg gtgtcgtttt caataatagc    240
gctaatgaat ccagtacttc acttgccgga aatattcagg gaaacagtaa tctgacctcc    300
gggtcggcga aggtgatcct gaatgaggtt acttccaaaa atccttcaac cattaatggg    360
atgatggaag ttgcagggga taaagcggat ctgattattg ccaaccccgaa tggtattact    420
gtaaacggtg gcggttcaat caatacaggt aaacttacct taaccaccgg gacgccggat    480
atccaggatg acaagctggc cggttactcc gtgaacggcg gtaccattac gctcggtaaa    540
ctggataacg ccagcccgac agaaattctg tcccgtaacg tggtagttaa cggcaaagtg    600
tctgccgatg agctgaacgt tgttgctggc aataactatg ttaatgccgc aggccaggtg    660
accggtagcg tatccgccac ggggtcccgt aacggttaca gcgtagatgt tgccaaactg    720
ggcggaatgt atgcgaacaa aatcagtctg gtcagcaccg agaaaggtgt gggggttcgc    780
aacctcggcg ttattgctgg gggtgttaat ggtgtcagca tcgattccaa aggtaacctg    840
ttaaacagta acgcccagat tcagtctgca agcacgatca acctgacaac aaatggtact    900
ctggataaca ccaccggtac ggtgacatct gtaggcacta tctcgcttaa taccaacaag    960
aatactatcg tgaatacccg tgcgggtaac atctctacga tgggcgatat ctacgttaac   1020
agcggtacga ttgacaatac taacggcaag cttgcggctg caggaatgct ggcggttgat   1080
accaataacg ccacgctgat taactctggt aaagggagtt ctgtcgggat tgaagcgggg   1140
ctcgtggcgc tgaaaaccgg aacgctcaac aacagcaatg gtcagattcg cggtggctat   1200
gtgggtcttg aatccgctgc gctgaataac aacaacggtg atatccagac caccggcgat   1260
atcgccatta tcagtaacgg taatgtggat aacaacaaag gtctgatccg ttcgtccacc   1320
gggcatatcg ttattggcgc ggcaggtagc gtaaataatg gttcaaccaa aaccgccgat   1380
accggcagtt ctgactctct gggcattatt gcagataccg gcgtagaaat tggtgcgaac   1440
aacatcaata acaacggcgg acagattgcg tctaatggca acgtctccct gtcaagttac   1500
agcacgatcg acgactatgc gggcaaaatt ctgtccaaca gcaaagtgat tatcaaggga   1560
agctctctgc gtaacgatac cgggggggatc agcggtaagc agggtattga agtcgccgtt   1620
ggcggcagcc tgaccaataa tattggcgtg atcagctctg aagagggtga tatctccctg   1680
ttagccaact ccgtggataa ccacggcggc ttcatgatgg ggcagaacat cacgatggag   1740
tcgatgtctg gcgtcaataa caacacagcg ctgatcgtgg ccagcaaaaa actgaagata   1800
aatgcgcgcg gcagtatcga aaaccgcgat ggcaataact tcggtaatgc ttatggtctg   1860
tacttcggca tgcctcagca aacgggtgga atggtcggca aggaaggcat cgagctttcc   1920
gggcagaaca tctataacaa caacagccgt cttatcgctg aggatggtcc tctgactctg   1980
caggcgcaga acacgttcga caacacgcgt gctctggtca ccagcggggc ggatgcatct   2040
attcaggttg gcggaacgta ttacaacaac tacgctacca cctggagtgc gggcaacctg   2100
```

```
gatatcgacg cgaccacgct gcaaaacagc agcagcggta cgatgatcga taacaatgcg    2160
accgggttca tagcatctga taaaaacctg tcactggaag tggtgaatag ccttaccaac    2220
tacggctgga tcagcggtaa aggcgatgtt gatgtcacgg tgaataacgg caacctgtat    2280
aaccgcaata ccattgcggc tgaaaagggg ctggatattg ccgcgttgaa cggtattgaa    2340
aactggaagg atatttctgc tggcggcgac ctgacgatga acaccaatcg ccatgtgacc    2400
aacaactcca acagcaatat ggtggggcag aatattgtta ttaacgcggt taacgatatc    2460
aacaaccgtg gcaacattgt cagtgacgct gacctgaacg tgacgaccaa aggcaacctg    2520
tataactatc tctatatggt agggtatggg gatatcgcat tgtcggcaaa tagcgtggcg    2580
aacaataacg cgaccatcga agcgacaggc gatctgatta tcgattcgaa gggtaacgtg    2640
ggtaacaacc gcggtaatct gcatgcgttg aacggcgtgt tgtctgttaa aggcaacaat    2700
ctgaacaacg ataacggtga aattcgtggt tatggcgatg tcacgctggc actgacgggc    2760
aactacgaca gctataaggg ttcgctgacc tctgaaacgg gcgacgtgac tctgacggcg    2820
aacattgtag acaacgccta tggtttgatt gccggtgaga atgtttctgt cgatgctaaa    2880
tcgacgattt acaacaacac tgcgctgatc gcggcgaata aaaagctggt tattaacgct    2940
ggcggcaacc tcgaaaaccg cgacgggaat aacttcctgc gtaataacgg cgcgctgttt    3000
ggaattaccg acaacgttgg cggcatcgta ggtaaagaag gtgtcacgct ttctgctcag    3060
aacgtctaca acaataacag cagcatcatc gctgaaaatg gtccgcttaa tctgctgtcc    3120
aggggaacgc tggataatac ccgcgcgctt cttagcagtg gggctgatgc catcatccgt    3180
gcggcaggga cgttctacaa caactatgcc accacgtaca gcgccggtaa tctcgacgtt    3240
tatgcggcgt cgttgaacaa cgccagcgat ggtcgcctgg aagacaatac cgccacgggc    3300
gtgattgcgt ctgacaaaaa cctggatctg agcgttgata acagtgtcac taactatggt    3360
tggatcagcg gtaaaggaga tgtgcatttc aatgttctga aaggcacgct gtataaccgt    3420
aatgccatcg cggcggacaa cgcgctgacc attaatgccc tgaacggtgt tgagaacttt    3480
aaagacattg tggcgggtac tgcgctgact attgatacgc agaagtatgt taccaacaac    3540
agcaacagta atatgttggg acaaaccatc gcgatcaatg ccgtgaatga cattaataac    3600
cgtggaaata ttgtgggtga ttattctctg ggtgttaaaa ccaccggtaa tatttataac    3660
tacctcaata tgctgagtta tggtgtcgct ggcgtatcgg caaataaggt tacgaatagc    3720
ggtaaagacg ctgttctcgg tggcttctac ggtttagcgt tagaagcaaa cgaaactgat    3780
aacaccggta ctattgtcgg catgtaa                                        3807
```

<212> Type : DNA
<211> Length : 3807
SequenceName : SEQ ID 385
SequenceDescription :
Sequence
--------
<213> OrganismName : Escherichia coli 0157:H7
<400> PreSequenceString :

```
gtgaacacaa tacacttgcg ctgtctcttc aggatgaatc ccctggtctg gtgcctgtgg      60
gctgatgttg cagcaaagct aaggtcgctt aaacgctact cagtattcac ttttcagagg     120
atgaaattta tgaacaggac cagtccctat tattgtcgtc gctcagtact ttccttattg     180
atatctgcct tgatatatgc cccgcccggg atggctgcct tcactcctga tgttattggt     240
gtggtaaacg atgagactgt agatggcagc caacgagtag atgaacgagg tacaacaaat     300
aacactcata ttatcaacca tggccagcag aatgtttatg gcggggtatc taatggaagt     360
cttattgaat ctggtggata tcaagatgta ggaaggcata acaattatgt ggggcagtct     420
aataatacca ccattaacgg gggcagacag tcaattcatg acgggggtat ttccacaggt     480
acgataatcg agagtggcaa tcaggacgtt tataaagggg gtatcagcaa tggaacgaca     540
attaagggcg gtgcttcacg cgtagaggga gggagtgcga atggaacact cattgatggt     600
ggtagccaga tagtaaaagt tcaagggcat gctgatggta caacgataaa taagtctggc     660
tctcaggacg tagtacaagg aagtctggca acgaacacaa ccataaatgg tggtcgacag     720
tatgttgaac agagcacagt agaaacaacc accatcaaaa atggcggtga gcaaagagta     780
tatgagagcc gtgcgctgga cacgacgatt gaaggcggaa ctcagtctct gaatagtaag     840
tcaacggcaa aaaatactca gatctattct ggtggtacgc aaattattga taacaccagc     900
tcctcggatg ttattgaagt ttattccggt ggcgtgcttg atgttagtgg tggtacggca     960
acaaatgtta cccagcacga tggtgcaatt ttaaaaacta acactaacgg tacgacggtg    1020
agcggtacga atagtgaagg tgcattctcc atccacaatc acgtggcaga caatgtgttg    1080
ctggaaaacg gtggtcattt agacataaac gcatatggtt cggcaaacaa gacgattatt    1140
aaagataaag gaacaatgtc agttttaacc aatgctaaag ctgatgcgac ccgaatagat    1200
aatggcgggg ttatggatgt tgcaggaaac gcgacaaata ccataattaa tggtggcaca    1260
cagaatatta ataattatgg catagccaca ggcaccaata tcaacagcgg aacgcaaaat    1320
atcaaaagcg gcgggaaagc tgacacaaca attatatcct ccgggagccg gcaggttgtt    1380
gagaaagatg gtacggcaat tggcagcaat attagcgccg gaggctcgct gattgtctat    1440
accggcggta ttgcacatgg ggttaaccag gagacgggca gtgctttagt tgccaacacg    1500
ggtgcaggga ctgatatcga aggatacaac aagctctctc acttcactat taccggaggg    1560
gaggctaatt atgttgtgct ggaaaatacc ggcgaactga cggtagtggc taaaacctcg    1620
gcgaaaaata ctaccattga tgctggcggt aagctgattg tccagaagga ggctaaaaca    1680
gatagcacca gacttaataa tggcggcgtt ctggaggttc aggacggtgg tgaggctaag    1740
```

```
catgttgagc aacaatccgg cggcgcatta attgcttcca cgacctccgg aacacttatc    1800
gaaggaacca acagttatgg tgatgctttc tacatcagga attcagaagc taaaaatgta    1860
gtgctggaaa acgctggctc attaacagtc gtcactggtt cccgggcagt tgacacgatt    1920
attaatgcca acggcaaaat ggatgtttat ggaaaagatg ttggcactgt actcaatagt    1980
gctggcaccc aaacaatata tgccagtgcc acttctgata aagcaaatat caaaggtggc    2040
aagcaaacgg tatatggttt agccactgaa gcaaatatcg aaagtggtga acaaattgtt    2100
gatggtgggt caacagagaa aacacacatc aatggtggca cgcaaaccgt tcagaattat    2160
ggtaaggcga tcaataccga tatcgtctct ggcctacaac aaattatggc aaacgggaca    2220
gcggaaggtt ccattattaa tggcggttca cagatagtta atgagggcgg tctggctgaa    2280
aactcggtgc ttaatgatgg cggcacactc gatgtgcggg agaaaggcag cgcaacgggg    2340
atacagcaga gtagccaggg cgcgttggtt gcaaccacca gggcgacgcg ggtcacagga    2400
acacgcgcgg atggcgtcgc gttcagcatc gagcagggtg cggcgaacaa tatcctgctg    2460
gcaaatggcg gagtgttaac cgtggagtca gacacctctt ctgacaaaac acaggtcaat    2520
acgggcggac gggagatcgt caaaacaaaa gccactgcga caggcacgac gctcaccggc    2580
ggtgaacaaa ttgtcgaggg tgtggcgaat gagacaacaa ttaacgacgg cggaatacaa    2640
acagtttcag ctaacggaga ggcaataaaa acaacgatca atgaaggcgg tacgctgaca    2700
gtcaacgata atggcaaagc gacagatatc gtccagaaca gcggtgccgc tctccagacg    2760
agcacggcta acggtattga aatcagcggt actcaccagt acggcacttt ttccatttcc    2820
ggcaatttag cgaccaatat gttgctggaa aatggcggta atttattggt attagcaggt    2880
accgaagctc gcgactccac ggttggcaag gggggggcaa tgcaaaacca gggtcaggac    2940
tccgccacaa aggttaactc tggtgggcaa tataccgttg ggcggtcaaa agatgagttt    3000
caggctctgg cccgggcaga agatctccag gttgctggcg ggacagcaat cgtctacgca    3060
ggtacgctgg cggatgcatc ggtcagtggc gcgacaggaa gcctgtcgtt aatgacgcca    3120
cgggataatg ttacgccagt taaactcgaa ggggcgatcc ggattaccga tagcgcgaca    3180
ttaactatcg gcaatggcgt tgatacgacg cttgccgacc tgacggctgc cagccggggc    3240
agtgtctggc ttaacagcaa taattcctgt gcaggcacca gcaactgcga gtatagagta    3300
aacagtttgc tacttaacga cggtaatgtt tatttatcag cacaaacagc agcgcctgcc    3360
acaactaacg gtatatacaa tacgctgaca accaatgaac tttccggtag cggtaatttc    3420
tacctgcata ccaacgttgc aggctctcgg ggcgatcaac tggtcgtcaa caacaacgcc    3480
actggtaatt ttaaaatctt tgttcaggat accggcgtca gtcctcagtc tgacgacgcg    3540
atgacgctgg tgaaaacagg gggaggggat gcttcgtttt cgctgggcaa tactggcggt    3600
ttcgttgatc ttgggaccta tgagtatgtc ctgaaaagcg atggcaacag caactggaac    3660
ctgaccaatg atgtcaaacc caacccggat cccaacccaa atcccaaccc aaatccgaag    3720
ccggatccaa aaccagaccc aaaaccggat ccgaaaccag acccgactcc cgagccaacg    3780
ccgacacccg ttccggagaa acgcatcacg ccttctaccg cagccgtact caatatggca    3840
gcaacattac cgttggtatt tgatgctgag ctaaacagta ttcgcgagcg gttgaacata    3900
atgaaagcga gtccacacaa caataatgtc tggggggcga cgtataacac ccgtaataat    3960
gtcaccaccg atgcgggggc cgggtttgag cagacgctga ccggaatgac agtggggatc    4020
gacagcccta atgatattcc tgaggggatt gcgacgctgg gcgcttttat gggttattcc    4080
cattcacata tcggttttga tcgcggagga catggcagtg tgggcagtta ttctctgggc    4140
ggctatgcca gttgggaaca tgaaagtggt ttctatctgg acggtgtcgt gaagctgaac    4200
cgttttgaaa gtaacgtagc cggtaaaatg agcagcggtg gagccgccaa tggcagttac    4260
cacagcaacg ggctgggcgg tcacattgaa accgggatgc gatttaccga tggtaactgg    4320
aacctgacgc cgtatgcatc gttaacgggg ttcaccgctg ataaccccga atatcattta    4380
tccaatggca tggaatcgaa atcagtcgat acccgcagta tatatcgtga actgggcgca    4440
acgctgagtt acaacatgcg tctggggaac ggtatggaaa ttgagccgtg gctgaaggcg    4500
gctgtgcgca aagaatttgt cgatgataac cgggtgaagg tgaataatga cggtaatttc    4560
gtcaatgatt tgtcgggcag acgtggaata taccaggcag gtattaaagc ctcattcagc    4620
agtacgttaa gcgggcatct tggggtgggg tatagccatg gtgccggtgt ggaatccccg    4680
tggaacgcgg tagctggtgt gaactggtcg ttctga                              4716
```

<212> Type : DNA
<211> Length : 4716
SequenceName : SEQ ID 386
SequenceDescription :
Sequence
--------
<213> OrganismName : Escherichia coli 0157:H7
<400> PreSequenceString :

```
atggcagtaa agatttcagg tgtactgaaa gacggcacag gaaaaccggt agagaactgc     60
accattcaac tgaaagccag acgtaacagc gccacggtgg tggtgaacac ggtggcctct    120
gaaaatccgg atgaagccgg tcgttacagc atggacgttg agtacggtca gtacagcgtt    180
attctgttgg tggaagggtt cccgccgtca catgccggga ccatcaccgt gtatgaagat    240
tctcaacccg gtacgctgaa tgatttcttt ggtgccatga ctgaggatga tgtccgtccg    300
gaggcactgc gccgttttga gctgatggtg gaagaggtgg cgcgtaacgc gtccgcggtg    360
gcacagaaca cggcagccgc gaagaagtca gccagcgatg ccagcacatc agcccgtgag    420
gcggcaaccc atgcgactga tgctgcggac tcagcacgcg cagccagcac gtcagccgga    480

caggccgcgt cgtcggctca gtcagcgtct tccagcgcag gaacggcatc aacaaaggct    540
actgaagcat caaaaagtgc tgccgctgca gagtcctcaa aagcgcggc ggctaccagt    600
gccggtgcgg cgaaaacgtc agaaacgaat gcggcagtgt cacaacaatc agccgccact    660
tctgcatcca ccgcgaccac gaaagcgtca gaagctgcct cctcagccag ggatgcgtcg    720
gcttcaaaag aggcggcaaa atcatcagaa acgagcgcag cctcgagcgc cagtagtgca    780
gcctcctcgg caacggcggc aggcaattcc gcgaaggcgg ccaaaacgtc tgagacaaac    840
gctaagtcct ctgaaacggc agcagaacag agtgcctccg cagcagcagg ctcaaaaaca    900
gcggctgcat tatctgccag tgccgcgtca acaagtgccg ggcaggcctc agccagtgcc    960
accgccgccg gaaaatcggc agaaagtgcc gcatcgtctg cttcaacagc cacaacgaag   1020
gctggcgaag ccactgaaca ggccagcgca gcagcgagtt ctgcttccgc agcgaagaca   1080
tccgaaacga acgcgaaagc gtcggaaacc agcgcagaat cctcaaaaac ggctgccgca   1140
tcgtcagcca gttcggcggc gtcatcggca tcatctgcgt ctgcttcaaa agatgaggcg   1200
accagacaag cgtcagcagc gaagagcagc gccacgacgg catccacgaa ggcgacagag   1260
gcagctggta gtgcgacggc agcagctcag agcaaaagta cggcggaatc tgcagcaacg   1320
cgcgctgaga cagcggcaaa acgggcagag gatattgcat ccgccgtggc gcttgaggat   1380
gcgagcacga cgaaaaaggg gatagtacag ctcagcagtg cgactaacag cacttccgag   1440
tcactggcgg caacgccaaa agccgttaag gccgcgtatg agctggctaa cggaaatac    1500
accgcacagg atgcaacgac agcacagaaa gggatagttc agcttagcaa cgcgaccaac   1560
agcacatctg aaatgctggc ggcaacgcca aagtcggtaa aggcagccta tgaccttgct   1620
aacgggaaat atactgctca ggacgctacg acagcacaaa aggaattgt ccagctcagt    1680
agtgcaacca acagcgcatc tgaaacgctt gccgcgacac cgaaagcagt gaaagcagct   1740
aatgataatg cgaatggtcg ggtaccttct gcccgtaagg tgaatggtaa ggcgctttca   1800
tcggatataa cactgacgcc gaaagatatt ggtacgctta actcaacaac aatgtcattc   1860
agcggtggtg ctggttggtt caaattagca acggtaacca tgccacaggc gagttctgtt   1920
gtttcaatta cgttgattgg tggcgcggga tttaacgtgg ggtcacctca acaggcaggt   1980
atatctgaac ttgttttgcg tgcaggtaat ggtaatccga aggggattac tggtgcttta   2040
tggcagcgca catcgacagg gtttacaaat tttgcctggg tcaatacatc tggtgatact   2100
tacgatattt acgttgcaat cggaaattat gcgactggtg taaatattca atgggattat   2160
accagtaatg ccagcgtgac gattcatacg tcaccagcat attctgctaa taagccggaa   2220
gggttaacgg acggtacagt ttattcactc tatacgccat cagagcagtt ttatccgcct   2280
ggcgcaccaa tcccgtggcc atcagatacc gttccgtctg gctatgccct gatgcagggg   2340
cagacttttg acaaatctgc atacccgaaa cttgcagccg cttatccgtc aggcgtgatc   2400
cctgatatgc gtggctggac gattaagggc aaacctgcca gtggtcgggc cgtattgtct   2460
caggaacagg acggcattaa atcgcacacc cacagcgcca gcgcatccag tacggatttg   2520
gggacgaaaa ccacatcgtc gtttgattac ggcactaaat ccacgaataa caccggggcg   2580
cacacgcaca gtgtgagcgg tacagccgca agtgccggaa accatactca tagtgtcaca   2640
ggcgcatcag cagtcagcca gtggtcacaa aatgggtcag tacataaggt agtgtctgcg   2700
gccagtgtga atacaagtgc tgcaggagcg cacactcata gtgtcagcgg cacagctgca   2760
tctgcaggtg ctcacgcaca tactgtcggt attggtgctc atacgcactc tgttgcgatt   2820
ggctcacatg gacacaccat caccgttaac gctgcgggta acgcggaaaa cactgtcaaa   2880
aacatcgcat ttaactacat tgtgaggctt gcataa                             2916
```

<212> Type : DNA
<211> Length : 2916
SequenceName : SEQ ID 387
SequenceDescription :
Sequence
--------
<213> OrganismName : Escherichia coli 0157:H7
<400> PreSequenceString :

```
atgaaacgag ttattaccct gtttgctgta ctgctgatgg gctggtcggt aaatgcctgg      60
tcattcgcct gtaaaaccgc caatggtacc gctatcccta ttggcggtgg cagcgctaat     120
gtttatgtaa accttgcgcc tgccgtgaat gtggggcaaa acctggtcgt agatctttcg     180
acgcaaatct tttgccataa cgattatccg gaaaccatta cagactatgt cacactgcaa     240
cgaggctcgg cttatggcgg cgtgttatct aatttttccg ggaccgtaaa atatagtggc     300
agtagctatc catttccgac caccagcgaa acgccgcggg ttgtttataa ttcgagaacg     360
gataagccgt ggccggtggc gctttatttg acgcctgtga gcagtgcggg cggggtggcg     420
attaaagctg gctcattaat tgccgtgctt attttgcgac agaccaaaaa ctataacagc     480
gatgatttcc agtttgtgtg gaatatttac gccaataatg atgtggtagt gcctactggc     540
ggctgcgatg tttctgctcg tgatgtcacc gttactctgc cggactaccc tggttcagtg     600
ccaattcctc ttaccgttta ttgtgcgaaa agccaaaacc tggggtatta cctctccggc     660
acaaccgcag atgcgggcaa ctcgattttc accaataccg cgtcgttttc accagcgcag     720
ggcgtcggcg tacagttgac gcgcaacggt acgattattc cagcgaataa cacggtatcg     780
ttaggagcag taggaacttc ggcggtaagt ctgggattaa cggcaaatta cgcacgtacc     840
ggcgggcagg tgactgcagg gaatgtgcaa tcgattattg gcgtgacttt tgtttatcaa     900
taa                                                                   903
```

<212> Type : DNA
<211> Length : 903
SequenceName : SEQ ID 388
SequenceDescription :
Sequence
--------
<213> OrganismName : Escherichia coli 0157:H7
<400> PreSequenceString :

```
atgggctacg ttacaggtgg attaccaatg aagaataacc gtgcgtgggc gcttatcagt      60
ggtctgatat tgttcagcgg aacggcccca gctgccgata acctgcattt taccggtaat     120
ttgcttggta aatcctgtac tcctgtaatc aatggcaact tacttgcaga aattcatttc     180
cccacaattg ctgccagcga tttaatgcaa cgtggtcagt cagatcgcgt accgttagtt     240
tttcagttga aagattgcaa aagcaccacg gcgtttaatg tcaaggtgac cttgatggga     300
acagaagata ccgacttacc aggatttctg tcgattgatt cgtcatcttc tgcaacgggt     360
gttgggattg gcattgaaac tgccggaggg gcggctgtac ctattaacag taccacaggt     420
gcctcatttc cattaaatca gggaaataac agtgtcaatt ttaatgcctg gttacagacc     480
gtaaatggac gaaatgttac atcgggtgat ttcaccgcca caatgacggt aacttttgag     540
tatttttaa                                                             549
```

<212> Type : DNA
<211> Length : 549
SequenceName : SEQ ID 389
SequenceDescription :
Sequence
--------
<213> OrganismName : Escherichia coli 0157:H7
<400> PreSequenceString :

```
atgaaacgac atctgaacac cagctacagg ctggtatgga atcacattac gggcaccctg      60
gtggtggcct ccgaactggc ccgctcacgg ggaaaacgcg ccggtgtggc ggttgcgctg     120
tctcttgctg ctgtcacatc agtcccggca ctggctgctg acaaggttgt acaggcggga     180
gaaaccgtga acgatggaac actgacaaat catgacaacc agattgtctt cggtacggcc     240
aacggaatga ccatcagtac cgggctggaa ctggggccgg acagtgaaga aaacaccggt     300
gggcaatgga tacagaatgg cgggatagcc ggaaacacca ctgtcaccac aaatggtcgt     360
caggtcgtgc tggagggggg aacagccagt gatacggtta ttcgtgacgg cgggggacag     420
agcctgaacg gactggcggt gaacaccaca ctgaataaca gaggcgagca gtgggtgcat     480
gagggcgggg ttgccaccgg tacaattatc aaccgcgacg gttaccagag cgttaaaagt     540
ggcgggctgg caacaggaac catcatcaac accggcgcag aaggcggccc tgattctgac     600
aactcgtata cgggtcagaa ggtccaggga acagcagaat ccaccaccat caacaaaaat     660
ggacggcaga ttatcttatt ttccgggcta gcccgtgaca ctctcattta cgcaggtggt     720
gaccagtcgg tacacggaag ggccctgaat accacactga atggcggtta ccaatatgtg     780
cacagggacg gacttgcgct gaacacggta attaacgagg ggggctggca ggttgttaag     840
gcaggtggcg ctgccggtaa caccaccata aatcagaacg gtgaactgag ggtacatgcc     900
ggcggggaag ccactgcagt cacccagaac acgggcggtg cactggttac cagtactgct     960
gcaactgtca tcggcacaaa ccgtctgggg aatttcacgg tggaaaacgg taaggctgac    1020
ggtgttgttc tggaatccgg cggtcgtctg gatgtactgg agagccattc agcacagaat    1080
accctagtgg atgacggcgg taccctggca gtgtctgccg gcggtaaggc gacaagtgtc    1140
accataacat ccggtggtgc cctgattgca gacagtggtg ccactgttga ggggaccaat    1200
gccagcggta agttcagtat tgatggcaca tccggtcagg ccagcggcct gctgctggaa    1260
aatggcggca gctttacggt taatgccggg ggacaggctg gcaacaccac tgtcggacat    1320
cgtggaacac tgacgctggc tgccggggga agtctgagtg gcagaacaca gctcagtaaa    1380
ggcgccagta tggtactgaa tggtgatgtg gtcagtaccg gcgatattgt taacgcaggg    1440
gagattcgct ttgataatca gacgacaccg aatgccgcgc tgagccgtgc tgttgcaaaa    1500
agtaactccc cggtaacgtt ccataaactg accaccacga acctcaccgg ccagggcggc    1560
accatcaata tgcgtgttcg ccttgatggc agcaatgcct ctgaccagct ggtgattaat    1620
ggtggtcagg caaccggcaa aacctggctt gcgtttacaa atgtcggaaa cagcaacctc    1680
ggggtggcaa ccaccggaca gggtatccgg gttgtggatg cacagaatgg cgccaccaca    1740
gaagaaggtg cgtttgccct gagtcgcccg cttcaggccg gcgcctttaa ctacaccctg    1800
aaccgtgaca gcgatgaaga ctggtacctg cgcagtgaaa atgcttatcg tgctgaagtc    1860
cccctgtata catccatgtt gacacaggca atggactatg accggattct ggcaggctcc    1920
cgcagccatc agaccggtgt aaacggtgaa aataacagcg tccgtctcag cattcagggc    1980
ggtcatctcg gtcacgataa caacggcggt attgcccgtg gagccacgcc ggaaagcagc    2040
ggcagctatg gcttcgtccg tctggagggt gacctgctca gaacagaggt tgccggtatg    2100
tctctgacga caggggtgta tggtgctgca ggccattctt ccgttgatgt taaggatgat    2160
gacggttccc gcgccggcac ggtccgggat gatgccggca gtctgggcgg atacctgaat    2220
ctggtacaca catcctccgg cctgtgggct gacattgtgg cccagggaac ccgtcacagc    2280
atgaaagcgt catcggacaa taacgacttc cgcgcccggg gctggggctg gctgggctca    2340
ctggaaaccg gtctgccctt cagtatcact gacaatctga tgctggagcc acaactgcag    2400

tacacctggc agggactctc cctggatgac ggccaggata acgccggtta tgtgaagttc    2460
gggcatggca gtgcacaaca tgtgcgtgcc ggtttccgtc tgggcagcca caacgatatg    2520
acctttggtg aaggcacctc atcccgtgac accctgcgcg acagtgcaaa acacagtgtg    2580
agtgaactgc cggtgaactg gtgggtacag ccttctgtta tccgcacctt cagctcccgg    2640
ggtgacatga gcatggggac agccgcagcc ggcagtaaca tgacgttctc accgtcccgg    2700
aatggcacgt cactggacct gcaggccgga ctggaagccc gtatccggga aaatatcacc    2760
ctgggcgttc aggccggtta tgcccacagc gtcagcggca gcagcgctga aggctataac    2820
ggtcaggcta cgctgaatat gactttctga                                      2850
```

<212> Type : DNA
<211> Length : 2850
SequenceName : SEQ ID 390
SequenceDescription :
Sequence
--------
<213> OrganismName : Escherichia coli 0157:H7
<400> PreSequenceString :

```
atgaaaaaat ggcattatat attttgcata attctctttc atttagggtt accgtgcggg     60
tatgcggcaa atgatggcac gtgtgcaaca agaggcggca cacatacatt aagccttaat    120
tttcctctga caacggtcag tgcagcaaac aatgtgcctg gaaatacatt aatagatatt    180
gctaatgcaa catcttctga aaattatagc gttctgtgta actgtgattc aaaacatagc    240
aatggcgctt atcacgaaat atattatacc gcagaccctg ctcccggtat ggtttatagc    300
accaccgcaa gtggtcttgc tttttactat cttaacgaat atgtcgatgt gggaacaaaa    360
atatctgtgc taaatgcggg gtatacggca gttccttttg aacatgtttc caaccaggca    420
actacaacag atcacacttg tcagggaaac aaaactacag cggttggcgt gagcctgaaa    480
actggagcag atgcgaagat ttcatttcgt attaaacgtt caataaatgg aacggtagta    540
atacctatca ccgatattgc attgctgtat gccaacatat ccagcaccac gacccgtggt    600
gaggcgattg caaaagttcg aatttcaggc agtttgaccg caccacagtc ttgtcagata    660
aatgcaggac aggtgattta ttttgatttt gatactattc ctgcgtccga atttcatct     720
accgccgggc aagccattac ttcacgaaaa atcactaaaa cagtgagtat tgagtgtacg    780
gggatggggt atgagcgtac gcagaaagtc gatgcttctt ttacggggac gaaccgaagc    840
agtgacgata cgatggtggc gacagacaat gctgatgtcg ggatcaaaat ttacaataaa    900
tcgaatgctg aagttagcgt caacaacggc aagttacccg cagacatggg caacacgacc    960
atttttggtc gtaaaaatgg ttcggtaact ttttcggcag cacctgccag ctttaccggt   1020
gcccggcctc agcccggcgt ttttaacgct accgcgacct taaccattga atttgtaaac   1080
taa                                                                 1083
```

<212> Type : DNA
<211> Length : 1083
SequenceName : SEQ ID 391
SequenceDescription :
Sequence
--------
<213> OrganismName : Escherichia coli 0157:H7
<400> PreSequenceString :

```
atgtcacgtt ataaaacagg tcataaacaa ccacgatttc gttattcagt tctggcccgc     60
tgcgtggcgt gggcaaatat ctctgttcag gttctttttc cactcgctgt cacctttacc    120
ccagtaatgg cggcacgtgc gcagcatgcg gttcagccac ggttgagcat gggaaatact    180
acggtaactg ctgataataa cgtggagaaa aatgtcgcgt cgtttgccgc aaatgccggg    240
acatttttaa gcagtcagcc agatagcgat gcgacacgta attttattac cggaatggcc    300
accgctaaag ctaaccagga aatacaggag tggctcggga aatatggtac agcgcgcgtc    360
aaactgaatg tcgataaaga tttctcgctg aaggattctt cgctggaaat gctttatccg    420
atttatgata cgccgacaaa tatgttgttc actcaggggg caatacatcg tacagacgat    480
cgtactcagt caaatattgg ttttggctgg cgtcattttt caggaaatga ctggatggcg    540
ggggtgaaca cctttatcga ccatgattta tcccgtagtc atacccgcat tggtgttggt    600
gcggaatact ggcgcgatta tctgaaactg agcgccaatg gttatattcg ggcttctggc    660
tggaaaaaat cgccggatat tgaggattat caggaacgcc cggcgaatgg ttgggatatc    720
cgcgcagagg gctatttacc tgcctggccg cagcttggcg caagcctgat gtatgaacag    780
tattatggcg atgaagtcgg gctgtttggt aaagataagc gccagaaaga cccgcatgct    840
atttctgccg aggtgaccta tacgccagtg cctcttctga cactgagcgc cgggcataag    900
cagggcaaga gcggtgagaa tgacactcgc tttggcctgg aagttaacta ccgaattggc    960
gaacctttgg cgaaacaact cgatacggat agcattcgcg agcgtcgggt actggcaggc   1020
agccgctatg acctggttga gcgtaataac aacatcgttc ttgagtaccg caaatctgaa   1080
gtgatccgta ttgctctgcc tgagcgtatt gaaggtaagg gcggtcagac actttccctg   1140
gggcttgtgg tcagcaaagc aactcacgga ctgaaaaatg tgcagtggga agcgccgtca   1200
ttactggctg aaggtggcaa aattaccggt cagggtagtc agtggcaagt aacgctcccg   1260
gcttatcgtc caggcaaaga caattattat gcgatttcag cagttgccta cgataacaaa   1320
```

```
ggcaatacct caaaacgcgt gcagacagag gtggtcatta ccggagctgg tatgagcgcc    1380
gatcgcacgg cgttaacgct tgacggtcag agccgtattc aaatgcttgc taacggtaat    1440
gagcaaaaac cgctggtgct gtctctgcgc gacgccgagg gccagccagt cacgggcatg    1500
aaagatcaga tcaagactga actaactttc aaaccggctg gaaatattgt gactcgttcc    1560
ctgaaggcca ctaaatcaca ggcaaagcca acactgggtg agttcaccga aactgaagca    1620
ggggtgtatc agtctgtctt tactaccgga acgcagtcag gtgaggcaac gattactgtt    1680
agcgttgatg gcatgagcaa aaccgtcact gcagaactgc gggccacgat gatggatgtg    1740
gcaaactcca ccctgagcgc taacgagccg tcaggtgacg tggttgctga tggtcagcaa    1800
gcctatacgt tgacgttgac tgcggtggac tccgagggta atccggtgac gggagaagcc    1860
agccgcttgc gatttgttcc gcaagacact aatggtgtaa ccgttggtgc catttcggaa    1920
ataaaaccag gcgtttacag cgccgcggtt tcttcgaccc gtgccggaaa cgttgttgtg    1980
cgtgctttca gcgagcagta tcagctgggc acattacaac aaacgctgaa gtttgttgcc    2040
gggccgcttg atgcagcaca ttcgtccatc accctgaatc ctgataaacc ggtggttggg    2100
gggacagtta cggcaatctg gacggtaaaa gatgcctatg acaaccctgt gaccagcctc    2160
acgccggaag cgccgtcatt agcgggtgcc gctgctgaag gttctacggc atcgggctgg    2220
acaaataatg gtgatgggac gtggactgcg cagattactc tcggctctac ggcgggtgaa    2280
ttagaagtta tgccgaagct aaatggacag aatgcggcag caaatgcggc aaaagtaacc    2340
gtggtggctg atgcgttatc ttcaaaccag tcgaaagtct ctgtcgcaga agatcacgta    2400
aaagccggcg aaagcacaac cgtgacgctg gtggcgaaag atgcgcatgg caacgctatc    2460
agtggtcttg cgttgtcggc aagtttgacg gggaccgcct ctgaaggggc gaccgtttcc    2520
agttggaccg aaaaaggtaa cggttcctat gttgctacgt tgactacagg tggaaagacg    2580
ggcgagcttc gcgtcatgcc tctcttcaac ggccagccag cagccaccga agccgcgcag    2640
ttgacggtca tcgccggaga gatgtcatca gcgaactcta cgcttgttgc ggacaataag    2700
gctccgaccg tcaaaacgac gacggaactc accttcaccg tgaaggatgc gtacgggaac    2760
ccggtcaccg ggctgaagcc agatgcacca gtgtttagcg gtgccgccag cacggggagt    2820
gagcgtcctt cagcaggaaa ctggacagag aaaggtaatg ggtctacgt gtcgacctta     2880
acgctgggat ctgccgcggg tcagttgtct gtgatgccgc gagtgaacgg ccaaaatgcc    2940
gttgctcagc cactggtgct gaacgttgca ggtgacgcat ctaaggctga gattcgtgat    3000
atgacagtga aggttaataa ccaactggct aatggacagt ctgctaacca gataaccctg    3060
accgttgtgg acacctatgg taacccgttg caggggcagg aagttacgct gactttaccg    3120
cagggtgtga ccagcaagac ggggaataca gtaacaacta atgcggcagg taaagcggac    3180
attgagctta tgtcaacggt tgcgggagaa cacaatattt ccgcttcggt gaatggtgct    3240
cagaagacgg tcacggtgaa attcaacgcg gatgccagca ccggtcaggc aaacctgcag    3300
gtagacgccg ctgctcaaaa agtggcaaac ggcaaagatg cctttacgct gacggcgaac    3360
gttgaggata aaaatggtaa ccctgttcca gggagcctgg tgacctttaa tctgccccgg    3420
ggtgtcaagc cgcttacagg cgataatgtc tgggtgaaag ccaacgatga ggggaaagca    3480
gagttgcagg tggtttcagt gactgccgga acgtatgaga tcacggcatc ggcagggaat    3540
agccagcctt cgaatacgca gactataacg tttgtagccg ataaggctac cgcaaccgtc    3600
tccggtattg aggtgattgg caactatgca ctggcggacg gcaatgccaa acagacgtat    3660
aaagttacgg tgactgatgc caataacaac ctgttgaaag atagcgaagt gacgctgact    3720
gccagcccgg caaatttagt tctgactccc aatgggacgg cgaaaactaa tgagcaagga    3780
caggctattt tcaccgccac gaccactgtc gcagcgaaat atacactcac ggcgaaagtg    3840
agtcaggccg acggtcagga atcgacgaaa actgccgaat ctaaattcgt cgcggatgat    3900
acaaatgcag tactcaccgc atcatctgat gtgacttctc tggtggcgga tgggatatcg    3960
actgcgaagc tggaggtgac actgatgtcg gcaaataacc ccgttggggg gaatatgtgg    4020
gtcgacatta agacgccaga aggggtgacg gagaaggatt atcagttcct gccgtcgaaa    4080
aatgaccatt tcgtgagcgg aaaaatcacg cgtacattta gtaccagcaa gcctggtgtc    4140
tatacgttca catttaacgc cctgacgtat ggcgggtacg aaatgaagcc agtgacggtg    4200
accattaccg cggtggatgc cgatacggca aagggcgagg aggcgatgaa ctaa          4254
```

<212> Type : DNA
<211> Length : 4254
SequenceName : SEQ ID 392
SequenceDescription :
Sequence
--------
<213> OrganismName : Escherichia coli 0157:H7
<400> PreSequenceString :

```
atggcgcgtg gttgggcgtc ttcagaagcc tcaggcgcga tgactgattg gttaaataac      60
tttggtactg cgagaatctc tctgggtgtg gatgaagatt ttagcctgaa aaattcgcaa     120
ttcgacttcc tgcatccgtg gtatgacaca cctgattatc tgctcttcag ccagcatacc     180
cttcaccgaa cagacgatcg tacccagatc aacaccggtt tgggctggcg tcatttcacc     240
tccagctgga tgtcaggcat caaccttttt tttgaccacg acctgagccg ctatcactcc     300
cgcgcagggc ttggcgcaga atactggcgt gattatctga agttgagcag caacgcttat     360
atcggcctga ccggctggcg tagcgcacca gaattggata acgacttcga agcccgcccg     420
gccaacggct gggatttacg cgcggaaggc tggttacctg cctggccaca actgggggga     480
```

aaactggtct atgaacaata ctatggcgat gaagtggcgc tgtttgacaa gaatgatcgt 540
caaagtaacc cccatgctat taccgcaggc ctcaactata ccccttccc gcttctgact 600
ctcagtgcgg aacagcgtca ggggaagcaa ggtgaaaatg acacacgttt tgccgttgat 660
ctgacctggc aacccagcag ttcaatgcag aaacagctta atccggacga agtggccgga 720
cggcgcagtc tggccggtag tcgttatgac ctgattgatc gcaacaacaa catcgttctg 780
gaataccgca agaaagagct gattcgcctg agtctgctgg atccggtgaa agggaagtct 840
ggagaaataa aaccgctggt ttcctcgcta cagaccaaat atgcccttaa aggctataac 900
atcgaagccg ctgcgctgga agctgccgga ggtaaagtca gcacgtctgg aaaagatatc 960
acggtcacgc tgccaggtta ccgcttcact aacaccccag aaaccgataa tacatggtcg 1020
atagacgtta ccgccgagga tgtaaaaggt aacctgtcac ggcatgaaca aagcatggta 1080
gttattcagg ctccgacatt aagccagaaa gattctctgt tatccgtcaa tccgctaacc 1140
gtggctgcag ataaaaaatc gacgaccaca ttgaccgtta ctgcgcacga ttccgacgga 1200
actccggtgc cggggctggc gctgcaaacc cgcagtgaag gcgttcagga tatcaccctg 1260
tctgactgga cagataacgg tgatggtagt tacacacaga tactgaccgc cggaacgaca 1320
tcaggttcag taacactgac gccgcaaatt aacggtgaga gtgcggtaaa agaatccatc 1380
gtcgttaata tcgtccctgt tgtctcatcc cgcgaccatt catcaataac aattgataac 1440
gtatcgtatt atgccggaga cgacatcaag gttagggtgg aactgaaaga cgatagcaat 1500
caaccggttg catatcaaaa agaggaattg gtaaaagccg ttactgtcga aaacagcaaa 1560
cctggcgcca cgattgtctg gcacgaagag cagccggggg tttatgccgc gaattatccg 1620
gcctataagc aagggactgc actaagggca caacttagcc ttcacaactg gaatgctcca 1680
ctgcaatcgc atatttataa cattgaggca aaccagaata aggctcgcgt tgccacatta 1740
tcagcgacaa ataatgacgt ttacgccgat aaaaagacat ttaataccct cacgatcaac 1800
gtcactgatg agagtgataa tcccctgaca aatcatcagg tcacctttaa gaatgaaaaa 1860
ggaagcgcgg agtttgtcga accgccgcag caaaatacgg atgcatatgg tgttgccaca 1920
ataaacatgg taagtcaggt tgcggaagaa aatacgatta gcgccacgct gccaaatggt 1980
ttttcacaac ggataattgc gaaattcgtt agcgattcga gtacgccaaa attcaaacaa 2040
ctggttgccg atccagatac cattattgct ggcaacagcc agggcagtac tctgaccgcc 2100
atcatcacag actttcataa caacccgtta aaagatatga aagtgaattt tgtggcacct 2160
ggtggctcgc aactggacaa cacgaccgcc acaacagacc agtccggtat tgtgcgggtg 2220
cacctgacca gttcaaaagc tggtagctat tccgtcgatg cctcgcttga ggtggataaa 2280
aatattcacc agtcggtcac gatcaccgtg gtcccaaaca gggaacaatc ggtaatgacc 2340
ttgaatgccg ggtcgggcag tgcgatcgct aacaatacaa atatcgttac cctgactgcc 2400
agtgtgaaag atgtttatgg acacccgttg ccggatgagg atgtgaaatt taccttgcca 2460
gcctccatga ccgggaactt cacgctaagt agtgaaaccg cccgcaccga tgcaaacggt 2520
gatgccgtgg tcacattgcg aggcacaaaa gcgggtgagt ttacagttac ggcgacgctg 2580
accagaaata ataccgttgc ttatcagcaa gtcactttta ttggggatac aaacagtgcg 2640
cagctccagc cgctgactgc ctcattaaat tccattgttg cgggtaacag tacggggagt 2700
accctgacgg caacgatcct ggacgcttac caaaatccgc ttaaagacca gttggtcact 2760
ttccagagta acgatgtcac tctaagcgaa acagaagtca ccaccaatac gctgggtcag 2820
gcgacggtaa caatgaccag caatattgcc ggacaacata acgtcgtggt gagccggaaa 2880
gcgcaagctt ccgataataa aacgtttagt ttatcagtgc taccggatga aagttcggcg 2940
aaggtaataa gtataaccgg agccgaaaaa acgataacgg tgggcgaaaa catcacgcta 3000
cggatactcg tccaggacgc gtttaacaat gtaatcgcgg gtcaacgcgt cagattaagt 3060
gcgcagccaa caactaacat tacgataggc gatacggctt acaccgataa taacggttat 3120
gcgtacgtta accttctcag cacccaacct ggggtttatc aggtgacggc aacgctggac 3180
aataacagta gtagtaaggt tgacgtgaat gtggcaaatg gcaaactcga gttaacatca 3240
tcgaaaccag aaactacggt ccataatagt gagggtatta cgctgaccgc aacggcgaga 3300
aatgcgcggg gtgaattgat gccagggcaa attatcacct ttagcgtaac gcctgaaggt 3360
gcaacgctaa gcaatacagg ggaagtcctt actgaccagt caggtcaggc caaagtgacg 3420
ctgaccagtg acaaagtgaa tgtctatacc gttacggcca taatgggcaa agatgttccc 3480
gttcagagcc aggtaacggt tgcggttaag gcagatgcta aacggcaca tgttgtgagc 3540
gtcgtggctt ctcctgacac catcaccgcc gacggcatcg atagcagcac catcacttca 3600
cgagtagaag atgattacgg attcccggtt gaaggtgtcg atattagtca tggcttagac 3660
accaaaggca gcccggtagt taatattcca actacgcgta ccgatcagtc cgggcaagtc 3720
acggcgacaa taaccagtac attggcagaa accttaacag tcaatgtgca agttcctggc 3780
acagccaacc aatccgcaac cattacattg gttgccggca cggccgatga aagtaagtca 3840
atttgaaat ccgatgttga cactctgaag gctgactacc agcagagcgc aaaacttacg 3900
ctaacattgc aagacaagta cggtaacccg atagtgacgt ctgatcatct ggaatttgtc 3960
cagtcaggcc ccttcgtgaa ctttctcaag ttgagcgata ttgattacag ccaaagaaat 4020
tatggcgagt acaccgtgac tgtcactggc ggaaaagagg gaacagcgac actcattccc 4080
atgctgaacg gggttcatca ggcaaactta agcatatcgc tgaatctcat ccaatcgata 4140
aaagaaatgt ccggtcatgt cactgcaaac aaccatacct tctccacggc taaattcccg 4200
agcgaaggct ttgcaggagc gtattacaca ctcaacaatg ataactttga agcgggtaaa 4260
accgttgatg attatatgtt ttcaagttca cagggttggg tgtctgtcga tgcttcgggt 4320
aaagtttctt tcgcaaatat cggcgatcaa acgtcagtca caataagcgc tgttccccga 4380
caaggaggta caacctacca gaccttaatt aagctgaaag gctggtgggt gaataatgga 4440
aatcatacca atatctggct agctgccaat gcgctctgtc atgctaaaaa tgatggatat 4500

```
aatcttcctg gcatcacaca tttgacgtct ggcgaaaaca aacgcacgca gggatcactg    4560
tatggtgaat gggggaacgt tggagcgttt tccagtaatt cgcaatttac accgggagct    4620
tactggacaa gtgaatctga tgattacagt cggcactact atgtgcagat gctaaccggt    4680
atgaccggaa gcgacgctga ttccagcccc caactgaccg cctgccgtaa atcactttaa    4740
```

<212> Type : DNA
<211> Length : 4740
SequenceName : SEQ ID 393
SequenceDescription :
Sequence
--------
<213> OrganismName : Escherichia coli 0157:H7
<400> PreSequenceString :

```
atgattactc atggttgtta tacccggacc cggcacaagc ataagctaaa aaaaacattg      60
attatgctta gtgctggttt aggattgttt ttttatgtta atcagaattc atttgcaaat     120
ggtgaaaatt attttaaatt gggttcggat tcaaaactgt taactcatga tagctatcag     180
aatcgccttt tttatacgtt gaaaactggt gaaactgttg ccgatctttc taaatcgcaa     240
gatattaatt tatcgacgat ttggtcgttg aataagcatt tatacagttc tgaaagcgaa     300
atgatgaagg ccgcgcctgg tcagcagatc attttgccac tcaaaaaact tccctttgaa     360
tacagtgcac taccactttt aggttcggca cctcttgttg ctgcaggtgg tgttgctggt     420
cacacgaata aactgactaa aatgtccccg gacgtgacca aaagcaacat gaccgatgac     480
aaggcattaa attatgcggc acaacaggcg gcgagtctcg gtagccagct tcagtcgcga     540
tctctgaacg gcgattacgc gaaagatacc gctcttggta tcgctggtaa ccaggcttcg     600
tcacagttgc aggcctggtt acaacattat ggaacggcag aggttaatct gcagagtggt     660
aataactttg acggtagttc actggacttc ttattaccgt tctatgattc cgaaaaaatg     720
ctggcatttg gtcaggtcgg agcgcgttac attgactccc gctttacggc aaatttaggt     780
gcgggtcagc gtttttttcct tcctgcaaac atgttgggct ataacgtctt cattgatcag     840
gatttttctg gtgataatac ccgtttaggt attggtggcg aatactggcg agactatttc     900
aaaagtagcg ttaacggcta tttccgcatg agcggctggc atgagtcata caataagaaa     960
gactatgatg agcgcccagc aaatggcttc gatatccgtt ttaatggcta tctaccgtca    1020
tatccggcat taggcgccaa gctgatatat gagcagtatt atggtgataa tgttgctttg    1080
tttaattctg ataagctgca gtcgaatcct ggtgcggcga ccgttggtgt aaactatact    1140
ccgattcctc tggtgacgat ggggatcgat taccgtcatg gtacgggtaa tgaaaatgat    1200
ctcctttact caatgcagtt ccgttatcag tttgataaat cgtggtctca gcaaattgaa    1260
ccacagtatg ttaacgagtt aagaacatta tcaggcagcc gttacgatct ggttcagcgt    1320
aataacaata ttattctgga gtacaagaag caggatattc tttctctgaa tattccgcat    1380
gatattaatg gtactgaaca cagtacgcag aagattcagt tgatcgttaa gagcaaatac    1440
ggtctggatc gtatcgtctg ggatgatagt gcattacgca gtcagggcgg tcagattcag    1500
catagcggaa gccaaagcgc acaagactac caggctattt tgcctgctta tgtgcaaggt    1560
ggcagcaata tttataaagt gacggctcgc gcctatgacc gtaatggcaa tagctctaac    1620
aatgtacagc ttactattac cgttctgtcg aatggtcaag ttgtcgacca ggttggggta    1680
acggacttta cggcggataa gacttcggct aaagcggata acgccgatac cattacttat    1740
accgcgacgg tgaaaaagaa tggggtagct caggctaatg tccctgtttc atttaatatt    1800
gtttcaggaa ctgcaactct tggggcaaat agtgccaaaa cggatgctaa cggtaaggca    1860
accgtaacgt tgaagtcgag tacgccagga caggtcgtcg tgtctgctaa aaccgcggag    1920
atgacttcag cacttaatgc cagtgcggtt atattttttg atcaaaccaa ggccagcatt    1980
actgagatta aggctgataa gacaactgca gtagcaaatg gtaaggatgc tattaaatat    2040
actgtaaaag ttatgaaaaa cggtcagcca gttaataatc aatccgttac attctcaaca    2100
aactttggga tgttcaacgg taagtctcaa acgcaagcaa ccacgggaaa tgatggtcgt    2160
gcgacgataa cactaacttc cagttccgcc ggtaaagcga ctgttagtgc gacagtcagt    2220
gatggggctg aggttaaagc gactgaggtc actttttttg atgaactgaa aattgacaac    2280
aaggttgata ttattggtaa caatgtcaga ggcgagttgc ctaatatttg gctgcaatat    2340
ggtcagttta aactgaaagc aagcggtggt gatggtacat attcatggta ttcagaaaat    2400
accagtatcg cgactgtcga tgcatcaggg aaagtcactt tgaatggtaa aggcagtgtc    2460
gtaattaaag ccacatctgg tgataagcaa acagtaagtt acactataaa agcaccgtcg    2520
tatatgataa aagtggataa gcaagcctat tatgctgatg ctatgtccat ttgcaaaaat    2580
ttattaccat ccacacagac ggtattgtca gatatttatg actcatgggg ggctgcaaat    2640
aaatatagcc attatagttc tatgaactca ataactgctt ggattaaaca gacatctagt    2700
gagcagcgtt ctggagtatc aagcacttat aacctaataa cacaaaaccc tcttcctggg    2760
gttaatgtta atactccaaa tgtctatgcg gtttgtgtag aataa                    2805
```

<212> Type : DNA
<211> Length : 2805
SequenceName : SEQ ID 394
SequenceDescription :

Sequence
--------
<213> OrganismName : Escherichia coli 0157:H7
<400> PreSequenceString :

```
atgttagtac ttagcgaaag cttcaagaat aaattgcttc ccatgaatgg gtatatgaaa    60
ggcggcagcg actccggatc taaagcccag gcacgcgcaa ctgaaaaggg catcgaactg   120
cagcgtgaaa tgtggcagac gaacatgcaa aaccttgcac cgttcacgcc actcgctcag   180
cagtacgtat cacagttgca gaatctttcc tctcttcagg ggcaaggtca ggcgcttaac   240
cagtattaca actctcagca gtataaagac cttgcagggc aggcgcgcta tcagagtctg   300
gcagcagcag aggcaacggg tggattaggc tctacagcaa caggaaacca gttagcagca   360
atcgcaccta cactcggtca aaactggctg tcaggtcaga tgaacaacta caacaatctg   420
gcaaatatcg gccttggtgc tcttacaggt caggcaaacg ccggacagaa ctacgctaac   480
aatgtcagcc aattgtatca acagcaggcg gcagcatcgg cagcaaatgc gaataagcct   540
tcaggcctac agagttttgc tacaggtgcc attggtgggg ccgcatcagg tgcaatgatt   600
ggtagtgcag ttcctgttat tgggactggt attggtgctc ttgctggcgg tgttatcggt   660
ggtcttggat cattgtttta a                                             681
```

<212> Type : DNA
<211> Length : 681
SequenceName : SEQ ID 395
SequenceDescription :
Sequence
--------
<213> OrganismName : Escherichia coli 0157:H7
<400> PreSequenceString :

```
atgaaaaaaa tattatcagg gttgattctg ctgctttgct gtccttatgg tttcgccgct    60
aatggtgatg gcgcaacgca catgtcaaat ttatcatttg gtccgctgac ggtggcagcg   120
gcgaataacc actccggata caatattttc gaggcactga gcaacacgac tggaacatac   180
ccggtgcgct gtcactgtga tgacacgcat ggcggaccgg gccaacaaac agcatttttt   240
cctatcttct acacgggaga tgccgcaccg gggcttgtgc ttgagcgcac tcttaatggg   300
ttaaattact atgctctgaa tgattattta tcggtcggcg tgacgatttt tattattaat   360
aaccaatatg cggccattcc ttttgaacac ttatccaacc aatccacctc accgcaacat   420
acctgtggag caggtaataa tgggagcact gtaaatctgg attcagggcg ctcggcaaaa   480
ttatctttct atgttcggca ttctattact ggcacggtga caatacccac aacggaagtc   540
gcctggttgt acgcgggcat gtccgatcat tttcccaaaa cgacccccgt ttctaaagtg   600
acaattcgcg gacaactaac ggccccgcag aactgtgagt taacgccaaa tcagagcatc   660
gatgtcgatt ttcaaaaaat taatagcgct gagttctcct caacggcggg ttcaattatt   720
gcagaaagaa agattaaaac cgaagtcaca gtatcctgta ccgggatgga agacgtaagg   780
tccacggagg tggtgagtgc gtcgatgatt gcggcaaaca gaagtgccga tgccaccatg   840
atcgtgacga gtaatccgga tgtgggaatt aagattttg ataagaacga ccgtccagtg   900
aatgtggatg ggggcaactt acccgctgat atgggtgcta ttagtcgatt aggaaaaacc   960
gatggtagcg taacgtttta ttcagcgccc gccagtctga cgggcgcaaa accagcgcct  1020
gataatggat ttaccgctac agccacgctg gttattgaat ttactaacta a           1071
```

<212> Type : DNA
<211> Length : 1071
SequenceName : SEQ ID 396
SequenceDescription :
Sequence
--------
<213> OrganismName : Escherichia coli 0157:H7
<400> PreSequenceString :

```
atgaataaaa tatatcggct aaagtggaac aggtcccgta actgttggag cgtctgctcg 60
gagctgggga gcagagtaaa aggaaaaaag tcccgggctg ttttaattag cgcgataagt 120
ttatattcat ctctggtatt cgccgatgat gtcatcgtaa accaggataa aactattgat 180
tttggcaaag agaaccagag catcgattac cgtattacgg tgacagacaa tgccaatctg 240
gtaatcaatg cgacagatac ttcccgtccg cgtctgactc tcgcttctgg tggtgggttg 300
gatattaccg gaggaaaggt aacaatcaat ggcccactta actttttgct gaaaggtacg 360
gggttcctga atgtctccaa tgctggcagc gagttatatg ctgatgattt gtatgaatca 420
aactcaggca tgagacacga tcgcggctat tttaatgtct ccaacggcgg caaaatccat 480
gttaagggca ccagccgtct gacctatttg cagggaaatg tcagtggtga aggtagccag 540
gtaaattccg aaaccttctt tatgggcgtt tacggcagtt acggtggtaa tcagtacctg 600
tcagttaata acggcggtga agttaatgcc aggaagcaaa ttagcctggg ctattatgat 660
caagtctccg atacaacact tgctgtttcg gaaggtggta aaatttctgc gcctactatt 720
agtttaagca ccaactctga gttagcgtta ggggcacagg aaggaagcgc agcgaaggca 780
gcagggatta ttgatgccga aaaaattgag tttgtgtggg caaagacatc cgagaagaaa 840
atcaccttaa accacacgga taaagacgcg actatttccg cggatattgt cagtggcagc 900

gagggcctgg gctatatcaa tgcgctcaat ggcacgactt acttaaccgg tgataactct 960
gcctttagtg gtaaagtcaa aattgagcaa aatggcgctt tagggatcac ccaaaatata 1020
ggtacagcag agatcaacaa ccgcgggaaa ttacacctga aggctgacga tagcatgacc 1080
tttgccaata agatctctgg caacggtaca ataagtatcg acagtgggac ggtggagttg 1140
accggcaata actatgcatt cagcggatat attgatgttg cttctggtgc tgtcgctgtt 1200
atttctgaag acaagaatat cggtcgtgca gagctggatg tcgatggcaa attgcaaatt 1260
aatgccaaca aagattgggt atttgataac gatcttgaag gtagaggcat tgttgaaata 1320
aacatgggga atcacgaatt ctccttcgat gagtttgctt atacagactg gttccagggt 1380
tcactggcgt tccagaacac gacatttaat ctggaaaaga atgctgagtt tctgcagaaa 1440
ggcgggatca ctgcgggtca gggaagcctg gtaacagtgg gtaagggcgc tcactccatt 1500
agcactttgg gattctccgg cggaaccgtt gattttggtg ccctgacagc aggtgcacag 1560
atgacagaag ggacggtcaa cgttagtaaa acgctggatt tgcgcggcga gggtgtgatt 1620
caggtttctg acagtgacgt tgtccgctca gtatctcgtg atattgactc tgcgttatcg 1680
ctcactgaag tcgatgatgg taacagcacc attaagttgg ttgatgcgca aggtgcggaa 1740
gttctgggcg atgcgggcaa tctgcaattg caggataaaa atgggcaaat cctctccagc 1800
agcgcccaac gtgatattca gcagaatggg caaaaagcgg ccgtcggcac ttacgactat 1860
cgtctgacga gtggggtaaa caatgacggt ctgtatattg gttacggcct gacccagctt 1920
gatttacacg ctaccgacag cgatgctctg gtgctgagct ctaacggtaa aagcgagaat 1980
gccgccgatc tcagcgcaaa gattaccggc agtggtgacc tggcattcag cagccagaag 2040
ggtcagaccg tatcgctttc taacaaagac aacgactata ccggtgttac cgatctgcgc 2100
agtgggacgc ttttgttgaa taacgataac gtgttgggta atacccatga actgcgtctg 2160
gcggcagaga ctgaactgga catgaatggt cacagccaga ccgtgggcac gctcaatggc 2220
agcgccgatt cactgctgag cttaaatggc ggcagtctga cggttaccaa cgggggcact 2280
tcaaccggtt cgttaacggg gagcggagag ctgaatattc agggcggcac gctggacatc 2340
gcgggcgata acagcaacct gacggcgaat gtgaacattg ctaattcggc taatgtcctg 2400
gtaagtcatg cgcagggatt gggtagcgca aacgttgaga acaacggtac cctggcgttg 2460
aataatagcg ctgaaaaaag agcggctgcg tctgtgaatt acgccctggg cggcaatctg 2520
accaacaacg gtacgctgat gaccggaatg tcaggacagc aagctggcaa tgtgttagtg 2580
gtgaagggga actaccacgg taataacggt caactagtaa tgaatacggt actgaatggc 2640
gatgactcag taaccgataa attggttgtc gagggcgata ctagcggcac gactgccgtt 2700
acggtgaata acgctggcgg tacaggtgcg aaaaccctta acggtatcga acttatccat 2760
gtagacggta agtctgaggg cgaatttgtt caggctgggc gtatcgttgc gggggcgtat 2820
gactacactc tcgcgcgtgg acaaggggca aatagtggta actggtatct gaccagcggc 2880
agtgattctc ctgaactgca gccggagcca gacccgatgc cgaatccaga gccaaacccg 2940
aatccagagc cgaaccctaa cccgacacct acgccgggtc cggatctgaa tgtggataat 3000
gacctgcgac cggaggcggg tagctacatt gcgaaccttg cagcagcgaa taccatgttc 3060
accacgcgtc tgcatgagcg tctgggtaat acgtactata ccgacatggt gacgggtgag 3120
cagaaacaaa ccactatgtg gatgcgccat gaaggtggtc ataataaatg gcgtgatggc 3180
agcggccagc tgaaaaccca aagcaatcgc tatgttctgc aactgggagg cgatgtcgcg 3240
cagtggagcc aaaacggcag cgaccgctgg catgttgggg tcatggcggg atatggcaac 3300
agcgacagca aaaccatttc ctcgcgaacc ggttatcgtg caaaagcgag tgtgaacgga 3360
tatagcacag gcctctatgc cacctggtat gccgatgacg agtcgcgtaa tggcgcgtat 3420
ctcgacagtt gggcgcagta cagctggttt gataacacag tgaaagggga tgacttacaa 3480
agtgaatcct ataaatcaaa aggatttacc gcttcactgg aagctggata caaacacaaa 3540
ttagctgaat ttaatggcag ccagggaacg cgtaatgaat ggtatgttca gccgcaagca 3600
caggttacct ggatgggagt caaagccgat aagcaccgcg aaagcaacgg aaccctcgtt 3660
catagcaacg gtgatggcaa tgttcaaacc cgacttggcg taaaaacctg gctgaagagc 3720
caccataaaa tggatgacgg taaatcccgc gagttccagc cgtttgtaga agtgaactgg 3780
ctacataaca gtaaggattt cagcaccagt atggatggcg tgtctgtcac tcaggatgga 3840
gcccgaaata ttgctgagat aaaaaccggg gtggaaggac agctaaatgc caacctgaat 3900
gtctggggga atgtgggcgt tcaggttgcc gatagggggat ataatgacac ctctgcaatg 3960
gttggcatta agtggcaatt ctga 3984
```

<212> Type : DNA
<211> Length : 3984
SequenceName : SEQ ID 397
SequenceDescription :
Sequence
--------
<213> OrganismName : Escherichia coli 0157:H7
<400> PreSequenceString :

```
atgataacga tgaaaaaaag tgtattgacg gcgtttataa ctgtggtatg tgcaacgtcc    60
agcgttatgg ctgctgatga taatgctatc acggatggct cagtaacatt taatggtaaa   120
gttattgctc cagcttgtac cctggtagct gcgacgaaag attccgtggt gactttgcca   180
gatgttagtg ccacgaagtt gcaaaccaat ggtcaggttt ctggcgtgca aactgatgtg   240
ccaattgaat taaaagattg tgatactacc gtaacaaaaa atgcaacgtt cacctttaat   300
ggcactgcgg atactactca gattacagcg tttgctaacc aggcctcatc tgatgctgct   360

acaaacgtgg ccctgcaaat gtatatgaat gatggtacaa cggccatcaa gccagacaca   420
gaaaccggga acattttgtt gcaagatgga gatcagacgt tgacttttaa agttgattat   480
atcgctacgg ggaaagcgac ttctggtaat gtgaatgcgg taacaaattt ccatattaac   540
tattattaa                                                           549
```

<212> Type : DNA
<211> Length : 549
SequenceName : SEQ ID 398
SequenceDescription :
Sequence
--------
<213> OrganismName : Escherichia coli 0157:H7
<400> PreSequenceString :

```
atgagtaagt ttgtaaaaac agctattgct gcaacaatgg taatgggtgc gttcgcttct    60
acttcaacaa tcgccgctgg caacaatggt acagcacgtt tctacggcac cattgaagat   120
tccccgtgct ctatcgttcc tgatgatcac aaactggaag ttgatatggg tgacattggt   180
tcagggatcc tgaaaaataa cgggacttct acaccgaaag ctttccagat ccatctgcaa   240
gactgtgtgt ttgacaccca gacaacgatg accactacct tcaccggtaa cgcgtcttct   300
accaacagcg gcaattacta caccatttac aataccgata ctggtgcggc atttaacaat   360
gtcagcctgg ccattggtga cgctcaggga acctcttata aagcggcgc gggtatcgaa   420
cagaaaatcg taaacgatac ggcgaccaac aaaggcaaag cgaagcagac gctggacttt   480
aaagcctggc tggtgggcgc tgctgatgcg ccagatttag gtaattttga agccaacacg   540
accttccaga ttacttatct ctaa                                          564
```

<212> Type : DNA
<211> Length : 564
SequenceName : SEQ ID 399
SequenceDescription :
Sequence
--------
<213> OrganismName : Escherichia coli O157:H7
<400> PreSequenceString :

```
atgcgggtta tctttctacg caaggagtat ttatctctac tcccgtcaat gattgcatct     60
cttttctctg ctaacggtgt cgcggcggcc attgatttat gccagggata tgatatcaaa    120
gcgagttgtc acgccagcag gcaaagcctt tcaggcatta cgcaggtctg gagtattgcc    180
gatgggcaat ggctggtttt ttcggatatg accaataatg ccagcggtgg ggccgtattt    240
ttgcaacaag gagcggaatt tacattatca ccagaaaatg aaactggaat gactctgttt    300
gccaataaca ccgtttcagg agaatataat aacggcgggg caatatttgc taaagaaaac    360
tcaacgctga atcttacgga tgttattttt tctggtaacg tcgcaggcgg ctatggtggc    420
gcaatctatt cttctggtac taacgatacc ggtgccatcg atttacgtgt cactaacgcc    480
gtgtttcgca ataacatcgc taatgacggc aaaggtggtg caatttatac catcaataat    540
gatatctatt taagtgatga tgtttttaac aataaccagg catatacatc aacaagttac    600
agtgatggcg atggcggcgc aatcgatgtc acagataata atagcgacag caagcatcct    660
tcaggttata cgataataaa taacactgcc tttacaaata acactgccga aggttatggc    720
ggggcgatat ataccaatag cgcgacggct ccctatctta ttgatatttc tgttgatgac    780
agctacagcc agaacggagg cgtgttagtc gatgagaaca atagcgcagc aggctatgga    840
gatggtcctt cctctgcggc gggtggcttt atgtatctcg gcttaagtga agttaccttt    900
gatattgccg acggaaaaac gctggttatt ggcaatacag agaatgacgg agctgttgac    960
tctattgctg gtaccgggtt aatcaccaaa acaggttccg gcgatctggt acttaatgca   1020
gataacaatg actttactgg tgagatgcag attgaaaacg gtgaagttac cctgggccgc   1080
agcaactccc tgatgaatgt cggcgatacg cattgccagg acgatccgca agactgctac   1140
ggtctgacga tagggagtat tgataagtac cagaatcagg cagagctaaa tgttggctcc   1200
acccaacaaa cctttgcgca ctcattgacg ggctttcaga atggcacttt aaatatcgat   1260
gctggtggca atgttactgt taatcaaggc agttttgctg gcaccatcga aggtgctggt   1320
cagctcacca ttgcgcaaaa cggcagctat gtgctggcgg gggcgcagtc gatggcgcta   1380
accggcgata tagtggtgga tgctggtgcg gtgctttcgc tggaaggcga cgcggcagat   1440
cttgccgctc tccaggacga tccgcagtcg atcgtgttaa acggcggtat gctcgatctc   1500
tctgatttct ccacctggca gagcggtaca tcatacaaag atggccttga agtcagtggc   1560
agcagcggaa cggttatcgg cagtcaggat gtggtagatc ttgcaggcgg aaacgatatg   1620
catatcggcg gcgacgggaa agatggcgtc tacgtggtga tcgatgcggg tgacgggcag   1680
gtcagcctgg caaatgacaa tcaatacctc ggcacaacgc aaatcgcttc cggtacgctg   1740
atggtgagcg acaactcgca gcttggatat acccattata accgccaggt tatctttacc   1800
gataagccac aagaaagcgt gatggagatt actgccaatg tcgatactcg ctctacaacg   1860
actgagcatg ggcgtgatat tgaaatgcgc gccgacggtg aagtggcagt tgatgcgggg   1920
gtagacacgc agtggggcgc actgatggct gacagcagcg ggcagcatca ggatgagggt   1980
agcacattga ctaaaacggg ggcgggtaca ctggagctga ccgccagcgg tacaacgcag   2040
tcagcggtga gagtagaaga gggcacgctg caaggtgatg ttgcggatat cttcccttat   2100

gcttcgtcgc tatgggtcgg tgacggggca acgttcgtta ctggcgcgga tcaggatatt   2160
cagtcaattg atgctacttc cagcggcact atcgacatca gcgatggtac ggttttgcgc   2220
ctgaccgggc aggatacttc cgtcgccctt aatgcctcac tgtttaactg cgatgggacg   2280
ctggtgaatg ccaccgatgg tgtgacgttg acaggtgagc ttaataccaa ccttgaaact   2340
gacagcctga cttatctttc caacgtgacg gttaatggca atctgaccaa tacgtccggt   2400
gcggttagcc tgcaaaatgg cgtcgctggc gatacgctga cggtaaacgg tgattatacc   2460
ggcggcggta cgctactgct cgatagcgaa ttaaacggcg atgactcggt aagcgatcaa   2520
ttggtgatga acggtaatac tgctggcaac acaactgtgg tggttaactc cattacaggg   2580
attggtgagc cgacatcgac aggcattaaa gtggttgatt tcgcagctga tcccacgcag   2640
tttcaaaaca atgcgcagtt cagtctggca ggcagcggct acgtcaatat gggagcgtat   2700
gactacacgc tggtggaaga taacaacgac tggtatctgc gatcgcaaga agtaacgccg   2760
ccatcgccac ctgatccaga cccgactccc gatcctgatc ccacgcagga tcctgatcca   2820
acacccgacc cggaacctac gcctgcttac cagccggtgt tgaatgccaa agttggcggt   2880
tatctcaata acctgcgggc ggcaaatcag gcgtttatga tggagcgacg cgatcacgca   2940
ggtggcgatg gtcagacgct gaatttacgt gttatcggcg gagattatca ttacacagca   3000
gcggggcaac tggctcaaca tgaagacact tctacggtgc aacttagcgg cgatctgttt   3060
agcgggcgct ggggcacgga tggcgagtgg atgcttggga ttgttggtgg ctacagcgat   3120
aaccagggcg acagccgctc gagtatgacc ggaactcgcg ccgataacca gaaccacggt   3180
tatgcggttg ggctgacctc aagctggttt cagcacggta agcagaagca gggggcctgg   3240
ctggataact ggttgcagta cgcgtggttt agcaatgatg tttctgaaca tgaagatggc   3300
gtggatcatt accattcgtc ggggattatc gcctcgctgg aagcggggta tcagtggtta   3360
ccggggcgtg gtgtggtgat tgaaccgcag gcgcaggtga tttatcaggg cgtgcagcag   3420
gatgatttta ccgccgctaa ccgtgcgcgc gtgtcacaat cgcagggtga tgatattcag   3480
acgcggctgg gtttacacag cgaatggcgt accgctgttc atgtcatacc aacattagat   3540
ctgaattatt atcacgatcc ccattcgacg gaaattgaag aagatgccag cactatcagt   3600
gacgatgcgg tgaagcaacg gggtgaaata aaagtgggag tcacgggcaa tatcagtcag   3660
cgagtttcgc tgcgtggtag cgtggcgtgg cagaaaggga gtgatgattt tgcccagacg   3720
gcagggtttt tgtcgatgac ggtgaaatgg taa                                3753
```

<212> Type : DNA
<211> Length : 3753
SequenceName : SEQ ID 400
SequenceDescription :

Sequence
--------
<213> OrganismName : Escherichia coli 0157:H7
<400> PreSequenceString :

```
atgcactcct ggaaaaagaa acttatagta tcacaattag cattggcttg cactctggct     60
atcacctctc aggctaatgc agcgaccaac gatatttctg gtcaaactta caatactttc    120
catcactaca acgacgccac ctatgctgat gacgtttact atgatggtta tgtaggctgg    180
aacaactatg ccgctgatag ctattacaac ggcgatatct acccggtcat taataacgct    240
accgttaacg gcgtgatttc tacctactat ctggacgacg gtatttctac caataccaac    300
gccaatagtc tgacaatcaa aaacagcact attcacggta tgattacctc tgagtgcatg    360
actactgatt gtgctgatga ccgtgctact ggttatgttt atgatcgtct gacactgagc    420
gttgataatt caacgatcga tgacaactac gagcattata cttacaacgg tacctataat    480
aatgccgctg acactcatgt tgtagatgtt tacgatatgg gtactgctat tacactggat    540
caggaagttg atctgtccat cactaataac tctcatgtag caggtattac gctgactcag    600
ggttatgagt gggaagatat tgacgacaac acagtcagca ctggcgtaaa cagcagcgaa    660
gtgtttaata acactattac tgttaaagat tctactgtga cctctggttc atggactgat    720
gaaggtacta ctggttggtt tggccatact ggtaatgcca gcaactatag caacacgctg    780
actgcagacg atgttgcaat tgccgcaatc gcaaatccgt atgctgataa tgcgatgcag    840
actacagtaa ctttagacaa ctcaacactg atgggtgatg ttgttttctc cagtaatttc    900
gatgaaaact tcttcccgca aggtgctaac agctatcgcg atgctgatgg tgatgtagat    960
accaacggtt gggatggcac agaccgtatg gatgtgactc tgaacaacgg cagcaagtgg   1020
gttggcgctg caatgtctgt tcatatggtt gatgaagatg gtgatggttc ttacgacgga   1080
tatgctgttg gtactgaagc aactgcaact ctgctcgata ttgcagctaa cagcctgtgg   1140
ccttcatcaa ctgtcggtgt tgataacatc aatactcaat atgacgaaaa tggccatatc   1200
gtaggaaacg aagtttacca gagcggtttg tttaatgtga ctttgaacgg tggttcagag   1260
tgggatacaa caaaatcttc tctgattgat actttaagta ttaacagcgg ttcccaagtt   1320
aatgttgcag actctcgtct gatctctgac actgtctctc tgactggcgg ttctaacctg   1380
aacatcggtg aagacggtca tgtagcgact aataccctga ccatcgacaa tagtaccgtt   1440
aaaatgtctg atgatgtttc tgcgggctgg ggtttagaag atgctgcact gtacgcaaat   1500
accatcaccg taactaacga cggtctgttg gatattaacg ttgatcagtt cgatgctaac   1560
ccgttccagg ccgataccct gaatctgacc agtaccactg atactaacgg caacattcac   1620
gctggtgtat tcgatatcca tagcagtgat tacgtaatgg ataccgatct ggtcaacgat   1680
cgtaccaacg atactaccaa gtcaaactac ggttatggct taatcgcaat gaactctgat   1740
ggtcacctga ctattaacgg taacggcgat aacgacaaca ctgcttctat cgaagctggt   1800

cagaacgaag ttgataacaa cggtgaccat gttgcagccg cgaccggtaa ctacaaagtt   1860
cgtatcgaca acgctactgg tgctggttct atcgctgact acaacggcaa cgagctgatc   1920
tacgtcaacg acaaaaacag caacgcgacc ttctctgctg ctaacaaagc tgacctgggt   1980
gcatacacct atcaggctga acagcgcggt aacaccgttg ttctgcaaca gatggagttg   2040
accgactacg ctaacatggc gctgagcatc ccatctgcga acaccaatat ctggaacctg   2100
gaacaagaca ccgttggtac tcgtttgacc aactctcgtc atggcctggc tgataacggc   2160
ggcgcatggg taagctactt cggtggtaac ttcaacggcg acaacggcac catcaactat   2220
gatcaggatg ttaacggcat catggtcggt gttgatacca aaattgacgg taacaacgct   2280
aagtggatcg tcggtgcggc tgcaggcttc gctaaaggtg acatgaatga ccgttctggt   2340
caggtggatc aagacagcca gactgcctac atctactctt ctgctcactt cgcgaacaac   2400
gtctttgttg atggtagctt gagctactct cacttcaaca acgacctgtc tgcaaccatg   2460
agcaacggta cttacgttga cggtagcacc aactccgacg cttggggctt cggtttgaaa   2520
gccggttacg acttcaaact gggtgatgct ggttacgtga ctccttacgg cagcatttct   2580
ggtctgttcc agtctggtga tgactaccag ctgagcaacg acatgaaagt tgacggtcag   2640
tcttacgaca gcatgcgtta tgaactgggt gtagatgcag gttatacctt cacctacagc   2700
gaagatcagg ctctgactcc gtacttcaaa ctggcttacg tctacgacga ctctaacaac   2760
gataacgatg tgaacggtga ttccatcgat aacggtactg aagggtctgc ggtacgtgtt   2820
ggtctgggta ctcagttcag cttcaccaag aacttcagcg cctataccga tgctaactac   2880
ctcggtggtg gtgacgtaga tcaagactgg tccgcgaacg tgggtgttaa atatacctgg   2940
taa                                                                 2943
```

<212> Type : DNA
<211> Length : 2943
SequenceName : SEQ ID 401
SequenceDescription :
Sequence
--------
<213> OrganismName : Escherichia coli O157:H7
<400> PreSequenceString :

```
atgaaactca aacatgttgg tatgattgtc gtttctgtgt tggcgatgtc gtctgctgcg      60
gtaagcgcag ccgagggtga tgaatcagta acgaccactg ttaatggcgg tgttattcat     120
tttaaaggtg aagtggtaaa tgccgcttgt gcgattgatt ccgaatcaat gaaccaaacg     180
gttgagctgg gtcaggttcg ttcttctcgc ctggctaaag cgggtgacct cagctccgcc     240
gttggcttca atatcaagct gaatgattgt gataccaatg tttccagtaa tgcagctgtt     300
gcattcctgg gtactactgt caccagtaat gacgatacgt tagcgctgca gagttcagcg     360
gcaggctctg cccaaaatgt cggtattcaa attttggacc gtacgggtga ggtattaata     420
cttgatgggg ccacttttag tgctaaaacc gacttgattg atggcacgaa tatactacca     480
ttccaggctc gttatattgc tctcgggcag tccgtagctg gtactgcaaa cgcagatgcg     540
accttcaaag ttcaatatct ataa                                            564
```

<212> Type : DNA
<211> Length : 564
SequenceName : SEQ ID 402
SequenceDescription :
Sequence
--------
<213> OrganismName : Escherichia coli O157:H7
<400> PreSequenceString :

```
atgaaacttt taaaagtagc agcaattgca gcaatcgtat tctccggtag cgctctggca      60
ggtgttgttc ctcagtacgg cggcggtggc ggtaaccacg gtggtggcgg taataacagc     120
ggcccgaatt cagagctgaa tatttatcag tacggtggtg gtaactctgc acttgctctg     180
caagctgatg ctcgtaactc tgatcttact attacccagc atggtggtgg taacggtgca     240
gatgttggtc agggctcaga tgacagctca atcgatctga cccaacgtgg ctttggtaac     300
agcgccactc ttgatcagtg gaacggtaaa gactctcata tgacagttaa acaattcggt     360
ggcggcaacg gtgcagcggt tgaccagact gcatctaatt ccaccgtcaa cgtaactcag     420
gttggctttg gtaacaacgc gaccgctcat cagtactaa                            459
```

<212> Type : DNA
<211> Length : 459
SequenceName : SEQ ID 403
SequenceDescription :
Sequence
--------
<213> OrganismName : Escherichia coli O157:H7
<400> PreSequenceString :

```
atgcctattg gtaatcttgg tcataatccc aatgtgaata attcaattcc tcctgcacct      60
ccattacctt cacaaaccga cggtgcaggg gggcgtggtc agctcattaa ctctacgggg     120
```

```
ccgttgggat ctcgtgcgct atttacgcct gtaaggaatt ctatggctga ttctggcgac     180
aatcgtgcca gtgatgttcc tggacttcct gtaaatccga tgcgcctggc ggcgtctgag     240
ataacactga atgatggatt tgaagttctt catgatcatg gtccgctcga tactcttaac     300
aggcagattg gctcttcggt atttcgagtt gaaactcagg aagatggtaa acatattgct     360
gtcggtcaga ggaatggtgt tgagacctct gttgttttaa gtgatcaaga gtacgctcgc     420
ttgcagtcca ttgatcctga aggtaaagac aaatttgtat ttactggagg ccgtggtggt     480
gctgggcatg ctatggtcac cgttgcttca gatatcacgg aagcccgcca aaggatactg     540
gagctgttag agcccaaagg gaccggggag tccaaaggtg ctggggagtc aaaaggcgtt     600
ggggagttga gggagtcaaa tagcggtgcg gaaaacacca cagaaactca gacctcaacc     660
tcaacttcca gccttcgttc agatcctaaa ctttggttgg cgttggggac tgttgctaca     720
ggtctgatag ggttggcggc gacgggtatt gtacaggcgc ttgcattgac gccggagccg     780
gatagcccaa ccacgaccga ccctgatgca gctgcaagtg caactgaaac tgcgacaaga     840
gatcagttaa cgaaagaagc gttccagaac ccagataatc aaaaagttaa tatcgatgag     900
ctcggaaatg cgattccgtc aggggtattg aaagatgatg ttgttgcgaa tatagaagag     960
caggctaaag cagcaggcga agaggccaaa cagcaagcca ttgaaaataa tgctcaggcg    1020
caaaaaaaat atgatgaaca acaagctaaa cgccaggagg agctgaaagt ttcatcgggg    1080
gctggctacg gtcttagtgg cgcattgatt cttggtgggg gaattggtgt tgccgtcacc    1140
gctgcgcttc atcgaaaaaa tcagccggta gaacaaacaa caacaacaac tactacaact    1200
acaactacaa gcgcacgtac ggtagagaat aagcctgcaa ataatacacc tgcacagggc    1260
aatgtagata cccctgggtc agaagatacc atggagagca gacgtagctc gatggctagc    1320
acctcgtcga ctttctttga cacttccagc atagggaccg tgcagaatcc gtatgctgat    1380
gttaaaacat cgctgcatga ttcgcaggtg ccgacttcta attctaatac gtctgttcag    1440
aatatgggga atacagattc tgttgtatat agcaccattc aacatcctcc ccgggatact    1500
actgataacg gcgcacggtt attaggaaat ccaagtgcgg ggattcaaag cacttatgcg    1560
cgtctggcgc taagtggtgg attacgccat gacatggag gattaacggg ggggagtaat    1620
agcgctgtga atacttcgaa taacccacca gcgccgggat cccatcgttt cgtctaa       1677
```

<212> Type : DNA
<211> Length : 1677
SequenceName : SEQ ID 404
SequenceDescription :
Sequence
--------
<213> OrganismName : Escherichia coli O157:H7
<400> PreSequenceString :

```
atgttttcta ctttcaaaaa agcagctctg ctggcagcta ttgcattacc tttttcaact      60
atggctgcgc ctacagtcac ttttcagggt gaagtaaccg atcagacctg ttccgtaaat     120
atcaacggtc aaaccaattc agtagtattg atgccgaccg tagccatggc tgacttcggt     180
gcaactttag ctgatggtca gagcgcaggc cagacgccgt ttacggtttc tgtgtctaac     240
tgccaggctc caactggtgc agatcaggca atcaacacca ccttcctggg ctacgacgtt     300
gacgctagca cgggtgttat gggaaaccgt gataccagca gcgatgcggc gaaaggcttt     360
ggcattcagt taatggattc cagcacttct ggtaacccag taactctggc tggcgcgact     420
aacgtaccgg gtctgaccct gaaagttggc gataccgaag ccagctacga cttcggtgcg     480
cgttacttcg ttatcgatag cgctgctgcc actgccggta aaattaccgc tgtcgcagaa     540
tacaccctga gctacctcta a                                               561
```

<212> Type : DNA
<211> Length : 561
SequenceName : SEQ ID 405
SequenceDescription :
Sequence
--------
<213> OrganismName : Escherichia coli O157:H7
<400> PreSequenceString :

```
atgaacagtg aaggaggaaa accggggaat gtactgaccg ttaacggcaa ctataccgga      60
aacaatggcc tgatgacgtt caacgcgacg ctgggcggcg ataattcacc caccgataag     120
atgaacgtga aaggcgatac ccaagggaac actcgcgttc gggttgataa cattggcggc     180
gtcggtgcgc aaacggtcaa cggtattgaa ctcattgagg ttggcggtaa ttctgcaggt     240
aatttcgcgc tgaccaccgg aactgtcgaa gctggggctt acgtctacac gctggctaaa     300
gggaaggggga atgacgagaa aaactggtat ctgaccagta aatgggacgg cgtaacgcca    360
gcggatacac ccgatcccat caataatccc cctgttgtgg atccggaagg cccatcagtt     420
tatcgcccgg aggccggaag ctatatcagc aacattgccg cagccaactc gctgtttagc     480
catcgcttac acgaccgtct gggtgagccg caatatacag attcactgca ttctcaggat     540
tcagcaagca gtatgtggat gcgtcatgtc gggggcacg aacgttccag tgccggagac      600
ggccagctaa atactcaggc taaccgctat gtattgcagc taggcggcga tttggcgcag     660
tggagtagca acgcgcagga tcgctggcat cttggcgtga tggcaggcta cgccaatcag     720

cacagtaata ctcagagtaa tcgtgtgggt tataaatcgg atgggcgcat cagcggttac     780
agcgctgggc tgtacgcgac ctggtatcag aacgatgcga ataagaccgg cgcttatgtt     840
gacagctggg cgctgtataa ctggtttgat aacagcgtca gttccgataa ccgttctgct     900
gacgactatg attctcgcgg tgtgacggcc tctgttgagg gtgggtatac ctttgaagcg     960
ggaacatgta gcggcagcga agggacgctg aatacctggt acgtccagcc acaggcgcaa    1020
atcacctgga tgggtgtgaa agattctgac catgcccgga aagacggaac gcgcattgaa    1080
acggaaggcg acggaaacgt gcaaacgcga cttggggtga aaacctacct gaatagccat    1140
caccagcgtg acgatggtaa acagcgtgag ttccagcctt acattgaagc gaactggatc    1200
aacaatagca aagtctacgc cgtgaagatg aatggtcaaa ccgtaagccg tgatggtgcg    1260
cgaaatctcg gtgaagtacg taccggggtt gaggcgaaag taaataacaa ccttagcctg    1320
tgggggaatg tcggtgtgca actaggtgat aaaggctata gcgatactca gggcatgctg    1380
ggagtgaaat atagctggta a                                              1401
```

<212> Type : DNA
<211> Length : 1401
SequenceName : SEQ ID 406
SequenceDescription :
Sequence
--------
<213> OrganismName : Escherichia coli O157:H7
<400> PreSequenceString :

```
atgtcatatc tgaatttaag actttaccag cgaaacacac aatgcttgca tattcgtaag      60
catcgtttgg ctggtttttt tgtccggctc tttgtcgcct gtgcttttgc cgtacaggca     120
cctttgtcat ctgccgaact ctattttaat ccgcgctttt tagcggatga tccccaggct     180
gtggccgatt tatcgcgttt tgaaaatggg caagaattac cgccagggac gtatcgcgtc     240
gatatctatt tgaataatgg ttatatggca acgcgtgatg tcacatttaa tacgggcgac     300
agtgaacaag ggattgttcc ctgcctgaca cgcgcgcaac tcgccagtat ggggctgaat     360
acggcttctg tcgccggtat gaatctgctg gcggatgatg cctgtgtgcc attaaccaca     420
atggtccagg acgctactgc gcatttagat gttggtcagc agcgactgaa cctgacgatc     480
cctcaggcat ttatgagtaa tcgcgcgcgt ggttatattc ctcctgagtt atgggatccc     540
ggtattaatg ccggattgct caattataat ttcagcggaa atagtgtaca gaatcggatt     600
gggggtaaca gccattatgc atatttaaac ctacagagtg ggttaaatat tggtgcgtgg     660
cgtttacgcg acaataccac ctggagttat aacagtagcg acagatcatc aggtagcaaa     720
aataaatggc agcatatcaa tacctggctt gagcgagaca taataccgtt acgttcccgg     780
ctgacgctgg gtgatggtta tactcagggt gatattttcg atggtattaa ctttcgcggc     840
gcacaattgg cctcagatga caatatgtta cccgatagcc aaagaggatt tgccccggtg     900
atccacggta ttgctcgtgg tactgcacag gtcactatta aacaaaatgg gtatgacatt     960
tataatagta cggtgccgcc ggggcctttt accatcaacg atatctatgc cgcaggtaat    1020
agtggtgact tgcaggtaac gattaaagag gctgacggca gcacgcagat tttaccgta    1080
ccctattcgt cagtcccgct tttgcaacgt gaagggcata ctcgttattc cattacggca    1140
ggagaatacc gtagtggaaa tgcgcaacag gaaaaacccc gctttttcca aagtacatta    1200
ctccacggcc ttccagctgg ctggacaata tatggtggaa cgcaactggc agatcgttat    1260
cgtgctttta attttggtat cgggaaaaat atgggggcac tgggcgctct gtctgtggat    1320
atgactcagg ctaattccac acttcccgat gacagtcagc atgacggaca atcggtgcgt    1380
tttctctata acaaatcgct caatgagtca ggcacgaata ttcagttagt gggttaccgt    1440
tattcgacca gcggatattt taatttcgct gatacaacat acagtcgaat gaatggctac    1500
aacatcgaaa cacaggacgg agttattcag gttaagccga aattcaccga ctattacaac    1560
ctcgcttata acaaacgcgg gaaattacaa ctcaccgtta ctcagcaact cgggcgctca    1620
tcaacactgt atttgagtgg tagccatcaa acttattggg gaacgagtaa tgtcgatgag    1680
caattccagg ctggattaaa tactgcgttc gaagatatca actggacgct cagctatagc    1740
ctgacgaaaa acgcctggca aaaaggacgt gatcagatgt tagcgcgtaa cgtcaatatt    1800
cctttcagcc actggctgcg ttctgacagt aaatctcagt ggcgacatgc cagtgccagc    1860
tacagcatgt cacacgatct caacggtcgg atgaccaatc tggctggtgt atacggtacg    1920
ttgctggaag acaacaacct cagctatagc gtgcaaaccg gctatgccgg gggaggcgat    1980
ggtaatagcg gaagcacagg ctacgccacg ctgaattatc gcggtggtta cggcaatgcc    2040
aatatcggtt acagccatag cgatgatatt aagcagctct attacggagt cagcggtggg    2100
gtactggctc atgccaatgg cgtaacgctg ggcagccgt taaacgatac ggtggtgctt    2160
gttaaagcgc ctggcgcaaa agatgcaaaa gtcgaaaacc agacgggggt gcgtaccgac    2220
tggcgcggtt atgccgtgct gccttatgcc actgaatatc gggaaaatag agtggcgctg    2280
gataccaata ccctggctga taacgtcgat ttagataacg cggtcgctaa cgttgttccc    2340
actcgtgggg cgatcgtgcg agcagagttt aaagcgcgcg ttgggataaa actgctcatg    2400
acgctaaccc acaataataa gccgctgccg tttggggcga tggtgacatc agagagtagc    2460
cagagtagcg gcattgttgc ggataatggt caggtttacc tcagcggaat gcctctagcg    2520
ggaaaagttc aggtgaaatg gggagaagag gaaaatgctc attgtgtcgc caattatcaa    2580
ctgccaccag agagtcagca gcagttatta acccagctat cagctgaatg tcgttaa       2637
```

<212> Type : DNA
<211> Length : 2637
SequenceName : SEQ ID 407
SequenceDescription :
Sequence
--------
<213> OrganismName : Escherichia coli O157:H7
<400> PreSequenceString :

```
atgcagataa tctttggaga aaaatgcgtg tcattactac gactattttt tgccgccgtc      60
ttaatgctat ggtgcgctca aaccgctgct tatagcgggc agtgtcatac cactcagggg     120
aatccgtata ttggcgtcaa ttttggcgtt aaaaccctgg aggaagaaga aaatacgact     180
ggggtagtaa aagacaaatt ttatcagtgg aacgaatcga atgattatta tgtttcctgt     240
gattgcgata aagacaatgt cagaagtggc cgatgggcat tcgccgcgga ttcaccgtta     300
gtctatttag gcgacaactg gtacaaaatt aatgactatc ttgccgccaa agttttattg     360
caggttaaag gcagttctcc tacagcggtt cctttcgaaa acgtggggac tggggcagat     420
acccggtggc atatttgtga ccccggcggt caacgtttag gcggccaggg agctagcggt     480
aatagcggta gcttttccct gaaaatattg cagccgttcg ttggttcggt cgtcattcct     540
cctatggcgc tggcgcgatt atttgaatgc tacaacatac ccgcaggtga ttcctgcacg     600
actacaggca caccggtttt agtgtattac ctgtctggta ctatcaattc acttggctca     660
tgttccgtca atgccggaga aacaatcgag gtcgatctgg gcgacgtatt tgcggctaac     720
tttcgtgttg tagggcataa gcctcttggg gccagaacgg cagaacttgc aattccagtc     780
aggtgtaaca cgggaaacgc ggggttagtt aacgtcaacc tgagtctgac ggcaaccaca     840
gaccccagct atccccaggc gattaagacg tcacgtcctg gcgtgggcgt ggtggtgacc     900
gatagccaga acaacattat ttcccctgct ggtggaacat taccgctctc tattcctgat     960
gatgcagaca gtatcgcgtg a                                               981
```

<212> Type : DNA
<211> Length : 981
SequenceName : SEQ ID 408
SequenceDescription :
Sequence
--------
<213> OrganismName : Escherichia coli 0157:H7
<400> PreSequenceString :

```
atgaaaatta aaactctggc aatcgttgtt ctgtcggctc tgtccctcag ttctacagcg      60
gctctggccg ctgccacgac ggttaatggt gggaccgttc actttaaagg ggaagttgtt     120
aacgccgctt gcgcagttga tgcaggctct gttgatcaaa ccgttcagtt aggacaggtt     180
cgtaccgcat cgctggcaca ggacggagca accagttctg ctgtcggttt taacattcag     240
ctgaatgatt gcgataccaa tgttgcatct aaagccgctg ttgccttttt aggtacggtg     300
attgatgcgg gtcataccaa cgttctggct ctgcagagtt cagctgcggg tagcgcaaca     360
aacgttggtg tgcagatcct ggacagaacg ggtgctgcgc tgacgctgga tggtgcgaca     420
ttcagtgagc aaacaaccct gaataacggt actaacacca ttccgttcca ggcgcgttat     480
tatgcaatcg gcgaggcaac cccgggtgct gctaatgcgg atgcgacctt caaggttcag     540
tatcaataa                                                             549
```

<212> Type : DNA
<211> Length : 549
SequenceName : SEQ ID 409
SequenceDescription :
Sequence
--------
<213> OrganismName : Escherichia coli 0157:H7
<400> PreSequenceString :

```
atgaaattaa aagtcatcgc tacactgatt gctactgttg ccgtgggtgt aagctttaac      60
agcaattttg cttctgcgag tacaacgtcc gcttctttaa ccgtaaacag taacctgact     120
atgggtacct gcagtgctca gataatggat aatagtaata aagtgatcaa tgaagtggtc     180
tttggcaatg tttatatttc tgaactcggt gcaaaaagca aagtgcaaca gtttaaaatt     240
cgctttagca attgctctgg ccttccccaa aacagcgccc aaatagtgct ggcacctaat     300
ggtatatcct gtgctggttc tcaatcgtca tcggcgggtt tttctaacaa gtttactgac     360
gctagcgcag caaccagaac ggctgtggaa gtatggacta cagatacacc ggaaagcaat     420
ggcagtacgc aattccattg tgctcaaaag ataccagtgc ctgtgacgct tcccgccgac     480
accacaactc agccttacga ttacccgtta agtgcacgga tgaccgttgc ggaaggtaga     540
ttggtaaccg atgtaagacc gggtaatttc cgctctccca cgactttcac gatcacttat     600
cagtaa                                                                606
```

<212> Type : DNA
<211> Length : 606
SequenceName : SEQ ID 410
SequenceDescription :

Sequence
--------
<213> OrganismName : Escherichia coli 0157:H7
<400> PreSequenceString :

```
ttggcatcaa cagttgagta tggtgagaca gttgatggtg ttgtcctgga aaaagatatc      60
cagctggttt atgggaccgc caataatacg aaaatcaatc ctggcggaga acagcatatt     120
aaagaatttg gtataagtag taatactgaa attaacggcg ggtatcagta cattgaaatg     180
aatggcaccg cagaatactc agtattaaat gatggttatc aaattgttca aatgggtggc     240
gcggcaaacc agactacgct caataatggt gtgctacagg tttatggcgc agcgaatgat     300
cccacgatta aaggcgggcg cttaatcgtt gaaaaagatg ggattaccgt ccttgccgct     360
atcgaaaagg gaggattact ggaggttaaa gagggggat tagcgattgc ggtagatcag      420
aaagcaggcg gtgctattaa agcaagcacg cgggtcatgg aggtattcgg aacaaaccgt     480
ctcggtcagt tcgaaatcaa gaatggtatt gctaacaata tgctgttgga aaacggcgga     540
agtttgcgag ttgaagaaaa tgacttcgct tataatacta ctgtagatag tggcggctta     600
ctggaggtta tggatggcgg gactgcaact ggcgttgata aaaaagcagg cggaaaatta     660
attgtctcaa cgaatgcgct ggaagtgagt ggtacaaaca gtaaaggcca atttagtata     720
aaagatggtg tgtcaaaaaa ttatgaactg gatgatggtt ccgggcttat tgttatggag     780
gacacgcagg ccattgacac tatcctcgat gagcatgcca ctatgcaatc gctgggaaag     840
gatactggta cgagagtgca ggcaaatgcg gtatatgatc tcggtcgatc agatcagaat     900
ggaagtataa cgtattcctc taaagccatc tctgaaaata tggttatcaa caatggccgc     960
gctaacgtct gggctggcac aatggttaac gtgtcagtca gaggaaatga tggcattctt    1020
gaggttatga agccgcaaat aaattatgca cccgcaatgt tggtgggtaa ggtagtggtt    1080
tctgagggcg cttctttaag aacgcatggt gccgtggata ccagcaaagc ggatgtttcg    1140
ctcgaaaata gcgcatggac catcattgcc gatatcacta cgacgaacca aaacacccgc    1200
cttaacttag ccaaccttgc gatgtctggc gcaaatgtga ttatgatgga tgagtcagtg    1260
actcgttcat ctgtgacggc aagtgcggaa aatttcacta cgttgaccac caataccctg    1320
tcgggaaacg gcaatttttta tatgcgtacc gatatggcga atcatcagag cgatcagctc   1380
aacgtcaccg gtcaggcaac aggtgatttc aaaatattcg tgacggacac cggtgccagc    1440
ccggcagcag gagatagcct tacactggta acaacgggcg gcggtgatgc tgcatttacg    1500
ttgggcaatg ccggaggcgt tgttgatatc ggtacgtatg aatatacctt gctggataat    1560
ggtaaccata gctggagtct ggcagagaat cgcgcgcaaa ttacccccttc aaccactgat   1620
gtgctgaata tggcggccgc acaaccgctg gtatttgatg cagaactgga caccgtgcgt    1680
gagcgtcttg gtagcgtaaa aggcgttagt tacgatacgg cgatgtggag ttcggcaatt    1740
aacacccgca acaacgtgac cactgatgcg ggagctggtt ttgagcaaac attgacgggc    1800
ctgacgctcg gtatcgatag ccgtttctcc cgtgaagaaa gcagcacaat tcgcggcttg    1860
ttctttggtt actctcattc tgatattggt tttgatcgcg gcggcaaagg caatgtcgat    1920
agctataccc tgggggctta tgccggttgg gagcatcaga acggtgccta tgttgatgga    1980
gtggtgaaag ttgaccgttt tgccaacacc atccatggca agatgagtaa tggggcaaca    2040
gcgtttggcg attacaatag taacggcgcg ggtgctcatg tcgagagcgg gttccgttgg    2100
gttgacggat tgtggagtgt tagaccctat ctggccttta ccggctttac cacagatggt    2160
caggactaca cgttatcaaa cggcatgcgc gcggatgtgg gaaatacccg gatattacgc    2220
gctgaagcgg gaacggcggt aagctatcac atggacctgc aaaacggtac gacgctggaa    2280
ccctggctga aagccgccgt gcgtcaggaa tacgccgatt ctaaccaggt gaaagttaat    2340
gacgatggca aatttaataa tgatgtggct ggaacccgtg gcgtttatca ggctgggata    2400
aggtcatcgt ttaccccgac gttaagcggt catttgtcag tcagctatgg caatggcgca    2460
ggggtagaat cgccgtggaa tacccaggcg ggtgtggtct ggacgttctg a             2511
```

<212> Type : DNA
<211> Length : 2511
SequenceName : SEQ ID 411
SequenceDescription :
Sequence
--------
<213> OrganismName : Escherichia coli 0157:H7
<400> PreSequenceString :

```
atgcaaagga aaggcaataa actgttgatt cagttatgca gtgtgatact gctatttttt      60
accacatcct ggtatgcatt ggcgaatgaa tgttatatag agagaaatgc tgaaggggat     120
tatcacatga agataagctc tactcagctt agtctggcgt cacaaatggt cgaggttccg     180
acagaaatag ccgaagctac atgggatgta aatattcaac taagaggcga tgccataggg     240
tgtaaatctc ttggggatag taaggcagtt cactttctta atacagctga cccaagttta     300
atatccacgt acaccacaac gaatggcgca gcgttattaa aaacaactgt tccaggcatt     360
gtgtattctg tcgagttatt atgccttagt tgtggtgccg cagatgaact tgatttatgg     420
ctacctgcac aaagtggcgc agataacttc ataccaagca cccagacgaa atgggcctat     480
gagtacagtg atcaaagttg gtatttacgt tttcgcttat tcataactcc tgaatttaaa     540
```

```
cccaagaatg gtgtttccag cggaacaacg atagcaggaa agattgcgtc atggtatata    600
ggtaccaatg accagccgtg gatcaacttt tacattgaca atgactcttt aaagtttttc    660
gtcgatgaac cgacctgtgc aacagttgcc ctggcacaag atcagggcaa cgtcagtggc    720
aatcaggtaa cgcttgggaa cagctatgtt tcggaagtga aaaatgggct tacgcgggaa    780
atccctttttt ctatccgtgc tgaatactgt tatgccagta aaattacggt taagttgaaa    840
gcggcaaata aacccagcga tgccacactg gtgggtaaaa cgactggctc ggcttcaggc    900
gtggctgtaa aagtaaattc aacttatgac aatagcaaag tattgttaaa agcagatggt    960
agcaacacgg ttgactacaa cttcgccgcc tggtcaaaca acctgctgtt tttacctttt   1020
acggcgcagc tggtaccgga tggtagcggt aatgctgtcg gtgttggaac attttcaggt   1080
aacgcgacct tctcctttac ctacgaataa                                    1110
```

<212> Type : DNA
<211> Length : 1110
SequenceName : SEQ ID 412
SequenceDescription :
Sequence
--------
<213> OrganismName : Escherichia coli 0157:H7
<400> PreSequenceString :

```
ttgtaccagt ttactcatca aaaaagccgt atcccgaaaa aaacgctact tgcggcctgt     60
tgtgccctgt tttatagcag caacggtgct gcggcggaca ccgtggaata tgacagttcc    120
tttttaatgg gaactggcgc atcaacgatt gatgttaaac gttatgctca aggcaacccg    180
acaccgccgg gtctctataa tgtccgcgta tttgtaaacg gtcaggcgac ttccagctta    240
gaaattccgt ttgtggatat tggcgaaaac agtgcggcgg cctgtcttac ccataaaaac    300
ctggcgcaac ttcacattaa gcaacctgaa cagcctgtca ctttactcgc cagagaaggt    360
gaagaagagg attgtctgga tctggcaaag tcatacgaaa aggcggatgt gtgctttgac    420
ggtagtgacc agtttctcga tctgacgatc cctcaggcct atgttctgaa aagctatggc    480
ggctacgttg acccttcttt atgggaatcg ggaattaacg ctgccacact ggcatatacc    540
ctgaacgcgt atcacacaag ttcagataac gacaatagtg acagcgtcta tggcgcgttc    600
aactcaggta tcaatttagg agcctggcac tttcgtgcgc gcggtaacta taactggaca    660
acagataacg gcagcgattt cgatttccag gatcgttact tacagcgtga cattccggca    720
atccgttccc agataattat gggtgatgcc tataccaccg gtgaaacgtt tgactctgtc    780
aacgtccgtg gtgttcgcct gtacagcgac agccgtatgc tgccttcggc gctggccagt    840
tacgctccga ccatccgcgg tgtagcaaac tccaacgcca aagtcaccgt gacgcaaagc    900
ggatataaaa tttatgaaac caccgttccg cccggtgaat ttgttataga cgacattagc    960
ccttccggct ttggtagcga actggtcgtg accattgaag aagcggatgg ttccaaacgc   1020
acctttacgc aacccttctc gtcggttgta caaatgcaac gtcctggtgt gggccgttgg   1080
gatttcagcg cgggtaaagt cattgatgac agtctgcgat ccgaacccaa tatggggcaa   1140
gcctcttatt actatggtct gaataacctc ttcacgggtt ataccggcat tcagttcacc   1200
gataataact atcttgccgg gctgttaggt gtgggtatca acaccagcat cggcgccttt   1260
gcggtagacg ttacccattc ccgtgctgaa attccggatg ataaaaccta ccaggggcaa   1320
agttatcgcg tgacctggaa caaacttttc caggataccg ggacatcatt taacctcgcg   1380
gcgtaccgct attccaccca ggattacctg ggcctgcatg atgcgttagt cctcattgac   1440
gacgccaagc atttgtctgc cgatgaagac aaaaacacca tgcagacgta ctcacgtatg   1500
aaaaaccagt ttaccgtcag cattaaccag ccattgaata tcgcctatga agattacggt   1560
tcgctgttta tttccggtag ctggacgtat tactgggcgg cgaacaatag ccgcactgaa   1620
tataatgttg gttacagtaa aagcgtttcg tggggcagtt tcagcgtcaa cctacaacgt   1680
agctggaatg aagacggcga gaaagatgac gcgatgtacg tcagcgttag cgtacctatt   1740
gagaatattt taggtggcaa acgtaagtct tctggtttcc gcaatttaaa tactcagctc   1800
aataccgatt tcgatggttc acatcagttg aatgttaaca gttccggtaa cactgaaaac   1860
aatctggtga actacagtgt caacgcaggt tatagcctcg ataaaaacgc cggcgattta   1920
gcctctgttg gtggttatct caactatgaa tctgggttag gcggtatttc cgcttcggcc   1980
tcggccactt ctgataacag ccaacagtac tccatctcaa ccgatggcgg ctttgtatta   2040
cacagtggtg gtttaacgtt cactaacaac agtttcagca gtaacgacac gctggtgtta   2100
atcaacgccc taggtgctaa aggcgcacga atcaataaca gtaataacga aatcgatcgc   2160
tggggatatg ccgtgacgtc ctctgtcagc ccatatcgtg aaaaccgggt aggtctgaac   2220
attgaaacac tggaaaacga tgttgaactg aaaagtacca gcgccaccac cgtaccacgt   2280
agcggctccg ttgttttgac ccgtttcgaa actgacgagg ggcgttctgc cgtgctgaat   2340
attactgccg ccaatggcaa atccattccg tttgctgcgg aggtttacca gggtgaggtg   2400
atgatcggca gcatgggcca gggtggtcag gcatttgtac gcggtattaa cgacagcggg   2460
gaattaatcg tgcgctggta tgaaaacaac caaaccattg actgtaagtt gcactaccag   2520
ttcccggcgc agccacaaac gcagggaagc accaacacct tattacttaa caatcttacc   2580
tgtcaggtag caaatcacta a                                             2601
```

<212> Type : DNA
<211> Length : 2601

SequenceName : SEQ ID 413
SequenceDescription :
Sequence
--------
<213> OrganismName : Escherichia coli 0157:H7
<400> PreSequenceString :

```
atgaagttca aacgattgct gcatagcggc atcgccagtt tgagtctggt tgcctgcggg      60
gtgaatgcgg cgacggatct tggcccggca ggggatattc atttctccat cactatcacc     120
actaaagctt gcgagatgga aaaaagcgat ctcgaagtcg atatgggaac aatgacgctg     180
caaaaacctg cggcagtcgg tacggtgttg agcaagaaag atttcaccat tgaactcaaa     240
gagtgcgatg ggatatccaa agcgaccgtt gagatggaca gtcagtcgga cagcgatgat     300
gattccatgt ttgcccttga ggctggtggc gcaacgggtg ttgcgttgaa gatagaggac     360
gataaaggaa cgcagcaagt tcccaaaggc tccagcggaa cgccgattga atgggcgatt     420
gatggcgaaa ccacgtcgct tcactaccag gcgagttatg tggtcgtcaa cactcaggcc     480
actggtggca cagcgaatgc ccttgtaaat ttttccatca cctatgagta a              531
```

<212> Type : DNA
<211> Length : 531
SequenceName : SEQ ID 414
SequenceDescription :
Sequence
--------
<213> OrganismName : Escherichia coli 0157:H7
<400> PreSequenceString :

```
atgaaataca ataacattat tttcctcggt ttatgtctgg ggttaaccac ctattctgct      60
ttatccgcag atagcgttat taaaattagc gggcgcgtcc tcgattatgg ctgcacagtc     120
tcatcggatt cgcttaattt taccgtagat ctccaaaaaa acagtgccag acaatttcca     180
acgaccggta gcacaagtcc agccgtccct tttcagatta cgttaagtga atgcagcaaa     240
gggacaacgg gggttcgggt tgcatttaac ggtattgagg acgcagaaaa taatactctg     300
ttgaaactgg atgagggaag caatacggcc tccggtttag gtatagaaat actggacgga     360
aatatgcgtc cggtgaaact gaatgacctt catgccggga tgcagtggat cccactggta     420
ccagaacaga acaatatttt gccttactcc gctcgtctga agtcaactca gaagtccgtc     480
aatccgggac tggtgagggc ttcggcaacc tttacccttg aatttcaata a              531
```

<212> Type : DNA
<211> Length : 531
SequenceName : SEQ ID 415
SequenceDescription :
Sequence
--------
<213> OrganismName : Escherichia coli 0157:H7
<400> PreSequenceString :

```
atgaaatggc gcaaacgtgg gtatttattg gcggcaatat tggcgctcgc aagtgcgacg      60
atacaggcag ccgatgtcac catcacggtg aacggtaagg tcgtcgccaa accgtgcaca     120
gtttccacca ccaatgccac ggttgatctc ggcgatcttt attctttcag tctgatgtct     180
gccggggcgg catcggcctg gcatgatgtt gcgcttgagt tgactaattg tccggtggga     240
acgtcaaggg tcactgccag cttcagcggg gcagccgaca gtaccggata ttataaaaac     300
caggggaccg cgcaaaacat ccagttagag ctacaggatg acagtggcaa cacattgaat     360
actggcgcaa ccaaaacagt tcaggtggat gattcctcac aatcagcgca cttcccgtta     420
caggtcagag cattgacggt aaatggcgga gccactcagg gaaccattca ggcagtgatt     480
agcatcacct atacctacag ctga                                            504
```

<212> Type : DNA
<211> Length : 504
SequenceName : SEQ ID 416
SequenceDescription :
Sequence
--------

<213> OrganismName : Escherichia coli 0157:H7
<400> PreSequenceString :

```
atgaaaagag cgcctcttat aacaggactt ttgttgatat ccacatcctg cgcttatgcc     60
tcctcagaag ggtgtggagc tgacagcact agcggtgcga caaattacag cagtgtggtt    120
gatgatgtta cggtgaacca gacagataac gtgacaggac gggagtttac ctctgcaacg    180
ctaagtagca ctaactggca atacgcctgt tcctgctctg cgggtaaggc agttaaactt    240
gtctatatgg tcagccccgt acttaccacc actggacatc agacaggata ttacaaactc    300
aatgacagcc tggatattaa aaccatgaac cgccccggaa atcctggaga ctaa          354
```

<212> Type : DNA
<211> Length : 354
SequenceName : SEQ ID 417
SequenceDescription :
Sequence
--------
<213> OrganismName : Escherichia coli 0157:H7
<400> PreSequenceString :

```
atgaaaaaag cacttctcgc agccgctctg gttatggctt ctggttccgc cctggctgta     60
gatggtggtc atatcgactt taacggtatg gtacagtccg gtacctgtaa agtgggtgtg    120
gtagatactg gtatgcatag cgttaccact gatggcgtgg ttaccctgga tactgcgaat    180
gttactgata cttttgctga agttagcgca actgctgtcg gtttactgcc gaaagagttc    240
atgatttctg ttgagtgtga tccaggtgct ccgaagaatg ctgagttaac tatgggttct    300
gcaagttacg cgaacaccag cggtaccctg aataacaata tgaacatcac tgttaacggt    360
attgcaccgg ctcagaacgt aaacattgca gttcataaca tgaaaaacaa agctggcgct    420
gctgaaatta agcaggtcca tatgaacaac tcttctgaag ttcaggaact gacattagac    480
gcagaaggta aaggccagta cgtatttaac gcatcttacg ttaaagcacc gaacagcccg    540
gctgtaactg ctggtcatgt aaccactaac gcgctgtaca ccgttgctta taagtaa       597
```

<212> Type : DNA
<211> Length : 597
SequenceName : SEQ ID 418
SequenceDescription :
Sequence
--------
<213> OrganismName : Escherichia coli O157:H7
<400> PreSequenceString :

```
atgaaaccaa atatgattgt aggagcatta gcgttaactt ctgtgtttat ggcaggtcac     60
ctacaggcgg ctgatggaac agtccatttc cgtggtgaaa ttattgacag tacttgcgaa    120
gtcactcctg aaactaaaga tcaggtcgtt gatttaggca aagtaaaccg tacagccttt    180
agtggcgtcg atgatgtggc tgccccgacg gctttttcta tcgatctgac tcaatgcccg    240
gaaaccttta agtccgccgc aattcgtttc gatggtaatg aagatgctca tggtaatggc    300
aacctggcaa ttggtacccc gctggataac tctaacgatg ctgccgctgg tattagcccg    360
agtgataaca gtggggatta tactggtgcg ggtgccgtta gtgcagcgaa aggcgtagct    420
attcgtttat ataaccgtgc agataacact caggtcaagt tatatgaaaa ttctgcatca    480
actccgattt ctaatggtaa tgcatccatg aagttcatgg ctcgttatat tgctacggaa    540
acgactattg accctggtac agctaacgcc gactcgcagt ttacagttga atatataaaa    600
taa                                                                  603
```

<212> Type : DNA
<211> Length : 603
SequenceName : SEQ ID 419
SequenceDescription :
Sequence
--------
<213> OrganismName : Escherichia coli O157:H7
<400> PreSequenceString :

```
gtgccaattt tccagcgtga aggccatctc aaatatagct ttgccgcagg tgaatatcag     60
gccgggaatt atgacagcgc ctcgccgcgt ttcgggcagc ttgatctgat ctacggttta    120
ccgtgggggga tgacggccta cggcggcgta ttaatctcta ataattacaa tgcatttaca   180
ttagggatag ggaaaaactt tggttatatc ggggcgattt ccattgatgt gacgcaggct    240
aaaagcgaac tgaataacga tcgcgatagc cagggacaat cttatcgttt cttatattcc    300
aagagcttcg aaagcggcac cgatttccgc cttgcgggct atcggtactc taccagcggt    360
ttctatacct tccaggaagc caccgatgtg cgcagtgacg ctgacagcga ctataaccgt    420
tatcacaagc gcagcgaaat acagggtaac ctgacgcagc aattaggggc ctatggctct    480
gtttatttaa atttaacgca gcaggattac tggaacgacg caggtaaaca gaacacggta    540
tcggcgggtt acaacggacg tattggcaag gtcagttaca gtattgcata tagctggaat    600
aaaagccctg aatgggatga aagcgatcgc ttgtggtctt tcaatatttc cgttccacta    660
ggccgggcct ggagtaacta tcgcgtcacg accgaccagg atggtcgtac caatcaacag    720
gttggggtca gcggaacgct gcttgaggat cgcaacctga gctacagtgt ccaggaaggc    780
tacgccagca acggtgtggg taacagcggt aacgctaacg ttggctatca gggtgggtcc    840
ggtaatgtca acgtaggcta tagctacggg aaagattacc ggcagctcaa ctacagcgtt    900
cgcggcggcg tgatagttca tagcgaaggc gtgacgcttt cccaaccgct aggcgaaacc    960
atgacgctca tctccgtacc cggtgcgcgc aatgcccgcg tggtgaataa cggcggcgtt   1020

caggttgact ggatgggtaa cgcgatcgtg ccttatgcca tgccgtatcg tgaaaacgaa   1080
atctcactgc gtagcgattc gttgggtgac gatgttgacg ttgaaaatgc gttccagaaa   1140
gtggtgccaa cgcgtggagc gattgtcaga gcgcgttttg atacccgcgt tggttaccgc   1200
gtattaatga cgctgcttcg ttccgcgggc agcccggtgc cctttggagc aacggcaacg   1260
ctaatcaccg ataaacaaaa cgaggtgagc agtatcgttg gtgaagaagg acagctctat   1320
attagcggaa tgccagagga aggacgggta ttgattaaat ggggtaatga cgcgtcgcag   1380
caatgcgtgg cgccttataa attatccctg gaattaaaac agggcggaat tattcctgtt   1440
tcggccaatt gccagtaa                                                 1458
```

<212> Type : DNA
<211> Length : 1458
SequenceName : SEQ ID 420
SequenceDescription :
Sequence
--------
<213> OrganismName : Escherichia coli O157:H7
<400> PreSequenceString :

```
atgagtggtt acaccgtcaa gcctcctacc ggagacagca atgagcagac acaatttatt     60
gattatttta atctgttcta cagtaagcgt gatcaggaac aaataagcat ctctcagcag    120
cttggaaatt acggtgcgac atttttcagt gccagtcgcc aaagttactg gaacacgtca    180
cgcagcgacc agcaaatatc atttggatta aatgtgccgt ttggtgatat tacgacttcg    240
ctgaattaca gctattccaa taatatatgg caaaacgatc gggatcattt actcgctttt    300
acgcttaatg ttcccttcag tcattggatg cgtacagaca gtcagtcggc atttcgtaat    360
tcaaacgcca gttacagtat gtcaaacgat ttgaaaggcg gcatgaccaa tctatcgggg    420
gtttatggca ctctgctgcc ggataataac ctgaattata gcgttcaggt cggtaacacc    480
cacggaggta atacatcgtc tggcaccagt ggttacagta ctcttaatta tcgtggagct    540
tacggcaata ctaatgtcgg ttacagtcgg agtggtgaca gcagccagat ttattacgga    600
atgagtggtg ggattattgc tcatgctgat ggcatcacct ttggacagcc gctgggcgac    660
acaatggttc tggttaaggc tcctggcgct gataatgtca aaatagagaa ccagaccgga    720
attcataccg actggcgtgg ctatgccata ttaccatttg cgacagaata tagagaaaat    780
cgtgtcgctc ttaacgcgaa ttcccttgca gataatgttg aactggatga aaccgtggtc    840
actgtcatcc caactcacgg tgctattgcc agagcaacat ttaatgcaca aatcggcggg    900
aaagtattaa tgacgttgaa gtacggtaat aaaagcgttc cattcggtgc aattgtcact    960
cacggagaga ataaaaatgg cagcattgtc gcggaaaacg gtcaggttta tctgactgga   1020
cttccacagt cagggaaatt acaggtttca tggggcaatg ataaaaactc aaactgtatt   1080
gtcgattaca agcttcctga agtctctcct ggaaccttgc tgaaccagca gacagcaatc   1140
tgtcgctaa                                                           1149
```

<212> Type : DNA
<211> Length : 1149
SequenceName : SEQ ID 421
SequenceDescription :
Sequence
--------
<213> OrganismName : Escherichia coli 0157:H7

<400> PreSequenceString :

```
atgtctgctt tgtatgaacg ttcacagctg acgcaggtga tgatttcatc tgccccggcg     60
actgctgaaa ccatggagaa ggcggaatat ctgcgcctgg actgcaccat caaggaagtc    120
cagttcaccg ccggtcagaa acaggatatt gatgtgacca cgctctgctc cacagagcag    180
gagaacatca acggtctggg ggcgtcgtcc gagatttcca tgtcgggtaa tttttatctg    240
aatcaggccc agaacgccct gcgtgatgcc tatgacaatg acacggtgta tgcgtttaag    300
gtgcagtttc cgtccggtaa gggctttaag ttcctggcgg aagtgcgtca gcacacctgg    360
tcatccggta ccaacggcgt ggtggctgca acgttttcac ttcgcctgaa gggtaaaccg    420
gtgtcctatg tggtaccgct ggcgtttgtg aaaaatctgg ataagacact taccgtgaat    480
accggtgcgc tgctgacaat gtcagtcagt gtcaacgggg gaacgccgcc ttataaacac    540
gcctggaaga aggatggtca gccggtagag ggacagacta ctgacacttt cagtaagcca    600
ggtgcgcagt caggtgataa gggggcttat acctgcgagg taacggattc tgcagaacag    660
ccgcagagca ttacctctga tgcgtgtaca gtaacggtta atggtgcggg cggataa       717
```

<212> Type : DNA
<211> Length : 717
SequenceName : SEQ ID 422
SequenceDescription :
Sequence
--------
<213> OrganismName : Escherichia coli 0157:H7
<400> PreSequenceString :

```
atgagaaaca aacctttttta tcttctgtgc gctttttttgt ggctggcagt gagtcacgct     60
ttggctgcgg atagcacgat tactatccgc ggctatgtca gagataacgg ctgtagtgtg    120
gccgctgaat caaccaattt tactgttgat ctgatggaaa acgcggcgaa gcaatttaac    180
aacattggcg cgacgactcc tgtcgttcca tttcgtattt tgctgtcacc ctgtggtaac    240
gccgtttctg ccgtaaaagt tgggtttacc ggcgttgcag atagccacaa tgccaacctg    300
cttgcacttg aaaatacggt gtcagcggct tcgggactgg gaatacagct tctgaatgag    360
cagcaaaatc agataccccct taatgctcca tcgtccgcga tttcgtggac gaccctgacg    420
ccgggtaaac caaatacgtt gaatttttac gcccggctaa tggcgacaca ggtgcctgtc    480
actgcggggc atatcaatgc cacggctacc ttcactcttg aatatcagta a              531
```

<212> Type : DNA
<211> Length : 531
SequenceName ; SEQ ID 423
SequenceDescription :
Sequence
--------
<213> OrganismName : Escherichia coli 0157:H7
<400> PreSequenceString :

```
atgaataaat ccgttgtgtc aatttctgcg gcaatgttgg ttttactttg ccaaccggtc     60
atggggagcg aaatctcacc cgcaacaccg tcagatgaag acaactacac ctttgacccg    120
caactcttcc gcggcagcag atttagtcag tcgtcattag caaaactgac aacacgtgag    180
tctgttgcac cgggcaatta taaaatggat atctacacca acaataagtt gtcaggcagt    240
tggaatgtca cgtttaaaga agccgctgat ggtcgcgttc tgccctgcct gacgcctgaa    300
gtcgcggacg cgataggcct caaaacaggg gaagataagg gggaaaaaga tcctgtctgt    360
acgtttgcta aggaactcgc tcccggcatc accagccaga cacagttgtc acaattgcgc    420
ctggacttat cggtgccaca gagtcaattg attagtcgcc ctcgcggcta tgttccccc    480
agcgagctgg ataccggagc atcgctggcg ttcatgaatt atattgccaa ctattacaac    540
gttgcctatt cagggcagaa tgctcatagc cagcgttcgc tatgggcatc atttaatggt    600
ggcatcaacc ttggtgcctg gcaatatcgt cagttatcca acatgacctg ggataatgac    660
aaagggaatc agtggaacaa tattcgtagc tatttgcaac gcccgctgcc cgccataaat    720
agccagttaa tgatggggca gcttatcacc agcggaagat tttctctgg actcagttat    780
cacggcgtta gtctcgcgac cgatgaacgt atgctgccgg actccatgcg cggctatgcg    840
ccgactattc gcggcgtggc cgcaacaaac gccagagtct cggtaatgca aaacggtcat    900
gaaatatatc agaccaccgt ggctcctggc cctttcgaga taaacgacct ataccccacc    960
agctacagcg gcgatctgga tgtcaccgtt acggaagcta acggcgcagt cagtcgtttc   1020
agtgtcccct tttcagccgt accagaatcg atgcgtccag gaacttcccg ttataacgtg   1080
gaagtaggta aaacgcagga tagtggtgat gactcgatgt ttggtgacct tacctggcag   1140
cacgggatga ctaatacgct gacatttaac agtggttcgc gtatcgctga tggctaccag   1200
gcgctgatgc tgggcggagt ctatggcagt tcgctggggg catttggggc aaacctcact   1260
tggtcccatg cgcgtgttcc cgaaagcgaa gcgcagagtg gttggatgtc gcaattaacc   1320
tggagtaaaa ctttccagcc tacttcaacc accgtctccc tggcaggtta tcgatactct   1380
accagcggct atcgtgatct ggctgatgtg ctgggagagc gtcatgctgc cagcaataaa   1440
cagtcatggg actccagcca gtggcgtcaa cagtcgcgct tcgatcttac gttaagtcag   1500
agccttgcga attacggcaa cctgtttgtg tcaggttcaa cacagaacta ccgtggcggc   1560
aagagccgtg atacacagct tcagttaggt tacagcaata gctttagcca tggcatcagt   1620
atgaaccttt ccgtcggacg ccaaagaatg ggcggctata aagacaattc tgatgatatg   1680
cagacggtaa catccctttc attctcattc ccacttggcg gcaatggacc tcgtgtacca   1740
agtcttagca acagctggac ccattcaact gacggtagct cgcaattaca aagctcgcta   1800
accggaatgc ttgatgaagc acagaccacc aactacagcc tgaacgtcat gcgcgatcaa   1860
caatataagc agacgacgct tagcggaaac atgcaaaaac gtttttcaca aactaccgtc   1920
ggattgaacg catcgaaggg ccaggattac tggcaggctt caggtaacgt acaaggcgcg   1980
atggctgtgc atggtggcgg cattactttc ggaccttatc tgggtgaaac gttcgccctg   2040
gtcgaagcta aaggcgcaga aggtgcaaaa gtctataact ccagtcagct ggaaattaat   2100
gacagtggct atgcgcttgt tccggcagta acgccctatc gctacaaccg tatatctctc   2160
gatccacaag gaatggatgg cgatgccgag ttggtcgaca gtgaaagaca ggtagcaccg   2220
gttgcgggtg cggcggtgaa agtaattttc cgtacccgtc ctggtaaagc gttgctgatt   2280
aaatcccgca tggcagatgg ttcggaactg ccaatgggag ccgatgtgct ggatgagaat   2340
aatacagtcg tcggtatagc cggtcagggg gggcaaattt acctccgcac agaacagaca   2400
aaaggccact tgtcagttcg ctggggtgaa ggtgctaacg atagctgcca attgcccttt   2460
gatatcagcg ggaaggacag caatagccct atcatccgcc tgaatgaaac ctgtcagtct   2520
tga                                                                 2523
```

<212> Type : DNA
<211> Length : 2523
SequenceName : SEQ ID 424
SequenceDescription :
Sequence
--------
<213> OrganismName : Escherichia coli 0157:H7
<400> PreSequenceString :

```
atgaaactcg ccgcctgttt tctgacactc cttcctggct tcgccgttgc cgccagctgg      60
acttctccgg ggttccctgc ctttagcgaa cagggaacgg gaacatttgt cagccacgcg     120
cagttgccca aggtacgcg tccactcacg ctaaattttg accagcagtg ctggcagcct     180
gcagatgcga taaaactcaa tcagatgctt tccctgcaac cttgtagcaa cacgccgcct     240
caatggcgat tgttcaggga cggcaaatat acgctgcaaa tagaoacccg ctccggtacg     300
ccaacattga tgatttccat ccagaacgcc gccgaaccgg tagcaaacct ggtccgtgaa     360
tgcccgaaat gggatggatt accgctcacg ctggatgtca gcgccacttt cccggaagga     420
gccgccgtac gggattatta cagccagcaa attgcgatag tgaagaacgg tcaaataacg     480
ttacaacccg ctgctaccag caacggttta ctcctgctgg aacgggcaga aactgacgcc     540
tctgcccctt tcgactggca taacgccacg gtttactttg tgctgacaga tcgtttcgaa     600
aacggcgatc ccagtaatga ccagagttac ggacgtcata aagacggtat ggcggaaatt     660
ggcacttttc acggcggcga tttacgcggc ctgaccaaca aactggatta cctccagcag     720
ttgggcgtta atgctttatg gataagcgcc ccatttgagc aaattcacgg ctgggtcggc     780
ggcggtacaa aaggcgattt cccgcattat gcctaccacg gttattacac acaggactgg     840
acgaatcttg atgccaatat gggcaacgaa gccgatctac ggacgctggt tgatagcgca     900
catcagcgcg gtattcgtat tctctttgat gtcgtgatga accacaccgg ctatgccacg     960
ctggcggata tgcaggagta tcagtttggc gcgttatatc tttctggtga cgaagtgaaa    1020
aaaacgctgg gtgaacgctg gagcgactgg aaacctgccg ccgggcaaac ctggcatagc    1080
tttaacgatt acattaattt cagcgacaaa acaggctggg ataaatggtg ggaaaaaaac    1140
tggatccgta ccgatatcgg cgattacgac aatcctggat tcgacgatct caccatgtcg    1200
ctagcctttt tgccggatat caaaaccgaa tcaactaccg cttctggtct gccggtgttc    1260
tataaaaaca aaacggatac ccacgctaaa gccatcgacg gctttacccc tcgcgattac    1320
ttaacccact ggttaagtca gtgggtccgc gactatggga ttgatggttt tcgggtcgat    1380
accgccaaac atgttgagtt gcccgcttgg cagcaactga aaaccgaagc cagcgccgcg    1440
cttcgcgaat ggaaaaaagc taaccccgac aaagcattag atgacaaacc tttctggatg    1500
accggtgaag cctggggcca cggcgtgatg caaagtgact actatcgcca cggcttcgat    1560
gcgatgatca atttcgatta tcaggagcag gcggcgaaag ctgtcgattg tattgcgcag    1620
atggatacga cctggcagca aatggcggag aaattgcagg gtttcaacgt gttgagctac    1680
ctctcgtcgc atgatacccg tctgttccgt gaagggggcg acaaagcagc agagttatta    1740
ctattagcgc caggcgcggt acaaatcttt tatggcgatg aatcctcgcg tccgttcggt    1800
cctacaggtt ctgatccgct gcaaggtaca cgttcggata tgaactggca ggatgttagc    1860
ggtaaatctg ccgccaacgt cgcgcactgg cagaaaatca gccagttccg cgcccgccat    1920
cccgcaattg gcgcgggcaa acaaacgaca ctttcgctga agcagggcta cggctttgtt    1980
cgtgagcatg gcgacgataa agtgctggtc atctgggctg ggcaacagtg a             2031
```

<212> Type : DNA
<211> Length : 2031
SequenceName : SEQ ID 425
SequenceDescription :
Sequence
--------
<213> OrganismName : Escherichia coli 0157:H7
<400> PreSequenceString :

```
atgccacaac gacaccacca gggacataaa cgcacaccga aacagttggc gctcattatc      60
aaacgctgtt tgccgatggt gctcactggc agcggcatgc tttgcactac cgctaacgcc     120
gaagagtatt atttcgaccc cattatgctg gaaaccacaa aaagtggtat gcaaacaacc     180
gatctgtcac gtttttcaaa aaaatacgca caactaccag gaacttatca ggttgatatc     240
tggctgaata aaaagaaggt ttcacagaaa aaaattacat ttaccgccaa tgcagagcaa     300
cttctgcagc cacagtttac ggtagaacaa ctacgtgagc tgggtattaa ggtggatgaa     360
atcccggcgc tggctgaaaa agatgacgat agcgtgatca actcgcttga acaaatcatt     420
cccggtacag ctgctgaatt tgatttcaat catcagcgac ttaatttgag cattccccaa     480
attgcactgt accgtgatgc aagaggttac gtctcccctt ctcgttggga cgatggtata     540
ccaacgctgt ttaccaacta ctcgtttaca ggttctgata accgttaccg ccagggcaat     600
cgtagccaac gacagtacct aaatatgcaa aatggtgcca attttggccc ctggcgatta     660
cgtaactatt ctacgtggac acgcaacgat caggcgtcaa gctggaacac tatcagtagt     720
tatttacaac gtgatatcaa ggcgttgaag tctcagttgc ttctgggaga aagcgccacc     780
agcggcagta tttttccag ctacaacttt actggcgtgc aactcgcttc cgacgataat     840
atgttgccaa acagccagcg cggatttgcc ccaacggtac gcggtatcgc aaacagtagt     900
gcaatcgtga ctatcaggca aaatggttat gtgatctatc aaagcaacgt gccagcgggt     960
gcctttgaaa ttaacgatct ctacccctct tccaacagcg gcgatttaga agtcacgatt    1020
gaagaaagtg acggtacgca acgtcgcttt atccagcctt attcttcatt acccatgatg    1080
```

```
cagcgacctg ggcatctaaa atatagcgcg accgctggac gctatcgcgc tgatgcaaac    1140
agtgatagca aggaacccga atttgctgaa gccacggcaa tatatggttt gaataatact    1200
tttacgctgt atggcggcct gctcggttct gaagattatt atgcgctggg gatcggtatc    1260
ggcggcacac ttggcgcact gggcgcgttg tcgatggata tcaacagagc tgacacccaa    1320
ttcgataacc agcactcttt tcatggctat caatggcgta cgcagtacat caaagatatc    1380
ccggaaacca acaccaatat cgctgtcagc tactatcgct ataccaacga tggctatttt    1440
agttttgatg aagccaatac ccgcaattgg gactataaca gtcgccaaaa aagtgaaatt    1500
caattcaaca tcagccagac aatatttgat ggggtaagtc tgtatgcctc cggttcacag    1560
caagactatt ggggcaataa cgagaaaaac aggaatatct ctgttggggt ttccggccag    1620
caatggggaa ttggttacag cctgaattat caatacagcc gctacactga tcaaaataat    1680
gaccgcgcac tctctttgaa tctcagtatt ccgttagaac gctggttacc gcgtagccgg    1740
gtttcctatc agatgaccag ccagaaagat cgcccaaccc aacatgaaat gcgtcttgat    1800
ggctcactgc tggatgatgg tcgcctgagc tatagtctgg aacaaagtct ggatgacgat    1860
aacaaccata acagtagcgt gaacgccagt taccgttcac cttatggaac cttcagtgcc    1920
ggatacagtt acggtaatga cagtagccaa tacaattacg gcgttaccgg cggcgtggtt    1980
atccatcctc atggtgtgac gctctcgcaa tatctgggca acgcttttgc gcttattgat    2040
gctaacgggg catctggcgt gaggatacaa aactatccgg ggattgctac tgatcccttt    2100
ggctatgcag tggttcctta tctcacaact tatcaggaaa accgtctctc ggtagatact    2160
acgcagctgc ccgataacgt cgatcttgaa caaacaacac agtttgtggt gcccaacaga    2220
ggtgcaatgg tagcggcgcg tttcaacgcc aatatcggtt atcgcgtact tgttacagtc    2280
agcgatcgca acggtaaacc gttgcccttt ggcgctcttg ccagcaacga tgatacgggg    2340
caacaaagta tcgtcgatga gggcggcata ctatatctct ctgggatatc gagtaaatca    2400
caaagctgga ctgtacgctg gggaaatcag gcagatcaac aatgtcagtt tgcttttagt    2460
acaccggatt cagaaccaac aacctctgta ttacaaggca cagcgcagtg ccattaa       2517
```

<212> Type : DNA
<211> Length : 2517
SequenceName : SEQ ID 426
SequenceDescription :
Sequence
--------
<213> OrganismName : Escherichia coli 0157:H7
<400> PreSequenceString :

```
atgatgttca gaaatagaat attactaata tttatattgt gggctaattt tacctgggct      60
gggtgtcgta ctactgcatc attaaatatt acagatggta ttaatgttgg ggagatttta     120
gcgaatgaaa cttcctttag taaaagtgtc gtgtttactg ggatatcttg tgatacgagc     180
acggataaaa tagtttataa aaatatccaa agtgattggg ttgaagttgg gccttttggt     240
aatggcgaaa aattaaaggt taaaatagag tctttaggta aaaccagcga cacaattggg     300
aaatccagca atgcgcaggc agtattacct tatgtggtta aaatagccag aggcacacct     360
gattttactg gagaaagaaa atctacctgg tttatttcag ataccgtgat tgcaaatatt     420
ggcggtgagt catcgtcatc catcgatttt tggttgggta tttgtaaggc attgaagttt     480
aactggtgtg tgaattatct caccagcaaa ctggcggggg atacatttac gcttgggtta     540
aatatttcct attatcctaa aaatacgacc tgtaagcctg aaaacaccgt tataaaagta     600
gatgatatcg ccttgttcca gctcagaaat cagggaaaga ttgcggcgaa cagtaaggaa     660
ggaacaatta cgttgaaatg tgataatctt ttcggcgaca aaaaacaagc atcgcggaat     720
atggttgtat atctttctag cagtgactta gttaaaggaa gtaatactat tttgcgtggt     780
aaaacagata atggtgtagg gtttgtgttg gatctaacag aaccaccaaa agggactgag     840
gctgccatta aaatttcggc caacggcgat cagggcgcgg cgacatcatt atggaaaaca     900
gataaaccag gagtttcatt aaatagcaac attattaata taccagtcat ggccagttac     960
tatgtatatg atgaaaaaaa agttaaatct ggcgcactgg aagcaaccgc attaatcaac    1020
gtgaaatacg attaa                                                     1035
```

<212> Type : DNA
<211> Length : 1035
SequenceName : SEQ ID 427
SequenceDescription :
Sequence
--------
<213> OrganismName : Escherichia coli 0157:H7
<400> PreSequenceString :

```
atgattaaaa aagcttcgct gctgacggcg tgttctgtca cagccttttc cgcttgggca      60
caggatacca gcccggatac tctcgtcgtt actgctaacc gttttgaaca gccgcgcagc     120
actgtgcttg caccaaccac cgttgtgacg cgtcaggata tcgaccgctg gcagtcgacc     180
tcggttaatg atgtgctgcg ccgtcttccg ggcgtcgata tcacccaaaa cggcggttca     240
ggtcagctct catctatttt tattcgcggt acaaatgcca gtcatgtgtt ggtgttaatt     300
gatggcgtac gcctgaatct ggcggggggg agtggttctg ccgaccttag ccagttccct     360

attgcgcttg tccagcgtgt tgaatatatc cgtgggccac gctccgccgt ttatggttcc     420
gatgcaatag gtggggtggt gaatatcatc acgacgcgcg atgaacccgg aacggaaatt     480
tcagcagggt ggggaagcaa tagttatcaa aactatgatg tctctacaca gcaacaactg     540
ggggataaga cacgagtaac gttgttgggc gattatgccc atactcatgg ttatgatgtt     600
gttgcctatg gtaataccgg aacgcaagcg cagccagata acgatggttt tttaagtaaa     660
acgctttatg gcgcgctgga gcataacttt actgatgcct ggagcggctt tgtgcgcggc     720
tatggctatg ataaccgtac caattatgac gcgtattatt ctccgggttc accattggtc     780
gatacccgta aactctatag tcaaagttgg gacgccgggc tgcgatataa cggcgaactg     840
attaaatcac aactcattac cagctatagc catagcaaag attacaacta cgatccccat     900
tatggtcgtt atgattcgtc ggcgacgctc gatgagatga agcaatacac cgtccagtgg     960
gcaaacaaca tcatcattgg ccacggtaat gttggtgcgg gtgttgactg gcagaagcag    1020
agcacggcac cgggcacagc ttatgttaag gatggatatg atcaacgtaa taccggcatc    1080
tatctgaccg ggctgcaaca agtcggcgat tttacctttg aaggcgcagc acgcagcgac    1140
gataactcac agtttggtcg tcatggaacc tggcaaacca gcgccggttg ggaattcatc    1200
gaaggttatc gcttcattgc ttcctacggg acatcttata aggcaccaaa tctggggcaa    1260
ctgtatggct tctacggaaa tccgaatctg gacccggaga aaagcaaaca gtgggaaggc    1320
gcgtttgaag gcttaaccgc tggggtgaac tggcgtattt ccggatatcg taacgatgtc    1380
agtgacttga tcgattatga tgatcacacc ctgaaatatt acaacgaagg gaaagcgcgg    1440
attaagggcg tcgaggcgac cgccaatttt gataccggac cactgacgca tactgtgagt    1500
tatgattatg tcgatgcgcg caatgcaatt accgacacgc cgttgttacg ccgtgctaaa    1560
cagcaggtga aataccagct cgactggcag ttgtatgact tcgactgggg tattacttat    1620
cagtatttag gcactcgcta tgataaggat tactcatctt atccttatca aaccgttaaa    1680
atgggcggtg tgagcttgtg ggatcttgcg gttgcgtatc cggtcacctc tcacctgaca    1740
gttcgtggta aaatagccaa cctgttcgac aaagattatg agacagtcta tggctaccaa    1800
actgcaggac gggaatacac cttgtctggc agctacacct tctga                    1845
```

<212> Type : DNA
<211> Length : 1845
SequenceName : SEQ ID 428
SequenceDescription :
Sequence
--------
<213> OrganismName : Escherichia coli 0157:H7
<400> PreSequenceString :

```
atgaaaaaca aattgttatt tatgatgtta acaatactgg gtgcgcctgg gattgcagcc      60
gcagcaggtt atgatttagc taattcagaa tataacttcg cggtaaatga attgagtaag     120
tcttcattta atcaggcagc cataattggt caagctggga ctaataatag tgctcagtta     180
cggcagggag gctcaaaact tttggcggtt gttgcgcaag aaggtagtag caaccgggca     240
aagattgacc agacaggaga ttataacctt gcatatattg atcaggcggg cagtgccaat     300
gatgccagta tttcgcaagg tgcttatggt aatactgcga tgattatcca gaaaggttct     360
ggtaataaag caaatattac acagtatggt actcaaaaaa cggcaattgt agtgcagaga     420
cagtcgcaaa tggctattcg cgtgacacaa cgttaa                               456
```

<212> Type : DNA
<211> Length : 456
SequenceName : SEQ ID 429
SequenceDescription :
Sequence
--------
<213> OrganismName : Escherichia coli 0157:H7
<400> PreSequenceString :

```
atgaacattt ttgcatattt actggtactt gtattttcca tgagcatgag cagcagcgcg     60
tttgccagcg tggtaatgac cggaacccgt attattttcc ctggtgacgc aaaggaaaaa    120
accatccagt tgcgaaatac cagcgatcag ccctatatca ttaatatcca tgttgaggat    180
gaacgtggtt ctgacaagaa tgtaccgttt atgccaaccc cgcagacatt tcgcatggaa    240
gctgccgcag gtcaggcgtt acgcctgctc tacactggta ataatttacc gcaggatcgc    300
gagtctgttt tctggtttag tttcagtcaa ctaccttatc tgaataagaa tgataaaagt    360
cagaaccagc tcatcctggc cctgactaat cgagtcaaaa ttttctatcg tcccagctcg    420
attgtcggta aatccagtga cgcacccaaa aacctgactt accaggtaaa acagaaccgc    480
attgaagtga cgaatcccac gggctattac gtcacaattc gcgccgctga actgcttaat    540
aatggtaaaa aagtccccct cgcgaattcg gtaatgattg ctcctcaaag cacaactgaa    600
tggacactac cctctggcat cagtgtcgct cccggtgcgc agatccattt agtgaccgtc    660
aacgactatg gcgtaaatgt tacgtctgag catgccttat aa                       702
```

<212> Type : DNA
<211> Length : 702
SequenceName : SEQ ID 430
SequenceDescription :
Sequence
--------
<213> OrganismName : Escherichia coli 0157:H7
<400> PreSequenceString :

```
atgaaaagac ttcacaagag gttcctctta gctacgtttt gcgcgttatt aacagcaact     60
ctccaggccg ccgatgtcac tatcactgtt aatggtcggg tagtcgctaa accctgcact    120
attcaaacca aagaagctaa cgttaatctc ggggatcttt atacgcgcaa tctgcaacaa    180
cctggttctg catctggctg gcacaatatt actttgtcat taaccgattg tccggctgaa    240
acaagtgcag tgacggcaat cgtgacaggt tcaactgaca atacgggtta ttacaaaaat    300
gaaggtactg ccgaaaatat tcagatagag cttagggatg accaggatgc gacgttaaaa    360
aatggcgata gcaaaacggt tattgttgat gagatcactc gtaatgcaca gtttccactt    420
aaggcaagag ctatcacggt gaatggaaac gcaagccagg gaacgatcga ggcgctaatc    480
aatgtgatct acacctggca ataa                                           504
```

<212> Type : DNA
<211> Length : 504
SequenceName : SEQ ID 431
SequenceDescription :
Sequence
--------
<213> OrganismName : Escherichia coli 0157:H7
<400> PreSequenceString :

```
atgcgggcga aacttctggg aatagtcctg acaacccccta ttgcgatcag ctcttttgct     60
tctaccgaga ctttatcgtt tactcctgac aacataaatg cggacattag tcttggaact    120
ctgagcggaa aaacaaaaga gcgtgtttat ctagccgaag aaggaggccg aaaagtcagt    180
caactcgact ggaaattcaa taacgctgca attattaaag gtgcaattaa ttgggatttg    240
atgccccaga tatctatcgg ggctgctggc tggacaactc tcggcagccg aggtggcaat    300
atggtcgatc aggactggat ggattccagt aaccccggaa cctggacgga tgaaagtaga    360
caccctgata cacaactcaa ttatgccaac gaatttgatc tgaatatcaa aggctggctc    420
ctcaacgaac ccaattaccg cctgggactc atggccggat atcaggaaag ccgttatagc    480
tttacagcca gaggtggttc ctatatctac agttctgagg agggattcag agatgatatc    540
ggctccttcc cgaatggaga aagagcaatc ggctacaaac aacgttttaa aatgccctac    600
attggcttga ctggaagtta tcgttatgaa gattttgagc taggtggcac atttaaatac    660
agcggctggg tggaagcatc tgataacgat gagcactatg acccaggaaa aagaatcact    720
tatcgcagta aagtcaaaga ccaaaattac tattctgttt cagtcaatgc aggttattac    780
gtaacaccta acgcaaaagt ttatgttgaa ggcacatgga atcgggttac gaataaaaaa    840
ggtaatactt cactttatga tcacaatgat aacacttcag actacagcaa aaatggtgca    900
ggcatagaaa actataactt catcactact gctggtctta agtacacctt ttaa          954
```

<212> Type : DNA
<211> Length : 954
SequenceName : SEQ ID 432
SequenceDescription :
Sequence

--------
<213> OrganismName : Escherichia coli 0157:H7
<400> PreSequenceString :

```
ttgtttttta agcgaggaaa gattttgagt gcgggacgcc tgaataaaaa atctctgggt 60
atcgtgatgt tgttatcggt tggactgctt ttggcgggct gttcgggtag caaatcatcc 120
gatacaggaa cgtattccgg ctccgtttac accgtgaaac ggggggatac gctatatcgt 180
atttcgcgca ccacgggaac cagcgtaaaa gagctggcgc gactgaacgg catttccccc 240
ccttacacca ttgaagttgg tcagaaacta aaactgggtg gggcgaaaag tagcagtagt 300
acacgtaaat caaccgccaa atcaacgacc aaaaccgcat cggttacacc gtcatcagcg 360
gtaccgaaat cttcctggcc gccagtaggg caacgttgtt ggttatggcc aacgacaggg 420
aaagttatca tgccgtattc gacagcagat ggcggcaata aagggattga tatctcagct 480
ccacggggta cacctattta cgccgcgggt gcaggaaagg tggtgtatgt gggcaaccag 540
ctgcgtggct acggtaatct catcatgatt aaacacagtg aagattacat tacggcttac 600
gcccataatg acacgatgct ggtaaataat gggcaaagcg tgaaggctgg gcaaaaaatc 660
gccaccatgg ggagcacgga tgcggcatct gttcgcctgc atttccagat tcgttaccgt 720
gcaacggcaa ttgatccgct acgttacttg ccgcctcagg gcagcaagcc aaaatgctga 780
```

<212> Type : DNA
<211> Length : 780
SequenceName : SEQ ID 433
SequenceDescription :
Sequence
--------
<213> OrganismName : Escherichia coli 0157:H7
<400> PreSequenceString :

```
atgccaacac caaatcctct ggcaccggtg aaaggggccg ggaccacact gtgggtttat 60
aacgggaacg gcgacccata tgcaaacccg ctttcagaca atgactggtc gcgtctggca 120
aaggttaaag acctgacgcc cggcgaactg accgctgagt cctatgacga cagttatctc 180
gatgatgaag atgcggactg ggccgcgacc ggacaggggc agaaatccgc tggagatacc 240
agcttcacgc tggcgtggat gcccggagag caggggcagc aggcgctgct ggcgtggttt 300
aatgaaggtg atacccgtgc ctataaaatc cgcttcccga acggcacggt cgatgtgttc 360
cgcggctggg tcagcagtat cggtaaggcg gtgacggcga aggaagtgat cacccgcacg 420
gtgaaagtca ccaacgtggg acgtccgtcg atggcagaag atcgcagcac ggtaacagcg 480
gcaaccggca tgaccgtgac gcctgccagc acctcggtgg tgaaagggca gagcacgacg 540
ctgaccgtgg cattccagcc ggaaggcgca accgacaaga gcttccgtgc ggtgtctgcg 600
gataaaacaa aagccaccgt gtcggtcagt ggtatgacca tcaccgtgaa aggtgttgct 660
gcaggcaagg tcaacattcc ggtcgtatcc ggtaatggtg agtttgctgc ggttgcagaa 720
atcaacgtca ccgccagtta a 741
```

<212> Type : DNA
<211> Length : 741
SequenceName : SEQ ID 434
SequenceDescription :
Sequence
--------
<213> OrganismName : Escherichia coli 0157:H7
<400> PreSequenceString :

```
atgtctgctt tgtatgaacg ctcacagctg acgcaggtga tgatttcatc tgccccggcg 60
actgctgaaa ctatggataa ggcggaatat ctgcgcctgg actgcaccat caaggaagtg 120
cagttcaccg ccgggcagaa acaggatatt gatgtgacca cgctctgctc cacagagcag 180
gagaacatca atggtctggg ggcgtcgtct gagatttcca tgtcgggcaa tttttatctg 240
aatcaggccc agaacgccct gcgtgatgcc tatgacaatg acgcgttgta tgcgtttaag 300
gtgctgtttc cgtccggtaa gggctttaaa ttcctggcgg aagtgcgcca gcacacctgg 360
tcatccggta ccaacggcgt ggtggctgca acgttctcac tgcgtctgaa aggcaaaccg 420
gtgtcctttg tggtaccgct ggcgtttgtg aaaaatctgg ataagacact taccgtgaat 480
accggtgcgc tgctgacaat gtcagtcagt gccaacgggg gaacgccgcc gtataaatac 540
gcctggaaga aggatggtca gccggttgac gggcagacga cagacacctt cagtaagcca 600
ggtgcgcagt ccgctgatgc ggggaaatat acctgtgtgg tgaccgattc ggcagagaaa 660
gcacagagtg tgacgtctgt tgaatgcacc gtgacagtga gcgcagccgc cggataa 717
```

<212> Type : DNA

<211> Length : 717
SequenceName : SEQ ID 435
SequenceDescription :
Sequence
--------
<213> OrganismName : Escherichia coli 0157:H7
<400> PreSequenceString :

```
atgaaaaaga gcactctggc attagtggtg atgggcattg tggcatctgc atccgtacag      60
gccgcagaaa tatataacaa agacggtaat aaactggatg tctatggcaa agttaaagcc     120
atgcattata tgagtgataa cgacagtaaa gatggcgacc agagttatat ccgttttggt     180
tttaaaggcg aaacacaaat taacgatcaa ctgactggtt atggtcgttg ggaagcggag     240
tttgccggaa ataaagcgga gagtgatact gcacagcaaa aaacgcgtct cgcttttgcc     300
ggattgaagt ataaagattt gggttctttc gactatggcc gtaacctggg cgcgttgtat     360
gacgtggaag cctggaccga tatgttcccg gaatttggtg gcgactcctc ggcgcagacc     420
gacaacttta tgaccaaacg cgccagcggt ctggcgacgt atcggaacac cgacttcttc     480
ggcgttatcg atggcctgaa cttaaccctg caatatcaag ggaaaaacga aaaccgcgac     540
gttaaaaagc aaaacggcga tggcttcggc acgtcattga catatgactt tggcggcagc     600
gatttcgcca ttagtggggc ctataccaac tcagatcgca ccaacgagca gaacctgcaa     660
agccgtggca caggcaagcg tgcagaagct tgggctacag gtctgaaata cgatgccaat     720
aatatttatc tggcaacttt ttattctgaa acacgcaaaa tgacgccaat aactggcggc     780
tttgccaata agacacagaa ctttgaagcg gtcgctcaat accagtttga ctttggtctg     840
cgtccatcgc tgggttatgt cttatcgaaa gggaaagata ttgaaggtat cggtgatgaa     900
gatctggtca attatatcga tgtcggggct acatattatt tcaacaaaaa tatgtcagcg     960
tttgttgatt ataaaatcaa ccaactggat agcgataaca aattgaatat taataatgat    1020
gatattgtcg cggttggcat gacctatcag ttttaa                             1056
```

<212> Type : DNA
<211> Length : 1056
SequenceName : SEQ ID 436
SequenceDescription :
Sequence
--------
<213> OrganismName : Escherichia coli 0157:H7
<400> PreSequenceString :

```
atgcgtgtca aacatgcagt agttctactc atgcttattt cgccattaag ttgggctgga      60
accatgactt tccagttccg taatccaaac tttggtggta acccaaataa tggcgctttt     120
ttattaaata gcgctcaggc ccaaaactct tataaagatc cgagctataa cgatgacttt     180
ggtattgaaa caccctcagc gttagataac tttactcagg ccatccagtc acaaatttta     240
ggtgggctac tgtcgaatat taataccggt aaaccgggcc gcatggtgac caacgattat     300
attgttgata ttgctaaccg cgatggtcaa ttgcagttga acgtgacaga tcgtaaaacc     360
ggacaaacct cgaccatcca ggtttcgggt ttacaaaata actcaaccga tttttaa        417
```

<212> Type : DNA
<211> Length : 417
SequenceName : SEQ ID 437
SequenceDescription :
Sequence
--------
<213> OrganismName : Escherichia coli 0157:H7
<400> PreSequenceString :

```
atgaaaagaa aagttctggc aatgctggtc ccggcgttat tagttgctgg cgcagcaaat    60
gcggctgaaa tttataataa agatggcaat aaactggatt tgtacggaaa agtagcgggc   120
ctgcactact tctctgatga tgctagcagc gatggcgaca tgtcatatgc ccgtatcggt   180
ttcaaaggtg aaactcagat cgctgaccaa ttcactggtt atggtcagtg ggaatttaac   240
attggcgcaa acggtcctga aagcgacaag ggtaataccg caacgcgtct ggcatttgca   300
ggtttcggct ttggtcagaa tggtactttc gactatggtc gtaactacgg tgtcgtatat   360
gacgtagaag catggaccga tatgctgcca gaatttggtg gagataccta tgctggcgct   420
gacaacttca tgaacggtcg tgctaacagc gtagcaacct atcgtaacaa tggtttcttt   480
ggtcaagttg atggtctgaa ctttgcactc cagtatcagg gtaacaacga gaaaagcgga   540
ttatttgatc aagaaggttc aggtaacggt aatggacgta aacttgctaa agagaacggc   600
gacggttcag tatgtccact tcctatgact ttgactttgg tttaa                   645
```

<212> Type : DNA
<211> Length : 645
SequenceName : SEQ ID 438
SequenceDescription :
Sequence
--------
<213> OrganismName : Escherichia coli 0157:H7
<400> PreSequenceString :

```
ttgaacacgg tgactctgga aggaggaacc ttcaataaca acggaacgct taatgacgtc    60
gtaaaaattg agaaaaacag caacgcggta attaataaca ccggttccct gtcaacttta   120
caacttcacg atggtacggt gaataacagc ggtattgcgt cggcgcgcgt taacgctcag   180
ggcgatgcgg tattcaataa ccttgcaggc ggcgaggcgc gtaaaggcgc gattctctat   240
aactctgcgg tagtgaataa cgcgggtacc tggaaaatgg gttatcagga tgaaaacaac   300
aatgccggga cgctggatat tgacgataag tcaacgttca acaacagcgg caaactcatc   360
cttgataaca gcaaaaacgc cattcgcttc cagggcagca atgctaacgc cacgttatat   420
aacaccggtg aaatgacgct ggatgccgca ttaggtgcgg gcgctattct ctacgacgat   480
ggcgccagcg agtttattaa caagggcgtg gtggatgcga aagtcaccgt ggcggtaagt   540
actgccggtg cgacagaaag cgatgccttc ttgtggaacc aggatggcgg ggtaattaac   600
ttcgataaag acaacgccag cgcggttaaa ttcacccaca acaactatgt tgctctcaac   660
gatggtgtaa tgaacatcag cggcaacaac gccgtggcga tggaaggcga taaaaacgca   720
cagctggtta acaacggcgt tatcaatctc ggtaccgaag gcaccaccga taccggattg   780
actggtatgc aactggatgc caatgccacc gccgatgcgg taattgagaa caacggcacg   840
atcaatattt ttgctaacga ctcgtttgcg ttcagcgtac tgggcacaga aggtcatatc   900
gttaacaacg gtacggtggt gattgccgac ggcgtgactg gttcggggtt gattaagcag   960
ggcgacagcg tgaatgtgga aggggtgaac ggcaacagcg gtaacaatac cgaagtgcat  1020
tacaccgact acacgttgcc ggacatgcca aacacttaca ccacttcacc tttcagtgaa  1080
acgactgata gcggtagtag cgatggcagc agtaacaacc tcaacggcta tatcgtcggt  1140
accaacgttg acggcagcgc cggtaaactg aaggtcaaca acgccagcat gaatggtgtc  1200
gggatcaaca ccggtttcgc cgccggtacg gcagacacca cggtcagttt cgacaacgtg  1260

gtggaaggca tcaacctgac cgacgccgat gccatcacct caacgtccgt ggtatggacc  1320
gccaaaggca gcaccgatgc cagcggcaac gttgacgtca tcatgagcaa aaacgcctac  1380
accgatgtgg cgaccgatgc ttcggtgaac gatgtggcga aggcactgga tgcgggttac  1440
accaataacg agctgtatac cagcctgaac gtgggcacca ctgctgaact gaatagcgcc  1500
ctgaagcagg tgagcggtag ccaggcgacc acggtattcc gtgaagcgcg tgtgttaagc  1560
aaccgcttca gcatgctggc ggatgccgcg ccgaaggtgg gcaatggcct ggcctttaac  1620
gtggtggcga aaggtgaccc gcgtgcggaa ctcggaaata acaccgagta tgacatgctg  1680
gcactgcgta aaaccgttga cctgagcgaa agccagagca tgagcctgga atacggtatc  1740
gcgcgtctgg atggcgacgg tgcgcagaaa gcgggcgaca atggcgtaac cggcggctac  1800
agccagttct ttggcctgaa gcaccagatg tccttcgaca atggtatgcg ttggaacaac  1860
gcgctgcgtt atgacgtgca taatctcgac agcagccgct cggtcgctta cggcgacgtc  1920
agcaaaaacgg cggatacgga tgtgaaacag cagtacctgg agttgcgtag cgaaggggcg  1980
aaaacctttg agccgcgcga agggctgaaa atcacccgt acgccggagt gaaactgcgt  2040
cactcgctgg aaggcggcta tcaggagcgc aatgccggag actttaacct gagcatgaac  2100
agcggcagcg aaacggcggt ggacagcatc gtcgggctga aactggacta cgcagggaaa  2160
ggcggctgga gcgcgaatgc gacgctggaa ggcgggccga acctgagcta cagcaagagc  2220
cagcgcacgg caagccttgc aggggcaggc agccagcact ttaacgtcga tgacggtcag  2280
aagggcggcg gtatcaacag cctggcgagc gtcggcgtga agtacagtag caaagaaagt  2340
tcgctgaatc tggatgcgta tcactggaaa gaggacggca tcagcgacaa aggcgtgatg  2400
ctgaacttta agaaaacgtt ctaa                                         2424
```

<212> Type : DNA

<211> Length : 2424
SequenceName : SEQ ID 439
SequenceDescription :
Sequence
--------
<213> OrganismName : Escherichia coli 0157:H7
<400> PreSequenceString :

```
atgcttaatg gaattagtaa cgctgcttct acactagggc ggcagcttgt aggtatcgca      60
agtcgagtga gctctgcggg gggaactgga ttttctgtag cccctcaggc cgtgcgtctt     120
actccggtga aagttcattc cccttttttct ccaggctcgt cgaatgttaa tgcgagaacg    180
atttttaatg tgagcagcca ggtgacttca tttactccct ctcgtccggc accgccgcca     240
ccgacaagtg gacaggcatc cggggcatcc cgacctttac cgcccattgc acaggcatta     300
aaagagcact tggctgccta tgaaaaatcg aaaggtcctg aggctttagg ttttaagccc     360
gcccgtcagg caccgccgcc accgacaagt ggacaggcat ccggggcatc ccgaccttta     420
ccgcccattg cacaggcatt aaaagagcac ttggctgcct atgaaaaatc gaaaggtcct     480
gaggctttag gttttaagcc cgcccgtcag gcaccgccgc caccgacaag tggacaggca     540
tccggggcat cccgaccttt accgcccatt gcacaggcat taaaagagca cttggctgcc     600
tatgaaaaat cgaaaggtcc tgaggcttta ggttttaagc ccgcccgtca ggcaccaccg     660
ccaccgacag ggcctagtgg actaccgccc cttgcacagg cattaaaaga tcatttagct     720
gcctatgagc aatcgaagaa agggtaa                                         747
```

<212> Type : DNA
<211> Length : 747
SequenceName : SEQ ID 440
SequenceDescription :
Sequence
--------
<213> OrganismName : Escherichia coli 0157:H7
<400> PreSequenceString :

```
atgaacaaga agattcattc cctggccttg ttggtcaatc tggggattta tggggtagcg      60
caggcacaag agccgaccga tactcctgtt tcacatgacg atactattgt cgttaccgcc     120
gccgagcaga acttacaggc gcctggcgtt tcgaccatca ccgcagatga aatccgcaaa     180
aaccccgttg cccgcgatgt ttcggagatc atccgtacca tgccaggcgt taacctgacc     240
ggtaactcca ccagtggtca gcgagggaat aaccgacaga ttgatattcg cggtatgggt     300
ccggaaaaca cgctgatttt gattgacggc aagccggtaa gcagccgtaa ctcggtgcgt     360
cagggctggc gtggcgagcg cgatacccgt ggtgatacct cctgggtgcc acctgaaatg     420
attgaacgta ttgaagttct gcgtggtccg gcagctgcgc gttatggcaa cggcgcggcg     480
ggcggcgtgg ttaacatcat taccaaaaaa ggcagcggcg agtggcacgg ctcctgggac     540
gcatatttca atgcgccaga acataaagag gaaggtgcca ccaaacgcac taactttagc     600
ctgaccggtc cgctgggcga cgaattcagc ttccgcttgt atggcaacct cgacaaaacc     660
caggctgacg cgtgggatat caaccagggt catcagtccg cgcgtgccgg aacgtatgcc     720
acgacgttac cagccgggcg cgaaggggtg atcaataaag atattaatgg cgtggtgcgc     780
tgggacttcg caccattgca atcgctggaa ctggaagcgg gttacagccg ccagggtaac     840
ctgtatgcgg gcgataccca gaacaccaac tctgacgctt acactcgctc gaaatatggc     900
gatgaaacca accgcctgta tcgccagaac tactcgctga cctggaacgg tggctgggat     960
```

```
aacggcgtga ccaccagcaa ctgggtgcag tacgaacaca cccgtaactc gcgtattccg     1020
gaaggtctgg cgggcggtac cgaagggaaa tttaacgaaa aagcgacaca ggatttcgtc     1080
gataacgatc ttgatgacgt gatgctgcat agcgaagtta acctgccgat tgatttcctc     1140
gttaaccaga cgctgacgct gggtacggag tggaatcagc agcggatgaa ggacttaagt     1200
tccaacaccc aggcactgac cggaacgaat accggtggcg ctattgatgg cgtgagtgcc     1260
accgaccgta gcccgtattc aaaagcagaa atttctcgc tgtttgccga aaacaacatg     1320
gagctgactg acagcaccat cgtaacgccg gggctgcgtt tcgatcatca cagtattgtc     1380
ggcaataact ggagcccggc gctgaacata tcgcaaggtt taggcgatga cttcacgctg     1440
aaaatgggca ttgcccgcgc ctataaagcg ccgagcctgt accagactaa cccgaactac     1500
attctctaca gtaaaggtca gggctgctat gccagcgcgg gcggctgcta tctgcaaggt     1560
aacgatgacc tgaaagcaga aaccagcatc aacaaagaga ttggtctgga gttcaaacgc     1620
gacggctggc tggcgggcat cacctggttc cgtaacgatt atcgcaataa gattgaagca     1680
ggctatgttg ctgtagggca aaacgcagtc ggcaccgatc tctatcagtg ggataacgtg     1740
ccgaaagcgg tggttgaagg tctggaagga tcgttaaacg taccggttag cgaaacggtg     1800
atgtggacta ataacatcac ttatatgctg aagagtgaaa acaaaaccac gggcgaccgt     1860
ttgtcgatca tcccggagta tacgttgaac tcaacgctga gctggcaggc acgagaagat     1920
ttgtcgatgc aaacgacctt cacctggtac ggtaagcagc agccgaagaa gtacaactat     1980
aaaggtcagc cagcggttgg accggaaacc aaagaaatta gtccttacag cattgttggg     2040
ctgagcgcga cgtgggatgt gacgaagaat gtcagtctga ccggcggcgt ggacaatctg     2100
ttcgacaaac gtttgtggcg tgcgggtaat gcccagacca cgggcgattt ggcaggggcc     2160
aactatatcg ccggtgccgg ggcgtatacc tataacgagc cgggacgtac gtggtatatg     2220
agcgtaaaca ctcacttctg a                                               2241
```

<212> Type : DNA
<211> Length : 2241
SequenceName : SEQ ID 441
SequenceDescription :
Sequence
<213> OrganismName : Escherichia coli 0157:H7
<400> PreSequenceString :

```
ttgggagggc gatttagtct caggtacaaa aaactctcat atagattcgt ttttctgacc       60
ttggcaggtt gttcttcagt aggcaaccag tcattgaaaa atgagacgca ggaaagtgtg      120
aaaaccaaaa ttgttaaagg caaaactaca aaacaggacg tgttagcatc gttcggtgaa      180
cctgacagcc gttctttgat cgatggtgaa gaacaatggt catacactat gtataacagc      240
cagtccaaag caacctcttt catccccgtt gtgggactgc ttgcaggtgg cgcagactca      300
caaactaaat ctctgacagt ttctttcaaa ggcgaaaaag tcagcacata catctttaat      360
gctggaacaa gcaacgtgaa gactggcatt ttttag                                396
```

<212> Type : DNA
<211> Length : 396
SequenceName : SEQ ID 442
SequenceDescription :
Sequence
--------
<213> OrganismName : Escherichia coli 0157:H7
<400> PreSequenceString :

```
atgaaaaaaa ttgcatgtct ttcagcactg gccgcagttc tggctttcac cgcaggtact       60
tccgtagctg cgacttctac cgtaactggc ggttacgcac agagcgacgc tcagggccaa      120
atgaacaaaa tgggcggttt caacctgaaa taccgctatg aagaagacaa cagcccgctg      180
ggtgtgatcg gttcattcac ttacaccgag aaaagccgta ctgcaagctc tggtgactac      240
aacaaaaacc agtactacgg catcactgct ggtccggctt accgcattaa cgactgggca      300
agcatctacg gtgtagtggg tgtgggttat ggtaaattcc agaccactga atacccgacc      360
tacaaacacg acaccagcga ctacggtttc tcctacggtg ctggtctgca gttcaacccg      420
atggaaaacg ttgctctgga cttctcttac gagcagagcc gtattcgtag cgttgacgta      480
ggcacctgga ttgccggtgt tggttaccgc ttctaa                                516
```

<212> Type : DNA
<211> Length : 516
SequenceName : SEQ ID 443
SequenceDescription :
Sequence
--------

<213> OrganismName : Escherichia coli 0157:H7
<400> PreSequenceString :

```
atgaagagta tagcaacact ggttgtgtgt gcaatctccg ggattgcctg tgtaaattta     60
tctgcacatg cagcagaagg agagcataca atttctctgg ggtatgcgca ctttcagttt    120

ccgggactga aggattttgt aaaggatgcg actgctcata acagggagac tttcagtcat    180
ttcgtcaaca gaaactactt ttcttcattg ggcgaatata cagatggtcg ggtcagtgga    240
tatgaaggca aggataaaaa tccacagggc attaatatca ggtatcgcta cgagataacg    300
gatgattttg gcgttatcac ctcttttaca tggacgcgtt ctctcactaa ctcacagaca    360
tttattgatg tgcagtcagc cgatcatacc aggaagatta agaatccggc agcttctgcc    420
agaacggata tcagggcgaa ttactggagt ctgttagcgg ggccttcatg gcgggttaat    480
cagtacatga gtttatatgc gatggcaggg atgggcgttg ctaaagttag cgctgacctg    540
aaaattaagg acaatattaa cagtagtggc ggatttctg aaagcaacag cacgaaaaaa     600
acctcccttg cgtgggctgc aggtgcacag tttaacctga atgagagtgt tacactggat    660
gtggcttacg aaggttccgg ctctggcgac tggcgcacga gtggcgttac tgctggcatt    720
ggcctgaaat tctga                                                     735
```

<212> Type : DNA
<211> Length : 735
SequenceName : SEQ ID 444
SequenceDescription :
Sequence
--------
<213> OrganismName : Escherichia coli 0157:H7
<400> PreSequenceString :

```
atgcgtaaac tttatgccgc cattttgtcc gcagccattt gtctgaccgt atccggtgcg     60
cctgcatggg cgtctgagca gcaggccacg ctgagcgcgg ggtatcttca tgtctcgacg    120
aacgctcccg gtagcgataa tcttaacggg attaacgtga aataccgtta tgaattcacg    180
gacacgctgg ggctggtgac gtcattcagc tatgcaggag acaggaatcg ccagattacc    240
cgttacagcg atacccgctg gcatgaagat tccgtgcgta accgctggtt cagcgtaatg    300
gcggggccgt ctgtgcgcgt gaatgaatgg ttcagcgcgt atgcgatggc gggagtggct    360
tacagccgtg tgtcgacttt ctccggggat tatctccgcg taactgacaa caaggggaaa    420
acgcacgatg tgctgaccgg aagtgatgac ggtcgccaca gcaacacgtc tctggcgtgg    480
ggggctggcg tgcagtttaa cccgaccgaa tccgtggcca ttgatattgc ttatgaaggc    540
tccggcagtg gcgactggcg cactgacggt ttcatcgtgg gtgtcggtta taagttctga    600
```

<212> Type : DNA
<211> Length : 600
SequenceName : SEQ ID 445
SequenceDescription :
Sequence
--------
<213> OrganismName : Escherichia coli 0157:H7
<400> PreSequenceString :

```
atgcgtaaac tttatgccgc cattttgtcc gcagccatct gtctggccgt atccggcgcg     60
cctgcatggg cgtctgagca gcaggccacg ctgagcgcgg ggtatcttca tgcccggacg    120
agcgctcccg gtagcgataa tcttaacggg attaacgtga aataccgtta tgaatttacg    180
gacacgctgg ggctggtgac gtcattcagc tatgcaggag acaagaatcg ccagcttacc    240
cgttacagcg atacccgctg gcatgaagat tccgtgcgta accgctggtt cagcgtaatg    300
gcggggccgt ctgtgcgcgt gaatgaatgg ttcagcgcgt atgcgatggc gggtgtggct    360
tacagccgtg tgtcgacttt ctccggggat tatcttcgcg taactgacaa caaggggaaa    420
acgcacgatg tgctgaccgg aagtgatgac ggtcgccaca gcaacacgtc tctggcgtgg    480
ggggctggcg tgcagtttaa cccgaccgaa tccgtggcca ttgatattgc ttatgaaggc    540
tccggcagtg gcgactggcg cactgacggt ttcatcgtgg gtgtcggtta taagttctga    600
```

<212> Type : DNA
<211> Length : 600
SequenceName : SEQ ID 446

SequenceDescription :
Sequence
--------
<213> OrganismName : Escherichia coli 0157:H7
<400> PreSequenceString :

```
atgcgtaaac tttatgccgc cattttgtcc gcagccatct gtctggccgt atccggcgcg      60
cctgcatggg cgtctgagca gcaggccacg ctgagcgcgg ggtatcttca tgcccggacg     120
agcgctcccg gtagcgataa tcttaacggg attaacgtga aataccgtta tgaatttacg     180
gacacgctgg ggctggtgac gtcattcagc tatgcaggag acaagaatcg ccagcttacc     240
cgttacagcg atacccgctg gcatgaagat tccgtgcgta accgctggtt cagcgtaatg     300
gcggggccgt ctgtgcgcgt gaatgaatgg ttcagcgcgt atgcgatggc gggtgtggct     360
tacagccgtg tgtcgacttt ctccggggat tatcttcgcg taactgacaa caaggggaaa     420

acgcacgatg tgctgaccgg aagtgatgac ggtcgccaca gcaacacgtc tctggcgtgg     480
ggggctggcg tgcagtttaa cccgaccgaa tccgtggcca ttgatattgc ttatgaaggc     540
tccggcagtg gcgactggcg cactgacggt ttcatcgtgg gtgtcggtta taagttctga     600
```

<212> Type : DNA
<211> Length : 600
SequenceName : SEQ ID 447
SequenceDescription :
Sequence
--------
<213> OrganismName : Escherichia coli 0157:H7
<400> PreSequenceString :

```
atgcgtaaac tttatgccgc cattttgtcc gcagccatct gtctggccgt atccggcgcg      60
cctgcatggg cgtctgagca gcaggccacg ctgagcgcgg ggtatcttca tgcccggacg     120
agcgctcccg gtagcgataa tcttaacggg attaacgtga aataccgtta tgaatttacg     180
gacacgctgg ggctggtgac gtcattcagc tatgcaggag acaagaatcg ccagcttacc     240
cgttacagcg atacccgctg gcatgaagat tccgttcgta accgctggtt cagcgtaatg     300
gcggggccgt ctgtgcgcgt gaatgaatgg ttcagcgcgt atgcgatggc gggtgtggct     360
tacagccgtg tgtcgacttt ctccggggat tatcttcgcg taactgacaa caaggggaaa     420
acgcacgatg tgctgaccgg aagtgatgac ggtcgccaca gcaacacgtc tctggcgtgg     480
ggggctggcg tgcagtttaa cccgaccgaa tccgtggcca ttgatattgc ttatgaaggc     540
tccggcagtg gcgactggcg cactgacggt ttcatcgtgg gtgtcggtta taagttctga     600
```

<212> Type : DNA
<211> Length : 600
SequenceName : SEQ ID 448
SequenceDescription :
Sequence
--------
<213> OrganismName : Escherichia coli O157:H7
<400> PreSequenceString :

```
atggtcatga gccagaaaac cctgtttaca aagtctgctc tcgcagtcgc agtggcactt     60
atctccaccc aggcctggtc ggcaggcttt cagttaaacg aattttcttc ctctggcctg    120
ggccgggctt attcagggga aggcgcaatt gccgatgatg caggtaacgt cagccgtaac    180
cccgcattga ttaccatgtt tgaccgcccg acattttctg cgggtgcggt ttatattgac    240
ccggatgtaa atatcagcgg aacgtctcca tctggtcgta gcctgaaagc cgataacatc    300
gcgcctacgg catgggttcc gaacatgcac tttgttgcac cgattaacga ccaatttggt    360
tggggcgctt ctattacctc taactatggc ctggcaacag agtttaacga tacttatgca    420
ggcggctctg tcggggggtac aaccgacctt gaaaccatga acctgaactt aagcggtgcg    480
tatcgcttaa ataatgcatg gagctttggt cttggtttca acgccgtcta cgctcgcgcg    540
aaaattgaac gtttcgcagg cgatctgggg cagctggttg ctggtcagat tatgcaatct    600
cctgccggga agactcctca agggcaagca ttggcagcta ccgccaacgg tatcgacagt    660
aataccaaaa tcgctcatct gaacggcaac cagtggggct ttggatggaa cgccggtatc    720
ctgtatgaac tggataaaaa taaccgctat gcactgacct accgttctga agtgaaaatt    780
gacttcaaag gtaactacag cagcgatctt aatcgtgtgt ttaataacta cggtttgcca    840
attcctaccg ccacaggtgg cgcaacgcaa tcgggttatc tgacgctgaa cctgcctgaa    900
atgtgggaag tgtcgggtta taaccgtgtt gatccgcagt gggcgattca ctatagcctg    960
gcttacacca gctggagtca gttccagcag ctgaaagcga cctcaaccag tggcgacacg   1020
ctgttccaga aacatgaagg ctttaaagat gcttaccgca tcgcgttggg taccacttat   1080
tactacgatg ataactggac cttccgtacc ggtatcgcct ttgatgacag cccagttccg   1140
gcacagaatc gttctatctc cattccggac caggaccgtt tctggctgag tgcaggtacg   1200
acttacgcgt ttaataaaga tgcttcagtc gacgttggtg tttcttatat gcacggtcag   1260
agcgtgaaaa ttaacgaagg cccataccag ttcgagtctg aaggtaaagc ctggctgttc   1320
ggtactaact ttaactacgc gttctga                                       1347
```

<212> Type : DNA
<211> Length : 1347
SequenceName : SEQ ID 449
SequenceDescription :
Sequence
--------
<213> OrganismName : Escherichia coli O157:H7
<400> PreSequenceString :

```
atggcctttt ctcaagcggt tagcggatta aacgctgccg ccaccaacct cgatgttatt     60
ggcaacaata tcgccaactc cgccacctac ggctttaaat caggcacggc ctcttttgcc    120
gatatgtttg ccggttcgaa agtgggactg ggggtaaaag ttgccggtat cactcaggac    180
tttaccgatg gcacgaccac caacaccggg cgtggtctgg acgttgctat cagccagaac    240
ggtttttttcc gtctggtaga tagcaacggt tcggtgttct acagccgtaa cggacaattt    300
aagctggatg aaaatcgtaa cctggtgaat atgcaaggtt tacagctgac gggttacccg    360
gcaaccggta cgccgccgac tattcagcaa ggggcgaatc cgactaacat ttcgatcccg    420
aataccctga tggcagcgaa aactaccacc acggcgtcga tgcagatcaa cctgaattcc    480
agcgatccgc ttccctctgt taacgcattt gatgccagca atgcggatag ctataacaaa    540
aaaggttcgg tgactgtttt cgacagtcag ggtaatgctc atgacatgag cgtctacttt    600
gtgaagaccg gggataataa ctggcaggtc tacacccagg atagcagtga tccaacaggt    660
acagccgagc ctgcaatgaa gctggtgttt aatgccaatg gcgttctgac ctcaaatcca    720
acagagaata ttaccaccgg cgcaattaac ggcgcagaac ccgccacgtt tagcctgagc    780
ttcctcaact ccatgcagca aaataccggc gctaacaaca ttgtggcaac cacccagaat    840
ggctacaaac cgggcgatct ggtgagttat caaatcaatg atgacggtac ggttgtcggc    900
aactattcca acgaacaaac ccaactgctg gggcagattg tactggcgaa ctttgccaac    960
aacgaaggtc tggcatccga aggcgacaac gtctggtctg cgacgcaatc ttctggcgtg   1020
gcgctgttgg ggacagccgg gacgggcaac tttggcaccc tgaccaacgg tgcgttggaa   1080
gcgtccaacg tcgatctcag taaagaactg gtcaatatga tcgttgccca gcgtaactat   1140
cagtctaacg cccagaccat caaaacccag gaccagatcc tcaacacgct ggttaactta   1200
cgctaa                                                              1206
```

<212> Type : DNA
<211> Length : 1206
SequenceName : SEQ ID 450
SequenceDescription :
Sequence
--------
<213> OrganismName : Escherichia coli O157:H7

<400> PreSequenceString :

```
atgtctaaat cgactttttt acacattctg attagctcca tcatattggt ggcactaata      60
caatcatccg cttgggctaa ctgtacaaac acacagatag gtcaaactga agatggtcgg     120
acagcgctta ttgagttcgg aaaaattaat atgaccgaca cctattttgc gccagcaggt     180
tcactcctcg cgacaacggt cgtacctccc actaactaca catcaggtgg cgcgacagga     240
agctccgtat tgtgggaatg tgatgcaaca gatttgccaa acatctattt tctggtcgct     300
accaatggcg acgaccgcgt ggggggcttt tacgatgcag gcgggcctga tggtctgagt     360
gatgtctatg ccacctggtt cgcttttgtc ggtctcaagc agaccatggc gggcgtgacg     420
cttggtcgtt actggaagaa agtgcccatc accagttatg ccactcaggg aactaaaatc     480
cagattcgct tacaggatat ccctcctctt catgctgagc tttatcgcat cagtacgcta     540
cctgatacat cagcaacaac aagttggtgc ggtaataata atacagatag tagtggagtc     600
ggattcgcaa aaccttccgg tacaatctat aactgtgttc agcccaatgc ctatattcag     660
ctttccggta ccagcggcat tttatttggt catgatgagc ccggcgaaga tagttctgtt     720
cattgggatt tctggggtgc tgataatggt tttggttacg gaatgcgttc ggccaatcga     780
ctctacaaca atgccacctg cgttgcccgc agcgccacgc cgttagtatt gctgccgaca     840
attgcagaag cacaactgaa tgcgggcatg gaaagtaccg gtaattttaa tgtccgcgtc     900
gagtgtagta actcggttca atcagggatt agcgatactc agacagcatt aggaatccag     960
gtgtctgaag gtgcatatac agcggcgcaa aaactgggga ttatcaatag caacggcggc    1020
gtcagcgccc tggtctctga taattatgac gcagcagaga tggcaaaggg cgttgggatc    1080
tacatttcta acagtgctca ccccgatacg gcgatgacgc tggttggtca accgggcatc    1140
gcgaagttaa cccccggagg aaatgcagcg gggtggtatc ctgtatttga aggggcaaca    1200
ttagaaggtg cgactcaccc cggatactcc agctatagtt actcttttat cgcccggttg    1260
aagaaactgc caaatcagac agtcagtgcg ggaaaagtgc gggcaacggc ttatatattg    1320
gtgaaaatgc aatga                                                     1335
```

<212> Type : DNA
<211> Length : 1335
SequenceName : SEQ ID 451
SequenceDescription :
Sequence
--------
<213> OrganismName : Escherichia coli 0157:H7
<400> PreSequenceString :

```
atggaaaaca atcgcaattt ccctgccaga caatttcatt cgctcacgtt ctttgccggt      60
ctttgtattg gcatcacgcc tgtggctcag gcactcgccg ccgaagggca aactaacgcg     120
gatgacacgc tggttgtcga agcatcaacg ccttcgcttt atgcgccaca acaatctgcc     180
gatccgaaat tctcgcgtcc ggtagcggat actacccgca cgatgacggt gatttctgaa     240
caagtgatta aagatcaggg cgcaaccaac cttaccgatg cgctcaaaaa cgtccccggc     300
gtgggtgcgt tttttgcggg tgagaacggt aactccacca ctggcgacgc catttatatg     360
cgcggtgctg atacctctaa cagtatttat attgatggca ttcgcgatat cggcagcgtc     420
```

```
tcgcgcgaca ccttcaatac tgagcaggtc gaagtgatta aagggccttc cggcaccgac    480
tacgggcgca gcgcgccgac gggctcgatc aatatgatca gcaagcagcc gcgcaatgat    540
tccggcattg acgcctccgc cagtattggc agcgcctggt tccgccgcgg cacgctggac    600
gtcaatcagg tcattggtga taccactgcg gtgcgcctga atgtgatggg cgaaaaaacg    660
cacgatgccg gacgcgacaa agtcaaaaat gagcgttacg gcgtcgcccc ttctatcgct    720
tttggccttg gtacagcgaa tcgtttgtat cttaattatc tgcatgtcac ccagcacaac    780
acgccagacg gcggcattcc aaccatcggt ttgccgggct attctgcccc atctgcagga    840
acggcgaccc tgaatcattc cggaaaagtt gatacgcata acttttacgg cacggattcc    900
gattacgacg attcgaccac cgacaccgcc accatgcgtt ttgagcacga tatcaacgat    960
aacaccacca ttcgcaacac tacccgttgg tcgcgcgtga agcaggatta cctgatgacg   1020
gcgattatgg gtggggcgtc gaatattacc cagcccacca gcgatgtgaa tagctggacg   1080
tggtcacgca cggcgaatac caaagatgtg agtaataaaa ttctcaccaa ccagaccaac   1140
ctgacctcga cattctatac cgcttctatc ggtcatgatg tcagtaccgg cgtggaattt   1200
acccgtgaaa cgcagactaa ctacggcgtt aatccggtga cgttacctgc ggtaaatatt   1260
tatcatcctg acagcagcat tcatcccggc ggcctgacgc gcaacggcgc aaacgccaat   1320
ggtcagacgg ataccttcgc aatttacgca ttcgatacgc tgcaaatcac ccgtgatttt   1380
gagctgaacg gcgggatccg tctggataat tatcatactg aatatgacag tgccaccgcc   1440
tgcggcggca gcggacgcgg tgccatcacc tgcccagctg gtgtggcaaa aggttctccg   1500
gtcaccaccg tcgacaccgc caagtcgggc aatctggtga actggaaagc cggggcgctg   1560
tatcacctga cggaaaacgg caatgtctat attaactatg ccgtttccca gcagcctccg   1620
ggcggcaaca acttcgccct tgcgcagtct ggcagcggta acagtgccaa ccgcaccgat   1680
tttaaaccgc aaaaagccaa caccagcgag attggcacca aatggcaggt tctggataaa   1740
cgcctgttgc tcaccgccgc gctgttccgt actgatatcg aaaatgaagt tgagcaaaat   1800
gatgacggga cttactcgca atacggtaag aaacgcgtcg aaggctatga gatatccgtg   1860
gcagggaata tcactcccgc gtggcaggtg attggcggct atacccagca aaaagcaacc   1920
atcaaaaacg gcaaagatgt tgcccaggat ggttcctcat cgctgccgta taccccggag   1980
cacgccttca ccttatggag ccaatatcag gcaaccgatg atatctctgt tggcgcgggc   2040
gcacgctata tcggcagtat gcataaaggt tcagacggcg cggtgggaac gccagcgttt   2100
accgaaggtt actgggtcgc cgatgccaaa ctggggtata gggttaatcg caatctcgac   2160
ttccagctaa acgtctacaa cctgtttgat accgattacg tcgcctcaat caataagagc   2220
ggctaccgtt atcacccggg cgagccaaga accttcttgc tcacagccaa tatgcatttc   2280
tga                                                                 2283
```

<212> Type : DNA
<211> Length : 2283
SequenceName : SEQ ID 452
SequenceDescription :
Sequence
--------
<213> OrganismName : Escherichia coli 0157:H7
<400> PreSequenceString :

```
atgcaaatga agaaattgct ccccattctt atcggcctga gcctttctgg gttcagttcg     60
ttgagccagg ccgagaacct gatgcaagtt tatcagcaag cacgccttag taacccggaa    120
ttgcgtaagt ctgccgccga tcgtgatgct gcctttgaaa aaattaatga agcgcgcagt    180
ccattactgc cacagctagg tttaggtgca gattacacct atagcaacgg ctaccgcgac    240
gcgaacggca tcaactctaa cgcgaccagt gcgtccctgc agttaactca atccattttt    300
gatatgtcga aatggcgtgc gttaacgctg caggaaaaag cagcagggat tcaggacgtc    360
acgtatcaga ccgatcagca aaccttgatc ctcaacaccg cgaccgctta tttcaacgtg    420
ttgaatgcta ttgacgttct ttcctataca caggcgcaaa aagaagcgat ctaccgtcaa    480
ttagatcaaa ccacccaacg ttttaacgtg ggcctggtag cgatcaccga cgtgcagaac    540
gcccgcgcgc agtacgatac cgtgctggcg aacgaagtga ccgcacgtaa taaccttgat    600
aacgcggtag agcagctgcg ccagatcacc ggtaactact atccggaact ggcggcgctg    660
aatgtcgaaa actttaaaac cgacaaacca cagccggtta acgcgctgct gaaagaagcc    720
gaaaaacgca acctgtcgct gttacaggca cgcttgagcc aggacctggc gcgcgagcaa    780
attcgccagg cgcaggatgg tcacttaccg actctggatt taacggcttc tagcgggatt    840
tctgacacct cttatagcgg ttcgaaaacc cgtggtgccg ctggtaccca gtatgacgat    900
agcaatatgg gccagaacaa agttggcctg agcttctcgc tgccgattta tcagggcgga    960
atggttaact cgcaggtgaa acaggcacag tacaactttg ttggtgccag cgagcaactg   1020
gaaagcgcgc atcgtagcgt cgtgcagacc gtacgttcct ctttcaacaa cattaatgct   1080
tctatcagta gtattaacgc ctacaaacaa gccgtagttt ccgctcaaag ctcattagac   1140
gcgatggaag cgggctactc ggtcggtacg cgtaccattg ttgatgtgtt ggatgcgacc   1200
accacgctgt acaacgccaa gcaagagctg gcgaatgcgc gttataacta cctgattaat   1260
cagctgaata ttaagtcagc cctgggtacg ttgaacgagc aggatctgct ggcactgaac   1320
aatgcgctga gcaaaccggt ttccactaat ccggaaaacg ttgcccccgca aacgccggaa  1380
cagaatgcta ttgctgatgg ttatgcgcct gatagcccgg cacccgtcgt tcagcaaaca   1440
tccgcacgca ctaccaccag taacggtcat aaccctttcc gtaactga                1488
```

<212> Type : DNA
<211> Length : 1488
SequenceName : SEQ ID 453
SequenceDescription :
Sequence
--------
<213> OrganismName : Escherichia coli O157:H7
<400> PreSequenceString :

```
gtgaccaaac tcaaacttct ggcacttgga gtgcttatcg caacgtctgc aggcgtagcg 60
cacgctgaag gtaaattttc cctgggcgca ggcgtaggtg tcgttgaaca cccatataaa 120
gattacgata ccgatgttta cccagtaccg gtaatcaact atgaaggcga taacttctgg 180
ttccgtggct taggtggtgg ttactacctg tggaatgacg caacggataa actttcaatt 240
accgcttact ggtcgccgct ttacttcaaa gctaaagaca gtggcgatca ccaaatgcgt 300
cacctggatg accgtaagag caccatgatg gctggtctgt cttatgctca ctttacccag 360
tacggttacc tgcgtaccac cctggctggc gataccctgg ataacagcaa cggcatcgtc 420
tgggatatgg cctggttgta tcgttacacc aacggtggcc tgaccgtgac tccgggtatt 480
ggtgtgcagt ggaacagcga aaaccagaac gaatactatt atggcgtatc gcgcaaagag 540
tccgctcgca gcggtctgcg tggctataac tcgaacgaca gctggagccc ttacctggag 600
ctgagcgcca gctacaactt cctcggcgac tggagtgttt acggtaccgc gcgctacacc 660
cgtctgtctg atgaagttac tgacagcccg attgtggata aatcctggac tggcctgatt 720
tctaccggga tcacctacaa attctga 747
```

<212> Type : DNA
<211> Length : 747
SequenceName : SEQ ID 454
SequenceDescription :
Sequence
--------
<213> OrganismName : Escherichia coli O157:H7
<400> PreSequenceString :

```
atgaaaaaaa cattactggc agccggtgcg gtactggcgc tctcttcgtc ttttactgtc 60
aacgcagctg aaaacgacaa accgcagtat ctttccgact ggtggcacca gagcgttaac 120
gttgtcggaa gctatcacac ccgtttcgga ccgcagatcc gcaacgatac ctaccttgag 180
tacgaagcat tcgctaaaaa agactggttc gacttctatg gttatgcgga tgcgccggta 240
ttcttcggcg gtaactccga tgcaaaaggt atctggaacc acggttctcc tctgtttatg 300
gaaatcgaac cacgtttctc catcgacaag ctgaccaata ctgaccttag cttcggtccg 360
ttcaaagagt ggtacttcgc gaacaactac atttacgaca tgggtcgtaa taaagatggt 420
cgccagagca cctggtacat gggtctgggt accgatatcg acactggcct gccgatgagc 480
ctgtccatga acgtctatgc gaaataccag tggcagaact atggcgcagc gaacgaaaac 540
gagtgggacg gttaccgttt caaaattaaa tactttgtgc cgattaccga tctgtggggc 600
ggtcagctga gctacatcgg cttcaccaac ttcgactggg gttccgattt aggggatgac 660
agcggtaacg caatcaacgg tattaagacc cgtactaata actctatcgc ttccagccat 720
attctggctc tgaactacga tcactggcac tactctgtcg tagctcgtta ctggcacgac 780
ggtggtcagt ggaacgacga tgcagaactg aacttcggca acggcaactt caacgttcgc 840
tctaccggct ggggtggtta cctggtagta ggttacaact tctga 885
```

<212> Type : DNA
<211> Length : 885
SequenceName : SEQ ID 455
SequenceDescription :
Sequence
--------
<213> OrganismName : Escherichia coli O157:H7
<400> PreSequenceString :

```
atgctttcaa cacaatttaa cagggataat caatatcaag ccatcaccaa accgtcacta      60
cttgccggtt gcatagcact ggcactatta ccttctgccg cttttgctgc accagccact     120
gaagaaacgg tgattgttga gggttcagcc acagctccag atgatggcga aaatgattac     180
agcgtaacgt ctacctctgc gggtaccaaa atgcagatga ctcaacgtga tattcctcag     240
tcggtcacta ttgttagcca gcagcggatg gaagatcagc agttacaaac gctgggcgaa     300
gtgatggaaa acacgctggg gatcagcaaa agtcaggcgg attccgatcg tgctctttat     360
tattcccgcg gattccagat cgataactat atggttgatg gtatccccac ctattttgaa     420
tcgcgctgga atctgggcga cgcactttct gatatggcac tgtttgaacg cgtagaagta     480
gtgcgtggcg cgacaggact catgaccggg acgggtaatc catctgcggc aattaatatg     540
gttcgaaaac acgcgaccag tcgtgaattt aaaggcgatg tctcggcgga atacggtagc     600
tggaacaaag aacggtatgt ggcggattta caaagcccac tcaccgaaga cggtaaaatc     660
cgcgcgcgaa ttgtcggcgg ctaccagaat aacgactcat ggctggaccg ctacaatagt     720
gaaaagacct tcttctccgg cattgtcgat gctgatttag gcgatcttac gacgctttca     780

gccggttacg aatatcagcg cattgatgtt aatagcccaa cctggggcgg tttaccgcgc     840
tggaatactg atggcagcag caacagttac gatcgcgcac gcagtaccgc acctgactgg     900
gcgtacaacg ataaagagat caacaaggtc tttatgaccc tgaagcagcg gtttgctgat     960
acctggcaag cgacactgaa tgccacccac tctgaagtcg aatttgacag caaaatgatg    1020
tatgtcgatg cctatgtaaa caaagcggat ggtatgctgg ttgggccata cagtaattat    1080
ggacctggct ttgattatgt cggcggcacc ggttggaaca gtggcaaacg taaagttgat    1140
gcgctggatt tgttcgctga cggtagttat gaattgtttg gtcgtcagca caatctaatg    1200
tttggtggca gttacagcaa acaaaacaat cgttacttca gttcatgggc caacatcttc    1260
ccggatgaaa ttggcagttt ctacaacttt aatggcaatt tcccacaaac cgactggtca    1320
ccacagagcc tggcgcagga cgataccaca catatgaaat cgttatatgc tgccactcgt    1380
gtcacccttg ccgatccgct gcatctgatc ctcggcgcac gttataccaa ctggcgggtt    1440
gatacgctga cttacagcat ggagaaaaac cacaccacgc cttacgctgg tctggtgttt    1500
gacatcaatg acaactggtc gacctacgcc agctatacct ctattttcca gccgcaaaat    1560
gatcgtgaca gttcaggcaa atatctggct ccaatcaccg gtaacaacta cgagctgggt    1620
ctgaaatcgg actggatgaa tagccgtctg accaccacgt tagccatctt ccgtattgag    1680
caggataatg tcgctcagtc caccggtaca cctatccccg gcagcaacgg cgaaaccgcc    1740
tataaagcgg tggatgggac agtcagtaaa ggggtggaat ttgaactcaa cggcgcaatt    1800
accgacaact ggcagctgac atttggcgca acgcgctata ttgcagagga taacgaagga    1860
aacgccgtta atcctaatct gccacgcacc acggttaaaa tgttcaccag ctatcggttg    1920
cctgtcatgc cagagttgac agtcggcggt ggtgttaact ggcaaaatcg cgtgtatacc    1980
gacaccgtga caccgtatgg caccttccgc gccgagcaag gtagctacgc gttggtggat    2040
ctcttcaccc gctaccaggt gacgaaaaac ttctcgttac aggggaacgt caataacctg    2100
ttcgacaaaa cctacgatac caacgtggaa ggttctatcg tctacggcgc accgcgtaat    2160
ttcagcatta ccggcacgta tcaattctga                                    2190
```

<212> Type : DNA
<211> Length : 2190
SequenceName : SEQ ID 456
SequenceDescription :
Sequence
--------
<213> OrganismName : Escherichia coli O157:H7
<400> PreSequenceString :

```
atggcgcgat tccagtttaa aaaccgtaaa aataatggac ttattttttt tataagtttt      60
atggtgatgg gagaagctgc aattgctgca ccgctgccac aatgggcgaa tgccccagcg     120
gtaacgccag ttgcccaatt atctttacag gaaagtatat tacgcgcctt tgcgcgaaat     180
cccggtgtca ctcaacaggc ggcgcagata ggtattggcg aagcgcaaat tgatgaagcc     240
aaaagtgcct ggtatccgca cgttggatta accggcaacg cggggccgtc ccgacaaacg     300
gactccagcg gcaggcttga taacaacgtt tcgtatggca taaccctgac acaactggtg     360
tatgactttg gtaaaactaa caacgatatc aatctgcaaa ctgccgcccg tgacagctac     420
cgctttaaat tgatggcaac cttaaccgat gttgcagaga aaacggcgac tgcctacatg     480
gaagtcagtc gttatcaggc tttgtgcgat gcggctcaac gcaatattca ctcgctggaa     540
aacgtctaca acatggcggc attgcgcgct aacgcaggcc tgaactcatc gtcggatgaa     600
ttacaggccc agacgcgtat tgccggaatg cgctcaacgc tggagcaata tcaggcgcag     660
atggcgagcg ccaaagcgca actggcggtg ctcactggcg tacagccgga ggcgatagcc     720
gcgccacctg ctgaattagc cgagcagccg gtatcgctga agaatattga ttaccagtcc     780
atcccgctgg tgctggcggc agaaaactta cgccagtcag cacagtacgg cgtggaaaaa     840
acgaaagcgc aatactggcc aacgctcagt attcaggggg gtaaaacgcg ctaccagacc     900
agcgaccgct cgtattggga tgatcagcta caactgaacg ttaacgcgcc gctgtatcag     960
ggcggcgcgg tttctgccca ggtgcaacag gccgaagggc aacaaaaaat ctctgcctcg    1020
caggtcgaac aggcaaaact ggatgtgctg caacgagcgt ctgtggcata tgcgaactgg    1080
accggcgcac ggggtcgtga agaagccggt ttagcgcaat ccgaaagtgc gcacaaaacg    1140
cgagatgtgt accaaaatga atataaattg ggtaaacgca gtctgaatga tctattaacc    1200
gtcgaacaag atgtctttca ggcgcaatcg gctgaaataa atgccaatta tgatggctgg    1260
gtcgccgccg taaattatgc cgctgcggtg aataacctta ttccattagc gggaattaaa    1320
cagggcttat acaacgactt acccgacttg aaataa                               1356
```

<212> Type : DNA
<211> Length : 1356
SequenceName : SEQ ID 457
SequenceDescription :
Sequence
--------
<213> OrganismName : Escherichia coli O157:H7
<400> PreSequenceString :

```
atggctaagt tcacaccttc attctcagga atcaaaggtc gggcgctctt ttcactgctc      60
tttgcagcac caatgattca tgcaaccgac actgcaacga ccaaagatgg cgaaacaatc     120
```

actgttacag cggatgcaaa taccgcaact gaggcgaccg atggttatca acctctgagc 180
acttccacgg cgacattaac cgatatgccg atgctggata tcccgcaggt ggtcaatacg 240
gttagcgatc aggttctgga aaatcagaat gcaacgacgc tggatgaagc gctttataac 300
gtcagtaacg tggtacagac caatacatta ggcggaactc aggacgcctt tgtacgtcgt 360
ggttttggtg ctaaccggga tggctccatc atgaccaacg gcctgcgaac tgtacttcct 420
cgcagtttca acgccgccac agaacgtgtg gaagtgctaa aaggtccggc ctccacgctg 480
tatggcattc tcgatcctgg tggattgatt aacgtcgtga ccaagcgccc ggaaaaaaca 540
ttccatggtt ctgtctcagc cacgtcctcc agttttggcg gaggcactgg gcaacttgat 600
atcacaggtc ccattgaagg cactcagctg gcataccgcc tgacggggga agtgcaggat 660
gaagattact ggcgaaactt cggtaaagag cgcagtacat ttattgcccc gtcactcacc 720
tggtttggtg ataatgcaac agtaaccatg ctctattccc atcgggacta taaaactccg 780
ttcgatcgtg gaacgatttt cgaccttacg acgaaacagc ccgtaaacgt tgatcgaaaa 840
atacgttttg acgaaccgtt taatattaca gatggtcagt ccgatctggc gcaactcaac 900
gcagaatatc atctcaatag ccagtggaca gcgcgctttg attacagcta cagccaggat 960
aaatacagcg acaatcaggc tcgcgttacc gcgtatgatg caacgacagg aacgctgaca 1020
cggcgtgttg atgcaactca gggatctacc cagcgtatgc attctactcg tgcggatctg 1080
caagggaatg ttgatattgc tgggttctat aatgagattc tgggtggggt gtcatatgaa 1140
tattatgatc ttctgcgcac agatatgatt cgctgtaaaa acgctaaaga tttcaatatc 1200
tacaaccccg tttatggcaa taccagcaaa tgcacaacgg tttcggcgtc ggacagtgat 1260
cagacgatca aacaggagag ctactcagct tatgcacagg atgcgctcta tctgaccgat 1320
aactggattg ccgtcgccgg gatccgctat cagtattaca cgcagtatgc gggtaaaggc 1380
cgtcctttta atgtcaatac tgacagccgc gatgaacaat ggacgcccaa actggggtta 1440
gtctacaaac tgacgccatc ggtatcctta tttgctaatt attcgcaaac atttatgccg 1500
caatcgtcaa ttgccagcta catcggagat cttccaccgg aatcatctaa tgcttacgaa 1560
gtcggggcaa aattcgagct gttcgatggt ataaccgcag atattgcgct gtttgatatc 1620
cataaacgta acgtgttgta taccgaaagt attggtgatg aaaccatcgc caaaacggca 1680
ggccgcgttc gttcaagagg ggtagaagtc gaccttgcgg gagcattaac tgaaaacatt 1740
aatatcattg ccagctacgg ctataccgat gctaaggttc tggaagatcc tgattatgca 1800
gggaaaccat tgccgaatgt tcctcgtcat accggttcgc tattcctgac ctatgacatt 1860
cataacatgc caggcaataa cacactgacg tttggcggtg gtggacattg cgtaagccgt 1920
cgttcggcaa ccaatggggc tgactattat ctgccaggct atttcgttgc cgatgccttc 1980
gccgcataca aaatgaaatt gcagtatccg gtcacactgc aattaaacgt caaaaacctg 2040
tttgataaaa cgtattacac ctcttccatc gccacaaata atctggggaa tcagattggc 2100
gatccgcgtg aagtgcaatt cacggtgaaa atggaatttt ga 2142

<212> Type : DNA
<211> Length : 2142
SequenceName : SEQ ID 458
SequenceDescription :
Sequence
--------
<213> OrganismName : Escherichia coli O157:H7
<400> PreSequenceString :

atgcggactc tgcagggctg gttgttgccg gtgtttatgt tgcctatggc agtatatgca 60
caagaggcaa cggtgaaaga ggtgcatgac gcgccagcgg tgcgtggcag tattatcgcc 120
aatatgctgc aggagcatga caatccgttc acgctctatc cttatgacac caactacctc 180
atctacaccc aaaccagcga tctgaacaaa gaagcgattg ccagttacga ctgggcggaa 240
aatgcgcgta aggatgaagt aaagtttcag ttgagcctgg catttccgct gtggcgtggg 300
attttaggcc cgaactcggt gttgggtgcg tcttatacgc aaaaatcctg gtggcaactg 360
tccaatagcg aagagtcttc accgtttcgt gaaaccaact acgaaccgca attgttcctc 420
ggttttgcca ccgattaccg ttttgcaggt tggacgctgc gcgatgtgga gatggggtat 480
aaccacgact ctaacgggcg ttcagacccg acctcccgca gctggaaccg cctttatact 540
cgcctgatgg cagaaaacgg taactggctg gtagaagtga agccgtggta tgtggtgggt 600
aatactgacg ataacccgga tatcaccaaa tatatgggtt actaccagct taaaatcggc 660
tatcacctcg gtgatgcggt gctcagtgcg aaaggacagt acaactggaa caccggctac 720
ggcggcgcgg agttaggctt aagttacccg atcaccaaac atgtgcgcct ttatactcag 780
gtttacagcg gctatggcga atcgctcatc gactataact tcaaccagac ccgtgtcggt 840
gtgggggtta tgctaaacga tttgttttga 870

<212> Type : DNA
<211> Length : 870
SequenceName : SEQ ID 459
SequenceDescription :
Sequence
--------

<213> OrganismName : Escherichia coli O157:H7
<400> PreSequenceString :

```
atggcggttc aaaagaatgt tatcaaaggc atactggcag gtacgtttgc gctaatgctg     60
agcggttgtg tcactgtgcc ggacgccatt aaaggcagca gtcccacgcc gcaacaagat    120
ttagttcggg tgatgagtgc gccgcagctg tacgttggtc aggaggcacg ctttggtggc    180
aaagtggttg cggtacaaaa ccagcaaggg aaaacccgcc tggaaattgc taccgtaccg    240
ctggacagcg gagccagacc gacgctggga gaaccttctc gcggtcgcat ttatgccgat    300
gtgaacggtt ttctggaccc ggtggatttc cgtggacaac tggttacggt agtcgggcca    360
atcactggtg cggttgacgg aaaaatcggc aacacgccct ataaatttat ggtgatgcag    420
gcaacaggtt acaaacgttg gcatttaacc cagcaggtga ttatgccgcc tcagccgatt    480
gatccctggt tttatggcgg tcgtggctgg ccctatggcc acggcggatg gggctggtat    540
aatcccggcc ccgcgagagt acaaacagtt gtaactgaat aa                       582
```

<212> Type : DNA
<211> Length : 582
SequenceName : SEQ ID 460
SequenceDescription :
Sequence
--------
<213> OrganismName : Escherichia coli O157:H7
<400> PreSequenceString :

```
atgcgtaagc agtggctcgg gatctgcatc gcggcaggaa tgctcgcggc atgtacaagc     60
gatgatggtc agcaacagac agtaagtgta ccgcagcctg cggtatgtaa cggccccata    120
gttgaaatta gcggggcgga cccgcgtttc gaaccactga acgcgacggc aaatcaggat    180
taccagcgcg acggtaaaag ctacaaaatc gtgcaggatc cgtctcgatt tatccaggcg    240
ggactggcgg caatctatga tgccgaacca ggcagtaacc tgacggcctc tggcgaagct    300
ttcgatccga cacagctgac ggcggcccat ccaacgcttc cgatccccag ctacgccaga    360
atcactaacc tggctaacgg gcgaatgatc gtggtgcgca ttaatgatcg cggtccttac    420
ggcaacgacc gcgttatttc gctttctcgc gcagcagctg accgtcttaa cacgtcaaac    480
aacaccaaag ttcgtatcga tccgattatt gtcgcccagg atggttcgct ttctggtcct    540
ggtatggctt gtaccacagt cgccaaacag acttacgccc tgcctgcacc tcccgattta    600
agcggtggcg cgggaacaag ttcagtgtct ggcccgcagg gtgacattct tccggtcagt    660
aattcgacgc taaaaagcga agatccgacc ggcgcgccgg taaccagcag cggtttcctc    720
ggcgcaccaa cgaccttagc gccaggtgta ctggaaggca gcgaaccgac gcctgctcca    780
cagcccgttg ttacagctcc gtcgacaacg cctgcaacct cgcctgcaat ggtgacaccg    840
caagccgcct cgcaaagcgc cagcggcaac tttatggtgc aggtcggggc cgtaagcgat    900
caggctcgtg cgcaacagta ccaacagcaa ctgggacaga agttcggcgt ccccggtcgc    960
gtaactcaaa atggcgcggt ctggcggatc cagcttggcc cattcgccaa caaagccgaa   1020
gccagtacct tgcagcaacg tttgcaaacc gaagcccaat tacagtcatt tattaccacc   1080
gcgcagtag                                                           1089
```

<212> Type : DNA
<211> Length : 1089
SequenceName : SEQ ID 461
SequenceDescription :
Sequence
--------
<213> OrganismName : Escherichia coli 0157:H7
<400> PreSequenceString :

```
atgattaaac gcgtattggt tgtttcaatg gtaggtctgt ctcttgtcgg ttgtgttaat     60
aacgacaccc tgtcaggggga tgtttatacc gcttctgaag cgaaacaagt acagaatgtc   120
agctatggaa ccatcgttaa cgtacgtccg gtacagattc agggcggtga tgattccaac    180
gttatcggtg caattggcgg tgctgttctt ggtggtttcc tgggaaatac tgttggtggc    240
ggaaccgggc gttctctggc tactgcagca ggcgctgttg caggtggcgt agctggtcag    300
ggcgtacaga gtgcaatgaa caaaacgcaa ggtgtcgagc tggaaattcg taaagacgat    360
ggtaatacca tcatggtggt acagaaacaa ggcaacactc gtttctctcc gggccaacgt    420
gtcgtactgg ccagcaatgg cagtcaggtg accgtttctc cgcgctaa                 468
```

<212> Type : DNA
<211> Length : 468

SequenceName : SEQ ID 462
SequenceDescription :
Sequence
--------
<213> OrganismName : Escherichia coli O157:H7
<400> PreSequenceString :

```
gtgtcaaagg caaccgaaca aaacgacaag ctcaagcggg cgataattat ttcagcagtg      60
ctgcatgtca tcttatttgc ggcgctgatc tggagttcgt tcgatgagaa tatagaagct     120
tcagctggag gcggcggtgg ttcgtccatc gacgctgtca tggttgattc aggtgcggta     180

gttgagcagt acaaacgcat gcaaagccag gaatcaagcg cgaagcgttc tgatgagcag     240
cgcaagatga aggaacagca ggctgctgaa gaactgcgtg agaaacaagc ggctgaacag     300
gaacgcctga agcaacttga gaaagagcgg ttagctgctc aggaacagaa aaagcaggct     360
gaagaagccg caaaacaggc cgagttaaag cagaagcaag cggaagaggc ggcagcgaaa     420
gcggcggcag atgctaaagc gaaggccgaa gcggatgata aagctgcgga agaagcagcg     480
aagaaagcgg ctgcagacgc gaagaaaaaa gcagaagcag aagccgccaa agccgcagcc     540
gaagcgcaga aaaaagccga ggcagcagct gcggcgctga agaagaaagc ggaagcggca     600
gaagcagctg cagctgaagc aagaaagaaa gcggcagcag agaaagctgc agccgacaaa     660
aaagcagcag aaaaagcggc tgctgaaaag gcagcagctg ataagaaagc agcggcagaa     720
aaagccgccg cagacaaaaa agcggcagct gcaaaagcag cagctgaaaa agccgctgca     780
gcaaaagctg ccgcggaggc agatgatatt ttcggtgagc taagctctgg taagaatgca     840
ccgaaaacgg ggggaggggc gaaagggaac aatgcttcgc ctgccgggag tggtaatact     900
aaaaacaatg gcgcatcagg ggccgatatc aataactatg ccgggcagat taaatctgct     960
atcgaaagta agttctatga cgcatcgtcc tatgcaggca aaacctgtac gctgcgcata    1020
aaactggcac ccgatggtat gttactggat atcaaacctg aaggtggcga tcccgcactt    1080
tgtcaggctg cgttggcagc agctaaactt gcgaagatcc cgaaaccacc aagccaggca    1140
gtatatgaag tgttcaaaaa cgcgccattg gacttcaaac cgtaa                    1185
```

<212> Type : DNA
<211> Length : 1185
SequenceName : SEQ ID 463
SequenceDescription :
Sequence
--------
<213> OrganismName : Escherichia coli O157:H7
<400> PreSequenceString :

```
gtgatgaaat ttaaaaaatg tcttctgcct gtggcaatgt tagcgtcatt cactctggca      60
ggatgccagt caaatgctga cgatcatgcc gccgatgttt atcaaaccga tcaactgaat     120
accaaacaag aaactaaaac cgttaatatt atttccattc ttcccgcaaa agttgccgta     180
gacaactccc aaaataaacg gaacgcacaa gccttcggcg cgcttattgg cgcagtcgct     240
ggcggtgtta tcggccacaa cgtcgggtct ggcagcaatt ccggaacgac ggcaggtgca     300
gttggcggcg gagctgtagg cgcggcagcg ggttctatgg tgaatgataa aaccttagtg     360
gaaggtgttt ctttaacgta taaggaaggc accaaagtgt atacctctac tcaggtgggt     420
aaagagtgcc agtttacgac aggtttagcc gttgttatta ccacgacgta taacgaaacg     480
cgtattcagc caaataccaa atgtcctgaa aagagctaa                            519
```

<212> Type : DNA
<211> Length : 519
SequenceName : SEQ ID 464
SequenceDescription :
Sequence
--------
<213> OrganismName : Escherichia coli 0157:H7
<400> PreSequenceString :

```
atgttgctta gcataatcac tgtcgcgttt cgtaacctcg aagggatagt caaaacacat      60
gcctcgctgg cgcatctggc gcaggcggaa gatatcagct tcgaatggat tgttgtcgat     120
ggcggttcca atgacggcac tcgtgagtac ctggaaaatc tcaatggtat ctataaccta     180
cgctttgtca gcgagccaga taacggtatc tacgacgcca tgaacaaggg cattgcgatg     240
gcacaaggca agttcgcgtt gtttctcaac tcgggcgata tttttcatca ggatgccgca     300
tattttgtcc gtaagttaaa aatgcaaaaa gataacgtga tgatcaccgg cgatgcgctg     360
ctggattttg gcgacgggca taaaattaaa cgtagcgcca aaccgggctg gtatatttat     420
cacagcctgc ccgccagtca tcaggcgata tttttcccgg tatccggttt gaaaaaatgg     480
cgttatgacc tggaatataa agtttcttct gattacgcgc tggcagccaa aatgtataaa     540
gcaggttatg catttaaaaa actcaatggc ctggtgtctg aatttttctat gggtggggta    600
tctaccacca ataatatgga attgtgtgct gacgcgaaaa aagtccaacg gcaaatatta     660
catgtgcctg gcttttgggc tgaattatcc tggcatttac gccagcgtac tacctcaaag     720
acgaaagcct tatataacaa aagctga                                         747
```

<212> Type : DNA
<211> Length : 747
SequenceName : SEQ ID 465
SequenceDescription :
Sequence
--------
<213> OrganismName : Haemophilus influenzae Rd
<400> PreSequenceString :

```
atgaaattaa caacactgca aaccttgaaa aaagggttta cattaatcga gctaatgatt      60

gtgattgcaa ttattgctat tttagccacc atcgcaattc cttcttatca aaattatacc     120
aaaaaagctg cggtatccga attactgcaa gcgtctgcgc cttataaggc tgatgtggaa     180
ttatgtgtat atagcacaaa tgaaacaaca agctgtacag ggggaaaaaa tggtattgca     240
gcggatataa agacagcaaa aggctatgta gcctcagtta tcactcaatc aggtggtatt     300
acagtaaaag ggaatggcac attggcaaat atggaatata ttttgcaagc taaaggtaat     360
gctgcagcag gtgtaacttg gacaacaacc tgcaaaggaa cggatgcctc tttatttcca     420
gcaaattttt gcggaagtgt cacaaaatga                                      450
```

<212> Type : DNA
<211> Length : 450
SequenceName : SEQ ID 466
SequenceDescription :
Sequence
--------
<213> OrganismName : Haemophilus influenzae Rd
<400> PreSequenceString :

```
atgctaaata aaaaattcaa actcaatttt attgcgctta ctgtcgccta cgcattaacc     60
ccttatacag aagctgcgtt agtgagagac gatgtggatt atcaaatatt tcgtgatttt    120
gcagaaaata aagggagatt ttctgttggt gcaacaaatg tggaagtgag agataaaaat    180
aaccactctt taggcaatgt tttacctaat ggcattccga tgattgattt tagtgttgtg    240
gatgtagata aacgcatcgc cacattgata aatccacaat atgtagtagg tgtaaaacac    300
gttagtaacg gcgtgagtga actacatttt gggaacttaa atggcaatat gaataatggc    360
aatgctaaat cgcaccgaga tgtatcttca gaagaaaata gatattttc cgttgagaaa    420
aatgagtatc caactaaatt gaatggaaaa gcagtaacta ctgaagatca aactcaaaaa    480
cgccgtgaag actactatat gccacgtctt gataaatttg ttaccgaagt tgcaccaata    540
gaggcttcaa ctgcaagtag tgatgctggc acatataatg atcagaataa atatcctgct    600
tttgtaagac taggaagtgg tagtcaattt atttataaaa aaggagataa ttacagctta    660
attttaaata atcatgaggt tggaggcaat aatcttaaat tggtgggcga tgcctatacc    720
tatggtattg caggcacacc ttataaagta aaccacgaaa ataatggact aattggtttt    780
ggcaattcaa aagaggaaca cagcgatcca aaaggaatat tatctcaaga tccgcttacc    840
aattatgctg ttttaggcga cagtggctcc ccattatttg tatatgatag agaaaaagga    900
aaatggcttt ttcttgggtc ttatgatttt tgggcaggtt ataacaaaaa atcttggcaa    960
gaatggaata tttataaacc tgaatttgca aaaactgttc tagataaaga tactgcaggt   1020
tctttaactg gttctaacac ccaatacaat tggaatccta ctggcaaaac aagcgttatt   1080
tctaatggtt ctgaatctct aaatgttgat ttattcgata gtagtcagga tactgactct   1140
aagaagaaca atcacggaaa aagtgtgact cttagaggaa gtggaacgct taccttaaat   1200
aataatatcg atcaaggcgc aggcggcttg ttctttgaag gagattatga agttaaaggc   1260
acttctgata gtaccacttg gaaaggagct ggcgtttctg ttgctgatgg aaaaacagta   1320
acgtggaaag tacataaccc gaaatctgat cgtttagcta aaatcggcaa aggaacatta   1380
attgtagaag gaaagggaga aaataaaggt tcgctaaaag tgggcgatgg tactgttatc   1440
ttaaaacaac aagctgatgc caataataaa gttaaagcct tttcacaagt aggtatagta   1500
agtggtcgct caactgttgt acttaatgat gataagcaag tagatccaaa ttccatttac   1560
tttggcttta gaggtggtcg attagatgcc aatggcaata atctcacttt tgaacatatc   1620
cgtaatattg atgatggcgc aagactagta aatcacaata ccagcaaaac ctctactgta   1680
acaattactg ggaaaagtct aattacagat ccaaatacaa ttactccata taatatagac   1740
gcaccagatg aagataatcc ttatgccttt cgacggatta aagatggagg acagctctat   1800
ttaaatttgg aaaattacac ttattatgcg ttaagaaaag gtgcgagcac tcgttcagaa   1860
ttacctaaaa atagtggcga aagcaatgaa aattggctat atatgggtaa aacttccgat   1920
gaagccaaaa gaaatgtaat gaaccatatc aacaacgagc gtatgaatgg ctttaacggt   1980
tattttggcg aggaagaggg taaaaataac ggtaatctaa atgtgacttt taaaggcaaa   2040
agtgagcaaa atcgcttttt attaacaggc ggaacaaacc ttaatggcga tttaaaggtt   2100
gaaaaaggca cattattcct ttctggcaga ccaacaccgc acgcaagaga tattgcaggt   2160
atttcttcga caaaaaaaga tcaacacttt gctgaaaata atgaagtggt agtagaagat   2220
gactggatta accgcaattt taaagcaaca aatattaatg taaccaataa cgcaacccttt  2280
tattcaggtc gcaatgttgc aaacattact tcaaatatca cagcttctga taatgcaaaa   2340
gtacatattg gctataaagc aggcgatacc gtttgtgtac gttctgacta tacgggctat   2400
gtgacttgca ctactgacaa gttatccgat aaagccctta atagctttaa cgccaccaat   2460
gtatctggca atgtaaattt atcaggtaat gcaaactttg tcttaggcaa agctaactta   2520
ttcggcacaa ttagcggcac gggaaatagc caagtacgtt taaccgaaaa tagccattgg   2580
catttaacag gcgatagcaa tgttaatcag ttaaatttag acaaggggca tattcattta   2640
aatgcacaaa acgatgcaaa taaagtaact acatataaca cgctgactgt gaatagctta   2700
tcaggtaacg gttctttcta ttatttaact gatctttcca ataaacaagg cgacaaagtt   2760
gttgtaacta aatccgccac aggtaacttt acattacaag tggcagataa aacaggcgag   2820
cctacaaaaa atgaactcac gctttttgat gcgtcaaatg ctacaagaaa taatttgaat   2880
gtgtcattag ttgggaatac cgttgattta ggtgcttgga aatataaatt acgtaatgtt   2940
aatggacgtt acgatttgta taacccagag gtggaaaaaa gaaatcaaac tgtcgatacg   3000
acaaatatca caacacctaa taatattcaa gctgatgtgc ctagcgtacc aagtaacaat   3060
```

```
gaagaaatag cccgtgttga aacaccagtt ccaccacctg cgcctgctac accatcagag   3120
acaactgaaa cagtggctga aaatagtaag caagaaagta aaacagtaga gaaaaacgag   3180
caagacgcaa ccgagacaac agctcaaaat ggagaagttg cagaagaagc taaaccaagt   3240
gtaaaagcta atactcaaac aaatgaagtg gctcaaagtg gaagtgaaac cgaggaaact   3300
caaacgactg aaataaaaga aacagctaaa gtagaaaaag aggaaaaggc taaagtagaa   3360
aaagatgaaa ttcaagaagc acctcaaatg gcttctgaaa cgtctccgaa acaagcaaag   3420
cctgctccta aagaagtttc aactgatacg aaagtagaag aaactcaagt tcaagctcaa   3480
ccgcaaacac aatcgacaac tgttgctgcg gcagaggcaa cttcgccaaa cagtaaacca   3540
gcggaagaaa ctcaaccaag tgaaaaaact aacgctgaac ctgtaacgcc tgtagtatca   3600
aaaaatcaaa cagaaaatac gaccgaccaa ccaacagaaa gagagaaaac ggctaaagta   3660
gaaacagaga aaactcaaga accccctcaa gtggcttctc aagcgtctcc gaaacaggaa   3720
cagtctgaaa ctgttcaacc gcaagcagtg cttgaaagtg aaaatgttcc gactgttaat   3780
aatgcagaag aagttcaagc tcaactgcaa acacaaacaa gtgcaacagt aagcactaaa   3840
caacctgcac cagagaattc aataaatact ggatctgcaa ccgcaataac agaaactgct   3900
gaaaaatccg ataaaccaca aacggaaact gcggcttcga ctgaagatgc tagtcagcat   3960
aaagcgaata ctgttgcgga taattctgta gcaaataatt cagaaagcag tgatccaaag   4020
agtagacgta gaagaagtat tagccagcct caagagactt ctgctgaaga aacaacagca   4080
gcttctactg acgaaacaac aatagctgat aattcaaaac gcagtaagcc aaatcgtaga   4140
agtagaagaa gtgttcgctc ggaaccaact gttacaaatg gcagcgatcg ttctacagta   4200
gcattgcgcg atctcacaag tacaaacaca aatgcggtaa tttctgatgc aatggcaaaa   4260
gcacaatttg ttgcattaaa tgtgggggaaa gcagtttctc aacatattag ccagttagaa   4320
atgaataacg aggggcaata taacgtttgg gtatctaata cttcaatgaa cgaaaattat   4380
tcctcaagtc aatatcgtcg ttttagttct aaaagtacgc aaactcaact tggttgggat   4440
caaacaatct caaacaatgt tcagttaggt ggcgtgttta cttatgttcg caatagtaac   4500
aactttgata aggcaagcag taaaaatact ctagcacaag ttaatttcta ttctaaatat   4560
tatgcggata atcattggta tttgggcatt gatttaggct acggcaagtt ccaaagcaac   4620
ctaaaaacca atcataatgc gaaatttgct cgccatactg cacaatttgg tttaaccgca   4680
ggcaaagcat ttaatcttgg caattttggt attacgccaa tagtaggcgt gcgttatagc   4740
tatttatcaa acgctaattt tgcattagct aaagatcgca ttaaagtaaa tccaatatct   4800
gtcaaaacag cctttgctca agttgattta agttatactt atcacttagg cgagttttcc   4860
gttacgccaa ttttgtctgc tcgatatgat acaaatcaag gcagcggaaa aattaatgta   4920
aatcaatatg attttgctta caacgtggaa aaccaacagc aatataacgc agggcttaaa   4980
ttgaaatatc ataatgtgaa attaagtcta ataggcggat taacaaaagc gaaacaagcg   5040
gaaaaacaaa aaactgcaga attaaaacta agttttagtt tttaa                   5085
```

<212> Type : DNA
<211> Length : 5085
SequenceName : SEQ ID 467
SequenceDescription :
Sequence
--------
<213> OrganismName : Haemophilus influenzae Rd
<400> PreSequenceString :

```
atggcattag taaacaaaat taaaacatta tcatcagtag gtattctagc ggctacatta     60
tttcttgcag gctgccaagc acaatcaaat atattagcat ttacaccgcc tgcaccaagt    120
gcttcaatga atgttaatcg aactgccgtt gtatctgtga caacaaaaga tagccgtgca    180
atacaagaga ttgcgagtta tacgaaacac ggggaactga ttaaattaaa tgcatcccca    240
agtgttacac aattatttca gcaagtgatg cagcaaaatt taattagtaa aggttttaga    300
gttgggcaat taaatggttc aaatgcgtgg gtaactgtgg atgtgcgtga atttggtacg    360
caagtagaac aaggtaatct tcgttataaa cttaatacca aaattcaagc gacagtttat    420
gtacaaggtg cgaaaggttc gtataataaa tcatttaatg tcacgcactc acaagagggc    480
gtatttaatg cgggcaatga tgaaattcat aaagtgctat ctcaaacttt taatgatatt    540
gtgaacaata tttatcaaga tcaagaagtt gcggctgcga taaaccaata ttctaattaa    600
```

<212> Type : DNA
<211> Length : 600
SequenceName : SEQ ID 468
SequenceDescription :
Sequence
--------
<213> OrganismName : Haemophilus influenzae Rd
<400> PreSequenceString :

```
atgctgtgct ggattggcta caaaaatggg attttaccgc agcagaacag cacgctatat      60
ccttggctga atccgtcaaa gtgcggcgta atttttgatg gttttcaact tgtgggcgat     120
gatttcaatt cagatcaaac ggctgaaaat acatcgccag cttggcaagt gctttacaca     180
acccatttac aaagttgctc gccgattcat agtggagaaa atttcgcacc catccctttg     240

tataaacaac taaaaaatca accgcaccta agccaagatc tgattaaatg gcaagaaaat     300
tggcaagcct gcgatcaact acaaatgaat ggtgcggtat tagaacaaca atctttggca     360
gaaatttccg atcatcaaag tacgctttca aaacacggac gatatttagc tcaagaaata     420
gaaaaagaaa ctggcatacc gacttactat tatttatatc gtgtaggtgg gcaatcttta     480
gaatctgaaa aatcccgttg ctgcccttct tgtggtgcaa attgggcgtt aaaagacgcg     540
atttttgata cctttcattt taaatgcgat acctgtcgat tagtttcgaa tctatcgtgg     600
aattttttgt aa                                                         612
```

<212> Type : DNA
<211> Length : 612
SequenceName : SEQ ID 469
SequenceDescription :
Sequence
--------
<213> OrganismName : Haemophilus influenzae Rd
<400> PreSequenceString :

```
ttgggtgcat ttgcctttgc ttctgttaca aatgcgaata tttatgctga gggcgatatc      60
ggtttatctc aaactaaagc aaacggtagt aacaatacaa gagttggacc tcgcgtatcc     120
gtgggttata aagtaggaaa tacacgtgtt gcgggtgatt atactcatca tggaaaagtt     180
gatggcacaa aaattcaagg tttaggtgca tcagtattat atgattttga cacgaattct     240
aaagtgcaac cttatgttgg tgctcgtgta gcgactaatc aatttaaata caccaatcgc     300
gcagaacaaa agtttaaaag ttcttctgat attaagctcg gatatggggt tgtagcaggt     360
gcaaaatata agttagatgg caactggtac gcaaatggtg gagttgagta caatcgttta     420
ggtaattttg atagtaccaa agttaataac tatggtgcaa aagttggtgt ggggtacgga     480
ttctaa                                                                486
```

<212> Type : DNA
<211> Length : 486
SequenceName : SEQ ID 470
SequenceDescription :
Sequence
--------
<213> OrganismName : Haemophilus influenzae Rd
<400> PreSequenceString :

```
atgaaaaaac ttctaatcgc aagtttatta ttcggtacga caacgactgt gtttgccgca     60
ccttttgtgg caaaagatat tcgtgtggat ggtgttcaag gtgacttaga acaacaaatc    120
cgagcaagtt tacctgttcg tgctggtcag cgtgtgactg acaatgatgt ggctaatatt    180
gtccgctctt tattcgtaag tggtcgattc gatgatgtga aagcgcatca agaaggcgat    240
gtgcttgttg ttagcgttgt ggctaaatcg atcatttcag atgttaaaat caaaggtaac    300
tctattattc ccactgaagc actaaaacaa aacttagatg ctaacgggtt taaagttggc    360
gatgttttaa ttcgagaaaa attaaatgaa tttgccaaaa gtgtaaaaga gcactatgca    420
agtgtaggtc gctataacgc aaccgttgaa cctattgtca atacgctacc aaataatcgc    480
gctgaaattt taattcaaat caatgaagat gataaagcaa aattggcatc attaactttc    540
aaggggaacg aatctgttag tagcagtaca ttacaagaac aaatggaatt acaacctgat    600
tcttggtgga aattatgggg aaataaattt gaaggtgcgc aattcgagaa agatttgcag    660
tcaattcgtg attattattt aaataatggc tatgccaaag cacaaatcac taaaacggat    720
gttcagctaa atgatgaaaa aacaaaagtt aatgtaacca ttgatgtaaa tgaaggttta    780
cagtatgacc ttcgtagtgc acgcattata ggtaatctgg gaggtatgtc tgccgagctt    840
gaacctttac tgtcagcatt acatttaaat gatactttcc gccgtagtga cattgcagat    900
gtagaaaatg caattaaagc aaaacttgga gaacgcggtt acggtagcgc aacggtaaat    960
tcagtacctg attttgatga tgcaaataaa acattagcga taacccttgt tgttgatgct   1020
ggacgacgtt taactgttcg ccaacttcgc tttgaaggaa ataccgtttc tgctgatagc   1080
actttacgtc aggaaatgcg ccaacaagaa ggaacttggt ataattcaca attagttgag   1140
ttaggaaaaa ttcgcttaga tcgtacaggt ttcttcgaaa cagtcgaaaa ccgaattgat   1200
cctatcaatg gtagtaatga tgaagtggat gtcgtatata aagtcaaaga acgtaacacg   1260
ggtagtatca actttggtat tggttacggt acagagagtg gtattagtta tcaagcaagt   1320
gttaaacaag ataatttctt gggaacaggg gcggcagtaa gtatagctgg tacgaaaaat   1380
gattatggta cgagtgtcaa tttgggttat accgagccct attttactaa agatggtgta   1440
agtcttggtg gaaatgtttt ctttgaaaac tacgataact ctaaaagtga tacatcctct   1500
aactataagc gtacgactta tggaagtaat gttactttag gtttccctgt aaatgaaaat   1560
aactcctatt atgtaggatt aggccatacc tataataaaa ttagtaactt tgctctagaa   1620
tataaccgta atttatatat tcaatcaatg aaatttaaag gtaatggcat taaaacaaat   1680
gactttgatt tttcttttgg ttggaactat aacagcctta atagaggcta tttcccaact   1740
aaaggggtta aagcaagtct tggtggacga gttactattc caggttctga taacaaatac   1800
tacaaactaa gtgcagacgt acagggtttc tacccattag acagagatca cctctgggtt   1860
gtatctgcaa aagcatctgc aggatatgca aatggttttg gaaacaagcg tttaccgttc   1920
tatcaaactt atacagcggg tggcatcggt tcattacgtg gttttgctta tggtagtatt   1980

ggacctaacg caatttatgc agaacatggt aatggtaatg gtacttttaa gaagataagt   2040
tctgatgtga ttggtggtaa tgcaatcaca actgcgagtg cagaacttat tgtaccaaca   2100
ccgtttgtga gtgataaaag ccaaaataca gtccgaacct ccctatttgt tgatgcggca   2160
agtgtttgga atactaagtg gaaatcggat aagagtgggt tagataacaa tgtattaaaa   2220
agcttacccg attatggcaa atcaagccgt attcgcgcct ctacaggtgt cggattccaa   2280
tggcaatctc ctattgggcc attggtattc tcttatgcca aaccaattaa aaaatatgaa   2340
aatgatgatg tcgaacagtt ccaatttagt attggaggtt ctttctaa                2388
```

<212> Type : DNA
<211> Length : 2388
SequenceName : SEQ ID 471
SequenceDescription :
Sequence
--------
<213> OrganismName : Haemophilus influenzae Rd
<400> PreSequenceString :

```
atgttgaaaa aaacatctct tatttttacc gcacttttaa tgactggctg tgtgcaaaat     60
gcgaatgtaa caacacctca agcgcaaaaa atgcaagtag aaaaagtgga taaagcctta    120
caaaaaggcg aagctgatcg atatttatgt caagatgata gagttgttcg tgttgtacac    180
gccacgcata aaaaatacaa aaaaaatttg cattatgtta ctgtcacttt tcaaggcgta    240
tcagaaaaac taaccttaat gatttctgaa cgtggtaaaa attacgccaa tattcgttgg    300
atgtggcaag agcgtgatga ttttagtacg ctaaaaacga atctcggcga aattttagca    360
acgcaatgtg tctcacaaac aagtgaacgc ttatctggac aataa                    405
```

<212> Type : DNA
<211> Length : 405
SequenceName : SEQ ID 472
SequenceDescription :
Sequence
--------

<213> OrganismName : Haemophilus influenzae Rd
<400> PreSequenceString :

```
ttgagaatta ttattatttt ttttatggga ttaaatatga ccaattttag attagaacgt     60
gcttgcctat tccgttatgc ttgggctaac ggcaggtgtt gcttatgcag ctcaaccaac    120
caaccaacca accaaccaac caaccaacca accaaccaac caaccaacca accaaccaac    180
caaccaacca accaaaatag taatgtttct gaacaactag aacaaataaa tgtatctggt    240
tctaccgaaa atagtgatac aaaaacacca ccaaaaattg ctgaaacggt aaaaacagct    300
aaaacgctgg aaagagaaca agcaaacaac attaaagaca tcgttaaata cgaaacgggc    360
gttactgttg ttgaagctgg acgttttggg caaagcggtt ttgccattcg tggtgtagat    420
gaaaatcgtg tagcgattaa tattgatgga ttacgtcaag ctgaaacctt atcttctcaa    480
ggctttaaag agctttttga aggttatggt aatttcaata acacgcgtaa tggtgcagaa    540
attgaaacct taaaagaagt aaatatcaca aaaggggctg attctattaa gaatggtagt    600
ggttccttag gtggttctgt aatttataaa acaaaagatg cgagagatta tctcataaac    660
aaggattact atgtaagtta caaaaaggga tacgctacgg aaaataatca atcattcgat    720
acccttactc ttgcaggacg ttataaaaag ttcgatgtgc tagtggttac aacaagcaga    780
aatgggcatg aacttgagaa ctatggttat aaaaattata acgataaaat tcaaggtaaa    840
aaaagagaaa aagcagatcc atataaaatt gaacaagata gtacattatt aaaattatct    900
ttcaatccta cagaaaatca tcgtttttacc tttgccgctg atttatatga acatcgttct    960
cgtgggcaag atttatccta tacattaaaa tatcaacgta gtggtaatga aacccctgaa   1020
gttgattcta gacacaccaa tgataaaaca aagagacgta atatttcatt tagttatgaa   1080
aatttctctc aaactccatt ttgggatacg ctaaaactca cttattctga tcaacgtatt   1140
aaaactcgtg cacgcacaga tgagtattgt gatgctggtg taagacattg tgaaggcaca   1200
gacaatccta cgggactaaa agtaacaaat gggaaaataa cacgtcgaga tggttcagac   1260
cttcaatttg aggaaaaaaa caatacagct aagagtagtg ataaaaccta tgacttcaag   1320
aaatttattg atactgataa gagagtaata gacgataaac tagtcctaaa caacccctct   1380
gacacttggt atgattgttc aatatttaat tgtgaaaata acgcaaaaat aaaagttttt   1440
aaaggtaaca attattatgg ctatgatgga aaatggaaag aagttgacct tgaaataaaa   1500
gaattaaatg gcaaaaaatt cgctaaaata aaggataatg ataggaaaat aaaatctatt   1560
cttccctctt cacctggtta tttagaacgc ctctggcaag agagagattt ggacaccaac   1620
acccaacaat taaatttaga tttaaccaaa gacttcaaaa tttggcatat tgaacataat   1680
ctacaatatg gtggatcata taataccgcg atgaagcgca tggttaatcg tgctggcaac   1740
gatgcttctg atgtgcaatg gtgggcaaca cctacgcttg gtgaggattc ttggactgga   1800
aaacctcaca cttgtgcaac gacttatgag tggaatgcta acctttgtcc tcgagttgat   1860
cctgaatttt cttacttatt acccattaaa acaacaggaa aatcagtcta tctctttgat   1920
aattttgtta taactgatta tttatctttt gatttgggtt atcgttatga caatatccat   1980
tatcaaccaa aatataaaca cggtatcaca cccaaattac ccgatgatat tgtgaaagga   2040
ttatttattc cattaccaaa caattcaaat tcagatccta ataaagttaa ggaaaatgta   2100

caacaaaata ttgactatat cgccaaacag aacaaaaaat ataaagcaca ttcttatagt   2160
tttgtttcaa ccattgatcc aacgagtttt cttcgtttac aactaaaata ttctaaaggt   2220
tttagaacac caacttcaga tgaaatgtat ttcacctttta aacaccctga tttcactatt   2280
ttgccaaata ctgatttaaa accagaaata gcaaaaacca aagaaattgc tttcacatta   2340
cataatgatg attggggatt tatctcgaca agtctgttta aaactaacta taaaaacttt   2400
attgacctaa tatttaaaaa gcaagaaact tttaaagtag gcggctctgg aagaggtgaa   2460
acattaccat tttctcttta tcaaaatata aatagagata atgcgtcttt aaaaggtatt   2520
gaaattaatt caaaagtatt ccttggtaaa atggcaaaat ttatggatgg atttaaccta   2580
agctataaat atacctatca aaaaggcaga atgaatggca atattcctat gaatgcaatt   2640
cagcctagaa ctatggtata tggtttagga tatgatcatc caaatcataa atttggtttc   2700
gatttctata cgacacatgt agcaagtaaa aatccagaag atacttataa tatgttctat   2760
aaagaagaaa ataaaaaaga cagcacaatt aaatggagaa gcaaatctta tactattcta   2820
gatttaattg gatatgtaca accaattaaa aatttaacca taagagctgg tgtatataat   2880
cttacaaacc gtaaatacat tacttgggat tctgctcgtt caattcgttc atttggaaca   2940
agtaatgtta tagatcaatc aacaggatta ggcattaacc gcttctacgc accaggtaga   3000
aattataaaa tgtcagttca gtttgaattt taa                                 3033
```

<212> Type : DNA
<211> Length : 3033
SequenceName : SEQ ID 473
SequenceDescription :
Sequence
--------
<213> OrganismName : Helicobacter pylori, strain J99
<400> PreSequenceString :

```
atgacttata gaaatggcaa aatagattta aaggaacgct ttagtaaaaa ccgctctttt     60
aagggcatta aaaagaaaat cgctaaaaaa tatacaatca aaaactcgct ttctataatt    120
tattccttaa aaacgcattc aaattcttct ctatccatta ataaaaaaat cttcttaggg    180
ctagggttcg tttcggcttt gagcgctcaa agtgaagatt ataatagttc ggtgtattgg    240
ctcaatagcg tgaatgaaaa caataataac aaatcctact atattagccc cttacgcact    300
tgggctgggg ggaataggag ttttacgcaa aattataaca atagtcaatt atacataggg    360
acaaaaaacg cttccgcaac gcccaatcat tcttctgtgt ggtttggaga aaagggctat    420
atcggtttta ttacaggggt ttttaaggct agagacattt ttatcacagg agctgttgga    480
tcgggtaatg agttaaaaac cggtgggggg gcgatactgg tttttgaaag ctcaaacgaa    540
ctaaccacta acggggctta ttttcaaaat aacagagccg ggacacaaac ttcttggatc    600
aatttgattt ccaataacag cgtgaatttg acaaacacgg attttggcaa tcaaacccct    660
aatgggggct ttaatgttat ggggcgaaag attacttata atggtgggag cgtcaatggt    720
gggaattttg gctttgataa cgtggatagc aatggcgcaa ccaccattag cggggtaact    780
ttcaacaata acggtgcgct cacttataag ggtgggaatg gtattggagg gagcatcact    840
ttcactaact ctaatatcaa tcattacaag ctcaatctta acgctaatag cgttaccttt    900
aataacagca ctctagggag tatgcctaat ggcaacgcta acactatagg gaatgcctac    960
attcttaatg caaataatat tacttttaat aatttgacct ttaatggggg ttggttcgtt   1020
tttaatagat ctgatgctca tgttaatttt caaggcacaa ctacgatcaa taaccccact   1080
tcacccttttg tcaatatgac cggtaaagtt accattaatc ctaatgcgat ttttaatatt   1140
caaaattaca cgcccacgat agggaacgct tacacgctct ttagcatgaa aaatggcaat   1200
atcgcttatg atgatgtgaa taatttatgg aatattatca ggcttaaaaa cacgcaagcc   1260
acaaaagaca atagcaaaaa cgccacttcc aataacaaca cccacactta ctatgtaact   1320
tacaatttag gcggcacgct ctatcatttc aggcaaattt ttagccctga ttccattgtt   1380
ttacaatccg tctattatgg cgcgaataat ctttactaca ccaatagcgt gaatatccat   1440
gacaatgtct ttaatttaaa aaatattaac gatgataggg ctgatacgat tttttatctt   1500
aatggcttga acacttggaa ttacacgcaa gcgagattcg ctcaaaccta tggcgggaaa   1560
aacagcgctt tagtctttaa cgccacgact ccttgggcta atggtgcgat ccctaaatct   1620
aacagcacgg tgcgttttgg ggggtatgag ggagtcaatt gggggaaaac gggctatatc   1680
accggcactt tcacagccga tagggtttat atcaccggta acatgatgtc tggcaatggc   1740
gctcaaaccg gtgggggggc gactttgaat tttgtgggcg cgactgaaat taatatcgct   1800
ggagccactt ttaaaaactt aaaaaccact tcacaaaact cttacatgac ttttatggcg   1860
ttagggaatg gctctgggag tggtaagatc aatgtttctc agtctgattt ttacgattgg   1920
acggatggag ggtatgattt taccggtaat ggcgtttttg acagcgtgaa tttcaacaag   1980
gcttattaca aatttcaagg cgctgaaaat tcttacaatt ttaaaaacac gaatttttta   2040
gcagggaatt tcaaattcca gggcaagacc accattgaaa aatccgtttt aaacgacgct   2100
tcttacgctt ttgatggcgt gaataacgcc tttaatgaag acaaatttaa tggcggatcg   2160
tttaatttca accacgcaga gcaaacaaac gcttttaata acaactcgtt tagtggcgga   2220
tcgtttagtt ttaacgccaa gcaagtggat tttaatggga attcgtttaa tggggggggtg  2280
tttaatttca ataatacccc taaagccagt tttactaacg acactttttaa tgtgaataac   2340
caattcaaaa taaatggcgc tcaaacggat tttactttca gtaagggcgt tgttttcaac   2400
atgcaagggc ttttgagcag tttgagcgta ggcacgactt atcaattgct taacgctaaa   2460
agcgtgggtt ataaggataa caataacgct ttgtatcaaa tgttgcgctg gactagcgga   2520
```

gaaaatccta gcggtaaatt agtagatgaa aataaaaccg cgccaaacag cgctaaaatt 2580
tataatgttc aattcactga taacggcttg acttactaca ttaaagaaaa ttttaataat 2640
gggatcacgc tcactcgttt atgcactcta ggctacacgc attgcgtgaa tattgataac 2700
gatgcgttta atcttaaaaa tgtcaataat aacgctagta acaccgtgtt ctatctcaac 2760
ggcatgacga cttggaagac tgctggcaca ggagttttca cgcaagatta cagcggcact 2820
aacagcgttt tagtgttcaa tcagaccacc cctttcttg ctggggcgaa tcccacttcc 2880
aatagcgtgg tgggttttgg gaaaacttca ggggctgaat gggggctagt gggctatatt 2940
caaggcgttt ttaaagccaa tcaaattgat attaccggca cgattcgctc tggtaatgga 3000
gccaaaaccg gtgggggcgc gactttagtg tttaacgctc aagagcgttt gaatatcgct 3060
aacgctaatt tgaataacga taaagccggt ttgcaaaact catggatgaa tttcattgtt 3120
aataatggca atttgaacgt aacaaacgca aattttagca accaaacccc gcatggaggc 3180
tttaacctta aagccaataa tatcacttgg gataaaggct ctgtgagtgg ggggggggaat 3240
tttggtgtgg ataacgccaa cgctaatggg aatgcggtga ttaagaatgt taatttcagc 3300
gataacggca ctttgattta taaaggggggt gaaaacagcg ccggaaattc cctgacctta 3360
gaaaacaaca ccttcaattc ctataatatc aacgccaaag cgcaaaacct tatttcaac 3420
aacaactcgt ttaacagcgg tagctattcg tttaacgaca ctaaaaatgt tacttttaaa 3480
ggcacgaaca cgctcattaa cagcgatcct ttcagccgcc ttaaaggatc agtttctatt 3540
gacaataata gtatctttaa cattgaaagg gatttgaccg ataaaaccac ttacacgctt 3600
ttaagtgggg ataacatcaa atacaataac caagctttag cggataatgt tttttcaaaa 3660
aatttatggg atttgatcca ttatgacggc gaacaaggga ctctattaag aacggataac 3720
aacacttatt ttgtgcaatt cacgcagagc aacggccaaa aatttgtttt tgaagagact 3780
tttaatcctg gctctatcac ctataaatat ttcactatcc attcttcgcc tttccacaca 3840
gaagctgatt ctaaggatat ttggaatcag gtgaggaagc agtttgattt tattccagga 3900
aaaaccccgg tgtgcgttgg cgtgtgctat atcgcaccct ataaaaatca agatcttatc 3960
ggctctagcg cttttgcgtg gtcgctgaat tttggggcta cggtggtggg gactttgctt 4020
ttagggagcg ctcaagaaaa agccaataac aatggcggct cgatctggtt tggtaagaat 4080
aatttgctgt atttgcatgg caattttaac gcgactaata tctttttaac gaataacttt 4140
aatgtcggca accctaacgc cggcggtggg gcaacgatta attttaacgc tgatgaaacc 4200
ttgagcgctg acgggttgaa ttacacgaat ttccaaaccg tggctatggg cttacaaact 4260
agcgcgagcc agcattcatg ggcgaatttt aattccaagc tttctatgga gattaaaaac 4320
tccaacttta gggatttcac atggggaggc tttaggttca attcagggcg tatcactttt 4380
gaaaacacca cttttagcgg ctggactaat attaacgggg cgactgaaag cggttcatcg 4440
tatgtgaaca tggttgcgaa tacggatttg attttcactg attccatttt aggagggggc 4500
attcgctatg atttgaaggc taataacatt attttcaata acactcaaat ggtggttgat 4560
gtgtctaaaa acgtgaatca gtcttcattg aatgggaatg ttactttcaa tcattccagg 4620
ctttcagtca aacccaatgc ggctatcaat attgggggggg atcagaccca aacgacttta 4680
gaaaacgctt caagcctttc tttttataac gatagcgtag cgaattttaa cggcacgacc 4740
gcttttaacg gggtgtctta cttgaattta aaccctaacg ctcaagtcag cttcaatcaa 4800
gcgaatttca ataacgctaa tgtaaccttt tatggcattc cgctatttgg taaaacgccc 4860
aattttggca actctgtgcg ccttatcaat ttcaaagggg acgcaaagtt taatcaagcc 4920
acgctcaatt taagggctaa aatatccat ttgaatttcc aaggggcttc cacttttgaa 4980
aataactcta cgatgaattt ggctgaaagt tctcaagcga gctttaacgc tcttagcgtg 5040
gagggggaaa cgaatttcaa tctcaacggc tcaagtttat tgagtttcaa tggtaacagc 5100
gtttttaacg cccctgtgaa tttctacgct aataattctc aaatttcttt cactcattcg 5160
gcgactttta atgcagacgc ttcatttgat ttaggcaata acagcaccct gaattttcaa 5220
agcgttcttt taaacagcgc tctaaacctt ttaggcaatg gcggtaacaa tctagcgatt 5280
aatgctaaag ggaattttag ttttggatct caagggattt tgaatctgtc ttatatgaat 5340
ctatttggag ggggataaaaa agcttccgtt tatgatgtgt tgcaagccca aaatattgat 5400
ggcttaaggg ggaataacgg ctatgagaag atccgttttt atggcataca gattgaaaag 5460
gccgattact cgttcaataa tggcgttcat tcttggagct tcactaaccc gctcaacacg 5520
actgaaacca ttaccgaaac cttacataat aaccgcttga aagtgcagat ctctcaaaac 5580
ggtgcttcta ataatgcgat gtttaatctc gctcctagct tgtatgatta ccaacaaaac 5640
ccttatgatg aaagcgagaa ttcctataat cacacaagcg ataaagccgg cacttattat 5700
ttgagtagca gtatcaaagg ctttggtaag aataatgaaa tacccgggac ttataacgcg 5760
caaaaccaac ccttacaagc tttacacatt tataatcagg ctatcagtaa gcaggatttg 5820
aacatgatcg ccagtttggg taaggaattt ttgcctaaag tggctaaact tatcgcttca 5880
ggggctttag acaatctcaa tctcaatagc ccggatagct ttgaaacgat ttttagtatc 5940
ttaaaagaat atggcattac tttaaaccaa gcgaattgga agagcttatt gaagatcatc 6000
aataattttt ctaacacggc taattatcat ttttctcaag gtagtctcgt tgtgggggcg 6060
atcaaagaag ggcaaacgaa cacgaatagc gtggtgtggt ttgggggcga tgggtataaa 6120
aatccatgcg cggttgggga taacacttgc cagatgttca ggcagactaa tttagggcag 6180
ttgcttaatt ctagcgtgcc ttatttgggt tacattaacg ctaattttaa ggctaaaaac 6240
atttatatca ccggaaccat cggcagtggg aacgcttggg ggagcggagg gagcgcgaat 6300
gtgtcttttg aaagcgcgac aaatttggtg cttaatcaag ccaatattga cgctcaaggg 6360
accgataaga tcttttctta cttgggcaaa gagggcattg ataagctttt tggagaaaaa 6420
ggtttaggga atgtgctttc taatattgtt tatgaagaga gcttgaatga taacgctatc 6480
cctaaagatt tagccaacat gatccctaaa gatttgggat ctaaaacttt aagctctttg 6540

cttagcccta ctgaagtgaa taacctctta ggcgtgagtg cttttaaaaa cgcgatcatg 6600
gaaatcttaa attctaaaac ggtgggcgat gtttttggtg aaaacgggct tttaaacgcg 6660
ctagatcctg taaaaagaaa agaaattgat caaatgcttt tagagcaaat ccaagcccat 6720
tcttcagggt ttgaaaaatt catcgttaaa actttaggga ttgaaaatgt agagaatttc 6780
atcaataact ggtatggcaa gcaaagcttg agttcttttg ccaataattt tgtgcctgga 6840
ggcttgaatc aagccctaga taaaataggt tctagctctg atgccaaaga cttacagagt 6900
ttcttagata aaacgacttt tggggatatt ctcaatcaaa tgatcaatca agccccttta 6960
atcaataagc tcatttcttg gctcggcccg caggatttga gcgtgttagt gaatatcgct 7020
ttaaatagta tcactaaccc tagtaaggaa ttattgggtg cgatttctgg catgggtcaa 7080
aaagtgctga acgatttgct aggcgaaggt gtagtgaata aaatcatgag caatcaagtt 7140
ttagggcaaa tgatcaataa aatcatcgct gataagggct ttggaggcgt ttatcatcaa 7200
ggtctaggct caatactccc taaatcctta caagatgagt tgaagaaatt gggtatgggc 7260
tctttactca aacctaaagg cttgcacaac ctctggcaaa aagggaactt caatttcgtg 7320
gctaaaaacc atgtgtttgt gaataacagc ttgtttagta acgccacagg gggggaattg 7380
aattttgtag cgggcaagtc cattattttt aatgggaaaa acaccattaa tttcacgcag 7440
tatcagggca ggctttcttt tgtatctaaa gatttttcta atatttcatt agacacttta 7500
aacgctacca acggcttaac gcttaacgct tctaaaaatg atattagcgt tcaaaaaggt 7560
caaatttgcg tgaatgtctt agattgcatg accgctaaag ggaaaaccac tcaaactaat 7620
tcctcttcaa gcgcgacagc cccaactaat gaaacgctag aagtgagtgc gaataatttc 7680
gctttcttag gcaccattaa ggctaatgga ttagtggatt tttcaaaagt cttacaaaat 7740
acgactatcg ggactttgga tttagggcca aacgctactt ttaaagcgaa taatttgatc 7800
gtgaataacg cttttaataa taactctaat tacagggcta atatcagcgg taatttcaat 7860
gtggccaagg gtgcaacttt tagcacgaat gaaaatggtt tgaatgtggg ggggaatttt 7920
aacagcgaag ggccattaat ctttaatctt aataacccca cccatcaaac gattatcaat 7980
gtaactggca cttctacaat catgtcttat aacaatcaag ctttaatcaa ctttaacacc 8040
caactcaagc aaggcgctta tacgcttatt aacgctaatc gcatggttta tggctatgat 8100
aatcaaacga ttcttggggg gagcttgagc gattacctca aactttacac tctcattgat 8160
tttaacggca aacgcatgca attgaatggc gattcgttaa gctatgacaa ccaaccggtc 8220
agtattaaag atgggggtct tgtggtaagc tttaaagaca atcaagggca aatggtgtat 8280
tcatctatcc tttatgataa aatccaagtt accgtctctg ataaacccat gagcattcaa 8340
gcccctagtt tggagtatta tgttaaacgc attcaaggta gtgctggttt gaatgcgatc 8400
aaatctgcgg gcaataattc cattatgtgg ttgagtgagc tttttgcggc taagggggt 8460
aatcccttgt tcgcccctta ttatttgcaa gacaatccca ctgaacacat tgttacttta 8520
atgaaagata ttaccagcgc tttaggaatg ctctctaact ccaatctcaa aaacaactcc 8580
actgatgttt tacagctcaa cacttacacg caacaaatga gccgtttagc caagctttct 8640
aatttcgctt cttttgattc aacggatttt agcgaacgct tgagcagtct taaaaaccaa 8700
agatttgccg atgctgtccc taatgcgatg gatgtgattt taaaatactc tcaaagggat 8760
aaactaaaaa acaacctttg ggcgaccggt gttgggggcg tgagctttgt ggaaaatggc 8820
acaggaacgc tctatggtgt caatgtgggc tatgatcgct ttgttagagg ggtaattgtt 8880
ggagggtatg cggcttatgg gtatagcggg ttttatgagc gcatcactag ttctaaatcc 8940
gataatgtgg acgtgggttt gtatgctagg gcttttatca aaaagagcga gctgactttt 9000
agcgttaatg aaacttgggg ggctaataaa acccaaatca gctccaacga cgctttgctt 9060
tctatgatca atcagtccta taaatacagc acatggacaa cgaacgcgaa agttaattac 9120
gggtatgatt tcatgtttaa aaacaaaagc atcattttaa aacctcaaat tggtttaagg 9180
tattactata ttggtatgag cggtttagaa ggggtgatga ataacgtgct ctataaccag 9240
tttaaagcga acgccgatcc gtctaaaaaa tccgttttaa cgattgattt tgctttggag 9300
aaccgccatt atttcaacac aaactcttat ttttatgcga ttggtggcgt tggtagagac 9360
ttgttagtta attctatggg ggataaattg gtgcgtttta ttggtaacaa cactttgagt 9420
tacaggaaag gcgatcttta taacactttt gcgaacatca ctacaggcgg ggaagtgagg 9480
ttgtttaaaa gcttttatgc gaacgctggg gtgggggcta ggtttggatt ggattacaaa 9540
atgatagata ttataggaaa tattggaatg cgtttagcgt tttaa 9585

<212> Type : DNA
<211> Length : 9585
SequenceName : SEQ ID 474
SequenceDescription :
Sequence
--------
<213> OrganismName : Helicobacter pylori, strain J99
<400> PreSequenceString :

```
atgaaacaat ttaaaaagaa accaaaaaag ataaaacgat cgcatcaaaa tcaaaaaaca     60
atcttaaagc gtcctttatg gcttatgcct ttactgattg gcgggtttgc tagtggggtg    120
tatgcggatg gaacagacat tttggggctt agttgggggg aaaaaagcca aaaggtatgc    180
gtgcatcgtc catggtatgc tatatggagt tgcgataaat gggaggaaaa aacacaacaa    240
tttacaggaa accaactcat cacaaaaact tgggcagggg gtaatgcggc taactactac    300
cactctcaaa acaaccaaga catcacagcc aatttaaaaa atgataacgg cacttatttt    360
ttaagcggtc tgtataacta caccggaggg gaatataatg gggggaattt agacattgaa    420
```

ttaggcagta acgctacttt taatctaggt gcgagtagtg ggaatagctt cacttcttgg 480
tatcctaatg ggcatactga tgttactttt agcgctggga ctatcaatgt gaataacagc 540
gtagaagtgg gcaatcgtgt gggatcggga gctggcacgc acaccggcac agccacttta 600
aacttgaacg ctaataaggt tactatcaat tccaatatca gcgcgtataa aacttcgcaa 660
gtgaatgtag gcaatgctaa cagcgttatt accattaatt cggtttcttt aaatggggat 720
acttgcagtt ctttagctag ggtgggcgta ggggctaatt gctccacttc tgggcctagc 780
tattctttta aagggacgac taacgctact aacacgactt ttagcaattc aagcggcagt 840
ttcacttttg aagagaacgc cacttttagc ggggcgaaat taaatggggg ggcattcact 900
ttcaataaaa agtttaacgc taccaataat accgctttta atagcggtag ttttactttt 960
aaaggcacaa gctcttttaa tggtgcgaat tttagtaacg cttcctatac ttttaataat 1020
caagccactt tccaaaacag ctcctttaat ggggggactt ttacttttaa tgaccagacc 1080
aatcaaagca cccagcaccc ccaaattcaa aacagctctt ttagcggcag tgctaccact 1140
cttaagggtt ttgcgacttt tgagcaagcc tttaacaatt caaaccacca actaacgata 1200
caaaacgctt cctttaataa cgctactttc aacaataccg gtaaaatcac tatagaaaaa 1260
gatgcgagct ttaataacac ttcgttcaac actcctgttg atacaaacaa catgactatt 1320
agtggtggcg ttactttaag cggtaaaaat gacttgaaaa atggtgcaac ccttgatttt 1380
gggagttcta aaatcactct cactcaaggg acgactttca acctcacaag tttaggcagt 1440
gagaagagcg taacgatttt aaattctaga ggtgggatca cttacaatca tcttttaaac 1500
catgcgatca atagcttgac aaacgcccta aaaacgaacg aaagctcttc aaaaccgcaa 1560
agtttcgctc aaggtttgtg ggatatgatc acttacaatg ggttaccgg gcagcttttg 1620
aatgaaaacg ctgcaacatc taaacccact gactcttcgc cctctaaatc ctctacaaac 1680
tctacgcaag tctatcaagt gggttacaaa ataggggata ctatctacaa actgcaagaa 1740
actttcagcc acaattccat tattattcag gctttagaga gcgggactta cacgccaccc 1800
cctgtcatta acggctccaa atttgactta tccgcttcaa attatatcaa tgctgacatg 1860
ccttggtata accataaata ttatattcct aaatcccaaa attttacaga gagcgggact 1920
tattacttgc cgagcgttca aatatggggg agctacacta actcgtttaa acaaaccttt 1980
agcgcaagta atagcaatct ggtgattggg tataacgcaa catggactga tcacaatgtt 2040
tcttctagcg acacggtggc ttttggggac acttcaggga gcgctcttaa tgggcattgc 2100
gggccttggc cctattacca atgcacaggc acgactaacg gcacttatag cgcttatcat 2160
gtctatatca cagcgaatct gcgttctggc aatcgtatag gcaccggtgg ggcagccaat 2220
ctaatcttta atggggtaga tagtatcaat atcgctaacg ctaccatcac gcaacataac 2280
gccggggctt attcaagctc tatgactttt tccacgcaaa acatggacaa ttcgcagaat 2340
ttgaatggcc taaattctaa cggcaagctt ttggtgtatg gcacaacttt cactaaccaa 2400
gccaaagacg ggaaattcat tttcaatgca gggcaagcga cttttgaaaa caccaacttt 2460
aatggaggga gttaccaatt cagcggcgat agcttgaatt tttcaaataa caaccagttc 2520
aatagcggtt cgtttgagat tggcgcaaaa aatactattt ttaataacgc taattttaac 2580
aacagcactt cttttaattt caataattct agcgcgacca cttcgtttgt gggggatttc 2640
actaacgcta attcaaattt gcaaatcgct gggaacgctg tttttgggaa ctctactaat 2700
ggctctcaaa ataccgctaa ttttaataat accggctctg ttaatattgc agggaatgca 2760
acctttgata acgtggtatt taacagccct acgaacacga gcgtgaaagg gaaagttact 2820
ctcaataaca tcactttaaa aaacttgaac gctcctttgt cttttggcga tgggacgatt 2880
gtttttagcg ctcattcggt gattaatatt ggtgaagcta tcacaaatgg caaccctatc 2940
acccttgtaa gctcttctaa agcaattgaa tacaacgacg ctttcagtaa aaatctatgg 3000
cagctcatca actaccaagg gcatggggct agcagtgaaa agctcgtttc tagtgcgggt 3060
aatggcgtct atgatgtggt gtattctttc aacaaccaaa cctacaattt ccaagaggtt 3120
ttttcaccca acagcatttc tatccggcgt ttgggcgttg gcatggtgtt tgattatgtg 3180
gatatggaaa aatcggatcg tttgtattat caaaacgctc tcggttttat gacctacatg 3240
cctaatagct ataacaataa tttagggaat ttaaacaaca ccatttacta ttacgacaac 3300
agcattgact tttatgcgag cgggaaaact ctattcacta aagcggaatt ttctcaaacg 3360
ttcactgggc aaaacagcgc gatcgttttt ggggctaaaa atatatggac gagcgtaagc 3420
gatgcgccgc aatctaatgt gatcattcgc tttggggaca ataagggagc agggagtaat 3480
gatgcgagtg ggcattgctg gaatttgcaa tgcataggct ttatcacagg gcattatgaa 3540
gcgcaaaaga tttacatcac cggcagtatt gaaagcggga accgcatttc tagcggtggg 3600
ggcgcgagcc ttaattttaa cgggcttcaa ggcattcttt taacgaacgc gactttgtat 3660
aaccgcgccg ctggcacgca aagctcttct atgaattttg tttctaacag cgcgaacatt 3720
caggctcaaa actcctattt tatagacgat accgcacaaa ataaaggcaa ccctaatttt 3780
agtttcaacg ctttgaatct ggatttttct aacagctctt ttagaggcta tgtggggcaa 3840
acgcagtctg tttttaaatt caatgccgtt aatgcgatca gtttcactaa cagctctaat 3900
ttaagctctg gtttgtatca aatgcaagct aaaagcgtgt tgtttgacaa ttccaattta 3960
agcgtttcag tggggacaag cagcattaaa gccaatgcga tcaatctttc tcaaaacgcc 4020
tctatcaatg cgagcaacca ttcaacctta gaacttcaag gcgatttgaa tttgaacgac 4080
accagctcgc tcaacctcaa ccaaagcgcc attaatgttt ctaacaacgc cacgatcaac 4140
gattatgcga gcttgattgc gagtaatggc tctcacctta attttaacgg ggcggttaat 4200
ttcaattcag cgaatattac tacgagtttg agtagttcct ctatcgtgtt taagggggcg 4260
gtctctttac gagggcagtt taatttaagc aataattctt ctttagattt tcaaggctct 4320
agcgctatca cctctaacac ggcgtttaat ttctatgata acgctttttc tcaaagcccc 4380
atcactttcc atcaagccct tgacattaaa gtgcccttga gtttgggagg caacctctta 4440

aaccctaaca acagtagcgt gctgaattta aaaaacagcc agcttgtttt tagcgatcaa 4500
gggagcttga atatcgctaa cattgattta ctaagcgatc tgaatggtaa taaaaatcgt 4560
gtgtataaca tcattcaagc ggacatgaat ggtaattggt atgagcgtat caacttcttt 4620
ggcatgcgca ttaatgatgg gatttatgac gctaaaaacc aaacttatag tttcactaac 4680
cctctcaata acgccctaaa aatcaccgag agctttaaaa ataaccaact gagcgttacg 4740
ctctctcaaa tcccgggcat taaaaacacg ctctataaca ttggctctga aatctttaac 4800
taccaaaagg tttataacaa cgctaatggc gtgtattctt atagcgatga cgcacaaggc 4860
gtgtttttatc tcacgagcag cgtgaaaggc tattacaacc ccaaccaatc ctatcaagcc 4920
agcggcagca ataacaccac gaaaaataac aatctaacct ctgaatcttc tgtcatttcg 4980
caaacctata acgcgcaagg caaccctatc agcgcgttac acgtctataa caagggctat 5040
aatttcagta atatcaaagc gttagggcaa atggcgctca aactctaccc tgaaatcaaa 5100
aagatattag ggaatgattt ttcgctttca agtttgagca atttaaaagg cgatgcgcta 5160
aaccagctta ccaagctcat cacgcctagc gattggaaaa acattaacga gttgattgat 5220
aacgcaaaca attcggtcgt gcaaaatttc aataacggca ctttgattat aggagcgact 5280
aaaatagggc aaacagacac caatagtgcg gtggtttttg ggggcttggg ctatcaaaag 5340
ccttgcgatt acactgatat tgtgtgccaa aaatttagag gcacttattt ggggcagctt 5400
ttggagtcca tctcggctga tttgggctat attgacacga cttttaacgc taaagaaatt 5460
tatcttaccg gcactttagg gagcgggaac gcatggggga ctgggggggag tgcgagcgta 5520
acttttaaca gccaaacttc gctcattctc aaccaagcga atatcgtaag ctcgcaaacc 5580
gatgggattt ttagcatgct gggtcaagag ggcatcaata aggttttcaa tcaagccggg 5640
ctcgctaata ttttgggcga agtggcaatg caatccatta acaaagccgg gggattaggg 5700
aatttgatag taaatacgct agggagtgat agcgtgattg gggggtattt aacgcctgag 5760
caaaaaaatc aaaccctaag ccagcttttg ggcagaata attttgataa cctcatgaac 5820
gatagcggtt tgaacacggc gattaaggat ttgatcagac aaaaattagg cttttggacc 5880
gggctagtgg ggggattagc cggactgggg ggcattgatt tgcaaaaccc tgaaaagctt 5940
ataggcagca tgtccatcaa tgatttattg agtaaaaagg ggttgttcaa tcagatcacc 6000
ggctttattt ccgctaacga tatagggcaa gtcataagcg tgatgctgca agatattgtc 6060
aagccgagcg acgctttaaa aaacgatgta gccgctttgg gcaagcaaat gattggcgaa 6120
tttttaggcc aagacacgct caattcttta gaaagcttgc tgcaaaacca gcagattaaa 6180
agcgttttag acaaagtctt agcggctaaa ggattagggt ctatttatga acaaggtttg 6240
ggggatttga tccctaatct tggtaaaaag gggatttcg ctccctatgg cttgagtcaa 6300
gtgtggcaaa aaggggattt tagtttcaac gcgcaaggca atgtttttgt gcaaaattcc 6360
actttctcta acgctaatgg aggcacgctc agttttaacg caggaaattc gctcattttt 6420
gccggaaaca accacatcgc tttcactaac cattctggaa cgctcaattt gttgtctaat 6480
caagtttcta acattaacgt caccatgctt aacgctagca acggccttaa gattaacgcc 6540
actaataaca atgtttccgt gtctcaaggc aatctgttta tcaacgctag ctgcgtgcaa 6600
caaagcgatc caacgacagc tagcgccaca aaccccttgca ccaccgctca aaataacgct 6660
tcttctagta atgcgtcaaa caacgcgcca atcgccttaa ataataacga tgaaagcttg 6720
gtggttacgg cgaatggttt caatttttca ggcaatattt acgctaacgg ggtggttgat 6780
ttttcaaaaa ttaaaggctc tgcaaacgtt aaaaacctgt atctttacaa taacgctcaa 6840
ttccaagcca acaacctcac gatttccaac caagcggtat tagagaaaaa cgctagcttt 6900
gtaacgaata acttaaacat tcaaggagcg tttaacaaca acgccacgca aaaaatagag 6960
gtgcttcaaa atttagtgat cgcttcaaac gcttctttaa gcaccgggat ttatgggtta 7020
gaagtagggg gggcattgaa taatttggga gcgatccatt ttaatttaga aaattctcaa 7080
acgcctgtaa atccgctcat tcaagtaggg gggatcatta atctcaacac cacccaaacg 7140
ccttttatga atgtcagcgt ggctaatggc ggaacttaca ctttattaaa aagcagccgt 7200
tatattgatt acaatatcaa ccctaacagc ttgcaatcgt atttgaagct ctatacctta 7260
atcaatatca acggaaacca catagaggaa aaaaacggcg tattgactta tttgggccaa 7320
cgggttttat tacaagataa ggggttatta ttgagtgtag cactacctaa ctcaaacaac 7380
gcctctcaaa acaacatttt aagcctttct gtccttcaca accagattaa aatgtcttat 7440
ggtaataaag tgatggactt taccctccc accttacagg attacattgt gggcattcaa 7500
ggacaaagcg cactcaatca aattgaagct gttgggggga ataacgctat caagtggctt 7560
tcaacattga tgatggagac taaagaaaac ccgctttttg cgccgattta tttagaaaac 7620
cactctttaa atgaaatctt aggcgtaaca aaagatcttc aaaacaccgc aagcttgatt 7680
tctaacccta attttagaaa taacgctacc agccttttag aaatggcgag ttacacccaa 7740
caaaccagcc gtttgacaaa actctctgat tttagggcta gagagggaga gtccaatttt 7800
tcagagcgct tgttagagct taaaaacaag cgttttagcg atcctaaccc tagtgaggtt 7860
tttgtcaaat actctcaact cagcaaacac cccaataacc tttggattca aggggtggga 7920
ggagcgagct ttatttctgg gggcaatggc acgctttatg gcttgaatgt gggctatgac 7980
cgattggtta aaagcgtgat ccttgggggt tatgtggctt atggctatag cggtttttaac 8040
gggaacatca tgcattcttt ggctaataat gtggatgtgg ggatgtatgc gagggctttt 8100
ttgaaaagaa acgaattcac tttgagcgcg aatgaaactt atggaggcaa tgcgagtcat 8160
atcaattctt ctaattcctt gctctctgtg ttgaaccaac gctacaacta caacacctgg 8220
acaacgagcg tgaatgggaa ttacggctat gatttcatgt tcaaacaaaa aagcgtggtg 8280
ctaaaacctc aagtgggctt gagctatcat ttcataggct tgagcgggat gaaaggtaaa 8340
atgcaaaatc cagcttacca acaattcgtc atgcattcaa acccttctaa cgaatcggtt 8400
ttaacgctca acatggggtt agagagccgt aaatattttg gtaaaaattc ctattatttt 8460

```
gtaacggcga ggttgggtag ggatcttttg atcaaagcta aaggcgacaa tgtggtgcgt    8520
tttgtgggtg aaaacacttt attgtaccgc aagggggaaa tttttaacac ttttgcgagc    8580
gtgatcacag gaggcgaaat gcatttgtgg cgtttgatgt atgtgaatgc gggggtgggg    8640
cttaaaatgg gcttgcaata ccaagatctt aatatcactg ggaatgtggg catgcgagtg    8700
gcgttttag                                                            8709
```

<212> Type : DNA
<211> Length : 8709
SequenceName : SEQ ID 475
SequenceDescription :
Sequence
--------
<213> OrganismName : Helicobacter pylori, strain J99
<400> PreSequenceString :

```
atggcgttta aaaaggccag attgatttcc aggtttattt caaaaggatc tttcaaattg     60
aataagatct caaagaaatt tttcacattg aatcaaatct taaagcgtga aaagccctta    120
aaacgccata aaaaaacaaa atctattgaa aagcccttta ataaaaacaa atcttttta     180
aaagcttcgg ttttattgat aggagcgcta ggggggttat cccacctaag ggctaacgaa    240
tgccgttatt ggtcatggtc gtcttggagt tatcaagaca atattgaaag cggtcctaat    300
tcacccacgc acaactctta ttgtcttttt agtagcgctc aaggctctgg gacttattat    360
ttaaacactc ttaccactta tagcgctggt ggggctagtt tcacgcaaaa attcaatggt    420
ggcacgcttg atataggggg gaatatccgc tttggaggca caggtattaa tggaggtgat    480
gtagggtata tcactggaac ttataatgct caaacgatga attttaattc tagccatatc    540
acaaccggaa actcatacgc tgatggcggt gggaccacgc tcaattttaa cgcgactaac    600
aatatcacta tcaatcaagc gagctttgat aacagcgatg cagggacaca aaaatcttac    660
atgaatttta aggctctaa tatcaagatc agtggctcta gctttacaga cgacaccaat     720
ggaggtttta atttcagcgg taataacaat aatagcacca tctctttcaa tcaaaccagc    780
ttcaatcaag ggacttataa ttttagtaac agcgccactt taagcttcaa taacagcaat    840
ttcaatcaag ggacttatca ctttaacagc gcccaatcca cttttgaaaa cagcaatttc    900
aatcaaggca cttataattt taatgacaat actagcttta ataacgacac cttcaatcaa    960
ggcacttata attttaatag cagcaaggtg agtttttcag gcgctaacac tttaaattca   1020
agttcgcctt ttgctagcct taaaggcagt gtgtctttta attctggtgc gatttttaac   1080
ctcaatcaaa cccttaataa taatcaaacc tatgacattc tcactacaaa cggagcgatc   1140
cagtatgggg tttatcaaag ctatttgtgg gatctaatca actataaggg cgataaagcc   1200
attagccatg ttgaagtgag taataacact tatgatgtaa cctttgacat taacgggcaa   1260
gatgaaacct tacaagaaac ctttagcaac caatctatta ttacccaatt tttaggagac   1320
gatttacaac aacaagccca acaaacctat caagaggatg tagctaattc ccagaacgct   1380
ttgaataagg ttgctagcga caacacgatc gcaaataacg atacaagcta cactcaaagc   1440
agtaacccca ctatccttaa agacgctcaa ggtttagaaa acaccaacca acaaatccaa   1500
caagacgaaa aagccttaga aaaagattta gcccaaatca agcaattagc caactccacc   1560
acaggcttta acgaacaagc tttcactcaa gctcaaaaac aagaacaaca agatgaacaa   1620
gccttacaga acgatgaaaa cgcttttaat acggaacaag agggattaga acaagcgata   1680
gctaacgcta aacatgccaa ccccacacca aatccgacac caagccccac acccactcct   1740
ataaaacaca cagcgccaaa cactccccct agtcaagtcc cgcccacacc ccctagtcaa   1800
aatttaccta aaacaaatgt gtggaatggg gtttattggc ttcaaaacaa aacttactca   1860
aacaaaggca tttattatat tgatcccaat ctttcaggac agagcggtca aagcggcaac   1920
acgctcagca cctatacagc taatttgtta gggagaagtt ttggcgtcaa tgctaacaat   1980
ggcactttga tcatagggaa taatacagag agtgtgaatg ataacgggtt gatttggata   2040
gggcatggag gctttggcta tattacggga acttttagtg cggctaacat ttacttgacc   2100
aataatttta aaaccggtga aggcgtttca aattcagatg gtgggggagc gaacattacc   2160
tttaaagcaa gcgataatat cactatggat ggcttgaatt acaataacgc tgaaaccgtt   2220
actaaaatga ttcaaacagg ggccagtcag cattcctata ccacttttga cgctaccaat   2280
aatatcagtg taactgattc tgattttagc gatatgactt gggggaaatt cagttttagc   2340
gctaagaata tttcgttttc taacgcttcg ttcagcggct ttacaaaccc tggaggatca   2400
agcactatca gcacgaatgc ttctaattct ttaagcttta cagattctcg cttgaatggt   2460
ggagcaatct ataatttaca ggctaatagc cttattttca ataacacgca agcggttttt   2520
aatgtcttgt attctagggg gacaagcaat tttaacgcca ccacacagct tttaggcaac   2580
acgagtttta cgcttagctc tcaaagtttg ctaaacttta atggcgatac aaccttgcaa   2640
aacaacgcta atatcacgct tggcaataaa agtcaagccg cttttaaaaa ttctttaacg   2700
cttgataaca attctaattt aagcttagac aatcaaagcg ttttgaacgc gaatggcacg   2760
agtgctttta acaatcaagc gagtctcaac atttataatg ggagtcaagc ggcctttagc   2820
agtctctttt ttaatggcgg aacactcagt cttaacgcga atagcaagct caacgcttct   2880
agcgctagtt tttcaaacaa caccactatt aatttagacg atagcgtttt gaatgcgaat   2940
aacacaagct ctttaaacgc taatatcaat tttcaaggcg caagccaggc tgattttgga   3000
ggcaacacga ctattgatac agcaagcttt aattttgaca gcgcaagttc attgaatttt   3060
aataacctta cggctaatgg ggcgttaaat tttaatggtt atgcgccctc tttaactaag   3120
gctttaatga atgtcagcgg gcagtttgtt ttagggaata atggggatat taatttatct   3180
```

```
gacatcaata tctttgacaa catcacaaaa tctgtaactt acaacatctt aaacgctcaa 3240
aaagggatta ctggcattag tggggctaat ggctatgaaa aatccttttt ttatggcatg 3300
aaaatccaaa acgctaccta tagcgataat aacaacatcc aaacttggtc gtttataaac 3360
cctctcaatt cttctcaaat cattcaagag agcattaaaa atggggatct aaccatagaa 3420
gttttaaata accctaactc ggcttccaac actattttta atatcgctcc tgagctttat 3480
aattaccaag attctaagca aaatcctacc ggctatagct atgattatag cgacaatcaa 3540
gcaggcactt attacttgac aagcaacatt aaaggtcttt tcacccctaa aggctctcaa 3600
acgcctcaaa ccccaggcac ttatagccca tttaaccagc ctttgaatag tttgaatatc 3660
tacaataagg gtttttctag cgagaattta aaaacgcttt tagggatcct ttctcaaaat 3720
tccgccacct taaaagaaat gattgaatcc aaccaactag acaatatcac taacattaat 3780
gaagtgttgc aactcttaga taagattaaa atcacccaag cgcaaaagca agcgctccta 3840
gaaacgatca accatttgac tgacaacatc aatcaaacct ttaataacgg gaatctcgtt 3900
ataggcgcta cccaagataa tgttacaaac tctactagct ctatatggtt tggggggcaat 3960
ggctatagca gcccttgcgc gctagatagc gccacttgtt cttcttttag aaacacttac 4020
ttggggcaat tattaggctc aacttcccct tatttaggct acattaacgc tgattttaaa 4080
gctaaaagca tttatattac cgggacaatt ggaagtagta acgcttttga aagcggaggg 4140
agcgcggatg taacctttca aagcgctaat aacttagtgt tgaataaagc taacatagaa 4200
gctcaagcca cagacaatat ctttaatctt ttgggtcaag aagggattga taaaatcttt 4260
aatcagggga atttagcgaa tgttcttagt caaatggcta tggaaaaaat caagcaagcc 4320
ggcggtttag ggaactttat agaaaacgct ctaagcccttt tgagtaagga attacccgct 4380
agcttgcaag atgaaacctt aggccaactt ataggtcaaa ataacttaga tgatttattg 4440
aataatagtg gagtcatgaa tgaaatccaa aacattatca gtcaaaaaact aagcattttt 4500
ggcaattttg ttaccccatc catcatagaa aactaccttg ctaagcagtc tttaaaaagc 4560
atgctagacg ataaagggct tttgaatttt atcggtgggt atatagacgc ttctgaatta 4620
agctctattt taggcgtgat tttaaaggat attactaacc cccctacaag cctgcaaaaa 4680
gacattggtg tggtagcgaa cgacttgttg aacgagtttt taggacaaga tgttgtcaaa 4740
aagctagaaa gtcaaggctt ggtgagtaat atcatcaata atgttatttc tcaaggcggg 4800
ttgagcggcg tttataatca aggtttaggg agcgtgttgc cgccctcttt acaaaacgcg 4860
ctcaaagaaa acgatttagg cactctttta tcgcctagag gcttgcatga tttttggcaa 4920
aaagggtatt ttaacttttt aagcaatggc tatgtttttg tcaataacag ctcttttagt 4980
aacgctactg gggtagttt gaattttgtc gccaacaagt ctattatctt taatggcgat 5040
aatacgattg actttagcaa gtatcaaggc gcattgattt ttgcttctaa tggtgtttct 5100
aatatcaata tcaccaccct aaacgccact aatggcttaa gccttaatgc gggtttgaat 5160
aatgtgagcg ttcaaaaagg agaaatttgt atcaatttag ccaattgccc tacaaccaaa 5220
aacagctctc ctgcaaactc tagcgtaacc cccactaatg agtctttaag cgtgcacgct 5280
aataatttca ctttcttagg cacaatcatc tctaatgggg ctattgattt gtctcaagta 5340
acaaataata gcgttatagg cacgctcaat ctcaatgaaa atgcgacctt gcaagctaat 5400
aatttaacga tcaccaacgc ttttaacaac gcctctaact ctacggctaa tattgatggt 5460
aatttcacct taaaccaaca agcgacttta agcactaacg ctagtggttt gaatgtcatg 5520
gggaatttta atagctatgg cgatttggtg tttaacctca gtcattcagt tagtcatgct 5580
attatcaata ctcaaggcac agcgacgatc atggccaata ataacccttt gatccaattc 5640
aacgcttctt caaaagaagt gggtacttac acgctgattg atagcgctaa agccatttat 5700
tacgggtata acaaccaaat cacaggaggc agtagcctgg ataattacct taagctttat 5760
gcgctcattg atattaatgg caagcacatg gtgatgactg acaacggctt aacctataac 5820
gggcaagccg tgagcgttaa agatggcggt ttagttgtag gctttaagga ctctcaaaat 5880
caatacattt acacttccat tctttataat aaagtgaaaa tcgctgtttc taatgatcct 5940
atcaataacc cacaagcccc cactttaaaa caatatatcg ctcaaattca gggcgttcaa 6000
agcgtggata gcatcgatca agctgggggga aatcaagcga ttaattggct caataaaatc 6060
tttgaaacta aaggaagccc tttattcgct ccctattatc tagagagcca ctccacaaaa 6120
gatttaacca cgatcgctgg agatattgct aacactttag aagtcatcgc taaccctaat 6180
tttaaaaatg acgccactaa tattttacag atcaacacct acacgcagca aatgagtcgt 6240
ttagccaagc tctctgacac ttcaactttc gcccgttctg atttcttaga acgcttagaa 6300
gcccttaaaa acaagcgatt cgctgatgcg atccctaacg ctatggatgt gattttaaaa 6360
tactctcaaa ggaatagagt taaaaataat gtgtgggcga caggagttgg aggggctagt 6420
ttcattagtg gaggtactgg aactttatat ggtatcaatg tagggtatga taggtttatt 6480
aagggcgtga ttgtgggagg ttatgccgct tatgggtata gcgggttcca tgcaaacatc 6540
actcaatcag gctctagcaa tgtcaatgtg ggcgtttata gccgagcgtt tatcaaaaga 6600
agcgagctaa ccatgagctt gaatgagact tggggataca ataaaacttt catcaactcc 6660
tatgaccccc tactctcaat catcaatcag tcttacagat acgacacttg gacgactgac 6720
gctaaaatca attatggcta tgatttcatg tttaaagata aaagcgttat ttttaaaccc 6780
caagtaggct taagctatta ttacattggt ttgtctggtt taaggggcat tatggatgat 6840
cctatttaca accaattcag agccaatgct gaccctaata aaaaatccgt tctaacgatc 6900
aattttgccc tagaaagtcg gcattatttc aataaaaact cttattattt tgtgattgcg 6960
gatgtgggca gagacttatt cattaattct atgggggata aaatggtgcg tttcatcggt 7020
aataacaccc taagctatag agatggtggc agatacaaca cttttgctag cattatcaca 7080
ggcggggaga taagattgtt caaaaccttt tatgtgaatg cgggcatagg ggctaggttt 7140
gggcttgatt ataaagatat taatattacc ggaaatattg gtatgcgcta tgcttttttaa 7200
```

<212> Type : DNA
<211> Length : 7200

SequenceName : SEQ ID 476
SequenceDescription :
Sequence
--------
<213> OrganismName : Helicobacter pylori, strain J99
<400> PreSequenceString :

```
atggaaatac aacaaacaca ccgcaaaatc aatcgccctt tagtttctct cgttttagca    60
ggagcgttga ttagcgccat accgcaagag agtcatgccg ccttttcac gaccgtgatc    120
attccagcca ttgttggggg tatcgccaca ggcactgctg taggaacggt ctcagggctt    180
cttagttggg gactcaaaca agccgaagaa gcgaataaaa ccccagataa acccgataaa    240
gtttggcgca ttcaagcagg aaaaggcttt aatgaatttc ctaacaagga atacgactta    300
tacaaatccc ttttatccag taagattgat ggaggttggg actgggggaa cgccgctagg    360
cattattggg tcaaaggcgg gcaatggaac aagcttgaag tggatatgaa agacgctgta    420
gggacttata aactatcagg gcttagaaac tttactggtg gggatttaga cgtgaatatg    480
caaaaagcca ctttgcgttt gggccaattc aatggcaatt ctttcacaag ctataaggat    540
agcgctgatc gcaccacgag agtgaatttc aacgctaaaa atatttcaat tgataatttt    600
gtagaaatca ataatcgtgt gggttctgga gccgggagaa aagccagctc tacggttttg    660
actttgcaag cttcagaagg gatcactagc agtaaaaatg cggaaatttc tctttatgat    720
ggcgccacgc tcaatttggc ttcaaacagc gttaaattaa atggtaatgt gtggatgggc    780
cgtttgcaat acgtgggagc gtatttagcc ccttcataca gcacgatcaa cacttcaaaa    840
gttcaagggg aagtggattt taaccatctc actgtggggg atcaaaacgc cgctcaagcg    900
ggcattatcg ctagcaataa gactcatatt ggcacactgg atttgtggca aagcgccggg    960
ttaaatatca ttgcccctcc agaaggtggc tacaaggata aacctaatag taccacttct   1020
caaagtggca ctaaaaacga caagaaagag atcagtcaaa ataacaatag caacacagag   1080
gtcattaacc cacccaataa cacgcaaaaa acagaaactg aacccacgca agtcattgat   1140
gggccttttg ctggcggcaa agacacggtt gtcaatattt tccacttaaa cactaaagcc   1200
gatggcacga ttaaagtggg agggtttaaa gcttctctta ccacgaatgc ggctcatttg   1260
aatatcggca aaggcggtgt caatctgtcc aatcaagcga gcgggcgcac ccttttagtg   1320
gaaaatctaa ccgggaatat caccgttgat gggcctttaa gagtgaataa tcaagtgggt   1380
ggctatgctt tggcaggatc aagcgcgaat tttgagttta aggctggtgt ggatactaaa   1440
aacggcacag ccactttcaa taacgatatt agtttgggaa gatttgtgaa tttaaaggtg   1500
gatgctcata cagctaattt taaaggtatt gatacgggta atggtggttt caacacctta   1560
gattttagtg gtgttacaga caaagtcaat atcaacaagc tcatcacagc ttccactaat   1620
gtggccgtta aaaacttcaa cattaatgaa ttgattgtta aaaccaatgg gataagtgtg   1680
ggggaataca ctcattttag cgaagatata ggcagtcaat cgcgtatcaa taccgtgcgt   1740
ttggaaactg gcactaggtc aatcttttct gggggtgtca aatttaaaag cggtgaaaaa   1800
ctagttatca atgattttta ctatagccct tggaattatt ttgacgctag gaatgttaaa   1860
aatgttgaaa tcaccagaaa attcgcttct tcaaccccag aaaacccttg gggcacatca   1920
aagctcatgt ttaataatct aaccttgggt caaaatgcgg tcatggacta tagtcaattt   1980
tcaaatttaa ccattcaggg ggattttatc aacaatcaag gcactatcaa ctatctggtc   2040
cgaggcggga aagtggcaac cttaaatgta ggcaatgcag cagctatgat gtttaataat   2100
gatatagaca gcgcgaccgg attttacaaa ccgctcatca agattaacag cgctcaagat   2160
ctcattaaaa atacagagca tgttttattg aaagcgaaaa tcattggtta tggtaatgtt   2220
tctacaggta ccaatggcat tagtaatgtt aatctagaag agcaattcaa agagcgccta   2280
gccctttata acaataataa ccgcatggat acttgtgtgg tgcgaaatac tgatgacatt   2340
aaagcatgcg gtatggctat cggcaatcaa agcatggtga acaaccctga caattacaag   2400
tatcttatcg gtaaggcatg gagaaatata ggcatcagta aaacggctaa cggctctaaa   2460
atttcggtgt attatttagg caattctacg cctactgaga atggtggcaa taccaccaac   2520
ttacccacaa acaccactaa taatgcgcat tctgctaact acgctctcgt gaagaacgct   2580
cctttcgctc acagcgccac tcctaattta gtcgctatca atcagcatga ttttggcact   2640
attgagagcg tgtttgaatt ggctaaccgc tctaaagata ttgacacgct ctatactcat   2700
tcaggcgcgc aaggcaggga tctcttgcaa actttattga ttgatagcca tgatgcgggt   2760
tatgccagac aaatgattga taacacaagc accggtgaaa tcaccaagca attgaatgcg   2820
gccactgacg ctttaaacaa cgtagccagt ttagagcata aacaaagcgg cttacaaacc   2880
ttgagcttga gtaatgcgat gattttaaat tctcgtttag tcaatctctc taggaagcac   2940
accaaccata ttaactcgtt cgctcaacgc ttacaagctt taaaaggcca agaattcgct   3000
tctttagaga gcgcggcaga agtgttgtat caatttgccc ctaaatatga aaaacctacc   3060
aatgtttggg ctaacgctat tgggggagcg agcttgaata gcggctctaa cgcttcattg   3120
tatggcacaa gcgccggcgt agacgctttc cttaacggga atgtggaagc cattgtgggc   3180
ggttttggaa gctatggtta tagctccttt agcaatcaag cgaactctct taactctggg   3240
gccaataacg ctaattttgg cgtgtatagc cgtttttttg ccaaccagca tgaatttgac   3300
tttgaagctc aaggggcgct agggagcgat caatcaagct tgaatttcaa aagcactcta   3360
ttacaagatt tgaatcaaag ctataattac ttagcctata gcgccacagc aagagcgagt   3420
```

```
tatggttatg acttcgcgtt ttttaggaac gctttagtgt taaaaccaag cgtgggcgtg    3480
agctataacc atttaggttc aaccaacttt aaaagcaata gccaatcaca agtggcttta    3540
aaaaatggcg cgagcagtca gcatttattc aacgctaacg ctaacgtgga agcgcgttat    3600
tattatgggg acacttcata cttttatttg catgcgggag ttttacaaga gttcgctcac    3660
tttggatcga atgatgtggc gtctttaaac acctttaaaa tcaatgccgc tcgcagtcct    3720
ttaagcacct atgcaagagc gatgatgggt ggggaattgc aattggctaa agaagtgttt    3780
ttgaatttgg gcgtggttta tttgcacaat ttgatttcca acgcaagcca tttcgcttcc    3840
aatttaggaa tgaggtatag tttctaa                                        3867
```

<212> Type : DNA
<211> Length : 3867
SequenceName : SEQ ID 477
SequenceDescription :
Sequence
--------
<213> OrganismName : Helicobacter pylori, strain J99
<400> PreSequenceString :

```
atgaaaaaac acatcctttc attaacttta ggatcgcttt tagtttccac tttgagcgct      60
gaagacgacg gcttttacac aagcgtaggc tatcagatcg gtgaagccgc tcaaatggta     120
acaaacacca aaggcatcca agatctttca gatcgttatg aaagtttgaa caacctttttg    180
aatagataca gcaccctaaa caccccttatc aaattgtccg ctgacccgag cgcgattaat    240
gcggtgcggg aaaatctggg cgcgagcgcg aagaatttga tcggcgataa agccaattcc     300
ccggcgtatc aagccgtgct tttagcgatc aacgcggcgg tagggttttg gaatgtcgta     360
ggctacgtga cgcaatgcgg gggtaacgcc aatggtcaaa aaagcatctc ttcaaagacc     420
atcttcaaca acgagccagg gtatcgatcc acttccatca cttgttcttt gaacgggcat     480
tctcctggat actacggccc tatgagcatt gaaaatttca aaaagcttaa cgaagcctac     540
cagatcctcc aaacggcttt aaaacgaggc ttgcccgcgc tcaaagaaaa caacgggaaa     600
gtcaatgtaa cctatactta cacatgctca ggggacggga ataataactg ctcgtcacaa     660
gtcacaggtg taaataatca aaaagacgga accaagacta aaatccaaac catagacggc     720
aaaagcgtaa ccaccacgat cagttcaaaa gtggttgata gtcgtgcaga tggtaataca     780
acaggggtgt cctacaccga aatcaccaac aaattagaag gtgtgcctga tagcgctcaa     840
gcgctcttag cgcaagcgag tacgctcatt aacaccatca acaacgcatg cccgtatttc     900
catgctagta atagtagtga ggctaacgcc ccaaaattct ctactactac tgggaaaata     960
tgcggcgctt tttcagaaga aatcagcgcg atccaaaaga tgatcacgga cgcgcaagag    1020
ctggtcaatc aaacgagcgt cattaacgag catgaacaaa caactccggt aggcaataac    1080
aatggcaagc ctttcaaccc tttcacggac gctagttttg cgcaaggcat gctcgctaac    1140
gctagtgcac aagccaagat gctcaatcta gccgaacaag tggggcaagc cattaaccct    1200
gagaggctta gcgggacttt tcaaaatttt gttaaaggct ttttagccac atgcaacaac    1260
ccatcaaccg ctggtactgg tggcacgcaa ggttcagctc caggcacagt taccactcaa    1320
actttcgctt ccggttgcgc ctatgtagga caaacgataa caaatcttaa aaacagcatc    1380
gcccattttg gcactcaaga gcagcagata cagcaagccg aaaacatcgc tgacactctg    1440
gtgaatttca aatctagata cagcgaattg ggcaacactt ataacagcat caccactgcg    1500
ctctctaata tccctaacgc gcaaagcttg caaaatgcgg tgagtaaaaa gaataacccc    1560
tatagcccgc aaggcataga caccaattac tacctcaatc aaaactctta caaccaaatc    1620
caaaccatca accaagaact cgggcgtaac ccctttagga aagtggggat tgttagttct    1680
caaaccaata atggcgcgat gaatgggatc ggtattcagg tgggttacaa acaattcttt    1740
ggccaaaaaa gaaaatgggg cgctaggtat tacggctttt ttgattacaa ccatgcgttc    1800
attaaatcca gcttcttcaa ctcggcttct gacgtgtgga cttatggctt tggagcggac    1860
gctctttata atttcatcaa cgataaagcc accaatttct taggcaaaaa caacaagctt    1920
tctgtggggc tttttggggg tattgcatta gccgggactt catggcttaa ttctgagtat    1980
gtgaatttag ccaccatgaa taacgtctat aacgctaaaa tgaacgtggc gaatttccaa    2040
ttcttattca acatgggagt gaggatgaat ttagccaggc ctaagaaaaa agacagcgat    2100
catgcggctc agcatgggat tgagttaggg cttaaaatcc ccaccatcaa cacgaactac    2160
tactccttta tgggggctga actcaaatac cgaaggctct atagcgtgta tttgaattac    2220
gtgttcgctt attaa                                                     2235
```

<212> Type : DNA
<211> Length : 2235
SequenceName : SEQ ID 478
SequenceDescription :
Sequence
--------
<213> OrganismName : Helicobacter pylori, strain J99
<400> PreSequenceString :

```
atgataaaaa aagctaaaaa attcatacca ttctttttaa ttggctccct cttagctgaa      60
gacaatggct ggtatatgtc tgtaggctat caaatcggtg gcacgcagca attcatcaat     120
aacaaacaac ttttagaaaa tcaaaatatc atcaatagca tcactcaaag cgcgatcaac     180

attgcagggc ctactaccgg ccttatcact ttaagctctc aaaccgtcat tgacgcttta     240
ggctatggcg tgagtaacac tgttggcaac caattagagg gcatttctaa catcttgaat     300
caaattggca aaagaaaaga ctttattct agccgtcaaa tctctagcat ttcccagcaa      360
atcatagggc ttaaaggaag ctctgatccc ttaaaagccc attcttcaca aatcacagcc     420
aaactccttt ccaacaccca aagcgcgttt gatcagggca tcgctctaag ctctaatatc     480
attagtgcag tcaatagcct aaaccctagc aacaactccc aagaagtcaa agcccagctc     540
caaaacaccg cgcaatccat ggcggaatta ttgcaacaaa ttgaacacag catcactaaa     600
accactagca ccacttacgc acaatcctta ctctccaatc tgaccgatgc ggtgaatgca     660
tctagcaata ataccactta tgtgagcgct cttgttaacg ctttaaacac tttaggggtg     720
ggggttttcc ccaccacaac ctcaacgcat gtggtgctaa acccaccggg acaagtcgta     780
ttctatccaa ctaattccct tttaggctct acttcttcaa acagcaataa ccaacaacaa     840
tacaacaaca ccctttttaat gaacacctta caaggggaat taagcactaa caatcaaaat    900
aaccccaatg gttgcgccaa tcaaatccag tgtttagagc aattcatcca aaatttaacc     960
cctttagccg caaccccac ttcaactaac caggccaacc agcaagtcca agccatcgct     1020
caaaaacttc aaagcgttgc tatcaacgct ttagacaaca atgcgatcaa caacaccacc    1080
tataatttaa acaacttgca caacgctttg aatttccaag cctatcaaag cacgatagaa    1140
caatacaata acgctttaaa gcaaatttcg tggattagtt ttagcgagcc taaaaacttg    1200
ctcaaaaaca cttccaataa ctaccaaatc ggcacggtta ccaacgatca agggcaaaat    1260
atcagcgcct atgattgcac aagcgctacc ggaagccttt ctagcgatgc ttctagtggg    1320
atttcatgct cagccacaag ctccacaaat aacacaaata gttttgacaa ttctttagtc    1380
gctacctcca aagtccaaac catcaacggc aaagagcaga tcggcgtgaa ttcttttaat    1440
cttgtctctc aagtgtggag cgtttataac tctttaaaaa cttcagaaga aaatttgcaa    1500
aaaaacgcca aaatattatg caacaatgga tcgcaatctg ggacaagccc atgcaatagc    1560
tcttcagggg gtttgagcat cagcgggaac gcccaattgc aaaatatttt aagccctact    1620
aatgggacta ccactaatac tcaagctaaa agcaacgctt ccaaactaaa agcgatggta    1680
atggtgaata atgaagaaga agccaaaacg accaatttca atcaaagcag tgggccaacc    1740
acacaatctt ctaacagcac ggtgatggga gctttaaaca ccgtattgca aaatgtcagc    1800
aatttccaac aaagcattca aagcgctttt caaaaccaag aaaataatat ccaagcttgg    1860
gcgaacgcac tttataacac tagtaaccct aatgggaatc aatcgcaaaa tttaacccact   1920
aacaataacc aagatttacg catccaatta agggcgaatt tttaccagct catcaatacc    1980
attaaccagc aagtgcctac agacatgaac gctttaatta atcaaagcca acaaacccag    2040
caaacaagcg gatcagcaag caccacgaac aacgcatgcg cgagcggaat ggggagtagt    2100
ggcaactggt gctaccagca gtggtccgat tctaaggctt attacagcgg gttgcaaagc    2160
gctttagggt atcaaacaca agcgacaact caaaatggga gcagtggtgg gagcaatatc    2220
acctacaatg tccaacaaat cacgctcact agcggtggtt tgctcaatca aattatcaca    2280
aaccttaaga gcgttaatgg gggcagtaat gggggaagca gtgggaatgg cactagtcaa    2340
atcaacacag cctaccaaat gctcacagac gctagcgatg ggaaattagg gacttataat    2400
agtagcaata gtagcaatag tagcaatagt ggcaataata acggctatac gccatgcaat    2460
agcaccaacg ggagcaatgg gacgagtggg agcaattgtt atgaacccaa caaacaacaa    2520
aacgccacca ccgcaaccac cacgaccgac agcaatttac aaaaagtcta taatgacgcc    2580
caaaaaatag ccaatattat cgccagctct gggaacaata aaggcgttga aaacggctta    2640
aaacaattct ttgaagcgtt aaaaagtaat agcagcagtc ttagtaattt atgtggtaat    2700
ggtagtagcg gtagtagctc tacttgctcc ggtgggctta tcaacctttt aggggcaatc    2760
cccacaaacg gagtgagcga tacgaataat ttaattaatc tgctcactga attcattaaa    2820
accgccgggt ttatccaaaa taaggatagt aatgtatcta ctagtcttac aagcgctttt    2880
caagccatta cgagcgctat ttctcaaggg tttcaagcct tgcaaaacga tattagccct    2940
aatgcgattt tgaccttgct ccaagaaatc acttctaaca ccaccaccat tcagtcattc    3000
tcgcaaacct tacggcagct tttaggggat aaaaccttct ttatggtgca acaaaagctc    3060
attgatgcga tgattaacgc cagaaatcag gttcaaaacg cgcaaaatca agccaataac    3120
tacggctctc aacccgtttt aagccagtat gcggccgcta aagcacccca acacggcatg    3180
agcaatggct taggggttgg cataggctat aaatacttct ttggtaaggc taggaaatta    3240
ggccttaggc attatttttt ctttgattac ggctttagtg aaataggcct agccaatcaa    3300
agcgtgaaag cgaatatctt tgcttatggg gtaggcacgg attttttatg gaatctattc    3360
aggaggactt acaacactaa agcgttgaat tttgggctat ttgccggggt ccaactgggc    3420
ggtgcaactt ggcttagttc cttaaggcaa caaatcattg acaactgggg gaacgctaat    3480
gacatccatt caacgaattt tcaagtggcg ctgaattttg ggtgcgcac caatttcgcg     3540
gagtttaagc gttttgctaa gaaattccac aatcaagggg tcatcagcca aaagagcgtg    3600
gaatttggga tcaaggtgcc tctcatcaat caagcgtatt tgaatagtgc tggggctgat    3660
gtgagctaca ggaggcttta tactttctat atcaattaca tcatggggtt ttaa          3714
```

<212> Type : DNA
<211> Length : 3714
SequenceName : SEQ ID 479
SequenceDescription :

Sequence
--------
<213> OrganismName : Helicobacter pylori, strain J99
<400> PreSequenceString :

```
atgaaacaaa atttaaagcc attcaaaatg attaaggaaa atttaatgac acaatctcaa      60
aaagtaagat tcttagcccc tttgagccta gcgttaagct tgagcttcaa tccagtgggc     120
gctgaagaag atgggggctt tatgaccttt gggtatgaat taggtcaggt ggtccagcaa     180
gtgaaaaacc cgggtaaaat caaagccgaa gaattagcgg gcctgttaaa ctctaccacg     240
acaaacaaca ccaatatcaa tattgcaggc acaggaggga atgtcgccgg gactttgggc     300
aacctttta tgaaccaatt gggcaatttg attgatttgt atcctacttt gaaaactaat      360
aatcttcacc aatgcggtag cactaatagc ggtaatggcg ctactgctgc cgctgctact     420
aacaatagcc cttgtttcca aggtaacctg gctctttata acgaaatggt tgactctatc     480
aaaactttga gtcaaaacat cagcaagaac atctttcaag gcgacaacaa caccacgagc     540
gctaatctct ccaaccagct cagtgagttg aacaccgcta gcgtttattt gacttacatg     600
aactcgttct taaacgccaa caaccaagcg ggtgggattt ttcaaaacaa caccaatcaa     660
gcttacgaga atggtgttac cgctcaacaa atcgcttatg tcctaaagca agcttcaatc     720
actatggggc caagcggtga tagtggggct gcgggagcgt ttttagacgc cgctttagcc     780
caacatgttt tcaactcggc taacgctggg aacgatttga gcgctaagga attcactagc     840
ttggtgcaaa acatcgtcaa taattctcaa aacgctttaa cgctagccaa caacgctaac     900
atcagcaatt caacaggcta tcaagtgagc tatggtggga atattgatca agcgcgctct     960
acccaactgt taaacaacac cacaaacact ttggctaaag ttaccgctct aaacaacgag    1020
cttaaagcta acccatggct tgggaatttc gctgctggta acagctctca agtgaatgcg    1080
tttaacgggt ttatcactaa aatcggttat aagcaattct tcggggaaaa caagaatgtg    1140
ggcttacgct actacgggtt cttcagctat aacggcgcgg gcgtgggtaa tggccccact    1200
tacaatcaag tcaatctgct cacttatggg gtggggactg atgtgcttta caatgtgttt    1260
agccgctctt ttggcagtag gagtcttaat gcgggcttct ttgggggggat ccaactcgca   1320
ggggacactt acatcagcac gctaagaaac agccctcagc ttgcgagcag acctacagcg    1380
acaaaattcc aattcttgtt tgatgtgggc ttacgcatga actttggtat cttgaaaaaa    1440
gacctaaaaa gccataacca gcattctata gaaatcggtg tgcaaatccc tacgatttac    1500
aacacttact ataaagctgg tggcgctgaa gtgaaatact tccgccctta tagcgtgtat    1560
tgggtctatg gctacgcctt ctaa                                           1584
```

<212> Type : DNA
<211> Length : 1584
SequenceName : SEQ ID 480
SequenceDescription :
Sequence
--------
<213> OrganismName : Helicobacter pylori, strain J99
<400> PreSequenceString :

```
atgaaaaaaa cccttttact ctctctctct ctctcgtttg ggctccacgc tgaagacgac      60
ggcttttacg caagcgcggg aattcggatc ggtgaagccg ctcaaatggt gaaaaacacc     120
aaaggcattc aacagctttc agagaattat gaaaagttga acaatctttt aaataattac     180
aacaccctaa acactcttgt aaagctgtcc tccgatccga gtgctgtcaa cgacgcaagg     240
gataatctag gctcaagcac taggaatttg ctagatgtca aagccaattc ccccgcctat     300
caagcggtgc ttttagcatt gaacgctgca gtgggcttgt ggcaagttac aagctatgcc     360
tttaccgctt gtggtcctgg tagcaatgag aacgcgaatg gaggtatcca aacctttaat     420
aatgtgccag gacaaaacac gacgaccatc acttgtaatt cgtattatga gccaggacat     480
ggcgggccaa tatccactaa aaattatgcg atcatcaaca aggcttatca aatcattcaa     540
aaggctttga cagccaatgg agaagggatc ccagttttaa gcaacaccac tacaaaactt     600
gatttcacta tcaatggaga caaaagaacg ggtggcgaac caaataaaaa attagtatac     660
ccatggagtc atgggaaagc tatttcaacc tcgtggaatg caaccataac agcaccaaca     720
acagaaaata tcaatacaac caatagcgct caagagcttt taaaacaagc gagcatcatt     780
atcactaccc tgaatagtgc atgcccaaac ttccaaaatg gtggtagcgg ttattgggca     840
gggataagtg gcaatgggac aatgtgtggg atgtttaaga atgaaatcag cgctatccaa     900
ggcatgatcg ctaacgcgca agaagctgtc gcgcaagcca aaatcgttag tgaaaacacg     960
caaaatcaaa acagcctaga cgctggaaaa ccattcaacc cctacacaga cgctagtttt    1020
gctgaaagca tgctcaaaaa cgcgcaagcc caagcggaga ttttaaacca agccgaacaa    1080
gtggtgaaaa actttgaaaa aatccctaca gcctttgtaa atgactcttt aggggtgtgt    1140
tatgaagtgc aaggaggtga gcgtcgtggc accaatccgg gtcagacgac ttctaacact    1200
tgggggggcag gctgtgcgta tgtaggacaa acgataacaa atcttaaaaa cagcatcgcc    1260
cattttggca ctcaagagca gcagatacag caagccgaaa acatcgctga cactctggtg    1320
aatttcaaat ctagatacag cgaattgggc aacacttata acagcatcac cactgcgctc    1380
tctaatatcc ctaacgcgca aagcttgcaa aatgcggtga gtaaaaagaa taacccctat    1440
agcccgcaag gcatagacac caattactac ctcaatcaaa actcttacaa ccaaatccaa    1500
accatcaacc aagaactcgg gcgtaacccc tttaggaaag tggggattgt tagttctcaa    1560
accaataatg gcgcgatgaa tgggatcggt attcaggtgg gttacaaaca attctttggc    1620
caaaaaagaa aatggggcgc taggtattac ggcttttttg attacaacca tgcgttcatt    1680
aaatccagct tcttcaactc ggcttctgac gtgtggactt atggctttgg agcggacgct    1740

ctttataatt tcatcaacga taaagccacc aatttcttag gcaaaaacaa caagcttttct   1800
gtggggcttt ttgggggtat tgcattagcc gggacttcat ggcttaattc tgagtatgtg    1860
aatttagcca ccatgaataa cgtctataac gctaaaatga acgtggcgaa tttccaattc    1920
ttattcaaca tgggagtgag gatgaattta gccaggccta agaaaaaaga cagcgatcat    1980
gcggctcagc atgggattga gttagggctt aaaatcccca ccatcaacac gaactactac    2040
tcctttatgg gggctgaact caaataccga aggctctata gcgtgtattt gaattacgtg    2100
ttcgcttact aa                                                         2112
```

<212> Type : DNA

<211> Length : 2112

SequenceName : SEQ ID 481

SequenceDescription :

Sequence

--------

<213> OrganismName : Helicobacter pylori, strain J99

<400> PreSequenceString :

```
atgataaaga agaatagaac gctgtttctt agtctagccc tttgcgctag cataagttat     60
gccgaagatg atggagggtt tttcaccgtc ggttatcagc ttgggcaggt catgcaagat    120
gtccaaaacc caggcggcgc taaaagcgac gaactcgcca gagagcttaa cgctgatgta    180
acgaacaaca ttttaaacaa caacaccgga ggcaatgtcg caggggcgtt gagtaacgct    240
ttctcccaat acctttattc gcttttaggg gcgtatccca cgaaactcaa tggtaacgac    300
gtgtctgcga acgctctttt aagtggtgcg gtaggcagtg ggacttgcgc ggctgcaggg    360
acggctggtg gcacaactct taacactcaa agcgcttgca ccgctgcggg ctattactgg    420
ctccctagct tgactgatag gattttaagc acgatcggca gccagactaa ctacggcacg    480
aacaccaatt tccccaacat gcaacaacag ctcacctact tgaatgcggg gaatgtgttt    540
tttaatgcga tgaataaggc tttagagaag aatgggactg ctactgctaa tagcactagt    600
agcactagcg gtgcgactgg ttcagatggt caaacttact ctcaacaagc tattcaatac    660
cttcaaggcc aacaaaatat cttaaataac gcagcgaact tgctcaagca agatgaattg    720
ctcctagaag ctttcaactc tgccgtagct gctaacattg ggaataagga attcaattca    780
gccgctttta caggtttggt gcaaggcatt attgatcaat ctcaattggt ttataacgag    840
ctcactaaaa acaccattag cgggagcgcg gttaataacg ctgggataaa ctccaaccaa    900
gctaacgctg tgcaagggcg tgctagtcag ctccctaacg ctctttataa cgtgcaagta    960
actttggata aaatcaacgc gctcaacaat caggtgagaa gcatgcctta cttgccccaa   1020
ttcagagccg ggaacagccg tgcaacgaat attttaaacg ggttttacac taaagtgggc   1080
tataagcaat tcttcgggaa gaaaaggaat atcggtttgc gctattatgg tttcttttct   1140
tataacggag cgagcgtggg ctttagatcc actcaaaata atgtagggtt atacacttat   1200
ggggtgggga ctgatgtgtt gtataacatc tttagccgct cctatcaaaa ccgctctgtg   1260
gatatgggct tttttagcgg tatccaatta gccggtgaga ccttccaatc cacgctcaga   1320
gatgacccca atgtgaaatt gcatgggaaa atcaataaca cgcacttcca gttcctcttt   1380
gacttcggta tgagaatgaa cttcggtaag ttggacggga aatccaaccg ccacaaccag   1440
cacacggtgg aatttggcgt agtagtgcct acgatttata acacttatta caaatcagca   1500
gggactaccg tgaagtattt ccgtccttat agcgtttatt ggtcttatgg gtattcattc   1560
taa                                                                 1563
```

<212> Type : DNA
<211> Length : 1563
SequenceName : SEQ ID 482
SequenceDescription :
Sequence
--------
<213> OrganismName : Helicobacter pylori, strain J99
<400> PreSequenceString :

```
atgaagaaaa aatttctgtc attaacctta ggttcgcttt tagtttccgc tttaagcgct     60
gaagacaacg gctttttgt gagcgccggc tatcaaatcg gtgaatccgc tcaaatggtg    120
aaaaacacca aaggcattca agatctttca gacagctatg aaagattgaa caacctttta    180
acgaattata gcgtcctaaa cgctctcatc aggcagtccg ccgaccccaa cgccatcaat    240
aacgcaaggg gcaatttgaa cgcgagcgcg aagaatttga tcaatgataa aaagaattcc    300
ccggcgtatc aagccgtgct tttagccttg aatgcggcag cggggttgtg gcaagtcatg    360
agctatgcga tcagcccttg tggtcccggt aaagacacaa gcaaaaatgg gggcgttcaa    420
actttccaca acacgccttc aaatcaatgg ggaggcacta ccattacttg tggcactact    480
ggttatgaac caggaccata cagcatttta tccactgaaa attacgcgaa aatcaataaa    540
gcttatcaaa tcatccaaaa ggcttttggg agcagcggaa aagatattcc tgccttaagc    600
gacaccaaca cagaactcaa attcacaatc aataaaaata atggaaacac gaatacgaat    660
aataatggag aagaaattgt tacaaaaaat aacgctcaag ttcttttaga acaggctagc    720
accattataa ctacccttaa tagcgcatgc ccatggatca acaatggtgg tgcaggtggt    780
gcgagtagtg gtagtttatg ggaaggaata tatttgaaag gcgatgggag cgcttgcggg    840
atttttaaaa atgaaatcag cgcgattcaa gacatgatca aaaacgctgc aatagccgta    900
```

```
gagcaatcca agatcgttgc tgcaaacgcg caaaaccagc gcaacctaga caccgggaag     960
acattcaacc cctataaaga cgccaacttc gcccaaagca tgttcgctaa cgccaaagcg    1020
caagcggaga ttttaaaccg cgcccaagca gtggtgaaag actttgaaag aatccctgca    1080
gagttcgtaa aagactcttt aggggtgtgc catgaagtgc aaaacggcca tctccgtggc    1140
acgccatccg gcacggtaac tgataacact tggggagccg gttgcgcgta tgtgggagag    1200
accgtaacga atctaaaaga cagcatcgct cattttggcg accaagccga gcgaatccat    1260
aacgcgcgca acctcgccta cactttagcg aacttcagca gtcagtatca aaaactaggc    1320
gaacactatg acagcatcac agcggccatt tcaagcttgc ctgatgcgca atctttacaa    1380
aatgtggtga gcaaaaagac taaccctaat agcccacaag gcatacagga taactactat    1440
attgactcca atatccattc tcaagtgcaa tctaggagtc aagaactcgg cagtaaccct    1500
ttcaggcgtg ctggcttaat cgccgcttct accaccaata acggcgcgat gaacgggata    1560
ggctttcaag tgggctataa gcaattcttt gggaaaaaca aacgatgggg cgcaaggtat    1620
tacggctttg tggattacaa ccacacctat aacaaatccc aatttttcaa cgcctcttct    1680
gatgtctgga cctatggcgt ggggagcgat ttgttagtga atttcatcaa cgataaagcc    1740
actaaacaca ataagatttc ttttggcgcg tttggcggta tcgccttagc cgggacttca    1800
tggcttaatt ctcagtatgt gaatttagcg aacgtgaata attattataa ggccaaaatc    1860
aacacggcga atttccaatt cttattcaat ctgggtttga gaatgaacct cgctaggaaa    1920
aagcatagag cgaccgataa cgcggcccaa catggcattg aactaggcac aaagatcccc    1980
acgatcaaca cgaattacta ttctttgcta ggcactacct tgcaatacag aaggctttat    2040
agcgtgtatc tcaactatgt gttcgcttac taa                                 2073
```

<212> Type : DNA
<211> Length : 2073
SequenceName : SEQ ID 483
SequenceDescription :
Sequence
--------
<213> OrganismName : Helicobacter pylori, strain J99
<400> PreSequenceString :

```
atgaaaatca aaaaatccct ctttgctctc tctttctctc tcatggcttc attatcaagg      60
gctgaagatg acggatttta catgagtgtg ggctatcaaa tcggtgaagc ggtccaaaaa     120
gtgaaaaaca ctggagcatt acaaaatctt gcagacagat acgataactt gagcaacctt     180
ttaaaccaat acaattactt aaattcctta gtcaatctag ccagcacgcc tagcgcgatt     240
accggtgcga ttgacaatct aagctcaagc gcgatcaatc tcactagcgc taccaccact     300
tctccggcct atcaagctgt ggctttagcg ctcaatgcgg ctgtgggcat gtggcaagtc     360
atagcctttg gcatcagctg tggccctggc cccaatcttg gcccagaaca tttagaaaat     420
gggggcgttc gatcgtttga caacacgcca aactacagct acaacaccgg tagcggaacg     480
accaccacca cttgtaatgg agccagtaat gtagggccca atggtatcct atctagcagc     540
gaataccagg ttctcaatac cgcttatcaa actatccaaa ccgctttaaa ccaaaaccaa     600
ggaggcggga tgcctgcctt gaatagctcc aaaaatatgg tagtcaatat caatcaaact     660
ttcacaaaaa accctaccac agaatacact taccccgatg ggaatggcaa ttattattca     720
ggcggttcat caatcccaat ccagctaaag attagtagcg tcaatgacgc tgaaaacctt     780
ttgcaacaag ccgctactat catcaatgtc cttaccaccc aaaacccgca tgtgaatggt     840
ggcggtgggg catgggggtt tggcggtaag accgggaatg tgatggatat ttttggcgat     900
agttttaacg ctattaacga aatgatcaaa aacgctcaag ccgttttaga aaaaacccaa     960
cagcttaacg ctaatgaaaa cacccaaatc acgcaaccag acaatttcaa cccctacact    1020
tctaaagaca cgcagttcgc tcaagaaatg ctcaatagag ctaacgctca agcagagatt    1080
ttgagcttag cccaacaagt agcggacaat ttccacagca ttcaagggcc tatccaacaa    1140
gatctagaag aatgcaccgc aggatcagct ggtgtgatta acgacaacac ttatggttca    1200
ggttgcgcgt ttgtgaaaga gactctcaat tccttagagc aacacaccgc ttattatggc    1260
aaccaggtca atcaggatag ggctttgtct caaaccattt tgaattttaa agaagccctt    1320
agcactttag ggaacgactc aaaagcgatc aatagcggta tctctaactt gcctaacgct    1380
aagtcccttc aaaacatgac gcatgccact caaaacccta attccccaga aggtttgctc    1440
acttattctt tggataccag caaatacaac cagctccaaa ctgttgcgca agaattaggc    1500
aaaaacccct ttaggcgcat cggcgtgatt aactatcaaa acaataacgg ggcgatgaac    1560
ggcatcggcg tgcaagcggg ctataagcaa ttctttggca aaaaaaggaa ttggggggtta    1620
aggtattatg gtttctttga ttataaccat gcttatatca aatctaattt ttttaactcg    1680
gcttctgatg tgtggactta tggggtgggt atggacgcgc tttataactt catcaacgat    1740
aaaaacacca actttttagg caaaaataac aagctttctg tggggctttt tggtggcttt    1800
gcgttagccg ggacttcgtg gcttaattcc caacaagtga atttgaccat gatgaatggc    1860
atttataacg ctaatgtcag cgcttctaac ttccaatttt tgtttgattt aggcttgaga    1920
atgaacctcg ctaggcccaa gaaaaaagac agcgatcatg ccgctcagca tggcatggaa    1980
ttgggcgtga aaatccccac cattaacacg gattattatt ctttcatggg ggctgaactc    2040
aaatacagaa ggctctatag cgtgtatctc aattatgtgt ttgcttacta a             2091
```

<212> Type : DNA

<211> Length : 2091
SequenceName : SEQ ID 484
SequenceDescription :
Sequence
--------
<213> OrganismName : Helicobacter pylori, strain J99
<400> PreSequenceString :

```
atgaaaatca aaaaatccct ctttgctctc tctttctctc tcatggcttc attatcaagg      60
gctgaagatg acggatttta catgagtgtg ggctatcaaa tcggtgaagc ggtccaaaaa     120
gtgaaaaaca ctggagcatt acaaaatctt gcagacagat acgataactt gagcaacctt     180
ttaaaccaat acaattactt aaattcctta gtcaatctag ccagcacgcc tagcgcgatt     240
accggtgcga ttgacaatct aagctcaagc gcgatcaatc tcactagcgc taccaccact     300
tctccggcct atcaagctgt ggctttagcg ctcaatgcgg ctgtgggcat gtggcaagtc     360
atagcctttg gcatcagctg tggccctggc cccaatcttg gcccagaaca tttagaaaat     420
gggggcgttc gatcgtttga caacacgcca aactacagct acaacaccgg tagcggaacg     480
accaccacca cttgtaatgg agccagtaat gtagggccca atggtatcct atctagcagc     540
gaataccagg ttctcaatac cgcttatcaa actatccaaa ccgctttaaa ccaaaaccaa     600
ggaggcggga tgcctgcctt gaatagctcc aaaaatatgg tagtcaatat caatcaaact     660
ttcacaaaaa accctaccac agaatacact taccccgatg ggaatggcaa ttattattca     720
ggcggttcat caatcccaat ccagctaaag attagtagcg tcaatgacgc tgaaaacctt     780
ttgcaacaag ccgctactat catcaatgtc cttaccaccc aaaacccgca tgtgaatggt     840
ggcggtgggg catgggggtt tggcggtaag accgggaatg tgatggatat tttggcgat      900
agttttaacg ctattaacga aatgatcaaa aacgctcaag ccgttttaga aaaaacccaa     960
cagcttaacg ctaatgaaaa cacccaaatc acgcaaccag acaatttcaa cccctacact    1020
tctaaagaca cgcagttcgc tcaagaaatg ctcaatagag ctaacgctca agcagagatt    1080
ttgagcttag cccaacaagt agcggacaat ttccacagca ttcaagggcc tatccaacaa    1140
gatctagaag aatgcaccgc aggatcagct ggtgtgatta acgacaacac ttatggttca    1200
ggttgcgcgt ttgtgaaaga gactctcaat tccttagagc aacacaccgc ttattatggc    1260
aaccaggtca atcaggatag ggctttgtct caaaccattt tgaattttaa agaagccctt    1320
agcactttag ggaacgactc aaaagcgatc aatagcggta tctctaactt gcctaacgct    1380
aagtcccttc aaaacatgac gcatgccact caaaacccta attccccaga aggtttgctc    1440
acttattctt tggataccag caaatacaac cagctccaaa ctgttgcgca agaattaggc    1500
aaaaacccct ttaggcgcat cggcgtgatt aactatcaaa acaataacgg ggcgatgaac    1560
ggcatcggcg tgcaagcggg ctataagcaa ttctttggca aaaaaaggaa ttgggggtta    1620
aggtattatg gtttctttga ttataaccat gcttatatca aatctaattt ttttaactcg    1680
gcttctgatg tgtggactta tggggtgggt atggacgcgc tttataactt catcaacgat    1740
aaaaacacca actttttagg caaaaataac aagctttctg tggggctttt tggtggcttt    1800
gcgttagccg ggacttcgtg gcttaattcc caacaagtga atttgaccat gatgaatggc    1860
atttataacg ctaatgtcag cgcttctaac ttccaatttt tgtttgattt aggcttgaga    1920
atgaacctcg ctaggcccaa gaaaaaagac agcgatcatg ccgctcagca tggcatggaa    1980
ttgggcgtga aaatccccac cattaacacg gattattatt ctttcatggg ggctgaactc    2040
aaatacagaa ggctctatag cgtgtatctc aattatgtgt ttgcttacta g             2091
```

<212> Type : DNA
<211> Length : 2091
SequenceName : SEQ ID 485
SequenceDescription :
Sequence
--------
<213> OrganismName : Helicobacter pylori, strain J99
<400> PreSequenceString :

ttgcataaaa aagttctgtt ggctttaact gccagcttga tttgccaaga gtctttgttc 60
gctaaggata aagactacac tttgggcaag gtttctactg ccggtaaaaa ggatagatct 120
gactattctg ggcaggtcaa tttgggttat agcgggatta ccgcgcctaa gagttggcaa 180
gatgaagaag tgaaaaaata cacaggaagc cgcacggtga tctctaacaa agcgctcacc 240
caacaagcta accaaagcat tgaagaagct ttacagaatg tccccggtct gcaaattagg 300
aatgccacag gtgtgggggc tatgcctact atccaaatcc gtggctttgg agcggggggt 360
tcagggcata gcgatgcgac gctcatgtta gttaatggta ttcctgttta tatggcccct 420
tacgctcaca ttgagctaga cattttccct gttacctttc aagccattga tcgcattgat 480
gtgatcaaag gtggaggcag cgtgcaatat gggcctaaca cttatggggg tattgtcaat 540
atcatcacta aacctatccc taatcaatgg gaaaaccaag cggctgaaag gatcacttat 600
tgggctaagg ctagaaacgc tgggtttgcc gctccccctg ataaaaccgg cgatccttct 660
ttcatcaagt ctttaggcaa caacctcctc tataacactt atgtgaggag cggagggatg 720
atcaataagc atgtgggtat ccaagcgcaa gctaactggg ttagaggcca aggctttagg 780
gacaatagcc cctctagtat ttcaaactat tggctggatg gggtctatga catcaatgaa 840
agcaatggga ttaaagccta ttaccaatac tacgattttg ctatcgccca accgggatca 900

ctcagcgagc aagattacaa aataaaccgc ttcgctaatt tgcgcccctt aaaccaaaaa 960
ggcgggcgct cacaacgctt tggggctgtg tatgaaaacc gcttcgggga tttagacaga 1020
gtgggcggga ctttcagctt cacttactac gggcagttga tgactaggga ctttcaggtg 1080
agctctagct acaatagcgc taacatggtt acttgtttta gcgaagcggc atgcagggcg 1140
gcagggcttc cggcagggta taacttggct gtgccttatt atgccactaa ctacaatggt 1200
tgggcggagg tagaaaaccc tgtgcgttcc attaacaacg cttttgagcc taaagtgaat 1260
ctgatcgtca ataccgggaa agtcaggcaa acctttatca tgggtttgcg tttcatgacc 1320
accacttttt tacaacgcca atacttaaac accaatgaat gcgccactaa aacgagcggt 1380
gagggggcag gcttcttgtg tgagggccct aacgtgatga gcggttggaa accccacatc 1440
aagcatggcg tttatagaaa ctggaataac tggcgcaaca attacacagc ggtctatttg 1500
agcgatcgca ttgaagcttg ggacgggcgc tttttcatcg tgcctggttt gcgctacgct 1560
tttgtgcaat acaacaacga aaatgcgtct aactggatgc aaatccctga gaaggattta 1620
agaaaaatca agcacatgaa caattggatg ccctcaacca acattggctt tatccctgtg 1680
caaggcgatc acaatgtgct tacctacttc aactaccaac gctctttcgt cccgcctcaa 1740
ttagacgttt tgagctatgg aggagcggag tattttaccc aacactttga cacggtggaa 1800
gcaggagcgc gctacaccta taaagataaa ttcagcttca atgcggacta ctttaggatt 1860
tgggcgcgcg attttgccac cgggcagtat tcagtctata cgagcgggcc catgaaaggt 1920
aatgtgcgcc ccattaatgg ctattctcaa ggcgtggagc tggaattgta ttacaggccc 1980
attagagggt tgcaattcca tgccgctttc aactacattg acactcgtgt aactagccat 2040
ggccctttaa ccgacttgaa cggggatgtg ctaaaaggga ctagctataa caagcatttc 2100
ccttttgtaa gccctttcca attcattttt gacgctcgct acaattggcg taaaaccacc 2160
attggtattt ctagctattt ttatagccgt gcttatagcg ggattagcaa cagcgcagca 2220
ggaggctatt atgggatgca atactatagt ggggggaaca actatgaaag cgttcttaat 2280
agcggttatc aatgcgaagc ttggtgtatg acccaacatg aagggctctt gccttggtat 2340
tgggtgtgga atatccaagt gagccaaatt ttctgggaaa acggaagaca cagagttaca 2400
ggaagcttac aaatcaataa catcttcaac atgaagtatt attttacagg gattggctct 2460
agccctgcag gcttgcaacc tgcgcctgga agatcggtta cagcgtattt gaactacact 2520
ttctaa 2526

<212> Type : DNA
<211> Length : 2526
SequenceName : SEQ ID 486
SequenceDescription :
Sequence.
--------
<213> OrganismName : Helicobacter pylori, strain J99
<400> PreSequenceString :

```
atgaaaaaaa cccttttact ctctctctcc gcttcatcgc ttttaaacgc tgaagacaac     60
ggcttttta tcagcgcggg ctatcaaatc ggtgaagccg ctcaaatggt gaaaaacacc     120
ggcgaattga aaaaactttc agacacttat gagaatttga gcaacctttt aaccaatttt    180
aacaacctca atcaggcggt aacgaacgcg agcagccctt cagaaatcaa tgctgcgatc    240
gataatttaa aagcaaacac gcaagggcta attggcgaaa aaacccaattc cccggcgtat   300
caagcggtgt atttggcgct caatgcggcg gtagggctgt ggaatgtcat cgcctataat    360
gtccaatgcg gtcctggtaa cagtggacaa caaagcgtaa cctttgaggg ccaaccagga    420
cataattcaa gttccattaa ttgcaattta accggttata acaacggggt tagcggccct    480
ttatccattg agaattttaa aaagcttaat caggcttatc aaactatcca acaagcttta    540
aaacaagata gcggatttcc tgttttggat agtgcaggaa aacaagtaac tataacaata    600
acaacgcaaa ctaatggagc taataaaagt gaaactacta ctactactac tactactaat    660
gacgctcaaa cccttttgca agaagccagt aaaatgataa gcgtcctcac tacaaactgc    720
ccatgggtca atcacaatca aggacaaaac gggggcgcgc cgtggggttt agatacggca    780
gggaatgtgt gtcaggtttt tgccacggaa tttagcgccg ttactagcat gatcaaaaac    840
gcccaagaaa tcgtaacgca agctcaaagc cttaaccagc aaaacaatca aaacgcgccg    900
caagatttca atccttacac ctctgctgat agggctttcg ctcaaaacat gctcaatcac    960
gcgcaagcgc aagccaagat acttgagcta gccgatcaaa tgaaaaaaga ccttaacact   1020
atcccaagcc aatttatcac aaattacttg gcagcttgcc acaatggggg tgggacatta   1080
cctgatgcgg gggttactaa caacacttgg ggggccggtt gcgcgtatgt ggaagagacg   1140
ataacggctt taaacaacag ccttgcgcat tttggcactc aagctgagca aatcaagcaa   1200
tctgagttgt tggcgcgcac catacttgat tttagaggca gccttagtaa tttaaacaac   1260
acttataaca gcatcaccac gaccgcttca aacacgccta attccccatt ccttaaaaat   1320
ttgataagcc aatccactaa ccctaataac cccgggggct tacaggccgt ttatcaagtc   1380
aaccaaagcg cttattcgca attattaagc gccacgcaag aattagggca taaccctttc   1440
agacgcgttg gattaatcag ctctcaaacc aacaatggtg ccatgaatgg gatcggtgtg   1500
caagtgggct acaaacaatt ttttggtgaa aagagaaggt gggggttaag gtattacggc   1560
ttttttgact ataaccatgc ttatatcaaa tctagctttt tcaattcggc ttctgatgtg   1620
ttcacttatg ggtagggac agatgtcctc tataacttta tcaatgataa aaccaccaaa    1680
aacagcaaga tttcttttgg ggtgtttggg gggattgcgt tagctggcac ttcatggctg   1740
aattcccagt atgtgaattt agcgaccttc aataatttct atagcgctaa aatgaatgtg   1800

gcgaatttcc aattcttgtt caatttaggc ttgagaatga acctcgctaa gaataagaaa   1860
aaagcgagcg atcatgcggc tcagcatggc gtggaattag gcgtgaagat ccccacgatc   1920
aacacgaatt actattcttt gctaggcact caactccaat accgaagatt gtatagcgtg   1980
tatttgaatt atgtgttcgc ttactaa                                       2007
```

<212> Type : DNA
<211> Length : 2007
SequenceName : SEQ ID 487
SequenceDescription :
Sequence
--------
<213> OrganismName : Helicobacter pylori, strain J99
<400> PreSequenceString :

```
atgagaaaac tattcatccc acttttatta ttcagcgctt tagaagcgaa cgagaaaaac      60
ggctttttca tagaagccgg ctttgaaact gggctattag aaggcacaca aacgcaagaa     120
aaaagacaca ccaccacaaa aaacacttac gcaacttaca attatttacc cacagacacg     180
attttaaaaa gagcggctaa tttattcacc aatgccgaag cgatttcaaa attaaaattc     240
tcatctttat cccctgttag agtgttgtat atgtataatg gtcaattaac tatagaaaac     300
ttcttgcctt ataatttaaa taatgttaag cttagtttta cagacgctca aggcaacacg     360
attgatctag gcgtgataga gaccatcccc aaacactcta agattgtttt acccggggag     420
gcgtttgata gtttaaaaga ggcgtttgat aaaattgacc cctatacttt atttcttcca     480
aaatttgaag ccactagcac ttctatttct gatactaaca cgcagagggt gtttgaaacg     540
ctcaataaca ttaaaacaaa tcttataatg aaatatagta atgaaaatcc aaacaatttc     600
aacacttgtc cttacaataa taatggtaat acaaaaaatg attgttggca aaatttcacc     660
ccacaaaccg cagaagaatt caccaattta atgttgaaca tgatcgctgt cttagactcc     720
caatcttggg gcgatgcgat cttaaacgct cctttgaat tcactaacag ctcaacagat      780
tgcgatagcg atccttcaaa atgcgtaaat cccggagtaa atgggcgtgt tgatactaaa     840
gtcgatcaac aatatatact caacaaacaa ggtattatta ataattttag aaaaaaaata     900
gaaattgatg cggttgtttt aaaaaattca ggggttgtag ggttagccaa tggatatggc     960
aatgatggtg aatatggcac attaggggta gaagcctatg ctttagatcc taaaaaactc    1020
tttggcaacg accttaagac tatcaattta gaagatttaa gaaccatctt gcatgaattc    1080
agccacacta aaggctatgg gcataacggg aatatgacct atcaaagagt gccggtaacg    1140
aaagatggtc aagtggaaaa ggatagtaat ggcaagccaa aagattctga tggcctcccc    1200
tataatgtgt gttcgcttta tgggggatcc aatcagcccg ctttccctag caactaccct    1260
aattccatct atcacaattg tgcggatgtc ccggctggct ttttaggggt aacagcagcg    1320
gtttggcagc agctcatcaa tcaaaacgcc ttgccgatca actacgctaa cttggggagt    1380
caaacaaact acaacctaaa cgctagttta aacacgcaag atttagccaa ttccatgctc    1440
agcaccatcc aaaaaacctt tgtaacttct agcgttacca accaccattt ttcaaacgca    1500
tcgcaaagtt ttagaagccc tattttaggg gttaacgcta aaataggcta tcaaaactac    1560
tttaatgatt tcatagggtt ggcttattat ggcatcatca aatacaatta cgctaaagct    1620
gttaatcaaa aagtccagca attgagctat ggtgggggga tagatttgtt attggatttc    1680
atcaccactt actccaataa aaatagccct acaggcattc aaaccaaaag gaatttttct    1740
tcatcttttg gtatctttgg ggggttaagg ggcttgtata acagctatta tgtgttgaac    1800
aaagtcaaag gaagcggcaa tttagatgtg gctaccgggt tgaactaccg ctataagcat    1860
tctaaatatt ctgtagggat tagcatccct ttaatccaaa gaaaagctag cgtcgtttct    1920
agcggtggcg attatacgaa ctcttttgtt ttcaatgaag ggggctagcca ctttaaggtg   1980
tttttcaatt acgggtgggt gttttag                                        2007
```

<212> Type : DNA
<211> Length : 2007
SequenceName : SEQ ID 488
SequenceDescription :
Sequence
--------
<213> OrganismName : Helicobacter pylori, strain J99
<400> PreSequenceString :

```
atgaataaaa caacaattaa aatattaatg ggcatggcgt tattatcatc gcttcaagcc      60
gcagaggcag agcttgatga aaaatcaaaa aaacctaaat ttgcggatag gaatacgttt     120
tatttagggg ttgggtatca gcttagcgcg atcaacacgt cttttagcac cagttctata     180
gataaatcgt atttcatgac cggcaatggt tttggcgtgg tgttgggggg gaaatttgtg     240
gctaaaacgc aagctgtaga gcatgtgggt tttcgttacg ggttgtttta tgatcagacc     300
ttttcttctc acaaatccta tatttctacc tatggtttag aatttagcgg tttgtgggac     360
gctttcaatt cgccaaagat gtttttgggg ttggagtttg gcttaggcat cgctggggcg     420
acttacatgc caggaggggc catgcatggg attatcgctc aatatttagg caaagaaaat     480
tcgcttttcc aattgcttgt gaaagtgggt tttcgttttg gctttttcca caatgaaatc     540
acctttgggt tgaaattccc tgtcattcct aacaaaaaaa cggaaatcgt tgatggcttg     600
agcgcgacca ctttatggca acgcttgccg gtagcctatt tcaattatat ctataatttt     660

tag                                                                  663
```

<212> Type : DNA
<211> Length : 663
SequenceName : SEQ ID 489
SequenceDescription :
Sequence
--------

<213> OrganismName : Helicobacter pylori, strain J99
<400> PreSequenceString :

```
atgaaaaaaa cgaaaaaaac gattctgctt tctctaactc ttgcggcgtc attgctccat     60
gctgaagaca acggcgtttt tttaagcgtg ggctatcaaa tcggtgaagc ggttcaaaag    120
gtgaaaaacg ccgacaaggt acaaaagctt tcagacgttt atgaacaatt aagcaagctt    180
ttagccaacg ataatggcac tagctcaaaa acaagcgcgc aagcgatcaa tcaagcggtt    240
aataatttga atgaaagcgc aaaaacttta gccggtggga caaccaattc ccctgcctat    300
caagccacgc ttttagcatt gagatcggcg ttagggttat ggaatagcat gggctatgcg    360
gtcgtatgcg gaggttatat taaaaaaccg ggcgaaaaca atcaaaaaaa tttccactac    420
accgatgaga atggcaacgg cactacaatc aattgcggtg ggagcacaaa tagtaatggc    480
actcatagtc ctaatggcac aaatacatta aaagcagaca aaaatgtttc tctatctatt    540
gagcaatatg aaaaaatcca tgaagcctat caaatccttt caaaggcttt aaaacaagct    600
gggcttgctc ctttaaatag caaaggggaa aagttagaag cgcatgtaac cacatcaaag    660
gatcaacaag gaacatccag tgaccaaact acaaccacaa cttctgttat tgatacgact    720
aatgatgcgc aaaatctttt gactcaagcg caaacgattg tcaataccct taaagattat    780
tgccccatgt tgatagcgaa atctagtagt aatggtggaa ctaatggcgc aaacacccct    840
tcatggcaaa cagccggtgg cggcaaaaat tcatgtgcga cttttggtgc ggagtttagt    900
gctatttcag acatgattag taacgctcaa aaatcgttc aagaaaccca acaacttaac    960
gccaaccaac ccaaaaatat cacccaaccc aataatttca accttaactc tcctggcagt   1020
cttacggctt tagctcaaag catgctcaaa aacgctcaat ctcaaacaga aattttaaaa   1080
ttagccaatc aggtagcaag cgattttgac aaactttctt caggctatct taaagattac   1140
atagggaaat gcgatgtgag tggtgtgagt agttcaaata tgacaccgca aaatatgaat   1200
accacttggg ggaaaggctg cgcgggcgtg gaagaaactc taacttcgtt aaaagcaagc   1260
accactgatt ttaacaacca gacaacgccc caactcgatc aagcgcaaac cctagccaat   1320
acccttactc aagaactcgg caataaccct ttcaaacgag tgggtatcat tggctctcaa   1380
accaataacg gggcgatgaa tggccttggg gtgcaagcgg gttataagca attctttggt   1440
caaaaaagaa ggtgggggtt aaggtattac ggctttttg actacaacca tacctacatc    1500
aaatccagct tttttaactc gtcttctgac gttttgactt atggggtggg tagcgattta   1560
ttgtttaatt tcatcaatga taaaaacacc aatttcttag gcaagaacaa taagatttct   1620
gtggggcttt ttggaggtat cgccttagca gggacttcgt ggcttaattc tcaattcgtg   1680
aatttaaaaa ccatcagcaa tgtttatagc gctaaagtga atacggctaa tttccaattc   1740
ttattcaatt taggcttgag aaccaatctc gctaggccta agaaaaaaga cagcgatcat   1800
tccgcgcaac atggcatgga attgggcgtg aaaatcccta ccattaacac gaattactat   1860
tcttacttgg gaactaaact agaataccga agactctata gcgtgtatct caattatgtg   1920
tttgcgtatt ga                                                        1932
```

<212> Type : DNA
<211> Length : 1932
SequenceName : SEQ ID 490
SequenceDescription :
Sequence
--------
<213> OrganismName : Helicobacter pylori, strain J99
<400> PreSequenceString :

```
atgaaaaaaa cgattttact ttctctcatg gtgtcatcgc tctttgctga aaatgacggc     60
gtttatatga gcgtgggcta tcaaatcggc gaagccgcac aaatggtgaa aaacaccggc    120
gaaatccaaa aagtctccaa cgcttacgaa aatttgaaca accttttaac ccgctataat    180
gaactcaaac aaacggcctc taacactgat tcaagcaccg ctcaagcgat tgacaatcta    240
gaaaagagcg ctagcagatt gaaaacgacc cctaataccg ccaatcaagc cgtgtcctca    300
gcgctcagct ctgcggtggg catgtggcaa gtgatagcct ctaatttagc caacaactcg    360
ctatcttcta gcgaatacga aaaactcaaa gcgacttctc aattgctcca aaatacccta    420
gaaaataaaa acaataatct taaaattgaa aatgactatg accagctttt aactcaagct    480
agtaccatta ttaataccct tcaaagccaa tgcccaggcg tagatggggg caatggcaaa    540
ccatggggca ttaatacaag cgggaacgca tgcgctattt ttggtagcac ctttaacgcc    600
attaatagca tgattgatag cgctaaaaaa gccgccgcag atgcccgaag aactgcccca    660
gaaagtccaa accaacaaaa cgcgtttacc aacgctgatt tcaataaaaa cctcaatcaa    720
gtctcaagcg ttatcaatga caccatctct tacctcaaag ggacaattt agaaaccatc     780
tacaacacca ttcaaaaaac gcctaattct aaagggtttc aaagtttggt gagccggtct    840
agctatagtt attctctcaa cgaaacccaa tattctcaat tccaaactac caccaaagag    900
```

```
tttggtcata acccttcag aagcgtggga ttaatcaact ctcaaagcaa taacggggcg     960
atgaatggcg tgggcgtgca actaggctat aagcaattct ttgggaaaaa taaatttttt    1020
gggatccggt attatggctt ttttgattac aactatgcgt atatcaaatc caatttttc     1080
aactccgctt ccaatgtttt cacctatggc gcgggcagtg atcttttatt gaacttcatc    1140
aatggcggat ccgatcgaaa ccgcaaagtc tcttttggca tttttggggg catcgctcta    1200
gcgggaacga catggcttaa taaccaatct gcgaatttaa aaatcaccaa tagcgcctac    1260
agcgctaaga tcaacaacac caatttccaa ttcttgttca ataccggttt aaggcttcaa    1320
gggatccatc atggcattga attaggcgtg aaaatcccta cgatcaacac caattactat    1380
tctttcatgg gcgctaaatt agcctaccgc aggctttata gcttgtacct caattatgtt    1440
ttggcttatt ga                                                        1452
```

<212> Type : DNA
<211> Length : 1452
SequenceName : SEQ ID 491
SequenceDescription :
Sequence
--------
<213> OrganismName : Helicobacter pylori, strain J99
<400> PreSequenceString :

```
atgccaaagg caagtcaggt tttattcttt ggagcgtttt taagcacttc tttacaaggt      60
tttgaagcta agctcaacgg ctttgtggat caatccagca cgatcggttt taaccagcat     120
aaaatcaata aagaaagagg catctaccct atgcagcaat tcgcaacgat tgcgggctat     180
ttagggcttg gttttagcct gttacccaaa aaggtttcag accatgttct aaaaggcaaa     240
atagggggca tggtcggatc tattttctat gacggcacga agaagtttga agacggctct     300
gtggcttaca acctctttgg ttattacgat gggtttatgg gggtctatac taatatctta     360
caaaccgata gccttgagac acagaacatg aaacacaaca aaaatgtccg caattatgtc     420
tttagcgacg cgtatttaga atacgcttat aagaattatt ttgaaataaa agccgggcgc     480
tatctctcca ctatgcctta taaaagcggt caaacgcaag gctttcaagt ttctgggcaa     540
tacaagcatg cgcgcttgac ttggtttagc tcatggggta gggcgtttgc ttatggttcg     600
tttttaatgg attggtttgc cgcacggacc acttatagcg gaggctttac caaaaacaat     660
aatggaggtt atgatagcca tgggcgaaag gtgctttatg gcacgcatgc ggtgcaactc     720
acctataaac ctcatcgttt cctcatagaa ggcttttatt acctttcgcc tcaaatcttt     780
aacgctccag gcgttaagat tggttgggac tctaacccta attttagcgg cacaggcttt     840
cgctctgata cggctatcat agggtttttc cccatttact acccttggat gatcgttaaa     900
tccaatggaa gcccggtcta tagatacgac acgcctgcca ctcaaaacgg gcaaaacctc     960
attatccgcc aacgctttga catcaacaat tacaatgttt caatcgcttt ttataaagtc    1020
tttcaaaacg ctaatggttg gataggcaac atggggaatc caagcggtgt gatcatgggg    1080
agtaacagcg tctatgcagg ttttacaggc acagccctta aaagagacgc cgctaccatt    1140
ttcctttctt gtggtggcac tcattttgcc aaaaaattca catggaaatt cgccacacaa    1200
tactccaatt cagtggtctc ttgggaagca agagcgatga tctctttagg ctataaattc    1260
actgaatatt tgagcggtag cgtggatctt gcgtattatg gcgtgcatac taacaaaggc    1320
tttaaaccgg gtgaaaacgg gcctgtgcct aaaaacttcc ccgcccttta ttctgacagg    1380
agcgctttat acacggctct agtagcgtct ttttga                              1416
```

<212> Type : DNA
<211> Length : 1416
SequenceName : SEQ ID 492
SequenceDescription :
Sequence
--------
<213> OrganismName : Helicobacter pylori, strain J99
<400> PreSequenceString :

```
atgctaaggc tcgttagtaa aacgatttgt ttgtctttaa ttagcttgtt caacccttta      60
gaagcctttc aaaaacacca aaaagacgtc ttttttgtag aagctgggtt tgaaaccggg     120
ctattagaag gcgcgcaaac caaagaacaa gcaatagccc aaaacaccca aaacacccaa     180
aaaatttatg aaaacccccct aacccacccc caaactaaag aacaacctaa agaacaaaac    240
aaaagcgata cagccacccc acaaagcgtc tatgggagat actacatcct ccaaaacacc     300
attttagaaa aagcgactga gctattcaca gcggctaata tcaatggcaa cggcttaact     360
ttttattctc aaaaccctgt gtatgtgatg gcatacaata aagataatgc cgagtttgaa     420
ggctatggca ataacagcgt ggttgtgata caaaacttcc tgccctacaa tttaaacaat     480
attgagctga gttatacaga cgctcaaggc aaggcagtca atttaggcgt gatagagacc     540
atccctaaag attctcaaat catcttgcct gcaagtttgt ttaataattt ttcaaacgat     600
tcaccattca actctgatgg cctccaacaa ctccaaacca ctaccacccc cttttctgat     660
gctaacacgc agagtttgtt tgaaaagctc agtcaaatca cgaccaatct tcaaatgact     720
tatgagaata cagacccctt ttctagcggc aacaacgatc ctaatggccc tctcgcttct     780
cctaaacctc attatgaatg ccctggttat aaaaagagtt gtcaagtcgc ttcggtgtct     840
ttcaccccac aaaccgcaga agaattgacc aatttaatgt tagacatgat tgcggtgttt     900

gactctaaat cttgggaaga agccgtttta aacgcccctt tccaattttc taacagccca     960
tcagagtgcg gcattgatta ccctaaatgc gttaatccct ttaataacgg gcttgttgat    1020
cctaaagatg aaaaatacgc gctaacccca gaagaggtta tcaatagtta tagagtcgcc    1080
aatgaactta ccgtgaacct cttgaatgcg gccaaggggt ttctagggct aggatcccaa    1140
ctgggtagcg ccaatgcccc cgatgatgat ggcttcaatc aaggtgtttt agggatagcg    1200
ccttttgctt tagatcctga aaaattgttc ggtaaaaatt tgaataaagt ggctattttg    1260
gcattaagag acattatcca tgaatatggg catactttag gctataccca taacgggaac    1320
atgacttatc aaagggtgcg tttatgccaa gaaggaaacg ggccagaggc acgctgtgag    1380
ggcgggcatg aagtggagaa aaacggcaaa gaagagctag aattcagtaa tgggcatgaa    1440
gtgcgagacc atgatggtta cacctatgat gtttgctctc gttttggcgg caaaaatcag    1500
cccgctttcc ctagcaatta ccccaattcc atctatacca attgcgctca agtccccgct    1560
gggcttatag ggttactac cgctgtttgg caacagctca tcaatcaaaa cgccctgccc     1620
attaatttcg ctaacctaaa tagccaaacc agccatttaa acgccgggtt gaatgcgcaa    1680
aattttgcaa cctctatggt cagcgcgatc gcgcaaaatt tttccaccac ttccactacc    1740
acttaccgct cttcaagtaa gaattttaga agccctattt taggggttaa tgttaaaata    1800
ggctaccaac attatttcaa tgactacatc gggttagcct attacggcat tatccaatac    1860
aactacgctc aagctaacga tgaaaaaatc cagcaattaa gctatggtgg gggaatggat    1920
gtgctgtttg atttcatcac cacttacacc aataaaaagc aagaccatcc aactaaaaag    1980
gtttttgctt cctcttttgg ggtgtttggg gggttaaggg gcttatacaa tagctactat    2040
gtcttcaatc aagtcaaagg aagcggtaat ttagatatag taaccgggtt taattaccgc    2100
tacaagcatt ctaaatattc cataggcgtt agcgttcctt taatccaaag cggtattaag    2160
atcgcttcta ataatggtat ctatgcagac tctgttgttt tgaatgaagg gggtagccat    2220
tttaaagtgt tttttaatta cgggtgggta ttttaa                              2256
```

<212> Type : DNA
<211> Length : 2256
SequenceName : SEQ ID 493
SequenceDescription :
Sequence
--------
<213> OrganismName : Helicobacter pylori, strain J99
<400> PreSequenceString :

```
ttgcaaaact ttgtttttaa taaaaaatgg ctcatctatt ctagcctact ccccttattt      60
tttcttaacc ctttaatggc agaagacgat gggtttttta tgggggtgag ttatcaaact     120
tctttggccg ttcaaagggt ggataactca gggcttaacg ccagtcaaga cgcatccact     180
tatatccgcc aaaacgctat cgctctagaa tctgcggcgg tgcctttagc ctattattta     240
gaagcgatgg gccaacaaac gagagtctta atgcaaatgc tctgccctga tccttctaaa     300
agatgtttgc tctatgcagg gggctatcaa aacggacaaa ataataacgg cgatacaggc     360
aacaaccccc caagaggcaa tgtcaatgcc acctttgata tgcaatctct agtcaataat     420
ttaaacaagc tcacccaact catcggcgaa actttaatcc gtaaccctga aaatcttcct     480
aactccaaag tctttaacgt caaatttggc aatcaaagca ctgttattgc attgcctgag     540
ggtctagcca ataccatgga cgctttaaac aatgacatca ccaacgcttt aaccacgctc     600
tggtataacc aaaccttaac gaataaatct tttagcaccc ctagtaacac ttctgtgaat     660
tttagccccc aagtcttgca acacctttta caagacggct tagccacagc aaataataat     720
caaaccattt gcagcactca aaaccaatgc accgccacta atgaagctaa atctatcgct     780
caaaacgccc aaaacatctt ccaggcttta atgcaagcag ggattttagg gggcttagcc     840
aatgaaaagc aatttggctt cacttacaac aaagccccca atggcagcga ttcccaacaa     900
ggctatcaaa gctttagcgg cccgggttat tacaccaaaa acgacaacac cacgcaagcg     960
cccttaaaag cattacccgc tggagcgaca attggatcag gcaatggcca atacacctac    1020
caccccagct cggcagtcta ttatttagcc gatagcatca tcgctaatgg catcaccgct    1080
tctatgattt tttcaggcat gcaaaatttc gccaataaag ccgctaaact gataggcact    1140
tcaagctata accagatgca agatgcgatc aactatgggg aaagcttgct tagtaacacc    1200
gtagcgtatg gggatttcat caccaattgg gtcgccccct atttggattt aaacaataaa    1260
ggtttgaatt tcttgcctaa ttatgggggg caattgaatg gcgctaataa tcaaacccca    1320
caattaaccc cacaacaagc ccaacaagaa caaaaagtga tcatgaacca attagagcaa    1380
gccacaaacg cccccacccc cgcgcaaata aacaggattt tagccaaccc ctattccccc    1440
acggcaaaaa ctttaatggc ttatgggctc tatcgctcta aagcagtgat tggcggagtg    1500
attgatgaaa tgcaaactaa agtgaatcaa gtctatcaaa tgggctttgc taggaatttt    1560
ttggagcata actctaattc taataacatg aacggctttg gcgtgaaaat gggctataag    1620
caatttttcg gcaaaaagcg catgtttggg cttaggtatt atggttttta tgattttggt    1680
tacgctcaat ttggcacaga atcttcttta gtgaaagcca ccctctctag ctatggagcg    1740
ggcacagact ttctttataa tgtttttacc cgaaaaagag ggactgaagc gatagatata    1800
ggtttttttg ccggtatcca acttgcaggg caaacctgga aaacgaattt tttagatcaa    1860
gtggatggca accatcttaa acctaaggac acttctttcc aattcctttt tgatttgggc    1920
ataaggacca atttttccaa aatcgctcat caaaaaagat cccgtttttc tcaagggata    1980
gaatttggcc ttaaaatacc ggtgctttat cacacctatt accaatcaga aggcgttaca    2040
gcgaagtata gaagagactt tagtttttat gtgggctaca acataggctt ttga          2094
```

<212> Type : DNA
<211> Length : 2094
SequenceName : SEQ ID 494
SequenceDescription :
Sequence
--------
<213> OrganismName : Helicobacter pylori, strain J99
<400> PreSequenceString :

```
atgaaaaaga caattctact ctctctctct ctctctctcg cttcatcgct cttgcacgct      60
gaagacaacg gcttttttgt gagcgcgggc tatcaaatcg gcgaagcggt gcaaatggtc     120
aaaaacaccg gtgaattgaa aaacttgaac gaaaaatacg agcaattaag ccagtattta     180
aatcaagtgg cttcgttgaa gcaaagcatt caaaacgcca acaacattga gctggtcaat     240
agctctttaa actatttaaa aagctttacc aacaacaact acaacagcac cacccaatcg     300
cccatcttta acgccgtgca agccgttatc acttcggtat tgggtttttg gagtctttat     360
gcggggaact atctcacttt ttttgtggtt aataaggata ctcaaaaacc cgctagtgtc     420
cagggtaacc ctcctttttc aactattgtt caaaactgct caggaattga aaactgcgcg     480
atgaatcaaa ccacttatga taagatgaaa aagctcgctg aagatctcca agcagcccaa     540
caaaacgcta ccactaaagc gaacaatctt tgcgctttat ccggatgcgc cacaacacaa     600
ggccaaaacc caagctcaac cgtaagcaac gctcttaact tagcgcaaca gcttatggat     660
ttgatcgcaa acactaagac ggctatgatg tggaaaaata tcgtcatcgc tggcgtttca     720
aacgtatccg gcgctatcga ttccactggc tacccaacgc aatacgcggt gtttaacaac     780
attaaggcga tgatacctat cttgcaacaa gcggttacgc tttctcaaag taaccacaca     840
ttatctgcta gcttgcaagc tcaagctaca ggatctcaaa caaaccccaa attcgctaaa     900
gacatctacg ctttcgctca aaaccaaaag caagtcattt cttacgctca agacattttc     960
aacctcttta gttctatccc taaagatcag tatcgttatt tagagaaagc ctatttgaaa    1020
atacccaatg cgggtaaaac gcctactaac ccttacagac aggaggtgaa tttaaaccaa    1080
gaaattcaaa cgatccaaaa caatgtgagt tattatggta atcgggtgga tgcggcttta    1140
agcgtggcta aagatgttta taatttaaaa tccaatcaaa cagaaatcgt aaccacctat    1200
aacaacgcta agaatttgag ccaagagatt tctaaactcc cctataacca agtcaataca    1260
aaagacatta tcacactgcc ttacgatcaa aacgctccgg cagcgggcca atacaactac    1320
cagatcaacc cagagcagca atccaatctt tctcaagctt tagcggcgat gagcaataac    1380
ccctttaaaa aagtgggcat gatcagctct caaaacaata acggcgcttt gaacgggctt    1440
ggcgtgcaag tgggttataa acaattcttt ggcgaaagca aaagatgggg gttaaggtat    1500
tatggtttct ttgattacaa ccacggctat atcaaatcca gcttttttaa ttcttcttct    1560
gatatatgga cttatggcgg tgggagcgat ttgttagtga attttatcaa cgatagcatc    1620
acaagaaaga acaacaagct ttctgtgggt ctttttggtg gtatccaact agcagggact    1680
acatggctta attctcaata catgaattta acagcgttca ataaccctta cagcgcgaaa    1740
gtcaatgctt ccaatttcca atttttgttc aatctcggct tgaggacgaa tctcgctaca    1800
gctaagaaaa aagacagcga acgttccgcg caacatggcg ttgaactggg cattaaaatc    1860
cctaccatta acaccaatta ttattctttt ctaggcacta agctagaata cagaaggctt    1920
tatagcgtgt atctcaatta tgtgtttgct tattaa                              1956
```

<212> Type : DNA
<211> Length : 1956
SequenceName : SEQ ID 495
SequenceDescription :
Sequence
--------
<213> OrganismName : Helicobacter pylori, strain J99
<400> PreSequenceString :

```
atgttaaaac tcgccagtaa aacgatttgt ttgtccctaa tcagctcatt cacggctgta      60
gaagcctttc aaaaacacca aaaagacggc tttttcatag aagccggctt tgaaaccggg     120
ctattacaag gcacacaaac ccaagaacaa accatagcca ccactcaaga aaaacccaaa     180
cccaaaccca aaccaaaacc cattacccct caaagcacct atgggaaata ctacatctcc     240
caaagcacca ttttaaagaa tgcgactgag ttgtttgcag aggataatat caccaactta     300
acctttact  ctcaaaaccc tgtgtatgta accgcttata accaagaaag cgctgaagaa     360
gctggctatg gtaataacag cttgattatg atacaaaact tcttgcctta taacttgaac     420
aacattgagc tgagttacac ggacgatcaa ggcaatgtgg tcagtttggg cgtgatagag     480
actatcccta aacaatctca aatcattctg cccgcaagct tgtttaacga cccacagctt     540
aacgccgatg gcttccaaca actccaaacc aacaccacac gattttctga tgccagcacg     600
cagaatctgt ttaacaagct cagcaaggtt acaaccaatc ttcaaatgac ttatatcaat     660
tacaaccaat tttctagcgg taacggcagt ggctctaaac ccccatgccc cccatacgaa     720
aaccaagcaa attgtgtggc taaagtgccg cctttcacct ctcaagacgc taaaaatttg     780
accaatttaa tgctgaacat gatggcggtg tttgattcta aatcttggga agacgccgtc     840
ttaaacgctc ctttccaatt cagcgacaac aacctgtcag cgccatgtta ttctgattac     900
```

cttacatgcg tgaatcctta caacgatggg cttgttgatc ctaaattgat cgccaaaaat 960
aaaggagatg aatacaatat agaaaacggg caaacaggct cagtgatatt aacgccgcaa 1020
gatgttatct atagctatag agtcgctaat aatatttatg tgaatctctt gcccacaaga 1080
ggaggggatt tagggttagg gtctcaatat ggtggcccga atggcccagg cgatgatggc 1140
accaattttg gcgctttagg gatattgtcc cctttcttag accctgaaat attgtttggc 1200
aaagaattga ataaagtcgc catcatgcaa ttaagagaca tcatccatga atacggccat 1260
actttaggct atacgcataa cgggaacatg acttatcaaa gagtgcgcat gtgcgaagaa 1320
aacaatgggc cagaagagcg ctgtcagggc ggaaggatag agcaagtgga tgggaaagaa 1380
gtgcaagtgt ttgacaacgg gcatgaagtg cgagacaccg atggctctac ctatgatgtg 1440
tgttctcgtt ttaaagataa gccctataca gcgggcagct atcctaattc catctatacc 1500
gattgctctc aagtccccgc tgggcttata ggcgttacca gcgctgtttg gcaacaactc 1560
attgatcaaa acgccctacc ggtggatttt actaatttga gcagccaaac caactatttg 1620
aacgccagct tgaacacgca agactttgcg accaccatgc ttagcgcgat cagtcaaagc 1680
ctttcatctt ctaaatctag cgccactact tatcgcactt caaaaacctc acggcccttt 1740
ggagcccccc tattaggcgt taatcttaaa atgggctatc aaaaatattt taatgattat 1800
ctagggttgt cttcttatgg cattatcaaa tacaactacg ctcaagccaa caacgaaaaa 1860
atccagcaat taagctatgg cgtgggaatg gatgtgctgt ttgatttcat caccaattac 1920
actaacgaaa agaaccccaa aagcaatcta accaagaaag ttttcacttc ctctcttggg 1980
gtgtttgggg ggttaagggg cttatacaac agctattatt tgttgaacca atacaaaggg 2040
agcggtaatt taaatgtgac cggtgggttg aattaccgct acaagcattc caaatattct 2100
ataggcatta gcgttccttt ggtccagttg aaatctagga tcgtttctag cgatggtgct 2160
tataccaatt ctatcaccct caatgaaggg ggcagtcatt ttaaagtgtt ttttaattac 2220
gggtggattt tctaa 2235

<212> Type : DNA
<211> Length : 2235
SequenceName : SEQ ID 496
SequenceDescription :
Sequence
<213> OrganismName : Helicobacter pylori, strain J99
<400> PreSequenceString :

atgagaaaac tattcatccc acttttatta ttcagcgctt tagaagcgaa cgagaaaaac 60
ggctttttca tagaagccgg ctttgaaact gggctattag aaggcacaca aacgcaagaa 120
aaaagacaca ccaccacaaa aaacacttac gcaacttaca attatttacc cacagacacg 180
attttaaaaa gagcggctaa tttattcacc aatgccgaag cgatttcaaa attaaaattc 240
tcatctttat cccctgttag agtgttgtat atgtataatg gtcaattaac tatagaaaac 300
ttcttgcctt ataatttaaa taatgttaag cttagtttta cagacgctca aggcaatgtg 360
atcgatctag gcgtgataga gactatcccc aaacactcta agattgtttt gcccggagag 420
gcatttgata gtctaaaaat tgacccctat actttatttc ttccaaaaat tgaagccact 480
agcacttcta tttctgacgc taacacgcag agggtgtttg aaacgctcaa taagattaag 540
acaaatttgg tcgtaaatta taggaatgaa aacaaattta aagatcacga aaatcattgg 600
gaagccttta ccccacaaac cgcagaagaa ttcactaatt taatgttgaa catgatcgct 660
gttttagact cccaatcttg ggcgatgcg atcttaaacg ctccttttga gttcactaac 720
agcccaacag attgcgataa tgatccttca aaatgcgtaa atcctgggac aaacgggctt 780
gtcaattcta aagtcgatca aaaatatgtg ttaaacaaac aagacattgt caataaattt 840
aaaaacaaag cggatcttga tgtaattgtt ttaaaggatt caggggttgt agggcttggg 900
agtgatatta ccccctagcaa caatgatgat ggcaagcatt atggccagtt aggggtagta 960
gcttctgctt tagatcctaa aaaactcttt ggcgataacc ttaagactat caatttagag 1020
gatttaagaa ccatcttgca tgaattcagc cacactaaag gctatgggca taacgggaat 1080
atgacctatc aaagagtgcc ggtaacgaaa gatggtcaag tggaaaagga tagtaatggc 1140
aagccaaaag attctgatgg cctcccctat aatgtgtgtt cgctttatgg gggatccaat 1200
cagcccgctt tccctagcaa ctaccctaat tccatctatc acaattgtgc ggatgtcccg 1260
gctggctttt taggggtaac agcagcggtt tggcagcagc tcatcaatca aaacgccttg 1320
ccgatcaact acgctaactt ggggagtcaa acaaactaca acctaaacgc tagtttaaac 1380
acgcaagatt tagccaattc catgctcagc accatccaaa aaacctttgt aacttctagc 1440
gttaccaacc accatttttc aaacgcatcg caaagtttta gaagccctat tttaggggtt 1500
aacgctaaaa taggctatca aaactacttt aatgatttca tagggttggc ttattatggc 1560
atcatcaaat acaattacgc taaagctgtt aatcaaaaag tccagcaatt gagctatggt 1620
gggggggatag atttgttatt ggatttcatc accacttact ccaataaaaa tagccctaca 1680
ggcattcaaa ccaaaaggaa tttttcttca tcttttggta tctttggggg gttaaggggc 1740
ttgtataaca gctattatgt gttgaacaaa gtcaaaggaa gcggcaattt agatgtggct 1800
accgggttga actaccgcta taagcattct aaatattctg tagggattag catcccttta 1860
atccaaagaa aagctagcgt cgtttctagc ggtggcgatt atacgaactc ttttgttttc 1920
aatgaagggg ctagccactt taaggtgttt ttcaattacg ggtgggtgtt ttag 1974

<212> Type : DNA

<211> Length : 1974
SequenceName : SEQ ID 497
SequenceDescription :
Sequence
--------
<213> OrganismName : Helicobacter pylori, strain J99
<400> PreSequenceString :

```
atgagtctag ctacgagtta caatgtgagt aataattttt ctaagtttaa tattaagaga     60
gtcagaggat atttgatttg tcttgtttgt aacacaccta aaatgataca aagaggattg    120
aatggtgtct cattttatgg ttgctctgat tatgtaaata aaggcgactg taagggcgtt    180
ttacgagaaa taaatggctc aatgaaaatg gtctgcttac attgtgaaaa cacgcccata    240
atggaaaaag tagaaagtgg taggggagga gcttacgctt gtaagaattg caataggaag    300
ttttacttta tcgatcttgc aaaacaaaac gaacgaaaaa aagatttaga aaaagaagaa    360
aaagaattgc ttaataagat tgaaaagcaa aaaatcaaac accttgagcg tttcattttg    420
gctggtgtaa aagctaatat taaagaaaat tcttttttct taggatgtaa aaattatcct    480
aagtgcgaat ggactgctag tatggattca caagatctta aatgtcccaa atgcaacaga    540
ttaatgaaaa gaaaaaagaa tttcaaaaac aatgagtttt ttacagctac atcgcttacc    600
ttaaatgcaa tagaattttg tctctatatt aatttgaaaa aaaaggaaac caatgtttag    660
```

<212> Type : DNA
<211> Length : 660
SequenceName : SEQ ID 498
SequenceDescription :
Sequence
--------
<213> OrganismName : Helicobacter pylori, strain J99
<400> PreSequenceString :

```
ttggaaatta agaaatattt tctttacgct ctattttttt tgctttttttc tggtcttttt     60
ttatccaaac ttcaagctta taaattcaac atgagtattg ttggaaaggt gagcagctat    120
actaagtttg gctttaacaa ccaaagatac cagccttcta aagacattta tcctacaggt    180
agttatactt ctttactcgg cgaattgaat ttgagcatgg gattatacaa gggcttgagg    240
gcagaagtag gggctatgat ggcagcgctt ccctatgact ctaccgccta tcaaggcaat    300
aatatcccta atggccagcc cggatctagg acggatcctt ttggggcggg tatcttttgg    360
caatacattg gctggtatgc aggacatagc ggtttaaacg tgcaaaaacc tcgtttggct    420
atggtgcata acgctttttt gagctacaac tacaagaaag acaaattcag ttttggcgtg    480
aaagggggggc gctatgatgc tgaagagtat gattggttca cttcttacac tcaaggggtt    540
gaaggctttg tcaaatacaa agacaccagg ttaagggtga tgtattcaga cgctagggct    600
tcagcgtcaa gcgactggtt ttggtatttt gggcgttact atacaagcgg taaggctcta    660
atgattgcgg atttgaaata cgaaaaagac aacctaaaaa tcaaccctta tttttatgcg    720
atctttcaaa gaatgtatgc gccaggcatt aatatcactt acgacaccaa ccctaatttc    780
aacaataagg gctttcgttt tgtaggcact ttcgtggggt ttttccccat ttttgccact    840
ccggctaatc aaaatgatat tatcctattc caacaagtgc cattaggaaa gagcgggcaa    900
acttatttct tccgcactcg tttttactat aacaagtggc aatttggggg tagcgtctat    960
aaaaatatcg gtaacgctaa tggcgatata ggtatttatg gcgaccctct agggtataac   1020
atttggacga atagtattta tgacgcagaa attaacaata tcgttggcgc tgatgttatt   1080
aacgggtttt tatatgtagg ctcgcagtat agggggttta gttggaaaat tttaggccgt   1140
tggacggata gcccaagggc tgatgaaagg agtctcgcgc tctttttgag ttatttttct   1200
aataagtata atattagaat ggatttgaaa ctagaatatt atggcaatat caccaaaaaa   1260
ggctattgta ttgggtattg tggcatgtat gttccagttg atcctaatgg gcctggcacg   1320
caacctttaa cacacaacgt gtattctgac aggagccata tcatgtttaa cattacttat   1380
ggttttagga tttactag                                                 1398
```

<212> Type : DNA
<211> Length : 1398
SequenceName : SEQ ID 499
SequenceDescription :
Sequence
--------
<213> OrganismName : Helicobacter pylori, strain J99
<400> PreSequenceString :

```
atgaaaaaga caattctact ctctctctct ctctctctcg cttcatcgct cttgcatgct 60
gaagacaacg gctttttttgt gagcgcgggc tatcaaatcg gcgaagcggt gcaaatggtc 120
aaaaacaccg gcgaattgaa aaacttgaac gacaaatacg agcagttaag ccaatcttta 180
gcccaactgg cttcgttaaa aaaaagcatt caaacggcga acaacattca ggctgtcaac 240
aatgctttaa gcgatttaaa aagctttgcg agtaacaacc acacaaacaa agaaacatcg 300

cccatctaca acaccgcgca agctgttatc acttcagtat tggctttttg gagtctttat 360
gcagggaacg ctctcagttt tcatgtgacc ggtttgaatg atggatctaa ttctccttta 420
ggaagaatcc atagagatgg gaactgcaca ggattacaac aatgttttat gagcaaagaa 480
acttatgata aaatgaagac acttgccgaa aacctccaaa aagctcaagg caatctctgt 540
gccttatcag aatgctctag caatcaatca aatggaggca aaacttccat gactacagct 600
cttcaaaccg cgcaacagct catggactta atcgaacaga ccaaggtttc tatggtgtgg 660
aaaaatatcg tcatcgcagg tgttacaaac aaacccaatg gtgctggcgc tatcacatcc 720
actggtcatg taaccgacta tgcggtgttt aacaacatca aggcgatgct acctatcttg 780
caacaagcgc ttacgctttc tcaaagtaac cacaccctat ccactcagtt gcaagctcga 840
gctatgggat ctcaaacaaa tcgtgaattc gctaaagaca tctacgcttt agctcaaaac 900
caaaagcaaa tcctttctaa cgcttcaagt atcttcaatc tctttaattc cattcctaaa 960
gaccaactta agtatttgga gaacgcttac ttgaaagtgc cacatttggg taaaacccct 1020
actaaccctt acagacagaa tgtgaatttg aataaagaaa ttaatgcggt tcaagacaat 1080
gtagctaatt atggtaatcg tttggattcg gctttaagcg tggctaaaga tgtttataac 1140
ctaaaatcca atcaaacaga gatcgtaacc acttataacg atgctaagaa tttgagcgaa 1200
gagatttcta aacttcccta taaccaagtc aatgtaacaa acatcgttat gtcgcctaaa 1260
gattctacag cgggccaata ccaaatcaac ccagagcagc aatccaatct taaccaagct 1320
ttagcggcga tgagcaataa ccccttttaaa aaagtgggca tgatcagctc tcaaaacaat 1380
aacggcgctt tgaacgggct tggcgtgcaa gtgggttata aacaattctt tggcgaaagc 1440
aaaagatggg ggttaaggta ttatggtttc tttgattaca accacggcta tatcaaatcc 1500
agcttttttta attcttcttc tgatatatgg acttatggcg gtgggagcga tttgttagtg 1560
aattttatca acgatagcat cacaagaaag aacaacaagc tttctgtggg tctttttggt 1620
ggtatccaac tagcagggac tacatggctt aattctcaat acatgaattt aacagcgttc 1680
aataaccctt acagcgcgaa agtcaatgct tccaatttcc aatttttgtt caatctcggc 1740
ttgaggacga atctcgctac agctaagaaa aaagacagcg aacgttccgc gcaacatggc 1800
gttgaactgg gcattaaaat ccctaccatt aacaccaatt attattcttt tctaggcact 1860
aagctagaat accgaaggct ttatagcgtg tatctcaatt atgtgtttgc ttattaa 1917
```

<212> Type : DNA
<211> Length : 1917
SequenceName : SEQ ID 500
SequenceDescription :
Sequence
--------
<213> OrganismName : Helicobacter pylori, strain J99
<400> PreSequenceString :

```
atgaaattaa aaaaacgaaa agttgcggct acattgctaa agcgtttgac cttgccacta       60
ttgttcacta cgggttcatt aggggcggtt acttatgaag tgcatgggga ttttatcaac      120
ttctccaaag tgggttttaa ccgttcgcct attaaccctg ttaaaggtat ctatcctaca      180
gaaacttttg ttaaccttac gggtaagcta gaggggtctg tgcatttagg taggggatgg      240
accgtgaatg taggcggtgt tttgggcgga caagtttatg ataacactag gtatgatagg      300
tgggcaaagg attttacccc cccaagctat tgggataaaa cttcttgcgg cactgattct      360
ttgagccttt gtatgaatgc gactaaaatg tggcaacagc aagggccagg tggtatcatt      420
gaccctaggg gtattggcta tatgtatatg ggtgagtgga acggcttgtt ccctaattac      480
tatccggcta acgcctactt gcccgggcat tcaaggcgct atgaagttta taaagcgaat      540
cttacctatg acagcgacag agtccatatg gtaatggggc gctttgatgt taccgagcag      600
gagcaaatgg attggattta ccaattgttc caaggttttt atgggacttt caagcttact      660
aagaacatga aattcttgct ctttagctct tggggtcgtg gtatcgctga tggtcaatgg      720
ttgttcccta tctatcgtga aaagccttgg ggtattcata aggcgggtat tatttatcgc      780
cctacaaaga atctaatgat ccacccttat gtgtatctca tcccaatggt aggtacattg      840
cccggtgcta aaatagaata cgataccaat cctgagttta gcggtagagg tataaggaat      900
aaaacgactt tctatgtgtt gtatgactat cgttggaata acgctgaata cggccgttac      960
gcacccgctc gttataacac ttgggatccg ttcttggata atggtaagtg gcgtggcttg     1020
caaggtcctg gtggtgcgac gctctatttg caccaccaca tagacattaa caactacttt     1080
gtggttggtg gtgcttacct caacatcggt aaccctaaca tgaacttagg tacttggggt     1140
aaccctgtgg ctcttgatgg tatcgaacaa tgggtcggtg gcatctacag cttaggcttt     1200
gcggggattg acaacattac cgatgctgat gcgttcactg agtatgttaa aggtggaggt     1260
aagcatggta agttcagttg gagcgtttat cagcgcttca ctaccgcacc aagggctttg     1320
gaatatggta ttggtatgta tctagactat cagttcagca agcatgttaa agcgggtctc     1380
aaactcgtgt ggttagagtt ccaaatccgt gcgggttaca accctggaac cggtttcctt     1440
gggccaaacg gtcagccgct caacttgaat aatggtttgt ttgaatcttc ggcgttcgcg     1500
caaggccctc aaaacatggg tggtatcgca aaaagcatta ctcaagacag aagccatttg     1560
atgacacaca tcagttatag tttctaa                                         1587
```

<212> Type : DNA
<211> Length : 1587
SequenceName : SEQ ID 501
SequenceDescription :
Sequence
--------
<213> OrganismName : Helicobacter pylori, strain J99
<400> PreSequenceString :

```
atgaaaaact tttccccact ctattgtctt aaaaagctca aaaaacgcca tttaatcgct       60
ctgagtctgc ccttgctttc ttatgcgaat ggctttaaaa tccaagagca aagcttgaat      120
ggcacggctt taggctcggc gtatgtcgct ggggctaggg gtgctgacgc ttctttttac      180
aacccggcta acatgggctt tactaacgat tggggcgaaa acagaagcga atttgaaatg      240
accaccaccg tgatcaatat cccggccttt agctttaaag tccctacgac caatcaaggc      300
ttatattcgg taacaagttt agaaattgat aaaagccaac aaaatatttt aggcatcatc      360
aacactatag ggttaggcaa tatccttaaa gcgcttggca atacggccgc taccaatggc      420
ttatcacaag ctatcaatcg tgttcaaggg cttatgaact taaccaatca aaaagtcgta      480
accctcgctt caaaacctga cactcaaatc gtgaatggct ggacaggcac gactaatttt      540
gttttaccta aattctttta taaaacgcgc acgcataacg gcttcacttt tgggggggagt      600
tttaccgctc ctagtgggtt gggtatgaaa tggaatggta agggggggga atttttgcat      660
gacgtgttta tcatgatggt agagcttgcc cctagcatga gttatactat taataaacgc      720
ttttctgtgg gtgtgggttt aagggggctt tatgcgaccg ggagctttaa taacaccgtt      780
tatgtgcctt tagagggcgc ttcagttttg agcgcggagc aaatcttaaa cttacccaac      840
aatgtttttg ccgatcaagt gccaagtaac atgatgactt tattaggcaa tattggctac      900
caaccagcgc ttaattgcca aaaagccggt ggggacatga gtgatcagag ctgtcaagag      960
ttttacaacg gcttgaaaaa aatcatgggt tatagcggtt taatcaaagc gagcgcgaat     1020
ctttatggca cgactcaagt cgtgcaaaaa tctaacggac aaggcgtatc gggggggtat     1080
agagtgggtt cgagtttgcg tgtgtttgat catggcatgt tttctgtggt gtataattct     1140
tcagttacct ttaacatgaa aggcggtttg gtggctatca cagagcttgg cccttcttta     1200
gggagcgttt tgactaaagg cagcttgaat atcaatgttt cactccccca aactttaagc     1260
ttagcctacg cccaccaatt ttttaaagat cgcctaaggg ttgaaggggt gtttgagcgc     1320
actttttgga gtcaagggaa taaatttttta gtcacccctg attttgcgaa cgccacttac     1380
aagggcttga gcgggacggt ggcttccttg gactctgaaa cgcttaaaaa aatggtaggc     1440
ctagcgaatt ttaaaagcgt gatgaacatg gggctggct ggagggacac caacaccttt     1500
agattagggg taacttacat gggtaaaagc ttgcgtttaa tgggcgctat tgattatgat     1560
caagccccaa gcccccaaga cgcgataggc attccggact ctaatggcta taccgtggct     1620
tttgggacta aatacaattt taggggcttt gatttgggcg tagcggggag tttcactttt     1680
aagagcaacc gctccagttt gtatcaatcc ccaactattg ggcaattgag aatctttagc     1740
gcctctttag gctatcgctg gtaa                                            1764
```

<212> Type : DNA
<211> Length : 1764
SequenceName : SEQ ID 502
SequenceDescription :
Sequence
--------
<213> OrganismName : Helicobacter pylori, strain J99
<400> PreSequenceString :

```
atggcttttc aggtcaatac aaatatcaat gcgatgaatg cgcatgtgca atccgcactc     60
actcaaaacg cacttaaaac ttcattggag cgattgagtt caggtttaag gatcaataaa    120
gcggctgatg acgcatcagg catgacggtg gcggattctt tgcgttcgca agcgagcagt    180
ttgggtcaag cgattgccaa cacgaatgac ggcatgggga ttatccaggt tgcggataag    240
gctatggatg agcaattaaa aatcttagac accgttaagg ttaaagcgac tcaagcggct    300
caagatgggc aaactacgga atctcgtaaa gcgattcaat ctgacatcgt tcgtttgatt    360
caaggtttgg ataatatcgg taacacaacg acttataacg ggcaagcgtt attgtctggt    420
caattcacta acaaagaatt ccaagtaggg gcttattcta accaaagcat taaggcttct    480
atcggctcta ccacttccga taaaatcggt caggttcgta tcgctacagg cgcgttaatc    540
acggcttctg ggatattag cttgactttt aaacaagtgg atggcgtgaa tgatgtaact     600
ttagagagcg taaaagtttc tagttcagca ggcacaggga tcggcgtgtt agcagaagtg    660
atcaataaaa actctaaccg aacagggtt aaagcttatg cgagcgttat caccacgagc     720
gatgtggcgg tccagtcagg aagtttgagt aatttaacct taaatgggat tcatttgggt    780
aatatcgcag atattaagaa aaacgactca gacggaaggt tagtcgcagc gatcaatgcg    840
gtcacttcag aaaccggtgt ggaagcttat acggatcaaa aagggcgctt gaatttgcgc    900
agtatagatg gtcgtgggat tgaaatcaaa accgacagcg tcagtaacgg gcctagcgct    960
ttaacgatgg tcaatggcgg tcaggattta acaaaaggct ctactaacta cggaaggctt   1020
tctctcacac gattagacgc taagagcatc aatgtcgttt cggcttctga ctcacagcat   1080
ttaggcttca cggcgattgg ttttggggaa tctcaagtgg cagaaaccac ggtgaatttg   1140
cgcgatgtta ctgggaattt taacgctaat gtcaaatcag ccagtggcgc gaactataac   1200
gccgtgatcg ctagcggtaa ccaaagcttg ggatctgggg ttacaacctt aagaggcgcg   1260
atggtggtga ttgacattgc cgaatcagcg atgaaaatgt tggataaagt ccgctctgac   1320

ttaggttctg tgcaaaatca aatgattagc actgtgaata acatcagcat cactcaagtg   1380
aatgttaaag cggctgaatc tcaaatcagg gatgtggatt tcgctgaaga gagcgcgaat   1440
ttcaacaaaa acaacatttt ggcgcaatca ggcagctatg cgatgagtca agccaatacc   1500
gttcaacaaa atatcttaag gcttttaact tag                                 1533
```

<212> Type : DNA
<211> Length : 1533
SequenceName : SEQ ID 503
SequenceDescription :
Sequence
--------
<213> OrganismName : Helicobacter pylori, strain J99
<400> PreSequenceString :

```
atggcaggca cacaagctat atatgaatca tcttctgcag gattcttatc gcaagtctcc      60
tcaatcatct caagcacaag tggtgtcgca gggccatttg caggaatagt agcgggcgct     120
atgacagcag cgattattcc tattgttgtg ggatttacta atccgcaaat gaccgctatc     180
atgacccaat acaatcaaag catcgctgaa gctgtaagcg tgcctatgaa agccgctaac     240
caacaataca accaattgta tcaaggtttt aacgatcaaa gcatggctgt ggggaacaat     300
atcttaaata tcagcaaatt aacaggggaa tttaacgcgc aaggcaacac gcaaagcgcg     360
caaattagtg ctgtcaatag tcagattgca agcattttag cgagtaacac taccccctaaa    420
aatcctagcg ctattgaagc ttatgcgacg aatcaaatcg ctgttcctag cgtgccaaca     480
acggttgaaa tgatgagcgg tatattaggc aatattacaa gcgcagcacc aaaatacgcc     540
ctagctctac aagagcaact gcgttctcaa gcaagcaaca gctcaatgaa tgatacagcc     600
gattcccttg atagctgtac cgctttaggc gcacttgttg gctcatcaaa agtgtttttc     660
agttgcatgc aaatttctat gactcctatg agtgtttcta tgcccactgt ttatgccaaa     720
taccaagcgg ttgccactaa agccctaact tcaggcgtta atcctatgac cactcctgca     780
tgccctattg ggacaaggt tcttgccgtt tattgctatg ctgaaaaagt agcagaaatt      840
ttgagagaat actatataga atttgtgaaa aacaatacca atttgttgca gaacgcttct     900
caaatgatac ttaatcaatc aggattagct actagcacct atgacactca agcgatttct     960
aacataagct cgctatataa ttacaatata gtagcgaata aatctttttt gaaatcgcat    1020
ttgacttatc ttgattacat caaagacaag cttaaggggc aaaaagatag ctacttaaca    1080
gaaagggtgc agactaaaat aatcgtgaag tga                                 1113
```

<212> Type : DNA
<211> Length : 1113
SequenceName : SEQ ID 504
SequenceDescription :
Sequence
--------
<213> OrganismName : Helicobacter pylori, strain J99
<400> PreSequenceString :

```
atgactaacg aagccattaa ccaacaacca caaaccgaag cggcttttaa cccgcagcaa      60
tttatcaata atcttcaagt ggcttttatt aaagttgata atgttgtcgc ttcatttgat     120
cctaatcaaa aaccaatcgt tgataagaat gatagggata ataggcaagc ttttgagaaa     180
atctcgcagc taagggagga attcgctaat aaagcgatca aaaatcctac caaaaagaat     240
cagtattttt caagctttat cagtaagagc aatgatttaa tcgacaaaga caatctcatt     300
gatacaggtt cttccataaa gagctttcag aaatttggga ctcagcgtta ccaaattttt     360
atgaattggg tgtcccatca aaacgatccg tctaaaatca acacccaaaa aatccgaggt     420
tttatggaaa atatcataca accccctatc tctgatgata aagagaaagc ggagtttttg     480
aggtctgcca aacaagcttt tgcaggaatt atcataggaa accaaatccg atcggatcaa     540
aaattcatgg gcgtgtttga tgaatctttg aaagagaggc aagaagcaga aaaaaatgga     600
gagcctaatg gagatcctac tggtggggat tggcttgata ttttttatc atttgtgttt      660
aacaaaaaac aatcttccga tctcaaagaa acgctcaatc aagaaccagt tcctcatgtc     720
caaccagatg tagccactac caccactgac atacaaagct taccgcctga agctagggat     780
ttgcttgatg aaaggggtaa tttttctaaa ttcactcttg gcgatatgaa catgttagat     840
gttgagggag tcgctgacat tgatcctaat tacaagttca accaattatt gatccacaat     900
aacgctctgt cttctgtgtt aatggggagt cataatggca tagaacctga aaaagtttca     960
ttgttgtatg gaaacaatgg tggtcctgaa gctaggcatg attggaacgc caccgttggt    1020
tataaaaacc aacgaggcga caatgtggct acactcatta atgtgcatat gaaaaatggc    1080
agtgggttag tcatagcagg tggtgagaaa gggattaaca accctagttt ttatctctac    1140
aaagaagacc aactcacagg ctcacaacga gcattgagtc aagaagagat ccaaaacaaa    1200
gtggatttca tggaatttct tgcacaaaat aatgctaaat tagacaactt gagcaagaaa    1260
gagaaagaaa aattccaaaa tgagattgaa gattttcaaa aagactctaa ggcttattta    1320
gacgccctag ggaatgatca cattgctttt gtttctaaaa aagacaaaaa acatttagct    1380
ttagttgctg agtttggtaa tggggaattg agctacactc tcaaagatta tgggaaaaaa    1440
gcagataaag ctttagatag ggaggcaaaa accactcttc aaggtagcct aaaacatgat    1500
ggcgtgatgt ttgttgatta ttctaatttc aaatacacca acgcctccaa gagtcctgat    1560
```

```
aagggtgtgg gtgctacgaa tggcgtttcc catttagaag caggctttag caaggtagct    1620
gtctttaatt tgcctaattt aaataatctc gctatcacta gtgtcgtaag gcaggattta    1680
gaggataaac taatcgctaa aggattgtcc ccacaagaag ctaataagct tgtcaaagat    1740
tttttgagca gcaacaaaga attggttgga aaagctttaa acttcaataa agctgtagct    1800
gaagctaaaa acacaggcaa ctatgacgag gtgaaacaag ctcagaaaga tcttgaaaaa    1860
tctctaaaga aacgagagcg tttggagaaa gatgtagcga aaaatttgga gagcaaaagc    1920
ggcaacaaaa ataaaatgga agcaaaatct caagctaaca gccaaaaaga tgagattttt    1980
gcgttgatca ataaagaggc taatagggat gcaagagcaa tcgcttacgc tcagaatctt    2040
aaaggcatca aaagggaatt gtctgataaa cttgaaaata tcaacaagga tttgaaagac    2100
tttagtaaat cttttgatga attcaaaaat ggcaaaaata aggatttcag caaggcagaa    2160
gaaacactaa aagcccttaa aggctcggtg aaagatttag gtatcaatcc agaatggatt    2220
tcaaaagttg aaaaccttaa tgcagctttg aatgaattca aaaatggcaa aaataaggat    2280
ttcagcaagg taacgcaagc aaaaagcgac cttgaaaatt ccattaaaga tgtgatcatc    2340
aatcaaaaga taacggataa agttgataat ctcaatcaag cggtatcagt ggctaaagca    2400
acgggtgatt tcagtggggt agagcaagcg ttagccgatc tcaaaaattt ctcaaaggag    2460
caattggctc aacaagctca aaaaaatgaa gatttcaata ctggaaaaaa ttctgcacta    2520
taccaatccg ttaagaatgg tgtaaacgga accctagtcg gtaatgggtt atctaaagca    2580
gaagccacaa ctctttctaa aaacttttcg gacatcaaga aagagttgaa tgcaaaactt    2640
ggaaatttca ataacaataa caataatgga ctcgaaaaca gcacagaacc catttatact    2700
caagttgcta aaaaggtaaa agcaaaaatt gaccgactcg atcaaatagc aagtggtttg    2760
ggtgatgtag ggcaagcagc gagcttcctt ttgaaaaggc atgataaagt tgatgatctc    2820
agtaaggtag ggctttcagc taaccatgaa cccatttacg ctacgattga tgatctcggc    2880
ggacctttcc ctttgaaaag gcatgataaa gttgatgatc tcagtaaggt agggctttca    2940
agggagcaaa aattgactca gaaaattgac aatctcaacc aggcggtatc agaagctaaa    3000
gcaagtcatt ttgacaacct agatcaaatg atagacaagc tcaaagattc tacaaaaaag    3060
aatgttgtga atctatatgt tgaaagtgca aaaaaagtgc ctactagttt gtcagcgaaa    3120
ttggacaatt acgctactaa cagccacaca cgcattaata gcaatgtcaa aaatggaaca    3180
atcaatgaaa aagcgaccgg catgctaacg caaaaaaatt ctgagtggct caagctcgtg    3240
aatgataaga tagttgcgca taatgtggga agtgctcctt tgtcagcgta tgataaaatt    3300
ggattcaacc aaaagaatat gaaagattat tctgattcgt tcaagttttc caccaggttg    3360
agcaatgccg taaaagacat taagtctggc tttgtgcaat ttttaaccaa tatattttct    3420
atgggatctt acagcttgat gaaagcaagt gtggaacatg gagtcaaaaa tactaataca    3480
aaaggtggtt tccaaaaatc ttaa                                           3504
```

<212> Type : DNA
<211> Length : 3504
SequenceName : SEQ ID 505
SequenceDescription :
Sequence
--------
<213> OrganismName : Helicobacter pylori, strain J99
<400> PreSequenceString :

```
atgaaaacaa atggtcattt taaggatttt gcatggaaaa aatgcttttt aggcgcgagc      60
gtggtggctt tattagtggg gtgtagcccg catattattg aaaccaatga agttgctttg     120
aaattgaatt accatccagc tagcgagaaa gttcaagcgt tagatgaaaa gattttactt     180
ttaaggccag ctttccaata cagcgataat attgctaaag agtatgaaaa caaattcaag     240
aatcaaacca cgcttaaagt tgaagagatc ttgcaaaatc agggctataa ggttattaat     300
gtggatagca gcgataaaga cgatttttct tttgcgcaaa aaaaagaagg gtatttggct     360
gtcgctatga atggcgaaat tgttttacgc cccgatccta aaggaccat acagaaaaaa     420
tcagaacccg ggttattatt ctccactggt ttggataaaa tggaaggggt tttaatcccg     480
gctgggtttg tcaaggttac catactagag cctatgagtg gggaatcttt ggattctttt     540
acgatggatt tgagcgagtt ggacatccaa gaaaaattct taaaaaccac ccattcaagc     600
catagcggag ggttagttag cactatggtt aagggacgg ataattctaa tgacgcaatt     660
aagagcgctt tgaataagat ttttgcaagt atcatgcaag aaatggataa gaaactcact     720
caaaggaatt tagaatctta tcaaaaagac gccaaggaat taaaaaacaa gagaaaccga     780
taa                                                                  783
```

<212> Type : DNA
<211> Length : 783
SequenceName : SEQ ID 506
SequenceDescription :
Sequence
--------
<213> OrganismName : Mycoplasma pneumoniae
<400> PreSequenceString :

```
atgaaatcga agctaaagtt aaaacgttat ttactgtttt taccactttt accgctaggg      60
acgttgtcac tagccaacac ctacctcctc caagaccaca acaccctcac cccctacacg     120
ccctttacga caccgctcaa tggggggctg gatgtcgtgc gcgccgccca tttacacccc     180
tcatacgaac tcgtggactg aaagcgggtg ggggatacca agttggtggc gctggtccgc     240
tcagcgttgg tcagggtgaa attccaggac acaacgagtt cggatcaaag taataccaac     300
caaaatgcct tgagttttga tacccaagaa tcacagaagg cacttaatgg ctcgcagagt     360
ggatcttctg acacttccgg gtctaactcc caagacttcg ccagctatgt cctcatcttt     420
aaagccgcgc ccagggccac gtgggtgttt gaacgcaaga ttaagttggc gttgccctac     480
gttaagcagg aaagtcaggg ttccggcgat caaggttcca atggtaaggg ctccctctac     540
aaaaccctcc aagacctcct cgtcgaacaa cccgtgaccc cttacacccc gaatgcgggg     600
ttagcccggg tgaatggggt tgctcaggat acggttcatt ttggttcggg tcaagaatcg     660
agttggaatt cccaacgttc ccaaaaaggc cttaaaaaca accccggacc caaagccgtc     720
accggcttta agctcgataa gggccgcgcg taccggaagc tgaatgaaag ttgaccggtg     780
tatgaacccc tggattcgac caaggagggg aaggggaagg atgagagctc ttggaaaaat     840
tcggaaaaaa caacagcgga aaatgatgcc ccgttggtgg ggatggttgg aagtggtgcg     900
gctggaagtg cttctagttt acaaggcaat ggctcgaaca gttcggggtt aaaatcgctc     960
ttgagatcag cacctgtcag tgttccacca agcagtacaa gtaatcaaac tttaagctta    1020
tctaaccccg ctcctgtggg cccacaagcg gttgtaagcc aacccgcggg gggtgctacg    1080
gcagcagtgt ccgtcaatcg cacagcgagt gacaccgcca cctttagcaa gtacctcaac    1140
accgcccagg ccttgcacca gatgggggtg attgttccgg ggttggaaaa atgaggtggt    1200
aacaacggta cgggtgtagt ggctagccga caggatgcta cttccactaa cctgccccat    1260
gcggcaggtg cttcccaaac gggtttggga actggttcgc cccgcgaacc agctttaacc    1320
gcaacgtcac agcgtgccgt cacggtggtt gctggccccc ttcgtgcggg caatagcagt    1380
gaaactgatg ccctaccgaa tgtcatcacc cagctctatc atacttcaac cgcccaactc    1440
gcttacttaa atggccagat cgttgtgatg ggttccgacc gggtaccgag tctttggtat    1500
tgagttgtcg gggaggacca ggaatcgggc aaagcgacct gatgagcgaa aaccgagctc    1560
aactggggca ccgacaagca gaagcagttt gtcgaaaacc agttggggtt taaagatgac    1620
tcaaattcgg attccaaaaa ttcgaatttg aaggcccaag gcctcaccca acccgcctac    1680
ctcatcgccg gtcttgacgt tgtggccgac cacctcgtct ttgcggcctt taaagcgggc    1740
gcggtggggt atgatatgac gactgattcg agcgcttcga cctacaacca agcactcgcc    1800
tggtcgacca cggccgggtt ggacagtgat ggggggtaca aggccttggt ggaaaacacg    1860
gccgggctca acggcccgat taatggcttg tttaccctgc tcgacacctt tgcgtatgtg    1920
acccccgtga gtgggatgaa agggggggagt cagaataatg aagaagtgca aacgacttac   1980
ccggtcaagt ccgaccaaaa ggccaccgcc aaaattgcct ccttaattaa tgccagccca    2040
ctcaacagtt atggggatga tggggtgacc gtgtttgatg ccctgggcct taactttaac    2100
tttaagttga acgaggagcg cttgccatcg cgcaccgacc aactgcttgt gtatgggatt    2160
gtaaacgaaa gtgaactgaa gtccgcacgg gaaaatgccc agtcgacctc cgatgataat    2220
tcaaacacca aagtcaagtg aaccaacacc gcctcgcact acctccccgt gccgtattac    2280
tacagtgcca atttccccga agcgggtaac agaaggcgag cggagcagcg gaatggggtg    2340
aagattagca ccttggaatc gcaagccact gatggctttg ccaactcgtt acttaacttt    2400
ggtaccggtc ttaaagccgg tgttgaccca gctccagtag cacggggtca taaaccgaac    2460
tatagtgcag tactactagt gcgtggtggc gttgtaaggt taaactttaa ccccgatact    2520
gataaactgt tggattctac tgacaaaaac agtgaaccta tctccttctc ctatacccca    2580
tttgggtctg ctgaaagtgc cgtagacctc accacgttga aggatgtgac ctatattgct    2640
gaaagtggtc tgtggttcta tacctttgac aatggtgaaa aaccaacgta cgatggtaaa    2700
caacaacagg tcaaaaaccg caagggttat gctgtgatta ccgtatcacg taccggaatt    2760
gaatttaacg aggacgctaa taccacaacc ttaagccaag ccccagctgc tttggctgtc    2820
caaaacggga ttgcttccag tcaggacgac ctcacaggca tcctaccgtt atccgatgag    2880
ttctccgctg tgattaccaa ggatcaaaca tggaccggta aggttgatat ctataagaac    2940
accaacgggt tgtttgaaaa ggatgatcag ctatcggaaa acgtgaagag gcgtgacaac    3000
ggtttggtcc ctatttacaa cgaaggtatc gtcgatattt ggggcagagt ggattttgct    3060
gccaacagtg ttttgcaagc gcgtaacctc actgataaaa cggttgatga ggtgatcaat    3120
aaccccgata tcctccaaag cttctttaag tttaccccag cctttgataa ccaaagagca    3180
atgctagtgg gggaaaagac atcggatact accttaacgg ttaaaccgaa gattgagtac    3240
ttggatggta acttctatgg tgaggattcc aagattgctg gaattccgct caacattgat    3300
ttcccttccc ggatttttgc tggctttgct gctttaccgt cctgggtcat tccggtatca    3360
gtcggttcat cggtgggcat tctcttaatc ctgctcatct taggccttgg tattggaatt    3420
ccaatgtata aggtccgcaa gcttcaagac tccagctttg ttgatgtgtt taaaaaggtg    3480
gatacgttga caaccgctgt gggtagcgtg tacaagaaga ttatcaccca aacgagtgtg    3540
atcaaaaaag ctcctagtgc gttgaaagct gctaataacg ctgctcctaa agcaccagtt    3600
aaaccagctg ctccaacagc tccaagacca ccagtccaac cacctaaaaa ggcttaa       3657
```

<212> Type : DNA
<211> Length : 3657
SequenceName : SEQ ID 507
SequenceDescription :

Sequence
--------
<213> OrganismName : Mycoplasma pneumoniae
<400> PreSequenceString :

atgcaccaaa ccaaaaaaac tgccttgtcc aagtccactt ggattctcat cctcaccgcc 60
accgcctccc tcgcgacggg actcaccgta gtgggacact tcacaagtac caccacgacg 120
ctcaagcgcc agcaatttag ctacacccgc cctgacgagg tcgcgctgcg ccacaccaat 180
gccatcaacc cgcgcttaac cccgtgaacg tatcgtaaca cgagcttttc ctccctcccc 240
ctcacgggtg aaaatcccgg ggcgtgggcc ttagtgcgcg acaacagcgc taagggcatc 300
actgccggca gtggcagtca acaaaccacg tatgatccca cccgaaccga agcggctttg 360
accgcatcaa ccacctttgc gttacgccgg tatgacctcg ccgggcgcgc cttatacgac 420
ctcgattttt cgaagttaaa cccgcaaacg cccacgcgcg accaaaccgg gcagatcacc 480
tttaacccct ttggcggctt tggtttgagt ggggctgcac cccaacagtg aaacgaggtc 540
aaaaacaagg tccccgtcga ggtggcgcaa gacccctcca atccctaccg gtttgccgtt 600
ttactcgtgc cgcgcagcgt ggtgtactat gagcagttgc aaaggggggtt gggcttacca 660
cagcagcgaa ccgagagtgg tcaaaatact tccaccaccg gggcaatgtt tggcttgaag 720
gtgaagaacg ccgaggcgga caccgcgaag agcaatgaaa aactccaggg cgctgaggcc 780
actggttctt caaccacatc tggatctggc caatccaccc aacgtggggg ttcgtcaggg 840
gacaccaaag tcaaggcttt aaaaatagag gtgaaaaaga aatcggactc ggaggacaat 900
ggtcagctgc agttagaaaa aaatgatctc gccaacgctc ccattaagcg gagcgaggag 960
tcgggtcagt ccgtccaact caaggcggac gattttggta ctgccctttc cagttcggga 1020
tcaggcggca actccaatcc cggttccccc accccctgaa ggccgtggct tgcgactgag 1080
caaattcaca aggacctccc caaatgatcc gcctcgatcc tgattctgta cgatgcgcct 1140
tatgcgcgca accgtaccgc cattgaccgc gttgatcact tggatcccaa ggccatgacc 1200
gcgaactatc cgcccagttg aagaacgccc aagtgaaacc accacggttt gtgggactga 1260
aaggcgcgcg atgttttgct ccaaaccacc gggttcttca acccgcgccg ccacccccgag 1320
tggtttgatg gcgggcagac ggtcgcggat aacgaaaaga ccgggtttga tgtggataac 1380
tctgaaaaca ccaagcaggg ctttcaaaag gaagctgact ccgacaagtc ggccccgatc 1440
gccctcccgt ttgaagcgta cttcgccaac attggcaacc tcacctggtt cgggcaagcg 1500
cttttggtgt ttggtggcaa tggccatgtt accaagtcgg cccacaccgc gcctttgagt 1560
ataggtgtct ttagggtgcg ctataatgca actggtacca gtgctactgt aactggttga 1620
ccatatgcct tactgttctc aggcatggtc aacaaacaaa ctgacgggtt aaaggatcta 1680
ccctttaaca ataaccgctg gtttgaatat gtaccacgga tggcagttgc tggcgctaag 1740
ttcgttggta gggaactcgt tttagcgggt accattacca tgggtgatac cgctaccgta 1800
cctcgcttac tgtacgatga acttgaaagc aacctgaact tagtagcgca aggccaaggt 1860
cttttacgcg aagacttgca actcttcaca ccctacggat gagccaatcg tccggattta 1920
ccaatcgggg cttgaagtag tagtagtagt agtagtcaca acgcacccta ctacttccac 1980
aataaccccg attgacaaga ccgtccaatc caaaatgtgg ttgatgcctt tattaagccc 2040
tgagaggaca agaacggtaa ggatgatgcc aaatacatct accccttaccg ttacagtggc 2100
atgtgagctt gacaggtata caactggtcc aataagctca ctgaccaacc attaagtgct 2160
gactttgtca atgagaatgc ttaccaacca aactccttgt ttgctgctat tctcaatccg 2220
gaattgttag cagctcttcc cgacaaggtt aaatacggta aggaaaacga gtttgctgct 2280
aacgagtacg agcgctttaa ccagaagtta acggtagctc ctacccaagg aacaaactga 2340
tcccacttct cccccacgct ttcccgtttc tccaccgggt tcaaccttgt ggggtcggtg 2400
ctcgaccagg tgttggatta tgtgccctgg attgggaatg ggtacaggta tggcaataac 2460
caccggggcg tggatgatat aaccgcgcct caaaccagcg cggggtcgtc cagcggaatt 2520
agtacgaaca caagtggttc gcgttccttt ctcccgacgt tttccaacat cggcgtcggc 2580
ctcaaagcga atgtccaagc caccctcggg ggcagtcaga cgatgattac aggcggttcg 2640
cctcgaagaa ccctcgacca agccaacctc cagctctgaa cgggggcggg gtgaaggaat 2700
gataaggctt caagtggaca aagtgacgaa aaccacacca agttcacgag cgctacgggg 2760
atggaccagc agggacaatc aggtacctcc gcggggaatc ccgactcgtt aaagcaggat 2820
aatattagta agagtgggga tagtttaacc acgcaggacg gcaatgcgat cgatcaacaa 2880
gaggccacca actacaccaa cctccccccc aacctcaccc ccaccgctga ttgaccgaac 2940
gcgctgtcat tcaccaacaa gaacaacgcg cagcgcgccc agctcttcct ccgcggcttg 3000
ttgggcagca tcccggtgtt ggtgaatcga agtgggtccg attccaacaa attccaagcc 3060
accgaccaaa aatggtccta caccgactta cattcggacc aaaccaaact gaacctcccc 3120
gcttacggtg aggtgaatgg gttgttgaat ccggcgttgg tggaaaccta ttttgggaac 3180
acgcgagcgg gtggttcggg gtccaacacg accagttcac ccggtatcgg ttttaaaatt 3240
cccgaacaaa ataatgattc caaagccacc ctgatcaccc ccgggttggc ttgaacgccc 3300
caggacgtcg gtaacctcgt tgtcagtggc accacggtga gcttccagct cggcgggtgg 3360
ctggtcacct tcacggactt tgtcaaaccc cgcgcgggtt acctcggtct ccagttaacg 3420
ggcttggatg caagtgatgc gacgcagcgc gccctcattt gggccccccg gccctgagcg 3480
gcctttcgtg gcagttgggt caaccggttg ggccgcgtgg agagtgtgtg ggatttgaag 3540
ggggtgtggg cggatcaagc tcagtccgac tcgcaaggat ctaccaccac cgcaacaagg 3600
aacgccttac cggagcaccc gaatgctttg gcctttcagg tgagtgtggt ggaagcgagt 3660
gcttacaagc caaacacgag ctccggccaa acccaatcca ctaacagttc cccctacctg 3720
cacttggtga agcctaagaa agttacccaa tccgacaagt tagacgacga tcttaaaaac 3780
ctgttggacc ccaaccaggt tcgcaccaag ctgcgccaaa gctttggtac agaccattcc 3840
acccagcccc agccccaatc gctcaaaaca acgacaccgg tatttgggac gagtagtggt 3900

```
aacctcagta gtgtgcttag tggtgggggt gctggagggg gttcttcagg ctcaggtcaa     3960
tctggcgtgg atctctcccc cgttgaaaaa gtgagtgggt ggcttgtggg gcagttacca     4020
agcacgagtg acggaaacac ctcctccacc aacaacctcg cgcctaatac taatacgggg     4080
aatgatgtgg tgggggttgg tcgactttct gaaagcaacg ccgcaaagat gaatgacgat     4140
gttgatggta ttgtacgcac cccactcgct gaactgttag atggggaagg acaaacagct     4200
gacactggtc cacaaagcgt gaagttcaag tctcctgacc aaattgactt caaccgcttg     4260
tttacccacc cagtcaccga tctgtttgat ccggtaacta tgttggtgta tgaccagtac     4320
ataccgctgt ttattgatat cccagcaagt gtgaacccta aaatggttcg tttaaaggtc     4380
ttgagctttg acaccaacga acagagctta ggtctccgct tagagttctt taaacctgat     4440
caagataccc aaccaaacaa caacgttcag gtcaatccga ataacggtga cttcttacca     4500
ctgttaacgg cctccagtca aggtccccaa accttgttta gtccgtttaa ccagtgacct     4560
gattacgtgt tgccgttagc gatcactgta cctattgttg tgattgtgct cagtgttacc     4620
ttaggacttg ccattggaat cccaatgcac aagaacaaac aggccttgaa ggctgggttt     4680
gcgctatcaa accaaaaggt tgatgtgttg accaaagcgg ttggtagtgt ctttaaggaa     4740
atcattaacc gcacaggtat cagtcaagcg ccaaaacgct tgaaacaaac cagtgcggct     4800
aaaccaggag caccccgccc accagtacca ccaaagccag ggctcctaa gccaccagtg      4860
caaccaccta aaaaacccgc ttag                                            4884
```

<212> Type : DNA
<211> Length : 4884
SequenceName : SEQ ID 508
SequenceDescription :
Sequence
--------
<213> OrganismName : Mycoplasma pneumoniae
<400> PreSequenceString :

```
atgggttata agttaaagcg atgaccttta gttgcgttta catttaccgg aattggccta      60
ggggtggtgc tagcggcgtg ttctgcactc aatacctcca atctgtttcc ccgtcaaaac     120
cgctccaagc agttgattgg gtttaccgaa aacaacatca ttaaacctga agctgtacta     180
aaagccgctt tagctgagga caatggtacg gaaaccattt taagggttaa ctttggtgaa     240
gcgttaaaga gttggtacca aaacaataag gatcgcaaca ttgctacccg tttaactatc     300
tttagtgaaa acgtggagga tgaacatgat aacctgttgg accaaaaaca acaggccgaa     360
ccgattaatt gaccaataga gctccaaaag gaatacgacc agtggggtgg gagtgaaagt     420
tcctgaaagg ccttaaagtt atacgaccgt ttaatagccg acttccaatc acttatcttt     480
agcaacattg ttgctaatgt gcagttgact gatggtagtg atcagtttaa acccactacc     540
aaagataacc tcgacagtac tagcaacaaa atcaagtttg ttaattccaa accaaatgat     600
cctaacgggg agttctttgc taacctccag gcttatctat ttgcccagtg agtagtggag     660
gaaaaccccc tccccctcac ccaagccttc ttcgcgtacc aagcacccaa ggacgggctc     720
gacagcttat acgaccaagc cgcgattggt agtgccttac agttgggcta tgccttcccc     780
gcgtttcgcg agcccaataa tggacaaagc caaggtaaaa ccaccttcga ccccaccccc     840
aacagtgccc aaaacttcgg ggatttcatt aaggcggtgt ttccggagca gaagaatggt     900
caaacccaac aatccaatac atcctcccgc accgggttgt ttgactggca aaccaagtga     960
aacaccaatg gcgctgccaa taagctcttg gtgaccaagt ccaacctgcg cggggccttt    1020
aagggcgtgg ggttagctac ggccattatt gaccagtacg agtacctggt gggtggaagt    1080
aaaacgagtt cgttgcccga ggtcaaggta gattcgaata agtccaacca aaatccgctg    1140
gatagttttt ttatggaggg gaaggatgcg gtggcgatta gatccatagt ttcgcgtgcg    1200
aaaattgcca tgaccgacca aacccccggg tttaaggtca acccggcgtt tgtcaaggtg    1260
aagcagagtc agcagaacga taccttttac caaaaccagc gcaaactgag cggcgggcaa    1320
agtggggata ataactccca gggtaaacac cactacctcc aagacgcggt gcggttgacg    1380
agtagtcaag caatggcggc agcttctact ggggcagatt ctagtagtgg taccaatgtt    1440
ggtggtagct ccgggggcaa ctctgtttta attccgctcc caaggagtgc cgcccttacc    1500
cacacccagc aacaagttca acaaaccacc tccacactcc aaacccctgt ttacgccccgt    1560
ggtgacgatg gtacatacgc cctggcaatt gacggtggtg attatttttt ggcgaacaac    1620
aaacgcgatt tcaccaaaca agccgacatc ttgctgtacc gctatttaca agctaagagt    1680
aataacttta aggaaaacgg cgtcgagttt agtttgaact tactggaatc tggcagcctg    1740
ttccaaacgt gagcacaaac ggggttaacc gcgaagttgt acggtgcttt ggtggcgatg    1800
atgggttcgg gtcaggggac ccaagttaag ggcagtgtgc aggggagtag ccgagcagct    1860
tctgttagcg ttcaaactac ccagcaaaac agacagcaat cgactgatac ccaagaatcg    1920
gaagtggtta agttagccaa gtccctgctg aaatcaagtg cggatttggc caaacccttc    1980
actgataacc ccacctttaa aaaggcctta accgacatcc aatccgagta caaggattat    2040
ttggctgctg cggggaagtt aagtgagttt aagaaagatt tgggggaggt gagtggctta    2100
cagcaagcta ttattgaccg cgctgataag tacattcaac tagaaaaaca agcccaaaag    2160
agtgcgattg gcttgggcca accgctccct taccaacgcg ctagtgatgg tagttacccc    2220
gctttagaga agttctttat tccggaagat agtgcagcag atggaaaagt aaaagcatct    2280
gaatcaggat ctgccgcttt agtaacacta aagacaacag atagtcaaaa aagcacaaac    2340
accgttaaac aaccggacat caaaccgacc cgggaaaaca acgacaaaaa gttaaagcag    2400
ttaactagtg atgtagaaac taaagcatct agtttaatca ccaaatgagg tgcaacaccc    2460

caaattggta gtcagttctc cgaaattgtt tcgctcaaga gtaaggacaa taagccccag    2520
accaacatga tcttggcctt attgtctgat gttgggatta agtgaaccaa gattctcaat    2580
tcctttaagg agtggttctt tactaatacc aacgacttta agaacaatta cgattctgaa    2640
aagaaagaac ttaaaggcaa tgagtacaag gactttaacg acctcgttaa acaaaccttg    2700
tacttacgtt cctgacaacg actcacttcc aaggaaaagt ttggttatta caaggagttg    2760
ggtagtgtaa aagcacaagc agctcaatca ggaatggttt cactttcaag tagcgcagca    2820
gttgcaaatg ctgtagcttc aagtggcatg caaaaatctg gtgatcaaac cctcttggaa    2880
ctcggtaaaa aagcgtttga aagtgaactg gaagcatcta gtagtgatgg gcagtacaag    2940
tacctgcgct tcttgtccac cttaatgtga ctagttaagg atggggcgaa gaactataag    3000
cgcttgctcc aacaagcgat taccgtgggt acccgtgcct ttgtgtcgtg aacggtgagc    3060
tatgatgata cggctacggc atcagcagcc gcagcaaaag cgcaagtagc agtattaaag    3120
acagctcagg caacaaatac tcaaagcgac aacccttca ataagtttgt ccaaaacccc    3180
gattatgtcc agggttcgga aaccaactga tttaacgata agagtacgcc aattaaaccc    3240
gattcgttac tggaaagcga aagcacttac aactttaccg cggaaccgtt tgatgataag    3300
acgaaaagtc aaaagaggtc tactggtggt accactaacg aaaaacactt ctttggcttt    3360
aacggtttaa cgattaactc gccccaaagt gtgtcgactg ctagtgccgg actcacggaa    3420
caaatcttta acaactttgg tcagttagta acaagtagtg acaaatccgg ggccttgagc    3480
cagtacaagg ataaagcaac cctgaaacgc ttaatccaaa acactaattc cgatgccgag    3540
ctcaatgcct ttggtgaagt gttgcaccgt gcagttaatg tcgataccag taacttagga    3600
cgttttaact ccagtgggga accgttgatt agctttgaca acaagaagaa gttcttggtt    3660
gatgtagtgg acaaactgga tgatgtttac tttaacaagt tcgaaggtta tgttggccaa    3720
accaaggtga agatgtctga ttcaagtagt agttcccaag gtacgaagac tataagaaag    3780
ccaaagccgc accactcacc aaggactcga gtgtcaagac tttgagctat gtcattcaga    3840
ttaccaacga ggacgttaac aaaatttctg ctagttgaaa aactgataag aacagtgctt    3900
tag                                                                  3903
```

<212> Type : DNA

<211> Length : 3903
SequenceName : SEQ ID 509
SequenceDescription :
Sequence
--------
<213> OrganismName : Mycoplasma pneumoniae
<400> PreSequenceString :

```
atgaaaaaac ttctaataaa accccagttt tgattcttaa cccttggtgg ctttatctct      60
tcaagtgtta ttttagttgc ctgtgctacc ccttccaatt cagcactcca aactgttttt     120
aaagcacgtt cgaaccagtt cttcaacggg gaacagggta gtttacaaaa cgctttagcc     180
actgctttaa aagatccaga ggccaacaaa cagtttgtcg ctgctcccct tttaaaggca     240
ttgacagctt ggtatgaaaa taaccaagac aaacaggtca cccagttctt taaagacacc     300
aaaaagagtg ttgatgagca atacaaccaa gccgtagata aggtggtatc ggctagccga     360
aacaaaaacc tctttgtcca acaagacttg ctggatagcg ccggggggt tagaaacttg      420
aaaagcccag aagttgtttg aacagctcat taa                                  453
```

<212> Type : DNA
<211> Length : 453
SequenceName : SEQ ID 510
SequenceDescription :
Sequence
--------
<213> OrganismName : Mycoplasma pneumoniae
<400> PreSequenceString :

```
atgcaacagc aaggagaaac taaagatcaa tataacactt ttggcctgag acttgtgcgt      60
aacagtgttg gcgtatcagt tttaggactt gatggttttg ttaaattcat aaaaggcggt     120
agcggaggta gcaatggcgg ttctagtagt gccaaaaaga ttgataaaga agagcaaaag     180
aaattcttaa agttccgtgc ttttcaagcc aaaattggca catttataa cactaacttt      240
gcctttagtt ttcccctaaa cgaaactta aaaggttggt ttgacaaaca ccgcggattg      300
atcttagcga acgccttagt taaagttact ttagacacaa aggaaaaagc aagtaaggcc     360
ttagtggatg cttttagttc ttataaaaac tgattgagtg aatacacccc ggttggttta     420
gctaccacca tgattagctt ttattttgac caaatgaaag ctctcaacaa taagctgtta     480
gaacgagtac ggagcttaaa ccaaaatgtt aaccaagcca atcctacccc ttggttaaat     540
gggttatcgg ctaaactacc ttacgttaac actaacggta attacgaaaa gttaaacaac     600
tactttacct ttttaattac caaagttttg tgacctaagg tgggcactga ggatactaat     660
gtcagtgaag aaaaaagcaa actcaaaact aaaactgaag atgttaacaa gattagggaa     720
aagattttga acaacatcga cagtaagctc aaaactttg tccaaaaact caaacctacc      780
ttagcacccc gaccagctta cagtaacgta atcttgttaa acattaacaa tgataaggtg     840
tggtctgctg gcgctaattg aagtttggca gttttactag atccgaaaaa ggttaacccg     900
ctttcgttta tgttgctcaa acaaatgttt gatcaaaaca gtttgtttaa aaaagcaaag     960
```

```
actttattcg aaaacattca aaataaagca aaaactagtg gaagtggtaa aagtggtaca 1020
accaccaacg atgatgccga tgctttgagc aaagtcattg gcaactatta ctacaacact 1080
tgagctaagc taaccgataa atcaatttat ggcaacctta aggatgacaa atttgatgat 1140
ctctttaaat tggcttttga cagtagtatt aacgaaaagt cctttaatgt agattataag 1200
gcagtgattg aacactaccg ctttatctat accttagagt ggttggtaga caaaaaccta 1260
aagaacttca aggatttatt aaaggcaaac ctcaagtttg gtgaaattgc ttttattgct 1320
tacaagaata ctgaaacgca gaacttctct aacccgcaag gtatattcgg ttcttacttt 1380
aattacgaaa acgaaacgaa tgcggctaag agtgctacgc aaattataga ccccaacagt 1440
ttcttctata aaaccaccac taaaccagaa gcgaaaacca ctcaaagtgc taatacagct 1500
gtgatggttc aaaatacgca gatgaacaat cagcaaacta acagttatgg ctttactggt 1560
ttgagtacca gtagtggttc gatgttaggt gctgctaccc agcaagccat tttggatcaa 1620
ataaccaaaa cttccttgca acagtatggt tctcaagctg acctgaaaaa gatcattggc 1680
gaaactaaaa atcaattatt attagaccga attgccaacc aactaatagc cttaaaaccg 1740
aatacaagtg gcaacagtgg tacccaaaaa acaattgctg catacttcca aacagatgcg 1800
gttggcaatc ctactttgga ctttaaagcg aagcaaaaac tcttattgga tgttttagat 1860
caatacaaag atttctttgg taataatgca caagcagttc aaagagattc tggtaagagt 1920
ggaactggca actatttaac ctataccgac ggtagtgata agatcactta tttgcagttt 1980
tcctataaag atattgacgg tttaagtttg agtagttcaa acggaactag cagcaaattt 2040
gccagtgatg ttgtagcagc gcttttatta ttccaggcag cctataaagg tactcaacaa 2100
ctggctttaa gttccatcaa taaaccacaa ttaccaattg gcgataaacg cataaaaaca 2160
gggatcgatt tactgaaata g 2181
```

<212> Type : DNA
<211> Length : 2181
SequenceName : SEQ ID 511
SequenceDescription :
Sequence
--------
<213> OrganismName : Mycoplasma pneumoniae
<400> PreSequenceString :

```
atgaagagtt ttttaagaaa acccaagttt tgacttttat tgttgggtgg cctttccact 60
agtagtatta ttctcagtgc ctgtgcaaca ccttcaaact cagcactgca agcagttttt 120
aaaccaactt ccaaccaatt ttttaacggt gagcatggta ccattcaaag cgctttaaac 180
accgctttaa gagatccgga aactaacaaa aaatttgtag ctgctccact tttaaaagca 240
ttagaagctt ggtacgagaa taatcaagat aaaaacatta ctcaattctt aaaagacacc 300
aagactaatg ttgataacca atacaaaacc gttgtagata aagtagtttc agcaccacgc 360
aataaatcct tatttgtaca acaagattta ttggacagta gcggtggtag tgaggccact 420
tgaaaagcgc gcaaactgtt tgaacagcta attagtgatt ttgcttcacg agtttttcaa 480
aagaattatt tgtcttataa ggaaaatggc aaagtatcag ccggtccgtt tttatacgac 540
acgatttcaa aaaatagtaa ttgacagaat atagtttttg atgctgttaa ttttccagaa 600
actaatgatg attttttcgc gaagattcaa agcgaagttt ttgatcaatg ggctgagtac 660
accgatccaa ctattattag ctcagttacc ttaaagtatt cagctcctaa ttaa 714
```

<212> Type : DNA
<211> Length : 714
SequenceName : SEQ ID 512
SequenceDescription :
Sequence
--------
<213> OrganismName : Mycoplasma pneumoniae
<400> PreSequenceString :

```
atgattaact tcttgtttaa ccaaatgaac gcgttgaaca ataagttttt ggagcgcgct 60
aaagcattaa accaaaatgt gaatcaggcc aatcctacac cttggttaaa tggtttatca 120
gctaaattgc cttatgttag aacaaatggt aattacgaaa aactcaacaa ttacttcact 180
ttcttaattg ttaaatacat gtggaagaag gtcggtaatg aagacgcttc attatctaaa 240
gatagtagca ttaacaagct caaaactaag accgaagacg ttaacaaaat tagagataaa 300
atcttagaag acattcaaaa gaaggttcaa gaatttgtta aaaacaagct taaaccaacc 360
ttagcaccac gacaaactta cagtaatgta attttgttaa acgttaacaa cgataaggtt 420
tgatcgatgg gcgccaattg agctttagct aacttattgg atacgagcaa aattaatcca 480
ctttcattta tgttgctcaa acaaacgttt gaccaaaacg acttgtttaa gaaagctaag 540
aaactttttg aagatattca aagtaaaaca aatggtggaa gttcaggtgg gatgcaaggt 600
agcaatacct cgagtagtga aggagctgat gccttgagta aagtaattgg caactattac 660
tacaacagtt gagctaaatt gactgataaa tccatttatg gaaatcctaa ggacaacaaa 720
tttgatgact tatttaaatt agcttttgaa gatagtatta acgaaaagtc ctttaatgtt 780
gattacaaag cggtcattga acactaccgc tttatctata ccttggaatg actagttaac 840
ggtaacttaa agaactttaa ggacttatta aaagcaaatt taaagtttgg tgaaattgct 900

ttcattgctt ataaaaacac tgaaaccaaa gaattttcca atccgcaagg tgtatttggt 960
tctgctttta attacgaaaa cgaaactaat gaagttaaga ttgctgcgca aaacttagac 1020
ccaaataatt tcttttataa aacaactacc aaaccagaag aagttaaaac cgcacaaaat 1080
ggtgcaagca tgatggttat gcaacaaaaa atgcaaagta ccatgcaaga tagtaatcat 1140
tatggtttta ccgggctaaa caccagtact agttcaatgt taggtgccgc tacgcagcaa 1200
gccattttgg atcaaataac caagaattcc ttgcaacaat atggttcgca gcaagaactg 1260
aaaactttga ttgaaaagac taataaccaa cttttattag accgaatcgc tagtcaatta 1320
agtggattaa atccttcaac cactggaaat agtaataatg gaaagggtaa aaatattgct 1380
acttatttcc aattagatgc cattggcaac ccaaccctta gctttcaaca aaaacgaaaa 1440
cttttattag atgttttaga tcagtacaaa gatttctttg gtacaaatac tcaggctgca 1500
caaagagatt ctggtaaagg tggacatgga agctattcaa cttaccaaga tggcagtgac 1560
aagatcactt accttcagtt ctcctataaa gatatagata acttaagttt gagtgataaa 1620
ggaaatagta agcttgctag cgatgttgtg gctgcccttt tacttttcca agcagcggat 1680
aaaggtaccc aacaattagc cttgagtgct attaattaa 1719
```

<212> Type : DNA
<211> Length : 1719
SequenceName : SEQ ID 513
SequenceDescription :
Sequence
--------
<213> OrganismName : Mycoplasma pneumoniae
<400> PreSequenceString :

```
atgaaaaagt ttctaagaaa accccaattt tgactcttaa cattaggcgg ttttctgtct    60
actagtgtta ttttagctgc ttgtgctacc ccttccaact cggctttaca aaccgttttt   120
aaagcgcgtt ccagtcagtt tttcaatggt gaacagggta gtttacaaag tgccctaaca   180
acggctttaa aaaatccagt ggccaacaaa caatttatcg ctgcaccact tttaaaagca   240
ctagaagctt ggtacgaaaa caacgaagac aaaaagatta cccagttttt aaaagacact   300
aagtccaatg ttgacagtca gtacacaacg gcagtcgata aagtggtatc agcatcacgc   360
aataaatcac tttttgtgca acaagatttg ttggataacg ccggtggtag tgaagcaacc   420
tggaaagcgc aaaagctgct tgaacagctc attagtgact ttgctagtcg ggttttccaa   480
aagaactacc tcaattacaa aaaagatgga caagtttcta ctggtccatt tacttatgat   540
gaactacaca aggaagaaag ctgaaaaaac tttgaattta gtgccccacg ttttagtgaa   600
actaatgatg actttttcgc caaaattcaa agccaagtat ttgaccaatg ggtggagtac   660
actgatccca ctttaattag tcaagttaac tataagtatt ctgctcccag tcaagggtta   720
ggtcagatct ataacagaga gaagttaaag gataaactaa caccttctta tgcctttcct   780
ttctttgccg aagaaaaaga cattgcaccc aaccaaaacg ttggtaataa gcgctgaaag   840
cagttagtta aaggtgaggg cgctattact gataataata tcggtcaaag cggtacaaac   900
agccaaaaaa ctggtctgct caaataccgt aatgaatcaa ataagggtga ttttcttgat   960
tttcccttaa atttatcaga tactaacgaa acgaagcaat tagtagacgc ttctaacatt  1020
gtcgatcagt tagaagctgc taacttaggt gcagctttaa atttaaaact gcaagttttt  1080
gagcaagata atgacgaatt gccgcaaatt aaggagctca aagaagacct taacaacacg  1140
attgttgtcg ataaaagtaa ggacgtagaa aaagcttcca aaactaacgc actgttttac  1200
aatgatcaag aaggtaagca acaacaaagt gactcagatc caattgctgg cgctttagat  1260
gacatttttg ctcaaaacac aagcgaaggc actaacttaa gtaagttagc ggagcaggta  1320
aagaaagcag ctgcaacaaa aatggaagcg aaaacagctg ttttaagaac taacaattct  1380
aagggccaac aaaacaatta cgttgtttta gatgcagcta ttcctacatt taattcaaca  1440
acatcaaaat cgaaaaataa tagcgcttct aatgaagttt tagttgcctt aaaatctgga  1500
tctataaatt taaggcaggt tcagcaaact gatcaaaaca gttacagtcc cattaaattc  1560
cggattgtgc gtaacagcac cggagtaact gttttcggcc ttgatggtgg gagctattat  1620
ttaaaacaag attcaacaaa taaaaaatca gtttctaagc aaagtttaac cttattaact  1680
aaatctagtt caggtaacag taataaagta ttaagagacc ttgacaagca aaaacaattc  1740
ttaaagtttc gtgcctttca agctaaaact aacactttct acagtactaa ctttgccttt  1800
agttttccct taaacgaaac gctaaaaagt tggtttgata aacaccggga actaatttg   1860
gccaacgcct tagttaacgc tagtcttgac caaaaggata aagctagcaa agctttaact  1920
gaagctttta atccttataa agagttaatt aaagaatttg cgccggtggc tttagcaacc  1980
acaatgatca gcttttattt tgatcaaatg aaagcgctca ataacaagtt gctagagcgc  2040
gcccgtaatc ttaaccaaaa cgtcaaccaa gccaatccaa ccccttggtt gaacggtttg  2100
tcagccaagt taccttacgt taatactaac ggtaattacg aaaagttaaa caactacttt  2160
accttcttaa tcacaaaaac attgtgacct aaagttggtc aagaagaaac aagcataagt  2220
gaagaaagca ataagctcaa aactaaaact gctgacgttg acaagattag ggacaaaatc  2280
ttggaaaaca tccaaactaa agtaaatgat tttgttaaaa acaaactcaa acctgcttta  2340
gcaccccgac cagcttacag taacgtcatt ttgttaaacg ttaacaatga caaagtactt  2400
tctagtggtg ctaactggag cttagctagc ttattacaga gcgacaaagt caacccgctt  2460
tcgtttatgt tgctcaaaca agcgttcgat aataacgatt tatttaagaa agcacaaaag  2520
ttgtttaaag acattcagga aaagtccagt aataatggtg gaatgcaaag tagttccaca  2580
accaacagtg atgccgatgc attaagtaaa gttatcggta actattacta cactacttgg  2640

gctaaactca cggacaaatc gatttacggt aacccgaagg acaacaagtt tgatgagctt  2700
ttcaaactgg cttttgaagc tagcatcgac gaaaagtcct ttaacgttga ttacaaagcg  2760
gtcattgacc attaccgttt tatctatacc ttacagtggc tagtggacca aaaattaaag  2820
aactttaagt cactgttaaa aacaaacctt aagtttggtg aagttgcttt tatagcttac  2880
aagaacactg aaaccactaa cttctctaat ccccaaggcg tatttggttc ctacttcaac  2940
tatgagaact ccgctagcga agttaaagaa tccactcaaa cactagatcc caataacttc  3000
ttttacaaaa caacaaccaa gccaacggta caagccattc aacaagttgc tagcttagca  3060
ttagtacaaa aacaacaaat gcaacaaaat tcaactgatc actatggttt tactggtttg  3120
agtaccagca ctagttcgat gttcgatgct agttcccgtg atgccatttt gcagcaaatt  3180
acgaaaacct ccttgcaaca gtacggttcc aaggatcaac tcaaaaagat cattcaaggg  3240
actaataacc aattgttact agaccggatt gcagtccagt taagtggatt aaatccttcc  3300
acaactaatg gtggcagtgg caaaacaatt gcgacctact tccaagtgga tgccgttggc  3360
aacccaactt tggacttcca agctaagcgt aaactgttat tagacctctt ggaccagtac  3420
caaaactact ttggtaatgg tgcccaaaag agccaaaggg atttctactcc aagtggaact  3480
ggcaactacc tcacttacca aaatggtagt gacaagtaca cctacaccca gttcacttac  3540
caagacattg acagcttgag tctaacaact acaagcggta ccaacaataa aattgccagt  3600
gatgttgtgg cagcgctgct tttattccaa gcagcagaca agggcacaca acagttggca  3660
cttagtgcca ttaataagcc gcagttaaat attggtgata aacggataga aagtgggtta  3720
aaattgctca aatag                                                    3735
```

<212> Type : DNA
<211> Length : 3735
SequenceName : SEQ ID 514
SequenceDescription :

Sequence
--------
<213> OrganismName : Mycoplasma pneumoniae
<400> PreSequenceString :

```
atggttggaa gtggtgcggc tggaagtgct tctagtttac aaggcaatgg ctcgaacagt      60
tcggggttaa aatcgctctt gagatcagca cctgtcagtg ttccaccaag cagtacaagt     120
aatcaaactt taagcttatc taaccccgct cctgtaggcc cacaagcggt tgtaagccaa     180
cccgcggggg gtgctacggc agcagtgtcc gtcaatcgca cagcgagtga caccgccacc     240
tttagcaagt acctcaacac cgcccaggcc ttgcaccaga tgggggtgat tgttccgggg     300
ttggaaaaat gaggtggtaa caacggtacg ggtgtagtgg ctagccgacg ggatgctact     360
tccactaacc tgccccatgc ggcaggtgct tcccaaacgg gtttgggaac tggttcgccc     420
cgcgaaccag ctttaaccgc aacgtcacag cgtgccgtca cggtggttgc tggccccctt     480
cgtgcgggca atagcagtga aactgatgcc ctaccgaatg tcatcaccca gctctatcat     540
acttcaaccg cccaactcgc ttacttaaat ggccagatcg ttgtgatgag ttccgccccgg    600
gtaccgagtc tttggtattg agttgtcggg gaggaccagg aatcgggcaa agcgacctga     660
tgagcgaaaa ccgagctcaa ctggggcacc gacaagcaga agcagtttgt cgaaaaccag     720
ttggggttta aagatgactc aaattcggat tccaaaaatt cgaatttgaa gacccaaggc     780
ctcacccaac ccgcctacct catcgccggt cttgacgttg tggccgacca cctcgtcttt     840
gcggcattta aagcgggcgc ggtggggtat gatatgacga ctgattcgaa cgcttcgacc     900
tacaaccaag cactcgtctg gtcgaccacg gccgggttgg acagtgatgg ggggacaagg     960
ctttggtag                                                             969
```

<212> Type : DNA
<211> Length : 969
SequenceName : SEQ ID 515
SequenceDescription :
Sequence
--------
<213> OrganismName : Mycoplasma pneumoniae
<400> PreSequenceString :

```
atgccagttt ttctaaaatt aacgcacaca attagaaaag tgctaagagt tgcaagactt      60
tctagactag cattactatc actaaccgct gttattttta gcggttgtgc caatattaat     120
ttaattagtg ctgttggttc ttcttcggta caaccgttgt taagcaaact cagttcgcac     180
tatgtcttga accacaatga caaggataac cttgtagaaa ttagtgtcca agcgggtggc     240
tctagtgctg gggtgaaagc aatcaccaag ggactagctg acattggtaa tgtctcgaaa     300
aacaccaaga gctatgctga ggaaaacaag cagttgtgga tggacaaaaa gctcaaaaca     360
attacacttg gcaaagatgc cattgctgtt atttataaag caccatcaga gtttaagggc     420
aaactagttc tcactaaaga caacctcaac gatctttacg atctgttcgc tggtagcaaa     480
agcgttgaca ttaataagtt tgtcgaaaac ggacaaacca ctaaaaacag taatcataat     540
ttgataggct tcccccgtac tgggggcgct tttgcttctg gtaccgctga agctttcttg     600
aagttttcgg gtcttacaca gacaaaaact ttagataaag attccaaaga aattttggaa     660
ggtcaacgca actatggccc caatgcgcga ccaactagtg aaaccaacat tgaggccttt     720
aatacctttg tcacaacttt gcgacaaccc aatttatacg gcatggtgta cctcagttta     780
```

```
gggtttgtga ataacaacat gaacctaatt aagagtgaag gttttgaggt tttaaaagtc     840
aaatatgata ataacgcagt taccccctcc agtcaagcag tttctagcaa cacttacaaa     900
tgggtacgcc cgttgaactc agtggtttcc ctgttaccaa aacaaaaaac actgccaagt     960
atccaacgct tttttaactg attgttattt agcaacaaca gtgaaattaa gaaaatatac    1020
gatgactttg gtgtgttaga gttaacggct gacgaaaaga aaaagatgtt taaaacaggt    1080
aatgcagaaa tgagcaacat tgccaacttc tgggttgatg attacagtct gaacaaccaa    1140
accttcggtg cactctag                                                  1158
```

<212> Type : DNA
<211> Length : 1158
SequenceName : SEQ ID 516
SequenceDescription :
Sequence
--------
<213> OrganismName : Mycobacterium tuberculosis H37Rv
<400> PreSequenceString :

```
atgagcttcg cggtgctgcc cccggagatc aattcggcgc gcctgtacgt gggtgccggg      60
ttggcgccga tgctggacgc ggcggccgct tgggatggac tggccgacga attgggttcg     120
gccgcggcct cgttttcggc ggtgacggcg gggctggcag gttcctcgtg gctgggcgcg     180
gcgtcgacgg cgatgacggg agcggccgcc ccctatctgg gctggttgag cgcggcggcg     240
gcgcaggccc agcaggcggc cacccaaacc cggctggcgg cggccgcctt cgaggcagcc     300
ctggcggcga cggtacatcc ggcgatcatc tcggccaacc gggcactgtt cgtgtcgctg     360
gtggtctcga acctgctggg ccaaaacgcc ccggcgatcg cggccaccga ggccgcctac     420
gagcagatgt gggcccagga cgtggcggcg atgtttggct accatgccgg ggcttcggcg     480
gccgtctcgg cgttgacacc gttcggccag ggcgctgccga ccgtggcggg cggcggtgcg     540
ctggtcagcg cggccgcggc tcaggtgacc acgcgggtct tccgcaacct gggcttggcg     600
aacgtcggcg agggcaacgt cggcaacggt aatgtcggga acttcaatct cggctcggcc     660
aacatcggca acggcaacat cggcagcggc aacatcggca gctccaacat cgggtttggc     720
aacgtgggtc ctgggttgac cgcagcgctg aacaacatcg gtttcggcaa caccggcagc     780
aacaacatcg ggtttggcaa caccggcagc aacaacatcg gcttcggcaa taccggagac     840
ggcaaccgag gtatcgggct cacgggtagc ggtttgttgg ggttcggcgg cctgaactcg     900
ggcaccggca acatcggtct gttcaactcg ggcaccggaa acgtcggcat cggcaactcg     960
ggtaccggga actggggcat tggcaactcg ggcaacagct acaacaccgg ttttggcaac    1020
tccggcgacg ccaacacggg cttcttcaac tccggaatag ccaacaccgg cgtcggcaac    1080
gccggcaact acaacaccgg tagctacaac ccgggcaaca gcaataccgg cggcttcaac    1140
atgggccagt acaacacggg ctacctgaac agcggcaact acaacaccgg cttggcaaac    1200
tccggcaatg tcaacaccgg cgccttcatt actggcaact tcaacaacgg cttcttgtgg    1260
cgcggcgacc accaaggcct gattttcggg agccccggct tcttcaactc gaccagtgcg    1320
ccgtcgtcgg gattcttcaa cagcggtgcc ggtagcgcgt ccggcttcct gaactccggt    1380
gccaacaatt ctggcttctt caactcttcg tcgggggcca tcggtaactc cggcctggca    1440
aacgcgggcg tgctggtatc gggcgtgatc aactcgggca acaccgtatc gggtttgttc    1500
aacatgagcc tggtggccat cacaacgccg gccttgatct cgggcttctt caacaccgga    1560
agcaacatgt cgggattttt cggtggccca ccggtcttca atctcggcct ggcaaaccgg    1620
ggcgtcgtga acattctcgg caacgccaac atcggcaatt acaacattct cggcagcgga    1680
aacgtcggtg acttcaacat ccttggcagc ggcaacctcg gcagccaaaa catcttgggc    1740
agcggcaacg tcggcagctt caatatcggc agtggaaaca tcggagtatt caatgtcggt    1800
tccggaagcc tgggaaacta caacatcgga tccggaaacc tcgggatcta caacatcggt    1860
tttggaaacg tcggcgacta caacgtcggc ttcgggaacg cgggcgactt caaccaaggc    1920
tttgccaaca ccggcaacaa caacatcggg ttcgccaaca ccggcaacaa caacatcggc    1980
atcgggctgt ccggcgacaa ccagcagggc ttcaatattg ctagcggctg gaactcgggc    2040
accggcaaca gcggcctgtt caattcgggc accaataacg ttggcatctt caacgcgggc    2100
accggaaacg tcggcatcgc aaactcgggc accgggaact ggggtatcgg gaacccgggt    2160
accgacaata ccggcatcct caatgctggc agctacaaca cgggcatcct caacgccggc    2220
gacttcaaca cgggcttcta caacacgggc agctacaaca ccggcggctt caacgtcggt    2280
aacaccaaca ccggcaactt caacgtgggt gacaccaata ccggcagcta taacccgggt    2340
gacaccaaca ccggcttctt caatcccggc aacgtcaata ccggcgcttt cgacacgggc    2400
gacttcaaca atggcttctt ggtggcgggc gataaccagg gccagattgc catcgatctc    2460
tcggtcacca ctccattcat ccccataaac gagcagatgg tcattgacgt acacaacgta    2520
atgaccttcg gcggcaacat gatcacggtc accgaggcct cgaccgtttt cccccaaacc    2580
ttctatctga gcggtttgtt cttcttcggc ccggtcaatc tcagcgcatc cacgctgacc    2640
gttccgacga tcaccctcac catcggcgga ccgacggtga ccgtccccat cagcattgtc    2700
ggtgctctgg agagccgcac gattaccttc ctcaagatcg atccggcgcc gggcatcgga    2760
aattcgacca ccaacccctc gtccggcttc ttcaactcgg gcaccggtgg cacatctggc    2820
ttccaaaacg tcggcggcgg cagttcaggc gtctggaaca gtggtttgag cagcgcgata    2880
gggaattcgg gtttccagaa cctcggctcg ctgcagtcag gctgggcgaa cctgggcaac    2940
tccgtatcgg gctttttcaa caccagtacg gtgaacctct ccacgccggc caatgtctcg    3000
ggcctgaaca acatcggcac caacctgtcc ggcgtgttcc gcggtccgac cgggacgatc    3060
```

```
ttcaacgcgg gccttgccaa cctgggccag ttgaacatcg gcagcgcaaa tctcggcgac     3120
ttcaacctgg gcagcggaaa cgtcggcagc ttcaacgtct tctccggaaa ccagggctca     3180
tacaatatcg gtccggcgaa cctgggtaac tacaacatcg gtttcgcgaa cctgggtaac     3240
tacaacatcg gcttcggcaa cgccggcgat ttcaaccaag gctttgccaa caccggcaac     3300
aacaacatcg gatttgccaa caccggcaac aacaacatcg gcatcgggct gtccggcgac     3360
aaccagcagg gcttcaattt tgctggcggc tggaactcag gcaccgcgaa catcggcttg     3420
ttcaactccg gcaccaacaa cgtcggcatc ggcaactcgg gcaccggcaa ctggggtatc     3480
ggcaactccg gcagcggcaa caccggcatc ggcaacaccg gcagcactaa cacgggcttc     3540
ttcaacaccg gcatcgtcaa caccggtgtc gccaacgcgg gcagctacaa caccggctgg     3600
tacaacaccg gcgacaccaa caccggcatc gccaacctgg gcgacttcaa cacgggcttc     3660
tacaacaccg gcaatttcag tacgggcttt gccaaccagg gtgatatcgc caccggggct     3720
ttcatcaccg gcgacatggg caacggcgcc ttctggcgcg gcgaccagca gggcctattc     3780
agcgcgggct atcgggtcca tgttcccgaa atacccgcac acgtcaccgt ggaagttccc     3840
gtcaacatcc ccatcaccgc cagcttcacc aacaccgtct acagcggcat aacgcttgag     3900
caaatcaact tcggtttcac catcgacatc gcagggatcc ccctgctggc cggtgcaatc     3960
agcaaggccg ttctcccgcc catcaccggg accggtcccg cgatcacggt caacatcggc     4020
gaccctggcg gttcgaccgc gatcaggatc ccggccaccg caagcgtcgg tcccttcgat     4080
gtcacgttcg tcaacattgc ggctaccacg ggctttttca acgccaccac cgatccgtcc     4140
tcgggcttct tcaacggcgg ccccggaacc gtatcgggca tcgccaacat cggcgccaac     4200
atttccggct tccagaacgt cgcgaactcc gcgacctcgg gcttcaacaa ctacggctcg     4260
ctgcaatcgg gactggcgaa cctgggcgat accgtctcgg gcgtattcaa caccggcatc     4320
ggggcaccgg ccaacgtctc gggcatgttc aacatcggca gcaacctcgc ggggttcttc     4380
cacgaccagg cgaccgggat gtcgatgttc aacctcggcc tgggaaacat cggccaattc     4440
aacgtcggct tctccaacgt aggcgacagc aacgccggct tggcgaacat cggcagcttc     4500
aacctcggca gcggcaacct cggcagcttc aacgtcttcg gcggaaacca gggctcatac     4560
aacatcggcc cggcgaacct gggtaactac aacatcggcc taggcaacct gggcagctac     4620
aacttcggat tcggcaacgc cggcgacttc aacctgggct tcgccaacac cggcaacaac     4680
aacatcgggt ttgccaacac cggcaacaac aacatcggta tcggcctgtc cggcgacaac     4740
cagcagggct tcaattttgc cggtggctgg aactccggca gcggcaacag cggcctgttc     4800
aactctggca ccaacaacat cggtttgttc aactcgggca ccggcaacat cggcatcggc     4860
aactcgggca ccggaaactg gggcatcgcc aacaccggtg acaccaacac cggcatcttc     4920
aacaccggcg acgtcaacac cggcttgctc aacgccggca acgtcaacac cggcatcttc     4980
aacaccggcc attacaacac cggcagcttc aacgccggca gcttcaacac ggccggcttc     5040
aacccgggta gctacaacac gggttatttg aacaccggca gctacaacac cggactggcc     5100
aactcgggcg atgtcaacac cggcggcttc atcaccggca attacagcaa cggcttctgg     5160
tggcggggcg attaccaagg cctggcgggg atcagccaaa cgatcaccgt gcccgacacc     5220
gccgttccgg tgaaactgca cgtgccgatc ttcctcgata tcccggtcac cggcacactt     5280
ggcacgttca ccgttcatgg cttcagattc ccggagatca ccggcgatat cttcttgatc     5340
ggcataccgt tcaatgccgc cacactcgat gcattcagtt tcccgaacat ctcgattgtc     5400
cttcccaata tcggcatcaa cctgggtagc gggccggacc cgctgatcga tatcgccggc     5460
accggcggtc tattgccgat caagattcca ctcatcgata taccggcggc cccgggattc     5520
gggaactcga cgaccacccc gtcgtcggga ttcttcaacg ccggtaccgg taccgtgtcg     5580
ggcgttggca acgtgggcag caatagttcc ggcttcttca acctcacctc tggaagctcg     5640
ggaatctcgg gcgtccagaa cttcggcgag ctgatctccg gcgggttcaa cttcggtaac     5700
actgtctccg gccttgtcaa tgcgagcacg cttgggcttt cgatgccggc caatctctcc     5760
ggcggaggga atgtcggtgc tacggtcgcc ggcttcgtca acaacaccca gatcctcaac     5820
ctcgggtttg gcaacgtagg cagcgggaat gtcggccacg gcaatatcgg cgactccaac     5880
gtcggcctcg gaaacctcgg caacgcgaac gtcggccatg gcaacatcgg cagcttcaac     5940
gtcttctccg gaaacctggg ctcatacaat atcggcccgg cgaacctggg taactacaac     6000
atcggcctag gcaacctggg cagctacaac ttcggattcg gcaacgccgg cgatttcaac     6060
ctgggcttcg ccaacagcgg cagcaacaac atcgggtttg ccaacaccgg caacaacaac     6120
atcggtatcg ggctgtccgg ccacaatcaa caggggttcg gctcctggaa ctccggtacc     6180
gccaacaccg gcctgttcaa ctcgggcacc aacaacatcg gtttgttcaa ctcgggcacc     6240
ggaaacatcg gcattggcaa ctcgggcatc ggaaacaccg ggatcggcaa cccgggtgtc     6300
ggcaacaccg gcttggggaa ctcgggcacc ggcaactggg gcctgtggaa cccaggcacc     6360
ggcaatatgg gcgtcgccaa cgtgggcacc tacaacaccg gtggctacaa cgtgggcagc     6420
accaacaccg gcatcgccaa cgtgggcatc gccaacacgg gcagctacaa caccggcagc     6480
accaacaccg gcagcttcaa cgacggcgac ttcaataccg gcttctacaa caccggcgac     6540
tacaacaccg gcttctacaa caccggcgac gtgaacaccg gcgccttcat cgggggcaac     6600
ttcagcaacg gcgccttctg gcagagcgat caccaaggcc agtggggcgc acactacgca     6660
atcactgttc cccagatccc gctactgaac tttagcctca acattccggt caacatcccc     6720
atccatctcg acttcggtac ccttgccgtc aacggcttcc agattccggc tatcaccctc     6780
cgcgccctcg ggtcacccca cttcagcgtc ggacccatca tcgttccgag gatcgccggc     6840
accttaccgg tgatcgatat caacatcggc gaccccggcg gttcatcctc gatacccatc     6900
acgatcacca gcggcgccgg cccggtcgtc atcccgctac tggacatccc gccggccccc     6960
ggtttcggaa actcgaccac cggcccctca tcgggcttct tcaactccgg caccggcagc     7020
tcgtctggat tcggaaacgt cggcgccaac aattcgggct tctggaacac cgctttcgcc     7080
```

```
ggcataggaa actctggctt gcagaacttc ggctcgctgc aatccggctg ggcgaacttg    7140
ggcaacaccg tctcgggctt ctacaacacc agtgcggcgg acttcgcgac gccggctaac    7200
ctctcgggac tctccaacgt cggcgccgac ctgaccggcg tgctccgcgg cccgaacggg    7260
tcgaccttca acgcgggcct ggcaaacctc ggccaattca acgtgggcag tgcaaacctc    7320
ggcagtgcaa acctcggcag tgcaaacctc ggcagtgcaa acctcggcaa ttcaaacgtc    7380
ggcttcggca acattggcaa cgcgaacatc ggcggcgcaa acatcggcga ctttaacgtc    7440
gggatcgcaa acaccggtcc ggggctaacg gcggctgtca acaacatcgg tatcggcaac    7500
accggcaact acaacatcgg tgtcggcaac accggtaact acaacatcgg cttcggcaac    7560
accggcaaca acaacatcgg catcggcctg tccggcgaca accagatcgg gttcggcccg    7620
ctgaacgccg gcatcgccaa catgggcctg ttcaacctgg gcgacaacaa ctttggcatg    7680
gccaacgcgg gcaacttcaa ccagggcatt gccaacaccg gcaacaacaa catcggcttg    7740
ttcaacaccg gcaacaacaa cgtcggcatc tggctgaccg gcgacggctt gtccggcttc    7800
agctccctga actccggcgc cggcaacacc ggtttcttca actccggcac cgccaacacc    7860
ggcttgttca actccggcac cggcaacacc ggcttgttca actcgggcac cggcaacgtc    7920
ggcatcggca acatgggcac cggcggcttc ggcgtcggcc tatccggcga cagccaggtg    7980
ggcatcggcg gcaccaactc gggcagtttc aacatcggct tgtttaactc gggcaccggc    8040
aatgtcggca tcggcaactc gggcaccggc aacgtcggca tcggcaacac cggcaccggc    8100
aacaccggca tcggaaacag cggcaactac aacaccggct tgctcaacgc gggcctggtc    8160
aacaccggca tcgccaaccc gggcaaccac aacaccggcc tgttcaacat cggcaccttc    8220
aacaccggca tcgccaaccc gggccactac aacaccggct cctacaacac cggtagctac    8280
aacaccggca tggcaaacgc cggagactac ggcaccggcg cgttcatcac cggcagcatg    8340
aacaacggct tgctctggcg cgccgaccgg cagggcctgc tggcggccaa ctacaccatc    8400
accatcgagc gacctgccgc gttcctcaat gtcgacatcc cggtcaacat ccccatcacc    8460
ggcgacatca ccaatgtctc catccccgcc attacgttcc ccagaatcga cgccagcgga    8520
agcgtcgaca taggcatcct cagtggcacc gtcttggccc cggtcggtcc gatcaccctg    8580
catggcgggg acgcgtcggc cccgctggac acacccatcg aaattgactt cggcccctcg    8640
ccggcgatca acctcaacat cggcaagccc gacggctcca ccgtgatcaa catcgtgggc    8700
ggcgccggcg ccggcccgat cagcattccg atcatcgact tgcggccagc gcccggcttc    8760
ttcaacgcca ccaccggccc gtcgtcgggc ttcctcaact ggggtgctgg cagcgcatcg    8820
ggcttgctga acttcggcaa caactcgggc ctctacaact tcgccactag cagcatggga    8880
aattcgggct tccaaaacta tgggtcgctg cagtcgggct gggcgaattt gggcaacagc    8940
atctcgggca tctacaacac cggcttggga gcaccggcaa atgtctcggg cttgctcaac    9000
atcggcacca acctggctgg gtggttgcag aacggcccga ccgagacgac cttcagcgtg    9060
ggcttggcca acctcgggtt ctggaatctg ggtagcgcaa acatcggcaa ctacaacctg    9120
ggcagcgcca acatcggcgt ctacaacctg ggcagcgcca acatcggcga cttcaacctg    9180
ggcagcgcca acatcggcga cttcaacctg ggcagcgcca acatcggcag ctccaacatc    9240
gggttcggca acgtcggtcc ggggctgacg gcggccatcg gcaacatcgg cttcggcaac    9300
accggaaacg gaaacatcgg catcggcaat accggcaccg gcaacatcgg cttcggcaac    9360
accggaaacg gaaacatcgg catcgggctg accggcgaca ccatgaccgg gttcggcggc    9420
tggaactcgg gcaccggcaa catcgggcta ttcaactccg gcaccggcaa catcggcttc    9480
ggcaactccg gcaccggcaa ctggggcatc ggaaactccg gtgactacaa caccggcatc    9540
ggcaacaccg gcagcaccaa ctccggcttc ttcaacaccg gcctggtcaa caccggcatt    9600
ggcaactccg gtgactacaa caccggccta ttcaacgccg ggaacaccaa caccggcagc    9660
ttcaaccccg gcgactacaa caccggcggc ttcaaccccg gtaactacaa caccggctac    9720
ttcaacccgg gcaactccaa caccggcatc gccaactccg gcgatgtcaa caccggcgcc    9780
ttcaattcgg gcaactacag caacggcttc ttctggcggg gcgactacca gggcctaggc    9840
ggtttcgcct accagagcgc cgtttccgaa atcccgtgga gctacgacag gttccaacat    9900
tga                                                                 9903
```

<212> Type : DNA
<211> Length : 9903
SequenceName : SEQ ID 517
SequenceDescription :
Sequence
--------
<213> OrganismName : Mycobacterium tuberculosis H37Rv
<400> PreSequenceString :

```
atgaacctgg tctccacaac gtcgggaatg tcgggcttcc tcaacgtcgg cgcgctggga      60
tcgggtgtgg cgaatgtggg caacaccatc tcgggtatct acaacgtggg cacgtcggac     120
ctctcgacgc ccgccgttaa ctccgggttg gcaaatatcg gaaccaatat tgccggcctg     180
ctgcgcgacg gcgcgggtac tgcggctatt aacttgggct tggccaacca cggcaacctc     240
aacgtgggct tcgcaagtct cggcggcttt aacttcggcg gcgccaccat cggccacaac     300
aacgtcggca tcgggaacac cggaatcttc gatgtcggcc tggcgaacct gggcagctac     360
aacatcggct tcggaaacct tggcgacgac aacctgggct tcggcaactt cggcagctac     420
aacatcggct tcggcaacgt cggcaacgac aatctgggtt tcgctaacgc gggcggcggc     480
aacatcggct ttgcgaacac cggcagcaac aatgtcggct ttgggaacac gggcagcaac     540
aatgtcggca tcgggctcac gggcaacgga cagatcgggt tcggcagctt caactcgggc     600
```

agcggaaaca tcggcctgtt caactcgggc agcaacaaca tcggattctt caattccggc 660
agcggcaact tcggcatcgc aaactcgggc agcttcaaca ctggcatcgg aaacaccggc 720
aacaccaata ccggcctatt caactccggc gacgtcaaca cgggcgcctt caacccgggc 780
agcttcaaca ccggtagctt caacaccggc agcttcaaca ccggtggctt caatccgggc 840
aataccaaca ccggctacct caacattggc aactacaaca ccggcatcgc caacaccggc 900
gacgttgaca ccggggcttt catcaccgga aactacagca acgggttgtt cttaagcggc 960
gattaccagg gcctggtcgg cctcaacctg gtgatcgata tgcctctccc cataagcctc 1020
ggcgtgaata ttcccatcga tatcccgatc accgcctcgg ccggcaacat caccccttatg 1080
ggcgtcacga ttccgcccac cggcgatatc gtcctttcgt caatagcggg ccagcgagcc 1140
cactttggcc ccattaccat tccgaacatc acggttgtcg gccccacgac gacagtcgcc 1200
ataggagggc cgaataccgc gatcaccata actggcggtg gcgccattag gatcccgctc 1260
atcagtatcc cggcggcgcc aggtttcgga aactcgacca ccaacccgtc gtcaggtttc 1320
ttcaataccg gcgccggcgg cgcctcgggc ttcggcaact tcggcggcgc caattcgggc 1380
ttttggaacc tggcctccgc gacctcgggg gcgtcggggc tcctcaacgt cggcgccctg 1440
ggatcaggtc tggcgaacgt gggcaccacc gtctcggggt tctacaacac cagcacgtcg 1500
gacctcgcga cgccggcctt caattcaggc ctggccaaca tcagcaccag tatcgccggc 1560
ctgctgcgcg acagcacggg caccatggtc ctcaacctgg gcttggcaaa ccacggcacc 1620
ctcaacgtcg gcattgcaaa cctcggcgac tacaacatcg gctttgcaaa cctcggcagc 1680
gccaacttcg gcagcgccaa tatcggtggc aacaacatcg gcggcgcaaa caccggaata 1740
ttcgacatcg gtttggcaaa tctgggcagt tacaacatcg gcttcggaaa cttcggcgat 1800
gacaacctgg gcttcggaaa cctcggcagc tacaacgtcg gcttcggaaa cttgggcaac 1860
gacaacctgg gcttcgccaa caccggcagc aacaatatcg ggttcgcgaa caccggcagc 1920
aacaatatcg gcattgggct cacgggcgac ggccagatcg ggttcggctc cctgaattct 1980
ggcagcggaa acatcggctt gttcaactcg ggcagcggaa acatcggctt tttcaactcg 2040
ggcaacggaa acgttggcat cggcaacacc ggcaccgcaa acttcgggct tggaaacacc 2100
ggcagcacca acaccggctt cttcaactcc ggcgacgtca ataccggtat cggcaacacc 2160
ggcagcttca acaccggcag cttcaatccg ggcgattcca acaccgggga tttcaaccca 2220
ggcagctaca acacgggact cggaaacacc ggcgatgttg acaccggcgc cttcatctcc 2280
ggcagctaca gcaacgggtt cttgtggagt ggaaattatc agggcctcat tggcttgcac 2340
gcggcgctag cgattcccga aatcgcccta acctttggcg tcgacatccc gatacatata 2400
cccatcaaca tcgacgccgg ggtcgtcacc ctccagggct tcagcatcgt agctgccgaa 2460
aataatatcg acttcacccc catcatcatc ccgaccatca atatcacctt gcccacggcg 2520
gcgatcaccg tgggcggacc caccacctcg atcggtatca ccgccagcgc cggtatcggc 2580
tccatcacca tcccgatcat cgacattccc gcgacatcgg gcttcggcaa ctcgaccact 2640
agtccgtcgt cgggcttctt caactccgga gcgggcagcg cgtcgggctt tttgaacgtg 2700
gtcgccggcg cctcagggat ttcgggttat ctcaatgtcg gtgcgctggg gtcgggtgtg 2760
actaacgtgg gtcacaccgt ctcgggtttc tacaacgcga gcgcgttgga cctcgtgacg 2820
ccggcctttg cctccggtct catgcgcgac ggtatgggca cgatgactct gaaccttggg 2880
ctggcaaacc tgggcagcaa taacgccggc ttcggcaaca ccgggatctt tgacgtcggc 2940
gtggcgaatc tgggcaacta caacatcggc ttcggaaact tcggcgacga caacctgggc 3000
tttgccaacc taggcagcta caacatcggc gttgccaaca ccggcagcaa caatatcggc 3060
tttgccaaca ccggcagcaa caatatcggc atcgggctca ccggtaccgg ccaaatcggg 3120
atcggcgctc tgaactcggg cagcggaaac atcggcttgt tcaactcggg cgacggaaac 3180
atcggcttct ttaactcggg caccgggaac ttcggcatcg gcaacaccgg caccggaaac 3240
ttcggcatcg gcaactcggg cagcaccagc acgggcttgt tcaactcggg cgacggcaac 3300
accggcggct tcaaccccgg taacttcaac accggcaatt tcaataccgg cagcttcaac 3360
accggcggct tcaacgcggg taacaccaac accggccact tcaacaccgg gaactacaac 3420
accggcatcg cgaatacggg cgacgtcagc accggcgctt tcatctccgg caactacagc 3480
aacggcatct tgtggcgggg cgactaccag ggcctgatcg gttactccta cgcgctgact 3540
attccggaga ttccggcgca cttggacgtc aatatcccaa tcgacatacc gatcaccggc 3600
agtttcaccg acctcgtggt ggacaatttc actatcccca tcatcggctt cgaatccttc 3660
gcgtttagct ttcacatcca taccgagccg gacatcggtc ccatcattgt cccgagcttc 3720
gtgctcagcg ttcccacgtt cgcgatcgcc gtgggcggac ccacgaccgc gatcaacatc 3780
agcgccaccg ccggcctcgg ccccatcacc atcccgatca tcgacattcc ggcagcgccg 3840
ggcatcggaa actcgaccac cagcccgtcg tcaggcttct tcaacaccgg cgccggcacc 3900
gcatccgggt tcggcaacgt cggcggcaac acatcgggcc tgtggaacct tgcgtcggca 3960
gcctcaggag tctcgggctt gctcaacgtc ggcgcgttgg gatcgggtgt ggcgaatgtg 4020
ggcaacacca tctcgggtat ctacaacacg agcccgctgg acctcgggac gccggccttc 4080
ggctccggcc tcgcaaacat cgccggcctg ctgcagggcg gcgccggcac gacgatcctc 4140
gacttggccg gcctcggcaa cctcaatgtc ggcttggcaa acctcggggg ctctaacttc 4200
gggatcggga acaccggaat cttcaatgtc ggtttcgcaa acgtgggcaa ccacaacatt 4260
ggcttggcaa acctgggcaa ctacagcgtc ggcttcgcca actcgggcaa ctaccatatc 4320
ggcattgcta acaccggcag tgccaatatc ggcttcgcca acaccggtag cggcaatatc 4380
ggcatcgggc tcaccggcac cggtcagatc gggttcggca gcttcaactc gggcagccac 4440
aacatcggct tgttcaactc cggtgacgga aacgtaggat tcttcaactc gggcaccggc 4500
aacgtgggca tcggaaacac cggcaccgca aacttcggca tcgcaaactc gggcagcttc 4560
aacaccggcc tcgggaacac gggcagcacc aacacgggcc tgttcaaccc gggcaacgtc 4620

```
aacaccggcg tcggcaacac cggcagcatc aacaccggca gcatcaacac cggcagcttc    4680
aacactggca gcaccaatac cggcagcttc aacctcggcg atcacaacac cggcagcttc    4740
aactccggtg actacaacac gggctacttc aacgcgggtg actacaacac gggtgtggcc    4800
aacacgggca acgtcaacac cggcgcgttc atctccggca attacagcaa cggcttcttc    4860
tggcgaggtg actaccaggg gttgattggc ctttccacaa cgatcaccat tcccgaaatc    4920
ccctaccgct acgacttgag tgttccaatc gacataccca tcaccggcac cgtcgtcgcc    4980
accacgccaa acagtttcac cattcccggt ttccagatac gagtcttgct tggtcctgcg    5040
gcggtgcttg tcaacgagat gatcggcccc atcacgatcg atgtcaatca agtcatcgcc    5100
atcgattcgc ccattcagca aaccatcagc atggttggca ccggcggctt cggcccgatc    5160
cccatcggca tcagcatcgg tggtaccccg ggtttcggca actcgaccac cggcccgtcg    5220
tcgggtttct tccacaccgg cgccggccat gtatcgggct tcgggaactt cggcgccggc    5280
aacatgtcgg gctccgggaa cttcggcgct ggcaattcgg gcttctttaa cgccggcggc    5340
ttgggcaatt cgggcctact gaatttcggc gcgctgcagt cgggtctggc gaacctgggc    5400
aacaccatct cgggcgtcta caacacgagc acgctggacc tcgcgacgcc cgccttcggc    5460
tcgggcatcg caaacatcgg cgccaacctg gccggcctgt tcctcgacaa caccggcaac    5520
ctgacgctga acttcggcgt cgcaaaccag ggcggcctca acgcgggcat cgggaacctg    5580
ggcagcgtca acatcggctt cgttaatacc ggcgactcca acctgggcat cggcaacctc    5640
ggcgacctca acttcggcgg ggtcaacatc ggcggtaaca acatcggcat cgccaacacc    5700
gggatcttcg atatcggctt ggcgaacctg ggcagctaca acatcgggtt ggcaaatctg    5760
ggcgacgaca acctgggctt tggcaacgcc ggcagctaca acatcggctt cgcgaacttc    5820
ggcagcgaca acctgggctt tgccaacacc ggcagctaca acatcggctt cgcgaatacc    5880
ggtaacaaca acatcggcgt cgggctcacc ggcaacggcc agatcgggat cggcagcctc    5940
aactcgggca gcaacaacat cgggctgttc aactccggca gcggaaacat cggtttcttc    6000
aactcgggca ccggcaacgt cggcatcttt aacaccggca ccggcaactt cggtctcgcg    6060
aactcgggcg gcttcaacac cggcatcggc aacgcgggca gcaccaacac gggcgtgttc    6120
aaccccgggg acctcaacac cggcagcttc aacccgggca gcttcaacac cggcggcttc    6180
aacccggcca gtggcaacac gggctacctc aacaccggtg actacaacac gggcgtggcg    6240
aacacgggcg atgtggacac cggtgcgttc attaccggca gctacagcaa cggcttcttg    6300
gtgagtggcg actatcaggg cctgatcggc ctgccgctgt tgggcattcc ggtgaccccc    6360
ggctacttca acctcactgg cggcccgtcg tcgggcttct tcaacagcgg cgccggaagc    6420
gtatcgggat tcgtgaactc cggtgccggc ctgtcgggct acctcaatac cggcgcgctg    6480
ggatcgggtg tcgccaacgt gggcaacacc atctcgggct ggttgaacgc cagcgcgctg    6540
gatctcgcga cgccggggtt cctttccggc atcggtaact ttggcaccaa cctggcgggt    6600
ttctttaggg gataa                                                     6615
```

<212> Type : DNA
<211> Length : 6615
SequenceName : SEQ ID 518
SequenceDescription :
Sequence
--------
<213> OrganismName : Mycobacterium tuberculosis H37Rv
<400> PreSequenceString :

```
atgtcgttcg tgttgatcgc accggaattc gtgacagcag ccgcggggga tctgacgaat      60
ctgggttcgt cgattagcgc ggccaacgcg tcggcagcca gtgcgaccac gcaggtgctg     120
gctgcgggcg ccgatgaggt gtctgcccgt attgcggcgc tgttcggcgg gtttggcctg     180
gagtaccagg cgattagtgc gcaggtggcg gcctaccacc agcggtttgt gcaggccttg     240
agtaccggcg cgggcgcata tgcctcggcc gaggccgccg ccgctgagca gatcgtgctg     300
ggcgtgatca atgcgcccac ccaggcgctg ctggggcgcc cgttgatcgg tgacggcgcc     360
aatgcgacga ctcccggcgg ggccggcggg gccggcggtc tgctgttcgg caacggcggg     420
gccggggcag ccggggcgcc cggccaggcc ggcgggcctg gcgggcccgc cggattgtgg     480
ggcaacggcg ggcccggcgg ggccggcggc agcggtgggg gcaccggcgg tgccggcggc     540
gccggtgggt ggctgttcgg ggttggcggc gccggcggtg tcggtggggc cggtggcggc     600
accggcgggg cgggcgggcc cggtggtttg atctggggcg gcggcggggc cggcggtgtc     660
ggtggggccg gtggcggcac cggcggggcc ggcggccgcg ccgagctgct gttcggcgcc     720
ggcggtgcgg gtggggcggg caccgacggc gggcccggtg ctaccggcgg gaccggcgga     780
cacggcggag tcggcggcga cggcggatgg ctggcacccg gcggggccgg cggggccggc     840
gggcaaggcg gggcaggtgg tgccggcagc gatggtggcg cgttgggtgg taccggcggg     900
acgggcggta ccggcggcgc cggtggcgcc ggcggtcgcg gcgcactgct gctgggcgct     960
ggcggacagg gcggcctcgg cggcgccggc ggacaaggcg gcaccggcgg ggccggcgga    1020
gatggcgttc tggggggtgt cggtggcact ggtggtaagg gcggtgtcgg cggcgtggct    1080
ggcctcggcg gggccggtgg tgccgcgggc cagctcttca gcgccggagg cgcggcgggt    1140
gccgttgggg ttggcggcac cggcggccag ggtggggctg gcggtgccgg agcggccggc    1200
gccgacgccc ccgccagcac aggtctaacc ggtggtaccg ggttcgctgg cggggccggc    1260
ggcgtcggcg gccagggcgg caacgccatt gccggcggca tcaacggctc cggtggtgcc    1320
ggcggcaccg gcggccaagg cggcgccggc ggcatgggtg gctccggtgc tgataatgcc    1380
agcgggattg gcgccgacgg cggcgcgggt gggactggcg gtaacgccgg cgccggcggg    1440
```

```
gccggcgggg ccgccggcac cggaggaacc ggcggggttg tcggcgccgc gggcaaggcc 1500
ggtatcggcg gcaccggcgg ccaaggcggc gccggcggcg cgggcagcgc cggcacggat 1560
gcgaccgcta ccggtgccac cggcggcacc gggttttccg gtggagccgg cggggccggc 1620
ggggccggcg gcaacaccgg ggttggcggc accaacggct ccggcgggca aggcggcacc 1680
ggcggcgcgg gcggcgccgg tggtgctggc ggtgtcggcg ccgacaaccc caccggcatc 1740
ggcggcaccg gcggcaccgg cgggaaaggc ggcgccggcg gggccggcgg gcagggcggt 1800
agcagcggtg ccggcggcac caacggctct ggtggcgctg gcggcaccgg cggacaaggc 1860
ggcgccgggg gcgctggcgg ggccggcgcc gataacccca ccggcatcgg cggcgccggc 1920
ggcaccggcg gcaccggcgg agcggccgga gccggcgggg ccggtggcgc catcggtacc 1980
ggcggcaccg gcggcgcggt gggcagcgtc ggtaacgccg ggatcggcgg taccggcggt 2040
acgggtggtg tcggtggtgc tggtggtgca ggtgcggctg cggccgctgg cagcagcgct 2100
accggtggcg ccgggttcgc cggcggcgcc ggcggagaag gcggagcggg cggcaacagc 2160
ggtgtgggcg gcaccaacgg ctccggcggc gccggcggtg caggcggcaa gggcggcacc 2220
ggaggtgccg gcgggtccgg cgcggacaac cccaccggtg ctggtttcgc cggtggcgcc 2280
ggcggcacag gtggcgcggc cggcgccggc ggggccggcg gggcgaccgg taccggcggc 2340
accggcggcg ttgtcggcgc caccggtagt gcaggcatcg gcggggccgg cggccgcggc 2400
ggtgacggcg gcgatggggc cagcggtctc ggcctgggcc tctccggctt tgacggcggc 2460
caaggcggcc aaggcggggc cggcggcagc gccggcgccg gcggcatcaa cggggccggc 2520
ggggccggcg gcaacggcgg cgacggcggg gacggcgcaa ccggtgccgc aggtctcggc 2580
gacaacggcg gggtcggcgg tgacggtggg gccggtggcg ccgccggcaa cggcggcaac 2640
gcgggcgtcg gcctgacagc caaggccggc gacggcggcg ccgcgggcaa tggcggcaac 2700
gggggcgccg gcggtgctgg cggggccggc gacaacaatt tcaacggcgg ccagggtggt 2760
gccggcggcc aaggcggcca aggcggcctg ggcggggcaa gcaccacctc gatcaacgcc 2820
aacggcggcg ccggcggcaa cggcggcacc ggcggcaaag gcggcgccgg tggtgcggga 2880
accctgggcg tcggcggctc cggcggcacc ggcggggacg gcggcgatgc gggctctggt 2940
ggtggcggcg gcttcggcgg ggccgcgggt aaggccggcg gcggcggaaa cggcggccgc 3000
ggcggtgacg gcggcgatgg ggccagcggt ctcggcctgg gcctctccgg ctttgacggc 3060
ggccaaggcg gccaaggcgg ggccggcggc agcgccggcg ccggcggcat caacggggcc 3120
ggcggggccg gcggcaacgg cggcgacggc ggggacggcg caaccggtgc cgcaggtctc 3180
ggcgacaacg gcggggtcgg cggtgacggt ggggccggtg gcgccgccgg caacggcggc 3240
aacgcgggcg tcggcctgac agccaaggcc ggcgacggcg gcgccgcggg caatggcggc 3300
aacgggggcg ccggcggtgc tggcggggcc ggcgacaaca atttcaacgg cggccagggt 3360
ggtgccggcg gccaaggcgg ccaaggcggc ctgggcgggg caagcaccac ctcgatcaac 3420
gccaacggcg gcgccggcgg caacggcggc accggcggca aaggcggcgc cggtggtgcg 3480
ggaaccctgg gcgtcggcgg ctccggcggc accggcgggg acggcggcga tgcgggctct 3540
ggtggtggcg gcggcttcgg cggggccgcg ggtaaggccg gcggcggcgg aaacggcggt 3600
gttggcggtg acggcggcga gggagccagc ggtctcggcc tgggcctctc cggctttgac 3660
ggcggccaag gcggccaagg cggggccggc ggcagcgccg gcgccggcgg catcaacggg 3720
gccggcgggg ccggcggcac cggcggggcc ggtggtgacg gcgccccggc gaccctgatc 3780
ggcggacccg acggcggtga cggcggccaa ggcggcatcg gcggggacgg cggcaacgcc 3840
ggattcggcg ccggtgttcc cggcgacggc ggggacggcg gcaacgccgg attcggcgcc 3900
ggtgttcccg gcgacggcgg gatcggcggc accggcgggg ccgggggcgc cggcggcgcc 3960
ggcgccgacg ggaccccag cattgacggc ggccaaggtg gtgccggcgg ccacggcggc 4020
caaggcggca aaggcggcct gaacagcacc gggctagcca gcgccgccag cggtgacggc 4080
ggcaacggcg gggccggcgg ggccggcggc aacggcggcg acggcgacgg ctttatcggc 4140
gggtccggcg gcaccggcgg gaccggcggc gacgccggcg tcggcggcct ggccaacacc 4200
ggcggaaccg cgggcaacgc cggtatcggc ggggccggcg gccgcggcgg cgacggcggg 4260
gccggcgaca gcggcgccct ctcccaagac ggcaacggct tcgccggcgg ccaaggcggc 4320
caaggcgggg tcggcggcaa cgccggcgcc ggcggcatca acggggccgg cggcaccggc 4380
ggcaccggcg gggccggtgg tgacggccag aacggaacga caggcgtggc gagcgagggc 4440
ggcgccggcg gccaaggcgg tgacggcggc caaggcggca tcggcggggc cggcggcaac 4500
gccggattcg gcgccggtgt tcccggcgac ggcgggatcg gcggcaccgg cggggccggg 4560
ggcgccggcg gcgccggcgc cgacggggac cccagcattg acggcggcca aggtggtgcc 4620
ggcggccacg gcggccaagg cggcaaaggc ggcctgaaca gcaccgggct agccagcgcc 4680
gccagcggtg acggcggcaa cggcggggcc ggcggggccg gcggcaacgg cggcgacggc 4740
gacggcttta tcggcgggtc cggcggcacc ggcgggaccg gcggcgacgc cggcgtcggc 4800
ggcctggcca acaccggcgg aaccgcgggc aacgccggta tcggcggggc cggcggccgc 4860
ggcggcgacg gcggggccgg cgacagcggc gccctctccc aagacggcaa cggcttcgcc 4920
ggcggccaag gcggccaagg cggggtcggc ggcaacgccg gcgccggcgg catcaacggg 4980
gccggcggca ccggcggcac cggcggggcc ggtggtgacg gccagaacgg aacgacaggc 5040
gtggcgagcg agggcggcgc cggcggccaa ggcggtgacg gcggccaagg cggcatcggc 5100
ggggccggcg gcaacgccgg attcggcgcc ggtgttcccg gcgacggcgg gatcggcggc 5160
accggcgggg ccgggggcgc cggcggcgcc ggcgccgacg ggaccccag cattgacggc 5220
ggccaaggtg gtgccggcgg ccacggcggc caaggcggca aaggcggcct gaacagcacc 5280
gggctagcca gcgccgccag cggtgacggc ggcaacggcg gggccggcgg ggccggcggc 5340
aacggcggag ccggcgggct cggcgggggc ggtggcacag gcggcaccaa cggcaacggc 5400
ggcctcggcg gaggcggcgg caacggcgga gccggcggtg ccgggggaac gcccaccggc 5460
```

```
agtggcaccg aggggaccgg cggcgacggt ggagatgccg gcgccggcgg caacggcggc    5520
tctgccaccg gcgtcggtaa cggcggtaac ggcggtgatg gcggcaacgg cggcgacggc    5580
ggcaacggcg cacccggcgg cttcggtggc ggcgctggcg ccggcggctt gggcggctcc    5640
ggcgccggcg gcggcaccga cggcgacgac ggcaacggcg gcagccccgg caccgacggc    5700
agctaa                                                                5706
```

<212> Type : DNA
<211> Length : 5706
SequenceName : SEQ ID 519
SequenceDescription :
Sequence
--------
<213> OrganismName : Mycobacterium tuberculosis H37Rv
<400> PreSequenceString :

```
atgtcgttgg tgatcgtggc cccggagacg gtggcggccg cagccttaga tgtggcgcgc      60
atcgggtcat cgatcggcgc ggccaatgcg gcggcggcgg ggtcgaccac cagcgtgctg     120
gccgcgggcg ccgatgaggt gtcggcggcg atcgcgacgc tgtttggcag ccatgctcgg     180
gagtatcagg cgatcagcac gcaggtggcg gcgtttcatg accgatttgc gcagacgtta     240
agcgccgcgg tcggctcgta tgtcagcgcg gaggcgacca acgccgcacc gttggcgacg     300
ctggagcaca acgtgctcaa tgccctcaat gcgcccaccc aggcgttgct gggtcgcccg     360
ttgatcggtg atggggcggc tggagcaccc ggcaccgggc aggccggcgg ggccggcggg     420
atcttgtggg gcaacggtgg ggccggcggc tcgggcgcgc ccggccaagt cggcggggcc     480
ggcggggccg ccgggttgtt cggcaccggc ggggccggtg gggccggtgg ggccggcgcc     540
gccggtgggg ccgggggtag cggcggctgg ctgctgggca atggtggagt cggcggggcc     600
ggcgggcaga gcctgctggg cggggcaacc ggcggggccg gcggcaacgc cggactgttc     660
ggggtcggcg gaaccggcgg gcccggcggg cccggcgggc ccggcggggt cggcggtacc     720
ggtggtgccg gtggcctggg cgggaccctc tacggggccg gcgggcacgg aggtgccggc     780
gggcccggcc cgatcggtgg tgtgggtgga cacggcggtg tcgggggtgc ggccgggctg     840
ttgggcgtgg gcgggcacgg tggtgccggc ggacacggcg cggagggtgt ggccggcgca     900
gccggtgagg acttgtcccc gcacggtacg tccggtgggg tcggcggcga cgccggcgat     960
ggcggcaccg gagggcgggg cggctggctg gccggcgccg gtggggccgg cggggccggt    1020
ggggttggcg ggaccggcgg ggccggcggg gccggatttt ctcgtgcctt gattgtcgct    1080
ggggataacg gcggtgaccc cggcgccggc ggggccggag gcaccggcgg agccggctcc    1140
acgatcggtg cccacggcgc ggccggggcc agccccacca gcggcggcaa cggcggggcc    1200
ggcggcaacg gcgcccactt ctcatcgggc ggcaaagccg gcggtaacgg cggggccggc    1260
ggggccggcg ggctggtcgg caatggcggc gccggaggtg ccggcggcaa cggcgccccc    1320
ggtgccccgc cttctggcgg cgaccctaac ggcggtggcg gcggagctgg cggcgctggc    1380
ggtaagggcg gcgacggcgg agcccaagca ggcgacggcg gtgctggcgg cgccggcggt    1440
aagggcggca acggcggcaa cggcgccacc ggcgccaccg gccttaacgg cctgggggca    1500
ggcgcggatg gcaccgacgg cggcaagggc ggcaacggtg gagccggcgg tggcggtgga    1560
gccggtgggc aaggcggtaa ggcgcttgca gccacccacc aggacggcag catgggtgcc    1620
ggcggggccg gcggaaacgg cggcgccggc ggcatgggcg gtgatggcgg caacggtgcc    1680
aagggcacgt tcgataacgg cggcgatgga gttggtggca acggcggcaa cggcggtagc    1740
cgcggcatcg gtggtgctgg tgggatcggc ggcgccggat ccaccgcggg tgcagacggt    1800
gcccgcggcg ccaccccgac cagcggcggc aacggcggca ccggcggcaa cggcgcgaac    1860
gccaccgtcg ccggcggggc cggcggggcc ggcggcaagg gcggcaacgg cgggcttgtt    1920
ggtaatggcg gggccggcgg caaaggcggg gacggcatgg ccggtgtcgc cggttcctcg    1980
cccaccaccg cgggcgaatc cggcacgagc ggccagaacg gcggggctgg cggggcgggc    2040
ggggccggcg gccggggcgg agacttcggg ggcgacggcg ggaccggtgg ggccggcggc    2100
aacggcgcca acggcgccaa cgccaccacg ccgggcgcca agggcggcga cggcggccac    2160
ggcgggcccg gcgcgcaagg cggtaacggc ggccaaggcg ggcccggcgg tttggcgggg    2220
aacctctttg gccagaacgg aatccagggt gtcggcgggt ccggcggcaa aggggggggcc   2280
ggcgggctcg ccggtgacgg cggcaacggc gccaacggca acttcgcctt cggcgatggt    2340
aacggcggtc acggcggtaa cggcggtaac ccggcgccg gcgggcaggg cggtagcggt     2400
ggcgccggct ctaccccagg cgccaagggc gcccacggct tcactccaac cagcggcggc    2460
gacggcggcg acggcggcaa cggcggcaac tcccaagtgg tcggcggcaa cggcggcgac    2520
ggcggcaatg gcggcaacgg cggcagcgcc ggcacgggcg gcaacggcgg ccgcggcggc    2580
gacggcgcgt ttggtggcat gagtgccaac gccaccaacc ctggtgaaaa cgggccaaac    2640
ggtaaccccg gcggcaacgg tggcgccggc ggggccggcg gggccggcct gaacggcggt    2700
aacggtggcg ccggcggcaa cggcggcctc ggcggattcg gcggcaacgg cgccgccggt    2760
gccaacggcg tggccgtggg tgcgcccgga caacccggcg gtgccggcgg gcacggcggc    2820
gccggcggca acggcggggc cggcggcaac ggcggtcaag gcgtagtcag cgacggcgcg    2880
ggcggtgccg gtggggccgg cggcgacggc ggtgctcccg gtgacggggc caacggcggc    2940
aacggccagg gagcaggggc cttcgccggc ggcggcggcg ggcgaggcgg cgacggcggc    3000
aacgccggca acgccggtgc cggcggcccc ggcggcaccg gctccaccgc aggcaaggcg    3060
gggccggccg gcagcatcct gcacgacggc ggcaacggcg gtcacggcgg ccacggcgct    3120
gccagcggcg gaaacggcgg ccccggcggc cacgggggta acggcggtaa cggcggcacc    3180

ggcgccaacg gcggcaacgg tggcatcggc gggactggcg gcgccggcag caccggcgcc    3240
aagggcgtcc tcggcaccaa cgagggcgat ggcggtgacg gcggcagagg cggcaacggc    3300
ggcagaggcg gcaacggcgg ccaaggcctc accggagccg gcggcaacgg tgggaccggc    3360
gggacacccg gcaacggcgg caacggtggc aacggcgcca gtggcgacct tgtcacctca    3420
cctggtgacg gcggcggcgg gggccgcgga ggcgatgcag gacgcggagg cgatgcagga    3480
cttggcggtt ccagcggccc aggtggcacc cccggcgact ggggcaccgg cggcaccggc    3540
ggcaccggcg gcaccggcgg ccagggtgcc aacggcggcc tcaccggcgg cagaggcggc    3600
actggcggca acggcggcaa cggtaacacc ggcggcaccg gcggcgctgg cggcaccggc    3660
ggcaccggcc acaacggctc tcagcctggg atgggtggca acggaggtgc gggcggcttt    3720
ggcggcaatg gttttgccgg cgtcggtggc cggggcggga tggtggctc cggggggcacc    3780
ggcggcaccg gcgacgcggg gccctttgga acaggcaccg gtgggaccgg tggccacggg    3840
gggcagggcg gtggcggcgg cttcagcatc ctccttggtc tcggcggtct cggcggcctc    3900
ggtagcccgg ggtccatcgc cacaggtacc gccggcggcg ccggtggcgg cggtggcttc    3960
ggcgggctcg gcggcggcga attcgtgtag                                     3990
```

<212> Type : DNA
<211> Length : 3990
SequenceName : SEQ ID 520
SequenceDescription :
Sequence
--------
<213> OrganismName : Mycobacterium tuberculosis H37Rv
<400> PreSequenceString :

```
atgtcatatg tgatcgcgac gccggagatg atggcaactg cggcttttga tctggcgcgt     60
attggttcgc aggtgagcgc ggctagtgcg gtcgcggcga tgccgacgac ggaagtggtg    120
gccgccggcg ccgacgaggt gtcggcgggc atcgcggcgt tgttcagcgc gcacgctcag    180
gagtatcagg cgctaagtgc gcaggccgcg gcgtttcacg accagttcgt gcacacgctg    240
accgcggccg cgaggtggta cacggccacc gagatcgcca acgccgcggc gatgcgagtc    300
gtgctcggag ccgtgaatgc gcccacccag acgctactgg gacgcccgct gatcggcgat    360
ggtgcgcacg ggacagcgcc tgggcagccc ggtggggccg gtggattgtt gttcggcaat    420
ggcggcaacg gcgctgccgg tgccgtcggg caggtgggcg gcgccggcgg ggcggccggg    480
ttatttggga tcggcggcgc cggtggcgcg ggcggggcgg gcgcacccgg aggtaccggc    540
gggacgggtg gatggctggc gggtgggggc ggcgtcggcg gtatgggtgg ggctggtggt    600
ggcgccggcg gggcgggtgg caacgcgggc ctgttcggca acggcggcgc cggtggtgcc    660
ggtggggctg gtggtggcgc cggcggcgcg ggcggtaacg cggggtggtt tggtcatggg    720
ggcgctggcg gcgtgggtgg tgtaggtgcg gccggggcca acggtgctac gcccggtcag    780
gatggggcgg ctggtgttgc cgggtcggac gacggtgccg gcggtgacgg tttggcaggg    840
tcggacgggg gcgatggcgg tgccggtggg gtgggcggca acggtggtcg gggagggtgg    900
cttctcggta acggcggcgc cggtggcgtt ggcggtgtcg gcggggccgg tggtgccggt    960
gcggccggcg gtgccggcgg tgccggggct accggaataa atgggccggc cggtatctcg   1020
gcggccggcg gtgacggcgg cgccggcggc aatggcggtg ccggcggaaa cggcggcgtg   1080
ggcggcgccg gggtgctggc cgggtcggct ggactattgg ggtatgtcgg ccgggccgga   1140
gacggcgggg ctggcggggg tggggggctg ggtggagcgc ctggtgacgg cggtgccggc   1200
ggcaacggtg gcagttggct ggccgccggt gacggcgggg ccggcggtca cggtggcgac   1260
ccgggcctgg gtggggctgg cggagccggg gggcgtcgg gcggcgccgg tgctcgcgcg   1320
ggggccaatg gtctggcggc cggcaacgac gggccggtca gcggcggcaa cggcggcaaa   1380
ggtggcaatg gcgcccacgc accggtcgcc ggcggtcatg gcggtaacgg cggtgccggt   1440
ggcaacggcg ggttggtcgg tgacggtggg gccggcggtc atggcggtga cggagccgcg   1500
ggtgccggct atgccgatat gacggcgatc ttcctgggtt catccggtac cccggtgag   1560
gatggaggta acggcggggc cggtggggcc ggcggcgcgg gtggggccca cgccggcgat   1620
ggcggggccg gcggtgccgg cggcaacggt ggggccggcg gggccggtgg taacggagct   1680
cacggtttca atgctgtgct cgtatctgac ggcggcaacg gtggtgatgg cggagccggc   1740
ggtcgcggcg gtgacggtgg ggccggtggg gctggcggtg acgcacctgc gggtcgggcg   1800
ggcagccagg gtgttggcgg ggacggtggt gccggcgggg ctggtggggc gcccggtaac   1860
gggggcagcg gtggccgcgg cgacatggct ttcaaggatg gtgacggtgg ggccggcggc   1920
gatggtggtg acccaggcgc cggtgggaag ggtggcgccg gcggcgccgg cgccaccgag   1980
ggtgtgaccg gcgcgaccgg cgctaccgtg cacagtggtg gcaacggcgg caagggcggt   2040
aacggcgccg acgccaccgt ggctggcgcg aacggcggca agggcggtgc cggtggtaac   2100
ggcgggttgg tcggtgacgg cggggccggc ggcgacggtg gtagcggcgc ggccggtgcg   2160
aatggcgcga acgtaggtga ggacggcgcc gacggcaccc tctcggggca accgggcgaa   2220
ggaagcgagg ccaacggcgg tcaaggcggc gttggcggtg gcggcgcggg cggtgccggc   2280
ggtgacggtg gtgccgggag ttcggcgtta ggtagtggcg gcaacggcgg tcgaggcgat   2340
gcggggcagg ccggcggcgc gggcggtgcc ggcggagccg gtggcgccgg tgggtcggtg   2400
tcgggcgacg gtggacccgg tggcaagggc ggggccggcg gtgcgggtgg tgccggagct   2460
agtggtggcg gcggcggcaa gggcgcctcc ggcgccgaca gcgctgaggc cgttggaggc   2520
gccggcggga aaggcggcga cggcggtgtc ggcggcgttg gcggtgacgg cgggcccggc   2580
ggtgatggcg gcgccggcgg ggccgcaccc gcgggtcagg tcggcagcca cggtgtcggc   2640
```

```
ggcgttggcg gtgacggcgg gctcggcgga gccggcggca acggcggtga cggcggtcat   2700
ggcagtgatg gcggggacgg cggtgacggc ggtgaccccg gcgcgggagg cctcggcggc   2760
ttgggcggcg acagcggcaa cggcacccgc gcggccagcg gtgtggacgc cagcgaccac   2820
gggcccggca gcggcggcaa cggtggtaac ggtggcaacg gtgcgcaagc cagcgtcgcg   2880
ggcggcgccg ggggcaacgg aggcgacggc ggcaatgccg gccgggtcgg cgatggtggc   2940
gccggcggca atggtggcga tggcgcggcc ggcgccaacg ggccaattc gggcgcgcct    3000
ggttcagatg ccctcgccct cggccaaccg ggaggcaacg ggggtcaggg cgacgcgggg   3060
caggccggcg gtgccggtgg tgccggtggt gccggtggcg ccggtgggtc ggtgtcgggt   3120
gacggcggtg ccggcggcaa cggcggggcc ggcggcaacg gcggtgtggg cgctagcggt   3180
ggggccgggg ccagggcgc caacggtatc gacagtatcg gcggcaccgg tggggccggc    3240
ggtggtggcg gcgatggcgg tgccggcggg gtcggtggac atggcgggga cggtggggtc   3300
ggcggggccg caccttcggg gacggtcggc agtcacggca ccggtggggt cggtggcgac   3360
ggcggactcg gcggcgccgg cggcgttggc ggggccggcg gcaacggcgg cattggtatc   3420
accgtcggcg gcgccggcgg agcggccggt aacggcgctg [illegible] aggcggtcgc   3480
ggcggtctgg ggggtgacag cggcaacggc acctccgcgg ccaacggcgt ggacgccagc   3540
aaacacgggc cactgaccgg cggcgacggc ggcgtcggcg gcaacggtgc caaggccgcc   3600
gcggccggcg gcgacggcgg ccagggcggt gacggcggca acgccgggct attcggggac   3660
ggcggagccg gtggtgatgg ggccgacggc accgctgccg aagctctcgg cggtgatggc   3720
ggggccggtg gggctggagg caagggcggt gacgccggcg acatcggtga cggcggtgac   3780
ggcggcaagg gtggcgacgg cgcgcacggt gccctcggag ggctcaccgt tgctggcggc   3840
aacggtgggg ctggtggcgc cggtggggct ggtggcgccg gcggagcatt tctgggcgac   3900
ggcgggaacg gcggagccgg cggccaaggt ggggctggcc ggggcggcag ccccggcggc   3960
ggcggcgggg ttggtggaca cggcggggcg ggcggcgacg ccgggatgaa cggcggcggc   4020
ggaaccggcg gccagggagg caacggcgcg gccggtggcg cgggttggtc gcccgactcc   4080
gacctaaagg gcttcgacgg cttcgacggc ggcagcggtg gggcgggagg cgacggcggg   4140
gccggtggcg ccggcggaac tcagaccggc gacggtggtg acggcggggc cggaggccta   4200
ggcggggctg gtggggtcgg cggtaacggc gttgacggct ttgacattaa cgaaacgacg   4260
ggccgcgacg gcggcgacgg cggcgacggt ggctacggcg ggtggggcgg cgccggcgga   4320
aacggcgggg ccggcgggtc ggcacccgcc ggcgaggtcg gcaatcgagg cgttggcggg   4380
gacggcggtg acggcgggtc gggcggggac gctggtaatg gtggcttggg cggtgacggc   4440
tttacctatc tcgcggattt tgacggagag cctggcggcg acggaggtga cggcggcgac   4500
ggcgggtggg gccgccccgg cggacaaggc ggtttcggtt ccacaagtgg cgcgcacggc   4560
aaggccggct tcggcgcccc cggcggtgac ggaggcgacg gcgggaacgg cggtcacggt   4620
ggggacggca acggcagctt tgctgatgca ggcgacggcg ggccgggtgg caacggcggc   4680
aacggcggct tgggtggggc cgggcgagac ggtggcgccc ccggtggtga cggaggcgac   4740
ggcgggaccg gcggctccgg cggcttcggc gcccctccgc ctcgcagcat cggcggcggt   4800
gacggcgggg acggcgggcg cggaggtgac ggcggacgcg gtgccggtgg cttgacctcc   4860
ggcggcgtcg gctcgtcggg cgagtcgggt ggctccggca acgggagggg cgaccctggc   4920
tccggcggtt ccggcggtga aggcggcgaa ggcggcccct ccatctccgt taacgtcacg   4980
tag                                                                4983
```

<212> Type : DNA

<211> Length : 4983

SequenceName : SEQ ID 521

SequenceDescription :

Sequence

--------

<213> OrganismName : Mycobacterium tuberculosis H37Rv

<400> PreSequenceString :

```
atgtcgtttg tgttggtttc gccggagacc gtggcggcgg tggccacgga tctcaagcgc     60
atcggcgcct cgctggccca cgaaaacgcg tcggcggccg cttcgacgac ggcggtggtc    120
tccgcggccg ccgacgaggt atcgacggcg gtcgccgctc tgttctccca acacgcccag    180
ggctaccaag cggcggccgc tcaggtagca gcgtttcata gccggtttgt gcaagccctg    240
acggccggtg ccggggcgta cgcatttgcc gaggcggcca acgcgtcgcc gctacagtca    300
gccatgggtg cggtaagcgc gtctgcgcag acgctgttgt cgcgcccgtt gatcggcaat    360
ggcgccaatg cgacgacgcc gggcggtaac ggcggcgacg gcggatggct attcggcagc    420
ggcggcaacg gcgcgcccgg cgcggcgggc cagtccggcg gtaacggcgg gtcagccgga    480
ctgtggggta acggcggcgc gggtggcgcc ggcggcagcg gcggcgccgc cggcggcaac    540
ggcggtaacg gcgggtggct gttcggcgcc ggcggcaccg gcggtatcgg cggcaccggt    600
gctcccggcg ccatgggcgg caccggcggc aacggcggca acggcgcgct gctgatcggc    660
ggcggcggcc tcggcggcgc cggcggcatg ggtggcaccg gcggcggcac cggcggcacc    720
ggcggcaacg gcggcaacgg cgcgctgctg atcggcgctg gtggtgtcgg aggtgctggc    780
gggatcggtg gccagggtac cggcgccggc ggtgccgccg gcgccggcgg caccgggggc    840
aacggcggcg ccgggggtt gttcatgaac ggcggcgacg gcggcgccgg cggtcaaggc     900
ggcgacggtg cggccggcga cgcggctgcc agcgccggcg gcaccggcgg caaaggcggc    960
caaggcggcg acggcggcac cggaggggcc ggcggcgcag gcccagtgct gttcggccac   1020
ggcggcgccg gcggcatggg cggccaaggc ggcaccggtg gaatgggcgg cgccggcgga   1080
```

```
gacggcacca ccgtcatcgc ggccggtacc ggggggggagg gcggcaccgg cggcgcggcc     1140
ggcgccggcg gagccgcagg cgctcgcggg gctctcacca gcggcggcct agccggcggc     1200
gtcggggccg gcggcaccgg cggcaccggc ggtaccggcg gcaacggcgc tgacgccgct     1260
gctgtggtgg gcttcggcgc gaacggcgac cctggcttcg ctggcggcaa aggcggtaac     1320
ggcggaatag gtggggccgc ggtgacaggc ggggtcgccg gcgacggcgg caccggcggc     1380
aaaggtggca ccggcggtgc cggcggcgcc ggcaacgacg ccggcagcac cggcaatccc     1440
ggcggtaagg gcggcgacgg cgggatcggc ggtgccggcg gggccggcgg cgcggccggc     1500
accggcaacg gcggccatgc cggcaacaca ggtgacggcg gcgacggcgg gaccggcggt     1560
aacggcggca acggcaccgg aggcgtgaac ggcgccgaca acaccctcaa ccccgacacc     1620
cccggcggcg ccggggagcc cggcggggcc ggcggggccg gcggggccgg cggggccgcc     1680
ggcggcccgg gcggtaccgg cggtaccggc ggtaacggcg gcaacggcgg caacggcggc     1740
aacggcggca acggcggcaa cggcggcaac ggcggcaatg ccggcaacaa cagcaccaat     1800
gccccagtcg gtggcgaagg cggcgccggc ggcgacggcg gcgccggcgg cgcaggcggg     1860
gccgccaacg gcggcaccgc gggcagccag ggcactgggg gcgtcggcgg cgacggcggc     1920
gcgggcggca acggcggcgg cggcaaggct ggcaccggca acagcggcaa ctttggggtg     1980
gacggcgaag ccggcttcag cggcggcgcc ggtggcaacg gcggcgtagg cggggccgcc     2040
ggcgccaatg gcggaaccgg cggcagcggt ggtaatggcg gtgacggcgg tgcgggaggc     2100
attggcgggg ccggcggcaa cggcataccg ggcactggca cagagcctgc cgggggcacc     2160
ggcgccaaag gtggagacgg cggcgacggt ggcgccggcg gcgcaggcgg caatgccggc     2220
ggggccggcg gccagggcgg caatgccggc cagggtggcg ccggcggtgc gggcggcaac     2280
gccgtgattc ccggcgacgg cgtcgggaag gcgccgcacg gcgacgcggg cggcagcggc     2340
ggagacggcg gcaaaggcgg ccagggcggt agtggcggca ccggcggatc cggtgccccg     2400
atcggtggcg gcgccggagg caccggaggg tccggcggac acgccggcaa gggtggcgcc     2460
ggcggcatcg gcgcacaggg caccaccatc accgtgcccg ggaacggcgg caacgccggc     2520
gacggcggca acggcggcaa cgccggcgcc ggtggaaacg gcggctccgg cgacttcggt     2580
ggcaatacca ccagcggcgc ctccggcagc ggcggcaacg gcggcaacgc cggcaccgcg     2640
ggtagcggcg gtgcgggcgg aaccggcggc accggcctta gcggcggcaa cggtggcaac     2700
ggcggcaacg gcggcaacgg cggtgacggc ggtaacggcg cccacggcac cgtcggcgcc     2760
cagttcgtcc cggccaccag cttgcccaca cccaacggcg gggccggtgg caacggtggc     2820
accggaagca acggcggcgc gcccggcccc gccggggcgc ccggccccac taccggcggt     2880
aacgctggca gccagggcat cggcggcgac gggggcaacg gcggcgacgg cggtaaaggc     2940
ggtgacggcg ccgacgctgt caacgtcgta ttcatgccga ctgagccaca ggccgcgacc     3000
ggcactgccg gcagcgccgg tgaccccacc ggcggtaacg gagggcccgg cactcccggc     3060
agccccatgg ttgccccgcc cccgccaacg ccaatcactc aagtccaaca gggcggtgac     3120
ggtggcgccg ggggcaccgg atccaccaac gccaacgacg gcacagccac cggcggaaag     3180
ggcggagaag gcggagtcgg cagcattctc ggcgggcccg gcggcaacgg cggaactggc     3240
ggcaacgcct cggcaaccgg caccaacggg gtggccaacg ccgggaatgg cggcaagggt     3300
ggcgacggcg gccagtttgg ggccggcggc aacggtggtg ccggcggcag cgtaaccgac     3360
ggatccgccg gcagcaccgc aggcaacggc ggcaacggcg gcaacgcaac caacggcacc     3420
atcgcaggcc aacccgccgg cggcaacggc tcggccggcg ggaaaggcgg cgacggcggc     3480
aacatcgccg ccggtgccac cggcaccgcc ggcaacggcg ggaacggcgg caacggcaac     3540
gacggcgccg tcaacgccgg caccggcggc tccggcggga acggcggtaa cgccggtggc     3600
ggcggcgcca atggcggcga cggcggcgcc ggcggcgccg gcggggccgg cgggcgtggc     3660
ggcaagggca tcgacggcgg gttcggcggt gacggcggca acggcggcag caacaacggc     3720
accggcgccg gtggcaacgg cggcaacggc ggcaccggcg gggtcggctc ggttggcgcg     3780
gctggtggcg atggcggcaa cggcggcacc ggcggcttcg ccggtttcgg cggcaccgca     3840
ggcaatggcg gttccggcgg cacgggcggg gccggcggcg acggcggcac cggcggggac     3900
ggcggcaacg gcgttatcgc cggcggcggg gggaccggcg gcaacggcgg cgccagcggg     3960
gccggcggcg ccggcggcac gggcgggttc gccggcaacg gcaatgccgg cggcaatggc     4020
ggcaccggcg gcgcgagcga ggacggcgac aacggcaacg ctggcagcgg cgccaccggc     4080
ggtaccggcg gcaacggcgg caccggcggc gacggcggcg ctgccgggct gggcggcgtc     4140
gcgtga                                                                4146
```

<212> Type : DNA
<211> Length : 4146
SequenceName : SEQ ID 522
SequenceDescription :
Sequence
--------
<213> OrganismName : Mycobacterium tuberculosis H37Rv
<400> PreSequenceString :

```
atgtcgttcg taatcgcgac gccggagatg ctgaccacgg cggcaacgga tttggcgaaa      60
attggttcga cgatcactgc ggccaacacc gcagcggccg cggtggcgaa agtgctgccc     120
gcgtcagccg acgaggtgtc ggtggccgtt gcggcgttgt tcggcacgca cgcccaggaa     180
tatcagaccg tcagcgccca agtggcgacg tttcatgacc ggttcgtgca gaccctgtcc     240
gcggccgcga gctcgtacgt ggccgccgag gcggtcaacg ttgaacagag tttgctagcc     300
gcggtcaatg cgcccaccca ggcgctgttc ggacgcccgc tgatcggcaa cggcgccgac     360

ggctcccccg ggaccgggca ggccggcggg ccaggcggca tcttgtacgg caacggcggc     420
aacggcgggt ctggggcgcc aggacaacga ggcggggccg gcggggcggc gggcctaatc     480
ggtaacggcg gcaacggtgg agccggcggc gtgggtacca ccggcggggc cggtggtcac     540
ggcggcgcgg gcgggtggct gtatggcaac ggaggcgccg gggttttggc cggggccggg     600
gcggtcggcg gcaacggcgg ggccggcggt accgccgggt tgttcggtgt cggcggggcc     660
ggtggggccg gaggcaacgg catcgccggt gtcacgggta cgtcggccag cacaccgggt     720
ggatccggca ccgctggcgg ggccggcggg atcggcggca acggcggggc cggcggggcc     780
ggcggggtgc tgatgggcaa cggcggcaac ggcggggccg gcggcgaggg cgggcccggc     840
ggcgccggcg gtgcgggcgc cagcggcgcc cacgccacca acttgggcgc tgacggtcaa     900
gccggcggaa acggcgggaa tggtggggct ggcgggaccg gcggggtcgg cgggcccggc     960
ggcggccacg gtctgctcgg cctcggcggc agccacggcg ccggcggggc tggcggtagc    1020
ggcggtgacg gcggagctcc cggtgacggc ggcaacggcg ctaccgggac gtggggtcac    1080
aaccttggtg ccggcgggac cggtggcaat ggcggcaacc cgggcgccgg tggggccggt    1140
ggcgccggtg gcgccagcgt cggcggcagc gcgcatggcg cgaacggcgc gcccggcacc    1200
acctccacca gcggtggtaa cggcggcgac ggcggcaaag gcgccgacgc tatcagcagc    1260
ggacagaccg gcgcgaacgg tggcaggggt ggcgacggcg gtcaggtggg taatggcggt    1320
gccggtgggg ccggtggccg cggcggggcc ggcggtctcg gatttggatc cgaagcgccc    1380
ggtcgccccg gcggggccgg cggcaccggc ggggccggcg gcaacggcgg aactcaggcc    1440
ggtgacggcg gcaccggagg tgctggcggg gccggcggcg atggcggatc cggaggtgct    1500
ggcagcatcg gcttcaatgc gtccgctccc ggggccgcag gctcacccgg cgggaatggc    1560
ggtaacggtg ggcctggcgg agccggcggc gagggcgggg ccggcggtct cgcgttggcc    1620
gcctcgggcc agaacggcag ccagggcgct ggcggtgacg gcggagccgg cgggaacggc    1680
gggactcctg gcaacggcgg tcacggcgcc gccggcgcgc tcggtgtcaa cggtggtgta    1740
ggtggcgccg gtggccacgg cggtgatccc ggtgtcggcg gtgccggcgg ccagggcgga    1800
agcggctcca cccccggtgc caacggcgca cccggcaaca cccccaccag cggcggcaac    1860
ggcggcaacg gcggcagagg cgccgatgcc accggctttg gccagaccgg cgcgtccggc    1920
ggcaggggcg gtgatggcgg tttggtcggc aacggcggcg ccggcggcgc cggcggcaac    1980
ggcagcaaag gcctgcccgg tctgggcagg ctcggcaacc ccggcctgga tggcggcacc    2040
ggcggaaacg gtggggccgg tggatccggc ggcgcctggg cgggcaacgg tggcaccggc    2100
ggggccggcg gcaccggcgg cgtcggcggg actggagggt ccggcagcga cggtgtcaac    2160
ggttccagcg cgggcgcgga cgggcacccc ggcggcaccg gcggagttgg cggtaccggc    2220
ggaaaaggtg gggacggcgg ggacggcggg gccgcaccca acggcgtcgc cggcagccaa    2280
ggccccggcg gtgccggcgg cgacggtggg accggcggag ttggcggtaa cggcggccgc    2340
ggcattgacg gcgccgacgg cgccaccgcg ggtgcccgcg gccaggatgg cggcgccggc    2400
ggggccggcg gaaagggcgg ccgaggcggg accggcgggc cgggcggggc cgggccggcg    2460
ggcacaaccg gcagccaagg cgccggcggc aacggcggca gcggcggcac cggcggggac    2520
cccggtgacg gcggcaacgg cgctaacggc agcgtgttca ccaacaacgg catcggcggc    2580
aacggcggca acggcggcaa cgccggcccc agcggggccg gcgggagtgg cggcgccggc    2640
tccactttcg gtgcgaccgg ctcaagcagc agcattcatg tcaacggcgg caacggcggc    2700
aacggcggca acggcgacca tgccctcagc ggcaacggcg cggccggcgg caacggcggc    2760
aacggcggca acggcagcct gcgcggcagc ggtggggccg gcggccacgg cggcaacggc    2820
ggcaatgcca gcaggggcat gggcggtgac ggcggaaccg gtggcgccgg cggaaacgcc    2880
gggcagattg gcaacggcgg agcgggcggc aatggcggag acggcggcac cggtagtgac    2940
ggcaatcccg gagccattac cggcagcggc ggccgcggcg gcgacggcgg cgtaggcggg    3000
caaggtggga gcgtcgccgg cgacggcgct gacggcggcc ggggcggggc cggcggcacg    3060
ggcgggacgg gcctgcgggg caccaccggc gccaccggtg cgaccggcac attcgacgct    3120
ggcgcggacg gtcacggcgg taacggtggc accggcgggg ttggcgggac cggcggcgcc    3180
ggcggcggcg gcggcaacgg cggggccggc ggcaaagcgc tgtcgccaac gggcaacaac    3240
ggcagccaag gcgccggcgg agatggtggg gccggtggcg ccggcggcac cggcggcacc    3300
ggcggcgacg gcggcagagg tgcccacggc actctcttca gcagcctcgc tggcactggc    3360
gggactggcg gaaatggcgg caccggcggc accggcggca ccggtggtgc cggcggtgcc    3420
ggcgggaccg gttccaccct gggcgcgacc ggtgcgacag gggcggccgg ccgcgccgga    3480
aacggtggcg tcggcggcag tggcggcctt ggctccgcct ttggccccgg cggcaccggc    3540
ggcatgggcg gcgccggcgg caccagcacc gtcagcgccg gcggtgacgg cggaaggggc    3600
ggcttcggcg gcgacggcct tgatgccagt tccggtggga atggcgggga cggcgggcac    3660
ggtggcgacg gcttcaggac cgctggggcg ggcgggcgag gtggggacgg cggcaaaggg    3720
gccgaccccg gcggtttatt cccgatccct ggggctggcg gtaagggtgg caccggtggc    3780
accggcggca ccgcacacct cgggcccctg gccatcatcg gccaatccgg ccagcccggc    3840
cagttcggca gccccggcgc cgacggccgc ggcggggccg gggcgcagg cgggggcggc    3900
ggcgccggcg gcagcttcta g                                              3921
```

<212> Type : DNA

<211> Length : 3921
SequenceName : SEQ ID 523
SequenceDescription :
Sequence
--------
<213> OrganismName : Mycobacterium tuberculosis H37Rv
<400> PreSequenceString :

atgtcggcgg ccgcggtcgc ctgggaccaa ctggcgatgg aattggcctc ggcagcggcc 60
tctttcaact ccgtgacgtc gggcctggtc ggcgaatcgt ggctcggacc gtcatcggcg 120
gcgatggccg ctgcggtagc gccgtaccta ggatggcttg ccgcggcagc ggcccaggcc 180
cagcggtcgg caacccaagc cgcggccctc gtggccgagt tcgaggctgt ccgggcggcg 240
atggtgcaac cggcgctggt ggcggccaac cgctccgacc tggtgtcatt ggtgttctca 300
aacttcttcg ggcagaatgc tccggcgatc gctgcgattg aggccgcata cgaacagatg 360
tgggccatcg atgtgtcggt gatgtcggcc taccatgccg gggcatcggc ggtggcgtcg 420
gccctgacgc cgttcactgc gccgccgcag aatctgacag acctgcccgc ccagttggcg 480
gccgctccgg cggccgtcgt caccgcggcg atcaccagtt ccaagggtgt gctggcaaat 540
cttagcttag gcctggcaaa ctcgggcttc ggacagatgg gcgccgctaa ccttggcatt 600
ttgaacttgg gcagcctaaa tcccggcggc aacaacttcg gccttggaaa tgtcggcagc 660
aacaacgttg gcttgggcaa caccggcaac ggaaacatcg gcttcggcaa cacgggcaac 720
ggaaacatcg gcttcggcct caccggcgac aaccagcagg ggttcggcgg ctggaactcg 780
gggaccggca atatcggctt gttcaactca ggcaccggca acatcggcat cggcaatacg 840
ggcaccggaa acttcggcat cgggaactca ggtaccagct acaacacggg tatcggcaac 900
acgggccaag ccaacacggg cttcttcaac gccggcatcg ccaacactgg catcggcaac 960
acgggcaact acaacacggg cagcttcaat ctaggcagct tcaacacggg cgacttcaac 1020
acgggcagct ccaacacagg cttcttcaac cccggcaacc tcaacaccgg cgtgggaaac 1080
accggcaacg tcaacaccgg tggattcaac tctggcaact acagcaacgg cttcttctgg 1140
cgaggcgact accagggctt gatcggcttc tccggcacac tgaccattcc cgctgctggc 1200
ctagacctca acggcctcgg ctccgtcggc cccatcacca tcccgtccat caccattccc 1260
gaaatcggcc tgggcattaa cagttccgga gcgttggtgg ggccgatcaa tgttccgcct 1320
attactgttc ccgccatcgg gctgggcatc aacagtaccg gggcactcgt tggccccatc 1380
aacatcccgc cgatcactct aaattctatt ggcctagagc tatcggcgtt ccaggtcatt 1440
aacgtgggat cgatttcgat ccccgcgtct ccgcttgcga tcggcttatt cggcgtaaat 1500
cccaccgttg gcagcatagg cccgggtagc atatcgatac agctaggcac tcctgagatt 1560
cccgcgattc cgccattttt ccccggattc cctccagatt acgtgacagt gagtggtcaa 1620
atcggtccca tcaccttctt atcgggtggt tatagtttgc cggctattcc gttgggtatt 1680
gatgtgggtg gagggttagg cccgtttacg gtgttcccgg atggctactc acttccggca 1740
atcccgttgg gtattgatgt gggcggaggg ttaggcccgt ttacggtgtt cccggatggc 1800
tactcacttc cggcaatccc gttgggtatt gatgtgggcg gagggttagg cccgtttacg 1860
gtgttcccgg atggctactc acttccggca atcccgttgg gtattgatgt gggcggcgcc 1920
atcggccccc tcactacccc gccaatcacg atcccctcaa tcccgttggg catcgacgtg 1980
tccggcagcc tcgggccgat caacatcccg atcgaaatcg cgggcacccc aggcttcgga 2040
aactcgacca ccaccccgtc atcgggcttc ttcaacagcg gtacgggcgg cacatcgggt 2100
ttcgggaacg tcggttcggg cggatctggc ttctggaaca ttgctgggaa tctcggcaac 2160
tccggattcc ttaacgtcgg gccactgaca tcgggaatct tgaacttcgg caacacagtc 2220
tcaggcctct acaacaccag cacgctgggc ctagcgacat cggcctttca ctccggcgtc 2280
ggtaacaccg acagccaact cgccggcttc atgcgcaacg ccgcaggtgg gacgttattc 2340
aacttcggct tcgccaacga cggcacactc aacttgggca acgcaaacct cggcgactac 2400
aacgtgggta gcggaaacgt cggtagctac aacttcggta gcggaaacat cggcaacgga 2460
agtttcggtt tcggaaacat cggaagcaac aacttcggtt ttggaaacgt cggcagcaac 2520
aaccttgggt ttgcaaacac gggtccgggg ttgacggagg ccctgcacaa cattggcttt 2580
gggaacatcg gcggcaacaa ctacggcttc gcgaacatcg gtaacggcaa catcggcttc 2640
ggcaacacgg gcactggaaa tattggtatc gggctcaccg gcgacaatca ggtcggattc 2700
ggggcgctga actccggcag cggcaacatc ggcttcttca actccggcaa cggcaacatc 2760
ggcttcttca actcaggcaa cggaaacgtc ggcatcggca actccggcaa ctacaacacc 2820
ggcctgggta acgtgggcaa cgccaacacg ggcctgttca acaccggcaa cgtcaacact 2880
ggaatcggca acgcaggaag ctacaacaca ggcagctaca acgccggcga caccaacacg 2940
ggcgacctca acccgggcaa cgccaacacg gggtacctaa acctcggcga cctcaacacc 3000
ggctggggaa acattggcga ccttaacacc ggcgccctga tctcgggcag ctacagcaac 3060
ggcatactgt ggaggggcga ttaccagggt ctgattggct actcagacac actcagcatt 3120
cccgccatcc cactgagcgt cgaagtgaat ggtggcatcg gtccgattgt ggtgccggat 3180
attactattc ctggtattcc gttgagcctg aacgcgctgg gtggtgtcgg tccgattgtg 3240
gtgccggata ttactattcc tggtattccg ttgagcctga acgcgctggg tggtgtcggt 3300
ccgattgtgg tgccggatat tactattcct ggtattccgt tgagcctgaa cgcgctgggt 3360
ggtgtcggtc cgattgtggt gccggatatt actattcctg gtattccgtt gagcctgaac 3420
gcgctgggtg gtgtcggtcc gattgtggtg cctgatatta ctattcctgg tattccgttg 3480
agcctgaacg cgctgggtgg tgtcggtccg atcaccgttc ccggcgtccc tatttcccgc 3540
atccccctta cgattaacat caggataccg gtcaacatca ctctcaacga acttccgttt 3600
aacgtcgctg gtatcttcac gggctacatc ggccccatcc cgcttagcac attcgtatta 3660
ggcgtcacgc tggccggcgg caccctggag tctggcatcc agggattcag tgttaatccg 3720
ttcggtttga atattccgct gagcggtgct accaacgctg tcacgatccc tggtttcgcg 3780
attaatccgt ttgggttgaa tgttccgttg agcgggggca cgagcccggt tacgatccct 3840
ggtttcgcga ttaatccgtt tgggttgaat gttccgttga gcgggggcac gagcccggtt 3900

```
acgatccccg gcttcaccat tcccggatcc cccctgaact tgaccgccaa cggcggcttg   3960
ggaccgatca acatcccgat caacatcacg agcgccccgg gcttcggaaa ctccaccacc   4020
accccgtctt cgggcttctt caacagtggc gatggaagcg catccggctt cggcaacgtc   4080
gggcccggca tttcgggcct ctggaaccag gtgccgaacg cgctgcaagg cggagtctcg   4140
ggaatctaca acgtcgggca gctggcgtcg ggcgtggcga acctaggcaa caccgtctcg   4200
ggcttcaaca acacgagcac cgttggtcac ctcaccgctg cgtttaactc gggcgtcaac   4260
aacatcggcc aaatgctcct gggcttcttc tcaccgggtg ccgggccgta a            4311
```

<212> Type : DNA
<211> Length : 4311
SequenceName : SEQ ID 524
SequenceDescription :
Sequence
--------
<213> OrganismName : Mycobacterium tuberculosis H37Rv
<400> PreSequenceString :

```
atggagtttc cggtgttgcc accggaaatc aactccgtgc tgatgtattc gggtgcgggg     60
tcgagcccgt tgctggcggc ggccgcggcg tgggatgggc tggctgagga gttggggtcg    120
gcggcggtgt cgtttgggca ggtgacgtcg ggcctgacgg cgggggtgtg gcagggtgcg    180
gcggcggcgg cgatggcggc cgcggcggcg ccgtatgcgg ggtggttggg ttcggtggcg    240
gccgcggccg aggcggtggc cgggcaggcg cgggtggtgg tgggggtctt tgaggcggcg    300
ttggcggcga cggtggatcc ggcgctggtg gcggccaacc gggcgcggct ggtggcgttg    360
gcggtgtcga atctgttggg gcagaacacg ccggcgatcg cggccgccga ggccgagtac    420
gagctgatgt gggccgccga tgtggcggcg atggccggct accattccgg cgcgtcggct    480
gctgccgcgg cgttgccggc gttcagccca ccggcgcagg cgctgggggg aggtgtcggc    540
gcgttcctta ccgccctgtt cgccagccct gcgaaggcgc tgagcctgaa tgcgggtttg    600
ggcaatgtcg gcaattacaa cgtcgggttg ggcaatgtcg gggtgttcaa cctgggcgcg    660
ggcaatgtgg gtgggcagaa tctgggtttc gggaatgccg gtggcaccaa tgtcgggttc    720
ggcaacctcg gtaacgggaa tgtcgggttc ggcaactccg gtctgggggc gggcctggcc    780
ggcttgggca atatcgggtt gggcaatgcg ggcagcagca actatggttt cgcaaacctg    840
ggtgtgggca acatcggttt cggcaacacc ggcaccaaca acgtcggcgt cgggctcacc    900
ggcaaccacc tgacgggtat cgggggcctg aattcgggca ccgggaatat cgggttgttc    960
aactccggca ccgggaatgt ggggttcttc aattcgggga ccgggaactt cggggtgttc   1020
aactcgggta attacaacac cggtgtcggt aatgcgggga cggccagcac ggggttgttc   1080
aatgccggca atttcaacac cggcgtggtg aacgtgggca gttacaacac cggcagtttc   1140
aacgccggcg acaccaacac cggtggcttc aaccccggcg gtgtgaacac cggctggctg   1200
aacaccggca acaccaacac cggcatcgcc aactcgggca acgtcaacac cggcgcgttc   1260
atctcgggca acttcaacaa cggcgtgctg tgggtgggtg actaccaggg cctgttcggc   1320
gtctccgccg gctcgtcgat ccccgcaatt cccatcggcc tggtgctcaa cggcgacatc   1380
ggcccgatca ccatccagcc catcccgatc ctgcccacca tcccgctcag cattcaccaa   1440
accgtcaact tgggcccgct ggtggttccc gacatcgtga tccccgcctt cggcggcggt   1500
atcggcatac ccatcaacat cggcccgctg accatcacac ccatcaccct gtttgcccaa   1560
cagacatttg tcaaccaatt gccctttccc accttcagtt tagggaaaat cacaattcca   1620
caaatccaaa cctttgattc taacggtcag cttgtcagct ttatcggccc tatcgttatc   1680
gacaccacca ttcccggacc caccaatcca cagattgatt taacgatcag atgggatacc   1740
cctccgatca cgctgttccc gaatggcatc agtgctcccg ataatccttt ggggttgctg   1800
gtgagtgtgt cgatcagtaa cccgggcttt accatcccgg gatttagtgt tcccgcgcag   1860
ccgttgccgt tgtcgatcga tatcgagggc cagatcgacg ggttcagcac cccgccgatc   1920
acgatcgatc gcatccccct gaccgtgggg ggcggggtca cgatcggccc catcacgatc   1980
cagggccttc atatcccggc ggcgccggga gtggggaaca ccaccacggc cccgtcgtcg   2040
ggattcttca actccggtgc gggtggggtg tcgggtttcg gcaacgtcgg cgcgggcagc   2100
tcgggctggt ggaaccaggc gccgagcgcg ctgttggggg ccggttcggg tgttggcaac   2160
gtgggcaccc tgggctcggg tgtgctcaac ctgggctcag ggatctcggg gttctacaac   2220
accagcgtgt tgcctttcgg gacaccggcg gcggtgtcgg gcatcggcaa cctgggccag   2280
cagctgtcgg gggtgtcggc ggcgggaacc acgctgcgct cgatgctcgc cggcaacctc   2340
gggttggcca atgtgggcaa cttcaacacc gggttcggaa atgtcgggga cgtcaacctg   2400
ggtgcggcca acatcggtgg gcacaacctg ggcctgggca atgtcgggga cggcaacctg   2460
gggttgggca acatcggcca tggcaacctg gggtttgcca acttgggcct gaccgccggc   2520
gcggcggggg tgggcaatgt tggttttggc aatgccggca tcaacaacta tggcttggcg   2580
aacatgggtg tgggcaatat tgggtttgcc aacaccggca cgggcaacat cgggatcggg   2640
ctggtcgggg accatcggac cgggatcggg ggcttgaact ccggcatcgg caatatcggg   2700
ttgttcaact ccggcaccgg caacgtcggg ttcttcaatt ccgggaccgg caacttcggc   2760
atcgggaact ccggccgctt caacaccggg atcggtaata gcggaacggc cagcaccggg   2820
ctcttcaatg ccggcagctt cagcaccggc atcgccaaca ctggtgacta caacacgggc   2880
agcttcaacg ccggcgacac caacaccggt ggcttcaacc cgggcggcat caacaccggc   2940
tggttcaaca ccgggcatgc caacaccggg ttggccaacg cgggcacctt cggcaccggc   3000
```

```
gccttcatga cgggcgacta cagcaacggc ctgttgtggc ggggcggcta cgagggcctg 3060
gtcggcgtcc gcgtcgggcc cacgatctcc caattcccgg tcaccgtgca cgcgatcggc 3120
ggggtgggcc cgctgcatgt ggcgcccgtc ccggtacccg ccgtgcacgt cgagatcacc 3180
gacgccaccg tcggcctggg tccgttcacc gtcccaccga tcagcattcc ctcacttccc 3240
atcgccagca tcaccggaag cgtggacctg gccgcaaaca ccatctcgcc gattcgcgct 3300
cttgacccgc tcgccggttc gatagggctt tttctcgagc cgttccgcct cagtgaccca 3360
tttatcacca ttgatgcgtt ccaagttgtt gccggtgtct tgttcctaga gaacatcatt 3420
gtgcccggcc tcacggttag cggtcagata ttggtcaccc cgacaccaat tcccctaacc 3480
ctcaacttgg acaccacccc gtggacgctt ttcccgaatg gtttcaccat tcccgcgcaa 3540
acccccgtga cggtgggtat ggaggtcgcc aacgacgggt tcaccttctt cccgggtggg 3600
ctgacctttc cgcgggcctc cgccggggtc accggactgt ccgtggggct ggacgcgttc 3660
acgctgttgc ccgacgggtt caccctcgac accgtgccgg cgaccttcga cggcaccatc 3720
ctcatcggcg atatcccgat cccgatcatc gatgtgccgg cggtgccggg gttcggcaac 3780
accaccacgg ccccatcgtc ggggttcttc aacaccggcg gcggcggtgg atcggggttc 3840
gccaacgtcg gcgcgggcac gtcgggctgg tggaaccagg ggcacgacgt gttagcaggg 3900
gcgggctcgg gagttgccaa tgccggcacg ctgagctcgg gcgtgctgaa cgtcggctcg 3960
gggatctccg ggtggtacaa caccagcacc ctgggagcgg gcaccccggc ggtggtctcg 4020
ggcatcggca acctcggcca gcagctgtcg gggttcttgg caaatgggac cgtgctcaac 4080
cggagcccca ttgtcaatat cgggtgggcc gatgtgggcg cgttcaacac cgggttgggc 4140
aatgtggggg acctcaactg gggtgcggcc aacatcggcg cgcagaacct gggcctgggc 4200
aatctcggca gcgggaacgt cgggttcggc aacatcggtg ccggcaacgt cgggttcgcc 4260
aactcgggtc cggcggtggg cctggccggc ctgggcaacg tggggttgag caatgccggc 4320
agcaacaact gggggctggc caacctgggt gtgggcaaca tcgggttggc caacaccggc 4380
acgggcaaca tcgggatcgg gctggtcggc gactaccaga ccggcatcgg cggcctcaac 4440
tcgggtagtg gcaatatcgg attgttcaat tccggcaccg gcaatgtcgg gttcttcaac 4500
accggcaccg gcaacttcgg actgttcaac tccggtagtt tcaacaccgg catcggtaat 4560
agcggaaccg gcagtactgg gctcttcaat gccggcaatt tcaacaccgg catcgccaac 4620
cccgggtcgt acaacacggg cagcttcaat gtcggtgata ccaacaccgg tggtttcaac 4680
ccgggcgaca tcaacaccgg ctggttcaac accggcatta tgaatacggg cacccgcaac 4740
accggcgccc tcatgtcggg gaccgacagc aacggcatgc tgtggcgcgg cgaccacgag 4800
ggcctgttcg gcctgtccta tggcatcacg atcccgcaat tcccgatccg catcaccacg 4860
actggcggta tcggccccat cgtcatcccg gacaccacga tccttccgcc gctgcacctg 4920
cagatcaccg gcgacgcgga ctacagcttc accgtgcccg acatccccat ccccgccatc 4980
cacatcggca tcaatggcgt cgtcaccgtc ggcttcaccg ccccggaagc caccctgctg 5040
tccgccctga agaataacgg tagcttcatc agcttcggcc ccatcacgct ctcgaatatc 5100
gatattccgc ccatggattt cacgttaggc ctgcccgttc ttggtcctat cacgggccaa 5160
ctcggaccaa ttcatcttga gccaatcgtg gtggccggga tcggtgtgcc cctggagatc 5220
gagcccatcc ccctggatgc gatttcgttg agtgagtcga ttcctatccg catacctgtt 5280
gatattccgg cctcggtcat cgatgggatt tcaatgtcgg aagtggtgcc gatcgatgcg 5340
tccgtggaca tcccggcggt cacgatcaca ggcaccacca tttccgcgat cccgctgggc 5400
ttcgacattc gcaccagtgc cggacccctc aacatcccga tcatcgacat cccggcggcg 5460
ccgggcttcg ggaactcgac ccagatgccg tcgtcggggt tcttcaacac cggtgccggc 5520
ggcggatcgg gcatcggcaa cttgggtgcg ggcgtgtcgg gcctgctcaa ccaggccggc 5580
gcggggtcac tggtggggac actctcgggg ctgggcaatg ccggcaccct ggcctcgggt 5640
gtgctgaact ccggcaccgc catctccggg ctgttcaacg tgagcacgct ggacgccacc 5700
accccggcgg tgatctcggg gttcagcaac ctcggcgacc atatgtcggg ggtgtccatc 5760
gatggcctga tcgcgatcct caccttccca cctgccgagt ccgtgttcga tcagatcatc 5820
gacgcggcca tcgccgagct gcagcacctc gacatcggca acgctttggc cttgggcaat 5880
gtcggcgggg tgaacctcgg tttggctaac gtcggtgagt tcaacctggg tgcgggcaac 5940
gtcggcaaca tcaacgtcgg cgccggcaac ctcggcggca gcaacttggg gttgggcaac 6000
gtcgggaccg gcaacctcgg gttcggcaac atcggtgccg gcaatttcgg attcggcaac 6060
gcgggcctga ccgcgggcgc ggggggcctg ggcaatgtgg ggttgggtaa cgccggcagc 6120
ggcagctggg ggttggccaa cgtgggtgtg ggcaatatcg ggttggccaa caccggcacc 6180
ggcaacatcg ggatcggggct gaccggggac tatcggaccg ggatcggcgg cctgaactcg 6240
ggcaccggga acctcgggtt gttcaactcg ggcaccggca acatcgggtt cttcaacacc 6300
gggaccggga acttcgggct gttcaactcg ggcagttaca gcaccggtgt ggggaatgcg 6360
ggcacggcca gcaccgggtt gttcaacgcg gggaacttca acaccggtct ggccaatgcc 6420
ggctcctaca acaccggcag cctcaacgtg ggcagcttca acaccggcgg cgtcaacccg 6480
ggcaccgtca acaccggctg gttcaacacc ggccacacca acaccggcct gttcaacacc 6540
ggcaacgtca acaccggcgc gttcaactcc ggcagcttca acaacggggc gctgtggacc 6600
ggtgactacc acgggctggt cggcttctcc ttcagcatcg acatcgccgg cagcaccctg 6660
ctggacctca acgaaaccct caacctgggc cccatccaca tcgagcagat cgacatcccc 6720
ggcatgtcgc tgttcgacgt ccacgaaatc gtcgagatcg gacccttcac catcccgcag 6780
gtcgatgttc ccgcgatacc gctagagatc cacgaatcga tccacatgga tcccatcgtc 6840
ctggtgcccg ccaccacaat tcccgcacag acgagaacca ttccgctgga catccccgcc 6900
tcacccgggt caaccatgac gcttccgctc atcagcatgc gcttcgaagg cgaggactgg 6960
atcctcgggt cgaccgcggc gattcccaat ttcggagacc ccttcccggc gcccacccag 7020
```

ggcatcacca ttcacaccgg ccctggcccc ggaacgaccg gcgagctcaa gatatctatt 7080
ccgggtttcg agattccgca aatcgctacc acgagattcc tgttggacgt gaacatcagc 7140
ggtggtctgc cggccttcac cttgttcgcg ggtggcctga cgatccccac gaacgccatc 7200
ccgttaacga tcgatgcgtc cggcgcgctg gatccgatca cgattttccc gggtgggtac 7260
acgatcgacc cgctgccgct gcacctggcg ctgaatctca ccgtgcccga cagcagcatc 7320
ccgatcatcg atgtcccgcc gacgccaggg ttcggcaaca ccacggcgac cccgtcgtcg 7380
gggttcttca actccggcgc cggtggggtg tcggggttcg gaaacgtcgg gtcgaacctg 7440
tcgggctggt ggaaccaggc ggcgagcgcg ctggcggggt cgggatcggg ggtgttgaat 7500
gtcggcacgc tgggctcggg tgtgctcaac gtcggctcgg gtgtctcggg gatctacaac 7560
accagcgtgt tgccgctcgg gacgccggcg gtgctgtcgg gcctcggcaa cgtcggccat 7620
cagctgtcgg gcgtgtctgc ggccgggacc gcgttgaacc agatccccat cctcaacatc 7680
gggttggcgg atgtgggcaa cttcaacgtc gggttcggca acgtcgggga cgttaacctg 7740
ggcgcggcca acctcggtgc gcaaaacctg gggctgggca acgtcggcac cggcaacctc 7800
ggcttcgcca acgtcggcca cggcaatatc ggtttcggca attcgggtct gaccgccggc 7860
gcggccggcc tgggcaacac ggggttcggc aatgccggca gcgccaacta tggtttcgcc 7920
aaccagggcg tgcgcaacat cgggttggcc aacaccggca ccggcaacat cgggatcggg 7980
ctggtggggg acaacctcac cggcatcggg ggcctgaact ccggtgccgg caatatcggc 8040
ttgttcaact ccggcaccgg caacatcggg ttcttcaact ccgggaccgg caacttcggc 8100
atcggtaact cgggcagctt caacaccggc atcggcaata gcggaacggg cagcactggg 8160
ctcttcaatg ccggcagctt caacaccggc gtggccaacg ccggcagcta caacaccggc 8220
agcttcaatg ccggcgacac caacaccggg ggcttcaacc cgggcaccat caacaccggc 8280
tggttcaaca ccggccacac caataccggc atcgccaact cgggcaacgt cggcaccggc 8340
gcgttcatgt cgggcaactt cagcaacggc ctgttgtggc ggggtgatca cgagggcctg 8400
ttcagcctgt tctacagcct cgacgtgccc cggatcacca tcgtggacgc ccacctcgac 8460
ggcggcttcg gacccgtggt cctcccgccc atcccggtgc cggccgttaa tgcgcacctg 8520
accggaaacg tcgcgatggg cgcattcacc attccgcaga tcgacatccc cgcactcacc 8580
ccaaacatca ccggaagcgc cgccttccgc atcgttgtgg ggtccgtgcg cattccgccg 8640
gtgagtgtca ttgtggagca aataatcaac gcctcggttg gggcggagat gaggatagat 8700
cccttcgaaa tgtggactca aggcactaat ggccttggta taaccttcta ttcattcgga 8760
tcggccgacg gttcgcccta cgccaccggc ccactcgttt tcggcgccgg cacgagcgac 8820
ggaagccatc tcaccatttc cgcgtccagc ggggcgttta ccactccgca gctcgaaact 8880
ggcccgatca cgttgggctt ccaggtgccc ggcagcgtca acgcgatcac cctcttcccc 8940
ggtggtttga cgttcccggc gacctcgctg ctgaacctgg acgtgaccgc cggcgccggc 9000
ggcgtggaca tcccggccat cacctggccc gagatcgcgg cgagcgccga cggctcggtg 9060
tatgtcctcg ccagcagcat cccgctgatc aacatcccgc ccaccccggg cattgggaac 9120
agcaccatca ccccgtcgtc gggcttcttc aacgccggcg cgggcggggg atcgggcttc 9180
ggcaacttcg gcgcgggcac ctcgggctgg tggaaccagg cgcacaccgc gctggcgggg 9240
gcgggctcgg gttttgccaa cgttggcacg ctgcattccg gtgtgctcaa cctgggctcg 9300
ggtgtctcgg ggatctacaa caccagcacg ctgggggtgg ggaccccggc gctggtctca 9360
ggcctgggca acgtcggcca ccaactgtcg gggctgcttt ccggcgggtc cgcggtgaac 9420
ccggtgaccg ttctgaatat cgggttggcc aacgtcggca gccacaacgc cggtttcggc 9480
aatgtcgggg aggtcaacct gggcgcggcc aacctcggcg cgcacaacct gggcttcgga 9540
aatatcggcg ccggcaacct ggggttcggc aatattggcc acggcaatgt cggagtcggc 9600
aactcgggtc tgaccgcggg cgtgccgggc ctgggcaatg tggggttggg caatgccggc 9660
ggcaacaact gggggttggc caacgtgggc gtgggcaata tcgggttggc caacaccggc 9720
accggcaaca ttgggatcgg gctgaccggc gactaccaga ccggcatcgg cggcctaaat 9780
tccggtgccg gcaacctggg gttgttcaac tccggcgccg gcaacgtcgg gttcttcaac 9840
accgggaccg gcaacttcgg gttgttcaac tccggcagct tcaacaccgg cgtcggcaat 9900
agcggaacgg gcagcactgg gctcttcaat gccggcagtt tcaacaccgg tgtggccaac 9960
gccggcagct acaacacggg cagcttcaat gtcggtgaca ccaacaccgg gggcttcaac 10020
ccgggcagca tcaacaccgg ctggctcaac gccggcaacg ccaacaccgg ggtggccaac 10080
gcgggcaatg tcaacaccgg cgccttcgtc accggcaact tcagcaacgg catcctgtgg 10140
cgcggcgact accagggcct ggccggcttc gccgtgggct acaccctccc gctgttcccc 10200
gcggtgggcg ccgacgtcag cggcgggatc ggcccgatta ccgtgctgcc gcccatccac 10260
atcccgccca ttccggtcgg cttcgccgcg gtcggtggca tcggcccgat cgccatcccg 10320
gacatctctg ttccatccat tcacttgggc ctcgaccccg ccgtccatgt cggctccatc 10380
accgtcaacc ccattaccgt caggaccccg cccgtgctcg tcagttactc ccaaggagcc 10440
gtcaccagca cgtccggacc aacctcagag atttgggtca agcccagctt cttccccgga 10500
atccggatcg cgccctctag cggcgggggt gcaacgtcca cgcaaggggc atactttgtg 10560
gggcccatct ccatcccctc cggcacggtg accttcccgg gattcaccat ccccctcgac 10620
ccgatcgaca tcggcctgcc ggtgtcgctg accatcccgg ggttcaccat cccgggcggc 10680
accctgatcc ccaccctccc gctgggcctc gcgttgtcca atggcatccc gcccgtcgac 10740
atcccggcca tcgttctcga ccggatcttg ctggacctgc acgccgacac cactatcggc 10800
ccgatcaacg tcccgatcgc cgggttcggc ggggcgccgg gtttcgggaa ctcgaccacg 10860
ctgccgtcgt cgggcttctt caacaccgga gctggcggcg gttcgggctt tagcaacacc 10920
ggcgcgggca tgtcgggatt gctcaacgcg atgtcggatc cgctgctcgg gtcggcgtcg 10980
ggcttcgcca acttcggcac ccagctctcc ggcatcctca accgcggcgc cggcatctcg 11040

ggcgtgtaca acaccggcgc gctgggtgtt gtcaccgcgg ccgtcgtctc gggtttcggc 11100
aacgtcggcc agcaactgtc gggcttgctc ttcaccggcg tcgggcccta a 11151

<212> Type : DNA
<211> Length : 11151
SequenceName : SEQ ID 525
SequenceDescription :
Sequence
--------
<213> OrganismName : Mycobacterium tuberculosis H37Rv
<400> PreSequenceString :

```
ttgaatttc cagttctgcc accggaaatc aactccgtgc tgatgtattc gggtgcgggg     60
tcgagcccgt tgctggcggc ggccgcggcg tgggatgggc tggctgagga gttggggtcg    120
gcggcggtgt cgtttgggca ggtgacgtcg ggcctgacgg cgggggtgtg gcagggtgcg    180
gcggcggcgg cgatggcggc cgcggcggcc ccgtatgcgg ggtggttggg ttcggtggcg    240
gcgcaggccg tggcggtggc cgggcaggcg cgggccgcgg tggcggcgtt tgaggcggcg    300
ttggcggcga cggtggatcc ggcggcggtg gcggtcaacc ggatggcgat gcgggcgttg    360
gcgatgtcga acctgctggg gcagaacgcc gcagcgatcg cggccgtcga ggccgagtac    420
gagttgatgt gggccgccga tgtggcggcg atggccggct accattccgg cgcgtcggct    480
gctgccgcgg cgttgccggc gttcagccca ccggcgcagg cgttgggggg tggtgtcggc    540
gcgttcctca atgctctatt tgccggaccc gcgaagatgt tgaggcttaa cgcgggcttg    600
ggcaatgtcg gtaattacaa cgtcgggttg ggcaatgtcg ggatattcaa cctgggcgca    660
gccaatgtcg gtgcgcagaa tttgggtgct gccaacgccg gtagcgggaa tttcggtttc    720
ggcaatatcg gcaacgccaa cttcgggttc ggcaactcgg gtcttgggtt gccgccgggc    780
atgggcaata ttgggttggg caatgcgggc agcagcaact acggcctcgc aaacctgggt    840
gtgggcaaca tcggttttgc caacacgggt agcaacaaca tcgggatcgg gttgaccggg    900
gacaacctga ctggcattgg gggcctgaat tcaggaaccg gtaatctggg gttgttcaac    960
tccggcaccg gcaacattgg gttcttcaat tcggggaccg gcaacttcgg ggtattcaac   1020
tcgggcagct acaacaccgg tgtcggtaat gcggggacgg ccagtaccgg gttgttcaac   1080
gttggtgggt tcaacacggg tgtggccaac gtgggtagct ataacacggg cagcttcaac   1140
gcgggcaaca ccaatacggg tggcttcaac ccgggcaacg tcaacaccgg ctggctgaac   1200
accggcaaca ccaacaccgg catcgccaac tcgggcaatg tcaacaccgg cgcgttcatc   1260
tcgggcaact tcagcaacgg tgtgctgtgg cggggtgact acgagggcct gtgggggctc   1320
tccggtggat cgaccattcc ggcgatcccc attggtctcg agctcaacgg cggcgtcggc   1380
cccatcaccg tgttgccgat ccagattttg cccaccatcc cgctcaacat tcaccaaacc   1440
ttcagcctcg gcccgctggt cgttccggac atcgtgatcc ccgcttttgg tggcggtacg   1500
gccataccta tcagcgtcgg ccccatcacc atctcgccca tcaccctgtt cccggctcag   1560
aacttcaaca cgactttccc cgtcggcccc ttctttggct tgggggtcgt caacatttca   1620
ggaatcgaaa tcaaagatct tgccggcaac gtcaccctcc aattaggtaa ccttaatatc   1680
gacaccagaa ttaaccagtc attcccggtg accgtcaact ggagtacccc ggcagtaacg   1740
atcttcccga atggcatcag tattcccaac aatccactgg cgctgctggc cagcgcgtcg   1800
atcggcacgc tgggattcac gatcccgggc ttcaccattc ccgctgcgcc gctgccgctg   1860
acgatcgaca tagacggcca gattgacggc ttcagcaccc cgccgatcac gatcgaccgc   1920
atcccgctga acctcggcgc cagcgtcact gtcggcccta tcctgatcaa cggcgttaat   1980
atcccggcga ccccgggctt tggcaacacg accaccgctc cgtcgtcggg tttcttcaac   2040
tccggcgacg gtggggtgtc gggcttcggg aatttcggtg cgggcagctc gggttggtgg   2100
aaccaggcgc agaccgaggt ggctggggcg ggttcgggtt tcgccaattt cggttcgctg   2160
ggatcgggtg tgctgaactt cggctcgggt gtgtcggggc tgtacaacac cggcgggttg   2220
ccgccgggga ccccggcggt ggtctcgggc atcggcaatg ttggtgagca gctgtcgggg   2280
ttgtcctcgg cggggacggc actcaaccag agcctcatca tcaatctcgg gttggccgat   2340
gtgggcagcg taaacgtcgg tttcggcaac gtcggggact tcaacctggg tgcggccaat   2400
atcggcgact tgaacgtggg tttgggcaat gtcggcggcg gcaacgtcgg gttcggcaat   2460
atcggcgatg ccaacttcgg gttgggcaat gcgggtctgg cggcgggcct ggccggggtg   2520
ggcaacatcg ggttgggcaa tgccggcagc ggcaacgtcg gcttcggcaa catgggtgtg   2580
ggcaacatcg ggttcggtaa caccggcacc aacaacctcg ggattgggct gaccggggac   2640
aaccagactg ggatcggcgg cttgaactcc ggtgccggca acatcgggtt gttcaactcc   2700
ggcaccggca acgtcgggtt gttcaactcc gggaccggga acttcgggtt gttcaactcg   2760
ggcagcttca acaccggcat cggcaatggc ggaacgggca gtactgggct tttcaatgcc   2820
ggtaatttca ataccggtgt ggccaaccct gggtcgtaca acacgggcag cttcaatgtg   2880
ggtgacacca acaccggtgg tttcaacccg ggcagcatca acaccggctg gttcaacacc   2940
ggcaacgcca acaccggcgt cgccaattcg ggcaatgtcg acaccggcgc cctcatgtcg   3000
ggcaacttca gcaacggcat cttgtggcga ggcaacttcg agggcctgtt cggcctgaac   3060
gtcggcatca cgattcccga attcccgatc cactggactt caaccggcgg catcggcccc   3120
attatcatcc cggacaccac gatccttccc cccatccacc tgggcctcac gggacaagcg   3180
aactacggct tcgccgtgcc ggacatcccc attccggcaa tccacatcga cttcgacggt   3240
gccgccgacg ccggcttcac cgccccggcc accaccctgc tttctgcgct gggcattacc   3300
```

ggacaattca ggttcggccc gatcaccgtc tcaaacgtcc agctcaatcc gttcaacgtt 3360
aacctcaagc ttcagttcct ccacgacgcg ttcccaaatg aatttcccga tcccacaatc 3420
tcggttcaga tacaggtcgc cataccccctt acttcggcaa cgctgggcgg attggccctg 3480
ccgctgcagc agaccatcga cgccatcgaa ttgccggcaa tctcgttcag ccaatccata 3540
cccatcgaca ttccgccgat cgacatcccg gcctccacta tcaacggaat ttcgatgtcg 3600
gaggtcgtgc cgatcgatgt gtccgtcgac attccggcgg tcaccatcac cggcaccagg 3660
atcgacccga ttccgctgaa cttcgacgtt ctcagcagcg ccggacccat caacatctcg 3720
atcatcgaca ttccggcgct gccgggcttt ggcaactcga ccgagctgcc gtcgtcgggc 3780
ttcttcaaca ccggcggcgg tggcggctcg ggcatcgcca acttcggcgc gggggtgtcc 3840
ggcttgctga accaggcctc gagtccgatg gtggggacgc tctccggcct gggcaatgcc 3900
ggcagcctgg catccggtgt gctgaactcc ggcgtcgaca tctcgggcat gttcaacgtg 3960
agcacgctgg gctccgcgcc ggcggtgatc tcgggtttcg gcaacctggg caaccacgtg 4020
tcgggggtgt ccatcgatgg cctgctggcg atgctgacca gcggcgggtc gggcggctcc 4080
gggcagccga gcatcatcga cgcggcgatc gccgagctgc ggcacctgaa tccgctgaac 4140
atcgtcaacc tgggcaacgt cggcagctac aacctcggct tcgccaacgt cggcgacgtc 4200
aacctgggcg cgggcaacct cggcaacctc aacctcggcg gtggcaacct cggcgggcag 4260
aacctggggt tgggcaacct cggggacggc aacgtcgggt tcggcaacct cggccacggc 4320
aatgtcgggt tcggcaactc gggcctgggg gcgctgccgg ggatcggcaa catcgggttg 4380
ggcaacgccg gcagcaacaa cgtcggcttc ggcaacatgg gcctgggcaa catcgggttc 4440
ggcaataccg gcaccaacaa cctcgggatc gggctgaccg gcgacaacca gaccgggttc 4500
ggcggcctga actccggtgc cggcaacctg gggttgttca actccggcac cggcaacatc 4560
gggttcttca acaccgggac cggaaactgg gggttgttca actcgggcag ctacaacacc 4620
ggcatcggta acagcggaac gggcagtacc gggcttttca atgccgggag tttcaacacg 4680
ggtctggcca atgccggtag ttacaacacc ggcagcctca acgcgggcaa caccaacacc 4740
ggcggcttca accctggcaa tgtcaacacc ggctggttca acgccggcca caccaacacc 4800
ggcggcttca acacgggcaa tgtcaacacc ggcgcgttca actccggcag cttcaacaac 4860
ggcgcgctgt ggaccggtga tcaccacggg ctggtcggct tctcctacag catcgaaatc 4920
accggcagca ccctggtgga catcaacgaa accctcaacc tcggtcccgt ccacatcgat 4980
cagatcgata ttcccggcat gtcgctgttc gacatccacg aactcgtcaa catcgggccc 5040
ttcaggatcg agcccatcga tgtccccgca gtggtgctgg acatccacga aacgatggtc 5100
atcccgccca tcgtcttcct gccgagcatg acgatcggcg gtcagaccta cacgattccg 5160
ctcgacacgc cccggccccc cgccccgccg cccttcagac ttccgttgct gttcgtgaat 5220
gcgctcggcg acaactggat cgttggggcg tccaactcaa ccggaatgag tggtggcttt 5280
gtcaccgcac ccactcaggg catcctgatc cataccggtc ccagcagcgc aaccaccggt 5340
agcctcgcac taaccctccc aaccgtcacc atcccaacga tcacgacatc gcctatcccg 5400
ctcaagatcg atgtgtcggg cggtcttccg gccttcacgc tgttccccgg tggcctcaac 5460
atcccgcaaa atgcgatccc gttgaccatc gatgcgtccg gcgtgctgga tccgatcacg 5520
atattcccgg gtggtttcac gatcgatccg ctgccactga gcctggccct caacatcagc 5580
gtgccggaca gcagcgttcc gatcatcatc gttccgccga cgcccggctt cgggaacgcg 5640
accgccaccc cgtcgtcggg tttcttcaac tccggcgcgg gcggggtgtc gggtttcggc 5700
aacttcgggg ccggcagctc aggctggtgg aaccaggcgc atgccgcgtt ggcgggcgcg 5760
ggctcgggcg ttctcaacgt tggcacgctg aactcgggtg tgctgaacgt cggctcgggg 5820
atatcggggc tgtacaacac cgctatcgtg ggtttgggga cgccggcgct ggtgtcgggt 5880
gccggcaacg tgggccagca gctgtcgggg gtgttggcgg ccgggacggc gttgacccaa 5940
agccccatca tcaacctcgg gttggccgat gtcggcaact acaacctcgg gttgggcaac 6000
gttggggact tcaacctggg cgcggccaac ctcggcgacc tcaacctagg gttgggcaat 6060
atcgggaacg ccaacgtcgg cttcggcaat atcggccacg gcaacgtcgg gtttggcaat 6120
tcgggcctgg gggcggcgct cggcatcggc aatatcgggt tgggcaatgc gggcagcacc 6180
aacgttggcc tggccaacat gggtgtgggc aacatcgggt tcgccaacac cggcaccaac 6240
aacctcggga ttgggctgac cggcgacaac cagaccggca tcggcggctt gaactccggt 6300
gccggcaaca ttggcctgtt caactccggc accggcaaca tcgggttctt caactccggg 6360
accggaaact gggggttgtt caactcgggc agcttcaaca ccggcatcgg taatagcgga 6420
acgggcagta ctgggctttt caatgccggt ggtttcacta cggtctggc caacgccggg 6480
tcgtacaaca cgggcagctt caatgtcggt gacaccaaca ccggtggctt caacccgggc 6540
agcatcaaca ccggctggtt caacaccggt aacgccaaca ccggcatcgc gaactcgggc 6600
aatgtcgaca ccggcgccct catgtcgggc aacttcagca acggcatcct gtggcggggc 6660
aactacgaag gcctattcag ctattcctac agcctcgacg ttccccggat caccatcctg 6720
gacgcgcatt tcaccggggc cttcggcccg gtggtcgtcc cgcccatccc ggttctggcg 6780
atcaacgcgc acctgaccgg caacgcggcg atgggcgcct tcaccatccc gcaaatcgat 6840
attcccgccc tcaatccgaa cgtcaccgga agcgtcggct tcggccccat cgcggtcccc 6900
tcggtcacca ttcccgccct gaccgccgca cgagcggtcc tcgatatggc cgcgtcggtc 6960
ggggcgacca gcgaaataga gccgtttatc gtctggacgt catccggtgc gatcggccca 7020
acgtggtact cggtcggcag aatctacaac gccggtgacc tgttcgtcgg cggcaatatc 7080
atctcgggaa tcccgacgct cagcacgacc ggcccggtgc atgccgtctt caatgcggca 7140
tctcaggcgt tcaacacccc ggcgctcaat attcaccaga tcccgttggg tttccaggtg 7200
ccgggcagca tcgacgcgat caccctgttc cccggtggtc tgacgttccc ggcgaactcg 7260
ctgctgaacc tggatgtgtt cgtcggcacc cccggcgcca ccattccggc gatcacgttc 7320

```
ccggagatcc cggcgaacgc cgacggcgaa ctctacgtca tcgccggcga catcccgctg    7380
atcaacatcc cgcccacccc gggcattggg aacaccacca ccgttccgtc gtcgggcttc    7440
ttcaacaccg gggcgggcgg gggctcgggt ttcggcaact tcggcgcgaa catgtcgggg    7500
tggtggaacc aggcgcacac cgctttggca ggcgcgggtt cgggtattgc caacgtcggc    7560
acactgcact ccggcgtgct caacctcggt tcggggctgt cggggatcta caacaccagc    7620
acgctgccgc ttgggacgcc ggcgttggtg tcgggcctgg gcaacgtcgg tgatcacctg    7680
tcgggcttgt tggcctccaa cgtggggcaa aaccccatca ccatcgtcaa catcgggttg    7740
gctaacgtcg gcaacggcaa cgtcggcctc ggcaacatcg gcaacctcaa cctgggtgcg    7800
gccaacattg gcgacgtgaa cctgggattc ggcaacattg gcgacgtgaa cctgggcttc    7860
ggcaacatcg gcggcggcaa cgtcgggttc ggcaatatcg gcgatgccaa cttcgggttc    7920
gggaattcgg gtctggcggc gggcctggcc ggcatgggca atatcgggct gggcaacgcc    7980
ggcagcggca acgtcggctg ggccaacatg ggcctgggca acatcgggtt tggcaacacc    8040
ggcaccaaca acctcgggat cgggctcacc ggcgacaacc agtccggcat cggcggcttg    8100
aactccggca ctggcaacat tggcctgttc aactccggca caggcaatat cggcttcttc    8160
aactccggga ctgccaactt cgggttgttc aactccggca gctacaacac cggtatcggc    8220
aactccgggg tggccagcac cgggttggtc aacgccggcg gcttcaacac cggtgtggca    8280
aacgccgggt cgtacaacac gggcagcttc aatgctggtg acaccaacac cggtggcttc    8340
aacccaggca gcaccaacac cggctggttc aacaccggta acgccaacac cggcgtcgcc    8400
aacgcgggca atgtcaacac cggcgccctc atcacgggca actttagcaa cggcatctta    8460
tggcggggca attacgaggg cttggccggc ttctccttcg ggtaccccat tccgctgttc    8520
cccgcggtgg gcgccgacgt caccggcgac atcggccccg ccaccatcat tccgcccatc    8580
cacatcccgt ccatcccgtt gggcttcgcc gcgatcggcc acatcgggcc gatcagcatc    8640
ccgaacatcg ccatcccctc gatccacctg ggcatcgatc ccaccttcga cgtcggccct    8700
atcaccgtgg accccatcac cctcaccatc cctggcctaa gtttggatgc tgccgtctcg    8760
gagatcagga tgacgtccgg aagcagctcc ggattcaagg tcagacccag cttttcattc    8820
ttcgcggtcg gacccgacgg catgcccggg ggcgaggtct ccatacttca accattcacc    8880
gtggcaccca tcaacttgaa cccgacgaca ctgcacttcc ccggattcac cattcccacc    8940
ggacccatcc acatcggcct gccgctgtcg ctgaccattc cgggcttcac catcccgggc    9000
ggcaccctga ttccccaact cccgctgggc ctcggtttgt ccggcggcac cccacccttt    9060
gatctcccga cggtcgttat cgaccggatc ccggtggagt tacacgccag caccaccatc    9120
ggccccgtca gcctcccgat tttcgggttc ggcggagcac cgggctttgg caacgacacc    9180
accgcgccgt cgtcgggctt cttcaacacc ggcggtggtg gcgggtccgg cttctccaac    9240
tccgggtcgg gcatgtcggg ggtgctcaac gcgatctcgg atccgctgct cgggtcggcg    9300
tcgggcttcg ccaatttcgg cacccagctc tccggcatcc tcaaccgtgg cgcgggcatc    9360
tcgggcgtgt acaacacggg cacgcttggc ctggtcacat cggccttcgt ctcgggcttt    9420
atgaacgtcg gccagcagct gtcgggcctg ctgttcgcgg gcaccgggcc gtaa          9474
```

<212> Type : DNA
<211> Length : 9474
SequenceName : SEQ ID 526
SequenceDescription :
Sequence
--------
<213> OrganismName : Mycobacterium tuberculosis H37Rv
<400> PreSequenceString :

```
atgagttttg tcgtaatgcc gccagagatc aactccctgc tgatatatac cggggcgggt      60
ccgggccccc tgttggcggc ggccgcggcc tgggatgagc tggccgccga gctgggctcg     120
gcggcggcgg ctttcgggtc ggtgacctcg gggctggtcg gtggtatctg gcaggggccg     180
tcctcggtcg cgatggcggc ggcggccgcc ccgtatgcgg ggtggttgag cgcggcggcg     240
gcctccgccg agtcggcggc cgggcaggcc cgggcggtgg tgggtgtgtt cgaggcggcg     300
ttggccgaga cggtggaccc cttcgtcatc gcggccaatc ggtcgaggtt agtgtcgctg     360
gcgttatcga atctgttcgg ccagaacacg ccggcgatcg cggccgcgga gttcgactac     420
gagctgatgt gggcccagga cgtggccgcg atgctggggt accacaccgg cgcctcggcg     480
gcggccgagg cgttggcgcc gtttggctcg ccgctggcaa gcctggcggc cgccgccgag     540
ccggccaagt cgctcgccgt caatctgggt ttggccaacg ttggcctctt caacgcggga     600
agcggcaacg tcggcagcta caacgtgggg gccggcaacg tcgggagcta caacgtgggc     660
ggcggcaata tcggtggcaa caatgtcggg ttgggcaatg tcgggtgggg caactttggg     720
ctcgggaatt cggggttaac gccgggtctg atgggtctgg gtaatatcgg gtttggtaat     780
gccggcagct acaatttcgg tttggcgaat atgggtgtgg gcaatattgg gttcgctaac     840
accggcagtg ggaatttcgg tattgggttg accggtgata atctgaccgg gttcggtggt     900
ttcaataccg gtagcgggaa tgtggggttg tttaattcgg ggaccggtaa tgtggggttc     960
tttaactccg gcaccgggaa ctgggggggtg ttcaattcgg ggagctataa caccgggatc    1020
ggtaattcgg ggattgccag cacggggttg ttcaacgcgg gtgggttcaa tacgggtgtg    1080
gtcaatgcgg gtagctacaa caccggcagt ttcaacgcgg gggaggccaa tacgggcggt    1140
ttcaacccgg gcagtgtcaa cacgggttgg ttgaacaccg gtgacatcaa caccggggtg    1200
gccaactccg gcgacgtcaa caccggtgcc ttcatctccg gtaactacag caacggcgtc    1260
```

```
ttgtggcggg gcgactacca gggcctgctc ggcttctcct cgggagcgaa cgttcttcct 1320
gtcattcccc tcagcctgga cataaacggt ggtgtcggcg ccatcactat cgagcccatc 1380
cacatacttc ccgacatccc gatcaacatc aacgaaaccc tctacctcgg acccttggtc 1440
gtcccgccca tcaacgtccc ggcaatctcg ctcggcgtcg gcatacccaa tatttcgatc 1500
ggccccatca aaatcaatcc catcaccctg tggccggcac aaaacttcaa ccaaaccatc 1560
acgctggcct ggcccgtctc gtccataaca attccccaaa tccaacaggt cgccctcagc 1620
ccttccccca ttcccacaac cttgatcggc ccaatacata tcaataccgg gttttccatc 1680
ccagtaacct tcagttattc caccccagcc ctcacccttt tcccggttgg cctcagcatt 1740
cccaccgggg ggccgctcac cctgactctt ggcgtaacgg caggcaccga ggccttcacc 1800
attccgggat tctcgattcc cgagcaacca cttccgctgg cgatcaacgt gattggccac 1860
atcaacgccc tgagcacccc ggcaatcacc atcgacaaca tccccctgaa cctgcacgcc 1920
atcggcggcg tcgggcccgt cgacatcgtg ggcggcaacg ttccggcgtc gccggggttc 1980
gggaattcga ccaccgcccc gtcctcgggc ttctttaaca ccggcgccgg cggggtgtcg 2040
ggattcggga acgtcggcgc gcacacctcg ggctggttta accagtccac ccaggccatg 2100
caggtgttgc cgggaacggt ttcgggctat ttcaactccg gcacgttgat gtcgggtatc 2160
ggcaatgtag gtactcagtt gtcggggatg ttgtcgggcg gcgcgctggg cggcaacaac 2220
ttcggattgg gcaatatcgg gttcgacaac gtcgggttcg gcaatgccgg cagcagcaat 2280
ttcggcctgg cgaacatggg catcggcaac atcgggttgg ccaataccgg caacgggaat 2340
atcggtatcg ggctcagcgg ggacaatctg actgggttcg gtggtttcaa ttcgggcagc 2400
gagaatgtgg gattgttcaa ttcgggtacc ggcaatgtcg ggttcttcaa ctccggcacc 2460
gggaatttgg gcgtattcaa ttcgggaagc cacaacaccg gtttcttctt aacgggcaac 2520
aacatcaacg tcctggcccc cttcaccccc ggcacccttt tcaccatttc cgaaatcccc 2580
attgacctgc aggtgattgg cgggatcggt cccatccacg tccagcccat cgatattccg 2640
gcattcgaca tacaaatcac cgggggattc atcgggatcc gcgaattcac tctcccggag 2700
atcaccattc ccgcgatccc gatccacgtg accggaaccg tcggcctcga gggttttcac 2760
gtgaatcccg cgtttgttct tttcgggcaa accgccatgg cagaaatcac cgcggacccg 2820
gtcgtcttgc cggacccgtt catcacgatc gaccactacg gcccgccgct aggcccaccc 2880
ggcgcgaaat tcccctccgg gtcgttctac ctcagcatca gcgacctgca gatcaatgga 2940
cccatcatcg gcagctacgg cggcccgggc acgatccccg gcccgttcgg cgcgactttc 3000
aatctgtcca catcgtcctt ggccttgttc ccggccggcc tcaccgttcc cgaccaaaca 3060
ccggtgaccg tcaacctgac cggcggcctg gacagcatca cgctattccc gggcggcctg 3120
gcgttcccgg aaaaccccgt ggtcagcctc accaacttct ccgtcggcac cggtggattc 3180
accgtgttcc cgcagggttt cacggttgac cgcatcccgg tggacctgca caccaccctt 3240
tctattggcc ctttcccgtt ccggtgggac tacatcccgc caaccccggc caacggcccc 3300
attccggcgg tcccaggcgg ctttggcctc accagcgggc tgtttccatt tcacttcacc 3360
ctcaacggcg gcatcggccc catcagcatc ccgaccacca ccgttgtgga tgcgctaaat 3420
cccctcctga ccgtcacggg aaacctcgag gtcggcccct ttaccgtccc ggacatcccc 3480
atccccgcca tcaattttgg cctcgacgga aacgtcaatg tgagttttaa cgcccccggcc 3540
actaccctgc tgtccggcct gggcatcacg ggaagcatcg atatctccgg aatccaaatc 3600
acgaatatac agacccagcc cgcccagctg ttcatgtcgg tcggtcagac tcttttcttg 3660
tttgacttca gagacggcat cgaactgaat ccgattgtaa ttccgggaag ttctattccc 3720
atcaccatgg cgggactcag tatcccgttg cccactgtca gtgaatccat accgctcaac 3780
ttctctttcg gttccccggc atccaccgta aagtcaatga ttctgcacga aatcctgcct 3840
atcgacgtgt ccatcaacct cgaggacgcc gtcttcatcc cggccaccgt gctgcccgca 3900
attccgctga acgtggacgt cacaatcccg gtgggcccca tcaacatccc gatcatcacc 3960
gagccgggct cagggaactc caccaccacc acgtcggatc ccttcagcgg tttggctgtc 4020
ccgggcctgg gtgtgggact gttgggcctg ttcgacggga gcatcgccaa caacctgatc 4080
tcgggcttca attccgcggt cgggatcgtc ggcccgaacg tggggttgag caacctcggt 4140
ggcggcaatg tcgggttggg caatgtcggg gactttaacc tgggtgcggg caacgtcggt 4200
gggttcaatg tgggtggcgg caatatcggc ggcaacaatg tcgggttggg caatgtcggg 4260
tttggcaatg tcgggttggc gaattcgggg ttaacgccgg gtctgatggg tttgggtaat 4320
atcgggtttg gtaatgccgg cagctacaat ttcggtttgg cgaatatggg tgtgggcaat 4380
attgggttcg ctaacaccgg cagtgggaat ttcggtattg ggttgaccgg tgataatctg 4440
accgggttcg gtggtttcaa taccggcagc gggaatgtgg ggttgtttaa ttcggggacc 4500
ggtaatgtgg ggttctttaa ctctggcacc gggaactggg gggtgttcaa ttcggggagt 4560
tataataccg ggatcggtaa ttcggggatt gccagcacgg ggttgttcaa cgcgggtggg 4620
ttcaacacgg gtgtggtcaa tgcgggtagc tacaacaccg gcagtttcaa cgcggggcag 4680
gccaatacgg gcggtttcaa cccgggcagt gtcaacacgg gttggttgaa caccggtgac 4740
atcaacaccg ggtggccaa ctccggcgac gtcaacaccg gcgccttcat ctccggtaac 4800
tacagcaacg gcgccttctg gcggggcgac taccagggcc tgctcggctt ctcctaccgc 4860
cccgccgtgc ttccccaaac gccgttcctg gacctcaccc tcaccggcgg actgggctcc 4920
gtcgttatcc ccgccatcga cattcccgcg atccgccccg agttcagcgc caacgtcgcc 4980
atcgacagct tcactgtgcc gagcatcccg attccccaga tcgacctggc cgccaccacg 5040
gtcagcgtcg gcctcggccc catcaccgtc ccgcacctcg atattccacg ggtgcccgtc 5100
acgctgaatt acttgtttgg ctcacaaccc ggcgggcccc tgaaaatcgg tccgattacg 5160
ggactcttca acacccccat cggccttacc cccctggcgt taagccagat agtcatcggt 5220
gctagctcgt cgcaagggac catcacggct ttcctggcca acctgccgtt cagcaccccc 5280
```

```
gtcgtcacca ttgacgagat cccgctgctg gccagcatta ccggccacag cgagcccgtc     5340
gacatcttcc cgggcggcct cacgatcccc gcgatgaacc cgctgagcat caacctgtcc     5400
ggtggcaccg gcgccgtcac cattccggca atcaccatcg gcgaaatccc ctttgacctc     5460
gtggcccaca gcacgctcgg ccccgttcac atcctcatcg accttcccgc cgtgccgggg     5520
ttcgggaata cgaccggtgc tccgtcgtcg ggtttcttca actccggtgc gggtggggtg     5580
tcggggtttg ggaatgtcgg cgcgatggtg tcgggtggct ggaatcaggc tccgtcggcg     5640
ttgctgggtg ggggttcggg tgttttcaac gccggcacgc tgcattcggg tgtgctgaat     5700
ttcggctctg gcatgtcggg gctgttcaac accagcgtgt tggggttggg tgcgccggcg     5760
ttggtgtcgg gtttgggtag tgtcggtcag cagttgtcgg gattgttggc gagcgggacg     5820
gcgctgcatc agggtctggt cctcaatttc gggttggccg atgtggggtt gggcaatgtc     5880
gggttgggca atgtcgggga ctttaacctg ggtgcgggca acgtcggtgg gttcaatgtg     5940
ggtggcggca atatcggcgg caacaatgtc gggttgggca atgtcgggtg ggcaactttt     6000
gggctcggga attcggggtt aacgccgggt ctgatgggtt tgggtaatat cggtttggt      6060
aatgccggca gctacaattt cggtttggcg aatatgggtg tgggcaatat tgggttcgct     6120
aacaccggca gtgggaattt cggtattggg ttgaccggtg ataatctgac cgggttcggt     6180
ggtttcaata ccggcagcgg gaatgtgggg ttgtttaatt cggggaccgg taatgtgggg     6240
ttctttaact ctggcaccgg gaactggggg gtgttcaatt cggggagtta taacaccggg     6300
atcggtaatt cggggattgc cagcacgggg ttgttcaacg cgggtgggtt caacacgggt     6360
gtggtcaatg cgggtagcta caacaccggc agtttcaacg cggggcaggc caatacgggc     6420
ggtttcaacc cgggcagtgt caacacgggt tggttgaaca ccggtgacat caacaccggg     6480
gtggccaact ccggcgacgt caacaccggc gccttcatct ccggcaacta cagcaacggc     6540
gccttctggc ggggcgacta ccagggcctg ctcggcttct cctacaccag caccatcatt     6600
cccgaattca ctgtcgcgaa catccacgcg tccggcggcg ccggacccat catcgttccg     6660
tcgatccaat ttccggcaat tcccttggac ctcagcgcaa ccggccacat cggcggcttc     6720
accatcccgc cggtgtccat ttccccgatc acggttcgca tcgacccagt cttcgacctc     6780
ggccccatca ccgtccagga catcacgatt cccgccctgg gactcgaccc cgcaaccggt     6840
gtcaccgtgg gcccgatatt cagctcaggc tccatcatcg atccattcag ccttacgctg     6900
ctggggttca tcaacgttaa tgtccccgcc atccaaacgg cgcccagcga gattctgcca     6960
ttcaccgtgc tgctgagttc gcttggcgtg acccatctaa caccggaaat caccatcccg     7020
ggattccaca tacccgtcga tccaatccat gtcgagctgc ccctgtccgt caccatcgga     7080
cccttcgtga gcccggaaat caccattccc caactcccgc tgggcctcgc gttgtccggc     7140
gccacccccg ccttcgcctt ccctctggag atcaccatcg accgaatccc agtggttctc     7200
gacgtcaacg cgctgctcgg ccccatcaac gccgggttgg tcatcccgcc cgtcccggga     7260
ttcggcaaca ccaccgcggt cccgtcgtcg ggttcttca acatcggcgg tggcggcggg      7320
ttgtcgggct tccacaacct cggcgcgggc atgtcgggcg tgctcaacgc gatctcggat     7380
ccgctgctcg gatcggcgtc gggcttcgcc aacttcggca cccaactctc cggcatcctc     7440
aaccgcggcg cggacatctc gggtgtgtac aacaccggcg cgctgggcct gatcacctcg     7500
gccctggtct ccggcttcgg caacgtcggc cagcaactgg cgggcctgat ttacaccggc     7560
accgggccct aa                                                          7572
```

<212> Type : DNA
<211> Length : 7572
SequenceName : SEQ ID 527
SequenceDescription :
Sequence
--------
<213> OrganismName : Mycobacterium tuberculosis H37Rv
<400> PreSequenceString :

```
atgtcatttg tgattgcggt gccggaagca ttgacgatgg cggcttcgga tctggccaac       60
attgggtcga cgatcaacgc ggcgaatgcg gcggcggcat tgccgaccac gggggtggtg      120
gcggctgccg cggatgaggt ttcggcggca gttgcggcct tgttcgggtc gtacgcgcag      180
agctatcagg cttttggtgc gcagctgtcg gcgtttcacg cccagttcgt gcagtccctt      240
acgaacggcg cgcgctcata cgtagttgcc gaggccacca gtgctgcgcc gttgcaggat      300
ttgttgggcg tggtaaatgc cccgcccag gcgttgttgg ggcgcccgtt gatcggcaat       360
ggcgctaacg gggccgacgg gacgggggct cccggtgggc cgggcgggct gttgcttggc      420
aacggcggga atggcggatc gggtgcgccg ggtcagccag gtggtgctgg cggggatgcg      480
gggttgatcg gtaacggcgg gactggcggt aaaggtgggg acgggctggt cgggtccggt      540
gctgccgggg gtgtcggtgg tcgcggtgga tggttgctgg gtaatggcgg gaccggtggg      600
gctggtgggg ctgcaggggc cactttggtc ggcggtactg gcggtgtcgg tggggcgacg      660
gggttgatcg gcagcggggg cttcggcggt gctggcgggg ccgcggcggg ggtgggcacc      720
accggcggcg tgggcgggag cggtggcgtt ggcggcgtgt tcggcaatgg tggattcggc      780
ggggccggtg gccttggcgc cgccggcggc gtcggagggg cggccagcta cttcgggacc      840
gggggcggtg gcggcgttgg tggggacggt gcgcccggtg gtgacggcgg tgcgggtccg      900
ctattgatcg gcaatggcgg tgttgggggt ctgggtgggg ccggggcggc cggtggtaat      960
ggcggtgccg gcgggatgtt gttgggcgat ggcggtgccg gcggacaggg tgggccggcc     1020
gtggcgggtg tcctgggcgg gatgcccggc gcgggcggca acggcggtaa tgccaactgg     1080
ttcgggtccg gtggtgccgg cgggcagggt ggcaccggtc tggccgggac aaacggggtc     1140
```

```
aaccccggct cgattgcgaa ccccaacacc ggtgcgaacg gtaccgacaa cagcggcaac     1200
gggaatcaaa ctggcgggaa cgggggtccc ggccccgccg gtggcgtcgg cgaggctggc     1260
ggcgtcggcg ggcagggcgg gctgggggag tcgctcgacg gcaacgacgg caccggcggt     1320
aagggtggag ccgggggtac tgccggtacc gatggcggtg ccggcggcgc tggcggcgct     1380
ggcggcatag gtgagaccga cggcagcgcc ggcggcgtgg ctaccggggg tgagggggt      1440
gacggtgcca ccggaggggt cgacggtggc gtgggtggtg ctggcggcaa ggggggcag      1500
gggcacaaca cgggtgtagg tgacgctttc ggcggtgacg gcggaatcgg cggtgacggt     1560
aacggggcac taggcgcggc gggcggtaac ggcggcaccg gtggtgccgg tggaaacggt     1620
ggacgtggcg ggatgttaat cggcaacggc ggcgccggtg gggccggcgg gacgggcggc     1680
accggtggtg gtggcgccgc cggcttcgcg ggcggtgtcg gcggcgcggg cggagagggt     1740
ctcaccgacg gtgcgggtac cgcggaaggc ggcaccggcg gtctgggggg cctcggcggt     1800
gtcggcggta ccggcggtat gggtggcagc ggcggtgtcg gcggcaacgg cggggcggct     1860
gggtcgctca tcgggcttgg tggtggcggg ggtgccggcg gtgtcggcgg caccggtggc     1920
atcggcggca tcggtggtgc cggcggcaac ggtggcgccg gcggcgcggg taccaccacc     1980
ggcgggggag cgacaattgg cggtggcggc ggtacaggcg gcgtgggggg cgctggtggc     2040
actggcggta ccggcggcgc cggcgggacc accggcggca gcggcggagc cggcgggctg     2100
atcgggtggg caggagctgc cggcggcacc ggcgcaggcg gcacgggtgg gcaaggtggc     2160
ctcggcggcc agggcggcaa cggcggcaac ggcgggaccg gcgcgaccgg cggtcagggc     2220
ggcgatttcg cgctgggcgg caacgggggc gccggcggcg cgggtgggtc accgggtggc     2280
agctccggca tccagggcaa tatgggcccg cccggcaccc agggcgccga cggatag        2337
```

<212> Type : DNA
<211> Length : 2337
SequenceName : SEQ ID 528
SequenceDescription :
Sequence
--------
<213> OrganismName : Mycobacterium tuberculosis H37Rv
<400> PreSequenceString :

```
ccccaaggcg ccgacggcaa cgccggcaac ggcggtgacg gcggggtcgg cggcaacggc     60
ggaaacggcg cagacaacac caccaccgcc gccgccggca ccacaggcgg ggccggcggg    120
gccggcgggg ccggcggaac cggcggaacc ggcggagccg ccggcaccgg caccggcggc    180
caacaaggca acggcggcaa cggcggcaac ggcggcaccg gcggcaaagg cggcaccggc    240
ggcgacggtg cactcgcagg cagcagcggt ggtgccggcg gtaaaggcgg caacggcggc    300
gacgccggca aggccggtac cggctccgct cctggcacgg cggggaccgg cggcgatggg    360
ggtaagggcg gcaacggcgg cattggcgct gccggcacaa ccggccccgt aggcaccggc    420
gcgtccggcg gcaccggtgg tagtggtggc gccggcggaa ccggcggtga cggcggcgcc    480
gccaacggcg gcaccgccgg ggctggcggg gcgggcggca atggcggcaa aggcggcgac    540
ggtggagcag gcgtcaccag cagcaccgcc ggcaacagcg gcggcgcggg cggcagcggc    600
ggaaagggcg gagacgcggg cgcgggcggc gccggtgcca ctccgggcgc caacggtatc    660
gctggcaatg gcggcgacgg cggagatggc gcggctggtg ccgtcggcat ctccggcgca    720
accggcgctg gcgacggcgg gcatggcgga accggcgcgg ccggcggcaa cggtggaacc    780
ggcggtgctg gcggtagcgg catcgacggc gtcggcggcg ggaccggagg taccggcggc    840
aacggcggca acggcgccat cggcggcgct ggcggagacg ccggtggtag cggaaatagc    900
ggcggaaacg gtgggattgg cggaaagggc ggaaacgccg gtgccggtgg tgccgcgggc    960
agcaacggcg gtaccgtcgg cgccaacggt accggcggcg acggcggcaa cggcggcgct   1020
gccggggccg ccacggctgg cagcaacggt ggggccggca ccggctcggc cggcggcaac   1080
ggcggcaccg gcggcagagg cggcagtggt ggcgccggcg gcgacggtat cggtggcgtc   1140
ggcggcggca agggcggcaa cggcgcggac ggcgaagtcg gcggtgcggg cggcgccggc   1200
ggcagcgggc ccaacaccag tcccggcggc aacggcgggc aaggaggtca aggcggcagc   1260
ggtggtgccg gtggggcggc cggggctggc ggcgccggtg gcggcgctaa cggcaccgct   1320
ggcaacggcg gccaaggcgg tgccggcggc accggcggcg ccggcgcagc ctcctcagct   1380
accaacggcg gcagcggcgg cgccggcggc accggaggcg acggcggcag cggcggcgcc   1440
ggcggcaccg gaggcgccgg cggcaccggc ggggcggccg gcgacggcgg acaaggtggc   1500
cagggcggcg ccggcggcgg tgccggtggt caaggtggtg ccggcggtgc cggcgggacc   1560
ggcggcaacg gcggcaatat caccggcggc accgcgggca ccgcgggggc cgccggtaac   1620
ggcggcgccg ccggaaaggg tggcgccggc ggccaaggcg gcaccggtgg cgggaccggg   1680
ggtcagggtg gcgccggcgg cgacggcggt gccggcggca ccggcggcga ccgcaccgtc   1740
ggcggtggca cggtccccgc cggctccggt ggacaaggcg gtaacgctgg cggtggtggg   1800
gccggcgggc agggtggagc cgacggcggc agcggcggcg acggcggcga cgccggcaca   1860
ggtggcaatg gcggtaacgg cggcaaccgt aattccggca atggcaccgg cggcgctggc   1920
ggcaacggtg gtggtggtgc taacggtggc gccggcggcg ctgggggcag cggcggcggc   1980
accggcggca acggcggcgc tggcggcgac gccggcgacg ccggcaacgg cggcaacggc   2040
aacggcaccg gcaacggcgg caacggcggc aacggcggca tcgccggcat gggcggcaac   2100
ggcggtgccg ggacgggcag cggcaacggc ggcaacggcg gcagcggcgg caacggcggc   2160
aacgccggca tgggcggcaa cagcggcacc ggcagcggcg acggcggtgc cggcgggaac   2220

ggcggcgcgg cgggcacggg cggcaccggc ggcgacggcg gcctcaccgg tactggcggc   2280
accggcggca gcggtggcac cggcggtgac ggcggtaacg gcggcaacgg agcagataac   2340
accgcaaaca tgactgcgca ggcgggcggt gacggtggca acggcggcga cggtggcttc   2400
ggcggcgggg ccggggccgg cggcaccggc ttgaccgctg gcgccaacgg caccggcggg   2460
caaggcggcg ccggcggcga tggcggcaac ggggccatcg gcggccacgg cccactcact   2520
gacgaccccg gcggcaacgg gggcaccggc ggcaacggcg gcaccggcgg caccggcggc   2580
gcgggcatcg gcagccttgg cggcggcact ggcggcgatg gcggcaacgg cggcaacggc   2640
ggtaccggcg gcgagggcgg cgaggtcggc ggcgccggcg gcaccggcgg tgcggccggc   2700
aatggcggcg atggcggcac cggcggcacc ggcggcgggg acgggggcgc cggcggcacc   2760
ggcggcaccg gcggcaccgg cggcctcggc gacccccggg tcggcggatc cggcggcgac   2820
ggcggcaccg gcggcagcgg cggtgcggcc ggcaatggcg gcaacggcgg caacgccggc   2880
gcgggaggca atggcaacgg cggcaccggt ggggccggcg gtatcggcgg caccggcggc   2940
aatggcggcg acgccgagcc cggagtgccc ccgggagccg gtggtgctgg cggcgccggc   3000
accaccggcg gcaagggtgg caccggcggc aacggcagtg gcaccggctc gggcggcacc   3060
ggcggcgatg gcggcaccgg cggtggtggt gggaacggcg gcaccggctg gaatggcggc   3120
aagggagaca ccggcagcgg cggtggcgcc ggagacggtg gtaaggcacc agccggtggc   3180
accggcggcg ccggcggcga cggcggagcg ggcggcaagg gcggcagcgg cggcgtctag   3240
```

<212> Type : DNA
<211> Length : 3240
SequenceName : SEQ ID 529
SequenceDescription :
Sequence
--------
<213> OrganismName : Mycobacterium tuberculosis H37Rv
<400> PreSequenceString :

```
atggtcatgt cgctgatggt ggcgccggag ctggtggcgg cggccgcggc ggacttgacc      60
gggattgggc aggccatcag cgcggcgaat gcggcggcag cgggcccgac gacgcaggtg     120
ttggcggccg ccggtgatga ggtgtcggcg gcgatcgcgg cgttgtttgg tacccacgcg     180
caggagtacc aggcgttgag cgcccgggtg gcgacgtttc atgagcagtt tgtgcgctcg     240
ctgaccgcgg ctggcagcgc gtatgcgact gccgaggcgg cgaatgcatc accgctgcag     300
gcgctggagc agcaagtgtt gggtgcgatc aacgcgccca cacagctgtg gttggggcgc     360
ccgctgatcg gtgatggcgt tcacggggcg ccggggaccg ggcagcccgg tggggccggg     420
gggttgttgt ggggtaatgg cggtaacggc ggttcggggg cggccggtca agtcggtggg     480
cccggcggcg cggccgggtt gttcggcaac ggcgggtccg gcgggtccgg cggggccggc     540
gctgccggcg gtgtcggcgg atccggcggg tggttgaacg gcaacggcgg ggccggcggg     600
gccggcggga ccggcgctaa cggtggtgcc ggcggcaacg cctggttgtt cggggccggc     660
gggtccggcg gcgccggcac caatggtggc gtcggcgggt ccggcggatt tgtctacggc     720
aacggcggcg ccggcgggat cggcggcatc ggaggtatag gcggcaacgg tggcgacgcc     780
gggctgttcg ggaacggcgg cgccgggggg gccggggccg cgggcctgcc gggtgccgcc     840
ggcctcaacg gcggcgacgg cagcgacggc ggcaacggcg gaaccggcgg caacggcggg     900
cgcggcgggt tattggttgg caacggcggg gccggcgggg ccggcggcgt cggcggcgac     960
ggtggtaagg gcggcgctgg cgatccgagt ttcgccgtca acaacggtgc cggcggtaac    1020
ggcggtcacg gcggcaaccc cggcgtgggc ggggccggtg gggccggcgg cctgctggcg    1080
ggtgcgcacg gtgccgccgg cgccaccccc accagcggcg gcaacggcgg cgatggcggc    1140
atcggcgcca ccgccaactc acccctacaa gccggcgggg ccggcggtaa tggcggtcat    1200
ggcgggttgg tcggcaacgg cggcaccggc ggcgccggcg gtgccggtca tgcgggttcc    1260
accggcgcta ccggtaccgc cttacaaccg acgggcggta acggcaccaa tggcggcgcc    1320
gggggccacg gcggtaatgg cggaaatggc ggcgcccagc acggcgacgg cggcgtcggc    1380
ggcaagggcg gtgccggcgg tagcggcggc gccggcggaa acggattcga cgccgccacc    1440
ttgggttcgc ccggtgccga tggcggtatg ggcggcaacg gcggcaaggg cggtgacggc    1500
ggcaaggccg gtgatggcgg agccggtgcc gccggtgatg tgaccttggc cgtcaaccag    1560
ggtgccggcg gtgacggcgg caacggcggt gaagtgggcg ttggcggcaa gggtggggcc    1620
ggcggtgtta gcgcgaaccc ggccctgaac ggttcggccg gggcgaacgg caccgcgccc    1680
accagcggcg gcaacggtgg caacggaggt gccggcgcca cccccaccgt cgcgggagaa    1740
aacggcggcg ccggtggtaa cggcggccat ggcgggtcgg tcggtaacgg cggtgcgggt    1800
ggtgccggcg gaaatggcgt cgccggcacc ggccttgccc tcaacggcgg caacggcggc    1860
aacggcggca tcggcggcaa cggcggatcg gcggccggca cgggcgggga cggcggcaag    1920
ggcggcaacg gggcgccgg agccaacggc caagacttct ccgcgtccgc caatggcgcg    1980
aatggcggac agggcggcaa cggcggcaac ggcggcatcg gcggcaaggg tggtgacgcc    2040
ttcgccacgt tcgctaaggc cggcaacggc ggtgccggcg gcaacggcgg caatgtgggc    2100
gttgccggcc agggtggggc cggcggcaag ggcgccattc cagccatgaa gggtgcgacc    2160
ggcgccgatg gcaccgcacc caccagcggc ggtgacggcg gcaacggcgg caacggcgcc    2220
agccccaccg tcgcgggcgg caacggcggt gacggcggca agggcggcag cggcgggaat    2280
gtcggcaatg gcggcaatgg cggggccggc ggcaacggcg cggccggcca agccggtacg    2340
ccgggcccta ccagcggtga ttccggcacc tcgggcaccg acggtggggc tggcggcaac    2400
```

ggcggggcgg gcggcgccgg cggaacactg gccggccacg gcggcaacgg tggtaagggt 2460
ggtaacggcg gccagggtgg catcggcggc gccggcgaga gaggcgccga cggcgccggc 2520
cccaatgcta acggcgcaaa cggcgagaac ggcggtagcg gtggtaacgg tggcgacggc 2580
ggcgccggcg gcaatggcgg cgcgggcggc aaggcgcagg cggccgggta caccgacggc 2640
gccacgggca ccggcggcga cggcggcaac ggcggcgatg gcggcaaagc cggtgacggc 2700
ggggccggcg aaaacggcct aaacagcggg gccatgctgc cgggcggcgg caccgtagga 2760
aaccccggta ccggcggcaa cggcggcaac ggcggcaacg ccggcgtcgg cggcaccgga 2820
ggcaaggccg gcaccggctc cttgacgggc ttggacggca ccgacggcat cacccccaac 2880
ggcggtaacg gcggcaatgg cggcaacggc ggcaagggcg gcaccgccgg caacgggagc 2940
ggcgcggccg gcggcaacgg cggcaacggc ggctccggcc tcaacggcgg tgacgccggc 3000
aacggcggca acggcggtgg ggcgctgaac caggccggct tcttcggcac gggcggcaaa 3060
ggcggtaacg gcggcaatgg cggtgccggc atgatcaacg gcggcctcgg cggcttcggc 3120
ggcgccggcg gtggcggcgc cgttgacgtc gccgcgacaa cgggcggcgc tggcggcaat 3180
ggcggtgccg gcggcttcgc tagcaccggg ttgggtggcc caggcggcgc cggcggtccc 3240
ggcggcgcgg gcgactttgc tagcggtgtt ggcggtgtcg gcggcgccgg cggggacggc 3300
ggtgccggcg gggtcggcgg cttcggcggc cagggcggca tcggcgggga agggcgcaca 3360
ggcggcaacg gcggtagcgg cggcgacggc ggtggcggca tttccttagg cggcaacggc 3420
ggcctcggcg gcaacggcgg cgtctccgag actgggtttg gcggcgccgg cggcaacggc 3480
ggctacggcg gtccgggagg ccccgaaggc aatggcggcc tcggcggcaa cggcggcgcc 3540
ggcggcaacg gcggcgtcag caccacgggc ggcgacggcg gcgccggcgg caagggcggc 3600
aacggcggcg acggcgggaa cgtcggtttg ggcggtgacg ccggctccgg cggcgcgggc 3660
ggcaatggcg gtatcggcac cgacgcgggc ggtgccggag gggccggtgg cgctggcggt 3720
aacggcggta gcagcaaaag cacgaccacc ggcaacgccg gctccggtgg tgccggcggt 3780
aatgggggca ctggcctcaa cggcgcgggc ggtgctggcg gggccggcgg caacgcgggt 3840
gtcgccggcg tgtccttcgg caacgctgtg ggcggcgacg gcggcaacgg cggcaacggc 3900
ggccacggcg gcgacggcac gacgggcggc gccggcggca agggcggcaa cggcagcagc 3960
ggtgccgcca gcggctcagg cgtcgtcaac gtcaccgccg gccacggcgg caacggcggc 4020
aatggcggca acggcggcaa cggctccgcg ggcgccggcg gccagggcgg tgccggcggc 4080
agcgccggca acggcggcca cggcggcggt gccaccggcg gcgacggcgg caacggcggc 4140
aacggcggca actccggcaa cagcaccggc gtcgcgggct tggccggtgg tgccgccggc 4200
gccggcggca acggcggcgg cacttccagc gccgccggcc acggcggcag cggcggcagc 4260
ggtggcagcg gcaccacggg cggcgccggc gcggccggcg gcaacggcgg cgccggtgct 4320
ggcgggggca gcctgagcac aggccagtcc ggcggcccac ggcggcagcg gtggtgccgg 4380
tggcaacggc ggcgctggct cggccggcaa cggcggcgcc ggtggtgccg gtggcaacgg 4440
cggtgccggc ggcaacggtg gcggtggcga tgccggcaac gccggctcag gcggcaatgg 4500
cggcaagggc ggcgacggtg tcggccctgg ctccaccggc ggcgcgggcg gcaagggcgg 4560
cgctggcgcc aacggcggtt ccagcaacgg caacgctcgc ggtggcaacg ccggtaa 4617

<212> Type : DNA
<211> Length : 4617
SequenceName : SEQ ID 530
SequenceDescription :
Sequence
--------
<213> OrganismName : Mycobacterium tuberculosis H37Rv
<400> PreSequenceString :

atgtccttcg tggtcacagc accgccggtg ctcgcgtcgg cggcgtcgga tctgggcggt 60
atcgcgtcca tgatcagcga ggccaacgcg atggcagcgg tccgaacgac ggcgttggcg 120
cccgccgccg ccgacgaggt ttcggcggcg atcgcggcgc tgttttccag ctacgcgcgg 180
gactatcaaa cgctgagcgt ccaggtgacg gccttccacg tgcagttcgc gcagacattg 240
accaatgcgg ggcagctgta tgcggtcgtc gacgtcggca atggcgtgct gttgaagacc 300
gagcagcagg tgctgggtgt gatcaatgcg cccacccaga cgttggtggg tcgtccgctg 360
atcggcgatg gcacccacgg ggcgccgggg accgggcaga acggtggggc gggcggaatc 420
ttgtggggca acggcggtaa cggcgggtcc ggggctcccg gacagccggg cggccggggc 480
ggtgatgccg gcctgttcgg ccacggcggt catggcggtg tcgggggggcc gggcatcgcc 540
ggtgccgctg gcaccgcggg cctgcccggg ggcaacggcg ccaacggcgg aagcggcggc 600
atcggcggcg ccggcggcgc cggcggcaac ggcgggctgc tattcggcaa cggtggtgcc 660
ggcggccagg gtggctccgg cggacttggg ggctccggcg ggacgggcgg cgcgggcatg 720
gctgccggtc ccgccggcgg caccggcggc atcgggggca tcggcggcat cggcggcgcg 780
ggcggggtcg gcggccacgg ctcggcgttg ttcggccacg ggggaatcaa cggcgatggc 840
ggtaccggcg gcatgggtgg ccagggcggt gctggcggca acggctgggc cgctgagggc 900
atcacggtcg gcattggtga gcaaggcggc cagggcggcg acgggggagc cggcggcgcc 960
ggcgggatcg gtggttcggc gggtgggatc ggcggcagcc agggtgcggg tgggcacggc 1020
ggcgacggcg gccagggcgg cgccggcggt agtggcggcg ttggcggcgg cggcgcaggc 1080
gccggcggcg acggcggcgc gggcggcatc ggcggcactg gcggtaacgg cagcatcggc 1140
ggggccgccg gcaatggcgg taacggcggc cgcggcggcg ccggtggcat ggccaccgcg 1200

ggaagtgatg gcggcaatgg cggcggcggc ggcaacggcg gcgtcggtgt tggcagcgcc 1260
ggaggggccg gcggcaccgg cggtgacggc ggggcggccg gggcgggcgg cgcgccgggc 1320
cacggctact tccaacagcc cgcgccccaa gggctgccca tcggaaccgg cgggaccggc 1380
ggcgaaggcg gtgccggcgg cgccggtgga gacggcgggc agggcgacat cggcttcgat 1440
ggcggccggg gtggcgacgg cggcccgggc ggtggcggcg gcgccggcgg tgacggcagc 1500
ggcaccttca atgcccaagc caacaacggc ggcgacggtg gtgccggcgg tgttgggggga 1560
gccggcggca ccggcggcac gggtggggtc ggggccgacg ggggtcgcgg gggggactcg 1620
ggccgcggcg gcgacggcgg caacgccggc cacggcggcg ccgcccaatt ctccggtcgc 1680
ggcgcctacg gcggtgaagg tggcagcggc ggcgccggcg gcaacgccgg tggcgccggc 1740
accggtggca ccgcgggctc cggcggtgcc ggaggtttcg gcggcaacgg tgccgatggc 1800
ggcaatggcg gcaacggtgg caacggcggc ttcggcggaa ttaacggcac gttcggcacc 1860
aacggtgccg gcggcaccgg cgggctcggc accctgctcg gcggccacaa cggcaacatc 1920
ggcctcaacg ggccaccgg cggcatcggc agcaccacgt tgaccaacgc gaccgtaccg 1980
ctgcagctgg tgaataccac cgagccggtg gtattcatct ccttaaacgg cggccaaatg 2040
gtgcccgtgc tgctcgacac cggatccacc ggtctggtca tggacagcca attcctgacg 2100
cagaacttcg gccccgtcat cgggacgggc accgccggtt acgccggcgg gctgacctac 2160
aactacaaca cctactcaac gacggtggat ttcggcaatg gccttctcac cctgccgacc 2220
agcgttaacg tcgtcacctc gtcatcaccg ggaaccctgg gcaacttctt gtcgagatcc 2280
ggtgcggtgg gcgtcttggg aatcgggccc aacaacgggt tcccgggcac cagctccatc 2340
gttaccgcga tgcccggcct gctcaacaac ggtgtgctca tcgacgaatc ggcgggcatc 2400
ctgcagttcg gtcccaacac attaaccggc ggtatcacga tttctggagc accgatttcc 2460
accgtggctg ttcagatcga caacgggccg ctgcaacaag ctccggtgat gttcgactcc 2520
ggcggcatca acggaaccat cccgtcagcc ctcgccagcc tgccgtccgg gggattcgtg 2580
ccggcgggaa cgaccatttc ggtctacacc agcgacggcc agacgctgtt gtactcctac 2640
accaccaccg cgacaaacac cccatttgtc acctccggcg gcgtgatgaa caccgggcac 2700
gtccccttcg cgcagcaacc gatatacgtc tcctacagcc ccaccgccat cgggacgacc 2760
acctttaact ga 2772

<212> Type : DNA
<211> Length : 2772
SequenceName : SEQ ID 531
SequenceDescription :
Sequence
--------
<213> OrganismName : Mycobacterium tuberculosis H37Rv
<400> PreSequenceString :

```
ttgatcggca acggcggggc cggcgggtcc ggggcgcccg gcgccatcgg tggggccggc      60
gggcccgcgg ggttgatcgg tgtcggaggt gccggcgggg ccggtggaga ctccgcggtc     120
gcgggtgtca tcggaggggc cggtggggca ggcggggctg ccctgctgtt cggtgccggt     180
ggggccggcg gggccggggg ttccggcggt tccggcgcag ctggtggggc cggtggcgcc     240
ggtggggccg gcgggctgtt cgccagcggc ggcagcggcg ggttcggcgg gttcgcatcg     300
acgggcaccg gtggggccgg cggcaccggt ggggctggtg ggttgttcgc cagcggcggg     360
gtcggcggta ctggcggggg agccgggtcc ggcggtaccg gtggggttgg tgggacgggt     420
ggggccggag ggctgttcgc tagcggcggc gctggcgggg ccggcgggtc cggcggtacc     480
ggtggggctg gtgggacggg tggggccggc gggctgttcg gagccggtgg cgctggcggg     540
ctcggcgggc aaggcaacca caccggcggg cacggtgggg ccggtggcag cgccggcctg     600
ctcgcccttg gcgacggcgg cgctggcggg gccggcgggg ccgctaccac cggaaccggc     660
ggggccggcg gggcgggtgg caaggccggc ctgctgttcg gctccggtgg ggccggtggg     720
tccggtgggg ctgccggcac cttcggtgac accggtaact ccggcggggc cggtggggcg     780
ggtggcaagg ccggcctgct gttcggctcc ggtggggccg gtgggtccgg cggcgctggg     840
ggcttcgcca acggctctac cggcggtgcc ggcggggccg gcggcggggc cgggctgatc     900
ggcaacggcg gcaacggtgg cagcggcggc acgtcggttg ccaccggggg ggccgggaac     960
ggcggtgccg gcggcgccgg cggcggggcc gggctgatcg gcaacggcgg caacggcggc    1020
agtggcggaa tgggcgatgc cccgggcggc accggcgtcg gcggcatcgg tgggctgttg    1080
ttgggtttgg acggcgccaa cgccccggcc agcaccaacc cgctgcacac cgcgcagcag    1140
caggcgttgg ccgcagtcaa cgcgcccatc caggccgtga ccgggcgccc gctgatcggc    1200
aacggcgcca acggcgcccc gggcagcggg gcccccggcg ggcacggcgg gtggttgttc    1260
ggcggcggag ggaccggcgg gtccggcgtc agcggcgggg cgggcggaga tggcggggcc    1320
ggcgggatct tgttcggcgc cggcggggcc ggcggcgcgg gcggggccgt cacgggaacc    1380
ggcgccaccg gcgggtccgg tggggccggc ggtggagcct tgctgtttgg ggccggtggg    1440
gccggtggag ccggcgggtc cagcgggatt ggcgggttcg ccgcgggcgg ggccggtggg    1500
cccggagggg ccggtgggct gttcaacggc ggcggggccg gcggggccgg cgggtccggc    1560
gtcagcggcg gggctggcgg ggagggcggg gccggcgggg ccggtggcct gttcgccggt    1620
ggcggggccg gcggggccgg cggatcgggc aacaacgtcg ggggggccgg cggggccggt    1680
ggggtcggtg ggctgttcgg ggccggcggg gccggcggat ccggcggcgg cggtagcgtt    1740
gctggcgaca gtggggccgg cggcaacgcg ggcttgctcg cccccggtct cgccggcggt    1800
gccggcggtg gcggcgggca gggttttgac accggcgggg ccggcgggcc cggcggcgac    1860

gccggcctgc tggtcggctc cggcggggtc ggaggtgccg gcggattcgg cctcactacg    1920
ggtgggcctg gggcggccgg cggcgacgcc ggcctgctgt tcggctccgg cggcgctggc    1980
ggggccggcg gctccggccg aaccgacctc ggcggcgctg gcggagccgg cggcaaggcc    2040
gggctgatcg gcaacggcgg taacggcggg gccggcgggg ccggcgggaa cggcggcggg    2100
gacggcgggc ccggtggagc cgccttcggg ctcggtaacg gcggcaacgg cggcaacggg    2160
gggaccggca cgtccgcggg cagccccggt gccggcggcg ccggtggttc gctgatcggc    2220
gcggaggggc tgcccgggct gctgccctag                                     2250
```

<212> Type : DNA
<211> Length : 2250
SequenceName : SEQ ID 532
SequenceDescription :
Sequence
--------
<213> OrganismName : Mycobacterium tuberculosis H37Rv
<400> PreSequenceString :

```
atgtcgtttg tgattgctgc gccggaggcg ttggtcgcgg tcgcttcgga tctggcgggc      60
attgggtcgg cgctggcgga ggccaacgcc gcggcgttgg ccccgacgac ggcgttgttg     120
gccgcgggtg ccgatgaggt gtcggcggcg atcgcggcgc tgtttggcgc gcacgggcag     180
gcgtatcaga cggttagcgc ccaggcgtcg gcgtttcatg cccagtttgt gcaggcgttg     240
actggcggcg gcggggcgta tgcggctgcc gaggccgcca acgtctcggc ggcgcagagc     300
accgaccagc ggctgctcga tctgatcaat gggcccaccc aggcgttgtt ggggcgtcca     360
ctgatcggtg atggcgccaa cggcgggccg gggcaagacg gcgggcccgg ggggttgctg     420
tacggcaacg gcggcaacgg cggcactagt accaccgccg gggtggccgg cggcaacggt     480
ggcgccgccg ggctgatcgg caacggcggg gccgggggcg gcggcggggc cggcgcggcc     540
ggcggcaatg gcggtgcggg cgggtggctg tatggcaacg gcggcgccgg cggggccggt     600
gggacatcgg tgatacccgg tgtcgccggc ggcaatggcg gggctggcgg gtccgcggga     660
ctgtggggta ccggcggggc cggtggcgac ggcggcaacg gccggtcggg gccagtcaac     720
gtcgccggca gcgcgggcgg caacggtggc gctggtggcg ccgccgggtt attcggtgac     780
gccggggccg gtggcaacgg cggcaagggc ggtgctggcg gcgccgcctt tagcattaac     840
ttcaccgcag gcgatggcgg tgcgggaggt gccggtgggt ccggcggcca cgcattgctg     900
tggggcgccg gcggagccgg gggtaacggc ggatccggcg gcacgggggg tgccggcggc     960
agcaccgctg gcgctggcgg caacggcggg gccgggggtg gcggcggaac cggtgggttg    1020
ctcttcggca acggcggtgc cggcgggcac ggcgccgccg ccggaaacgg cttagccgcg    1080
ggtaatggcg tcagcagcag cggcggcggc ggtgccggtg ggaccggcgg ggccggtggg    1140
gacggtggcg ccggcggggc cggaggcaac gccaggctgt ggggcgtcgg tggcgccggc    1200
ggggccggcg gggacggtgg cgccggcggg gccggcggca aaggcggctc tggcctcagc    1260
ggtaacgcca acggcggggc cggcggcgac agcggccgtg gcggcacggg cggcgccggc    1320
ggcgagggcg gcgccgccgg gctgctggtg ggcaccggcg ggcacggcgg tgacggcggg    1380
gccggcggcg ccgccgtcaa gggcggtgac ggcggggccg ccgccggcac gggcatcgcc    1440
ggcgctggcg gccgtggcgg cgcgggcggc agcggtggca gcggtggtga cggcgggggc    1500
ggggccgccg gccccgccgg gtggctgttc ggcgatggcg gggctggcgg gaacggcggg    1560
gccgcggccg ccggcggcgc cggcggccaa gccggcggtg gcggcgggaa cggcggcaat    1620
ggcggcaacg gcggcaatgg cggcaatggc ggcaacggcg ccaccggggg gtggctgtac    1680
ggcaacggcg gggccggcgg ccagggcgcc accgccggag ccggcggagc cggcgctaac    1740
ggcgtcagca gcaccaatgg cggcggcacc ggcggcaacg gggggatcgg cgggaccggt    1800
gggtccggcg gggccggtgg caacgccggg ctgttgggcg tgggcggcgc cggcgggcac    1860
ggcgcctccg gcggcgccgg cgataggggc ggcgctggcg gtaccgggtt cataagcagt    1920
gacggcggtg ctggcggtga tggcggtgat ggcggcaacg gcggggccgg cggcaccggt    1980
gggctgttgt tcggtgccgg cggcaatggt ggcccccggcg ggtctggcgg tgccgccgat   2040
attggcggca acggcggcgc cggtaacggc gggggcaccg acgggaacgg cggtaatggc    2100
gggtccggcg gcggcgccgg cagcggcggt gacggcggcg ggctggcgg caacggtgcg     2160
tggctgttcg gcaatggcgg cgccggcggg ggcggcggaa aaggcggcaa cggtgccggc    2220
ggcgggcttg gcggcggttc attcggcctc cccggcctga acggcagcgg cggcgacggt    2280
ggcgacggcg gtaacggtgc ccccggcggg gtgctgtatg gcaatggcgg cgccggcggc    2340
caggggtcaa gcggtggcat cggcggcccc ggcgccaccg gcggtgccgg cggcaaaggc    2400
ggtgatggtg gcgatgcgca gctgatcggc gacggcggca atgggggcaa cggaggcgcg    2460
ggcggcaccg gggcaccccc ggggcccggc ggacccggcg ggtccggcgg gcttggaggc    2520
ctgctgttcg gccaaaccgg cacggctggc gtgtcgccgt ag                       2562
```

<212> Type : DNA
<211> Length : 2562
SequenceName : SEQ ID 533
SequenceDescription :
Sequence
--------
<213> OrganismName : Mycobacterium tuberculosis H37Rv
<400> PreSequenceString :

```
atgtcctatc tcgtcgtggt gccggagttg gtcgcagcgg cggcaacaga tttggcgaac 60
atcggttcgt cgattagtgc agccaacgcg gccgcggcgg caccgaccac ggcactggtc 120
gcagccggcg gcgacgaggt atcggcggcc atagccgcgt tgttcggagc gcatgctcgg 180
gcatatcaag cgttgagtgc ccaggcggcg atgtttcatg aacagtttgt ccgggccctc 240
gccgccggcg gtaactccta cgccgtcgct gaggcggcaa ccgcgcaatc ggttcagcaa 300
gatctgctca acctgatcaa tgcgcccacc caggcgctgt tggggcgtcc gctgatcggc 360
aacggcgcca acgggctgcc gggtacgggc cagaacggcg gcgacggcgg gattctgtac 420
ggcaacggcg gcaacggtgg gtccggcggg gtcaaccagg ccggtggcaa tggcgggaat 480
gctgggctgt ggggcaatgg cggatccggc ggagccggcg ggaacgccac cactgccggc 540
cgcaacggct tcaacggggg cgccggggga agcggcggtt tgctgtgggg caatggcggt 600
gccggcgggg ccggtgggaa cggcggtccg gctccgctcg tgggcggggt gggcaccacc 660
ggtggcgccg gcgggaacgg cggcggcgcc gggttgttct acggtttcgg cggcgccggt 720
gggaacggcg ggatgggcgg ggtggcaccg agcaccggcc cctcgatggg catcctcccg 780
gccggcggtg tcggcgggcc tggtggctcc ggcggggcga gcgcgcttgc cttcggctcc 840
ggcggcgtcg gcggtgccgg tggcttgggc gggccgaccg atggcaccgt ccagggggtg 900
ggcggcttcg gcggtcaggg cggcaacggc gggcagagcg gcttgttgtt tggcaacgcg 960
ggagccggcg gggcaggcgc tgccggcgga gccggcaccg gcgacaccga gagcttcggc 1020
ggccacggcg gggccggcgg tgatggcggc gctgttggct tgatcggtaa cggcggggcc 1080
ggcggcaccg gatctcccgg cgctgtggtg ggtggtaacg gcggcgtcgg tggtctgggt 1140
ggcgccggca gtcccggggg tctgttgtac ggcaccgggg gggccggcgg caatggcgga 1200
ccgggtggtg acggtggtac tggcgcgacg gtgggctttg ccggctccgg cggtttcggc 1260
ggtgcggggg gcatcgccca gctgtttggc acgggtggca tgggtggtag cggcggtggt 1320
ataggcgctg gcaccacgac cgtggtgccg cccgacgtcg ccccggtggg tggcacaggc 1380
ggcaatggcg gtcgcgccgg gctgctgttg ggtgtgggtg gcatgggcgg taatggcggt 1440
gccaccagcg tcggcgggac gctctacgcc gccggtggaa acggcggcga cggcgggttg 1500
gtgtggggca acggtggcac cggcgggagc ggtggcgccg gcggggcggg cagcgtcggc 1560
aacggcggtg cgggtggcaa cgcggcactg ctgttcggca acggcggggc gggcggggcc 1620
ggcggcgccg gcggcatcgg tgccggcgga gccggcggct tcggcgcggt tctgtttggc 1680
aacggcgggg ctggcgggag cggtgccccc ggtggcatcg gcgccggtgg caatggcgga 1740
aacgcgctgc tggtcggcaa cggcggcaac ggtggggcag gtaccggtgg ggctgctggc 1800
ggtgccggtg gctcgggcgg gttgctattc ggccaaaatg ggatgcccgg gccgtga 1857
```

<212> Type : DNA
<211> Length : 1857
SequenceName : SEQ ID 534
SequenceDescription :
Sequence
--------
<213> OrganismName : Mycobacterium tuberculosis H37Rv
<400> PreSequenceString :

```
atgaattttt ctgtactgcc gccggagatc aattcagcgc tgatattcgc cggggcaggg 60
ccggaaccga tggcggcggc cgcgacggcc tgggacgggt tggccatgga attggcctcg 120
gccgcagcct ctttcggctc agtgacatcc ggactcgtgg gcggggcgtg gcagggcgcg 180
tcgtcgtcgg cgatggcggc agcggcagcc ccctatgcgg cgtggcttgc cgcggcggcg 240
gtccaggccg agcagacggc cgctcaggct gcggcgatga tagccgagtt tgaagcggtc 300
aagacggcgg tggtgcagcc gatgctggtg gcggccaacc gtgccgacct ggtgtcgctg 360
gtgatgtcga acctgtttgg acagaacgct ccggcgatcg ctgccattga agccacgtac 420
gagcaaatgt gggctgccga tgtgtcggcg atgtctgcct accatgccgg ggcatcggcg 480
atcgcctcgg cgctgtcccc gttcagtaaa ccgctgcaga acctggctgg cttgccggct 540
tggttggcca gcggcgcgcc tgcggccgcc atgaccgcag ccgcaggcat accggcgctt 600
gcgggcggac ccaccgccat caacctgggc atagccaacg tcggcggtgg caacgtcggc 660
aacgccaaca acggccttgc caacatcggc aacgccaacc ttggcaacta caatttcggg 720
tccggaaatt tcggtaactc caatatcggc tcagcaagcc tgggtaataa caacatcggc 780
ttcgggaacc tcggcagcaa caatgtcggc gtgggaaacc ttggcaatct caacaccggg 840
tttgccaaca ccggcttggg caacttcggc tttggcaaca ctggcaacaa caacatcggc 900
atcggtctta ccggcaacaa ccagatcgga atcggcgggc tcaactcggg caccgggaat 960
ttcggattgt tcaactcggg cagcggaaac gtcggcttct tcaactccgg caatggaaac 1020
tttggcatcg gaaactcggg taatttcaac accggtggct ggaattctgg acacgggaac 1080
acgggcttct tcaatgcggg ctcgtttaac accggtatgt tggacgtcgg caacgcgaac 1140
acaggcagcc tgaacaccgg cagttataac atgggcgact tcaatccggg gtcgtccaac 1200
accggcacgt tcaacacggg aaatgctaac acgggtttcc tcaacgccgg aaatatcaac 1260
actggtgtct tcaatattgg ccacatgaat aatgggctgt tcaacacggg tgacatgaac 1320
aatggcgtct tctaccgggg cgtggggcag ggcagcctgc agttcagtat tacgacacct 1380
gatctgactc tgccgccgct gcaaataccg gggatatcgg ttcccgcctt cagtctgccg 1440
gcaataacgc tgccgtcgct gaacatcccg gccgccacca caccggccaa catcaccgtc 1500
```

```
ggcgccttca gcctgcccgg gttgacgttg ccgtcgttga acatcccggc cgccaccaca 1560
ccagccaaca tcaccgtggg tgccttcagc ctgcccgggt tgacgttgcc gtcgttgaac 1620
atcccggccg ccaccacacc agccaacatc accgtcggcg ccttcagcct gcccgggttg 1680
acgttgccgt cgttgaacat cccggccgcc accacaccag ccaacatcac cgtcggcgcc 1740
ttcagcctgc ccgggttgac gttgccgtcg ttgaacatcc cggccgccac cacaccagcc 1800
aacatcaccg tcggcgcctt cagcctgccc gggttgacgt tgccgtcgtt gaacatcccg 1860
gccgccacca cacccgccaa catcaccgta agcggctttc agttgcctcc gctgagtatt 1920
ccttccgtag ccattccgcc ggtgacggtc ccgcccatta cggtgggtgc ttttaatttg 1980
ccgccattgc agattccgga agtaactatt ccgcagctga cgatacccgc gggtatcaca 2040
atcggtggct ttagtctacc tgcgatacat actcaaccga taacggtcgg ccagattggc 2100
gtgggccaat ttggcctgcc ctccataggc tgggatgttt tcctaagcac acctaggata 2160
acagtaccgg cttttggaat accctttacc ctacaattcc agaccaatgt gcctgcgctt 2220
cagccgcccg gcggcgggct tagtactttc accaatggcg ccctcatctt cggtgagttt 2280
gacttaccac aattggtggt tcacccatac acattgaccg gccctattgt catcggttca 2340
ttctttctgc ccgccttcaa catacccggg atcgatgtcc ccgctatcaa cgtcgatggc 2400
ttcaccctgc cgcagatcac caccccagct atcaccaccc cggagttcgc gatccctccg 2460
atcggcgtgg gcggcttcac tctgccgcag atcaccaccc aggaaatcat caccccggag 2520
ctaaccatca actcgatcgg cgtcggcggg ttcaccctgc cgcaaatcac caccccaccc 2580
atcaccaccc caccgctgac catcgacccc atcaacctca ccggcttcac cctcccccaa 2640
atcaccaccc cacccatcac cacccccacg ctgaccatcg accccatcaa cctcaccggc 2700
ttcaccctcc cccaaatcac caccccaccc atcaccaccc caccgctcac catcgagccg 2760
atcggcgtgg ggggcttcac cacgcccccg ctcaccgttc ccggcatcca cctgcccagc 2820
accacgatcg ggggccttcgc gatccccggg gggccgggct acttcaactc gagcaccgcg 2880
ccttcgtcgg gcttcttcaa ttccggtgcg ggcggcaact cgggcttcgg caacaacggc 2940
tcgggcctct cgggttggtt caacaccaac ccggccgggc tgttgggcgg ctcgggctat 3000
cagaacttcg gcgggctatc ctcgggcttt tccaaccttg gcagcggcgt ctcaggcttc 3060
gccaacaggg gcatcctgcc gttctcggta gccagcgtcg tttccggctt tgccaatatc 3120
ggcaccaacc tggcgggttt cttccaaggc accacgtcct aa 3162
```

<212> Type : DNA
<211> Length : 3162
SequenceName : SEQ ID 535
SequenceDescription :
Sequence
--------
<213> OrganismName : Mycobacterium tuberculosis H37Rv
<400> PreSequenceString :

```
atgttgtatg tagttgcgtc acccgacttg atgaccgcgg cggctaccaa tctggcggag 60
attggttcgg cgatcagcac ggcaaatggt gcggcggcac tcccgactgt tgaggtggtg 120
gccgcggccg ccgacgaggt gtccacgcag atcgcggctc tattcggagc gcatgccagg 180
agctaccaaa ccctcagcac ccaggcagcg gcgtttcata gtcggtttgt gcaggcgttg 240
accacggccg cggcttccta cgccagcgta gaggccgcca acgcgtcgcc acttcaggtt 300
gcgctagacg tgattaatgc gcccgcccag acactgctcg gacgtccgct aattggtaac 360
ggcgccgacg gatcgacacc ggggcaggcc ggcgggcccg gcgggttgct gtacggcaac 420
ggcggtaatg gcgccgccgg tgggcccaac caggccggcg gcgccggcgg caacgccggc 480
ttgatcggca acggcggggc gggcggcgcc gggggtgttg gcgcggtcgg cggtaaacgc 540
ggcacgggcg gcctgctatt cggcaacggc ggggccggcg ggcaaggcgg gctcggcctc 600
gcaggtatca acggcggcag cggcgggcag ggaggccacg gtggcaacgc catcctgttc 660
ggccagggcg gtgccggcgg gccaggtggc accggcgcca tgggcgtcgc cggcaccaat 720
cccaccccca tcggcaccgc agcgcctggc agcgacggcg taaatcagat tgggaacggt 780
ggtaacacgg acctcaccgg cggcgccggt ggcgacggca atgccggcag caccaccgtg 840
aacggcggca acggcggtac cggcggcgca gctaggaact catctggtgg taccggtaac 900
tcctttggtg gtgccggcgg cgccggaggc gacggcgcca acggcggcga cggtggcgct 960
ggcggggaag ccctcaccga aggcggtgcc accgccgtta gtggtgctgg tggtaaggga 1020
ggtaacgccg aggcttccgg cggcgccggc ggcaacggcg gcaaaggtgg ctttgctcag 1080
gccaccacca gcgtgaccgg gggtaacggc ggtaacggtg gcaatggcca cgacagtaac 1140
gcgccgggcg gcgctggcgg cagcggtggc gtcggcggtg acggcggccg tggcggcctg 1200
ctggccggca acggcggcac cggcggtgcc ggtggcaacg gcggtaccgg tggcgccggt 1260
gcccccggcg gtgccggcgg cgccggcggc aaagccgaca tcgccaacag cctcggcgac 1320
aatgccaccg taaccggggg caatggcggg acaggcggag acggcggcag cgcgctgggc 1380
accgggggggg ctgggggtgc cggaggtcta ggtggtcacg ggggtgcagg cgggctgctg 1440
attggcaacg gcggcgccgg tggcgctggc ggcctcggcg gtgcgggcgg cgccggcggt 1500
gcgggcggtg agggcggtgc cggcggcgcc ggaggcgaag ctattcccgg cggggcgtcc 1560
accaactccg ccggcggtga cggagggggcg ggcggtactg gcggcaatgg cggtgacggc 1620
ggtgccggcg gagcccccgg cctcggtggc gcgggcgggg ccggcggatg gttgatcggc 1680
cagtcgggca gcaccggcgg cggtggcgcc ggcggtgccg gtggtgccgg aggtgccggt 1740
ggcgcgggcg gcagcggcgg tgcgggtggc catggcgaca ctacctccgg caagaacggt 1800
```

tcgtctggca ccgcgggctt cgacggcaac cccgggcagc ccggctga 1848

<212> Type : DNA
<211> Length : 1848
SequenceName : SEQ ID 536
SequenceDescription :
Sequence --------
<213> OrganismName : Mycobacterium tuberculosis H37Rv
<400> PreSequenceString :

```
atgcattact cagtgttgcc gccggagatc aactcggcct tgatcttcgc cggggcgggc     60
tccggaccga tgctggcggc ggcgtcggcc tgggacgggc tggcaaccga attagcctcg    120
gctgctgtct ctttcggctc ggtgacagcc gggctggtcg gcgggtcgtg gcagggtcgg    180
tcatcggtgg cgatggcagc ggcggcagcc ccgtatgcgg ggtggctggc cgcggcggcg    240
acccaggccg agcaggcggc cacccaggcc caggtgatgg tggccgagtt cgaggctgtg    300
cggctggcga tggtacaacc ggcgctggtg gccgccaacc gttccggcct catatcgctg    360
gtgatatcga accttttttgg tcaaaacgct cccgcgatcg cggccgccga agccgcatac    420
gaggagatgt gggctctgga tgtatcggcg atggcggcct accattccgg ggcgtcggcg    480
gtcgctgtgg cgctaccggc attcgccctc ccgctgcggc ttccggcggg tctggcggcc    540
gggcccgcgg ccgtggtgac cgcgctcacc acggccgtgg gcatgccgac ttttgccggc    600
cgggcgatcg ccgctagcct cggcttggcc aacgtcggtg gtggcaacct cggcaatgcc    660
aacaatgggc tcggcaacat cggcaacgcc aaccttggca acaacaatct ggggtccggc    720
aacttcggta gcttcaatat cggctcggcc aacctaggtg gcaacaacat cggcatagga    780
aacgcgggcg ccaacaactt cggacttgca aacctgggca atttgaacac gggattcgcc    840
aatgcaggca tcggcaactt cggaattgcg aacaccggca acaacaatat cggcaacggc    900
ctgactggaa acaaccaaat cggcattggc ggactcaatt ccggcaacgg taacgtcgga    960
ttattcaacg cgggtagcgc caatatcggt ttcttcaact ccggcaatgg caactttggc   1020
atcgggaact ccggtaactt cagcactggc ctgttcaacc ccggacacgg caacaccgga   1080
ttcctgaatg cgggctcttt caatacgggc atgttcgacg ttgggaacgc gaacaccggc   1140
agcttcaacg tcggccacta caacttcggt gccttcaacc cgggcccgtc gaacacgggt   1200
accttcaaca cgggcggcgc caacaccggc tggttcaaca caggaagcat caacaccggc   1260
gccttcaaca taggcgacat gaataacggc ttgttcaaca cgggcgacat gaacaatggt   1320
gtcttttacc gtggtgtggg ccaaggcagc ctgcagttcg ccatcaccag ccctgatttg   1380
acgcttccgt ctctggaaat acccggaatc tcggttcccg cgttcagcct gcccgcgata   1440
accttgccgt cgttgacgat tccggcggtg acgacgccgg ccaacgttac cgtgggtgcg   1500
tttgatttgc cggggttgac ggtgccgtcg ttgacgattc cagcggcgat gacgccagct   1560
aacatcacgg tgggtgcgtt tgatttgccg gggttgacgg tgccgtcgtt gacgattcca   1620
gctacaacga caccagccaa catcacggta ggtgcgttta acttgcctca gttgagtatt   1680
ccgtcggtga cggttccgcc gatcacgatt ccggctggca cagcgctagg tgcgttcaat   1740
ctgccgacgc tgagtattcc gtcggtgacg gttccgccga tcacgattcc ggctggcacc   1800
actgtcggcg gatttacgct acccacgata cacacccgt taataagtac accccaaata    1860
agtataggcg gctttagcac tcccggcata gccacgcaag caaattctgg tgtcatcaat   1920
cttcccacct ttagccttaa cggcattacg ataactaatt tggtggtgtt cattccgaac   1980
aacatcactg ccttgcaaac caatatgccc ggggtattcc cgcagattgg cggcttcgct   2040
aatacacctc ctgcctttat taatactggg accattaccg tgggtggagg tcaaatcaac   2100
ggcgtcggct tctcgatcgg cgcaatcaac gtcacccct tcaccctccc caacgtcgtc    2160
atccaaccgt ggtccctcgg ggggatctcg gtcgacgggt tcaccctgcc agagatcagc   2220
acccaagaat tcaccactcc ggcgttgacg atcagtccga ttggtgtcgg tgcattgagc   2280
ctgccggata tcactactca acagttcacg accccggagt tgaccatcga cccgatcacg   2340
ctgggtgggt ttacgctgcc gcagctcagc atcccggcga ttaccaccc ggcgttcacg    2400
atcgatccga tagcgctggg tggtttcacg cttcctcaga tcatgacgcc cgagataacg   2460
actccaccgt tcgccatcga cccgatcgga cttagcggtt tcaccctccc ccaggtcaat   2520
atcccggaga tcaccacgcc agagttcacc atccagccgg tgggcttggc ggccttcacc   2580
acacccgcac tcaccatcgc cagcatccac ctgccgagca ccaccatggg cggattcgca   2640
atcccagcgg ggccgggata cttcaactcg agcgcaacgc cctcgttggg ctttttcaac   2700
gccggaatcg gtgggaactc gggcttcggc aacagcggct cgggactgtc gggttggttc   2760
aacacaagtc ctgttgggct gctagccggc tcgggctacc agaactacgg tggtcttatc   2820
tccggcttct ccaaccttgg cagcggcata tcgggcttcg ccaacaccgg caccctgccg   2880
tttgccgtga ccagcttggt ctccggtttg gccaacatcg gcaacaacct gtcgggcctg   2940
ttcttccaga gcaccacgcc ataa                                          2964
```

<212> Type : DNA
<211> Length : 2964
SequenceName : SEQ ID 537
SequenceDescription :
Sequence

--------

<213> OrganismName : Mycobacterium tuberculosis H37Rv

<400> PreSequenceString :

```
atgtcgtttg tagtcgtggc gccggaggtg ttggcggcgg ccgcttcgga tctagcgggc   60
atcgggtcga cactggcgca ggccaacgcc gcggcgttgg cgccgaccac cgcggtgttg  120
gccgcgggtg ctgatgaggt ttccgcggca atcgcgtcgc tgtttggggc gcatggtcag  180
gcgtatcagg cggtgagcgc ccaaatgtcg gcgtttcacg cccagttcat gcaggcgttg  240
acgggtgccg gcggggctta tgcggctgcg gaggcggtca acgtctcggc ggcgcagagc  300
gtggaacaag acctgttggc cgcgatcaac gctcgcttcg agcggatttt tgggcgcccg  360
ctgatcggtg atggcgccaa cggcgggccg ggacaagacg gcgggcccgg cgggttgctg  420
tacggcaacg gtggcaacgg cggcaccagc acgaccgtgg ggatggccgg cggcaacggt  480
ggtgccgccg ggctgatcgg caacggtggg ttcgggggcg gcggcgggcc cggcgcggcc  540
ggcggcaacg gcggcgccgg cgggtggcta ttcggcaacg gcggcgccgg cggtgccggc  600
ggcctcggcg tagcgcccgg cgtgcccggc ggcgccggcg gtgccggcgg cgccggcggt  660
gtcggcggac ccgccgggtt gtggggccac gggggtgccg gcggggcggg tggtgccggc  720
gtggctggcg ccggcggctt cgaggggacg atcggtgccg gcggtgccgg cggtgtcggc  780
ggtgccggcg gtgtcggcgg tgccggcggt gccggcgggt ggctgtacgg cgacgccggt  840
gccggtgggg atggtggtgt cggcggtgcc ggcggcaccg gcgggttagg caaccgtggc  900
ggcgccggtg gcgccggggg cgccggtggt gtcggcggcg ccgggggtgc cgccgggctg  960
tggggcggcg gtggtgccgg cggggtgggt gggaccggcg gcggcgccgg cctcggtgct 1020
cagagcgtca ccttcagtag tagcttaagt ggcctttccg gtggcgacgg cggcgccggc 1080
ggggccggtg gcgccggtgg cgccggtggc accggtgggt ggctgtatgg cggcggtggt 1140
gccgccggat ccggcgggga cggtggtacc ggcggtcagg gcggcgccgg cggcgccggt 1200
gtatttagcc tattcggatc cggtggcggc cccggcggca acggcggcgt cggcggcgtc 1260
ggcggtgtcg gcggtgctgg cgggcgtgcc ggcttgttcg gcgtcggggg cctcggcggc 1320
gcgggtggcg acgccggtga ctccggcgaa ggcggcttcg gcgggccggg gctcgccggc 1380
gggctgttcg gcaaccccgg caacggcggc gtcggcggga tcggcggcga cgccgcagcc 1440
ggcggcgccg gtggggccgg aggcaacggt ggggccggag gcaacggtgg gtggttgttc 1500
ggcaatggtg gtgccggcgg ctccggtggc gacggcggcg ccgccggccg tggcggtgcc 1560
ggcaacttgg gctcggccgg gggtatcaac gcccccgccg gtaaccccgg cagcggctcg 1620
gtcggcatcg gcggtgccgg tggtgccggc ggcaccgccg ggctgttcgg cgacggtggg 1680
gctggtgggg ccggtggtgc cggcgccgcc ggcggcttcg gcggcatcag cgccgccacc 1740
ccctcggcgg gcagtgaggg cgccatgggt ggggccggtg gtgttggcgg caacgccagg 1800
ctgttgggca ctggtggcgc cggtggagtc ggcggcggcg gcggggccgg cggcgacgga 1860
ggccgcggcg gagtcgcaac cccggcggt cagggcggtg acgctgggga cggtggcgcc 1920
ggcggggccg gcggcaatgg cggcggcgcc agcggcgccg gcgggtggct gttggggacc 1980
ggtggtgccg gtggtgccgg tggtaacggc ggcaatggcg gaaaagccgg ttttagccct 2040
gggccgacca acttcggtct caacggcgcc ggtggtggtg gtggtgtcgg cggcaacggc 2100
gccaccggac cctggctgtt cggcgacggc ggccccaccc caggcagcac cggtgccggt 2160
gcggccggtg gtcacggcgg cgacgcccag ctgatcggca acggcggcca cggcggggcc 2220
ggcggcaccg gggtgccgaa cgggtcaggt ggtgccggcg gcctcagcgg gctgctgttc 2280
ggcgagccgg gggcgaacgg gtag                                       2304
```

<212> Type : DNA

<211> Length : 2304

SequenceName : SEQ ID 538

SequenceDescription :

Sequence

--------

<213> OrganismName : Mycobacterium tuberculosis H37Rv

<400> PreSequenceString :

```
atgtcgtttg tgatcgcgaa ccccgagatg ctggcagcgg cggcgaccga tttggccggc      60
atccggtcgg cgatcagcgc cgcgaccgcg gcggccgcgg ccccgacgat ccaggttgcc     120
gcggccggcg ccgacgaggt gtcgctggcc atctcggcgc tgtttggcca gcacgcccag     180
gcctatcagg cgctcagcgc ccaggcgacg atctttcacg accagttcgt gcaggccctg     240
acctccggcg gcaacctgta tgcggccgcc gagagccaca ccgtcgagca gatggtgctc     300
aacgcgatca acgcgcccac ccagacactg ttcggccgcc cgctgatcgg cgacggcgcc     360
aacgggaccg cggagaaccc ggacggccaa aacggcggcc tgctgttcgg caacggcggc     420
aacggcttta cccagacgac cgccggggtg gccggcggca acggcggcag cgcggggttg     480
atcggcaacg gcggggccgg cggcggcggc ggggccggcg ccgccggcgg cctcggcggc     540
aacggcgggt ggctgtacgg caacggcggg gccggcggca tcgggggcgc gggcaccgga     600
accggtggtc acggcggggc cggcggggcc ggcggccggg cctggctgtg gggcaccggc     660
ggggccggcg gagccggcgg tgacggcggc tggttgttcg gcgacggcgg ggccggcggc     720
accggcggca acggcggcag cggctttaac agcttgacct cttcggtcgg cggcgccggc     780
ggggccggtg ggcacgccgg gctgttcggc gccggcggga ccggcgggac cggcggcatc     840
ggcgggcaaa acaccgagac cggcccggcc gccagcaacg gcggcgcggg cggcgccggt     900
ggcggcggcg ggtacctggt cggcgatggc ggcgccggcg ggaccggcgg ggccggcggg     960
aagaattcca gcggtggcgc caccctcacc gggggcaccg gagggaccgg cggggccggc    1020

ggggcggccg ggtggctcta cggcagcggc ggcgccggcg gtgccggcgg cgccggcggg    1080
ctcaacaacg ccggtggtgc caccggcggc accggcggta ccggcggagc cggcggctct    1140
ggagcgtggc tgtacggcaa cggcgggcc gccggggccg gcggcaacgg cggcaacaat     1200
accagcgccg gcaccggtgg tgtcggggct agcggcggga ccggcggaaa cgccgggctg    1260
atcggcgccg gcggccacgg cggggccggc ggcgccggcg gaaaccaaac cggtggcgtg    1320
ggcaacggcg gggccggcgg gaacggcggc gccggcgggg ccggtggtca gctgtacggc    1380
aacggcgggg acggcggcaa cggcggggcc ggcggggcca acatcgccgg cggcaatggc    1440
agcgacggcg gcgccgccgg ccacggcggg gccggcggga gcgcccggct gatcggagcc    1500
ggcggccacg gcggggacgg cggcgccggc gggaacaccg ccggcagaag ggccgacgcg    1560
atcgccggca ccggcgggga cggcggcaac ggcgggaatg gcggcttgct aagcggcaac    1620
gccggggccg gcggccacgg cggggcgggc gggagcagca ccgcgaccac caccaccgga    1680
acacccccaa cgggtgcaac gggcggcaat ggcggcaacg gcggggccgg cggcacggcc    1740
gggtttaccg gcagcggcgg catcggcggc aacggcgggg ccggcggcac cggcggtaac    1800
gccggtgtcg ccttgtcggt tggcagcacg ggcggactgg gcggtaacgg cggcagcggg    1860
ggcctcggcg gcggcggcgg gtcgctcttc ggcaatggcg gggccggcgg tgtcggcgca    1920
accggcggaa acggcggaag cggtatcggg cccgccagcg tgggtggcaa cggcggcaag    1980
ggcggcgttg gtgcggccgg cgggcttgcc gggcagatcg gcaacggcgg tagtggtggg    2040
tccggcggtg ccgggggcaa cggcgggacc ggcgataccg ccggcaacgg tggcaatggt    2100
ggtgccggcg cggtcggcgg caacgcccag ctcatcggca acggcggcaa cggcggtggc    2160
ggcgggaacg gcggaaccgg cgccgacggc acctaa                              2196
```

<212> Type : DNA

<211> Length : 2196

SequenceName : SEQ ID 539

SequenceDescription :

Sequence

--------

<213> OrganismName : Mycobacterium tuberculosis H37Rv

<400> PreSequenceString :

```
atgccggggc ggttcagaaa cttcggtagc caaaacctgg gtagcggcaa catcggcagc      60
accaacgtgg gcagcggcaa catcggcagc accaacgtgg gcagcggcaa catcggcgac     120
acgaacttcg gtaacggaaa caacggcaac ttcaactttg gtagcggcaa taccggcagt     180
aacaacatcg gcttcggaaa caccggcagc gggaatttcg gtttcggaaa cacgggcaac     240
aacaacatcg gtatcgggct caccggcgat ggtcagatcg gcatcggcgg actgaactcg     300
ggcagcggaa acatcggttt cgggaactcc ggcaccggaa acgtcggttt gttcaactcc     360
ggcaccggca acgtaggctt cgggaactcc ggtactgcga acactggatt cgggaacgcg     420
ggcaacgtca acaccggatt ttggaacggc ggcagcacaa acactggcct cgctaacgcc     480
ggcgccggca acacaggctt tttcgacgct ggcaactaca acttcggcag tcttaacgcc     540
ggaaacataa actcgagttt tgggaattcg ggtgacggca acagtggttt cctcaatgct     600
ggcgacgtca actccggtgt gggcaatgcg ggtgatgtca acactggctt agggaactcg     660
ggcaacatca atactggtgg gtttaatccg ggcacgctca acacgggctt cttcagcgcg     720
atgacccaag ctggtccgaa ttcgggcttc ttcaacgccg gtaccggtaa ctctggtttc     780
gggcacaacg acccggctgg cagtggcaac tcgggcattc agaactcggg cttcggcaac     840
tcgggctatg tcaataccag caccacaagc atgttcggcg gtaactcagg ggtgctcaac     900
acgggctacg gcaactcagg tttctataac gcggccgtca acaacaccgg gatttttgtg     960
accggcgtga tgagttcggg atttttcaat tttgggacgg gcaactcggg cctgctggtc    1020
agcggcaatg ggctttcggg tttcttcaag aacttgttcg gatag                    1065
```

<212> Type : DNA
<211> Length : 1065
SequenceName : SEQ ID 540
SequenceDescription :
Sequence
--------
<213> OrganismName : Mycobacterium tuberculosis H37Rv
<400> PreSequenceString :

```
atgtcgttcg tgctggcgat gccggaggtg ttggggtcgg cggcaacgga tctggccgct      60
ctgggctcgg tgctgggcgc ggccgatgcg gccgcggcgg ctacgacgac gggcatcgtg     120
gccgcggccc aggatgaggt gtcggcggcg atcgcggcgt tgttttccgc ccacggccgg     180
gcctatcagg tggccagtgc gcaggcggcg gcggttcacg cccagttcgt ggaggcgttg     240
agcgcgggtg cgggggccta cgccagcgcg gaggccgccg gcgcggcggt gctggccaac     300
ccggcgcaga gcgtgcagca ggacctgctg gccgccgtca atgcgcaaag tgtcgcgctc     360
acggggcgcc cgttgatcgg caacggcgcc aacggggccc cgggcacggg ggccaatggt     420
gcgccgggcg ggtggttgct cggtaatggt ggggccggcg ggtccgccgc cgctggctcg     480
ggcctgcccg gcggggccgg cggggccgcc gggttgttcg gcaccggcgg ggctggtggg     540
gccggcggga gttccacggt aggtgatggc gaggccgggg gtgccggtgg atcaggtggc     600
tggttgttgg gcaccggtgg ggtcggcggg gtcggcgggc tcggggccgg cgccggtggg     660
```

```
gccggcgggg ttggtggggc cggcgggctg ttgggtgctg gcgggcacgg cggcgccggc     720
gggctaggcg ccgtcaccgg tggggtcggg ggaactggcg gagccggtgg gctgctggcc     780
gggctgctgg ccgggccggg cggggccggc gggaccggcg gacgtggctt tctcaacaac     840
ggtggggtcg gtggggctgg cggcaacgcc gggctgctgt tcggtgccgg cggcaccggt     900
ggatccggcg gagccggcct aggtggtgac ggtggggccg gtggggccgg cggcaacacc     960
ggtgtgctgt tcggcaacgc cggatccggg gggaccggcg ggttcggcga taccgacggg    1020
ggagccggcg gtgccggcgg tgacgccggc tggttgggct ccggtggggt cggcggggcc    1080
ggcgggttcg gcgaaaccgg tgacgggggt gtcggcgggg ccggcggcaa ggccgggttg    1140
ctgatcggta acggcggggc cggcggcgcc ggtgggcaag gcgccgtgac cggcggtacc    1200
ggcggggccg gcggcgacgg ggtgctgatc ggcaacggcg gcaacgccgg catcggcgga    1260
accggaccga ccgcgggtga taccggcgcg ggtgggatca gtgggctgct gctgggcgcc    1320
gacggcttca acaccccggc cagcgcctct ccgctgcaca ccctgaaaca acaggcgctg    1380
gccgcgatca acgcgccgac ccagacactg accgggcgac cgctgatcgg caacggcacc    1440
cccggggcgg tcggcagcgg ggccaccggg gcccccggtg ggtggctgct cggcgacggc    1500
ggggccggcg ggtccggcgc ggcgggctcg ggcgcgcccg gcggggcggg cggggctgcc    1560
gggctgtggg gtaccggcgg ggccggcggg gccggaggca gctcggcggg tggcggcggg    1620
gccggtgggg ccggcggggc cggcggctgg ctgctcggcg acggcggggc cggcgggatc    1680
ggcggagcca gcaccgtact cggcggcacc ggcgggggag gcggggtcgg tgggctgtgg    1740
ggcgccggtg gggccggcgg ggccggtgga accggccttg ttggtggcga cggcggggcc    1800
ggtggggccg gcgggaccgg cggactgctg gccgggctga tcggtgccgg cggaggtcac    1860
ggcgggaccg gcgggctcag cactaatggc gacggcgggg ttggcggggc cggcgggaat    1920
gccggaatgc tcgccgggcc gggcggcgcc ggcggagccg gcggtgacgg cgaaaacctg    1980
gacaccggtg gggacggcgg ggccggcggt agcgcagggc tgctgttcgg cagcggcggc    2040
gccggcggcg ccggcggatt tggtttcctc ggtggggacg gcggggccgg tggcaacgcc    2100
gggctgctgt tgtccagcgg cggggccggc gggttcggcg ggttcggcac cgccggtggg    2160
gtcggtgggg ccggcggcaa tgccggctgg ctgggcttcg gcggggccgg tggcgtcggc    2220
ggcagcgccg ggctgatcgg caccggcggc aacggcggca acggcggcac cggcgccaac    2280
gccggcagcc ccggaaccgg cggcgccggc gggttgctgc tgggccaaaa cgggctcaac    2340
gggttgccgt ag                                                        2352
```

<212> Type : DNA
<211> Length : 2352
SequenceName : SEQ ID 541
SequenceDescription :
Sequence
--------
<213> OrganismName : Mycobacterium tuberculosis H37Rv
<400> PreSequenceString :

```
gtgtcgttgg tgatcgcgac gccgcagctg ctggcaactg cggctttgga tttagcgagt 60
attggttcgc aggtgagcgc ggctaatgcg gccgcggcga tgccgacgac ggaagtggtg 120
gctgcggctg ccgatgaagt gtcggcggcg attgcggggt tgttcggggc ccatgctcgg 180
cagtatcagg cgctcagcgt acaggtggca gcgtttcacg agcagtttgt gcaggcgttg 240
actgcggccg cgggtcggta tgccagcact gaggccgctg ttgagcggag tctgctgggt 300
gcggtgaatg cgcccaccga ggcgcttttg gggcgcccgt tgatcggaaa cggcgccgac 360
gggacggcac ccgggcagcc tggcgcggcc ggcgggttgc tgtttggcaa cggtggcaac 420
ggcgcggctg gcgggttcgg tcaaaccggc ggcagcggag gcgcggccgg gttgatcggc 480
aacggcggca acggcggggc cggtggtacc ggcgcggccg gcggtgccgg tgggaacggg 540
gggtggttgt ggggcaacgg cggcaacggc ggtgtcggcg gcaccagcgt ggccgcaggc 600
atcgggggtg cgggcggtaa cggcggcaac gccgggctgt tcggccatgg cggcgccggt 660
ggtaccggcg gcgccggcct cgccggggca aacggggtca atcccacgcc cggccccgcg 720
gccagcaccg gggacagccc ggcagatgtg tccggcatcg gtgatcaaac cggcggcgac 780
ggcggcacgg gcggccatgg cactgccggc acgccgaccg gtggcaccgg cggcgacggt 840
gccaccgcga cggcaggctc gggcaaggcc accggcggtg ccggtggtga cggcggtacc 900
gccgctgccg gtggcggcgg cggcaacggc ggcgacggcg gagtcgcgca gggcgacatt 960
gcgagcgcct ttggcggtga tggtggcaac gggtccgacg gtgtagccgc cggcagtggg 1020
ggtggtagcg gcggcgccgg aggcggcgct ttcgtacaca tcgccactgc cacctctacc 1080
ggtggtagcg gcggtttcgg tggtaacggg gctgccagtg ccgcctccgg cgccgacggt 1140
ggcgcagggg gagctggcgg caatggtggc gccggcgggt tgctattcgg tgatggcggc 1200
aacggtggcg ccggtggcgc gggtggtatc ggtggtgacg gcgccacggg ggggcccggg 1260
ggaagcggcg gcaacgctgg catcgcgagg tttgacagcc cagaccccga ggcagaaccc 1320
gatgtggtcg gcggcaaggg tggtgatggc ggcaagggcg gcagcggcct tggcgtcggc 1380
ggcgccggcg ggaccggcgg cgcgggcggc aacggcggcg ccggcgggtt gttgttcggc 1440
aacggcggca acggcggcaa cgccggggcc ggcggggatg gcggcgccgg cgttgccggt 1500
ggggttggcg gtaacggcgg cggtggtggc accgcgacgt ttcacgaaga cccggtcgct 1560
ggtgtctggg cggtcggtgg cgtaggtggt gatggtggct ccggcggcag ctcgcttggt 1620
gtcggcgggg tgggcggagc cggtggcgtg ggtggcaagg gtggcgccag cggcatgttg 1680
atcggcaacg gcggcaacgg tggcagcggc ggagtcggtg gggccggtgg agtcggcggg 1740

gctggcggtg acggcggcaa cggcggctcc ggtggcaacg ccagtacttt tggcgatgag 1800
aactccatcg gcggggccgg cgggacgggc ggcaacgggg gcaacggcgc aaacggcggt 1860
aacggtggcg ctggcggtat tgccggcggt gcgggtgggt ccggagggtt cctcagcggt 1920
gccgcaggag tcagcggcgc tgacggtatc ggtggcgcgg gcggcgcagg cggtgccggt 1980
ggcgcgggcg gtagcggcgg tgaggcaggc gcggggggcc tcaccaacgg ccccgggtcc 2040
cctggcgttt ccggcaccga aggcatggcc ggcgcgcccg gctag 2085
```

<212> Type : DNA
<211> Length : 2085
SequenceName : SEQ ID 542
SequenceDescription :
Sequence
--------
<213> OrganismName : Mycobacterium tuberculoses H37Rv
<400> PreSequenceString :

```
atgtcgtttg tgattgcggc gccagaggtt atcgcggcag cggcaacgga tttagccagt     60
ctcgagtcga gcatcgccgc ggccaacgcg gccgcggcgg ccaacaccac agcactgctg    120
gccgcgggtg ccgatgaagt ctcgacggcg gttgcggcgc tgttcggcgc ccacggccag    180
gcctatcagg cgctcagcgc ccaagcgcag gcgtttcatg cccagttcgt gcaggcgttg    240
acctccggtg gcggcgcgta cgccgccgcc gaggccgccg ccacctcgcc gctgctcgcc    300
ccgatcaacg agttcttcct ggcgaatacc gggcgcccgc tgatcggcaa cggcaccaac    360
ggcgcccccg gcaccggggc caatggcggg gacggcggct ggttaatcgg caacggcggc    420
gccggaggat ccggcgcggc cggcgtcaac ggcggggccg gcggcaacgg cggcgccggc    480
gggctcatcg gcaacggcgg cgccggcggc gccggcggga gggccagcac ggggaccggc    540
ggcgccggcg gcgccggcgg ggccgccggc atgctgttcg gggccgccgg ggtcggcggt    600
cccggcggat tcgcagccgc tttcggcgcc accggcggcg ccggcggggc cggcggaaac    660
ggcgggctgt tcgccgacgg cggggtcggt ggcgccggcg gggcaaccga cgccggcacc    720
ggcggggccg gcggatccgg cggaaacggc gggctgttcg gcgccggcgg caccggtggg    780
cccggcggat tcggcatctt tggcggcggc gccggcgggg atggcggctc tggcgggctg    840
ttcggcgccg gcggcaccgg cggtagtggc gggacgagca taatcaacgt cggcgggaac    900
ggtggggccg gcggcgacgc cggcatgctc agcctcggtg ccgccggcgg cgccggcggc    960
agcggcggat ccaacccgga cggtggcggc ggtgccggtg ggatcggagg cgacggcggc   1020
acgttgttcg gctccggcgg cgccggcggc gtctgcgggc tgggcttcga cgccggcggg   1080
gcgggcggcg ccggcggaaa ggcgggtctg ctcatcgggg cgggcggtgc cggcggcgcc   1140
ggcggcgggt ccttcgcggg cgccggcgga accggtgggg ccggcggcgc gcccgggctc   1200
gtcggcaacg ccggcaacgg cggcaacggc ggcgccagcg cgaacggcgc cggcgccgcc   1260
ggcggcgccg gcggaagcgg cgtgctgatc ggcaatggcg gtaacggtgg tagtggcggc   1320
actggcgcac ccgccggcac ggccggcgcc ggcggcttag gcgggcagct actaggtcga   1380
gacggattca acgcccccgc cagcacgcca ctgcacaccc tgcagcaaca aatcctcaac   1440
gcgatcaacg agcccaccca agccctcacc gggcgaccgc tgatcggcaa cggcgccaac   1500
ggcactccgg ggaccggggc cgacggtggg gccggcgggt ggttgttcgg caacggcggc   1560
aacggcggcc acggagcgac cggcgccgac ggcggagacg gcggatccgg cggggccggc   1620
ggaatcctgt caggtattgg cggcaccggc gggtccggcg gcatcggaac gacgggccaa   1680
ggcgggaccg gcgggaccgg cggagccgcc ttgctgatcg gctccggcgg caccggcggc   1740
agcggcgggt ttggcctgga caccggcggg gccggcgggc gcggcggtga cgccggcttg   1800
ttcctcggtg ccgcagggac cggcggccag gccgccctct cccaaaactt tatcggtgcc   1860
ggcggcaccg ccggagccgg tggtaccggt gggctgttcg ccaacggcgg ggccggcggg   1920
gccggcgggt ttggcgcgaa tggcgggacc ggggggaacg gattgctctt cggcgccggc   1980
ggcaccggcg gggcgggcac gctcggcgcc gacggcggcg ccggggggca cggcgggctg   2040
ttcggcgccg gcggcactgg cggtgctggc ggcagcagcg gcggcacctt cggcggcaac   2100
ggcggcagcg gcggcaacgc cggcctactc gccctcggcg cctccggcgg cgccggcggc   2160
agcggcggca gcgccttaaa tgtcggcggg accggcgggg tcggcgggaa cggcggcagt   2220
ggcggctcgc tcttcggctt cggcggtgcc ggcggcaccg gtggctccag cggcatcggc   2280
agcagcggcg gcaccggcgg tgacggcggt acggccgggg tgttcggcaa cggcggcgac   2340
ggcggcgccg gcgggtttgg cgctgacacc ggtgggaatt cgagctcggt ccccaacgcc   2400
gtgctgatcg gcaacggcgg caacggcggc aacggcggga aagccggcgg cacacccggc   2460
gccggcggca ccagcgggct gatcatcggc gagaacgggc tcaacggcct gtaa         2514
```

<212> Type : DNA
<211> Length : 2514
SequenceName : SEQ ID 543
SequenceDescription :
Sequence
--------
<213> OrganismName : Mycobacterium tuberculosis H37Rv
<400> PreSequenceString :

```
atgtcgtttg tgatcgcggt gccggagact atcgcggcgg cggcgacgga tctagccgat      60
ctcggctcga cgatcgctgg ggccaacgcg gctgcggcgg ccaacacgac gagcctgctg     120
gccgccggtg ccgatgagat ctcggcggca atcgctgcgt tgttcggcgc gcacggccgg     180
gcctatcagg cggcgagcgc cgaggcggcg gcgtttcatg gtcggttcgt gcaggcgctg     240
accaccgggg ggggcgccta tgcggccgcc gaggccgccg ccgtgacgcc gctgctcaac     300
tcgatcaacg cgcccgtcct ggccgctacc ggccgtccgc tgatcggtaa cggggctaac     360
ggtgctcccg gcaccggggc caacggaggg gatgccggct ggttgatcgg caacggtggc     420
gccggcggat ccggtgcaaa gggcgccaac ggcggggctg gtggccctgg tggggccgcc     480
gggctgttcg gcaacggcgg ggccggcggt gccggcggaa ccgccaccgc caacaacggg     540
atcggcgggg ccggtggcgc tggcgggtcc gccatgctgt ttggggccgg cggcgccggc     600
ggcgccggcg gggctgcgac gtctcttgtc ggtggcatcg gcggtaccgg cggaaccggc     660
ggcaacgccg gtatgctcgc cggcgccgcc ggggccggcg gtgccggcgg gttcagcttc     720
agcactgccg gtggggctgg cggcgccggc ggggccggtg ggctgttcac caccggcggt     780
gtcggcggcg ccggtgggca gggtcacacg ggcggggcgg gcggcgccgg cggggccggc     840
gggttgtttg gtgccggcgg catgggcggg gcgggcggat tcggggatca cggaacgctc     900
ggcaccggcg gggccggcgg ggacggtggg ggcggcggtt tgttcggcgc cggcggggac     960
ggcggggcag gcgggtcagg actgaccacc ggcggcgctg ccggtaacgg tggtaacgcc    1020
gggacgcttt ccctgggtgc cgccggtggc gccggcggca ccggtggggc tggcggcact    1080
gtcttcggtg gtggtaaggg cggcgccggc ggagccggcg gtaacgccgg catgctcttc    1140
ggctccggcg ggggtggcgg caccggcggg ttcggcttcg ccgccggcgg gcagggtggg    1200
gtcggcggca gcgccggcat gctcagcggc tccggcggct ccggcggtgc tggcggctct    1260
gggggccccg cgggcaccgc cgcaggtggg gcgggtgggg cgggtggggc gcccgggttg    1320
atcggcaacg gcggcaacgg cggcaacggc ggcgagagtg gcggcaccgg tggtgtcggc    1380
ggggccggtg gaaatgccgt gctgatcggc aacggcggcg agggcggcat cggcgcgctc    1440
gccggcaagt ccggcttcgg cggcttcggc gggttgctgc tgggcgccga cggatataac    1500
gctcccgaga gcacctcgcc atggcacaac ctgcagcagg acattctcag tttcatcaac    1560
gaacccaccg aggcattgac cggacgcccg ctgatcggta acggcgacag tgggacgccg    1620
ggaaccgggg acgatggcgg tgccggcggc tggttgttcg gcaacggcgg caacggcggg    1680
gccggtgcgg ccggcaccaa cggcagcgcg ggcggcgccg gtggggcagg cgggatcctg    1740
tttggcaccg gtggcgccgg cggggccggc ggcgtcggaa cggcgggtgc cggcggggcc    1800
ggtggcgccg gcggatccgc cttcttgatc ggttccggcg gtaccggtgg tgtcggcggg    1860
gccgccacca ccaccggcgg cgtcggcggg gccggcggga acgccggctt gctcatcggc    1920
gcggctgggc tcggcgggtg tggcggcggc gctttcaccg caggcgttac cactggcggc    1980
gccggcggga ctggcggcgc tgccgggttg ttcgccaacg ggggggccgg cggcgccggc    2040
gggaccggca gcaccgccgg gggcgccggc ggggccggcg gggccggcgg gctgtacgcc    2100
cacgggggaa ccggcggacc aggtgggaac ggcggctcca cgggggccgg agggacaggc    2160
ggtgccggcg ggcccggtgg gctgtacggt gccggcggct ctggcggggc cggcggtcat    2220
gggggcatgg ccggcggggg tggcggtgta ggcggcaatg ctggctcgct caccctcaat    2280
gcgtcgggcg gtgccggcgg cagcggcggc tccagcctgt caggcaaggc cggtgctggc    2340
ggcgccggcg gcagcgcggg attgttctac ggctccggcg gggccggcgg caacgggggc    2400
tacagcctca atggcactgg cggtgatggc ggcaccggtg ggccggcca aatcaccggc     2460
cttcgcagcg gcttcggcgg cgccggcggc gccggcggcg ccagcgatac cggcgccggc    2520
gggaacggcg gcgccggcgg caaggccggg ctgtacggca acggcggtga cggtggcgcc    2580
ggcggggatg gcgccaccag cggcaagggt ggagccggcg gcaacgccgt ggtgatcggc    2640
aacggcggca acggcggcaa tgccggaaaa gccgggggca cggcgggtgc cggcggcgcc    2700
ggtgggctgg tactcggccg ggatgggcag cacggcttga cgtag                    2745
```

<212> Type : DNA
<211> Length : 2745
SequenceName : SEQ ID 544
SequenceDescription :
Sequence
--------
<213> OrganismName : Mycobacterium tuberculosis H37Rv
<400> PreSequenceString :

```
atgtcgttgg tgatcgtaac cccggagacg gtggccgcgg cggcctcgga tgtggcgcgc     60
atcgggtcat cgatcggtgt agccaacagc gcggcggcgg ggtcaaccac cagcgtgctg    120
gccgcgggcg ccgatgaggt gtcggcggcg atcgcgacgc tgtttggcag ccatgctcgg    180
gagtatcagg cgatcagcac gcaggtggca gcgtttcatg accgatttgc gcagacgtta    240
agcgccgcgg tcggctcgta tgtcagcgcg gaggcgacca acgccgcacc gttggcgacg    300
ctggagcaca acgtgctcaa tgccctcaat gcgcccaccc aggcgttgct gggtcgcccg    360
ttgatcggtg atggggcggc tggagcaccc ggcaccgggc aggccggcgg ggccggcggg    420
atcttgtggg gcaacggtgg ggccggcggc tcgggcgcgc ccggccaagt cggcggggcc    480
ggcggggccg ccgggttgtt cggcaccggc ggggccggtg ggccggtgg ggccggcgcc     540
gccggtgggg ccgggggtag cggcggctgg ctgctgggca atggtggagt cggcggggcc    600
ggcgggcaga gcctgctggg cggggcaacc ggcggggccg gcggcaacgc cggactgttc    660
ggggtcggcg gaaccggcgg gcccggcggg cccggcgggc ccggcggggt cggcggtacc    720

ggtggtgccg gtggcctggg cgggaccctc tacggggccg gcgggcacgg aggtgccggc    780
gggcccggcc cgatcggtgg tgtgggtgga cacggcggtg tcgggggtgc ggccgggctg    840
ttgggcgtgg gcgggcacgg tggtgccggc ggacacggcg cggagggtgt ggccggcgca    900
gccggtgagg acttgtcccc gcacggtacg tccggtgggg tcggcggcga cgccggcgat    960
ggcggcaccg gagggcgggg cggctggctg gccggcgccg gtggggccgg cggggccggt   1020
ggggttggcg ggaccggcgg ggccggcggg gccggatttt ctcgtgcctt gattgtcgct   1080
ggggataacg gcggtgatgg tggtaacggc gggatgggcg gggctggcgg ggctggcggc   1140
cccggcgggg ccggcggcct gatcagcctg ctgggcggcc aaggcgccgg cggggccggc   1200
gggaccggcg gcgctggtgg cgtcggcggt gaccgcggag ccggcggccc cggcaaccag   1260
gccttcaacg caggtgccgg cggggccggc ggccatggcg gtgaccccgg cgccggcggg   1320
gccggaggca ccggcggagc cggctccatc accggcgctc agggcgccat cggcgccacc   1380
cccaccagcg gcggcaacgg cggggccggc ggcaacggcg ccaacgccac caccgcgggc   1440
accaacggcg ccaacggcgg acccggcggc catggcgggc tggtcggcaa cggcggcgcc   1500
ggcggcaacg gcgccaacgg cgccgcaggg acgaacgcga gcgattcggg cgcagtcggt   1560
ggcaaaggca atagcggcgg caacggcggc cagggcggcg ccggcggcga cggcggaact   1620
ctcgccggca atggcggggc cggtgggact ggcggccgcg gcgcagacgg cgggctcggt   1680
ggtagtggtg ccgagggtgc caatgccacc accgcgggcg agcgtggcca ggacggtggt   1740
aaaggcggca acggtggggt cggcgggacc ggcggcaacg ccgtcgcacc cggtgccaac   1800
ggcggccatg gcggcaacgg cggcaatcct ggcttcagcg gcgctggcgg gctcgggggg   1860
ctcagcggtg acggcgtgac ccgcgccgcg cagggtgcga cccccgactt tgcagacacc   1920
gggggcaaag gcggcaacgg cggtaacggc gccaacgccg ttgcacccgg cggcaccggc   1980
gccagtggcg gagccggcgg gaacgctggg gctggcggca agggcgggga aaacatcatc   2040
ggcgatggcg gcggcggaaa cggcggggcc ggcggcaagg gcggcgccgg caccctgcta   2100
ggtctcacgg tatttggcga caatggcggc gccggcgtcc tcggcgactc gacggaccca   2160
gatggcagcg gcggtgctgg tggcgcgggc ggcgccggtg gcgccggtgg cgatccaacc   2220
atctga                                                             2226
```

<212> Type : DNA
<211> Length : 2226
SequenceName : SEQ ID 545
SequenceDescription :
Sequence
--------
<213> OrganismName : Mycobacterium tuberculosis H37Rv
<400> PreSequenceString :

```
atgtcgtttg tcacggcagc tccagagatg ctggcgacgg cggcgcagaa tgtcgcgaat      60
atcggcacat cgctgagtgc ggcaaacgcg acggcagcgg cgtccacgac ctcggtgctg     120
gcggccggag ccgacgaggt atcgcaggct atcgcaaggc tgttcagtga ttacgccacg     180
cactatcagt cgctgaacgc tcaagccgcg gcatttcatc acagcttcgt gcaaacgttg     240
aacgccgccg gtggcgccta ttcgagcgcc gaggcggcca acgcttcggc gcaggcgttg     300
gaacagaatc tgttggccgt gatcaatgcg cccgcccagg cgttgttcgg gcgtcccctg     360
atcggcaatg gcgcgaatgg aacagcggcc agccccaacg gcggtgatgg tgggattttg     420
tacggcaacg gcggcaacgg cttctcccaa acgaccgccg gggtggccgg cggcgccggt     480
ggttccgcgg gcctgatcgg caacggcggc aatggtggcg ccggtggggc cggtgctgcc     540
ggcggggccg gcggcgccgg cggatggctg ctcggcaacg gtggcgccgg cggtcccggc     600
ggcccaacgg acgttcctgc cggcacaggt ggagccggcg gggccggcgg cgacgcccca     660
ttgatcggct ggggcggcaa cggcgggccc ggcggtttcg ctgcttttgg aaacggtggg     720
gccggcggca acggcggcgc cagcggttcg ctctttggcg tcggcggcgc cggcggcgtc     780
ggcggatcga gcgaagacgt cggcggcacc ggcggggccg gcggcgctgg ccgcggtcta     840
ttccttggcc tgggcggtga tggcggcgcc ggcggcacca gcaacaacaa cggcggtgac     900
ggtggcgccg gcggcaccgc gggaggtcga ttgttcagcc tgggcggtga cggtggcaac     960
ggtggtgccg gtaccgcaat cggatccaac gccggtgacg gtggcgccgg cggtgacagc    1020
agcgccctga tcggctacgc ccagggcggc tccggcggcc tcggcggctt cggcgaaagt    1080
accggcggcg acggcggcct gggcggcgcc ggcgctgtgc tcatcggcac gggcgtcggc    1140
ggtttcggcg gcctcggtgg cggctccaac ggcaccgggg gcgcgggcgg cgcgggcggc    1200
acgggcgcca cgctgatcgg cctgggcgcc ggcggcggcg gcggcatcgg cgggttcgcc    1260
gtcaacgtgg gcaacggcgt cggcggtctg ggcggccagg gcggccaggg cgccgcgctg    1320
atcggcctgg gcgccggcgg tgccggcggt gccggcggcg ccacagtcgt tggacttggt    1380
ggcaatggcg gtgacggcgg tgacggtggc ggcctgttta gtatcggcgt cggtggggac    1440
ggcggcaacg ccggcaacgg cgccatgcct gccaatggcg gcaacggcgg caacgccggg    1500
gtcattgcca acggctcctt tgccccgtcg ttcgtcggct tcggcggcaa cggcggcaac    1560
ggcgtcaatg gcggcaccgg cggcagcggc gggatccttt ttggcgccaa cggcgcgaac    1620
ggaccgtcgt ag                                                       1632
```

<212> Type : DNA
<211> Length : 1632
SequenceName : SEQ ID 546
SequenceDescription :
Sequence
--------
<213> OrganismName : Mycobacterium tuberculosis H37Rv
<400> PreSequenceString :

```
atgtcgtatg tattggcgac gccggagatg gtggcagcgg cagcaaacaa tttggcgcag 60
atcggctcga cgttgagcgc ggccaatgcc gcggcgctgg ccccgaccac cggagtgctg 120
gccgcgggcg ccgacgaggt gtcggcagcg gtggcgtcgc tgttttccgg gcacgcccag 180
gcctatcaga cactcggcac gcaggcggcc gcgtttcatg aacggtttat ccaggccttg 240
agcacggctg cgggcgcata tggtagcgcc gaggccgcaa atgcctcccc gctgcagcaa 300
gcgttgaatg tgatcaacgc gcccacgcag acgctgctcg ggcgcccccct gatcggcaac 360
ggcaccaatg gtgcgccggg taccgggcag gccggcgggc cgggtggctt gttgtatggc 420
aacggcggca atggcgggtc cggcggggtc ggccaagccg gcggagccgg cggcagcgcc 480
gggttgatcg gcatcggcgg gaccgggggg gccggagggg ccggcgcggt cggcggcgtc 540
ggcggcaacg gggggtggtt atacggcaat ggcggggccg gcgggctcgg ggggaccggg 600
gtggccggcg tcaatggcgg tatgggcgca gcaggaggtg ccggcggcaa cgcctacctg 660
ttcggttctg gcggcgcggg cgggcagggc ggtatggggg cagccggggc agacggcgtc 720
aaccccacac ccaccggcac cgctgatgcc ggcagtaccg gcaccgacca gacgctgggc 780
ggcaacgcca taggcggtaa cggtggcccc ggcgatgccg gcgacgcgat gacgtccggc 840
ggcgctggcg ggtctggcgg gaacgccgtc tcaaccgtca acggcgacgc cgtgggcggt 900
gagggaggca aagggggtga gggcgcctat ggcggcgctg gcggcgccgg cggcagcgcc 960
gcttccatcg gcaatgcagc cataggaggt aacggcggtg ccggcgggaa cgcccaggcc 1020
cccggcggtg tgggggggcgc cggcggcgaa ggcggggatg cccaggtggg caccaattct 1080
cccagcaacg cggaagccgg taacggcggc agcggcggca acggcttcga cagcttcgct 1140
tccggcggta ccggcggagc gggcggaacc gggggcgccg gtggccgcgg cgggctgctg 1200
atcggcgacg gcggcgccgg cggcgcgggc ggagtcggtg gtaccggtgg tagcggtgcc 1260
cccggcggcg gcggcggcgc cgggggcgac ggcggtgccg ccaacaccga cagcgccggc 1320
agttcacgca aggcgttcgg gggcgatggc ggtgtgggcg gtgacggcgc gagcgccctc 1380
ggcaccggtg gcgaaggcgg tatcggcggc cagggcggta acggggggtgc tggcgggctg 1440
ctcatcggca acggcggcgc cggtggtgtc ggcggcacgg ccggtgccgg tggtactggt 1500
ggttccggtg gtgctggagg tgccggaggc gccggtggtg gcggcaccaa cagtggtccg 1560
ggcgcagcgt ttggcggtaa cggcaacacc ggcggcaacg gcggcaacgg cggcgccccc 1620
ggcgccctcg gcggcaaggg cgggtccggc gggctgattg gccgcgcggg cagcgacggt 1680
ggcgttggtg ccggaggagc gggcggcgcg ggtggcgccg gcggcacagg cggtgagggc 1740
ggcacgggcg gcgacgggaa aaccaccgac ggcaatcccg gcatgggcgg cagcccgggc 1800
agcgccggcc aacccggcta a 1821
```

<212> Type : DNA
<211> Length : 1821
SequenceName : SEQ ID 547
SequenceDescription :
Sequence
--------
<213> OrganismName : Mycobacterium tuberculosis H37Rv
<400> PreSequenceString :

```
atgtcgtttt tgtttgcaca gccggaaatg ctgggcgcgg cggcgacgga tctggcaagc 60
atcggctcgg cgatcagcac ggccaatgct gcggccgcgg ccgccacgac gcgtgtgctg 120
gccgccggtg ccgatgaggt gtccgccgcc gtggcggcgc tgtttagcgg ccacgcgcag 180
acctatcagg cgctgagaac tcaggcggcg gcgtttcacc agcagatcgt gcagaccctc 240
acgagcacgg caggcgcgta tgccagcgcc gaggccgcca acgtcgagca gcagctgctg 300
ggtgcgatca acgcgccgac catggcgctg ctggggcgcc cgctgatcgg ccacggcgcc 360
gacggggcgc cggggaccgg gcaggccggc ggggccggcg ggatcctgta cggcaacggc 420
ggcaacggcg ggtccggcgc caccggtcag gccggcggtg cgggcggggc ggccgggctg 480
atcggccacg gcggggccgg cggcctgggt ggcaccggcg cgtccggtgg tgccggcggg 540
gccggcggat ggctgtgggg caacggcggg gccggcggca atggcggggt cggggtggcc 600
ggcgaccccg gtggtgtcgg cggtgccggc ggtgccggcg gcgccgccgg attgtggggc 660
agcggagggt ccggcggcac cggcgggcaa ggggggggtcg gtggcggcaa gtccggcgac 720
ggcggcacgg ggggtatcgg cggcgccggt ggcggtggtg gctggctgca cggcgacggc 780
ggcgccggcg gacacggcgg gcagggcgga accggcgtca gctcaggagg caacggcggg 840
gccggcggca ccggcggcga cggccgcggc ctgtcgggca gcggcggggc cggcgggcgc 900
ggcgggcaaa ctggggttgg aggcaaagtc ggggagaata acttcggggg cgcgggcggt 960
gccggcggta ccggcgggct catcggcaac ggcggtgccg gcggcaacgg cgggcaaggc 1020
gcaattagcg gcgccggcgg ggccggtggc aatgcctggc tgatcggcga cggcggtgcc 1080
ggcggcaacg gcggtgatat ccgtggccag ggcggcggcg ccggtggagc cggcggcgct 1140
ggtgggcaac tcattggcaa cggcggcacc ggggggggcag gcgggaccgt caccagtccg 1200
aacggtcttg gcggtgctgg cggagccggc gggtccgccg gtctgattgg ccacggcggc 1260
accggcgggg ccggcgggca cagcgcccag gggcccgacg gcaatggcgg aattggtggt 1320
```

```
gccggcgggg ccggtggcaa cggcggacaa ctctacggca ccggcggcac cggcggcacc    1380
ggcggcaagg gcggcgacgg cttcggcgtg ttcggcaagg gcggcgccgg cgggaccggc    1440
gggcgaggcg gtgccgccgg cctgatcggc gacgccggga ccggcgggac cggcggaaag    1500
ggcggcaccg ccggcgagga cggtaccggc ggcaacggcg ggaccggcgg aaacggtggg    1560
gccgccgtcc taatcggcaa cggcgggggc ggcggcgccg gcggaaacgg tggggccggc    1620
aacgatggca cccccggcaa cggcgggggc ggcggcgtcg gcgggaccgg gggaaccctg    1680
ttcggccagc ccggccaacc cggtccgccc ggccagcccg gccccgccta a             1731
```

<212> Type : DNA
<211> Length : 1731
SequenceName : SEQ ID 548
SequenceDescription :
Sequence
--------
<213> OrganismName : Mycobacterium tuberculosis H37Rv
<400> PreSequenceString :

```
gtgtggacgt cgcagatgat cgtggcgccg gcgtttgtcg atgcagcggc aaaggaccta      60
gcaactattg gttcggcgat tagccgggcc aacgccgaag cgttggtccc gataacggcg     120
ttactgcctg cgggcgctga cgacgtgtcg gctgcgattg cggcgctgtt cgcaacgcac     180
gggcaggcat accaggagct cagtgcccac gcggtcgcat tccatgagca gttcgtccag     240
ctcatgagcg cgggggcggc ccagtacgcc agcgccgagg cagccaactc gtcaccattg     300
caaatcgtgg gccaaaccgc cctcgatgcc atcaattcac ccgtgcagac gctgaccggg     360
cgtccgctta tcggcaacgg tgccaacggg gtcgcaggaa ccgggcaaaa cggtggcgat     420
ggcggatggc tatacggcaa cggtggcaac ggcgggtccg gcgggacggg ccaaaatggc     480
ggcaacggcg ggtcggctgg gctatggggt agcggcggca atggcggcca gggcgggggcg    540
ggtgccaacg gcgcagccgg tcaacccggg aaagcgggcg ggtccggcgg caacggcggc     600
gccggtggat ggatctatgg tcacggtgga catggtgggg ccggcggaaa cgggggcaac     660
gccacagcgc ccgggggtgc gtcggcaggc ttcgatgggg gcgccggcgg aaacgggggt     720
tcgggtggtc gcggtggact gttgttcggc aacggcggca acggctcggt cggtggcatg     780
ggaggacaag gcactaatga cacagccgga gattcggccg gcagcggcgg attaggaggc     840
aacgggggca acggcgccca gggcgggtgg ctgatcggca acgggggcga aggtggggac     900
agcggcgccg gcggcggcac cgactccact caaacgggcg tcatgaacgg cgcttccggc     960
ggttcggccg ggatagccgg taacggcggt gacgcgggcc tagtcggcaa cggcggggcc    1020
ggtggcaacg gcgggaatgg agcggccggg tctgcgctgg gtactaccat cttcggcggg    1080
agcggcgggg tcggtggctc aggcggcgac ggcggcaacg gcgggtggtt gttcggcagc    1140
ggcgcgtccg gcggcaacgg gggtcagggc ggtgacgcag gcaccaacgg atttgcgggc    1200
tttggcggct ctgctggcgg tggcggctgg gtaggtgccg ttaacttcgg accgattagt    1260
gtccagggtt ttgggttgtt tggtcacggc ggtgacggtg gcaacggtgg tgacgttggg    1320
gccggcagcc tcagcattca atttggtgca tcagggggtg acggcggcca aggtggggtg    1380
ctatacggca atggcggcaa cggtggtaac gccggcagcg gcggaggtac cggcttcgaa    1440
ggcagcgccg gccagggtgg cgccgccatt ctgatcggca acggcggggc cggagggaat    1500
ggggcgaccg gtgggaccgg agtaggcaac attattcagg aagccggagg tgacggcagc    1560
gacggtggcg cgggcggcag cggaggccta ctgttcggta gcggcggggc cggcggcatc    1620
ggcggggccg gcggcgtcgg cgggtcgggc aacgacggcg gcaacggcgg cgatggtggc    1680
caaggcgggg caagcggcct ggggatcggc aacggcggac ccgggggaag tggcggtacc    1740
ggtggggccg gcggaaccgg cggcagtgcg ggcactggcg gtgccggcgg tgacggtggt    1800
aacgccgccc tgctgatcgg taccggtggc gacggcggag atggtgtccc acccgcaccc    1860
gggggccaag gcggcaaggg cggattgatc ggtttgcctg ggcagaacgg gcagccgtag    1920
```

<212> Type : DNA
<211> Length : 1920
SequenceName : SEQ ID 549
SequenceDescription :
Sequence
--------
<213> OrganismName : Mycobacterium tuberculosis H37Rv
<400> PreSequenceString :

```
atgtcatggg tgatggtttc gccggagctg gtggtggcgg cggcagcgga tttggcgggg      60
atcgggtcgg cgattagctc ggctaatgcg gcggcggccg tcaacacgac gggattgttg     120
accgcgggtg ccgatgaggt gtcgacagcg attgcggcgt tgttcggtgc ccaaggccag     180
gcctaccagg cggcgagcgc acaggcggcg gcgttttacg cccagttcgt gcaggccctg     240
agcgccggcg gaggcgcgta tgcggccgcc gaggccgccg ccgtgtcgcc gctgctggcc     300
ccgatcaacg cgcaattcgt ggcggccacc gggcgcccgc tgatcggcaa cggcgccaac     360
ggcgcccccg ggaccggagc caacggcggg cccggcgggt ggttgatcgg caacggcggc     420
gccggcgggt ctggcgcccc cggcgctggg gccggcggta acggcggggc cggcgggctg     480

ttcggcagcg gcggggccgg cggggcctcc accgacgtcg ccggcggggc cggtggggcc     540
ggcggggccg gcggaaacgc cggcatgctg ttcggcgccg ccggggtcgg cggcgtcggc     600
ggattctcga acggcggtgc caccggcggg gcaggcgggg ccggcggggc gggcgggctg     660
tttggcgccg gaagggaacg cggcagcggc gggtcgggca acctcactgg cggggccggc     720
ggggccggcg gcaacgccgg gacactcgcc actggtgatg gcggggccgg cgggaccggc     780
ggcgctagtc gcagcggcgg attcggcggg gccggcggag ccggcggcga cgccggcatg     840
ttcttcggct ccggcggctc cggcggcgcc ggcggcatta gtaaaagcgt cggggacagc     900
gccgccggcg gggccggcgg ggcccccggg ctgatcggca acggcggcaa cggcggcaac     960
ggcggcgcga gcaccggcgg cggggacggt gggcccggcg gggccggcgg caccggcgtg    1020
ttgatcggca acggcggcaa cggcggcagc ggcgggaccg gcgcgaccct gggcaaggcc    1080
ggcatcggcg gtaccggggg ggtgctgttg ggcctggacg gctttacggc ccccgccagc    1140
acctcgcccc tgcacaccct gcagcaggac gtgatcaata tggtgaacga ccccttccag    1200
acgctcaccg ggcgtccgct gatcggcaac ggcgccaacg gcactccggg gaccggggct    1260
gacggcggag ccggcggctg gttgttcggc aacggcggaa acggcgggca gggaacgatc    1320
ggcggcgtca acggcggggc cggcggggcc ggcggggccg gcgggatctt gttcggcacc    1380
ggcggcaccg ggggcagcgg cgggcccggc gccaccggcc tcggcgggat tggcggggcc    1440
ggcggagccg ccttgctctt cggctccggc ggggccggcg gaagcggtgg tgccggcgcg    1500
gtcggtggca atggcggggc cggcggcaac gccggtgcgc tcttgggcgc cgccggggcc    1560
ggcggggccg gtggtgccgg cgcggtcggt ggcaatggcg gggccggcgg taacggcggg    1620
ctgttcgcca acgggggagc cggcgggccc ggtgggtttg gcagccccgc tggggctggc    1680
gggatcggcg gggcaggtgg gaacggcggg ctgttcggcg ccggcgggac cggcggggcc    1740
ggcgggggaa gcaccctcgc cggcggcgcc ggcggggcgg gcggcaacgg cgggctgttc    1800
ggcgccggcg gcaccggcgg cgccggcagc catagcaccg ccgccggagt ttccggaggg    1860
gccggcgggg ccggcggcga cgccggcttg ctctccctcg gcgcctccgg cggggccggc    1920
ggcagcggcg gttccagcct gaccgccgcc ggcgtggtcg gcggcatcgg cggcgccgga    1980
ggcttgctct tcggctccgg cggcgccggc gggagcggcg ggttcagcaa ctctggcaac    2040
ggcggcgccg gcggggccgg cggcgacgcg ggtttgctcg tcggctccgg cggggccggc    2100
ggggccggcg cctccgccac cggcgccgcc accggcgggg acggcggggc cggcggcaag    2160
tccggagcgt tcggtctcgg aggtgacggc ggcgccggcg gcgccaccgg tttgtccggt    2220
gctttccaca tcggcggcaa gggcggcgtc ggcggcagcg ccgtgctgat cggcaacggc    2280
ggcaacggcg gcaacggcgg taacagcggt aacgccggga aatccggggg tgcacccggc    2340
cccagcggcg ccggcggcgc cggcgggctg ctgctcggtg agaacgggct gaacggcttg    2400
atgtag                                                                2406
```

<212> Type : DNA
<211> Length : 2406
SequenceName : SEQ ID 550
SequenceDescription :
Sequence
--------
<213> OrganismName : Mycobacterium tuberculosis H37Rv
<400> PreSequenceString :

```
ggccagagct atcaagcggt cagcgcccag gcggcggcgt ttcatgaccg gttcgtccaa      60
ctgcttaacg ccggtggagg ttcatatgcg agcgccgaga ttgccaacgc gcagcagaac     120
ctgctgaacg cggtgaacgc gcccacccag acgctgctgg ggcgtccgct ggtcggcgac     180
ggcgccgacg gggccagtgg tccggtggga cagcccggcg gggacggcgg catcttgtgg     240
ggcaacggcg gcaacggtgg cgacagcacg agccccgggg ttgccggggg agccggcggg     300
tcagcggggc tgatcggcaa cggcggcagg ggcggcaacg gtgcgcccgg cggtgcagga     360
ggcaatggcg gcctgggcgg attgctgctg ggcaacgggg gtgccggggg agtcggcggt     420
accggtgaca acggtgtggg agacctcggt gctggcggcg gggaggcga tggcggtttg     480
ggtggacggg cggggctgat cggtcacggc ggtgccggcg gaaacggtgg ggacggtggg     540
cacggcggga gcggcaaggc cggcggcagt ggcggcagtg gcggcttcgg ccagttcggt     600
ggcgccggcg ggctgctgta cggcaatgga ggggcggccg gttccggcgg caacggtggc     660
gatgcaggta ccggcgtctc cagcgacggt ttcgccgggt tgggcggcag cggtggccgg     720
ggcggcgacg cggggctgat tggtgtcggc ggcggcggcg gcggcaacgg cggcgaccca     780
gggctcggtg cgcgcctgtt ccaggtaggt agtcgcggcg gcgacggtgg ggtcggcggg     840
tggctgtacg gcgatggcgg cggcggcggc gacggcggta atgggggct gccatttatt     900
ggctccacca acgccggcaa cggcggcagc gcgcggctca tcggcaacgg tggtgccggc     960
ggtagcggcg ggagcggcgc gcctggctcg gtcagcagcg gtggcgtcgg gggcgcgggc    1020
aaccccggcg gcagcggtgg caacggcggc gtgtggtacg gcaacggtgg cgccggcggg    1080
gccgccggcc aagggggcc cggcatgaac accacctcgc ccggcgggcc gggcggtgtc    1140
ggcgggcacg gcggcaccgc catcttgttc ggcgacggtg gcgccggtgg ggctggcgcc    1200
gccggcggac ccggtactcc ggacggggcc gcggggcccg gcggcagcgg cggcaccggc    1260
gggctgctgt tcggagtccc cggcccgtcc ggcccggacg ggtaa                    1305
```

<212> Type : DNA
<211> Length : 1305
SequenceName : SEQ ID 551
SequenceDescription :
Sequence
--------
<213> OrganismName : Mycobacterium tuberculosis H37Rv
<400> PreSequenceString :

```
atggctcatt tttcggtgtt gccgccggag atcaactcgt tgcggatgta cctgggtgcc      60
ggttcggcgc cgatgcttca ggcggcggcg gcctgggacg ggctggccgc ggagttggga     120
accgccgcgt cgtcgttctc ctcggtgacc acggggttaa ccgggcaggc gtggcagggc     180
ccggcgtcgg cggcgatggc cgccgcggcg gcgccgtatg cgggcttttt gaccacagcc     240
tcggctcaag cccagctggc tgccgggcag gctaaggcgg tggccagcgt gttcgaggcc     300
gccaaggccg cgatcgtgcc tccggccgcg gtggcggcca accgtgaggc gttcttggcg     360
ttgattcggt cgaattggct ggggctcaac gcgccgtgga tcgccgccgt tgaaagcctt     420
tacgaggaat actgggccgc tgatgtggcg gcgatgaccg gctatcacgc cggggcctcg     480
caggccgccg cgcagttgcc gttgccggcc ggcctgcaac agttcctcaa caccctgccc     540
aatctgggca tcggcaacca gggcaacgcc aacctcggcg gcggcaacac cggcagcggc     600
aacatcggca acggaaacaa aggcagctcc aacctcggcg gcggcaacat cggcaataac     660
aacatcggca gcggcaaccg aggcagcgac aacttcggcg ccggcaacgt cggcaccgga     720
aacatcggct tcggcaacca gggccccata gacgttaacc tcttggcgac gccgggccag     780
aacaacgtgg gcctgggcaa catcggcaac aacaacatgg gcttcggcaa caccggcgac     840
gccaacaccg gcggcggcaa caccggcaac ggcaacatcg gtggcggcaa caccggcaac     900
aacaacttcg gcttcggcaa caccggcaac aacaacatcg gaatcgggct caccggcaac     960
aatcagatgg gcatcaacct ggccgggctg ctgaactccg gcagcggcaa tatcggcatc    1020
ggcaactccg gcaccaacaa catcggcttg ttcaactccg gcagcggcaa catcggcgtc    1080
ttcaacaccg gagccaatac cctggtgcct ggcgacctca acaacctggg cgtcgggaat    1140
tccggcaacg ccaacatcgg cttcgggaac gcgggcgttc tcaacaccgg cttcgggaac    1200
gcgagcatcc tcaacaccgg cttggggaac gcgggtgaat taaacaccgg cttcggaaac    1260
gcgggcttcg tcaacacggg gtttgacaac tccggcaacg tcaacaccgg caatgggaac    1320
tcgggcaaca tcaacaccgg ctcgtggaat gcgggcaatg tgaacaccgg tttcgggatc    1380
attaccgaca gcggcctgac caactcgggc ttcggcaaca ccggcaccga cgtctcgggc    1440
ttcttcaaca cccccaccgg ccccttagcc gtcgacgtct ccgggttctt caacacggcc    1500
agcgggggca ctgtcatcaa cggccagacc tcgggcattg gcaacatcgg cgtcccgggc    1560
accctctttg gctccgtccg gagcggcttg aacacgggcc tgtttaacat gggcaccgcc    1620
atatcggggt tgttcaacct gcgccagctg ttggggtag                            1659
```

<212> Type : DNA
<211> Length : 1659
SequenceName : SEQ ID 552
SequenceDescription :
Sequence

--------

<213> OrganismName : Mycobacterium tuberculosis H37Rv

<400> PreSequenceString :

```
atgtcgttcc tgattgcttc gccggaggcg ctagcggcga cagccacata tttgacaggt     60
atcggttcgg caatcagcgc ggcgaacgcg gtcgcggccg ccccgacaac agagatcctg    120
gcggcgggga ccgacgaggt gtccaccgcc atctcagcgc tgttcggcgc tcatgcccag    180
gcatatcagg cgctcagcgc ccacgtggcg gcatttcacg accagttcgt gcataccttg    240
accgccggtg ccggctcata catggccgcc gaggccgccg ccgcctcgcc tctgcaggct    300
ttgcagctgg agctgctcaa cgccatcaat gcacccaccc tggcgctgtt gggacgcccg    360
ttgatcggcg acggcaccga tgcggcgccg gggagcgggg gggccggcgg ggccggcggc    420
atcttgatcg gcaacggcgg gacggcggc gccagcgact tagccgggac cggccgcggc    480
ggggtcggcg gggcgggcgg cgccggcggg ctcttcggca tcggcggcgc cggcgggggc    540
tgcgggtccg cggtggcgat cgggggtgac ggcggggctg gtggcgccgg cggcgtgttc    600
agcggcggcg gcgccggcgg ggccggcgac gccatcgggg gtagcggcgg cgcgggcggc    660
accggtgggc tgttgggtgg tggcggcggc gcgggcggcg ccggcggcgc cggcggcaat    720
ggcgggggcg ccagcaacag cgcaagtatc gggggtgacg gtgggtccgg cggcgcgggc    780
ggcatgctct acggtgccgg cggcgtcggc ggcaacggcg gggccgcggt cgctatcggg    840
ggtgacggcg gggccggcgg cagggccgga gcgatcggca acggcggtga cggcggcaac    900
ggcgggactt ccaacacccc cggcggtagc ggcggcgacg gcggcaatgg cgggaacgcc    960
ggactgatcg gcaacggcgg taacggcggc aacgccgaga ttgtcatctc cggcggtagc   1020
gtcgccggca ccggtggcaa cggcgggttg ctgttgggct tcaacggcac gaacgggctg   1080
ccgtag                                                              1086
```

<212> Type : DNA

<211> Length : 1086

SequenceName : SEQ ID 553

SequenceDescription :

Sequence

--------

<213> OrganismName : Rickettsia prowazekii strain Madrid E

<400> PreSequenceString :

```
atgaaaaaat caaaaatttt aagaaaattt ttagcaacag cctcactatg tgggacatta     60
ttcactaact ctaatgcaac aggaacaatt attcctaata atggtagcgt aagcttgaat    120
actgacgcag gtcttgtagg aggagtattt aacaatggcg atattatcca aatagttaat    180
ggagggcgtg aaattaaaat atcggcagat aaagcaaatg ctatcattgg aggaatcaat    240
acattaaaag aactgcctga ttttggtggt gttgaagtaa gtcaaaacgt ctcaataggc    300
cctcttaatg caggagaaga tcttaatact aattttggcc ctcttaaatt tattagtaat    360
aatgttacgt caattattac aggagtcggt actaaaacat ttagtaatat cgattttgct    420
ggtaaaaatg ctactttaca aattaataaa gatttaaata ttacaactaa aatagataat    480
acagtagccg gaaataatgg ttcaataaca tttgaaggta gcggtattat atcaaatcac    540
ataggctaca ctaactctct tttaggaata aatgtaggaa acggagaagc caagatttat    600
gccccagaag caaataatat tacaattaat gctaaaaata taaatcttac tcacaataac    660
tctatactta ctctttgtga cggtaacata actacattaa agggtaatat aaataatact    720
acagaaattg acggtcaagg tatattaaat ctagcttatg atcttggtag tagtagcata    780
ataacaggtg atataggtaa tataggctca ttagatacaa taaatgtttt acttggatct    840
gcaacattta attctacaat attaaaagcg actaatatta atttaaaaca taatacttca    900
acactcaatt tagatgataa cataattgtt attggtaata taaaaggtaa taataacaaa    960
gatatattaa attttaaagt gcatggtact aatttagata acgaaatgat tattcctgct   1020
cctcaaaaaa ctcatggtac attaaatttt aaaggaaatg ctacacttaa tgggaatata   1080
aataatttaa atatacttaa gtttagtgga ggtcacggta aaactttgaa tttacaaggt   1140
aatactaaag tagataatct tgtttttgca gatagtgttt tagattcagg tactataagt   1200
gttaacgggt tgttagatac agactgtgtg acatttaaca atagtaatgt taacggcgga   1260
acattaataa taaatgccaa gaacacaatc agtgcaaaat tattaaatgc tacaaaagca   1320
aaaatacaaa tcaatgctaa tttaacgatg aatcatccaa gtgctgggga tataagtgat   1380
attagaatag cggataatac aatctataca atagatgcaa aaaatgggaa tgtaaatttg   1440
ctaaataaca acgcaaagat catatttgaa ggagctgatt ctatgttagc tttaatcaat   1500
actggtgtta cagctgatag aacctttacc atatataata atttaaatca atctggcaat   1560
gatgaatatg gaatagtaaa aatagaagca attaaaaaag taataactat agcaaatcaa   1620
agtggacctt atactatagg gcaggataat acacatcgtc ttaaggaatt aatagtagag   1680
ggagcaggtg atatcataat agatgatacg atatttacga agttacttag tataaacagt   1740
acaggacaaa taacatttaa tcgtacttta gatttaggtg caggtggtaa tattgcattt   1800
ggaaagcatg gtacattagt agtaaacggt gtcactggtt caattacaac ttctgaaaat   1860
aatcaaggaa tattaacaat caatagcggg aatattactg gtgttattgg cactaatgaa   1920
ctcggcttaa aacttgtcaa tattggtgca gatcctgtta cgtgctcagc aaatgtattt   1980
gcatcggtag ctttaactaa cccaagttct gtgttaattt tagcagatgg tgttacgcta   2040
actggtgaag taacaacaca taataataca aaaggtgtat tatcacttgg aacaggaagt   2100
aatataacag gtcaaatcgg tactaatagc gcagctcttg agaaaataaa tattggggct   2160
ggagctagta atattgacag taatatatat gcaggttcta cagtactcac agatcaaaca   2220
tcggaattaa ctttaaacaa tgatgtagtc gttaacagta atatcataac tactgccggg   2280
aacaatagcg gaaagttaat atttacaggt aatggcggca taacaggaaa cataggagca   2340
aatggtgcag ctttacaaga ggttgtattt aacggtacta ctaatatagg tggtacagcg   2400
aactcacaga actttactgt tgcacattct gcagcaaatg tggtgattac aggacttact   2460
actggtgcat taaaatacaa agatactggt acaattatag ctcatggagg attggtagga   2520
gacatcgatt ttaataataa agctggtaaa ttcattttag gcgatggtgc tatgattgat   2580
ggatcagtgt tatgtaatgg aggggttgct ggtacattgg attttatagg tgacggtaat   2640
gtaactcaaa atataggcgc agataatgca aatagtattt cgactatcaa cattcaaggc   2700
gataatacaa aaaacgtaac tatagcaaat gatatatttg tagataatat tcattttaca   2760
aacggtggga tattacagct tggcggaaat ctcacaacgc ataatattga tttcggagca   2820
aatggtggta ctttagaatt taatggtaat aatacatata acttaaatgc tattattgta   2880
aacggacaaa acggtatatt aaatgctttc acaaacttaa aggctagcga tgatactatt   2940
ggtacggtta aaataattaa tataggacaa ataggaacac cgcaaaactt tactattcaa   3000
gttaataaca aaaatttaac tctagtaagt agcgtaaata gtagcattaa ttttggtgat   3060
gctaattcgc aattaatatt atctgcacca gtagatcaaa ctattaaatt tattaataat   3120
ttaaacgaaa ctggaggtgg tattatcact ttagatagta atggtaataa tttaaccata   3180
agtggtaata atggaataaa gcttggtagt aaaggtaatg agttatctag cttaaatatt   3240
aaaggaaaag ttactgtaac taatgattta gatatacaaa atattcatca attaaatata   3300
aacaatggtg cattatttga tgaccaaagt ctcacatctg ctaaaatcaa aaatataaat   3360
attggtacag tagcaggcgg agctacttat actttagatg ctataaatga taattttgat   3420
ttaaatacta gtggtatggt atttaaacat caagattcaa tattagaact gaaaaatagt   3480
tcaaatacta atgaccacac tataacatta acatctgctt tagatccagg taataatcaa   3540
tttggtataa ttaaactcat aacagatact aataaattaa ctatagacaa taatggcaat   3600
gtggcttata cactcggtac agcaaatcat atgttgaagc aattaacttt tgctagtata   3660
gataatgggg ctatagcttt aaaagtaggg atcaatgttg aaaatgttac cttaaatatt   3720
aaggatatag agttaaatga ggtgaatgca aatgttctgt ttaacaaaaa cacaacatat   3780
```

```
accgcaacag gtaatataaa cggtcatgta gatttccaag gtaatgcagg tgtaataaat    3840
cttaatgatg atatagaaat tgacggtagt gttacaagta ccggtaatgt aaacggtaca    3900
ttaaatttca atggatcagg taaagtgact ggtttaatca ataacatagt aatgctgcaa    3960
gcaggagcag gtgatgtatc actatctgct agcggtaatt attctattac tgaaattcaa    4020
ggtaacggta ataataattt gacatttgct gctaattcac acttaacaac tgatattaat    4080
aagacaggcg gtcaggattt aaatttagta ttcataaatg gtggtagcgt tagcggttct    4140
atcggcgcaa atgcagcagt tggtgatatt attataaacg caggaagtgt aaatttcagt    4200
aatactctta aaagcggtaa tattgttata tcggatggtg ccacgatgca agttaataat    4260
aacgtaactg ctactgatat ctcaggcaaa aatgcaaata acggaacttt aaaactaaat    4320
aatcatacac ctattaatat cactagtaca cttggtaata ataatgctat agggaccata    4380
gaagtcgcaa ataatgatgt taccattaca gggacattac aagcgcaaaa tattcatttt    4440
tcaaatgcca ctcaggcagc taccttaact ttaggcgcag catcgcaagt gactaatatt    4500
actacggcag gaaataatat tcatacttta gaagtaacag attttgatac tggtaatgac    4560
ggcataatag gagatgcaaa taatagatta aaatcaatag aattgacagg caatggcaca    4620
gtgactatta attctccaca tgtttattca tctattacta ctgcaaataa cgcgcaaggt    4680
aacgtaaaac tcaatataga aggcggtatt acttatgatt taggaagtaa aataaaaagt    4740
ttagcaaatg tacaaattag tgaggatact actattagag gtgatgtgta ttctaaatat    4800
cttaatatag acgcaggtaa aactataaat tttgatagag gtgataataa catgaatccc    4860
aaaaatttag atataccaga tgctctaata gatttagatg tattaccacg ctcgctctca    4920
ctatttaatt acttcactga tattaaagcg gataatttaa attttgcaga tgatactgct    4980
acagcaaatt ttaaagatgc tgttgtaata gatgcacata ttgataatgg tggcatttta    5040
aaatttaatg acaatgcttg gttaacacaa gaaattaaaa atgctaacat catagaaatt    5100
gcatctgata aatttatgtt gcttcagaaa aacattaaag cagctacttt aatagctgat    5160
aatgcaaatt tagtattatt agataatgtg gaagtaaata ctaatctaaa tgttagagat    5220
attgtattag atttagctaa ttatgaatta aaatatactg gtaatgttac acataatgga    5280
ttactgacta ttatcactta ttttgatact gcattacaaa aaggtgggca tatattagtt    5340
agtcaaggat ctaatgtcga tatgtccgat ttagataatt taataattaa aattaaagca    5400
cactccgata tcactaacat tacctcagat actaaacacc aaatagtaaa actcgaaaca    5460
ggtgcaatat atactccagt accgcaaact aaagtcatta ttgatgcaag cgaggaacaa    5520
aataaatttg tgaaatgggt cgctgatgca aacggtttgg tattacttac tgatactgga    5580
ggtcgagacg atactggagg tcgagacgat actaggggtc gaggcaatac tgacaatggc    5640
tgtcgtgata attgtgatgt agggaatatc agcaataaca gtagtaatga agcaggtggg    5700
tcaagtagcg ataaaaatta tggcatcact gatgttgtac caatttttga tccatctcct    5760
atcttagatt atactaaaaa taattatgta gcttctggta tagcaaacca acttattaat    5820
catgttaaag attttggtaa tactactgat gcaggtaaat tattaaatga tttaggtttt    5880
atgtctccga atagagttac tgaaacatta gatagactta gtaatagaat aaatgttaat    5940
ggacttaatg aaggagtagt aggactgaac ggtatcgagg ttgagaattt tttaacagat    6000
atagcaataa atatggataa tttcactgct aaagagattg gcaataggtt agaagaatta    6060
agcgatgcaa atactgtaaa tggtctcaac aaaacaaaca cattgcttaa taataagatt    6120
aatctaaaaa gactgaatac taataatcag gcaataattg ctgcaggtga tgaagataat    6180
atagtaacgg gcatttgggg catgtcattt tatggtaaaa taaagcaaaa ctctaaaaac    6240
agtgcaagcg gttatcaatc taatacaggt ggtggtataa taggctttga ttataatatt    6300
gataattcta tagttatagg ggcggcttat actatggctg atagtaaagt caagcacaaa    6360
aatgataaaa atggtgatag aaccaaagct aaaagtaata tatattctat ctatgggctt    6420
tataattggc ttactaataa ctttttgtt gaagctatag gtgtgtatgg tagaaataaa    6480
atcaaaaatt atgaaaaacg tataactact attactgatc aaatcgcaat aggtaaattt    6540
attaatactt tttatagcta tgaattacta ggtggctata actatctaat atcgcatcgt    6600
accactataa cgccaatgtt tggtatgcgt tatgctacat ttaaaaataa tggttacaaa    6660
gaaaataata ctactttcca aaatttatct ataaagaaaa attactatga taaatttgaa    6720
actatattag gtttaaatag tgtaactcat tatttatcac aagatataat aataaagccc    6780
gaattacatt ggtttataaa ttatcaatgt aaaaataagt taccaaatat tgatgcacgc    6840
cttgacggta tagatgaacc attgacaaca attagattta aacctgcaaa gataacatat    6900
aatttaggcg gtggtatttc tactaaaaat aatatgatag aatttggtat tagatataac    6960
ttatctcttg cgaagaaata tacagcacat caaggatcct taaagattaa agtgaacctg    7020
taa                                                                  7023
```

<212> Type : DNA
<211> Length : 7023
SequenceName : SEQ ID 554
SequenceDescription :
Sequence
--------
<213> OrganismName : Rickettsia prowazekii strain Madrid E
<400> PreSequenceString :

```
atggctcaaa aaccaaattt tctaaaaaaa ataatttccg caggattggt aactgcttcc      60
acggctacta tagtagctgg tttctctggt gtagcaatgg gtgctgctat gcaatataat     120
aggacaacaa atgcagcagc tacaaccttt gatggtatag gctttgatca agctgctggt     180
```

```
gctaatattc ctgtcgctcc aaattcagtt attactgcta atgctaataa tcctattact     240
tttaatactc caaacggtca tttaaatagt ttatttttgg atactgcaaa tgatttagca     300
gtaacaatta atgaggatac taccttagga tttataacaa atattgctca gcaggctaag     360
ttctttaatt ttactgttgc tgctggtaaa attcttaaca taacagggca gggtattact     420
gttcaagaag cttctaatac aataaatgct caaaatgctc ttacaaaagt gcatggtggc     480
gctgctatta acgctaatga tcttagcggg ctaggatcaa taacctttgc tgctgcgcct     540
tctgtattag aatttaattt aataaatcct acaactcaag aagctcctct tacacttggt     600
gctaattcta aaatagttaa tggtggtaat gggacattaa atattactaa tggatttatt     660
caggtttcag ataacacttt tgctggtatt aagaccatta atatcgatga ttgtcaaggt     720
ttaatgttta attctactcc tgatgccgct aatactttaa atttacaagt aggtggtaat     780
actattaatt ttaatggaat agacggtact ggtaaattag tattagtcag taagaatggt     840
gctgctaccg aatttaatgt tacaggaact ttaggtggta atctaaaagg tattattgaa     900
ttgaacactg cagcagtagc tggtaaactt atctctcaag gaggtgctgc taatgcagta     960
ataggtacag ataatggagc aggtagagct gcaggattta ttgttagtgt tgataatggt    1020
aatgcagcaa caatttctgg acaagtttat gctaaaaaca tggtgataca aagtgctaat    1080
gcaggtggac aagtcacttt tgaacacata gttgatgttg gtttaggcgg taccaccaac    1140
tttaaaactg cagattctaa agttataata acagaaaact caaactttgg ttctactaat    1200
tttggtaatc ttgacacaca gattgtagtc cctgatacta agattcttaa aggtaacttc    1260
ataggtgatg taaaaaataa cggtaatact gcaggtgtga ttacttttaa tgctaatggt    1320
gctttagtaa gtgctagtac tgatccaaat attgcagtaa caaatattaa tgcaattgaa    1380
gcagaagggg ccggggttgt agaattatca ggaatacata ttgcagaatt acgtttaggg    1440
aatggtggct ctatctttaa acttgctgat ggcacagtaa ttaatggtcc agttaaccaa    1500
aatgctctta tgaataataa tgctcttgca gctggttcta ttcagttaga tgggagtgct    1560
ataattaccg gtgatatagg taacggtggt gttaatgctg cgttacaaca cattacttta    1620
gctaacgatg cttcaaaaat attagcactc gatggcgcaa atattatcgg ggctaatgtt    1680
ggtggtgcaa ttcattttca agctaacggt ggtactatta aattaacaaa tactcaaaat    1740
aatattgtag ttaattttga tttagatata actactgata aaacaggtgt tgttgatgca    1800
agtagtttaa caaataatca aactttaact attaatggta gtatcggtac tgttgtagct    1860
aatactaaaa cacttgcaca attaaacatc gggtcaagta aaacaatatt aaatgctggc    1920
gatgtcgcta ttaacgagtt agttatagaa aataatggtt cagtacaact taatcacaat    1980
acttacttaa taacaaaaac tatcaatgct gcaaaccaag gtcaaataat cgttgccgct    2040
gatcctctta atactaatac tactcttgct gatggtacaa atttaggtag tgcagaaaat    2100
ccactttcta ctattcattt tgccactaaa gctgctaatg ctgactctat attaaatgta    2160
ggtaaaggag taaatttata tgctaataat attactacta acgatgctaa tgtaggttct    2220
ttacacttta ggtctggtgg tacaagtata gtaagtggta cagttggtgg acagcaaggt    2280
cataagctta ataatttaat attagataat ggtactactg ttaagttttt aggtgataca    2340
acatttaatg gtggtactaa aattgaaggt aaatccatct tgcaaattag caataattat    2400
actactgatc atgttgaatc tgctgataat actggtacat tagaatttgt taacactgat    2460
cctataaccg taacattaaa taaacaaggt gcttattttg gtgttttaaa acaagtaatt    2520
atttctggtc caggtaacat agtatttaat gagataggta atgtaggaat tgtacatggt    2580
atagcagcta attcaatttc ttttgaaaat gcaagtttag gtacatcttt attcttacct    2640
agtggtactc cattagatgt tttaacaatt aaaagtaccg taggtaatgg tacagtagat    2700
aattttaatg ctcctattgt agttgtatca ggtattgata gtatgatcaa taacggtcaa    2760
atcatcggtg ataaaaagaa tattatagct ctatcgcttg gaagtgataa cagtattact    2820
gttaatgcta atacattata ttcaggtatc agaactacaa aaaataatca aggtactgtg    2880
acacttagtg gtggtatgcc taataatcct ggtacaattt atggtttagg tttagagaat    2940
ggtagtccaa agttaaaaca agtgacattt actacagatt ataacaactt aggtagtatt    3000
attgcaaata atgtaacaat taatgattat gtaactctta ctacaggagg tatagcaggg    3060
acagattttg acgctaaaat tactcttgga agtgttaacg gtaacgctaa cgtaaggttt    3120
gttgatagta cattttctga tcctagaagt atgattgttg ctactcaagc taataagggt    3180
actgtaactt atttaggtaa tgcattagtt agtaatatcg gtagtttaga tactcctgta    3240
gcttctgtta gatttacagg taatgatagt ggggcaggat tacaaggcaa tatttattca    3300
caaaatatag atttggtac ttataattta actattctaa attctaatgt cattttaggt    3360
ggtggtacta ctgctattaa tggtgaaatc gatcttctga caaataattt aatatttgca    3420
aatggtactt caacatgggg tgataatact tctattagta caacgttaaa tgtatcaagc    3480
ggtaatatag gtcaagtagt cattgccgaa gatgctcaag ttaacgcaac aactacagga    3540
actacaacca ttaaaataca agataatgct aatgcaaatt tcagtggcac acaagcttat    3600
actttaattc aaggtggtgc tagatttaat ggtactttag gagctcctaa ctttgctgta    3660
acaggaagta atattttcgt aaaatatgaa ctaatacgtg attctaacca ggattatgta    3720
ttaacacgta ctaacgatgt attaaacgta gttacaacag ctgttggaaa tagtgcaatt    3780
gcaaatgcac ctggtgtaag tcagaacatt tctagatgct tagaatcaac aaatacagca    3840
gcttataata atatgctttt agctaaagat ccttctgatg ttgcaacatt tgtaggagct    3900
attgctacag atacaagtgc ggctgtaact acagtaaact taaatgatac acaaaaaact    3960
caagatctac ttagtaatag gctaggtaca cttagatatc taagtaatgc tgaaacttct    4020
gatgttgctg gatctgcaac aggtgcagtg tcttcaggtg atgaagcgga agtatcttat    4080
ggtgtatggg ctaaaccttt ctataacatt gcagaacaag acaaaaaagg tggtatagct    4140
ggttataaag caaaaactac tggggttgta gttggtttag atactctcgc tagcgataac    4200
```

```
ctaatgattg gggcagctat tgggatcact aaaactgata taaaacacca agattataag    4260
aaaggtgata aaactgatat taatggttta tcattctctc tatatggttc ccaacagctt    4320
gttaagaatt tctttgctca aggtaatgca atctttacct taaacaaagt caaaagtaaa    4380
agtcagcgtt acttcttcga gtctaatggt aagatgagca agcaaattgc tgctggtaat    4440
tacgataaca tgacatttgg tggtaattta atatttggtt atgattataa tgcaatgcca    4500
aatgtattag taactccaat ggcaggactt agctacttaa aatcttctaa tgaaaattat    4560
aaagaaaccg gtacaacagt tgcaaataag cgcattaata gcaaatttag tgatagagtc    4620
gatttaatag taggggctaa agtagctggt agtactgtga atataactga tattgtgata    4680
tatccggaaa ttcattcttt tgtggtgcac aaagtaaatg gtaaattatc taactctcag    4740
tctatgttag atggacaaac tgctccattt atcagtcaac ctgatagaac tgctaaaacg    4800
tcttataata taggcttaag tgcaaacata aaatctgatg ctaagatgga gtatggtatc    4860
ggttatgatt ttaattctgc aagtaaatat actgcacatc aaggtacttt aaaagtacgt    4920
gtaaacttct aa                                                        4932
```

<212> Type : DNA
<211> Length : 4932
SequenceName : SEQ ID 555
SequenceDescription :
Sequence
--------
<213> OrganismName : Porphyromonas gingivalis W83
<400> PreSequenceString :

```
atggcacgaa ttatcttgga ggctcacgat gtatgggaag acggcacagg ctatcaaatg      60
ctctgggatg cagatcacaa tcagtacggc gcatccattc ccgaagaatc tttttggttt     120
gccaacggaa cgatcccggc cggtctttac gatcctttcg agtataaagt tccggtcaat     180
gccgatgcat ctttttctcc cacgaatttc gtgcttgatg gaacagcatc agccgatatt     240
cctgccggca cttatgacta tgtaatcatt aaccccaatc ctggcataat atatatagta     300
ggagagggtg tctccaaagg taacgattat gtggtagagg ccggtaagac ttatcatttc     360
actgtccaac gacaaggccc cggcgatgct gcgtccgttg tagtgaccgg agaaggtggc     420
aatgaattcg ctcccgtaca gaatctccaa tggtctgtat ccgggcagac agtgaccctc     480
acttggcaag cccccgcatc cgacaaacgg acttatgtgt tgaacgaaag cttcgatacg     540
caaacgcttc ctaacggctg gacaatgatc gatgctgatg gtgatggtca caattggcta     600
tctacaataa acgtttacaa cactgctact catacaggtg acggtgctat gtttagcaaa     660
tcatggacag ctagcagtgg tgcaaaaatt gatttgagtc ctgacaacta tttggtaact     720
cctaagttta cggttcctga gaatggtaaa ctttcttatt gggtttcatc tcaagagcct     780
tggactaatg agcattatgg agtgttcttg tccacaaccg gaaacgaggc tgcaaacttt     840
acgataaagc tgctggaaga aaccctcgga tccggcaaac ctgctccgat gaacttggtg     900
aagagtgaag gagtaaaggc tccggcacct tatcaggaaa gaaccatcga tctctctgcc     960
tatgccggac aacaggtgta cttggcattc cgtcatttcg gctgtacagg tatattccgt    1020
ctttatcttg atgacgtggc tgtttctggt gaaggttctt ccaacgacta cacgtacacg    1080
gtatatcgtg acaatgttgt tatcgcccag aatctcacgg caacgacatt caatcaggaa    1140
aatgtagctc ccggccagta caactactgt gttgaagtta agtacacagc cggcgtatct    1200
ccgaaggtat gtaaagacgt tacggtagaa ggatccaatg aatttgctcc tgtacagaac    1260
ctgaccggta gtgcagtcgg ccagaaagta acgctcaagt gggatgcacc taatggtacc    1320
ccgaatccga atccgggaac aacaacactt tccgaatcat tcgaaaatgg tattcctgcc    1380
tcatggaaga cgatcgatgc agacggtgac ggcaacaatt ggacgacgac ccctcctccc    1440
ggaggctcct cttttgcagg tcacaacagt gcaatctgtg tctcttcggc ttcttatatc    1500
aactttgaag gccctcagaa ccctgataac tatctggtta caccggagct ttctcttcct    1560
aacggaggaa cgcttacttt ctgggtatgt gcacaagatg ccaattatgc atcagagcac    1620
tatgccgtgt atgcatcttc tacgggtaac gacgcttcca acttcgccaa cgctttgttg    1680
gaagaagtgc tgacggccaa gacagttgtt acggcaccgg aagccattcg tggcactcgt    1740
gttcagggca cctggtatca aaagacggta cagttgcctg cgggtactaa gtatgttgcc    1800
ttccgtcact tcggctgtac ggacttcttc tggatcaacc tcgatgatgt tgagatcaag    1860
gccaacggca agcgcgcaga cttcacggaa acgttcgagt cttctactca tggagaggca    1920
ccagcggaat ggactactat cgatgccgat ggcgatggtc agggttggct ctgtctgtct    1980
tccggacaat tgggatggct gacagctcat ggcggcacca acgtagtagc ctctttctca    2040
tggaatggaa tggctttgaa tcctgataac tatctcatct caaaggatgt tacaggcgca    2100
acgaaggtaa agtactacta tgcagtcaac gacggttttc ccggggatca ctatgcggtg    2160
atgatctcca agacgggcac gaacgccgga gacttcacgg tcgttttcga agaaacgcct    2220
aacggaataa ataagggcgg agcaagattc ggtctttcca cggaagccaa tggcgccaaa    2280
cctcaaagtg tatggatcga gcgtacggta gatttgcctg cgggcacgaa gtatgttgct    2340
ttccgtcact acaattgctc ggatttgaac tacattcttt tggatgatat tcagttcacc    2400
atgggtggca gccccacccc gaccgattat acctacacgg tgtatcgtga tggtacgaag    2460
atcaaggaag gtttgaccga aacgaccttc gaagaagacg gcgtagctac gggcaatcat    2520
gagtattgcg tggaagtgaa gtacacagcc ggcgtatctc cgaaagagtg tgtaaacgta    2580
actgttgatc ctgtgcagtt taatcctgta cagaacctga ccggtagtgc agtaggtcag    2640
aaagtaacgc ttaagtggga tgcacctaat ggtaccccga atccgaatcc cggaacaact    2700
```

```
acactttccg aatcattcga aaatggtatt cctgcctcat ggaagacgat cgatgcagac    2760
ggtgacggca acaattggac gacgacccct cctcccggag gcacctcttt tgcaggtcac    2820
aacagtgcga tctgtgtctc ttcggcttct tatatcaact ttgaaggccc tcagaaccct    2880
gataactatc tggttacacc ggagctatct cttcctaacg gaggaacgct tactttctgg    2940
gtatgtgcac aagatgccaa ttatgcatca gagcactatg ccgtgtatgc atcttctacg    3000
ggtaacgacg cttccaactt cgccaacgct ttgttggaag aagtgctgac ggccaagaca    3060
gttgttacgg cacctgaagc cattcgtggc actcgtgttc agggcacctg gtatcaaaag    3120
acggtacagt tgcctgcggg tactaagtat gttgccttcc gtcacttcgg ctgtacggac    3180
ttcttctgga tcaacctcga tgatgttgag atcaaggcca acggcaagcg cgcagacttc    3240
acggaaacgt tcgagtcttc tactcatgga gaggcaccag cggaatggac tactatcgat    3300
gccgatggcg atggtcaggg ttggctctgt ctgtcttccg gacaattgga ctggctgaca    3360
gctcatggcg gcaccaacgt agtagcctct ttctcatgga atggaatggc tttgaatcct    3420
gataactatc tcatctcaaa ggatgttaca ggcgcaacga aggtaaagta ctactatgca    3480
gtcaacgacg gtttcccgg ggatcactat gcggtgatga tctccaagac gggcacgaac    3540
gccggagact tcacggttgt tttcgaagaa acgcctaacg gaataaataa gggcggagca    3600
agattcggtc tttccacgga agccaatggc gccaaacctc aaagtgtatg gatcgagcgt    3660
acggtagatt tgcctgcggg cacgaagtat gttgctttcc gtcactacaa ttgctcggat    3720
ttgaactaca ttcttttgga tgatattcag ttcaccatgg gtggcagccc caccccgacc    3780
gattatacct acacggtgta tcgtgatggt acgaagatca aggaaggttt gaccgaaacg    3840
accttcgaag aagacggcgt agctacgggc aatcatgagt attgcgtgga agtgaagtac    3900
acagccggcg tatctccgaa agagtgcgta aacgtaactg ttgatcctgt gcagttcaat    3960
cctgtacaga acctgaccgg tagtgcagtc ggccagaaag taacgctcaa gtgggatgca    4020
cctaatggta ccccgaatcc gaatccggga acaacaacac tttccgaatc attcgaaaat    4080
ggtattcctg cctcatggaa gacgatcgat gcagacggtg acggcaacaa ttggacgacg    4140
acccctcctc ccggaggcac ctcttttgca ggtcacaaca gtgcgatctg tgtctcttcg    4200
gcttcttata tcaactttga aggccctcag aaccctgata actatctggt tacaccggag    4260
ctttctcttc ctaacggagg aacgcttact ttctgggtat gtgcacaaga tgccaattat    4320
gcatcagagc actatgccgt gtatgcatct tctacgggta acgacgcttc caacttcgcc    4380
aacgctttgt tggaagaagt gctgacggcc aagacagttg ttacggcacc ggaagccatt    4440
cgtggtactc gtgttcaggg cacctggtat caaaagacgg tacagttgcc tgcgggtact    4500
aagtatgttg ccttccgtca cttcggctgt acggacttct tctggatcaa cctcgatgat    4560
gttgagatca aggccaacgg caagcgcgca gacttcacgg aaacgttcga gtcttctact    4620
catggagagg caccagcgga atggactact atcgatgccg atggcgatgg tcagggttgg    4680
ctctgtctgt cttccggaca attgggatgg ctgacagctc atggcggcac caacgtagta    4740
gcctctttct catggaatgg aatggctttg aatcctgata actatctcat ctcaaaggat    4800
gttacaggcg caacgaaggt aaagtactac tatgcagtca acgacggttt tcccggggat    4860
cactatgcgg tgatgatctc caagacgggc acgaacgccg gagacttcac ggtcgttttc    4920
gaagaaacgc ctaacggaat aaataagggc ggagcaagat tcggtctttc cacggaagcc    4980
aatggcgcca aacctcaaag tgtatggatc gagcgtacgg tagatttgcc tgcgggcacg    5040
aagtatgttg ctttccgtca ctacaattgc tcggatttga actacattct tttggatgat    5100
attcagttca ccatgggtgg cagccccacc ccgaccgatt atacctacac ggtgtatcgt    5160
gatggtacga agatcaagga aggtttgacc gaaacgacct tcgaagaaga cggcgtagct    5220
acgggcaatc atgagtattg cgtggaagtg aagtacacag ccggcgtatc tccgaaagag    5280
tgcgtaaacg taactattaa tccgacacag ttcaatcctg tacagaacct gacggcagaa    5340
caagctccta acagcatgga tgcaatcctt aaatggaatg caccggcatc taagcgtgcg    5400
gaagttctga acgaagactt cgaaaatggt attcctgcct catggaagac gatcgatgca    5460
gacggtgacg gcaacaattg gacgacgacc cctcctcccg gaggctcctc ttttgcaggt    5520
cacaacagtg cgatctgtgt ctcttcggct tcttatatca actttgaagg tcctcagaac    5580
cctgataact atctggttac accggagctt tctcttcctg gcggaggaac gcttactttc    5640
tgggtatgtg cacaagatgc caattatgca tcagagcact atgccgtgta tgcatcttct    5700
acgggtaacg acgcttccaa cttcgccaac gctttgttgg aagaagtgct gacggccaag    5760
acagttgtta cggcaccgga agccattcgt ggtactcgtg ttcagggcac ctggtatcaa    5820
aagacggtac agttgcctgc gggtactaag tatgttgcct tccgtcactt cggctgtacg    5880
gacttcttct ggatcaacct tgatgatgtt gtaatcactt cagggaacgc tccgtcttac    5940
acctatacga tctatcgtaa taatacacag atagcatcag gcgtaacgga gactacttac    6000
cgagatccgg acttggctac cggtttttac acgtacggtg ttaaggttgt ttacccgaac    6060
ggagaatcag ctatcgaaac tgctacgttg aatatcactt cgttggcaga cgtaacggct    6120
cagaagcctt acacgctgac agttgtagga aagacgatca cggtaacttg ccaaggcgaa    6180
gctatgatct acgacatgaa cggtcgtcgt ctggcagcgg gtcgcaacac ggttgtttac    6240
acggctcagg gcggccacta tgcagtcatg gttgtcgttg acggcaagtc ctacgtagag    6300
aaactcgctg taaagtaa                                                   6318
```

<212> Type : DNA
<211> Length : 6318
SequenceName : SEQ ID 556
SequenceDescription :
Sequence
--------
<213> OrganismName : Porphyromonas gingivalis W83

<400> PreSequenceString :

atgaaaacat ctgaaagaat attaagttat ttcttcctct tatgtgctgt attcagtctg 60
ggctcatgcg aaggacttta tgcacaggta actttcccaa attattcgcc tacggcggct 120
tcgtccattg ctgtatgttc tggagaagag acattgatca ttgactttac tgtagtccag 180
gaggattcga atggtatcaa agttaatgtg aaacttgccg acggtgtcga gtatgtggtc 240
ggaacggctg tcgtaagtgt tacacagggc aatgcagtga cggtggcgga aaccaatgtt 300
tctaatccga acgaacctgt atttacggta aaatcggcgg atggaaacaa tgtggtagag 360
cttggaacca tcgttaagct gacgattaag aggagagctg tctgtaccgc atggagcaat 420
gccattaatg ctgccgaaac gggttttgtc ttcaaagaca aggtaacggt gactatcggc 480
gatcatagtg atagcaagga atcaaactcc tattcggtaa actatccgaa cctgacgatc 540
aaacagcctg cgccgcaagt gaacaagcag attgggggaga ccatcgtacg agagttttct 600
ataaccaatg gttctcagaa cccgacccag acagtttatc tttcgataga gtatcccgat 660
gaagcctacc tcacaggggt gggggcgatg acgcttcagg ctaaactggg tgcttccggc 720
acctatgccg atcttactcc taccgtcact aacggtaagg tccgcatcta tacactttcg 780
ggttccagtt tggggcctga tcatctcttg accaacggcg agatcatcta tctgaaagaa 840
acatttaagc tgaaaacttg tgcaccggtt acggtctata gggtaggttg gggttgtagc 900
atagatagcc agtgtgagat aaaaactacc gctgctacaa ttactatggc agctggtgcg 960
gctaatatca cgggatattc agttactggt cctgattatc gttctccaac tttttctctt 1020
tgccaaccgt ttgagttgac tattaagttc tcaaattccg gtgctggtgg ctctatgggg 1080
gcagcattca atatcaatac tattggtaga aacgactatt atagaccaag agggtttgtt 1140
ttacacgaat ttattgatgt caaagtaaac ggtaagccgg taacgaattt caaaaccgat 1200
ggctcagagc tcgaccttcg ttttgatgga cagtttacag aagatcctga cggaccgggg 1260
gttggtttgg atgatgttga cggtgacgga ttttatgatg atcttcctgt cggagctact 1320
attacgatta ctgtaacggt gcggctaaag tgtgatcagt ttacggcatg caacaacgct 1380
ccaaatgatt tgtccgatag gggcttgatt cttaaaacac tatatcagac atcttgtgat 1440
agaacctcat ggatagatcc caacacgtgg ttcaatcttt ctagtactca tttgtatttg 1500
tctcgtgagt cggtacaaga tgcctctcac atgcctactg taatagagaa ggatacgcct 1560
ttcgacctga agataatgac ttcctactat tccatcctca gctcatataa taatatatgg 1620
tacgccaatc ccaatacgcg gtatgtggta gaaatagtat tcccgcaagg tatgactatg 1680
cctcccaaat cggatataga atggaccaat ataaaaaatc atccgataga cgggtcctta 1740
gttttcactc caccgattaa cctccctgat gcaaatatca cgacatcagg aaacacaatg 1800
actatcgttt cgcccagcca agaaagaggt tttgtaaccc tccatggtgt gaaatacgac 1860
tgtacaaata atcacgaaat ggttgtggag tataagatta gagaggtatt caactacctt 1920
cacttccctg attgtctttg cccggtaggt cctattatgt gtaacacggc aaagcgttat 1980
gttttgggct gcgatcctcc ctgcggtaga ggtgcggaaa cttcggtgcc taagatagaa 2040
cgtgccgaca attcgttggg ctggacggat tatacgatgc ggacccgtca atctcgtagc 2100
aatatatcgg cttacgactt ggctaaagcc ctatatatgg acgaagtcaa cattacagcg 2160
acttctatcc agcatggtac tgcttcgtct ttgggcgccc gttttgtttt ggctacgggt 2220
gtcgatcgag tagaaacgct tactcctctt tcggccgata ttaagatctt ccgtgatggg 2280
gttcagattg tatcggtaga tggatatacg acattccgtt caatacgccg aaataataat 2340
gcagagcagg tgatcgactg ggattttact tcaatccttc ctgctggtgg attgcttgat 2400
agagacaaag tggatgttgt tacccgttat cgggtaacat cccagaatgc tcatagagta 2460
gatacgcaag tcggtaggga gtggttcttc tacaactcta ccgccaatgt gtcaccgata 2520
tgggatgaag ccaatccgtt aacttgtctc atacttgtgc ccgagatata catcatgggt 2580
acttttgttg taaacggtac cgatccacat gtcatttcac aatgtactcc aacagatctg 2640
ggacgcgttg ctaaccacta cgcccgtcgt ttcggctctg gtgcatttga atatgccaat 2700
gaatatcgtc ctggtgtaaa gattagaaat atctatctga aagtaccgaa gtcctacacg 2760
ctgaataggg tggagtatag caatcaccgt aaccatagtt cgttaggtac aaccatgcct 2820
ttcgaggaaa taaatcatac agatgtgact tcacagggtg aatataacat ctataagtat 2880
caacttgcag acaacgaaaa ggcgcacttc aatattacag taaaaaatgc ctatggagca 2940
gctcttaaag taaatgtatc tcccacttgt gcgtcgtctg ctgtagcaac taattatgat 3000
aaaatttcat actatgtcga ttacattgac tattactatt atgcagcaac gcagccaaca 3060
gtacctaata gccttgacat agtagccgat caatcggctg gcagcaacgg aatctacagt 3120
gtttccgccc tcaatgttta caacaggcct atcctttata ctaacaaacc ttctattgcg 3180
ctcgtcaatc agtcaggtga ggtagagctt gtgggtaaaa cgggagagtg gaagctgcgt 3240
atcagcaacc catcgagtgc aacggctccc tatgtttggt tggcattgcc tacaacatcg 3300
gggctgacca tcgaaaaagt aactgatgcg gcaggtactg aaatggcgtt tacaacttat 3360
tctggtggca agatgtatcg tttgtcggaa gctggtgttc cagtaggttc tgcgcttgac 3420
tataccattc actttaccta ttctggttgc tctcctatcg ctttgaaggc gatgggggc 3480
tggaactgta gtgcatatcc ccttagtttg gatgagtatg tttgcagttc gcaggtgatc 3540
gatctcaagc tcaagccact gccagctgcc atggagctta ctgagatagc tgttccagat 3600
cctacagctg ctgctacatt gtgtagtaca ttggaatata tttacagcat tcaatcgaca 3660
gataatgcga acgtttatag tcctactttc agcatcttcc ccgaagaggg attggtggtg 3720
acaccgaatc aggtacaggt ggaatatcct gccggttccg gtaattgggc tgcactcaat 3780
gtggtcaata attccgtcaa tctgttgcag catcctgcat tgactaccat aggctatctc 3840

aagggcctga aagaggggga atccaacgac aatcaacgta aaattttggt gaagttctac 3900
ataaagaccg agtgttcgtt cgtatctggc aagaacttcc gtgtaagagc tgacggccgt 3960
aatgcttgta atcagaatgc caagggatcg ggtcttgcca taagtacgcc tccaattaga 4020
ataaatggag ctatagagcc ctacacgact tctgcttcta cgcagcttgt tacgaccaca 4080
acatcacaat cggactgtaa agctcccaaa agagtaaaag tggtgcaaac ggtcgtaggt 4140
ggagaaacta cccccaaggc atatttggaa atcacgctgc cgttgggctt taagtatgtg 4200
acgggttctt atgctccgga caatacgcat ccgggaggag tcaatgcctc acctgccgga 4260
acggaagaag tcactttgac cgcgaacggt gaagacaaga ttaagataaa tgtcaaggcc 4320
ggtctgacgt caggtcaatc gtttgcttat acactcgaaa tgaaggaaga cgatgataat 4380
gtgccggctt gtggcaatca taccatcgaa attgtcaatg ttgaggagat tgaaggtttg 4440
tggtgtgaag gcgttcagtg tgcagaaact ttggtcgtca cgggtgccaa caagtttgaa 4500
tttgagcttg ataagcctta cttggatatt acggttattt cagcagtatc gactttcagt 4560
ggtggtaagg aaaatcttac aattgagtat aaggtaagca atacatcgac cacccagcct 4620
ctgaaaccgg gagcggttgt aacgctgttc agcgataagg ataacaatca agtcttctcc 4680
ggcggagatg ttgctgttgc aacacaggag ttggtcgcag aaataactaa taccacacct 4740
cttacgcaga taatgaaggt aaaaggagtg agctcttccc atacgggcaa tttggttctt 4800
acgatactgc ccaaagacgg ttgctactgt gagatcaaat cccctatggt cacgttaaac 4860
catcttcctt cgaattactg gatcggagga actgtaggta agcctaacga atggaaagag 4920
ccgaacaact ggaccaatga ccaagttccc gatgcggcag aggatgttga attcgccact 4980
gaggtgaata acccgactga tccgaataat ccgaagtcgg gtcctgcgaa ggagaacctg 5040
catctggacg atatacacca gaatggcaca gccggtcgcg ttatcggcaa cctgatcaat 5100
gactctgaca aagatctggt aatcacaacg ggcaatcaat tgacgatcaa cggcgtggtt 5160
gaggataaca atccgaatgt cggtacgatc gtcgtgaagt cgtcgaaaga caatcctacg 5220
gggacgttgc ttttcgccaa tccgggcaat aatcaaaatg taggggggac tgtcgagttt 5280
tacaatcagg gatatgattg tgccgattgt ggtatgtatc gcaggagctg gcagtatttc 5340
ggtatccctg tcaatgaatc agattttcca tatgatcatg ttgatggaaa cgcgaccgtc 5400
aaccaatggg ttgagccttt caatggcgat aagtggcggc ctgcacctta tgcacctgat 5460
acaaagcttc agaaattcaa gggctatcag atcacgaatg acgtgcaggc acagcctacg 5520
ggagtttaca gcttcaaggg tacgctttgt gtgtgcgatg ccttcctgaa cctgacacgc 5580
acgtccggtg tcaactactc gggcgccaac ttgatcggca actcatacac tggagctatc 5640
gacatcaagc aaggtattgt cttcccgccg gaagtcgagc agacggtgta tctgttcaac 5700
acgggaacac gcgaccagtg gcgtaagctt aatggaagca cggtttcagg ctatcgagcc 5760
ggtcagtacc tctctgtacc taagaataca gcgggtcagg acaatcttcc ggatcgtatt 5820
ccatcgatgc attccttctt ggtgaagatg cagaacggag cctcttgtac gttgcagatc 5880
ttgtacgata agctgctcaa gaacacgact gtaaacaacg gtaatggtac gcagatcaca 5940
tggcgatccg gcaactccgg atcggcgaat atgccgtcac ttgtgatgga tgttcttggt 6000
aatgagtcgg ccgaccgttt gtggatcttt accgatgggg gtctttcgtt cggattcgac 6060
aacggctggg atggtcgcaa gctgactgaa aaaggtttgt cacaacttta tgcgatgtct 6120
gacatcggta atgataaatt ccaggttgca ggggttccgg agttgaataa cctgctgatc 6180
ggcttcgatg cggataagga tggtcaatac acgttggagt ttgctctttc ggatcatttt 6240
gcgaaaggcg gagttttcct tgaggatctt agccgtggag ttacacggcg agtagtcgac 6300
ggcggttcgt attcattcga tgccaagcga ggagactccg gggctcgttt ccgtctctct 6360
tatgacgaag agtgggttga atcggcagag gtttctgttt tggttggtac ggccgggaag 6420
cgaatcgtta tcacgaataa cagtgagcat gcctgtcagg ccaatgttta tacaactgac 6480
ggaaaacttc tgattcgatt ggacgtaaaa cccggaagta agtctatgac ggaaccattg 6540
gtcgatggag tctacgttgt cagtctgcaa agtcctgcca cgagtagcaa tgtaaggaaa 6600
gttgtagtca actaa 6615

<212> Type : DNA
<211> Length : 6615

SequenceName : SEQ ID 557
SequenceDescription :
Sequence
--------
<213> OrganismName : Porphyromonas gingivalis W83
<400> PreSequenceString :

atgaacaaat tttacaaatc acttttgcag tcaggactgg ctgccttcgt gtcgatggca 60
actgcactga ccgcttctgc acagatttcg ttcggagggg aacccttgag tttctcttca 120
agatccgccg gaacgcattc attcgacgat gcaatgacta tccgccttac tccggatttc 180
aatccggaag acctgatcgc acagagccgt tggcaatcgc aaagagatgg ccggcccgtc 240
cggataggac aagtaatacc ggtggatgtg gactttgcat ccaaggcttc gcacatctct 300
tccatcggag acgtagatgt atatcgcctg caattcaagt tggaaggagc caaagccatt 360
acgctttatt acgatgcatt caatattccg gagggcggac gcctctatat ctataccccc 420
gaccatgaaa ttgtgttggg agcatatacg aacgccactc atcgccgcaa cggagctttt 480
gccacagagc cggtaccggg gagtgagctt attatggatt atgaagtgtc tcgcggaggg 540
actttgcctg acatcaagat ctccggtgcg ggttatatat tcgacaaagt cggcggacgc 600
cccgtaacgg ataaccatta cgggatcggt gaggacgatt ccgattcgga ttgcgagatc 660

```
aacatcaatt gtcctgaagg tgcagactgg caggcagaga agaacggtgt ggtgcaaatg     720
atcatggtaa aaggacagta tatctcaatg tgctcaggca acctgctcaa taatacgaaa     780
ggagacttta ctccgctgat catttctgcc ggacactgtg cttccataac aaccaatttc     840
ggtgtaacgc aatccgagtt ggataagtgg atcttcactt tccactatga aaaaagagga     900
tgcagcaatg gtacattggc catcttccgt ggcaacagta tcatcggagc ttccatgaag     960
gctttcctcc cgatcaaagg taaatccgat ggtctcttgc tgcaactcaa cgatgaagtc    1020
cctctgcgct atcgtgtcta ttacaatgga tgggacagta cgcccgatat tccctcgagc    1080
ggtgccggta ttcatcatcc ggccggagat gccatgaaga tttccatcct aaagaagact    1140
ccggctctga atacatggat ctcctccagt ggttccggag ggactgacga tcacttctat    1200
ttcaaatacg atcaaggtgg tacggaagga ggatcgtccg gttcttctct cttcaatcag    1260
aataagcacg tggtcggcac actgaccgga ggtgccggca attgtggcgg gacggagttc    1320
tacggcagac tgaacagtca ttggaacgag tatgcatccg atggcaatac gagccgcatg    1380
gacatctatc tggatcccca aaacaatggc cagacgacca tcctcaacgg aacgtatcgt    1440
gacggttata agcctttgcc ctctgtgccc cggctattgt tgcagtctac aggcgatcag    1500
gtcgaattga attggacggc tgttcctgcc gatcaatatc catcatctta tcaggtcgaa    1560
taccacatat tccgaaatgg aaaggaaata gctacgacaa aggagttgtc ctattcggat    1620
gccatcgacg aaagtattat cggtagcggt atcattcgat acgaagtaag cgcacgcttc    1680
atttatccct cgccgttgga tggagtggaa tcttataagg atacggacaa gacttctgcc    1740
gaccttgcca taggagacat tcagaccaag ctgaagccgg acgtaacacc tctccccgga    1800
ggaggagtat cattaagctg gaaagttcct ttcttaagcc agttggtttc ccgattcgga    1860
gaaagcccca atcctgtgtt caaaaccttt gaagtgccct atgtttctgc cgcagccgca    1920
caaaccccca atcctcccgt tggcgtagtc attgcagaca agtttatggc cggtacatat    1980
cccgaaaagg ctgctatcgc tgccgtttat gtaatgccat ccgctccgga ctctactttc    2040
cacctcttcc tcaagagcaa cacaaacaga agattgcaga aggtgacaac tccctccgat    2100
tggcaggccg gaacatggtt gaggatcaat ttggataagc cgttcccggt gaataatgac    2160
catatgcttt ttgccggtat cagaatgcct aataagtaca agctcaatcg tgctatccgt    2220
tatgtaagaa atccggataa ccttttctcc attaccggta agaagatttc atataacaac    2280
ggagtctctt tcgaaggcta cggaataccc tcgctcttgg gctatatggc tatcaaatat    2340
ctggtggtaa ataccgatgc tccgaagatc gatatgtcgc ttgtacagga gccttatgct    2400
aagggaacga atgtggctcc attccccgaa ttggtcggca tatatgtcta taagaacgga    2460
acatttatcg gcacacagga tccatccgtc acaacttatt cggtttcaga cggaacagag    2520
agcgatgaat acgaaataaa actggtatat aagggatcgg gcatttcgaa tggcgttgct    2580
cagattgaga ataacaatgc tgtcgttgca tatccgtctg ttgtaacaga tcgtttcagc    2640
attaagaacg ctcatatggt tcacgctgcc gccctctact cattggatgg caagcaggtt    2700
cgttcttgga acaacctccg caatggcgtg acattcagtg ttcaaggact tacggccggt    2760
acttatatgc tcgttatgca gacggcaaac ggccctgtga gccaaaagat cgtgaagcag    2820
tag                                                                  2823
```

<212> Type : DNA
<211> Length : 2823
SequenceName : SEQ ID 558
SequenceDescription :
Sequence
--------
<213> OrganismName : Porphyromonas gingivalis W83
<400> PreSequenceString :

```
atgaaaaact tgaacaagtt tgtttcgatt gctctttgct cttccttatt aggaggaatg      60
gcatttgcgc agcagacaga gttgggacgc aatccgaatg tgagattgct cgaatccact     120
cagcaatcgg tgacaaaggt tcagttccgt atggacaacc tcaagttcac cgaagttcaa     180
acccctaagg gaatggcaca agtgccgacc tatacagaag gggttaatct ttctgaaaaa     240
gggatgccta cgcttcccat tctatcacgc tctttggcgg tttcagacac tcgtgagatg     300
aaggtagagg ttgtttcctc aaagttcatc gaaaagaaaa atgtcctgat tgcaccctcc     360
aagggcatga ttatgcgtaa cgaagatccg aaaaagatcc cttacgttta tggaaagagc     420
tactcgcaaa acaaattctt cccgggagag atcgccacgc ttgatgatcc ttttatcctt     480
cgtgatgtgc gtggacaggt tgtaaacttt gcgcctttgc agtataaccc tgtgacaaag     540
acgttgcgca tctatacgga aatcactgtg gcagtgagcg aaacttcgga gcaaggcaaa     600
aatattctga acaagaaagg tacatttgcc ggctttgaag acacatacaa gcgcatgttc     660
atgaactacg agccagggcg ttacacaccg gtagaggaaa aacaaaatgg tcgtatgatc     720
gtcatcgtag ccaaaaagta tgagggagat attaaagatt tcgttgattg gaaaaaccaa     780
cgcggtctcc gtaccgaggt gaaagtggca gaagatattg cttctcccgt tacagctaat     840
gctattcagc aattcgttaa gcaagaatac gagaaagaag gtaatgattt gacctatgtt     900
cttttgattg gcgatcacaa agatattcct gccaaaatta ctccggggat caaatccgac     960
caggtatatg gacaaatagt aggtaatgac cactacaacg aagtcttcat cggtcgtttc    1020
tcatgtgaga gcaaagagga tctgaagaca caaatcgatc ggactattca ctatgagcgc    1080
aatataacca cggaagacaa atggctcggt caggctcttt gtattgcttc ggctgaagga    1140
ggcccatccg cagacaatgg tgaaagtgat atccagcatg agaatgtaat cgccaatctg    1200
cttacccagt atggttatac caagattatc aaatgttatg atccgggagt aactcctaaa    1260
```

```
aacattattg atgctttcaa cggaggaatc tcgttggcca actatacggg ccacggtagc 1320
gaaacagctt ggggtacgtc tcacttcggc accactcatg tgaagcagct taccaacagc 1380
aaccagctac cgtttatttt cgacgtagct tgtgtgaatg gcgatttcct attcagcatg 1440
ccttgtttcg cagaagcatt gatgcgtgca caaaaagatg gtaagccgac aggtactgtt 1500
gctatcatag cgtctacgat caaccagtct tgggcttctc ctatgcgcgg gcaggatgag 1560
atgaacgaaa ttctgtgcga aaaacacccg aacaacatca agcgtacttt cggtggtgtc 1620
accatgaacg gtatgtttgc tatggtggaa aagtataaaa aggatggtga gaagatgctc 1680
gacacatgga ctgtattcgg cgacccctcg ctgctcgttc gtacacttgt cccgaccaaa 1740
atgcaggtta cggctccggc tcagattaat ttgacggatg cttcagtcaa cgtatcttgc 1800
gattataatg gtgctattgc taccatttca gccaatggaa agatgttcgg ttctgcagtt 1860
gtcgaaaatg gaacagctac aatcaatctg acaggtctga caaatgaaag cacgcttacc 1920
cttacagtag ttggttacaa caaagagacg gttattaaga ccatcaacac taatggtgag 1980
cctaacccct accagcctgt ttccaacttg actgctacaa cgcagggtca gaaagtaacg 2040
gtcaagtggg atgcaccgag cacgaaaacc aatgcaacca ctaataccgc tcgcagcgtg 2100
gatggcatac gagaactggt tcttctgtca gtcagcgatg cccccgaact tcttcgcagc 2160
ggtcaggccg agattgttct tgaagctcac gatgtttgga atgatggatc cggttatcag 2220
attcttttgg atgcagacca tgatcaatat ggacaggtta tacccagtga tacccatact 2280
ctttggccga actgtagtgt cccggccaat ctgttcgctc cgttcgaata tacggttccg 2340
gaaaatgcag atccttcttg ttcccctacc aatatgataa tggatggtac tgcatccgtt 2400
aatataccgg ccggaactta tgactttgca attgctgctc ctcaagcaaa tgcaaagatt 2460
tggattgccg gacaaggacc gacgaaagaa gatgattatg tatttgaagc cggtaaaaaa 2520
taccatttcc ttatgaagaa gatgggtagc ggtgatggaa ctgaattgac tataagcgaa 2580
ggtggtggaa gcgattacac ctatactgtc tatcgtgacg gcacgaagat caaggaaggt 2640
ctgacggcta cgacattcga agaagacggt gtagctgcag gcaatcatga gtattgcgtg 2700
gaagttaagt acacagccgg cgtatctccg aaggtatgta aagacgttac ggtagaagga 2760
tccaatgaat ttgctcctgt acagaacctg accggtagtg cagtcggcca gaaagtaacg 2820
cttaagtggg atgcacctaa tggtaccccg aatccaaatc caaatccgaa tccaaatccg 2880
aatcccggaa caactacact ttccgaatca ttcgaaaatg gtattcctgc ctcatggaag 2940
acgatcgatg cagacggtga cgggcatggc tggaagcctg gaaatgctcc cggaatcgct 3000
ggctacaata gcaatggttg tgtatattca gagtcattcg gtcttggtgg tataggagtt 3060
cttacccctg acaactatct gataacaccg gcattggatt tgcctaacgg aggtaagttg 3120
actttctggg tatgcgcaca ggatgctaat tatgcatccg agcactatgc ggtgtatgca 3180
tcttcgaccg gtaacgatgc atccaacttc acgaatgctt tgttggaaga gacgattacg 3240
gcaaaaggtg ttcgctcgcc ggaagctatt cgtggtcgta tacagggtac ttggcgccag 3300
aagacggtag accttcccgc aggtacgaaa tatgttgctt tccgtcactt ccaaagcacg 3360
gatatgttct acatcgacct tgatgaggtt gagatcaagg ccaatggcaa gcgcgcagac 3420
ttcacggaaa cgttcgagtc ttctactcat ggagaggcac cagcggaatg gactactatc 3480
gatgccgatg gcgatggtca gggttggctc tgtctgtctt ccggacaatt ggactggctg 3540
acagctcatg gcggcaccaa cgtagtaagc tctttctcat ggaatggaat ggctttgaat 3600
cctgataact atctcatctc aaaggatgtt acaggcgcaa cgaaggtaaa gtactactat 3660
gcagtcaacg acggtttttcc cggggatcac tatgcggtga tgatctccaa gacgggcacg 3720
aacgccggag acttcacggt tgttttcgaa gaaacgccta acggaataaa taagggcgga 3780
gcaagattcg gtctttccac ggaagccgat ggcgccaaac ctcaaagtgt atggatcgag 3840
cgtacggtag atttgcctgc gggcacgaag tatgttgctt tccgtcacta caattgctcg 3900
gatttgaact acattctttt ggatgatatt cagttcacca tgggtggcag ccccaccccg 3960
accgattata cctacacggt gtatcgtgat ggtacgaaga tcaaggaagg tttgaccgaa 4020
acgaccttcg aagaagacgg cgtagctacg ggcaatcatg agtattgcgt ggaagtgaag 4080
tacacagccg gcgtatctcc gaagaaatgt gtaaacgtaa ctgttaattc gacacagttc 4140
aatcctgtaa agaacctgaa ggcacaaccg gatggcggcg acgtggttct caagtgggaa 4200
gccccgagcg caaagaagac agaaggttct cgtgaagtaa aacggatcgg agacggtctt 4260
ttcgttacga tcgaacctgc aaacgatgta cgtgccaacg aagccaaggt tgtgctcgca 4320
gcagacaacg tatgggggaga caatacgggt taccagttct tgttggatgc cgatcacaat 4380
acattcggaa gtgtcattcc ggcaaccggt cctctcttta ccggaacagc ttcttccgat 4440
ctttacagtg cgaacttcga gtatttgatc ccggccaatg ccgatcctgt tgttactaca 4500
cagaatatta tcgttacagg acagggtgaa gttgtaatcc ccggtggtgt ttacgactat 4560
tgcattacga acccggaacc tgcatccgga aagatgtgga tcgcaggaga tggaggcaac 4620
cagcctgcac gttatgacga tttcacattc gaagcaggca agaagtacac cttcacgatg 4680
cgtcgcgccg gaatgggaga tggaactgat atggaagtcg aagacgattc acctgcaagc 4740
tatacctata cagtctatcg tgacggcacg aagatcaagg aaggtctgac cgaaacgacc 4800
taccgcgatg caggaatgag tgcacaatct catgagtatt gcgtggaagt taagtacaca 4860
gccggcgtat ctccgaaggt ttgtgtggat tatattcctg acggagtggc agacgtaacg 4920
gctcagaagc cttacacgct gacagttgta ggaaagacga tcacggtaac ttgccaaggc 4980
gaagctatga tctacgacat gaacggtcgt cgtctggcag ccggtcgcaa cacggttgtt 5040
tacacggctc agggcggcta ctatgcagtt atggttgtcg ttgacggcaa gtcttacgta 5100
gagaaactcg ctatcaagta a 5121
```

<212> Type : DNA
<211> Length : 5121
SequenceName : SEQ ID 559
SequenceDescription :

Sequence
--------
<213> OrganismName : Porphyromonas gingivalis W83
<400> PreSequenceString :

```
atgaaaagaa aaccgctatt ctcagccctt gtaatccttt ccggcttctt cggatcggtt     60
cacccggcct cagcacagaa agttcctgca cccgtcgatg gcgagcgcat tatcatggag    120
ctaagtgaag ccgatgtgga gtgtacaatc aaaatagaag ccgaggatgg ctatgccaac    180
gacatttggg cagacctcaa cggaaacggc aagtacgatt cgggggagag gctcgattca    240
ggtgagtttc gtgatgttga gttcagacaa acaaaggcca tcgtctatgg caaaatggcc    300
aaattcttgt ttagaggttc ttctgcaggg gactatggtg ctacctttat agatattagc    360
aattgtaccg gcctgactgc tttcgactgc tttgccaatc tgctgacaga actcgatctg    420
tccaaagcaa acggtctgac ttttgtaaac tgcggcaaaa accagctgac caagcttgac    480
ctgcccgcaa atgcggacat tgagacgctg aactgctcca aaaacaagat aacgagtctc    540
aacctatcga cctataccaa gctgaaagag ctttatgtgg gcgacaacgg gctgacagcc    600
ttggatctct ccgccaatac gctcctcgaa gagctggtgt attctaacaa cgaggtgact    660
acgataaacc tgtctgccaa tacgaacttg aaaagcctgt attgcataaa caataagatg    720
accggactcg atgtcgcagc caacaaagag ctgaaaatac tccactgcaa caacaatcag    780
ctgaccgccc tcaatctctc ggccaatacc aagctgacga ctctaagctt cttcaacaac    840
gagctgacaa atatcgatct ctccgacaac acggctttgg agtggctttt ctgcaacggc    900
aataagctga cgaagttaga tgtatctgcc aacgccaatc tgatagcact gcaatgcagc    960
aacaaccagc tgactgctct ggatctgtca aaaacgccga aactgacaac gttgaattgc   1020
tactccaacc ggatcaaaga taccgccatg cgtgcattga tcgaaagcct gcctacgatc   1080
actgaaggag aaggcaggtt cgttccttac aacgacgatg aaggaggaga agaggagaac   1140
gtgtgtacaa ccgaacacgt ggaaatggcc aaggccaaga attggaaggt acttacctcg   1200
tggggagagc ctttccccgg aataacggct ttgatttcca tcgaaggtga gagcgaatat   1260
tccgtatatg ctcaagatgg catcctctac ctctccggta tggagcaggg cttgcccgtt   1320
caggtatata ccgtgggagg aagcatgatg tactcatctg tcgcttccgg atcagccatg   1380
gaaatacagc tcccgagagg tgcagcctat gtagtacgta tcggcagcca tgcgatcaaa   1440
accgcgatgc cgtaa                                                    1455
```

<212> Type : DNA
<211> Length : 1455
SequenceName : SEQ ID 560
SequenceDescription :
Sequence
--------
<213> OrganismName : Shigella flexneri 2a str. 2457T
<400> PreSequenceString :

```
atgaaacgag ctattaccct gtttgctgta ctgctgatgg gctggtcggt aaatgcctgg     60
tcattcgcct gtaaaaccgc caatggtacc gctatcccta ttggcggtgg cagcgctaat    120
gtttatgtaa accttgcgcc tgtcgtgaat gtggggcaaa acctggtcgt agatctttcg    180
acgcaaatct tttgccataa cgattatccg gaaaccatta cagactatgt cacactgcaa    240
cgaggctcgg cttacggcgg cgtgttatct aatttttccg ggaccgtaaa atatagtggc    300
agtagctatc catttccgac caccagcgaa acgccgcggg ttgtttataa ttcgagaacg    360
gataagccgt ggccggtggc gctttatttg acgcctgtga gcagtgcggg cggggtggct    420
attaaagctg gttcattaat tgccgtgctt attttgcgac agaccaacaa ctataacagc    480
gatgattttc agtttgtgtg gaatatttac gccaataatg atgtggtggt gcccactggc    540
ggttgtgatg tttctgctcg tgatgtcacc gttactctgc cggactaccc tggttcagtg    600
ccaattcctc ttaccgttta ttgtgcgaaa agccaaaacc tggggtatta cctctccggc    660
acaaccgcag atgcgggcaa ctcgattttc accaataccg cgtcgttttc accagcgcag    720
ggcgtcggcg tacagttgac gcgcaacggt acgattattc cagcgaataa cacggtatcg    780
ttaggagcag taggaacttc ggcggtaagt ctgggattaa cggcaaatta cgcacgtacc    840
ggagggcagg tgactgcagg gaatgtgcaa tcgattattg gcgtgacttt tgtttatcaa    900
taa                                                                  903
```

<212> Type : DNA
<211> Length : 903
SequenceName : SEQ ID 561
SequenceDescription :
Sequence
--------
<213> OrganismName : Shigella flexneri 2a str. 2457T

<400> PreSequenceString :

atgggcatca aacaacacaa tgggaatacc aaagccgatc gtctcgctga attaaaaatc 60

cgttcgccct caattcaact gataaaattt ggcgctattg gtttgaatgc aattatcttt 120
tcccccctgc tgatagctgc tgatacagga agtcaatatg gcaccaatat tactattaat 180
gatggtgaca gaattacagg agataccgcc gatccatcag gaaacctcta tggtgtaatg 240
accccagcag gaaacacgcc tggcaatatc aacctgggta atgatgtcac cgtcaatgtc 300
aacgacgcct ctggatatgc aaaaggaatc attattcagg gcaaaaacag ctccctgaca 360
gctaaccgac tcacagtaga tgttgttggt caaacctctg ccatcggcat taacttaatt 420
ggtgactata cccatgctga cttaggcaca ggcagcacca ttaagagtaa cgatgacggc 480
atcattattg ggcatagctc aacactaaca gccactcaat tcaccattga aaactcgaac 540
ggtataggcc taaccatcaa tgactatggc accagtgtcg atcttggaag cggaagtaaa 600
atcaagaccg atggaagtac aggtgtttat atcggtggtc tcaacggcaa taacgccaat 660
ggtgctgcgc gttttacggc gacagacctg acaatcgatg ttcagggcta cagcgccatg 720
gggataaacg tacagaaaaa ctctgttgtc gatctcggaa caaacagtac cattaaaacc 780
aatggcgata atgctcacgg cctctggagc tttggccagg ttagcgcgaa tgcactcact 840
gttgatgtaa ctggagccgc ggccaatggc gtcgaagttc gtggtggtac aaccactatc 900
ggtgcagata gccatatttc ttccgcgcag ggcggtggcc tcgtcaccag tagttcagac 960
gcgacaatca atttttctgg cacggcagcg caacgaaaca gcatcttttc cggcggttct 1020
tatggtgcct cggcccagac ggcaacggct gttatcaaca tgcaaaatac cgatattacg 1080
gttgatcgta atggcagtct ggcgctgggt ttgtgggcgc tcagcggcgg tagaataacc 1140
ggagacagtt tggctatcac cggcgcggca ggagccagag ggatttatgc catgaccaac 1200
agccagatcg acctcacgag cgatctggtc attgatatga gtacacccga ccagatggcc 1260
atcgcaacgc aacatgacga tggttatgcc gccagccgca tcaacgcctc gggtcgtatg 1320
cttatcaacg gtagcgttct ttccaaaggt gggctaatca atctggatat gcaccctggg 1380
tcggtttgga caggttcctc cctcagcgat aatgtcaatg gcgggaaact ggacgttgca 1440
atgaataaca gcgtctggaa cgtaacaagt aattctaatc tcgacacgct ggcgctgagc 1500
cattcaactg tcgattttgc cagccacggg tcaactgccg gcacatttac cacattaaac 1560
gtagagaacc tgagcggtaa cagtaccttt attatgcgtg ctgatgttgt tggcgagggt 1620
aatggcgtta ataatagagg ggatttattg aatatcagcg ggagtagtgc tggtaatcac 1680
gtattggcta tccgcaacca gggcagcgag gccacaacgg gaaatgaagt tctgacagtg 1740
gtaaaaacca ctgacggcgc ggcctcgttc agcgcgtctt ctcaggttga gttgggggga 1800
tatctgtacg atgtgcgtaa aaatggcact aactgggagc tttacgcttc cgggacagtt 1860
ccggaaccga ctcctaatcc tgaacccaca ccagctcccg ctcagcctcc catagtcaac 1920
cccgatccta cgcctgaacc cgctcccacg cctaaaccca ccacgaccgc agatgctggc 1980
ggcaattatc tcaatgtcgg ttacttattg aactatgttg aaaaccgtac gctgatgcaa 2040
cggatgggtg acttgcgaaa tcagagtaaa gacggtaata tctggttgcg cagttatggg 2100
ggaagcctgg actcctttgc cagtggcaaa ctgagcggct ttgacatggg ttacagcggt 2160
atccagtttg gtggggataa acgtctctct gatgtaatgc cgttgtatgt cggtctgtat 2220
attgactcaa cacatgcatc gccggactat agcggaggcg acggtaccgc acgttcagac 2280
tacatgggaa tgtacgccag ttacatggca caaaacggtt tttacagcga tctcgttata 2340
aaagcatcgc gccagaaaaa tagtttccac gtactggaca gtcagaacaa cggcgttaac 2400
gccaacggca ctgcgaatgg aatgagcatc tccctggaag ccgggcagag gttcaacctg 2460
tcccctactg gttatgggtt ctatatagag ccgcaaaccc agcttacata cagccaccag 2520
aatgagatgg ctatgaaggc gagtaatggc ctcaatatac atctgaatca ctacgaatcg 2580
ctgctggggc gtgccagcat gatactgggg tatgacatca ccgcaggcaa cagccagctg 2640
aatgtctatg tgaagactgg cgctatccgc gagttttcag ggatacccga atatctgttg 2700
aacgactccc gggagaagta cagtttcaaa ggtaatggct ggaataacgg cgtgggagtc 2760
agtgcacagt ataacaaaca gcacacattc tatctcgaag cggattacac gcagggtaac 2820
ctctttgatc agaagcaagt caacggagga tatcgcttca gctttttaa 2868

<212> Type : DNA
<211> Length : 2868
SequenceName : SEQ ID 562
SequenceDescription :
Sequence
--------
<213> OrganismName : Shigella flexneri 2a str. 24 57T
<400> PreSequenceString :

```
atgagtaagt ttgttaaaac agctattgct gcggcaatgg tgatggcgt gttcacctct 60
acggcaacaa tcgctgcggg taacaacggt actgcacgtt tttacggcac cattgaagac 120
tcagtgtgtt ctatcgttcc ggacgatcac aaactggaag ttgatatggg tgatatcggt 180
gccgaaaaac tgaaaaataa cggcaccacc acgccgaaaa gtttccagat tcgtctgcaa 240
gattgcgtat ttgatactca ggaaacaatg accactacct ttactggtac cgtttcttct 300
gcaaatagcg gcaattatta caccattttc aataccgata ccggtgcggc atttaacaat 360
gttagcctgg cgatcggtga ctctctgggt acctcttaca aagcggcat gggtattgac 420
cagaaaatag tgaaagacac ttctaccaac aaaggcaaag cgaagcagac actgaacttt 480
aacgcctggc tggtcggcgc agctgatgcg ccagatctgg gtaattttga agcaaatacc 540
accttccaga ttacttacct gtaa 564
```

<212> Type : DNA
<211> Length : 564
SequenceName : SEQ ID 563
SequenceDescription :
Sequence
--------
<213> OrganismName : Shigella flexneri 2a str. 2457T
<400> PreSequenceString :

```
atgaaaatta aaactctggc aatcgttgtt ctgtcggctc tgtccctcag ttccgcagcg 60
gctctggccg atactacgac ggtaaatggt gggaccattc actttaaagg ggaagttgtt 120
aacgccgctt gcgcagttga tgcaggctct gttgatcaaa ccgttcagtt gggacaggtt 180
cgtaccgcta gcctgaagca ggctggagca accagctctg ccgttggttt taacattcag 240
ctgaatgatt gcgataccac tgttgccaca aaagccgctg ttgccttctt aggtacggca 300
attgatgcta cgcgtactga tgtactggct ctgcagagtt ctgctgcagg tagtgcaaca 360
aacgttggcg tgcagatcct agacagaaca ggcaatgctc tgacgctgga cggtgcgaca 420
tttagtgcac aaacaaccct gaataacggt accaacacca ttccgttcca ggcgcgttat 480
tatgcaatcg gcgaggcaac cccgggtgca gctaatgcgg atgcaacctt caaggttcag 540
tatcaataa 549
```

<212> Type : DNA
<211> Length : 549
SequenceName : SEQ ID 564
SequenceDescription :
Sequence
--------
<213> OrganismName : Shigella flexneri 2a str. 2457T
<400> PreSequenceString :

```
atggcaagta tttcatcgct gggagtcggg tcaggtctgg atttaagttc aatccttgat 60
agcctcaccg ccgcgcaaaa agcgacgcta acccctattt caaatcagca atcgtcgttt 120
accgctaaac ttagcgccta cggtacgctg aaaagcgcgc tgacgacttt ccagaccgcc 180
aatactgcat tgtctaaagc cgatcttttt tccgccacca gcaccaccag cagcaccacc 240
gcgttcagtg ccaccaccgc gggtaacgcc atcgccggga aatacaccat cagcgtcacc 300
catctggcgc aggcgcaaac cctgaccacg cgcaccacca gagatgatac gaaaacggcg 360
atcgccacca gcgacagcaa actcaccatt caacaaggcg acgacaaaga tccgattacc 420
attgatatca gcgcggctaa ctcatcgtta agcgggatcc gtgatgccat caacaacgca 480
aaagcgggcg taagtgcgag catcattaac gtgggtaacg gtgaatatcg tctgtcagtc 540
acatcaaatg acaccggcct tgataatgcg atgacactct cggtcagcgg tgatgatgcg 600
ctacaaagtt ttatgggcta tgacgccagt gccagcagca acggtatgga ggtctcggtc 660
gccgcccaga atgcgcagct gacggtcaac aacgtcgcca tcgagaacag cagcaacact 720
atcagcgacg cgctggaaaa catcactctg aacctgaacg atgtcaccac gggcaaccag 780
acgctaacca tcactcagga cacctccaaa gtgcaaacgg cgattaaaga ctgggtgaat 840
gcctataact cgctaataga taccttcagc agcctgacca aatacaccgc cgtagatgcg 900
ggagctgata gccagagttc tagcaatggc gcactgctcg gcgactccac gctgcggacg 960
attcagacgc agttgaagtc gatgctgagt aataccgtca gttcttccag ctataaaacg 1020
ctggcgcaga ttggtatcac gaccgatccc agcgatggca aactggaact ggatgccgac 1080
aaactcaccg ctgcactgaa aaaagatgcc agcggcgtag gtgcattgat tgttggcgat 1140
ggtaaaaaaa ccggcatcac aaccaccatc ggcagcaacc tgaccagttg gctttcgaca 1200
acgggcatta ttaaagccgc taccgatggc gttagtaaaa ccctgaataa gttaactaaa 1260
gactacaacg ccgccagcga tcgcattgat gcgcaggtcg cgcgctacaa agaacaattt 1320
acccaactgg acgttttaat gacctcgtta aacagcacca gcagctactt aacgcagcag 1380
ttcgaaaaca acagtaattc caagtaa 1407
```

<212> Type : DNA
<211> Length : 1407
SequenceName : SEQ ID 565
SequenceDescription :
Sequence
--------
<213> OrganismName : Shigella flexneri 2a str. 2457T
<400> PreSequenceString :

```
gtggagggta aagctgataa tgtcgtactg gaaaatggcg gacgcctgga tgtgctgacc      60
ggacacacag ccactaatac ccgcgtggat gatggcggaa cgctggatgt ccgcaacggt     120
ggcaccgcca ccaccgtatc catgggaaat ggcggtgtac tgctggccga ttccggtgcc     180
gctgtcagtg gtacccggag cgacggaaag gcattcagta tcggaggcgg tcaggcggat     240
gccctgatgc tggaaaaagg cagttcattc acgctgaacg ccggtgatac ggccacggat     300
accacggtaa atggcggact gttcaccgcc aggggcggca cactggcggg caccaccacg     360
ctgaataacg gcgccatact taccctttcc gggaagacgg tgaacaacga taccctgacc     420

atccgtgaag gcgatgcact cctgcaggga ggcgctctca ccggtaacgg cagcgtggaa     480
aaatcaggaa gtggcacact cactgtcagc aacaccacac tcacccagaa agccgtcaac     540
ctgaatgaag gcacgctgac gctgaacgac agtaccgtca ccacggatgt cattgctcag     600
cgcggtacag ccctgaagct gaccggcagc actgtgctga acggtgccat tgaccccacg     660
aatgtcactc tcgcctctgg tgccacctgg aatattcccg ataacgccac ggtgcagtcg     720
gtggtggatg acctcagcca tgccggacag attcatttca cctccacccg cacagggaag     780
ttcgtaccgg caaccctgaa agtgaaaaac ctgaacggac agaatggcac catcagcctg     840
cgtgtacgcc cggatatggc acagaacaat gctgacagac tggtcattga cggtggcagg     900
gcaaccggaa aaaccatcct gaacctggtg aacgccggca acagtgcgtc ggggctggcg     960
accagcggta agggtattca ggtggtggaa gccattaacg gtgccaccac ggaggaaggg    1020
gcctttatcc aggggaataa gctgcaggcc ggtgccttta actactccct caaccgggac    1080
agtgatgaga gctggtatct gcgcagtgaa aatgcttatc gtgcagaagt cccccctgtat   1140
gcctccatgc tgacacaggc aatggactat gaccggattc tggcaggctc ccgcagccat    1200
cagaccggtg taagcggtga aaataacagc gtccgtctca gcattcaggg cggtcatctc    1260
ggtcacgata acaacggcgg tattgcccgt ggagccacgc cggaaagcag cggcagctat    1320
ggcttcgtcc gtctggaggg tgacctgctc agaacagagg ttgccggtat gtctgtgacc    1380
gcgggggtat atggtgctgc tggccattct tccgttgatg ttaaggatga tgacggctcc    1440
cgtgccggca cggtccggga tgatgccggc agcctgggcg gatacctgaa tctgatacac    1500
aacgcctccg gcctgtgggc tgacattgtg gcccagggaa cccgccacag catgaaagcc    1560
tcatcggaca ataacgactt ccgcgtccgg ggctggggct ggctgggttc gctggaaacc    1620
ggtctgccct tcagtatcac tgacaatctg atgctggagc cgcaactgca gtacacctgg    1680
cagggactct ccctggatga cggccaggat aacgccagtt atgtgaagtt cgggcatggc    1740
agtgcacaac atgtgcgtgc cggcttccgt ctgggcagcc accacgatat gaactttggt    1800
aaaggcacct catcccgtga caccctgcgc ggcagtgcca aacacagtgt gcgtgaactg    1860
ccggtgaact ggtgggtaca gccttctgtt atccgcacct tcagctcccg gggggacatg    1920
agcatgggta cagccgcagc cggcagtaac atgacgttct caccgtcaca gaatggtacg    1980
tcactggacc tgcaggccgg actggaagcc cgtgtccggg aaaatatcac cctgggcgtt    2040
caggccagtt atgcccacag catcaacggc agcagcgctg aaggttataa cagtcaggcc    2100
acactgaatg taaccttctg a                                              2121
```

<212> Type : DNA
<211> Length : 2121
SequenceName : SEQ ID 566
SequenceDescription :
Sequence
--------
<213> OrganismName : Shigella flexneri 2a str. 2457T
<400> PreSequenceString :

```
atggcctttt ctcaagcggt tagcggatta aacgctgccg ccaccaacct cgatgttatt      60
ggcaacaata tcgccaactc cgccacctac ggctttaaat caggcacggc ctcttttgcc     120
gatatgtttg ccggttcgaa agtgggactg ggggtaaaag ttgccggtat cactcaggac     180
tttaccgatg gcacgaccac caacaccggg cgaggtctgg acgttgctat cagccagaac     240
ggtttttttcc gtctggtaga cagcaacggt tcggtgttct acagccgtaa cggacaattt    300
aagctggatg aaaaccgtaa cctgttgaat acgcaaggtt tacagctgac gggttacccg     360
gtaaccggta cgccgccgac tattcagcaa ggggcgaatc cgaccaatat ttcgatcccg     420
aataccctga tggcagcgaa aactaccacc acggcgtcga tgcagatcaa cctgaattcc     480
agtgatccgc ttcctactgt tacgccattc agcgccagca atgcggatag ctataacaaa     540
aaaggttcgg tgactgtttt cgacagtcag ggtaatgctc atgacatgag cgtctatttt     600
gtgaagaccg ggataataa ctggcaggtc tacacccagg atagcagtga tccaaacagc      660
attgcgaaga cagcgacaac actggaattt aatgctaatg gcacattagt ggatggtgcg     720
atggcgaata atatcgcaac cggcgcaatt aacggtgcag aacccgccac gtttagtctg     780
agcttcctca actccatgca gcaaaatacc ggcgctaaca acattgtggc aaccacccag     840
aatggctaca aaccgggcga tctggtgagt tatcaaatca atgatgacgg tacggttgtc     900
ggcaacaatt ccaacgaaca aacccaactg ctggggcaga ttgtactggc gaactttgcc     960
aacaacgaag gtctggcatc cgaaggcgac aacgtctggt ctgcgacgca atcttctggc    1020
gtggcgctgt tggggacagc cgggacggga aactttggca ctctgaccaa cggtgcgctg    1080
gaagcgtcca acgtcgatct cagtaaagaa ctggtcaata tgatcgttgc ccagcgtaac    1140
tataagtcta acgcccagac catcaaaacc caggaccaga tcctcaacac gcgggttaac    1200
ttacgctaa                                                            1209
```

<212> Type : DNA
<211> Length : 1209
SequenceName : SEQ ID 567
SequenceDescription :
Sequence
--------
<213> OrganismName : Shigella flexneri 2a str. 2457T
<400> PreSequenceString :

```
atgaaactcg tgcacatggc cagtggttta gcggttgcga ttgcgttggc ggcttgcgca      60
gataaaagcg cggatattca gacgccagcc ccggctgcaa atacgtctat ttcagcaaca     120
caacaacctg ctatccagca accgaatgtc tccggtactg tctggatccg tcagaaagtc     180
gcactgccgc ctgatgctgt gctgaccgtg acactttctg acgcgtcgtt agccgatgca     240
ccgtcaaaag tgctggcgca gaaagcggtg cgtactgaag gtaaacagtc accattcagc     300
tttgttctgc catttaaccc ggcagatgtt cagccgaacg cgcgtattct gttgagtgcg     360
gcgattaccg tgaatgacaa actggtattt atcaccgata ccgttcagcc ggtgatcaac     420
cagggcggaa ctaaagccga cctgacattg gtgccggtac agcaaaccgc cgtgccggtt     480
caggccagcg gtggcgcaac gactaccgta ccttcgactt caccaactca ggtgaatccg     540
tcttcggcag ttcccgctcc tacgcaatat taa                                 573
```

<212> Type : DNA
<211> Length : 573
SequenceName : SEQ ID 568
SequenceDescription :
Sequence
--------
<213> OrganismName : Shigella flexneri 2a str. 2457T
<400> PreSequenceString :

```
atgatcataa aaaaaagcgg tggtcgctgg cagctaagcc tgctggcgag cgtggtaatc      60
agtgcctttt ttctcaacac agcttacgcc tggcaacaag aatatatcgt tgatacgcaa     120
cccggacatt ccacagagcg ttacacctgg gatagtgatc atcaacctga ttacaacgat     180
attttgtcgc aacgtattca aagtagccaa agggcgctgg gactggaagt caatctggcg     240
gaagaaactc ctgtggatgt gaccagcagt atgagtatgg gctggaattt tcctttatat     300
gaacaggtta caaccggccc ggtcgcggca ttacattacg atggcacaac cacctcgatg     360
tataacgagt ttggcgacag tactaccacg ctgaccgatc cgttatggca tgccagcgtg     420
agtagcttag gctggcgtgt tgactcccgg cttggcgatc tccgaccctg ggcgcaaatc     480
agctataacc agcaatttgg cgagaatatc tggaaggcgc aatcaggcct gagccggatg     540
acggcgacaa accagaacgg caactggctg gatgtcaccg taggcgctga tatgttgctc     600
aatcaaaata ttgccgccta tgctgcgcta actcaggcag aaaataccac taataatagc     660
gactatctgt atacgatggg ggttagcgcc agattttaa                           699
```

<212> Type : DNA
<211> Length : 699
SequenceName : SEQ ID 569
SequenceDescription :
Sequence
--------
<213> OrganismName : Shigella flexneri 2a str. 2457T
<400> PreSequenceString :

```
atgaaatggt gcaaacgtgg gtatgtattg gcggcaatgt tggcgctcgc aagtgcgacg     60
atacaggcag ccgatgtcac catcacggtg aacggtaagg tcgtcgccaa accgtgtacg    120
gtttccacca ccaatgccac ggtagatctc ggcgatcttt attctttcag tcttatgtct    180
gccggggcgg catcggcctg gcatgatgtt gcgcttgagt tgactaattg tccggtggga    240
acgtcgaggg tcactgccag cttcagcggg gcagccgaca gcaccggata ttataaaaac    300
caggggaccg cgcaaaacat ccagttagag ctacaggatg acagtggcaa cacattgaat    360
actggcgcaa ccaaaacagt tcaggtggat gattcctcac aatcagcgca cttcccgtta    420
caggtcagag cattgacggt aaatggcgga gccactcagg gaaccattca ggcagtgatt    480
agcatcacct atacctacag ctga                                           504
```

<212> Type : DNA
<211> Length : 504
SequenceName : SEQ ID 570
SequenceDescription :
Sequence
--------
<213> OrganismName : Shigella flexneri 2a str. 2457T
<400> PreSequenceString :

```
atgaaaagag cgcctcttat aacaggactt ttgttgatat ccacatcctg cgcttatgcc     60
tcctcaggag ggtgtggagc cgacagcact agcggtgcga caaattacag cagtgtggtt    120
gatgatgtta cggtgaacca gacagataac gtgacaggac gggagtttac ctctgcaacg    180
ctaagtagca ctaactggca atacgcctgt tcctgctctg cgggtaaggc agttaaactt    240
gtctatatgg tcagccccgt acttaccacc actggacatc agacaggata ttacaaactc    300
aatgacagcc tggatattaa aaccacatta caggcaaacg acattccagg actcacaacc    360
gaccaggttg tctctgttaa cacccgattc acacagataa aaagcagcac cgtatattct    420
gctgcaaccc aaacgggtgt ttgccagggt gacacgtctc gttatggacc cgttaatatt    480

ggtgcaaata ccacctttac cctgtatgtc accaagccat ttctcggctc gatgaccatt    540
ccgaaaacgg atattgccgt cattaaaggc gcgtgggtcg atggaatggg aagcccgtct    600
acaggtgact tccatgattt agtcaagtta tcgattcagg gaaatctcac cgcccccacag   660
tcgtgcaaaa ttaatcaggg cgatgttatt aaagttaatt ttggattcat caatggtcag    720
aagtttacca cccgcaatgc catgccagac ggttttactc cagtagactt tgatatcact    780
tatgactgtg gtgatacttc aaagattaaa aactcgttgc aaatgcgcat cgacggtaca    840
actggggtag tagaccagta caacctggtc gccaggcgaa gaagttcaga caatgtgccc    900
gatgtcggta ttcgtattga aaatctcggc ggcggagttg caaatattcc ttttcagaac    960
ggtatccttc ccgttgatcc ttccgggcat ggcaccgtca atatgcgcgc ctggccagtt   1020
aatctggtcg gtggtgagct ggaaacagga aaatttcagg gcacagccac cattaccgtc   1080
atggtgcggt aa                                                       1092
```

<212> Type : DNA
<211> Length : 1092
SequenceName : SEQ ID 571
SequenceDescription :
Sequence
--------
<213> OrganismName : Shigella flexneri 2a str. 2457T
<400> PreSequenceString :

```
atgcaaaaaa acgctgcgca tacttatgcc atttccagct tgttggtgct ttcactaacc      60
ggctgcgcct ggataccctc cacgccgctg gtgcaggggg cgaccagtgc acaaccggtt     120
cccggtccga cgcccgtcgc caacggttct attttccagt ctgctcagcc gattaactat     180
ggctatcaac cgctgtttga agatcgtcga ccacgcaata ttggcgatac gctgaccatc     240
gtgttgcagg agaacgtcag cgccagcaaa agctcctctg cgaatgccag ccgtgacggt     300
aaaactaatt ttggctttga tactgtgccg cgctatttgc aggggctgtt tggtaacgct     360
cgtgccgatg tcgaagcctc cggtggtaac acgttcaacg gaaagggcgg ggccaatgcc     420
agcaatacct ttagcggcac gttgacggtg acggttgacc aggtactggt caacggcaac     480
ctgcatgtgg tgggtgaaaa acagattgcc attaatcagg gtaccgaatt tattcgcttc     540
tcgggcgtgg ttaatccacg cactatcagc ggcagcaata ccgtaccgtc tactcaggtg     600
gcggatgcgc gtattgaata cgtaggcaat ggctacatta acgaagcgca aaatatgggc     660
tggttgcagc gtttcttcct taacctgtcg ccaatgtaa                            699
```

<212> Type : DNA
<211> Length : 699
SequenceName : SEQ ID 572
SequenceDescription :
Sequence
--------
<213> OrganismName : Shigella flexneri 2a str. 2457T
<400> PreSequenceString :

```
atgaaacgac atctgaatac ctgctacagg ctggtatgga atcacattac gggcgctttc      60
gtggttgcct ccgaactggc ccgcgcacag ggtaaacgtg gcggtgtggc ggttgcactg     120
tctcttgccg cggtcacgtc actcccggtg ctggctgctg acatcgttgt gcacccgggt     180
gaaacagtga atggcggaac actggtaaac catgacaacc agtttgtatc cggaacagct     240
gatggcgtga ctgtcagtac cgggcttgag ctggggccgg acagtgacga aaacaccggc     300
gggcaatgga taaaagcggg tggcacaggc agaaacacca ctgtcaccgc aaatggtcgt     360
cagattgtgc aggcaggagg aactgccagt gatacggtta ttcgtgatgg cggagggcag     420
agccttaacg gactggcggt gaacaccacg ctggataaca gaggtgagca gtgggtacac     480
gggggaggga aagctgccgg tacaattatt aaccaggatg gttaccagac cataaaacat     540
ggcggactgg caaccggaac catcgtcaac accggtgcag aaggtggtcc ggagtctgaa     600
aatgtgtcca gcggtcagat ggtcggaggg acggctgaat ccaccaccat caataaaaat     660
ggccggcagg ttatctggtc ttcggggatg gcacgggaca ccctcattta cgccggtggt     720
gaccagacgg tacacggaga ggcacataac acccgactgg agggggtaa ccagtatgta      780
cacaacggtg gcacggcaac agagacgctg ataaaccgtg atggctggca ggtgattaag     840
gaaggaggaa ctgccgcgca taccaccatc aaccagaaag aaagctgcag gtga           894
```

<212> Type : DNA
<211> Length : 894
SequenceName : SEQ ID 573
SequenceDescription :
Sequence
--------
<213> OrganismName : Shigella flexneri 2a str. 2457T
<400> PreSequenceString :

```
atgatgatga aaactattaa acatcttctg tgctgtgcca ttgccgccag cgcattaatt      60
tccaccgggg tgcatgctgc gtcctggaaa gatgcgctct ccagcgccgc cagcgaactt     120
ggcaaccaaa acagcacgac acaggaaggc ggttggtcgc tcgcgtcatt aactaacttg     180
cttagcagcg gaaaccaagc cttaagcgca gataacatga acaatgccgc aggcattctg     240
caatactgcg cgaagcaaaa gctggcttcg gtaaccgatg ccgaaaacat caagaaccag     300
gtgctggaaa agctgggcct gaacagtgaa gagcaaaaag aagacaccaa ctatctggac     360
ggtattcagg gtttgctgaa aacaaaagat ggtcagcaac tcaatctgga taacatcgga     420
acgactccgc tggcagaaaa ggtgaaaacc aaagcctgcg atctggtgtt aaagcagggg     480
ctgaacttca tttcctga                                                   498
```

<212> Type : DNA
<211> Length : 498
SequenceName : SEQ ID 574
SequenceDescription :

Sequence
--------
<213> OrganismName : Shigella flexneri 2a str. 2457T
<400> PreSequenceString :

```
atgtttaaag gacaaaaaac attggccgca ctggccgtat ctctgctgtt cacagcacct      60
gtttatgcgg ctgatgaagg atccggtgaa attcacttta aggtgaagt tattgaagca       120
ccgtgtgaaa tacatcagga tgatattgat aaagaggttg aactcggtca ggtgaccacc     180
agccacatta atcagtcaca tcacagcgat gccgttgctg tcgacctgct cttagtcaac     240
tgtgatctgg aaaactccag caacggttcc ggtggcaaga tttctaaagt tgcagtcacc     300
tttgatagct cagcgaaaac caccggcgca gatccgattc tcaacaacac cagcacgggt     360
gaagccaccg gcgtcggcgt acgtttaatg aataaagatc aaagcaatat cgttttaggc     420
accgctactc cagatatcga cctggctccg acctccagcg aacaaacgct gaatttcttt     480
gcctggatgg aacaaattga tcaggctaca cctgtaacgc caggcgcagt tacggcgaat     540
gcaacctacg tgctggatta taaataa                                         567
```

<212> Type : DNA
<211> Length : 567
SequenceName : SEQ ID 575
SequenceDescription :
Sequence
--------
<213> OrganismName : Shigella flexneri 2a str. 2457T
<400> PreSequenceString :

```
atgagcgcgg gaagcccaaa attcaccgtt cgccgcattg cggctttgtc actggtttcg      60
ctatggctgg caggctgttc tgacacttca aatccaccgg caccggtcag ctccgttaat     120
ggcaatgcgc ctgcaaatac taattctggt atgttgatta cgccgccgcc gaaaatgggg     180
acgacgtcta cagcgcagca accgcaaatt cagccggtac agcagccaca aattcaggct     240
actcaacaac cgcaaatcca gccagtgcag ccagtagctc agcagccggt acagatggaa     300
aacggacgca tcgtctataa ccgtcagtat gggaacattc cgaaaggcag ttatagcggc     360
agtacctata ccgtgaaaaa aggcgacaca cttttctata tcgcctggat tactggcaac     420
gatttccgtg accttgctca gcgcaacaat attcaggcac catacgcgct gaacgttggt     480
cagaccttgc aggtgggtaa tgcttccggt acgccaatca ctggcggaaa tgccattacc     540
caggccgacg cagcagagca aggagttgtg atcaagcctg cacaaaattc caccgttgct     600
gttgcgtcgc aaccgacaat tacgtattct gagtcttcgg gtgaacagag tgctaacaaa     660
atgttgccga acaacaagcc aactgcgacc acggtcacag cgcctgtaac ggtaccaaca     720
gcaagcacaa ccgagccgac tgtcagcagt acatcaacca gtacgcctat ctccacctgg     780
cgctggccga ctgagggcaa agtgatcgaa acctttggcg cttctgaggg gggcaacaag     840
gggattgata tcgcaggcag caaaggacag gcaattatcg cgaccgcaga tggccgcgtt     900
gtttatgctg gtaacgcgct gcgcggctac ggtaatctga ttatcatcaa acataatgat     960
gattacctga gtgcctacgc ccataacgac acaatgctgg tccgggaaca acaagaagtg    1020
aaggcggggc aaaaaatagc aaccatgggt agcaccggaa ccagttcaac acgcttgcat    1080
tttgaaattc gttacaaggg gaaatccgta aacccgctgc gttatttgcc gcagcgataa    1140
```

<212> Type : DNA
<211> Length : 1140
SequenceName : SEQ ID 576
SequenceDescription :
Sequence
--------
<213> OrganismName : Shigella flexneri 2a str. 2457T
<400> PreSequenceString :

```
gtgattaaat ttctctctgc attaattctt ctactggtta cgacggcggc tcaggctgag      60
cgtattcgcg atctcaccag tgttcagggg gtaaggcaaa actcactgat tggctatggc     120
```

```
ttggtagtgg ggctggatgg cactggtgac cagacaaccc agacgccgtt taccacacaa     180
acgcttaata acatgctctc acagctggga attaccgttc cgacgggcac caatatgcag     240
ctaaaaaacg tcgctgcggt aatggtgaca gcgtcacttc ctccgtttgg acgtcagggg     300
caaaccatcg atgtggtggt ttcttccatg ggtaatgcca aaagcttgcg tggaggtacg     360
ttgttgatga caccgcttaa gggcgttgac agtcaggtgt atgcgctggc gcagggcaat     420
attctggttg gcggcgcagg agcctccgca ggcggtagca gtgttcaggt taaccaactg     480
aacggtggac ggatcaccaa tggtgcggtt attgaacgtg aattgcccag ccagtttggc     540
gtcgggaata cccttaattt gcaacttaac gacgaagatt tcagcatggc gcagcaaatc     600
gctgacacca tcaaccgcgt gcgtggatat ggcagcgcca ccgcgttaga tgcgcggact     660
attcaggtgc gcgtaccaag tggcaacagt tcccaggtcc gtttccttgc cgatatccag     720
aatatgcagg ttaatgtcac cccgcaggac gctaaagtag tgattaactc gcgcaccggt     780
tcggtggtga tgaatcgcga agtgaccctc gacagctgcg cggtagcgca gggaaatctc     840
tcagtaacag ttaatcgtca ggccaatgtc agccagccag atacaccgtt tggtggtgga     900
cagactgttg ttactccaca aacgcagatc gatttacgcc agagcggcgg ttcgctgcaa     960
agcgtacgtt ccagcgccag cctcaataac gtggtgcgtg cgctcaatgc gctgggcgct    1020
acgccgatgg atctgatgtc tattttgcaa tcaatgcaaa gtgcgggatg tctgcgggca    1080
aaactggaaa tcatctga                                                   1098
```

<212> Type : DNA
<211> Length : 1098
SequenceName : SEQ ID 577
SequenceDescription :
Sequence
--------
<213> OrganismName : Shigella flexneri 2a str. 2457T
<400> PreSequenceString :

```
atgaaacgtt caattattgc tgccgctgtc ttttcttctt tttttatgag cgctggagta      60
tttgctgcag acgttgatac cggaacatta accattaaag gaaatattgc agaatctccg     120
tgtaaattcg aagcgggtgg tgattcagta agtattaata tgccgactgt accaaccact     180
gtctttgaag gtaaagctaa atattctacc tatgatgatg cagtcggtgt aaccagcagc     240
atgttaaaaa ttagctgccc gaaagaagtt gctggtgtaa aactctcgtt gatcacaaac     300
gacaaaataa ccggtaacga taaggcgata gccagtagca acgataccgt aggtgataac     360
agcgatgtcc tagatgtttc tgcacctttt aacattgaga gttataaaac agcggaaggt     420
caatatgcta ttccgtttaa agcaaaatac ctgaaactga cagataactc agtgcaatca     480
ggtgatgtgt tatcttctct ggttatgcgt gtggcgcagg attaa                     525
```

<212> Type : DNA
<211> Length : 525
SequenceName : SEQ ID 578
SequenceDescription :
Sequence
--------
<213> OrganismName : Shigella flexneri 2a str. 2457T
<400> PreSequenceString :

```
atggcggttc aaaagaatgt tatcaaaggc atactggcag gtacgtttgc gctaatgctg      60
agcggttgtg tcactgtgcc ggacgccatt aaaggcagca gtaccacgcc gcaacaagat     120
ttagttcggg tgatgagtgc gccgcagctg tacgttggtc aggaggcacg ctttggtggc     180
aaagtggttg cggtacaaaa ccagcaaggg aaaacccgcc tggaaattgc taccgtaccg     240
ctggacagcg gagctaggcc gacgctggga gaaccttctc gcggtcgcat ttatgccgat     300
gtgaacggtt ttctggaccc ggtggatttc cgtggacaac tggttacggt agtcgggcca     360
atcaccggtg cggttgacgg caaaatcggc aatacgccct ataaatttat ggtgatgcaa     420
gtaacgggtt acaaacgttg gcatttaacc cagcaggtga ttatgccgcc tcagccgatt     480
gatccatggt tttatggcgg tcgtggctgg ccctatggct acggcggatg ggctggtat      540
aatcccggcc ccgcgagagt acaaacagtt gtaaccgaat aa                        582
```

<212> Type : DNA
<211> Length : 582
SequenceName : SEQ ID 579
SequenceDescription :
Sequence
--------

<213> OrganismName : Shigella flexneri 2a str. 2457T
<400> PreSequenceString :

```
atgagaaaca aacctttttta tcttctgtgc gctttttttgt ggctggcggt aagtcgcgtt     60
ttggctgcgg atagcacgat tactatccgc ggctatgtca gagataacgg ctgtagtgtg    120
gccgctgaat caaccaattt tactgttgat ctgatggaaa acgcggcgaa gcaatttaac    180
aacattggcg cgacgactcc tgtcgttcca tttcgtattt tgctgtcacc ctgtggtaat    240
```

```
gccgtttctg ccgtaaaagt tgggtttacc ggcgttgcag atagccacaa tgccaacctg    300
cttgcacttg aaaatacggt gtcagcggct gcgggactgg gaatacagct tctgaatgag    360
cagcaaaatc aaatacccct taatgctcca tcgtctgcga tttcgtggac gaccctgacg    420
ccgggtaaac caaatacgct gaatttttac gcccggctaa tggcgacaca ggtgcctgtc    480
actgcggggc atatcaatgc tacggctacc ttcactcttg aatatcagta a             531
```

<212> Type : DNA
<211> Length : 531
SequenceName : SEQ ID 580
SequenceDescription :
Sequence
--------
<213> OrganismName : Shigella flexneri 2a str. 2457T
<400> PreSequenceString :

```
atgaaaaagt taacagtggc ggctttggca gtaacaactc ttctttctgg cagtgccttt     60
gcgcatgaag caggcgaatt ttttatgcgt gcaggttctg caaccgtacg tccaacagaa    120
ggtgctggtg gtacgttagg aagtctgggt ggattcagcg tgaccaataa cactcaactg    180
ggcttgacgt ttacttatat ggcgaccgac aacattggtg tggaattact ggcagcgacg    240
ccgttccgcc ataaaatcgg cacccgggcg accggcgata ttgcaaccgt tcaccatctg    300
ccgccaacac tgatggcgca gtggtatttt ggtgatgcca gcagcaaatt ccgtccttac    360
gttggggccg gtattaacta caccaccttc tttgataatg gatttaacga tcatggcaaa    420
gaggcggggc tttccgatct cagtctgaaa gattcctggg gagctgccgg gcaggtgggg    480
gttgattatc tgattaaccg tgactggttg gttaacatgt cagtgtggta catggatatc    540
gataccaccg ccaattataa gctaggcggt gcacagcaac acgatagcgt acgcctcgat    600
ccgtgggtgt ttatgttctc agcaggatat cgtttttaa                           639
```

<212> Type : DNA
<211> Length : 639
SequenceName : SEQ ID 581
SequenceDescription :
Sequence
--------
<213> OrganismName : Shigella flexneri 2a str. 2457T
<400> PreSequenceString :

```
ttgtttttta agcgaggaaa gattttgagt gcgggacgcc tgaataaaaa atctctgggt     60
atcgtgatgt ttttatcggt tggactgcta ttggcgggct gttcgggtag caaatcatcc    120
gatacaggaa cgtattccgg ctccgtttac accgtgaaac gggggatac gctatatcgt    180
atttcgcgca ccacgggaac cagcgtaaaa gagctggcgc gactgaacgg catttccccc    240
ccttacacca ttgaagttgg tcagaaacta aaactgggtg gggcgaaaag tagcagtagt    300
acacgtaaat caaccgccaa atcaacgact aaaaccgcat cggttacacc gtcatcagcg    360
gtaccgaaat cttcctggcc gccagtaggg caacgttgtt ggttatggcc aacgacaggg    420
aaagttatca tgccgtattc gacagcagat ggcggcaata aagggattga tatctcagct    480
ccacggggta cacctattta cgccgcgggt gcaggaaagg tggtgtatgt gggcaaccag    540
ctgcgtggct acggtaatct catcatgatt aaacacagtg aagattacat tacggcttac    600
gcccataatg acacgatgct ggtaaataat gggcaaagcg tgaaggctgg gcaaaaaatc    660
gccactatgg ggagcacgga tgcggcatct gttcgcctgc atttccagat tcgttaccgt    720
gcaacggcaa ttgatccgct acgttacttg ccgccgcagg gcagcaagcc aaaatgctga    780
```

<212> Type : DNA
<211> Length : 780
SequenceName : SEQ ID 582
SequenceDescription :
Sequence
--------
<213> OrganismName : Shigella flexneri 2a str. 2457T
<400> PreSequenceString :

```
atggcacaag tcattaatac caacagcctc tcgctgatca ctcaaaataa tatcaacaag      60
aaccagtctg cgctgtcgag ttctatcgag cgtctgtctt ctggcttgcg tattaacagc     120
gcgaaggatg acgccgcggg tcaggcgatt gctaaccgtt ttacttctaa cattaaaggc     180
ctgactcagg ctgcacgtaa cgccaacgac ggtatttctg ttgcacagac cactgaaggc     240
gcgctgtccg aaatcaacaa caacttacag cgtatccgtg agctgacggt tcaggcttct     300
accgggacta actctgattc ggatctggac tccattcagg acgaaatcaa atcccgtctc     360
gacgaaattg accgcgtatc cggtcagacc cagttcaacg gcgtgaacgt actggcaaaa     420
gacggttcga tgaaaattca ggttggtgcg aatgacggcc agactatcac tattgatctg     480
aagaaaattg actctgatac gctggggctg aatgggttta atgtgaatgg tggcggggct     540


gtggctaata ctgctgcatc taaagctgac ttggtagctg ctaatgcaac agtggtcggc     600
aacaaatata ctgtgagtgc gggttacgat gctgctaaag cgtctgattt gctggctgga     660
gttagtgatg gtgatactgt tcaggcaacc attaataacg gcttcggaac ggcggctagt     720
gcaacgaatt acaagtatga cagtgcaagt aagtcatact cttttgatac cacaacggct     780
tcagctgccg atgttcagaa atatttgacc ccgggagttg gtgataccgc taagggcact     840
attactatcg atggttctgc acaggatgtt cagatcagca gtgatggtaa aattacggca     900
agcaatggag ataaactcta cattgataca actgggcgct taacgaaaaa cggctctggt     960
gcttctttga ctgaggctag tctgtccaca cttgcagcca ataataccaa agcgacaacc    1020
attgacattg gcggtacctc tatctccttt accggtaata gtactacgcc ggacactatt    1080
acttattcag taacaggtgc aaaagttgat caggcagctt tcgataaagc tgtatcaacc    1140
tcgggaaaca atgttgattt cactactgca ggttatagcg tcaacggcac aactggcgct    1200
gtaacaaaag gtgttgattc ggtttatgtt gataacaacg aggcgttgac cacatctgat    1260
actgtagatt tttacctaca ggatgatggt tcagtgacta acggcagcgg taaggcagtt    1320
tataaagatg ctgatggtaa attgacgaca gatgctgaaa caaaagcagc tacgacagcc    1380
gatcccctga aagctctgga tgaagccatc agctccatcg acaaattccg ctcctccctg    1440
ggtgcggtgc aaaaccgttt ggattccgca gtcaccaacc tgaacaacac cactaccaac    1500
ctgtctgaag cgcagtcccg tattcaggac gccgactatg cgaccgaagt gtccaacatg    1560
tcgaaagcgc agattatcca gcaggccggt aactccgtgc tggcaaaagc caaccaggta    1620
ccgcagcagg ttctgtctct gctgcagggt taa                                 1653
```

<212> Type : DNA
<211> Length : 1653
SequenceName : SEQ ID 583
SequenceDescription :
Sequence
--------
<213> OrganismName : Shigella flexneri 2a str. 2457T
<400> PreSequenceString :

```
atgaaaaaaa ttgcatgtct ttcagcactg gccgcagttc tggctttcac cgcaggtact      60
tccgtagctg cgacttctac cgtaactggc ggttacgcac agagcgacgc tcagggccaa     120
atgaacaaaa tgggcggttt caacctgaaa taccgctatg aagaagacaa cagcccgctg     180
ggtgtgatcg gttctttcac ttacaccgag aaaagccgta ctgcaagctc tggtgactac     240
aacaaaaacc agtactacgg catcactgct ggtccggctt accgcattaa cgactgggca     300
agcatctacg gtgtagtggg tgtgggttat ggtaaattcc agaccactga atacccgacc     360
tacaaacacg acaccagcga ctacggtttc tcctacggtg ctggtctgca gttcaacccg     420
atggaaaacg ttgctctgga cttctcttac gagcagagcc gtattcgtag cgttgacgta     480
ggcacctgga ttgccggtgt tggttaccgc ttctaa                              516
```

<212> Type : DNA
<211> Length : 516
SequenceName : SEQ ID 584
SequenceDescription :
Sequence

--------
<213> OrganismName : Shigella flexneri 2a str. 2457T
<400> PreSequenceString :

```
atgaagcgta atattatagg cggtgcattc actctggcat ctctaatgct ggccgggcat      60
gcactggcag aagatggtgt tgttaacttc gtcggtgaaa ttgtcgacac tacttgtgaa     120
gttacctccg atacagccga tcaaattgtc ccactgggta aagtcagtaa aaatgcattt     180
tcaggtgtag gtagtctggc gtcgccacag aagttcagta ttaaacttga aaattgcccg     240
gcaacgtaca ctcaagcagc cgttcgtttt gatggtacag aagcgcctgg cggcgacggc     300
gacctgaaag tgggtacgcc gcttacagca ggcaaccctg gtgattttac cggtacagga     360
caagcgattg cggcaaccgg cgttggtatt cgtattttta accagtccga taattcgcag     420
gttaaacttt ataacgactc tgcttatacc gctatcgatg ctgaaggcaa ggctgaaatg     480
aagtttattg cacgctatgt ggcaaccaat gcgaccgtaa cggctggtac ggcgaacgct     540
gattcacaat ttactgtcga atataagaaa taa                                   573
```

<212> Type : DNA
<211> Length : 573
SequenceName : SEQ ID 585
SequenceDescription :
Sequence
--------
<213> OrganismName : Shigella flexneri 2a str. 2457T
<400> PreSequenceString :

```
atgaaaaaga gcactctggc attagtggtg atgggcattg tggcatctgc atctgtacag      60
gctgcagaaa tatataataa agacggtaat aaactggatg tctatggcaa agttaaagcc     120

atgcattata tgagtgataa cgccagtaaa gatggcgacc agagttatat ccgttttggt     180
tttaaaggtg aaactcaaat taacgatcaa ctgactggtt atggtcggtg ggaagcggag     240
tttgccggaa ataaagcgga gagtgatact gcacagcaaa aaacgcgtct cgcttttgcc     300
gggttgaaat ataaagattt gggttctttc gattatggtc gtaacctggg cgcgttgtat     360
gacgtggaag cctggaccga tatgttcccg gaatttggtg gcgattcctc ggcgcagacc     420
gacaacttta tgaccaaacg cgccagcggt ctggcgacgt atcggaacac cgacttcttc     480
ggcgttatcg atggcctgaa cttaaccctg caatatcaag ggaaaaacga aaaccgcgac     540
gttaaaaagc aaaacggcga tggcttcggc acgtcattga catatgactt tggcggcagc     600
gatttcgcca ttagtggggc ctataccaac tcagatcgca ccaacgagca gaacctgcaa     660
agccgtggca caggcaagcg tgcagaagca tgggcaacag gtctgaaata cgatgccaat     720
aatatttatc tggcaacttt ctattctgaa acacgcaaaa tgacgccaat aactggcggc     780
tttgccaata agacacagaa ctttgaagcg gtcgctcaat accagtttga ctttggtctg     840
cgtccatcgc tgggttatgt cttatcgaaa gggaaagata ttgagggcat cggtgatgaa     900
gatctggtca attatatcga cgtcggtgct acgtattatt tcaacaaaaa tatgtcagcg     960
tttgttgatt ataaaatcaa ccaactggat agcgataata agctgaatat taataatgat    1020
gatactgtcg cggttgggat gacgtatcag ttttaa                              1056
```

<212> Type : DNA
<211> Length : 1056
SequenceName : SEQ ID 586
SequenceDescription :
Sequence
--------
<213> OrganismName : Shigella flexneri 2a str. 2457T
<400> PreSequenceString :

```
atgcgtaagc agtggctcgg gatctgcatc gcggcaggaa tgctcgcggc atgtacaagc      60
gatgatggtc agcaacagac ggtaagtgta ccgcagcctg cggtatgtaa cggccccata     120
gttgaaatta gcggggcgga cccgcgtttc gaaccactga acgcgacggc aaatcaggat     180
taccagcgcg acggtaaaag ctacaaaatc gtgcaggatc cgtctcgatt tagccaggcg     240
ggactggcgg caatctatga tgccgaacca ggcagtaacc tgacggcctc tggcgaagct     300
ttcgatccga caaagctgac ggcggcccat ccaacgcttc cgatccccag ctacgccaga     360
atcactaacc tggctaacgg gcgaatgatc gtggtgcgca ttaatgatcg cggcccttac     420
ggcaacgacc gcgttatttc gctttctcgc gcggcagctg accgtcttaa cacgtcaaac     480
aacaccaaag ttcgtatcga tccgattatt gtcgcccagg atggttcgct ttctggtcct     540
ggtatggctt gtaccacagt cgccaaacag acttacgccc tgcctgcacc tcccgattta     600
agcggtggcg cgggaacaag ttcagtgtct ggcccgcagg gtgacattct tccggtcagt     660
aattcgacgc taaaaagcga agatccgacc ggcgcgccgg taaccagtag cggtttcctc     720
ggcgctccaa cgaccttagc gcctggtgta ctggaaggca gcgaaccgac gcctgcgcca     780
cagcccgttg ttacagcttc gtcgacaacg cctgcaacct cgcctgcaat ggtgacaccg     840
caagccgcct cgcaaagcgc cagcggcaac tttatggtgc aagtcggggc cgtaagcgat     900
caggctcgtg cgcaacagta ccaacagcaa ctgggacaga agttcggcgt ccccggtcgc     960
gtaactcaaa atggcgcggt ctggcggatc cagcttggcc cattcgccag caaagccgaa    1020
gccagtacct tgcagcaacg tttgcaaacc gaagcccaat tacagtcatt tattactacc    1080
gcgcagtag                                                            1089
```

<212> Type : DNA
<211> Length : 1089
SequenceName : SEQ ID 587
SequenceDescription :
Sequence
--------
<213> OrganismName : Shigella flexneri 2a str. 2457T
<400> PreSequenceString :

```
atgaagaaaa aaacgatata tcagtgcgtt attttgttct ttagccttct taacatccat      60
gtcgggatgg ctgggcctga acaagtcagt atgcatattt atgggaatgt ggtcgatcag     120
ggctgtgatg tcgccaccaa aagtgcatta caaaatattc atattggtga ttttaatatc     180
agtgattttc aggccgcgaa taccgtaagc actgctgctg atttgaatat tgatatcacc     240
ggttgtgccg ctggtattac tggcgcggac gtcctttttta gcggcgaggc tgacaccctt     300
gcgccgacac tgctcaaact aactgacaca ggcggaagcg gtggtatggc aacggggatt     360
gccgtgcaaa ttcttgatgc gcaaagtcag caagaaatcc cgctcaatca ggtccagcct     420
cttacgccct taaaagccgg ggataacaca ctcaaatatc aacttcgtta taagtccaca     480
aaggcgggag caacgggcgg taatgcgacg gcggttctct attttgatct ggtttaccag     540
tga                                                                  543
```

<212> Type : DNA
<211> Length : 543
SequenceName : SEQ ID 588
SequenceDescription :
Sequence
--------
<213> OrganismName : Shigella flexneri 2a str. 2457T
<400> PreSequenceString :

```
atgaaaaaca aattgttatt tatgatgtta acaatactgg gtgcgcctgg gattgcagcc      60
gcagcaggtt atgatttagc taattcagaa tataacttcg cggtaaatga attgagtaag     120
tcttcattta atcaggcagc cataattggt caggctggga ctaataatag tgctcagtta     180
cggcagggag gctcaaaact tttggcggtt gttgcgcaag aaggtagtag caatcgggca     240
aagattgacc agacaggaga ttataacctt gcatatattg atcaggcggg cagtgccaac     300
gatgccagta tttcgcaagg tgcttatggt aatactgcga tgattatcca gaaaggttct     360
ggtaataaag caaatattac acagtatggt actcaaaaaa cggcagttgt agtgcagaga     420
cagtcgcaaa tggcaattcg cgtgacacaa cgttaa                              456
```

<212> Type : DNA
<211> Length : 456
SequenceName : SEQ ID 589
SequenceDescription :
Sequence

--------
<213> OrganismName : Shigella flexneri 2a str. 2457T
<400> PreSequenceString :

```
gtgatgaaat ttaaaaaatg tcttctgcct gtggcaatgt tagcgtcatt cactctggca      60
ggatgccagt caaatgctga cgatcatgcc gccgatgttt atcaaaccga tcaactgaat     120
accaaacaag aaactaaaac cgttaatatt atttccattc ttcccgcaaa agttgccgta     180
gacaactccc aaaataaacg gaacgcacaa gccttcggcg cgcttattgg cgcagtcgct     240
ggcggtgtta tcggccacaa cgtcggttct ggcagcaatt ccggaacgac ggcaggggca     300
gttggcggcg gagctgtagg cgcggcagcg ggttctatgg tgaatgataa aaccttagtg     360
gaaggtgttt ctttaacata taaggaaggc accaaagtgt atacctccac ccaggagggt     420
aaagagtgcc agtttacgac aggtttagcc gttgttatta ccacaacgta taacgaaacg     480
cgtattcagc caaataccaa atgtcctgaa aagagctaa                            519
```

<212> Type : DNA
<211> Length : 519
SequenceName : SEQ ID 590
SequenceDescription :
Sequence
--------
<213> OrganismName : Shigella flexneri 2a str. 2457T
<400> PreSequenceString :

```
atgcaaacga aaaaaaatga aatttgggtg ggtatctttt tattagcagc actgctggcg      60
gcgctgtttg tttgcctgaa ggcggcgaac gtgacgtcca tacgtactga atcgacctac     120
acgctttatg cgacgttcga taacattggc ggcctgaaag cgcgctctcc ggtcagcatt     180
ggtggtgttg ttgtgggccg ggtggcggat attacgctgg acccgaaaac ctatctgccg     240
cgcgtaacgc tggaaattga acaacgttat aaccacattc ctgataccag ttcgctgagc     300
attcgtactt ccggcctgct gggagaacaa tatctggcat taaacgtcgg ttttgaagac     360
ccggaactgg ggactgctat cctgaaggat ggcgatacaa ttcaggacac caagtctgcg     420
atggtgctgg aagatctcat tggtcagttc ctttacggta gtaaaggcga tgacaataag     480
aatagtggcg atgcgccagc tgctgcgcca ggtaataatg aaaccactga acctgtgggt     540
acaacgaaat aa                                                         552
```

<212> Type : DNA
<211> Length : 552
SequenceName : SEQ ID 591
SequenceDescription :
Sequence
--------
<213> OrganismName : Shigella flexneri 2a str. 2457T
<400> PreSequenceString :

```
atggcaccgt tagccttttc tgcacaatca ttggctgaat cattaacggt ggaacaacgc      60
cttgagttat tagaaaaagc gttaagagaa acgcaaagcg aactcaaaaa gtataaagat     120
gaagagaaga aaaaatatac gccagcgacg gtgaatcgta gcgtaagtac gaatgatcaa     180
gggtatgccg ccaatccgtt cccgaccagt agtgccgcaa aacctgatgc tgtactggtc     240
aaaaatgaag agaaaaatgc cagtgagaca ggctcgattt attcttccat gactctgaaa     300
gatttcagta agtttgtgaa agatgaaatt ggctttagtt acaacggcta ttaccgttct     360
ggttggggga ccgcctctca tggttcacct aaatcatggg cgattggttc tctgggccgc     420
tttggtaacg aatactccgg ctggtttgat ttgcagttaa aacaacgtgt ctacaacgaa     480
```

```
aacggcaaac gggttgatgc cgttgtgatg atagatggta acgttggtca gcagtactct 540
accggctggt ttggcgataa tgccggtggc gagaacttta tgcagttctc cgatatgtac 600
gttaccacca aaggtttcct gccctttgcg ccagaggctg atttctgggt gggtaaacac 660
ggtgcgccga aaattgaaat ccagatgctg gactggaaaa cgcagcgtac tgatgctgca 720
gcgggtgtag gtctggaaaa ctggaaagtc ggtccgggta aaattgatat cgcgctggtt 780
cgcgaagatg tcgatgatta cgatcgcagc ctgcaaaaca aacagcagat taatacccat 840
actattgatt tacgctataa agatatcccg ttatgggata aagcgacctt aatggtgagt 900
ggtcgttatg tcacggcaaa cgaaagcgca tcggaaaaag ataatcagga taataacggg 960
tattatgact ggaaagatac ctggatgttc ggcacatctt taacgcagaa atttgataaa 1020
ggtggcttca acgaattctc cttcctggtt gcgaataact ctatcgccag gaactttggc 1080
cgttatgctg gcgcaagtcc atttaccacc tttaatggtc gttattatgg tgatcacacc 1140
ggcggaacag cagttcgtct gacctcgcag ggcgaagcct atatcggcga tcattttatt 1200
gtggctaacg cgattgttta ctccttcggt aacaatattt atagctacga aacaggcgca 1260
cactctgatt tcgaatctat tcgtgcggtt gttcgcccgg cctatatttg ggaccaatat 1320
aaccagacag gtgttgaact gggctatttc acccagcaaa acaaagatgc gaatagtaat 1380
aaatttaatg agtctggtta taaaaccacg ctcttccata cctttaaagt caataccagt 1440
atgttgacct cgcgtctgga aattcgtttc tacgccacgt atatcaaagc cctggaaaac 1500
gaactggatg gcttcacctt cgaagacaat aaagacgccc agtttgctgt cggtgcccag 1560
gctgaaatct ggtggtaa                                               1578
```

<212> Type : DNA
<211> Length : 1578
SequenceName : SEQ ID 592
SequenceDescription :
Sequence
--------
<213> OrganismName : Streptococcus mutans UA159
<400> PreSequenceString :

```
atgaaaaaaa gaattttatc agcagtttta gtgagtggtg taactcttag ttctgcgaca 60
acattatcag ctgtaaaagc tgatgacttt gatgcgcaga ttgcgtcaca agattctaaa 120
atcaacaact tgaccgcaca acagcaagca gcacaagcac aagttaatac gattcaagga 180
caagtaagtg ctttacagac acaacaagct gaattacaag ctgaaaatca aagacttgaa 240
gctcagtctg ctactttggg tcaacaaatt caaacacttt caagcaaaat tgttgcacgt 300
aatgaatctt tgaagcaaca agctcgtagt gctcaaaaaa gtaacgcagc taccagctat 360
attaatgcta tcattaattc aaaatcagtt tctgatgcta ttaatcgtgt ttcggctatt 420
cgtgaagttg tatctgctaa tgaaaaaatg cttcaacaac aagagcaaga taaagcagct 480
gttgagcaaa agcaacaaga aaatcaagca gcaattaata ctgttgcagc taatcaggag 540
acaattgctc aaaatacaaa tgctttaaat acacagcaag ctcaattaga agcagcacaa 600
ctaaacttgc aagctgaatt gactactgca caagatcaaa aagctacttt agttgctcaa 660
aaagcggcag cagaggaagc tgcacgccaa gcagcagcag cacaagcggc agcagaagct 720
aaggccgcag cagaagcgaa agctttacaa gaacaagcag cgcaagcaca agcagcagca 780
aataataata ctcaagctac agatgtttct gaccaacaag cagcggcagc tgataacact 840
caagcagcac aaacaggtga ttcaactgag cagtcagcag cacaagcagt aaataattct 900
gatcaagaaa gtactacagc aacagaagca caaccatcag cttctagtgc ttcgacagct 960
gctgtagcag ctaatacttc ttctgctaat acatatccag cagggcaatg tacttggggt 1020
gttaaatcat tagctccttg ggtaggaaac tactggggta atggtggaca atgggcagca 1080
agtgcagcag cggcaggata tagagttggt tctacacctt cagctggagc tgtagctgta 1140
tggaatgatg gcggttatgg acacgttgct tatgttacag gtgttcaagg tggccaaatt 1200
caagttcaag aagctaacta tgcaggtaac caatctattg gtaactaccg tggttggttt 1260
aatccaggta gtgtaagcta tatctatcca aactaa                           1296
```

<212> Type : DNA
<211> Length : 1296
SequenceName : SEQ ID 593
SequenceDescription :
Sequence
--------
<213> OrganismName : Streptococcus mutans UA159
<400> PreSequenceString :

```
atgaaagtca aaaaaactta cggttttcgt aaaagtaaaa ttagtaaaac actgtgtggt      60
gctgttctag gaacagtagc agcagtctct gtagcaggac aaaaggtttt tgccgatgaa     120
acgaccacta ctagtgatgt agatactaaa gtagttggaa cacaaactgg aaatccagcg     180
accaatttgc cagaggctca agggagtgcg agtaaggaag ctgaacaaag tcaaaaccaa     240
gctggagaga caaatggttc aataccagtt gaagtaccta aaactgatct tgatcaagca     300
gcaaaaagatg ctaagtctgc tggtgtcaat gttgtccaag atgccgatgt taataaagga     360
actgttaaaa cagctgaaga agcagtccaa aaagaaactg aaattaaaga agattacaca     420
aaacaagctg aggatattaa gaagacaaca gatcaatata aatcggatgt agctgctcat     480
```

```
gaggcagaag ttgctaaaat caaagctaaa aatcaggcaa ctaaagaaca gtatgaaaaa 540
gatatggcag ctcataaagc cgaggttgaa cgcattaatg ctgcaaatgc tgccagtaaa 600
acagcttatg aagctaaatt ggctcaatat caagcagatt tagcagccgt tcaaaaaacc 660
aatgctgcca atcaagcagc ctatcaaaaa gcccttgctg cttatcaggc tgaactgaaa 720
cgtgttcagg aagctaatgc agccgccaaa gccgcttatg atactgctgt agcagcaaat 780
aatgctaaaa atacagaaat tgccgctgcc aatgaagaaa ttagaaaacg caatgcaacg 840
gccaaagctg aatatgagac taagttagct caatatcaag ctgaactaaa gcgtgttcag 900
gaagctaatg ccgcaaacga agcagactat caagctaaat tgaccgccta tcaaacagag 960
cttgctcgcg ttcaaaaagc caatgcggat gctaaagcgg cctatgaagc agctgtagca 1020
gcaaataatg ccaaaaatgc ggcactcaca gctgaaaata ctgcaattaa gcaacgcaat 1080
gagaatgcta aggcgactta tgaagctgca ctcaagcaat atgaggccga tttggcagcg 1140
gtgaaaaaag ctaatgccgc aaacgaagca gactatcaag ctaaattgac cgcctatcaa 1200
acagagctcg ctcgcgttca aaaagccaat gcggatgcta aagcggccta tgaagcagct 1260
gtagcagcaa ataatgccgc aaatgcagcg ctcacagctg aaaatactgc aattaaaaag 1320
cgcaatgcgg atgctaaagc tgattacgaa gcaaaacttg ctaagtatca agcagatctt 1380
gccaaatatc aaaaagattt agcagactat ccagttaagt taaaggcata cgaagatgaa 1440
caagcttcta ttaaagctgc actggcagag cttgaaaaac ataaaaatga agacggaaac 1500
ttaacagaac catctgctca aaatttggtc tatgatcttg agccaaatgc gaacttatct 1560
ttgacaacag atgggaagtt ccttaaggct tctgctgtgg atgatgcttt tagcaaaagc 1620
acttcaaaag caaaatatga ccaaaaaatt cttcaattag atgatctaga tatcactaac 1680
ttagaacaat ctaatgatgt tgcttcttct atggagcttt atgggaattt tggtgataaa 1740
gctggctggt caacgacagt aagcaataac tcacaggtta aatggggatc ggtactttta 1800
gagcgcggtc aaagcgcaac agctacatac actaacctgc agaattctta ttacaatggt 1860
aaaaagattt ctaaaattgt ctacaagtat acagtggacc ctaagtccaa gtttcaaggt 1920
caaaaggttt ggttaggtat ttttaccgat ccaactttag gtgtttttgc ttccgcttat 1980
acaggtcaag ttgaaaaaaa cacttctatt tttattaaaa atgaattcac tttctatgac 2040
gaagatggaa aaccaattaa ttttgataat gcccttctat cagtagcttc tcttaaccgt 2100
gaaaataatt ctattgagat ggccaaagat tatacgggta aatttgtcaa aatctctgga 2160
tcatctatcg gtgaaaagaa tggcatgatt tatgctacag atactctcaa ctttaggcag 2220
ggtcaaggtg gtgctcgttg gaccatgtat accagagcta gcgaaccggg atctggctgg 2280
gatagttcag atgcgcctaa ctcttggtat ggtgctggtg ctatccgcat gtctggtcct 2340
aataacagtg tgactttggg tgctatctca tcaacacttg ttgtgcctgc tgatcctaca 2400
atggcaattg aaactggcaa aaaaccaaat atttggtatt ctttaaatgg taaaatccgt 2460
gcggttaatg ttcctaaagt taccaaggaa aaacccacac cgccggttaa accaacagct 2520
ccaactaaac caacttatga aacagaaaag ccattaaaac cggcaccagt agctccaaat 2580
tatgaaaagg agccaacacc gccgacaagg acaccggatc aagcagagcc aaacaaaccc 2640
acaccgccga cctatgaaac agaaaagccg ttggagccag cacctgttga gccaagctat 2700
gaagcagagc caacaccgcc gacaaggaca ccggatcagg cagagccaaa taaacccaca 2760
ccgccgacct atgaaacaga aaagccgttg gagccagcac ctgttgagcc aagctatgaa 2820
gcagagccaa cgccaccgac accaacacca gatcaaccag aaccaaacaa acctgttgag 2880
ccaacttatg aggttattcc aacaccgccg actgatcctg tttatcaaga tcttccaaca 2940
cctccatctg taccaactgt tcatttccat tactttaaac tagctgttca gccgcaggtt 3000
aacaaagaaa ttagaaacaa taacgatatt aatattgaca gaactttggt ggctaaacaa 3060
tctgttgtta agttccagct gaagacagca gatctccctg ctggacgtga tgaaacaact 3120
tcctttgtct tggtagatcc cctgccatct ggttatcaat ttaatcctga agctacaaaa 3180
gctgcaagcc ctggctttga tgtcacttat gataatgcaa ctaatacagt caccttcaag 3240
gcaactgcag caactttggc tacgtttaat gctgatttga ctaagtcagt ggcaacgatt 3300
tatccaacag tggtcggaca agttcttaat gatggcgcaa cttataagaa taatttcacg 3360
ctcacagtca atgatgctta tggcattaaa tccaatgttg ttcgggtgac aactcctggt 3420
aaaccaaatg atccagataa tccaaataat aattatatta aaccaactaa ggttaataaa 3480
aacgaaaatg gcgttgttat tgatggtaaa acagttcttg ccggttcaac gaattattat 3540
gagctaactt gggatttgga tcaatataaa aacgaccgct cttcagcaga taccattcaa 3600
aaaggatttt actatgtaga tgattatcca gaagaagcgc ttgaattgcg tcaggattta 3660
gtgaagatta cagatgctaa tggtaatgaa gttactggtg ttagtgtgga taattatact 3720
aatcttgaag cagcccctca agaaattaga gatgttcttt ctaaggcagg aattagacct 3780
aaaggtgctt tccaaatttt ccgtgccgat aatccaagag aattttatga tacttatgtc 3840
aaaactggaa ttgatttgaa gattgtatca ccaatggttg ttaaaaaaca aatgggacaa 3900
acaggcggca gttatgaaaa tcaagcttac caaattgact ttggtaatgg ttatgcatca 3960
aatatcatta tcaataatgt tcctaagatt aaccctaaga aagatgtgac cttaacactt 4020
gatccggctg atacaaataa tgttgatggt cagactattc cacttaatac agtctttaat 4080
taccgtttga ttggtggcat tatccctgca gatcactcag aagaactctt tgaatacaat 4140
ttttatgatg attatgatca aacaggagat cactatactg gtcagtataa agtttttgct 4200
aaggttgata tcacttttaa agacggttct attatcaagt caggtgctga gttaactcag 4260
tatacgacag cggaagttga taccgctaaa ggtgctatca caattaagtt caaggaagcc 4320
tttctgcgtt ctgtttcaat tgattcagcc ttccaagctg aaagttatat ccaaatgaaa 4380
cgtattgcgg ttggtacttt tgaaaatact tatattaata ctgtcaatgg ggtaacttac 4440
agttcaaata cagtgaaaac aactactcct gaggatccta cagaccctac tgatccgcaa 4500
```

```
gatccatcat caccgcggac ttcaactgta attaactaca aacctcaatc aactgcttat    4560
caaccaagct ctgttcaaga aacattacca aatacgggag taacaaacaa tgcttatatg    4620
cctttacttg gtattattgg cttagttact agttttagtt tgcttggctt aaaggctaag    4680
aaagattga                                                            4689
```

<212> Type : DNA
<211> Length : 4689
SequenceName : SEQ ID 594
SequenceDescription :
Sequence
--------
<213> OrganismName : Streptococcus mutans UA159
<400> PreSequenceString :

```
atgttaacgg aattaaaagc ggttttaaaa aagcctatgc tttggattac gatggtagga      60
gtagcccttg tccctgccct atataatatt atttttttga gttctatgtg ggatccttat     120
ggcaaagtat ctgatttacc tgttgcagtt gttaataaag ataaaacggc aacttatgaa     180
ggaaagaaga tgactatcgg taaagatatg actgataata tggtccgtaa taaaagtttg     240
gactatcatt ttgttgatag cgaaaaagct caaaagggac ttgaaaaagg tgattactat     300
atgatcatta ctttaccaga agatctttct caaaatgcgg ctagtgtttt aacagatgaa     360
cctaaaaagc taacgattcc ttaccaaacg tctaaaggac atagttttgt tgcctccaaa     420
atgagtgaaa ctgctgctaa gactttaaaa gagtctgtgt cgaaaaacat tacaagttct     480
tacaccaaat cactttttaa gaatatgtca accctaaaaa caggtcttgg cagtgcggct     540
aatgcaagtc aaaaaatagc gactggttca aaacagttag caaatggcag tcaagtgatg     600
actgataatc tgaatttact ttcaaattca agtcaatcat ttgctcaagg gactaatacc     660
ttatattcgg gcttaacagc ttatacggga ggtgtcggtc agctttctgc aggtttaaat     720
aatttaaaca atggtttgac agcttataca aatggagttg gtcagttagc aaatggcagc     780
agccaactga gcaatcaatc tcagaagctt ctaggaggtg ttgcgcaatt agcgaatggt     840
tctgcttcta ttcaacaatt ggttaatgct agcagtcagt tgaatcaagg acttattaag     900
ctgtcaacag caacaggtct ttctgaagaa caagttcaac agtttagctc attgattaat     960
cagttgggaa ctttgaatca aagtattcaa aattacagtg ataatgggac agcaacgact    1020
gcaaatagtc ctgatcttag tacgtattta tctgccatta caacagcagc tcaagcaatt    1080
gttaattcag gaaatacgtc tcagcaaaca acaactaatc agtccaatgc attggctgca    1140
gtccaagcta caggtgctta tcaaagatta tccgctgagg atcaatcaga gatcgctgca    1200
gctttggcca acactggtag ttcaacaaca actacaggtg ctgatgcaaa cgcagtgtca    1260
caagcccaag ctatcctaaa caatgtccaa agtattcaaa gtgccttatc tactttacag    1320
acaacaactg ctaatacacc aacaagccca tcagccagct tgactcaaat taaaaataca    1380
gctaattctg tattacctag tgcggcaact tccttgacaa ccttgtcaag tggtttgaca    1440
caagcaaaaa cagctcttga ttcgcaagta gtccctgtca gcacagccct tgctaatggt    1500
acggctcaat taggctctac tttttcaaca ggcgccaatt ccttgatgac aggagtaggc    1560
caatacacta atgcagttga tattcttaat gcaggagcta atactttggc tgctaaaaat    1620
aaccagttaa cagatggtac aagtcaattg gtaaatggtg ccaatcaatt aaatagcaac    1680
tctggacaat taacgaaagg gactgcacag ttagcaaatg gtgctaatca aatagagaca    1740
ggagctggca aattggctgc aggtggagaa agtttaacgg cgggcttgac aactttgtca    1800
agtggtagtg gtgaactgtc gaaagcattg tcaactgcta aaaataaatt atcattagta    1860
gcagttgaca atgataatgc taagactttg tcaagtcctg tcactatcaa gcatacagat    1920
aaagataatg tcaaaacaaa tggtgttgga atggctcctt atatgatgtc agcagcttta    1980
atggtaatgg caatttcaac caatactatc tttagagtag cactttctgg taagcaagct    2040
aaaaccctac gagaatggat agatcaaaag ttagcagtca atggcttgat tgctgttact    2100
ggagctatta ttctctattt tggtgttcat attattggtt tatcagctaa ttttgaacta    2160
aaaactttag gattaattat tcttaccagt atcactttta tggtcttagt gacaactttg    2220
gtaacttggc atgataaatt tggctctttt gctgccttaa ttttactttt actacaatta    2280
ggttccagtg caggaaccta tcccttagcc gttacagata agtttttcca agttgtcaat    2340
ccttatttac caatgagtta ttcggtttct ggtttacgag aaaccatttc tatggctggt    2400
acaattggta atcaactact agcgctgagt ttatttttcc ttacttttgc tgctttagga    2460
ttgttaatcg ctcgaaggag gattaggtct gtcaaagtag cctaa                    2505
```

<212> Type : DNA
<211> Length : 2505
SequenceName : SEQ ID 595
SequenceDescription :
Sequence
--------
<213> OrganismName : Streptococcus mutans UA159
<400> PreSequenceString :

```
atggtatccc aaaaaaataa atctaaaaag ggtcaatcta aaacgtttac cttaatttca     60
aatagaatta atctcctatt ttttttgatt gtcgctttgt ttactgtttt gcttttgagg    120
ttagctcaga tgcagcttta tgatgcaaag ttttacaaat ctaaattgac agagtcaaca    180

acatatacta taaaaacctc cagccctcgc ggacaaattt atgacgctaa aggggtggct    240
ttagttgaaa acgaggttaa agaggttgtt gcctttacaa gaagcaatac catgactgcc    300
aaggatatta aagcaaatgc aaaaaagtta gcagatatgg tgactttaac tgaatctaag    360
gtaaccaaac gtcagaaaaa agattactat ctggcagatc caaaaaatta tcaaaaaatc    420
gttaaaaaat taccaaataa taaaaaatat gataactttg gaaataacct aacggaatcg    480
aagatctatg caaatgctgt taaagcagtt ccaaacagtg ctattgatta ttctgaagat    540
gagaaaaaaa tcattcatat tttcagccag atgaatgcaa cctctgtttt taatacagct    600
tcgttaacaa caggagattt aacagctgag caaattgcag tattggctac aagcaaatca    660
gatttaaagg gaatttctgt taagactgac tgggaacgta aaacagataa aaattccatt    720
acttctatta tcggtaaagt ttccagtcaa aaaaccggtc tgcctgctga agaagccaat    780
aactatgtta aaaaaggtta ttctctgaat gaccgcgttg gaacatctta tcttgaaaag    840
caatatgaaa acgatttgca aggtagtcgt actgttcaag caatcaaggt taacaaagaa    900
ggtaaaatta tcagtgataa gaccactgcc aaagggacta aaggaaaaaa tctgaagttg    960
acccttgatc ttgaatttca aaaaggtgtt gaacaaattc ttaaccaata ttttaattct   1020
gaattagcat ctggaaatac caagtattct gaaggcgttt atgctgttgt tcttaacccg   1080
aacacaggtg cagttctttc tatggctggt ttggaacacg accttaaaac gggcgaagta   1140
tcttctaatg ctcttggagc ggttactgaa gtctttactc cgggttccgt tgttaaagga   1200
gcgactttga cagctggttg ggaaaacggt gtcttatcag gcaatcaagt actcaatgac   1260
cagcctattc aatttgcggg ttcaagccct attaactctt ggtttaccaa tggatcaact   1320
cctcttacag caagtcaatc cttagagtat tcttctaata cttatatggt tcaattggct   1380
ttaaaattaa tgggacaaga ttatcatagt ggtatgacct tatcaacaga tggctataag   1440
gaagctatgg agaaattaag agctacttat gctcaatatg gtttgggtgt ctcaacagga   1500
attgaccttc cgggagagtc aaagggctat acgccagaac attatgatcc ttctaatgtt   1560
ttaacagaat catttgggca gtttgataac tatacggcta tgcagctggc acaatatgct   1620
gcagctgtcg caaatggtgg taaacgtatt gctccccatt tggttgaagg tatctatgat   1680
aataataaaa caggcggttt aggaaatctt gttcaatcta ttgacaccaa ggttttaaat   1740
aatgtttcta tctctagcga tgacatgggg attatcaagg aaggtttcta taatgttgtt   1800
aacggtggta gttatgcaac agggaagact cttgcaaaag gggcaagtgt tcccatttcg   1860
gcaaaaacag ggacagccga agcttacgtg acaggagatg atggtaaatc tgtttataca   1920
tccaatttaa acgttgttgc ttatgcacca agcagcaatc ctcaaattgc tgtagctgtt   1980
gtcttgccac atgagacaga ccttcatgga accactagtc atgctattac gagagatatc   2040
atcaatcttt atcaaaagat gtatccaatg aatcagtga                           2079
```

<212> Type : DNA

<211> Length : 2079

SequenceName : SEQ ID 596

SequenceDescription :

Sequence

--------

<213> OrganismName : Streptococcus mutans UA159

<400> PreSequenceString :

```
atgacagttc taaaatatgg actaggtatt ctcttaagcg ctattatttt agccattata      60
attggaggtc ttctgtttac ctattatgtc agcagtactc ctaaactatc agaagctaaa     120
cttaaagcta ctaattctag tttggtttat gatagcaata ataatctgat tgctgattta     180
ggtgctgaaa agcgcgaaag tatttcttca gacagtattc caatgaagtt agtaaatgcc     240
gttacctcta ttgaagatca ccgtttcttt aaacatcgtg gtgtcgacat ttatcgtatt     300
attggtgcag cttggagtaa tttacttcat aaatcaactc aagggggatc cactcttgat     360
cagcagctta tcaagctggc ctatttctct actaaagagt ctgatcagac cttaaaacgt     420
aaagctcaag aagtttggct gtctctacaa atggagaaaa aatacacgaa agaagagatt     480
ctaacttttt atgtcaataa ggtttacatg ggtaatggga attacggaat gcgcactgct     540
gcaaagtctt attatggcaa ggatcttaaa gacttatcaa ttgcccagct agcgacactc     600
gcaggtattc cgcaagcacc gacacaatat gatccttacg ctcagccaaa ggcagctaca     660
agcagacgta ataccgtttt gtcacagatg tataaacata aaaaaattac aaaacgagaa     720
tatgatgctg cagtagcaac accaatttct gatggcctgc aagaactgaa acgctcctct     780
agttatccaa aatatatgga taattatctg aaacaggtta tttcagaggt gaaaaaacgt     840
actggtcaag atatcttttc agcaggcatg aaggtttata caaatgttaa tgccgatgca     900
cagcaatatc tctggaacat ttataataca gatgaatata ttgcttatcc tgatgataat     960
ttccaagttg cttctactgt tatggatgtt actaatggta aagttattgc acagcttggc    1020
ggacgccatc aagataccaa tgtttctttt ggtaccaatc aggctgtctt aactgatcgt    1080
gactggggat caaccatgaa acctatttca gcatatggcc ctgctcttga aagcgaagct    1140
tttacgacaa ctgcacagat gctaaatgac tcggtctatt attatccagg tacaacaaca    1200
caagtctatg actgggatca tcgttataat ggttggatga ctatccaaac ggctatccaa    1260
caatctcgta atgtccctgc tgtcagagct attgatgccg ctggattaga tactgccaaa    1320
ggtttcttaa gcggtctcgg tattgattat cctgagatgc gttattcaaa cgccatttca    1380
agtaatacaa gtagttcaga acaaaagtat ggtgccagca gtgaaaaaat ggccgccgct    1440
tatgctgctt tttctaatgg tggaacttat tatgaaccac aatacgtcaa taaaatagaa    1500
tttaaggatg gaacatcaga gacctatgat gctaaaggca atcgtgcgat gaaagaaacg    1560

acagcctaca tgatgacaga tatgttaaaa acagtattaa catatggtac tggtactgag    1620
gctgctattc ctggtcttta tcaagcaggt aaaacaggaa catccaacta tgatgacaat    1680
gaattggtag agatgtctga aaaacttggt attaatcctt atggacttgg tactattgct    1740
ccagatgaaa actttgttgg ttatacacct cagtattcaa tggctgtttg gacaggatat    1800
aaaaatcgct taatgcctgt ttacggagac agtatgaaaa ttgctgcgca agtctatcgt    1860
actatgatgg cttatctttc tagctcaggt aattctgatt ggaccatgcc tgacggtctc    1920
tatcgcagcg gtggttatct ttacctaaat ggttcaagtg ggtcaaatag taggtatggt    1980
gcagctcctg caacttcatc gtcatcttct tcatcatctt cttctgattc aaacaataac    2040'
gatcaaaata ataatcaaac tacagaagcg tctagtgact catcttcatc aagttctgat    2100
gctacgacat cttctaatcc ataa                                           2124
```

<212> Type : DNA
<211> Length : 2124
SequenceName : SEQ ID 597
SequenceDescription :
Sequence --------
<213> OrganismName : Streptococcus mutans UA159
<400> PreSequenceString :

```
atgaaatcga aaactgctaa aattactttg ctaagcagcc ttgctttggc ggcttttgga      60
gcaacgaatg tttttgcaga tgaagcatca actcaattaa attctgatac tgttgcagca     120
cctactgctg atacacaagc atcagaaccg gctgcaacag aaaaagaaca gtctcctgtt     180
gtagctgttg tcgaaagtca cacacaagga aatacaacaa cgacaacatc tcaagttact     240
tctaaagaat tggaagatgc taaggctaat gctaatcagg aaggtttaga agtcactgaa     300
actgaagctc aaaaacagcc ttcggtagaa gctgcagatg cagataacaa agcacaggca     360
caaacaatta atacagcggt agctgattat caaaaggcaa aagctgaatt tcctcaaaaa     420
caagaacaat ataataaaga ttttgaaaag tatcagtctg atgtcaagga gtatgaagct     480
caaaaggcag cttacgagca atataaaaaa gaagttgcac aaggtttggc atctgggcgt     540
gttgaaaaag cccaaggact tgtgtttatt aatgaacctg aggcaaaact ttctattgag     600
ggtgttaatc agtacctaac aaaagaagca cgtcaaaaac atgcaactga agatattctt     660
cagcaatata atactgataa ttatacagct tctgatttta cccaagcaaa tccatatgat     720
ccaaaagaag atacttggtt caaaatgaaa gtgggagatc agatttcagt tacctacgat     780
aatatcgtta attcaaaata taatgataaa aagattagta aggtaaagat taattatact     840
ctcaatagtt caacgaataa tgaaggcagt gcactggtca atttgttcca tgatccaact     900
aagacaattt tcattggtgc acagacatct aatgctggca gaaatgataa aatcagtgtg     960
acgatgcaaa ttatttttta tgatgaaaat ggcaatgaaa tcgatttaag cggcaataat    1020
gccattatga gtctctcatc gttaaaccat tggacgacta agtatggcga tcatgtggaa    1080
aaagtaaacc ttggggataa tgaattcgtt aaaataccgg gctcatctgt tgacttacat    1140
ggcaatgaaa tctattcggc taaggacaac caatataaag ctaatggtgc aacctttaat    1200
ggtgatggag cagatggctg ggatgctgtc aatgctgatg gaacgccacg tgctgcgacg    1260
gcttattatg gtgcaggtgc tatgacttac aagggagaac ccttcacctt tactgttggt    1320
ggtaatgatc aaaacttacc aacaaccatt tggtttgcga ctaattcagc tgtagctgtg    1380
cctaaagatc cgggagctaa accaacaccg ccagaaaaac cagagttgaa aaaacctact    1440
gtgacttggc ataaaaatct tgttgttgaa actaaaactg aggaagttcc tccagtgaca    1500
ccaccaacaa ctcctgatga accaacgcca gaaaagccaa aaacaccaga ggatcctcaa    1560
tcacctgtcg tagctaagtc agtaagcttt agaacggcaa gaaaaggaga aatgcgtgtt    1620
agagagcgtg attatcaacc gactcttcca catgctgggg ctgctaaaca aaatggttta    1680
gctactcttg gtgctatttc aactgcattt gctgcggcta ctttgattgc agctagaaaa    1740
aaagaaaact ag                                                        1752
```

<212> Type : DNA
<211> Length : 1752
SequenceName : SEQ ID 598
SequenceDescription :
Sequence
--------
<213> OrganismName : Streptococcus mutans UA159
<400> PreSequenceString :

```
atggaacaga agatttttag caaacgaaaa agtaagattg ctgggctttg tggagctatt      60
ttaacgacta cagttgttgc ccttgcgtca ggtactgtaa tcgaggctga tgagacaata     120
gaacagcctg tcgcagctga gactgtctcg caagctgatg ggacaatcc cgaacaaaca      180
acaagcgttc aacaagaaac tgctcctcaa caaacgaaaa cttctcaaag ctcagacgca     240
accgtagata gtgaagagtc agcaacttcc ccatctgatg aacagaccgt aagtcaaaat     300
gattcaaact catcatctca aattgatcaa acgatagctg atacgaatcg ctctgactct     360
gatcatattt caaaaacatc agccgctaca actgaagatc aagaagagaa agttaattct     420
gcaaaagcac aaactgctgc cgcaaccaac aatcaagaca ctcgttatag tgcgaaagat     480
gcttatggca attccaattt taacaagaca ttaactgaat ttggaaaaaa tgctaatgtt     540

gctgatgtaa cctataatgg cgtgagggat gaatatattg tagttaacga tcctagtgct     600
ccttacgttc ctaatgcaaa cgaaattgca aaatacttaa aggaatattt aacagaactc     660
cgcaacatca ataatattgc tattcctgtg ccttctgttg atcaggttat gcaaaaatac     720
gcacaagatc gggctaacga agaagccaat gaaaaaaacg gcttggatca tgatactaat     780
ttacctatcc ctaataattt aacttgggtt gccgaagatg gacatttgga tatggatagc     840
agcattcaat ccaaaagtca agaaggctat acacttgctt ctgataaagc aaccgcctac     900
tatctagcgc ttaactggtt ttctgactat tttaatattt acgatgaccc caacgatggc     960
ctcaaatcgt ttggacacgc tgtcagtatt ttgtcagacg ggggaactgg aatgggctta    1020
ggtcttgctt caggtcaaga taatgaaaag ggaatgtggt acgcacaatt ggaatttggt    1080
ggtaacgata acgaagataa taccaacgat ttttcatctt taaaaaacgg caagggagaa    1140
tgggtattat attataaagg aagtcctgtt aagtttcttc ctaacactac cttttggtat    1200
gtaaaaaaag gcacttcccc tgatgcagct tctactcctc acaacagtga taaaccttca    1260
ttccagtcat ctaaagatct tgaccctaat ttcaaggccg ataatagatt ccaagaagga    1320
aaggaagcct ctgttcatca ggctattcct gcaacattta aatctcatcg cgatgaagtt    1380
ggtaataaag accaaaattc tctttctgct caactacctg atacaggagt tcaaaaaaat    1440
aatcaattag ccttgatagc tttaggaaca ggcttgattt tactttccgg acttcttctt    1500
tcaaaaagaa aatccttaaa ataa                                           1524
```

<212> Type : DNA
<211> Length : 1524
SequenceName : SEQ ID 599
SequenceDescription :
Sequence
--------
<213> OrganismName : Streptococcus mutans UA159
<400> PreSequenceString :

```
atgacatttg aaaagcaaaa acactttagt ttacgtaaac taaaatttgg tttagtttca      60
gttgcgatca tagctttctt atttgctgta acaaagactg cagaagctga cgagacagtg     120
ataactgaac aaaggcaaac aagtaagatt aacgctagtt ctcaaaaagt ggagaatcag     180
acttcaaatc aggtggaagc aaaaacggat agtgcaaaca aggatcctca agaaaaaaca     240
ggaagtgttg caactgatgc cccttcaatg aattcagcta ataatatgag tcagtctgac     300
aaacaaaata ctgttaatga aatatcttca gatagtcagc aaacaaaaac agatgaacaa     360
actgatttac cgcaaaacag ctttaaacaa caatctgctc atgttaaaat gactactgaa     420
gcagagaaga ccccatcaca ttcgattaat acctttgtta atgatggtaa tggtaattgg     480
tattaccttg gtgctgatgg tagaaatgtt acaggcagtc atacgattgg tggcaagact     540
atgtattttg ctcaagatgg taagcaagtt aaaggtgctt ttgctcaaga ttcggatgga     600
aataaacatt attatgatag agatagtggt gagatgtgga ccaatcgctt tgtcaatgat     660
caaggcaatt ggtattatct taataatgat ggtgtccctg tcaccggtag tattactgtc     720
aatggtcagt ctctgtattt taattcggat ggtagtcagg ttaaagggaa ttttgttgaa     780
gaagatggat ctttgcgtta ttatgataaa aattctggag atttactgag aaagacaagc     840
cgaaccatta atggtgttaa ctaccaattt gataatgatg gaaatgcaag ggcgattgac     900
aaaattgagg ttgttaagac cagtcttgta gttgatagtt atgaatttgg tccttctgtt     960
tcaaagatta ttcttgagtt caatcataag gtaactcctg ctgttgttca tgctggtgca    1020
atggtaacaa ctgctggagt tcaaagaaaa attcttaatt cttatgtctc taatgcttca    1080
ggacatgtgg tttactttga tagtagccat tatgtgacac ttgaattaga tattccttat    1140
gatccaaatg atagcagccg aaatgcatca ccttttattt ttgactcagc agcctttcgt    1200
aataactggg tcaacagtta tactgtcaaa gtagataatt tgcaggtgca agcagatggt    1260
tctaatagca gtcaaattat cagttcagag caagatgcta tcaataatcg tttcttgcct    1320
acaacggatc gtttctcaga acgtggtagt tatggtaatt ttaattatgc cgcctatcaa    1380
ccagaagcag ctattggcgg tgagaagaat ccattgattg tctggttgca tggtatagga    1440
gaagtaggca ctgatattaa tattccgctt ctagccagca atgtggctcg tttaacggaa    1500
gatcctattc aaagccattt cacttctaca ggtagtggtg gtcaaaaggg agcctatgtg    1560
ctagttcctc aaagttcaat tccttggtct caaaatcaaa cagctagctt aatggcgctc    1620
attaaagcct atgtagcaag ccatccagac atcgatagcc gacgcattta tttggcaggt    1680
gtttctaatg gtggcggtat gactctggat atgggagtcg cttatcctaa ctattttgca    1740
gccttagttc ctattgctgc ttcctatagt aatcaattaa cagataatca gattaccgct    1800
gctgctttga aagctctgaa aggtcaacca atgtggttga ttcatacacg aactgataaa    1860
acaatatctg cagatagtag tgttctacca ttctataaag agttacttca agctggcgca    1920
caaaataaat ggctttccta ttatgaaact aatgttggta aacatcactc tggagtcact    1980
tataacggtc actggtcttg gatttatttc ttgaatgatc aagtaactgg cactcaaaat    2040
actgataacg ccaagaattg gtctggactt tctggcatgg ttgcgaccaa tccaacctat    2100
ggtggtgatg ctaaggctac tgtcaatggc agaacttata gtaatgtctt tgattggcta    2160
aatggtcagc gaagaaggta a                                              2181
```

<212> Type : DNA
<211> Length : 2181
SequenceName : SEQ ID 600
SequenceDescription :
Sequence
--------
<213> OrganismName : Streptococcus mutans UA159
<400> PreSequenceString :

```
atgaaaattt ttataaaaaa acaccaacaa agtattcttt actatagtct tagttttctg 60
ctaccaagtt ttataatgtt tctcgttcta ttctccaaaa atatttattg ggggagtagc 120
acaactattt tagctagtga tggttttcat caatatgtga tttttgatgc tctttttcgt 180
aatattctcc atggaacgga tagtttgttt tactctttta aggctgggct tggttttaat 240
atttttgctc tgacaagtta ttacttggga agttttttaa cacctttttac ttactttttt 300
aatgtaaaaa atatggcaga tgcttttttat ctcttcactt taatcaaatt tggtctaata 360
ggtttatctg ctttttacag tcttgggcaa atttatacta aaatctctaa atcactcgtt 420
ttgatgctgt caacatctta tgctttaatg agctttacta gcagtcagtt agaattaaac 480
aattggttag atgtttttat cctgctacca cttattatgc ttggtttaca gcgtttagta 540
gaaaaaaggg ggatttttct ttattttcta actcttactt gtttatttat tcaaaattac 600
tattttggtt tcatgacagc tattttctta actctttggt tttttacgca agtctcgtgg 660
gatattagaa acagaatgaa acgattaagt gattttgtgc tcgtatcaat ctttgcaacg 720
ctgacaagtg cttttatgct gcttccaaca tttcttgatt taaagagcca tggtgaagta 780
ttaacagaac aaattagtct attttcatca gacatttggt atttcgattt ttttgctaaa 840
agtcttcttg gtagttatga tacgacaaaa tatggctcta ttccaacgat ttatatcggt 900
ttacttccct tgatttttgc cattactttt ttctttgtta aatctataaa atggcaagtt 960
aaagtagctt attttctttt attggctatt attattgcaa gttttatctt tcaaccactt 1020
gatttatttt ggcaaggaat gcattcacct aatatgtttt tgcatcgtta ttcttgggct 1080
ttctccttag ttattgtcat aatggcagct gaaacgttaa ctcggataaa ggatataaaa 1140
ttgaaaaatt tttatccagc ctttaccttc ttgggagtag gacttttagc aactttttta 1200
ttcaaggact attataatta tctgacacaa gttaatttta tattaacaac tatcttttta 1260
gttagttatt ttattattct ttttactttt tttaatcaat tagtttctta taaagttatt 1320
atttccttta cacttatctt tacaagtttt gaaatagctt taaatacttt ttatcaaatt 1380
gaaggtattc aaactgactg gaatttccct tcaagagagg tttatgaaga taatgtaaag 1440
gaaattgaca actatgttaa gaaaactaaa aaagataact tagaattttt tcgaacagaa 1500
aaacaaattc cccaaactta caatgatggt atgaaattta attataatag catttctcag 1560
ttctcatctg tcaaaaataa cttatcagca caattattga attctctagg ctactattca 1620
caaggaaatc attctaccat tagttatcct aataatacta ttttgatgga tagtcttttt 1680
tcaattaaat acaatattaa taatcaaaat cctcataaat ttggattcca tttaaaacag 1740
aaaaacaata agctgcaact ttacaaaaac ttctattctc ttcctttagc acttatgtca 1800
aatcatattt acaaagatgt caagtttgac tcttatcccc ttgataatca acaaaaattt 1860
gttaatgaat tgacagatct aaatcttaca cttttcaaag aaatccctat tatttcaagt 1920
gtcggaatgc aagttttaga taatcgtgtt actattaatg gttcaaaagg aaataaggca 1980
caagtttact atactgtaaa gtgtcctgca aatagtcaac tttatatcag ccttcctaac 2040
ttgacagtta ataataaaga cgaaaatgtc tttataacaa ctaacaagca cacaagttct 2100
tatatcatag acgaaagtta ttatcttttt aatttaggaa attataaaaa aactcaaaca 2160
ttaatattta agcttagttt tccaaaaaat aaaacggtta gttatgattt accacatatt 2220
tatgctctcg atttaactgc ctatcaaaaa agtataaagc aattaaaaag tcaaactgtt 2280
aaaacaacaa ctaagaaaaa taaaattttt actacctatg ttgccaaaaa gagaacttcc 2340
ttgatttaca ctttaccata tgataaaggt tggtttgcta aacaaaatgg aaaagcaatt 2400
aaaatatcta aagcacaaaa tggactaatg aaaattgatg tttctaaagg tagtgggaag 2460
attataatga cttttgtgcc ccaaggacta tatcaaggaa ttcttcttac ctgtctaggt 2520
atctttctct ttgtatttta ccaactttat tacaaaaaat ttaatttaaa ataa 2574
```

<212> Type : DNA
<211> Length : 2574
SequenceName : SEQ ID 601
SequenceDescription :
Sequence
--------
<213> OrganismName : Streptococcus mutans UA159
<400> PreSequenceString :

```
atgaaattga aacatatttt aagaattgga gcggttgctt ttgcctcaat tcttttgtta 60
actgcttgcg gatcaaaaac atctaaaaaa acagtaaccc ttgcgactgt tggaacaaca 120
aatccatttt cttatgagaa aaagggaaaa ttgacgggat atgatatcga agttgctaag 180
gaagttttca aagcttctga taaatacgat gtcaaatatc aaaaaacaga gtggaccagc 240
atttctctg gtctagatag tgacaaatat caaatcggag ctaacaatat cagttatact 300
aaagagcgtg ccaataaata tctttattct aatccaacgg cttccaatcc attggtatta 360
gtggttccaa aagatagtga tattaagtct tataacgata ttgctgggca tagcactcaa 420
gttgttcaag gaaatacaac agtgtctatg ctgcagaaat tcaataaaaa ccatgaaaac 480
aatcaagtta aactaaactt taccagtgaa gatcttgcgc atcaaatccg gaatgtcagt 540
```

```
gatggtaagt atgattttaa aattttgaa aaaatttcag cagaaacgat catcaaagag     600
caaggacttg ataatttgaa agttattgat cttccttcag accaaaaacc atatgtttac     660
tttatttttg cgcaagacca aaaagactta caaaagtttg tcaataaacg tctcaaaaaa     720
ctttacgaga atggtacact tgaaaaatta tcgaaaaaat accttggagg aagctatctt     780
ccagataaaa aagatatgaa ataa                                            804
```

<212> Type : DNA
<211> Length : 804
SequenceName : SEQ ID 602
SequenceDescription :
Sequence
--------
<213> OrganismName : Streptococcus mutans UA159
<400> PreSequenceString :

```
atgcgatttc ttgtctttct catcgcattt tttgctgctt tctataaatt tatcgagact      60
gaacggattg attcaaatac agttgctgta aaccctgatt cgctcatttt aaagcgattt     120
ttaaaaacaa atcaattaaa tgggatcatg attgtgacgg ggccagatgg taaggctcaa     180
gtattttcaa atcaaagcaa ggtagatggc agtcctgttt caattaagga ttattttcct     240
cttgcttctt tacaaaaatt gataacaggg gtggctatcc aacaattaat tgataaagga     300
aaactgtctt taaacacacc tttaagcaaa tattatcctc aaattgaaaa tagtgaaaat     360
atcacgatac aaaatttact tacccacaca agcggtttgg cagatcgaaa agaagttcct     420
cagcaagtgc tgacaactca agagcagcaa ttggattttt cattgaccaa ttatcgcgta     480
acttatcgaa aaaaatggaa gtatgctaac attaattatg ctttgctagc tggcattatc     540
agtcaaatta gcggtcaaaa ttatgcgact tatgttcgtc aacacttctt aacagctggt     600
aaggggtggc attttaaaaa gtatattcaa ataaaagata agtccaagtt agctgccttg     660
tcagtgatgg atcaaagtac gacttgggat aagctgtcaa aagaagtgac atctaccttt     720
ggagctggtg attatgcttc taggccagtg gattattgga aatttatgat ggcttttatt     780
aatgaccaat ttgttcctgt cagcgaatac caacgttcta tgaaaatgac ttctaagagc     840
tattatggcg gcctctatat cagccaaaag atgctgcatg caaatggtgg tggctttgat     900
acttactctt gttttgctta ttcaaatcct aaaaccaaac aggtcatggt tttgtttatc     960
acaaacggta agtataaacg ggtcaaatcc ttagcagcta aagcctttaa actatatgca    1020
gattcgtatg cgctgaggaa aaatgaaacg tcaaaataa                           1059
```

<212> Type : DNA
<211> Length : 1059
SequenceName : SEQ ID 603
SequenceDescription :
Sequence
--------
<213> OrganismName : Streptococcus mutans UA159
<400> PreSequenceString :

```
atgaagaaaa aaatagctct agcagctctt tcttttgtca gtgcagctgt tcttgcagct      60
tgcagctcag cacctggtgg ttcatcagat gcagctggta ataaaattgg agatactgta     120
aaaattggtt acaatcttga attatcagga gatgtagccg cttatggaca agctgaaaag     180
aacggtgcta accttgctgt tgaagagatt aataaggcag gcggcattga tggcaaaaag     240
attaaagtta tctcaaaaga taataaatct gataacggtg aagcatcaac aatctcaact     300
aatcttgcta cccaaagtaa agtaaatgct atcttgggac cagcaacatc tggtgctaca     360
gcggctgctg ctcccaatgc caacgatgct gcagtaccac tcgtaacgcc ttctggaaca     420
caagataatt tgacctattc aaaaggcaaa gttcaagatt acatcttccg tacaactttt     480
caagatagct tccaaggaaa gatcattgcc aaatatgcaa cagataattt gaaagctaaa     540
aaagtagcgc tttactatga taagtcaagt gattacgccc aaggtattgc tgatgcattc     600
aaaaaagcat ataaagggaa gattactgtt gaagatacct ttcaagctaa agaccaagat     660
ttccaagcag ctctgaccaa gtttaaaaat aaagactttg atgccattgt gataccaggt     720
tattatactg aaactggtct gattacaaag caagcacgtg atatggggct tacccagcct     780
atcttaggac ctgatggttt taatgatgaa aaatatgttg aaggtgctgg tgcagccaat     840
accaataatg ttcattatgt atctggttac tcaacaaaag ttgctttaac aaataaggct     900
gaaaaattcc tgaaagatta taaggctaag tatggtgaag agccaaatat gtttgccgct     960
cttgcttatg attccgttta tatgattgct gatgctgcaa aagatgccaa aacatctaag    1020
gatattgcaa caaacctagc taaattgaaa aactttaaag gtgtgacagg taaaatgaca    1080
attgataaga aacataaccc tgttaaatca gccgttatgg ttggtcttaa agatggtaaa    1140
gaagacacag ctactgctgt tgaagcaaaa taa                                 1173
```

<212> Type : DNA
<211> Length : 1173
SequenceName : SEQ ID 604
SequenceDescription :
Sequence
--------
<213> OrganismName : Streptococcus mutans UA159
<400> PreSequenceString :

```
atgaagaaat taagcttatt attactagtt tgtttatctt tattaggctt atttgcctgt   60
acttctaaaa aaacagccga caaaaaattg actgttgtgg ctaccaattc tattattgct  120
gatattacta agaatatcgc tggtaataag gttgtcttac atagtatcgt tcctgttggt  180
cgagatcctc acgaatatga gcctcttcct gaagatgtta aaaagacctc tcaggctgat  240
gtcatttttt ataatgggat taatcttgaa aatggaggca atgcttggtt taccaaacta  300
gttaaaaatg ctcataaaaa gacagacaag gattattttg cagtgagcga tagtgttaag  360
accatttatt tggaaaatgc aaaagaaaaa ggaaaggaag atcctcatgc ttggcttgac  420
cttaaaaatg gtattattta tgctaaaaat atcatgaaac gtctatctga aaaagatcct  480
aaaaacaaga gttattatca gaaaaatttt caagcctaca gcgccaaact tgaaaaacta  540
cacaaagtag ccaaagaaaa aatcagtcgt atccctactg agaagaaaat gatcgtaact  600
agtgaaggtt gtttcaagta tttctctaag gcttacgata ttccttctgc ctatatatgg  660
gaaattaata ccgaagaaga gggaacacca aatcaaatta aggctttagt gaaaaaatta  720
aggaaaagtc gggtgtctgc gctttttgta gaaagcagtg ttgatgatcg tccaatgaaa  780
actgtttcaa aagatacagg tatcccaatt gccgctaaaa tttttacaga ttcagttgct  840
aaaaaaggac aggctggaga tagttactat gcgatgatga agtggaatat agataaaatt  900
gcaaatggtc tgtcacaatg a                                              921
```

<212> Type : DNA
<211> Length : 921
SequenceName : SEQ ID 605
SequenceDescription :
Sequence
--------
<213> OrganismName : Streptococcus mutans UA159
<400> PreSequenceString :

```
atgtttgttc atactaagac taagaaaaaa agaaagtggc aaaggaaagt gtttctactg    60
ctgcttcttt ttttattgcc tattgtgtca gtattggctt ttattgtgtt atttattggc   120
ggtggtacag ctgagtctca tgatgtggaa gcgacgacag gggcgttaa gctttcagct    180
aagcaatttg cagataagac aaagttagga atttcagaag aggaagctaa aaatgcctta   240
gcttttgcgg ataggttgat gtctcgtcat cattttacag ctcaagcaac tgctggagta   300
ttggctgttg gctttcgtga aagtggcttt gatgtcaaag cagttaataa ttctggtggt   360
gtagctggct ttttccaatg gtctggctgg ggtagttctg ttaatggtga tcgttggaaa   420
gtagctagta aaagagagtt aactctagag gttgaggtag atttgatgag cactgaacta   480
gatggtcgat atgctgatgt tgtcaaaaaa gttggttctg cgactgatga aaaacaggct   540
gctaaggatt ggtctcagta ttatgaaggt gttgcggtta gtgatggtca aacgaaagct   600
gataaaattg agagttgggc aacaactatt tgtgaggctt taaagtctgg tggtacaaat   660
tatgctaaag tgaataatac gggaacaagt tctactgcta tcccgcaggg ttgggaaaat   720
attagtgctt ttgatggcca tgcttatgaa ggtagtgaaa attatcctca aggacaatgc   780
acttggtatg tttataatcg tgctaaacag ttgggtgtta gcttcagtcc ttatatgggg   840
aatggcggtc agtggtatca agtgcaaggc taccattcta gtcatacacc taaagcacat   900
acggctttat cttttgtcaa tggtcaggca ggttctgatc caacttatgg tcatgttgct   960
tttgtagagg ctgttaaaga tgatgggagt attctaatca gtgagatgaa cgtttatggt  1020
caaccagcta tgacggttgc ctatcggaca tttgatgctg aaactgctaa acaattttgg  1080
tatgtagagg gaaaataa                                                1098
```

<212> Type : DNA
<211> Length : 1098
SequenceName : SEQ ID 606
SequenceDescription :
Sequence
--------
<213> OrganismName : Streptococcus mutans UA159
<400> PreSequenceString :

```
atgaaaatga aacgtaaact attaagcttg gtttcagtcc ttactatttt attgggagct      60
ttttgggtaa cgaagattgt aaaagctgac caagtcacaa attatacaaa tacggcttct     120
atcacaaaat cagatggtac agcactttct aatgatccat ctaaggctgt taattattgg     180
gaaccacttt ctttcagtaa ttctattact ttcccagatg aagtcagtat taaggctggg     240
gatactttaa ccattaagtt gccagagcaa ttacaattta cgactgctct aactttcgat     300
gttatgcata ccaatgggca attagctggt aaagcaacaa ctgatcctaa tacaggagaa     360
gtaacagtta cctttactga tatttttgaa aaactgccta atgataaggc tatgacatta     420
aattttaatg cacaattgaa tcataacaat atttctattc ctggtgttgt aaactttaac     480
tataataatg ttgcttatag ctcttatgtt aaagacaaag atattacgcc aataagtcca     540
gatgttaaca aagtgggtta tcaggataaa agtaatcctg gtttgattca ctggaaagtt     600
ctcattaaca acaaacaagg tgctattgat aatttgactt tgactgatgt tgtcggagaa     660

gatcaagaaa tcgtaaaaga ttccttggtt gctgcacgct tgcagtacat tgctggtgat     720
gatgttgaca gtttagatga agctgcttcg cgaccttatg ctgaggattt ttcaaaaaat     780
gttacttatc aaactaatga tttaggattg acaacaggat ttacctatac aattccagga     840
tccagtaaca acgctatctt tatctcttat actactcgtt taacttcttc tcaatctgct     900
ggtaaagatg tcagcaacac tattgctatt tcaggaaata atattaatta ttccaatcaa     960
acaggctacg ctcgtattga atccgcatat ggtagagcta gttctagagt aaagaggcaa    1020
gcagaaacaa caactgttac tgaaacaaca acttcgtcat cttctgaaac gacaactagt    1080
gaagcgacaa cagaaacaag tagtacaaca aataataatt caactactac agaaacagct    1140
actagcacaa caggagcttc aacaacacaa acaaaaacga ctgcttctca aacgaatgtt    1200
ccgacaacaa caaacataac aacaacttca aaacaagtaa ccaagcaaaa agcgaaattt    1260
gttttaccat caacaggtga acaagcaggg cttttgttaa ctactgtagg tcttgtaatt    1320
gttgctgtgg caggtgtcta tttctataga acacgtcgtt aa                        1362
```

<212> Type : DNA
<211> Length : 1362
SequenceName : SEQ ID 607
SequenceDescription :
Sequence
--------
<213> OrganismName : Streptococcus mutans UA159
<400> PreSequenceString :

```
atgacattta aaaagttagt tttaggtttg ttgagttttg tggctgtatt tactttagta      60
gcttgcagtt cttctaattc aaaaaattta caggatgata ttaaagaaaa gaaaaagtta     120
gttgttgctg ttagtccgga ctatgctcct tttgagttca aggctcttgt gaacggtaag     180
gatactgttg ttggtgctga tattgatttg gcaaaagcaa ttgctaaaga attgggagtg     240
aaactggaat tatcttccat gagttttgat aatgtcttgt ccagtttaaa aacaggaaaa     300
gcagacatag ctatctctgg tttatcttat accaaggaac gtgctcaagc ctatgacttt     360
tcagaagctt attataaaac ggaaaatgct attcttatta aaaagtctga tttgaacaaa     420
tatacaatga tttcttcttt taataataag actaaagtag ctgttcaaaa aggaacgatt     480
gaagaaggat tagctaaaaa tcaattaaaa caatcaaaca ttacctcttt gacttcgatg     540
ggcgaagctg ttaatgagct caaatctggt caggttgatg ctattgatct tgaaaaacca     600
gtggcagaag gttatgtgtc tcaaaatagt gatttggttc ttgccaaagt tgccttaaaa     660
acgggtgaag gggatgccaa agcagttgct ctgcctaaag acagtggtca attagttaag     720
acggtgaata aggttattaa gaaactcaaa aaagaagata aatacaagca gtttatcagc     780
gatgctgtta aattaactgg tcagcaagtg gattga                               816
```

<212> Type : DNA
<211> Length : 816
SequenceName : SEQ ID 608
SequenceDescription :
Sequence --------
<213> OrganismName : Streptococcus mutans UA159
<400> PreSequenceString :

```
atgaaaaagc attttttcat gacttttagc ctcttgctag cggctgtttt tctagttgct      60
tgttccaatc tttccgattc tggacagagg aattgggata agataaataa gagaggaatg     120
cttaaaattg ctactgcagg aacgctttat ccgcaatctt atcatgatga tcataataaa     180
ttgacgggtt atgatgttga aattctaaaa gaaataggaa aacgtttggg attgaaagtt     240
cagtttactg aaatgggtgt cgatggtatg ctgacagcca tcaagagcgg tcagatcgat     300
gttgctaatt attccctaga agacggcaac aaaaatatca gtaagttttt gagaacctct     360
ccctataaat attcttttac gtcaatggtt gtccgctcta aagatgattc aggtattcat     420
tcttggtcag accttaaggg aaaaaaagct gccggagctg ccagcactaa ttatatgaag     480
attgctaaaa aattaggagc aaaattagtt gtctatgata atgtcaccaa cgatgtttat     540
atgaaagatt tagttaatgg tcgtacagat gtcattatca atgattatta tctgcaaaag     600
atagctgttg cagcagtcaa agacaaatac gctatcaaaa taaaccaagg actttatgcc     660
aatccttaca gcactagttt tacattgtct ttgaaaaaca aagtactgca aaagaaaatc     720
aataaggctg tgaaagacat gcgcaaggat ggcaccctaa ccaagctatc taagaagttt     780
ttccaaggag aagacgtcac taaaaaacat tataatagct ataaaaaaat tgatatttct     840
gacgttgatt aa                                                         852
```

<212> Type : DNA
<211> Length : 852
SequenceName : SEQ ID 609
SequenceDescription :
Sequence
--------
<213> OrganismName : Streptococcus pneumoniae R6

```
<400> PreSequenceString :
atgaagcttt tgaaaaaaat gatgcaagtc gcactagcca catttttctt cggtttgcta      60
gggaccagta cagtatttgc agatgattct gaaggatggc agtttgtcca agaaaatggt     120
agaacctact acaaaaaggg ggctctaaaa gaaacctact ggagagtgat agatgggaag     180
tactattatt ttgatccttt atccggagag atggttgtcg gctggcaata tatacctgct     240
ccacacaagg gggttacgat tggtccttct ccaagaatag agattgctct tagaccagat     300
tggttttatt ttggtcaaga tggtgtctta caagaatttg ttggcaagca agttttagaa     360
gcaaaaactg ctacgaatac caacaaacat catggggaag aatatgatag ccaagcagag     420
aaacgagtct attattttga agatcagcgt agttatcata ctttaaaaac tggttggatt     480
tatgaagagg gttattggta ttatttacag aaggatggtg gctttgattc tcgcatcaac     540
agattgacgg ttggagagct agcacgtggt tgggttaagg attaccctct tacgtatgat     600
gaagagaagc taaaagcagc tccatggtac tatctagatc cagcaactgg ctggcaaaac     660
cttgggaaca aatggtacta tctccgttca tcaggagcta tggcaactgg ttggtatcag     720
gaaggttcga cttggtacta tctaaatgca agtaatggag atatgaaaac aggctggttc     780
caagtcaatg gtaactggta ctatgcctat gattcaggtg ctttagctgt taataccaca     840
gtaggtggtt actacttaaa ctataatggt gaatgggtta agtaa                     885
```

<212> Type : DNA
<211> Length : 885
SequenceName : SEQ ID 610
SequenceDescription :
Sequence
--------
<213> OrganismName : Streptococcus pneumoniae R6
<400> PreSequenceString :

```
atgaaacttt tgaaaaaaat gatgcaagtt ctactagcag tctttttctt tggtttgcta      60
gctacaaata cggtatttgc gaataccaca ggtggccgat ttgttgataa ggataataga     120
aaatattatg taaaagatga tcataaagca atctattggc ataaaataga cggtaaaact     180
tactattttg gtgatattgg agagatggtt gtcggttggc aatacttaga aattcctgga     240
acaggttatc gtgataattt attcgataac caaccagtta atgaaattgg ccttcaggag     300
aagtggtact attttggaca agatggtgct ttgctagaac aaacagataa acaagtacta     360
gaggcaaaaa cgtctgaaaa tacaggaaaa gtatacggtg aacaatatcc tctatctgct     420
gaaaagagaa cttattattt tgataataat tatgctgtaa agacaggctg gatttatgaa     480
gacggcaatt ggtattattt aaataagcta ggaaattttg gcgatgattc ttacaatcca     540
ctaccaattg gtgaagttgc taagggttgg actcaagatt ttcatgttac tattgacatt     600
gatagaagca aacctgctcc atggtactac ctagatgctt caggtaagat gcttacagat     660
tggcaaaaag taaacggaaa atggtattat tttggctcct ctggttctat ggcaacaggt     720
tggaaatatg tacgaggcaa atggtattac ttagataata aaaatggtga tatgaaaaca     780
ggatggcaat accttggtaa caagtggtac tacctccgtt catcaggagc tatggtaact     840
ggctggtatc aagatggttt aacttggtac tacctaaatg caggtaatgg agacatgaag     900
acaggttggt tccaggtcaa tggcaaatgg tactatgctt atagctcagg tgccttggca     960
gtgaatacga ccgtagatgg ctattctgtc aactataatg gcgaatgggt tcaataa       1017
```

<212> Type : DNA
<211> Length : 1017
SequenceName : SEQ ID 611
SequenceDescription :
Sequence
--------
<213> OrganismName : Streptococcus pneumoniae R6
<400> PreSequenceString :

```
atgaataaga aaaaaatgat tttaacaagt ctagccagcg tcgctatctt aggggctggt      60
tttgttgcgt ctcagcctac tgttgtaaga gcagaagaat ctcccgtagc cagtcagtct     120
aaagctgaga aagactatga tgcagcgaag aaagatgcta agaatgcgaa aaaagcagta     180
gaagatgctc aaaaggcttt agatgatgca aaagctgctc agaaaaaata tgacgaggat     240
cagaagaaaa ctgaggagaa agccgcgcta gaaaaagcag cgtctgaaga gatggataag     300
gcagtggcag cagttcaaca agcgtatcta gcctatcaac aagctacaga caaagccgca     360
aaagacgcag cagataagat gatagatgaa gctaagaaac gcgaagaaga ggcaaaaact     420
aaatttaata ctgttcgagc aatggtagtt cctgagccag agcagttggc tgagactaag     480
aaaaaatcag aagaagctaa acaaaaagca ccagaactta ctaaaaaact agaagaagct     540
aaagcaaaat tagaagaggc tgagaaaaaa gctactgaag ccaaacaaaa agtggatgct     600
gaagaagtcg ctcctcaagc taaaatcgct gaattggaaa atcaagttca tagactagaa     660
caagagctca aagagattga tgagtctgaa tcagaagatt atgctaaaga aggtttccgt     720
gctcctcttc aatctaaatt ggatgccaaa aaagctaaac tatcaaaact tgaagagtta     780
agtgataaga ttgatgagtt agacgctgaa attgcaaaac ttgaagatca acttaaagct     840
gctgaagaaa acaataatgt agaagactac tttaaagaag gtttagagaa aactattgct     900

gctaaaaaag ctgaattaga aaaaactgaa gctgacctta agaaagcagt taatgagcca     960
gaaaaaccag ctccagctcc agaaactcca gccccagaag caccagctga acaaccaaaa    1020
ccagcgccgg ctcctcaacc agctcccgca ccaaaaccag agaagccagc tgaacaacca    1080
aaaccagaaa aaacagatga tcaacaagct gaagaagact atgctcgtag atcagaagaa    1140
gaatataatc gcttgactca acagcaaccg ccaaaagctg aaaaaccagc tcctgcacca    1200
aaaacaggct ggaaacaaga aaacggtatg tggtacttct acaatactga tggttcaatg    1260
gcgacaggat ggctccaaaa caacggttca tggtactacc tcaacagcaa tggtgctatg    1320
gctacaggtt ggctccaata caatggttca tggtattacc tcaacgctaa cggtgctatg    1380
gcaacaggtt gggctaaagt caacggttca tggtactacc tcaacgctaa tggtgctatg    1440
gctacaggtt ggctccaata caacggttca tggtattacc tcaacgctaa cggcgctatg    1500
gcaacaggtt gggctaaagt caacggttca tggtactacc tcaacgctaa tggtgctatg    1560
gctacaggtt ggctccaata caacggttca tggtactacc tcaacgctaa cggtgctatg    1620
gctacaggtt gggctaaagt caacggttca tggtactacc tcaacgctaa tggtgctatg    1680
gcaacaggtt gggtgaaaga tggagatacc tggtactatc ttgaagcatc aggtgctatg    1740
aaagcaagcc aatggttcaa agtatcagat aaatggtact atgtcaatgg tttaggtgcc    1800
cttgcagtca acacaactgt agatggctat aaagtcaatg ccaatggtga atgggtttaa    1860
```

<212> Type : DNA
<211> Length : 1860
SequenceName : SEQ ID 612
SequenceDescription :

Sequence
--------
<213> OrganismName : Streptococcus pneumoniae R6
<400> PreSequenceString :

```
atgaaaattt taccgtttat agcaagagga acaagttatt acttgaagat gtcagttaaa      60
aagcttgttc ctttttttagt agtaggattg atgctagcag ctggtgatag tgtctatgcc    120
tattccagag gaaatggatc gattgcgcgt ggggatgatt atcctgctta ttataaaaat    180
gggagccagg agattgatca gtggcgcatg tattctcgtc agtgtacttc ttttgtagcc    240
tttcgtttga gtaatgtcaa tggttttgaa attccggcag cttatggaaa tgcgaatgaa    300
tggggacatc gtgctcgtcg ggaaggttat cgtgtagata atacaccgac gattggttcc    360
attacttggt ctactgcagg aacttatggt catgttgcct gggtgtcaaa tgtaatggga    420
gatcagattg agattgagga atataactat ggttatacag aatcctataa taaacgagtt    480
ataaaagcaa acacgatgac aggatttatt cattttaaag atttggatag tggcagtgtt    540
gggaatagtc aatcctcagc ttcaacaggc ggaactcatt attttaagac caagtctgct    600
attaaaactg aacccctagt tagtgcaact gtgattgatt actattatcc tggggagaag    660
gttcattatg atcagatact tgaaaaagac ggctataagt ggttgagtta tactgcctat    720
aatggaagct atcgttatgt tcaattggag gctgtgaata aaaatcctct aggtaattct    780
gttctttctt caacaggagg aactcattat tttaagatca agtctgctat taaaactgaa    840
cccctagtta gtgcaactgt gattgattac tattatcctg gagagaaggt tcattatgat    900
cagatacttg aaaaagacgg ctataagtgg ttgagttata cggcttataa cggaagtcgt    960
cgctatatac agctagaggg agtgacttct tcacaaaatt atcagaatca atcaggaaat   1020
atctctagct atggatccaa taatagttca actgtcggtt ggaagaaaat aaatggtagt   1080
tggtatcatt tcaaatcaaa tggttctaaa tcaacaggat ggctgaaaga cggttctagc   1140
tggtattatt tgaaattatc tggtgaaatg cagacaggat ggttaaagga gaatggctcg   1200
tggtattatc tgggtagttc aggggcaatg aaaacaggct ggtaccaggt ctctggtgag   1260
tggtattatt cttactcttc aggcgcctta gctattaata cgacggtgga tggctacaga   1320
gtaaacagtg atggagaacg agtatag                                         1347
```

<212> Type : DNA
<211> Length : 1347
SequenceName : SEQ ID 613
SequenceDescription :
Sequence
--------
<213> OrganismName : Streptococcus pneumoniae R6
<400> PreSequenceString :

```
atgtttgcat caaaaagcga aagaaaagta cattattcaa ttcgtaaatt tagtattgga     60
gtagctagtg tagctgttgc cagtcttgtt atgggaagtg tggttcatgc gacagagaac    120
gagggaagta cccaagcagc cacttcttct aatatggcaa agacagaaca taggaaagct    180
gctaaacaag tcgtcgatga atatatagaa aaaatgttga gggagattca actagataga    240
agaaaacata cccaaaatgt cgccttaaac ataaagttga gcgcaattaa aacgaagtat    300
ttgcgtgaat taaatgtttt agaagagaag tcgaaagatg agttgccgtc agaaataaaa    360
gcaaagttag acgcagcttt tgagaagttt aaaaaagata cattgaaacc aggagaaaag    420
gtagcagaag ctaagaagaa ggttgaagaa gctaagaaaa aagccgagga tcaaaaagaa    480
gaagatcgtc gtaactaccc aaccaatact tacaaaacgc ttgaacttga aattgctgag    540
```

```
ttcgatgtga aagttaaaga agcggagctt gaactagtaa aagaggaagc taaagaatct     600
cgaaacgagg gcacaattaa gcaagcaaaa gagaaagttg agagtaaaaa agctgaggct     660
acaaggttag aaaacatcaa gacagatcgt aaaaaagcag aagaagaagc taaacgaaaa     720
gcagatgcta agttgaagga agctaatgta gcgacttcag atcaaggtaa accaaagggg     780
cgggcaaaac gaggagttcc tggagagcta gcaacacctg ataaaaaaga aaatgatgcg     840
aagtcttcag attctagcgt aggtgaagaa actcttccaa gctcatccct gaaatcagga     900
aaaaaggtag cagaagctga gaagaaggtt gaagaagctg agaaaaaagc caaggatcaa     960
aaagaagaag atcgccgtaa ctacccaacc aatacttaca aaacgcttga ccttgaaatt    1020
gctgagtccg atgtgaaagt taaagaagcg gagcttgaac tagtaaaaga ggaagctaag    1080
gaacctcgag acgaggaaaa aattaagcaa gcaaaagcga aagttgagag taaaaaagct    1140
gaggctacaa ggttagaaaa catcaagaca gatcgtaaaa aagcagaaga agaagctaaa    1200
cgaaaagcag cagaagaaga taaagttaaa gaaaaaccag ctgaacaacc acaaccagcg    1260
ccggctactc aaccagaaaa accagctcca aaaccagaga agccagctga acaaccaaaa    1320
gcagaaaaaa cagatgatca acaagctgaa gaagactatg ctcgtagatc agaagaagaa    1380
tataatcgct tgactcaaca gcaaccgcca aaaactgaaa aaccagcaca accatctact    1440
ccaaaaacag gctggaaaca agaaaacggt atgtggtact tctacaatac tgatggttca    1500
atggcaacag gatggctcca aaacaacggt tcatggtact atctaaacgc taatggtgct    1560
atggcgacag gatggctcca aaacaatggt tcatggtact atctaaacgc taatggttca    1620
atggcaacag gatggctcca aaacaatggt tcatggtact acctaaacgc taatggtgct    1680
atggcgacag gatggctcca atacaatggt tcatggtact acctaaacag caatggcgct    1740
atggcgacag gatggctcca atacaatggc tcatggtact acctcaacgc taatggtgat    1800
atggcgacag gatggctcca aaacaacggt tcatggtact acctcaacgc taatggtgat    1860
atggcgacag gatggctcca atacaacggt tcatggtatt acctcaacgc taatggtgat    1920
atggcgacag gttgggtgaa agatggagat acctggtact atcttgaagc atcaggtgct    1980
atgaaagcaa gccaatggtt caaagtatca gataaatggt actatgtcaa tggctcaggt    2040
gcccttgcag tcaacacaac tgtagatggc tatggagtca atgccaatgg tgaatgggta    2100
aactaa                                                               2106
```

<212> Type : DNA
<211> Length : 2106
SequenceName : SEQ ID 614
SequenceDescription :
Sequence
--------
<213> OrganismName : Streptococcus pneumoniae R6
<400> PreSequenceString :

```
atgaaaaaaa ctacaatatt atcattaact acagctgcgg ttattttagc agcatatgtc      60
cctaatgaac caatcctagc agcatatgtc cctaatgaac caatcctagc agatactcct     120
agttcggaag taatcaaaga gactaaagtt ggaagtatta ttcaacaaaa taatatcaaa     180
tataaggttc taactgtaga aggtaacata ggaactgttc aagtgggtaa tggagttact     240
cctgtagagt ttgaagctgg tcaagatgga aaaccattca cgattcctac aaaaatcaca     300
gtaggtgata aagtatttac cgttactgaa gtagctagtc aagcttttag ttattatcca     360
gatgaaacag gtagaattgt ctactatcct agctctatta ctatcccatc aagcataaaa     420
aaaatacaaa aaaaaggctt ccatggaagt aaagctaaaa ctattatttt tgacaaaggc     480
agtcagctgg agaaaattga agatagagct tttgattttt ctgaattaga agagattgaa     540
ttgcctgcat ctctagaata tattggaaca agtgcatttt cttttagtca aaaattgaaa     600
aagctaacct tttcctcaag ttcaaaatta gaattaatat cacatgaggc ttttgctaat     660
ttatcaaatt tagagaaact aacattacca aaatcggtta aaacattagg aagtaatcta     720
tttagactca ctactagctt aaaacatgtt gatgttgaag aaggaaatga atcgtttgcc     780
tcagttgatg gtgttttgtt ttcaaaagat aaaactcaat taatttatta tccaagtcaa     840
aaaaatgacg aaagttataa aacgcctaag gagacaaaag aacttgcatc atattcgttt     900
aataaaaatt cttacttgaa aaaactcgaa ttgaatgaag gtttagaaaa aatcggtact     960
tttgcatttg cggatgcgat taaacttgaa gaaattagct taccaaatag tttagaaact    1020
attgaacgtt tagcctttta cggtaattta gaattaaaag aacttatatt accagataat    1080
gttaaaaatt ttggtaaaca cgttatgaac ggtttaccaa aatttttaac attatctggt    1140
aataatatca actcattgcc gtccttcttc ctaagtggcg tcttagattc attaaaggaa    1200
attcatatta agaataaaag tacagagttt tctgtgaaaa aagatacatt tgcaattcct    1260
gaaactgtta agttctatgt aacatcagaa catataaaag atgttcttaa atcaaattta    1320
tctactagta atgatatcat tgttgaaaaa gtagataata taaaacaaga aactgatgta    1380
gctaaaccta aaaagaattc taatcaggga gtagttggtt gggttaaaga caaaggttta    1440
tggtattact taaacgaatc aggttcaatg gctactggtt gggttaaaga caaaggttta    1500
tggtattact taaacgaatc aggttcaatg gctactggtt gggttaaaga caaaggttta    1560
tggtattact taaatgaatc aggttcaatg gctactggtt gggttaaaga caaaggctta    1620
tggtattact taaacgaatc aggttcaatg gctactggtt gggttaaaga caaaggctta    1680
tggtattact taaacgaatc aggttcaatg gctactggtt gggttaaaga caaaggctta    1740
tggtattact taaatgaatc aggttcaatg gctactggtt gggttaaaga caaaggctta    1800
tggtattact taaacgaatc aggttcaatg gctactggtt gggttaaaga caaaggctta    1860
```

```
tggtattact taaacgaatc aggttcaatg gctactggtt gggttaaaga caaaggctta    1920
tggtattact taaatgaatc aggttcaatg gctactggtt gggttaaagt ttctggtaaa    1980
tggtactata cctataattc aggagattta ttagtaaaca cgactacacc cgatggctat    2040
cgagtcaatg ctaacggtga gtgggtagga tag                                 2073
```

<212> Type : DNA
<211> Length : 2073
SequenceName : SEQ ID 615
SequenceDescription :
Sequence
--------
<213> OrganismName : Streptococcus pneumoniae R6
<400> PreSequenceString :

```
atggaaatta atgtgagtaa attaagaaca gatttgcctc aagtcggcgt gcaaccatat      60
aggcaagtac acgcacactc aactgggaat ccgcattcaa ccgtacagaa tgaagcggat     120
tatcactggc ggaaagaccc agaattaggt tttttctcgc acattgttgg gaacggttgc     180
atcatgcagg taggacctgt tgataatggt gcctgggacg ttgggggcgg ttggaatgct     240
gagacctatg cagcggttga actgattgaa agccattcaa ccaaagaaga gttcatgacg     300
gactaccgcc tttatatcga actcttacgc aatctagcag atgaagcagg tttgccgaaa     360
acgcttgata cagggagttt agctggaatt aaaacgcacg agtattgcac gaataaccaa     420
ccaaacaacc actcagacca cgttgaccct tatccatatc ttgctaaatg gggcattagc     480
cgtgagcagt ttaagcatga tattgagaac ggcttgacga ttgaaacagg ctggcagaag     540
aatgacactg gctactggta cgtacattca gacggctctt atccaaaaga caagtttgag     600
aaaatcaatg gcacttggta ctactttgac agttcaggct atatgcttgc agaccgctgg     660
aggaagcaca cagacggcaa ctggtactgg ttcgacaact caggcgaaat ggctacaggc     720
tggaagaaaa tcgctgataa gtggtactat ttcaacgaag aaggtgccat gaagacaggc     780
tgggtcaagt acaaggacac ttggtactac ttagacgcta aagaaggcgc catggtatca     840
aatgccttta tccagtcagc ggacggaaca ggctggtact acctcaaacc agacggaaca     900
ctggcagaca ggccagaatt cacagtagag ccagatggct tgattacagt aaaataa        957
```

<212> Type : DNA
<211> Length : 957
SequenceName : SEQ ID 616
SequenceDescription :
Sequence
--------
<213> OrganismName : Neisseria meningitidis serogroup A strain Z2491
<400> PreSequenceString :

```
atgactttcg cctattggtg tattctgatt gcctacctat tgccgctttt ttgtgcggcg      60
tatgccaaaa aagcgggcgg attccggttt aaagacaacc acaatccgcg cgattttctg     120
gcgcgcacgc aaggcacagc cgcccgtgcc cacgccgcgc agcaaaacgg ttttgaagcc     180
tttgcaccgt ttgcagccgc cgttttgacg gcacacgcaa ccggcaatgc cggacaagca     240
accgtcaaca cgcttgccgg cctgttcatc ctgttccgcc tcgcctttat ctggtgctac     300
atcgcagaca aagcagcatt acgctcgctg atgtgggtgg gcggatttgt ctgcaccgtc     360
gggctgtttg tcgtggctgc ttga                                            384
```

<212> Type : DNA
<211> Length : 384
SequenceName : SEQ ID 617
SequenceDescription :
Sequence
--------
<213> OrganismName : Neisseria meningitidis serogroup A strain Z2491
<400> PreSequenceString :

```
atgaacaaaa tataccgcat catttggaat agtgccctca atgcctgggt cgccgtatcc      60
gagctcacac gcaaccacac caaacgcgcc tccgcaaccg tgaagaccgc cgtattggcg     120
acactgttgt ttgcaacggt tcaggcgaat gctaccgatg aagatgaaga agaagagtta     180
gaatccgtac aacgctctgt cgtagggagc attcaagcca gtatggaagg cagcggcgaa     240
ttggaaacga tatcattatc aatgactaac gacagcaagg aatttgtaga cccatacata     300
gtagttaccc tcaaagccgg cgacaacctg aaaatcaaac aaaacaccaa tgaaaacacc     360
aatgccagta gcttcaccta ctcgctgaaa aaagacctca caggcctgat caatgttgaa     420
actgaaaaat tatcgtttgg cgcaaacggc aagaaagtca acatcataag cgacaccaaa     480
ggcttgaatt tcgcgaaaga aacggctggg acgaacggcg acaccacggt tcatctgaac     540
ggtatcggtt cgactttgac cgatacgctt gcgggttctt ctgcttctca cgttgatgcg     600
ggtaaccaaa gtacacatta cactcgtgca gcaagtatta aggatgtgtt gaatgcgggt     660
tggaatatta agggtgttaa aactggctca acaactggtc aatcagaaaa tgtcgatttc     720

gtccgcactt acgacacagt cgagttcttg agcgcagata cgaaaacaac gactgttaat     780
gtggaaagca aagacaacgg caagagaacc gaagttaaaa tcggtgcgaa gacttctgtt     840
attaaagaaa aagacggtaa gttggttact ggtaaaggca aaggcgagaa tggttcttct     900
acagacgaag gcgaaggctt agtgactgca aaagaagtga ttgatgcagt aaacaaggct     960
ggttggagaa tgaaaacaac aaccgctaat ggtcaaacag gtcaagctga caagtttgaa    1020
accgttacat caggcacaaa tgtaaccttt gctagtggta aaggtacaac tgcgactgta    1080
agtaaagatg atcaaggcaa catcactgtt atgtatgatg taaatgtcgg cgatgcccta    1140
aacgtcaatc agctgcaaaa cagcggttgg aatttggatt ccaaagcggt tgcaggttct    1200
tcgggcaaag tcatcagcgg caatgtttcg ccgagcaagg gaaagatgga tgaaaccgtc    1260
aacattaatg ccggcaacaa catcgagatt agccgcaacg gtaaaaatat cgacatcgcc    1320
acttcgatgg cgccgcagtt ttccagcgtt tcgctcggcg cgggggcaga tgcgcccact    1380
ttaagcgtgg atgacgaggg cgcgttgaat gtcggcagca aggatgccaa caaacccgtc    1440
cgcattacca atgtcgcccc gggcgttaaa gagggggatg ttacaaacgt cgcacaactt    1500
aaaggcgtgg cgcaaaactt gaacaaccgc atcgacaatg tggacggcaa cgcgcgtgcg    1560
ggcatcgccc aagcgattgc aaccgcaggt ctggttcagg cgtatctgcc cggcaagagt    1620
atgatggcga tcggcggcgg cacttatcgc ggcgaagccg gttacgccat cggctactcc    1680
agtatttccg acggcggaaa ttggattatc aaaggcacgg cttccggcaa ttcgcgcggc    1740
catttcggtg cttccgcatc tgtcggttat cagtggtaa                           1779
```

<212> Type : DNA
<211> Length : 1779
SequenceName : SEQ ID 618
SequenceDescription :
Sequence
--------
<213> OrganismName : Neisseria meningitidis serogroup A strain Z2491
<400> PreSequenceString :

```
attcttttgg ctgaaggtca aaaatcagcc gtcaccgagt attacctgaa tcacggcaca      60
tggcccagca acaacagtga tgccggcgtg gcatccaccg ccaccgacat caaaggaaaa     120
tatgttaaag aagttaaagt cgaaaaaggc gtcattaccg ccacaatgct ttcaagcggc     180
gtaaacaacg aaatcaaagg caaaaaactc tccctgtggg ccaagcgtca agccggttcg     240
gtaaaatggt tctgcggaca gccggttgag cgcgccgcca acaacgccgc caacgacgcc     300
gtcaccgccg ccaccgccaa cggcaacggc aagatcgaca ccaaacacct gccgtcaacc     360
tgccgcgacg cagcatctgc cgtttgcata gaaacaccac ctacggcttt ctataaaaat     420
acctaa                                                               426
```

<212> Type : DNA
<211> Length : 426
SequenceName : SEQ ID 619
SequenceDescription :
Sequence
--------
<213> OrganismName : Neisseria meningitidis serogroup A strain Z2491
<400> PreSequenceString :

```
atgaaaacaa ccgacaaacg gacaaccgaa acacaccgca aagccccgaa aaccggccgc      60
atccgcttct cgcctgctta cttagccata tgcctgtcgt tcggcattct tccccaagct     120
tgggcgggac acacttattt cggcatcaac taccaatact atcgcgactt tgccgaaaat     180
aaaggcaagt ttgcagtcgg ggcgaaagat attgaggttt acaacaaaaa aggggagttg     240
gtcggcaaat caatgacaaa agccccgatg attgattttt ctgtggtgtc gcgtaacggc     300
gtggcggcat tggtgggcga tcaatatatt gtgagcgtgg cacataacgg cggctataac     360
aacgttgatt ttggtgcgga aggaagaaat cccgatcagc accgtttttc ttaccaaatt     420
gtgaaaagaa ataattataa gcctgacaat tcacaccctt acaacggcga ttaccatatg     480
ccgcgtttgc ataaatttgt cacagatgca gaacctgtcg aaatgacgag tgacatgagg     540
gggaatacct attccgataa agaaaaatat cccgagcgtg tccgcatcgg ctcaggacac     600
cactattggc gttatgatga tgacaaacac ggcgatttat cctactccgg cgcatggtta     660
attggcggca atacacatat gcagggttgg ggaaataatg gcgtagttag tttgagcggc     720
gatgtgcgcc atgccaacga ctatggccct atgccgattg caggtgcggc aggcgacagc     780
ggttcgccaa tgtttattta tgacaaaaca aacaataaat ggctgctcaa cggagtttta     840
caaaccggct acccttattc cggcagggaa aacggtttcc agctgatacg caaagattgg     900
ttctacgatg acatttacag aggcgataca cataccgtct tttttgaacc gcgcagtaac     960
ggacattttt cctttacatc caacaacaac ggtacgggta cggtaacaga aaccaacgaa    1020
aaggtttcca atccaaagct taaagtacag acagtccgac tgtttgacga atctttgaat    1080
gaaactgata aagaaccagt ttacgcggca gggggtgtta atcagtaccg tccaaggtta    1140
aacaacggtg aaaacctttc ttttatcgat tacggcaacg gcaaactcat cttatcaaac    1200
aacatcaacc aaggcgcggg cggtttgtat tttgaaggtg attttacggt ctcgcctgaa    1260
aacaacgaaa cgtggcaagg cgcgggcgtt catatcagtg aagacagtac cgttacttgg    1320
aaagtaaacg gcgtggcaaa cgaccgcctg tccaaaatcg gcaaaggcac gctgcacgtt    1380
```

```
caagccaaag gggaaaacca aggctcgatc agcgtgggcg acggtacagt cattttggat    1440
cagcaggcag acgataaagg caaaaaacaa gcctttagtg aaatcggctt ggtcagcggc    1500
aggggtacgg tgcaactgaa tgccgataat cagttcaacc ccgacaaact ctatttcggc    1560
tttcgcggcg gacgtttgga tttaaacggg cattcgcttt cgttccaccg tattcaaaat    1620
accgatgaag gggcgatgat tgtcaatcat aatgccacaa caacatccac cgttaccatt    1680
acagggaatg aaagtattac acaaccgagt ggtaagaata tcaatagact taattacagc    1740
aaagaaattg cctacaacgg ttggtttggc gagaaagata cgaccaaaac gaacgggcgg    1800
ctcaaccttg tttaccagcc cgccgcagaa gaccgcaccc tgctgctttc cggcggaaca    1860
aatttaaacg gcaacatcac gcaaacaaac ggcaaactgt tttcagcgg cagaccgaca     1920
ccgcacgcct acaatcattt aggaagcggg tggtcaaaaa tggaaggtat cccacaagga    1980
gaaatcgtgt gggacaacga ctggatcaac cgcacgttta aagcggaaaa tttccatatt    2040
cagggcgggc aggcggtgat ttcccgcaat gttgccaaag tggaaggcga ttggcatttg    2100
agcaatcacg cccaagcagt tttggtgtc gcaccgcatc aaagccatac aatctgtaca     2160
cgttcggact ggacgggtct gacaaattgt gtcgaaaaaa ccattaccga cgataaagtg    2220
attgcttcat tgactaagac cgacatcagc ggcaatgtca gccttgccga tcacgctcat    2280
ttaaatctca cagggcttgc cacactcaac ggcaatctta gtgcaaatgg cgatacacgt    2340
tatacagtca gccacaacgc cacccaaaac ggcaacctta gcctcgtggg caatgcccaa    2400
gcaacattta atcaagccac attaaacggc aacacatcgg cttcgggcaa tgcttcattt    2460
aatctaagca acaacgccgc acaaaacggc agtctgacgc tttccgacaa cgctaaggca    2520
aacgtaagcc attccgcact caacggcaat gtctccctag ccgataaggc agtattccat    2580
tttgaaaaca gccgctttac cggacaactc agcggcagca aggatacagc attacactta    2640
aaagacagcg aatggacgct gccgtcaggc acggaattag gcaatttaaa ccttgacaac    2700
gccaccatta cactcaattc cgcctatcgc cacgatgctg caggcgcgca aaccggcagt    2760
gtgtcagaca cgccgcgccg ccgttcgcgc cgttccctat tatccgttac accgccaact    2820
tcggtagaat cccgtttcaa cacgctgacg gtaaacggca aattgaacgg tcaaggaaca    2880
ttccgcttta tgtcggaact cttcggctac cgaagcgaca aattgaagct ggcggaaagt    2940
tccgaaggca cttacacctt ggcggtcaac aataccggca acgaacccgt aagcctcgat    3000
caattgacgg tagtggaagg gaaagacaac aaaccgctgt ccgaaaacct taatttcacc    3060
ctgcaaaacg aacacgtcga tgccggcgcg tggcgttacc aactcatccg caaagacggc    3120
gagttccgcc tgcataatcc ggtcaaagaa caagagcttt ccgacaaact cggcaaggca    3180
gaagccaaaa aacaggcgga aaaagacaac gcgcaaagcc ttgacgcgct gattgcggcc    3240
gggcgcgatg ccgccgaaaa gacagaaagc gttgccgaac cggcccggca ggcaggcggg    3300
gaaaatgtcg gcattatgca ggcggaggaa gagaaaaaac gggtgcaggc ggataaagac    3360
agcgccttgg cgaaacagcg cgaagcggaa acccggccgg ctaccaccgc cttcccccgc    3420
gcccgccgcg cccgccggga tttgccgcaa ccgcagcccc aaccgcaacc tcaaccccaa    3480
ccgcagcgcg acctgatcag ccgttatgcc aatagcggtt tgagtgaatt ttccgccacg    3540
ctcaacagcg ttttcgccgt acaggacgaa ttggaccgcg tgtttgccga agaccgccgc    3600
aacgccgttt ggacaagcgg catccgggac accaaacact accgttcgca agatttccgc    3660
gcctaccgcc aacaaaccga cctgcgccaa atcggtatgc agaaaaacct cggcagcggg    3720
cgcgtcggca tcctgttttc gcacaaccgg accgaaaaca ccttcgacga cggcatcggc    3780
aactcggcac ggcttgccca cggcgccgtt ttcgggcaat acggcatcgg caggttcgac    3840
atcggcatca gcacgggcgc gggttttagc agcggcagtc tttcagacgg catcggaggc    3900
aaaatccgcc gccgcgtgct gcattacggc attcaggcac gataccgcgc cggtttcggc    3960
ggattcggca tcgaaccgta catcggcgca acgcgctatt tcgtccaaaa agcggattac    4020
cgctacgaaa acgtcaatat cgccaccccc ggtcttgcgt tcaaccgcta ccgcgcgggc    4080
attaaggcag attattcatt caaaccggcg caacacattt ccatcacgcc ttatttgagc    4140
ctgtcctata ccgatgccgc ttcgggcaaa gtccgaacac gcgtcaatac cgccgtattg    4200
gctcaggatt tcggcaaaac ccgcagtgcg gaatggggcg taaacgccga aatcaaaggt    4260
ttcacgctgt ccctccacgc tgccgccgcc aaaggcccgc aactggaagc gcaacacagc    4320
gcgggcatca aattaggcta ccgctggtaa                                     4350
```

<212> Type : DNA
<211> Length : 4350
SequenceName : SEQ ID 620
SequenceDescription :
Sequence
--------
<213> OrganismName : Neisseria meningitidis serogroup A strain Z2491
<400> PreSequenceString :

```
atgaacaccc ttcaaaaagg ttttaccctt atcgagctga tgattgtgat tgccatcgtc     60
ggcattttgg cggcagtcgc ccttcctgct tatcaagact acacagcccg cgcacaagtt    120
tccgaagcca ttcttttagc cgaaggtcaa aaatcagccg tcaccgagta ttacctgaat    180
cacggcgaat ggcccagcaa caacacttct gccggcgtgg catcctccac cgacattaaa    240
ggcaaatatg ttcaaagcgt tgaagtcaaa aacggcgtcg ttaccgccac aatggcttca    300
agcaacgtaa acaacgaaat caaaggcaaa aaactctccc tgtgggccaa gcgtcaagac    360
ggttcggtaa aatggttctg cggacagccg gttaagcgca acgacaccgc caccaccaac    420
gacgacgtca aagccgacac cgccgccaac ggcaagcaga tcgacaccaa gcacctgccg    480
```

tcaacctgcc gcgacgcagc atctgccgga taa 513

<212> Type : DNA
<211> Length : 513
SequenceName : SEQ ID 621
SequenceDescription :
Sequence
<213> OrganismName : Neisseria meningitidis serogroup A strain Z2491
<400> PreSequenceString :

```
atgcaagcac ggctgctgat acctattctt ttttcagttt ttattttatc cgcctgcggg 60
acactgacag gtattccatc gcatggcgga ggtaaacgct ttgcggtcga acaagaactt 120
gtggccgctt ctgccagagc tgccgttaaa gacatggatt tacaggcatt acacggacga 180
aaagttgcat tgtacattgc aactatgggc gaccaaggtt caggcagttt gacaggggggt 240
cgctactcca ttgatgcact gattcgtggc gaatacataa acagccctgc cgtccgtacc 300
gattacacct atccacgtta cgaaaccacc gctgaaacaa catcaggcgg tttgacaggt 360
ttaaccactt ctttatctac acttaatgcc cctgcactct cgcgcaccca atcagacggt 420
agcggaagta aaagcagtct gggcttaaat attggcggga tgggggatta tcgaaatgaa 480
accttgacga ctaacccgcg cgacactgcc tttctttccc acttggtaca gaccgtattt 540
ttcctgcgcg gcatagacgt tgtttctcct gccaatgccg atacggatgt gtttattaac 600
atcgacgtat tcggaacgat acgcaacaga accgaaatgc acctatacaa tgccgaaaca 660
ctgaaagccc aaacaaaact ggaatatttc gcagtagaca gaaccaataa aaaattgctc 720
atcaaaccaa aaaccaatgc gtttgaagct gcctataaag aaaattacgc attgtggatg 780
ggaccgtata aagtaagcaa aggaattaaa ccgacagaag gattaatggt cgatttctcc 840
gatatccaac catacggcaa tcatatgggt aactctgccc catccgtaga ggctgataac 900
agtcatgagg ggtatggata cagcgatgaa gcagtgcgac gacatagaca agggcaacct 960
tga 963
```

<212> Type : DNA
<211> Length : 963
SequenceName : SEQ ID 622
SequenceDescription :
Sequence
--------
<213> OrganismName : Neisseria meningitidis serogroup A strain Z2491
<400> PreSequenceString :

```
atgcgcccaa tcttcctatc tttcgtttta ttccctattt tgataaccgc ctgcagcaca 60
ccggacaagt ctgcccgatg ggaaaatatc ggcacaatct caaacggcaa tattcataca 120
tatatcaata aagacagcgt gagaaaaaac ggaaatctga tgattttcca agataaaaaa 180
gttgttacca atctaaaaca agaacgtttt gccaacaccc ccgcatacaa gactgccatt 240
gccgagtggg aaatccactg caacaacaaa acataccgct taagttcgct acaattgttt 300
gatacaaaaa acacggaaat ttccacacaa aactacacag cctcttccct ccgcccgatg 360
agcatcctgt ccgggacatt aaccgaaaaa caatatgaaa ccgtatgcgg aaaaaaactc 420
tga 423
```

<212> Type : DNA
<211> Length : 423
SequenceName : SEQ ID 623
SequenceDescription :
Sequence
--------
<213> OrganismName : Neisseria meningitidis serogroup A strain Z2491
<400> PreSequenceString :

```
atgaacaaac ttttcattac cgccctgtcc gccctcgcct tgtccgcctg cgccggcact 60
tgggagggcg cgaaacaaga caccgcccgc aaccttgaca aaacacaggc cgccgccgaa 120
cgcgccgccg aacaaacagg caacgccgtc gaaaaaggct gggacaaaac caaagaagcc 180
gtcaaaaaag gcggcaatgc cgtcggacgc ggcatttccc atctcggcgg aaaaatcgaa 240
aacgccaccg aataa 255
```

<212> Type : DNA
<211> Length : 255
SequenceName : SEQ ID 624
SequenceDescription :
Sequence
--------
<213> OrganismName : Neisseria meningitidis serogroup A strain Z2491
<400> PreSequenceString :

```
atgaaactcc tcttcatccc cctagtcctc ttcgtcgccg tcgaacattt ctacatcgcc     60
tggcttgaaa tgacgcagat tcccagcgaa aaagcggcgg aaacgttcaa gctgccttat    120
gaatttatgg aacaaaatcg cgtgcagacc ctgttcggca accaagggct gtataacggc    180
tttctcggca tcgggctggt gtggtcgcgg tttgccgctc cggataacgc ggtgtacggc    240
gcaacggtac tgtttctcgg cttcgtcctg attgccgccg cgtggggcgc gttctcttcc    300
ggcaacaaag gcatactcgt caaacaaggt ttgcccgcat ttttggcagc ggcggcggtg    360
ttggcggtat ga                                                        372
```

<212> Type : DNA
<211> Length : 372
SequenceName : SEQ ID 625
SequenceDescription :
Sequence
--------
<213> OrganismName : Neisseria meningitidis serogroup A strain Z2491
<400> PreSequenceString :

```
atggcttcaa gcaacgtaaa caacgaaatc aaagacaaaa aactctccct gtgggccaag     60
cgtcaagacg gttcggtaaa atggttctgc ggacagccgg ttaagcgcga cgccgccacc    120
gacgccgacg tcaccgccga cagcggcaac gaaatcgaca ccaagcacct gccgtcaacc    180
tgccgcgacg cagcatctgc cgtttgcaca aaaacacccg agtattaccc gaatcacggc    240
gaatggccga aaaacttcgt cattcccgcg caggcgggaa tccaggtctg tcggcacgga    300
aacttatcgg gtaaaaaggt ttctccggtc ctgagttcta gattcccact ttcgtgggaa    360
tga                                                                  363
```

<212> Type : DNA
<211> Length : 363
SequenceName : SEQ ID 626
SequenceDescription :
Sequence
--------
<213> OrganismName : Neisseria meningitidis serogroup A strain Z2491
<400> PreSequenceString :

```
atcctttag ccgaaggtca aaaatcagcc gtcaccgagt attacctgaa tcacggcgaa      60
tggcccagca acaacacttc tgccggcgtg gcaacctcca ccgacattaa aggcaaatat    120
gttcaaagcg ttgaagtcaa aaacggcgtc gttaccgcca caatggcttc aagcaacgta    180
aacaacgaaa tcaaaggcaa aaaactctcc ctgtgggcca agcgtcaaga cggttcggta    240
aaatggttct gcggacagcc ggttaagcgc aacgacaccg ccaccaccaa cgacgacgtc    300
aaagccgaca ccgccgccaa cggcaagcag atcgacacca agcacctgcc gtcaacagca    360
tcgacaagaa aatcgacacc aaactag                                        387
```

<212> Type : DNA
<211> Length : 387
SequenceName : SEQ ID 627
SequenceDescription :
Sequence
--------
<213> OrganismName : Neisseria meningitidis serogroup A strain Z2491
<400> PreSequenceString :

atgcccatcc cctttaaacc cgtattggct gccgccgcca tcgcccaagc gtttcccgcc 60
tttgcggcag accccgcgcc gcagtccgcc caaacgctga acgaaatcac cgttaccggc 120
acgcacaaaa cccaaaaact cggcgaagaa aaaatccgcc gcaaaacttt agacaagctc 180
ttggtcaacg acgaacacga cctggtgcgc tacgaccccg gcatttccgt cgtcgaaggc 240
ggcagggcgg gttctaacgg ctttaccata cgcggcgtgg acaaagaccg cgtcgccatc 300
aacgttgacg ggctggcgca ggcggaaagc cgctcttccg aagccttcca agaattgttc 360
ggcgcgtacg gcaacttcaa cgccaaccgc aacacttccg agccggaaaa cttttccgaa 420
gtaaccatca ccaaaggcgc ggactcgctc aaatccggca gcggcgcatt gggcggcgca 480
gtcaattacc aaaccaaatc cgcaagcgat tatgtttccg aagacaagcc ctaccatttg 540
gggataaagg gcggcagcgt cggcaaaaac agccaaaaat tcagcagcat caccgccgcc 600
ggcaggctct ttggtttgga tgccttattg gtttataccc gccgcttcgg caaagaaacc 660
aaaaaccgct cgaccgaggg cgatatcgaa attaaaaacg acggatatgt ctataacccg 720
accgatacag gcggacccag caagtacctg acctatgtag ccacaggggt tgcgcgctcc 780
caacccgacc cgcaagaatg ggtaaacaaa agcaccctgt tcaagctggg ctacaacttc 840
aacgatcaaa accgtatcgg ctggattttt gaagactcgc gcaccgaccg ttttaccaac 900
gagctgtcta atttgtggac gggtacgacc acgtctgccg caacgggcga ctaccgccac 960
cgccaagacg tgagctaccg ccgccgctcc ggtgtcgaat acaaaaacga attggaacac 1020
ggcccgtggg acagccttaa gctgcgctac gacaagcagc gcatcgatat gaacacttgg 1080
acttgggaca tcccgaaaaa ttacgataaa agaggcatca acggcgaggt ttaccattcg 1140

ttccggcata tccgccaaaa caccgcgcaa tggactgccg attttgaaaa acaactcgac 1200
ttttccaaag ccgtttgggc ggcgcaatac ggcttgggcg gcggcaaagg ggacaatgcc 1260
aactcggatt acagctattt cgcaaaactg tacgacccca aaatcctcgc ttccaaccaa 1320
gccaaaatca caatgctgat cgaaaaccgg tcgaaatata aatttgccta ttggaacaat 1380
gcgtttcact tgggcggcaa cgaccgcttc cgcctgaatg cgggcatacg ctacgacaaa 1440
aacagcagca gcgcgaaaga cgatccgaaa tacaccaccg ccatccgggg gcagattccc 1500
catttgggtt cggaacgcgc gcacgcgggc ttcagctacg gcacggggtt cgactggcgg 1560
tttaccaagc atctgcactt gttggcaaaa tacagcaccg gcttccgcgc accgacttcg 1620
gacgaaactt ggctactgtt cccacacccc gatttctacc tgaaagccaa cccaaacctg 1680
aaagccgaaa aagccaaaaa ctgggaattg ggtctggcgg gcagcggcaa agcgggcaac 1740
ttcaagctct cgggcttcaa aaccaaatac cgcgacttta tcgaattgac gtatatgggc 1800
gtttcgtcag acgataaaaa caaccccaga tacgccccgc tttcagacgg tacggcattg 1860
gtcagctcgc ccgtttggca aaaccaaaac cgctccgccg cctgggtgaa aggcatagag 1920
tttaacggca cgtggaacct cgacagtatc ggtttgccca aagggctgca caccggcctc 1980
aacgtcagct acatcaaggg caaggcaacg caaaacaacg gcaaagaaac gcccatcaac 2040
gcgctttcgc cgtggacggc ggtttacagc ctgggctatg acgcgccttc caaacgctgg 2100
ggcatcaacg cctacgccac gcgcaccgcc gccaaaaagc cgtccgacac cgtccacagc 2160
aacgacgact tgaacaaccc gtggccttat gccaaacaca gcaaggccta tacgctgttc 2220
gacctttccg cctacctcaa catcggcaaa caggttacgc tccgcgccgc cgcgtacaac 2280
attaccaaca agcagtacta cacttgggaa tctttacgca gcatccgcga gttcggcacg 2340
gtcaaccgcg ttgacaacaa aacccacgcc ggcatccaac gctttacctc gccgggcagg 2400
agctacaatt tcaccatcga agcgaagttc taa 2433

<212> Type : DNA
<211> Length : 2433
SequenceName : SEQ ID 628
SequenceDescription :
Sequence
--------
<213> OrganismName : Neisseria meningitidis serogroup A strain Z2491
<400> PreSequenceString :

```
atgaaaaaat ccctgattgc cctgactttg gcagcccttc ctgttgcagc aatggctgac      60
gttaccctgt acggcaccat caaaaccggc gtagaaactt cccgctctgt agaacacaat     120
ggaggtcagg tggttagcgt tgaaaccggt accggcatcg ttgatttggg ttcgaaaatc     180
ggcttcaaag gccaagaaga cctcggtaac ggcctgaaag ccatttggca ggttgagcaa     240
aaggcatcta tcgccggcac tgactccggt tggggcaacc gccaatcctt catcggcttg     300
aaaggcggct tcggtaaatt gcgcgtcggc cgtttgaaca gcgtcctgaa agacaccggc     360
gacatcaatc cttgggatag caaaagcgac tatttgggtg taaacaaaat tgccgaaccc     420
gaagcacgcc tcatttccgt acgctacgat tctcccgaat ttgccggcct cagcggcagc     480
gtacaatacg cgcttaacga caatgtaggc agacataaca gcgaatctta ccacgccggc     540
ttcaactaca aaaacggcgg cttcttcgtg caatatggcg gtgcctataa aagacatcag     600
gatgtggatg acgtgaagat tgagaaatac cagattcacc gtttggtcag cggttacgac     660
aatgatgccc tatacgcttc cgtagccgta cagcaacaag acgcgaaact ggttgaagac     720
aattcgcaca actctcaaac cgaagttgcc gctaccttgg cataccgctt cggcaacgta     780
acgccccgcg tttcttacgc ccacggcttc aaaggctcgg ttgatgatgc aaaacgcgac     840
aatacttacg accaagtggt tgtcggtgcg gaatacgact tctccaaacg cacttctgcc     900
ttggtttctg ccggttggtt gcaagaaggc aaaggcgaaa acaaattcgt agcgactgcc     960
ggcggtgtcg gtctgcgcca caaattctaa                                       990
```

<212> Type : DNA
<211> Length : 990
SequenceName : SEQ ID 629
SequenceDescription :
Sequence
--------
<213> OrganismName : Neisseria meningitidis serogroup A strain Z2491
<400> PreSequenceString :

```
atgaaaaccc tgctcctcct catcccccctc gtcctcacag cctgcggcac actgaccggc      60
atacccgccc acggcggcgg caaacgcttt gccgtcgaac aagaactcgt cgccgcatcg     120
tcccgcgccg ccgtcaaaga aatggacttg tccgccctga aaggacgcaa agccgccctt     180
tacgtctccg ttatgggcga ccaaggttcg ggcaacataa gcggcggacg ctactctatc     240
gacgcactga tacgcggcgg ctaccacaac aaccccgaaa gtgccaccca atacagctac     300
cccgcctacg acactaccgc caccaccaaa tccgacgcgc tctccagcgt aaccacttcc     360
acatcgcttt tgaacgcccc cgccgccgcc ctgacgaaaa acagcggacg caaaggcgaa     420
cgctccgccg gactgtccgt caacggcacg ggcgactacc gcaacgaaac cctgctcgcc     480
aacccccgcg acgtttcctt cctgaccaac ctcatccaaa ccgtcttcta cctgcgcggc     540
atcgaagtcg taccgcccga atacgccgac accgacgtat tcgtaaccgt cgacgtattc     600

ggcaccgtcc gcagccgcac cgaactgcac ctctacaacg ccgaaaccct taaagcccaa     660
accaagctcg aatatttcgc cgttgaccgc gacagccgga aactgctgat tgcccctaaa     720
accgccgcct acgaatccca ataccaagaa caatacgccc tctggatggg accttacagc     780
gtcggcaaaa ccgtcaaagc ctcagaccgc ctgatggtcg atttctccga catcaccccc     840
tacggcgaca caaccgccca aaaccgtccc gacttcaaac aaaacaacgg taaaaaaccc     900
gatgtcggca acgaagtcat ccgccgccgc aaaggaggat aa                          942
```

<212> Type : DNA
<211> Length : 942
SequenceName : SEQ ID 630
SequenceDescription :
Sequence
--------
<213> OrganismName Neisseria meningitidis zerogoup A strain Z2491
<400> PreSequenceString :

```
atgaataaaa ccttgtctat tttgccggtg gcaatcttac tcggcggctg cgccgccggg      60
ggcggtaaca cattcggcag cttagacggc ggcacaggta tgggcggcag catcgtcaaa     120
atggcggtag aaagccaatg ccgtgcggaa ttgaacaaac gcagcgaatg gcgtttgacc     180
gcgctggcga tgagtgccga aaaacaggcg gaatgggaaa acaagatttg cgcttgcgtc     240
gcccaagaag cacccaacca gctgaccggc aacgatgtga tgcagatgct ggatccgtcc     300
acgcgcaatc aggcacttgc cgccctgacc gccaaaacgg tttccgcctg cttcaaacac     360
ctgtaccgct aa                                                          372
```

<212> Type : DNA

<211> Length : 372
SequenceName : SEQ ID 631
SequenceDescription :
Sequence
--------
<213> OrganismName : Neisseria meningitidis serogroup A strain Z2491
<400> PreSequenceString :

```
atgaatccac ttattcatca agcaaaggaa tcatctatgc aaacccgcat cctctccgcc      60
gtactgctgg ctttttcaac cgctgccttt gccgggggcg cattcacgct gcaattcgac     120
aacccgtccg aagacggcgg cttcacgcaa aaccagattt tgagcgcgcc ttacggcttt     180
ggctgttcgg gcggcaatgc ttcgcccgcg ctgtcgtgga aaaatccgcc cgccgggaca     240
aaaagtttcg tcctgaccgt ttacgataaa gacgcgccga ccggactggg ctggatgcac     300
tgggtggtcg ccgacattcc cgccgatgtc cgccgccgca atgcgacctc gctgcaatta     360
agccgctgcg ccagcatcgc cgacgaccag tccgcagcca tatcggcagt aatcagtttg     420
cagatttgcc gcatcaggtt gacgccttcg tacacggcaa aaccgatgcc gtcatgctgc     480
aaccacgcca acacgccgca aagcgcggcc tccgccgcat tgtgcggcac ttcttcatcc     540
gtcagcaccg ccgccgcata a                                               561
```

<212> Type : DNA
<211> Length : 561
SequenceName : SEQ ID 632
SequenceDescription :
Sequence
--------
<213> OrganismName : Neisseria meningitidis serogroup A strain Z2491
<400> PreSequenceString :

```
atgaataaaa ctttaaaaag gcgggttttc cgccataccg cgctttatgc cgccatcttg      60
atgttttccc ataccggcgg gggggggggg gcgatggcgc aaacccgtca atacgctatt     120
atcatgaacg agagaaacca gcccgaggta cagtggaatg ggtcatattc aataaaggac     180
aaagacagga agcgcgaata tactcatcat aatcaccaac aaggaggaag ctctgtctca     240
ttcaacaata gcgatgagct tgtttctcga caaagcggta ctgccgtttt tggcacagcc     300
acctacctgc cgccctacgg caaggtttcc ggttttgatg ccgccgctct gaaagagcgc     360
aacaatgccg tcgattggat tcataccacc cacccagggt tgataggcta cagctacgac     420
ggtgtcgtat gcagaagcgc cacagactgt cccaaacttg tctataaaac ccgattttcc     480
ttcgataatc ccgacttggc aaaaacagga ggcgggttgg ataagcacac agagccaagc     540
cgcgacaatt cgcccattta caaattgaag gatcatccat ggttgggcgt gtctttcaat     600
ttgggtgccg agggtatcgc caaaaatggt aagacaatca acaaattggt atcttctttt     660
aatgaaaaga atagtaataa caacctcgtc tataccacgg aaggccgcga tatttccttg     720
ggcaactggc agcgcgaaac gaccgccatg gcctattatc tgaacgccaa gctgcacctg     780
ctggataaaa aacagattca aaatatcacc gacaaaacag tgcagttggg tgtcttgaag     840
ccgagcatcg atgtgcggac aagaaatacg gggactgccg gcattctatc ttattgggct     900
aagtgggaca ttaaagatac cgggcagatt ccagtcaagc tcagcttgac gcaagtcaaa     960
gcaggccgct gcgtcaacaa agataacccc aataagaata ccaaaacctc ttcccccgca    1020
```

```
ctgactgccc ccgcgctgtg gttcggagct gggcaagatg gtaaggcgga gatgtattcc    1080
gcttcggttt ccacctaccc cgacagttcg agcagccgca tcttccttca aaatctgaaa    1140
agaaaaaccg acaccagcag acccggccgc tattccctcg caaccttgaa taagtcggat    1200
attgaaagtc gagagccgag tttcacaagt cggcaaaccg tcatccgatt ggatggcggc    1260
gtacagcaga tcaaactgga tagaaacaat actgaggtca ctggttttaa tggaaatgac    1320
ggcaaaaacg acactttcgg cattgttagt gaagggagct tcatgcctga tgccagcgag    1380
tggaaaaaag tattgctgcc ttggacggtt cgtgctttca attatgacgg tcgatttaac    1440
acagtcaaca aagaagaaaa caacggcaag ccaaaataca gtcaaaaata ccgcagccgc    1500
aacaacggca agcacgagcg caatttgggc gacatcgtca acagccccat cgtggcggtc    1560
ggcgagtatt tggctacttc cgccaacgac gggatggtgc atatcttcaa acaaagcggc    1620
ggggacaagc gcagctacaa tctgaagctc agctacatcc ccggcacgat gccgcgcaag    1680
gatattgaaa gcaaagactc caccccttgcc aaagagctgc gcgcctttgc cgaaaaaggc    1740
tatgtgggcg accgctacgg cgtggacggc ggctttgtct tgcgccgcat tacagatgac    1800
caagacaagc aaaaacactt ctttatgttc ggcgcaatgg gccttggcgg cagaggcgca    1860
tacgccttgg atttgaccaa agccgacgac aatgacccga caaaagcctc tttgtttgat    1920
gtaaaagata acggcaataa tggcaataac ggcaataatc gcgtggaatt aggctacacc    1980
gtcggcacgc cgcaaatcgg caaaacccac aacggcaaat acgccgcctt cctcgcctcc    2040
ggttatgcga ctaaacagat tgacagcggc gagaataaaa ccgcgctgta tgtgtatgat    2100
ttggaaagca acaacggtac gctgattaga aaaatcgaag taaccgacgg caagggcggg    2160
ctttcgtccc ccacgctggt ggataaagat ttggacggca cggtcgatat cgcctatgcc    2220
ggcgatcgcg gcggcaagat gtaccgcttt gatttaagcg gcaacaaccc gaacagttgg    2280
actgtacgca ctattttcca aggcacgaag ccgattactt ccgcgcccgc catttcccaa    2340
ctgaaagaca aacgcgtggt tatcttcggt acgggcagtg atttgagtga ggatgatgta    2400
ctcagtacgg atgaacaaca tatttacggt atttttgaca atgacacaaa cacgggtacg    2460
gcgcaagagg ggctgggcaa agggctgctc gagcaaaagc ttagtgagga aaataaaacc    2520
ttattcctga ccgattataa gcgatccgac ggctcgggcg acaagggctg ggtagtgaaa    2580
ttgaaggacg gacagcgcgt taccgtcaaa ccgaccgtgg tattgcgtac cgcctttgta    2640
accatccata aatatacggg taatgacaaa tgcggcgcgg aaaccgccat tttgggcatc    2700
aataccgccg acggcggcaa gctgaccaag aaaagcgcgc gcccgattgt gccggcagcc    2760
aattcgaagg tcgcgcaata ttccggcgat aagaaaactt ccagcggcaa atccatccct    2820
ataggttgta tggaaaaaga cgggggaacc gtctgcccga acggatatgt ttacgacaaa    2880
ccggttaatg tgcgctacct ggacgaaaag aaaacagacg gattttcaac aacggcagac    2940
ggcgatgcgg gcggcagcgg aacattcaaa gagggtaaaa aacccgcccg caataaccgg    3000
tgcttctccg gaaaaggggt gcgcaccctg ctgatgaacg atttggacag cttggatatt    3060
accggcccga tgtgcggtat gaaacgaatc agctggcgtg aagtcttcta ctga          3114
```

<212> Type : DNA
<211> Length : 3114
SequenceName : SEQ ID 633
SequenceDescription :
Sequence
--------
<213> OrganismName : Neisseria meningitidis serogroup A strain Z2491
<400> PreSequenceString :

```
atgaaacacc ccaaactcac ccttatcgcc gcattgctga ccactgccgc aactgccgcc      60
cccctgccgg ttgtaaccag cttcagcatt ttaggcgacg tagccaaaca aatcggcgga     120
gagcgcgtat ccatacaaag tttggtcgga gccaaccaag atacgcacgc ctatcatatg     180
accagcggcg acattaaaaa aatccgcagt gcaaaactcg tcctgattaa cggcttagga     240
cttgaagctg ccgacatcca acgtgccgtc aaacagagca aagtatccta tgccgaagcg     300
accaaaggca tccaaccccct caaagccgaa gaagaaggcg gacaccatca cgaccacgat    360
catgaccacg accatgacca cgaaggacac caccacgacc acggcgaata tgaccccccac    420
gtctggaacg accccgtcct tatgtccgcc tatgcccaaa acgtcgccga agccctgata     480
aaggccgacc ccgaaggcaa agtttattat caacaacgct tgggcaacta ccaaatgcag     540
ctcaaaaaac tgcacagtga cgcacaagcc gcatttaatg ccgtccctgc cgccaaacgc     600
aaagtcctga ccgggcacga tgccttttcc tatatgggca aacgttacca tatcgaattc     660
atcgccccac aaggtgtgag cagcgaagcc gagccttcag ccaaacaagt cgccgccatc     720
atccgacaaa tcaaacgcga aggcatcaaa gccgtattta ccgaaaatat caaagacacc     780
cgcatggttg accgcatcgc caaagaaacc ggtgtcaacg tcagcggcaa actgtattcc     840
gacgcactcg gcaacgcacc cgcagacacc tacatcggca tgtaccgcca caacatcaaa     900
gccttaacca acgcgatgaa gcaataa                                         927
```

<212> Type : DNA
<211> Length : 927
SequenceName : SEQ ID 634
SequenceDescription :

Sequence
--------
<213> OrganismName : Streptococcus pyogenes strain MGAS8232
<400> PreSequenceString :

```
atgaaaaaaa gaattttatc agcagttctt gtaagtggtg ttaccctcgg agcagctaca      60
actgtaggag cggaggattt gagtactaag attgctaagc aggattctat tatctcaaat     120
ctgactacag agcaaaaagc tgcacagaat caagtttcag cgttacaggc tcaagtaagt     180
tcactacaat ctgaacaaga taaactgacc gcaagaaata cagaacttga ggcactttca     240
aagcgatttg agcaagaaat taaggctcta acaagtcaaa ttgttgctcg taatgaaaaa     300
ttaaaaaatc aagctcgtag tgcttataaa aacaatgaaa cttctggtta cattaatgca     360
cttttgaatt ctaaatcaat ttctgatgtt gtaaaccgtt tagtagcaat taatagagct     420
gtctctgcta acgctaaatt gttagaacaa caaaaagctg ataaagtttc ccttgaagaa     480
aagcaagctg ctaaccaaac agctattaat accattgccg ctaatatggc aatggctgaa     540
gaaaaccaaa atacattacg tactcaacaa gctaatttgg aagctgcaac tgcaaattta     600
gctctccaat tagcatctgc tactgaagat aaagctaatt tggtagctca aaaagaagct     660
gcagaaaaag ctgctgctga agccttagca caagaacagg ctgctaaagt taaggcacaa     720
gaacaggctg cacaacaagc agcatctgtt gaagcagcaa aatctgctat tactccagca     780
ccacaagcta ctccggcagc gcaaagtagt aatgctattg aaccagctgc actcacggct     840
ccggcagctc cttctgcaag accacaaaca tcatatgatt cttctaatac ttatccagtt     900
ggacaatgca catggggagc taaatcttta gctccttggg caggaaataa ttggggaaat     960
ggtggtcaat gggcttatag tgctcaagca gctggttatc gtactggttc aacgccgatg    1020
gtaggtgcga ttgccgtttg gaacgatggt ggttatggac atgtcgccgt tgtagttgag    1080
gttcaaagtg cctcaagtat tcgtgtgatg gagtctaact acagtggtag acagtacatt    1140
gctgatcacc gtggttggtt taatccaaca ggtgttacat ttatttatcc acactaa        1197
```

<212> Type : DNA
<211> Length : 1197
SequenceName : SEQ ID 635
SequenceDescription :
Sequence
--------
<213> OrganismName : Streptococcus pyogenes strain MGAS8232
<400> PreSequenceString :

```
gtgattacaa ttaaaaatcc aaaaatcctt aagtggctaa agtatgtatt aagtgcaatt      60
cttagcctta ttatccttgt tattattatt ggtggtcttt tgtttacctt ctacattagc     120
agtgctccga aactgtcaga agcccagtta aaatcaacaa actctagctt ggtttatgac     180
ggtaataaca atctgattgc tgatttgggt tctgaaaagc gtgaaaatgt aacagctgat     240
agtatcccta ttaatctagt taatgctatt acctcaattg aagataaacg tttctttaac     300
catcgtggag tagatcttta tcgtattttt ggtgctgcct ttcataatct aacgagtcag     360
accactcaag gggggtcaac gcttgatcag caactcatta aactagccta tttttctact     420
aatgaatctg atcaaacctt aaaacgtaag gctcaagaag tttggcttgc tcttcaaatg     480
gagcgaaaat atactaaaca agaaatcctg actttttaca tcaacaaagt atatatgggt     540
aatggcaact atggtatgct gacagccgct aagtcttatt atggcaagga tcttaaggat     600
ttatcttatg cccagctagc cctattggct ggaatccctc aagctcctag tcaatatgat     660
ccttaccttc atcctgaagc tgctcaaaat cgccgtaacg tcgtgttgca acagatgtac     720
atggaaaaac atctgacgaa agcagaatat gaaactgcca tcgcaactcc cgtcgctgaa     780
ggtctacaat cactccaaca gcgctcaact tatccaaaat atatggataa ttatctaaaa     840
caagttattg aagaagtcaa aaaggaaacg aataaagata tttttaccgc tggtttaaaa     900
gtttatacca atattatccc cgatgcgcag cagactcttt ataatattta tcattctggt     960
gattatgttt actatccaga ccaagatttc caagttgctt caacgattgt tgatgtgaca    1020
aatggtcatg ttattgctca gcttggcgga cgtaatcagg atgaaaatgt ttcatttggg    1080
actaaccaag ctgttttaac tgatcgtgac tggggttcta ccatgaagcc aatcacagcc    1140
tatgctcctg ctattgaatc tggtgtttat acttctactg ctcagtcgac taatgactca    1200
gtctattatt ggcctggaac cactacccaa ttgtttaact gggaccttag atataacgga    1260
tggatgacaa tccaagctgc tattatgcta tcgcgaaatg tcccagcagt ccgagcactg    1320
gaagccgcag gacttgacta tgctcgatct ttcttaagca gtttaggtat taactatccc    1380
gaaatgcact actcaaacgc tatctcaagt aataacagta gctcagataa aaaatatggt    1440
gcaagtagtg aaaaaatggc cgctgcatac gctgcttttg caaatggtgg tatttatcat    1500
aaaccaaggt atgtcaataa agtggaattt agtgatggta caagtaaaac atttgatgaa    1560
aaaggaaaac gtgccatgaa agaaaccacg gcctatatga tgacagatat gttgaaaact    1620
gttctcactt atggtacagg tactgctgct gccattcctg gtgttgcgca agctggtaaa    1680
acagggactt ctaactacac tgatgaggaa ctagctaaaa ttggtgaaaa atacggcctt    1740
tatccagatt atgttggtac attagcgcca gacgaaaact ttgttggctt tactaagcgc    1800
tacgccatgg ctgtttggac aggttacaaa aaccgcttga ccccagtata cggatcaagt    1860
ctagagattg catctgacgt ttatcgtagc atgatgactt acttaacaaa tggttacagt    1920
gaagactgga ccatgccaaa tggtctttat cgcagtggtg gattcctcta cttaagcgga    1980
acctatgcga gcaacaccga ctatactaat tcggtttaca acaatcttta cagcaataac    2040

acaacaacag cttctagcca aacgacttca gatgatacta gtagtagcaa tgatacaagt    2100
aattcaacca atacagacaa caatggcagc catccatcta ccgatgataa aaagacaact    2160
cattaa                                                               2166
```

<212> Type : DNA
<211> Length : 2166
SequenceName : SEQ ID 636
SequenceDescription :
Sequence
--------
<213> OrganismName : Streptococcus pyogenes strain MGAS8232
<400> PreSequenceString :

```
atgattatta ctaaaaagag cttatttgtg acaagtgtcg ctttgtcgtt agcacctttg     60
gtgacagcgc aggcacaaga gtggacacca cgatcggtta cagaaatcaa gtctgaactc    120
gtcctagttg ataatgtttt tacttatact gtaaaatacg gtgacacttt aagcacaatt    180
gctgaagcaa tgggaattga tgtgcatgtc ttaggagata ttaatcatat tgctaatatt    240
gacttaattt ttccagacac gatcctaaca gcaaactaca accaacacgg tcaggcaacg    300
actttgacgg ttcaagcacc tgcttctagt ccagctagcg ttagtcatgt acctagcagt    360
gagccattac cccaagcatc tgccacctct caatcgactg ttcctatggc accatctgcg    420
acaccatctg atgttccaac gacaccatta gcatctgcaa agccagatag ttttgtgaca    480
gcgtcatctg agctcacatc gtcaacgaat gatgtttcga ctgagttgtc tagcgaatca    540
caaaagcagc cagaagtatc acaagaagca gttccaactc ctaaagcagc tgaaacgact    600
gaagtcgaac ctaagacaga catctcagaa gatccaactt cagctaatag gcctgttcct    660
aacgagagtg cttcagaaga agcttcttct gcggccccag cacaagctcc agcagaaaaa    720
gaagaaacct ctcagatgtt aactgcgcca gcggcacaaa aagctgtagc tgacaccaca    780
agtgttgcaa cctcaaacgg cctttcttac gctccaaacc atgcctacaa tccaatgaat    840
gcagggcttc aaccacaaac agcagcgttc aaagaagaag tggcttctgc ctttggtatt    900
acgtcgttta gtggttaccg tccaggagat ccaggagatc atggtaaagg attagccatt    960
gactttatgg taccggttag ctctacgctt ggtgatcaag ttgctcaata tgccattgac   1020
catatggcag agcgtggtat ttcatacgtt atttggaaac agcgattcta tgcgccattt   1080
gcaagtattt acggaccagc ctatacatgg aaccccatgc cagatcgcgg cagtattaca   1140
gaaaaccatt atgatcatgt tcatgtctcc tttaatgctt aa                      1182
```

<212> Type : DNA
<211> Length : 1182
SequenceName : SEQ ID 637
SequenceDescription :
Sequence
--------
<213> OrganismName : Streptococcus pyogenes strain MGAS8232
<400> PreSequenceString :

```
atgaaaaaga aaattctttt aatgatgagt ttaatcagtg tctttttttgc ttggcaactt     60
actcaggcaa aacaagtctt agcagagggt aaagtgaagg tggtgacaac tttctaccct    120
gtttatgaat ttacaaaagg ggttattggt aatgatggcg atgtttccat gcttatgaaa    180
gcaggaacgg aacctcatga ttttgagcct tctacaaaag acattaaaaa aatccaagat    240
gcagatgcat ttgtttatat ggatgacaat atggaaactt gggtttctga tgtgaaaaaa    300
tcattgacat ctaaaaaagt gaccatcgtc aagggaactg gtaacatgct cttggtagca    360
ggagctggac atgaccatca ccatgaggat gctgacaaaa agcatgagca taataaacat    420
agcgaagaag gacacaacca tgcttttgac ccacacgtgt ggttgtcacc ataccgtagc    480
attacggtcg ttgaaaatat tcgcgacagt ctttcaaaag cttacccaga aaaagcagag    540
aacttcaaag ccaatgccgc tacttatatt gaaaaattaa aagagcttga caaagactat    600
acggcagcac tttcagatgc taagcaaaag agctttgtga cacaacacgc agcttttggt    660
tatatggcac ttgactatgg cttgaaccaa atttctatta atggtgtcac accagatgca    720
gaaccatcag caaaacgtat tgctactttg tcaaaatacg ttaaaaaata tggcatcaaa    780
tacatttatt ttgaggaaaa tgcgtcaagt aaagtcgcaa aaaccctagc taaagaagca    840
ggagttaaag cggctgtgct tagtccgctt gaaggtttga ctaaaaaaga gatgaaagct    900
ggccaagatt actttacggt catgcgtaaa aaccttgaaa ccttacgctt aaccactgat    960
gtggctggta aagaaattct tccagaaaaa gacacgacta agacagttta caatggttat   1020
ttcaaagaca aagaagtcaa agatcgtcaa ttatctgact ggtcaggtag ctggcaatct   1080
gtttacccct atttacaaga tggtacttta gaccaagttt gggactataa ggctaaaaaa   1140
tctaaaggta aaatgacagc agccgagtac aaagattact acactactgg ttataaaact   1200
gacgtggaac aaatcaaaat caatggtaag aaaaagacca tgacctttgt tcgtaatggt   1260
gaaaagaaaa ccttcactta cacatacgcc ggcaaagaaa tcttgaccta tccaaaagga   1320
aatcgcggag ttcgtttcat gtttgaagct aaagaaccaa atgctggcga attcaaatac   1380
gttcaattca gtgaccatgc cattgctcct gaaaaagcag agcatttcca cctgtactgg   1440
ggtggtgaca gccaagaaaa attacataaa gagttagaac attggccaac ttactacggt   1500
tcagacttat ctggtcgtga aattgcccaa gaaatcaatg ctcattaa                1548
```

<212> Type : DNA
<211> Length : 1548
SequenceName : SEQ ID 638
SequenceDescription :
Sequence
--------
<213> OrganismName : Streptococcus pyogenes strain MGAS8232
<400> PreSequenceString :

```
atgaaaaaat ttcatcgttt tttggtctca ggagtaatcc ttttaggttt taatggtcta     60
gtacctacta tgccatctac acttatttcg caacaggaaa atcttgttca tgcagctgtt    120
ttaggcgata actatccgag taagtggaaa aaaggcaatg gaatcgattc gtggaacatg    180
tatatccgcc aatgcacttc ttttgcagct tttcgtttaa gctctgctaa tggttttcag    240
ttacctaaag gctacggtaa tgcctgcacg tggggacata tcgcgaaaaa tcagggttat    300
cctgtgaata agacaccaag catagggggct atcgcttggt ttgataaaaa cgcttatcag    360
tcaaatgctg cttacgatca tgtagcatgg gtagctgata tccgtggaga cactgtcact    420
atcgaagagt ataattacaa cgctggacaa ggccctgaaa gataccataa gcgtcaaatt    480
ccaaaatctc aggtaagtgg ttatatccat tttaaagact tatcatctca gacaagtcat    540
tcctacccaa gacaactaaa acacatttct caagcttcat ttgacccctc tggaacttat    600
cactttacaa ccagattacc agtcaaagga caaaccagta tcgatagccc tgatcttgct    660
tactatgaag caggtcaatc tgtttattac gataaagtcg tgactgctgg aggttataca    720
tggcttagct acctcagttt ttctggaaac cgacgctata ttcccattaa agagcccgca    780
cagtctgtgg ttcaaaatga caatacaaaa ccttccatta aggtcggtga tactgttacc    840
ttccctggcg tttttcgtgt agatcagctt gttaataatt tgatcgttaa taaagaatta    900
gccggaggag acccaactcc actaaactgg attgatccca caccattaga tgaaacagat    960
aaccaaggaa aagttttagg aaatcaaatt ctccgtgtgg gtgaatattt taccgtcact   1020
ggtagttata aagtattaaa aattgatcaa ccaagtaatg gtatttatgt tcaaatcgga   1080
tctcgtggaa catgggtaaa tgctgataaa gctaacaaat tatag                   1125
```

<212> Type : DNA
<211> Length : 1125
SequenceName : SEQ ID 639
SequenceDescription :
Sequence
--------
<213> OrganismName : Streptococcus pyogenes strain MGAS8232
<400> PreSequenceString :

```
atgttaaaat ttacttcaaa tattttagct actagtgtag ctgaaacaac tcaagttgct     60
cctggaggct gctgttgctg ctgtactact tgttgcttct caattgctac tggaagtggt    120
aattctcaag gtggtagcgg aagttatacg ccaggtaaat aa                       162
```

<212> Type : DNA
<211> Length : 162
SequenceName : SEQ ID 640
SequenceDescription :
Sequence
--------
<213> OrganismName : Streptococcus pyogenes strain MGAS8232
<400> PreSequenceString :

```
atgggagaat cttattctgt tgaagcggtt ttgacagctg ttgataaaac ctttggcaaa     60
acattacaat cggcaatccg ttcaatcgaa ggcttggaaa agcgttcaac cggtttttca    120
tcggtgtctc aaaaagctag ttccatgttt aaatccatgt taggagcgaa tttagctgga    180
caagctatct cggcaatgac aaggacagtg tcatcaggcc ttggctctat gcttggcgag    240
atgaatagtt cagcgaaagc gtggaaaact tttgacgcta atttagcgga cattgggttt    300
ggaaaaaaac aaattttggc agttaaaacg gcgatgcaag actatgcaac taaaacaatc    360
tactcggcat cagatatggc tagcacgtat gcacagttag cagcagttgg cgtgaaagat    420
accggaaagc tcgtaaaagc ttttggcggt ttagctgcat ctgctgaaaa tccgaagcag    480
gctatgaaat caattagtca gcaaatgaca caagctgttg gaagaccaac agttgcatgg    540
caagacttta ggataatgtt ggaacagacg cctgcaggga tggctaaagt cgctaaatct    600
atgggtaaaa atcttgatga actcgtcgcc gatatccagg cgggtagggt taaaaccagc    660
gatttttttgg aagcggtaaa aaaagcaggc aatgataaga gtttccaaaa gatggcaact    720
gagttcaaaa ctgttgacca agccatcgac ggtatgcgag aaggcttatc caacaaattg    780
caaccagcgt ttgaaaaagt gaaccaattt ggaattagag cgatcgaagc aatcggtaaa    840
caactcgata aagttgattt ttctaagttt gctagtaatc ttgggaaatt ccttgaagga    900
attaatatcg ataaaattgt atctaatatt tcatcggcgg tttcatctgt cacttcaaag    960
gttaaagaat tttgggacgg tttcaaacaa actggagcaa ttagtgcttt ttcaggagct   1020
ttgcagagcg tttggggagc tttaaaaaat gtcgctagcg ccatgagcgg agggaattgg   1080
```

```
aagacttttg gagcaacagt tggagggatt gttaaacacg tctctaactt cgctaaagct    1140
gtttccgatg ttttaggaaa gatggaccct ggcagactaa gaagttggat agctaccttc    1200
gccgcagtag ctggaggttt taagttattc gaaaaattaa cgggacaaag cgtcattggt    1260
tctttttttgg ataaaattgg cagcaaattt ggtctctttg gaaacaaagc caaagaagga   1320
acagacaaag cctctaacgg cgctagaaga agcggtggca ttattagcca aatcttcagc    1380
ggcttgggta atatcgttaa gtctgctggt acagccatat caacagctgc aaaaggtatc    1440
ggagttggta ttaaaactgc tttgtctgga atcccccct atcattag                  1488
```

<212> Type : DNA
<211> Length : 1488
SequenceName : SEQ ID 641
SequenceDescription :
Sequence
--------
<213> OrganismName : Streptococcus pyogenes strain MGAS8232
<400> PreSequenceString :

```
atgaaaaaag gttttttttct catggttatg gtcgtgagtt tagtaatgat agcagggtgt   60
gataagtcag caaaccccaa acagcctacg caaggcatgt cagttgtaac cagcttttac    120
ccaatgtatg cgatgacaaa agaagtatct ggagacctaa atgatgtgag gatgatccaa    180
tcaggtgcag gcattcattc ctttgaaccg tctgtaaatg atgtagcagc tatttatgac    240
gcggatttgt ttgtttacca ttcacatacc ttagaagctt gggcaaggga tctagaccct    300
aatttaaaaa aatcaaaggt tgatgtgttt gaagcgtcaa aacctttgac actagataga    360
gtcaaagggc tagaagatat ggaagtcaca caaggcattg accctgcgac actttatgac    420
ccacatacct ggacggatcc cgttttagct ggtgaagaag ctgttaatat cgctaaagag    480
ctaggacgtt tggatcctaa acacaaagac agttacacta aaaatgctaa ggctttcaaa    540
aaagaagcag agcaactaac tgaagaatac actcaaaaat ttaaaaaggt gcgctcaaaa    600
acattcgtga cgcaacacac ggcattttct tatctggcta aacgattcgg cttgaaacaa    660
cttggtatct cgggtatttc tccagagcaa gagccctctc ctcgccaatt gaaagaaatt    720
caagactttg tcaaagaata caacgtcaag actatttttg cagaagacaa tgtcaatccc    780
aaaattgctc atgctattgc gaaatcaaca ggagctaaag taaagacatt aagtccactt    840
gaagctgctc caagcggaaa caagacatat ctagaaaatc ttagagcaaa tttggaagtg    900
ctctatcaac agttgaagta a                                              921
```

<212> Type : DNA
<211> Length : 921
SequenceName : SEQ ID 642
SequenceDescription :
Sequence
--------
<213> OrganismName : Streptococcus pyogenes strain MGAS8232
<400> PreSequenceString :

```
gtggagaaaa agcaacgttt ttcccttaga aaatacaaat caggaacgtt ttcggtctta      60
ataggaagcg ttttcttgat gatgacaaca acagtagcag cagatgagct aagcacaatg     120
agtgaaccaa caatcacgaa tcacactcaa caacaagcgc aacatctcac caatacagag     180
ttgagctcag ctgaatcaaa atctcaagac acatcacaaa tcactcccaa gacaaatcgt     240
gaaaaagagc aaccacaagg tctagtctct gagccaacca caactgagct agctgacaca     300
gatgcagcac caatggctaa tacaggtcct gatgcgactc aaaaaagcgc ttctttaccg     360
ccagtcaata cagatgttca cgattgggta aaaaccaaag gagcttggga caagggatac     420
aaaggacaag gcaaggttgt cgcagttatt gacacaggga tcgatccggc ccatcaaagc     480
atgcgcatca gtgatgtatc aactgctaaa gtaaaatcaa aagaagacat gctagcacgc     540
caaaaagccg ccggtattaa ttatgggagt tggataaatg ataaagttgt ttttgcacat     600
aattatgtgg aaaatagcga taatatcaaa gaaaatcaat tcgaggattt tgatgaggac     660
tgggaaaact ttgagtttga tgcagaggca gagccaaaag ccatcaaaaa acacaagatc     720
tatcgtcccc aatcaaccca ggcaccgaaa gaaactgtta tcaaaacaga agaaacagat     780
ggttcacatg atattgactg gacacaaaca gacgatgaca ccaaatacga gtcacacggt     840
atgcatgtga caggtattgt agccggtaat agcaaagaag ccgctgctac tggagaacgc     900
tttttaggaa ttgcaccaga ggcccaagtc atgttcatgc gtgtttttgc caacgacgtc     960
atgggatcag ctgaatcact ctttatcaaa gctatcgaag atgccgtggc tttaggagca    1020
gatgtgatca acctgagtct tggaaccgct aatggggcac agcttagtgg cagcaagcct    1080
ctaatggaag caattgaaaa agctaaaaaa gccggtgtat cagttgttgt agcagcagga    1140
aatgagcgcg tctatggatc tgaccatgat gatccattgg caataaatcc agactatggt    1200
ttggtcggtt ctccctcaac aggtcgaaca ccaacatcag tggcagctat aaacagtaag    1260
tgggtgattc aacgtctaat gacggtcaaa gaattagaaa accgtgccga tttaaaccat    1320
ggtaaagcca tctattcaga gtctgtcgac tttaaaaaca taaaagatag cctaggttat    1380
gataaatcgc atcaatttgc ttatgtcaaa gagtcaactg atgcgggtta taaagcacaa    1440
gacgttaaag ataaaattgc tttaattgaa cgtgatccca ataaaaccta tgacgaaatg    1500
attgctttgg ctaagaaaca tggagccctg ggagtactta tttttaataa caagcctggt    1560
```

```
caatcaaacc gctcaatgcg tctaacagct aatgggatgg ggataccatc tgctttcata 1620
tcgcacgaat ttggtaaggc catgtcccaa ttaaatggca atggtacagg aagtttagag 1680
tttgacagtg tggtctcaaa agcaccgagt caaaaaggca atgaaatgaa tcatttttca 1740
aattggggcc taacttctga tggctattta aaacctgaca ttactgcacc aggtggcgat 1800
atctactcta cctataacga taaccactat ggtagccaaa caggaacaag tatggcctct 1860
cctcagattg ctggcgccag ccttttggtc aaacaatacc tagaaaagac tcagccaaac 1920
ttgccaaaag aaaaaattgc tgatatcgtt aagaacctat tgatgagcaa tgctcaaatt 1980
catgttaatc cagagacaaa aacgaccacc tcaccgcgtc agcaaggggc aggattactt 2040
aatattgacg gagctgtcac tagcggactt tatgtgacag gaaaagacaa ctatggcagt 2100
atatcattag gcaacatcac agatacgatg acgtttgatg tgactgttca caacctaagc 2160
aataaagaca aaacattacg ttatgacaca gaattgctaa cagatcatgt agacccacaa 2220
aagggccgct tcactttgac ttctcgctcc ttaaaaacgt accaaggagg agaagttaca 2280
gtcccagcta atggaaaagt gactgtaagg gttaccatgg atgtctcaca gttcacaaaa 2340
gagctaacaa aacagatgtc aaatggttac tatctagaag gttttgtccg ctttagagat 2400
agtcaagatg accaactaaa tagagtaaac attccttttg ttggttttaa aggacaattt 2460
gaaaacttag cagttgcaga agagtccatt tacaggttaa aatctcaagg caaaaccggt 2520
ttttactttg atgaatcagg tccaaaagac gatatctatg tcggtaaaca ctttacagga 2580
ctcgtcactc ttggttcaga gaccaatgtg tcaaccaaaa cgatttctga caatggtcta 2640
cacacacttg gtacctttaa aaatgcagat ggcaaattta tcttagaaaa aaatgcccaa 2700
ggaaaccctg tcttagccat ttctccaaat ggtgataaca accaagattt tgcagccttc 2760
aaaggtgttt tcttgagaaa atatcaaggc ttaaaagcaa gtgtctacca tgctagtgac 2820
aaggaacaca aaaatccact gtgggtcagc ccagaaagct ttaaaggaga taaaaacttt 2880
aatagtgaca ttagatttgc aaaatcaacg accctgttag gcacagcgtt ttctggaaaa 2940
tcgttaacag gagctgaatt accagatggg tattatcatt atgtagtgtc ttattaccca 3000
gatgtggtcg gtgccaaacg tcaagaaatg acatttgaca tgattttaga ccgacaaaaa 3060
ccggtactat cacaagcaac atttgatcct gaaacaaacc gattcaaacc agaacccccta 3120
aaagaccggg gattagctgg tgttcgcaaa gacagtgtct tttatctaga aagaaaagac 3180
aacaagcctt atacagttac gataaacgat agctacaaat atgtctcagt agaagacaat 3240
aaaacatttg tggagcgaca agctgatggc agctttatct tgccgcttga taaagcaaaa 3300
ttaggggatt tctattacat ggtcgaggat tttgcaggga acgtggccat cgctaagtta 3360
ggagatcacc taccacaaac attaggtaaa acaccaatta aacttaagct tacagacggt 3420
aattatcaga ccaaagaaac gcttaaagat aatcttgaaa tgacacagtc tgacacaggt 3480
ctagtcacaa atcaagccca gctagcagtg gtgcaccgca atcagccgca aagccagcta 3540
acaaagatga atcaggattt ctttatctca ccaaacgaag atgggaataa agacttcgtg 3600
gcctttaaag gcttgaaaaa taacgtatat aatgacttaa cggttaacgt atacgctaaa 3660
gatgaccacc aaaaacaaac ccctatctgg tctagtcaag caggcgctag tgcatcagct 3720
attgaaagta cagcctggta tggcataaca gcccgaggaa gcaaggtgat gccaggtgat 3780
tatcagtatg ttgtgactta tcgtgacgaa catggtaaag aacatcaaaa gcagtacacc 3840
atatctgtga atgacaaaaa accaatgatc actcagggac gttttgatac cattaatggc 3900
gttgaccact ttactcctga caagacaaaa gcccttggct catcaggcat tgtccgcgaa 3960
gaagtctttt acttggccaa gaaaaatggc cgtaaatttg atgtgacaga aggtaaagat 4020
ggtatcacag ttagtgacaa taagatgtat atccctaaaa atccagatgg ttcttacacc 4080
atttcaaaaa gagatggtgt cacactgtca gattattact accttgtcga agatagagct 4140
ggtaatgtgt cttttgctac cttgcgtgac ctaaaagcgg tcggaaaaga caaagcagta 4200
gttaactttg gattagactt accggtccct gaagacaaac aaatagtgaa ctttacttac 4260
cttgtgcggg atgcagatgg taaaccgatt gaaaacctag agtattataa taactcaggt 4320
aacagtctta tcttgccata cggcaaatac acggtcgaat tgttgaccta tgacaccaat 4380
gcagccaaac tagagtcaga taaaatcgtt tcctttacct tatcagctga taataacttc 4440
caacaagtta cctttaagat gacgatgtta gcaacctctc aaataactgc ccactttgat 4500
catcttttgc cagaaggcag tcgcgttagc cttaaaacag ctcaaggtca gctgatcccg 4560
cttgaacagt ccttgtatgt gcctaaagct tatggcaaaa ccgttcaaga aggcacttac 4620
gaagttgttg tcagcctgcc taaaggctac cgtatcgaag gcaacacaaa ggtgaatacc 4680
ctaccaaatg aagtgcacga actatcatta cgccttgtca aagtaggaga tgcctcagat 4740
tcaactggcg atcataaggt tatgtcaaaa aataattcac aggctttgac agcctttgcc 4800
acaccaacca agacaacgac ctcagcaaca gcaaaagccc taccatcagc gggtgaaaaa 4860
atgggtctca agttgcgcat agtaggtctt gtgttactcg gacttacttg cgtctttagc 4920
cgaaaaaaat caaccaaaga ttga 4944
```

<212> Type : DNA
<211> Length : 4944
SequenceName : SEQ ID 643
SequenceDescription :
Sequence
--------
<213> OrganismName : Treponema pallidum subsp. pallidum str. Nichols
<400> PreSequenceString :

atgatgcggt cacttttttc aggtgtgtct ggtatgcaga atcatcaaac gcgcatggat 60

gtcattggga acaacgtcgc gaacgttaac actaccggtt ttaagcgtgg gcgtgttaat 120
tttcaagatc ttatttctca gcaactgagt gcggctgcgc gtccgaatga agaagttgga 180
ggagtgaatc ccaaggaagt gggattgggc gtgctgattg caagcatcga tactgttcac 240
acgcaaggtg cactgcaaac gacgggtatc aatacggatg tgtctattca ggggagtggt 300
ttttttgtgc tgaaaagtgg ggaaaagacg tttttcaccc gcgcaggtgc ctttggggtt 360
gataatgcgg gcactctcgt gaaccctgcg aatggtatgc gcgttcaagg ttggatggcg 420
caggacgtgg cgggggagcg tttaattaat tcctctgcac agacgcagga tctcgttatc 480
cccattgggc aaaagataga tgcgcagcag accagcactg ttcactatgc ctgtaattta 540
gacaagcgtc tgcctgagct tgctgcagat gcgaacgaag cggacgtgcg taagtccacg 600
tggacaactg actttcaagt gtatgatagc ttcgggcagc agcatacgtt gcagattaac 660
ttttcgcgtg tgccggggac gaacaatcag tggcaggcca ctgtcgcagt ggatccgggg 720
acagaggtag atacgcaaac gcgtgtaggg gtggggacat ctgacggtgc ggcaaacacc 780
tttattgtaa attttgataa ttttggacac ctcgcttcag tgactgacac tgcagggaac 840
gtgaccggtc ctaccggaca ggtgctcctt gaagcgtcgt acgatgttgt cggtgcgaat 900
ccggacgatg cagggcaggt tacgcgccac gctttcacgc tcaacttggg tgaaattggc 960
accgcgcgca atacgattac gcagtttgct gaacgcagta ctaccaaagc ctaccggcag 1020
gacggttacg cgatgggata tttggaaaat tttaaaatag atcaaagcgg tgtcatcact 1080
ggtgtgtatt caaatggggt gagccaagac attggccagc tcgcacttgc aggatttgca 1140
aatcaaggtg gtcttgagaa ggcaggagag aacacctacg tacaatcgaa caactcaggg 1200
atagcgaaca ttagcacgtc gggggtgatg ggaagggaa agttgattgc agggacactt 1260
gagatgagca acgtagattt aaccgatcaa tttacggata tgatcattac ccaaaaaggg 1320
tttcaggcgg gcgcaaagac gattcagaca tcagacacca tgttggatac ggtgttgagt 1380
ttgaagcgct ga 1392

<212> Type : DNA
<211> Length : 1392
SequenceName : SEQ ID 644
SequenceDescription :
Sequence
--------
<213> OrganismName : Treponema pallidum subsp. pallidum str. Nichols
<400> PreSequenceString :

```
atggggtgca tgcggtgggg gagtgtgctg tgtgtggtgg tgggggtagg agcgagcggg    60
ggagtgctcg gacaggagtt ttccccgaag ctaactggct ctgccacact tgagtggggc   120
atcagctatg gcaagggggt aggcagtcat ggccaggccc ctggtgcagt tatgggcacc   180
ggtccctaca atctgaagca cgggtttcgt actaccaaca cggtgggagt atcctttccc   240
ctggttatgc gcaccaccca cacgcgccgt gggcagcacc cggcactgta tgcggagctg   300
aaggtggcgg acctgcaggc ggacctgagt caggggaagg caggttttgc cgttaagcgc   360
aaggggaagg tagaggcgac actacactgt tatggggcct acctgacgat tgggaagaac   420
cccacgtttc tgacgaactt tgcccggctg tggaagccgt gggtgacagc gcagtaccag   480
gaggatgcgg tacagtatgc gccggggttt gggggtttag gcggcaaggt tgggtatcgg   540
gcacaggaca ttgggggcag tggggtcagc cttgatgtgg ggtttctctc ctttgcctct   600
aacggtgcct gggatagtac tgaccccacg cacagtaagt atggctttgg ggcagacttg   660
aagctaatgt atgcgcgtgc aggacaccct ctgtgcacgg tagagcttgc cagcaatgtt   720
acgctagaag acggatacct catcggtgca cagaaggacg caaacaatca gaacaaggat   780
aaactgctgt ggaatgtagg gggccgactc accctcgaac caggcgccgg cttccgcttc   840
tccttcgccc tcgacgccgg taaccaacac cagagtgcac aggactttca aaatcgcaca   900
cagagggcgc agagtgaact caccgccctc tcaaataacc tcttccaggg agaaagtcaa   960
aaacaggaag cctgggtaac ccaggtagtg caacaggcga cgcagacagt aacggctgga  1020
gttcgaagcg cgctggaatc tcgggggact acgtacataa acgcgctaga ggcagttcag  1080
cctaatcctg ctaaacctac cggtaaggtt gtgcaaaatc ttcacacccc gcagggaagt  1140
ccgccgaacc tgccgccgct tcctgcactt cctgcatttt ccctgatggg gcaggttttg  1200
ctgcagtacg atgcggagca ggtggtgaag gggtttgagc aggtacagac gcaaatcgtc  1260
actgaaatta atcagaaagt gcaagcggct gtggcaaaaa ataatgcaaa catgcaagcg  1320
gtcgggggta gtctaggcga tactgcgaga atggtaggcg aagcgctcat taagcagcaa  1380
ctatcacgta agcagaacag cattctgacc atggtgagcg tgcaagatga ggtgaaacag  1440
gatctggcag atttagtgcc gatgatgcga acggaaataa cggcgttttt cgcgagtgtc  1500
cagcaacaca taaccgaaga agtgaagaag aagacggatg cgttgaatgc ggggcagcag  1560
atacgtcagg ctatacagaa cctgcgtgcg tctgcatggc gtgcctttct aatgggagtc  1620
agcgccgtgt gtctgtatct tgacacctac aatgtcgcct tcgatgcgct gtttacggcg  1680
cagtggaagt ggctgtcttc tggcatatac tttgccacag caccggcaaa cgtttttggc  1740
accagggtgt tagataacac catcgcaagc tgtggcgact ttgccggatt ccttaagctc  1800
gaaactaaga gcggtgaccc ctacacccac ctgctcaccg gcctggacgc cggcgttgaa  1860
acacgcgtgt acatccccct cacccatgac ctgtacaaaa ataataacgg gaaccctctc  1920
ccttccggcg gttcctcagg gcacattggc ctgccggtgg tggggaaggc gtggtgtagc  1980
tatcgcatcc cggtgcagga ttacggctgg gtgaagccaa gcgttacggt ccatgcctct  2040
accaaccgtg cacacctgaa tgcccctgct gcaggtggag cagtaggagc tacctatcta  2100

accaaggagt actgtgcaca gctgcgtgct ggtatttcag ccagtctcat agagaagacg  2160
gtattctccc ttgattggga acagggtatg ctctctgatg tcccgtacct gctggtgtcc  2220
gagtgcctca cccagggaat cggccgcatc gtgtgcggcg tcaccctctc ctggtag     2277
```

<212> Type : DNA
<211> Length : 2277
SequenceName : SEQ ID 645
SequenceDescription :
Sequence
--------
<213> OrganismName : Treponema pallidum subsp. pallidum str. Nichols
<400> PreSequenceString :

```
gtgggcaggc aggtgatgca agcgggggta cttgcgggca tggtatgtgc tgcttctggt 60
tatgcaggcg tactcactcc gcaggtcagt ggcacagccc agctccagtg gggcattgcg 120
ttccagaaga atccacgcac tggcccgggc aagcacaccc atgggtttcg cactaccaat 180
agtctgacta tttccctgcc gttggtgtca aagcacaccc acacccgccg aggggaggca 240
cgctcagggg tgtgggcaca gctgcagctg aaggacctgg cagtagagct tgcgtcttct 300
aaaagctcaa cggccctgtc ctttaccaaa cctaccgctt ccttccaggc aaccctgcac 360
tgttatgggg cctacctgac agtgggtacc agtccttcct gtgtggttaa ctttgcccag 420
ctgtggaaac cctttgtcac ccgtgcctat tcagaaaagg acactcgcta tgcccctggt 480
ttctccggct ccggggcaaa actcggctac caggcccaca atgtgggaaa cagcggagta 540
gatgtggaca tcggtttcct ctccttcctt tccaatggtg cctgggatag tactgacacc 600
acgcacagca agtatggctt cggggccgat gcaacgcttt cctatggcgt cgaccgtcag 660
cggctgctta cgttggagct ggcagggaat gccacactgg accagaacta cgttaagggt 720
accgaagact ccaagaacga aaacaaaaca gcactcctgt ggggagtagg aggccgactc 780
accctcgaac caggcgccgg cttccgcttc tccttcgccc tcgacgccgg taaccaacac 840
cagagtaacg cacatgctca gacccaagag agagctatcc tcaaagcaag ggaagtgttt 900
agacgggtgg aggggaaact cgtgcagaac cttcccaata tcatgatgcc accaggaatc 960
accgaacaaa ccactctcat agagatggta ggacttgctg ctttgattgc agaaggaacg 1020
ctcggcagcg ccattcaaac cgtgctagcc gctggcgcgc tcgcggcgct tgtatcgcaa 1080
cttgtaccga acatagagca aggagtacgt gatgtcttcc gctcttccga tccaagagtt 1140
gtcactgcta aacttctcgc tttccttgag cgcgcaccta tgaacgcgct caacatagac 1200
gcgctcctgc gtatgcagtg gaagtggctc tcttctggca tatactttgc caccgcaggc 1260
actaatatct ttggcaaacg cgtctttgct accactcgtg cgcactactt tgattttgcc 1320
ggattcctta agctcgaaac caaaagcggt gacccctaca cccacctgct caccggcctg 1380
aacgccggcg tcgaagcacg cgtgtacatc cccctcacct acatccgtta cagaaataac 1440
ggagggtacg aactgaatgg agctgtgccc cctgggacta tcaatatgcc aattttgggg 1500
aaggcgtggt gcagctatcg catccccctc ggttcccacg cctggcttgc accacacaca 1560
tccgtgctcg gcacaaccaa tcgctttaac attattaacc ccgcgggcaa cctgttgaat 1620
gaacgagcgc tccagtacca ggtgggactg acgttcagtc ccttcgagaa ggtggagctc 1680
agcgcccagt gggaacaggg cgtgcttgct gacgctcctt acatgggcat tgccgagagc 1740
atctggtccg aacgccactt cggcaccctt gtctgcggaa tgaaagtgac atggtaa 1797
```

<212> Type : DNA
<211> Length : 1797
SequenceName : SEQ ID 646
SequenceDescription :
Sequence
--------
<213> OrganismName : Treponema pallidum subsp. pallidum str. Nichols
<400> PreSequenceString :

```
gtgggcaggc aggtgatgca agcgggggta cttgcgggca tggtatgtgc tgcttctggt 60
tatgcaggcg tactcactcc gcaggtcagt ggcacagccc agctccagtg gggcattgcg 120
ttccagaaga atccacgcac tggcccgggc aagcacaccc atgggtttcg cactaccaat 180
agtctgacta tttccctgcc gttggtgtca aagcacaccc acacccgccg aggggaggca 240
cgctcagggg tgtgggcaca gctgcagctg aaggacctgg cagtagagct tgcgtcttct 300
aaaagctcaa cggccctgtc ctttaccaaa cctaccgctt ccttccaggc aaccctgcac 360
tgttatgggg cctacctgac agtgggtacc agtccttcct gtgtggttaa ctttgcccag 420
ctgtggaaac cctttgtcac ccgtgcctat tcagaaaagg acactcgcta tgcccctggt 480
ttctccggct ccggggcaaa actcggctac caggcccaca atgtgggaaa cagcggagta 540
gatgtggaca tcggtttcct ctccttcctt tccaatggtg cctgggatag tactgacacc 600
acgcacagca agtatggctt cggggccgat gcaacgcttt cctatggcgt cgaccgtcag 660
cggctgctta cgttggagct ggcagggaat gccacactgg accagaacta cgttaagggt 720
accgaagact ccaagaacga aaacaaaaca gcactcctgt ggggagtagg aggccgactc 780
accctcgaac caggcgccgg cttccgcttc tccttcgccc tcgacgccgg taaccaacac 840
```

```
cagagtaacg cacatgctca gacccaagag agagctatcc tcaaagcaag ggaagtgttt     900
agacgggtgg aggggaaact cgtgcagaac cttcccaata tcatgatgcc accaggaatc     960
accgaacaaa ccactctcat agagatggta ggacttgctg ctttgattgc agaaggaacg    1020
ctcggcagcg ccattcaaac cgtgctagcc gctggcgcgc tcgcggcgct tgtatcgcaa    1080
cttgtaccga acatagagca aggagtacgt gatgtcttcc gctcttccga tccaagagtt    1140
gtcactgcta aacttctcgc tttccttgag cgcgcaccta tgaacgcgct caacatagac    1200
gcgctcctgc gtatgcagtg gaagtggctc tcttctggca tatactttgc caccgcaggc    1260
actaatatct ttggcaaacg cgtctttgct accactcgtg cgcactactt tgattttgcc    1320
ggattcctta agctcgaaac caaaagcggt gacccctaca cccacctgct caccggcctg    1380
aacgccggcg tcgaagcacg cgtgtacatc cccctcacct acatccgtta cagaaataac    1440
ggagggtacg aactgaatgg agctgtgccc cctgggacta tcaatatgcc aattttgggg    1500
aaggcgtggt gcagctatcg catccccctc ggttcccacg cctggcttgc accacacaca    1560
tccgtgctcg gcacaaccaa tcgctttaac attattaacc ccgcgggcaa cctgttgaat    1620
gaacgagcgc tccagtacca ggtgggactg acgttcagtc ccttcgagaa ggtggagctc    1680
agcgcccagt gggaacaggg cgtgcttgct gacgctcctt acatgggcat tgccgagagc    1740
atctggtccg aacgccactt cggcaccctt gtctgcggaa tgaaagtgac atggtaa       1797
```

<212> Type : DNA
<211> Length : 1797
SequenceName : SEQ ID 647
SequenceDescription :
Sequence
--------
<213> OrganismName : SARS coronavirus Frankfurt 1
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttta aggatggtat ttattttgct gccacagaga aatcaaatgt tgtccgtggt      300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgctc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa atattttggt    2340
ggttttaatt tttcacaaat attacctgac cctctaaagc caactaagag gtcttttatt    2400
gaggacttgc tctttaataa ggtgacactc gctgatgctg gcttcatgaa gcaatatggc    2460
```

```
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aatacctttt    2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgtggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga ttttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                 3768
```

<212> Type : DNA
<211> Length : 3768
SequenceName : SEQ ID 648
SequenceDescription :
Sequence
--------
<213> OrganismName : SARS coronavirus HSR 1
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttta aggatggtat ttattttgct gccacagaga aatcaaatgt tgtccgtggt     300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgctc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
```

```
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aatacctttt 2640
gctatgcaaa tggcatatag gttcaatggc attggagtta cccaaaatgt tctctatgag 2700
aaccaaaaac aaatcgccaa ccaatttaac aaggcgatta gtcaaattca agaatcactt 2760
acaacaacat caactgcatt gggcaagctg caagacgttg ttaaccagaa tgctcaagca 2820
ttaaacacac ttgttaaaca acttagctct aattttggtg caatttcaag tgtgctaaat 2880
gatatccttt cgcgacttga taaagtcgag gcggaggtac aaattgacag gttaattaca 2940
ggcagacttc aaagccttca aacctatgta acacaacaac taatcagggc tgctgaaatc 3000
agggcttctg ctaatcttgc tgctactaaa atgtctgagt gtgttcttgg acaatcaaaa 3060
agagttgact tttgtggaaa gggctaccac cttatgtcct tcccacaagc agccccgcat 3120
ggtgttgtct tcctacatgt cacgtatgtg ccatcccagg agaggaactt caccacagcg 3180
ccagcaattt gtcatgaagg caaagcatac ttccctcgtg aaggtgtttt tgtgtttaat 3240
ggcacttctt ggtttattac acagaggaac ttcttttctc cacaaataat tactacagac 3300
aatacatttg tctcaggaaa ttgtgatgtc gttattggca tcattaacaa cacagtttat 3360
gatcctctgc aacctgagct tgactcattc aaagaagagc tggacaagta cttcaaaaat 3420
catacatcac cagatgttga tcttggcgac atttcaggca ttaacgcttc tgtcgtcaac 3480
attcaaaaag aaattgaccg cctcaatgag gtcgctaaaa atttaaatga atcactcatt 3540
gaccttcaag aattgggaaa atatgagcaa tatattaaat ggccttggta tgtttggctc 3600
ggcttcattg ctggactaat tgccatcgtc atggttacaa tcttgctttg ttgcatgact 3660
agttgttgca gttgcctcaa gggtgcatgc tcttgtggtt cttgctgcaa gtttgatgag 3720
gatgactctg agccagttct caagggtgtc aaattacatt acacataa 3768
```

<212> Type : DNA
<211> Length : 3768
SequenceName : SEQ ID 649
SequenceDescription :
Sequence
--------
<213> OrganismName : SARS coronavirus ZJ01
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttttta aggatggtat ttattttgct gccacagaga aatcaaatgt tgtccgtggt     300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgtta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgctc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860

atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa atattttggt    2340
ggttttaatt tttcacaaat attacctgac cctctaaagc caactaagag gtcttttatt    2400
gaggacttgc tctttaataa ggtgacactc gctgatgctg gcttcatgaa gcaatatggc    2460
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aatacctttt    2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgtggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga tcttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                 3768
```

<212> Type : DNA
<211> Length : 3768
SequenceName : SEQ ID 650

SequenceDescription :
Sequence
--------
<213> OrganismName : SARS coronavirus TW1
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
atacctttta aggatggtat ttattttgct gccacagaga aatcaaatgt tgtccgtggt     300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
```

```
aagaaccagt gtgtcaattt taattttaat ggactcactg gtactggtgt gttaactcct 1620
tcttcaaaga gatttcaacc atttcaacaa tttggccgtg atgtttctga tttcactgat 1680
tccgttcgag atcctaaaac atctgaaata ttagacattt caccttgctc ttttgggggt 1740
gtaagtgtaa ttacacctgg aacaaatgct tcatctgaag ttgctgttct atatcaagat 1800
gttaactgca ctgatgtttc tacagcaatt catgcagatc aactcacacc agcttggcgc 1860
atatattcta ctggaaacaa tgtattccag actcaagcag gctgtcttat aggagctgag 1920
catgtcgaca cttcttatga gtgcgacatt cctattggag ctggcatttg tgctagttac 1980
catacagttt ctttattacg tagtactagc caaaaatcta ttgtggctta tactatgtct 2040
ttaggtgctg atagttcaat tgcttactct aataacacca ttgctatacc tactaacttt 2100
tcaattagca ttactacaga agtaatgcct gtttctatgg ctaaaacctc cgtagattgt 2160
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aataccttt 2640
gctatgcaaa tggcatatag gttcaatggc attggagtta cccaaaatgt tctctatgag 2700
aaccaaaaac aaatcgccaa ccaatttaac aaggcgatta gtcaaattca agaatcactt 2760
acaacaacat caactgcatt gggcaagctg caagacgttg ttaaccagaa tgctcaagca 2820
ttaaacacac ttgttaaaca acttagctct aatttggtg caatttcaag tgtgctaaat 2880
gatatccttt cgcgacttga taaagtcgag gcggaggtac aaattgacag gttaattaca 2940
ggcagacttc aaagccttca aacctatgta acacaacaac taatcagggc tgctgaaatc 3000
agggcttctg ctaatcttgc tgctactaaa atgtctgagt gtgttcttgg acaatcaaaa 3060
agagttgact tttgtggaaa gggctaccac cttatgtcct tcccacaagc agccccgcat 3120
ggtgttgtct tcctacatgt cacgtatgtg ccatcccagg agaggaactt caccacagcg 3180
ccagcaattt gtcatgaagg caaagcatac ttccctcgtg aaggtgtttt tgtgtttaat 3240
ggcacttctt ggtttattac acagaggaac ttcttttctc cacaaataat tactacagac 3300
aatacatttg tctcaggaaa ttgtgatgtc gttattggca tcattaacaa cacagtttat 3360
gatcctctgc aacctgagct tgactcattc aaagaagagc tggacaagta cttcaaaaat 3420
catacatcac cagatgttga tcttggcgac atttcaggca ttaacgcttc tgtcgtcaac 3480
attcaaaaag aaattgaccg cctcaatgag gtcgctaaaa atttaaatga atcactcatt 3540
gaccttcaag aattgggaaa atatgagcaa tatattaaat ggccttggta tgtttggctc 3600
ggcttcattg ctggactaat tgccatcgtc atggttacaa tcttgctttg ttgcatgact 3660
agttgttgca gttgcctcaa gggtgcatgc tcttgtggtt cttgctgcaa gtttgatgag 3720
gatgactctg agccagttct caagggtgtc aaattacatt acacataa 3768
```

<212> Type : DNA
<211> Length : 3768
SequenceName : SEQ ID 651
SequenceDescription :
Sequence
--------
<213> OrganismName : SARS coronavirus CUHK-Su10
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttttta aggatggtat ttattttgct gccacagaga aatcaaatgt tgtccgtggt    300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260

cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgctc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa atattttggt    2340
ggttttaatt tttcacaaat attacctgac cctctaaagc caactaagag gtcttttatt    2400
gaggacttgc tctttaataa ggtgacactc gctgatgctg gcttcatgaa gcaatatggc    2460
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aatacctttt    2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgtggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga tcttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                3768
```

<212> Type : DNA

<211> Length : 3768

SequenceName : SEQ ID 652

SequenceDescription :
Sequence
--------
<213> OrganismName : SARS coronavirus Urbani
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttta aggatggtat ttattttgct gccacagaga aatcaaatgt tgtccgtggt     300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
```

aacttgtgtc cttttggaga ggtttttaat gctactaaat tcccttctgt ctatgcatgg 1020
gagagaaaaa aaatttctaa ttgtgttgct gattactctg tgctctacaa ctcaacattt 1080
ttttcaacct ttaagtgcta tggcgtttct gccactaagt tgaatgatct ttgcttctcc 1140
aatgtctatg cagattcttt tgtagtcaag ggagatgatg taagacaaat agcgccagga 1200
caaactggtg ttattgctga ttataattat aaattgccag atgatttcat gggttgtgtc 1260
cttgcttgga atactaggaa cattgatgct acttcaactg gtaattataa ttataaatat 1320
aggtatctta gacatggcaa gcttaggccc tttgagagag acatatctaa tgtgcctttc 1380
tcccctgatg gcaaaccttg caccccacct gctcttaatt gttattggcc attaaatgat 1440
tatggttttt acaccactac tggcattggc taccaacctt acagagttgt agtactttct 1500
tttgaacttt taaatgcacc ggccacggtt tgtggaccaa aattatccac tgaccttatt 1560
aagaaccagt gtgtcaattt taattttaat ggactcactg gtactggtgt gttaactcct 1620
tcttcaaaga gatttcaacc atttcaacaa tttggccgtg atgtttctga tttcactgat 1680
tccgttcgag atcctaaaac atctgaaata ttagacattt caccttgctc ttttgggggt 1740
gtaagtgtaa ttacacctgg aacaaatgct tcatctgaag ttgctgttct atatcaagat 1800
gttaactgca ctgatgtttc tacagcaatt catgcagatc aactcacacc agcttggcgc 1860
atatattcta ctggaaacaa tgtattccag actcaagcag gctgtcttat aggagctgag 1920
catgtcgaca cttcttatga gtgcgacatt cctattggag ctggcatttg tgctagttac 1980
catacagttt ctttattacg tagtactagc caaaaatcta ttgtggctta tactatgtct 2040
ttaggtgctg atagttcaat tgcttactct aataacacca ttgctatacc tactaacttt 2100
tcaattagca ttactacaga agtaatgcct gtttctatgg ctaaaacctc cgtagattgt 2160
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aataccttttt 2640
gctatgcaaa tggcatatag gttcaatggc attggagtta cccaaaatgt tctctatgag 2700
aaccaaaaac aaatcgccaa ccaatttaac aaggcgatta gtcaaattca agaatcactt 2760
acaacaacat caactgcatt gggcaagctg caagacgttg ttaaccagaa tgctcaagca 2820
ttaaacacac ttgttaaaca acttagctct aattttggtg caatttcaag tgtgctaaat 2880
gatatccttt cgcgacttga taaagtcgag gcggaggtac aaattgacag gttaattaca 2940
ggcagacttc aaagccttca aacctatgta acacaacaac taatcagggc tgctgaaatc 3000
agggcttctg ctaatcttgc tgctactaaa atgtctgagt gtgttcttgg acaatcaaaa 3060
agagttgact tttgtggaaa gggctaccac cttatgtcct tcccacaagc agccccgcat 3120
ggtgttgtct tcctacatgt cacgtatgtg ccatcccagg agaggaactt caccacagcg 3180
ccagcaattt gtcatgaagg caaagcatac ttccctcgtg aaggtgtttt tgtgtttaat 3240
ggcacttctt ggtttattac acagaggaac ttcttttctc cacaaataat tactacagac 3300
aatacatttg tctcaggaaa ttgtgatgtc gttattggca tcattaacaa cacagtttat 3360
gatcctctgc aacctgagct cgactcattc aaagaagagc tggacaagta cttcaaaaat 3420
catacatcac cagatgttga tcttggcgac atttcaggca ttaacgcttc tgtcgtcaac 3480
attcaaaaag aaattgaccg cctcaatgag gtcgctaaaa atttaaatga atcactcatt 3540
gaccttcaag aattgggaaa atatgagcaa tatattaaat ggccttggta tgtttggctc 3600
ggcttcattg ctggactaat tgccatcgtc atggttacaa tcttgctttg ttgcatgact 3660
agttgttgca gttgcctcaa gggtgcatgc tcttgtggtt cttgctgcaa gtttgatgag 3720
gatgactctg agccagttct caagggtgtc aaattacatt acacataa 3768

<212> Type : DNA
<211> Length : 3768
SequenceName : SEQ ID 653
SequenceDescription :
Sequence
--------
<213> OrganismName : SARS coronavirus
<400> PreSequenceString :

```
atgtttattt tcttattatt tcttactctc actagtggta gtgaccttga ccggtgcacc      60
acttttgatg atgttcaagc tcctaattac actcaacata cttcatctat gaggggggtt     120
tactatcctg atgaaatttt tagatcagac actctttatt taactcagga tttatttctt     180
ccattttatt ctaatgttac agggtttcat actattaatc atacgtttgg caaccctgtc     240
ataccttttta aggatggtat ttattttgct gccacagaga aatcaaatgt tgtccgtggt    300
tgggtttttg gttctaccat gaacaacaag tcacagtcgg tgattattat taacaattct     360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660

aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgcgc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa atattttggt    2340
ggttttaatt tttcacaaat attacctgac cctctaaagc caactaagag gtcttttatt    2400
gaggacttgc tctttaataa ggtgacactc gctgatgctg gcttcatgaa gcaatatggc    2460
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aataccttttt   2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgtggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga tcttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                  3768
```

<212> Type : DNA
<211> Length : 3768
SequenceName : SEQ ID 654

SequenceDescription :
Sequence
--------
<213> OrganismName : SARS coronavirus TOR2
<400> PreSequenceString :

```
atgtttattt tcttattatt tcttactctc actagtggta gtgaccttga ccggtgcacc      60
acttttgatg atgttcaagc tcctaattac actcaacata cttcatctat gagggggtt     120
tactatcctg atgaaatttt tagatcagac actctttatt taactcagga tttatttctt    180
ccattttatt ctaatgttac agggtttcat actattaatc atacgtttgg caaccctgtc    240
ataccttta aggatggtat ttattttgct gccacagaga aatcaaatgt tgtccgtggt     300
tgggtttttg gttctaccat gaacaacaag tcacagtcgg tgattattat taacaattct    360
```

```
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acctattttt     660
aagttgcctc ttggtattaa cattacaaat tttagagcca ttcttacagc cttttcacct     720
gctcaagaca tttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca ggagatgttg tgagattccc taatattaca     960
aacttgtgtc cttttggaga ggtttttaat gctactaaat tcccttctgt ctatgcatgg    1020
gagagaaaaa aaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tttgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgcgc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa atattttggt    2340
ggttttaatt tttcacaaat attacctgac cctctaaagc caactaagag gtcttttatt    2400
gaggacttgc tctttaataa ggtgacactc gctgatgctg gcttcatgaa gcaatatggc    2460
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aatacctttt    2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgtggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga tcttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                 3768
```

<212> Type : DNA
<211> Length : 3768
SequenceName : SEQ ID 655
SequenceDescription :
Sequence
--------
<213> OrganismName : SARS coronavirus GD01
<400> PreSequenceString :

```
atgtttattt tcttattatt tcttactctc actagtggta gtgaccttga ccggtgcacc      60

acttttgatg atgttcaagc tcctaattac actcaacata cttcatctat gagggggtt      120
tactatcctg atgaaatttt tagatcagac actctttatt taactcagga tttatttctt     180
ccattttatt ctaatgttac agggtttcat actattaatc atacgtttga caaccctgtc     240
ataccttta aggatggtat ttattttgct gccacagaga aatcaaatgt tgtccgtggt      300
tgggttttg gttctaccat gaacaacaag tcacagtcgg tgattattat taacaattct      360
actaatgttg ttatacgagc atgtaacttt gaattgtgtg acaacccttt ctttgctgtt     420
tctaaaccca tgggtacaca gacacatact atgatattcg ataatgcatt taattgcact     480
ttcgagtaca tatctgatgc cttttcgctt gatgtttcag aaaagtcagg taattttaaa     540
cacttacgag agtttgtgtt taaaaataaa gatgggtttc tctatgttta taagggctat     600
caacctatag atgtagttcg tgatctacct tctggtttta acactttgaa acccattttt     660
aagttgcctc ttggtattaa cattacaaat tttagagcca ttcttacagc ctttttacct     720
gctcaagaca cttggggcac gtcagctgca gcctattttg ttggctattt aaagccaact     780
acatttatgc tcaagtatga tgaaaatggt acaatcacag atgctgttga ttgttctcaa     840
aatccacttg ctgaactcaa atgctctgtt aagagctttg agattgacaa aggaatttac     900
cagacctcta atttcagggt tgttccctca agagatgttg tgagattccc taatattaca     960
aacttgtgtc cttttggaga ggtttttaat gctactaaat tcccttctgt ctatgcatgg    1020
gagaggaaaa gaatttctaa ttgtgttgct gattactctg tgctctacaa ctcaacattt    1080
ttttcaacct ttaagtgcta tggcgtttct gccactaagt tgaatgatct ttgcttctcc    1140
aatgtctatg cagattcttt tgtagtcaag ggagatgatg taagacaaat agcgccagga    1200
caaactggtg ttattgctga ttataattat aaattgccag atgatttcat gggttgtgtc    1260
cttgcttgga atactaggaa cattgatgct acttcaactg gtaattataa ttataaatat    1320
aggtatctta gacatggcaa gcttaggccc tttgagagag acatatctaa tgtgcctttc    1380
tcccctgatg gcaaaccttg caccccacct gctcttaatt gttattggcc attaaatgat    1440
tatggttttt acaccactac tggcattggc taccaacctt acagagttgt agtactttct    1500
tatgaacttt taaatgcacc ggccacggtt tgtggaccaa aattatccac tgaccttatt    1560
aagaaccagt gtgtcaattt taattttaat ggactcactg gtactggtgt gttaactcct    1620
tcttcaaaga gatttcaacc atttcaacaa tttggccgtg atgtttctga tttcactgat    1680
tccgttcgag atcctaaaac atctgaaata ttagacattt caccttgctc ttttgggggt    1740
gtaagtgtaa ttacacctgg aacaaatgct tcatctgaag ttgctgttct atatcaagat    1800
gttaactgca ctgatgtttc tacagcaatt catgcagatc aactcacacc agcttggcgc    1860
atatattcta ctggaaacaa tgtattccag actcaagcag gctgtcttat aggagctgag    1920
catgtcgaca cttcttatga gtgcgacatt cctattggag ctggcatttg tgctagttac    1980
catacagttt ctttattacg tagtactagc caaaaatcta ttgtggctta tactatgtct    2040
ttaggtgctg atagttcaat tgcttactct aataacacca ttgctatacc tactaacttt    2100
tcaattagca ttactacaga agtaatgcct gtttctatgg ctaaaacctc cgtagattgt    2160
aatatgtaca tctgcggaga ttctactgaa tgtgctaatt tgcttctcca atatggtagc    2220
ttttgcacac aactaaatcg tgcactctca ggtattgctg ctgaacagga tcgcaacaca    2280
cgtgaagtgt tcgctcaagt caaacaaatg tacaaaaccc caactttgaa agattttggt    2340
ggttttaatt tttcacaaat attacctgac cctctaaagt caactaagag gtcttttatt    2400
gaggacttgc tctttaataa ggtgacactc gctgatgctg gcttcatgaa gcaatatggc    2460
gaatgcctag gtgatattaa tgctagagat ctcatttgtg cgcagaagtt caatggactt    2520
acagtgttgc cacctctgct cactgatgat atgattgctg cctacactgc tgctctagtt    2580
agtggtactg ccactgctgg atggacattt ggtgctggcg ctgctcttca aataccttt     2640
gctatgcaaa tggcatatag gttcaatggc attggagtta cccaaaatgt tctctatgag    2700
aaccaaaaac aaatcgccaa ccaatttaac aaggcgatta gtcaaattca agaatcactt    2760
acaacaacat caactgcatt gggcaagctg caagacgttg ttaaccagaa tgctcaagca    2820
ttaaacacac ttgttaaaca acttagctct aattttggtg caatttcaag tgtgctaaat    2880
gatatccttt cgcgacttga taaagtcgag gcggaggtac aaattgacag gttaattaca    2940
ggcagacttc aaagccttca aacctatgta acacaacaac taatcagggc tgctgaaatc    3000
agggcttctg ctaatcttgc tgctactaaa atgtctgagt gtgttcttgg acaatcaaaa    3060
agagttgact tttgcggaaa gggctaccac cttatgtcct tcccacaagc agccccgcat    3120
ggtgttgtct tcctacatgt cacgtatgtg ccatcccagg agaggaactt caccacagcg    3180
ccagcaattt gtcatgaagg caaagcatac ttccctcgtg aaggtgtttt tgtgtttaat    3240
ggcacttctt ggtttattac acagaggaac ttcttttctc cacaaataat tactacagac    3300
aatacatttg tctcaggaaa ttgtgatgtc gttattggca tcattaacaa cacagtttat    3360
gatcctctgc aacctgagct tgactcattc aaagaagagc tggacaagta cttcaaaaat    3420
catacatcac cagatgttga tcttggcgac atttcaggca ttaacgcttc tgtcgtcaac    3480
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                 3768
```

<212> Type : DNA
<211> Length : 3768
SequenceName : SEQ ID 656

SequenceDescription :
Sequence
--------
<213> OrganismName : SARS coronavirus CUHK-W1
<400> PreSequenceString :

```
atgtttattt tcttattatt tcttactctc actagtggta gtgaccttga ccggtgcacc     60
acttttgatg atgttcaagc tcctaattac actcaacata cttcatctat gaggggggtt    120
tactatcctg atgaaatttt tagatcagac actcttttatt taactcagga tttatttctt    180
ccattttatt ctaatgttac agggtttcat actattaatc atacgtttga caaccctgtc    240
atacctttta aggatggtat ttattttgct gccacagaga aatcaaatgt tgtccgtggt    300
tgggtttttg gttctaccat gaacaacaag tcacagtcgg tgattattat taacaattct    360
actaatgttg ttatacgagc atgtaacttt gaattgtgtg acaacccttt ctttgctgtt    420
tctaaaccca tgggtacaca gacacatact atgatattcg ataatgcatt taattgcact    480
ttcgagtaca tatctgatgc cttttcgctt gatgtttcag aaaagtcagg taattttaaa    540
cacttacgag agtttgtgtt taaaaataaa gatgggtttc tctatgttta taagggctat    600
caacctatag atgtagttcg tgatctacct tctggtttta acactttgaa acctattttt    660
aagttgcctc ttggtattaa cattacaaat tttagagcca ttcttacagc cttttcacct    720
gctcaagaca cttggggcac gtcagctgca gcctattttg ttggctattt aaagccaact    780
acatttatgc tcaagtatga tgaaaatggt acaatcacag atgctgttga ttgttctcaa    840
aatccacttg ctgaactcaa atgctctgtt aagagctttg agattgacaa aggaatttac    900
cagacctcta atttcagggt tgttccctca ggagatgttg tgagattccc taatattaca    960
aacttgtgtc cttttggaga ggtttttaat gctactaaat tcccttctgt ctatgcatgg   1020
gagagaaaaa aaatttctaa ttgtgttgct gattactctg tgctctacaa ctcaacattt   1080
ttttcaacct ttaagtgcta tggcgtttct gccactaagt tgaatgatct ttgcttctcc   1140
aatgtctatg cagattcttt tgtagtcaag ggagatgatg taagacaaat agcgccagga   1200
caaactggtg ttattgctga ttataattat aaattgccag atgatttcat gggttgtgtc   1260
cttgcttgga atactaggaa cattgatgct acttcaactg gtaattataa ttataaatat   1320
aggtatctta gacatggcaa gcttaggccc tttgagagag acatatctaa tgtgcctttc   1380
tcccctgatg gcaaaccttg caccccacct gctcttaatt gttattggcc attaaatgat   1440
tatggttttt acaccactac tggcattggc taccaacctt acagagttgt agtactttct   1500
tttgaacttt taaatgcacc ggccacggtt tgtggaccaa aattatccac tgaccttatt   1560
aagaaccagt gtgtcaattt taattttaat ggactcactg gtactggtgt gttaactcct   1620
tcttcaaaga gatttcaacc atttcaacaa tttggccgtg atgtttctga tttcactgat   1680
tccgttcgag atcctaaaac atctgaaata ttagacattt caccttgctc ttttgggggt   1740
gtaagtgtaa ttacacctgg aacaaatgct tcatctgaag ttgctgttct atatcaagat   1800
gttaactgca ctgatgtttc tacagcaatt catgcagatc aactcacacc agcttggcgc   1860
atatattcta ctggaaacaa tgtattccag actcaagcag gctgtcttat aggagctgag   1920
catgtcgaca cttcttatga gtgcgacatt cctattggag ctggcatttg tgctagttac   1980
catacagttt ctttattacg tagtactagc caaaaatcta ttgtggctta tactatgtct   2040
ttaggtgctg atagttcaat tgcttactct aataacacca ttgctatacc tactaacttt   2100
tcaattagca ttactacaga agtaatgcct gtttctatgg ctaaaacctc cgtagattgt   2160
aatatgtaca tctgcggaga ttctactgaa tgtgctaatt tgcttctcca atatggtagc   2220
ttttgcacac aactaaatcg tgcactctca ggtattgctg ctgaacagga tcgcaacaca   2280
cgtgaagtgt tcgctcaagt caaacaaatg tacaaaaccc caactttgaa atattttggt   2340
ggttttaatt tttcacaaat attacctgac cctctaaagc caactaagag gtcttttatt   2400
gaggacttgc tctttaataa ggtgacactc gctgatgctg gcttcatgaa gcaatatggc   2460
gaatgcctag gtgatattaa tgctagagat ctcatttgtg cgcagaagtt caatggactt   2520
acagtgttgc cacctctgct cactgatgat atgattgctg cctacactgc tgctctagtt   2580
agtggtactg ccactgctgg atggacattt ggtgctggcg ctgctcttca aatacctttt   2640
gctatgcaaa tggcatatag gttcaatggc attggagtta cccaaaatgt tctctatgag   2700
aaccaaaaac aaatcgccaa ccaatttaac aaggcgatta gtcaaattca agaatcactt   2760
acaacaacat caactgcatt gggcaagctg caagacgttg ttaaccagaa tgctcaagca   2820
ttaaacacac ttgttaaaca acttagctct aattttggtg caatttcaag tgtgctaaat   2880
gatatccttt cgcgacttga taaagtcgag gcggaggtac aaattgacag gttaattaca   2940
ggcagacttc aaagccttca aacctatgta acacaacaac taatcagggc tgctgaaatc   3000
agggcttctg ctaatcttgc tgctactaaa atgtctgagt gtgttcttgg acaatcaaaa   3060
agagttgact tttgtggaaa gggctaccac cttatgtcct tcccacaagc agccccgcat   3120
ggtgttgtct tcctacatgt cacgtatgtg ccatcccagg agaggaactt caccacagcg   3180
ccagcaattt gtcatgaagg caaagcatac ttccctcgtg aaggtgtttt tgtgtttaat   3240
ggcacttctt ggtttattac acagaggaac ttcttttctc cacaaataat tactacagac   3300
aatacatttg tctcaggaaa ttgtgatgtc gttattggca tcattaacaa cacagtttat   3360
gatcctctgc aacctgagct tgactcattc aaagaagagc tggacaagta cttcaaaaat   3420
catacatcac cagatgttga tcttggcgac atttcaggca ttaacgcttc tgtcgtcaac   3480
attcaaaaag aaattgaccg cctcaatgag gtcgctaaaa atttaaatga atcactcatt   3540
gaccttcaag aattgggaaa atatgagcaa tatattaaat ggccttggta tgtttggctc   3600
ggcttcattg ctggactaat tgccatcgtc atggttacaa tcttgctttg ttgcatgact   3660
agttgttgca gttgcctcaa gggtgcatgc tcttgtggtt cttgctgcaa gtttgatgag   3720
gatgactctg agccagttct caagggtgtc aaattacatt acacataa               3768
```

<212> Type : DNA
<211> Length : 3768
SequenceName : SEQ ID 657
SequenceDescription :
Sequence
--------
<213> OrganismName : SARS coronavirus BJ01
<400> PreSequenceString :

```
atgtttattt tcttattatt tcttactctc actagtggta gtgaccttga ccggtgcacc     60
acttttgatg atgttcaagc tcctaattac actcaacata cttcatctat gaggggggtt    120
tactatcctg atgaaatttt tagatcagac actcttttatt taactcagga tttatttctt   180
ccattttatt ctaatgttac agggtttcat actattaatc atacgtttga caaccctgtc    240
atacctttta aggatggtat ttattttgct gccacagaga aatcaaatgt tgtccgtggt    300
tgggtttttg gttctaccat gaacaacaag tcacagtcgg tgattattat taacaattct    360
actaatgttg ttatacgagc atgtaacttt gaattgtgtg acaacccttt ctttgctgtt    420
tctaaaccca tgggtacaca gacacatact atgatattcg ataatgcatt taattgcact    480
ttcgagtaca tatctgatgc cttttcgctt gatgtttcag aaaagtcagg taattttaaa    540
cacttacgag agtttgtgtt taaaaataaa gatgggtttc tctatgttta taagggctat    600
caacctatag atgtagttcg tgatctacct tctggtttta acactttgaa acctattttt    660
aagttgcctc ttggtattaa cattacaaat tttagagcca ttcttacagc cttttcacct    720
gctcaagaca cttggggcac gtcagctgca gcctattttg ttggctattt aaagccaact    780
acatttatgc tcaagtatga tgaaaatggt acaatcacag atgctgttga ttgttctcaa    840
aatccacttg ctgaactcaa atgctctgtt aagagctttg agattgacaa aggaatttac    900
cagacctcta atttcagggt tgttccctca ggagatgttg tgagattccc taatattaca    960
aacttgtgtc cttttggaga ggtttttaat gctactaaat tcccttctgt ctatgcatgg   1020
gagagaaaaa aaatttctaa ttgtgttgct gattactctg tgctctacaa ctcaacattt   1080
ttttcaacct ttaagtgcta tggcgtttct gccactaagt tgaatgatct ttgcttctcc   1140
aatgtctatg cagattcttt tgtagtcaag ggagatgatg taagacaaat agcgccagga   1200
caaactggtg ttattgctga ttataattat aaattgccag atgatttcat gggttgtgtc   1260
cttgcttgga atactaggaa cattgatgct acttcaactg gtaattataa ttataaatat   1320
aggtatctta gacatggcaa gcttaggccc tttgagagag acatatctaa tgtgcctttc   1380
tcccctgatg gcaaaccttg caccccacct gctcttaatt gttattggcc attaaatgat   1440
tatggttttt acaccactac tggcattggc taccaacctt acagagttgt agtactttct   1500
tttgaacttt taaatgcacc ggccacggtt tgtggaccaa aattatccac tgaccttatt   1560
aagaaccagt gtgtcaattt taattttaat ggactcactg gtactggtgt gttaactcct   1620
tcttcaaaga gatttcaacc atttcaacaa tttggccgtg atgtttctga tttcactgat   1680
tccgttcgag atcctaaaac atctgaaata ttagacattt caccttgctc ttttgggggt   1740
gtaagtgtaa ttacacctgg aacaaatgct tcatctgaag ttgctgttct atatcaagat   1800
gttaactgca ctgatgtttc tacagcaatt catgcagatc aactcacacc agcttggcgc   1860
atatattcta ctggaaacaa tgtattccag actcaagcag gctgtcttat aggagctgag   1920
catgtcgaca cttcttatga gtgcgacatt cctattggag ctggcatttg tgctagttac   1980
catacagttt ctttattacg tagtactagc caaaaatcta ttgtggctta tactatgtct   2040
ttaggtgctg atagttcaat tgcttactct aataacacca ttgctatacc tactaacttt   2100
tcaattagca ttactacaga agtaatgcct gtttctatgg ctaaaacctc cgtagattgt   2160
aatatgtaca tctgcggaga ttctactgaa tgtgctaatt tgcttctcca atatggtagc   2220
ttttgcacac aactaaatcg tgcactctca ggtattgctg ctgaacagga tcgcaacaca   2280
cgtgaagtgt tcgctcaagt caaacaaatg tacaaaaccc caactttgaa atattttggt   2340
ggttttaatt tttcacaaat attacctgac cctctaaagc caactaagag gtcttttatt   2400
gaggacttgc tctttaataa ggtgacactc gctgatgctg gcttcatgaa gcaatatggc   2460
gaatgcctag gtgatattaa tgctagagat ctcatttgtg cgcagaagtt caatggactt   2520
acagtgttgc cacctctgct cactgatgat atgattgctg cctacactgc tgctctagtt   2580
agtggtactg ccactgctgg atggacattt ggtgctggcg ctgctcttca aatacctttt   2640
gctatgcaaa tggcatatag gttcaatggc attggagtta cccaaaatgt tctctatgag   2700
aaccaaaaac aaatcgccaa ccaatttaac aaggcgatta gtcaaattca agaatcactt   2760
acaacaacat caactgcatt gggcaagctg caagacgttg ttaaccagaa tgctcaagca   2820
ttaaacacac ttgttaaaca acttagctct aatttttggtg caatttcaag tgtgctaaat  2880
gatatccttt cgcgacttga taaagtcgag gcggaggtac aaattgacag gttaattaca   2940
ggcagacttc aaagccttca aacctatgta acacaacaac taatcagggc tgctgaaatc   3000
agggcttctg ctaatcttgc tgctactaaa atgtctgagt gtgttcttgg acaatcaaaa   3060
agagttgact tttgtggaaa gggctaccac cttatgtcct tcccacaagc agccccgcat   3120
ggtgttgtct tcctacatgt cacgtatgtg ccatcccagg agaggaactt caccacagcg   3180
ccagcaattt gtcatgaagg caaagcatac ttccctcgtg aaggtgtttt tgtgtttaat   3240
ggcacttctt ggtttattac acagaggaac ttcttttctc cacaaataat tactacagac   3300
aatacatttg tctcaggaaa ttgtgatgtc gttattggca tcattaacaa cacagtttat   3360
gatcctctgc aacctgagct tgactcattc aaagaagagc tggacaagta cttcaaaaat   3420
catacatcac cagatgttga tcttggcgac atttcaggca ttaacgcttc tgtcgtcaac   3480
```

```
attcaaaaag aaattgaccg cctcaatgag gtcgctaaaa atttaaatga atcactcatt    3540
gaccttcaag aattgggaaa atatgagcaa tatattaaat ggccttggta tgtttggctc    3600
ggcttcattg ctggactaat tgccatcgtc atggttacaa tcttgctttg ttgcatgact    3660
agttgttgca gttgcctcaa gggtgcatgc tcttgtggtt cttgctgcaa gtttgatgag    3720
gatgactctg agccagttct caagggtgtc aaattacatt acacataa                 3768
```

<212> Type : DNA
<211> Length : 3768
SequenceName : SEQ ID 658
SequenceDescription :
Sequence
--------
<213> OrganismName : Escherichia coli O157:H7
<400> PreSequenceString :

```
atgaacaaaa tatttaaagt tatctggaac cctgcgacag ggaattatac tgttaccagc      60
gaaacggcaa aaagccgtgg caagaaatct gggcgcagta agctgttaat ttctgcgctg     120
gttgcgggtg gaatgttgtc gtcgtttggg gcattggcga atgccgggaa tgacaacggt     180
caggtgttg attacggtag tggatcagct ggcgacggct gggttgctat aggcaaaggg      240
gcgaaagcaa atacttttat gaacaccagt ggttccagta ctgctgtggg ttatgacgct     300
atagctgaag gccaatatag ctctgccatc gggtcaaaaa cccatgcgat tggtggtgca     360
tcaatggcct ttggggttag tgcaatatca gaaggcgata gaagtatagc actgggtgcc     420
tcttcgtatt cattgggcca atactcaatg gccctcggcc gttattcaaa agcattgggt     480
aaattgtcta ttgctatggg ggactcttcc aaagcggaag gagcaaacgc cattgccctg     540
ggaaatgcca ctaaagctac tgagattatg agtattgctc ttggcgacac cgccaatgcg     600
tcaaaagcgt attcaatggc gctgggagca agtagcgtcg catctgaaga aaacgctatt     660
gcgataggtg ctgagaccga agccgctgaa aatgcaactg ctattggcaa taatgcgaag     720
gcaaaaggga ctaatagcat ggcaatgggg ttcggaagcc ttgccgataa agtcaatact     780
atcgcattag gaaatggcag ccaggctctg gcagataatg caatcgccat aggccagggc     840
aacaaagctg atggcgtgga tgccatcgct ctgggtaatg gtagccagtc gagaggctta     900
aacaccattg ccttaggcac agccagtaat gcaactggtg ataagagtct tgcgcttggt     960
agtaatagca gtgccaacgg tattaactct gtcgcgctgg gcgcagattc cattgcggat    1020
ttagacaata ccgtctctgt cggcaatagt tcattaaaac gcaagatcgt taatgtgaaa    1080
aatggcgcga tcaagtctga cagttacgat gccattaatg gttcacagct ttatgccatt    1140
agcgactcgg tagcaaaaag gcttggagga ggggctgcag tagatgttga tgacggtact    1200
gttacagcac caacctacaa tttaaaaaat ggtagcaaaa ataacgtagg ggctgcgctc    1260
gctgtacttg atgaaaacac cctgcaatgg gaccaaacca aaggcaaata cagcgctgct    1320
catggtacta gtagcccaac tgccagcgta atcaccgatg ttgcggatgg cacgatttca    1380
gcctccagta aggatgcggt taacggttcc caactgaaag ctaccaatga cgatgtcgaa    1440
gccaacaccg ccaatatcgc tactaatacc agcaacattg ccacgaatac ggcaaatatt    1500
gccaccaata ccaccaatat caccaacctg acggattccg ttggtgacct tcaggctgat    1560
gccctgctct ggaacgaaac taaaaaggca ttcagtgcag ctcacggcca ggataccacc    1620
agcaaaatca ccaacgttaa agatgccgac ctgacggctg acagcactga tgctgttaac    1680
ggctctcagc tgaaaaccac caacgatgct gtggcgacga ataccaccaa tatcgccaat    1740
aacacttcca atattgccac taacaccacc aacatctcta acctgactga gacggtgact    1800
aatcttggtg aggatgcgct gaaatgggat aaggacaatg gtgtattcac ggcagctcat    1860
ggcaccgaga ccaccagcaa aatcaccaac gttaaagatg gcgacctgac gactggcagc    1920
accgatgccg ttaacggctc tcagctgaaa accaccaacg atgccgtggc gacgaatacc    1980
accaatatcg ccactaacac caccaacatc tctaatctga ctgagacggt gactaatctt    2040
ggtgaggatg cgctgaaatg ggataaggac aatggtgtct tcactgcagc tcatggcaac    2100
aataccgcca gcaaaatcac caatatcctg gacggcacag tcactgcaac cagttccgat    2160
gccattaacg gtagccagct ttatgactta agcagcaata tcgccaccta cttcggcggc    2220
aatgcttctg tgaatactga cggtgtgttt accggtccaa cctacaaaat cggtgaaaca    2280
aattattata acgtcggcga tgcactggct gcgattaact cctcatttag cacgtctctc    2340
ggcgatgctc tgctttggga tgccaccgca ggtaaattca gtgccaaaca cggtactaat    2400
ggtgacgcaa gcgtgatcac tgatgtcgca gatggtgaaa tttcagactc cagttctgac    2460
gcagtaaacg gctcacaact ccacggcgtg agcagttatg ttgttgatgc gctggggggt    2520
ggtgccgaag tcaatgcaga cggcaccatc actgcgccga cgtacaccat tgctaatgct    2580
gattacgata atgtcggtga tgccctgaat gctatcgata ccactcttga cgacgctctg    2640
ctctgggatg cggacgccgg tgaaaatggt gcatttagcg ccgctcacgg aaaagataaa    2700
actgccagtg taatcactaa cgtcgctaac ggtgcaatct ctgctgccag cagcgacgcg    2760
attaacggct cacaactcta taccaccaat aagtacatcg ctgatgcgct gggtggtgac    2820
gcagaagtca acgctgacgg caccatcacc gcaccgactt acaccattgc gaacgccgag    2880
tacaacaacg tcggtgacgc cctggatgcg cttgatgata acgccctgct gtgggatgag    2940
actgccaatg gcggtgctgg agcctacaat gccagccatg acggtaaagc cagcatcatc    3000
actaatgtcg ctaatggcag tattagtgag gacagtaccg atgcagtgaa cggttctcag    3060
ttgaatgcga cgaatatgat gattgagcag aacacccaaa ttatcaatca gctcgctggt    3120
```

```
aacaccgacg caacctatat ccaagaaaac ggtgcgggta ttaactatgt gcgtactaac    3180
gacgacggct tagcgttcaa cgacgccagc gcacagggtg ttggcgctac agctataggt    3240
tataactctg tcgccaaagg cgatagcagc gtagctattg gtcagggcag ctacagcgac    3300
gttgatacgg gtatcgccct gggtagcagc tctgtttcca gccgagtgat tgccaaaggc    3360
tcccgtgaca ccagcataac ggaaaatggc gttgttattg gttacgacac cacggatggc    3420
gaactgctcg gtgcattgtc tatcggtgat gacggtaaat atcgtcaaat catcaacgta    3480
gccgatggtt ccgaagccca tgacgccgtt acggttcgtc aattgcagaa tgcgattggt    3540
gcggtcgcaa ccacgccgac taaatacttc cacgctaatt caacggaaga agattcactg    3600
gcagtgggaa ctgactcgct ggcaatgggt gcgaaaacca tcgtgaatgg cgataaaggt    3660
attggtatcg gttatggtgc ctacgtggac gcgaatgcac ttaacggcat tgccattggt    3720
agcaatgcgc aagtcattca tgtcaacagt attgcgatag gtaatggttc tacgaccact    3780
cgtggcgctc aaaccaatta taccgcctac aacatggacg caccgcagaa ctctgtcggt    3840
gaattctcag tcggtagtgc ggatggtcaa cgtcagatca ctaacgtcgc agcaggttcg    3900
gctgataccg atgcggtcaa cgtgggtcag ttgaaagtaa cggatgcgca ggtttcccag    3960
aatacccaga gcattactaa cctggataat cgggtaacga atcttgattc acgcgtcacc    4020
aatatcgaaa acggtattgg cgatatcgtc accaccggta gcaccaagta cttcaagacc    4080
aataccgatg gtgtagatgc cagcgcgcag ggtaaagata gcgtcgcgat tggttccggc    4140
tccattgctg ccgctgacaa cagcgtcgct ctgggtacag ggtctgtggc aaccgaagaa    4200
aatacgatct ctgtaggttc ctctactaac caacgtcgta tcaccaacgt agctgcaggt    4260
aaaaatgcta ccgatgctgt taacgtggca cagttgaagt cttccgaagc tggcggtgta    4320
cgttacgaca ccaaagctga tggttctatc gactatagca atatcaccct cggtggcggc    4380
aacggcggta cgactcgtat cagcaacgtc tccgctggcg tcaacaacaa cgacgtggtg    4440
aattacgcgc agttgaagca aagcgtgcag gaaacgaagc aatacaccga tcagcgaatg    4500
gttgagatgg ataacaaact gtctaaaact gaaagcaagt tgagcggtgg tatcgcttct    4560
gcaatggcaa tgaccggtct gccgcaggct tacactccag gtgccagcat ggcctctatt    4620
ggtggcggta cttacaacgg tgaatcggca gttgctttag gtgtatcgat ggtgagcgcc    4680
aatggtcgtt gggtctacaa attacaaggt agtaccaata gccagggtga atactccgcc    4740
gcactcggtg ccggtattca gtggtaa                                          4767
```

<212> Type : DNA
<211> Length : 4767
SequenceName : SEQ ID 659
SequenceDescription :
Sequence
--------
<213> OrganismName : Escherichia coli O157:H7
<400> PreSequenceString :

```
gtgccagctt ccgcagtagg tgcgctgggc gaagccagct acacggtgac ggcgaacgtc      60
accgacagcg caggcaacag caattccgcc agccataacg tgcaggtcaa taccgcgctg     120
cctggcgtca ccattaaccc agttgcgacc gacgatatta ttaacgccgc cgaatcgggc     180
aatgcgcaaa ccatcagcgg ccaggtgacg gggcggcgg cgggcgatac ggttaccgta     240
acgcttggcg ggaaaactta caccgctacc gtgcagggga atttaagctg gagcgtggac     300
gttccggcgg cggatattca ggccatcggc aatggcaatc tgacggttaa cgcttcggtg     360
accaacggcg ttggcaatac tggcagcggt tcgcgagata ttactatcga cgccaacctg     420
ccaggtctgc gcgttgacac cgtggcgggc gatgatgtgg tcaatagcat cgagcacgct     480
caggcgctgg tgataactgg tagcagcagc gggctggcgg cgggcgcggc gctgacggtg     540
gtgattaaca cggtcactta cgctgcaaca gtattagccg atggcacatg gagcgttggt     600
gttccggcgg cagacgtgag taactggcct gcgggtacgg tgaatatcac ggtaagtggc     660
actaacacag ccggaacaac atccaccatc acccatccgg tcaccgtcga tctggcggcg     720
gtggcgattt ccattaacac cgtttccggc gacgatgtga ttaacgccgc cgaaaaaggg     780
gcagatttaa ccctttccgg cagcacctcc ggcgtggaag tggggcaaac ggtcaccgtt     840
acctttggcg ggaaaaccta caccgctacc gtagcgggcg atggtagctg gacaaccacc     900
gtacccgccg ccgatctcag cgtgttacgc gacggcgacg ccaccgtgca ggccagcgtc     960
agcactatta acggcaacac ggcttcggca acccacgcct acagcgtcga tgccacggcc    1020
ccgacgcttg ccattaacac catcgccacc gacgatattc tgaacgctgc cgaggcgggc    1080
aatccgttaa ccatcagcgg tagcagcacc gccgaagcgg ggcagacggt aaccgtcacg    1140
cttaatggtg tgacttacag cggctccgtc caggcggacg gcagctggag cgtcagctta    1200
ccgacggcgg atctcagcaa tctgaccgcc agccagtaca ccgttagtgc ctcggtaagc    1260
gataaagcgg gtaacccggc gtccgctaac cacgggctgg cggtggatct caccgtgccg    1320
gtgctgacca tcaacaccgt ctccggcgat gacattatta acgccgccga acacggacag    1380
gcgctggtga tctccggctc cagcactggc ggcgaagcgg gtgatgtcat caccgtcaca    1440
ctaaacagta aaacctacac caccatgctg gacgcttccg gcaactggag cgtcggcgtt    1500
ccggcggctg acgtcactgc gcttggcagc ggcccgcaaa ctatcactgc ggcaattacc    1560
gacgcggcag gcaacagcga tgacgccagc cgcacggtca ccgtgaatct cgccgcgcca    1620
accattggta tcaacaccat cgccaccgat gacgtgatta aagccacgga aaaaggcgcg    1680
gacctgcaaa tcaccggcac cagtaatcag cctgcgggca ccaccattac ggtgacgctg    1740
aacgggcaaa attacaccgc tactaccgat agcaacggca actggagcgc cacggtgcca    1800
```

gcgtcagcgg ttagcgcatt gggtgaagcc aactacacgg taacggcaaa cgtcactgat 1860
acggcaggca acagtaattc cgccagtcat aatgtgctgg tcaacagcgc cttgcctgcc 1920
gttaccatta acgcggtggc gactgacgat attattaacg ctgccgaatc gggaaatgcg 1980
caaaccatca gcgggcaggt gacgggcgca gcgcaggggg atacggttac cgttacgctg 2040
ggcggcaaca cctacaccgc cacggtgcag tctaatttaa gctggagtgt ggacgttccg 2100
gcggcggata ttcaggcgct gggcaacggc gacctgacgg ttaatgcctc ggtcaccaat 2160
ggcgtcggca acaccggcag cggctcgcgc gatatcacga tcgacgccaa tctgcctggc 2220
ctgcgggtcg ataccgtggc gggcgatgat gttattaaca gcattgagca caatcaggcg 2280
ctggtgatca ccggcagcag cagcggatta acggcgggaa cggcgttaac ggtcgagatt 2340
aacaacgtta cttatggcgc gacggtatta gccgacggca cgtggagcct cggtgttccg 2400
gcggtagacg tcagcaactg gccagcgggt acggtgaata tcacggtaag cggcaccaac 2460
agtgccggaa caacctccac cattacccat ccggttaccg tcgatctggc tggggtcgcc 2520
atcaccatta acactctctc tggtgatgat gtgatcaacg ccgtcgaaaa aggcgaaacg 2580
ctggtcgtaa gcggcagcac cagcggtgtc gaagccgggc agacggtgac cgtcaccttt 2640
ggcggcaaaa attacaccac cacagtggaa gctaacggta gctggacggt gaatgtgccg 2700
cctgccgatc tcgctgcgct accggacggc gcgggcaacg tgcaggcgag tgtcagtaat 2760
attaacggca acagcgccca ggccgatcgc gcgtatagcg ttgatgccac cgcgccgctt 2820
gtgaccatca acaccatcgc cagcgacgat atccttaacg tgagcgaagc tggcgcgggg 2880
atcaccatca gcggcactac cacggcgcag gccgggcaga cgctcaccgt cacgctcaat 2940
aacaacacgt accagaccac cgttctggcg gatggcacct ggagcgtgaa tgttccggca 3000
gcggatttaa gcggattaac cgccagcagt tacaccgtga ccgccacggt gagcgacaaa 3060
gcgggtaacc cggcaagcgc cgaccacgcg ctggtggtag atatcactgc gccggatctc 3120
accattaaca ccgtcgcggg cgatgacatt atcaacgcca tcgaacatgg tcaggcgttg 3180
gtggtcagcg gcaccagtac gggcgcggcg gcgggtgatg tggtaaccgt cacgctgaac 3240
ggtaaaaact acaccaccac gctggatgcc tccggtaact ggagcgtggg cattccggcg 3300
gcggatgtca cggcgctggc gaccggtagc cagaccatca ccgccagcct gagcgatcgc 3360
gcgggcaaca gcgacagcac gactcacgat gtgaccgttg atcttagcgg cccgacgctg 3420
accattaaca ccgtctccgg cgatgacatt atcaacgccg ctgaaatcgt tgtggcgcag 3480
accatcagcg gtcaggtcac gggaacggcg gttgccggga atacggtgat tgtcaccatt 3540
ggcggcaatc aatataacgc caccgtgcag tcagatttaa gctggagcgt cagcgtaccg 3600
gcgaacgttt tgcaggcgct gggtaacggt gaactgacca tcagcgcctc gttgaccaat 3660
tccgcaaata ataccggcac cgcgacgcac gatatcgtga tagatgccaa cctgccaggt 3720
ctgcgcgtcg ataccgtggc aggcgatgat gtgattaaca gcatcgagca cactcaggcg 3780
ttggtgatca ccggtagcag tagcggactg gcggcgggcg cggcgttgac ggtggttatc 3840
aatagcgtca cctacggcgc aacggtttta gcggatggta gctggagcgt tggtgttccg 3900
gtggcagatg tcacaaactg gcctgcgggg acggtcaata ttgccgtctc tggcaccaac 3960
accgccggaa ccacaaccag cattagccat ccggtcacgg tcgatctcgc tgccgtggcg 4020
atcaccatca acaccctttc cactgacgat gtgattaacg ccgccgagaa aggctctgat 4080
ttgcagctct ccggcaccac ctccggcgtg gaagcggggc aaaccatcac cgttatcttc 4140
ggcggcaaaa gctacaccac cacggttgcc gcggataata cctgggggct gacgatccct 4200
gccgtcgatg ttgcaacctt gccagacggc gcagcaaacg tccaggccag cgtcagcaat 4260
gtcgcaggga acagcaccca ggcaacgcat gcttacagcg ttgatgccac tgcgccctcc 4320
gttaccatca acaccatcgc cacggacgat attcttaacg ccgccgaagc gggatcggcg 4380
ctgaccatca gcggcaccag cacggcggaa gccgggcaga cggtgaccgt aacgctaaac 4440
ggcgttaatt acagcggcaa tgtgcaggca gacgggagct ggagcgtcag cgtaccaacc 4500
ggcgatttag ctagccttac cgccagctcg tataccgtta acgcctcggt cagcgacaaa 4560
gccagaaatt cggcttcggc aacgcataat ctgacggtgg accttgccgc tccggtcgtc 4620
accatcaaca cggtggcggg cgatgacatc attaacgcca cggaacacgg acaggcgcag 4680
atcatcagcg gctcggcaac gggcgcgact accggtaata cggtttccgt gacgattggc 4740
acgaccacct ataccaccgt gctggacgcc aacggcaact ggagcatcgg cgtgcctgcc 4800
agcgtgattt ccgcgctggc gcagggcgat gtgaccatta ccgctacggt caccgactcc 4860
gcaggcaaca gcggcacggc ctcgcacact gtcaccgtgg cgctcggcgc tccggtgctc 4920
gccattaaca ccattgccgt cgatgacatc atcaacgccg cggagaaagg cgcggatctg 4980
gcgattaccg gcaccagcaa ccagcctgcg ggcacgcaga ttaccgttac gctcaacggg 5040
caaaattaca ccaccactgc cgatgcttcc ggtaactgga gcgtgaccgt tccggcgtca 5100
cgggtgagcg ccctcggtga agccacctac acggtgaccg cagccgccac tgacgccgat 5160
ggcaacagcg gttccgccag ccataacgta caggttaata ccgcgctgcc gggcgtcacc 5220
attaacgtgg tggcaacgga cgatattatt aacgccgccg aagcgggcgt ggaacagacc 5280
atcagcgggc aggtgacggg tgcggcggca ggcgacacgg tgaccgtcac gctcggcggg 5340
gcgacttaca ctgccacggt gcaggcgaat ttaagctgga gcgtcgatgt tccggcctcc 5400
gcgctacagg agttgggcaa cggcgaactg accatttcgg cttcggtgac gaacagcgta 5460
ggcaatactg gtaacggcac gcgcgaaatc accatagacg cgaatctccc cggtctgcgg 5520
gtcgacaccg tggcgggcga tgatgtggtt aatattatcg agcacgggca ggcgctggtg 5580
attaccggca gcagctccgg tctggcggcg ggcagcaacg tcacgctgac cattaacggg 5640
caaacctatg ttgcggcggt gctggcggat ggcacctgga gcgtcggcgt tccggcggtg 5700
gatgtcagcg cctggcctgc gggatcggtg acgattgcgg cgagcggtag cacctctgcc 5760
ggaaatccgg taagcgttac gcatccggtg acggtcgatc tctcggcggt ggcggtgagc 5820

atcaacgcca ttaccgccga tgatgtgatc aacgctgccg aaaaaggcgc ggcgttaacg 5880
ctctccggca gcacctctgg cgttgaagcc ggacaaacgg ttaccgtcac ctttggcggc 5940
aaaacttaca gcgccacggt ggctgcgaat ggttcctgga gcacctcggt tccggcggca 6000
gatatggcgg ctctgcgtga tggcgatgcc agcgcacagg ccagcgtcag caatgttaac 6060
ggcaacagcg ccaccacgac ccacgcttac agcgttgatg ccagcgcgcc aacggtgacc 6120
attaatacca ttgcgggcga tgatattctt aacgccgccg aagccggagc ggctctgacc 6180
atcaccggca gcagcacggc ggaagcgggg cagacggtga ccgtcacgct caatggcaca 6240
aactacaccg gcaccgtaca gacggacggc agctggagcg tcagcgtacc gtcagccgac 6300
ttaagcaccc tgaccgccag caactacacc gtgaacgcgg cggtgagcga caaagccgga 6360
aacccggcct cggttaatca caacctgacg gtggatacgt ccgttccggt cgtcaccatc 6420
aacacggtgg caggcgatga tgtgatcaac gcgacggaac acgcccaggc gcagatcatc 6480
agcggctccg ccactggagc ggcaaccggt agcaccgtga cggtgactat cggcacaaat 6540
acctttacca cggtgctgga tgccagcggc aactggagcg tcggcgttcc ggcaagcgtc 6600
gtctcggcac tggcgaatgg cacggtgacc atcaatgcca gcgtcaccga tgccggagga 6660
aacagcggca gcgctaccca tcaggtgacg gtcaataccg ggctgccgac cattaccttt 6720
aacgccatca gcggcgataa catcctgaac gccgatgaaa aaggccagcc gttgaccatc 6780
agcggcggca gtacggggct ggcgacgggc gcgcaggtca ccgtcacgct caacggtcac 6840
aactacagcg ccaccaccga cgcatcgggc aactggacct taaccgtgcc ggtgagcgat 6900
ctggcggcat taggtcaggc caactatacg gtcagcgcca gcgccaccag tgcagcaggc 6960
aacaccgcca gcagccaggc gaatttactg gtcgacagcg gcctgccgga cgtcaccatc 7020
aacaccgtgg caggcgacga tattatcaac gccgccgaag cgggggccga tcaaaccatc 7080
agcggggtgg tgactcgcgc cgccgctggc gatacggtca ccgtgacgct gggcgggaac 7140
acttacaccg ctacggtaca gagcaactta agctggagcg tcagcgttcc gacagccgat 7200
ctccaggcgt tgggcaatgg tgatttgacc attaccgcct cggtcaccaa cgctaatggc 7260
aacaccggga gcggcacgcg ggatatcacc attgatgcca acctgccggg gctgcgcgta 7320
gataccgtgg cgggcgatga tatcgtcaac agcatcgagc acgggcaggc gctggtgatc 7380
accggcggca gtagcggcct gaatgcaggt gctgtgctga cggttaccat caacagtgtg 7440
gcgtattccg ccacggtgca ggcggacgga agctggagcg ttggcattcc ggcggcaaac 7500
gtcagcgcct ggcctgcggg gccgttaacc gtggaggtag acgggcaaag cagcgccaat 7560
aacccagtca gcgtcagcca tccgttcacc gtcgatttaa cggcggtggc aatcagcatc 7620
aacaccgttg ccagcgacga cgtgattaac gccgcagaaa aaggcaccaa tctgactctt 7680
tccggcagta ccagcgggat tgagagcggg caaaccgtca ccgtcacttt tggcggtaaa 7740
acctacactg caagcgtcgc cgccaacggg agctggagtg taaacgttcc ggcggcagat 7800
ctggcaactc tgccagaggg cgcggcgaat gtgcaggcca gcgttagcag cgcgagcggt 7860
aacagtgcct cggcgaccca tgcgtatagc gttgacgcca gcgcgccgac gctcaccatt 7920
aacaccatcg ccagcgacga tatccttaac gccgcagaag ccggaagccc gctcaccatc 7980
agcggcacca gcaccgccga aaccgggcag acggtgaccg tcacccttaa cggcgcaacc 8040
tacaccggca ctgtgcaggc ggacggtagc tggagcgtca gcgttcccac ttcagccctg 8100
ggcgcgctca acgcaagcaa ttacaccgtc agcgccacgg tcaatgacaa agcgggcaac 8160
cccggcagcg ccagccataa tctggcggta gacaccaccg cgccggttct caccattaac 8220
accgtggcgg gcgatgacat catcaacgat gccgaacatg cgcaggcgct ggtgatctcc 8280
ggcaccagta gcggcgggga agcgggcgat gtggtgagcg ttgtgctcaa cggcaaaacc 8340
tacaccacca ccctggatgc ctccggcaac tggagcgttg gcgttccggc ggcagatgtt 8400
acggcgctgg gtagcggtgc gcagaccatc accgccagcg tcagcgatcg ggcaggcaac 8460
agcgacgacg ccagccgcac cgtgaccgtc agcctcagcg cgccagtgat tagcatcaac 8520
accatcgcgg gcgatgatgt gatcaacgcg acggaaaaag gatctgatct ggcgctttct 8580
ggcaccagcg atcagcctgc gggtacggcg atcaccgtca ccctgaacgg acaaaactac 8640
agcgccacca cggatgcctc cggcaactgg agtgttaccg tgcctgcctc ggcggtcagt 8700
gcgctgggcg aagcgaccta cagcgtgacg gcgagcgtca ccaatgctca gggtaacagc 8760
agcaccgcca gccataacgt gcaggttaat accgcgctgc cgggcatcac cattaatccg 8820
gtggcgacgg acgatattat caacgcttca gaggcaggca gcgcgcaaac tatcagcggc 8880
caggtaaccg gtgcggcggc gggcagcacc gtcaccgttg aactgggcgg taaaacttac 8940
accgccaccg tccaggctga tttaagctgg aatgtgagcg tacctgccgc cgactggcag 9000
gctctgggta acggtgagtt aacggttaat gcctcggtga ccaacgccgt tggcaacacc 9060
ggtagcggca cgcgcgatat caccatcgat gccagtctgc ctggcctgcg ggtggatacc 9120
gtcgcgggtg atgatgtggt caatatcatc gaacacgctc aggcgcaggt gatcaccggc 9180
agcagctccg gctttgccgc aggcacggcg ttgacggtgg tgattaacaa ccaaacttac 9240
gccgccacgg tgttggcgaa cggtagctgg agcgtcggcg ttcctgcgac ggatgtcagt 9300
aactggcctg cgggaacgct gaatattacc gttagcgggg cgaacagtgc cggaacgcaa 9360
accagcatta cccatccgct gactgttgat ctcaccgccg tcgccatcag catgaacagc 9420
atcaccagcg atgatgcgat taacgccgcc gaaaaaggcg cggcgttaac gctctccggc 9480
agcacgtccg gcgtcgaagc ggggcaaacc gtcaccgtca cctttggcgg caaaacctac 9540
accaccacgg tggcggcaaa cggtagctgg agcaccaccg ttccggcggc ggatttggcg 9600
gccctgcgtg atggcgatgc cagcgcccag gtgcgggtga ctaacgtcaa cggcaacagc 9660
gccacagcaa cgcacgaata cagcgtcgat agcgccgcgc caacggtgac catcaacacc 9720
attgccagcg ataacatcat caacgccagc gaagcggcgg cgggcgtaac ggtgtccggc 9780
accagcaccg cgcaaaccgg gcagacgctc accgtcacgc ttaacggcac taactaccag 9840

accacggtgc agacagacgg cagctggagt ttaacgctgc ccgccagcga cttaaccgca 9900
cttgctaata acggctacac cctgaccgcc acggtcagcg atctggcggg taaccttggc 9960
agcgccagca aaggcgtgac cgtcgatacc actgcgccgg tgatcagttt taacaccgtg 10020
gcgggcgatg atgtgattaa caacgtcgaa cacattcagg cgcagattat cagcggcacc 10080
gccacgggcg cggtggcggg cgaccgcctg gtggtgacca tcgccgggca gcagtatgtc 10140
accagcaccg atgccagcgg caactggagc gtcggcgtgc ctgccagcgt gatttccggc 10200
cttgcggatg gcacggtgac catcagcgcc accattaccg atagcgcagg caacagcagc 10260
acgcagacgc acaacgtaca ggtgaacacg gcggcagtgt cgctctcggt cagcactatc 10320
agcggcgata accttattaa cgccgccgaa gcgggcagtg cgctgaccct gagcggcact 10380
ggcactaatt tcgcgacagg tacagtggtg accgtgttgc ttaacggcaa aggctacagc 10440
gccaccattc agagcaacgg gagctggagc gtgaacgtgc ctgcggcgga tgttgcggca 10500
ctcagtgatg gcaccagcta cacggttagc gcctccgctc aggacagtgc cggaaacggc 10560
aacagcagca cgcagacgca caacgtacag gtgaacacgg cggcagtgtc gctctcggtc 10620
agcactatca gcggcgataa ccttattaac gccgccgaag cgggcagtgc gctgaccctg 10680
agcggcactg gcactaattt cgcgacaggt acagtggtga ccgtgttgct taacggcaaa 10740
ggctacagcg ccaccattca gagcaacggg agctggagcg tgaacgtgcc tgcggcggat 10800
gttgcggcac tcagtgatgg caccagctac acggttagcg cctccgctca ggacagtgcc 10860
ggaaacagcg ccacggcctc gcgcagcgtg gcggtggatc tcaccgcgcc ggttatcagc 10920
attaacaccg tttcgacgga cgaccgcctc aacgccgccg aacagcagca gccgttaacc 10980
cttaacggct cgaccagcgc ggaagtgggg cagacggtca ccgtcacctt tggcggcaaa 11040
acctataccg ccacggttgc cgccaatggt acctgggcgc tgaacgtgcc tgcggtggat 11100
ctggccgcgc tcgggcaggg cgcgcagacc attaccgcca gcgtgaacga tcgcgccggt 11160
aaccccggac aggccacgca cgccctgacc gtcgataccg ttgcgccaac ggtcaccatc 11220
gccacggtgg cgggtgacga tattatcaac aacgccgagc agcttgccgg gcagaccatt 11280
agcggcacca ccaccgccga agtgggccag acggtgaccg ttacctttaa cgggcaaacc 11340
tggagcgcaa cggttggcag cggcggaagc tggtcggtgt ttattccggc gcagcagttt 11400
gccggattaa gcgacggcag ctacaccatt agcgcgacgg ttagcgatca ggccggaaac 11460
cccggcagcg ccagccgtgg cgtgacgctg aacggcgatg tacctactgt caccatcaac 11520
acctttgcgg gcgacgatgt ggtgaatgca gcggaacacg gctcatcgct ggtgatcagc 11580
ggcaccacca ccgcgcccgt cgggcagacg ctgaccctga ccttaaacgg caaaacctac 11640
accaccacgg tgcagacggg cggtagctgg agctacaccc tcggcagcgc cgatgtcacc 11700
gcgctggcgg acggcaacgc ctacgtgatt aacgcctcgg tgagcaatgc cattggcaac 11760
accggtagca gtaatcacac cattaccgtc gatctcagcg ctccggcgat gggcattaat 11820
atcgattccc tgcaagccga cactggcctt agcgccagcg actttatcac cagcgtcagt 11880
ccggtagtgg tcaacggctc gctcaccgcc gcgcttgcca gtaacgagac ggcgcaaata 11940
agtatcgacg gcggcacaac ctggaccacg cttaccgtta ccggcacgac ctggcgctat 12000
aacgacagcc gcacgctgac cgatggcaac tatctctatc aggtgcgggt gattgacgca 12060
gcgggcaacg ttggcgcgac cgacagccag aatgtggtga tcgacactac cgcgccagat 12120
cccgcggtga aaaccatcgc catcagcgcg atcaccaccg atatggcctt gatcactaac 12180
gattttgtca ccagcgacac gacgcttgcg gtgagcggca cgctgggggc gacgctttct 12240
gccggtgagt tcgcgcaaat cagcctcgac ggcggcgtca cctggactac gttaaccgtc 12300
gttggcacca gctggagcta tgccgatggt cacacgctta ctgacggcac ctggaactac 12360
acggtgcggg tggtggatct ggcggggaac gttgggcaga ccgcaacgca aaacgtggtg 12420
gtcgacacca ccagcccgga agcggcgaaa agtatcacca ttaccggtat cagcgatgac 12480
accggaacca gcagcagcga ttttattacc agcgacacca cgcttaccgt gcgcggcgta 12540
ttgggcgcgg cgctcggcgc taatgagttc gcgcaaatca gtaccgacaa cggcgcaacc 12600
tgggtgaacg tgaccgtcgc cgcagacagc ctgaactgga gttacgttga cggacgaacc 12660
ctcaccaacg gcaccaccac ctggcaggtg cgggtggtcg atctggcggg caacgttggc 12720
gcaacgagca gccagtcggc gctgatcgat accgttaacc cggcgcaggt gctcaccatc 12780
gccagcatca gcaccgacac ggggagttcg gcaactgact ttatcaccag cgacaccatg 12840
ctcacgctga ccggttcgct gggggcgggg cttgccagcg gcgaagtggc gcagattagc 12900
cttgatagcg gcgcgacctg gacaacgctc accaccaacg gtacacagtg gacttacacc 12960
gacagccgca cgctgaccga cggcagctac gtttatcagg tgcgggtgct ggatctggcg 13020
gggaacaccg gcccggtggt gtcgaaaacg gtggtggtcg atacgattaa ccccaccgcc 13080
acaccaacga ttgtgtcgta taccgatgat gtcgggcagc ggcaggggac attaagcagt 13140
tcgcaggcca ccgacgacac tacgccgctg ctgaacggtg tactttccgc gccgcttgcc 13200
agcggtgaag tggtttacct ctaccgtaac gggctgctgt taggggcggt gacgatggtc 13260
ggcgctctga actggaccta cagcgacagc gggctggtga gcggtgccta tacctacagc 13320
gcgcgagtgg tggatttggc ggggaatatc acctcctcca gtgatttgt cctgacggtc 13380
gatacctcta ttccgaccac gctggcgcaa atcaccagcc agaccacgcg cgatactacg 13440
ccgattatta gcggggtgat caccgccgcg cttgccagtg ggcagtatgt tgaagtggtg 13500
atcaacggca aaacctacac ctctgaaccg ggcggcgcgg tagtggtcga tccggcgcac 13560
aacacctggt atgtacagtt gccggatacc gatgcgctga cagtttccgc gaccgcctat 13620
accgttactg cgcaggtaaa aagttcggcg ggtaacggca ataacgccaa tattagcaac 13680
ggcacggtga cggttaacgc ggcgattgat tacacaccga cctggactac cgccagcaaa 13740
accaccgcct gggggctgac ctacggcctc gactcgcacg ggatgtggac ggtgctggca 13800
aaccagcagg taatgcaatc gactgaccca ctcacctggt cgaagaccgc gctgacgctg 13860

```
tatcagagcg gcaacaacta cgccaccagc tccattgccg attacgaccg taacggcacg   13920
ggcgatctgt ttatcacccg tgatgactac ggtacgggct atattaacgg ctttaccaat   13980
aacggcgatg gcacctttc cagcgctatt caggtcaccg tcggcaccct gacgtggtac   14040
ggctcgattg tggcatttga taaagagggc gacggctatc tcgacttctg gattggtgac   14100
gctggcgggc cggactccaa caccttcctg tggaacaacg caggcacgct ggtaggcaac   14160
tccaccacgt cgaacagcgg cggtagcgcc acggtgggcg gggcggtgac ggggtatctt   14220
tcgctcaacg aaggttctgg cgtcgatctg aacaatgacg gcaggatcga cctggttcag   14280
cacacctata acctgaacaa ctattacacg ctgtcttcgc tcatcaacca ggggaatggg   14340
acgtttgtct gggggcagaa caccaccaat accttcctga gcggggcggg cagtggcgct   14400
atgagcagca gcgtttccat gacctgggcc gatttcgatg gtgacggcga tatggatctc   14460
ttcctgcccg ccagccaggg aagagctaac tacggctcgc tgttattcaa caccaacggc   14520
gtactgggtt gcccggtggc ggtgggcgca acggcaacca cctacgccag ccagtttagc   14580
ctggcggtgg actggaacca cgacggcctg atggatatcg cccgtatcgc ccagaccggg   14640
cagtcgtatc tttatactaa cgtcagcaac gccagcaact ggacgcaatc ggccctcggc   14700
ggcagccaga gcggtaccac cagcggcgtg gcggcaatgg actacgactg ggacggcgcg   14760
gtggatgtgc tggtgtccaa acagtcgggc agcgtgttcc tgagccgcaa caccaacacg   14820
gtgagctacg gcacttcgct acacctgcgc atcaccgatc ccaacggcat taacgtctat   14880
tacggcaata ccgtgaagct gtacaactcg gcgggagtgc tggtcgccac gcaaatcatc   14940
aacccgcagt cgggtatggg ggttaacgac acctcggcgc tggtcaactt ctacgggctg   15000
aatgccggag aaacctacaa cgcggtgctg atcaaatcca ccggcaccac cgccagcaat   15060
atcgaccaga cggtcaacac cagctggggc ggtttacagg ccaccgatgc cactcacgct   15120
tacgatctca gcgctgaagc gggtaccgcg agcaacaacg gcaagttcgt cggcaccggc   15180
tataacgaca ccttcttcgc caccgcaggc accgacactt acgacggttc cggcggctgg   15240
gtgtacagct ccggcaccgg aacgtggctg gcgaacggcg ggatggacgt ggttgatttc   15300
cggctttcga cggtgggcgt gacggctaac ttaagcagca ctgccgcgca ggccaccggt   15360
tttaacacct cgacatttac caatatcgaa ggcatttccg gctcgaattt taacgacatt   15420
ctgaccggca gcagcggcga taaccaactg gaagggcgcg gcggcaacga cacgctcaac   15480
atcggcaacg gcggccacga caccttgctc tataaactgc tcaacgccag cgacgccact   15540
ggcggcaacg gctcagacgt ggtgaatggc tttacggtag ggacatggga aggcaccgcc   15600
gacaccgatc gcattgatat tcgtgaactg ttacagggca gcggctacac cggcaacggc   15660
aaagccagct acgtcaacgg cgtggcaacg ctggatgcgc aggccggaaa catcggtgac   15720
tttgtcaaag tcacccagag cggcagcgac accatcgtgc agatcgaccg cgacggcacg   15780
ggcggcactt ttgcgacaac taacgtggtc acgctgacgg gcgtgcacac cgacctcgcc   15840
accttgctgg cgaatcatca gttgatggtg gtgtag                             15876
```

<212> Type : DNA
<211> Length : 1.5876
SequenceName : SEQ ID 660
SequenceDescription :
Sequence
--------
<213> OrganismName : Escherichia coli O157:H7
<400> PreSequenceString :

```
ttgggtgttc atacagccga agccacactg cctaacggca acaacgatac gaagatagtc     60
aatattgcgc ccgatgccag caacgcgcag gttacgctga acatccctgc tcaacaggtg    120
gtgacgaata acagcgacag cgtgcagctg acggcgacgg tgaaagatcc gtcgaatcat    180
ccggtggcgg gaataacggt gaacttcacc atgccacagg acgtggcggc aaactttacc    240
ctcgaaaata acggtattgc catcacccag gccaatgggg aagcgcatgt cacgctcaaa    300
ggtaaaaaag cggtacgca tacggttacc gcaacgctga gtaataacaa taccagtgat    360
tcacagccgg taacgtttgt ggcggacaaa acctcggctc tggttgttct tcagatatca    420
aaaaatgaga tcacaggtaa tggcgtcgat agcgcaacgc taactgcaac ggtcaaagat    480
cagttcgaca atgaggtgaa caatcttccg gtaacattca gcacagcttc ttcaggcctc    540
accctgaccc caggggaaag taataccaat gagtctggca tcgcgcaggc cactctcgca    600
ggcgttgcct ttggtgagca gacggtcact gcatcactgg ctaataatgg tgccagcgac    660
aacaaaactg tgcattttat tggcgacaca gcggcggcaa aaattatcga gttgacgcct    720
gtcccagaca gcataatcgc aggtaccccg cagaacagct ccggcagcgt catcaccgcc    780
acagtcgttg ataataatgg ctttccggtg aaaggtgtga ctgtgaactt caccagcaac    840
gcagcgacag ccgaaatgac gaatggcggt caagccgtga cgaacgaaca gggtaaggct    900
accgtcactt ataccaatac ccgctcctcg atagaatcag gagcgagacc ggataccgtt    960
gaggccagtc tggaaaatgg tagctccacg cttagcacat caattaatgt caacgctgat   1020
gcgtctacgg cacatctcac cttgctacag gcactttttg atacagtctc cgcaggcgac   1080
actaccaatc tgtatattga ggtgaaggat aattacggca acggagtacc ccagcaggag   1140
gtaaccctca gcgtttcacc aagtgaaggt gtgacccccca gtaataacgc tatatatacg   1200
accaatcacg acggcaattt ttacgcaagc tttaccgcta caaaagccgg ggtataccaa   1260
gtgacggcaa ccctcgaaaa tggcgattcg atgcaacaaa cagtgaccta tgtgccgaac   1320
gtagcgaatg ctgaaatctc gctggcagcc tcgaaggatc cggtaattgc caacaataac   1380
gatctcacga cactaacagc aacagtcgct gatacagagg gcaatgcgat agccaacagt   1440
```

```
gaggtaacat ttactctgcc ggaagatgtg agggcgaact tcacgctggg cgatggcggt 1500
aaagtggtta ctgatactga aggcaaagcg aaagtcacgc tgaaaggtac aaaagcaggc 1560
gctcatactg ttacagcatc gatggctggc ggtaagagtg agcagttggt ggtgaacttt 1620
attgcggata cactcactgc gcaggttaat cttaacgtta ccgaggacaa ttttatcgct 1680
aataacgtcg ggatgaccag gctgcaggca acagtgactg atggaaacgg caacccgtta 1740
gccaatgagg cggtgacatt cacgctaccg gcagatgtga gcgcaagctt tactctcgga 1800
caaggcggtt ccgccattac tgacatcaac ggcaaggctg aagttacact gagcggtaca 1860
aaatccggca cctaccccgt gacagttagc gtgaacaatt atggtgtcag tgatacgaaa 1920
caggtgactt tgattgccga tgctggtacc gcaaaactag cctccttaac ctctgtatac 1980
tcattcgtcg tcagcacgac cgagggcgcg accatgactg caagcgtcac tgacgctaac 2040
ggcaacccgg tagaaggtat aaaagttaat ttccgcggaa cttccgtcac gctaagcagc 2100
accagcgttg aaacggatga tcggggtttc gctgaaattc ttgtgacaag caccgaggtc 2160
ggactgaaaa cagtttcagc ctctctggca gataaaccta ctgaagtcat ctcgcgatta 2220
ctgaatgcaa aagcagatat taattctgca acgattacca gtctggagat acctgaaggt 2280
caggtcatgg tcgcacaaga cgtagcagtt aaagctcacg tcaacgacca gtttggcaat 2340
ccgattctta atgaatctgt aacattcagt gcagaaccac cagagcacat gaccatcagc 2400
caaaatattg tctctactga tacgcatggt atagccgagg tcactatgac gcccgaaaga 2460
aacggttcgt atatggtgaa agcatccctg gcgaatggat cctcttatga gaaggatctg 2520
gtggtaatcg atcaaaaact gacactctcg gcgtccagcc cgcttatcgg tgtcaattcc 2580
ccaacaggtg caactctgac ggcaacgcta acttctgcaa atggcactcc agtggagggt 2640
caggtcatca actttagcgt aacgccagaa ggtgcgacgt taagtggcgg aaaagtgaga 2700
accaactctt caggtcaggc tccagtcgtt ctgaccagca ataaagtcgg tacatatacg 2760
gtgactgcat cgttccataa cggcgtaaca atacagacac agacaatcgt gaaagtcact 2820
ggcaactcaa gcaccgccca tgttgctagc tttatcgctg atccatcgac tatagccgcc 2880
accaacagtg atttaagtac cttaaaggca acggttgagg atggcagtgg taacctgatc 2940
gaaggtctca ctgtgtactt cgccttaaaa agcggctctg ccacattaac gtcattaaca 3000
gcggtgacag atcaaaacgg aatcgcgaca acaagcgtga gaggagcgat aacggggagc 3060
gtcacggtaa gcgcagtcac gaccgctggt ggaatgcaaa cagtagatat aacgctggtg 3120
gcaggcccgg cagacgcctc gcagtccgtc cttaagaaca atcggtcatc attgaaagga 3180
gactttaccg atagtgctga gctacatctt gttctgcacg atatatcagg caatccgatc 3240
aaagtttctg aagggctgga atttgtgcag tcaggtacca acgcgcccta tgtgcaagtt 3300
agtgcaattg actacagtaa aaatttctca ggcgagtaca aagccactgt tacaggcggc 3360
ggagagggta tcgcaacgct gatccctgta ttgaatggtg ttcatcaagc gggtctgagt 3420
accacaatac aattcactcg cgcagaagac aaaataatga gcggtacagt gttagtcaat 3480
ggtgctaacc taccgacaac tacattccct tcgcaggggt tcactggggc gtattatcag 3540
ttgaataatg acaactttgc cccaggaaaa acggcggctg attatgagtt ttcaagctct 3600
gcctcctggg ttgatgttga tgctaccggt aaagtgacat ttaaaaatgt cggcagcaaa 3660
tgggagagga ttacggcgac gccaaaaaca ggcggcccta gctatatata cgaaatccga 3720
gtgaagagtt ggtgggtgaa cgccggcgat gctttcatga tatacagcct tgctgaaaat 3780
ttttgcagta gcaatggcta cacacttccc cttggagacc atttaaacca tagtcgttcc 3840
cgaggcatcg ggtcactgta cagtgaatgg ggagatatgg ggcattacac gactgaagct 3900
ggttttcatt caaatatgta ttggtcatcg agtcccgcaa actcaaacga acaatacgta 3960
gtttccctgg caacaggtga tcaaagcgta tttgaaaagc ttgggtttgc ttatgcgaca 4020
tgttataaaa acctctga 4038
```

<212> Type : DNA
<211> Length : 4038
SequenceName : SEQ ID 661
SequenceDescription :
Sequence
--------
<213> OrganismName : Escherichia coli O157:H7
<400> PreSequenceString :

```
atgagcctga ttattgatgt tatttcgcgt aaaacatccg tcaaacaaac gctgattaat 60
cctggcgacg tcacggttgt tatttatgag ccttccgtgg tgcaggttca tgctcaggcc 120
tctgccgttg cgcgttacgt ccgtgaagga aatgacctgc tgatctatat gcaggacggc 180
acggtgatcc gctgcaacgg ttatttcctg caagcggcga atacagctga acaatcggaa 240
ctggtgtttg ccgatggtca acagctaacc catatcacct ttgccgatac tgctgcgggt 300
ggattagccc ccgtagaact gactgcccag accactgcga ttgaaagcat tgcgccattt 360
cttgataccg ttgctcagac cagcgccttc ccgtggggtt ggctggcggg ggcggcggta 420
ggtggtggcg cgcttggtgc actgctggca agcggtggcg atggcgactc gaaaacagaa 480
gtgattaata accctacgcc acctgctgag cctggcaacg ccacaccatc atttttagtt 540
accgataatc agggcgatca gcgcggcatt ctagccacca atgacatcac cgatgacacc 600
acgccaacct ttagcggcag cgggcaggcg gggcgacta ttcagattaa agacagtaac 660
ggcaatacta ttgccagtac tcaggtagac aacaacggtc actggagtgt ctcgttaccc 720
acgcaaagtg caggtgaaca tacctggtca gtggtgcaaa ttgtcggcag taccatcact 780
gacgccggtt cgataacgtt aaccatcgac aatagtcagg ccagcgtgca ggttgccacc 840
```

accgcaggcg ataacattat taacgccagc gaacaggccg ccgggtttac gctttctggc 900
accagtagcc atctggcgca gggaacagaa ctcaccgtta cgctaaacgg caaaacctac 960
acgaccagcg taggcgctaa cggtgcctgg agcgtgcagg tgccgaccgc cgatgcacag 1020
gcgttaggcg aagggaatca ggcggtgctg gtcagtggga aagacgccac aggcaatacg 1080
gtcaccggcg cgcagctact aacggtcgat acccaaccgc caacgcttgc catcaacacc 1140
atcgctcagg acaacattat cagtgctgcg gaacataacg tcgcgctggt actgagcggc 1200
acgtcgaatg cagaagcggg gcaaaccgta acactgaccg tcaacgggaa aagccataca 1260
gcaaccgtcg gtagcgacgg aacctggcaa gtgacgctgc ctgccacgga agtccaggca 1320
ctggcggagg gtaattacgc tgtcaatgcc agtgtcagcg atcgggcagg gaacaccacc 1380
agccacagcg cgaatttcac ggtagacacc tcagcacccg tggtcagtgt taataccgtg 1440
gcgggcgacg atattcttaa taatgccgag caggccgtcg cgcagatcat ctccggacaa 1500
gtcagcggtg cttctccagg cgatacggta acggtgaaat tgggcactca tgtcctgacg 1560
ggcatcgtgc tggcagatgg cagctggaat gtggcgctgg acccagcggt aacccgcacg 1620
ctggatcgcg gagccaatac gatttttcgtc accgtgacag atgctgcagg aaatactggc 1680
gcggcgtctc gagcaatcac gctggtcggt gtttctccgt tgatcaccat taacaccgtc 1740
tccggcgatg acattatcag tggcgcagaa aaaggtgcgc cactgaccct taccggtagc 1800
actcaacagg ctgagacagg acaaaccgtc acagtaaccc tggctggaca gagttttacc 1860
actaccgtgc aggccgatgg ctcctggagt ctgacggtac ctgccgccgc gatgggaaat 1920
ctgcctgacg gcgcggtggc gattaccgct tctgtgacgg atctcagcgg caataccggc 1980
aacacttccc gcaccattac cgtcgatagc caggccccgg ccttaagcat tgatccactg 2040
accgctgata acatcattaa cgccgccgaa agcgggcagg atctgcccat caccggcacc 2100
accgacgctc agccggggca gacggtgacc gttacgttaa atgggcagac gtatcagggc 2160
gtcgtgcagc cagacggcac ctggagcgtg actgtgcccg ccgccaacgt gggcgcactg 2220
gctgacggca acgctacggt caccgccagc gtgaacgatg tcgccggtaa tccgagcagc 2280
gtttcacgcg tggcgctggt ggatgccacg ccgccggtgg taaccattaa tccggtggcg 2340
accgataacg tcatcaacac gccggaacat gctcaggcgc aaatcatcag cggcacggtt 2400
actggcgctc aggcgggcga tatcgtcacc gtgacgctga ataatgtgga ttacaccacg 2460
gtggtggatg gttccggcaa ctggagtctg ggcgttccgg cctcggtggt cagtgggctg 2520
gcggacggca gttatcctgt cagcgtctcg gtaaccgaca aagccggaaa cacgggcagc 2580
cagtcattga ccgtcacggt caataccgcc gcgcccctta tcggcattaa cagcattgcg 2640
ggcgatgatg tgattaacgc cagcgaaaaa ggggccgatc tccagattac cggcaccagc 2700
gatcagcctg ttaacaccgc catcaccgtg acgctgaacg ggcaaaatta caccaccacg 2760
accgacgcct ccggcaactg gagcgtcacc gttccggcat cggcggttac agcattaggc 2820
caggccaact atacggtaac ggcggcggtg accagcgata tcggcaacag cgccactgcc 2880
agccataacg tgctggtcga cagcgcgctg cccggtgtga ccattaatcc ggtggcaacc 2940
gacgatatta ttaacgccgc cgaagcgggc gtggcgcaaa ccatcagcgg gcaggtgact 3000
ggcgcggaag atggcgacac ggtaactatt acgttgggtg gtaatactta tacggcgacg 3060
gtgggcagca atctcacctg gagcgtggac gttccagcgg cagatattca ggcgctggga 3120
aatggcgatt taacggttaa tgcctcagtc accaatcaaa acggcaacac cggcagcggc 3180
acgcgggata tcaccatcga cgccaatctg cccggcctgc gggtcgatac ggtggcgggc 3240
gatgatgtgg tcaatatcat cgagcacggg caggcgctgg tggtcaccgg cagcagctcg 3300
gggctggctg aaagcacgcc gcttaccgtt acgattaata atgtggaata caccactgcg 3360
gtgcaggccg atggtagctg gagcgtgggc gtcacggcgg cgcaggttag cgcctggcct 3420
gcggggacgg ttaatattgc cgtttcaggg gaaagtagcg ccggaaactc ggtgagcatt 3480
acgcatccgg tgacggtgga tctcactccg gcagcgatca ccatcaacac catcgccacg 3540
gacgatgtga ttaacgccgc agaaaaaggc gctgatttaa ccctttccgg caccaccact 3600
aacgtagaac ccggtcaaac cgtcaccgtc acctttggcg ggaaaaatta cactgccagc 3660
gtagcgagcg atggtagctg gactgccacc gtacccgccg ccgatctggc gtcattaccc 3720
gagggcagcg cctccgcact ggccagcgtc agcaatatca acggcaatag cgcctcggcg 3780
gtgcacaact acagcgtcga cagcagcgcg ccaaccatca ttatcaatac cgtcgccagc 3840
gacaatatcg tcaacgccag cgaagccgat gcgggcgtga cggtgagcgg cagtaccacc 3900
gccgaagcgg ggcagattgt tacgataacg cttaacagcc cgaccgtgca gacctatcag 3960
gcaacggtgc aggcggacgg cagctggagc atcaatattc cggcggcaga tcttgaggca 4020
ttgaccgatg gcagccacac cctgaccgcc acggtcaatg acaaagcggg caatccggcg 4080
agcaccacgc ataatctggc ggtggatctc accgttccgg tgctgaccat caacaccatt 4140
gcgggcgatg acattattaa cgccaccgaa cacgggcagg cgctggtgat ttccggttcc 4200
agcaccggcg gagaagcggg ggatgtcgtc accgtcacgc taaacagtaa aacctacacc 4260
accaccctgg acgcctccgg caactggagc gtcggcgttc cggcggcgga tgtcacggcg 4320
cttggcagcg gcccgcaaac tgtcaccgcc acggttaccg atgcggcagg caacagcgac 4380
aattag 4386

<212> Type : DNA
<211> Length : 4386
SequenceName : SEQ ID 662
SequenceDescription :
Sequence
--------
<213> OrganismName : Escherichia coli O157:H7
<400> PreSequenceString :

```
atgaatagaa tctatcgcgt gatatggaat tgcactctac aggtatttca ggcctgctcg     60
gaattaactc gcagggtagg taaaacatca acggttaatt tgcgaaagtc ctctggactg    120
acaacgaaat tcagtagatt gacgctgggt gttttgctgg cactaagcgg ttcagtgtct    180
ggtgcgagtc tggaagttga taatggtcag attaccaata ttgatactga tgttgcttat    240
gatgcctacc tggttggctg gtatggcact ggagtgctta atattttggc tggcggtaat    300
gcctccttaa ccactattac taccagcgtc attggcggta atgaggattc ggagggtacc    360
gttaatgttt tgggtggcac ctggcgattg tatgatagcg gaaataatgc aaggccttta    420
aatgtgggtc aatccggaac ggggacgctg aatattaaac agaagggtca cgtcgatgga    480
ggctatttaa gattaggtac tcaagctgca ggcgtcggga cggttaatgt tgagggagag    540
gactcggttt tgacgaccga attattcgaa atagggagtt acggtacggg ctcattaaat    600
attacggata agggttacgt cacgagttca atagtcgcca ttttaggata tcaagcgaac    660
agtaatggta aggttgtcgt tgaaaagggt ggcgagtggc taataaaaaa taatgattcc    720
tcaattgaat ttcaaattgg taatcaagga actggggagg cgactattcg cgagggtggg    780
ctgattacgg ctgaaaatac gattatcggt ggcaatgcca ccggtgtcgg aaccctgaat    840
gtgcaggatc aagactctgt catcacggta cgcagactct ataatggata tttcggtaat    900
ggcgcagtca atatttccaa taatggacta attaataaca aagaatattc attggtgggc    960
gttcaggacg gttcccacgg tgtcgtcaac gtgaccgata aagggcattg gaatttcctc   1020
ggaacgggcg aagctttccg ctatatctat atcggtgatg ctggcgacgg tgaacttaat   1080
gtctcgcgtg aaggtaaagt agattcggga attatcactg cggggatgaa agaaacaggc   1140
acaggcaacc ttactgttaa ggataagaac tccgttatca ctaatctcgg aactaatctt   1200
ggttatgacg gccacggcga aatgaatatc agtaatgagg ggcttgttgt cagcaacgga   1260
ggaagctctc tcggttatgg agaaaccggc gtcgggaagg ttagcatcac cacgggggg   1320
atatgggagg tcaataagaa tgtctatacc accattggtg ttgcgggcgt cggaaacctc   1380
aatattagcg atggcggtaa gttcgtatcg caaaatatta ctttttgggg cgataaagca   1440
agcggtatcg gcacactgaa cctgatggat gcgacatcgt cgttcgatac tgtgggtatc   1500
aatgtcggta attttggtag cggtatcgta aatgtcagta atggtgccac ccttaattca   1560
acgggctatg gatttatcgg aggaaatgcc tccggtaagg gaatagttaa tatttcaacg   1620
gatagtctct ggaatttaaa gacgtcttct actaacgccc aattgctaca ggtcggtgta   1680
ttaggcacgg gtgaactgaa tattaccacc ggaggtatag ttaaagcgcg tgatacacag   1740
atagctctca atgacaaaag taagggcgac gtgagggtgg atgggcagaa ctctcttctt   1800
gaaacattca atatgtacgt agggacatct ggtacgggta cgttaaccct gacgaatagc   1860
ggcacgctga atgtcgaagg tggagaagtt tacttaggtg tttttgaacc tgctgtagga   1920
acgctaaaca ttggtgcagc tcacggtgag gcggcggcag atgccggatt tatcaccaac   1980
gcgacgaaag tagagtttgg ctctggcgaa ggtgtttttg tctttaatca tactaataac   2040
agtgatgccg gctaccaggt cgatatgctg attacaggtg acgataaaga cggaaaagtg   2100
atccatgatg caggccatac ggtgttcaat gcagggaata cttatagcgg taaaacgctg   2160
gtcaatgacg gcctcctgac cattgcgtct catacggcag atggggtaac gggcatgggg   2220
tcgagtgaag taaccattgc aagccccggt acgctcgaca ttctcgcatc aacgaacagt   2280
gcaggagatt acacgctgac caatgcgctc aaaggcgatg gcttgatgcg agttcagctg   2340
tcatcctccg acaagatgtt tggctttaca catgcaacag ggactgaatt cgccggtgtt   2400
gcccaactga aagacagtac cttcactctg gaacgcgaca acaccgctgc gcttactcac   2460
gcgatgttgc agtctgacat tgaaaatacc acatcggtaa acgtgggaga gcaatccatt   2520
ggtggactgg ccatgaatgg cggtacgctc attttcgata cggatattcc tgctgcgacg   2580
cttgcagagg gatatatcag cgtcgatacg ctggttgtcg gcgcgagtga ctacacctgg   2640
aaaggccgta actatcaggt aaacgggacg ggcgacgtgc ttatcggcgt gcctaaaccg   2700
tggaatgatc ctatggcgaa taaccctctg acgacgctca atttgctgga acacgatgat   2760
aaccatgtcg gcgttcaact ggtgaaggcg caaacggtta ttgggtcggg tggctcatta   2820
acgttacgtg atttacaggg cgacgaggtg gaagcggaca aaacgttaca cattgcgcaa   2880
aacggaacgg tggtcgccga gggtgattat ggattccgcc tcacgaccgc accaggtgat   2940
ggtttgtacg ttaactatgg gctgaaagcg ctgaacatcc atggagggca aaagctgacg   3000
ttagccgaac atggcggagc ctatggcgca acggccgata tgtcggcaaa aatcggtggt   3060
gaagggggatc tggcaatcaa tacggtgcga caggtttcgc tttccaacgg tcagaacgac   3120
tatcaggggg caacctacgt tcagatgggg acattacgta ccgatgcgga tggcgcgctg   3180
ggcaacaccc gggaactgaa catcagcaac gcagccatcg tcgatcttaa tggatcgacg   3240
cagacggtag agacattcac cgggcagatg ggttcgactg ttttgttcaa agaagggtcg   3300
ctgacggtaa ataaaggtgg gatcagtcag ggtgaactga caggtggcgg aaacctgaat   3360
gttacagggg gaacgctggc tgtcgagggg cttaatgcac gctacaatgc gttaaccagc   3420
gttagcccaa atgcggaagt cagcctcgat aatacgcagg ggttaggcag aggaaatatt   3480
gccaatgacg gtctgttaac gctaaaaaac gtgactggcg aactgcgtaa tagcataagc   3540
gggaagggta tcgtgagcgc aaccgccagg acagatgtag agctggatgg cgataatagc   3600
cgctttgtgg ggcaattcaa cattgataca ggcagcgcgc tcagcgtcaa cgagcagaaa   3660
aacctgggtg atgcttccgt tatcaataat ggcctgctca ccatctccac tgagcgtagc   3720
tgggcgatga cgcacagtat cagcggtagc ggtgatttga caaaactggg taccgggatc   3780
ctgactctta acaacgattc ctcggcgtat cagggtacga cggatatcgt ggggggggaa   3840
attgctttcg gttccgactc tgccattaat acggcaagtc aacacattaa tatccataac   3900

agcggtgtga tgtcgggaaa tgtcaccact gcaggtgatg tgaacgttat gtctgggggg   3960
acactgcgtg tcgctaaaac cacaatcggc gaatcggcgg caacctggag aatggcggca   4020
cggttcaaat ga                                                       4032
```

<212> Type : DNA
<211> Length : 4032
SequenceName : SEQ ID 663
SequenceDescription :
Sequence
--------
<213> OrganismName : Escherichia coli O157:H7
<400> PreSequenceString :

```
atgggcatca aacaacacaa tgggaatacc aaagccgatc gtctcgctga attaaaaatc      60
cgttcgccct caattcaact gataaaattt ggcgctattg gtttgaatgc aattctcttt     120
tccccctgc tgatagctgc tgatacagga agtcaatatg gcaccaatat tactattaat     180
gatggtgaca gaattactgg agataccgcc gatccatcag gaaacctcta tggtgtaatg     240
accccagcag gaaacacgcc tggcaatatc aacctgggta atgatgtcac cgtcaatgtc     300
aacgacgcct ctggatatgc aaaaggaatc attattcagg gcaaaaacag ctccctgaca     360
gctaaccgac tcacagtaga tgttgttggt caaacctctg ccatcggcat taatttaatt     420
ggtgactata cccatgctga cttaggcaca ggcagcacca ttaagagtaa cgatgacggc     480
atcattattg ggcatagctc aacactaaca gccactcaat tcaccattga aaactcgaac     540
ggtataggcc taaccatcaa tgactatggc accagtgtcg atcttggaag cggaagtaaa     600
atcaagaccg atggaagtac aggtgtttat atcggtggtc tcaacggcaa taacgccaat     660
ggtgctgcgc gttttacggc gacagacctg acaatcgatg ttcagggcta cagcgccatg     720
gggataaacg tacagaaaaa ctctgttgtc gatctcggaa caaacagttc cattaaaacc     780
agtggcgata atgcacacgg cctctggagc tttggccagg ttagcgcgaa tgcactcact     840
gttgatgtaa ctggagccgc ggccaatggc gtcgaagttc gtggtggtac aaccactatc     900
ggtgcagata gccatatttc ttccgcgcag ggcggtggtc tcgtcaccag tggttcagac     960
gcgacaatca atttttctgg cacggcagcg caacgaaaca gcatcttttc cggcggttct    1020
tatggtgcct cggcccagac ggcaacggct gttatcaaca tgcaaaatac cgatattacg    1080
gttgatcgta atggcagtct ggcgctgggt ttgtgggcgc tcagcggcgg tagaataacc    1140
ggagacagtt tggctatcac cggcgcggca ggagccaggg ggatttatgc catgaccaac    1200
agccagatcg acctcacgag cgatctggtc attgatatga gtacacccga ccagatggcc    1260
atcgcaacgc aacatgacga tggttatgcc gccagccgca tcaacgcctc gggtcgtatg    1320
cttatcaacg gtagcgttct ttccaaaggt gggctaatca atctggatat gcaccctggg    1380
tcggtttgga caggttcctc cctcagcgat aatgtcaatg gcgggaaact ggacgttgca    1440
atgaataaca gcgtctggaa cgtaacaagt aattctaatc tcgacacgct ggcgctgagc    1500
cattcaactg tcgattttgc cagccacggg tcaactgccg gcacatttac cacattaaac    1560
gtagagaacc tgagcggtaa cagtaccttt attatgcgtg ctgatgttgt tggcgagggt    1620
aatggcgtta agccctgggc ctga                                           1644
```

<212> Type : DNA
<211> Length : 1644
SequenceName : SEQ ID 664 SequenceDescription :
Sequence
--------
<213> OrganismName : Escherichia coli O157:H7
<400> PreSequenceString :

```
gtggggatcg acagccgtaa tgatattcct gaggggattg cgacgctggg cgcttttatg      60
ggttattccc attcacatat cggttttgat cgtggaggac atggcagtgt ggacagttat     120
tctctgggcg gctatgccag ttgggaacat gaaagtggtt tctatctgga cggtgtcgtg     180
aagctgaacc gttttgaaag taacgtagcc ggtaaaatga gcagcggtgg agccgccaat     240
ggcagttacc atagcaacgg gctgggcggt cacattgaaa ccgggatgcg atttaccgat     300
ggtaactgga acctgacgcc gtatgcctcg ttaacggggt tcaccgctga taaccccgaa     360
tatcatttat ccaatggcat ggaatcgaaa tcagtcgata cccgcagtat atatcgtgaa     420
ctgggtgcaa cgctgagtta caacatgcgt ctggggaacg gtatggaagt tgagccgtgg     480
ctgaaggcgg ctgtgcgcaa agaatttgtc gatgataacc gggtgaaagt gaatagtgac     540
ggtaatttcg tcaatgattt gtcgggcaga cgtggaatat accaggcagg tattaaagcc     600
tcattcagca gtacgttaag cgggcatctt ggggtggggt atagcaacgg tgctggtatg     660
gaatccccgt ggaacgcggt ggctggtgtg aactggtcgt tctga                     705
```

<212> Type : DNA
<211> Length : 705
SequenceName : SEQ ID 665
SequenceDescription :
Sequence
--------

<213> OrganismName : Escherichia coli O157:H7
<400> PreSequenceString :

```
atgaaaaaaa aggttctggc aatagctctg gtaacggtgt ttaccggcat gggtgtggcg      60
caggctgctg acgtaacagc tcaggctgta gcgacctggt cagcaacagc caaaaaagac     120
accaccagta agctggttgt gacgccactc ggtagcctgg cgttccagta tgccgaaggc     180
attaaaggtt ttaactcaca gaaaggtcta tttgacgtgg ctatcgaggg tgactcaacg     240
gctaccgcct ttaaactgac ctcacgtctt atcaccaaca cattaaccca gttggatacc     300
tcaggttcca cactgaatgt gggcgtggat tataacggcg cggcagtcga aaaaactggc     360
gataccgtga tgatcgatac cgccaacggc gtactggcgc gcaaccttag cccgctggca     420
aacggttaca atgccagcaa tcgtaccacc gcacaggatg gtttcacttt ctccatcatc     480
agcggcacca ccaatggtac caccgcagta accgattaca gcactctacc ggaaggcatc     540
tggagcggcg acgttagcgt acagttcgac gcgacctgga ccagttaa                  588
```

<212> Type : DNA
<211> Length : 588
SequenceName : SEQ ID 666
SequenceDescription :
Sequence
--------
<213> OrganismName : Escherichia coli O157:H7
<400> PreSequenceString :

```
atgacggcag aatcctacga tgataactac ctggatgatg aagacgcgga ctggaccgcg      60
accgggcagg ggcagaaatc tgcaggtgat accagtttta cgctggcctg gaaaccggga     120
gaggaaggcc agaaagggct tataggctgg tttgaaagcg gcgatgtccg ggcctataaa     180
atccgttttc cgaatggcac ggtggatgtg tttcgtggct gggtcagcag tatcggtaag     240
gccgtgacgg cgaaagaagt gatcacccgc acggtgaaag tcactaacgt gggtaaacct     300
tctgtagcgg aagaacgcag caaaattacg ccggtcagtg cgattaaggt gacgccgaca     360
tccggtacgg tggcaaaagg gaaaacaacc accctgacgg tttcttttga gccggaaagt     420
gcaacagaca agacgttcag agcggtttcc gccgatccgt cgaaagccac cattagtgtg     480
aaagatatga caattacggt aaacggcgtg gcgacaggta aggtgcagat ccctgtggtg     540
agcggaaatg gtcagttcgc cgcagtggct gaagtcaccg ttactgaagc gggcgctgca     600
gggtaa                                                                606
```

<212> Type : DNA
<211> Length : 606
SequenceName : SEQ ID 667
SequenceDescription :
Sequence
--------
<213> OrganismName : Escherichia coli O157:H7
<400> PreSequenceString :

```
atgacggcag aatcctacga tgataactac ctggatgatg aagacgcgga ctggaccgcg      60
accgggcagg ggcagaaatc tgcaggtgat accagtttta cgctggcctg gaaaccggga     120
gaggaaggcc agaaagggct tataggctgg tttgaaagcg gcgatgtccg ggcctataaa     180
atccgttttc cgaatggcac ggtggatgtg tttcgtggct gggtcagcag tatcggtaag     240
gccgtgacgg cgaaagaagt gatcacccgc acggtgaaag tcactaacgt gggtaaacct     300
tctgtagcgg aagaacgcag caaaattacg ccggtcagtg cgattaaggt gacgccgaca     360
tccggtacgg tggcaaaagg gaaaacaacc accctgacgg tttcttttga gccggaaagt     420
gcaacagaca agacgttcag agcggtttcc gccgatccgt cgaaagccac cattagtgtg     480
aaagatatga caattacggt aaacggcgtg gcgacaggta aggtgcagat ccctgtggtg     540
agcggaaatg gtcagttcgc cgcagtggct gaagtcaccg ttactgaagc gggcgctgca     600
gggtaa                                                                606
```

<212> Type : DNA
<211> Length : 606
SequenceName : SEQ ID 668
SequenceDescription :
Sequence

--------

<213> OrganismName : Escherichia coli O1S7:H7

<400> PreSequenceString :

```
atgttatata atataccttg tcgaatttat atcctttcca ctctgtcatt atgcatttct     60
gggatagttt ctactgcaac cgcaacttct tcagaaacaa aaatcagcaa cgaagagacg    120
ctcgtcgtga ccacgaatcg ttcggcaagc aacctttggg aaagcccggc gactatacag    180
gttattgacc aacaaacatt gcagaactcc accaatgcct ccatagccga taatttgcag    240
gacatccccg gagtagagat aacagacaac tccttggcag gccgtaaaca aatccgcatt    300

cgtggcgaag catcctcccg tgttttaatt ctcattgatg gtcaggaggt aacttatcag    360
cgcgccggag ataattatgg tgtgggactg ttgatagatg agtctgcgct ggagcgtgtt    420
gaggtagtga aggtccata ttccgtactg tacggttcac aggcaattgg cggtattgtt    480
aacttcatca ccaaaaaggg aggtgacaaa cttgcatctg gagttgtgaa agctgtttat    540
aattccgcaa cagcaggctg ggaagaatca atcgcggtcc aggggagcat cggtggattt    600
gattatcgca tcaacggtag ttattctgat cagggcaatc gtgatacgcc ggatggacgt    660
ctgccgaata ccaactatcg taacaatagt cagggtgtat ggttgggtta taactccgga    720
aaccatcgtt ttggcctctc gcttgatcgc tacagactcg cgacacaaac ttactatgag    780
gatccagacg gaagttatga ggcatttagt gtcaaaatac ctaaacttga acgagagaaa    840
gttggggtat tctatgacac agacgtggac ggtgactatc taaaaaaaat tcatttcgac    900
gcgtatgagc agaccatcca gcgccaattt gccaacgaag taaaaacgac acagcctgtt    960
cccagtccga tgattcaggc tctgaccgtt cataacaaga ctgacaccca tgataagcaa   1020
tacactcagg cggtcacatt gcagagtcac ttttcgctgc ctgctaataa tgaacttgtt   1080
accggtgcac agtacaaaca agatagggtc agccaaaggt ccggtggcat gacctcaagc   1140
aaatctctga ccggcttcat taataaggaa acacgaactc gctcctatta tgagtcagag   1200
caaagtacag tctcactatt cgcacaaaat gactggcaat tcgccgatca ctggacatgg   1260
acaatgggag ttcgccaata ctggctttct tcaaagttga cgcgtggtga cggagtatca   1320
tataccgcag gcattataag cgatacctct cttgccagag agtctgcgag tgatcacgaa   1380
atggtaacat ctacaagcct gcgctattca ggtttcgata acctggagtt acgcgctgcg   1440
ttcgcgcaag gctacgtatt tcccacactc tcccagcttt ttatgcagac atctgcgggc   1500
ggcagtgtca catacggaaa tcctgatctt aaggctgaac actccaataa ctttgaatta   1560
ggtgcacgat ataatggtaa tcagtggctg attgacagcg cagtttacta ctcagaagct   1620
aaagattata ttgcaagtct gatctgtgat ggcagtatag tttgcaatgg taacaccaac   1680
tcctcccgta gtagctacta ttattatgac aatattgatc gggcaaaaac atggggactg   1740
gaaataagcg cggaatataa tggctgggtt ttctcgccat atatcagtgg caatttaatt   1800
cgtcggcaat atgaaacttc aacattaaaa acaactaata caggtgaacc agcgataaac   1860
ggacgtatag ggctgaaaca tactcttgtg atgggtcagg ccaacataat ctctgatgtt   1920
tttattcgtg ctgcctctag tgcaaaagat gacagtaacg gtaccgaaac aaatgttccg   1980
ggctgggcca ctctcaactt tgcagtaaat acagaattcg gtaacgagga tcagtaccgg   2040
attaacctgg cactcaataa cctgacagac aaacgctacc gtacagcaca tgaaactatt   2100
cctgcagcag gttttaatgc agctataggt tttgtatgga atttctga                2148
```

<212> Type : DNA

<211> Length : 2148

SequenceName : SEQ ID 669

SequenceDescription :

Sequence

--------

<213> OrganismName : Escherichia coli O157:H7

<400> PreSequenceString :

```
atgacaaaaa tgagtcgcta cgccttgatt accgcgctgg cgatgtttct cgccgggtgt     60
gtggggcaac gtgaacctgc accggtagaa gaagtgaaac cagcgccgga acaaccagcc    120
gagccacaac agcctgtccc cacagtgccc tcggtgccga cgatcccgca gcagccaggc    180
ccaattgagc acgaagatca aactgcaccg cctgcgccgc atattcgcca ttatgactgg    240
aatggcgcaa tgcagccgat ggtcagtaag atgcttgggg ctgacggggt gactgcgggt    300
agcgtcctgc tggttgatag cgttaacaac cgtactaacg gttcgctgaa tgccgcagaa    360
gcgaccgaaa cgctgcgaaa tgcgctggct aataacggga aatttaccct ggtttccgcc    420
cagcagctgt cgatggctaa gcaacagtta ggtttgtcgc cgcaggatag tttaggcacc    480
cgtagtaaag ccataggcat tgcccgcaat gtcggcgctc attacgtgct gtactccagc    540
gcctctggca acgttaacgc tccgacccta caaatgcagc tgatgctggt gcagacgggc    600
gaaattatct ggtcaggtaa aggtgccgtt tcgcagcaat aa                       642
```

<212> Type : DNA
<211> Length : 642
SequenceName : SEQ ID 670
SequenceDescription :
Sequence
--------
<213> OrganismName : Escherichia coli O157:H7
<400> PreSequenceString :

```
atgaaaagca aagtactggc acttttaatt cctgccctgc tcggcgcagg tgctgcacat    60
gcagccgaag tttataataa agacggcaac aaattagatc tgtatggcaa agttgatggc   120
ctgcattatt tttctgataa ttcagcgaaa gatggcgacc agagctatgc gcgtctgggt   180
tttaaaggcg aaacccaaat taacgatcaa cttactggct acggtcaatg ggaatacaat   240
attcaggcaa acaacactga atcttcaaaa aaccagtcat ggacccgtct ggcatttgcc   300
ggactgaaat tttcagatta cggttctttc gattacggac gtaattatgg gctggaccga   360
tatgctgcct ga                                                       372
```

<212> Type : DNA
<211> Length : 372
SequenceName : SEQ ID 671
SequenceDescription :
Sequence
--------
<213> OrganismName : Escherichia coli O157:H7
<400> PreSequenceString :

```
atggcaacac caaatcccct tgagccggta aaaggtgccg gtaccactct gtgggtttac    60
aacggcaagg gtgatgctta tgcaaacccg ttgtcagacg atgactggca gcgactggct   120
aaggtgaagg atctgacgcc gggcgagatg acggcagaac cctacgatga taactacctg   180
gatgatgaag acgcggactg gaccgcgacc gggcaggggc agaagtctgc aggagatacc   240
agttttacgc tggcctggaa accgggagaa gaaggtcaga aagggcttat aggctggttt   300
gaaagcgggg atgtgcgggc ctataaaatc cgtttcccaa atggcacggt ggatgtgttc   360
cgtggctggg tcagcagtat cggtaaggcc gtgacggcga aagaagtgat cacccgcacg   420
gtgaaagtca ctaacgtggg caaaccttcc gtggcggaag aacgcagcga aattacgccg   480
gccactgcaa ttaaggtgac accgacatcc ggtacggtgg caaaaggaa aacaaccacc    540
ctgactgttt cttttgagcc ggaaagtgca accgacaaga cgttcagagc ggtttccgcc   600
gatccgtcga aagccaccat tagtgtgaaa gatatgacaa ttacggtaaa cggcgtggcg   660
acaggtaagg tgcagatccc tgtggtgagc ggaaatggtc agttcgccgc agtggctgaa   720
gtcaccgtta ctgaagcggg cgctgcaggg taa                                753
```

<212> Type : DNA
<211> Length : 753
SequenceName : SEQ ID 672
SequenceDescription :
Sequence
--------
<213> OrganismName : Escherichia coli O157:H7
<400> PreSequenceString :

```
atggcaacac caaatcccct tgagccggta aaaggtgccg gtaccactct gtgggtttac    60
aacggcaagg gtgatgctta tgcaaacccg ttgtcagacg atgactggca gcgactggct   120
aaggtgaagg atctgacgcc gggcgagatg acggcagaac cctacgatga taactacctg   180
gatgatgaag acgcggactg gaccgcgacc gggcaggggc agaagtctgc aggagatacc   240
agttttacgc tggcctggaa accgggagaa gaaggtcaga aagggcttat aggctggttt   300
gaaagcgggg atgtgcgggc ctataaaatc cgtttcccaa atggcacggt ggatgtgttc   360
cgtggctggg tcagcagtat cggtaaggcc gtgacggcga aagaagtgat cacccgcacg   420
gtgaaagtca ctaacgtggg caaaccttcc gtggcggaag aacgcagcga aattacgccg   480
gccactgcaa ttaaggtgac accgacatcc ggtacggtgg caaaaggaa aacaaccacc    540
ctgacggttt cttttgagcc ggaaagtgca accgacaaga cgttcagagc ggtttccgcc   600
gatccgtcga aagccaccat tagtgtgaaa gatatgacaa ttacggtaaa cggcgtggcg   660
acaggtaagg tgcagatccc tgtggtgagc ggaaatggtc agttcgccgc agtggctgaa   720
gtcaccgtta ctgaagcggg cgctgcaggg taa                                753
```

<212> Type : DNA
<211> Length : 753
SequenceName : SEQ ID 673
SequenceDescription :
Sequence
--------
<213> OrganismName : Escherichia coli O157:H7
<400> PreSequenceString :

```
atgggctgga ccgatatgct gcctgaattt ggcggtgact cttataccaa tgcagacaac      60
tttatgactg gtcgtgccaa tggcgtcgcg acttatcgta atactgattt cttcggtctg     120
gtaaatggtc tgaacttcgc ggtgcagtat caaggtaaca acgaaggtgc cagtaatggt     180
caggaaggca ccaacaacgg acgtgatgtt cgccatgaaa acggtgacgg ctggggtctt     240
tccacaacat atgatttagg catgggcttt agcgctggtg cggcatacac ctcttctgac     300
cgcaccaatg accaggttaa ccatactgcg gcgggtggtg ataaagcaga cgcgtggact     360
gctgggctaa aatacgatgc taacaatatt tacctggcaa ccatgtattc agaaacgcgt     420
aatatgaccc cgtttggcga cagcgattat gctgttgcaa acaaaaccca gaactttgaa     480
gtcactgcac agtaccagtt tgattttggt ctgcgtccgg cagtctcttt cctgatgtct     540
aaaggccgtg acctgcacgc tgccggtggt gcagacaacc cggcaggtgt tgatgataaa     600
gatctggtta aatacgccga tgttggcgcg acttactatt tcaataaaaa catgtccact     660
tatgttgact ataaaatcaa cctgttggat gaagatgaca gtttctacgc tgccaacggc     720
atctctaccg atgatattgt cgctttaggt ctggtttatc agttctaa                  768
```

<212> Type : DNA
<211> Length : 768
SequenceName : SEQ ID 674
SequenceDescription :
Sequence
--------
<213> OrganismName : Haemophilus influenzae Rd
<400> PreSequenceString :

```
atggggttta ttatgaaact aactaaaaca gcattatgta ctgcactttt tgccaccttt      60
actttttcag cgaacgcaca aacttatcct gatttgccag tggggatcaa aggaggaaca     120
ggcgcattaa ttggcgacac agtttatgtg ggattaggtt ctggtggcga taaattctat     180
accttagacc taaaagatcc ttcagcacaa tggaaagaaa ttgctacatt tccgggtggc     240
gaacgcaatc aacctgttgc agccgcagtg gatggaaaac tttatgtatt cggtggttta     300
caaaaaaatg aaaaaggcga acttcaactc gttaatgacg cttatcgcta taacccaagc     360
gataatactt ggatgaaact tcctactcgt tctccccgtg gtttagtagg atcaagtggt     420
gcatctcacg gagataaagt ttatattta ggcggctcta atctctctat atttaatggc     480
ttcttccaag acactgtggc agcaggtgaa gataaagcga aaaaagatga aatcgcagca     540
gcttatttcg atcaacgtcc agaagattat ttctttacaa cagaattatt gagctatgaa     600
ccatcaacca ataaatggcg caatgaaggt cgtattccat tctctggtcg tgctggtgca     660
gcctttacaa ttcaaggtaa tgagctagtg gttgtcaatg gcgaaattaa acctggactt     720
cgcaccgctg aaacccatca aggtaaattc actgctaaag gtgtgcaatg gaaaaactta     780
cctgacttac ctgctccaaa aggcaaatca caagatggtt tagctggtgc gctttcaggc     840
tatagtaacg gtcattattt agtcactggc ggcgcaaatt ttccaggttc aatcaaacaa     900
ttcaaagaag gaaaacttca cgcacataaa ggtttaagca aagcttggca taacgaagtt     960
tatacgttga ataatggtaa atggcgcatt gttggggaat taccaatgaa tattggttat    1020
ggttttctg tatcttacaa caataaagtt ttactgattg gcggtgaaac tgatggaggt    1080
aaggctttaa ctagcgtcaa agcaataagc tatgacggta aaaaattaac catcgaataa    1140
```

<212> Type : DNA
<211> Length : 1140
SequenceName : SEQ ID 675
SequenceDescription :
Sequence
--------
<213> OrganismName : Haemophilus influenzae Rd
<400> PreSequenceString :

```
atgggagaac aatatatgct tacgacgata ctcagcttcc tcattgtaac cactgtggtt      60
gcgtatgttt cttggttaaa aacaaaaggg gatgatttaa aatcttcaaa agggtatttt     120
ttggcagggc gtgggttaag cggtttagta attggctgct caatggtgct gacatcgctt     180
tcaactgaac aacttatcgg tgtcaatgcg gtgtcctata aaggtaattt ttccgtgatt     240
gcttggacag ttccaacggt tattccgctt tgtttcttgg ctctttatat aattggctgg     300
ctataa                                                                306
```

<212> Type : DNA
<211> Length : 306
SequenceName : SEQ ID 676
SequenceDescription :
Sequence
--------
<213> OrganismName : [Helicobacter pylori, strain J99
<400> PreSequenceString :

```
atgaaaaatc aacacaaaaa tcccctaaca aaagctttaa tgaaaactta tccatataac      60
catttttat ttttctgctt tattctagga gcgtttttat taggtttgct cagtccagct     120
tatgctttaa gtattatcac cactaaagaa attgacgcta atttgcttaa tggagcgata     180
gaaagcaggg tggtgttagg caagagggtg tttaaagtag aagctcatgg gttttatttt     240
agaaacaatg cgactaacag catagatata gaaatcacca gtcttttaag agacaatcaa     300
tcgtttcctt tgactagcag tgctaaaacc agtttaaaaa tacctcctaa cgccaagatt     360
aaaaaatcca ctatccttgt tttgaaaggc gagaacgctg aagaagtggc taagatttta     420
ggcgttagca aagaagaata ccaaaagcta gaaaacatcg ctcaaaccaa agcggctaat     480
gaccctatgt atgctaacac gccttttagt aatggttctg atagttcctt ttacgataac     540
aatcctaata gccctagcaa taacgctatc aatggcaaag atggcgcaaa tgggagtaac     600
ggctatgggg caaatggcaa tgatggggta aatggatca gtgggagtaa tggtgcaaat     660
gggagtcatt caaataataa tgcaataggc agtggtattg atacagatgg cgtgttaggg     720
gtggatgggg tgaatggctc tagttcttca agtggcggct ctgtaggggg ttatgagaat     780
aatttcacta atcatggctc tactaacaat aacacaggag ggtatgacaa ttttaataat     840
ggcagctcaa gtggtgggag tttagggaat ggggggcttt tccctattcc ttttggtaat     900

ggagacacaa acaattccaa taattccact aacaccacta gcccaactaa tggcagtagt     960
tctaataacg ccactaatcc tagttcgcaa gaaaacaatt actccagcca gtattgtaaa    1020
gtgccagagt taagccccaa caacacgatg aaactagatg ttatcgctaa agatggctct    1080
tgtatttcta tgaacgcttt aagagatgac actaaatgcg cttatagata cgattttgaa    1140
gccggtaaag ccatcaagca aacgcaatac tactatgtag atagggaaaa taaaacgcaa    1200
aatatcggtg gttgtgtgga tttacaaggc gctcaatacg ccatgcaact ttacaaagat    1260
gacagcaaat gcgccttaca aaccacgagc gataaaggtt atggtatggg gaaaacgcaa    1320
acctttcaaa ctgaaatcgt gtttcgtggg atggacaatt taatccatgt cgctgtgcct    1380
tgcagcgatt atgcaagggt gcaagacagg attgttaggt atgaaaaaaa tgataaaacc    1440
caaaccttaa cgcctatagt ggatcagtat tataatgatc ctaacaaccc taacaagcaa    1500
gagattttaa atcgtgggat tgccacccaa ttaagctcgc aatatcaaga atttgcatgc    1560
ggtcaatggg aatacaatga cgctaaatta gaagccaaaa gacctacaat gctaaaaagc    1620
tataacaagc ttaatggaga atgggtagaa gttacgccct gtaattttga agcagggatt    1680
aaaagcggtg cggttgttag cccttatgtg atgggcgtgc ctagttctaa agtcttaagc    1740
gatattacta caagccatta ttttaggata gaaaggaaaa attatggtga gagagaacaa    1800
tgccaaaaac tttatggagt caatcgttgc caaccgcaat attccatact gatcctagta    1860
tcaccgattg gagcgccact tacaaaacca ctaccaccca aaccactcaa ccttatttac    1920
gcccagccca agataatgaa aaacacccca caacctataa tcttatcacc actcaaacca    1980
ccatcaacag gactcaaagc gttttga                                        2007
```

<212> Type : DNA
<211> Length : 2007
SequenceName : SEQ ID 677
SequenceDescription :
Sequence
--------
<213> OrganismName : Helicobacter pylori, strain J99
<400> PreSequenceString :

```
atgcctgtta taagagtttt agtaatgctt gcaacaatga tgatgaaatt agtaaaaacg      60
gcaaaagaaa agaaagtttt taagaatgtg ggaatatcta taatggggat tgctttttgg     120
gaagcgataa aagactcgat aaaaaaacaa attaaaaaaa gcgattggat atgcgggaat     180
gttaagactg cggatgatta tttaaaaacg catcctaact catggtttaa ttcagcaata     240
ggtgtaacag cgataacagc catgcttatg aatgtgtgtt ttgctgatga ccaatccaaa     300
aaagaagtgg ctcaagctca aaaggaagct gaaaacgcta gggatagagc gaacaagagt     360
gggatagaac tggaacaaga agagcaaaag acagaacaag aaaaacaaaa gacagaacaa     420
gaaaaacaaa agacagaaca agaaaaacaa aagacagaac aagaaaaaca aaagacagaa     480
caagaaaaac aaaagacaag caatatagag actaacaatc aaataaaagt agaacaagaa     540
caacaaaaga cagaacagga aaaacaaaag acaaacaata cgcaaaaaga tttggttaac     600
aaagcagaac aaaattgcca agaaaatcat aatcaattct ttattaaaaa attaggaatt     660
aaggctggca ttgctataga aatagaagct gaatgcaaaa cccctaaacc cacaaaaacc     720
aatcaaaccc ctatccagcc aaaacacctc ccaaactcca aacaacccca ttctcaaaga     780
ggatcaaaag cgcaagagct tatcgcttat ttgcaaaaag agctagaatc tctgccctat     840
tcacaaaaag ctatcgctaa acaagtggat ttttataggc caagttctat cgcttattta     900
gaactagatc ctagagattt taacgctaca gaagaatggc aaaaagaaaa tttaaaaata     960
cgctctaaag ctcaagctaa aatgcttgaa atgaggagtt taaaaccaga cccacaagcc    1020
cacctttcaa cctctcaaag ccttttgctc gttcaaaaaa tatttgctga tgttagtaaa    1080
gaaataaaag tagttgctaa taccgagaaa aaagtagaaa aagcgggtta tggttatagt    1140
aaaaggatgt ag                                                         1152
```

<212> Type : DNA
<211> Length : 1152
SequenceName : SEQ ID 678
SequenceDescription :
Sequence
--------
<213> OrganismName : Helicobacter pylori, strain J99
<400> PreSequenceString :

```
atgaactacc ctaatctacc taacagcgct ttagagataa gcgaacagcc agaagtgaaa      60
gaaatcacta acgagctttt aaagcaatta caaaacgctt taaggagcaa cgcgcatttt     120
agcgagcaag tggaattaag ccttaaatgc atcgttagga ttttagaagt gcttttgagt     180
ttggattttt ttaagaatgc gaatgagatt gatagcagtt taagaaattc cattgagtgg     240
ctgactaacg ccggcgagag cttgaaatta aaaatgaaag aatacgagcg ctttttttagc    300
gagtttaata cgagcatgca tgccaacgag caggaagtaa ccaatacctt aaacgctaac     360
gccgagaaca ttaaaagcga aattaaaaag ctagaaaatc aattgataga aacccacgaca    420
agacttttaa cgagctatca aatcttttta aaccaagcca gagataacgc taacaaccaa     480
atcacaaaaa acaaaaccca aagccttgaa gcgattacac aagctaaaaa caacgctaat     540
aatgaaataa gcaacaatca aacgcaagcg ataactaata tcaccgaagc gaaaacgaac     600

gctaataatg aaataagcaa caatcaaacg caagcgataa ctaacattaa cgaagccaaa     660
gaaagcgcta caacgcaaat aaacgccaat aagcaagaag caataaataa catcacgcaa     720
gaaaaaaccc aagccacaag cgagatcacc gaagcgaaaa agaccgatca ttatcaaaac     780
attgattttt ttgagtttga ataa                                             804
```

<212> Type : DNA
<211> Length : 804
SequenceName : SEQ ID 679
SequenceDescription :
Sequence
--------
<213> OrganismName : Helicobacter pylori, strain J99
<400> PreSequenceString :

```
gtgaaatttt tttcaaagga tttgtttaaa aaagtaactc ctttattttt aagcgtttat    60
tttttaagcc ccacccttac gcaagccaaa agccgttttt atgtggcttc tcaataccag   120
gtggggaaaa tgatcatgaa aaaatacaac gatctcaaac gcacgattga aggggcgagc   180
ttttctttag gctgggagat taaccccact aattactggt tttattcgcg ctattacttt   240
tttatggatt acgggaatgt cattctcaat aaaagaacgg gcgctcaagc gaacatgttc   300
acttacggct ttggagggga tttgatcatg gaatacaata aaaacccttt gtatgtattt   360
tctctttttt atggcatgca agttgctgaa aacacatgga cgatttccaa acacagcgcg   420
aatttcatca ttgacgactg gcgcagcatt caagggtttt cgctcaaaac ttcaaatttc   480
aggatgttgg gtttagtggg gtttaaattc caaaccgtgc tattccacca tgacgctagt   540
attgaagtgg ggatcaaatg gcctttttgct tttgaatacg actcaccctt tgtaaggctt   600
ttttctgtct ttatttcgca cactttctac ctttaa                             636
```

<212> Type : DNA
<211> Length : 636
SequenceName : SEQ ID 680
SequenceDescription :
Sequence
--------
<213> OrganismName : Helicobacter pylori, strain J99
<400> PreSequenceString :

```
atgaaaaaat ttaccctatc gctatttttg tgttgcacct tacttaacgc tgaagaggat    60
atttttagga acaacacaaa tgaaactgat cttacaaatt cttttgaaca tggcaaagaa   120
aacaacaatc ttatcccagc aaaatccgat agtttagaaa gtttcaaaga acaagaaaac   180
aaagaaaaag ccaaacagct tatggattta aaggccttac agagcgtgta tttttctaaa   240
aatagaaaat tgcaagacaa taatttcaat gtcttgtatg tggcaggcaa caccaacaaa   300
atccgcttac gctatgcgat gactaccacc tttatttttg ataatgatcc tattatctat   360
gtgagtttag gagatcctag cgattttgaa ctcacttacc ccactaatga tcattacgat   420
ttgtctaaca tgctagtgat taagccgtta cttatagggg tggatacgaa cctaaccgta   480
gtcggagcga gcggcacaat ttataccttaa ttatttgttt ag                      522
```

<212> Type : DNA
<211> Length : 522
SequenceName : SEQ ID 681
SequenceDescription :
Sequence
--------
<213> OrganismName : Mycoplasma pneumoniae
<400> PreSequenceString :

```
gtgttggatt atgtgccctg gattgggaat gggtacaggt atgggaataa ccaccggggg    60
agcaacagta gtacgagtgg ggtaacgacc cagggacaat cccaaaatgc atccagtaac   120
gaacccgcac cgacgttttc gaatgtcggc gttggtctca aagccaatgt caacggcacc   180
ttaagtggtt cgcgaactac gcctaatcag caaggcactc cctggttaac cctcgaccaa   240
gccaacctcc agctctgaac gggcgcgggg tgaaggaatg ataagaacgg acaaagtgac   300
gaaaactaca ccaatttcgc gagtgctaag ggcagtacga accagcaggg ttcaacaacg   360
ggtggatcag cgggcaaccc cgactcgtta aagcaggata aggctgataa aagtggtgat   420
tcggtaacgg ttgcagaagc cacatcgggc gacaacttga cgaattacac caacctcccc   480
ccaacatcac ccccacatcc gactgaccga acgcgctgtc attcaccaac aagaacaacg   540
cccagcgggt gcagctgttc ctgcgcggcc tgttgggcag catcccggtg ttggtcaata   600
agagtgggca agatgataac agtaagttta attccaccga ccaaaaatgg tcttacaccg   660
aattaa                                                              666
```

<212> Type : DNA
<211> Length : 666
SequenceName : SEQ ID 682
SequenceDescription :
Sequence
--------
<213> OrganismName : Mycoplasma pneumoniae
<400> PreSequenceString :

```
gtggatgata taaccgcgcc tcaaaccagc gcggggtcgt ccagcggaac tagtacgaac      60
acaagtggtt cgcgttcctt tctcccgacg ttttcgaatg tcggcgttgg cctcaaagcg     120
aatgtccagg gcaccctcgg gggcagacag acgacgacta cggggaacaa tattcccaaa     180
tgagccaccc tcgaccaagc caacctccag ctctgaacgg gggcggggtg aaggaatgat     240
aagactacaa gtggttcaac cggcaatgcc aatgacacca agttcacgag tgctacgggc     300
agtgggagtg ggcagggcag ttcttcaggt acaaatactt ccgcggggaa tcctgatggg     360
cttcaggctg ataaagttga tcaaaacggt caggtgaaaa caagcgttca agaagccact     420
tcaggggaca acttgacgaa ttacaccaac ctccccccg ccaacctcac ccccaccgct     480
gattgaccga acgcgctgtc attcaccaac aagaacaacg cgcagcgcgc ccagctgttc     540
ctgcgcggcc tgttgggcag catcccggtg ttggttaata agtccggcca agatgataac     600
agtaagttta aggcggagga ccaaaaatgg tcctacaccg acttacagtc ggaccaaacc     660
aaactgaacc tccccgctta cggcgaggtg aatgggttgt tgaatccggc gttggtggaa     720
acctattttg ggaacacgcg agcgagtggt tcggggtcca acacgaccag ttcacccggt     780
atcggtttta aaattcccga acaaagtggc acaaacacaa cgtcgaaggc tgtgctgatc     840
acccccgggt tggcttgaac gccgcaagac gttggtaaca tcgttgtcag tggcaccagc     900
ttcagcttcc agctcggcgg gtggttagtt acgttcacgg actttatcaa accccgcgct     960
ggttacctcg ggctccagtt aacgggtctc gatgtaagtg aagcgaccca aagggagtta    1020
atttgggcca agcggccctg agcggccttt cgtggcagtt gggtcaaccg gctgggccgc    1080
gtggagagtg tgtgggattt caagggggtg tggcggatc aagctcagtt ggccgcgcaa    1140
gcagctacaa gtagtaccac caccaccgca acaggggcta ccttaccgga gcacccgaat    1200
gccctcgcgt accaaattag ctataccgac aaggattcgt acaaggcttc cactcaaggt    1260
tcgggtcaaa ccaattccca aaacaattcg ccctacctcc attttattaa acctaagaaa    1320
gtcgaaagca cgacccaact cgaccagggc ttaaaaaacc tgttggaccc caaccaggtt    1380
cgcaccaagc tgcgccaaag ctttggtaca gaccattcca cccagcccca gccccaatcg    1440
ctcaaaacaa cgacaccggt atttgggagg agtagtggta acctcagtag tgtgtttagt    1500
ggtgggggtg ctggaggggg ttcttcaggc tcaggtcaat ctggcgtgga tctctccccc    1560
gttgaacggg tgagtggtca ctaa                                           1584
```

<212> Type : DNA
<211> Length : 1584
SequenceName : SEQ ID 683
SequenceDescription :
Sequence
--------
<213> OrganismName : Mycoplasma pneumoniae
<400> PreSequenceString :

```
gtgttaaaac ttgctgttgg tatttttatt tcccccacgc tcaccaggtt tagtaccggg      60
ttcaacctcg cggggtcggt gctcgaccag gtgttggatt atgtgccctg gattgggaat     120
gggcacaggt atgggaataa ccaccggggc gtggatgata taaccgcgcc taaaaccggc     180
gcggggtcgt ccagcggaac tagtacgaac acaagtggtt cgcgttcctt tctcccgacg     240
ttctcgaatg tcggcgttgg cctcaaagcg aatgtccagg gcaccctggg tggcagtcag     300
acgacgacta cggggaaaga tattcccaaa tgacccaccc tcgacccagc caacctccag     360
ctctgaacgg gggcggggtg aaggaatgat aaggcttcaa acaaacaaag tgacgaaaac     420
cacaccacct ttaaaagcgc tacgggcagt ggccagcagg gtggttctac aacgggtggt     480
tcagcgggca atcccgactc gttaaagcag gataagatta gtaaatcagg tcagaactta     540
accacgcagg acggcgcgcc ccagtctaat tcgacgacgg aatccgcgtc gaattatgat     600
cacctccccc ccaacctcac ccccacatcc gattgaccga acgcgctgtc attcaccaac     660
aagaacaacg cgcagcgcgc ccagctcttc ctccgcggct tgttgggcag catcccggtg     720
ttggtgaatc gaagtgggtc agatgattcc aacaaattcc aagccaccga ccaaaaatgg     780
tcttacaccg acttaaagtc ggaccaaacc aagctcaacc tccccgctta tggtgaagtg     840
aatgggttgt tgaatccggc gttggtggaa acctattttg ggacgacgcg agcgggtggt     900
tcggggtcca acacgaccag ttcacccggt atcggtttta aaattcccga acaaaataat     960
gattcgaagg ctgtgctgat cacccccggg ttggcttgaa cgccgcaaga cgttggtaac    1020
ctcgttgtca gtggtaccag cttgagcttc cagttgggcg ggtggctggt caccttcacg    1080
gactttgtca aaccccgcgc gggttacctc gggctccagt taacgggctt ggatgcaagt    1140
gatgcgacgc agcgcgccct catttgggcc aagcggccct gagcggcctt tcgtggcagt    1200
tgggtcaacc gcttgggccg ggtcgagagt gtgtgggatt taaagggggt gtgacaagat    1260
caagctcagg cggccgcgca agcagctacc accgccgccg caacagggga cgccttaccg    1320
gagcacccca atgccctcgc gtaccaaatt agctccaccg acaaggattc gtacaaggct    1380
tccactcaaa gctccggtca aaccaattcc caaaacacct cccctacct ccatttgatt     1440
aaacctaaga aagtcgaaaa cacgacccaa ctcgaccagg gcttaaaaac ctgttggacc    1500
ccaaccaggt tcgcaccaag ctgcgccaaa gctttggtac agaccattcc acccaagcca    1560
```

```
aaccccaatc cctcaaaaca accacaccgg tgtttgggac gaatagtggt aacattggca    1620
gtgtgcttag tggtgggggt gctggaggag cagacagcac caattcggtg gacctctccc    1680
ccgttgaacg ggtgagtggg tggcttgtgg ggcaattacc cagtgggggt gggggaata     1740
gtagtgagga tattaaaagt gtgcaagaca ctcctattta tatctatatt cattagtata    1800
ttctttttaa attgttccct cactttattc atatgaacca ccgcctccct cgcgacggga    1860
ctcaccgtag tgggacactt cacaagtacc accacgacgc tcaagcgcca gcaatttagc    1920
tacacccgcc ctgacgaggt cgcgctgcgc cacaccaatg ccatcaaccc gcgcttaacc    1980
ccgtgaacgt atcgtaacac gagcttttcg tccctccccc tcacgggtga aaaccccggg    2040
gcgtgggcct tagtgcgcga caacaccgcc aagggcatca ctgccggcag tggcagtcaa    2100
caaaccacgt atgatcccac ccgaaccgaa gcggctttga ccaccgccac cacctttgtg    2160
ttacgccgat acgacctcgc cgggcgttgt acgacctcga cttttcgaag ttaa          2214
```

<212> Type : DNA
<211> Length : 2214
SequenceName : SEQ ID 684
SequenceDescription :
Sequence
--------
<213> OrganismName : Mycoplasma pneumoniae
<400> PreSequenceString :

```
gtgttggatt atattccctg gattgggaat gggcacagat atgggaatga ccaccggggt      60
agcaacagta gtacgagtgg ggtaacaacc cagggacaac aatcgcaaaa tgcatccgga     120
accgaacccg catccacttt ttcgaatgtc ggcgttggcc tcaaagcgaa tgtccagggc     180
accctgggtg gcagtcagac gacgactacg gggaaagata ttcctaaatg acccaccctc     240
gaccaagcca acctccagct ctgaacgggg gcggggtggc ggaatgataa ggcttcaagt     300
ggacaaagtg acgaaaacca caccaagttc acgagcgcta cgggcagtgg gcagcagggc     360
agttcttcag gtacaactaa ttccgcgggc aatcccgact cgttaaagca ggataaggtt     420
gataaaagtg gtgattcggt aacggttgca gaaaccactt caggggacaa cttgacgaat     480
tacaccaacc tccctcccaa cctcaccccc accgctgatt gaccgaacgc gctgtcattc     540
accaacaaga acaacgcgca gcgcgcccag ctcttcctcc gcgccctgtt gggcagcatc     600
ccggtgttgg tgaataagag tggccaagat gattccaaca agttccaagc caccgaccaa     660
aaatggtctt acaccgaatt aaagtcggat caaaccaagc tcaacctccc cgcttacggg     720
gaggtgaatg ggttgttgaa tccggcgttg gtggaggtgt acggtctgag ttccactcaa     780
ggttctagca ccggagccgg tggcgctgga ggtaacaccg gaggcgacac caatacccag     840
acttatgcac gacccggaat cggctttaaa ttaccctcca cggactcgga atcttccaaa     900
gccaccctga tcacccccgg gttggcttga acagcgcaag atgtcggtaa cctcgttgtc     960
agtggtacca gcttgagctt ccagctcggc gggtggttgg ttaccttcac ggattttatc    1020
aaaccccggt cgggttacct cgggctccag ttaacgggct tggatgcaaa tgacagcgac    1080
caaagggagt taatttgagc ccccccggcc ctgaaccgcc tttcgtggca gttgggtcaa    1140
ccgcttgggc cgcgtggaga gtgtgtggga tttcaagggg gtgtgggcgg atcaagctcg    1200
gtccgactcg caagcagcta caagtaccac caccgcaacg aaggctacct tatcggagca    1260
caccaatgct ttggcctttc aggtgagtta taccgaccag gattcgtaca aggcttccac    1320
tcaaagctcc ggccaaaacc aaaacacctc cccctacctg cacttggtgc aggggaaaaa    1380
agtaggttcc tctgataa                                                 1398
```

<212> Type : DNA
<211> Length : 1398
SequenceName : SEQ ID 685
SequenceDescription :
Sequence
--------
<213> OrganismName : Mycoplasma pneumoniae
<400> PreSequenceString :

```
ttgttgggca gcatcccggt gttggtgaat cgaagtgggt ccgattccaa caaattccaa      60
gccaccgacc aaaaatggtc ctacaccgac ttacagtcgg accaaaccaa actgaacctc     120
tccgcttacg gtgaggtgaa tgggttgttg aatccggctt tggtagaaac ctattttggg     180
acgacgcgta cgtctagcac tgcgaaccaa aacagtacaa ccgtccccgg tatcggtttt     240
aaaattcccg aacaaaataa tgattccaaa gccaccctga tcacccccgg gttggcttga     300
acgccccagg acgtcggtaa cctcgttgtc agtggcacca cggtgagctt ccagctcggc     360
gggtggctgg tcaccttcac ggactttgtc aaaccccgcg cgggttacct cgggctccag     420
ttaagtggcc tgaatgccag tgacagcgac caaagggagt taatttgggc cccccggccc     480
tgagcggcct ttcgtggcag ttgggtcaac cggttgggcc gcgtggagag tgtgtgggat     540
ttgaaggggg tgtgggcgga tcaagctcag ttggccgcgc aagcagctac aagtagtacc     600
accaccaccg caacaggggc taccttaccg gagcacccga atgctttggc gtaccaaatt     660
agctataccg acaaggattc gtacaaggct tccactcaag gttcgggtca aaccaattcc     720
caaaacaatt cgctctacct ccatttgatt aaacctaaga aagtcgaaag cacgacccaa     780
ctcgaccagg gcttaaaaaa cctgttggac cccaaccagg ttcgcaccaa gctgcgccaa     840
agctttggta cagaccattc cacccagccc cagccccaat cgctcaaaac aacgacaccg     900
gtgtttggag ccatgagtgg taacctcggc agtgtgctta gtggtggggg tgctggagga     960
gcaggcagca ccaattcggt ggacctctcc cccgttgaac gggtgagtgg gtcactaacc    1020
attaatagga atttttctta ttaa                                           1044
```

<212> Type : DNA
<211> Length : 1044
SequenceName : SEQ ID 686
SequenceDescription :
Sequence
--------
<213> OrganismName : Mycoplasma pneumoniae
<400> PreSequenceString :

```
atgggccagc agggacaatc aggtacctcc gcggggaatc cagactcgtt aaagcaggat      60
aagattagta agagtgggga tagtttaacc acgcaggacg gcaatgcgac cggtcaacaa     120
gaggccacta actacaccaa cctccccccc aacctcaccc ccaccgctga ttgaccgaac     180
gcgctgtcat tcaccaacaa gaacaacgcg catcgcgccc agctcttcct tcgcggcttg     240
ttgggcagca tcccggtgtt ggtgaatcga agtgggtccg attccaacaa attccaagcc     300
accgaccaaa aatggtccta caccgactta cagtcggatc aaaccaagct gaacctcccc     360
gcttacggtg aggtgaatgg gttgttgaat ccggcgttgg tggaaaccta ttttgggaac     420
acgcgagcgg gtggttcggg gtccaacacg accagttcac ccggtatcgg ttttaaaatt     480
cccgaacaaa ataatgattc caaagccacc ctgatcaccc ccgggttggc ttgaacgccc     540
caggacgtcg gtaacctcgt tgtcagtggc accagcttga gcttccagct cggcgggtgg     600
ctggtcagct tcacggactt tatcaaaccc cgcgcgggtt atttgggcct ccagttaagt     660
ggcctggatg ccagtgacag cgaccaaagg gagttaattt gggccaagcg gccctgagcg     720
gcctttcgtg gcagttgggt caaccggctg ggccgcgtgg agagtgtgtg ggatttaaag     780
ggggtgtggg cggatcaagc tcagttggcc gcgcaagcag ctacaagtga agcttccggg     840
tcagctttgg cacctcaccc gaatgctttg gcgtttcagg tgagtgtggt ggaagcgagt     900
gcttacagct cttcaacctc aagttcgggt tccgggtcaa gttcaaacac ctccccctac     960
ctccatttga ttaaacctaa gaaagtcgaa agcacgaccc aactcgacca gggcttaaaa    1020
aacctgttgg accccaacca ggttcgcacc aagctgcgcc aaagctttgg tacagaccat    1080
tccacccagc cccaatcgct caaaacaacg acaccggtat ttgggacgag tagtggtaac    1140
attggcagtg tgcttagtgg tgggggtgct ggagggggtt cttcaggctc aggtcaatct    1200
ggtgtggacc tctcccccgt tgaacgggtg agtggtcact aa                       1242
```

<212> Type : DNA
<211> Length : 1242
SequenceName : SEQ ID 687
SequenceDescription :
Sequence
--------
<213> OrganismName : Mycoplasma pneumoniae
<400> PreSequenceString :

```
ttgggcctcc agttaagtgg cctggatgcc agtgacagcg accaaaggga gttaatttgg      60
gccaagcggc cctgagcggc atttcgtggc agttgggtca accggttggg acgggtcgag     120
agtgtgtggg atttgaaggg ggtgtgggcg gatcaagctc acagcgcggt gtccgagtcg     180
caagcagcta caagtagtac caccaccacc gcaacagggg acaccttacc ggagcacccg     240
aatgccctcg cgtaccaaat tagctccacc gacaaggatt cgtacaaggc ttccactcaa     300
ggctccggtc aaaccaattc ccaaaacacc tccccctacc tgcatttgat taaacctaaa     360
aaagttactg cttccgacaa gttagacgac gatcttaaaa acctgttgga ccccaacgag     420
gttcgggtga agctgcgcca aagctttggt acagaccatt ccacccaacc ccaaccccaa     480
cccctcaaaa caacgacacc ggtgtttggg acgaatagtg gtaacctcgg tagtgtgctt     540
agtggtgggg gtaccacgca ggactcaagc accaccaatc aactgtcacc cgttcaacgg     600
gtgagtgggt ggcttgtggg gcagttacca agcacgagtg acggaaacac ctcctccacc     660
aacaacctcg cgcctaatac taatacgggg aatgaggtgg tgggggtggg agatttgtct     720
aagcgagctt ccattgaatc aagtcgtttg tgaatagcat taaaacctta g              771
```

<212> Type : DNA
<211> Length : 771
SequenceName : SEQ ID 688
SequenceDescription :
Sequence
--------
<213> OrganismName : Mycoplasma pneumoniae
<400> PreSequenceString :

```
gtgcgcgata acactgccaa gggcatcact gccggcagtg gcagtcaaca aaccacgtat      60
gatcctgcgc gaaccgaggc caccttgacc accaccacct ttgcgctgcg ccggtatgac     120
ctcgccgggc gcgcccttata cgacctcgac ttttcgaagt taaacccaca aacgccaacg    180
cgtgatgcca actgccagat cacctttaac ccctttggcg gctttggttt gagtggcagt     240
gcaccccaac agtgaaacga ggtcaaaaac aaggtccccg tcgaggtggc ccaagacccc     300
accgatcctt atcggtttgc cgttttactc gtgccgcgca gtgtggtgta ctatgagcag     360
ttgcaaaggg gattagcgct ccctaaccaa gggagttcgt caggctccgg tcaacaaaac     420
accaccattg gcgcgtatgg gctgaaggtg aagaacgccg aggcggacac cgcaaagagc     480
aatgaaaaac tccagggcga tgaatccaag tcttccaatg gatcttcaag cacttccacc     540
accacccaac gtggttcgac caattccgac accaaagtca aggctttaaa aatagaggtg     600
aaaaagaaat cggactcgga ggacaatggt cagctgcagt tagaaaaaaa tgatctcgcc     660
aacgctccca ttaagcgggg cgaggagtcg ggtcagtccg tccaactcaa ggcggacgat     720
tttggtactg ccccttccag ttcgggatca ggcggcaact ccaaccccgg ttcccccacc     780
ccctgaaggc cgtggcttgc gactgagcaa attcacaagg acctccccaa atgatccgcc     840
tcgatcctga ttctgtacga tgcgccttat gcgcgcaatc gtaccgccat tgatcgcgtt     900
gatcacttgg atcccaaggt gatgaccgcg aactatccgc ccagttgaag aatgcccaag     960
tgaaaccacc acgggttgtg ggactgaaag gcgcgcgatg ttttgttcca aaccaccggg    1020
tttgatgaaa gtaatacttc gaacaccaag cagggctttc aaaaggaagc tgactccgac    1080
aagtcggccc ccatcgccct cccgtttgaa gcgtacttcg ccaacattgg caacctcacc    1140
tggttcgggc aagcgctttt ggtgtttggt ggcaatggcc atgttaccaa gtcggcccac    1200
accgcgcctt tgagtatttg gctttatata tatttagtaa aggcagtcac ttttaggttg    1260
cttttagcta actctttatt atcaaaaagc aacatatata aaaaaacagc taattaa       1317
```

<212> Type : DNA
<211> Length : 1317
SequenceName : SEQ ID 689
SequenceDescription :
Sequence
--------
<213> OrganismName : Mycoplasma pneumoniae
<400> PreSequenceString :

```
gtgcgcgata acattgccaa gggcatcact gccggtagta acacccaaca aaccacgtat      60
gatcccaccc gaaccgaggc caccttgacc accgccacca cctttgcgtt acgccggtat     120
gacctcgccg ggcgcgcctt atacgacctc gatttttcga agttaaaccc acaaacgccc     180
acgcgcgacc aaaccggtca gatcaccttt aaccccttttg gcggctttgg gctgagtggg    240
gctgcacccc aacagtgaaa cgaggtcaag gataaggtcc ccgtcgaggt ggcccaagac     300
ccctccaatc cttatcggtt tgccgtttta ctcgtgccgc gtagcgtggt gtactatgag     360
cagttgcagc gggggttagc gctccctaac caagggagtt cgtcaggctc cggtcaacaa     420
aacaccacca ttggcgcgta tgggctgaag gtgaaaaacg ccgaggcgga caccgcgaag     480
agcaatgaaa aactccaggg ctatgaatcc aagtcttcca atggatcttc aagcacttcc     540
accacccaac gtgggggttc gtcaaatgaa aacaaagtca aggcgttgca ggtggcggtg     600
aaaaagaaat ccgggagtca gggcaactcc ggtgaccaag gcaccgaaca ggtggaactt     660
gaatctaatg atttagccaa cgcccccgatt aaacgaggct ccaataacaa ccagcaagtc    720
caactcaagg cggacgattt tggtactgcc ccttccagtt cgggatcagg cacccaagat     780
ggcaccccca ccccctgaac gccgtggtta acgactgagc aaattcacaa cgaccccgcc     840
aaattcgccg cctcgatcct cattctgtac gatgcgcctt atgcacgcaa ccgtaccgcc     900
attgaccgcg ttgatcactt ggatcccaag gtgatgaccg cgaactatcc gcccagttga     960
agaacgccca agtgaaacca ccacggtttg tgggactgaa aggcgcgcga tgttttgctc    1020
caaaccaccg ggttcttcaa cccgcgccgc caccccgagt ggtttgatgg cgggcagacg    1080
gtcgcggata acgaaaagac cgggtttgat gtggataact ccgaaaacac caagcagggc    1140
tttcaaaagg aagctgactc cgacaagtcg gccccgatcg ccctcccgtt tgaagcgtac    1200
ttcgccaaca ttggcaacct cacctggttc gagcaagcgc ttttggtgtt tgggatttgt    1260
ttgtcttaa                                                            1269
```

<212> Type : DNA
<211> Length : 1269
SequenceName : SEQ ID 690
SequenceDescription :
Sequence
--------
<213> OrganismName : Mycoplasma pneumoniae
<400> PreSequenceString :

```
atgctttggc ctttcaggtg agtgtggtgg aagcgagtgc ttacaagcca aacacgagct      60
ccggccaaac ccaatccact aacagttccc cctacctgca cttggtgaag cctaagaaag     120
ttacccaatc cgacaaagtt agacgacgat cttaaaaacc tgttggaccc caacgaggtt     180
cgcgccagaa tgctcaaatc atttggtaca gaaaatttca cccaacccca accccaaccc     240

caagccctca aaacaacgac accggtattt gggacgagta gtggtaacct cggtagtgtg     300
cttagtggtg gggggtacca cgcagggctc aagcaccacc aatcaactgt cacccgttca     360
acgggtgagt gggtggaccg ctaa                                            384
```

<212> Type : DNA
<211> Length : 384
SequenceName : SEQ ID 691
SequenceDescription :
Sequence
--------
<213> OrganismName : Mycoplasma pneumoniae
<400> PreSequenceString :

```
gtgcgcgaca acagcgccaa gggcatcact gccggtagtg aaagtcaaca aaccacgtat     60
gatcccactc gaaccgaagc ggctttgacc gcatcaacca cctttgcgtt acgccggtat    120
gacctcgccg ggcgcgcctt atacgacctc gactttagta ggttaaaccc acagacacca    180
acgcgcgacc aaaccgggca gatcaccttt aacccctttg gcggctttgg tttgagtggg    240
gctgcacccc aacagtgaaa cgaggtcaaa aacaaggtcc ccgtcgaggt ggcccaagac    300
ccctccaatc cttatcggtt tgccgtttta ctcgtgccgc gtagcgtggt gtactatgag    360
cagttgcagc gggggttagc gctccctaac caagggagtt cgtcaggctc cggtcaacaa    420
aacaccacca ttggcgcgta tgggctgaag gtgaagaacg ccgaggcgga caccgcgaag    480
agcaatgaaa aactccaggg cgatgaatcc aagtcttcca atggatcttc aagcacttcc    540
accaccaccc aacgtggggg ttcgtcaggg gacaccaaag tcaaggcgtt gcaggtggcg    600
gtgaaaaaga aatccgggag tcagggcaac tccggtgaac aaggcaccga acaggtggaa    660
cttgaatcta atgatctcgc caacgctccc attaagcggg gcgaggagtc gggtcagtcc    720
gtccaactaa aggcagccga cttcggcacc accccatcca gttcgggatc aggcggcaac    780
tccaaccccg gttcccccac cccctgaagg ccgtggcttg cgaccgagca aattcacaag    840
gacctcccca aatgatccgc ctcgatcctg attctgtacg atgcgcctta tgcgcgcaat    900
cgtaccgcca ttgatcgcgt tgatcacttg gatcccaagg tgatgaccgc gaactatccg    960
cccagttgaa gaacgcccaa gtgaaaccac cacgggttgt gggactgaaa ggccagagat   1020
gttttgctcc aaaccaccgg gttcttcaac tcgcggcgcc accccgagtg gtttgaccag   1080
ggccaagcgg tcgcggataa tacccaaacc gggtttgata cagatgacac cgataataaa   1140
aaaacaaggc tttcaaaagg aagctgactc cgacaagccg gccccgatcg ccctcccgtt   1200
tgaagcgtac ttcgccaaca ttggcaacct cacctggttc gggcaagcgc ttttggtgtt   1260
tgggatttgt ttgtcttaat taactaa                                        1287
```

<212> Type : DNA
<211> Length : 1287
SequenceName : SEQ ID 692
SequenceDescription :
Sequence
--------
<213> OrganismName : Mycoplasma pneumoniae
<400> PreSequenceString :

```
atgtttggct tgaaggtgaa gaacgccgag gcggacaccg cgaagagcaa tgaaaaactc     60
cagggcgctg aggccactgg ttcttcaacc acatctggat ctggccaatc cacccaacgt    120
gggggttcgt caggggacac caaagtcaag gcgttgcagg tggcggtgaa aaagaaatcc    180
gggagtcagg gcaactccgg tgaccaaggc accgaacagg tggaacttga atctaatgat    240
ttagccaacg ccccgattaa acggggctcc aatccagcaa gtccaactca aggcagccga    300
cttcggcacc accccatcca gttcggaatc tggtcaatca ggcaccccca ccccctgaag    360
gccgtggctt gcgaccgagc aaattcacaa ggacctcccc aaatgatccg cctcgatcct    420
cattctgtac gatgcgcctt atgcctttaa                                     450
```

<212> Type : DNA
<211> Length : 450
SequenceName : SEQ ID 693
SequenceDescription :
Sequence
--------
<213> OrganismName : Mycoplasma pneumoniae
<400> PreSequenceString :

```
atgtttggct tgaaggtgaa ggatgcaacc gtggatagtt cgaagcaatc aacggaaagc     60
ttaaagggcg aagaatcgag ttccagttcc accacatctt ccacctccac cacccaacgt    120
gggggttcgt caggggacac caaagtcaag gcgttgcagg tggcggtgaa aaagaaatcg    180
gactcggagg acaatggtca gatcgaactt gaaaccaaca acctcgccaa cgcccccgatt   240
aaacggggct ccaataacaa ccagcaagtc caactcaagg cggacgattt tggtacttcc    300
ccttccagtt cggaatctgg tcaatcaggc acccccaccc cctgaacgcc gtggcttgcg    360
accgagcaaa ttcacaagga cctccccaaa tgatccgcct cgatcctgat tctgtacgat    420
```

```
gcgccttatg cgcgcaatcg taccgccatt gatcgcgttg atcacttgga tcccaaggtg     480
atgaccgcga actatccgcc cagttgaaga acgcccaagt gaaaccacca cgggttgtgg     540
gactgaaagg cgcgcgatgt cctggtccaa accaccgggt tcttcaaccc gcgccgccac     600
cccgattggt ttgaccaggg ccaagcggtc gcggagaata cccaaaccgg gtttgataca     660
gatgacaccg ataataaaaa gcagggcttt cgcaaacaag gcgaacaatc ccctgccccc     720
atcgccctcc cgtttgaagc gtacttcgcc aacattggca acctcacctg gttcgggcaa     780
gcgcttttgg tgtttgggat ttgtttgtct taa                                  813
```

<212> Type : DNA
<211> Length : 813
SequenceName : SEQ ID 694
SequenceDescription :
Sequence
--------
<213> OrganismName : Mycoplasma pneumoniae
<400> PreSequenceString :

```
atgggcagtc aaaaccaggg ttctacaaca actacctcag cgggcaatcc cgattcttta      60
gttactgata aagttgatca aaaaggtcag gtgcaaacaa gtggtcaaaa tttaagtgat     120
accaactaca ccaacctctc ccccaacttc acccccacat ccgactgacc gaacgcgctc     180
agcttcacca acaagaacaa cgcgcagcgc gcccagctgt tcctgcacgg cttgttgggc     240
agcatcccgg tgttggttaa taagagtggc gaaaataacg aaaaattcca agccaccgac     300
caaaaatggt cctacaccga attaaagtcg gatcaaacca agctcaacct ccccgcttat     360
ggtgaggtga atgggctgtt gaatccggcg ttggtggaaa cctattttgg gacgacgcgt     420
acgtctagca ctgcgaacca aaacagtaca accgtccccg gtatcggttt taaaattccc     480
gaacaaaata atgattcaaa ggctgtgctg atcacccccg ggttggcttg aacgccccag     540
gacgttggta acctcgttgt cagtggcacc agcttcagct tccagctcgg cgggtggctg     600
gtcagcttca cggactttgt caaaccccgc gcgggttacc tcggactcca gttaacgggc     660
ttggatgcaa gtgatgcgac gcagcgcgcc ctcatttggg cccccccggc cctgagcggc     720
ctttcgtggc agttgggtca accggttggg ccgcgtggag agtgtgtggg atttgaaggg     780
ggtgtgggcg gatcaagctc agtccgactc gcaaggatct accaccaccg caacaggggc     840
taccttaccg gagcacccga atgctttggc ctttcaggtg agtgtggtgg aagcgagtgc     900
ttacaagcca aacacgagct ccggccaaac ccaatccact aa                         942
```

<212> Type : DNA
<211> Length : 942
SequenceName : SEQ ID 695
SequenceDescription :
Sequence
--------
<213> OrganismName : Mycoplasma pneumoniae
<400> PreSequenceString :

```
atgagttttg gtttagtggg cactgttaat aataacggtt ggaaatcgcc gtttcgacat      60
gaaacaaaat accgagcagg gtatgataag ttcaagtatt acaaaaccca ctaccggggt     120
gcaaaaaaag ctggaaccaa tgatgatcga tggagatgaa ctgcttggtt tgaccttgac     180
tttgcccacc aaaagatagt gctcattgaa aggggtgaac ttcaccgtca agcagattta     240
aaaaaatctg accccgcaac aaacgaaact tccaaaaccg tttggggtag cattaaagaa     300
aagctgttac aaaacgtcaa caacctccac tcagagaaag gagtattctt atggttccgt     360
caatctggtt ttacaactac tagaaactag                                      390
```

<212> Type : DNA
<211> Length : 390
SequenceName : SEQ ID 696
SequenceDescription :
Sequence
--------
<213> OrganismName : Mycobacterium tuberculosis H37Rv
<400> PreSequenceString :

```
atggctgaac cgttggccgt cgatcccacc ggcttgagcg cagcggccgc gaaattggcc      60
ggcctcgttt ttccgcagcc tccggcgccg atcgcggtca gcggaacgga ttcggtggta     120
gcagcaatca acgagaccat gccaagcatc gaatcgctgg tcagtgacgg gctgcccggc     180
gtgaaagccg ccctgactcg aacagcatcc aacatgaacg cggcggcgga cgtctatgcg     240
aagaccgatc agtcactggg aaccagtttg agccagtatg cattcggctc gtcgggcgaa     300
ggcctggctg gcgtcgcctc ggtcggtggt cagccaagtc aggctaccca gctgctgagc     360
acacccgtgt cacaggtcac gacccagctc ggcgagacgg ccgctgagct ggcaccccgt     420
gttgttgcga cggtgccgca actcgttcag ctggctccgc acgccgttca gatgtcgcaa     480
aacgcatccc ccatcgctca gacgatcagt caaaccgccc aacaggccgc ccagagcgcg     540
cagggcggca gcggcccaat gcccgcacag cttgccagcg ctgaaaaacc ggccaccgag     600

caagcggagc cggtccacga agtgacaaac gacgatcagg gcgaccaggg cgacgtgcag     660
ccggccgagg tcgttgccgc ggcacgtgac gaaggcgccg gcgcatcacc gggccagcag     720
cccggcgggg gcgttcccgc gcaagccatg gataccggag ccggtgcccg cccagcggcg     780
agtccgctgg cggcccccgt cgatccgtcg actccggcac cctcaacaac cacaacgttg     840
tag                                                                   843
```

<212> Type : DNA
<211> Length : 843
SequenceName : SEQ ID 697
SequenceDescription :
Sequence
--------
<213> OrganismName : Mycobacterium tuberculosis H37Rv
<400> PreSequenceString :

```
atgcgttatc tgatagcgac cgcagtgctc gttgctgtgg tcctggtggg ctggccggcg      60
gctggtgcgc cgccgtcatg cgccggcctg ggcggcactg tgcaggccgg ccagatctgc     120
catgtgcacg cctcgggccc taagtacatg ctggatatga catttcctgt cgactatccc     180
gaccagcagg cgctgaccga ctacatcacg caaaaccgcg acgggttcgt caacgtcgcg     240
caggggtccc cgctgcgaga ccagccctac caaatggacg ccaccagcga acagcacagc     300
tccggccagc cgccgcaggc cacccgcagc gtagtgctca aattcttcca ggacctcggt     360
ggggcacatc cgtccacctg gtacaaggcc ttcaactaca acctcgcgac ctcgcagccc     420
atcaccttcg acacgttgtt cgtgcccggc accacgccac tggacagcat ctaccccatc     480
gttcagcgcg agctggcacg tcagaccggt ttcggtgccg cgatattgcc ttcgaccggc     540
ctcgacccgg ctcactacca gaactttgct atcaccgacg acagtctgat tttctacttc     600
gcccagggtg agctgctgcc gtcgtttgtc ggcgcttgcc aagcccaggt gccgcgcagc     660
gccattccgc cgctggcaat ctaa                                            684
```

<212> Type : DNA
<211> Length : 684
SequenceName : SEQ ID 698
SequenceDescription :
Sequence
--------
<213> OrganismName : Mycobacterium tuberculosis H37Rv
<400> PreSequenceString :

```
atgaagatgg tgaaatcgat cgccgcaggt ctgaccgccg cggctgcaat cggcgccgct      60
gcggccggtg tgacttcgat catggctggc ggcccggtcg tataccagat gcagccggtc     120
gtcttcggcg cgccactgcc gttggacccg gcatccgccc ctgacgtccc gaccgccgcc     180
cagttgacca gcctgctcaa cagcctcgcc gatcccaacg tgtcgtttgc gaacaagggc     240
agtctggtcg agggcggcat cgggggcacc gaggcgcgca tcgccgacca caagctgaag     300
aaggccgccg agcacgggga tctgccgctg tcgttcagcg tgacgaacat ccagccggcg     360
gccgccggtt cggccaccgc cgacgtttcc gtctcgggtc cgaagctctc gtcgccggtc     420
acgcagaacg tcacgttcgt gaatcaaggc ggctggatgc tgtcacgcgc atcggcgatg     480
gagttgctgc aggccgcagg gaactga                                         507
```

<212> Type : DNA
<211> Length : 507
SequenceName : SEQ ID 699
SequenceDescription :

Sequence

--------

<213> OrganismName : Mycobacterium tuberculosis H37Rv

<400> PreSequenceString :

```
atgacctact cgccgggtaa ccccggatac ccgcaagcgc agcccgcagg cttcctacgga   60
ggcgtcacac cctcgttcgc ccacgccgat gagggtgcga gcaagctacc gattgtacctg   120
aacatcgcgg tggcagtgct cggcctggct gcgtacttcg ccagcttcgg ccccaatgttc   180
accctcagta ccgaactcgg cggaggtgat ggcgcagtgt ccggtgacac tgggctgccg   240
gtcggggtgg ctctgctggc tgcgctgctt gccggggtgg ctctggtgcc taaaggccaag   300
agccatgtga cggtagttgc ggtgctcggg gtactcggcg tatttctgat gggtctcggcg   360
acgtttaaca agcccagcgc ctattcgacc ggttgggcat tgtgggttgt gtttggctttc   420
atcgtgttcc aggcggttgc ggcagtcctg gcgctcttgg tggagaccgg cggctatcacc   480
gcgccggcgc cgcggcccaa gttcgacccg tatggacagt acgggcggta cgggcagtac   540
gggcagtacg gggtgcagcc gggtgggtac tacggtcagc agggtgctca gccaggccgcg   600
ggactgcagt cgcccggccc gcagcagtct ccgcagcctc ccggatatgg gttcgcagtac   660
ggcggctatt cgtccagtcc gagccaatcg ggcagtggat acactgctca gccccccggcc   720
cagccgccgg cgcagtccgg gtcgcaacaa tcgcaccagg gcccatccac gcccacctacc   780
ggctttccga gcttcagccc gccgccaccg gtcagtgccg ggacggggtc gccaggctggt   840

tcggctccag tcaactattc aaaccccagc gggggcgagc agtcgtcgtc ccccgggggg   900
gcgccggtct aa                                                        912
```

<212> Type : DNA

<211> Length : 912

SequenceName : SEQ ID 700

SequenceDescription :

Sequence

--------

<213> OrganismName : Mycobacterium tuberculosis H37Rv

<400> PreSequenceString :

```
atgaaatgtc caggcgtctc cgactgcgtt gcgaccgtaa ggcacgataa cgtgtttgct     60
attgctgctg gtttgcgttg gtcggccgct gtaccgccgc tacacaaagg ggacgctgtg    120
accaaactgc tcgtcggggc catcgcgggc ggaatgctag cttgcgcagc tatattgggc    180
gacggaatcg cttcggccga tactgcgttg atagtacccg gtaccgcacc gtccccgtac    240
gggccactca ggtcgctcta tcatttcaat cccgcgatgc agcctcagat cggcgcgaat    300
tactacaacc ccaccgctac ccgccacgtc gtttcatatc caggcagctt ttggcctgtc    360
acaggcttga attcgcccac cgtcggcagt tctgtcagtg ccgggacgaa caatctcgat    420
gcggcgatcc gcagcactga cggaccaatc ttcgtggccg ggttatcaca gggcacgctc    480
gtgcttgacc gcgagcaggc acggttagcg aatgacccga cggctcctcc ccctgggcaa    540
ctcacattca tcaaggccgg cgaccctaac aatcttcttt ggcgggcgtt taggccggga    600
acccacgtgc cgatcatcga ctacaccgtt ccggccccag cggaaagcca gtacgacaca    660
atcaatatcg tgggccagta cgacattttt tctgacccgc ctaatcgtcc gggcaaccta    720
ctcgctgacc tcaatgcgat tgccgcgggc ggatactacg gccacagcgc caccgcattc    780
tcggacccag ctcgcgttgc gcctagggac attacgacga caacgaacag tttgggtgcg    840
acgaccacga cctacttcat ccggaccgat cagctacctc tggtgcgggc gctggtggac    900
atggcgggcc tgcccccgca ggcggcggga acagttgatg ccgcactgcg gcccataatt    960
gacagggctt atcagcccgg accagcaccc gctgtgaacc cgcgtgattt ggtccagggc   1020
atccgcggta tccccgccat cgcccctgcc atcgccatcc ctatcggcag caccaccggg   1080
gccagtgccg ccaccagcac cgctgccgcc acggcagcag caacaaatgc gctccgcggg   1140
gccaacgtgg gcccgggcgc caacaaggcg ttgtcgatgg tccggggttt gctacccaaa   1200
gggaagaagc actag                                                    1215
```

<212> Type : DNA

<211> Length : 1215

SequenceName : SEQ ID 701

SequenceDescription :

Sequence

--------

<213> OrganismName : Mycobacterium tuberculosis H37Rv

<400> PreSequenceString :

```
gtgagcctgc taccaaccct gcagtccttc ctaccgccgc ccttcgacgc cattccgaac      60
cccatcgagg atcttgacgt tctcgtagcc gcagctgtag ccgtcgcggc tggcagtttg     120
ggggtatcgg cggcgcagct cggcgagatc taccgccacg acgtggtcga cgaggcccaa     180
aaggcgccgc attgcccagc cgaatccgac cagacacccg ctggggccgc gggtgacggg     240
gatctccctg aggtcggagg acgggtcacc agcccgccac agccgccggt cgccgcgctc     300
accggctact ccgctaacat cggcggactc tccgtgccgc acagctggaa tcttccgcca     360
gcggtgcgcc aagttgcggc gatgttcccc ggcgcgactc cgatgtatat gacggggagt     420
tcggacggct cctacgccgg cctggcagcg gcgggtttgg ccggcaccgg tctggccggt     480
cttgccgccc gcggtggctc cgcgccgacc ccggctgcag ccgcccccggc cggagccggc     540
ggagccggcc cggctgccac caggcccgcc gcccagcaga cgcccgcggt ccccgcggcg     600
gccgccgggt cagccatacc tggcctaccg cccggtttgc cgcccggcgt ggttgccaac     660
cttgcggcga ccctggcggc gatccccgga gcgaccatca tcgtggtacc gccgtccccg     720
aacgccaatc aatag                                                      735
```

<212> Type : DNA
<211> Length : 735
SequenceName : SEQ ID 702
SequenceDescription :
Sequence
--------
<213> OrganismName : Mycobacterium tuberculosis H37Rv
<400> PreSequenceString :

```
atggacgtcg ctttgggggt tgcggtcacg gatcgggtcg cgcgtctggc gctggtcgac      60
tcggctgcgc ccggcaccgt gatcgaccag ttcgtgctcg atgtggccga gcacccggtc     120
gaggtgttaa ccgagaccgt ggtgggcacg gatcggtcat tggccggcga aaaccaccgg     180
ctggtcgcta cccggctgtg ttggccggat caggccaaag ctgacgagct gcagcacgca     240

ctgcaggact ccggggtcca cgacgttgcc gtgatatccg aggcgcaggc cgccacggcg     300
ctggtcgggg cggcacatgc cggctctgcc gtgctgttgg tgggtgatga gacggcaacc     360
ttatcggtgg ttggtgaccc ggacgcgccg ccgacgatgg tggccgtcgc gccggtggcg     420
ggcgccgacg ccacatcgac cgtcgatacc ctgatggccc ggctcggcga ccaggccctc     480
gccccggggg atgtcttcct ggtgggtagg tccgccgagc acaccacggt tcttgccgac     540
cagctgcgcg cggcgtcgac gatgcgcgtg cagactcccg acgaccccac gttcgcgctg     600
gcccgtggcg cggcgatggc ggccggcgcc gctacgatgg cgcacccggc cctggtcgcg     660
gatgcgacca cttcgctccc ccgggccgag gcggggcaat cgggttctga aggcgagcag     720
ctggcgtact cgcaggccag cgattacgag ctgcttccgg tcgacgaata tgaggaacac     780
gacgaatacg gggcagccgc ggatcgctcg gcgccgttga gccgacggtc gctgctgatc     840
ggcaacgctg tcgtggcctt tgcggtgatc ggtttcgcct cgctggcggt ggcggtggcg     900
gtcaccatcc gaccgaccgc ggcctcaaaa ccggtagagg gacaccaaaa cgcccagcca     960
gggaagttca tgccgttgtt gccgacgcaa cagcaggcgc cggtcccgcc gcctccgccc    1020
gatgatccca ccgctggatt ccagggcggc accattccgg ctgtacagaa cgtggtgccg    1080
cggccgggta cctcacccgg ggtgggtggg acgccggctt cgcctgcgcc ggaagcgccg    1140
gccgtgcccg gtgttgtgcc tgccccggtg ccaatcccgg tcccgatcat cattcccccg    1200
ttcccgggtt ggcagcctgg aatgccgacc atccccaccg caccgccgac gacgccggtg    1260
accacgtcgg cgacgacgcc gccgaccacg ccgccgacca cgccggtgac cacgccgcca    1320
acgacgccgc cgaccacgcc ggtgaccacg ccgccaacga cgccgccgac cacgccggtg    1380
accacgccac caacgaccgt cgccccgacg accgtcgccc cgacgacggt cgctccgacc    1440
accgtcgccc cgaccacggt cgctccagcc accgccacgc cgacgaccgt cgctccgcag    1500
ccgacgcagc agcccacgca acaaccaacc caacagatgc caacccagca gcagaccgtg    1560
gccccgcaga cggtggcgcc ggctccgcag ccgccgtccg gtggccgcaa cggcagcggc    1620
gggggcgact tattcggcgg gttctga                                        1647
```

<212> Type : DNA
<211> Length : 1647
SequenceName : SEQ ID 703
SequenceDescription :
Sequence
--------
<213> OrganismName : Mycobacterium tuberculosis H37Rv
<400> PreSequenceString :

ttgaagaacg cccgtacgac gctcatcgcc gccgcgattg ccgggacgtt ggtgaccacg 60
tcaccagccg gtatcgccaa tgccgacgac gcgggcttgg acccaaacgc cgcagccggc 120
ccggatgccg tgggctttga cccgaacctg ccgccggccc cggacgctgc acccgtcgat 180
actccgccgg ctccggagga cgcgggcttt gatcccaacc tccccccgcc gctggccccg 240
gacttcctgt ccccgcctgc ggaggaagcg cctcccgtgc ccgtggccta cagcgtgaac 300
tgggacgcga tcgcgcagtg cgagtccggt ggaaactggt cgatcaacac cggtaacggt 360
tactacggcg gcctgcggtt caccgccggc acctggcgtg ccaacggtgg ctcggggtcc 420
gcggccaacg cgagccggga ggagcagatc cgggtggctg agaacgtgct gcgttcgcag 480
ggtatccgcg cctggccggt ctgcggccgc cgcggctga 519

<212> Type : DNA
<211> Length : 519
SequenceName : SEQ ID 704
SequenceDescription :
Sequence
--------
<213> OrganismName : Mycobacterium tuberculosis H37Rv
<400> PreSequenceString :

atgacgcggc tgataccggg ttgcacgctc gtcgggctga tgctgacgtt actgcccgcg 60
cccacctcgg cggccgggag caacaccgcc accaccctgt tcccggtcga cgaggtcacc 120
cagctggaga cgcacacctt cctcgattgc caccccaacg gcagctgcga cttcgtcgct 180
ggagcaaatc tgcgcacacc cgacggcccg acgggctttc cgcccgggct gtgggcgcgc 240
caaaccaccg agatccgttc gacgaaccgg ttggcctatc tggacgcgca cgccaccagc 300
cagttcgaac gggtaatgaa ggcgggcgga tccgacgtga tcaccaccgt ctacttcggc 360
gagggtccgc cggacaaata ccagaccacc ggggtcatcg actcgaccaa ttggtcgacc 420
ggtcaaccga tgaccgacgt caacgtcatc gtgtgtacac acatgcaggt ggtctacccg 480
ggggtcaacc tcacctcgcc cagcacctgc gcgcaagcca acttttccta g 531

<212> Type : DNA
<211> Length : 531
SequenceName : SEQ ID 705
SequenceDescription :
Sequence

--------
<213> OrganismName : Mycobacterium tuberculosis H37Rv
<400> PreSequenceString :
atgacaccgg gtttgcttac tactgcgggt gctggccgac cacgtgacag gtgcgccagg 60
atcgtatgca cggtgttcat cgaaaccgcc gttgtcgcga ccatgtttgt cgcgttgttg 120
ggtctgtcca ccatcagctc gaaagccgac gacatcgatt gggacgccat cgcgcaatgc 180
gaatccggcg gcaattgggc ggccaacacc ggtaacgggt tatacggtgg tctgcagatc 240
agccaggcga cgtgggattc caacggtggt gtcgggtcgc cggcggccgc gagtccccag 300
caacagatcg aggtcgcaga caacattatg aaaacccaag gcccgggtgc gtggccgaaa 360
tgtagttctt gtagtcaggg agacgcaccg ctgggctcgc tcacccacat cctgacgttc 420
ctcgcggccg agactggagg ttgttcgggg agcagggacg attga 465

<212> Type : DNA
<211> Length : 465
SequenceName : SEQ ID 706
SequenceDescription :
Sequence
--------
<213> OrganismName : Mycobacterium tuberculosis H37Rv
<400> PreSequenceString :

```
atgatgcaac aagcggtgtc gggcattacc ggcgcgctcg gcggcgcggt cggcggcgtc      60
atgggcccac tcacgcagct tccccagcag gccatgcaag cggggcaggg agcaatgcag     120
ccgctgatga gtgcgcttca acagacctat ggcgcggagg gactggacgt cgcggacggg     180
gcgcggctgg tggacagcat cgaaggtgag cccggcctcg gcggcgagcc gggcgctggt     240
gacgtcggcg ccggcggcgg gggtggtggc accaccccga cgggctatct gggtccccca     300
cccgtgccga cgtcgtcgcc accgacgact ccagccgggg cgccggccaa gtcggtgacg     360
ccggacccgg ttagtggcac cccgcgggcg tcggggccgg ccggcatgac cggcatgccg     420
atggtgccgc cgggcgcgtt gggtgcgggc gcggaaggag ccaataagga caagccggtc     480
gagaagcggg tgacgggctg tgccgaatgg tcaaccggtc aagggccgct taacagtacc     540
gccgagtgtt ccggtgaaat ctgccgacga caagccggtg gtcaccaagt cgacgcgacg     600
gatccttgtt gtgccgaacg acgacaaggt taa                                  633
```

<212> Type : DNA
<211> Length : 633
SequenceName : SEQ ID 707
SequenceDescription :
Sequence
--------
<213> OrganismName : Mycobacterium tuberculosis H37Rv
<400> PreSequenceString :

```
atgattcgcg aactggtcac caccgctgcg atcacgggtg ccgcgatcgg tggggcgcca      60
gtcgcgggcg cagacccgca gcgttatgac ggcgatgtgc cggggatgaa ctatgacgct     120
tcgctgggcg ccccatgctc cagctgggag cgcttcattt ttggacgagg cccctccggt     180
caggccgaag cctgtcattt tccgcctcct aaccagttcc cgccggccga aaccggctac     240
tgggtgatct cctacccgct atacggcgtc cagcaggtcg gtgcgccgtg tccgaagccg     300
caggcggccg cgcagtctcc ggatgggttg ccgatgctgt gtctgggagc ccgtggatgg     360
cagccgggat ggtttaccgg ggccgggttc ttccctccgg agccataa                  408
```

<212> Type : DNA
<211> Length : 408
SequenceName : SEQ ID 708
SequenceDescription :
Sequence
--------
<213> OrganismName : Mycobacterium tuberculosis H37Rv
<400> PreSequenceString :

```
atgaaaacca caggcacaac tatcaaactc ggcatcgtct ggttggtgct gtcggtgttc      60
accgtgatga tcatcgtggt gttcgggcag gtgcggttcc atcacaccac cgggtactcc     120
gcggtgttca cccatgtcag cgggctgcgg gccgggcaat ttgtccgcgc tgcgggcgta     180
gaggtcggca aggtcgccaa ggtaacgctg atcgacgggg acaagcaagt attggtggac     240
ttcaccgtgg atcgctcgct gtcactggat caggcgacga ccgcctcgat ccgctacctc     300
aacctgatcg gcgaccggta ccttgagctc ggccgcggtc acagcggtca gcggctggcg     360
ccgggtgcca cgatcccgct cgagcacacc catccggcct tggatctcga cgctctgctc     420
ggcgggtttc gcccactctt ccaaacgttg gacccagaca aggtcaacag catcgcctcc     480
tcgatcatca ccgtgttcca agggcaaggc gccaccatca acgacatcct cgaccagacc     540
gcctcgctga cggcaacgct ggccgaccgg gaccatgcga taggtgaggt cgtcaacaac     600
ttgaacaccg tgctggccac caccgtcaag catcaaacgg aattcgaccg cacggtcgac     660

aagctagagg tgctgatcac tggactgaag aacagggcgg acccgctggc cgcggcggcg     720
gcacacatca gcagcgccgc gggaacccta gccgacctgc tggggcggat cgtccattgc     780
tgcacagcag cttcgggcac ctcgagggca tccagcagcc gctcatag                  828
```

<212> Type : DNA
<211> Length : 828
SequenceName : SEQ ID 709
SequenceDescription :
Sequence
--------
<213> OrganismName : Mycobacterium tuberculosis H37Rv

<400> PreSequenceString :

```
atgactccac gcagccttgt tcgcatcgtt ggtgtcgtgg ttgcgacgac cttggcgctg    60
gtgagcgcac ccgccggcgg tcgtgccgcg catgcggatc cgtgttcgga catcgcggtc   120
gttttcgctc gcggcacgca tcaggcttct ggtcttggcg acgtcggtga ggcgttcgtc   180
gactcgctta cctcgcaagt tggcgggcgg tcgattgggg tctacgcggt gaactaccca   240
gcaagcgacg actaccgcgc gagcgcgtca aacggttccg atgatgcgag cgcccacatc   300
cagcgcaccg tcgccagctg cccgaacacc aggattgtgc ttggtggcta ttcgcagggt   360
gcgacggtca tcgatttgtc cacctcggcg atgccgcccg cggtggcaga tcatgtcgcc   420
gctgtcgccc ttttcggcga gccatccagt ggtttctcca gcatgttgtg gggcggcggg   480
tcgttgccga caatcggtcc gctgtatagc tctaagacca taaacttgtg tgctcccgac   540
gatccaatat gcaccggagg cggcaatatt atggcgcatg tttcgtatgt tcagtcgggg   600
atgacaagcc aggcggcgac attcgcggcg aacaggctcg atcacgccgg atga         654
```

<212> Type : DNA
<211> Length : 654
SequenceName : SEQ ID 710
SequenceDescription :
Sequence
--------
<213> OrganismName : Mycobacterium tuberculosis H37Rv
<400> PreSequenceString :

```
gtgataagca ccacaagaat tgatttccta tggatattgt cggtagcgtt cgcgtccatg    60
attgctcttg caacgctgtt gacgcttatc aatcaagtcg tcggcactcc gtatattccc   120
ggtggcgatt ctcccgccgg gaccgactgc tcggagctgg cttcgtgggt atcgaatgcg   180
gcgacggcca ggccggtttt cggagatagg ttcaacaccg gcaacgagga agccgccttg   240
gcggctcggg gctttcaaca gggaaccgcc cccaatgcct tggtgatcgg ttggaatggc   300
caccacacgg cggtgacgct gcccgatggc acgcccgtat ccagtggtga aggcggtggc   360
gtgcgggtcg gtggcggtgg cgcctaccag cccaaattca cccaccacat gtatctgccg   420
atggatgtgg acgcgggaga agaccagccg ccggcgccag atgagccggt caccgcggtc   480
gacgacgtgg aaccggaaat gcctgcaccg tgcccgaccc agcgcccgcc ggtgaccccg   540
agacataacc tgtgcaacaa actccggact atgccagggg cgctctcggc cgcgctggcc   600
gcggcggcgc cggtctggcc ggcccctata agcggctgcc gcgggttcag cacgtccctc   660
ttagcaaaaa gaaatcaccc agtaatcgtc gggaaatag                          699
```

<212> Type : DNA
<211> Length : 699
SequenceName : SEQ ID 711
SequenceDescription :
Sequence
--------
<213> OrganismName : Mycobacterium tuberculosis H37Rv
<400> PreSequenceString :

```
atgacgacga tgattactct tcggcgacgg ttcgcggtgg ccgtcgccgg cgtcgccact    60
gccgccgcga cgaccgtcac cctggctccc gcaccagcaa atgccgccga tgtctatggc   120
gcaattgcct actccggcaa cggctcgtgg ggccgatcgt gggactaccc aacccgggcg   180
gctgccgaag ccaccgccgt caagtcgtgt ggctactccg actgcaaggt gctcaccagt   240
ttcaccgcct gcggcgccgt cgccgccaac gatagggcat accagggagg agttggaccc   300
accttggccg ccgccatgaa ggacgccctg accaagctcg gcggcggcta catcgacacc   360
tgggcctgca actaa                                                   375
```

<212> Type : DNA
<211> Length : 375
SequenceName : SEQ ID 712
SequenceDescription :
Sequence
--------
<213> OrganismName : Mycobacterium tuberculosis H37Rv
<400> PreSequenceString :

```
atggccggac tgaacattta cgtgaggcgc tggcggacag cgcttcacgc aaccgtgtcg      60
gcattgatag ttgccatcct cggactcgcc atcaccccgg tcgctagtgc ggcgacggcc     120
agggcgacgt tgtcggtgac atcgacgtgg cagaccggtt tcatcgcccg cttcaccatc     180
acaaactcga gcacggcgcc gctaaccgat tggaagcttg aattcgactt gccggcagga     240
gaatccgtct tgcacacatg gaatagcacc gttgcacgat ctggcacgca ctacgttctc     300
agcccagcga attggaatcg catcattgcc cccggtggtt cagccacggg cggcctaaga     360
ggcgggctga ccggttctta ctcgccgccg tcgagttgtc tgctcaacgg gcaatatcct     420
tgcacctag                                                             429
```

<212> Type : DNA
<211> Length : 429
SequenceName : SEQ ID 713
SequenceDescription :
Sequence
--------
<213> OrganismName : Mycobacterium tuberculosis H37Rv
<400> PreSequenceString :

```
atgctgactc gcgctatcaa gacccagctg gtgttgttga cggtgttggc ggtcatcgcg      60
gtggtggtcc ttggttggta tttcctgcgg atacccagcc tggtcggcat cggtcgatac     120
acgctttatg ccgaattgcc tcggtccggg ggtctatacc gaacagccaa cgtcacatat     180
cggggcatca ccatagggaa ggtcaccggc gtcgaaccaa ccgagcgggg cgcgcgagca     240
accatgagca tcgacaatgg ctaccagatc ccccaccgacg cctcggccaa tgtgcactca    300
gtgtcggcgg tcggcgagca gttcgttgac ctggtgtcga cccgcaccag cggtccgtat     360
ctgcggcatg ggcagacgat caccacgact acggtcccca gccagattgg cccggcgctg     420
gacgccgcca accgtggatt ggcagtgctg cccaaagacc gggtcgcgtc ggtgctgcac     480
gaggcgtcgg aggccgtggg cgggctggga tcctcactga atcgcctcat cgaagccacc     540
caggcaatcg cccacgatgt caggggcagc ctcgaggaca tcgacgacat catcgagcgt     600
tcggcgccta tcatcgatag ccaggtcaat tccggcaacg agatcgcccg ctgggccgcc     660
aacctcaaca cgctggccgc tcagaccgcg cagaccgatc cggcggtgcg aagcattctg     720
gccaacgcgg caccgactgc cgatcaggtc aacgccacgt tcagcgacgt gcgggagtcg     780
ttgccgcaga cgctggccaa tctcgaggtc gtaatcgata tgctcaagcg ctaccacaac     840
ggcgtcgagc aggcgttggt gttcttgccg cagtccggcg cgatcgccca gtcggttact     900
acagagttcc ccggccaggc cggactgggt gtcggcggcc tggcgctcaa ccaaccaccg     960
ccgtgcctga ccggcttcct gccggcgtcg gagtggcggt cacctgctga caccagcacc    1020
gcaccgctac ccaagggcac ctactgcagg attccgatgg acgcgagcaa tgtggttcgt    1080
ggagcacgca acaacccgtg tgtagacgtg cccggcaagc gggcggcgac cccgcgggaa    1140
tgccgcagca atgaagctta tgtgcccggg ggcaccaatc cctggtatgg ggaccccaac    1200
cagatgctca gctgtcccgc gccggccgcg cgttgtgacc agccggtgaa gccaggccag    1260
gtgatcccgg cgccgtcagt taacaatggc atcaacccgc tgcccgccga tcagctgcca    1320
ggcacacctc caccggtcaa cgatcctttg cagcgacctg ggtcaggcac cgtccagtgc    1380
aatgggcaac aacccaaccc gtgcgtctac accccgagca catttcctac aaccatttac    1440
gacgtgcaga gcggcaaagt cgtagcaccc gacggtgtgg tgtattccgt tgaggcttcg    1500
actcatgccg gagccgacgg atggaaggtg atgctggcac caaccggctg a             1551
```

<212> Type : DNA
<211> Length : 1551
SequenceName : SEQ ID 714
SequenceDescription :
Sequence
--------
<213> OrganismName : Rickettsia prowazekii strain Madrid E
<400> PreSequenceString :

```
atgttaaata atacacaatt tttaaatttg atgaaatcct atatgaaacc agaattttat      60
atgagttcta taaaaaatac cactaatcta gatctttcat ctatcaccaa tacaattcaa     120
aaagccatga atattttttt taccactaac aaaatttcca cagaaagtat gcaatctttg     180
tttaagaaaa attccgagat tatacaaaat aatattaata ctattttaaa tagtactaaa     240
gaagtaataa attctaaaga ttttaaacaa gctactgaat atcatcaaaa atgtgtaaaa     300
tctatttatg aaacatctat ggacaatgct aaggaattag caaatattgc ttatgaagct     360
tcaaataaaa tatttgaagc cgcaaataaa catattacca agaatattca taatgcttct     420
aataatatac ataatactgc agaacaagta caaaaaaact ttaataacaa atctgcttaa     480
```

<212> Type : DNA

<211> Length : 480
SequenceName : SEQ ID 715
SequenceDescription :
Sequence
--------
<213> OrganismName : Rickettsia prowazekii strain Madrid E
<400> PreSequenceString :

```
atgaatatta aattagttac atatttttta atattagtaa gctcattaaa agtaaatgct     60
gatttaaatc atattcaaga tagttttaaa tatcaagaag cagagcagtt aacaatagaa    120
ttaccttgga atgactgtac tgcaattcat aaattcttag aagaaaagtt atttttttca    180
gaacaacaaa taaaaaaaga aaataaaatt catgagaaat ataagcaatt ttatttacaa    240
cataataata agctttctga tttttctatg caatttctag aaaaaaaatc tgaaattaat    300
agtgtcgaaa ctttaatatc aggcttttta aaattttgtg aagataattt tcaaacaagt    360
aaaagtaaat cgcattcttt aaatttttc caaaaacaac aagaccaatg gttacataat    420
ataagaaatg agaattataa aacatattat aagaagaaat atgaagacaa tacctttaga    480
aatattaatt aa                                                        492
```

<212> Type : DNA
<211> Length : 492
SequenceName : SEQ ID 716
SequenceDescription :
Sequence
--------
<213> OrganismName : Rickettsia prowazekii strain Madrid E
<400> PreSequenceString :

```
atgaaaaagt tacttttaat tgctactgca agtgcaacaa ttttatcttc tagtgtatcg     60
tttgcagagt gcattgataa tgaatggtat ttaagagcag atgcaggtgt agcaatgttt    120
aataaagaac aagataaggc aacaggtgtt aaattaaaat ctaataaggc tattccaatt    180
gatttgggta ttggttatta tatttctgaa aatgtacgtg ctgatttaac tttaggaact    240
acaataggtg gaaaactcaa gaaatacgga gcagcaacta atacacattt tactggtact    300
aacgtttcag tgagccataa gcctactgtt acacgtttgc ttattaacgg ttatgtagac    360
ttaacaagtt ttgatatgtt tgatgttttc gttggtggtg gtgtcggtcc tgcattagtg    420
aaagagaaaa ttagtggggt aagcggtctt gcatctaaca ctaaaaataa aaccaatgta    480
tcatataagc tgattttcgg tacttctgcg caaattgcag atggtgttaa agtagagcta    540
gcatatagct ggataaatga tggtaaaaca aaaactcata atgtaatgta caaaggggca    600
agtgtgcaaa ccggtggtat gcgttatcaa agtcataacc tgacagtagg tgtaagattt    660
ggtatataa                                                            669
```

<212> Type : DNA
<211> Length : 669
SequenceName : SEQ ID 717
SequenceDescription :
Sequence
--------
<213> OrganismName : Rickettsia prowazekii strain Madrid E
<400> PreSequenceString :

```
atgaagaaga atatgagaaa gcaaatgctt aaaatcatat caatcattat tatttctctt     60
ttattaagca gttgctccga atctacgcgt gatgaaaatg gattacttac agatagtcaa    120
agtactataa ttcgagatta tataatatcg caaaattcta aaaatcttaa agtgaacctt    180
aaagaaaagt ttggttccaa tttaaaagga gtaaaattaa taggaataaa gttaacaaat    240
gaagatttat cgggaataga tttcacttca tgcgaaatat tacggactga cttcatgggt    300
agcaacttag aaaaagcaat acttacaaat tcggtaattc aagaaagtaa ttttgcggat    360
tcagtaataa aaaatatttc aggctataat gctgattttc aaggttcaat ttttaataat    420
ataacattac aaaatacaaa ttttgttcaa tcaaatttca gtgatactgc ttttaataaa    480
agtactataa tcaatgtcaa ttttgaaaat tctaaattta gtaatgtatt atggtgtcac    540
agtaatattg acagtagtaa ttttcaaaaa actcatctaa aaaataatag ctttaaaaat    600
actaatgtaa tgaattcaat attttatggt gcagatttag gcaaaagtgt aataaataat    660
acaaatttta ctaataatta ttttgaatct agtgacctaa gtaacactaa attcacatca    720
gtaatcatta aagattctaa cttcacacaa agtattttta attcagtaaa tttcaataat    780
atacaaagta ataactcttt tttttcatat acttcctttg aagattcaac attacacaat    840
attcacctta ctaaatgtga tttacaaaac agcacaatta atagttcagt tttcaataat    900
tttaaaatcg acaatgctat attaacaaat atgagtctca acgataatac atttaataat    960
ttatcaataa aaaatagtaa tactaatttt gtaaggatta ataaatccaa agggtttaat   1020
attactttac tcaatactaa ctatagtaat gctattttta gcaataatga tttaaaagaa   1080
tttaaagtca ttaatactga tttaaacaac agtgaaataa taaactcaaa tttcactaat   1140
ggacaattta ataatgtaaa tttttctcaa tctttaatac aaaacgtaaa ttttacagac   1200
gtgaaaatta ctttaggcaa tttaaatcaa gtagctctaa taaattccaa tctaataaac   1260

actaatatta ttaactcagt cctttctaat tcacaaataa ataatattaa ctaccaagca   1320
tattatagtt ttatcaatac taatgtttct aataatattg ttataaatga taattcgaat   1380
caaattccac caaataatat agtaatcaat tctgaaaaag atttacaaaa catatctaat   1440
ttagcaaata tgaatttaac aaattttaac ttaagtaatt tagtgtttaa tggagtagat   1500
ttttcaaaaa gcatctttaa aaaagctaat ttaacaaata cagtaataaa aaattctatt   1560
ttaaaagatg ctaatttttc tgcagcaata cttactaaaa cagatttctc aaaatcgata   1620
ttaacaggta gtatatttaa gtttgctcaa attgatcaga catgttttag taattccgac   1680
ttaacaaata ctgattttac tgaagcaaca attaaaaata ctgcatttga taatgctaat   1740
acacacggta taaaaggatt agaataa                                        1767
```

<212> Type : DNA
<211> Length : 1767
SequenceName : SEQ ID 718
SequenceDescription :
Sequence
--------
<213> OrganismName : Porphyromonas gingivalis W83
<400> PreSequenceString :

gtgatacaaa aatttactaa tgtaaaacta aatgatatgc gaaaaatttt gagctttttg 60
atgatgtgct ctctgcattt aggtctacaa tctcagactt ggcatggaga tccggactca 120
gtggcagccc taccttctat cggtattcaa gagtcaagtt gtacccgaat cacgttcgag 180
gttgttttcc ccggatttta tagtgtggaa aaacgagaag gcaaccaagt ctttcagcgc 240
atttccatgc cgggttgtgg ctcgtttggg aatctgggcg aagctgaatt gcctgttttg 300
aaaaagatga tagccgttcc ggaattttca acagctaacg ttgctgtaaa aatcaaagag 360
acggagacat tcgacaatta taatatctat cctaatccta cctatgtcgt agaggagttg 420
cctgaggggg ggacttatct ggtagaggct ttcgcgataa acaatgacta ttatagccaa 480
aatgtaagcc tcccttctac tcactatgtc tattctcaag acgggtattt tcgctcacaa 540
agatttatcg aagttaccct gtatcctttt cgatacaacc ctgtccgaca agaaattcta 600
tttgcaaaaa aaatcgaggt tacaataact ttcgataatc ctcagccacc tttacaaaaa 660
aacaccggca tatttaacaa agtagcctcc tctgcattta ttaattatga agctgatggc 720
aaatcggcga tagaaaatga tatggtgttc agtcgtggta caacaacgta cataagcgga 780
aatgttgcca gcaacctccc tcagaactgt gactacttgg ttatttacga tgatatgttc 840
aacgtaaatc aacaaccaca cgacgaaatc aaacggctgt gcgaacatag agccttctac 900
aacggctttg atgtagctgc tgtaagtata aaggacgtat tgaatagctt cccatcaaat 960
gccacctcat acatcaacga aactaaactg aaaaatttca ttcgctcagt ttacaaccaa 1020
agcaatgcga agaggacttt agatggcaaa ctgggatacg tgctactgat cggaaaacca 1080
ttgagcaaat atttggctga cactgataat acaaaagtcc caacctcttt tattcataat 1140
gtctccttaa ttccaagtca tccaactttt ggttccatat gcgcctccga ctattttttt 1200
agttgtgttt cgccccttga tactgtcggc gatttgttta tcggtcgatt tagcgtcacc 1260
aatgctcatg aattgcacaa tctgattgaa aagactatca acaaagaaat ctcatataat 1320
cctattgcac acaaaaatat tctttacgca gaagggaaag gctgcgatgc tccaatctta 1380
cgtttattct taaaagaaat cgcctctggt tacacagtca actctatctt aaaatctaat 1440
caggtctctg caatagactc gatatttgac tgcttgaata atggttccca tcatttttat 1500
tttaacactc atggaatgcc gactgtttgg gggatagggc agggactcga cgtcaatact 1560
ctaacagccc gattgaacaa tacatcttcg cagggattat gtacgagtct atcatgtagt 1620
tcggctgtag cagattcaac tattagatcg cttggagaag tcctgaccac atacgcacct 1680
aacaagggat tctcggcttt cttaggagga agcagagcca cccaatatgc cgtttattta 1740
gaaggcccct gtcctccgtc agaattttat gaatatttac cttattcttt atatcacaat 1800
ctctcgactg ttgttggcga aatgttgcta tcatccatta tcaatactaa ttctgttgat 1860
acgtattcga aattcaactt caatttgctt ggcgaccctg cactaaacat tatggctcat 1920
ggcatggagg ttagtaattg tattacacta ccaaacaaca ccattataag cagtccgata 1980
acaataaaaa atggtggctg cctaaaaata ccggaaaaag gagttttgca ttttactaat 2040
aatggctcca tacaagtcat gtccggagga actctggaaa taggcaatca ggctaaaata 2100
tccggagaga ccggtgctaa ccccaccttt attaccgttt acggcgatgg tcttgcgatt 2160
aacaagcagg tagagataga caatatagac cgacttaact tgttttctac gcattcggtc 2220
atgcccaaat ttcattttga cagtgtgaaa ttcaacagtg ccccgctgta tacaacgaac 2280
tgtattgtgg agataagcaa ttgcgaattt accaatcgaa gtgacattat ttcaaagaat 2340
tgtgacctaa gcgttgaaaa cagtatgttt agcagttcgg ggataacggt attcaagcct 2400
atggctacaa gctccatcac cggattatct acaaaagcaa agattaccga caatactttt 2460
tttgcgacag gaaacttcgc ctaccatatc acaaacacgc caggcttaac agcaacctcc 2520
aatgctgcca tcaagttaga caatattcct gagtattaca tttccggtaa taaaatagtc 2580
aattgcgatg aggctcttgt actaaataat agtggcaaca gaacgaacag actccacaat 2640
atcacacgga atgtgataaa aaactgtagg attgggagca cgctttataa ttcctatggt 2700
atttacaacc gaaataagat cagtaacaat catataggag tacgtctcct caacaacagt 2760
tgtttttatt tcgataatgc tcctgtaatc aatgaagaag ataagcagac gtttatttct 2820
aataggactt ggcagctcta ttcatcaaac ggtacattcc ctctcaactt ccattacaac 2880
agcttgcagg ggggagatac agatacatgg atttacaacg acacgtatac gaatcgctat 2940

attgacgttt caaataatca ctggggcaac aatgatttgt ttgatccgaa tcaggttttc 3000
aatacgccag acttgttcat ttggatacct ttttgggatg gattgccaaa tgggagatcg 3060
ggcaatagct ctgctgaagc agtagaattc caaacagcat tggactgtat tggcaatagc 3120
gattatcttt cggcaaaagt ggctctcaag atgatggttg aaacctaccc ggaatccgac 3180
tttgcaatag ctgctttgaa ggaattgttc aggatagaga aaatgtcagg caacgattac 3240
gaaggcttga aagattattt cagatccaat ccaaccatca tctcttccca gaacttgttc 3300
ccgacagctg atttcctgtc tgcgcgatgc gatattgtgt gtgaaaacta tcagtctgcc 3360
atcgattggt acgaaaatcg cttgaatagt gaaatctcct atcaggacag tgtttttgca 3420
gtcattgacc ttggtgacat ttattggaat atgcagttag actcactcag agggactggt 3480
atagatttga acatactttc ctgtgaacaa aggaaatcgc tcgaaagcca tcaaaatgta 3540
aaaaattatt tgttgtcaac tcttcccgaa tcaacaggta ctctcctgcc tccattagaa 3600
tgcaacaaat caagccttga taaatccaag ataatctcta tttcgcccaa tccggcgaaa 3660
gctgttgtaa caataatcta ctataccgat aacccttcct gttctgtaat aaaaatatat 3720
ggaataaatg gagcctcggc tgatataacc gggttgccca aacatctatc cgaaggttat 3780
tacagcatac agttcaatac atccaacttt gatcccggtt tctacctggt aacgctaaat 3840
gttgatcaga aaattataga tacggaaaaa ttacgaatca aataa 3885

<212> Type : DNA
<211> Length : 3885
SequenceName : SEQ ID 719
SequenceDescription :
Sequence
--------
<213> OrganismName : Shigella flexneri 2a str. 2457T
<400> PreSequenceString :

```
ttgcagggta aaaacacgat tgtcacaacg ggggattact caattggttt gctaagtcaa      60
acgagtggaa acctgaatac tgacaccata ataagagtca actctgacgg ttcggttacc     120
ccctcttttt ctgacggaga tgatacattt attgttactg cgggtaatca cgcagtcggc     180
gttcttgcgt gtgcatctcc cggaagcgcg tgtgcgtgtg tatcttctct tgatgaagaa     240
agtactgccg atacaggaag taatgaaaat aatgccatag caaaactgga tatggcaaaa     300
ggggagatca caacccacgg aacagaaagt tatgccgcgt acgctaatgg cactgtcgtc     360
aaggctggtg acacgcttga ttataccaat gccagcgtta cattaaccga tgttgatatc     420
accacgcatg gggacaatgc tcatgccatc gctgcccgcc aggggacagt ttcatttaac     480
cagggagaaa tttatacaac gggtcctgac gccgcgattg ctaaaattta taatggcggc     540
acggtgacgc tgaaaaatac atctgcagtc gcgcatcagg ggtctggaat tgtgctggag     600
tcttccataa atggtcagga agcaacggta gatattttat ctggtagctc actacggtca     660
gcaaatgaaa tcctctacca taaagatgag acgagtaacg tgaccattac ggatagtgag     720
gtatcatcgg ctgcagatgt ttttatcaat aatattaaag gtcatttgac cgtcgatgca     780
actaattcaa aaataacggg ttcagctaat atttcaacag atgataacac tcatacctat     840
ctgtcgcttt cagataatag tacctgggat attaaagctg actcaacggt gagcaacctc     900
acggttgata atagtacggt ctatatttcc cgggcagatg gacgggatgt cgaaccaaca     960
agattaacaa taactgaaaa ttacgttggt aataatggcg tattgcatct cagaactgaa    1020
ttggatgatg ataattcagc tacggataaa gtcgttatta atggaaatac ctctggaaca    1080
acccgagtta aagttaccaa tgcaggaggc agcggagctt acacgttaaa tgggatagag    1140
attatcagcg ttgaggggga atcaaatggg gaatttatta aggattcgag gatttttcgcc    1200
ggtgcctacg aatattcatt aacccgaggt aataccgaag cgaccaataa aaactggtat    1260
ctgactaact tccaggcaac gagcggcggt gaaacaaact ccggaggaag ttcagcgcct    1320
actgttgcgc ctacccccgt cctgcgcccc gaagctggaa gttacgtcgc caacctggca    1380
gccgctaaca ctcttttgt tatgcgtctg aacgaccgtg cgggtgaaac gcgctacatc    1440
gatcctgtaa ctgaacagga gcgttcaagc cgactttggc tacgtcaaat tggcgggcat    1500
aatgcctggc gtgacagcaa cggacagttg agaacgacct cgcatcgcta cgtctcgcag    1560
ttagggggcg atctgttaac cggtggtttt accgatagtg acagttggcg tttgggagtg    1620
atggctggtt atgcccgcga ctacaactta actcattcca gcgtgtcgga ttatcgttcg    1680
aaagggagtg tcagaggcta tagcgcaggg ctgtatgcca cttggtttgc cgatgacatc    1740
agtaaaaaag gcgcatacat tgactcctgg gcgcaatata gctggtttaa aaactccgtg    1800
aaaggggatg aattagccta tgaatcctat agcgcgaaag gtgcaaccgt ctcgctggaa    1860
gcgggttacg gttttgccct gaataaatcc tttggtctgg aagcggcgaa atatacgtgg    1920
atcttccagc cacaggcaca ggctatctgg atgggcgtcg atcataatgc gcacacggaa    1980
gccaatggct cacgtattga gaatgacgca aataacaaca tccagacccg actcggcttc    2040
cgcacccttta ttcgtactca ggagaaaaac agcggtccgc acggtgacga ctttgaacct    2100
tttgttgaaa tgaactggat ccataacagt aaagattttg ctgtctcaat gaacggtgtg    2160
aaagtcgaac aagatggggt gagtaatttg ggggaaatta aacttggcgt aaatggcaac    2220
ctgaatccag cggccagcgt ctgggggaat gtgggcgtgc agctgggtga taatggctac    2280
aatgacaccg cagtgatggt gggcctgaaa tataagttct ga                       2322
```

<212> Type : DNA
<211> Length : 2322
SequenceName : SEQ ID 720
SequenceDescription :
Sequence
--------
<213> OrganismName : Shigella flexneri 2a str. 2457T
<400> PreSequenceString :

```
gtgaccaaac tcaaacttct ggcacttgga gtgcttatcg caacgtctgc aggcgtagcg      60
cacgctgaag gtaaattttc cctgggcgca ggcgtaggtg tcgttgagca cccatataaa     120
gattacgata ccgatgttta cccagtaccg gtaatcaact atgaaggcga taacttctgg     180
ttccgtggct taggtggtgg ttactacctg tggaatgacg caacggataa actttcaatt     240
accgcttact ggtcgccgct ttacttcaaa gcgaaagaca gtggcgatca ccaaatgcgt     300
cacctggatg accgtaagag caccatgatg gctggtctgt cttatgctca ctttacccag     360
tacggttacc tgcgtaccac cctggctggc gataccctgg ataacagcaa cggcattgtc     420
tgggatatgg cctggttgta tcgttacacc aacggtggcc tgaccgtgac tccgggtatt     480
ggtgtgcagt ggaacagcga aaaccagaac gaatactatt atggcgtatc gcgcaaagag     540
tccgctcgca gcggtctgcg tggctataac ccgaatgaca gctggagccc ttaccttgag     600
ctgagcgcca gctacaactt cctcggcgac tggagtgttt acggtaccgc gcgctacacc     660
cgtctgtctg atgaagttac tgacagcccg atggtggata aatcctggac tggcctgatt     720
tctaccggga tcacctacaa attctga                                         747
```

<212> Type : DNA
<211> Length : 747
SequenceName : SEQ ID 721
SequenceDescription :
Sequence
--------
<213> OrganismName : Shigella flexneri 2a str. 2457T
<400> PreSequenceString :

```
atgaaaaaaa ttgcgctagc aggtctggcc ggaatgctgt tggtttctgc atcggtcaat      60
gcaatgagca tcagcggcca ggcgggtaaa gaatacacca atattggtgt cggttttggg     120
actgaatcga cgggcctggc tttaagcggt aactggacac ataacgacga cgacggtgac     180
gtcgcgggcg tggggctggg gttgaatctg cctctcgggc cgttaatggc gaccgttggc     240
ggaaaaggcg tgtacaccaa cccgaattac ggcgatgaag gttatgccgc agcggtagga     300
ggtggtttgc agtggaaaat tggcaacagc ttccgtttgt ttggcgagta ttactactct     360
ccggattcgc tctccagcgg tattcaaagt tatgaggaag cgaatgctgg cgcgcgttac     420
accattatgc gtccagtcag tattgaggcg ggttatcgct acctgaatct gtcgggtaaa     480
gacggtaacc gcgacaacgc tgtggctgac ggcctgtatg ttggggttaa cgccagtttc     540
tga                                                                   543
```

<212> Type : DNA
<211> Length : 543
SequenceName : SEQ ID 722
SequenceDescription :
Sequence
--------
<213> OrganismName : Shigella flexneri 2a str. 2457T
<400> PreSequenceString :

```
atgactacct tgaccgccag ggtgtttaca acggcagaga taatttatcg gaaaacagtt      60
atcgcattgg tgtgtcattt aaactgtagt agacaggaga cagtcacaat gaataaaact     120
ataatggcgc ttgctatcat gatggcttca tttgccgcaa acgcgtctgt attaccggaa     180
actcctgtgc catttaaaag tggtaccgga gcaattgata acgacactgt ctacattggt     240
ttaggtagcg caggtacagc atggtacaag ctagatacac aggccaaaga taaaaaatgg     300
actgcgttag ctgcattccc tggtggaccg agagagcaag caacctctgc atttattgat     360
ggcaatctgt atgtgtttgg cggcattggc aaaaacagcg agggattgac tcaggtattt     420
aatgacgtac acaaatacaa ccccaaaacc aatagctggg ttaaattgat gtcgcacgcg     480
ccgatgggca tggcgggtca tgtgactttt gtacacaacg gcaaggctta tgttactggc     540
ggtgttaacc agaatatctt caatggctat tttgaagatc tcaacgaggc tggaaaagat     600
tcaaccgcta tagataaaat caacgcccac tattttgaca aaaaagcaga agattatttc     660
ttcaataagt ttctgttgtc ttttgatccc tcaacacagc aatggagtta cgctggcgaa     720
tcgccctggt acggaacggc tggtgcggcg gttgtgaata aaggtgataa aacctggctt     780
attaatggcg aagccaaacc aggattgcga acggatgccg tatttgaact tgatttcacc     840
ggtaataatt taaaatggaa taagcttgat cccgtctcat caccagatgg cgtcgctggc     900
ggttttgcgg ggataagcaa tgattctctt atatttgctg gaggggccgg attcaaaggt     960
tcacgagaaa attaccagaa cggtaagaac tatgcgcatg aaggcctgaa aaaatcatat    1020
agcactgata ttcatctttg gcataacggg aaatgggata aatcgggtga attatcgcaa    1080
ggtcgggcct acggagtatc attgccctgg aataatagtt tattaattat tggcggtgaa    1140
actgcaggcg gcaaagcggt gacggattca gttttaattt ctgtgaagga taataaagtc    1200
```

acagtacaaa actaa 1215

<212> Type : DNA
<211> Length : 1215
SequenceName : SEQ ID 723
SequenceDescription :
Sequence
--------
<213> OrganismName : Shigella flexneri 2a str. 2457T
<400> PreSequenceString :

```
atggcaactg gcggcgctgc cctggcaggt aaagcggtta tgggtgccgc agccggtgca      60
gccggaggtg caagtgcact ccaagcggct tttcagaaag catcagcgag tatggaaacc     120
ggcggtgaca tgtccagcat ggggtcagtt gtcagcagtg gcggaaacgg tggtggtgaa     180
gcgggtactg ctggcagtag cccattcgcc caagcggctg gctttggtga cagcggcagt     240
agctcaagcg gtggcggctt tgccaaggcc gcgaagctgg ccacaggcac ggcctccgag     300
ttagccaagg gtgtcggctc tcaagtgaag cagggattcc aggagcgagt gagcgaaacc     360
acaggcggaa aactggctgc ttcgatacgc gaaagcatgg agccgaaaga agcaagccaa     420
tctggccagt tcgagggcaa tagcttgggc gccgattctg gcccagatag taacgaagtc     480
aggagttag                                                             489
```

<212> Type : DNA
<211> Length : 489
SequenceName : SEQ ID 724
SequenceDescription :
Sequence
--------
<213> OrganismName : Shigella flexneri 2a str. 2457T
<400> PreSequenceString :

```
atgaagcgag ttcttattcc tggcgtcatt ttatgtggcg ctgatgtggc gcaggccgtc      60
gatgacaaaa acatgtacat gtattttttt gaagagatga cggtctatgc tcctgtccct     120
gtacccgtaa acggcaacac gcattacacc agtgaaagca tcgagcgttt accgaccggg     180
aatggcaata tcagcgatct gctgagaacc aaccctgcgg tacgcatgga ttcaacgcaa     240
agtacctcgt tgaaccaggg agatattcgc cctgagaaaa tctctattca cggtgcgtcg     300
ccctaccaga atgcctattt gattgacggt attagtgcaa ctaataacct gaacccagcg     360
aatgagtccg atgccagtag tgcaaccaat attagcggga tgtcacaggg gtattatctt     420
gatgtcagct tactggacaa tgtgacgctt tatgacagtt ttgtgccggt tgaatttggt     480
cgcttcaatg gcggggtaat tgatgcaaag atcaaacgct tcaacgctga tgatagcaag     540
gtgaaattgg gttatcgcac tacgcgtttg gactggttaa catcgcatat cgatgagaat     600
aacaagagcg catttaatca aggttcttca ggaagtacct atttctctcc agattttaaa     660
aagaactttt ataccttgtc gtttaatcag gagctggctg ataactttgg cgttaccgcc     720
ggtttatcgc gccgccagtc tgatatcacc cgcgcggatt atgtttcgaa tgacggcatt     780
gtcgccggtc gggcacagta taaaaacgtt atcgatactg cattgagcaa atttacctgg     840
tttgccagcg accgctttac ccacgattta accttaaaat ataccggctc cagccgtgat     900
tataatacca gcaccttccc gcagtctgat cgcgaaatgg gtaataaatc ctatggtctg     960
gcatgggata tggatacgca actcgcatgg gccaaactac gtaccaccgt tggttgggat    1020
catattagtg attataccgg tcacgatcat gacatctggt acaccgaact ttcatgtaca    1080
tatggtgata ttacagggcg ttgtacccgt ggcggattag gacacatttc ccaggctgta    1140
gataattaca ccttcaaaac acgcctggac tggcaaaaat tcgccgtggg tgatgtttcg    1200
catcaaccct acttcggcgc ggaatacatc tattccgatg catggactga acgccataac    1260
cagtctgaat cctatgtgat taatgctgca ggaaagaaaa ctaaccatac catttaccat    1320
aaaggtaaag gcagcctggg aattgacaac tacacgctgt atatggcgga tcacattagc    1380
tggcggaatg tgtcgttaat gcccggtgtg cgttatgact atgacaacta tctgtcaaac    1440
cacaatatct ccccgcgctt tatgacggaa tgggatattt ttgctgatca aacctcaatg    1500
attaccgccg gttataaccg ttactatggc gggaatattc ttgatatggg attacgtgat    1560
atccgcaata gctggacgga atcggtatca ggtaataaaa ccctgacgcg ttatcagaat    1620
ttgaaaacgc cttataacga tgaactggca atgggattgc agcaaaaaat cgataagaac    1680
gttattgcac gcgcaagtga agcgcatgat caaatcagca aaagcagtcg taccgacagc    1740
gcgactaaaa ccaccattac tgaatataac aacgatggca aaaccaaaac gcattcgttt    1800
aacctcagtt ttgaactggc cgaacccctg catatccgcc aggtagatat taacccgcaa    1860
attgtcttta gctatatcaa gagcaagggc aacttgtcgt taaacaatgg ttatgaggag    1920
agcaataccg gtgataacca ggtggtttat aacggtaatc tggtctctta cgatagcgtt    1980
ccagtggcag attttaataa cccattaaag atctccttaa acatggattt cacgcatcaa    2040
ccgagcgggt tagtgtgggc gaatacgctg gcctggcaag aagcgcgtaa agctcgcatt    2100
atccttggta agacaaatgc gcaatacatc agcgaatatt cagattacaa gcagtatgtt    2160
gacgaaaaac tggatagcag cctgacctgg gacacccgct tgtcctggac gccacaattt    2220
ctgaaacaac aaaacctgac gatcagtgcc gatattctca atgtactgga tagcaaaacc    2280
gctgttgata caacgaacac cggtgtggcg acctacgcca gtggccgtac tttctggctt    2340

gatgtcagca tgaaatttta a                                               2361
```

<212> Type : DNA

<211> Length : 2361

SequenceName : SEQ ID 725

SequenceDescription :

Sequence

--------

<213> OrganismName : Shigella flexneri 2a str. 2457T

<400> PreSequenceString :

```
atgaagaaaa cattgttagc aatcatgctg gcggggacag cttttgcttc tcaggcggga      60
actttagtaa gccagggtac tgaagcgtca gctaatctta ctttgaccaa gcctatcgtt     120
gttaataata ccattcaacc agtgaaaggg gtatatagcg gtacgttaac tgcatggacg     180
cctttggcaa caggtattgt tggcgcatct gatgggcaga gccacgacta tgctgtaact     240
ttcccggatg atatttatgc ggagagcagt acttcagcgg atgcagtaat ttctggtgac     300
aataacccgg accataaact gaaagtttcc ctgacaacgc ttgagcagga tcctccttca     360
gctgcctccg aagagattgg cggtaagcgt tatatgatgc tgaaaaatac tggaacaggt     420
ggtgcctatc gcgtcgtttc ccatatgaaa gaacaggttg ttgagccaga ctcttacacc     480
atccgtaccc aggcttatat ctacgcagaa taa                                  513
```

<212> Type : DNA

<211> Length : 513

SequenceName : SEQ ID 726

SequenceDescription :

Sequence
--------
<213> OrganismName : Shigella flexneri 2a str. 2457T
<400> PreSequenceString :

```
atgggtattt accactggtc ccggaagaca aaaatgaaac ggacaaaatc catacgccac      60
gcatcgttcc gcaaaaactg gagcgcacgc catctgacac cagtcgctct cgcggttgcc     120
actgtttttta tgctggctgg ctgtgaaaag agtgatgaaa cagtgtctct ctatcaaaat    180
gctgacgact gttcagctgc aaacccaggc aaaagcgccg aatgtaccac cgcgtacaac     240
aatgcgctga aagaagccga acgtactgcg ccgaaatacg ccacccgtga agactgtgtt     300
gctgaatttg gtgaaggtca gtgtcagcaa gcaccagccc aggctggcat ggcaccagaa     360
aaccaggcgc aggctcagca atccagcggg agtttctgga tgccgctgat ggccggttac     420
atgatggggc gtctgatggg cggcggcgcg ggatttgcac agcagccgct gttctcctcg     480
aaaaacccgg ccagtccggc ttacggtaaa tataccgacg cgacgggtaa aaactatggc     540
gcagcccagc caggccgcac catgaccgta ccgaagacgg caatggcacc aaaaccggcg     600
accaccacta ccgttacccg tggcggtttt ggtgaatctg ttgccaaaca aagcactatg     660
cagcgtagcg caaccggcac ctcttctcgt tcaatgggtg gctga                     705
```

<212> Type : DNA
<211> Length : 705
SequenceName : SEQ ID 727
SequenceDescription :
Sequence
--------
<213> OrganismName : Shigella flexneri 2a str. 2457T
<400> PreSequenceString :

```
atgacaaaaa tgagtcgcta cgccttgatt accgcgctgg cgatgtttct cgccgggtgt      60
gtggggcaac gtgaacctgc accggtagaa gaagtgaaac cagcgccgga acaaccagcc     120
gagccacaac agcctgtccc cacagtgccc tcggtgccga cgatcccgca gcagccaggc     180
ccaattgagc acgaagatcg aacggcaccg cctgcgccgc atattcgcca ttatgactgg     240
aatggcgcaa tgcagccgat ggtcagtaag atgcttgggg ctgacggggt gactgcgggt     300
agcgtcctgc tggttgatag cgttaacaac cgtactaacg gttcgctgaa tgccgcagaa     360
gcgaccgaaa cgctgcgaaa tgcgctggct aataacggga aatttaccct ggtttccgcc     420
cagcaactgt cgatggctaa gcaacagtta ggtttgtcgc cgcaggacag tttaggcacc     480
cgtagtaaag ccataggcat tgcccgcaat gtcggcgctc attacgtgct gtactcctgc     540
gcctctggca acgttaacgc tccgacccta caaatgcagc tgatgctggt gcagacgggc     600
gaaattatct ggtcaggtaa aggtgccgtt tcgcagcaat aa                        642
```

<212> Type : DNA
<211> Length : 642
SequenceName : SEQ ID 728
SequenceDescription :
Sequence
--------
<213> OrganismName : Shigella flexneri 2a str. 2457T
<400> PreSequenceString :

```
atgacgaaac ttatgcaatt tgttcagagg tgctattata tgactaataa gaaaatgtat      60
tttatattaa tacttgtttt cacattgcta caggtgtgtt tttttgctct ctggaaggct     120
cgtgatggta gcacaacgtc tcttgaatgt acgtcgacgt taacaagaaa tgctaaaaca     180
gatcattctt tatactactc tgctaacctt tcagtcattt taaaaaaaga tggtagcggt     240
agttttacga ttgttggctt aactgatgaa gatacaccga ggaaattttc ccattcgtat     300
ttttttacct acaaaataga tagtaacgga cggatctcag gtaatgctaa ggccaaagtt     360
tcgggactgg aaaatcagat aaaggatgag aacttcagac tcaattttct cgatgcatct     420
ttaacaggta aaggcaatgc gaggctaagc aagtttaata atgtctatat ttttagtatc     480
ccggggttga tcattaacac atgtgctcca atataa                               516
```

<212> Type : DNA
<211> Length : 516
SequenceName : SEQ ID 729
SequenceDescription :

Sequence
--------
<213> OrganismName : Shigella flexneri 2a str. 2457T
<400> PreSequenceString :

```
atgggcagga taagctcggg aggaatgatg tttaaggcaa taacgacagt cgccgcactg    60
gtcatcgcca ccagtgcaat ggcgcaggat gatttaacca ttagcagcct tgcaaagggc   120
gaaaccacca aagctgcgtt taatcagatg gtgcaagggc ataagctacc tgcatgggtg   180
atgaaaggcg gtacttatac tcccgcacaa accgtaacgt tgggagatga gacgtatcag   240
gtgatgagcg cgtgcaaacc gcatgactgt ggctcgcaac gtatcgctgt gatgtggtcc   300
gagaaatcta atcagatgac ggggctgttc tcggctattg atgagaaaac gtcgcaagag   360
aaactcacct ggctgaatgt gaacgatgcg ctttcgattg atggtaaaac ggtgctgttc   420
gcggcgttga ccggcagcct ggaaaaccat ccggatggct ttaattttaa ataa         474
```

<212> Type : DNA
<211> Length : 474
SequenceName : SEQ ID 730
SequenceDescription :
Sequence
--------
<213> OrganismName : Streptococcus mutans UA159
<400> PreSequenceString :

```
atgaaaaaac aatttttgga aaaagctgtg tttactgttg cggctacggc agcaacagtt    60
gttttaggaa ataaaatggc tgatgcagac acttatactc ttcaagaagg agattctttt   120
ttcagtgttg ctcaacgata tcatatggat gcttatgagt tagcttctat gaatggaaaa   180
gatattacca gtctgatttt gccgggtcag actttaactg ttaatggttc ggcagcaccg   240
gataatcagg cggcagcgcc aactgacact acgcaagcaa ccactgaaac gaatgatgcg   300
aatgccaata cttatcctgt tggtcaatgt acttgggggg ttaaagctgt tgcaacttgg   360
gcaggcgact ggtggggcaa tggcggtgat tgggcctcta gtgcttctgc acaaggttac   420
actgtcggta acactccggc agtagggtct attatgtgtt ggacagatgg tggttatgga   480
catgttgcct atgtcacagc tgttggtgaa gatggtaaag ttcaagtact ggaatccaat   540
tataaagatc aacaatgggt tgataactat cgtggttggt ttgatccaaa taatagtgga   600
acaccaggca gtgtcagtta tatttatcct aactaa                             636
```

<212> Type : DNA
<211> Length : 636
SequenceName : SEQ ID 731
SequenceDescription :
Sequence
--------
<213> OrganismName : Streptococcus mutans UA159
<400> PreSequenceString :

```
atgtctatta aaaatatttt agaaaacaaa acaacaacaa ttaaagttag ttttgcagga    60
attgcaacag cagctagttt aattttacct atggcagtac aggcagaaac tacttatact   120
gtgaaatcag gagatacttt atcagagatt gcttcaacac acggaacaac tgttgataaa   180
cttgctaagt taaataaaat taataatatc catcttatcc atgctggtca aattttagaa   240
ttagatgcag caacagaaga tactgatgca acgccagtac aagaaagtca gataaatgaa   300
gcagaaacct cagcatctgc caaaactagt caaacgagcg aagtgacgac aacagcaccg   360
gtacaagaaa gccaaacaag tgaagttata acttcagcac cagctgaaac cagtcagaca   420
agcgaagtgc caactgaagc caaccaaaca aatgaagtaa gttcagcagt atcggttgaa   480
accagtcaaa cgagtgaggc gacgacttca gctccagtgg aaactagtca gacaagcgaa   540

gcaacgacag cggaaccaac tgagaccaag accagccaaa caaatgaagt agcagcttca   600
gctgaagaaa accaaacaac atctaatact agcggtttga gcacatctga tgcagctgca   660
aaagaattca tcgctcaaaa agaatcaggt ggtaattata atgctaaaaa tggtcaatat   720
tatggacgtt atcaattgag tgattcttac ttgaatggtg atttgtcaga agaaaatcaa   780
gaacgtgtag cagatgctta tgtatcaagt cgttatggtt catggactgc tgctcaagct   840
ttctggaatg ctaatggttg gtattaa                                       867
```

<212> Type : DNA
<211> Length : 867
SequenceName : SEQ ID 732
SequenceDescription :
Sequence
--------
<213> OrganismName : Streptococcus, mutans UA159
<400> PreSequenceString :

```
atgaaatgtc aagcgtttga agattttaaa gctacaagtt tgaataagct gtcttatacg      60
acaggtggag ctactgatgg tgaaattata gctaatcgga tgttacaagg taaagctact     120
aagggcgaaa ttactatgta tacttggaac attattcaaa atggctgggt gaattcactc     180
gtgtcttggg gtattggtgg ttataatagt tctataggat actcagctca aggtaataga     240
ggatttagta attatccata cgatgtttct atggattcag ataatagcag tagttcaagt     300
aatacgacag gtggttatgt taattataac cagagtttta attctggatg gtaa           354
```

<212> Type : DNA
<211> Length : 354
SequenceName : SEQ ID 733
SequenceDescription :
Sequence
--------
<213> OrganismName : Streptococcus mutans UA159
<400> PreSequenceString :

```
atgcgctatt cacaaatttg tcgtaaaagt ttggctttgc tggctacagg aatgatttta      60
actacctcaa ccttacctag tataagtatt ctagctgagg acagtactgg agcacctgct     120
aggccagatg gacaagctcc tgctggaggt ggtgctaaca ccacaactta tgattacagc     180
ggaatcaaca gtggtgttct agttgctaat ggtagcaagg tcacttccag ttccaaaacc     240
aaatcaacta cttccgccca aaatacagct cttgttcaaa acggtggtag tttaacactg     300
cataaagcga atttaataaa atccggtgat gataacaatg gtgacaatga taatttttac     360
ggtattaatt ctattttact agcagtcaat gaaaggtcaa aagcttatgt ttcgaattcc     420
aaactaaaag ctagtagctc tggcagtaat gggatttttg caactgataa ggcaaccatc     480
tacgctaata aaacaagcat tgcgactaca gctgataatt cacggggact tgatgccact     540
tacaatggca atattattgc taataagatg gccatttcta caaaaggtgc tcacagcgct     600
gctattgcaa ctgaccgtgg tggcggcaat atttccacca ctaattccag tttaaatact     660
agtggctctg gctcacctct tctttattca acaggcaata ttcaagttaa tcacgttaca     720
ggaacatcta gtaacagcca aattgctggt atggaaggtc ttaataccat tcttattcat     780
aattctaatt taattagtac catgacaaac aaaactgcca gtgacccgat tgccaatggc     840
gttatcatct atcagtcaca atccggtgat gccgaagcaa caacggggca aagtgcccac     900
ttcgagctca gcaagtctaa attaacttct tccattactt caggttctat gttctacctg     960
acgaatacct ctgcaaacat tatccttaat caatccacct tgaattttga tgcaaataag    1020
gctaaacttt tgactgtagc aggcaatagt gccaataatt ggggaacccc cggtagtaat    1080
ggggcaacag ttaactttac tggccataag cagacactta aaggggatgt tgatgtggat    1140
agtatttcga ccttaaatat gtacctgctt gataaaacca actacactgg caaaactgct    1200
gtatcaacca acagtaccaa tatatcccca agcacgtctc ctattaccat gaatatttct    1260
aaaaattcca aatgggtgct aactggtcat tcgacagtaa ccaatctcaa tgctgaaaaa    1320
ggtgctaaaa ttgttgataa agacggaaaa accgtcagcg tcatctcttc aagcggacaa    1380
aaacttgtta aaggtaaaag caaatatagc ctaacagtca caggaactta cagtcaaaag    1440
gtaacaacca gctcaagtaa caaacccagc agcagttaca ttaaccgtag tgacttcgat    1500
aattatttta aaacaacaac agcctttgta aataatacca aaaatacaag taattaa       1557
```

<212> Type : DNA
<211> Length : 1557
SequenceName : SEQ ID 734
SequenceDescription :
Sequence
--------
<213> OrganismName : Streptococcus mutans UA159
<400> PreSequenceString :

```
atgaacaaga taggtgatac tctacgtgat gctcgtattg aaaaaaaatt gagttttgat     60
gatgttgtag ataaaactgg aattgctcct cactatatac tggcaatgga gttagatcaa    120
cttaaattat taccagaagg caaaacaaat gagtatttag aaaaatatgc tcatgctgtt    180
ggcttagatc cggtttctat tattcatggt tatcgcaatc aggaaatgag cgatgaactt    240
atcctacctt cttctgcaga attggctgct tcttcagata gcaatataga aaagaaaaat    300
gaaggaaaat caattgaaga acctcaagag ttagctattg atagtttgga tgttactcaa    360
aatattacag aagagactcc ccaaatagaa gattttaagg ttgaatcaga ggaggcaagt    420
aaaaaaatag aaaaaatacc atctcgactt agtaaatatg attatgatga agaaccgaaa    480
aagaaatttc catgggcttt aattctgcta attttacttg ctttaactat tatcagttat    540
gttggatatg tggtttataa tcagctgcaa actgattcaa ataagactga attaagtacc    600
tctacgaaaa aatctaagga tactaaaaat gatgccaatt caacgacaca aagtcagacc    660
agcataacaa cagactttgc tgatggtgga aataatatca ccttaagtaa tactaatgga    720
aaagtagagg ttacctttac tttgacaggg gatgaggaaa gttgggtttc agcaactaac    780
actactgatg gagaatctgg aacaactcta acagcaacag ataaaactta tactgttact    840
ttagcagaag gttcaacaac atctatgctg acagtcggat cacctagcgg tgttgaaatt    900
acgatcaatg gtcaaaaggt ggatactaca aaccttgtta atgctggttt gactaatatt    960
aatttgacag tccaataa                                                  978
```

<212> Type : DNA
<211> Length : 978
SequenceName : SEQ ID 735
SequenceDescription :
Sequence
--------
<213> OrganismName : Streptococcus mutans UA159
<400> PreSequenceString :

```
atgaagagca gaaaacgtca gcgaaaaggg ctggttcgaa aaaatgaaat cattatttta     60
actctttttg tcgcatcagc cgtttcactc ttagcattta ccaattcttt tggggtactt    120
gctaagagtc ttcatcttga aaaaatcaat aaatcgataa ctatttcgct tccttttggt    180
aaaaaaaaga tggaacaaac agcacgttac tattcaggtg aacaggttca gatcagttcc    240
tcagctaaaa aagacagtct tggaaaaggg ctgtctcatt accaaaattg gattgggaca    300
gttaaaaaga taaaatcaca aaaagacagt cgtcaaaagc atcattatag ttatgaagtt    360
acatttgata atggcaaagc tttgaaatat gttcaggaaa aagacttagt taaaacaaaa    420
agatctaaat acagcaaagg tcaaattgtc aaattaaaat cctctgcgac agctgattta    480
gatggcagca gtttaacgga ttatcgtgca tcagctggta aaatcgatca tatttcttat    540
aatcatagca ataccacagg tggctataag tatgatatca cctttgatga aggcggcaag    600
gtgactaata ttcaagaaaa agatttggat aaggtttatg aagttcagct aaaatcagaa    660
aatactgcag ctcaaaataa tgagattctt aaacaagctt ttgcatatgc taaacaacat    720
tctggtacta tcttaagttt gccaaacggt gaatttaaga ttggcagtca gacaccagac    780
aaggattata tcactttaac atctgatact gaaattcgtg gagataatac gacacttttg    840
gttgaaggat cggcttattg gtttgctttt gcaactggga catcagctag tgatggtgta    900
aagaatttca ccatgcgcaa tatcaatata aaagccagtg atttggaaaa aggaaatcag    960
tttatgatta tggctgatca tggtgataat tggaaaattt gtaacaatag ttttaccatg   1020
gtgcataaaa aaggaagcca cattttgac ttaggttcct tgcaaaattc agcttttgaa   1080
ggaaatcaat ttactggcta tgcccctgaa ttaactaatg tcagtaagat tgatgataat   1140
gcagatcttc atgattttta ttcagaagtt atccagctgg atgccgctga gtctagcggt   1200
gtttgggatg gtgggttgat aaaggctatt gatccaaatt atgaaaatta taataaagag   1260
aaacagttgt gcaataacat cacgattgct aataactctt ttgttcctta tatagacagt   1320
catggtaaaa taatagcata cagcggaact atcggacagc actcttctga tgtcggcctt   1380
gtcaaaattt atgataatgt ttttagtaac tctttggtca gccgtttcaa tcaaaatggc   1440
aaaagcgagg cgtggatttt taaagctatt cacctaaaat caaattataa taatgctgtt   1500
tatgccaatt ctatcagtta a                                             1521
```

<212> Type : DNA
<211> Length : 1521
SequenceName : SEQ ID 736
SequenceDescription :
Sequence
--------
<213> OrganismName : Streptococcus mutans UA159
<400> PreSequenceString :

```
atgagaaaac ttaaagtggc actttttgca agcagtattt taggaatgct ggctgttagt      60
tcttatacgg cagcagatac agaggataat caggtaacga ttagccatta taatgaacag     120
gctggaactt ttgatgtcaa cgctgtacag gcagccaacg gaaaaactat tcaatcgata     180
gatgttgcga tttggtctga agaaaatggg caggatgatt tgaaatggta tcatgccagt     240
aatgatggca gcaatcaatt gacagttcat tttaatgctg agaatcatgg cagtaaggta     300
ggttcttata ttgcgcatgc ttatattacc tatacagatg gtaatcgagt cggggttaat     360

ttgggaaaac gaaaattatc cttatctgca ccgcaattat ccttaaaaca aggtggcctt     420
caactatttt ctaagctgaa acctagtgca gcggatcaac ttttttcagc agtttggtcg     480
gatgagaatg gtcaagatga tcttcattgg tacacggcag atgctgacgg gaatactttg     540
gctggctatg ctaatcataa aggttatgga acttaccatg ttcatactta ccttaagcaa     600
aatggtaaga tgataccaat tagtgctcaa gatattgata ttcctaaacc gaaagtcaag     660
attcagattg ataaaataaa tgataccagt tatgatgttg ttgttaataa tgtccccccct    720
tatattagtt cagtagccat tcctgtgtgg agtgaacaaa atggccaaga cgatttgaaa     780
tggtatcagg caacaaaagt ggctgatggt atatttaaaa caactgttta tttaaagaca     840
catcgttttg aattaggcaa ctatcaagct catatttatg gcgatagcca attaagcaag     900
aaactggatg gtttaggaga aactcatttt aatgttccgt ctattattaa ctatgaagat     960
cctcaggtaa ctattgatca ttataatatt aacaaaggaa cgtttgatgt gactgtagct    1020
gaaacagata attcaaaagc gatacaatca atcagtgctg ctgtttggtc tgatgctaac    1080
caagctaatc tttattggta tgaagctaaa cagctagcaa atggaaaggc tgcaattact    1140
gtcgatgttc aaaagcatgg caatcaaaca ggaagctaca atgtccatgt ttatgttcat    1200
tataatgatg gcacgactag cggacatgtt ttggctaatc agcagctcaa tcaaattgtc    1260
cattatcaac cttctgcagt aagaataaca gcctatatga atgaaaaaaa tacttatcca    1320
gttggtcagt gtacttgggg agtgaaagaa ttagctcctt ggatacctaa ttggcttggc    1380
aatggcgggc agtgggcaag tactgtagct gttaagggat tcaaaatagg aactgttcct    1440
aaagttggtg ctattgcttg ttggagtgat ggtggttatg gccatgttgc ttacgttacc    1500
cacgttgaga gtaataaccg tattcaagtg aaagaagcta attataagaa tcaacaatat    1560
atttccaatt ttcgcggatg gtttgatccc acgacttcct atttgggaag attaacttat    1620
atttatcctg actaa                                                     1635
```

<212> Type : DNA
<211> Length : 1635
SequenceName : SEQ ID 737 SequenceDescription :
Sequence
--------
<213> OrganismName : Streptococcus mutans UA159
<400> PreSequenceString :

```
atggcaaata actattctcg tcgtcaacaa cccactaaaa aaacaaaggg gacaagtcgg      60
aaacgtccga ctgaacatat caaaacaggt ttttcagcac tgcaaaagag tgttgctatt     120
atcgctggta ttttagggat tattaccgct ttgattacta ttaataatta tcgcaatagt     180
tcacacaatg ataaaaaaga ttccacatct aaaaccacta tcatcaaaga aaaagaagtg     240
gatgactcaa atagtaacaa caatgctgct aattctcaag ctgaaaatga cagcaataac     300
aataataatt ctgcagaatc aaatcaaaac caaactgcaa caacagcaaa tgacagtaac     360
agcaattcgg ctaatcaaaa tcaagccaat agccaatcac aagcaaataa tcagcaaaat     420
caaaacaatg ctaatgctgg tcaataa                                         447
```

<212> Type : DNA
<211> Length : 447
SequenceName : SEQ ID 738
SequenceDescription :
Sequence
--------
<213> OrganismName : Streptococcus mutans UA159
<400> PreSequenceString :

```
ttgaaaatat ttagttttgg aaccattcga aacaacacag ctctaaaacc taactatgat      60
gacacaacag cttttagcgg ttttggaacc attcgaaaca acacagctct aaaacagagc     120
actaactgcg ctagctggtt caatcgtttt ggaaccattc gaaacaacac agctctaaaa     180
ctcaccatat taattaatgg cgtttccttt tgttttggaa ccattcgaaa caacacagct     240
ctaaaacctc gtggaccaat ttttgtctcg acatttcgta atcgcgccat tcatctcagc     300
cagatttcag cttcaaaatg a                                               321
```

<212> Type : DNA
<211> Length : 321
SequenceName : SEQ ID 739
SequenceDescription :
Sequence
--------
<213> OrganismName : Streptococcus mutans UA159
<400> PreSequenceString :

```
atgaaaagaa aacgaaatct ttactttctt attggtttat ttttgacagt ctttcttttg      60
ataggatgct caatgcagaa aaaaaccaaa tcagaaagca gctcgacttc tcaaaagact     120
actttacaaa caaaacagtc aagtgaaaaa tcaactgatg ctaagcaaac gacagaagct     180
cattcagaaa gcagtcagtc ttcttctcat tctaataacg aggaaaccct tgctcccatt     240
```

```
gatacaggcg ctgttttaaa ggctgattac agtagtatgg caggaacttg gaaaaatgaa     300
gaaggacaaa cgttgacatt tgatcagcga ggtctgacaa cccctggaat gacagtcagt     360
ctgttgaaca ttgatcaaga cggaaatctt ttgttaaatg ttgagactgg aacaaaaaag     420
aatctaactc tttatattgt gccagccaat aaaaccttat ctaatcaata tttttctaat     480
ggtcaaagcg atgaatccga taaaacaaaa gatcgtattg tttcttctga gagtttaaat     540
agtggcaaat ttacaaaccg agtttattat catgtttcaa ctcattaa                 588
```

<212> Type : DNA
<211> Length : 588
SequenceName : SEQ ID 740
SequenceDescription :
Sequence
--------
<213> OrganismName : Streptococcus mutans UA159
<400> PreSequenceString :

```
atgacaccta aaaaaatcaa aatagctcta acagctctta tctctttgat gctcgcctta      60
tttttattct tgtttaatca ccattcagta agagaaaaca gtcagcaaga aaagttaaag     120
ataagtaaag caagttctaa aaaatcacaa acaagcactt cttctgttat gacaagtagt     180
cgaaaagcta ctgaacaaac aagccaagca cagactcaaa gtcaatcaca agcagaacaa     240
agtaacccta atgtgatcct ccccattccg caagaattag tcggcaccta caaaggttcg     300
agtccacaag catctgaaat aacttttacc atttcttcaa atggtcaatt acgtgctcaa     360
gctaattttg atcctgcttc tgatataaat gacgttaccg ccactgttag tggtgttaga     420
aaagtcgggg cagataccta tatttgggag tttgtctctg gtagttcagc tgctctttta     480
ccgggtgtta caggtatagg agggcttgga aagatgcagc ctggtttcat cctaaaaggg     540
gggcaattaa cacctatcat gtttacaggt tctgtagatg gtgaaattga ttattcacat     600
cccaatccct atccagtatc attaaacaag cagtaa                             636
```

<212> Type : DNA
<211> Length : 636
SequenceName : SEQ ID 741
SequenceDescription :
Sequence
--------
<213> OrganismName : Streptococcus mutans UA159
<400> PreSequenceString :

```
atgaaaaaaa taattaatgt tatcgtctta tcactatccg tttttttcct gatagcttgc      60
agcaatagca gtaccgggga aaaaacaagt caatcatctg aagagactaa ggtccgatta     120
attgttaaaa cggattctaa taaaaccgat gaaaaagtcg ctttcaaaaa aggtgctact     180
gttatggatg tcttaaaaga caactataaa gttaaagaaa gcggtggttt tatcactact     240
attgatggtg tcactcagga taaaaaagca ggtaggtact ggatgtttga tgttaatgat     300
aagctggcat caaaagctgc tgataagatt aaggtcaaaa atggggataa aattgaattt     360
tatttgaaag tttataaagg taagaactag                                     390
```

<212> Type : DNA

<211> Length : 390
SequenceName : SEQ ID 742
SequenceDescription :
Sequence
--------
<213> OrganismName : Streptococcus mutans UA159
<400> PreSequenceString :

```
atgtcaaata aaccatggga agaaaaagta actgatgcaa ctactgataa tgaagaaatg      60
acaagaaatt caaaggatgc tagtattatc agtacaccta ttttaacaat cctattgagc     120
ctctttttct tgattattat tggtatttta ttttttgtac tttatacttc aaatggtgga     180
agcaatgaaa aagcagccac ttcgggtttc tatagttctt ccaaaacggt caaaaaagcc     240
aaaaatgagg caaatagtca aactgatgaa cagacaacag aagcagaaac aagttcaagt     300
gaaacgacaa gttcctcttc agacagtgat ggcgagacaa ttacagttca aggaggtgaa     360
ggggctgcag caattgctgc gcgtgcgggt atttctgttg ataaactcta tgagctgaat     420
ccagaacata tgacgcatgg ctattggtat gctaaccctg gagataacat caagattaag     480
taa                                                                   483
```

<212> Type : DNA
<211> Length : 483
SequenceName : SEQ ID 743 SequenceDescription :
Sequence
--------
<213> OrganismName : Streptococcus mutans UA159
<400> PreSequenceString :

```
atgccagata atcgcatgaa ctatagtatt gatagcaata tgcagtttcc cttggtagaa      60
attactttgg aaacaggaga atttgcttat attcaacgcg gtagcatggt ctatcacaca     120
cccagtgtca ctctcaatac caaagtcaat ggacgtggtt caggacttgg caagctagta     180
ggagcaattg gtcgttctgt aacgtctgga gaaagttttt tcattactca ggcagtatca     240
aatgctagcg atggtaaatt ggccttggcc ccttctatgc cgggccaagt tattgcttta     300
gaattgggag aaaaacaata tcgcctcaat gatggtgctt ttcttgcctt agatggttct     360
gctcaatatc aaatgaaagc tcagagtgtt ggacgtgccc tttttggcgg tcaaggcggt     420
ctttttgtta tgacaacaga aggtcaaggt accttgcttg ctaatagttt tggttctatc     480
aaaaaaatag aattacagaa tcaggaaatt acaattgaca atgctcatgt tgtagcttgg     540
agtagggatt tgaactatga cattcatttg gaaaatggct ttatgcaatc gatcggaacc     600
ggtgaaggcg ttgtcaatac tttccgagga acgggtgaaa tttatgtaca aagtcttaat     660
ctgcagcagt ttgctggtgt cctacagggt ttcattacca atactaatcg ttaa           714
```

<212> Type : DNA
<211> Length : 714
SequenceName : SEQ ID 744
SequenceDescription :
Sequence
--------
<213> OrganismName : Streptococcus mutans UA159
<400> PreSequenceString :

```
atgaaaaaaa attatttttg gtacggtctg cttgggcttc tcgcacttta ccttattact      60
attgctttta tcccgggatt tcatattttc tttagcaaca tgttgatgtt ggccctgttc     120
tttatgttaa tagctttgag taacaggagt atcttctttt tctttctagc cttaggtttt     180
cttagtatct acttgaaaga tatctttcat tttgactatt ctaccggacc gctttttacg     240
ggtataatca ttatcggcgt tattttaaac agtttcctca aaccacacta ttcttattct     300
tataaaggaa atcattattt taatatgaaa caacatgcta actacattga taacgaaaca     360
gatgtctttt taaaaacact tttttctgaa aataccagtt atgtgacttc tcaagaatta     420
aataaaatta ttattgatac taagtttgga gaacaatctg ttgatctctc tcaagctcaa     480
tttatgacag attctcccga aattcatata gatgttagct ttggtgaaac caatctgcgt     540
attccaaaca actggaaaat catcaataaa actcactccc cctttgcttc catttcattt     600
tcaggttttc ctagcacaaa tggtgatttt attaacgtta cattgactgg aacagtggct     660
atgggatctc taaacattca atattaa                                         687
```

<212> Type : DNA

<211> Length : 687
SequenceName : SEQ ID 745
SequenceDescription :
Sequence
--------
<213> OrganismName : Streptococcus pneumoniae R6
<400> PreSequenceString :

```
atgaaatcaa taactaaaaa gattaaagca actcttgcag gagtagctgc cttgtttgca      60
gtatttgctc catcatttgt atctgctcaa gaatcatcaa cttacactgt taaagaaggt     120
gatacacttt cagaaatcgc tgaaactcac aacacaacag ttgaaaaatt ggcagaaaac     180
aaccacattg ataacattca tttgatttat gttgatcaag agttggttat cgatggccct     240
gtagcgcctg ttgcaacacc agcgccagct acttatgcgg caccagccgc tcaagatgaa     300
actgtttcag ctccagtagc agaaactcca gtagtaagtg aaacagttgt ttcaactgta     360
agcggatctg aagcagaagc caaagaatgg atcgctcaaa aagaatcagg tggtagctat     420
acagctacaa atggacgtta tatcggacgt tacggttcat ggactgctgc taaaaacttc     480
tggcttaaca atggctggta ttaa                                            504
```

<212> Type : DNA
<211> Length : 504
SequenceName : SEQ ID 746
SequenceDescription :
Sequence
--------
<213> OrganismName : Streptococcus pneumoniae R6
<400> PreSequenceString :

```
atgaaacatt cacataaaaa atcatttgac tggtatagca tgcaacaacg ttattctatt      60
cgtaagtatt actttggtgc agctagtgtc ttgctcggta ccgctttggt attaggtgca     120
gcagctagtg tccaaacagt acaagcggaa gaaaacaaac aagaaactac caatagtatt     180
tctgttggta ggggagaagc agctactaaa ccagcagagg tttctgcgtc taataaagag     240
```

aaaacctatg cagctccaac tgtagctaat ccagtagaaa cgactccagt taaaactgaa 300
gaagttacta aaccagcaga aaaagttgaa gaagcaaaag acaaaaaaga ggaagtaacg 360
catcaagatg ccgttgacaa gtcaaaatta ttaacggctc tttcgcgtgc taaaaaatta 420
gaaagcaagt tatatacaga agcaagtgct gctaacttgc aaacaagtat ccaagctggt 480
caaagcttgc ttggaaaagc agatgcaact gaagctgaat tatcagcagc agagtcatct 540
attcaatcat ttattattgg tctagaactt cgttctaact ctaataaaga aactgtatca 600
gaaacgcctg tagcgaagaa agctgatgca gttgaatcaa aagaaggggc taaaccagct 660
gcaacaactg aacgttcagc tgttgatagc gctattttgc caactagcac agctgacaaa 720
gtagaaacaa cttcagctcc agcatctatt aatgaaatct tgaaactagg tttgagcctt 780
tctgatgctc gccaaaatcc agctatccgt aaggaagatg ttaatagagg gtatagtggt 840
tttagagcgg ctagcaatcc agccaaccca attgtctcag gttctggaaa tacagttgca 900
tttgcagata ttagccaagg tggtcgtagc tatagtttcc gtggctacgg gaactcacgt 960
ggtggaaatt ctattcatta cgatgtaaca acagtacgta gtggtaatag tgttaacttt 1020
acaattagtt attctgcgcc aggagattct agagagtttg ttaataataa ttttatcttg 1080
gataaagggg atggatttgg aaatccttca aatgcaacga tcacaagttc aaatccaaga 1140
gtaagggagc aatcaaaatc tattagtcag ggtgccaact acgtatctca ctcaggatac 1200
agtatgacct ctgctatttc aacaaatacg gaacaaacta tcagatttag tttgcctatc 1260
attaatctaa atggtgattt gtctgttcgt ttgaaacctg ttacttttaa cgtggaccaa 1320
ggtggtggtg gtgccgctac tagcaatgac ccatacagta actctaacta ttattataga 1380
gcaaacccac tatacttgga tgcaaaccct tatggtggta ctaataataa gactgtttca 1440
gaagacattg acttccaaac tgtctatctt ccaactagta agttaccaga aggtcaaact 1500
agattagttc gagaaggtga aaaaggacaa cgtcaaatta cctataaagt tcatcgattt 1560
ggtaacgaaa cacttttagg attgccgatt agtaatagtg ttactaaaga agctaagcca 1620
cgtattatgc aaattggtgt ggctaaagat ctaatcgata cagtaaaacc acgtgttgat 1680
caaaataaag tcggtgatac aaataacctc actttctatc ttgataacga tggaaacggt 1740
gtttatactg aaggtgtaga cgaacttgtt caaaaaattg ctattaaaga tggagctaaa 1800
ggtgaaaaag gagaccaagg tgaacgcggt ctaactggag ctaaaggtga aaaaggagac 1860
cgaggcgaac gcggtctaac tggagctcaa ggagctaaag gtgaaaaagg agaccgaggt 1920
gaacgcggtc taactggagc tcaaggagct aaaggtgaaa aaggagaccg aggtgaacgc 1980
ggtctaaccg gagctcaagg agctaaaggt gaaaaaggag accgaggcga acgcggtcta 2040
actggagctc aaggagctaa aggtgaaaaa ggagcccaag gtgaacgcgg tctaaccgga 2100
gcacaaggag ctaaaggtga aaaaggggac caaggtgaac gcggtctaac cggagcacaa 2160
ggtgccaaag gtgaaaaagg ggaccaaggt gaacgcggtc taaccggagc acaaggagct 2220
aaaggtgaaa aaggagccca aggtgaacgc ggtctaactg gaactcaagg agctaaaggt 2280
gaaaaaggag accgaggcga acgcggtctg actggagccc aaggagctaa aggtgaaaaa 2340
ggagaccgag gcgaacgcgg tctgactgga gcccaaggtg ctaaaggtga aaaaggagcc 2400
caaggtgaac gcggtctaac cggagcccaa ggtgctaaag gtgaaaaagg ggaccaaggc 2460
gaacgcggtc taactggagc acaaggtgaa aaaggagacc gaggcgaacg cggtttaact 2520
ggagctaaag gtgaaaaagg ggaccaaggc gaacgtggta tcactggagc taaaggtgaa 2580
aaaggagccc aaggtgaacg cggtctaacc ggagcccaag gtgctaaagg tgaaaaaggg 2640
gaccaaggtg aacgcggtct cactggagcc caaggtgaaa aaggagccca aggtcaggca 2700
ggtcgtgacg gtgtaactcc aaccgtaacc gttaaagata ataaaaatga cggcactcat 2760
actatcacta ttaacgacgg tagaggtaat gttacaagta ctgttgtaag agatggtttc 2820
gatggcgcaa gtccattagt tgcgactcaa cgaaatgacg cagataagac aacaactgtt 2880
atcttctatt acgataaaaa tggaaacaat gaattagatg cttctgataa gaaattaaaa 2940
gaagttgtaa ttgcagatgg tgctaaaggt gaaaaagggg acaaaggtga acaaggtctt 3000
caagggcgtg atggtgaaca aggaccaaaa ggagaagatg ggaaaacccc aacagttaaa 3060
gtaactgatg gtcaagatgg aacgcataca attacaatta acgatggtaa aggtggtata 3120
actactacag tagtaagaga tggatttgat ggtgcaagcc ctcttgtttc tactcataga 3180
aatgaagcag ataaaacaac aactgttatt ttctattatg atctaaatga taataatcaa 3240
tttgatgaag gagatacaaa acttaaagaa gttgttatcg cagatggaaa acaaggacca 3300
aaaggtgaca aaggtgataa cggaaaagat ggattcacac cagaagtaac agttacagat 3360
aacaataacg gaacacacac catcacaatc acacaaccag acaacagacc atcattaaca 3420
acaatcgtta aaaacggtga agatggaaaa acaccaaaag tcaaagcaga acgagatgat 3480
gcgaagaaac aaacaacatt aacattctac attgacaaag atggagatgg aagttacaca 3540
gcaggaaaag acgagttagt tcaaacaaca gtagttaaag acggacaaga cggagctgca 3600
ggagcatctg gacgtgatgg aaaagaagta ttaaacggaa aagtagaccc aacaacagaa 3660
ggtaaagatg gagacacatt cgtaaataca caaacaggag atgtattcgt taagaaaggt 3720
aacacttggg aaccagcagg aaacatcaaa ggaccgaaag gtgacaaagg tgcagatggt 3780
gccaagggtg aaaaaggagc ccaaggagaa cgcggcctga ctggagcgca aggtgtcaag 3840
ggtgaaaagg gagatcaggg tgaacgcggt ctaactggat ctaaaggtga aaaaggagac 3900
caaggtgaac gcggtttaac tggagcgcaa ggtgccaaag gtgacaaagg tgaacaaggt 3960
cttcaaggtc gtgatggagc tcaaggacca aaaggagcag atggacaaag aggaccagcg 4020
ggaccacaag gaccaaaagg agaacaaggt aatccaggaa ctccaggtaa agatggaaaa 4080
tctctaattg ctgttaaaaa tggtgtatta gtaacaatta caccagtaga aggtcgtcca 4140
caaacaacat ttgtagagga tggacaaaag ggtgctgatg ggaaaactcc aacagtaaca 4200
ataactgagg ggcaaaacgg cacacataca ttaacggttc ataatccagg aagtccagat 4260

```
gtgacaacta cgatccgtga tggagctaca ggacaagcag gtcgtgatgg taaagatgta    4320
ttaaacggaa aagtaaatcc acaaccaaac caaggtaaaa atggagataa atatattaat    4380
atcgaaaccg gtgatgtcta tgttaaaaac aatggaaact gggataaaga aggcaacatc    4440
aaaggcccta aaggtgacaa aggtgcagat ggtgctaaag gcgaaaaagg agaccaaggc    4500
gaacgcggcc taactggagc gcaaggagct aaaggtgcgg atggcgcagt aggtcgtgat    4560
ggacgtgacg gtaaagacgt gttgaacggc aaagctaacc cagaagcaca tcaaggtaaa    4620
gacggcgata aatacgttaa tacagaaaca ggcgacgtct tcgttaagaa taacggcaac    4680
tgggataaag agggcaacat caaaggccct aaaggtgaca aaggtgcaga tggtgctaaa    4740
ggcgaaaaag gagaccgagg cgaacgcggc ctgactggag cgcaaggagc taaaggtgcg    4800
gatggagcag caggtcgtga cggacgtgat ggacgtgacg gtaaagacgt gttgaacggc    4860
aaagttaacc cagaagcaaa tcaaggtaaa gacggcgata aatacgttaa tacagaaaca    4920
ggcgacgtct tcgttaagaa taacggcaac tgggataaag agggcaacat caaaggctct    4980
aagggtgaca aaggtgaacg cggagaagat ggtaagactc cagaagtaac tgtaactcca    5040
ggtaaagatg gccatagtac tgacattaca ttcactgttc caggtaaaga tccagttaca    5100
gtaaatgtta aggacggaga aaatggtctg aacggtaaaa ctccaaaagt tgatttactt    5160
cgtgtccaag gaaaaaacgg aaatccatct catacaattg tgacattcta tacagatgaa    5220
aacaatgacg gcaaatatac accaggaact gatgaacttc taggttcaga aatgattaaa    5280
gatggtgcta aaggcgcgga cggacgagat ggtaaatcat tgcttactgt caaggatggt    5340
aaagaaacta aagtttacca agaagatcca gctaacccag gacaaccatt aaatccagaa    5400
aaaccacttg cggtaattag agatggagta gatggaaaat cacctacagt tacagctgtt    5460
cgtaaagatg aagcagggca taaaggtgta gaaatcactg ttgataacca tgatggttca    5520
caaccaacta cagtctttgt tcaagatggt gctaagggaa aaactggtgc aaccggtcag    5580
gatggacaaa ctcctacaat cactactcaa cgtggacaag atggccaaag cactgttgta    5640
actatcacaa catcaggtaa agatccagta accttcactg taaaagatgg taagaatggt    5700
aaagatggcc gtgcaccgaa aatcaaagta gaagatatta cttcaccttc aagaattaga    5760
cgcgatacag atgctgctgc aactccaacg cgtaacggta tccgtgttac agtttatgat    5820
gatgttaatg acaatggggt atacgacgaa ggtgtcgata aagtattaaa tagtaaagat    5880
atttataacg gtatagatgg acgtgatggt tcagctccaa ctattactac aaaagataat    5940
ggagatggaa ctcacactat cacagttcaa aacccagatg gttctgaatc aacaacagtt    6000
gttaaagatg gtaaagacgg taaaactgcg aatatcacta caacagaaaa cccagatgga    6060
agccacacaa ttacggtgac aaatccagat ggttcaacta aagaaactgt tgttaaaaac    6120
ggtaaagacg gtaagactcc taaagttgaa gtaacggata acaacgatgg aactcatact    6180
gttaaagtta cagatggaga cggcaatgtt accaacgcta tcatcaaaga tggtaaagac    6240
ggtaaagctg caacagcaac aactactgaa aatccagatg gaagccacac agtaacaatc    6300
actaacccag acggaactaa gaatgagttt gttgttaaga atggacgtga cggtgttgac    6360
ggacgtactc caaccgcatc tgttcgtgat aatggagacg gaagtcatac aatcgttatt    6420
acaaatccag aaggtgtgac aactgaaacc acagttcgtg atggtaaatc accaaaagtg    6480
actataactg atgaacaaaa tggaactcat aagatctctg ttctaaatgg tgacggaaca    6540
actactgaaa caatcattaa agatggtaaa tcaccagtag caacagttag agataaccaa    6600
gatggtactt acactattcg tgtggaaaac ggtaatggta ctgtttctga aaccacagtt    6660
cgtgacggta aatcaccaac tgctaaggtt gtggataatg gagatggaac tcacactatc    6720
acagttgtga actcagacgg aataactaca acaactacag ttcgtgatgg tagagaacca    6780
aaacttgaag ttattgataa caacgatggt tcacacacta ttaaagtgac aggtgctgat    6840
ggtaaaggaa cgacaactac aatctttgat ggtaaatcac caaaagcgaa catcgttgat    6900
aacggagatg gaactcatac attaacaatc gtagattctg atggtcgtga atacaaatct    6960
attatcaaag atggtaaaga cggcaaagat agcgtttcac caactgtaac tgttaaaaat    7020
aataacgatg gaactcacgt tgttacaatc actaatccag atggaagtaa gacagaaatg    7080
gtgattaaag acggtaaaga tggtaaatca ccaaaagttt ctgttgaaga taatggtgat    7140
ggtagtcata caatcacaat catcaattct gatggaactg tgacaaaaac agttattaaa    7200
gatggcaaag atggtagaga tggacgtgat ggtcgagacg gcaaagacgg taaagatgga    7260
aaatgtggat gccaagacaa accagtaaca ccatcaaatg acaaaccagt tcctccaaca    7320
ccaaatgtgc cgacaccaga agtaccggtt aaacctgtgc cagcgcaacc aacaccaaat    7380
gtaccgacac cagaagtgcc agtacaacca actccagctg tttcaacacc agaagtaccg    7440
gttaaaccag taccagcggt tccagaacaa ccagtagtac caacaccggc tcaaccagca    7500
actccagtaa atgctaaccc agtagcacca actacaggta aagaaaaccg tggggacaaa    7560
ttacctgaaa ctggaagcca atctgattat atctctgttc ttttaggtag cggtattcta    7620
ttgagcctat atgtaggacg aagaaaagaa gattaa                              7656
```

<212> Type : DNA
<211> Length : 7656
SequenceName : SEQ ID 747
SequenceDescription :
Sequence
--------
<213> OrganismName : Streptococcus pneumoniae R6
<400> PreSequenceString :

```
atgaagaaaa gaatgttatt agcgtcaaca gtagccttgt catttgcccc agtattggca      60
actcaagcag aagaagttct ttggactgca cgtagtgttg agcaaatcca aaacgatttg     120
actaaaacgg acaacaaaac aagttatacc gtacagtatg gtgatacttt gagcaccatt     180
gcagaagcct tgggtgtaga tgtcacagtg cttgcgaatc tgaacaaaat cactaatatg     240
gacttgattt tcccagaaac tgttttgaca acgactgtca atgaagcaga agaagtaaca     300
gaagttgaaa tccaaacacc tcaagcagac tctagtgaag aagtgacaac tgcgacagca     360
gatttgacca ctaatcaagt gaccgttgat gatcaaactg ttcaggttgc agacctttct     420
caaccaattg cagaagctcc aaaagaagta gcatcaagtt cagaagttac aaagacagtg     480
attgcttctg aagaagtggc accatctacg ggcacttctg tcccagagga gcaaacggcc     540
gaaacaagca gtgcagttgc agaagaagct cctcaggaaa cgactccagc tgagaagcag     600
gaaacacaaa caagccctca agctgcatca gcagtggaag caactacaac aagttcagaa     660
gcaaaagaag tagcatcatc aaatggagct acagcagcag tttctactta tcaaccagaa     720
gaaacgaaaa taatttcaac aacttacgag gcaccagctg cgccccgatta tgctggactt     780
gcagtagcaa aatctgaaaa tgcaggtctt caaccacaaa cagctgcctt taaagaagaa     840
attgctaact tgtttggcat tacatccttt agtggttatc gtccaggaga cagtggagat     900
cacggaaaag gtttggctat cgattttatg gtaccagaac gttcagaatt aggggataag     960
attgcggaat atgctattca aaatatggcc agccgtggca ttagttacat catctggaaa    1020
caacgtttct atgctccatt cgatagcaaa tatgggccag ctaacacttg gaacccaatg    1080
ccagaccgtg gtagtgtgac agaaaatcac tatgatcacg ttcacgtttc aatgaatgga    1140
taa                                                                  1143
```

<212> Type : DNA
<211> Length : 1143
SequenceName : SEQ ID 748
SequenceDescription :
Sequence
--------
<213> OrganismName : Streptococcus pneumoniae R6
<400> PreSequenceString :

```
gtgacgattc taggaaaaga tacagttcaa caatctgcga aaggtgaatc tgtaactcaa      60
gaagctacac cagagtataa gctagaaaat acaccaggtg gagataaggg aggcaatact     120
ggaagctcag atgctaatgc gaatgaaggc ggtggtagcc aggcgggtgg atcagctcac     180
acaggttcac aaaactcagc tcaatcacaa gcttctaagc aattagctac tgaaaaagaa     240
tcagctaaaa atgccattga aaaagcagcc aagaacaagc aggatgaaat caaaggcgca     300
ccgctttctg ataaagaaaa agcagaactt ttagcaagag tggaagcaga aaaacaagca     360
gctctcaaag agattgaaaa tgcgaaaact atggaagatg tgaaggaagc agaaacgatt     420
ggagtgcaag ccattgccat ggttacagtt cctaagagac cagtggctcc taatgctgct     480
cctaagacaa caagtgcacc gcaagcaact gcaggaacaa tgcaagatgt tacctaccag     540
tcacctgctg gcaaacaatt acctaacaca ggttcagcat caagtgcagc acttgctagt     600
cttggtctag tggtggcaac aagtggtttt gctttgctag gaagaaagac tagacgtaga     660
aaatag                                                               666
```

<212> Type : DNA
<211> Length : 666
SequenceName : SEQ ID 749
SequenceDescription :
Sequence
--------
<213> OrganismName : Streptococcus pneumoniae R6
<400> PreSequenceString :

```
atgatgacga caggttgctc tatgggagcc tatcatgcac tcaatttctt cctccagcat      60
ccagatgtct ttaccaaagt gattgctctc agtggtgttt acgacgcacg tttctttgtc     120
ggtgattact acaacgatga tgctatttac caaaactcgc cagtagatta tatttggaac     180
caaaacgacg gctggtttat tgaccgttac cgtcaggcag agattgtgct gtgtacgggg     240
cttggagcct gggaacaaga tggtttgcca tccttttaca agctcaaaga agcctttgac     300
aagaaacaaa ttccagcctg gtttgctgaa tggggacatg atgtcgccca tgactgggaa     360
tggtggcgta aacaaatgcc ttatttcctc ggtaatctct atttataa                 408
```

<212> Type : DNA
<211> Length : 408

SequenceName : SEQ ID 750
SequenceDescription :
Sequence
--------
<213> OrganismName : Streptococcus pneumoniae R6
<400> PreSequenceString :

atgaataaag gattatttga aaaacgttgt aaatatagta ttcggaaatt ttcattaggt 60
gttgcttctg ttatgattgg agctacattc tttgggacaa gtccggttct tgcagatagc 120

gtgcagtctg gttccacggc gaacttacca gctgatttag ctactgctct tgcaacagca 180
aaagagaatg atgggcatga ttttgaagcg cctaaggtgg gagaagacca aggttctcca 240
gaagttacag atggacctaa gacagaagaa gaactattag cacttgaaaa agaaaaaccg 300
gctgaagaaa aaccaaaaga ggataaacct gcagctgcta aacctgaaac acctaagacg 360
gtaacccctg aatggcaaac ggtagagaaa aaagaacaac agggaacagt cactatccga 420
gaagaaaaag gtgtccgcta caaccaatta tcctcaactg ctcaaaatga taacgcaggt 480
aaaccagccc tgtttgaaaa gaagggcttg accgttgatg ccaatggaaa tgcaactgtt 540
gatttaacct tcaaagatga ttctgaaaag ggcaaatcac gctttggtgt cttcttgaaa 600
tttaaagata ccaagaataa tgtttttgtc ggttacgaca aggatggctg gttctgggag 660
tataaatctc caacaactag cacttggtat agaggtagtc gtgttgctgc tcctgaaaca 720
ggatcaacaa accgtctctc tatcactctc aagtcagacg gtcagctaaa tgccagcaat 780
aacgatgtca atctctttga cacagtgact ctaccagctg cggtcaatga ccatcttaaa 840
aatgagaaga agattcttct caaggcgggc tcttatgacg atgagcgaac agttgttagc 900
gttaaaacgg ataaccaaga gggggtaaaa acagaggata cccctgctga aaaagaaaca 960
ggtcctgaag ttgatgatag caaggtgact tatgacacga ttcagtctaa ggttctcaaa 1020
gcagtgattg accaagcctt ccctcgtgtc aaggaataca gcttgaatgg acatactttg 1080
ccaggacagg ttcaacagtt caaccaagtc tttatcaata accaccgaat cacccctgaa 1140
gtcacttata agaaaatcaa tgagacaaca gcagagtact tgatgaagct tcgcgatgat 1200
gctcacttaa tcaatgcgga aatgacagta cgcttgcaag ttgtggacaa tcaattgcac 1260
tttgatgtga ccaagattgt caaccacaat caagtcactc caggtcaaaa gattgatgac 1320
gaaagaaaac tactttcttc tattagtttc ctcggcaatg ctttagtctc tgtttctagt 1380
gatcaaactg gtgctaagtt tgatggggca accatgtcaa acaatacgca tgtcagcgga 1440
gatgatcata tcgatgtaac caatccaatg aaagatctag ccaagggtta catgtatgga 1500
tttgtttcta cagataagct tgctgctggt gtttggagta actctcaaaa cagctatggt 1560
ggtggttcga atgactggac tcgtttgaca gcctataaag aaacagtcgg aaatgccaac 1620
tatgtaggaa tccacagctc tgaatggcaa tgggaaaaag cttataaggg cattgttttc 1680
ccagaataca cgaaggaact tccaagtgct aaggttgtta tcactgaaga tgccaatgca 1740
gacaagaaag tcgattggca ggatggtgcc attgcttatc gtagcattat gaacaatcct 1800
caaggttgga aaaaagttaa ggatatcaca gcttaccgta tcgcgatgaa ctttggttct 1860
caagcacaaa acccattcct tatgaccttg gatggtatca agaaaatcaa tctccacaca 1920
gatggtcttg ggcaaggtgt tctccttaaa ggatatggta gcgaaggcca tgactctggt 1980
cacttgaact atgctgatat tggtaagcgt atcggtggtg tcgaagactt caagacccta 2040
attgagaagg ctaagaaata tggagctcat ctaggtatcc acgttaacgc ttcagaaact 2100
tatcctgagt ctaaatactt caatgaaaaa attctccgta agaatccaga tggaagctat 2160
agctatggtt ggaactggct agatcaaggt atcaacattg atgctgccta tgacctagct 2220
catggtcgtt tggcacgttg ggaagatttg aagaaaaaac ttggtgacgg tctcgacttt 2280
atctatgtgg acgtttgggg taatggtcaa tcaggtgata acggtgcctg ggctacccac 2340
gttcttgcta aagaaattaa caaacaaggc tggcgctttg cgatcgagtg gggccatggt 2400
ggtgagtacg actctacctt ccatcactgg gcagctgact tgacctacgg tggctacacc 2460
aataaaggta tcaacagtgc catcacccgc tttatacgta accaccaaaa agatgcttgg 2520
gtaggggact acagaagtta tggtggtgca gccaactatc cactgctagg tggctacagc 2580
atgaaagact ttgaaggctg gcaaggaaga agtgactaca atggctatgt aactaactta 2640
tttgcccatg acgtcatgac caagtacttc caacacttca ctgtaagtaa atgggaaaat 2700
ggtacaccgg tgactatgac cgataacggt agcacctata aatggactcc agaaatgcga 2760
gtggaattgg tagatgctga caataataaa gtagttgtaa ctcgtaagtc aaatgatgtc 2820
aatagtccac aatatcgcga acgtacagta actctcaacg gacgtgtcat ccaagatggt 2880
tcagcttact tgactccttg gaactgggat gcaaatggta agaaactttc tactgataag 2940
gaaaagatgt actacttcaa tacgcaggcc ggtgcaacaa cttggaccct tccaagcgat 3000
tgggcaaaga gcaaggttta cctttacaag ctaactgacc aaggtaagac agaagagcaa 3060
gaactaactg taaaagatgg taaaattacc ctagatcttc tagcaaatca accatacgtt 3120
ctctatcgtt cgaaacaaac caatcctgaa atgtcatgga gtgaaggcat gcacatctat 3180
gaccaaggat ttaacagtgg taccttgaaa cattggacca tttcaggcga tgcttctaag 3240
gcagaaattg tcaagtctca aggggcaaac gatatgcttc gtattcaagg aaacaaagaa 3300
aaagttagtc tcactcagaa attaactggc ttgaaaccaa ataccaagta tgccgtttat 3360
gtcggtgtcg ataaccgtag taatgccaag gcgagcatca ctgtaaatac tggtgaaaaa 3420
gaagtgacta cttataccaa taagtctctc gccctcaact atgtaaaagc ctatgcccac 3480
aatacacgtc gtaacaatgc tacagttgac gatacaagtt acttccaaaa catgtacgcc 3540
ttctttacaa ctggatcgga cgtatcaaat gttactctga cattgagtcg tgaagctggt 3600
gatgaagcaa cttactttga tgaaattcgt acctttgaaa acaattcaag catgtacgga 3660
gacaagcatg atacaggtaa aggcaccttc aagcaagact ttgaaaatgt tgctcagggt 3720
atcttcccat ttgtagtggg tggtgtcgaa ggtgtcgaag acaaccgcac tcacttgtct 3780
gaaaaacacg atccatatac acaacgtggt tggaatggta agaaagtcga tgatgttatc 3840
gaaggaaatt ggtcactcaa gacaaatgga ctagtgagcc gtcgtaactt ggtttaccaa 3900
actattccgc aaaacttccg ttttgaagca ggtaagacct accgtgtaac ctttgaatac 3960
gaagcaggtt cagacaatac ctatgctttt gtagtcggta agggagaatt ccagtcaggt 4020
cgtcgtggta ctcaagcaag caacttggaa atgcatgaat tgccaaatac ttggacagat 4080
tctaagaaag ccaagaaggc aaccttcctc gtgacaggtg cagaaacagg ggatacttgg 4140

```
gtaggtatct actcaactgg aaatgcaagt aatactcgtg gtgattctgg tggaaatgcc 4200
aacttccgtg gttataacga cttcatgatg gataatcttc aaatcgaaga aattacccta 4260
acaggtaaga tgttgacaga aaatgctctg aagaactact tgccaacggt tgccatgact 4320
aactacacca aagagtctat ggatgctttg aaagaggcgg tctttaacct cagtcaggcc 4380
gatgatgata tcagtgtgga agaagcgcgt gcagagattg ccaagattga agccttgaag 4440
aatgctttgg ttcagaagaa aacggctttg gtagcagatg actttgcaag tcttacagct 4500
cctgctcagg ctcaagaagg tcttgcaaat gcctttgatg gaaacttatc tagtttatgg 4560
catacatcat ggggcggagg agatgtaggc aagcctgcaa ccatggtctt gaaagaagca 4620
actgaaatca ctggacttcg ttatgttcca cgtggatcag gttcaaatgg taacttgcgt 4680
gatgtgaaac ttgttgtgac agatgagtct ggcaaggagc ataccttac tgcaactgat 4740
tggccagata acaataagcc aaaagacatt gattttggta agacaattaa ggctaagaaa 4800
attgtcctta caggtactaa gacttacgga gatggtggcg ataaatacca atctgcagcg 4860
gaactcatct ttactcgtcc acaggtagca gaaacacctc ttgacttgtc aggctatgaa 4920
gcagctttgg ctaaggctca gaaattaaca gacaaagaca atcaagagga agtagctagc 4980
gttcaggcaa gcatgaaata tgcgacggat aaccatctct tgacggaaag aatggtggaa 5040
tactttgcag attatctcaa ccaattaaaa gattctgcta cgaaaccaga tgctccaact 5100
gtagagaaac ctgagtttaa acttagctct gtagcttccg atcaaggtaa gacgccagat 5160
tataagcaag aaatagctag accagaaaca cctgaacaaa tcttgccagc aacaggtgag 5220
agtcaatttg acacagccct cttcctagca agtgttagcc tagccctatc tgctctcttt 5280
gtagtaaaaa cgaagaaaga ctag 5304
```

<212> Type : DNA
<211> Length : 5304
SequenceName : SEQ ID 751
SequenceDescription :
Sequence
--------
<213> OrganismName : Streptococcus pneumoniae R6
<400> PreSequenceString :

```
atgaaattat ataataaatc agaattacgt tattctcgca ttttctttga taagagacca 60
ccagcttttg cgtttattct cattatttca actgctatta tcttaagcgg tgcattggtt 120
ggcgcagctt atatacccaa aaactatatt gtaaaagcga atggaaattc agtcataaca 180
ggtacagagt tcctatcagc aattagttca gggaaggtag ttactttaca taagtcagaa 240
ggagatatgg taaatgctgg agatgtcatt atttcgttat caagtggaca agaaggttta 300
caagcgagct ctttaaataa acaattggtg aagttacgtg caaaagaagc tatctttcaa 360
aaatttgaac aatcattaaa tgagaaatac aaccgtatgt ctaattctgg tgaagaacag 420
gaatattatg ggaaagttga atactaccta tctcagttaa attcggaaaa ttataataat 480
ggtacccagt attcaaagat tcaggatgaa tatacgaagt tgaataaaat aacagctgaa 540
agaaatcagt tagatgccga cttgcaaact ctacaaaatg aattgattca acttcaacag 600
caaggagact ccccttcctt atcagatacg acatcagctg atgataaagc taagttagaa 660
actaagatat tagaaataac aacaaaaatc gaagcattaa aacaaatat tacttctaag 720
aatagcgaga ttgatagtca acaaagcaat attaaagata tgaaccgtac ctataatgat 780
ccaacttctc aggcttataa tatttatgct caattagtta gtgagttagg tactgctcgt 840
tcaaacaaca ataaaagtat tacagagctt gaggctaatc ttggagtggc aacaggtcaa 900
gataaagctc atagtatatt agcgccaaat gaaggtactc tgcattattt ggtacctttg 960
aaacaaggaa tgtctattca gcaggggcaa acgatagcag aagtttcagg gaaagaaaaa 1020
ggttactatg tagaagcttt tgtacttgcg agtgatattt ctcgtgtctc aaaaggagca 1080
aaagttgatg ttgctattac tggtgtaaat agtcaaaaat atggaacact aaaggacaa 1140
gtcagacaga ttgattcagg aacaatttcc caagaaacga aagaggggaa tattagcctc 1200
tataaagtca tgatagaatt agaaacctta actctaaaac atggaagtga gacagtcgtc 1260
ctccaaaagg atatgccagt tgaagtgcgg attgtctatg ataaagaaac ctatcttgat 1320
tggattttgg aaatgttaag tttcaagcaa taa 1353
```

<212> Type : DNA
<211> Length : 1353
SequenceName : SEQ ID 752
SequenceDescription :
Sequence
<213> OrganismName : Neisseria meningitidis serogroup A strain Z2491
<400> PreSequenceString :

```
atgaataaag gtttacatcg cattatcttt agtaaaaagc acagcaccat ggttgcagta      60
gccgaaactg ccaacagcca gggcaaaggt aaacaggcag gcagttcggt ttctgtttca     120
ctgaaaactt caggcgacct ttgcggcaaa ctcaaaacca cccttaaaac cttggtctgc     180
tctttggttt ccctgagtat ggtattgcct gcccatgccc aaattaccac cgacaaatca     240
gcacctaaaa accagcaggt cgttatcctt aaaaccaaca ctggtgcccc cttggtgaat     300
atccaaactc cgaatggacg cggattgagc cacaaccgct atacgcagtt tgatgttgac     360
```

```
aacaaagggg cagtgttaaa caacgaccgt aacaataatc cgtttctggt caaaggcagt      420
gcgcaattga ttttgaacga ggtacgcggt acggctagca aactcaacgg catcgttacc      480
gtaggcggtc aaaaggccga cgtgattatt gccaacccca acggcattac cgttaatggc      540
ggcggcttta aaaatgtcgg tcggggcatc ttaactatcg gtgcgcccca aatcggcaaa      600
gacggtgcac tgacaggatt tgatgtgcgt caaggcacat tgaccgtagg agcagcaggt      660
tggaatgata aaggcggagc cgactacacc ggggtacttg ctcgtgcagt tgctttgcag      720
gggaaattac agggtaaaaa cctggcggtt tctaccggtc ctcagaaagt agattacgcc      780
agcggcgaaa tcagtgcagg tacggcagcg ggtacgaaac cgactattgc ccttgatact      840
gccgcactgg gcggtatgta cgccgacagc atcacactga ttgccaatga aaaaggcgta      900
ggcgtcaaaa atgccggcac actcgaagcg gccaagcaat tgattgtgac ttcgtcaggc      960
cgcattgaaa acagcggccg catcgccacc actgccgacg gcaccgaagc ttcaccgact     1020
tatctctcca tcgaaaccac cgaaaaagga gcggcaggca catttatctc caatggtggt     1080
cggatcgaga gcaaaggctt attggttatt gagacgggag aagatatcag cttgcgtaac     1140
ggagccgtgg tgcagaataa cggcagtcgc ccagctacca cggtattaaa tgctggtcat     1200
aatttggtga ttgagagtaa aactaatgtg aacaatgcca aaggctcggc taatctgtcg     1260
gccggcggtc gtactacgat caatgatgct actattcaag cgggcagttc cgtgtacagc     1320
tccaccaaag gcgatactga attgggtgaa aatacccgta ttattgctga aaacgtaacc     1380
gtattatcta acggtagtat tggcagtgct gctgtaattg aggctaaaga cactgcacac     1440
attgaatcgg gcaaaccgct ttctttagaa acctcgaccg ttgcctccaa catccgtttg     1500
aacaacggta acattaaagg cggaaagcag cttgctttac tggcagacga taacattact     1560
gccaaaacta ccaatctgaa tactcccggc aatctgtatg ttcatacagg taaagatctg     1620
aatttgaatg ttgataaaga tttgtctgcc gccagcatcc atttgaaatc ggataacgct     1680
gcccatatta ccggcaccag taaaaccctc actgcctcaa aagacatggg tgtggaggca     1740
ggcttgctga atgttaccaa taccaatctg cgtaccaact cgggtaatct gcacattcag     1800
gcagccaaag gcaatattca gcttcgcaat accaagctga acgcagccaa ggctctcgaa     1860
accaccgcat tgcagggcaa tatcgtttca gacggccttc atgctgtttc tgcagacggt     1920
catgtatcct tattggccaa cggtaatgcc gactttaccg gtcacaatac cctgacagcc     1980
aaggccgatg tcaatgcagg atcggttggt aaaggccgtc tgaaagcaga caataccaat     2040
atcacttcat cttcaggaga tattacgttg gttgccggca acggtattca gcttggtgac     2100
ggaaaacaac gcaattcaat caacggaaaa cacatcagca tcaaaaacaa cggtggtaat     2160
gccgacttaa aaaaccttaa cgtccatgcc aaaagcgggg cattgaacat tcattccgac     2220
cgggcattga gcatagaaaa taccaagctg gagtctaccc ataatacgca tcttaatgca     2280
caacacgagc gggtaacgct caaccaagta gatgcctacg cacaccgtca tctaagcatt     2340
accggcagcc agatttggca aaacgacaaa ctgccttctg ccaacaagct ggtggctaac     2400
ggtgtattgg cactcaatgc gcgctattcc caaattgccg acaacaccac gctgagagcg     2460
ggtgcaatca accttactgc cggtaccgcc ctagtcaagc gcggcaacat caattggagt     2520
accgtttcga ccaagacttt ggaagataat gccgaattaa aaccattggc cggacggctg     2580
aatattgaag caggtagcgg cacattaacc atcgaacctg ccaaccgcat cagtgcgcat     2640
accgacctga gcatcaaaac aggcggaaaa ttgctgttgt ctgcaaaagg aggaaatgca     2700
ggtgcgccta gtgctcaagt ttcctcattg gaagcaaaag gcaatatccg tctggttaca     2760
ggagaaacag atttaagagg ttctaaaatt acagccggta aaaacttggt tgtcgccacc     2820
accaaaggca agttgaatat cgaagccgta aacaactcat tcagcaatta ttttcctaca     2880
caaaaagcgg ctgaactcaa ccaaaaatcc aaagaattgg aacagcagat tgcgcagttg     2940
aaaaaaagct cgcctaaaag caagctgatt ccaaccctgc aagaagaacg cgaccgtctc     3000
gctttctata ttcaagccat caacaaggaa gttaaaggta aaaaacccaa aggcaaagaa     3060
tacctgcaag ccaagctttc tgcacaaaat attgacttga tttccgcaca aggcatcgaa     3120
atcagcggtt ccgatattac cgcttccaaa aaactgaacc ttcacgccgc aggcgtattg     3180
ccaaaggcag cagattcaga ggcggctgct attctgattg acggcataac cgaccaatat     3240
gaaattggca agcccaccta caagagtcac tacgacaaag ctgctctgaa caagccttca     3300
cgtttgaccg gacgtacggg ggtaagtatt catgcagctg cggcactcga tgatgcacgt     3360
attattatcg gtgcatccga aatcaaagct ccctcaggca gcatagacat caaagcccat     3420
agtgatattg tactggaggc tggacaaaac gatgcctata ccttcttaaa aaccaaaggt     3480
aaaagcggca aaatcatcag aaaaaccaag tttaccagca cccgcgacca cctgattatg     3540
ccagcccccg tcgagctgac cgccaacggt atcacgcttc aggcaggcgg caacatcgaa     3600
gctaatacca cccgcttcaa tgcccctgca ggtaaagtta ccctggttgc gggtgaagag     3660
ctgcaactgc tggcagaaga aggcatccac aagcacgagt tggatgtcca aaaaagccgc     3720
cgctttatcg gcatcaaggt aggtaagagc aattacagta aaaacgaact gaacgaaacc     3780
aaattgcctg tccgcgtcgt cgcccaaact gcagccaccc gttcaggctg ggataccgtg     3840
ctcgaaggta ccgaattcaa aaccacgctg gccggtgccg acattcaggc aggtgtaggc     3900
gaaaaagccc gtgtcgatgc gaaaattatc ctcaaaggca ttgtgaaccg tatccagtcg     3960
gaagaaaaat tagaaaccaa ctcaaccgta tggcagaaac aggccggacg cggcagcact     4020
atcgaaacgc taaaactgcc cagcttcgaa agccctactc cgcccaaatt gtccgcaccc     4080
ggcggctata tcgtcgacat tccgaaaggc aatctgaaaa ccgaaatcga aaagctgtcc     4140
aaacagcccg agtatgccta tctgaaacag ctccaagtag cgaaaaacat caactggaat     4200
caggtgcagc ttgcttacga cagatgggac tacaaacagg agggcttaac cgaagcaggt     4260
gcggcgatta tcgcactggc cgttaccgtg gtcacctcag gcgcaggaac cggagccgta     4320
ttgggattaa acggtgcggc cgccgccgca accgatgcag cattcgcctc tttggccagc     4380
```

```
caggcttccg tatcgttcat caacaacaaa ggcgatgtcg gcaaaaccct gaaagagctg    4440
ggcagaagca gcacggtgaa aaatctggtg gttgccgccg ctaccgcagg cgtagccgac    4500
aaaatcggcg cttcggcact gaacaatgtc agcgataagc agtggatcaa caacctgacc    4560
gtcaacctag ccaatgcggg cagtgccgca ctgattaata ccgccatcaa cggcggcagc    4620
ctcaaagaca acttgggcga tgccgcactg ggtgcgatag tcagtaccgt acacggagaa    4680
gtagcgagca aaatcaaatt taatctcagc gaagactaca ttacccacaa gattgcccat    4740
gccatagcgg gctgtgcggc agcggcggcg aataagggta agtgtcagga tggtgcgatc    4800
ggtgcggctg tgggcgagat agtcggggag gctttgacaa acggcaaaaa tcctgccact    4860
ttgacagcta aagaacgcga acagattttg gcatacagca aactggttgc cggtacggta    4920
agcggtgtgg tcggcggcga tgtgaataca gcggcgaatg cggctaaagt cgcgattgaa    4980
aataacctat tatctcaaga agagtatgct cttagagaaa aattgatcaa aaaagccaaa    5040
gggaaaggcc tattatcttt agattggggc agcctgaccg aacaagaggc aaggcagttt    5100
atctatttga ttgagaaaga tcgatattct aatcaattgc ttgaccgata tcaaaaaaat    5160
ccaagtagtt taaataatca agaaaaaaat attcttgcat attttattaa ccaaacctct    5220
ggaggtaaca cagcttgggc agcttcgata ctgaaaacgc cccagtcaat gggtaatctc    5280
actattcctt ccaaagatat taataacacc ttatcgaaag cctatcaaac attgagtcgt    5340
tatgattctt ttgattacaa atcagctgtt gccgcacaac ctgcacttta cttattaaac    5400
ggaccgcttg gcttcagtgt caaagcagct actgtggcag caggaggata taacattgga    5460
cagggagcga aagcaatctc taatggagaa tatctgcatg gtacagttca ggttgttaat    5520
ggcacattga tggttgcagg atctgtatct gcacaggctg caatatcggc caagcctgca    5580
cctgttaccc gttatctgag caatgacagt gctcctgctt taagacaagc tttaactgct    5640
gaaagccaga gaatccgcat gaaactgccg gaagagtatc gacaaatagg gaatcttgcg    5700
atagcaaaaa ttgatgttaa aggattaccg caaaggatgg aagcatttag ttctttccaa    5760
aaaggggaac atggatttat ttcgttacct gaaacaaaaa tttttaaacc tatatctgtt    5820
gataaatatc ataatattgc ctctcctcct agaggaacat taagaaatat agatggagaa    5880
tataaattac ttgaaactat agcacagcaa ctcggaaata atcgtaatgt atcaggtaga    5940
attgatctat ttacagaatt aaaggcctgt caatcttgca gcaatgttat tttagagttt    6000
agaaatcgct atccaaatat tcaattaaat atttttacag gaaaatag                 6048
```

<212> Type : DNA
<211> Length : 6048
SequenceName : SEQ ID 753
SequenceDescription :
Sequence
<213> OrganismName : Neisseria meningitidis serogroup A strain Z2491
<400> PreSequenceString :

```
atggacttaa tccaaacccc gaataagcaa tttgtcgacg gcgaccgccg cacgcccggt      60
actcccgtac ccgcatggtg gctgaaccag ttacaaggcg agttgtacag cattttaaac     120
gcggttggca ttgagcctaa caaagccgac catgcccaag tcttatcggc cattaaaacg     180
ttggccgccg atgcttcgca ggttgccagt atcgatgctc tgcgtaaata cagcggcaca     240
ggctatgtga acgtcaacgc ctatcacgcc aatacaacag tgggcggcgg cgtgtttgtg     300
gcggataaag ccgataaatc taccgctgat aacggctgta ccgttattgt ttctaccgac     360
ggcacgcgct ggaagcgtgt gttttcaggg atgcttaacc tgcatgattt tggatatgtg     420
gccagcaaaa acaatgcact atccactttg aatgccgctg aatctgccgc gcttgacgta     480
gttgttgatt gcttgggttt gtcaattgat acgggtaata tctacccgca aaaaaacaaa     540
tacacaaacg gcaagtttgt gattaacggc aaaactgtcg atgttcaata ccagcctatc     600
agaagcggta tcggtcgatt catctccgga actggtgcag cagccaacct caaatcgaat     660
gaatggactg gcgcgggttt aatcgttatt ggcgaaggcg caatggagca gatggagaaa     720
tgtgtttcct caatcgctat tggcgaccgt gcgcagggct tttctaaagt aagcagggac     780
aacatcgcca ttggggccga cagcctgatt aatgtgcagg ccgctactga atggtacgac     840
cagtcacgca tggaaggcac gcgcaacatc ggtattggtg gtaatgcagg acgcggcatc     900
accagcggtt actctaatgt gtcaatcggg cgcaatgccg gacagggatt gggtgaaggc     960
tcgtcaaata ttgcacttgg cgcaggcgcg atggctggta ctgctccagt cggttttagt    1020
ggcgacattg aagttttttg gccgtcttcg acctcaagaa caatcgcaat cggcgaggct    1080
gtcttgcaaa catatcaggg ccgcgccgct caaaccgcaa ttggtgccaa tgcggcgcga    1140
aatacaaaaa aggccgaaaa agttaccgca atcggttctg ccgcgatgga gaatcttgag    1200
cgaaaccgcg ccccaaatgg cggagatgtt gtctggacgg gaacggaagc aggtacctac    1260
gcccaatctg gaaaaaacat cacgcttaca tttcccaaca ttcgcggtgc gcaagcgact    1320
tattgggtgg gcatccgcct tacatcaggc acggcgcaaa ccttacaaaa cgacgtcgta    1380
ccggctcagg tcgtatcagt gaatggcaat acattaatca tccaaagctc aaaagagctg    1440
accgccaccg gcgcggccga actgaaatac gtttattctg taaattcaac cgctactaaa    1500
aacgaagagt tgaccatcat cggcgcgaac gccatgaata aggcattgac cgcaggatac    1560
tcaactatca tcggcgtaga tgccgcgttg ttgggagaca attatcaaaa aacaaccgca    1620
atcggcgcat catctttacg aacaggtagt catatttcca caactgctat tgggtattgg    1680
gtaatccctt tggcaagtag tgagaaatgt gttgccattg gagatagtgc gggctatcgg    1740
aacgttcaag gcgacttttt gactgggaaa ataacaaact ccatcgccat cggatatggc    1800
```

```
gcaagaataa acggcgataa cgaaatccaa atcggtacga cagggcaaac tttatatgct    1860
ccaaccgcgg tgaacatccg ttctgacggc cgcgacaaag cagatgttaa gccgttgacg    1920
aacggtttag attttgtaat gaagctcaag ccgatgactg gctactacga ccgccgggat    1980
tcctacgttg acgaattatt caaagacttg ccggcagatg aacgagcgga caaagtccgc    2040
gaatggtggg cgaatccaat caaggacggc agtcataaag aagatcggtt gcggcattgg    2100
tttattgccc aggacattgc tgcgctggaa gatgaatatg gtcgattgcc gatggtaaat    2160
aaaacaaacg atacctacac cgtcgaatac gaaacgttca tccccgtttt gactaaagcc    2220
attcaggaaa tggccgcaag aattgaaaca ttagaaaccg aaatgaagga atcgaaaaaa    2280
tga                                                                  2283
```

<212> Type : DNA
<211> Length : 2283
SequenceName : SEQ ID 754
SequenceDescription :
Sequence
--------
<213> OrganismName : Streptococcus pyogenes strain MGAS8232
<400> PreSequenceString :

```
atgaaaaata tctcacgcaa atgctttatg accagtgtgg tatgtattat tctaggaggc      60
attcttctag ggctggcta tgcaactgga ggacttcagg acattaaaca ccaaacagct      120
cccaaaaagg tcatcaaaac atttgaccaa ataactgctc ttgatattga cagttctgcc     180
tcaactatta cagtagagac aggacccgtt caaagaccaa cagtgaccta ttacacacat     240
cctaaattta ttgaccctat cgtcacaaca ttaacaggta agaccctatc tctttcgcaa     300
aaacccaaag acattgtcat cactggtgga attgaaattt taggttttac tctcaataat     360
agtcgtcaag agaagaacta ccgttctatt accattactg ttcctgaaaa aactagcctc     420
aatgaagtta agggaagtaa tgtcccacat accactttgt caaatctaac tgtccaagat     480
atgcaattcg atggcaatct tactctttta cataccaaag tcaagaaagc tactatcact     540
ggtatgttgg aagccactaa aagtcagtta acaaatctcg agttaaaagc tgactattct     600
ttttcaaacc tgactgattc tagtgtggaa aatgggacca tcagcttagg aaatggacaa     660
ctaactacta aagataccac tctaaaagcc atcaatattc aatcattaca ccctggcggt     720
atagaagccg agagaacaac ccttgaaaat gtgaccttca ctgtttctaa aagcaaagaa     780
gaagaagagg agaacgacta ctatgacaat gatgctatct tcaccgctca tgcccttacc     840
cttaaaggga ctaatactat tagtggtggt gatattgatg ttgacataac cttgacaaaa     900
gcaaaggcca tcgcctacag ggcaaggacc gaaaatggta aagtctccct tggctcacag     960
ctgacaccag ctaagattgg taaggaatca acttcagatg ttatttctta tgtggctgag    1020
aataaagcag ctactggaaa tttaacggtt aatctcaata agggagacat tactatcaaa    1080
tga                                                                  1083
```

<212> Type : DNA
<211> Length : 1083
SequenceName : SEQ ID 755
SequenceDescription :
Sequence
--------
<213> OrganismName : Streptococcus pyogenes strain MGAS8232
<400> PreSequenceString :

```
atgtttaaga aagaaaattt aaaacaacgt tattttaatt ttggattagt agcgttagct      60
ctaacaatat tagccatcat ttttgccttc tcaagtaaaa atgctgatac taagtcttat     120
gctaagaagt cagaaagtaa aatggtaaca atcgacaagg ctccaaaaaa taatcatgct     180
attactaaag aagaaagcaa agaaaaagca aagagcattg cttcggagcc tattcccaca     240
gtagaaaact ctgtagctcc gacagtaaca gaggaagcac cggttgttca gcaagaagtg     300
actcaaactg ttcagcaggt atcttcagta gcctataatc caaataatgt ggtacttttcc    360
aatggaaata ctgctggtat tgtaggaagt caagcggcgg cacagatggc agcagcaaca     420
ggtgttccac aatcaacttg ggaacatata attgcgcgtg aatctaatgg aaatcctaac     480
gcagctaatg cttctggggc atcagggttg ttccagacaa tgccaggttg ggttctaca     540
gcaacggttg aagatcaagt caatgcagcc ttgaaagcct atagtgcaca aggtttatca     600
gcttggggtt actaa                                                      615
```

<212> Type : DNA
<211> Length : 615
SequenceName : SEQ ID 756

SequenceDescription :
Sequence
--------
<213> OrganismName : Streptococcus pyogenes strain MGAS8232
<400> PreSequenceString :

```
atgttagaag aattgaaaac acttattaaa aatccaaaat taatgattac aatgattggt      60
gtggccctag tgcctgcctt atataattta tcctttctag gctcaatgtg ggatccttat     120
ggtcgggtca atgaccttcc cattgctgtt gttaatcatg ataagcctgc aaagagagct     180
gataagtcat tgacaattgg gaatgatatg gtggacaaga tgtctaaaag taaagattta     240
gactatcatt ttgtatcttc aaaaagtgct caaaaaggtc ttaaaaaagg tgattattat     300
atggtcatta ctttacccga agatctttct cagcgagcaa caaccttatt aaatcccgaa     360
ccccaaaaac taactatccg ttaccaaact agtaaaggac atggaatggt cgctgctaag     420
atgggggaaa cagcgatggc taagctgaaa gagtctgttt cgcaaaacat tacgaagact     480
tatacctcag cagtttttag cagtatgaca gacctccaat caggattaaa agaagcctca     540
actggtagtc aagcattgga ttcaggagcg aagacagccc aaatgggtag tcaaatgctc     600
tcagataact tagcaggttt atctagtgct agttggcaat ttcaacaagg aactaatcgt     660
ttaacttcag gattaacggc ctatacagct ggtgttagtc aagtaaagga cggactaggg     720
cagctctcaa ccgatatgcc agtttacctg aatggggttt ctcggttatc acaaggagct     780
tctcaactta atcagggtct ttcacagttg acacaatcaa caacactttc tgatgataaa     840
gctaaaagaa tccaatcttt ggaggtagga ttaccagttt taaatcaagg tattcagcaa     900
ctaaatgaaa atctctcaac gatgcaggta ccaaaactta ataccgatga gttagggaat     960
aacttggctg ccattgctca agctgcgcaa cagttacttg ttaaagaagc tgctgctcac    1020
aaggaacaac tagcagtctt acaagccact agcgcttatc agtcactaac tgctgagcaa    1080
caaggggaat taactgctgc tcttacccaa actgataagg gtgaagctgt ggctcctgct    1140
caaacgattt taaggtctgt tcaaactttg tcaacaagtt tacagtctct ctctcaagaa    1200
gatcagtcaa aacagttgga gcaacttaag gaagctgttg cacagattgc taatcaatcc    1260
aatcaagctt tgccgggagc aagttctgct ttaactgaat tatcaacggg attagcaaag    1320
gtaaatggta gcttaaatca acaagttcta ccaggaagta atcaattgac aacaggatta    1380
gcacaattaa acaggtataa tactgccatt ggttctgggg taataaaact ctcagaaggt    1440
gccaatgcct tgtcatccaa gtccggagaa ttactagatg gtagccatca attatcagaa    1500
ggtgctacta aactagctga tggtagttct caattgagtc agggtggtca tcaattaacg    1560
agcggattga ctgaattatc aacaggattg tcaatcttaa atggttcctt agccaaagcc    1620
tctcagcagt tatcgcttgt ttctgtgact gataaaaatg ctaaagctgt cgcaaaacct    1680
cttgtgttaa atgagaaaga caaagatggt gttaagacga atgggatcgg gatggcacct    1740
tatatgattg ctgtttctct aatggttgtg gccctttcaa ccaacgtcat ttttgctaat    1800
tctttatctg gtcgtccggt caaagataaa tgggattggg ctaaacaaaa atttgttatt    1860
aatggtttta tttcgactat gggatccatt gttctctact tagctattca attattaggg    1920
tttgaagccc gttatggtat ggaaacctta ggatttatta tgctaagtgg ttggacgttt    1980
atggctcttg tcacagcttt ggtcggttgg gatgatcgat atggctcttt tgcttctttg    2040
gttatgttat tgcttcaggt tggctcttca ggtggctctt accccattga gttaagtgga    2100
gcatttttcc aaaagttaca tcctttctta ccaatgactt atgtggtatc tggtttacga    2160
caaaccattt cattatcagg tcatattgga gtagaagtga aagtcttaac tggtttctta    2220
ctggcattta tggtattatc actactcatt tatcgtccca agaaaacagt ctaa          2274
```

<212> Type : DNA
<211> Length : 2274
SequenceName : SEQ ID 757
SequenceDescription :
Sequence
--------
<213> OrganismName : Streptococcus pyogenes strain MGAS8232
<400> PreSequenceString :

```
atgagcagag acccaacata tacaataaac gagcacgact tatcttttgc agatggtcgt      60
ttttatgtga cctttaaggc agataagtca agtgagactg tgagacttaa cagtagttgc     120
cttggcaata ccataatcaa aaagctacag gtcgaggatg acaatacaat gcacgacttt     180
gtaaagccta aagttaccac tcaacaagct tttggactag ctcagcaggt caaagagctt     240
gatttacagc taaaagaccc taagtcagat ttgtggggca aaatcaagtt caataataag     300
gcaatgctag tcgagtacgc caacaaagag atgtcaagtg ccattgcgca atcagctgag     360
cagatattgt tacaagtcaa gtctattgat gatgaacgat attccaaatt tgagcaaact     420
ctgaatggta tcaaacaaac tgtcaaaagt gagtcagttg aatccgcacg tactcagcta     480
gcatcaatgt ttgatagtcg tattagtgga cttgatggca aatacagtcg tttgagccaa     540
acaattgata gtcttagcag tcgtcttgat gatggtgttg gtaactactc aacgctatct     600
caaaaggtaa gtggcattga tttacgagtt agtaatgcag ctaatgatgt ttctcgattg     660
tctcagacag cacaaggatt gcagtcacaa atcacaaatg caaaccaaaa ttacagcagt     720
ttgtctcaga ctgtacaggg actacaaaca actgtacgtg ataatcaatc aaatgctaca     780
agtcggatta atcagttaag tgacctgatc agcacaaagg tatcaaaagg tgatgttgag     840
acaactattg ctcagagtta cgacaagata gccttcgcaa tcagggataa actcccagca     900
agcaaaatgt ctggcagtga gattatctcg gcaatcaatc ttgataggtc tggggttaaa     960
atcactggaa aaaatatcac tcttgatggt aacagctaca tcagcaacgc tgtcatcaaa    1020
gatgctcata ttgctaacat ggatgccggt aagattaata ctggttatct taatgctaat    1080
aggattgcaa ccgaggccat tactggtgag aaaattaaga tggactatgc ctttttttaat   1140
aaactcactg ctaacgaggg atattttagg acgttgtttg ccaaagacat ctttgcaaca    1200


tcagtccaat ctgtaacact atcagctagc aaaattactg gaggtgtatt agccgctaca    1260
aatggggcaa gtcagtggga cctaaataat gccaatatga cctttaatcg agatgccaca    1320
attaatttta atagcaaaaa caatgcctta gtacgtaaag atggcacaca tactgctttt    1380
gtacatttta gtaatgccac accaaaaggc tatagaggct cagcgttgta tgcctctatc    1440
ggcattacct catcaggaga tggcatcgac agcgcttctt ccggtcgttt tgcagggcta    1500
aggtcattta ggtacgctac gggatataac catactgctg cagtcgacca aaccgagcta    1560
tacggtgata atgtcttgat tgcagatgac tttagcatca atcgaggatt taaatttaga    1620
ccagacaaaa tggaaaaagt gctcgacatg aacgacttgt atgcggctgt agtagcctta    1680
ggccgctgtt gggggcactt ggctaacgtc ggctggaata ctgctcatag caattttaca    1740
agtgctgtga gtagggaatt gaataactac atcactaaaa tttaa                    1785
```

<212> Type : DNA
<211> Length : 1785
SequenceName : SEQ ID 758
SequenceDescription :
Sequence
--------
<213> OrganismName : Streptococcus pyogenes strain MGAS8232
<400> PreSequenceString :

```
atggcagctg atggtaaggt aacgatactt gttgacgttg atggtaagca ggtaaaggta     60
ctcaatagtg agttagataa agttgccaag cacggtgaca aaggcagctc ctctcttaaa    120
aaatttgcgg ttggtgcagg agtctttaaa ttagcttcgg ctgcagttga tttggttagt    180
caatctcttg gcaaggctat cacaagattt gacacgcttg aaaaatatcc aagggtcatg    240
aaagctatgg ggcatagcgc tgaggatgtt gctagatcaa ctgataagtt agcgaacgga    300
attgatggac taccaacaac tttagacgag gttgtcggaa ccgctcaacg tttgacctct    360
attactaagg atatcaataa atcaactaat ctcacactag cattaaataa tgccttttta    420
gcttcaggag cttcatcaga ggctgcaagc cgagggctgg agcaatatgc ccaaatgcta    480
tcagctggta aggttgatat gcaagcttgg aaaaccctcc aagaaacaat gccttatgcc    540
ttacaacaaa ctgcggaagc ttttggattt gcaggggcat cggctcaaaa ggatttttat    600
gaggcgttaa aaaacgggca aataacattt gaccaatttt ctaataagtt gattgagtta    660
aatgatggtg tcggcggttt tgcagaacta gccaaagaaa atagtaaagg gattgaaacc    720
tcttttaaca acatcaagaa cgctattgca aaaggtgtgg ccaatagcat taaggctttg    780
gatgatttgt ctaaggctgc aacaggtaag ggcatagctg atcattttga tagtttgaaa    840
gttgttatca atgcctcttt tagcgccatc aatgcaagta ttaaagctag tacaccgcta    900
tttaaacttt tgtttagtgt tattggtgct ggaatatcag tcgtcaaagc tctgtcgcct    960
gccttagttg gtgtagcatc tggtctagct gccatgaggg cagttaatga gactataaca   1020
atgattaaag cgctaaatag agcttgggtt atggcatctg catcaatgag tattggagca   1080
acaaccatta agactgtgac tgcggtacaa gcggtaagta ccacgatgac taaagcagat   1140
atggttgcaa gactatctca gttaggtgtc ttaaaagcca gtaccgtgat ttatggtgtt   1200
atgacaggcg ctatcagttt atctactgct gcaaccatag ccagtactgc tgcggtaact   1260
gcgttaaaag cagcacttgt agccttaaca ggtcccgttg gttgggtagt tggagctatc   1320
ggtgctttag ttgctgtcgg agtaagctta tggtcatggc taactaaaga gtcagacgag   1380
accaaaaagc tgaaaaaaga gcaggagggg ctagtcgaaa gcaacaaaca gctaagagat   1440
tctgtccgtg agggcgtgca agagcgtaag aagggccttg agtccgtcaa agagagcact   1500
gcagctcatc aaaaattagc tgacgaaatc attaagttag ccgccaaaga aaacaaaact   1560
gcaggcgaaa aacaaaactt aaaaaataag attgatcagc ttaatgggtc tattgatggc   1620
ttaaacttgg cctatgacaa aaactccaat tctctttctc acaatgcaga tcaaattaag   1680
tcacgcatta gtgccatgga agcagaaagc acatggcaaa cagcacaaca aaatctgtta   1740
aatattgaac agaaacgtag cgaggttagc aaaaagctag ctgaaaatgc tgatttgcgt   1800
aaaaagtgga atgaagaagc taacgtctcc gattccgtcc gaaaagaaaa gattgcagaa   1860
ctcacagaag aagaagctaa acttaaaaat atgcagactc aactgcagga ggagtataac   1920
aagacatcag ctactcaaca agctgctgca gacgctatgg ctgccgctga agaatcagga   1980
tcagcaagac aggttatagc gtacgaaaat atgtcagaag ctcaacgaac tgccatagac   2040
aatatgcgca ctaagtactc tgaactttta gagacaacga catctatttt tgatgctata   2100
gaacaaaaga cggcattatc agtagatcaa atgaatacca accttgaaaa aaatagagct   2160
gctactgaac agtgggctac taatttggag attttggctc agcgtggtgt ggatcaaggt   2220
attttggagc aactaagacg catgggtcct gaaggagcca cacagacgca agtttttgtg   2280
gatgcaacag atgccgagct agcaccccttg caggaaaact ttagagcagc cacagaaact   2340
gctaaaaatg caatggggag cgttttagac tcagcaggtg tggaaatgcc agaaaaagtt   2400
aaagggatgg tcactaatgt ttctacggga ttacaggcgg aactgcaagc tgctaacttt   2460
gctcaacttg gccaagaaat ccctaatggg gtttctcaag gtataagtca aggggcaggt   2520
aaagcaagtg acgcaagtgt caaaatgggt caagaagtta aacgctcttt tcaaggagag   2580
ttgggtatcc actcgccatc gcgagtattt actgagtacg gtggccatat tactgatggc   2640
ttgagtaatg gtgtgacaaa tggaacgtca aaagttatgc aaaccatgca gagcttggct   2700
caacagatgt ctcaaaaagg acagcagatt gttaatgaca tgcgtagcaa gtcgaaccaa   2760
atcacagatg cttttagcac gatgagtggt ccaatgcact ctcatggtgt taatgccatg   2820
caaggtttgg ccaatggtat ttatgcaggg tcgggggcag ctttagcggc agctcaaagc   2880

attgcggcac gtatcaccgc aacaattcaa agtgccttag atatccactc gccatctcgt   2940
gttatgaggg atgaggttgg acgttttatc cctcagggta tcgctgtagg tattgatgcg   3000
gatagaaaag tcattgactc atctatgcaa aagctaaaag agtcaatgac gattaatgcg   3060
actccagaaa tagcctctgg atttggcgga ggagttgcgg ggattgctaa tcagaccaca   3120
aataactcaa ataacagttt taccccttaat gtcaaggttg atgaatccga cggtaatagc   3180
cacgagaaat atcaacgctt attcagagaa tttagctggt atattcaaca acaacaagga   3240
aggttaggtg atgttaaatg a                                               3261
```

<212> Type : DNA
<211> Length : 3261
SequenceName : SEQ ID 759
SequenceDescription :
Sequence
<213> OrganismName : Streptococcus pyogenes strain MGAS8232
<400> PreSequenceString :

```
atggctaaag aaccatggga agaaaaaatt gttgatgata ctatagggac acgaacacgt      60
aaatcaagaa atgctttcat tagcacgcct tggttgactg ctttattaag tgtattcttt     120
gtcatcattg ttgctatact ttttattttc ttctatacat caaatagcgg tagtaataga     180
caagctgaaa caaatggttt ttatggagca tccactcata aaaaaacaag gaaagcttct     240
aacgctaaaa aaacatcaag tagttcaaca actacagaca caacaccttc tagcgaagaa     300
acacttgctt ctagtgaagg aaccggcgaa acccttactg tattggcagg tgaggggca      360
gcttctattg cagctcgtgc aggtatttct gtggagcagt tacaagcact taatccagag     420
cacatgactc aaggatattg gtatgccaat ccaggagatc aagtcactat taaataa        477
```

<212> Type : DNA
<211> Length : 477
SequenceName : SEQ ID 760
SequenceDescription :
Sequence
--------
<213> OrganismName : Streptococcus pyogenes strain MGAS8232
<400> PreSequenceString :

```
atgtctaaaa gaggaaaaat taaaataaca acgaaaacaa agcttattac agctagtgtt      60
ataacgctag tattaattat aactggagta gtcttgtgga aacaacaaca aaatacgcta     120
acagctgata tcgctaaaga accttactct actgttagtg taactgaagg gagtattgct     180
tcttcgactt tactatcagg tactgtaaag gctttatcag aggaatatat ttattttgat     240
gctaataaag gaaatgatgc aactgttaca gttaaaatag gtgatcaggt aacgcagggc     300
cagcaattag ttcaatataa tacaacaaca gctcagtcag cttatgatac tgctgttagg     360
agtcttaaca agattggccg acaaattaat catcttaaaa catacggagt tcctgctgtt     420
agtacagaaa ctaataaaga tgaagctacc ggtgaagaga cgacaacaac agttcaacca     480
tcagctcagc aaaatgctaa ttataaacag cagctgcaag atttaaatga tgcttatgca     540
gatgcacaag cagaagtaaa taaagcgcag atagcgttaa atgatacagt agttatcagt     600
agtgtctctg gaactgttgt ggaagtaaat aatgatattg atccttcttc aaagaacagt     660
caaacacttg ttcacgtagc aaccgaagga cagcttcagg tgaaaggaac attgacagag     720
tatgatttag caaacgttaa ggttggtcaa tctgtaaaaa ttaagtctaa agtttattct     780
aatcaagaat ggactggaaa aatatcatat gtttcaaact atccaactga gtctaatgca     840
ggttcaacaa cgccagcagg tagcactgga gcgggaagtt ctacaggagc tgcctatgat     900
tacaagattg atattataag tcctcttaac cagcttaaac aaggtttcac tgtttctgtt     960
gaggttgtta atgaagctaa acaggcctta gttcctttaa cggctgttat taagaaagat    1020
aaaaaacact atgtttggac ttatgatgat gctactggca aagccaaaaa agtagaggtg    1080
acacttggaa acgcagatgc acaacaacaa gaaattcata aaggagtagc tgttggtgac    1140
attgttattg ccaatccaga taaaaatatc aaaccggata aaaaactaga aggggttatt    1200
tcaataggta ccaacacaaa accggaaaaa gattctcaat caaagaataa aaaatcaggg    1260
gtggataaat ag                                                        1272
```

<212> Type : DNA
<211> Length : 1272
SequenceName : SEQ ID 761
SequenceDescription :
Sequence
--------
<213> OrganismName : Treponema pallidum subsp. pallidum str. Nichols
<400> PreSequenceString :

```
gtgctccgct tgccaactgc gcgcgcgtgc attacaatgg gcaccatgat cagacataca      60
tttacgcata ggtgcggagc gctcctgtgc gcgctggcgt tgggaagctc cactatggct     120
```

```
gcgaccgccg ctgcaaaacc caagaaaggg caaatgcaga aattgcggca gcggccggtg      180
tgggcgccca ccggcgggcg gtatgcgtct ttggacggtg cgtttaccgc gctggcaaat      240
gatgcaagtt tctttgaggc aaatccggca ggaagtgcga acatgacgca cggggagctg      300
gctttcttcc ataccactgg ctttggctcg tttcacgccg aaacgctctc ttacgttggc      360
cagtcgggca actggggata cggcgcgtcg atgcgtatgt ttttccctga atctgggttt      420
gacttttcta ccaccacgga gcccgtgtgc acacctgctt cgaaccccat taagcagcgc      480
ggggcaattg gaatcatcaa ctttgcccgg cgtatcggag gtctctccct gggagccaac      540
ctgaaggcgg ggttccgcga cgcgcagggc ctgcagcaca cctctgtctc cagtgacatc      600
ggcttgcagt gggtggggaa cgttgccaag tcctttacct ctgaagagcc caacctgtac      660
atcgggcttg cggccaccaa cttgggattg accgtaaagg tctcggacaa gatagagaac      720
tgcacgagta cctgtgaaaa gtgtggttgc tgcaaggaga ggtgctgctg caacggcaag      780
aaggcgtgct gcaaggactg cgactgtaac tgcccctgtc aggactgcaa cgacaaaggt      840
acggtgcacg caacagacac catgctgcgt gcagggtttg cataccggcc cttcagctgg      900
ttcctcttta gccttggtgc caccaccagc atgaatgtgc agaccttggc tagtagtgac      960
gccaagtcgc tgtaccagaa cctggcttac agcataggcg ccatgtttga tcccttcagc     1020
ttcctgagct tgagttcgag cttccgcatc aaccacaagg ctaacatgcg agtgggagtg     1080
ggtgcagagg cgcgcattgc ccgcattaag ctgaacgcgg gataccgctg tgacgtcagc     1140
gacatcagca gtgggagtgg gtgcacaggc gcgaaggctt cgcactacct ttccttgggt     1200
ggcgcgatac tgctcggccg aaattaa                                         1227
```

<212> Type : DNA
<211> Length : 1227
SequenceName : SEQ ID 762
SequenceDescription :
Sequence
--------
<213> OrganismName : Treponema pallidum subsp. pallidum str. Nichols
<400> PreSequenceString :

```
atgagcagaa cgttccgcgc gtggcagtgc gttggtgcgc tgtgtgcgct ctctcccctg       60
ctgcctgcct acagctccga gggcgtgcga gaggtacccc cctcccagtc tccgcaggtg      120
gtggtggcgt acgagcccat tcgccccggg gatcagctgc tcaaaattgg cattgttgca      180
ggctgccagt tgtacatagc aggggggaaat ggaaccaacg gctcttcgag ttccggcacc     240
aacggtaacg gcaacggcaa actgctcggg ggcggggggt ttcacctcgg gtacgagtat      300
ttttttacca aaaacttttc cctcggcggg caagtttcct ttgagtgtta ccgcacgacc      360
gggtcaaact attacttttc tgttcccatc acggtaaacc ccacgtacac gtttgccgta      420
gggcgctggc gcataccgct ctccctgggc gttgggctca acattcagtc ctatctcagc      480
aagaaggcgc cggggcttat tgcggaagcc agcgcggggc tctactacca gtacaccccg      540
gactggtcca tcggcggcat tgttgcctac acgcagcttg gggacattgc aagctccccc      600
gacaagtgca gagccgtggg ccttgccacc attgactttg ggtgcgcgcta tcacttttag     660
```

<212> Type : DNA
<211> Length : 660
SequenceName : SEQ ID 763
SequenceDescription :

## Claims

1. A computer implemented computational method for identifying adhesin and adhesin-like proteins, said method comprising steps of:

   a. computing the sequence-based attributes of protein sequences using five attribute modules of a neural network software, wherein the attributes are, (i) amino acid frequencies, (ii) multiplet frequency, (iii) dipeptide frequencies, (iv) charge composition, and (v) hydrophobic composition,
   b. training an artificial neural Network (ANN) for each of the computed five attributes, and
   c. identifying the proteins having probability of being an adhesin (Pad) as ≥ 0.51, wherein the "Pad" is a weighted linear sum of the probabilities from five computed attributes, wherein

   - the amino acid frequency fi = (counts of ith amino acid in the sequence) / 1; i = 1...20, 1 is the length of the protein;
   - the multiplets are defined as homopolymeric stretches $(X)_n$ where X is any of the 20 amino acids and n is an integer > 2 and after identifying all the multiplets, the frequencies of the amino acids in the multiplets were computed as $f_i(m)$ = (counts of $i^{th}$ amino acid occurring as multiplet) / l;
- the frequency of a dipeptide (i, j) $f_{ij}$ = (counts of $ij^{th}$ dipeptide) / (total dipeptide counts); i, j ranges from 1 to 20;
- the charge composition refers to the input frequency of charged amino acids (R, K, E and D considering the ionization properties of the side chains at pH 7.2) given by $f_c$ = (counts of charged amino acids) / l; and to the distribution of said charged amino acids in the given protein sequence which is provided by computing the moments of the positions of the occurrences of the charged amino acids and general expression to compute moments of a given order; say 'i' is

$$Mr = r^{th} \text{ order moment of the positions of charged amino acids}$$

$$= \sum \frac{(X_i - X_m)^r}{N}$$

where, $X_m$ = mean of all positions of charged amino acids $X_i$ = position of $i^{th}$ charged amino acid N = number of charged amino acids in the sequence;
- the hydrophobic composition refers to the hydrophobic scores of five groups of amino acids: (-8 for K, E, D, R), (-4 for S, T, N, Q), (-2 for P, H), (+1 for A, G, Y, C, W), (+2 for L, V, I, F, M) with the following inputs given for each of the group,

(a) $f_i$ = (counts of $i^{th}$ group) / (total counts in the protein); i ranges from 1 to 5; and
(b) $m_{ji}$ = $j^{th}$ order moment of positions of amino acids in $i^{th}$ group; j ranges from 2 to5.

**2.** A method as claimed in claim 1, wherein the protein sequences are obtained from pathogens, eukaryotes, and multicellular organisms.

**3.** A method as claimed in claim 1, wherein the protein sequences are obtained from the pathogens selected from a group of organisms comprising *Escherichia coli, Haemophilus influenzae, Helicobacter pylori, Mycoplasma pneumoniae, Mycobacterium tuberculosis, Rickettsiae prowazekii, Porphyromonas gingivalis, Shigella flexneri, Streptococcas mutans, Streptococcus pneumoniae, Neisseria meningitides, Streptococcus pyrogenes, Treponema pallidum* and Severe Acute Respiratory Syndrome associated human coronavirus (SARS).

**4.** A method as claimed in claim 1, wherein the method is a non-homology method based on sequence composition properties combined with the power of the Artificial Neural Networks to identify adhesins and adhesin-like proteins inspecies belonging to wide phylogenetic spectrum.

**5.** A method as claimed in claim 1, wherein the method uses 105 compositional properties of the sequences.

**6.** A method as claimed in claim 1, wherein the method shows sensitivity of at least 90%.

**7.** A method as claimed in claim 1, wherein the method shows specificity of 100%.

**8.** A method as claimed in claim 1, wherein the method helps identifies adhesins from distantly related organisms.

**9.** A method as claimed in claim 1, wherein the neural network has multi-layer feed forward topology, consisting of an input layer, one hidden layer, and an outputlayer.

**10.** A method as claimed in claim 9, wherein the numbers of neurons in the input layer are equal to the number of input data points for each attribute.

**11.** A method as claimed in claim 1, wherein each trained network assigns a probability value of being an adhesin for the protein sequence.

**12.** A computer system adapted to perform the method of claim 1, said system comprising:

- a central processing unit, executing a software program for prediction of adhesion and adhesion-like proteins using neural networks, giving probabilities based on different attributes using Artificial Neural Network and in built other programs of assessing attributes, all stored in a memory device accessed by the central processing unit (CPU);
- a memory device accessed by the CPU;
- a display on which the central processing unit displays the screens of the above mentioned programs in response to user inputs; and,
- a user interface device.

**13.** Method as claimed in claim 1, wherein the training step (b) is achieved with fully connected multilayer feed forward Artificial Neural Network, the network comprising an input layer, a hidden layer and an output layer which are connected in the said sequence, wherein each neuron is a binary digit number and is connected to each neuron of the subsequent layer for identifying adhesin or adhesin like proteins, wherein the program steps comprise:-

[a] feeding a protein sequence in FASTA format;
[b] processing the sequence obtained in step [a] through the 5 modules named A, C, D, H and M, wherein attribute A represents an amino acid composition, attribute C represents a charge composition, attribute D represents a dipeptide composition of the 20 dipeptides [NG, RE, TN, NT, GT, TT, DE, ER, RR, RK, RI, AT, TS, IV, SG, GS, TG, GN, VI and HR], attribute H represents a hydrophobic composition and attribute M represents amino acid frequencies in multiplets to quantify 5 types of compositional attributes of the said protein sequence to obtain numerical input vectors respectively for each of the said attributes wherein the sum of numerical input vectors is 105;
[c] processing the numerical input vectors obtained in step [b] by the input neuron layer to obtain signals, wherein the number of neurons is equal to the number of numerical input vectors for each attribute;
[d] processing the signals obtained from step [c] by the hidden layer to obtain synaptic weighted signals, wherein the optimal number of neurons in the hidden layer was determined through experimentation for minimizing the error at the best epoch for each network individually;
[e] delivering the synaptic weighted signals obtained from step [d] to the output layer for assigning of a probability value for each protein sequence fed in step [a] as being an adhesin by each network module; and
[f] using the individual probabilities obtained from step [e] for computing the final probability of a protein sequence being an adhesin denoted by the Pad value, which is a weighted average of the individual probabilities obtained from step [e] and the associated fraction of correlation which is a measure of the strength of the prediction.

**14.** Method as claimed in claim 13, wherein the input neuron layer consists of a total of 105 neurons corresponding to 105 compositional properties.

**15.** Method as claimed in claim 13, wherein the hidden layer comprises of neurons represented as 30 for amino acid frequencies, 28 for multiplet frequencies, 28 for dipeptide frequencies, 30 for charge composition and 30 for hydrophobic composition.

**16.** Method as claimed in claim 13, wherein the output layer comprises of neurons to deliver the output values as probability value for each protein sequence.

**Patentansprüche**

**1.** Computerimplementiertes Rechenverfahren zum Identifizieren von Adhäsin und Adhäsin-ähnlichen Proteinen, wobei das Verfahren folgende Schritte umfasst:

a. Berechnen der sequenzbasierten Attribute von Proteinsequenzen unter Verwendung von fünf Attributmodulen einer neuralen Netzwerksoftware, wobei die Attribute (i) Aminosäurehäufigkeiten, (ii) Multipletthäufigkeit, (iii) Dipeptidhäufigkeiten, (iv) Ladungszusammensetzung und (v) hydrophobe Zusammensetzung,
b. Trainieren eines künstlichen neuronalen Netzwerks (KNN) für jedes der berechneten fünf Attribute, und
c. Identifizieren der Proteine, die wahrscheinlich ein Adhäsin (Pad) sind, als $\geq 0{,}51$, wobei "Pad" eine gewichtete lineare Summe der Wahrscheinlichkeiten von fünf berechneten Attributen ist, wobei

- die Aminosäurehäufigkeit

$$fi = (\text{Zählungen der i-ten Aminosäure in der Sequenz}) / \ell;$$

i = 1...20,
ℓ ist die Länge des Proteins;

- die Multipletts sind als homopolymere Abschnitte (X)n definiert, wobei X eine der 20 Aminosäuren ist und n eine ganze Zahl > 2 ist, und nach der Identifizierung aller Multipletts wurden die Häufigkeiten der Aminosäuren in den Multipletts berechnet als
fi(m) = (Zählungen der i-ten Aminosäure, die als Multiplett auftritt) / ℓ;
- die Häufigkeit eines Dipeptids (i, j)

fij = (Zählungen des ij-ten Dipeptids)/(Gesamtzahl der Dipeptide);
i, j reichen von 1 bis 20;

- die Ladungszusammensetzung bezieht sich auf die Eingangshäufigkeit geladener Aminosäuren (R, K, E und D unter Berücksichtigung der Ionisationseigenschaften der Seitenketten bei pH 7,2), gegeben durch

$$fc = (\text{Anzahl geladener Aminosäuren}) / \ell;$$

und auf die Verteilung der geladenen Aminosäuren in der gegebenen Proteinsequenz, die durch Berechnen der Momente der Positionen des Auftretens der geladenen Aminosäuren und des allgemeinen Ausdrucks zum Berechnen von Momenten einer gegebenen Ordnung bereitgestellt wird; sagen 'i' ist

$$Mr = \text{Moment r-ter Ordnung der Positionen geladener Aminosäuren} = \sum \frac{(X_i - X_m)^r}{N}$$

wobei Xm = Mittelwert aller Positionen von geladenem Amino
Säuren Xi = Position der i-ten geladenen Aminosäure
N = Anzahl geladener Aminosäuren in der Sequenz;

- die hydrophobe Zusammensetzung bezieht sich auf die hydrophoben Werte von fünf Gruppen von Aminosäuren: (-8 für K, E, D, R), (-4 für S, T, N, Q), (-2 für P, H), (+1 für A, G, Y, C, W), (+2 für L, V, I, F, M) mit den folgenden Eingaben für jede Gruppe,

(a) fi = (Zählungen der i-ten Gruppe) / (Gesamtzählungen im Protein), i reicht von 1 bis 5; und
(b) mji = Moment j-ter Ordnung von Positionen von Aminosäuren in der i-ten Gruppe; j reicht von 2 bis 5.

2. Verfahren nach Anspruch 1, wobei die Proteinsequenzen von Pathogenen, Eukaryoten und vielzelligen Organismen erhalten werden.

3. Verfahren nach Anspruch 1, wobei die Proteinsequenzen von Pathogenen erhalten werden, die aus einer Gruppe von Organismen ausgewählt sind, die Escherichia coli, Haemophilus influenzae, Helicobacter pylori, Mycoplasma pneumoniae, Mycobacterium tuberculosis, Rickettsiae prowazekii, Porphyromonas gingivalis, Shigella flexneri, Streptococcas mutans umfasst , Streptococcus pneumoniae, Neisseria meningitides, Streptococcus pyrogenes, Treponema pallidum und das mit dem schweren akuten respiratorischen Syndrom assoziierte menschliche Coronavirus (SARS).

4. Verfahren nach Anspruch 1, wobei das Verfahren ein Nicht-Homologie-Verfahren basierend auf Sequenzzusammensetzungseigenschaften in Kombination mit der Leistungsfähigkeit der künstlichen neuronalen Netze zur Identifizierung von Adhäsinen und Adhäsin-ähnlichen Proteinen in Arten ist, die zu einem breiten phylogenetischen Spektrum gehören.

5. Verfahren nach Anspruch 1, wobei das Verfahren Zusammensetzungseigenschaften der Sequenzen verwendet.

6. Verfahren nach Anspruch 1, wobei das Verfahren eine Empfindlichkeit von mindestens 90% aufweist.

**7.** Verfahren nach Anspruch 1, wobei das Verfahren eine Spezifität von 100 % zeigt.

**8.** Verfahren nach Anspruch 1, wobei das Verfahren dabei hilft, Adhäsine von entfernt verwandten Organismen zu identifizieren.

**9.** Verfahren nach Anspruch 1, wobei das neuronale Netzwerk eine Mehrschicht-Feed-Forward-Topologie aufweist, die aus einer Eingangsschicht, einer verborgenen Schicht und einer Ausgangsschicht besteht.

**10.** Verfahren nach Anspruch 9, wobei die Anzahl von Neuronen in der Eingabeschicht gleich der Anzahl von Eingabedatenpunkten für jedes Attribut ist.

**11.** Verfahren nach Anspruch 1, wobei jedes trainierte Netzwerk der Proteinsequenz einen Wahrscheinlichkeitswert zuweist, ein Adhäsin zu sein.

**12.** Computersystem, das angepasst ist, um das Verfahren nach Anspruch 1 auszuführen, wobei das System Folgendes umfasst:

- eine zentrale Verarbeitungseinheit, die ein Softwareprogramm zur Vorhersage von Adhäsion und adhäsionsähnlichen Proteinen unter Verwendung neuronaler Netze ausführt, Wahrscheinlichkeiten basierend auf verschiedenen Attributen unter Verwendung eines künstlichen neuronalen Netzes und in eingebaute andere Programme zur Bewertung von Attributen gibt, die alle in einer Speichervorrichtung gespeichert sind, auf die zugegriffen wird die Zentraleinheit (CPU);

    - Speichergerät, auf das von der CPU zugegriffen wird;
    - eine Anzeige, auf der die zentrale Verarbeitungseinheit die Bildschirme der oben erwähnten Programme in Reaktion auf Benutzereingaben anzeigt; und,
    - ein Benutzerschnittstellengerät.

**13.** Verfahren nach Anspruch 1, wobei der Trainingsschritt (b) mit einem vollständig verbundenen künstlichen neuronalen Mehrschicht-Feedforward-Netzwerk erreicht wird, wobei das Netzwerk eine Eingangsschicht, eine verborgene Schicht und eine Ausgangsschicht umfasst, die in der genannten Reihenfolge verbunden sind, wobei jedes Neuron ist eine binäre Ziffer und ist mit jedem Neuron der nachfolgenden Schicht verbunden, um Adhäsin oder Adhäsin-ähnliche Proteine zu identifizieren, wobei die Programmschritte umfassen:-

[a] Einspeisen einer Proteinsequenz im FASTA-Format;
[b] Verarbeiten der in Schritt [a] erhaltenen Sequenz durch die 5 Module namens A, C, D, H und M, wobei Attribut A eine Aminosäurezusammensetzung darstellt, Attribut C eine Ladungszusammensetzung darstellt, Attribut D eine Dipeptidzusammensetzung darstellt die 20 Dipeptide [NG, RE, TN, NT, GT, TT, DE, ER, RR, RK, RI, AT, TS, IV, SG, GS, TG, GN, VI und HR], Attribut H stellt eine hydrophobe Zusammensetzung dar und Attribut M stellt Aminosäurehäufigkeiten in Multipletts dar, um 5 Arten von Zusammensetzungsattributen der Proteinsequenz zu quantifizieren, um jeweils numerische Eingangsvektoren für jedes der Attribute zu erhalten wobei die Summe der numerischen Eingabevektoren 105 ist;
[c] Verarbeiten der in Schritt [b] erhaltenen numerischen Eingabevektoren durch die Eingabeneuronenschicht, um Signale zu erhalten, wobei die Anzahl von Neuronen gleich der Anzahl von numerischen Eingabevektoren für jedes Attribut ist;
[d] Verarbeiten der aus Schritt [c] erhaltenen Signale durch die verborgene Schicht, um synaptisch gewichtete Signale zu erhalten, wobei die optimale Anzahl von Neuronen in der verborgenen Schicht durch Experimentieren zum Minimieren des Fehlers in der besten Epoche für jedes Netzwerk individuell bestimmt wurde;
[e] Liefern der aus Schritt [d] erhaltenen synaptisch gewichteten Signale an die Ausgabeschicht zum Zuordnen eines Wahrscheinlichkeitswertes für jede Proteinsequenz, die in Schritt [a] als ein Adhäsin von jedem Netzwerkmodul zugeführt wird; und
[f] Verwenden der aus Schritt [e] erhaltenen individuellen Wahrscheinlichkeiten zum Berechnen der endgültigen Wahrscheinlichkeit, dass eine Proteinsequenz ein Adhäsin ist, bezeichnet durch den Pad-Wert, der ein gewichteter Durchschnitt der aus Schritt [e] erhaltenen individuellen Wahrscheinlichkeiten und der zugehörigen Fraktion ist der Korrelation, die ein Maß für die Stärke der Vorhersage ist.

**14.** Verfahren nach Anspruch 13, wobei die Eingangsneuronenschicht aus insgesamt 105 Neuronen besteht, die 105 Zusammensetzungseigenschaften entsprechen.

**15.** Verfahren nach Anspruch 13, wobei die verborgene Schicht Neuronen umfasst, die als 30 für Aminosäurefrequenzen, 28 für Multiplettfrequenzen, 28 für Dipeptidfrequenzen, 30 für Ladungszusammensetzung und 30 für hydrophobe Zusammensetzung dargestellt sind.

**16.** Verfahren nach Anspruch 13, wobei die Ausgabeschicht aus Neuronen besteht, um die Ausgabewerte als Wahrscheinlichkeitswert für jede Proteinsequenz zu liefern.

## Revendications

**1.** Un procédé informatique mis en œuvre par ordinateur pour identifier l'adhésine et les protéines de type adhésine, ledit procédé comprenant les étapes:

a. de calcul des attributs basés sur la séquence de séquences protéiques à l'aide de cinq modules d'attributs d'un logiciel de réseau neuronal, où les attributs sont (i) les fréquences d'acides aminés, (ii) la fréquence de multiplet, (iii) les fréquences de dipeptide, (iv) la composition de charge, et (v) la composition hydrophobe,
b. de formation d'un réseau neuronal artificiel (ANN) pour chacun des cinq attributs calculés, et
c. d'identification des protéines présentant la probabilité d'être une adhésine (Pad) ≥0,51, où le "Pad" est une somme linéaire pondérée des probabilités de cinq attributs calculés, où

- la fréquence d'acides aminés

fi = (comptes du $i^{ème}$ acide aminé dans la séquence) / $\ell$;
i = 1...20,
$\ell$ est la longueur de la protéine;

- les multiplets sont définis comme étant des tronçons homopolymères $(X)_n$ où X est l'un des 20 acides aminés et n est un entier > 2 et après identification de tous les multiplets, les fréquences des acides aminés dans les multiplets ont été calculées comme étant

$$fi(m)=(\text{comptes du } i^{ème} \text{ acide aminé apparaissant sous forme de multiplet}) / \ell;$$

- la fréquence d'un dipeptide (i, j)

$$fij = (\text{comptes du } ij^{ème} \text{ dipeptide})/(\text{comptages dipeptidiques totaux}) ;$$

i, j est compris entre 1 et 20;
- la composition de charge fait référence à la fréquence d'entrée des acides aminés chargés (R, K, E et D compte tenu des propriétés d'ionisation des chaînes latérales à pH 7,2) donnée par

$$fc = (\text{nombre d'acides aminés chargés}) / \ell;$$

et à la distribution desdits acides aminés chargés dans la séquence protéique donnée qui est fournie par calcul des moments des positions des occurrences des acides aminés chargés et l'expression générale pour calculer les moments d'un ordre donné ; disons que 'i' est

$$Mr = \text{moment d'ordre } r^{ème} \text{ des positions des acides aminés chargés}$$

$$= \sum \frac{(X_i - X_m)^r}{N}$$

où, Xm = moyenne de toutes les positions d'aminos chargés
acides Xi = position du $i^{ème}$ acide aminé chargé
N = nombre d'acides aminés chargés dans la séquence ;

- la composition hydrophobe fait référence aux scores hydrophobes de cinq groupes d'acides aminés: (-8 pour K, E, D, R), (-4 pour S, T, N, Q), (-2 pour P, H), (+1 pour A, G, Y, C, W), (+2 pour L, V, I, F, M) avec les entrées suivantes données pour chacun des groupes,

(a) fi = (comptes du $i^{ème}$ groupe) / (comptes totaux dans la protéine), i varie de 1 à 5 ; et
(b) mji = moment d'ordre $j^{ème}$ des positions des acides aminés dans le groupe $i^{ème}$; j varie de 2 à 5.

**2.** Un procédé tel que revendiqué dans la revendication 1, dans lequel les séquences protéiques sont obtenues à partir d'agents pathogènes, d'eucaryotes et d'organismes multicellulaires.

**3.** Un procédé tel que revendiqué dans la revendication 1, dans lequel les séquences protéiques sont obtenues à partir des agents pathogènes choisis dans un groupe d'organismes comprenant *Escherichia coli, Haemophilus influenzae, Helicobacter pylori, Mycoplasma pneumoniae, Mycobacterium tuberculosis, Rickettsiae prowazekii, Porphyromonas gingivalis, Shigella flexneri, Streptococcas mutans* , *Streptococcus pneumoniae, Neisseria meningitidis, Streptococcus pyrogenes, Treponema pallidum* et coronavirus humain associé au syndrome respiratoire aigu sévère (SRAS).

**4.** Un procédé tel que revendiqué dans la revendication 1, dans lequel le procédé est un procédé sans homologie basé sur des propriétés de composition de séquence combinées à la puissance des réseaux de neurones artificiels pour identifier les adhésines et les protéines de type adhésine d'espèces appartenant à un large spectre phylogénétique.

**5.** Un procédé tel que revendiqué dans la revendication 1, dans lequel le procédé utilise 105 propriétés de composition des séquences.

**6.** Un procédé tel que revendiqué dans la revendication 1, dans lequel le procédé présente une sensibilité d'au moins 90 %.

**7.** Un procédé tel que revendiqué dans la revendication 1, dans lequel le procédé présente une spécificité de 100 %.

**8.** Un procédé tel que revendiqué dans la revendication 1, dans lequel le procédé aide à identifier les adhésines d'organismes apparentés à distance.

**9.** Un procédé tel que revendiqué dans la revendication 1, dans lequel le réseau de neurones présente une topologie multicouche à anticipation, constituée d'une couche d'entrée, d'une couche cachée et d'une couche de sortie.

**10.** Un procédé tel que revendiqué dans la revendication 9, dans lequel les nombres de neurones dans la couche d'entrée sont égaux au nombre de points de données d'entrée pour chaque attribut.

**11.** Un procédé tel que revendiqué dans la revendication 1, dans lequel chaque réseau formé attribue une valeur de probabilité d'être une adhésine pour la séquence protéique.

**12.** Un système informatique adapté pour mettre en œuvre le procédé de la revendication 1, ledit système comprenant:

- une unité centrale de traitement, exécutant un programme logiciel pour la prédiction de l'adhérence et des protéines de type adhérence à l'aide de réseaux de neurones, donnant des probabilités basées sur différents attributs à l'aide d'un réseau de neurones artificiels et dans d'autres programmes construits d'évaluation d'attributs, tous stockés dans un dispositif de mémoire accessible par l'unité centrale de traitement (CPU);
- un dispositif de mémoire accessible par le CPU ;
- un affichage sur lequel l'unité centrale de traitement affiche les écrans des programmes mentionnés ci-dessus en réponse aux entrées de l'utilisateur ; et,
- un dispositif d'interface utilisateur.

**13.** Méthode telle que revendiquée dans la revendication 1, dans lequel l'étape de formation (b) est réalisée avec un réseau de neurones artificiels à anticipation multicouche entièrement connecté, le réseau comprenant une couche d'entrée, une couche cachée et une couche de sortie qui sont connectées dans ladite séquence, dans lequel chaque neurone est un nombre binaire et est connecté à chaque neurone de la couche suivante pour identifier l'adhésine ou les protéines de type adhésine, dans lequel les étapes de programme comprennent:-

[a] alimentation d'une séquence protéique au format FASTA ;
[b] traitement de la séquence obtenue à l'étape [a] à travers les 5 modules nommés A, C, D, H et M, où l'attribut A représente une composition d'acides aminés, l'attribut C représente une composition de charge, l'attribut D représente une composition dipeptidique de les 20 dipeptides [NG, RE, TN, NT, GT, TT, DE, ER, RR, RK, RI, AT, TS, IV, SG, GS, TG, GN, VI et HR], l'attribut H représente une composition hydrophobe et l'attribut M représente les fréquences d'acides aminés en multiplets pour quantifier 5 types d'attributs de composition de ladite séquence protéique pour obtenir des vecteurs d'entrée numériques respectivement pour chacun desdits attributs dans lequel la somme des vecteurs d'entrée numériques est 105 ;
[c] traiter les vecteurs d'entrée numériques obtenus à l'étape [b] par la couche de neurones d'entrée pour obtenir des signaux, le nombre de neurones étant égal au nombre de vecteurs d'entrée numériques pour chaque attribut ;
[d] traiter les signaux obtenus à partir de l'étape [c] par la couche cachée pour obtenir des signaux pondérés synaptiques, dans lequel le nombre optimal de neurones dans la couche cachée a été déterminé par expérimentation pour minimiser l'erreur à la meilleure époque pour chaque réseau individuellement ;
[e] délivrer les signaux pondérés synaptiques obtenus à l'étape [d] à la couche de sortie pour attribuer une valeur de probabilité à chaque séquence protéique alimentée dans l'étape [a] comme étant une adhésine pour chaque module de réseau ; et
[f] en utilisant les probabilités individuelles obtenues à l'étape [e] pour calculer la probabilité finale qu'une séquence protéique soit une adhésine désignée par la valeur Pad, qui est une moyenne pondérée des probabilités individuelles obtenues à l'étape [e] et la fraction associée de corrélation qui est une mesure de la force de la prédiction

**14.** Méthode telle que revendiquée dans la revendication 13, dans lequel la couche de neurones d'entrée est constituée d'un total de 105 neurones correspondant à 105 propriétés de composition.

**15.** Méthode telle que revendiquée dans la revendication 13, dans lequel la couche cachée comprend des neurones représentés soit 30 pour les fréquences d'acides aminés, 28 pour les fréquences de multiplet, 28 pour les fréquences de dipeptide, 30 pour la composition de charge et 30 pour la composition hydrophobe.

**16.** Méthode telle que revendiquée dans la revendication 13, dans lequel la couche de sortie comprend des neurones pour délivrer les valeurs de sortie en tant que valeur de probabilité pour chaque séquence protéique.

The Neural Network architecture

**Figure1**

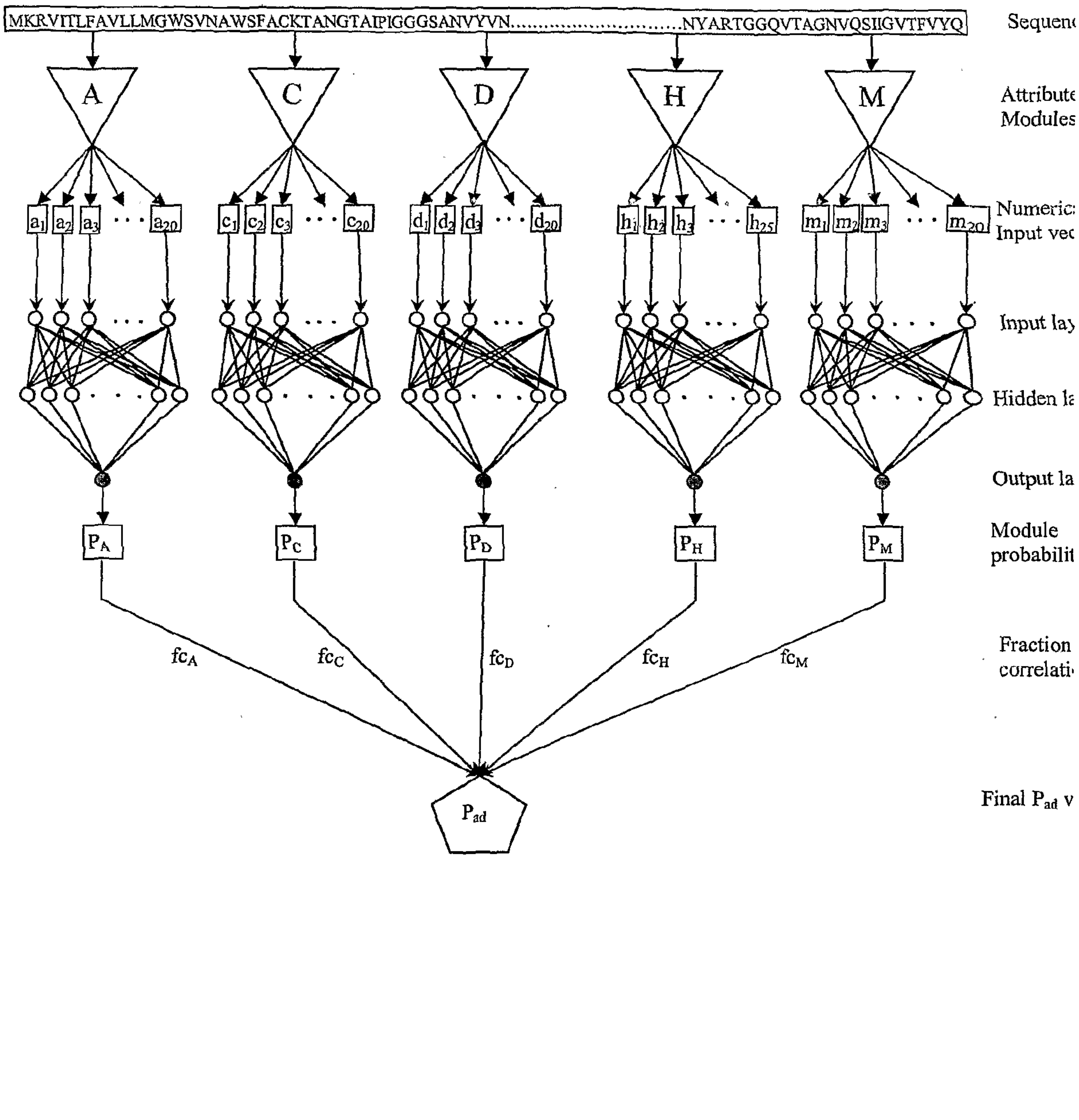

Assessment of SPAAN using defined test dataset.

**Figure 2**

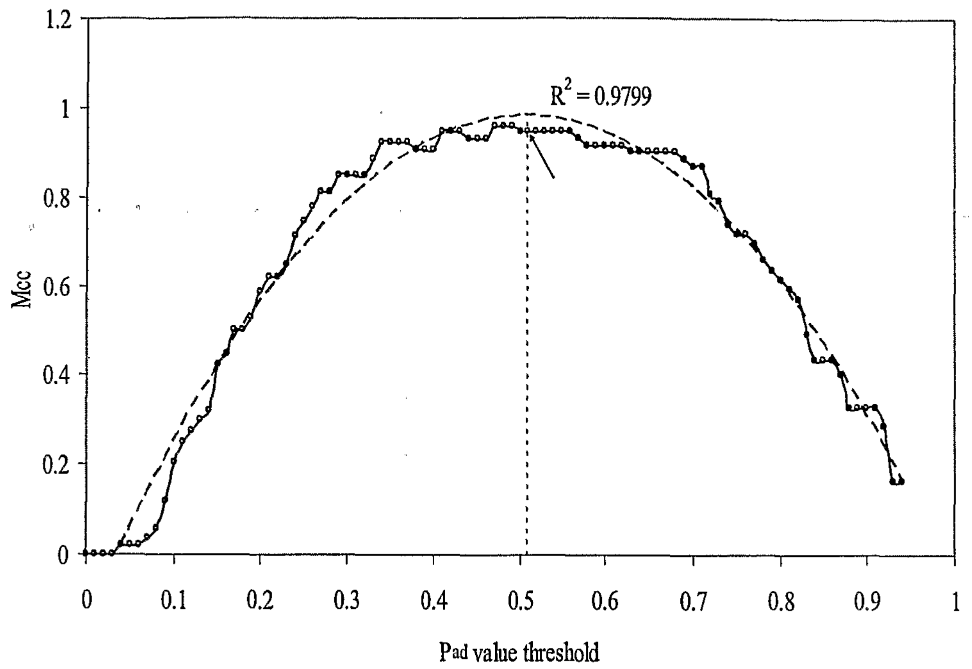

Figure 3 (a)

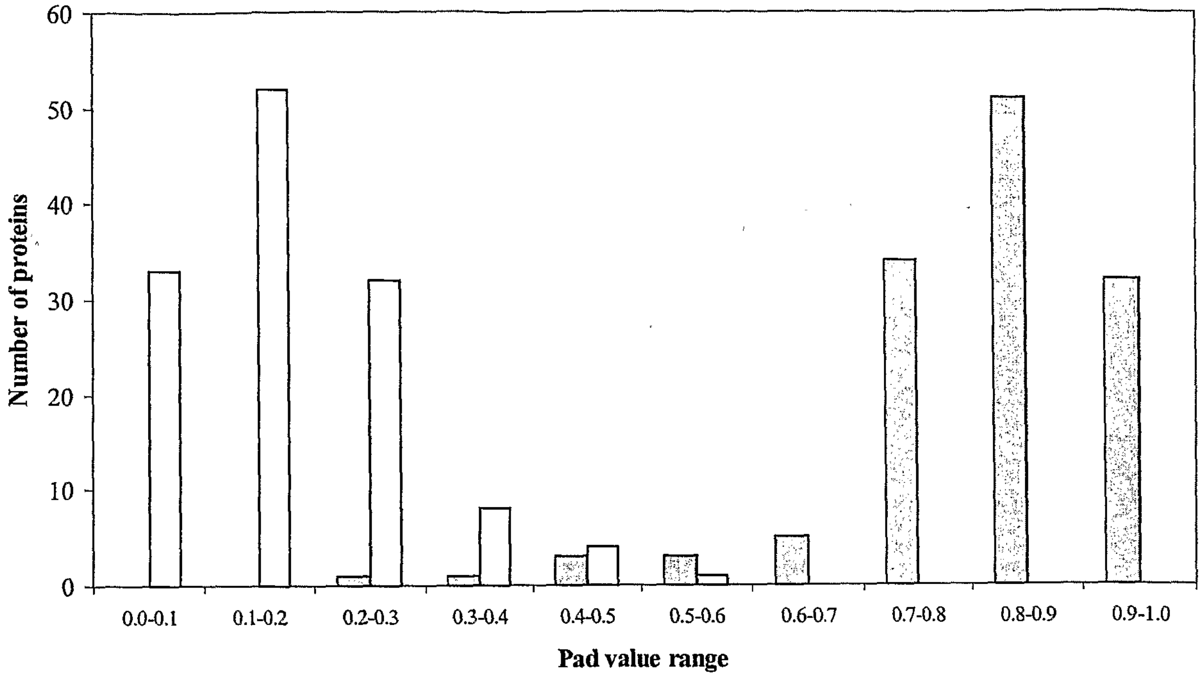
60
50
40
30
20
10
0
Number of proteins
0.0-0.1
0.1-0.2
0.2-0.3
0.3-0.4
0.4-0.5
0.5-0.6
0.6-0.7
0.7-0.8
0.8-0.9
0.9-1.0
Pad value range

Figure 3 (b)

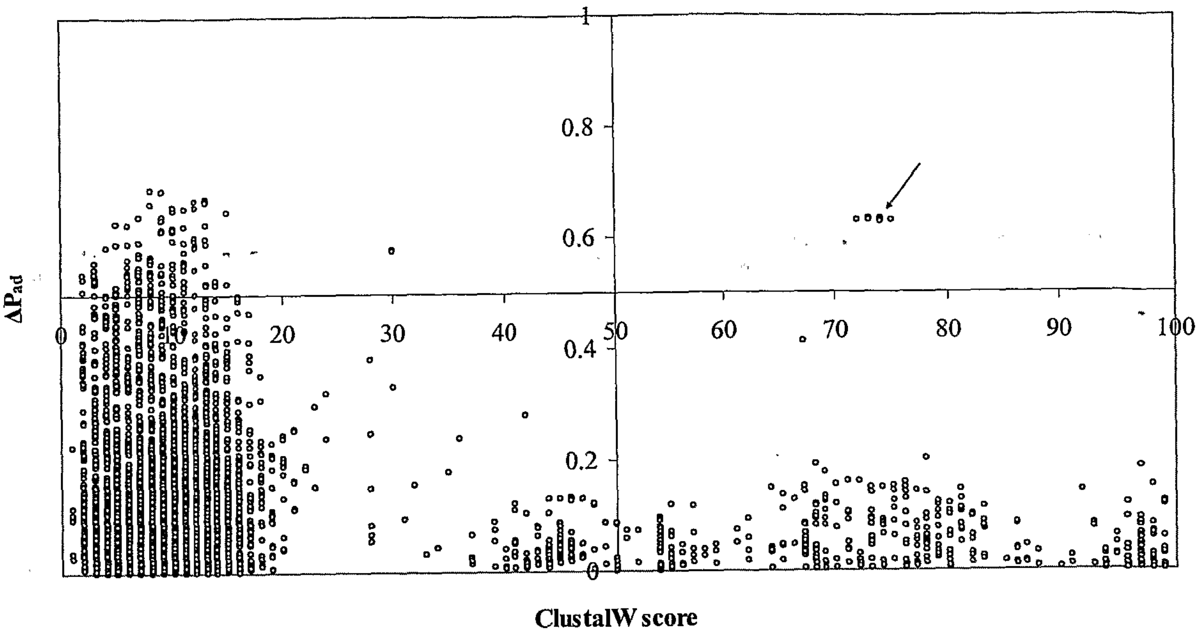

Figure 3(c)

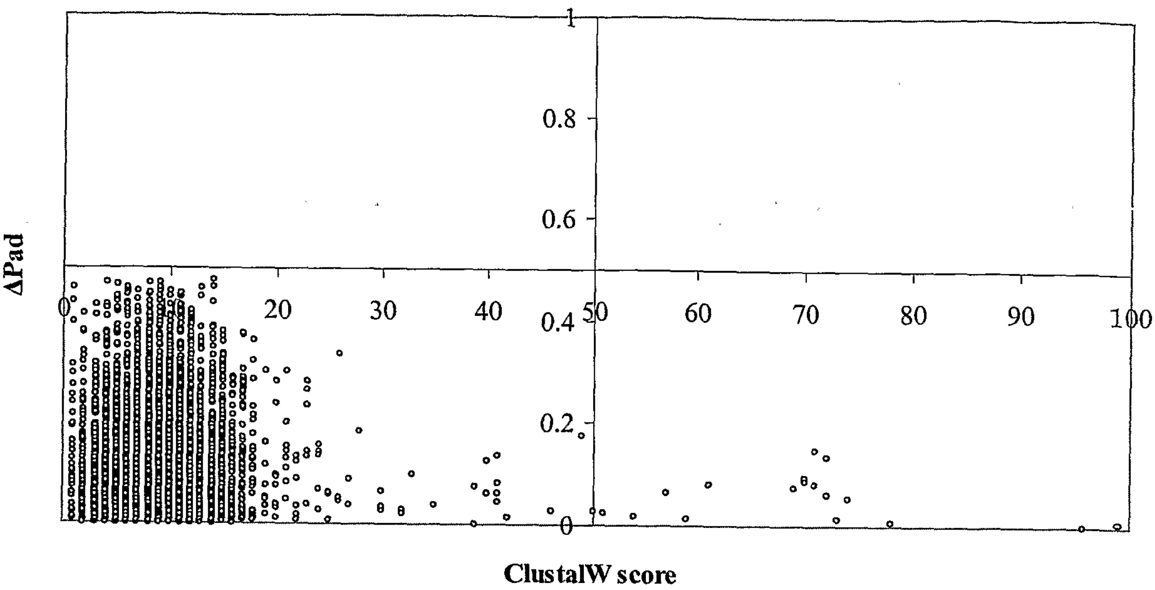

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FINLAY, B.B. ; FALKOW, S.** Common themes in microbial pathogenicity revisited. *MICROBIOLOGY AND MOLECULAR BIOLOGY REVIEWS,* 1997, vol. 61 (2), ISSN 1092-2172, 136-169
- Deciphering apicoplast targeting signals - feature extraction from nuclear-encoded precursors of Plasmodium falciparum apicoplast proteins. **ZUEGGE et al.** GENE: AN INTERNATIONAL JOURNAL ON GENES AND GENOMES. ELSEVIER, 12 December 2001, vol. 280, 19-26
- **BRADLEY PHIL et al.** BETAWRAP: Successful prediction of parallel beta-helices from primary sequence reveals an association with many microbial pathogens. *PROCEEDINGS OF THE NATIONAL ACADEMY OF SCIENCES OF THE UNITED STATES OF AMERICA,* 18 December 2001, vol. 98 (26), 14819-14824
- **CHARLES W. ANDERSON.** Department of Computer Science. Colorado State University
- **ANDREA, T.A. ; KALAYEH, H.** Applications of neural networks in quantitative structure-activity relationships of dihydrofolate reductase inhibitors. *J. Med. Chem.,* 1991, vol. 34, 2824-2836
- **ALTSCHUL SF ; GISH W ; MILLER W ; MYERS EW ; LIPMAN DJ.** Basic local alignment search tool. *J Mol Biol.,* 1990, vol. 215 (3), 403-410
- **BASSINET L ; GUEIRARD P ; MAITRE B ; HOUSSET B ; GOUNON P ; GUISO N.** Role of adhesins and toxins in invasion of human tracheal epithelial cells by Bordetella pertussis. *Infect Immun,* 2000, vol. 68 (4), 1934-1941
- **BOCK, K. et al.** Specificity of binding of a strain of uropathogenic Escherichia coli to Gal alpha 1----4Gal-containing glycosphingolipids. *J. Biol. Chem.,* 1985, vol. 260, 8545-8551
- **BRENDEL, V. ; BUCHER, P. ; NOURBAKHSH, I.R. ; EDWIN BLAISDELL, B. ; KARLIN, S.** Methods and algorithms for statistical analysis of protein sequences. *Proc. Natl. Acad. Sci. USA,* 1992, vol. 89, 2002-2006
- **BRENNAN, M.J., DELOGU, G., CHEN, Y., BARDAROV, S., KRIAKOV, J., ALAVI, M.** Evidence that Mycobacterial PE_PGRS proteins are cell surface constituents that influence interactions with other cells. *Infect. Immun,* 2001, vol. 69, 7326-7333
- **DE BK ; WOOLFITT AR ; BARR JR ; DANESHVAR MI ; SAMPSON JS ; ADES EW ; CARLONE GM.** Analysis of recombinant acylated pneumococcal surface adhesin A of Streptococcus pneumoniae by mass spectrometry. *Arch Biochem Biophys,* 2003, vol. 419 (2), 147-157
- **EGLAND PG ; DU LD ; KOLENBRANDER PE.** Identification of independent Streptococcus gordonii SspA and SspB functions in coaggregation with Actinomyces naeslundii. *Infect Immun,* 2001, vol. 69 (12), 7512-7516
- **FINLAY, B.B. ; FALKOW, S.** Common themes in microbial pathogenicity revisited. *Microbiol. Mol. Biol. Rev.,* 1997, vol. 61, 136-169
- **FRASER, C.M. ; EISEN, J. ; FLEISCHMANN, R.D. ; KETCHUM, K.A. ; PETERSON, S.** Comparative genomics and understanding of microbial biology. *Emerg. Infect. Dis.,* 2000, vol. 6, 505-6512
- **HALPERIN, S. A. ; SCHEIFELE, D. ; MILLS, E. ; GUASPARINI, R. ; HUMPHREYS, G. ; BARRETO, L. ; SMITH, B.** Nature, evolution, and appraisal of adverse events and antibody response associated with the fifth consecutive dose of a five-component acellular pertussis-based combination vaccine. *Vaccine,* 2003, vol. 21, 2298-2306
- **HARTFORD O ; MCDEVITT D ; FOSTER TJ.** Matrix-binding proteins of Staphylococcus aureus: functional analysis of mutant and hybrid molecules. *Microbiology,* 1999, vol. 145, 2497-2505
- **HOBOHM, U. ; SANDER, C.** A sequence property approach to searching protein databases. *J. Mol. Biol.,* 1995, vol. 251, 390-399
- **IDE T ; MICHGEHL S ; KNAPPSTEIN S ; HEUSIPP G ; SCHMIDT MA.** Differential modulation by Ca2+ of type III secretion of diffusely adhering enteropathogenic Escherichia coli. *Infect Immun,* 2003, vol. 71 (4), 1725-1732
- **LANGERMANN S et al.** Vaccination with FimH adhesin protects cynomolgus monkeys from colonization and infection by uropathogenic Escherichia coli. *J. Infect. Dis.,* 2000, vol. 181, 774-778
- **LOWE A.M. ; LAMBERT, P.A. ; SMITH, A.W.** Cloning of an Enterococcus faecalis endocarditis antigen: homology with adhesins from some oral streptococci. *Infect Immun.,* 1995, vol. 63, 703-706
- **MAURER, L. ; ORNDORFF, P.** Identification and characterization of genes determining receptor binding and pilus length of Escherichia coli type 1 pili. *J. Bacteriol.,* 1987, vol. 169, 640-645

- **MARCHLER-BAUER A ; PANCHENKO AR ; SHOEMAKER BA ; THIESSEN PA ; GEER LY ; BRYANT SH.** CDD: a database of conserved domain alignments with links to domain three-dimensional structure. *Nucleic Acids Res.,* 2002, vol. 30 (1), 281-283
- **NEUBAUER H ; HENSEL A ; ALEKSIC S ; MEYER H.** Evaluation of a Yersinia adhesion gene (yadA) specific PCR for the identification of enteropathogenic Yersinia enterocolitica. *Int J Food Microbiol.,* 2000, vol. 57 (3), 225-227
- **NISHIKAWA, K. ; KUBOTA, Y. ; OOI, T.** Classification of proteins into groups based on amino acid composition and other characters. II. grouping into four types. *J. Biochem.,* 1983, vol. 94, 997-1007
- **PEREGRIN-ALVAREZ, J.M. ; TSOKA, S. ; OUZOUNIS, C.A.** The phylogenetic extent of metabolic enzymes and pathways. *Genome Res,* 2003, vol. 13, 422-427
- **PRINZ, C. ; HAFSI, N. ; VOLAND, P.** Helicobacter pylori virulence factors and the host immune response: implications for therapeutic vaccination. *Trends in Microbiol.,* 2003, vol. 11, 134-138
- **RAPOLA, S. ; JÄNTTI, V. ; EEROLA, M. ; HELENA MÄKELÄ, P. ; KÄYHTY, H. ; KILPI, T.** Anti-PsaA and the risk of pneumococcal AOM and carriage. *Vaccine,* 2003, vol. 21, 3608-3613
- **RISON. S.C. ; TEICHMANN, S.A. ; THORNTON, J.M.** Homology, pathway distance and chromosomal localization of the small molecule metabolism enzymes in Escherichia coli. *J. Mol. Biol.,* 2002, vol. 318, 911-932
- **SPERANDIO V ; BAILEY C ; GIRON JA ; DIRITA VJ ; SILVEIRA WD ; VETTORE AL ; KAPER JB.** Cloning and characterization of the gene encoding the OmpU outer membrane protein of Vibrio cholerae. *Infect Immun.,* 1996, vol. 64 (12), 5406-5409
- **ST GEME JW ; CUTTER D.** The Haemophilus influenzae Hia adhesin is an autotransporter protein that remains uncleaved at the C terminus and fully cell associated. *J Bacteriol.,* 2000, vol. 182 (21), 6005-6013
- **THOMPSON, J.D. ; HIGGINS, D.G. ; GIBSON, T.J.** CLUSTAL W: improving the sensitivity of progressive multiple sequence alignment through sequence weighting, position-specific gap penalties and weight matrix choice. *Nucleic Acids Res.,* 1994, vol. 22, 4673-4680
- **VAN SCHILFGAARDE M ; VAN ULSEN P ; EIJK P ; BRAND M ; STAM M ; KOUAME J ; VAN ALPHEN L ; DANKERT J.** Characterization of adherence of nontypeable Haemophilus influenzae to human epithelial cells. *Infect Immun.,* 2000, vol. 68 (8), 4658-4665
- **WIZEMANN, T.M. ; ADAMOU, J.E. ; LANGERMANN, S.** Adhesins as targets for vaccine development. *Emerg. Infect. Dis.,* 1999, vol. 5, 395-403
- **WOLF, Y.I. ; ROGOZIN, I.B. ; KONDRASHOV, A.S. ; KOONIN, E.V.** Genome alignment, evolution of prokaryotic genome organization and prediction of gene function using genomic context. *Genome Res,* 2001, vol. 11, 356-372
- **YU J ; LEUNG WK ; GO MY ; CHAN MC ; TO KF ; NG EK ; CHAN FK ; LING TK ; CHUNG SC ; SUNG JJ.** Relationship between Helicobacter pylori babA2 status with gastric epithelial cell turnover and premalignant gastric lesions. *Gut,* 2002, vol. 51 (4), 480-484
- **ZUEGGE, J. ; RALPH, S. ; SCHMUKER, M. ; MCFADDEN, G.I. ; SCHNEIDER, G.** Deciphering apicoplast targeting signals--feature extraction from nuclear-encoded precursors of Plasmodium falciparum apicoplast proteins. *Gene,* 2001, vol. 280, 19-26